# EUROPEAN PATENT SPECIFICATION

(11) **EP 4 422 746 B1**
(45) Date of publication and mention of the grant of the patent: **02.12.2026**
(21) Application number: 22830974.6
(22) Date of filing: 28.10.2022
(51) Int. Cl.: A61P 1/16, A61P 13/12, C07C 237/38, C07C 255/60, C07C 311/46, C07D 207/09, C07D 213/56, C07D 249/04, C07D 261/20, C07D 275/06, C07D 277/28, C07D 285/12, C07D 307/87, C07D 309/08, C07D 413/06, C07D 413/12, C07D 417/12, C07D 471/04, C07D 493/04, C07D 498/04, A61K 31/4409

(54) **RXFP1 AGONISTS**
RXFP1-AGONISTEN
AGONISTES DE RXFP1

(30) Priority: 29.10.2021 US 202163273228 P
(43) Date of publication of application: 04.09.2024
(73) Proprietor: Bristol-Myers Squibb Company, Princeton, NJ 08543 (US)
(72) Inventor: SU, Shun, San Diego, CA 92130 (US); BILDER, Donna M., Princeton, NJ 08543 (US); CLARKE, Adam James, Princeton, NJ 08543 (US); FINLAY, Heather, Skillman, NJ 08558 (US); FRIENDS, Todd J., Chesterfield, NJ 08515 (US); MATHUR, Arvind, Princeton, NJ 08543 (US); MYERS, Michael C., Princeton, NJ 08543 (US); LAWRENCE, R. Michael, Princeton, NJ 08543 (US); LI, Jianqing, Cambridge, MA 02142 (US); PINTO, Donald J.P., Princeton, NJ 08543 (US); ORWAT, Michael J., Princeton, NJ 08543 (US); PABBISETTY, Kumar Balashanmuga, Princeton, NJ 08543 (US); SHAW, Scott A., Princeton, NJ 08543 (US); SMITH II, Leon M., Somerset, NJ 08873 (US); TORA, George O., Princeton, NJ 08543 (US); VOKITS, Benjamin P., Princeton, NJ 08543 (US); O'MALLEY, Daniel, La Jolla, CA 08543 (US); WURTZ, Nicholas R., Princeton, NJ 08543 (US); BHOGADI, Vikram, Bangalore 560099 (IN); PASUNOORI, Laxman, Bangalore 560099 (IN); SRINIVAS, Pitani Veera Venkata, Bangalore 560099 (IN); KUMAR, Sreekantha Ratna, Bangalore 560099 (IN); POTTURI, Hima Kiran, Bangalore 560099 (IN); HEGDE, Subramanya, Bangalore 560096 (IN)
(74) Representative: Dehns
(86) International application number: PCT/US2022/048277
(87) International publication number: WO 2023/076626

(56) References cited:
- WO-A1-2013/165606
- WO-A1-2022/122773

## Description

### BACKGROUND OF THE INVENTION

The present disclosure relates to novel compounds which are relaxin family peptide receptor 1 (RXFP1) agonists, compositions containing them, and the compounds for use, for example, in the treatment of heart failure, fibrotic diseases, and related diseases such as lung disease (e.g., idiopathic pulmonary fibrosis), kidney disease (e.g., chronic kidney disease), and hepatic disease (e.g., non-alcoholic steatohepatitis and portal hypertension).

The human relaxin hormone (also called relaxin or H2 relaxin) is a 6-kDa peptide composed of 53 amino acids whose activity was initially discovered when Frederick Hisaw in 1926 injected crude extracts from swine corpus luteum into virgin guinea pigs and observed a relaxation of the fibrocartilaginous pubic symphysis joint (Hisaw FL., Proc. Soc. Exp. Biol. Med., 1926, 23, 661-663*).* The relaxin receptor was previously known as Lgr7 but is now officially termed the relaxin family peptide receptor 1 (RXFP1) and was deorphanized as a receptor for relaxin in 2002 (Hsu SY., et al., Science, 2002, 295, 671-674). RXFP1 is reasonably well conserved between mouse and human with 85% amino acid identity and is essentially ubiquitously expressed in humans and in other species *(*Halls ML., et al., Br. J. Pharmacol., 2007, 150, 677-691). The cell signaling pathways for relaxin and RXFP1 are cell type dependent and quite complex (Halls ML., et al., Br. J. Pharmacol., 2007, 150, 677-691; Halls ML., et al. Ann. N YAcad. Sci., 2009, 1160, 108-111; Halls ML., Ann N Y Acad. Sci., 2007, 1160, 117-120). The best studied pathway is the relaxin-dependent increase in cellular levels of cAMP in which relaxin functions as an RXFP1 agonist to promote GαS coupling and activation of adenylate cyclase (Halls ML., et al., Mol. Pharmacol., 2006, 70, 214-226).

Since the initial discovery of relaxin much experimental work has focused on delineating the role relaxin has played in female reproductive biology and the physiological changes that occur during mammalian pregnancy (Sherwood OD., Endocr. Rev., 2004, 25, 205-234). During human gestation, in order to meet the nutritional demands imposed upon it by the fetus, the female body undergoes a significant ~30% decrease in systemic vascular resistance (SVR) and a concomitant ~50% increase in cardiac output (Jeyabalan AC., K.P., Renal and Electolyte Disorders. 2010, 462-518), (Clapp JF. & Capeless E., Am. J. Cardio., 1997, 80, 1469-1473). Additional vascular adaptations include an ~30% increase in global arterial compliance that is important for maintaining efficient ventricular-arterial coupling, as well as an ~50% increase in both renal blood flow (RBF) and glomerular filtration rate (GFR), important for metabolic waste elimination (Jeyabalan AC., K.P., Renal and Electolyte Disorders. 2010, 462-518), (Poppas A., et al., Circ., 1997, 95, 2407-2415). Both pre-clinical studies in rodents as well as clinical studies performed in a variety of patient settings, provide evidence that relaxin is involved, at least to some extent, in mediating these adaptive physiological changes (Conrad KP., Regul. Integr. Comp. Physiol., 2011, 301, R267-275), (Teichman SL., et al., Heart Fail. Rev., 2009, 14, 321-329). Importantly, many of these adaptive responses would likely be of benefit to HF patients in that excessive fibrosis, poor arterial compliance, and poor renal function are all characteristics common to heart failure patients (Mohammed SF., et al., Circ., 2015, 131, 550-559), (Wohlfahrt P., et al., Eur. J. Heart Fail., 2015, 17, 27-34), (Damman K., et al., Prog. Cardiovasc. Dis., 2011, 54, 144-153).

Heart failure (HF), defined hemodynamically as "systemic perfusion inadequate to meet the body's metabolic demands as a result of impaired cardiac pump function", represents a tremendous burden on today's health care system with an estimated United States prevalence of 5.8 million and greater than 23 million worldwide (Roger VL., et al., Circ. Res., 2013, 113, 646-659). It is estimated that by 2030, an additional 3 million people in the United States alone will have HF, a 25% increase from 2010. The estimated direct costs (2008 dollars) associated with HF for 2010 was $25 billion, projected to grow to $78 B by 2030 (Heidenreich PA., et al., Circ., 2011, 123, 933-944). Astoundingly, in the United States, 1 in 9 deaths has HF mentioned on the death certificate (Roger VL., et al., Circ., 2012, 125, e2-220) and, while survival after HF diagnosis has improved over time (Matsushita K., et al., Diabetes, 2010, 59, 2020-2026), (Roger VL., et al., JAMA, 2004, 292, 344-350), the death rate remains high with ~50% of people with HF dying within 5 years of diagnosis (Roger VL., et al., Circ., 2012, 125, e2-220), (Roger VL., et al., JAMA, 2004, 292, 344-350).

The symptoms of HF are the result of inadequate cardiac output and can be quite debilitating depending upon the advanced stage of the disease. Major symptoms and signs of HF include: 1) dyspnea (difficulty in breathing) resulting from pulmonary edema due to ineffective forward flow from the left ventricle and increased pressure in the pulmonary capillary bed; 2) lower extremity edema occurs when the right ventricle is unable to accommodate systemic venous return; and 3) fatigue due to the failing heart's inability to sustain sufficient cardiac output (CO) to meet the body's metabolic needs *(*Kemp CD., & Conte JV., Cardiovasc. Pathol., 2011, 21, 365-371). Also, related to the severity of symptoms, HF patients are often described as "compensated" or "decompensated". In compensated heart failure, symptoms are stable, and many overt features of fluid retention and pulmonary edema are absent. Decompensated heart failure refers to a deterioration, which may present as an acute episode of pulmonary edema, a reduction in exercise tolerance, and increasing breathlessness upon exertion (Millane T., et al., BMJ, 2000, 320, 559-562).

In contrast to the simplistic definition of poor cardiac performance not being able to meet metabolic demands, the large number of contributory diseases, multitude of risk factors, and the many pathological changes that ultimately lead to heart failure make this disease exceedingly complex (Jessup M. & Brozena S., N. Engl. J. Med., 2003, 348, 3007-2018). Injurious events thought to be involved in the pathophysiology of HF range from the very acute such as myocardial infarction to a more chronic insult such as lifelong hypertension. Historically, HF was primarily described as "systolic HF" in which decreased left-ventricular (LV) contractile function limits the expulsion of blood and hence results in a reduced ejection fraction (EF is stroke volume/end diastolic volume), or "diastolic HF" in which active relaxation is decreased and passive stiffness is increased limiting LV filling during diastole, however overall EF is maintained (Borlaug BA. & Paulus WJ., Eur Heart J., 2011, 32, 670-679). More recently, as it became understood that diastolic and systolic LV dysfunction was not uniquely specific to these two groups, new terminology was employed: "heart failure with reduced ejection fraction" (HFrEF), and "heart failure with preserved ejection fraction" (HFpEF) ( Borlaug BA. & Paulus WJ., Eur Heart J., 2011, 32, 670-679). Although these two patient populations have very similar signs and symptoms, whether HFrEF and HFpEF represent two distinct forms of HF or two extremes of a single spectrum sharing a common pathogenesis is currently under debate within the cardiovascular community (Borlaug BA. & Redfield MM., Circ., 2011, 123, 2006-2013), (De Keulenaer GW., & Brutsaert DL., Circ., 2011, 123, 1996-2004).

Serelaxin, an intravenous (IV) formulation of the recombinant human relaxin peptide with a relatively short first-phase pharmacokinetic half-life of 0.09 hours, is currently being developed for the treatment of HF (Novartis, 2014). Serelaxin has been given to normal healthy volunteers (NHV) and demonstrated to increase RBF (Smith MC., et al., J. Am. Soc. Nephrol. 2006, 17, 3192-3197) and estimated GFR (Dahlke M., et al., J. Clin. Pharmacol., 2015, 55, 415-422). Increases in RBF were also observed in stable compensated HF patients (Voors AA., et al., Cir. Heart Fail., 2014, 7, 994-1002). In large clinical studies, favorable changes in worsening renal function, worsening HF, as well as fewer deaths, were observed in acute decompensated HF (ADHF) patients in response to an in-hospital 48 hour IV infusion of serelaxin (Teerlink JR., et al., Lancet, 2013, 381, 29-39), (Ponikowski P., et al., Eur. Heart, 2014, 35, 431-441). Suggesting that chronic dosing of serelaxin could provide sustained benefit to HF patients, improvement in renal function based on serum creatinine levels was observed in scleroderma patients given serelaxin continuously for 6 months using a subcutaneous pump (Teichman SL., et al., Heart Fail. Rev., 2009, 14, 321-329). In addition to its potential as a therapeutic agent for the treatment of HF, continuous subcutaneous administration of relaxin has also been demonstrated to be efficacious in a variety of animal models of lung (Unemori EN., et al., J. Clin. Invet., 1996, 98, 2739-2745), kidney (Garber SL., et al., Kidney Int., 2001, 59, 876-882), and liver injury (Bennett RG., Liver Int., 2014, 34, 416-426).

In summary, a large body of evidence supports a role for relaxin-dependent agonism of RXFP1 mediating the adaptive changes that occur during mammalian pregnancy, and that these changes translate into favorable physiological effects and outcomes when relaxin is given to HF patients. Additional preclinical animal studies in various disease models of lung, kidney, and liver injury provide evidence that relaxin, when chronically administered, has the potential to provide therapeutic benefit for multiple indications in addition to HF. More specifically, chronic relaxin administration could be of benefit to patients suffering from lung disease (e.g., idiopathic pulmonary fibrosis), kidney disease (e.g., chronic kidney disease), or hepatic disease (e.g., non-alcoholic steatohepatitis and portal hypertension).

WO 2013/165606 relates to modulators of the human relaxin receptor 1 that are useful in treating mammalian relaxin receptor 1 mediated facets of human health, e.g. cardiovascular disease.

WO 2022/122773 relates to 4-(2-fluoro-4-methoxy-5-3-(((1-methylcyclobutyl)methyl)carbamoyl)bicyclo[2.2.1]heptan-2-yl) carbamoyl)phenoxy)-1-methylcyclohexane-1-carboxylic acid derivatives as RXFP1 modulators for the treatment of heart failure, heart failure with preserved ejection fraction, heart failure with mid-range ejection fraction, heart failure with reduced ejection fraction, chronic kidney disease and acute kidney injury.

### SUMMARY OF THE INVENTION

The present invention provides novel substituted norbornyl compounds, including stereoisomers, tautomers pharmaceutically acceptable salts, or solvates thereof, which are useful as RXFP1 receptor agonists.

Also described herein are processes and intermediates for making the compounds of the present invention.

The present invention also provides pharmaceutical compositions comprising a pharmaceutically acceptable carrier and at least one of the compounds of the present invention or pharmaceutically acceptable saltsthereof.

The compounds of the invention may be used, for example, in the treatment and/or prophylaxis of heart failure, fibrotic diseases, and related diseases, such as; lung disease (e.g., idiopathic pulmonary fibrosis), kidney disease (e.g., chronic kidney disease), or hepatic disease (e.g., non-alcoholic steatohepatitis and portal hypertension).

The compounds of the present invention may be used in therapy.

The compounds of the present invention may be used for the manufacture of a medicament for the treatment and/or prophylaxis of heart failure. The compounds of the invention can be used alone, in combination with other compounds of the present invention, or in combination with one or more, preferably one to two other agent(s).

These and other features of the invention will be set forth in expanded form as the disclosure continues.

### DESCRIPTION OF THE INVENTION

The invention encompasses compounds of Formula (I), which are RXFP1 receptor agonists and compositions containing them.

In a first aspect, the present invention provides, *inter alia,* compounds of Formula (I): 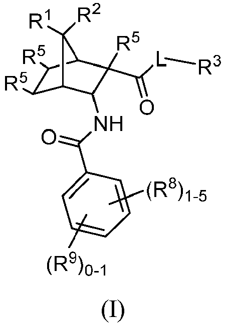 or pharmaceutically acceptable salts thereof, wherein:
L is -O- or -NH-;
R¹ and R² are combined to be =CR⁶R⁷ or =NOC₁₋₄ alkyl wherein "=" is a double bond; or R¹ and R² together with the carbon atom to which they are both attached form a dioxolanyl substituted with 0-1 aryl substituent;
R³ is C₁₋₈ alkyl substituted with 0-5 halo, CN, -OH, or -OC₁₋₃ alkyl substituents, - (CR^{d}R^{d})ₙ-C₃₋₁₀-carbocyclyl substituted with 0-5 R⁴, or -(CR^{d}R^{d})ₙ-3- to 12-membered heterocyclyl comprising 1-4 heteroatoms selected from O, S(=O)ₚ, N, and NR^{4c}, and substituted with 0-5 R⁴;
R⁴ is halo, CN, -OH, -SF₅, -S(=O)ₚR^{c}, C₁₋₄ alkyl substituted with 0-5 halo, -OH, or -OC₁₋₄ alkyl substituents, OC₁₋₄ alkyl substituted with 0-5 halo substituents, -(CR^{d}R^{d})ₙ-C₃₋₁₀ carbocyclyl substituted with 0-5 R^{e}, or -(CR^{d}R^{d})ₙ-4- to 6-membered heterocyclyl comprising 1-4 heteroatoms selected from O, S(=O)ₚ, N, and NR^{4c}, and substituted with 0-5 R^{e};
R^{4c} is H, C₁₋₄ alkyl, or -S(=O)₂CF₃;
each R⁵ is H, halo, -OH, C₁₋₄ alkyl substituted with 0-5 halo substituents, or -OC₁₋₄ alkyl substituted with 0-5 halo substituents;
R⁶ is H, halo, CN, C₁₋₇ alkyl substituted with 0-3 R^{6a}, C₂₋₇ alkenyl substituted with 0-3 R^{6a}, C₂₋₇ alkynyl substituted with 0-3 R^{6a}, -C(=O)OR^{6b}, -CONR^{6b}R^{6b}, -(CH₂)ₙ-C₃₋₁₀ carbocyclyl substituted with 0-5 R¹⁴, or 3- to 12-membered heterocyclyl comprising 1-4 heteroatoms selected from O, S(=O)ₚ, N, or NR^{14a}, and substituted with 0-5 R¹⁴;
R^{6a} is halo, -OH, -OC₁₋₄ alkyl, C₁₋₄ alkyl, aryl, or C₃₋₆ cycloalkyl substituted with 0-4 halo substituents;
R^{6b} is H, C₁₋₄ alkyl substituted with 0-1 aryl substituent, or C₃₋₆ cycloalkyl substituted with 0-4 halo substituents;
R⁷ is H or C₁₋₄ alkyl;
or R⁶ and R⁷ together with the carbon atom to which they are both attached form a cyclopentadienyl, an indanyl, or an indenyl;
R⁸ is H, halo, CN, -NR⁷R⁷, C₁₋₄ alkyl substituted with 0-5 halo or -OH substituents, or - OC₁₋₄ alkyl substituted with 0-5 halo, -OH, C₃₋₆ cycloalkyl, aryl, 4- to 9-membered heterocyclyl comprising 1-4 heteroatoms selected from O, S(=O)ₚ, and N, or -OC₁₋₃ alkyl substituted with 0-1 -OC₁₋₃ alkyl substituents;
R⁹ is aryl substituted with 0-3 R¹⁰ and 0-2 R¹¹ or 3- to 12-membered heterocyclyl comprising 1-5 heteroatoms selected from O, S(=O)ₚ, N, and NR^{11a}, and substituted with 0-3 R¹⁰ and 0-2 R¹¹;
R¹⁰ is halo, CN, C₁₋₄ alkyl, =O, -OH, or -OC₁₋₄ alkyl;
R¹¹ is C₁₋₄ alkyl substituted with 0-4 R¹² and 0-2 R¹³, -OR^{b}, -NR^{a}R^{a}, -NR^{a}C(=O)R^{b}, - NR^{a}C(=O)OR^{b}, -NR^{a}C(=O)NR^{a}R^{a}, -NR^{a}S(=O)ₚR^{c}, -C(=O)R^{b}, -C(=O)OR^{b}, -C(=O)NR^{a}R^{a}, -C(=O)NR^{a}S(=O)ₚR^{c}, -OC(=O)R^{b}, -S(=O)ₚR^{c}, -S(=O)ₚNR^{a}R^{a}, C₃₋₉ carbocyclyl substituted with 0-5 R^{e}, or 3- to 12-membered heterocyclyl comprising 1-4 heteroatoms selected from O, S(=O)ₚ, N, and NR¹⁵, and substituted with 0-5 R^{e};
R^{11a} is H, C₁₋₅ alkyl substituted with 0-4 R^{11b}, -C(=O)R^{b}, -C(=O)OR^{b}, -C(=O)NR^{a}R^{a}, C₃₋₆ cycloalkyl substituted with 0-5 R^{e}, aryl substituted with 0-5 R^{e}, 4- to 6-membered heterocyclyl comprising 1-4 heteroatoms selected from O, S(=O)ₚ, N, and NR¹⁵, and substituted with 0-5 R^{e};
R^{11b} is halo, -OH, -C(=O)OH, -C(=O)OC₁₋₄ alkyl, or aryl;
R¹² is halo, -C(=O)OR^{b}, -C(=O)NR^{a}R^{a}, -C(=O)NR^{a}OR^{b}, C₁₋₄ alkyl substituted with 0-3 halo or -OH substituents, or C₃₋₆ cycloalkyl;
R¹³ is -OR^{b}, -NR^{a}R^{a}, -NR^{a}C(=O)R^{b}, -NR^{a}C(=O)OR^{b}, -NR^{a}C(=O)NR^{a}R^{a}, -NR^{a}S(=O)ₚR^{c}, -NR^{a}S(=O)ₚNR^{a}R^{a}, -OC(=O)NR^{a}R^{a}, -OC(=O)NR^{a}OR^{b}, -S(=O)ₚNR^{a}R^{a}, - S(=O)ₚR^{c}, -(CH₂)ₙ-C₃₋₁₀ carbocyclyl substituted with 0-3 R^{e}, or (CH₂)ₙ-3- to 12-membered heterocyclyl comprising 1-4 heteroatoms selected from O, S(=O)ₚ, and N, and substituted with 0-3 R^{e};
R¹⁴ is halo, CN, C₁₋₄ alkyl substituted with 0-3 halo substituents, -OC₁₋₄ alkyl substituted with 0-3 halo substituents, -(CH₂)ₙ-NR^{a}R^{a}, -(CH₂)ₙ-aryl substituted with 0-3 R^{e}, - O-aryl substituted with 0-3 R^{e}, or -(CH₂)ₙ-3- to 12-membered heterocyclyl comprising 1-4 heteroatoms selected from O, S(=O)ₚ, and N, and substituted with 0-3 R^{e};
R^{14a} is H, C(=O)C₁₋₄ alkyl, or C₁₋₃ alkyl substituted with 0-3 Si(C₁₋₃ alkyl)₃ or aryl substituted with 0-2 halo substituents;
R¹⁵ is H, C₁₋₄ alkyl, or aryl;
R^{a} is H, -OC₁₋₆ alkyl, C₁₋₆ alkyl substituted with 0-5 R^{e}, C₂₋₆ alkenyl substituted with 0-5 R^{e}, C₂₋₆ alkynyl substituted with 0-5 R^{e}, (CH₂)ₙC₃₋₁₀ carbocyclyl substituted with 0-5 R^{e}, or (CH₂)ₙ-3- to 12-membered heterocyclyl comprising 1-4 heteroatoms selected from O, S(=O)ₚ, and N, and substituted with 0-5 R^{e}; or R^{a} and R^{a} together with the nitrogen atom to which they are both attached form a 3- to 12-membered heterocyclyl comprising 1-4 heteroatoms selected from O, S(=O)ₚ, and N, and substituted with 0-5 R^{e};
R^{b} is H, C₁₋₆ alkyl substituted with 0-5 R^{e}, C₂₋₆ alkenyl substituted with 0-5 R^{e}, C₂₋₆ alkynyl substituted with 0-5 R^{e}, -(CH₂)ₙ-C₃₋₁₀ carbocyclyl substituted with 0-5 R^{e}, or (CH₂)ₙ-3- to 12-membered heterocyclyl comprising 1-4 heteroatoms selected from O, S(=O)ₚ, and N, and substituted with 0-5 R^{e};
R^{c} is C₁₋₆ alkyl substituted with 0-5 R^{e}, C₂₋₆ alkenyl substituted with 0-5 R^{e}, C₂₋₆ alkynyl substituted with 0-5 R^{e}, C₃₋₆ carbocyclyl substituted with 0-5 R^{e}, or 3- to 12-membered heterocyclyl comprising 1-4 heteroatoms selected from O, S(=O)ₚ, and N, and substituted with 0-5 R^{e};
R^{d} is H, C₁₋₄ alkyl, or C₃₋₆ cycloalkyl;
R^{e} is halo, CN, NO₂, =O, C₁₋₆ alkyl substituted with 0-5 R^{g}, C₂₋₆ alkenyl substituted with 0-5 -R^{g}, C₂₋₆ alkynyl substituted with 0-5 -R^{g}, -(CH₂)ₙ-C₃₋₁₀ carbocyclyl substituted with 0-5 R^{g}, -(CH₂)ₙ-3- to 12-membered heterocyclyl comprising 1-4 heteroatoms selected from O, S(=O)ₚ, and N, and substituted with 0-5 R^{g}, - (CH₂)ₙOR^{f}, -C(=O)OR^{f}, -C(=O)NR^{f}R^{f}, -NR^{f}C(=O)R^{f}, -S(=O)ₚR^{f}, -S(=O)ₚNR^{f}R^{f}, - NR^{f}S(=O)ₚR^{f}, -NR^{f}C(=O)OR^{f}, -OC(=O)NR^{f}R^{f}, or -(CH₂)ₙNR^{f}R^{f};
R^{f} is H, C₁₋₆ alkyl substituted with 0-2 -OH or -OC₁₋₄ alkyl substituents, C₃₋₆ cycloalkyl, aryl, or 3- to 12-membered heterocyclyl comprising 1-4 heteroatoms selected from O, S(=O)ₚ, and N and; or R^{f} and R^{f} together with the nitrogen atom to which they are both attached form a 3- to 12-membered heterocyclyl comprising 1-4 heteroatoms selected from O, S(=O)ₚ, and N;
R^{g} is halo, CN, -OH, C₁₋₆ alkyl, C₃₋₆ cycloalkyl, or aryl;
n is zero, 1, 2, or 3; and
p is zero, 1, or 2.

In a second aspect within the scope of the first aspect, the present invention provides compounds of Formula (I) or pharmaceutically acceptable salts thereof, wherein:
R³ is C₁₋₆ alkyl substituted with 0-4 halo or -OH substituents, -(CHR^{d})₀₋₁-C₃₋₆ cycloalkyl substituted with 0-4 R⁴, C₆₋₉ spirocycloalkyl substituted with 0-4 R⁴, C₆₋₁₀ bicyclic carbocyclyl substituted with 0-4 R⁴, or 3 to 6-membered heterocyclyl comprising 1-2 heteroatoms selected from O, S(=O)ₚ, N, and NR^{4c}, and substituted with 0-4 R⁴;
R⁴ is halo or C₁₋₃ alkyl substituted with 0-4 halo substituents;
R^{4c} is H or C₁₋₄ alkyl;
R^{d} is C₁₋₃ alkyl.

In a third aspect within the scope of the first aspect, the present invention provides compounds of Formula (II): 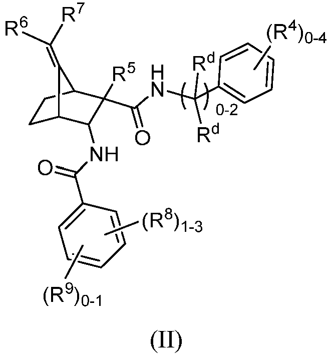 or pharmaceutically acceptable salts thereof, wherein:
R⁴ is halo, -S(=O)ₚC₁₋₄ alkyl substituted with 0-4 halo substituents, C₁₋₄ alkyl substituted with 0-4 halo substituents, -OC₁₋₄ alkyl substituted with 0-4 halo substituents;
R⁵ is H or halo;
R⁶ is halo, CN, C₁₋₇ alkyl substituted with 0-3 R^{6a}, C₂₋₇ alkenyl substituted with 0-3 R^{6a}, C₂₋₇ alkynyl substituted with 0-3 R^{6a}, -C(=O)OR^{6b}, CONR^{6b}R^{6b}, C₃₋₆ cycloalkyl substituted with 0-3 R¹⁴, C₃₋₆ cycloalkenyl substituted with 0-3 R¹⁴, aryl substituted with 0-3 R¹⁴, or 4- to 6-membered heterocyclyl comprising 1-3 heteroatoms selected from O, S(=O)ₚ, N, and NR^{14a} and substituted with 0-3 R¹⁴;
R^{6a} is halo, -OH, C₃₋₆ cycloalkyl, or aryl;
R^{6b} is H, C₁₋₄ alkyl substituted with 0-1 aryl substituent, or C₃₋₆ cycloalkyl substituted with 0-4 halo substituents;
R⁷ is H or C₁₋₃ alkyl;
R⁸ is halo, CN, -N(C₁₋₂ alkyl)₂, C₁₋₄ alkyl substituted with 0-5 halo or -OH substituents, or -OC₁₋₄ alkyl substituted with 0-4 halo, -OH, aryl, or -OC₁₋₄ alkyl substituents;
R⁹ is C₆ aryl substituted with 0-3 R¹⁰ and 0-2 R¹¹, or 3- to 12-membered heterocyclyl comprising 1-4 heteroatoms selected from O, S(=O)ₚ, N, and NR^{11a}, and substituted with 0-3 R¹⁰ and 0-1 R¹¹;
R¹⁰ is halo, CN, C₁₋₄ alkyl, =O, -OH, or -OC₁₋₄ alkyl;
R¹¹ is C₁₋₄ alkyl substituted with 0-1 R¹² and 0-1 R¹³, -OR^{b}, -NR^{a}R^{a}, -NR^{a}C(=O)R^{b}, - NR^{a}C(=O)OR^{b}, -NR^{a}C(=O)NR^{a}R^{a}, -NR^{a}S(=O)ₚR^{c}, -C(=O)R^{b}, -C(=O)OR^{b}, -C(=O)NR^{a}R^{a}, -C(=O)NR^{a}S(=O)ₚR^{c}, -OC(=O)R^{b}, -S(=O)ₚR^{c}, -S(=O)ₚNR^{a}R^{a}, C₃₋₆ cycloalkyl substituted with 0-5 R^{e}, 4- to 12-membered heterocyclyl comprising 1-4 heteroatoms selected from O, S(=O)ₚ, N, and NR¹⁵, and substituted with 0-5 R^{e};
R^{11a} is H, C₁₋₄ alkyl substituted with 0-2 R^{11b}, -C(=O)R^{b}, -C(=O)OR^{b}, -C(=O)NR^{a}R^{a}, C₃₋₆ cycloalkyl substituted with 0-5 R^{e}, 4- to 6-membered heterocyclyl comprising 1-4 heteroatoms selected from O, S(=O)ₚ, N, and NR¹⁵, and substituted with 0-5 R^{e};
R^{11b} is -OH, -C(=O)OH, or aryl;
R¹² is halo, -C(=O)OR^{b}, -C(=O)NHR^{a}, -C(=O)NHOR^{b}, or C₁₋₄ alkyl substituted with 0-3 halo or -OH substituents;
R¹³ is -OR^{b}, -NR^{a}R^{a}, -NR^{a}C(=O)R^{b}, -NR^{a}C(=O)OR^{b}, -NR^{a}S(=O)ₚR^{c}, -NR^{a}S(=O)ₚNR^{a}R^{a}, -OC(=O)NR^{a}R^{a}, -OC(=O)NR^{a}OR^{b}, -S(=O)ₚNR^{a}R^{a}, or -S(=O)ₚR^{c};
R¹⁴ is halo, CN, C₁₋₄ alkyl substituted with 0-3 halo substituents, -OC₁₋₄ alkyl substituted with 0-3 halo substituents, -(CH₂)₀₋₂-NR^{a}R^{a}, -(CH₂)₀₋₃-aryl substituted with 0-3 R^{e}, -O-aryl substituted with 0-3 R^{e}, or -(CH₂)₀₋₃-3- to 12-membered heterocyclyl comprising 1-4 heteroatoms selected from O, S(=O)ₚ, and N, and substituted with 0-3 R^{e};
R^{14a} is H, C(=O)C₁₋₄ alkyl, or C₁₋₃ alkyl substituted with 0-3 aryl substituted with 0-2 halo substituents;
R¹⁵ is H, C₁₋₃ alkyl, or aryl;
R^{a} is H, C₁₋₅ alkyl substituted with 0-5 R^{e}, C₂₋₅ alkenyl substituted with 0-5 R^{e}, C₂₋₅ alkynyl substituted with 0-5 R^{e}, -(CH₂)ₙ-C₃₋₁₀ carbocyclyl substituted with 0-5 R^{e}, or -(CH₂)ₙ-3- to 12-membered heterocyclyl comprising 1-4 heteroatoms selected from O, S(=O)ₚ, and N, and substituted with 0-5 R^{e}; or R^{a} and R^{a} together with the nitrogen atom to which they are both attached form a 3- to 12-membered heterocyclyl comprising 1-4 heteroatoms selected from O, S(=O)ₚ, and N, and substituted with 0-5 R^{e};
R^{b} is H, C₁₋₅ alkyl substituted with 0-5 R^{e}, C₂₋₅ alkenyl substituted with 0-5 R^{e}, C₂₋₅ alkynyl substituted with 0-5 R^{e}, -(CH₂)ₙ-C₃₋₁₀ carbocyclyl substituted with 0-5 R^{e}, or -(CH₂)ₙ-3- to 12-membered heterocyclyl comprising 1-4 heteroatoms selected from O, S(=O)ₚ, and N, and substituted with 0-5 R^{e};
R^{c} is C₁₋₅ alkyl substituted with 0-5 R^{e}, C₂₋₅ alkenyl substituted with 0-5 R^{e}, C₂₋₅ alkynyl substituted with 0-5 R^{e}, C₃₋₆ carbocyclyl substituted with 0-5 R^{e}, or 3- to 12-membered heterocyclyl comprising 1-4 heteroatoms selected from O, S(=O)ₚ, and N, and substituted with 0-5 R^{e};
R^{d} is H or C₁₋₄ alkyl;
R^{e} is halo, CN, =O, C₁₋₆ alkyl substituted with 0-5 R^{g}, C₂₋₆ alkenyl substituted with 0-5 R^{g}, C₂₋₆ alkynyl substituted with 0-5 R^{g}, -(CH₂)ₙ-C₃₋₆ cycloalkyl substituted with 0-5 R^{g}, -(CH₂)ₙ-aryl substituted with 0 5 R^{g}, -(CH₂)ₙ-3- to 12-membered heterocyclyl comprising 1-4 heteroatoms selected from O, S(=O)ₚ, and N, and substituted with 0-4 R^{g}, , -(CH₂)ₙOR^{f}, -C(=O)OR^{f}, -C(=O)NR^{f}R^{f}, -NR^{f}C(=O)R^{f}, - S(=O)ₚR^{f}, -NR^{f}C(=O)OR^{f}, -OC(=O)NR^{f}R^{f}, or -(CH₂)ₙNR^{f}R^{f};
R^{f} is H, C₁₋₅ alkyl, C₃₋₆ cycloalkyl, or aryl; or R^{f} and R^{f} together with the nitrogen atom to which they are both attached form a 3- to 9-membered heterocyclyl;
R^{g} is halo, CN, -OH, C₁₋₅ alkyl, C₃₋₆ cycloalkyl, or aryl;
n is zero, 1, 2, or 3; and
p is zero, 1, or 2.

In a fourth aspect within the scope of the first aspect, the present invention provides compounds of Formula (III): 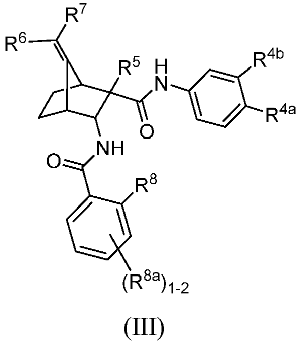 or pharmaceutically acceptable salts thereof, wherein:
R^{4a} is halo;
R^{4b} is C₁₋₄ alkyl substituted with 0-4 halo substituents;
R⁵ is H or F;
R⁶ is halo, C₁₋₄ alkyl substituted with 0-3 R^{6a}, C₂₋₄ alkenyl substituted with 0-1 phenyl or - OH substituent, -C(=O)OR^{6b}, C(=O)NHR^{6b}, C₃₋₆ cycloalkyl substituted with 0-3 R¹⁴, C₃₋₆ cycloalkenyl substituted with 0-3 R¹⁴, phenyl substituted with 0-3 R¹⁴, naphthyl substituted with 0-3 R¹⁴, or 5- to 6-membered heterocyclyl comprising 1-3 heteroatoms selected from O, S, N, and NR^{14a} and substituted with 0-3 R¹⁴;
R^{6a} is halo, -OH, C₃₋₆ cycloalkyl, or phenyl;
R^{6b} is H or C₁₋₄ alkyl;
R⁷ is H or C₁₋₃ alkyl;
or R⁶ and R⁷ together with the carbon atom to which they are both attached form a cyclopentadienyl, an indanyl, or an indenyl;
R⁸ is -N(C₁₋₄ alkyl)₂ or -OC₁₋₄ alkyl substituted with 0-1 -OC₁₋₄ alkyl substituent;
R^{8a} is halo;
R¹⁴ is halo, CN, C₁₋₄ alkyl substituted with 0-3 halo substituents, -OC₁₋₄ alkyl substituted with 0-3 halo substituents, -(CH₂)₀₋₂-NR^{a}R^{a}, -(CH₂)₀₋₂-aryl substituted with 0-3 R^{e}, -O-aryl substituted with 0-3 R^{e}, or -(CH₂)₀₋₂-3- to 12-membered heterocyclyl comprising 1-4 heteroatoms selected from O, S(=O)ₚ, and N, and substituted with 0-3 R^{e};
R^{14a} is H, C(=O)C₁₋₃ alkyl, or C₁₋₃ alkyl substituted with 0-3 aryl substituted with 0-2 halo substituents;
R^{a} is H, C₁₋₆ alkyl substituted with 0-5 R^{e}, -(CH₂)ₙ-phenyl substituted with 0-5 R^{e}, or -(CH₂)ₙ-3- to 12-membered heterocyclyl comprising 1-4 heteroatoms selected from O, S(=O)ₚ, and N, and substituted with 0-5 R^{e}; or R^{a} and R^{a} together with the nitrogen atom to which they are both attached form a 3- to 12-membered heterocyclyl comprising 1-4 heteroatoms selected from O, S(=O)ₚ, and N, and substituted with 0-5 R^{e};
R^{b} is H, C₁₋₆ alkyl substituted with 0-5 R^{e}, -(CH₂)₀₋₁-phenyl substituted with 0-5 R^{e}, or -(CH₂)ₙ-3- to 12-membered heterocyclyl comprising 1-4 heteroatoms selected from O, S(=O)ₚ, and N, and substituted with 0-5 R^{e};
R^{e} is halo, CN, =O, C₁₋₆ alkyl, or C(=O)OH; and
n is zero, 1, 2, or 3.

In a fifth aspect within the scope of the first and third aspects, the present invention provides compounds of Formula (IV): 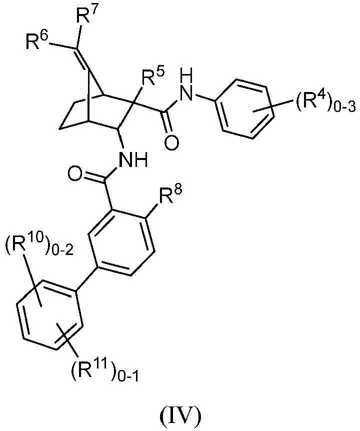 or pharmaceutically acceptable salts thereof, wherein:
R⁴ is halo, C₁₋₄ alkyl substituted with 0-3 halo substituents, or -OC₁₋₄ alkyl substituted with 0-3 halo substituents;
R⁵ is H or F;
R⁶ is halo, CN, C₁₋₆ alkyl substituted with 0-3 R^{6a}, C₂₋₆ alkenyl substituted with 0-3 R^{6a}, C₂₋₆ alkynyl substituted with 0-3 R^{6a}, -C(=O)OR^{6b}, C(=O)NR^{6b}R^{6b}, C₃₋₆ cycloalkyl substituted with 0-3 R¹⁴, C₃₋₆ cycloalkenyl substituted with 0-3 R¹⁴, phenyl substituted with 0-3 R¹⁴, or 5- to 6-membered heteroaryl comprising 1-3 heteroatoms selected from O, S(=O)ₚ, N, and NR^{14a}, and substituted with 0-3 R¹⁴;
R^{6a} is halo, C₃₋₆ cycloalkyl, or phenyl;
R^{6b} is H, C₁₋₃ alkyl substituted with 0-1 aryl substituent, or C₃₋₆ cycloalkyl substituted with 0-4 halo substituents;
R⁷ is H or C₁₋₂ alkyl;
R⁸ is -OC₁₋₄ alkyl substituted with 0-4 halo, -OH, aryl or -OC₁₋₄ alkyl substituents;
R¹⁰ is halo, CN, C₁₋₃ alkyl, -OH, or -OC₁₋₄ alkyl;
R¹¹ is C₁₋₄ alkyl substituted with 0-2 R¹² and 0-1 R¹³, -OR^{b}, -NR^{a}R^{a}, -NR^{a}C(=O)R^{b}, - NR^{a}C(=O)NR^{a}R^{a}, -NR^{a}S(=O)ₚR^{c}, -C(=O)R^{b}, -C(=O)OR^{b}, -C(=O)NR^{a}R^{a}, -C(=O)NR^{a}S(=O)ₚR^{c}, -OC(=O)R^{b}, -S(=O)ₚR^{c}, -S(=O)ₚNR^{a}R^{a}, C₃₋₆ cycloalkyl, 4-to 9-membered heterocyclyl comprising 1-4 heteroatoms selected from O, S(=O)ₚ, N, and NR¹⁵, and substituted with 0-4 R^{e};
R¹² is halo, -C(=O)OR^{b}, -C(=O)NHR^{a}, -C(=O)NHOR^{b}, or C₁₋₄ alkyl substituted with 0-3 halo or -OH substituents;
R¹³ is -OR^{b}, -NR^{a}R^{a}, -NR^{a}C(=O)R^{b}, -NR^{a}C(=O)OR^{b}, -NR^{a}S(=O)ₚR^{c}, -NR^{a}S(=O)ₚNR^{a}R^{a}, -OC(=O)NR^{a}R^{a}, -OC(=O)NR^{a}OR^{b}, -S(=O)ₚNR^{a}R^{a}, or -S(=O)ₚR^{c};
R¹⁴ is halo, CN, C₁₋₄ alkyl substituted with 0-3 halo substituents, -OC₁₋₄ alkyl substituted with 0-3 halo substituents, -(CH₂)₀₋₂-NR^{a}R^{a}, -(CH₂)₀₋₂-aryl substituted with 0-3 R^{e}, -O-aryl substituted with 0-3 R^{e}, or -(CH₂)₀₋₂-3- to 12-membered heterocyclyl comprising 1-4 heteroatoms selected from O, S(=O)ₚ, and N, and substituted with 0-3 R^{e};
R^{14a} is H, C(=O)C₁₋₃alkyl, C₁₋₃ alkyl substituted with 0-2 aryl substituted with 0-2 halo substituents;
R¹⁵ is H, C₁₋₂ alkyl, or phenyl;
R^{a} is H, C₁₋₅ alkyl substituted with 0-4 R^{e}, C₂₋₅ alkenyl substituted with 0-4 R^{e}, C₂₋₅ alkynyl substituted with 0-4 R^{e}, -(CH₂)ₙ-C₃₋₁₀ carbocyclyl substituted with 0-4 R^{e}, or -(CH₂)ₙ-3- to 12-membered heterocyclyl comprising 1-4 heteroatoms selected from O, S(=O)ₚ, and N, and substituted with 0-4 R^{e}; or R^{a} and R^{a} together with the nitrogen atom to which they are both attached form a 3- to 12-membered heterocyclyl comprising 1-4 heteroatoms selected from O, S(=O)ₚ, and N, and substituted with 0-4 R^{e};
R^{b} is H, C₁₋₅ alkyl substituted with 0-4 R^{e}, C₂₋₅ alkenyl substituted with 0-4 R^{e}, C₂₋₅ alkynyl substituted with 0-4 R^{e}, -(CH₂)ₙ-C₃₋₁₀ carbocyclyl substituted with 0-4 R^{e}, or -(CH₂)ₙ-3- to 12-membered heterocyclyl comprising 1-4 heteroatoms selected from O, S(=O)ₚ, and N, and substituted with 0-4 R^{e};
R^{c} is C₁₋₅ alkyl substituted with 0-4 R^{e}, C₂₋₅ alkenyl substituted with 0-4 R^{e}, C₂₋₅ alkynyl substituted with 0-4 R^{e}, C₃₋₆ carbocyclyl, or 3- to 12-membered heterocyclyl comprising 1-4 heteroatoms selected from O, S(=O)ₚ, and N;
R^{e} is halo, CN, NO₂, =O, C₁₋₆ alkyl substituted with 0-5 R^{g}, C₂₋₆ alkenyl substituted with 0-5 R^{g}, C₂₋₆ alkynyl substituted with 0-5 R^{g}, -(CH₂)ₙ-C₃₋₆ cycloalkyl, - (CH₂)ₙ-aryl, -(CH₂)ₙ-3- to 12-membered heterocyclyl comprising 1-4 heteroatoms selected from O, S(=O)ₚ, and N, -(CH₂)ₙOR^{f}, S(=O)ₚR^{f}, C(=O)NR^{f}R^{f}, C(=O)OR^{f}, NR^{f}C(=O)R^{f}, S(=O)ₚNR^{f}R^{f}, NR^{f}S(=O)ₚR^{f}, NR^{f}C(=O)OR^{f}, OC(=O)NR^{f}R^{f}, or -(CH₂)ₙNR^{f}R^{f};
R^{f} is H, C₁₋₆ alkyl, C₃₋₆ cycloalkyl, or aryl; or R^{f} and R^{f} together with the nitrogen atom to which they are both attached form a heterocyclyl;
R^{g} is halo, CN, -OH, C₁₋₅ alkyl, C₃₋₆ cycloalkyl, or aryl;
n is zero, 1, 2, or 3; and
p is zero, 1, or 2.

In a sixth aspect within the scope of the first aspect, the present invention provides compounds of Formula (V): 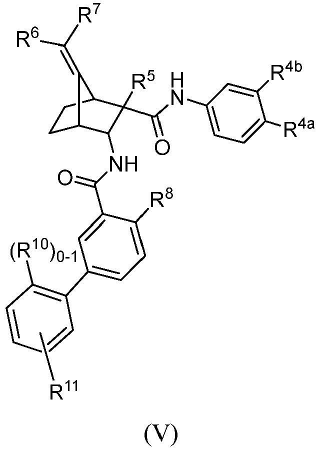 or pharmaceutically acceptable salts thereof, wherein:
R^{4a} is halo or C₁₋₂ alkyl;
R^{4b} is C₁₋₄ alkyl substituted with 0-4 halo substituents;
R⁵ is H or F;
R⁶ is halo, CN, C₁₋₄ alkyl substituted with 0-3 R^{6a}, C₂₋₄ alkenyl substituted with 0-3 R^{6a}, - C(=O)OR^{6b}, C(=O)ONR^{6b}R^{6b}, C₃₋₆ cycloalkyl substituted with 0-3 R¹⁴, phenyl substituted with 0-3 R¹⁴, or 5- to 6-membered heteroaryl comprising 1-3 heteroatoms selected from O, S(=O)ₚ, N, and NR^{14a} and substituted with 0-3 R¹⁴;
R^{6a} is halo, -OH, C₃₋₆ cycloalkyl, or phenyl;
R^{6b} is H, C₁₋₃ alkyl substituted with 0-1 aryl substituent, or C₃₋₆ cycloalkyl;
R⁷ is H or C₁₋₂ alkyl;
R⁸ is -OC₁₋₄ alkyl substituted with 0-4 halo, -OH, -OC₁₋₄ alkyl, or aryl substituents;
R¹⁰ is halo or C₁₋₃ alkyl;
R¹¹ is C₁₋₄ alkyl substituted with 0-2 R¹² and 0-1 R¹³, -OH, -OC₁₋₄ alkyl, -NR^{a}C(=O)R^{b}, - NR^{a}C(=O)NR^{a}R^{a}, -NR^{a}S(=O)ₚR^{c}, -C(=O)R^{b}, -C(=O)OR^{b}, -C(=O)NR^{a}R^{a}, -C(=O)NR^{a}S(=O)ₚR^{c}, -OC(=O)R^{b}, -S(=O)ₚR^{c}, -S(=O)ₚNR^{a}R^{a}, C₃₋₆ cycloalkyl, 4-to 9-membered heterocyclyl comprising 1-4 heteroatoms selected from O, S(=O)ₚ, N, and NR¹⁵, and substituted with 0-3 R^{e};
R¹² is halo, -C(=O)OR^{b}, -C(=O)NHR^{a}, -C(=O)NHOR^{b}, or C₁₋₄ alkyl substituted with 0-3 halo or -OH substituents;
R¹³ is -OR^{b}, -NR^{a}R^{a}, -NR^{a}C(=O)R^{b}, -NR^{a}C(=O)OR^{b}, -NR^{a}S(=O)ₚR^{c}, -NR^{a}S(=O)ₚNR^{a}R^{a}, -OC(=O)NR^{a}R^{a}, or -OC(=O)NR^{a}OR^{b};
R¹⁴ is halo, CN, C₁₋₄ alkyl substituted with 0-3 halo substituents, -OC₁₋₄ alkyl substituted with 0-3 halo substituents, -(CH₂)₀₋₂-NR^{a}R^{a}, -(CH₂)₀₋₁-aryl substituted with 0-3 R^{e}, -O-aryl substituted with 0-3 R^{e}, or -(CH₂)₀₋₁-3- to 9-membered heterocyclyl comprising 1-4 heteroatoms selected from O, S(=O)ₚ, and N, and substituted with 0-3 R^{e};
R^{14a} is H, C(=O)C₁₋₃ alkyl, C₁₋₃ alkyl substituted with 0-1 aryl substituted with 0-2 halo substituents;
R¹⁵ is H, C₁₋₂ alkyl, or phenyl;
R^{a} is H, C₁₋₄ alkyl substituted with 0-5 R^{e}, C₂₋₄ alkenyl substituted with 0-5 R^{e}, C₂₋₄ alkynyl substituted with 0-5 R^{e}, -(CH₂)ₙ-C₃₋₁₀ carbocyclyl substituted with 0-5 R^{e}, or -(CH₂)ₙ-3- to 12-membered heterocyclyl comprising 1-4 heteroatoms selected from O, S(=O)ₚ, and N, and substituted with 0-5 R^{e}; or R^{a} and R^{a} together with the nitrogen atom to which they are both attached form a 3- to 9-membered heterocyclyl comprising 1-4 heteroatoms selected from O, S(=O)ₚ, and N, and substituted with 0-5 R^{e};
R^{b} is H, C₁₋₄ alkyl substituted with 0-5 R^{e}, C₂₋₄ alkenyl substituted with 0-5 R^{e}, C₂₋₄ alkynyl substituted with 0-5 R^{e}, -(CH₂)ₙ-C₃₋₁₀ carbocyclyl substituted with 0-5 R^{e}, or -(CH₂)ₙ-3- to 12-membered heterocyclyl comprising 1-4 heteroatoms selected from O, S(=O)ₚ, and N, and substituted with 0-5 R^{e};
R^{c} is C₁₋₄ alkyl substituted with 0-5 R^{e}, C₂₋₄ alkenyl substituted with 0-5 R^{e}, C₂₋₄ alkynyl substituted with 0-5 R^{e}, C₃₋₆ carbocyclyl, or 3- to 9-membered heterocyclyl comprising 1-4 heteroatoms selected from O, S(=O)ₚ, and N;
R^{e} is halo, CN, =O, C₁₋₆ alkyl substituted with 0-5 R^{g}, C₂₋₆ alkenyl substituted with 0-5 R^{g}, C₂₋₆ alkynyl substituted with 0-5 R^{g}, -(CH₂)ₙ-C₃₋₆ cycloalkyl, - (CH₂)ₙ-aryl, -(CH₂)ₙ-4- to 6-membered heterocyclyl comprising 1-4 heteroatoms selected from O, S(=O)ₚ, and N, -(CH₂)ₙOR^{f}, S(=O)ₚR^{f}, C(=O)NR^{f}R^{f}, C(=O)OR^{f}, NR^{f}C(=O)R^{f}, S(=O)ₚNR^{f}R^{f}, NR^{f}S(=O)ₚR^{f}, NR^{f}C(=O)OR^{f}, OC(=O)NR^{f}R^{f}, or -(CH₂)ₙNR^{f}R^{f};
R^{f} is H, C₁₋₆ alkyl, C₃₋₆ cycloalkyl, or aryl; or R^{f} and R^{f} together with the nitrogen atom to which they are both attached form a heterocyclyl;
R^{g} is, halo, CN, -OH, C₁₋₆ alkyl, C₃₋₆ cycloalkyl, or aryl;
n is zero, 1, 2, or 3; and
p is zero, 1, or 2.

In one embodiment of Formula (V), R^{4a} is F or CH₃; R^{4b} is CF₃; R⁶ is phenyl or 5-membered heteroaryl comprising 1-2 heteroatoms selected from O and N; R⁷ is H; R⁸ is - OC₁₋₂alkyl; R¹⁰ is halo; R¹¹ is -CH₃, -CH₂CH₃, -CF₃ -OCF₃, -NHS(=O)₂C₁₋₂ alkyl, - C(=O)OH, -C(=O)OC₁₋₄ alkyl, -C(=O)NHC₁₋₄ alkyl substituted with 0-1 R^{e}, or a 5-membered heterocyclyl comprising 1-4 heteroatoms selected from O, N, and NR¹⁵ and substituted with 0-3 R^{e}; and R^{e} is =O or C(=O)OH.

In a seventh aspect within the scope of the sixth aspect, the present invention provides compounds of Formula (V) or pharmaceutically acceptable salts thereof, wherein:
R^{4a} is halo;
R^{4b} is CF₃;
R⁶ is C₁₋₄ alkyl substituted with 0-3 halo substituents or C₃₋₆ cycloalkyl substituted with 0-3 halo substituents;
R⁸ is -OC₁₋₄ alkyl;
R¹⁰ is F;
R¹¹ is -OH, -OC₁₋₄ alkyl, -NR^{a}C(=O)R^{b}, -NR^{a}S(=O)ₚR^{c}, -C(=O)OR^{b}, -C(=O)NR^{a}R^{a}, -C(=O)NR^{a}S(=O)ₚR^{c}, 4 to 9-membered heterocyclyl comprising 1-4 heteroatoms selected from O, S(=O)ₚ, N, and NR¹⁵, and substituted with 0-3 R^{e};
R¹⁵ is H or C₁₋₂ alkyl;
R^{a} is H or C₁₋₄ alkyl substituted with 0-5 R^{e};
or R^{a} and R^{a} together is 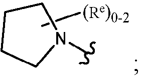
R^{b} is H or C₁₋₄ alkyl substituted with 0-5 R^{e};
R^{c} is C₁₋₃ alkyl substituted with 0-5 R^{e} or C₃₋₆ carbocyclyl;
R^{e} is halo, =O, C₁₋₄ alkyl substituted with 0-5 R^{g}, C(=O)OH, -OR^{f}, or -NR^{f}R^{f}; and
R^{f} is H and C₁₋₆ alkyl; or R^{f} and R^{f} together with the nitrogen atom to which they are both attached form a heterocyclyl; and
R^{g} is halo.

In an eighth aspect within the scope of the first aspect, the present invention provides compounds of Formula (VI): 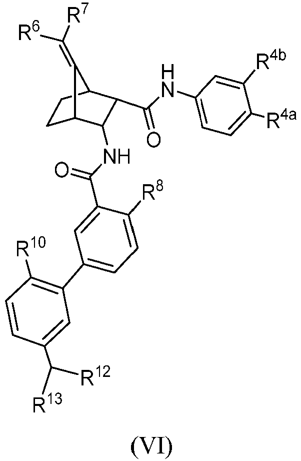 or pharmaceutically acceptable salts thereof, wherein:
R^{4a} is halo;
R^{4b} is CF₃;
R⁶ is C₁₋₄ alkyl substituted with 0-3 halo substituents or C₃₋₆ cycloalkyl substituted with 0-3 halo substituents;
R⁷ is H;
R⁸ is -OC₁₋₄alkyl substituted with 0-1 aryl substituent;
R¹⁰ is halo;
R¹² is -C(=O)OH, -C(=O)OC₁₋₄ alkyl, -C(=O)NHC₁₋₄ alkyl, -C(=O)NHOC₁₋₃ alkyl, or C₁₋₃ alkyl substituted with 0-3 halo substituents;
R¹³ is -OR^{b}, -NR^{a}R^{a}, -NR^{a}C(=O)R^{b}, -NR^{a}C(=O)OR^{b}, -NR^{a}S(=O)ₚR^{c}, -NR^{a}S(=O)ₚNR^{a}R^{a}, -OC(=O)NR^{a}R^{a}, or -OC(=O)NR^{a}OR^{b};
R^{a} is H, C₁₋₄ alkyl substituted with 0-5 halo substituents, phenyl substituted with 0-4 R^{e}, C₃₋₁₀ cycloalkyl substituted with 0-4 R^{e}, spirocycloalkyl substituted with 0-4 R^{e}, or 3- to 9-membered heterocyclyl comprising 1-4 heteroatoms selected from O, S(=O)ₚ, and N, and substituted with 0-4 R^{e}; or R^{a} and R^{a} together with the nitrogen atom to which they are both attached form a 3- to 12-membered heterocyclyl comprising 1-4 heteroatoms selected from O, S(=O)ₚ, and N, and substituted with 0-4 R^{e};
R^{b} is H, C₁₋₄ alkyl substituted with 0-5 R^{e}, -(CH₂)ₙ-phenyl substituted with 0-4 halo substituents, C₃₋₆ cycloalkyl substituted with 0-4 halo substituents, or 3- to 12-membered heterocyclyl comprising 1-4 heteroatoms selected from O, S(=O)ₚ, and N, and substituted with 0-4 R^{e};
R^{c} is C₁₋₄ alkyl substituted with 0-4 R^{e},
R^{e} is halo, CN, =O, C₁₋₅ alkyl substituted with 0-5 R^{g}, C₃₋₆ cycloalkyl, aryl, 4- to 6-membered heterocyclyl comprising 1-4 heteroatoms selected from O, S(=O)ₚ, and N, or -OR^{f};
R^{f} is H, C₁₋₄ alkyl, C₃₋₆ cycloalkyl or aryl;
R^{g} is halo;
n is zero or 1; and
p is zero, 1, or 2.

In a ninth aspect within the scope of the eighth aspect, the present invention provides compounds of Formula (VI) or pharmaceutically acceptable salts thereof, wherein:
R^{4a} is F
R^{4b} is CF₃;
R⁶ is CF₃ or C₃₋₆ cycloalkyl;
R⁸ is -OCH₃ or -OCH₂-phenyl;
R¹⁰ is F;
R¹² is -C(=O)OH, -C(=O)OC₁₋₄ alkyl, -C(=O)NHC₁₋₄ alkyl, -C(=O)NHOC₁₋₄ alkyl, CH₃, CHF₂, or CF₃;
R¹³ is -OH, -NR^{a}R^{a}, -NHC(=O)R^{b}, -NHS(=O)ₚC₁₋₄ alkyl, -OC(=O)NR^{a}R^{a}, or - OC(=O)NHOC₁₋₄ alkyl;
R^{a} is H, C₁₋₄ alkyl substituted with 0-4 F substituents, 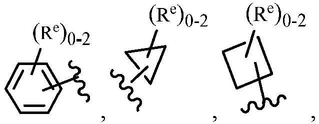 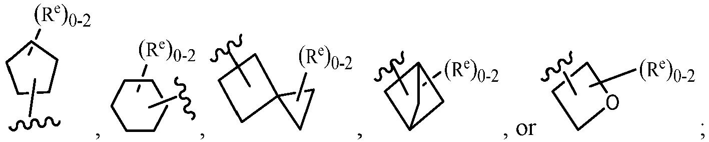
or R^{a} and R^{a} together is 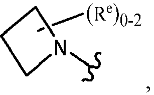
R^{b} is H, C₁₋₄ alkyl substituted with 0-5 R^{e}, phenyl, or 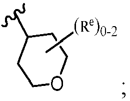 and
R^{e} is halo, =O, aryl, 4- to 6-membered heterocyclyl comprising 1-4 heteroatoms selected from O, S(=O)ₚ, and N, or -OR^{f}; and
R^{f} is H, C₁₋₃ alkyl, C₃₋₆ cycloalkyl, or phenyl.

In a tenth aspect within the scope of the third aspect, the present invention provides compounds of Formula (VII): 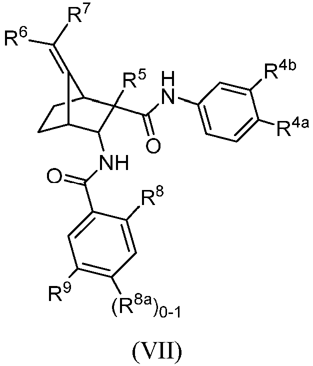 or pharmaceutically acceptable salts thereof, wherein:
R^{4a} is halo;
R^{4b} is C₁₋₄ alkyl substituted with 0-3 halo substituents, or -OC₁₋₄ alkyl substituted with 0-3 halo substituents;
R⁵ is H or F;
R⁶ is halo, CN, C₁₋₆ alkyl substituted with 0-3 R^{6a}, C₂₋₆ alkenyl substituted with 0-3 R^{6a}, C₂₋₆ alkynyl substituted with 0-3 R^{6a}, C₃₋₆ cycloalkyl substituted with 0-3 R¹⁴, C₃₋₆ cycloalkenyl substituted with 0-3 R¹⁴, phenyl substituted with 0-3 R¹⁴, or 5- to 6-membered heteroaryl comprising 1-3 heteroatoms selected from O, S(=O)ₚ, N, and NR^{14a}, and substituted with 0-3 R¹⁴;
R^{6a} is halo, C₃₋₆ cycloalkyl, or phenyl;
R⁷ is H or C₁₋₂ alkyl;
R⁸ is halo, CN, or -OC₁₋₄ alkyl substituted with 0-4 halo, -OH, or -OC₁₋₄ alkyl substituents;
R^{8a} is halo or CN;
R⁹ is a 3- to 12-membered heterocyclyl comprising 1-4 heteroatoms selected from O, S(=O)ₚ, N, and NR^{11a}, and substituted with 0-3 R¹⁰ and 0-1 R¹¹;
R¹⁰ is halo, CN, C₁₋₃ alkyl, =O, -OH, or -OC₁₋₃ alkyl;
R¹¹ is C₁₋₃ alkyl substituted with 0-1 R¹² and 0-1 R¹³, -OR^{b}, -NR^{a}R^{a}, -NR^{a}C(=O)R^{b}, - NR^{a}C(=O)OR^{b}, -NR^{a}C(=O)NR^{a}R^{a}, -NR^{a}S(=O)ₚR^{c}, -C(=O)R^{b}, -C(=O)OR^{b}, -C(=O)NR^{a}R^{a}, -C(=O)NR^{a}S(=O)ₚR^{c}, -OC(=O)R^{b}, -S(=O)ₚR^{c}, -S(=O)ₚNR^{a}R^{a}, C₃₋₆ cycloalkyl substituted with 0-5 R^{e}, 4- to 6-membered heterocyclyl comprising 1-4 heteroatoms selected from O, S(=O)ₚ, N, and NR¹⁵, and substituted with 0-4 R^{e};
R^{11a} is H, C₁₋₄ alkyl substituted with 0-2 R^{11b}, -C(=O)R^{b}, -C(=O)OR^{b}, -C(=O)NR^{a}R^{a}, C₃₋₆ cycloalkyl, 4- to 6-membered heterocyclyl comprising 1-4 heteroatoms selected from O, S(=O)ₚ, N, and NR¹⁵, and substituted with 0-4 R^{e};
R^{11b} is -OH, -C(=O)OH, or aryl;
R¹² is -C(=O)OR^{b}, -C(=O)NHR^{a}, -C(=O)NHOR^{b}, or C₁₋₄ alkyl substituted with 0-3 halo or -OH substituents;
R¹³ is -OR^{b}, -NR^{a}R^{a}, -NR^{a}C(=O)R^{b}, -NR^{a}C(=O)OR^{b}, -NR^{a}S(=O)ₚR^{c}, -NR^{a}S(=O)ₚNR^{a}R^{a}, -OC(=O)NR^{a}R^{a}, -S(=O)ₚNR^{a}R^{a}, or -S(=O)ₚR^{c};
R¹⁴ is halo, CN, C₁₋₄ alkyl substituted with 0-3 halo, -OC₁₋₄ alkyl substituted with 0-3 halo, -(CH₂)₀₋₂-NR^{a}R^{a}, -(CH₂)₀₋₂-aryl substituted with 0-3 R^{e}, -O-aryl substituted with 0-3 R^{e}, or -(CH₂)₀₋₂-3- to 12-membered heterocyclyl comprising 1-4 heteroatoms selected from O, S(=O)ₚ, and N, and substituted with 0-3 R^{e};
R^{14a} is H, C(=O)C₁₋₃ alkyl, or C₁₋₃ alkyl substituted with 0-2 aryl substituted with 0-2 halo substituents;
R¹⁵ is H, C₁₋₂ alkyl, or phenyl;
R^{a} is H, C₁₋₅ alkyl substituted with 0-4 R^{e}, C₂₋₅ alkenyl substituted with 0-4 R^{e}, C₂₋₅ alkynyl substituted with 0-4 R^{e}, -(CH₂)ₙ-C₃₋₁₀ carbocyclyl substituted with 0-4 R^{e}, or -(CH₂)ₙ-3- to 12-membered heterocyclyl comprising 1-4 heteroatoms selected from O, S(=O)ₚ, and N, and substituted with 0-4 R^{e}; or R^{a} and R^{a} together with the nitrogen atom to which they are both attached form a 3- to 12-membered heterocyclyl comprising 1-4 heteroatoms selected from O, S(=O)ₚ, and N, and substituted with 0-4 R^{e};
R^{b} is H, C₁₋₅ alkyl substituted with 0-4 R^{e}, C₂₋₅ alkenyl substituted with 0-4 R^{e}, C₂₋₅ alkynyl substituted with 0-4 R^{e}, -(CH₂)ₙ-C₃₋₁₀ carbocyclyl substituted with 0-4 R^{e}, or -(CH₂)ₙ-3- to 12-membered heterocyclyl comprising 1-4 heteroatoms selected from O, S(=O)ₚ, and N, and substituted with 0-4 R^{e};
R^{c} is C₁₋₅ alkyl substituted with 0-4 R^{e}, C₂₋₅ alkenyl substituted with 0-4 R^{e}, C₂₋₅ alkynyl substituted with 0-4 R^{e}, C₃₋₆ carbocyclyl, or 3- to 12-membered heterocyclyl comprising 1-4 heteroatoms selected from O, S(=O)ₚ, and N;
R^{e} is halo, CN, =O, C₁₋₆ alkyl substituted with 0-4 R^{g}, C₂₋₆ alkenyl substituted with 0-4 R^{g}, C₂₋₆ alkynyl substituted with 0-4 R^{g}, -(CH₂)ₙ-C₃₋₆ cycloalkyl substituted with 0-4 R^{g}, -(CH₂)ₙ-aryl substituted with 0-4 R^{g}, -(CH₂)ₙ-4- to 6-membered heterocyclyl comprising 1-4 heteroatoms selected from O, S(=O)ₚ, and N, and substituted with 0-4 R^{g}, -(CH₂)ₙOR^{f}, C(=O)OR^{f}, C(=O)NR^{f}R^{f}, NR^{f}C(=O)R^{f}, S(=O)ₚR^{f}, NR^{f}S(=O)ₚR^{f}, NR^{f}C(=O)OR^{f}, OC(=O)NR^{f}R^{f}, or -(CH₂)ₙNR^{f}R^{f};
R^{f} is H, C₁₋₆ alkyl, C₃₋₆ cycloalkyl, or aryl;
R^{g} is halo, CN, -OH, C₁₋₄ alkyl, C₃₋₆ cycloalkyl, or aryl;
n is zero, 1, 2, or 3; and
p is zero, 1, or 2.

In an eleventh aspect within the scope of the tenth aspect, the present invention provides compounds of Formula (VII) or pharmaceutically acceptable salts, thereof, wherein:
R^{4a} is halo;
R^{4b} is C₁₋₄ alkyl substituted with 0-3 halo substituents;
R⁵ is H;
R⁶ is C₁₋₂ alkyl substituted with 0-2 F substituents or C₃₋₆ cycloalkyl;
R⁸ is -OC₁₋₃ alkyl;
R^{8a} is F or CN;
R⁹ is 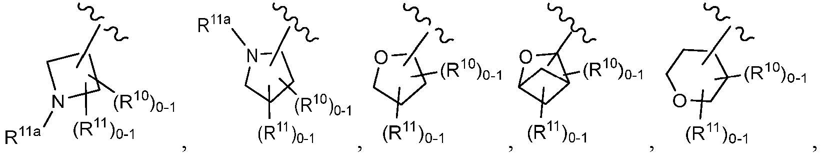 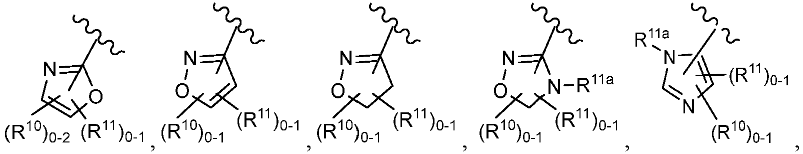 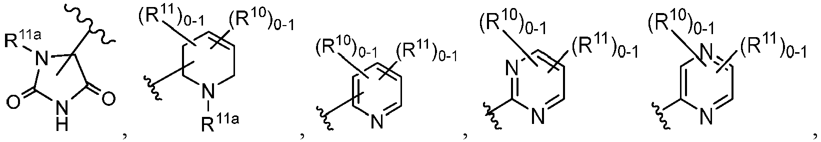 or 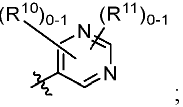
R¹⁰ is halo, CN, C₁₋₂ alkyl, =O, -OH, or -OC₁₋₂ alkyl;
R¹¹ is C₁₋₃ alkyl substituted with 0-1 R¹² and 0-1 R¹³, -OR^{b}, -NR^{a}R^{a}, -NR^{a}C(=O)R^{b}, -C(=O)R^{b}, -C(=O)OR^{b}, -C(=O)NR^{a}R^{a}, or C₃₋₆ cycloalkyl substituted with 0-5 R^{e};
R^{11a} is H, -C(=O)R^{b}, -C(=O)NR^{a}R^{a}, or C₁₋₄ alkyl substituted with 0-1R^{11b};
R^{11b} is -OH or aryl;
R¹² is -C(=O)OR^{b}, -C(=O)NHR^{a}, -C(=O)NHOR^{b}, or C₁₋₄ alkyl substituted with 0-2 halo or -OH substituents;
R¹³ is -OH, -OC₁₋₄ alkyl substituted with 0-2 -OH substituents, or -S(=O)₂C₁₋₄alkyl;
R^{a} is H or C₁₋₆ alkyl or R^{a} and R^{a} together with the nitrogen atom to which they are both attached form a 3 to 9-membered heterocyclyl substituted with 0-4 R^{e};
R^{b} is H, C₁₋₄ alkyl substituted with 0-1 R^{e}, or C₃₋₆ cycloalkyl substituted with 0-1 R^{e};
R^{e} is -OR^{f}; and
R^{f} is H or C₁₋₄ alkyl.

In a twelfth aspect within the scope of the eleventh aspect, the present invention provides compounds of Formula (VII) or pharmaceutically acceptable salts thereof, wherein:
R^{4a} is halo;
R^{4b} is CF₃;
R⁵ is H;
R⁶ is CF₃ or C₃₋₆ cyclopropyl;
R⁸ is -OC₁₋₃ alkyl;
R⁹ is 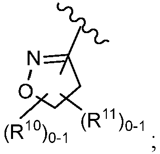
R¹⁰ is C₁₋₂ alkyl, -OH, or -OC₁₋₄ alkyl;
R¹¹ is C₁₋₃ alkyl substituted with 0-1 R¹² and 0-1 R¹³, -C(=O)OR^{b}, or -C(=O)NR^{a}R^{a};
R¹² is -C(=O)OR^{b};
R¹³ is -OH;
R^{a} is H or C₁₋₄ alkyl; and
R^{b} is H or C₁₋₄ alkyl.

In a thirteenth aspect within the scope of the tenth aspect, the present invention provides compounds of Formula (VI) or pharmaceutically acceptable salts thereof, wherein:
R^{4a} is halo;
R^{4b} is C₁₋₄ alkyl substituted with 0-3 halo substituents;
R⁵ is H;
R⁶ is C₁₋₃ alkyl substituted with 0-3 F substituents or C₃₋₆ cycloalkyl;
R⁸ is -OC₁₋₃ alkyl;
R⁹ is 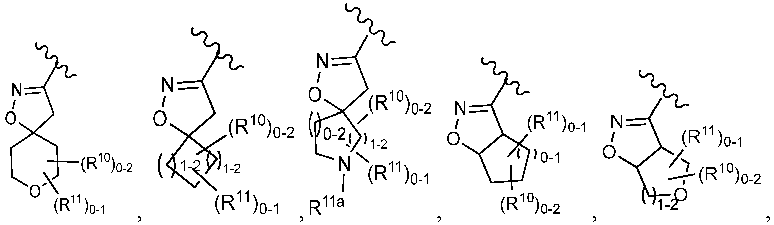 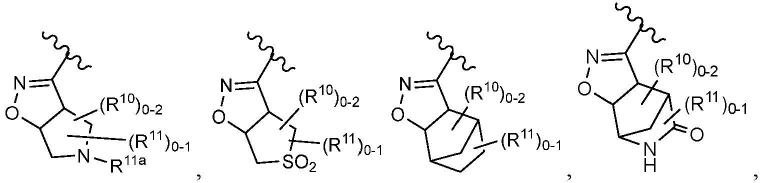 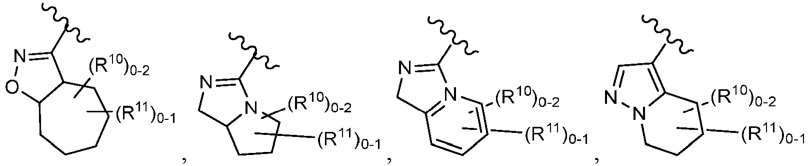 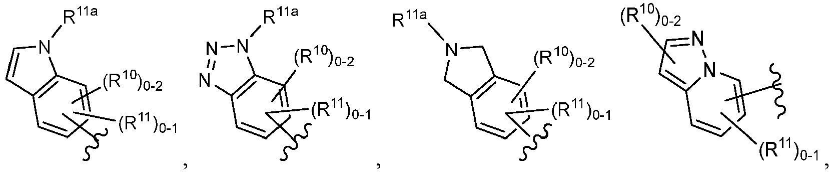 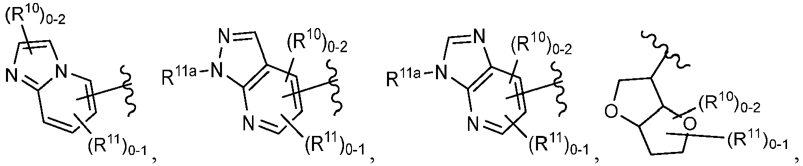 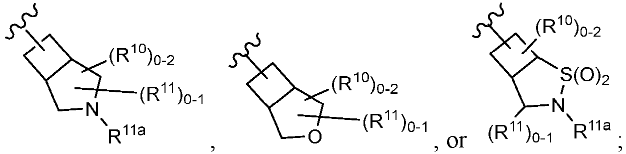
R¹⁰ is halo, C₁₋₃ alkyl, -OH, or -OC₁₋₃ alkyl;
R¹¹ is C₁₋₃ alkyl substituted with 0-1 R¹² and 0-1 R¹³;
R^{11a} is H, C₁₋₄ alkyl substituted with 0-2 R^{11b}, or -C(=O)OC₁₋₄ alkyl;
R^{11b} is -OH, -C(=O)OH, or aryl;
R¹² is C(=O)OR^{b} or C₁₋₃ alkyl substituted with 0-3 halo substituents;
R¹³ is -OH; and
R^{b} is H or C₁₋₄ alkyl.

In one embodiment of Formula (VII), R^{4a} is F; R^{4b} is CF₃; R⁵ is H; R⁶ is C₁₋₄ alkyl substituted with 0-3 F substituents or C₃₋₆ cycloalkyl; R⁸ is -OCH₃ or - OCH₃(CH₂)₂OCH₃; R⁹ is 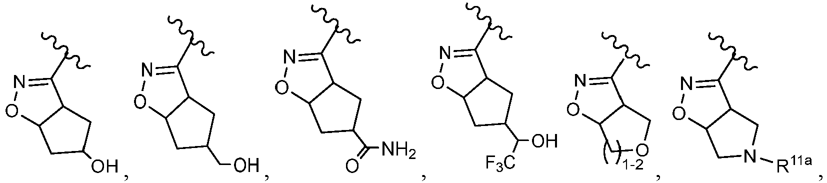 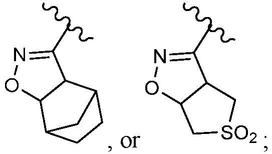
R¹¹ is C₁₋₂ alkyl substituted with 0-1 R¹³;
R^{11a} is H, C₁₋₃ alkyl substituted with 0-2 R^{11b}, -C(=O)C₁₋₄ alkyl substituted with 0-1 R^{11b}, or -C(=O)OC₁₋₄ alkyl; and R^{11b} is -OH, -C(=O)OH, or aryl; and
R¹³ is -OH.

In one embodiment of Formula (VII), R^{4a} is F; R^{4b} is CF₃; R⁵ is H; R⁶ is C₁₋₃ alkyl substituted with 0-3 F substituents or C₃₋₆ cycloalkyl; R⁸ is -OCH₃; R⁹ is 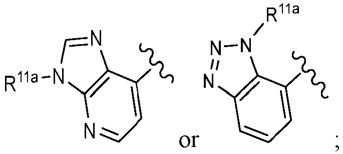 R^{11a} is H or C₁₋₂ alkyl substituted with 0-1 R^{11b}; and R^{11b} is -C(=O)OH.

In a fourteenth aspect within the scope of the third aspect, the present invention provides compounds of Formula (VIII): 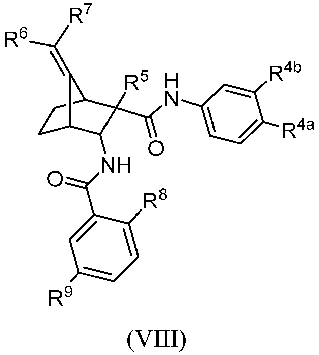 or pharmaceutically acceptable salts thereof, wherein:
R^{4a} is halo;
R^{4b} is C₁₋₄ alkyl substituted with 0-4 halo substituents;
R⁶ is C₁₋₂ alkyl substituted with 0-2 F substituents, C₃₋₆ cycloalkyl, or aryl;
R⁷ is H;
R⁸ is -OC₁₋₃ alkyl;
R⁹ is 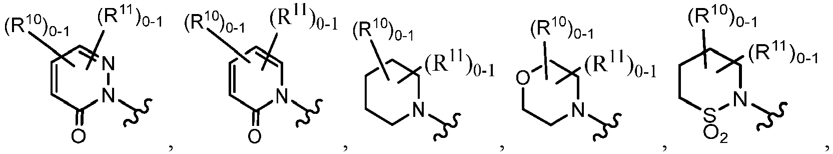 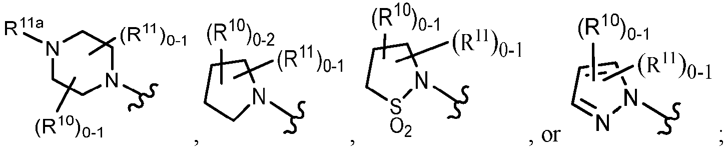
R¹⁰ is halo, CN, C₁₋₄ alkyl, =O, -OH, or -OC₁₋₄ alkyl;
R¹¹ is C₁₋₂ alkyl substituted with 0-1 R¹² and 0-1 R¹³, -NR^{a}R^{a}, -NR^{a}C(=O)R^{b}, - NR^{a}C(=O)OR^{b}, or -C(=O)OR^{b};
R¹² is -C(=O)OR^{b}, -C(=O)NHR^{a}, -C(=O)NHOR^{b}, or C₁₋₄ alkyl substituted with 0-3 halo or -OH substituents;
R¹³ is -OH or -NR^{a}C(=O)R^{b};
R^{a} is H or C₁₋₄ alkyl; and
R^{b} is H, C₁₋₄ alkyl, or 3 to 9-membered heterocyclyl comprising 1-4 heteroatoms selected from O, S(=O)ₚ, and N.

In one embodiment of Formula (VIII), R^{4a} is F; R^{4b} is CF₃; R⁵ is H; R⁶ is CF₃ or cyclopropyl; R⁸ is -OCH₃; R⁹ is

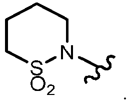

In a fifteenth aspect within the scope of the first aspect, the present invention provides compounds of Formula (IX): 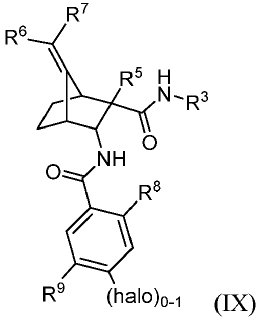 or a pharmaceutically acceptable salt thereof, wherein:
R³ is C₁₋₅ alkyl, CF₃, -(CR_{d}R_{d})₀₋₁-C₃₋₆ cycloalkyl substituted with 0-4 R⁴, or phenyl substituted with 0-4 R⁴;
R⁴ is halo, CN, CH₃, or CF₃;
R⁵ is H;
R⁶ is C₁₋₆ alkyl, CF₃, or C₃₋₆ cycloalkyl substituted with 0-2 F substituents;
R⁷ is H;
R⁸ is halo, -N(C₁₋₃ alkyl)₂, -OC₁₋₃ alkyl substituted with 0-1 -OC₁₋₄ alkyl substituent;
R⁹ is 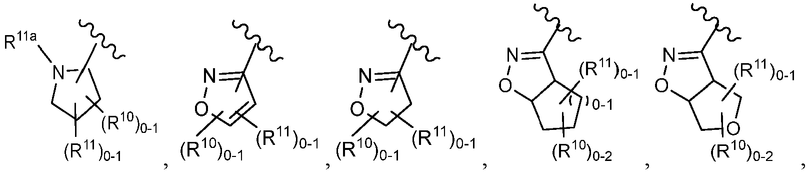 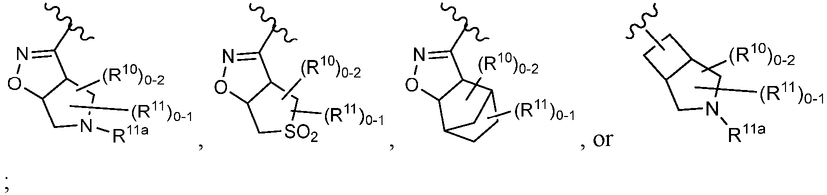
R¹⁰ is halo, C₁₋₄ alkyl, -OH, or -OC₁₋₄ alkyl;
R¹¹ is C₁₋₄ alkyl substituted with 0-2 R¹² and 0-2 R¹³, -C(=O)OR^{b}, -C(=O)NR^{a}R^{a}, or C₃₋₆ cycloalkyl substituted with 0-2 R^{e};
R^{11a} is H, C₁₋₄ alkyl substituted with 0-2 R^{11b}, -C(=O)R^{b}, or -C(=O)OC₁₋₄ alkyl;
R^{11b} is -OH;
R¹² is C₁₋₃ alkyl substituted with 0-3 halo substituents or -C(=O)OR^{b};
R¹³ is -OH;
R^{a} is H or C₁₋₃ alkyl;
R^{b} is H or C₁₋₄ alkyl substituted with 0-1 R^{e};
R^{e} is -OR^{f}; and
R^{f} is H or C₁₋₆ alkyl.

In a sixteenth aspect within the scope of the first aspect, the present invention provides compounds of Formula (X): 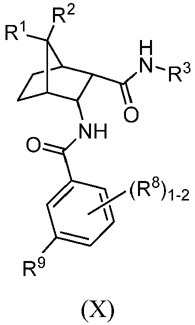 or a pharmaceutically acceptable salt thereof, wherein:
R¹ and R² are combined to be =CR⁶R⁷;
R³ is C₁₋₆ alkyl substituted with 0-5 halo, CN, or -OC₁₋₃ alkyl substituents, -(CHR^{d})ₙ-C₃₋₁₀carbocyclyl substituted with 0-5 R⁴, or 5 to 6-membered heteroaryl comprising 1-3 heteroatoms selected from O, S, N, and substituted with 0-3 R⁴;
R⁴ is halo, CN, S(=O)₂CF₃, or C₁₋₄ alkyl substituted with 0-5 halo substituents;
R⁶ is halo, C₁₋₅ alkyl substituted with 0-3 R^{6a}, C₃₋₆ cycloalkyl substituted with 0-3 R¹⁴, or 5 to 6-membered heterocyclyl comprising 1-3 heteroatoms selected from O, S, and N, and substituted with 0-3 R¹⁴;
R^{6a} is halo, -OH, or C₃₋₆ cycloalkyl;
R⁷ is H;
R⁸ is H, halo, CN, C₁₋₄ alkyl, or -OC₁₋₄ alkyl substituted with 0-5 halo, -OH, C₃₋₆ cycloalkyl, or -OC₁₋₄ alkyl substituents;
R⁹ is 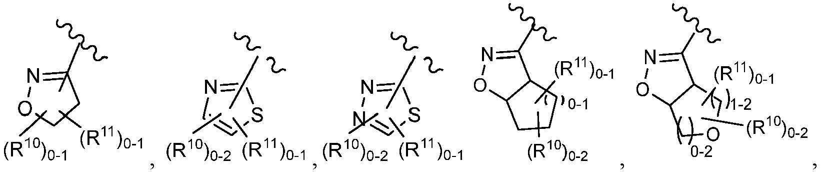 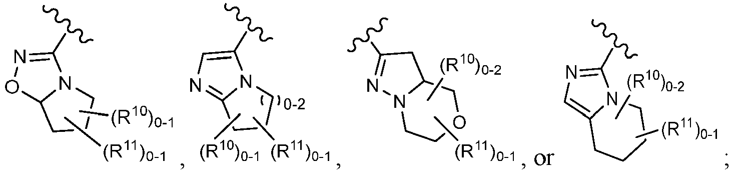
R¹⁰ is halo, CN, C₁₋₄ alkyl, or -OH;
R¹¹ is C₁₋₃ alkyl substituted with 0-3 R¹² and 0-1 R¹³, -OR^{b}, -NHC(=O)R^{b}, or C(=O)OR^{b};
R¹² is halo;
R¹³ is -OR^{b} or C₃₋₆ carbocyclyl;
R¹⁴ is halo, CN, or C₁₋₄ alkyl substituted with 0-3 halo substituents;
R^{b} is H or C₁₋₃ alkyl substituted with 0-5 R^{e};
R^{d} is H or C₁₋₄ alkyl;
R^{e} is -OH; and
n is zero or 1.

In a seventeenth aspect within the scope of the first aspect, the present invention provides compounds of Formula (XI): 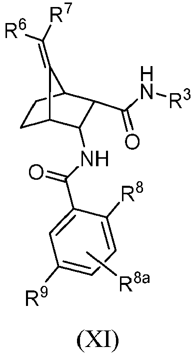 or a pharmaceutically acceptable salt thereof, wherein:
R³ is C₁₋₅ alkyl or 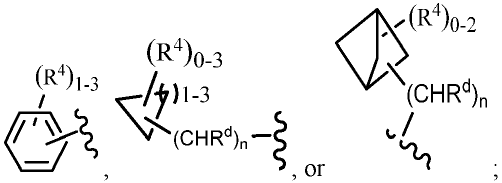
R⁴ is halo, CN, -S(=O)₂CF₃, or C₁₋₄ alkyl substituted with 0-5 halo substituents;
R⁶ is C₁₋₅ alkyl substituted with 0-2 R^{6a}, C₃₋₆ cycloalkyl substituted with 0-2 R¹⁴, or 5- to 6-membered heterocyclyl comprising 1-3 heteroatoms selected from O, S, and N, and substituted with 0-2 R¹⁴;
R^{6a} is halo, -OH, or C₃₋₆ cycloalkyl;
R⁷ is H;
R⁸ is -OC₁₋₃ alkyl substituted with 0-5 halo, -OH, C₃₋₆ cycloalkyl, or -OC₁₋₃ alkyl substituents;
R^{8a} is H, halo, CN, or C₁₋₃ alkyl;
R⁹ is 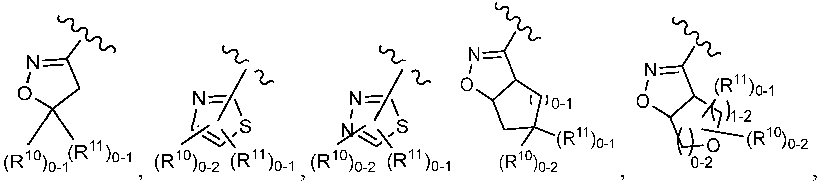 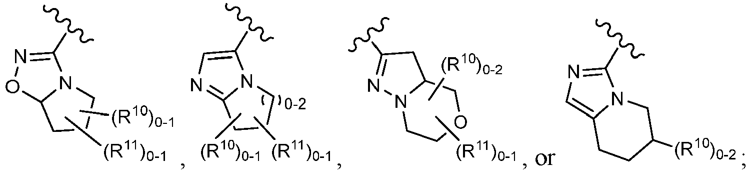
R¹⁰ is halo, CN, C₁₋₄ alkyl, or -OH;
R¹¹ is C₁₋₃ alkyl substituted with 0-3 R¹² and 0-1 R¹³, -OR^{b}, -NHC(=O)R^{b}, or -C(=O)OR^{b} ;
R¹² is halo;
R¹³ is -OR^{b} or C₃₋₆ carbocyclyl;
R¹⁴ is halo or C₁₋₄ alkyl substituted with 0-3 halo substituents;
R^{b} is H or C₁₋₃ alkyl substituted with 0-5 R^{e};
R^{d} is H or C₁₋₂ alkyl; and
n is zero or 1.

For a compound of Formula (I), the scope of any instance of a variable substituent, including R¹, R², R³, R⁴ (R^{4a}, R^{4b}), R^{4c}, R⁵, R⁶, R^{6a}, R^{6b}, R⁷, R⁸ (R^{8a}), R⁹, R¹⁰, R¹¹, R^{11a}, R^{11b}, R¹², R¹³, R¹⁴, R^{14a}, R¹⁵, R^{a}, R^{b}, R^{c}, R^{d}, R^{e}, R^{f}, and R^{g} can be used independently with the scope of any other instance of a variable substituent. As such, the invention includes combinations of the different aspects. In particular, R^{4a} and R^{4b} are a subset of variable R⁴ and R^{8a} is a subset of variable R⁸.

In one embodiment of Formula (XI), R³ is 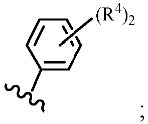 R⁴ is halo, CF₃, or - OCF₃; R⁶ is C₃₋₆ cycloalkyl or C₁₋₃ alkyl substituted with 0-3 R^{6a}; R^{6a} is halo; R⁸ is -OC₁₋₃ alkyl substituted with 0-1 CF₃ or -OCH₃ substituent; R⁹ is 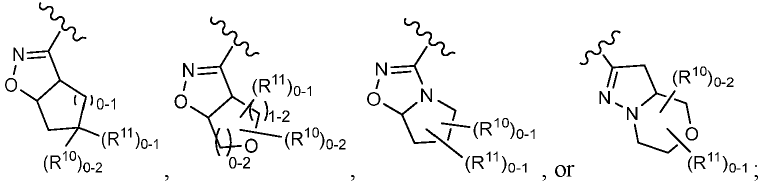 R¹⁰ is C₁₋₄ alkyl or -OH; R¹¹ is C₁₋₃ alkyl substituted with with 0-3 R¹² and 0-1 R¹³; and R¹³ is -OH.

In another embodiment of Formula (XI), R³ is 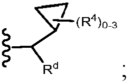 R⁴ is halo or C₁₋₂ alkyl substituted with 0-3 halo substituents; R^{d} is C₁₋₂ alkyl; R⁶ is 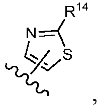 C₃₋₆ cycloalkyl substituted with 0-3 R^{6a}, or C₁₋₃ alkyl substituted with 0-3 R^{6a}; R^{6a} is halo or -OH; R¹⁴ is C₁₋₂ alkyl substituted with 0-3 halo substituents; R⁸ is -OC₁₋₂ alkyl substituted with 0-1 C₃₋₆ cycloalkyl substituents; R^{8a} is H or halo; R⁹ is 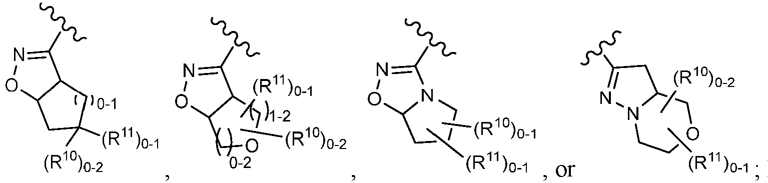 R¹⁰ is C₁₋₄ alkyl or -OH; and R¹³ is -OH.

In one embodiment of Formula (IX), R³ is C₁₋₄ alkyl; R⁶ is CF₃ or cyclopropyl; R⁸ is -OC₁₋₂ alkyl; R⁹ is 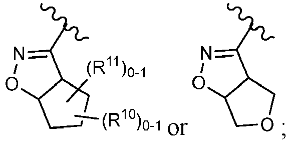 R¹⁰ is -OH, or -OC₁₋₄ alkyl; R¹¹ is C₁₋₂ alkyl substituted with 0-2 R¹² and 0-2 R¹³.

In another embodiment of Formula (IX), R³ is cyclopentyl substituted with 0-1 R⁴, R⁴ is CN or C₁₋₂ alkyl; R⁶ is CF₃ or cyclopropyl; R⁸ is -OC₁₋₂ alkyl; R⁹ is 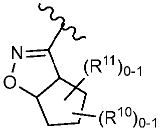 or 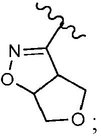 R¹⁰ is -OH or -OC₁₋₄ alkyl; R¹¹ is C₁₋₂ alkyl substituted with 0-2 R¹² and 0-2 R¹³.

In another embodiment of Formula (IX), R³ is phenyl substituted with 0-2 R⁴, R⁴ is halo or CF₃; R⁶ is CF₃ or cyclopropyl; R⁸ is -OC₁₋₂ alkyl; R⁹ is 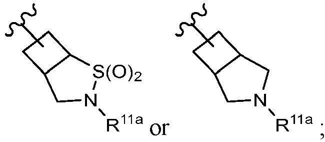 R^{11a} is H, C₁₋₂ alkyl substituted with 0-2 R^{11b}.

In another embodiment of Formula (I), R¹ and R² together with the carbon atom to which they are both attached form a dioxolanyl.

In another embodiment of Formula (I), R¹ and R² combined are =NOC₁₋₄ alkyl wherein "=" is a double bond.

In another embodiment of Formula (I), R¹ and R² combined are =CR⁶R⁷ wherein "=" is a double bond.

In another embodiment of Formula (I), R¹ and R² combined are =CR⁶R⁷; R⁶ and R⁷ are both methyl.

In another embodiment of Formula (I), R¹ and R² combined are =CR⁶R⁷; R⁶ is methyl ethyl, propyl, or butyl, each optioinally substituted with -OH or halo; R⁷ is H.

In another embodiment of Formula (I), R¹ and R² combined are =CR⁶R⁷; R⁶ is CF₃; R⁷ is H.

In another embodiment of Formula (I), R¹ and R² combined are =CR⁶R⁷; R⁶ is halo; R⁷ is H.

In another embodiment of Formula (I), R¹ and R² combined are =CR⁶R⁷; R⁶ is phenyl substituted with 0-1 R¹⁴; R⁷ is H; R¹⁴ is halo, -OC₁₋₄ alkyl, or phenyl.

In another embodiment of Formula (I), R¹ and R² combined are =CR⁶R⁷; R⁶ is 5-membered heterocyclyl comprising 1-3 heteroatoms selected from O and N; R⁷ is H.

In another embodiment of Formula (I), R¹ and R² combined are =CR⁶R⁷; R⁶ is C(=O)NH-phenyl; R⁷ is H.

In another embodiment of Formula (I), R¹ and R² combined are =CR⁶R⁷; R⁶ is C(=O)OC₁₋₄ alkyl; R⁷ is H.

In another embodiment of Formula (I), R¹ and R² combined are =CR⁶R⁷; R⁶ is C(=O)N(Me)₂; R⁷ is H.

In another embodiment of Formula (I), R¹ and R² combined are =CR⁶R⁷; R⁶ is C₃₋₆ cycloalkyl; R⁷ is H.

In another embodiment of Formula (I), R¹ and R² combined are =CR⁶R⁷; R⁶ is - CH₂-C₃₋₆ cycloalkyl substituted with halo; R⁷ is H.

In another embodiment of Formula (I), R¹ and R² combined are =CR⁶R⁷; R⁶ is cyclopropyl; R⁷ is H.

In another embodiment of Formula (I), R¹ and R² combined are =CR⁶R⁷; R⁶ and R⁷ together with the carbon atom to which they are both attached form a cyclopentadienyl, an indanyl, or an indeny.

In one embodiment of Formula (I), R³ is C₁₋₆ alkyl substituted with 0-2 R⁴.

In another embodiment of Formula (I), R³ is methyl, ethyl, propyl, or butyl, or pentyl.

In another embodiment of Formula (I), R³ is

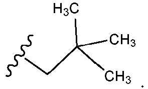

In another embodiment of Formula (I), R³ is C₃₋₆ cycloalkyl substituted with 0-2 R⁴.

In another embodiment of Formula (I), R³ is C₃₋₆ cycloalkenyl substituted with 0-2 R⁴. In another embodiment of Formula (I), R³ is

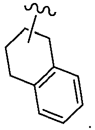

In another embodiment of Formula (I), R³ is

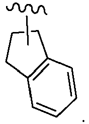

In another embodiment of Formula (I), R³ is

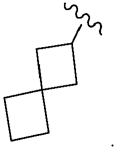

In another embodiment of Formula (I), R³ is -(CR^{d}R^{d})₁₋₂-phenyl substituted with 0-2 R⁴; R⁴ is halo, CF₃ or OCF₃; R^{d} is H or methyl.

In another embodiment of Formula (I), R³ is -(CHR^{d})-C₃₋₆ cycloalkyl substituted with 0-2 R⁴; R⁴ is halo or C₁₋₂ alkyl; R_{d} is H or C₁₋₂ alkyl .

In another embodiment of Formula (I), R³ is 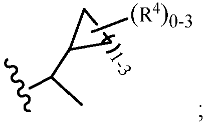 R⁴ is halo or C₁₋₃ alkyl.

In another embodiment of Formula (I), R³ is 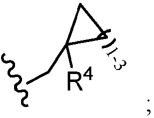 R⁴ is C₁₋₂ alkyl.

In another embodiment of Formula (I), R³ is 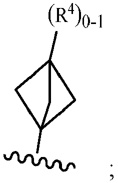 R⁴ is halo or CN.

In another embodiment of Formula (I), R³ is -(CR^{d}R^{d})₁₋₂-5-membered heterocyclyl comprising 1-2 heteroatoms selected from O and N; R^{d} is H or methyl.

In another embodiment of Formula (I), R⁴ is halo, CN, C₁₋₂ alkyl substituted with 0-3 halo.

In another embodiment of Formula (I), R³ is cyclopropyl, cyclobutyl, cyclopentyl substituted with 0-1 R⁴, or cyclohexyl; R⁴ is CN or C₁₋₂ alkyl.

In one embodiment of Formula (I), R⁵ is H, halo, or -OH.

In another embodiment of Formula (I), R⁵ is H or -OH.

In one embodiment of Formula (I), R⁶ is C₁₋₄ alkyl substituted with 0-3 R^{6a} or C₃₋₆ cycloalkyl substituted with 0-3 R¹⁴, or 5 to 6-membered heterocyclyl comprising 1-3 heteroatoms selected from O, S, N, and NR^{14a} and substituted with 0-3 R¹⁴; R^{6a} is halo, - OH, or C₃₋₆ cycloalkyl substituted with 0-3 halo substituents; R¹⁴ is halo or C₁₋₃ alkyl substituted with 0-3 halo substituents.

In another embodiment of Formula (I), R⁶ is C₃₋₆ cycloalkyl substituted with 0-3 R¹⁴; R¹⁴ is halo substituents.

In another embodiment of Formula (I), R⁶ is isopropyl.

In one embodiment of Formula (I), R⁷ is H or C₁₋₂ alkyl.

In one embodiment of Formula (I), there are two R⁸ variables. One of R⁸ is -OC₁₋₃ alkyl. The other R⁸, sometimes referenced as R^{8a}, is halo or CN.

In one embodiment of Formula (I), R⁹ is phenyl substituted with 0-3 R¹⁰ and 0-2 R¹¹.

In another embodiment of Formula (I), R⁹ is phenyl substituted with 0-3 R¹⁰ and 0-2 R¹¹; R¹⁰ is halo; R¹¹ is C₁₋₅ alkyl substituted with 0-4 R¹² and 0-2 R¹³; R¹² is halo or C(=O)OH; R¹³ is -OC(=O)NHR^{a}; R^{a} is C₁₋₄ alkyl, C₃₋₆ alkyl, or phenyl.

In another embodiment of Formula (I), R⁹ is phenyl substituted with 0-3 R¹⁰ and 0-2 R¹¹; R¹⁰ is halo; R¹¹ is C₁₋₅ alkyl substituted with 0-4 R¹² and 0-2 R¹³; R¹² is halo or C(=O)OH; R¹³ is -NHC(=O)R^{b}; R^{b} is 3- to 6 membered heterocyclyl comprising 1-3 heteroatoms selected from O, S, and N.

In another embodiment of Formula (I), R⁹ is phenyl substituted with 0-1 R¹⁰ and 0-1 R¹¹; R¹⁰ is halo; R¹¹ is 4- to 9-membered heterocyclyl comprising 1-4 heteroatoms selected from O, S(=O)ₚ, N, and NR¹⁵, and substituted with 0-3 R^{e}; R^{e} is -COOH or C₁₋₃ alkyl substituted with 0-5 R^{g}; R^{g} is -OH.

In one embodiment of Formula (I), R⁹ is 3- to 12-membered heterocyclyl comprising 1-5 heteroatoms selected from O, S(=O)ₚ, N, and NR^{11a}, and substituted with 0-3 R¹⁰ and 0-2 R¹¹. In another embodiment of Formula (I), R⁹ is 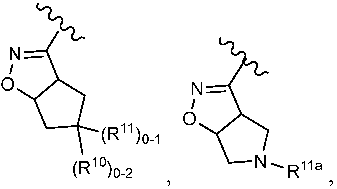 or 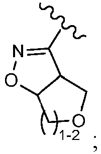 R¹⁰ is C₁₋₂ alkyl; R¹¹ is C₁₋₃ alkyl substituted with -OH substituent, R^{11a} is -C(=O)C₁₋₄ alkyl substituted with 0-1 R^{11b}; R^{11b} is -OH.

In another aspect, the present invention provides compounds of Formula (IIIa): 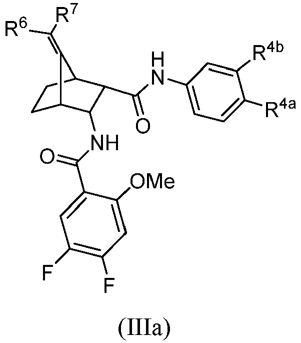 or pharmaceutically acceptable salts thereof, wherein:
R^{4a} is halo;
R^{4b} is C₁₋₄ alkyl substituted with 0-4 halo substituents;
R⁶ is halo, C₁₋₄ alkyl substituted with 0-3 R^{6a}, C₂₋₄ alkenyl substituted with 0-1 phenyl or - OH, -C(=O)OR^{b}, C(=O)NHR^{a}, C₃₋₆ cycloalkyl, C₃₋₆ cycloalkenyl substituted with 0-3 R¹⁴, phenyl substituted with 0-3 R¹⁴, naphthyl, or 5 to 6-membered heterocyclyl comprising 1-3 heteroatoms selected from O, S, N, and NR^{14a} and substituted with 0-3 R¹⁴;
R^{6a} is halo, -OH, C₃₋₆ cycloalkyl, or phenyl;
R⁷ is H;
or R⁶ and R⁷ together with the carbon atom to which they are both attached form a cyclopentadienyl, an indanyl, or an indenyl;
R¹⁴ is halo, CN, C₁₋₄ alkyl substituted with 0-3 halo substituents, -OC₁₋₄ alkyl substituted with 0-3 halo substituents, -(CH₂)₀₋₂-NR^{a}R^{a}, -(CH₂)₀₋₂-aryl substituted with 0-3 R^{e}, -O-aryl substituted with 0-3 R^{e}, or -(CH₂)₀₋₂-3- to 12-membered heterocyclyl comprising 1-4 heteroatoms selected from O, S(=O)ₚ, and N, and substituted with 0-3 R^{e};
R^{14a} is H or C₁₋₃ alkyl;
R^{a} is H or C₁₋₃ alkyl;
R^{b} is H or C₁₋₃ alkyl; and
p is zero or 2.

In one embodiment of Formula (V), R^{4a} is For CH₃; R^{4b} is CF₃; R⁶ is phenyl or 5-membered heteroaryl comprising 1-2 heteroatoms selected from O and N; R⁷ is H; R⁸ is - OC₁₋₂alkyl; R¹⁰ is halo; R¹¹ is -NHS(=O)₂C₁₋₂ alkyl, -C(=O)OH, -C(=O)OC₁₋₄ alkyl, - C(=O)NHC₁₋₄ alkyl substituted with 0-1 R^{e}, or a 5-membered heterocyclyl comprising 1-4 heteroatoms selected from O, N, and NR¹⁵ and substituted with 0-3 R^{e}; and R^{e} is =O or C(=O)OH.

In another aspect, the present invention provides compounds of Formula (VIb): 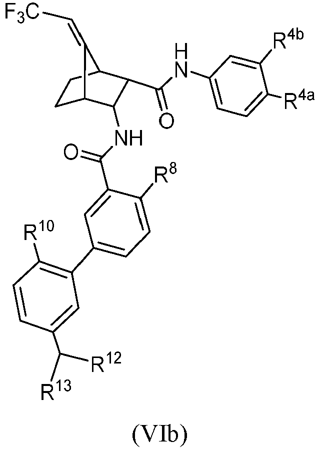 or pharmaceutically acceptable salts thereof, wherein:
R^{4a} is halo;
R^{4b} is CF₃;
R⁸ is-OC₁₋₄alkyl;
R¹⁰ is halo;
R¹² is -C(=O)OH, -C(=O)OC₁₋₄ alkyl, -C(=O)NHC₁₋₄ alkyl, -C(=O)NHOC₁₋₃ alkyl, or C₁₋₃ alkyl substituted with 0-3 halo;
R¹³ is -OR^{b}, -NR^{a}R^{a}, -NR^{a}C(=O)R^{b}, -NR^{a}C(=O)OR^{b}, -NR^{a}S(=O)ₚR^{c}, -NR^{a}S(=O)ₚNR^{a}R^{a}, -OC(=O)NR^{a}R^{a}, -OC(=O)NR^{a}OR^{b}, -S(=O)ₚNR^{a}R^{a}, or -S(=O)ₚR^{c};
R^{a} is H, C₁₋₆ alkyl substituted with 0-5 halo substituents, phenyl, C₃₋₆ cycloalkyl substituted with 0-4 R^{e}, spirocycloalkyl, or 3- to 12-membered heterocyclyl comprising 1-4 heteroatoms selected from O, S(=O)p, and N, and substituted with 0-4 R^{e}; or R^{a} and R^{a} together with the nitrogen atom to which they are both attached form a heterocyclyl substituted with 0-4 R^{e};
R^{b} is H, C₁₋₆ alkyl substituted with 0-5 R^{e}, -(CH₂)ₙ-phenyl, C₃₋₆ cycloalkyl substituted with 0-4 halo substituents, or 3- to 9-membered heterocyclyl comprising 1-4 heteroatoms selected from O, S(=O)ₚ, and N, and substituted with 0-4 R^{e};
R^{c} is C₁₋₆ alkyl substituted with 0-4 R^{e},
R^{e} is halo, CN, =O, C₁₋₅ alkyl substituted with 0-5 R^{g}, C₃₋₆ cycloalkyl, aryl, 4- to 6-membered heterocyclyl, or -OR^{f};
R^{f} is H, C₁₋₆ alkyl, C₃₋₆ cycloalkyl or aryl;
R^{g} is halo;
n is zero or 1; and
p is zero, 1, or 2.

In another aspect, the present invention provides compounds of Formula (VII), or pharmaceutically acceptable salts thereof, wherein:
R^{4a} is F;
R^{4b} is CF₃;
R⁵ is H;
R⁶ is C₁₋₃ alkyl substituted with 0-3 F substituents or C₃₋₆ cycloalkyl;
R⁸ is -OCH₃;
R⁹ is 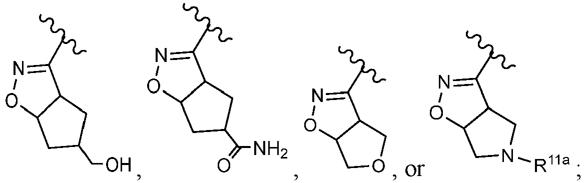
R^{11a} is H, C₁₋₃ alkyl substituted with 0-2 R^{11b}, -C(=O)C₁₋₄ alkyl substituted with 0-1 R^{11b}, or -C(=O)OC₁₋₄ alkyl; and
R^{11b} is -OH, -C(=O)OH, or aryl.

Unless specified otherwise, these terms have the following meanings.

"Halo" includes fluoro, chloro, bromo, and iodo.

"Alkyl" or "alkylene" is intended to include both branched and straight-chain saturated aliphatic hydrocarbon groups having the specified number of carbon atoms. For example, "C₁ to C₁₀ alkyl" or "C₁₋₁₀ alkyl" (or alkylene), is intended to include C₁, C₂, C₃, C₄, C₅, C₆, C₇, C₈, C₉, and C₁₀ alkyl groups. Additionally, for example, "C₁ to C₆ alkyl" or "C₁-C₆ alkyl" denotes alkyl having 1 to 6 carbon atoms. Alkyl group can be unsubstituted or substituted with at least one hydrogen being replaced by another chemical group. Example alkyl groups include, but are not limited to, methyl (Me), ethyl (Et), propyl (*e.g.,* n-propyl and isopropyl), butyl (*e.g.,* n-butyl, isobutyl, t-butyl), and pentyl (*e.g.,* n-pentyl, isopentyl, neopentyl). When "C₀ alkyl" or "C₀ alkylene" is used, it is intended to denote a direct bond. "Alkyl" also includes deuteroalkyl such as CD₃.

"Alkenyl" or "alkenylene" is intended to include hydrocarbon chains of either straight or branched configuration having one or more, preferably one to three, carboncarbon double bonds that may occur in any stable point along the chain. For example, "C₂ to C₆ alkenyl" or "C₂₋₆ alkenyl" (or alkenylene), is intended to include C₂, C₃, C₄, C₅, and C₆ alkenyl groups; such as ethenyl, propenyl, butenyl, pentenyl, and hexenyl.

"Alkynyl" or "alkynylene" is intended to include hydrocarbon chains of either straight or branched configuration having one or more, preferably one to three, carboncarbon triple bonds that may occur in any stable point along the chain. For example, "C₂ to C₆ alkynyl" or "C₂₋₆ alkynyl" (or alkynylene), is intended to include C₂, C₃, C₄, C₅, and C₆ alkynyl groups; such as ethynyl, propynyl, butynyl, pentynyl, and hexynyl.

"Carbocycle", "carbocyclyl", or "carbocyclic residue" is intended to mean any stable 3-, 4-, 5-, 6-, 7-, or 8-membered monocyclic or bicyclic or 7-, 8-, 9-, 10-, 11-, 12-, or 13-membered bicyclic or tricyclic hydrocarbon ring, any of which may be saturated, partially unsaturated, unsaturated or aromatic. Examples of such carbocyclyls include, but are not limited to, cyclopropyl, cyclobutyl, cyclobutenyl, cyclopentyl, cyclopentenyl, cyclohexyl, cycloheptenyl, cycloheptyl, cycloheptenyl, adamantyl, cyclooctyl, cyclooctenyl, cyclooctadienyl, [3.3.0]bicyclooctane, [4.3.0]bicyclononane, [4.4.0]bicyclodecane (decalin), [2.2.2]bicyclooctane, fluorenyl, phenyl, naphthyl, indanyl, adamantyl, anthracenyl, and tetrahydronaphthyl (tetralin). As shown above, bridged rings are also included in the definition of carbocyclyl (e.g., [2.2.2]bicyclooctane). A bridged ring occurs when one or more carbon atoms link two non-adjacent carbon atoms. Preferred bridges are one or two carbon atoms. It is noted that a bridge always converts a monocyclic ring into a tricyclic ring. When a ring is bridged, the substituents recited for the ring may also be present on the bridge. When the term "carbocyclyl" is used, it is intended to include "aryl," "cycloalkyl," "spirocycloalkyl," "cycloalkenyl." Preferred carbocyclyls, unless otherwise specified, are cyclopropyl, cyclobutyl, cyclopentyl, cyclohexyl, phenyl, and indanyl.

"Cycloalkyl" is intended to mean cyclized alkyl groups, including mono-, bi- or multicyclic ring systems. "C₃ to C₇ cycloalkyl" or "C₃₋₇ cycloalkyl" is intended to include C₃, C₄, C₅, C₆, and C₇ cycloalkyl groups. Non-limiting examples of monocyclic cycloalkyls include cyclopropyl, cyclobutyl, cyclopentyl, cyclohexyl, cycloheptyl and cyclooctyl. Non-limiting examples of multicyclic cycloalkyls include 1-decalinyl, norbornyl and adamantyl.

"Cycloalkenyl" is intended to mean cyclized alkenyl groups, including mono- or multi-cyclic ring systems that contain one or more double bonds in at least one ring; although, if there is more than one, the double bonds cannot form a fully delocalized pielectron system throughout all the rings (otherwise the group would be "aryl," as defined herein). "C₃ to C₇ cycloalkenyl" or "C₃₋₇ cycloalkenyl" is intended to include C₃, C₄, C₅, C₆, and C₇ cycloalkenyl groups.

"Spirocycloalkyl" is intended to mean hydrocarbon bicyclic ring systems with both rings connected through a single atom. The ring can be different in size and nature, or identical in size and nature. Examples include spiropentane, spriohexane, spiroheptane, spirooctane, spirononane, or spirodecane.

"Bicyclic carbocyclyl" or "bicyclic carbocyclic group" is intended to mean a stable 9- or 10-membered carbocyclic ring system that contains two fused rings and consists of carbon atoms. Of the two fused rings, one ring is a benzo ring fused to a second ring; and the second ring is a 5- or 6-membered carbon ring which is saturated, partially unsaturated, or unsaturated. The bicyclic carbocyclic group may be attached to its pendant group at any carbon atom which results in a stable structure. The bicyclic carbocyclic group described herein may be substituted on any carbon if the resulting compound is stable. Examples of a bicyclic carbocyclic group are, but not limited to, naphthyl, 1,2-dihydronaphthyl, 1,2,3,4-tetrahydronaphthyl, and indanyl.

"Aryl" groups refer to monocyclic or polycyclic aromatic hydrocarbons, including, for example, phenyl, naphthyl, and phenanthranyl. Aryl moieties are well known and described, for example, in Lewis, R.J., ed., Hawley's Condensed Chemical Dictionary, 13th Edition, John Wiley & Sons, Inc., New York (1997).

"Benzyl" is intended to mean a methyl group on which one of the hydrogen atoms is replaced by a phenyl group, wherein said phenyl group may optionally be substituted with 1 to 5 groups, preferably 1 to 3 groups.

"Heterocycle", "heterocyclyl" or "heterocyclic ring" is intended to mean a stable 3-, 4-, 5-, 6-, or 7-membered monocyclic or bicyclic or 7-, 8-, 9-, 10-, 11-, 12-, 13-, or 14-membered polycyclic heterocyclic ring that is saturated, partially unsaturated, or fully unsaturated, and that contains carbon atoms and 1, 2, 3 or 4 heteroatoms independently selected from the group consisting of N, O and S; and including any polycyclic group in which any of the above-defined heterocyclic rings is fused to a benzene ring. The nitrogen and sulfur heteroatoms may optionally be oxidized (*i.e.,* N→O and S(O)ₚ, wherein p is 0, 1 or 2). The nitrogen atom may be substituted or unsubstituted (*i.e.,* N or NR wherein R is H or another substituent, if defined). The heterocyclic ring may be attached to its pendant group at any heteroatom or carbon atom that results in a stable structure. The heterocyclic rings described herein may be substituted on carbon or on a nitrogen atom if the resulting compound is stable. A nitrogen in the heterocyclyl may optionally be quaternized. It is preferred that when the total number of S and O atoms in the heterocyclyl exceeds 1, then these heteroatoms are not adjacent to one another. It is preferred that the total number of S and O atoms in the heterocyclyl is not more than 1. Bridged rings are also included in the definition of heterocyclyl. When the term "heterocyclyl" is used, it is intended to include heteroaryl.

Examples of heterocyclyls include, but are not limited to, acridinyl, azetidinyl, azocinyl, benzimidazolyl, benzofuranyl, benzothiofuranyl, benzothiophenyl, benzoxazolyl, benzoxazolinyl, benzthiazolyl, benztriazolyl, benztetrazolyl, benzisoxazolyl, benzisothiazolyl, benzimidazolinyl, carbazolyl, 4a*H*-carbazolyl, carbolinyl, chromanyl, chromenyl, cinnolinyl, decahydroquinolinyl, 2*H*,6*H*-1,5,2-dithiazinyl, dihydrofuro[2,3-*b*]tetrahydrofuran, furanyl, furazanyl, imidazolidinyl, imidazolinyl, imidazolyl, 1*H*-indazolyl, imidazolopyridinyl, indolenyl, indolinyl, indolizinyl, indolyl, 3H-indolyl, isatinoyl, isobenzofuranyl, isochromanyl, isoindazolyl, isoindolinyl, isoindolyl, isoquinolinyl, isothiazolyl, isothiazolopyridinyl, isoxazolyl, isoxazolopyridinyl, methylenedioxyphenyl, morpholinyl, naphthyridinyl, octahydroisoquinolinyl, oxadiazolyl, 1,2,3-oxadiazolyl, 1,2,4-oxadiazolyl, 1,2,5-oxadiazolyl, 1,3,4-oxadiazolyl, oxazolidinyl, oxazolyl, oxazolopyridinyl, oxazolidinylperimidinyl, oxindolyl, pyrimidinyl, phenanthridinyl, phenanthrolinyl, phenazinyl, phenothiazinyl, phenoxathiinyl, phenoxazinyl, phthalazinyl, piperazinyl, piperidinyl, piperidonyl, 4-piperidonyl, piperonyl, pteridinyl, purinyl, pyranyl, pyrazinyl, pyrazolidinyl, pyrazolinyl, pyrazolopyridinyl, pyrazolyl, pyridazinyl, pyridooxazolyl, pyridoimidazolyl, pyridothiazolyl, pyridinyl, pyrimidinyl, pyrrolidinyl, pyrrolinyl, 2-pyrrolidonyl, 2*H*-pyrrolyl, pyrrolyl, quinazolinyl, quinolinyl, 4*H*-quinolizinyl, quinoxalinyl, quinuclidinyl, tetrazolyl, tetrahydrofuranyl, tetrahydroisoquinolinyl, tetrahydroquinolinyl, 6*H*-1,2,5-thiadiazinyl, 1,2,3-thiadiazolyl, 1,2,4-thiadiazolyl, 1,2,5-thiadiazolyl, 1,3,4-thiadiazolyl, thianthrenyl, thiazolyl, thienyl, thiazolopyridinyl, thienothiazolyl, thienooxazolyl, thienoimidazolyl, thiophenyl, triazinyl, 1,2,3-triazolyl, 1,2,4-triazolyl, 1,2,5-triazolyl, 1,3,4-triazolyl, and xanthenyl. Also included are fused ring and spiro compounds containing, for example, the above heterocyclyls.

"Bicyclic heterocyclyl" "bicyclic heterocyclyl" or "bicyclic heterocyclic group" is intended to mean a stable 9- or 10-membered heterocyclic ring system which contains two fused rings and consists of carbon atoms and 1, 2, 3, or 4 heteroatoms independently selected from the group consisting of N, O and S. Of the two fused rings, one ring is a 5- or 6-membered monocyclic aromatic ring comprising a 5-membered heteroaryl ring, a 6-membered heteroaryl ring or a benzo ring, each fused to a second ring. The second ring is a 5- or 6-membered monocyclic ring which is saturated, partially unsaturated, or unsaturated, and comprises a 5-membered heterocyclyl, a 6-membered heterocyclyl or a carbocyclyl (provided the first ring is not benzo when the second ring is a carbocyclyl).

The bicyclic heterocyclic group may be attached to its pendant group at any heteroatom or carbon atom which results in a stable structure. The bicyclic heterocyclic group described herein may be substituted on carbon or on a nitrogen atom if the resulting compound is stable. It is preferred that when the total number of S and O atoms in the heterocyclyl exceeds 1, then these heteroatoms are not adjacent to one another. It is preferred that the total number of S and O atoms in the heterocyclyl is not more than 1.

Examples of a bicyclic heterocyclic group are, but not limited to, quinolinyl, isoquinolinyl, phthalazinyl, quinazolinyl, indolyl, isoindolyl, indolinyl, 1H-indazolyl, benzimidazolyl, 1,2,3,4-tetrahydroquinolinyl, 1,2,3,4-tetrahydroisoquinolinyl, 5,6,7,8-tetrahydroquinolinyl, 2,3-dihydrobenzofuranyl, chromanyl, 1,2,3,4-tetrahydroquinoxalinyl, and 1,2,3,4-tetrahydroquinazolinyl.

"Heteroaryl" is intended to mean stable monocyclic and polycyclic aromatic hydrocarbons that include at least one heteroatom ring member such as sulfur, oxygen, or nitrogen. Heteroaryl groups include, without limitation, pyridyl, pyrimidinyl, pyrazinyl, pyridazinyl, triazinyl, furyl, quinolyl, isoquinolyl, thienyl, imidazolyl, thiazolyl, indolyl, pyrroyl, oxazolyl, benzofuryl, benzothienyl, benzthiazolyl, isoxazolyl, pyrazolyl, triazolyl, tetrazolyl, indazolyl, 1,2,4-thiadiazolyl, isothiazolyl, purinyl, carbazolyl, benzimidazolyl, indolinyl, benzodioxolanyl, and benzodioxane. Heteroaryl groups are substituted or unsubstituted. The nitrogen atom is substituted or unsubstituted (*i.e.,* N or NR wherein R is H or another substituent, if defined). The nitrogen and sulfur heteroatoms may optionally be oxidized (*i.e.,* N→O and S(O)ₚ, wherein p is 0, 1 or 2).

As referred to herein, the term "substituted" means that at least one hydrogen atom is replaced with a non-hydrogen group, provided that normal valencies are maintained and that the substitution results in a stable compound. When a substituent is keto (*i.e.,* =O), then 2 hydrogens on the atom are replaced. Keto substituents are not present on aromatic moieties. When a ring system (*e.g.*, carbocyclic or heterocyclic) is said to be substituted with a carbonyl group or a double bond, it is intended that the carbonyl group or double bond be part (*i.e.,* within) of the ring. Ring double bonds, as used herein, are double bonds that are formed between two adjacent ring atoms (*e.g.,* C=C, C=N, or N=N).

In cases wherein there are nitrogen atoms (*e.g.*, amines) on compounds of the present invention, these may be converted to N-oxides by treatment with an oxidizing agent (*e.g*., mCPBA and/or hydrogen peroxides) to afford other compounds of this invention. Thus, shown and claimed nitrogen atoms are considered to cover both the shown nitrogen and its N-oxide (N→O) derivative.

When any variable occurs more than one time in any constituent or formula for a compound, its definition at each occurrence is independent of its definition at every other occurrence. Thus, for example, if a group is shown to be substituted with 0-3 R groups, then said group may optionally be substituted with up to three R groups, and at each occurrence R is selected independently from the definition of R. Also, combinations of substituents and/or variables are permissible only if such combinations result in stable compounds.

When a bond to a substituent is shown to cross a bond connecting two atoms in a ring, then such substituent may be bonded to any atom on the ring. When a substituent is listed without indicating the atom in which such substituent is bonded to the rest of the compound of a given formula, then such substituent may be bonded via any atom in such substituent. Combinations of substituents and/or variables are permissible only if such combinations result in stable compounds.

The invention includes all pharmaceutically acceptable salt forms of the compounds. Pharmaceutically acceptable salts are those in which the counter ions do not contribute significantly to the physiological activity or toxicity of the compounds and as such function as pharmacological equivalents. These salts can be made according to common organic techniques employing commercially available reagents. Some anionic salt forms include acetate, acistrate, besylate, bromide, chloride, citrate, fumarate, glucouronate, hydrobromide, hydrochloride, hydroiodide, iodide, lactate, maleate, mesylate, nitrate, pamoate, phosphate, succinate, sulfate, tartrate, tosylate, and xinofoate. Some cationic salt forms include ammonium, aluminum, benzathine, bismuth, calcium, choline, diethylamine, diethanolamine, lithium, magnesium, meglumine, 4-phenylcyclohexylamine, piperazine, potassium, sodium, tromethamine, and zinc.

Throughout the specification and the appended claims, a given chemical formula or name shall encompass all stereo and optical isomers and racemates thereof where such isomers exist. Unless otherwise indicated, all chiral (enantiomeric and diastereomeric) and racemic forms are within the scope of the invention. Enantiomers and diastereomers are examples of stereoisomers. The term "enantiomer" refers to one of a pair of molecular species that are mirror images of each other and are not superimposable. The term "diastereomer" refers to stereoisomers that are not mirror images. The term "racemate" or "racemic mixture" refers to a composition composed of equimolar quantities of two enantiomeric species, wherein the composition is devoid of optical activity.

The invention includes all tautomeric forms of the compounds, atropisomers and rotational isomers.

The term "counterion" is used to represent a negatively charged species such as chloride, bromide, hydroxide, acetate, and sulfate.

The symbols "R" and "S" represent the configuration of substituents around a chiral carbon atom(s). The isomeric descriptors "R" and "S" are used as described herein for indicating atom configuration(s) relative to a core molecule and are intended to be used as defined in the literature (IUPAC Recommendations 1996, Pure and Applied Chemistry, 68:2193-2222 (1996)).

The term "chiral" refers to the structural characteristic of a molecule that makes it impossible to superimpose it on its mirror image. The term "homochiral" refers to a state of enantiomeric purity. The term "optical activity" refers to the degree to which a homochiral molecule or nonracemic mixture of chiral molecules rotates a plane of polarized light.

The invention is intended to include all isotopes of atoms occurring in the compounds. Isotopes include those atoms having the same atomic number but different mass numbers. By way of general example and without limitation, isotopes of hydrogen include deuterium and tritium. Isotopes of carbon include ¹³C and ¹⁴C. Isotopically-labeled compounds of the invention can generally be prepared by conventional techniques known to those skilled in the art or by processes analogous to those described herein, using an appropriate isotopically-labeled reagent in place of the non-labeled reagent otherwise employed. Such compounds may have a variety of potential uses, for example as standards and reagents in determining biological activity. In the case of stable isotopes, such compounds may have the potential to favorably modify biological, pharmacological, or pharmacokinetic properties.

### BIOLOGICAL METHODS

RXFP1 Cyclic Adenosine Monophosphate (cAMP) Assays. Human embryonic kidney cells 293 (HEK293) cells and HEK293 cells stably expressing human RXFP1, were cultured in MEM medium supplemented with 10% qualified FBS, and 300 µg/ml hygromycin (Life Technologies). Cells were dissociated and suspended in assay buffer. The assay buffer was HBSS buffer (with calcium and magnesium) containing 20 mM HEPES, 0.05% BSA, and 0.5 mM IBMX. Cells (3000 cells per well, except 1500 cell per well for HEK293 cells stably expressing human RXFP1) were added to 384-well Proxiplates (Perkin-Elmer). Cells were immediately treated with test compounds in DMSO (2% final) at final concentrations in the range of 0.010 nM to 50 µM. Cells were incubated for 30 min at room temperature. The level of intracellular cAMP was determined using the HTRF HiRange cAMP assay reagent kit (Cisbio) according to manufacturer's instructions. Solutions of cryptate conjugated anti-cAMP and d2 fluorophore-labelled cAMP were made in a supplied lysis buffer separately. Upon completion of the reaction, the cells were lysed with equal volume of the d2-cAMP solution and anti-cAMP solution. After a 1 h room temperature incubation, time-resolved fluorescence intensity was measured using the Envision (Perkin-Elmer) at 400 nm excitation and dual emission at 590 nm and 665 nm. A calibration curve was constructed with an external cAMP standard at concentrations ranging from 2.7 µM to 0.1 pM by plotting the fluorescent intensity ratio from 665 nm emission to the intensity from the 590 nm emission against cAMP concentrations. The potency and activity of a compound to inhibit cAMP production was then determined by fitting to a 4-parametric logistic equation from a plot of cAMP level versus compound concentrations.

The examples disclosed below were tested in the human RXFP1 (hRXFP1) HEK293 cAMP assay described above and found to have agonist activity. Table 1 lists EC₅₀ values in the hRXFP1 HEK293 cAMP assay measured for the examples.

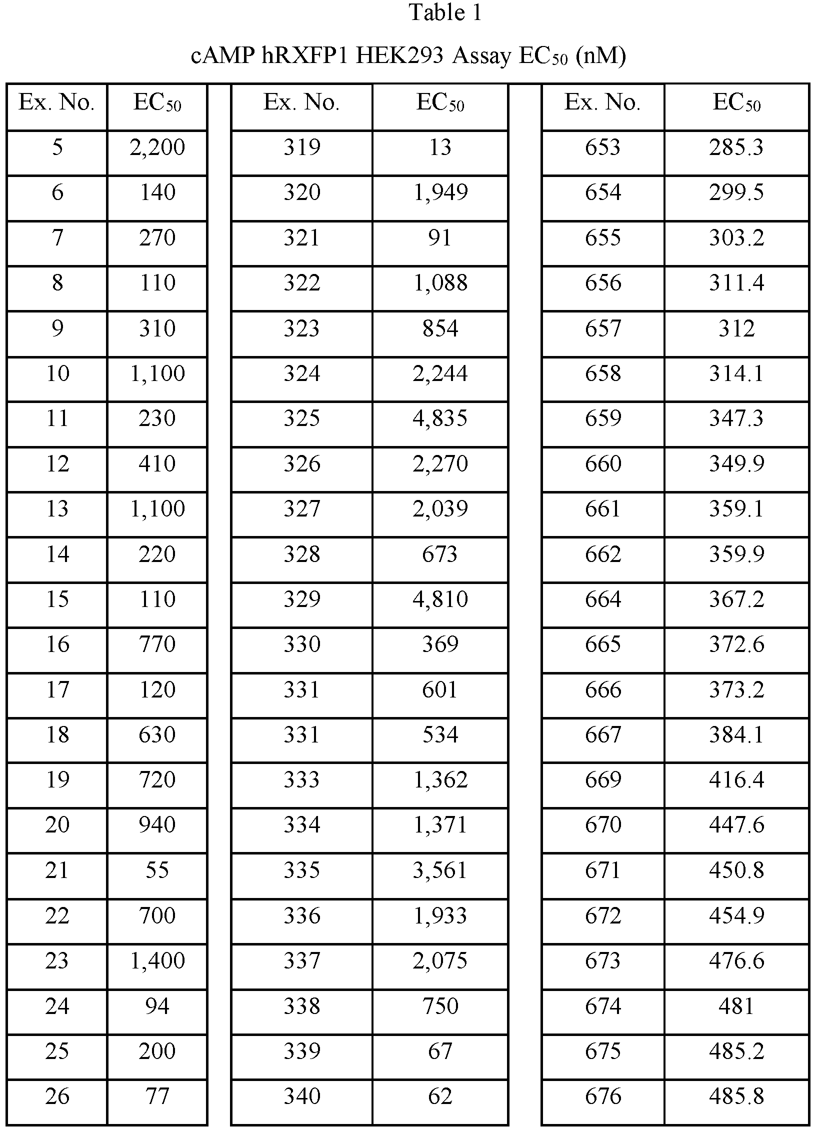

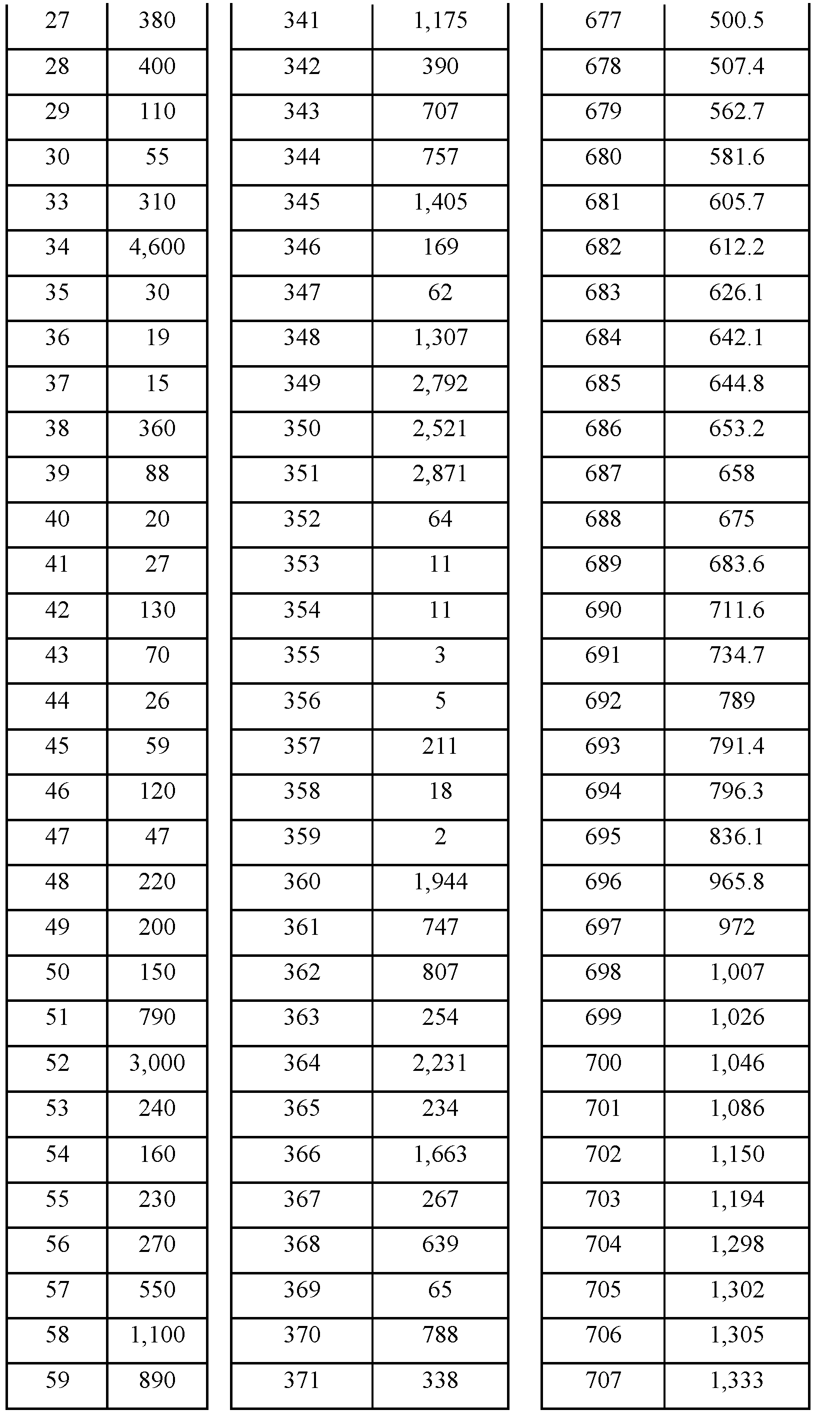

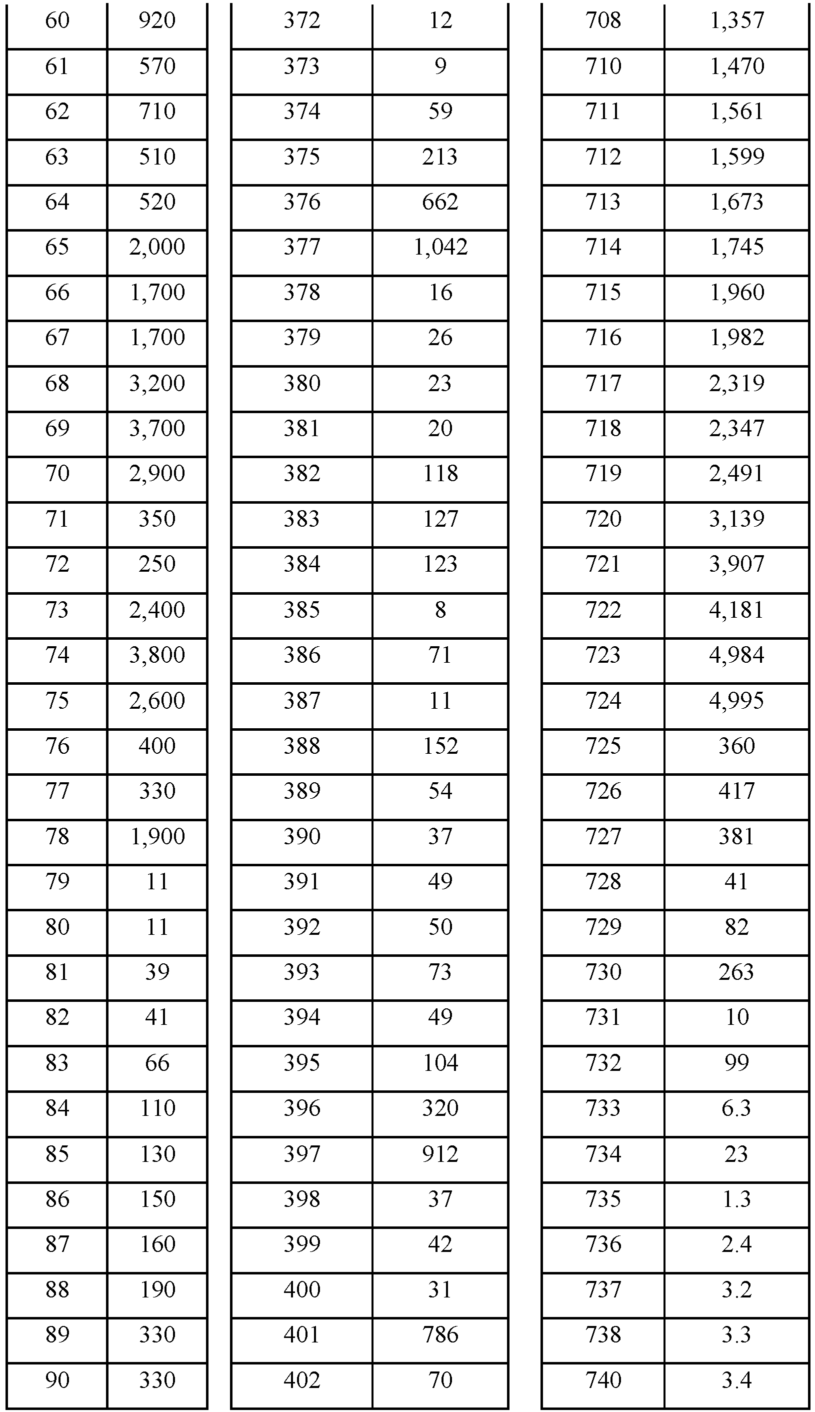

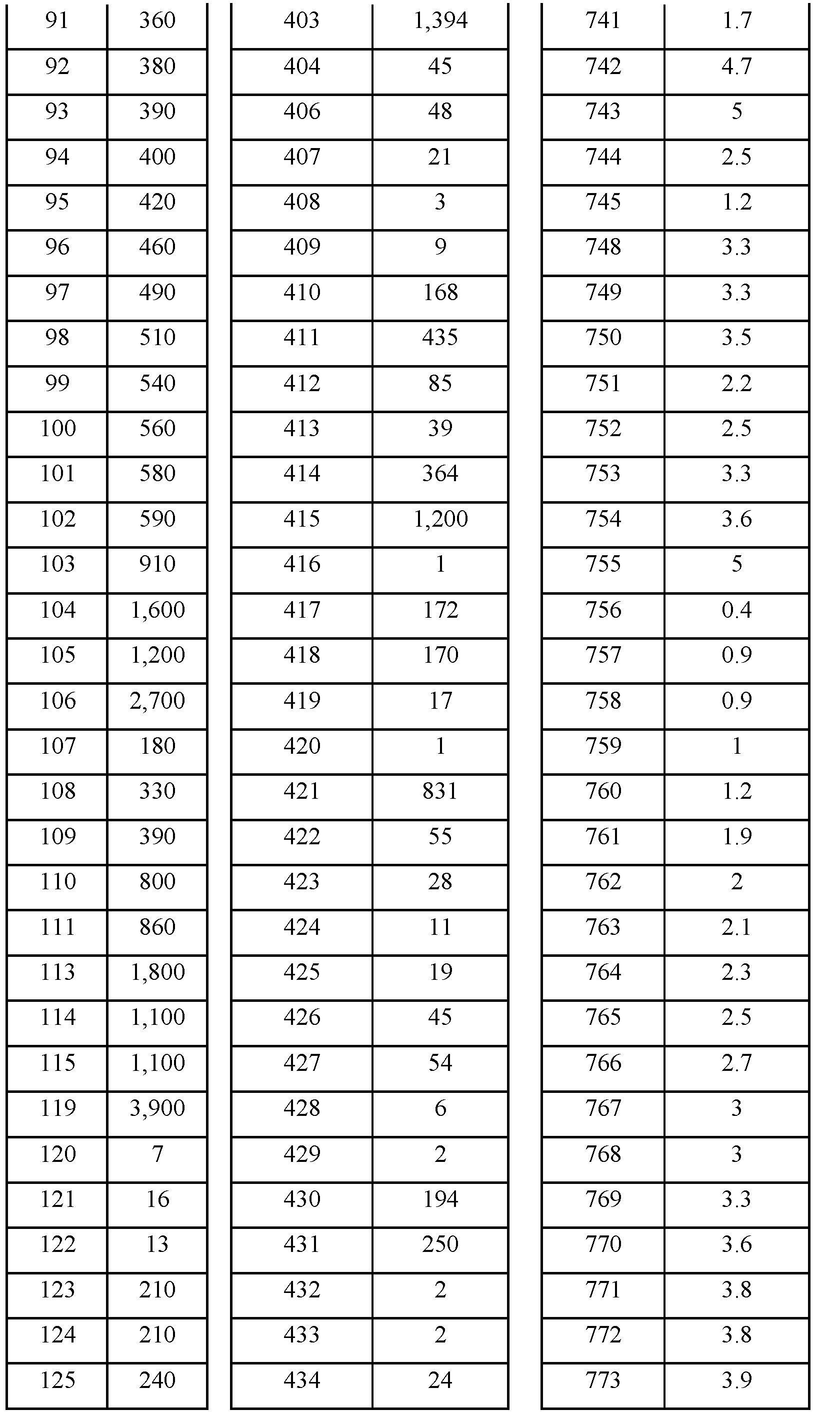

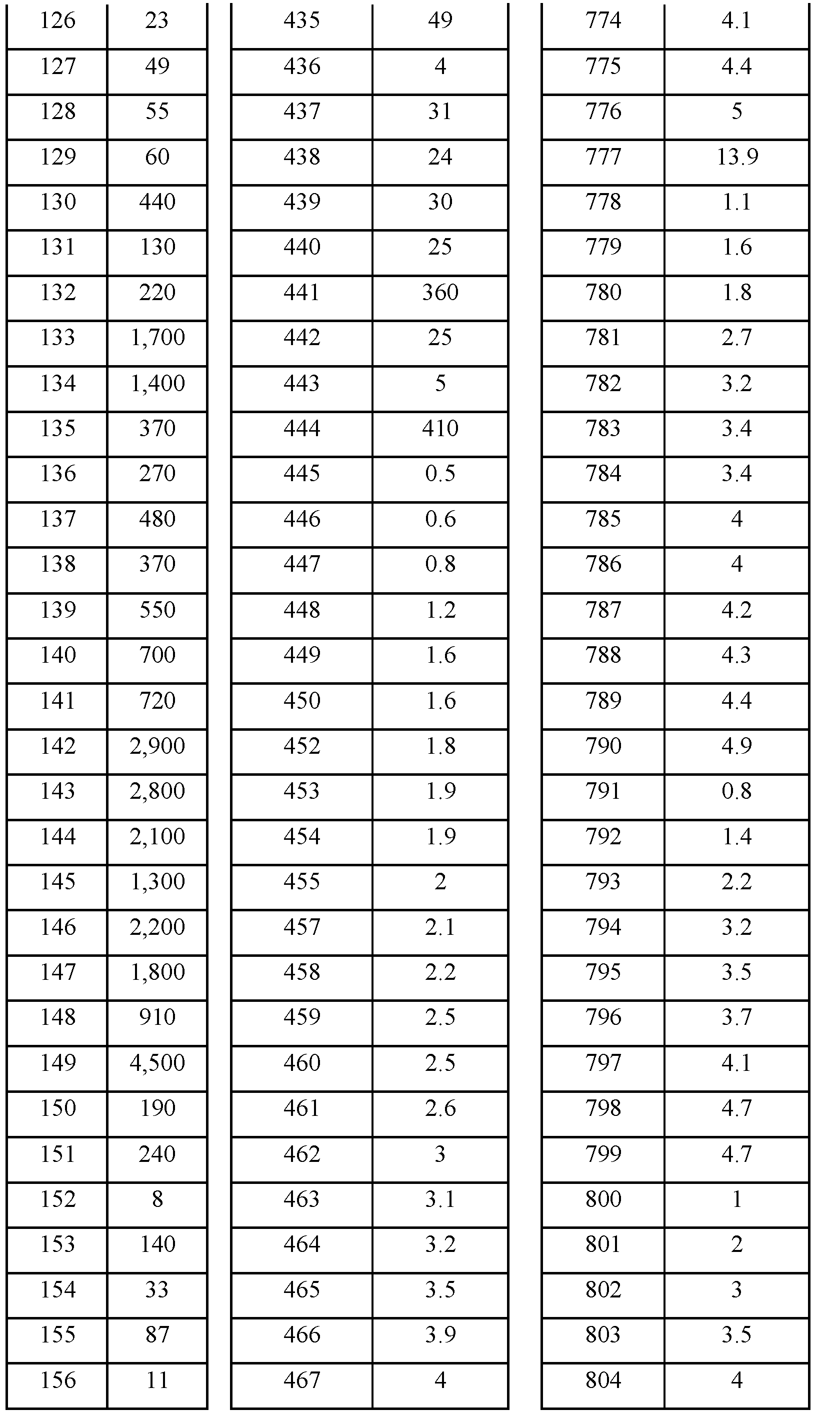

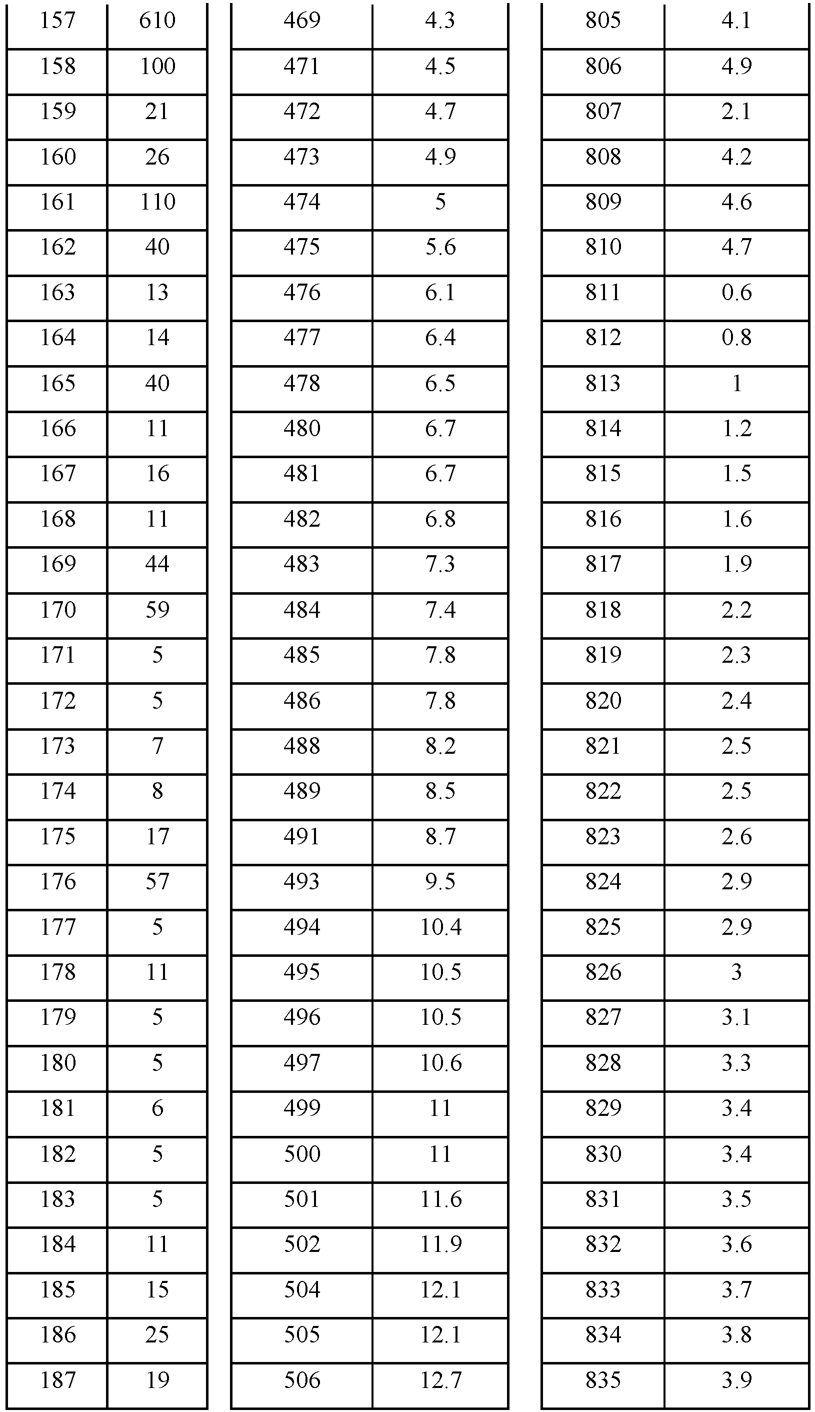

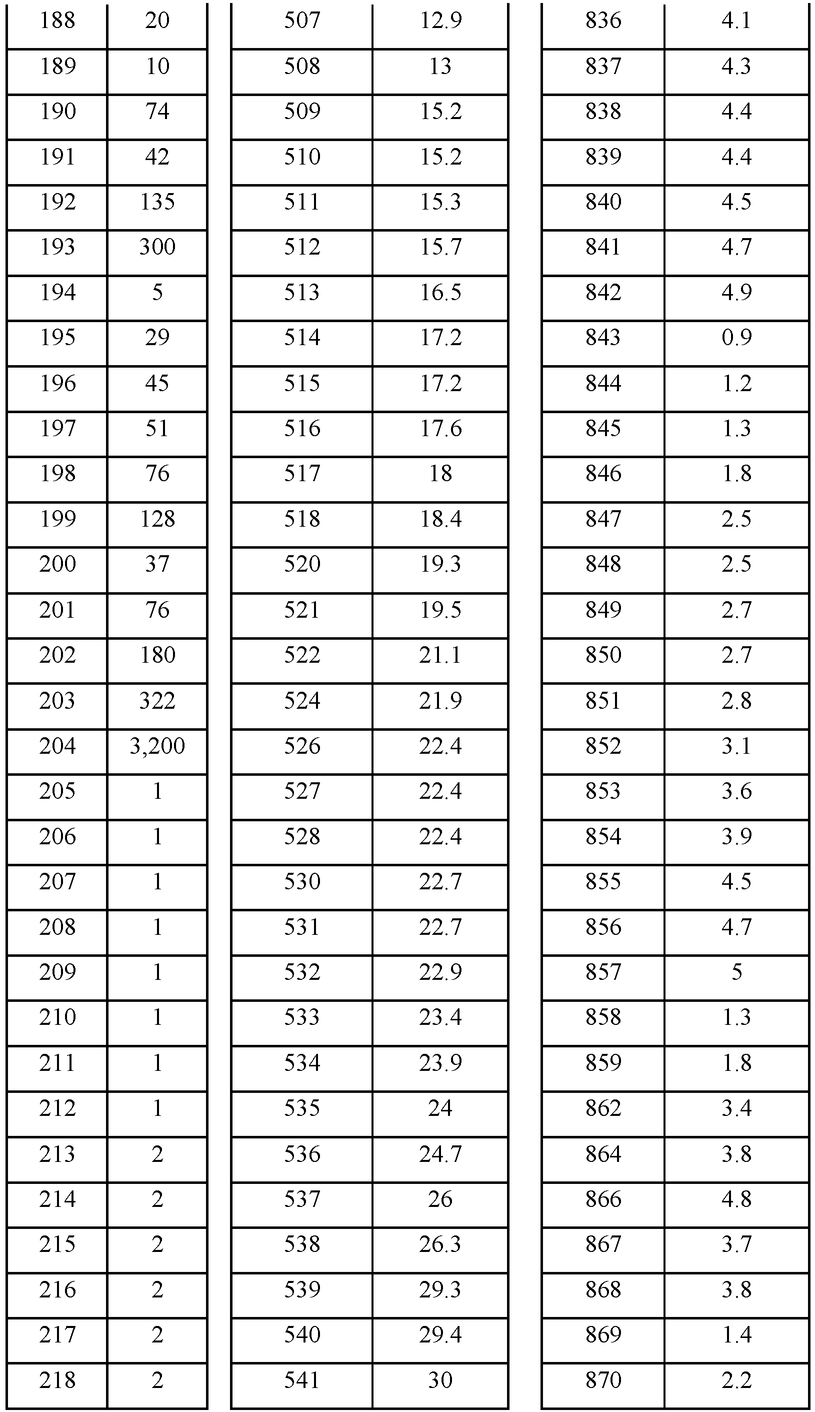

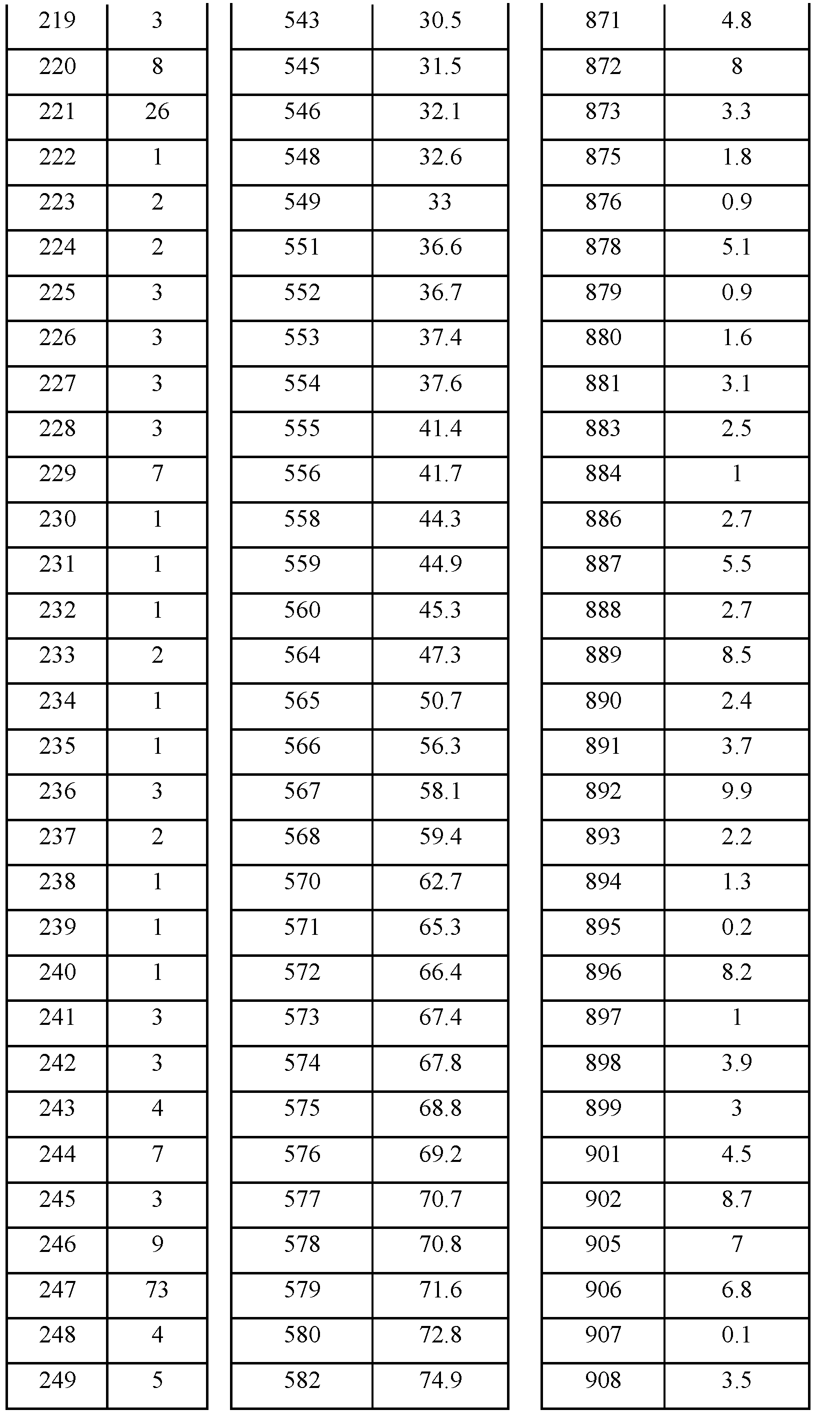

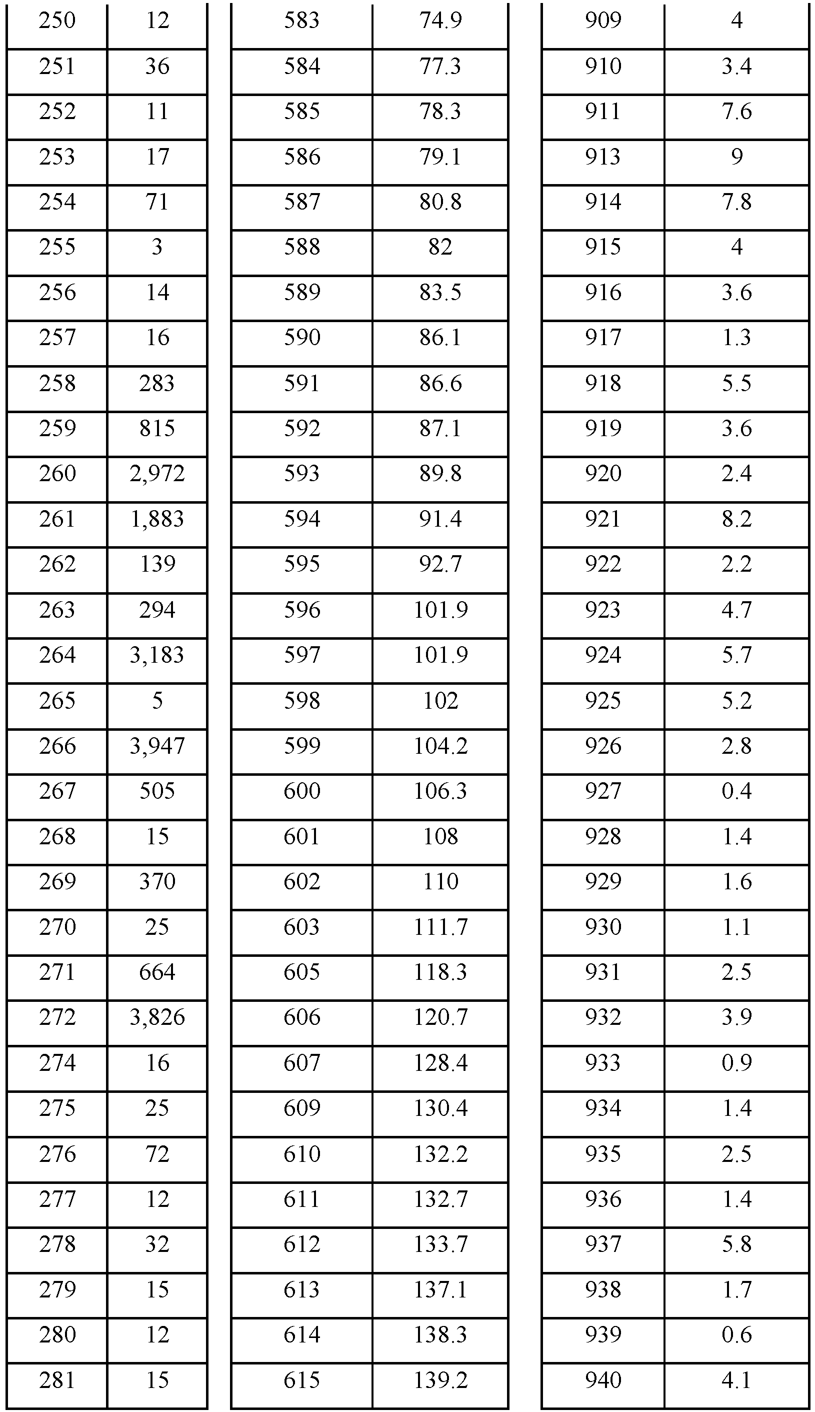

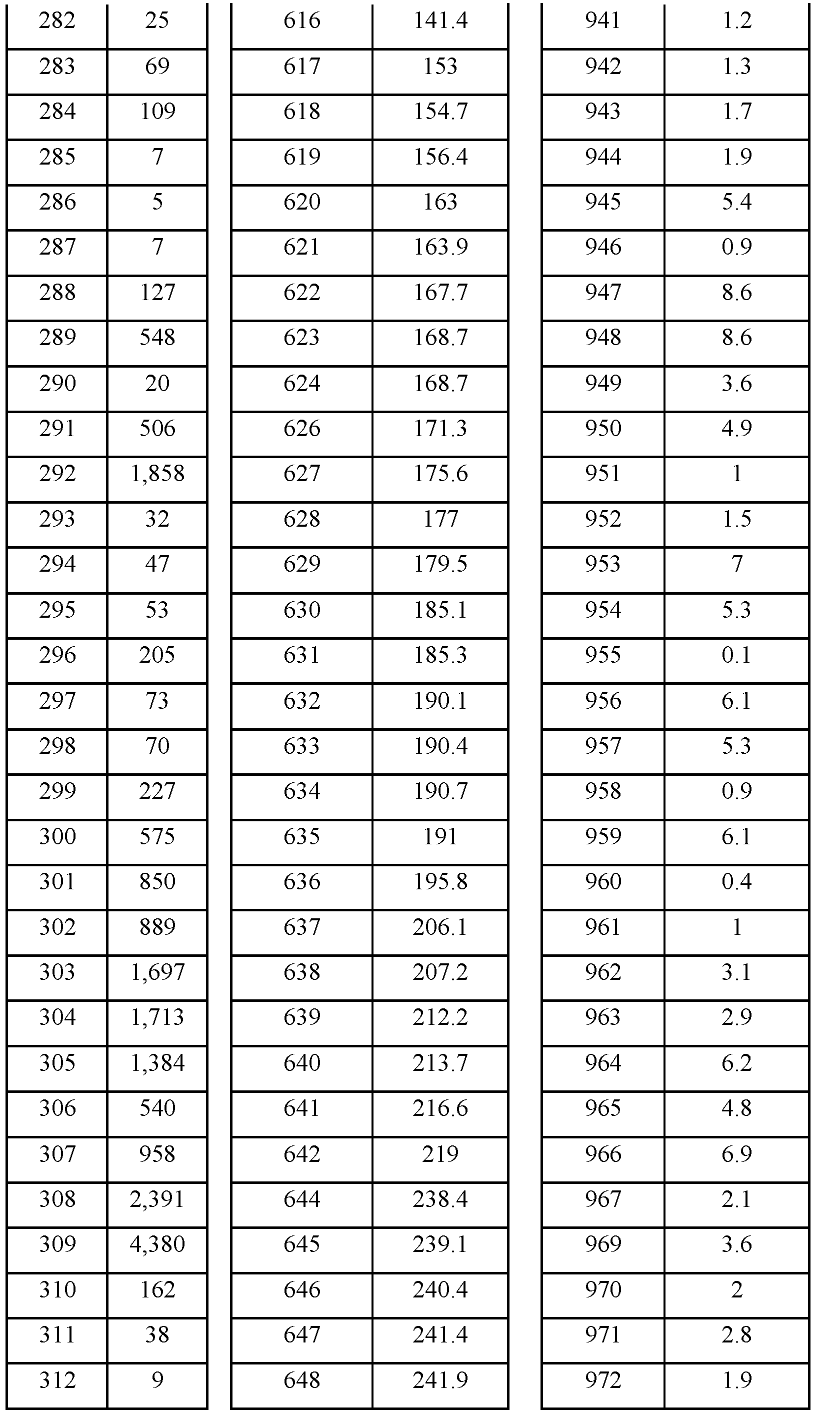

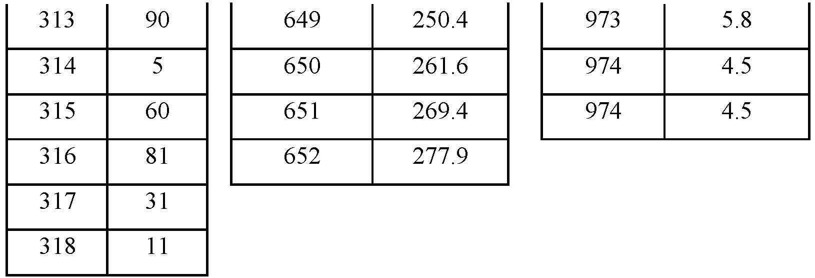

### PHARMACEUTICAL COMPOSITIONS AND METHODS OF USE

The compounds of Formula (I) are RXFP1 receptor agonists and may find use in the treatment of medical indications such as heart failure (e.g., heart failure with reduced ejection fraction (HF_{R}EF) or heart failure with preserved ejection fraction. (HF_{P}EF)), fibrotic diseases, and related diseases such as lung disease (e.g., idiopathic pulmonary fibrosis or pulmonary hypertension), kidney disease (e.g., chronic kidney disease), or hepatic disease (e.g., non-alcoholic steatohepatitis and portal hypertension). The compounds of Formular (I) can also be used to treat disorders that are a result of or a cause of arterial stiffness, reduced arterial elasticity, reduced arterial compliance and distensibility including hypertension, kidney disease, peripheral arterial disease, carotid and cerebrovascular disease (i.e stroke and dementia), diabetes, microvascular disease resulting in end organ damage, coronary artery disease, and heart failure. The compounds described herein may also be used in the treatment of pre-eclampsia.

Another aspect of the invention is a pharmaceutical composition comprising a compound of Formula (I) and a pharmaceutically acceptable carrier.

Another aspect of the invention is a pharmaceutical composition comprising a compound of Formula (I) for use in the treatment of a relaxin-associated disorder and a pharmaceutically acceptable carrier.

Another aspect of the invention provides compounds of Formula (I) for use in a method of treating a disease associated with relaxin comprising administering an effective amount of a compound of Formula (I).

Another aspect of the invention provides compounds of Formula (I) for use in a method of treating a cardiovascular disease comprising administering an effective amount of a compound of Formula (I) to a patient in need thereof.

Another aspect of the invention provides compounds of Formula (I) for use in a method of treating heart failure comprising administering an effective amount of a compound of Formula (I) to a patient in need thereof.

Another aspect of the invention proviees compounds of Formula (I) for use in a method of treating fibrosis comprising administering a therapeutically effective amount of a compound of Formula (I) to a patient in need thereof.

Another aspect of the invention provides compounds for Formula (I) for use in a method of treating a disease associated with fibrosis comprising administering a therapeutically effective amount of a compound of Formula (I) to a patient in need thereof.

Another aspect of the invention provides compounds of Formula (I) for use in a method of treating idiopathic pulmonary fibrosis comprising administering a therapeutically effective amount of a compound of Formula (I) to a patient in need thereof.

Another aspect of the invention provides compounds of Formula (I) for use in a method of treating a kidney disease (e.g., chronic kidney disease), comprising administering a therapeutically effective amount of a compound of Formula (I) to a patient in need thereof.

Another aspect of the invention provides compounds of Formula (I) for use in a method of treating or preventing kidney failure, comprising administering a therapeutically effective amount of a compound of Formula (I) to a patient in need thereof.

Another aspect of the invention provides compounds of Formula (I) for use in a method of improving, stabilizing or restoring renal function in a patient in need thereof, comprising administering a therapeutically effective amount of a compound of Formula (I) to the patient.

Another aspect of the invention provides compounds of Formula (I) for use in a method of treating idiopathic pulmonary fibrosis comprising administering a therapeutically effective amount of a compound of Formula (I) to a patient in need thereof.

Another aspect of the invention provides compounds of Formula (I) for use in a method of treating a kidney disease (e.g., chronic kidney disease), comprising administering a therapeutically effective amount of a compound of Formula (I) to a patient in need thereof.

Another aspect of the invention provides compounds of Formula (I) for use in a method of treating a hepatic disease comprising administering a therapeutically effective amount of a compound of Formula (I) to a patient in need thereof.

Another aspect of the invention provides compounds of Formula (I) for use in a method of treating non-alcoholic steatohepatitis and portal hypertension comprising administering a therapeutically effective amount of a compound of Formula (I) to a patient in need thereof.

Another aspect of the invention is a compound of Formula (I) for use in the prophylaxis and/or treatment of a relaxin-associated disorder.

Unless otherwise specified, the following terms have the stated meanings.

The term "patient" or "subject" refers to any human or non-human organism that could potentially benefit from treatment with a RXFP1 agonist as understood by practioners in this field. Exemplary subjects include human beings of any age with risk factors for cardiovascular disease. Common risk factors include, but are not limited to, age, sex, weight, family history, sleep apnea, alcohol or tobacco use, physical inactivity arrythmia or signs of insulin resistance such as acanthosis nigricans, hypertension, dyslipidemia, or polycystic ovary syndrome (PCOS).

"Treating" or "treatment" cover the treatment of a disease-state as understood by practitioners in this field and include the following: (a) inhibiting the disease-state, *i.e.,* arresting it development; (b) relieving the disease-state, *i.e.,* causing regression of the disease state; and/or (c) preventing the disease-state from occurring in a mammal, in particular, when such mammal is predisposed to the disease-state but has not yet been diagnosed as having it.

"Preventing" or "prevention" cover the preventive treatment (*i.e.,* prophylaxis and/or risk reduction) of a subclinical disease-state aimed at reducing the probability of the occurrence of a clinical disease-state as understood by practitioners in this field. Patients are selected for preventative therapy based on factors that are known to increase risk of suffering a clinical disease state compared to the general population. "Prophylaxis" therapies can be divided into (a) primary prevention and (b) secondary prevention. Primary prevention is defined as treatment in a subject that has not yet presented with a clinical disease state, whereas secondary prevention is defined as preventing a second occurrence of the same or similar clinical disease state. "Risk reduction" or "reducing risk" covers therapies that lower the incidence of development of a clinical disease state. As such, primary and secondary prevention therapies are examples of risk reduction.

"Therapeutically effective amount" is intended to include an amount of a compound of the present invention that is effective when administered alone or in combination with other agents to treat disorders as understood by practitioners in this field. When applied to a combination, the term refers to combined amounts of the active ingredients that result in the preventive or therapeutic effect, whether administered in combination, serially, or simultaneously.

"Disorders of the cardiovascular system" or "cardiovascular disorders" include for example the following disorders: hypertension (high blood pressure), peripheral and cardiac vascular disorders, coronary heart disease, stable and unstable angina pectoris, heart attack, myocardial insufficiency, abnormal heart rhythms (or arrhythmias), persistent ischemic dysfunction ("hibernating myocardium"), temporary postischemic dysfunction ("stunned myocardium"), heart failure, disturbances of peripheral blood flow, acute coronary syndrome, heart failure, heart muscle disease (cardiomyopathy), myocardial infarction and vascular disease (blood vessel disease).

"Heart failure" includes both acute and chronic manifestations of heart failure, as well as more specific or related types of disease, such as advanced heart failure, postacute heart failure, cardio-renal syndrome, heart failure with impaired kidney function, chronic heart failure, chronic heart failure with mid-range ejection fraction (HFmEF), compensated heart failure, decompensated heart failure, right heart failure, left heart failure, global failure, ischemic cardiomyopathy, dilated cardiomyopathy, heart failure associated with congenital heart defects, heart valve defects, heart failure associated with heart valve defects, mitral stenosis, mitral insufficiency, aortic stenosis, aortic insufficiency, tricuspid stenosis, tricuspid insufficiency, pulmonary stenosis, pulmonary valve insufficiency, heart failure associated with combined heart valve defects, myocardial inflammation (myocarditis), chronic myocarditis, acute myocarditis, viral myocarditis, diabetic heart failure, alcoholic cardiomyopathy, heart failure associated with cardiac storage disorders, diastolic heart failure, systolic heart failure, acute phases of worsening heart failure, heart failure with preserved ejection fraction (HFpEF), heart failure with reduced ejection fraction (HFrEF), chronic heart failure with reduced ejection fraction (HFrEF), chronic heart failure with preserved ejection fraction (HFpEF), post myocardial remodeling, angina, hypertension, pulmonary hypertension and pulmonary artery hypertension.

"Fibrotic disorders" encompasses diseases and disorders characterized by fibrosis, including among others the following diseases and disorders: hepatic fibrosis, cirrhosis of the liver, NASH, pulmonary fibrosis or lung fibrosis, cardiac fibrosis, endomyocardial fibrosis, nephropathy, glomerulonephritis, interstitial renal fibrosis, fibrotic damage resulting from diabetes, bone marrow fibrosis and similar fibrotic disorders, scleroderma, morphea, keloids, hypertrophic scarring (also following surgical procedures), naevi, diabetic retinopathy, proliferative vitreoretinopathy and disorders of the connective tissue (for example sarcoidosis).

Relaxin-associated disorders include but are not limited to disorders of the cardiovascular system and fibrotic disorders.

The compounds of this invention can be administered by any suitable means, for example, orally, such as tablets, capsules (each of which includes sustained release or timed release formulations), pills, powders, granules, elixirs, tinctures, suspensions (including nanosuspensions, microsuspensions, spray-dried dispersions), syrups, and emulsions; sublingually; bucally; parenterally, such as by subcutaneous, intravenous, intramuscular, or intrasternal injection, or infusion techniques (*e.g*., as sterile injectable aqueous or non-aqueous solutions or suspensions); nasally, including administration to the nasal membranes, such as by inhalation spray; topically, such as in the form of a cream or ointment; or rectally such as in the form of suppositories. They can be administered alone, but generally will be administered with a pharmaceutical carrier selected on the basis of the chosen route of administration and standard pharmaceutical practice.

"Pharmaceutical composition" means a composition comprising a compound of the invention in combination with at least one additional pharmaceutically acceptable carrier. A "pharmaceutically acceptable carrier" refers to media generally accepted in the art for the delivery of biologically active agents to animals, in particular, mammals, including, *i.e.,* adjuvant, excipient or vehicle, such as diluents, preserving agents, fillers, flow regulating agents, disintegrating agents, wetting agents, emulsifying agents, suspending agents, sweetening agents, flavoring agents, perfuming agents, anti-bacterial agents, anti-fungal agents, lubricating agents and dispensing agents, depending on the nature of the mode of administration and dosage forms.

Pharmaceutically acceptable carriers are formulated according to a number of factors well within the purview of those of ordinary skill in the art. These include, without limitation: the type and nature of the active agent being formulated; the subject to which the agent-containing composition is to be administered; the intended route of administration of the composition; and the therapeutic indication being targeted. Pharmaceutically acceptable carriers include both aqueous and non-aqueous liquid media, as well as a variety of solid and semi-solid dosage forms. Such carriers can include a number of different ingredients and additives in addition to the active agent, such additional ingredients being included in the formulation for a variety of reasons, *e.g.,* stabilization of the active agent, binders, etc., well known to those of ordinary skill in the art. Descriptions of suitable pharmaceutically acceptable carriers, and factors involved in their selection, are found in a variety of readily available sources such as, for example, Allen, L.V., Jr. et al., Remington: The Science and Practice of Pharmacy (2 Volumes), 22nd Edition, Pharmaceutical Press (2012).

The dosage regimen for the compounds of the present invention will, of course, vary depending upon known factors, such as the pharmacodynamic characteristics of the particular agent and its mode and route of administration; the species, age, sex, health, medical condition, and weight of the recipient; the nature and extent of the symptoms; the kind of concurrent treatment; the frequency of treatment; the route of administration, the renal and hepatic function of the patient, and the effect desired.

By way of general guidance, the daily oral dosage of each active ingredient, when used for the indicated effects, will range between about 0.01 to about 5000 mg per day, preferably between about 0.1 to about 1000 mg per day, and most preferably between about 0.1 to about 250 mg per day. Intravenously, the most preferred doses will range from about 0.01 to about 10 mg/kg/minute during a constant rate infusion. Compounds of this invention may be administered in a single daily dose, or the total daily dosage may be administered in divided doses of two, three, or four times daily.

The compounds are typically administered in admixture with suitable pharmaceutical diluents, excipients, or carriers (collectively referred to herein as pharmaceutical carriers) suitably selected with respect to the intended form of administration, *e.g.,* oral tablets, capsules, elixirs, and syrups, and consistent with conventional pharmaceutical practices.

Dosage forms (pharmaceutical compositions) suitable for administration may contain from about 1 milligram to about 2000 milligrams of active ingredient per dosage unit. In these pharmaceutical compositions the active ingredient will ordinarily be present in an amount of about 0.1-95% by weight based on the total weight of the composition. A typical capsule for oral administration contains at least one of the compounds of the present invention (250 mg), lactose (75 mg), and magnesium stearate (15 mg). The mixture is passed through a 60 mesh sieve and packed into a No. 1 gelatin capsule. A typical injectable preparation is produced by aseptically placing at least one of the compounds of the present invention (250 mg) into a vial, aseptically freeze-drying and sealing. For use, the contents of the vial are mixed with 2 mL of physiological saline, to produce an injectable preparation.

The compounds may be employed in combination with other suitable therapeutic agents useful in the treatment of diseases or disorders including: anti-atherosclerotic agents, anti-dyslipidemic agents, anti-diabetic agents, anti-hyperglycemic agents, anti-hyperinsulinemic agents, anti-thrombotic agents, anti-retinopathic agents, anti-neuropathic agents, anti-nephropathic agents, anti-ischemic agents, anti-hypertensive agents, anti-obesity agents, anti-hyperlipidemic agents, anti-hypertriglyceridemic agents, anti-hypercholesterolemic agents, anti-restenotic agents, anti-pancreatic agents, lipid lowering agents, anorectic agents, memory enhancing agents, anti-dementia agents, cognition promoting agents, appetite suppressants, agents for treating heart failure, agents for treating peripheral arterial disease, agents for treating malignant tumors, and anti-inflammatory agents.

The additional therapeutic agents may include ACE inhibitors, β-blockers, diuretics, mineralocorticoid receptor antagonists, ryanodine receptor modulators, SERCA2a activators, renin inhibitors, calcium channel blockers, adenosine A1 receptor agonists, partial adenosine A1 receptor, dopamine β-hydroxylase inhibitors, angiotensin II receptor antagonists, angiotensin II receptor antagonists with biased agonism for select cell signaling pathways, combinations of angiotensin II receptor antagonists and neprilysin enzyme inhibitors, neprilysin enzyme inhibitors, soluble guanylate cyclase activators, myosin ATPase activators, rho-kinase 1 inhibitors, rho-kinase 2 inhibitors, apelin receptor agonists, nitroxyl donating compounds, calcium-dependent kinase II inhibitors, antifibrotic agents, galectin-3 inhibitors, vasopressin receptor antagonists, RXFP1 receptor modulators, natriuretic peptide receptor agonists, transient receptor potential vanilloid-4 channel blockers, anti-arrhythmic agents, *I_{f}* "funny current" channel blockers, nitrates, digitalis compounds, inotropic agents and β-receptor agonists, cell membrane resealing agents for example Poloxamer 188, anti-hyperlipidemic agents, plasma HDL-raising agents, anti-hypercholesterolemic agents, cholesterol biosynthesis inhibitors (such as HMG CoA reductase inhibitors), LXR agonist, FXR agonist, probucol, raloxifene, nicotinic acid, niacinamide, cholesterol absorption inhibitors, bile acid sequestrants, anion exchange resins, quaternary amines, cholestyramine, colestipol, low density lipoprotein receptor inducers, clofibrate, fenofibrate, bezafibrate, ciprofibrate, gemfibrizol, vitamin B6, vitamin B12, anti-oxidant vitamins, anti-diabetes agents, platelet aggregation inhibitors, fibrinogen receptor antagonists, aspirin and fibric acid derivatives, PCSK9 inhibitors, aspirin, and P2Y12 Inhibitors such as Clopidogrel.

The additional therapeutic agents may also include nintedanib, Pirfenidone, LPA1 antagonists, LPA1 receptor antagonists, GLP1 analogs, tralokinumab (IL-13, AstraZeneca), vismodegib (hedgehog antagonist, Roche), PRM-151 (pentraxin-2, TGF beta-1, Promedior), SAR-156597 (bispecific Mab IL-4&IL-13, Sanofi), simtuzumab ((anti-lysyl oxidase-like 2 (anti-LOXL2) antibody, Gilead), CKD-942, PTL-202 (PDE inh./pentoxifylline/NAC oral control. release, Pacific Ther.), omipalisib (oral PI3K/mTOR inhibitor, GSK), IW-001 (oral sol. bovine type V collagen mod., ImmuneWorks), STX-100 (integrin alpha V/ beta-6 ant, Stromedix/ Biogen), Actimmune (IFN gamma), PC-SOD (midismase; inhaled, LTT Bio-Pharma / CKD Pharm), lebrikizumab (anti-IL-13 SC humanized mAb, Roche), AQX-1125 (SHIP1 activator, Aquinox), CC-539 (JNK inhibitor, Celgene), FG-3019 (FibroGen), SAR-100842 (Sanofi), and obeticholic acid (OCA or INT-747, Intercept).

The above other therapeutic agents, when employed in combination with the compounds of the present invention may be used, for example, in those amounts indicated in the *Physicians' Desk Reference,* as in the patents set out above, or as otherwise determined by practitioners in the art.

Particularly when provided as a single dosage unit, the potential exists for a chemical interaction between the combined active ingredients. For this reason, when the compound of the present invention and a second therapeutic agent are combined in a single dosage unit they are formulated such that although the active ingredients are combined in a single dosage unit, the physical contact between the active ingredients is minimized (that is, reduced). For example, one active ingredient may be enteric coated. By enteric coating one of the active ingredients, it is possible not only to minimize the contact between the combined active ingredients, but also, it is possible to control the release of one of these components in the gastrointestinal tract such that one of these components is not released in the stomach but rather is released in the intestines. One of the active ingredients may also be coated with a material that affects a sustained-release throughout the gastrointestinal tract and also serves to minimize physical contact between the combined active ingredients. Furthermore, the sustained-released component can be additionally enteric coated such that the release of this component occurs only in the intestine. Still another approach would involve the formulation of a combination product in which the one component is coated with a sustained and/or enteric release polymer, and the other component is also coated with a polymer such as a low viscosity grade of hydroxypropyl methylcellulose (HPMC) or other appropriate materials as known in the art, in order to further separate the active components. The polymer coating serves to form an additional barrier to interaction with the other component.

The compounds of the present invention are also useful as standard or reference compounds, for example as a quality standard or control, in tests or assays involving the RXFP1. Such compounds may be provided in a commercial kit, for example, for use in pharmaceutical research involving RXFP1 activity. For example, a compound of the present invention could be used as a reference in an assay to compare its known activity to a compound with an unknown activity. This would ensure the experimenter that the assay was being performed properly and provide a basis for comparison, especially if the test compound was a derivative of the reference compound. When developing new assays or protocols, compounds according to the present invention could be used to test their effectiveness. The compounds of the present invention may also be used in diagnostic assays involving RXFP1.

Also disclosed is an article of manufacture. As used herein, article of manufacture is intended to include, but not be limited to, kits and packages. The article of manufacture may comprise: (a) a first container; (b) a pharmaceutical composition located within the first container, wherein the composition, comprises a first therapeutic agent, comprising a compound of the present invention or a pharmaceutically acceptable salt form thereof; and, (c) a package insert stating that the pharmaceutical composition can be used for the treatment of dyslipidemias and the sequelae thereof. Alternatively, the package insert may state that the pharmaceutical composition can be used in combination (as defined previously) with a second therapeutic agent for the treatment of dyslipidemias and the sequelae thereof. The article of manufacture can further comprise: (d) a second container, wherein components (a) and (b) are located within the second container and component (c) is located within or outside of the second container. Located within the first and second containers means that the respective container holds the item within its boundaries.

The first container is a receptacle used to hold a pharmaceutical composition. This container can be for manufacturing, storing, shipping, and/or individual/bulk selling. First container is intended to cover a bottle, jar, vial, flask, syringe, tube (*e.g.,* for a cream preparation), or any other container used to manufacture, hold, store, or distribute a pharmaceutical product.

The second container is one used to hold the first container and, optionally, the package insert. Examples of the second container include, but are not limited to, boxes (*e.g.,* cardboard or plastic), crates, cartons, bags (*e.g.,* paper or plastic bags), pouches, and sacks. The package insert can be physically attached to the outside of the first container via tape, glue, staple, or another method of attachment, or it can rest inside the second container without any physical means of attachment to the first container. Alternatively, the package insert is located on the outside of the second container. When located on the outside of the second container, it is preferable that the package insert is physically attached via tape, glue, staple, or another method of attachment. Alternatively, it can be adjacent to or touching the outside of the second container without being physically attached.

The package insert is a label, tag, marker, etc. that recites information relating to the pharmaceutical composition located within the first container. The information recited will usually be determined by the regulatory agency governing the area in which the article of manufacture is to be sold (*e.g.,* the United States Food and Drug Administration). Preferably, the package insert specifically recites the indications for which the pharmaceutical composition has been approved. The package insert may be made of any material on which a person can read information contained therein or thereon. Preferably, the package insert is a printable material (*e.g.,* paper, plastic, cardboard, foil, adhesive-backed paper or plastic, etc.) on which the desired information has been formed (*e.g.,* printed or applied).

### CHEMICAL METHODS

The compounds of this invention can be made by various methods known in the art including those of the following schemes and in the specific embodiments section. The structure numbering and variable numbering shown in the synthetic schemes are distinct from, and should not be confused with, the structure or variable numbering in the claims or the rest of the specification. The variables in the schemes are meant only to illustrate how to make some of the compounds of this invention.

The disclosure is not limited to the foregoing illustrative examples and the examples should be considered in all respects as illustrative and not restrictive, and all changes which come within the meaning and range of equivalency of the claims are therefore intended to be embraced.

It will also be recognized that another major consideration in the planning of any synthetic route in this field is the judicious choice of the protecting group used for protection of the reactive functional groups present in the compounds described in this invention. An authoritative account describing the many alternatives to the trained practitioner is Greene, T.W. et al., Protecting Groups in Organic Synthesis, 4th Edition, Wiley (2007)).

Abbreviations are defined as follows: "1 x" for once, "2 x" for twice, "3 x" for thrice, " °C" for degrees Celsius, "aq" for aqueous, "eq" or "equiv." for equivalent or equivalents, "g" for gram or grams, "mg" for milligram or milligrams, "L" for liter or liters, "mL" for milliliter or milliliters, "µL" for microliter or microliters, "N" for normal, "M" for molar, "nM" for nanomolar, "pM" for picomolar, "mol" for mole or moles, "mmol" for millimole or millimoles, "min" for minute or minutes, "h" for hour or hours, "rt" for room temperature, "RT" for retention time, "atm" for atmosphere, "psi" for pounds per square inch, "conc." for concentrate, "aq" for "aqueous", "sat." for saturated, "MW" for molecular weight, "MS" or "Mass Spec" for mass spectrometry, "ESI" for electrospray ionization mass spectroscopy, "LC-MS" for liquid chromatography mass spectrometry, "HPLC" for high pressure liquid chromatography, "RP HPLC" for reverse phase HPLC, "NMR" for nuclear magnetic resonance spectroscopy, "SFC" for super critical fluid chromatography, "¹H" for proton, "δ " for delta, "s" for singlet, "d" for doublet, "t" for triplet, "q" for quartet, "m" for multiplet, "br" for broad, "Hz" for hertz, "MHz" for megahertz, and "α", "β", "R", "S", "E", and "Z" are stereochemical designations familiar to one skilled in the art.
- AcCl: acetyl chloride
- AcOH: acetic acid
- AIBN: Azobisisobutyronitrile
- BHFFT: bis(tetramethylene)fluoroformadmidinium hexafluorophosphate
- Boc: tert-butyloxycarbonyl
- BuLi: butyl lithium
- DAST: Diethylaminosulfur trifluoroide
- DCE: Dichloroethane
- DCM: Dichloromethane
- DIEA: diispropyl ethylamine
- DMAP: 4-dimethylamino pyridine
- DMF: Dimethylformamide
- DPPA: Diphenyl phosphorylazide
- Et₂O: diethyl ether
- EtOAc: Ethylacetate
- EtOH: Ethanol
- HATU: (1-[Bis(dimethylamino)methylene]-1H-1,2,3-triazolo[4,5-b]pyridinium 3-oxid hexafluorophosphate)
- HMPA: hexamethylphosphoramide
- IPA: isopropanol
- i-Pr: Isopropyl
- KHMDS: potassium bis(trimethylsilyl)amide\
- LDA: lithium diisopropyl amide
- MeCN: Acetonitrile
- MeOH: Methanol
- Me: Methyl
- NBS: N-bromosuccinimide
- Pd/C: palladium on carbon
- pTsOH: p-toluenesulfonic acid
- PyBroP: Bromotripyrrolidinophosphonium hexafluorophosphate
- T3P: 2,4,6-Tripropyl-1,3,5,2,4,6-trioxatriphosphorinane-2,4,6-trioxide
- TBAF: tetra-n-butyl ammonium fluoride
- t-Bu: tert-butyl
- Teoc: 2-(trimethylsilyl)ethyl carboxylate
- TFA: trifluoro acetic acid
- TFAA: trifluoro acetic anhydride
- THF: Tetrahydrofuran
- TsOH: Tolulenesulfonic acid
- XPhos-Pd-G2: 2nd generation XPhos precatalyst CAS no. 1310584-14-5

The following methods were used in the exemplified examples, except where noted otherwise. Purification of intermediates and final products was carried out via either normal or reverse phase chromatography. Normal phase chromatography was carried out using prepacked SiO₂ cartridges eluting with either gradients of hexanes and ethyl acetate or DCM and MeOH unless otherwise indicated. Reverse phase preparative HPLC was carried out using C18 columns with UV 220 nm or prep LCMS detection eluting with gradients of Solvent A (90% water, 10% MeOH, 0.1% TFA) and Solvent B (10% water, 90% MeOH, 0.1% TFA) or with gradients of Solvent A (95% water, 5% ACN, 0.1% TFA) and Solvent B (5% water, 95% ACN, 0.1% TFA) or with gradients of Solvent A (95% water, 2% ACN, 0.1% HCOOH) and Solvent B (98% ACN, 2% water, 0.1% HCOOH) or with gradients of Solvent A (95% water, 5% ACN, 10 mM NH₄OAc) and Solvent B (98% ACN, 2% water, 10 mM NH₄OAc) or with gradients of Solvent A (98% water, 2% ACN, 0.1% NH₄OH) and Solvent B (98% ACN, 2% water, 0.1% NH₄OH).
LC/MS methods employed in characterization of examples are listed below.

### Method A:

Instrument: Waters Acquity coupled with a Waters MICROMASS^{®} ZQ Mass Spectrometer
Linear gradient of 2 to 98% B over 1 min, with 0.5 min hold time at 98% B UV visualization at 220 nm
Column: Waters BEH C18, 2.1 x 50 mm
Flow rate: 0.8 mL/min (Method A)
Mobile Phase A: 0.05% TFA, 100% water
Mobile Phase B: 0.05% TFA, 100% acetonitrile

### Method B:

Instrument: Shimadzu Prominence HPLC coupled with a Shimadzu LCMS-2020 Mass Spectrometer
Linear gradient of 0 to 100% B over 3 min, with 0.75 min hold time at 100% B UV visualization at 220 nm
Column: Waters Xbridge C18, 2.1 x 50 mm, 1.7 um particles
Flow rate: 1 mL/min
Mobile Phase A: 10 mM ammonium acetate, 95:5 water:acetonitrile
Mobile Phase B: 10 mM ammonium acetate, 5:95 water:acetonitrile

### Method C:

Instrument: Shimadzu Prominence HPLC coupled with a Shimadzu LCMS-2020 Mass Spectrometer
Linear gradient of 0 to 100% B over 3 min, with 0.75 min hold time at 100% B UV visualization at 220 nm
Column: Waters Xbridge C18, 2.1 x 50 mm, 1.7 um particles
Flow rate: 1 mL/min
Mobile Phase A: 0.1% TFA, 95:5 water:acetonitrile
Mobile Phase B: 0.1% TFA, 5:95 water:acetonitrile

### Method D:

Instrument: Waters Acquity coupled with a Waters MICROMASS^{®} ZQ Mass Spectrometer
Linear gradient of 10% B to 98% B over 1 min, with 0.5 min hold time at 98% B UV visualization at 220 nm
Column: Waters Acquity GEN C18, 2.1 x 50 mm, 1.7 um particles
Flow rate: 1 mL/min
Mobile Phase A: 0.05% TFA, 100% water
Mobile Phase B: 0.05% TFA, 100% acetonitrile

### Method E:

Instrument: Shimadzu Prominence HPLC coupled with a Shimadzu LCMS-2020 Mass Spectrometer
Linear gradient of 0 to 100% B over 1 min, with 0.5 min hold time at 100% B UV visualization at 220 nm
Column: Waters Acquity BEH C18, 2.1 x 50 mm, 1.7 um particles
Flow rate: 1 mL/min
Mobile Phase A: 10 mM ammonium acetate, 95:5 water:acetonitrile
Mobile Phase B: 10 mM ammonium acetate, 5:95 water:acetonitrile
NMR Employed in Characterization of Examples. ¹H NMR spectra were obtained with Bruker or JEOL^{®} Fourier transform spectrometers operating at frequencies as follows: ¹H NMR: 400 MHz (Bruker or JEOL^{®}) or 500 MHz (Bruker or JEOL^{®}). Spectra data are reported in the format: chemical shift (multiplicity, coupling constants, number of hydrogens). Chemical shifts are specified in ppm downfield of a tetramethylsilane internal standard (δ units, tetramethylsilane = 0 ppm) and/or referenced to solvent peaks, which in ¹H NMR spectra appear at 2.51 ppm for DMSO-d₆, 3.30 ppm for CD₃OD, 1.94 ppm for CD₃CN, and 7.24 ppm for CDCl₃.

Scheme I describes how norbornyl examples may be prepared starting from norbornyl intermediates **I-1**, which are either commercially available (R¹ = R² = H) or prepared as described in subsequent Schemes. Starting from protected amino esters such as **I-1**, the olefin may be reduced under hydrogenation conditions (e.g., Pd/C, H₂). The resulting Boc-protected amine **I-2** may then be deprotected using TFA, followed by subsequent acylation with a benzoic acid employing a variety of amide bond forming conditions (e.g., HATU or BOP-Cl, with DIEA) to furnish **I-3**. Ester **I-3** could then be converted directly to examples of the general structure **I** by treatment with an appropriate amine and AlMe₃. Alternatively, the sequence of amide bond forming reactions may be reversed starting with saponification of **I-2** followed by treatment with T3P^{®} and an appropriate amine to produce **I-4**. Deprotection and acylation according to the previously described conditions would then also yield Examples of the general structure **I.** In addition, the initial hydrogenation step may be delayed until any point in the sequence without altering the outcome of the steps described in Scheme 1. 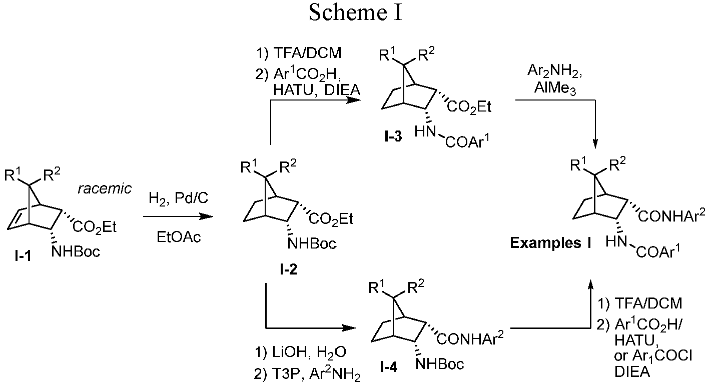 Scheme II shows one method for the production of norbornyl analogs with substitution at the C7 position starting from **II-1.** Treatment of **II-1** with malic anhydride furnished **II-2,** which was selectively hydrogenated and solvolyzed to produce **II-3**. Curtius reaction of **II-3** with DPPA in the presence of trimethylsilanol led to the formation of **II-4**. Deprotection of the Teoc group under standard conditions led to the formation of amine **II-5** which could be elaborated to Examples of the general structure **II** directly. Alternatively, structure **II** could be treated with ozone to furnish ketone **II-6**, which could in turn be functionalized via a variety of standard transformations including but not limited to organometallic addition (e.g., R-Li, R-MgBr), Wittig or Horner-Wadsworth Emmons (HWE) olefination, or acetal formation. These products could serve as Examples of the general structure **I** or **II** themselves, or alternatively could serve as intermediates for further elaboration. In addition, the ozonolysis step could be conducted earlier in the synthetic sequence for strategic reasons without altering the outcome of the synthetic steps outline in Scheme II. 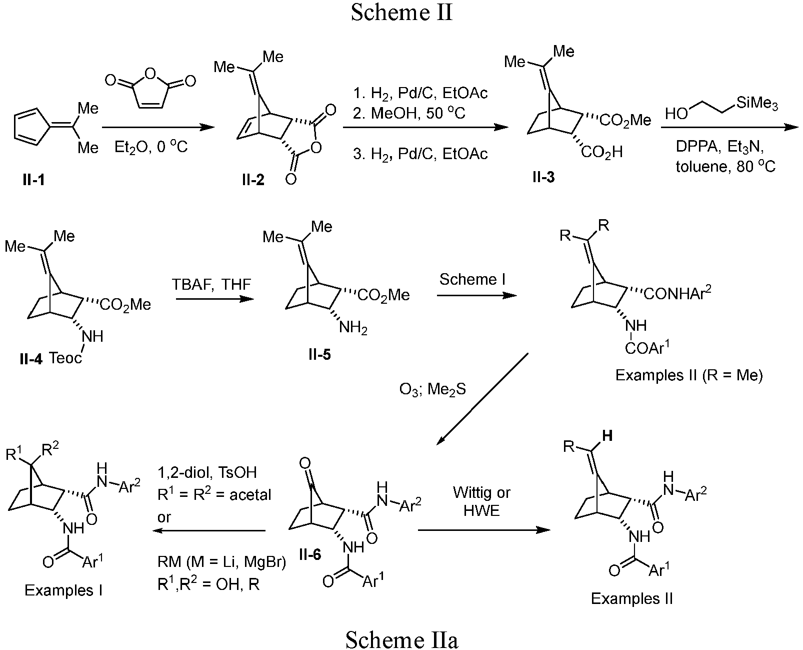 The norbornyl intermediate IIa-8 could also be prepared by the general route shown in Scheme IIa from furan-2,5-dione and ferrocenium hexafluorophosphate. Diels Alder condensation, followed by hydrolysis to IIa-2, Curtius rearrangement to the intermediate amine which was reduced under hydrogenation conditions and subsequently protected to generate intermediate IIa-3. Cleavage of the benzyl ester and cross coupling to NR₁R₂ generated intermediates with the general structure IIa-5. Conversion of the C7 hydroxy group to the ketone followed by Wittig olefination generated major isomer intermediate IIa-8. The major isomer was separated from the minor isomer by chromatography and the racemate separated into enantiopure IIa-8 (-).

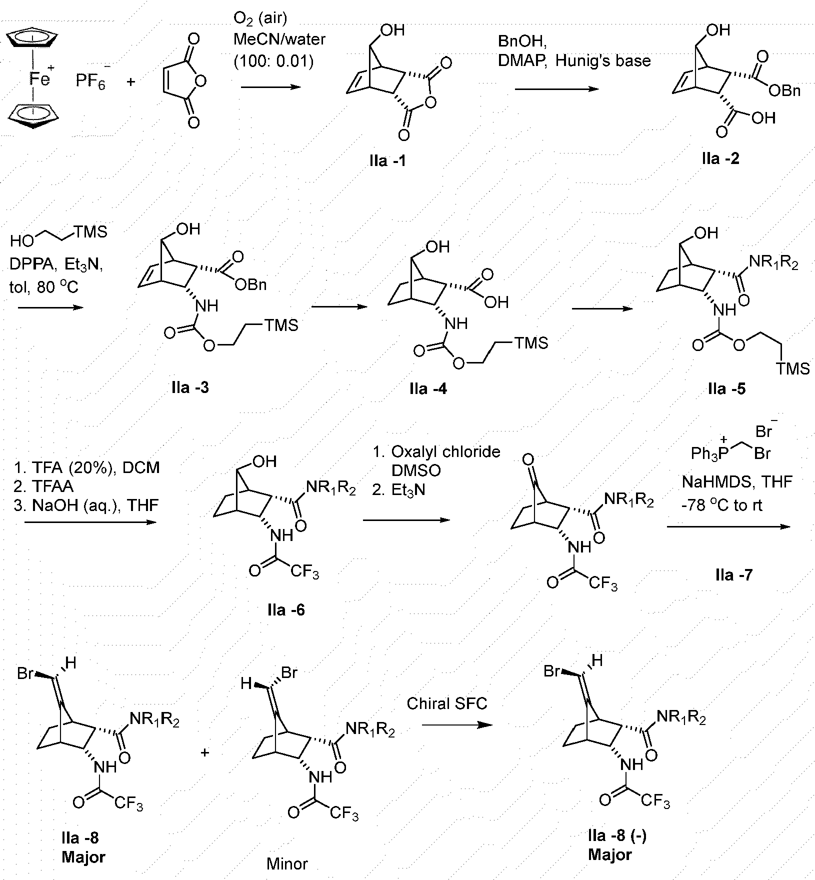

Scheme III shows how the norbornyl cores may be fluorinated. Staring with II-4, the material was deprotonated with LDA and fluorinated with N-fluoro-bisbenezenesulfonimide, then subsequently elaborated to Examples of the general structure III according to the path outlined in Scheme I. Alternatively, III-1 could be treated with ozone similarly to as in Scheme II to furnish III-2. Intermediate III-2 could then be treated with Wittig or HWE conditions and processed as in Scheme II to furnish Examples of the general structure III).

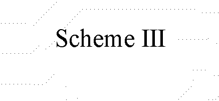

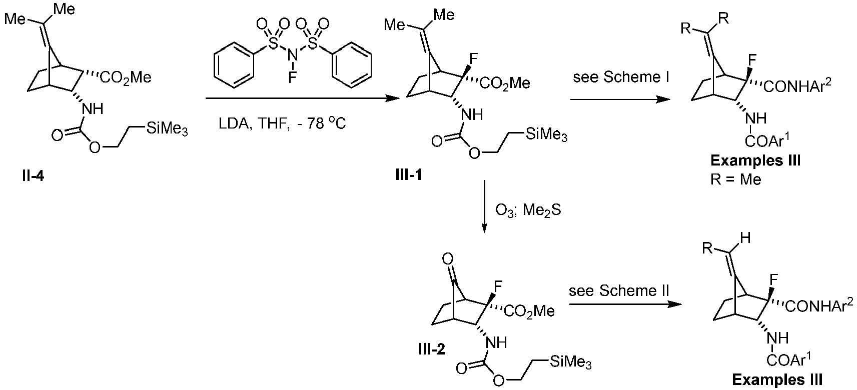

Scheme IV demonstrates the preparation of a variety of diverse C-7 methylidene substituted norbornyl cores from a common intermediate bromide. II-4 was converted to IV-1 through standard Teoc-deprotection, TFA acylation procedure. Ester IV-1 was converted to amide IV-2 according to the AlMe3 procedure outlined in Scheme I and IV-2 was ozonolyzed to ketone IV-3 as in Scheme II. Wittig methylination furnished olefin IV-4, which was treated with bromine and KHMDS to furnish IV-5 and IV-6 as a mixture of isomers that was separated by silica gel chromatography. Isomer IV-6 was then subjected to chiral SFC purification to produce a single enanatiomer of IV, which was then deprotected to IV-7. Amine IV-7 could then be acylated according to the methods outlined in Scheme 1 to yield IV-8. Vinyl bromides may also be further functionalized (e.g., Suzuki, Negishi and Semmelhack reaction conditions, among others) which led to diverse Examples of the general structure IV or corresponding intermediates which could then be elaborated further. Alternatively, the vinyl bromide functionalization steps could be performed on the IV-6, and the resulting material processed similarly to Examples of the general structure IV.

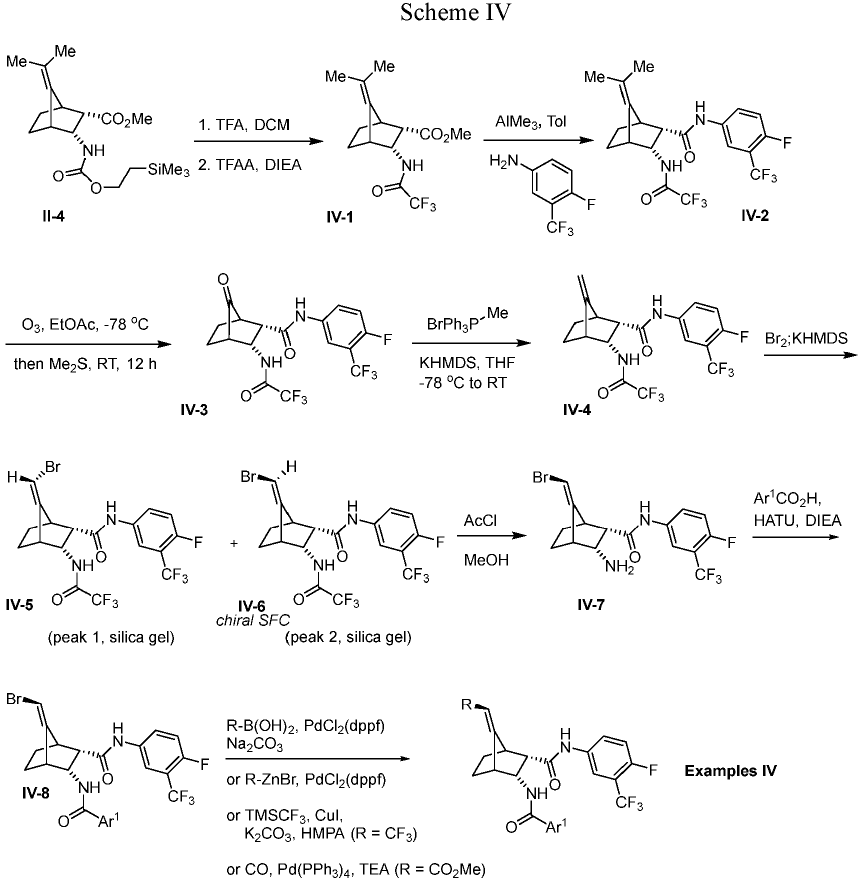

Scheme V demonstrates a method for the introduction of diverse amides on highly elaborated norbornyl carboxylates. An intermediate **V-1,** prepared according to the methods described in Schemes I-IV, could be treated with pivaloyl chloride, DMAP, and DIEA to furnish **V-2.** The resulting imide could be displaced directly with an amine in the presence of AlMe₃ to furnish Examples of the general structure II. Alternatively, **V-2** could be hydrolyzed through the use of hydroxide (e.g., LiOH, NaOH, etc.) to furnish **V-3,** which could functionalized further according the methods outlined in Scheme I to furnish Examples of the general structure **II.**

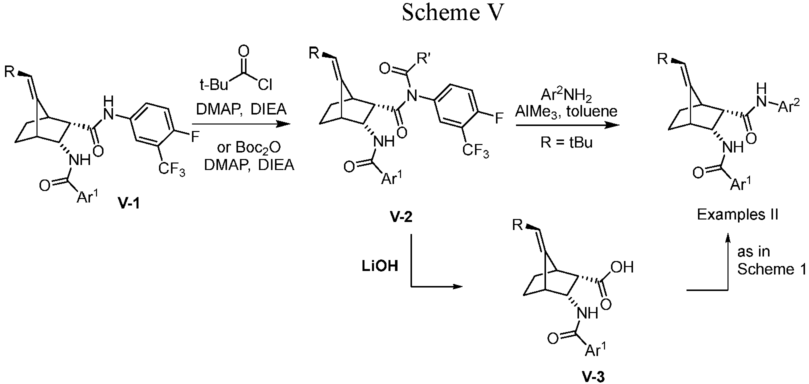

Scheme VI describes the synthesis of bicyclic benzoates (Ar1 = -Ar'-Ar", where Ar" = substituted phenyl, heteroaryl, or heterocyclic olefin) for use in Schemes I-IV. Aryl bromides **VI-1** (where R can be H, Me, Bn, tBu among others) were treated with aryl, heteroaryl, and heterocyclic vinyl boronic acids (or esters) **VI-2,** a palladium catalyst (e.g., Pd(PPh₃)₄, PdCl₂(dppf), etc.), an appropriate base (e.g., Na₂CO₃, K₃PO₄, etc.) under Suzuki reaction conditions to furnish bicycle **VI-3.** Alternatively the coupling partners could be reversed, employing aryl boronic acid **VI-4** and halide **VI-5** under similar conditions to likewise yield **VI-3.** Where **VI-3,** R ≠ H, the benzoate could be cleaved employing saponification (e.g., LiOH, water for R = Me), acidic (e.g., TFA/DCM for R = tBu), or hydrogenolytic conditions (e.g., Pd/C, H₂ for R = Bn) to furnish **VI-6.** Benzoic acid **VI-6** could then be coupled to the norbornyl cores as is outlined in Schemes I-IV to furnish Examples of the general structures I or II or intermediates that could be further elaborated to Examples.

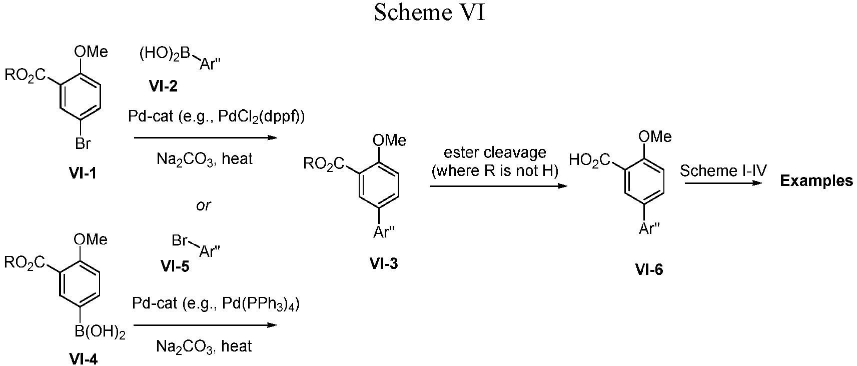

Scheme VII, outlines a synthesis of N-linked nitrogen-heterocycle bicyclic benzoates from benzoate intermediates **VI-1 or VI-4.** Treatment of **VI-1** with amine **VII-1** under either Hartwig-Buchwald reaction (e.g., Pd(OAc)₂, BINAP, Cs₂CO₃ among others) or Ullman reaction (e.g., CuI, proline, Cs₂CO₃ among others) conditions to furnish bicycle **VII-2.** Alternatively, **VII-2** could be prepared from **VI-4** according to Chan-Evans-Lam conditions (e.g., Cu(OAc)₂, TEA, O₂ among others). Intermediates **VII-2** could then be further functionalized in a similar manner to **VI-3** in Scheme VI via ester cleavage where required and further manipulation according to Schemes I-IV either directly to Examples of the general structures I or II or to intermediates that could be further elaborated to Examples.

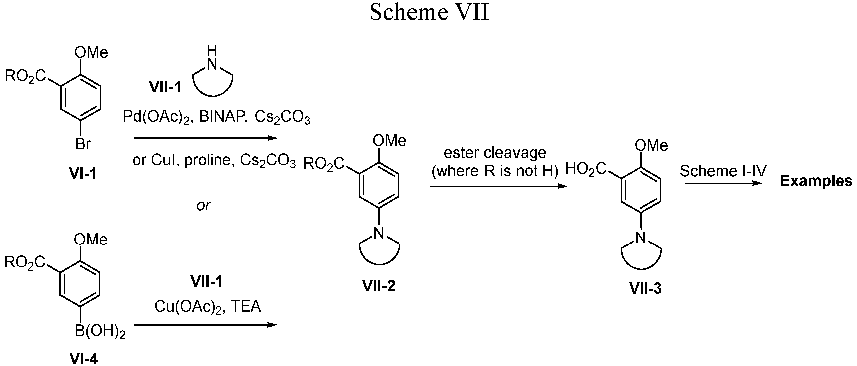

Scheme VIII illustrates a general route to mandelic acid-based biaryl analogs. Commercially available **VIII-1** was converted to the t-butyl ester **VIII-2,** then brominated to furnish **VIII-3.** Displacement of the bromide with acetic acid furnished intermediate **VIII-4,** which was then subjected to a Suzuki reaction as was described in Scheme VI to furnish **VIII-5** (acetate cleavage was concomitant with biaryl formation). The resulting acid was directly coupled to a norbornyl amine intermediate **VIII-6** as was described in Scheme I to furnish **VIII-7.** The t-butyl ester **VIII-7** could then be cleaved (TFA/DCM) to furnish Examples of the general structure **VIIIa.** Alternatively, the hydroxyl group in **VIII-7** could be elaborated with either the appropriate isocyanate or a two-step carbamate forming protocol (e.g., nitrophenyl chloroformate, TEA, followed by an amine) to furnish **VIII-8,** which could then be cleaved (TFA/DCM) to furnish Examples of the general structure **VIIIb.** 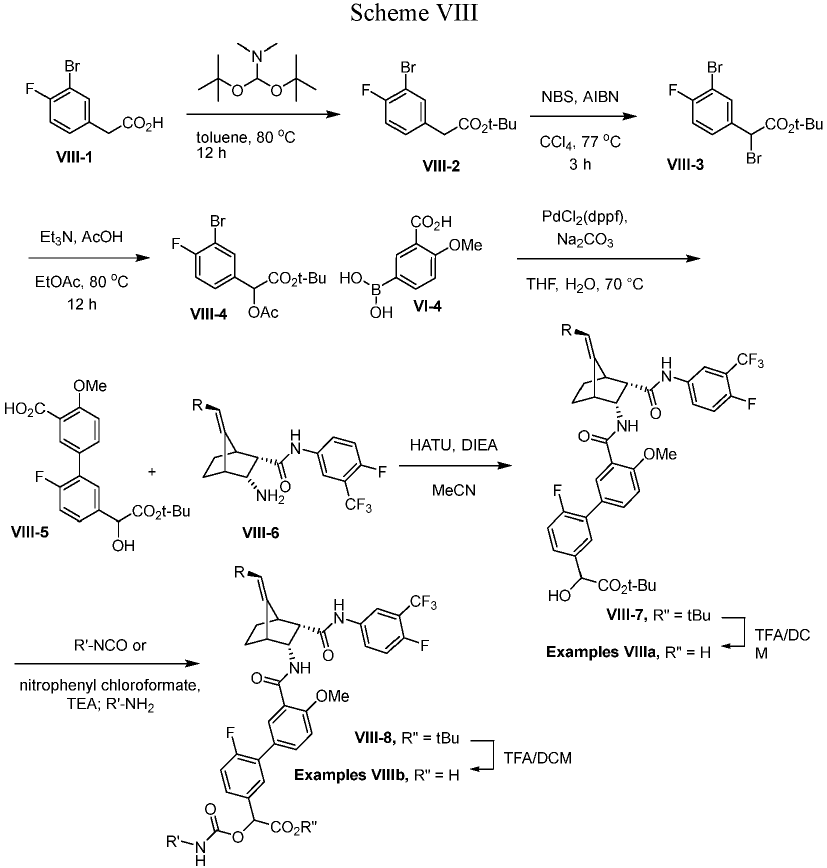 Scheme IX shows a modification to the steps in Scheme VIII that allow for the preparation of phenylglycine-based biaryl analogs. Intermediate **VIII-3** was treated with ammonia, followed by acylation to furnish intermediate **IX-1,** which was elaborated according to the methods outlined in Scheme VIII to furnish Examples of the general structure **IX.**

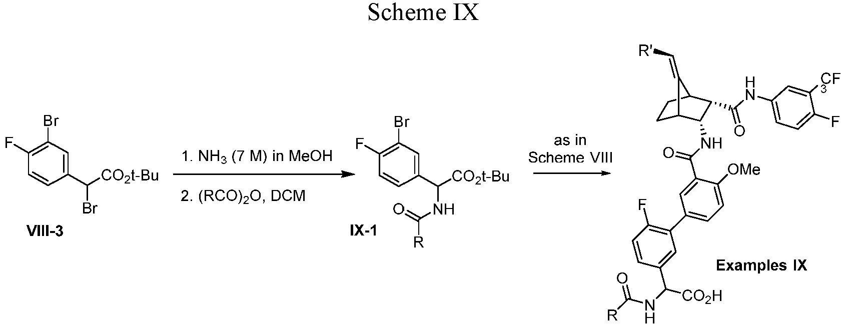

Scheme X describes a method whereby analogs with diverse aliphatic C-7 substituents could be prepared from Intermediate **X-1,** itself prepared according to the route outlined in Scheme VIII. Treatment of intermediate **X-1** with alkyl bromides under the conditions outlined in MacMillan et. al. (J. Am. Chem. Soc. 2016, 138, 8084-8087) followed by subsequent deprotection of the tBu ester led to Examples of the general structure **X.**

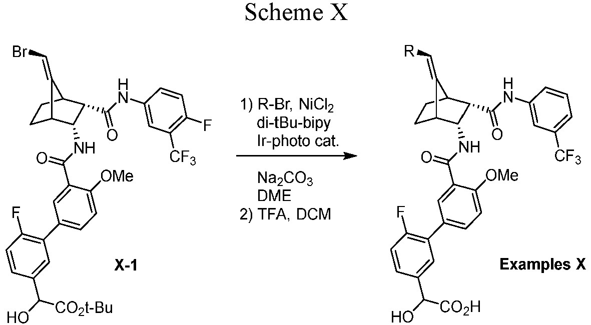

Scheme XI demonstrated a route to the preparation of analogs with diverse aliphatic aryl-substituents (R) which can be prepared from **Example 292.** Treatment of **Example 292** with alkyl bromides under the conditions outlined in MacMillan et. al. (J. Am. Chem. Soc. 2016, 138, 8084-8087) provided analogs of the general structure **XI.** 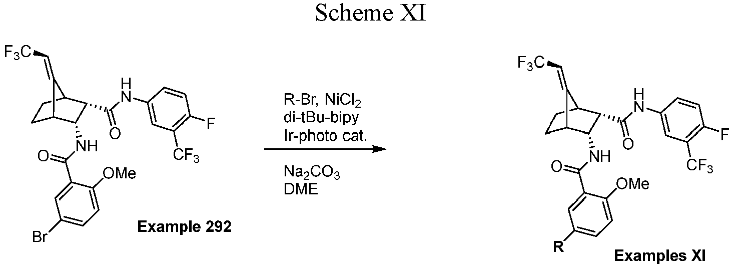 Scheme XII describes a route for the production of substituted isoxazoline analogs. Treatment of **XII-1** with NaOCl, followed by a substituted olefin with subsequent saponification of the ester provided intermediates **XII-2.** These intermediates were coupled with norbornyl amines according to the methods outlined in Scheme 1 to furnish Examples of the general structure **XII.**

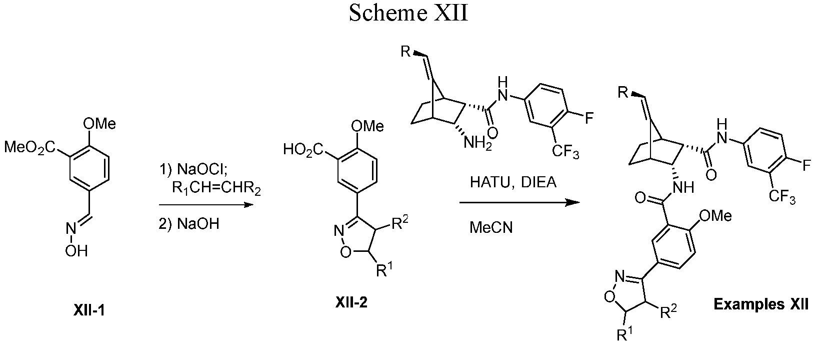

Scheme XIII describes a route for generation of analogs with diverse aryl substituents (Ar). Boronic acid VI-4 was treated with pinacol, followed by amide coupling with a norbornyl amide (prepared according to the Schemes above) to furnish **XIII-1.** Treatment of **XIII-1** with aryl halides under standard anhydrous Suzuki conditions led to the formation of analogs **XIII.**

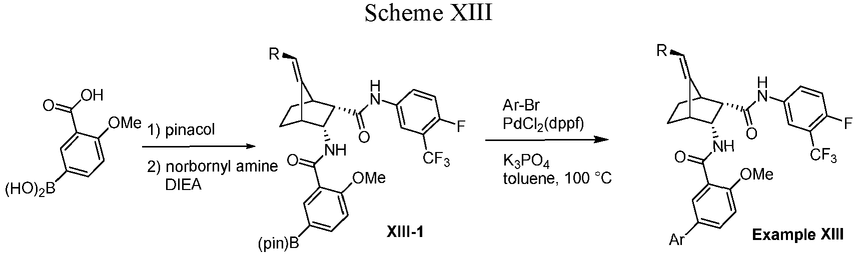

### Examples

### Example 5

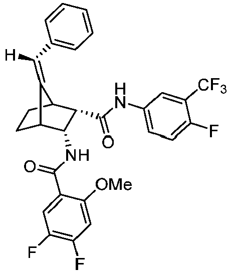

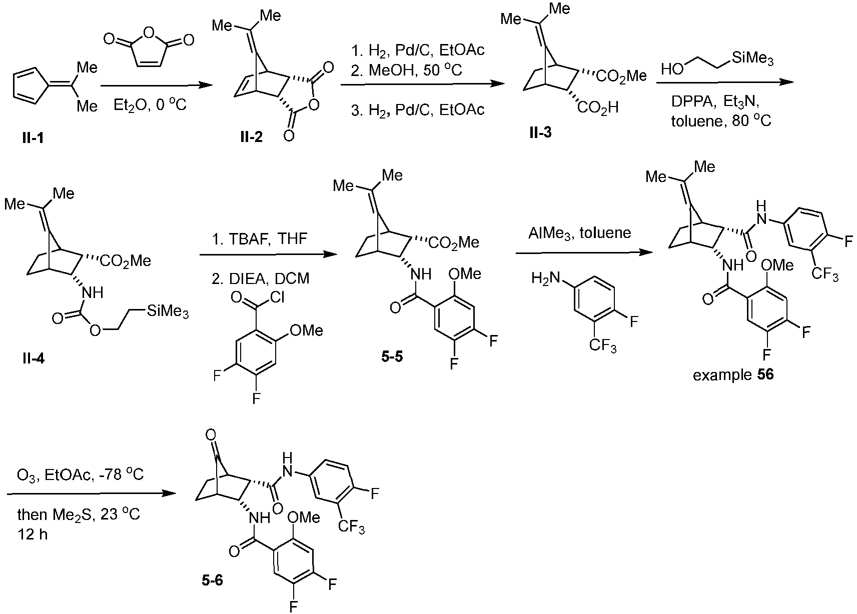

Intermediate **II-2**: At 0 °C, into the reaction vessel was added Et₂O (100 mL), 5-(propan-2-ylidene)cyclopenta-1,3-diene (**II-1,** 10 g, 94 mmol), and furan-2,5-dione (10 g, 102 mmol). The reaction mixture was stirred at 0 °C for 18 h, concentrated under reduced pressure and purified via silica gel chromatography to provide **II-2** (3.74 g, 18.3 mmol, 19.0% yield). Intermediate **II-2** is a known compound; please see: PCT Int. Appl., 2011163502, 29 Dec 2011.

Intermediate **II-3**: Into the reaction vessel was added **II-2** (2.74 g, 13.4 mmol), EtOAc (100 mL), pyridine (0.540 mL, 6.71 mmol), and Pd/C (70 mg, 0.070 mmol). The reaction mixture was stirred at 23 °C under 1 atm H₂ (H₂ balloon) for 60 min filtered through Celite, and concentrated under reduced pressure. The resulting intermediate was dissolved in methanol (50 mL) and heated at 50 °C for 12 h. Concentration of the reaction mixure under reduced pressure (azeotrope with toluene 3 x 15 mL) produced **II-3** (3.21 g, 13.5 mmol, 100% yield) that was used without further purification.

Intermediate **II-4**: Into the reaction vessel was added **II-3** (3.2 g, 13 mmol), Et₃N (3.38 mL, 24.3 mmol), toluene (75 mL), and diphenylphosphoryl azide (4.35 mL, 20.2 mmol). The reaction mixture was stirred at 23 °C for 1 h. The reaction mixture was subsequently heated at 85 °C for 30 min and 2-(trimethylsilyl)ethanol (4.83 mL, 33.7 mmol) was added. After stirring at 85 °C for 66 h, the reaction mixture was allowed to cool to 23 °C and purified via silica gel chromatography to provide racemic **II*-*4** (3.71 g, 10.5 mmol, 78.0% yield) LC-MS RT = 1.25 min; (M+H) = 354.1. Method A. Racemic **II-4** was separated into individual enantiomers using chiral SFC. Preparative chromatographic conditions: Instrument: Thar 350 SFC; Column: Whelko-RR, 5 x 50 cm, 10 micron; Mobile phase: 13% IPA/87% CO₂; Flow conditions: 300 mL/min, 100 Bar, 35 °C; Detector wavelength: 220 nm; Injections details: 4 injections of 3.5 mL of 59 g / 490 mL MeOH:DCM (4:1) 120 mg/mL in IPA. Analytical chromatographic conditions: Instrument: Thar analytical SFC; Column: Whelko-RR (0.46 x 25 cm, 5 micron; Mobile phase: 5% IPA/95% CO₂; Flow conditions: 3 mL/min, 140 Bar, 40 °C; Detector wavelength: 200-400 nm UV. Peak 1, RT = 3.496 min, >99% ee; Peak 2, RT = 4.417 min, >99% ee. Intermediate **II-4** product Peak #1 was collected and carried forward to produce chiral **5-5.**

Intermediate **5-5:** Into the reaction vessel was added chiral **II-4** (3.71 g, 10.5 mmol), THF (80 mL), and TBAF (31.5 mL, 31.5 mmol). The reaction mixture was stirred at 23 °C for 12 h, diluted with EtOAc (15 mL), and the organic portion washed with sat. NaHCO₃ (15 mL). The organic phase was collected, dried over Na₂SO₄, concentrated under reduced pressure and dissolved in DCM (50 mL). After cooling to 0 °C, DIEA (5.50 mL, 31.5 mmol), and 4,5-difluoro-2-methoxybenzoyl chloride (2.4 g, 12 mmol) were added. The reaction mixture was stirred at 0 °C for 1 h, then allowed to warm to 23 °C, concentrated under reduced pressure, and the residue purified via silica gel chromatography to produce 5-5 (2.9 g, 7.7 mmol, 74% yield). LC-MS RT = 1.13 min; (M+H) = 380.1. Method A.

**Example 56:** Into the reaction vessel was added 4-fluoro-3-(trifluoromethyl)aniline (4.87 g, 27.2 mmol), toluene (40 mL) and trimethylaluminum (13.59 mL, 27.20 mmol). After stirring at 23 °C for 30 min, **5-6** (2.95 g, 7.76 mmol) in toluene (80 mL) was added. The reaction mixture was stirred at 65 °C for 30 min. After allowing to cool to 23 °C, the reaction mixture was diluted with EtOAc (50 mL) and washed with an aqueous solution sat. with Rochelle salt. The organic layer was dried over Na₂SO₄, filtered, concentrated under reduced pressure and purified via silica gel chromatography to Example **56** (3.23 g, 6.14 mmol, 79.0% yield). LCMS RT = 1.23 min; (M+H) = 527.1; Method A.

Intermediate **5-6:** Into the reaction vessel was added **Example 56** (110 mg, 0.210 mmol) and EtOAc (5 mL). The reaction mixture was cooled to -78 °C and O₃ was bubbled through the solution for 10 min, (until blue color appeared). After bubbling N₂ to remove excess O₃, dimethyl disulfide (0.370 mL, 4.18 mmol) was subsequently added and the reaction mixture was allowed to warm to 23 °C and stirred for 12 h. The reaction mixure was concentrated under reduced pressure to produce a residue, **5-6** (100 mg, 0.20 mmol, 96% yield) that was used without further purification. LC-MS RT = 1.08 min; (M+H) = 501.1; Method A.

Procedure for **example 5:** Into the reaction vessel was added diethyl benzylphosphonate (0.290 mL, 1.40 mmol), THF (10 mL). The mixture was cooled to -78 °C and KHMDS (1.4 mL, 1.4 mmol) was added. This mixture was stirred at -78 °C for 15 min and **5-6** was added at -78 °C. After stirring at -78 °C for 10 min, the mixture was allowed to warm to 23 °C, stirred at 23 °C for 1 h, quenched with sat. NaHCO₃ and extracted with EtOAc. The organic phase was collected, dried over Na₂SO₄, filtered, concentrated, and purified via silica gel chromatography to produce the *E*-isomer **example 5** (59 mg, 0.10 mmol, 51% yield) and the Z-isomer **example 6** (34 mg, 0.060 mmol, 29% yield). ¹H NMR (400MHz, CDCl₃) δ 9.53 (d, J=7.7 Hz, 1H), 8.03 (dd, J=11.2, 9.5 Hz, 1H), 7.93 (dd, J=6.1, 2.5 Hz, 1H), 7.83 (s, 1H), 7.55 - 7.47 (m, 1H), 7.37 - 7.21 (m, 5H), 7.10 (t, J=9.4 Hz, 1H), 6.78 (dd, J=11.6, 6.1 Hz, 1H), 6.31 (s, 1H), 4.83 - 4.72 (m, 1H), 4.00 (s, 3H), 3.46 - 3.39 (m, 1H), 3.12 (dd, J=10.7, 4.1 Hz, 1H), 2.89 (t, J=4.0 Hz, 1H), 2.31 - 2.20 (m, 1H), 1.94 - 1.84 (m, 1H), 1.79 - 1.65 (m, 2H). LC-MS RT: 1.25 min; MS (ESI) m/z = 575.2 (M+H)+; Method A.

### Example 6

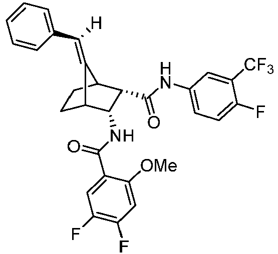

Procedure for **example 6: Example 6** was prepared as a byproduct of **example 5.** ¹H NMR (400MHz, CDCl₃) δ 9.51 (d, J=7.7 Hz, 1H), 8.03 (dd, J=11.4, 9.5 Hz, 1H), 7.94 (dd, J=6.2, 2.6 Hz, 1H), 7.72 (s, 1H), 7.53 (dt, J=8.5, 3.7 Hz, 1H), 7.37 - 7.30 (m, 4H), 7.25 - 7.19 (m, 1H), 7.12 (t, J=9.4 Hz, 1H), 6.78 (dd, J=11.7, 6.2 Hz, 1H), 6.33 (s, 1H), 4.83 (ddd, J=10.9, 7.6, 3.9 Hz, 1H), 3.99 (s, 3H), 3.49 (br. s., 1H), 3.16 (dd, J=10.8, 3.7 Hz, 1H), 2.87 (br. s., 1H), 2.22 (t, J=8.8 Hz, 1H), 1.91 (t, J=8.7 Hz, 1H), 1.69 (d, J=6.4 Hz, 2H). LC-MS RT: 1.25 min; MS (ESI) m/z = 575.2 (M+H)+; Method A.

### Example 11

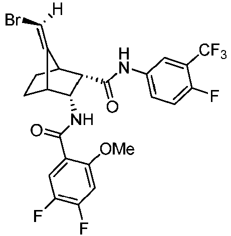

Procedure for **example 11: Example 11** was prepared from **5-6,** employing bromo(bromomethyl)triphenylphosphorane, according to the method described for **example 5.** ¹H NMR (500MHz, CDCl₃) δ 9.31 (d, J=8.0 Hz, 1H), 8.01 (dd, J=11.3, 9.4 Hz, 1H), 7.94 - 7.86 (m, 2H), 7.51 (dt, J=8.7, 3.6 Hz, 1H), 7.10 (t, J=9.4 Hz, 1H), 6.78 (dd, J=11.6, 6.1 Hz, 1H), 5.98 (s, 1H), 4.87 - 4.76 (m, 1H), 3.97 (s, 3H), 3.28 (t, J=3.7 Hz, 1H), 3.16 - 3.09 (m, 1H), 2.93 (t, J=3.7 Hz, 1H), 2.35 - 2.25 (m, 1H), 1.91 - 1.82 (m, 1H), 1.75 - 1.63 (m, 2H). LC-MS RT: 1.22 min; MS (ESI) m/z = 578.9 (M+H)+; Method A.

### Example 12

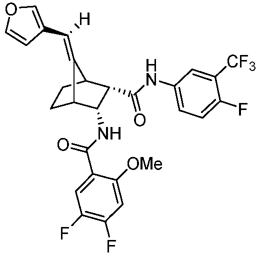

Procedure for **example 12:** Into the reaction vessel **example 11** (10 mg, 0.020 mmol) was added followed by furan-3-ylboronic acid (9.7 mg, 0.090 mmol), PdCl₂(dppf)-CH₂Cl₂ adduct (4.2 mg, 5.2 µmol), (THF 2 mL), and Na₂CO₃ (0.5 mL, 1.00 mmol). The reaction mixture was degassed by bubbling N₂ for 10 min, sealed, and stirred at 60 °C for 2 h. After the reaction mixture was allowed to cool to 23 °C, the reaction mixture was concentrated and the residue was purified via preparative RP-HPLC to produce **example 12** (7.7 mg, 0.010 mmol, 77% yield). ¹H NMR (500MHz, CDCl₃) δ 9.54 (d, J=7.4 Hz, 1H), 8.03 (dd, J=11.3, 9.4 Hz, 1H), 7.94 (dd, J=6.3, 2.8 Hz, 1H), 7.69 (s, 1H), 7.53 (dt, J=8.9, 3.4 Hz, 1H), 7.44 (d, J=0.8 Hz, 1H), 7.39 (t, J=1.7 Hz, 1H), 7.12 (t, J=9.4 Hz, 1H), 6.79 (dd, J=11.6, 6.3 Hz, 1H), 6.54 (d, J=1.1 Hz, 1H), 6.08 (s, 1H), 4.82 - 4.73 (m, 1H), 4.00 (s, 3H), 3.37 (t, J=3.6 Hz, 1H), 3.12 (dd, J=10.7, 3.9 Hz, 1H), 2.84 (t, J=3.3 Hz, 1H), 2.20 - 2.14 (m, 1H), 1.90 (t, J=8.5 Hz, 1H), 1.69-1.65 (m, 2H). LC-MS RT: 1.22 min; MS (ESI) m/z = 565.0 (M+H)+; Method C.

### Example 13

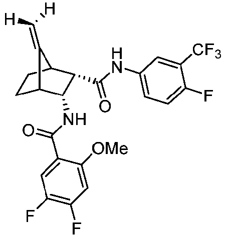

Procedure for **example 13: Example** 13 was prepared from **5-6** (5.0mg, 8,7 µmol), employing [1,1'-biphenyl]-4-ylboronic acid, according to the method described for **example 12.** In the case of the formation of **example 13,** the cross-coupled product was not obtained, but the dehalogenated byproduct was observed and isolated (2.3 mg, 4.6 µmol, 53%). ¹H NMR (500MHz, CDCl₃) δ 9.44 (d, J=6.6 Hz, 1H), 8.05 (dd, J=11.3, 9.5 Hz, 1H), 7.99 - 7.93 (m, 1H), 7.76 (bs, 1H), 7.58 - 7.50 (m, 1H), 7.13 (t, J=9.3 Hz, 1H), 6.80 (dd, J=11.6, 6.1 Hz, 1H), 4.86 (s, 1H), 4.85 (s, 1H), 4.79 - 4.73 (m, 1H), 3.11 (dd, J=10.8, 4.0 Hz, 1H), 2.83 (br. s., 1H), 2.79 (br. s., 1H), 2.24 - 2.15 (m, 1H), 1.88 - 1.80 (m, 1H), 1.69 - 1.63 (m, 2H). LC-MS RT: 1.17 min; MS (ESI) m/z = 499.1 (M+H)+; Method C.

### Example 33

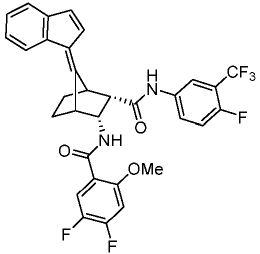

Procedure for **example 33:** Into the reaction vessel was added 1H-indene (34.8 mg, 0.300 mmol) and THF (2 mL). The reaction mixture was cooled to -78 °C and nBuLi (0.19 mL, 0.30 mmol) was added. After stirring at -78 °C for 10 min and at 23 °C for 10 min, the reaction mixture was again cooled to -78 °C and **5-6** (15 mg, 0.030 mmol) was added. The reaction mixture was allowed to warm to 23 °C, stirred for 15 min, and quenched by the addition of sat. NaHCO₃ and extracted with EtOAc. The organic phase was dried over Na₂SO₄, filtered, concentrated, and dissolved in Et₂O (2 mL). After the addition of Burgess reagent (14.3 mg, 0.0600 mmol) (1 equiv. was added and then added another 1 equiv. after 3 h), the reaction mixture was stirred at 45 °C for 12 h. The resulting solution was concentrated, and purified via silica gel chromatography to produce the E-isomeric product (3.4 mg, 5.6 µmol, 19% yield) and Z-isomer **example 33** (4.6 mg, 7.5 µmol, 25% yield). ¹H NMR (500MHz, CDCl₃) δ 9.63 (d, J=7.7 Hz, 1H), 8.07 (dd, J=11.3, 9.4 Hz, 1H), 8.00 (dd, J=6.2, 2.6 Hz, 1H), 7.87 (d, J=7.4 Hz, 1H), 7.80 (s, 1H), 7.57 (dt, J=8.7, 3.5 Hz, 1H), 7.37 (d, J=7.4 Hz, 1H), 7.32 - 7.26 (m, 1H), 7.25 - 7.21 (m, 1H), 7.17 (t, J=9.4 Hz, 1H), 6.90 (d, J=5.5 Hz, 1H), 6.83 (dd, J=11.6, 6.1 Hz, 1H), 6.67 (d, J=5.5 Hz, 1H), 4.94 - 4.86 (m, 1H), 4.04 (s, 3H), 3.98 (t, J=4.0 Hz, 1H), 3.43 (t, J=3.9 Hz, 1H), 3.19 (dd, J=10.9, 4.0 Hz, 1H), 2.40 - 2.33 (m, 1H), 2.14 - 2.07 (m, 1H), 1.84 - 1.70 (m, 2H). LC-MS RT: 1.27 min; MS (ESI) m/z = 599.1 (M+H)+; Method A.

### Example 34

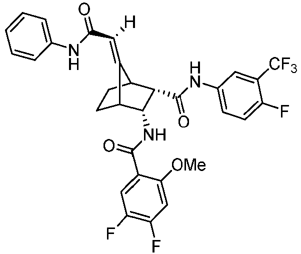

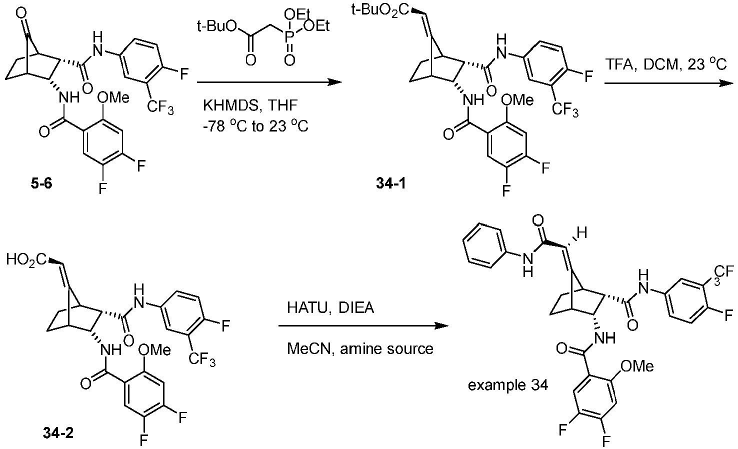

Intermediate **34-1:** Intermediate **34-1** was prepared from **5-6** and tert-butyl 2-(diethoxyphosphoryl)acetate in the same manner as the general Wittig reaction in Example 5. LC-MS RT = 1.25 min; (M+H) = 599.1; Method A.

Intermediate **34-2:** Into the reaction vessel was added **34-1** (50 mg, 0.080 mmol), DCM (2 mL), and TFA (0.200 mL, 2.59 mmol). After stirring at 23 °C for 12 h, concentration of the reaction contents, under reduced pressure, provided **34-2** (46 mg, 0.080 mmol, 98% yield), that was used without further purification. LC-MS RT = 1.08 min, (M+H) = 543.1; Method A.

Procedure for **example 34:** Into the reaction vessel was added **34-2** (5 mg, 9 µmol), MeCN (1 mL), DIEA (5 µl, 0.03 mmol), and HATU (7 mg, 0.02 mmol). The reaction mixture was stirred at 23 °C for 3 h, the solution concentrated under reduced pressure, and the residue was purified via preparative HPLC to produce **example 34** (3.8 mg, 6.8 µmol, 74% yield). LC-MS RT: 1.18 min; MS (ESI) m/z = 618.1 (M+H)+; Method B.

### Example 51

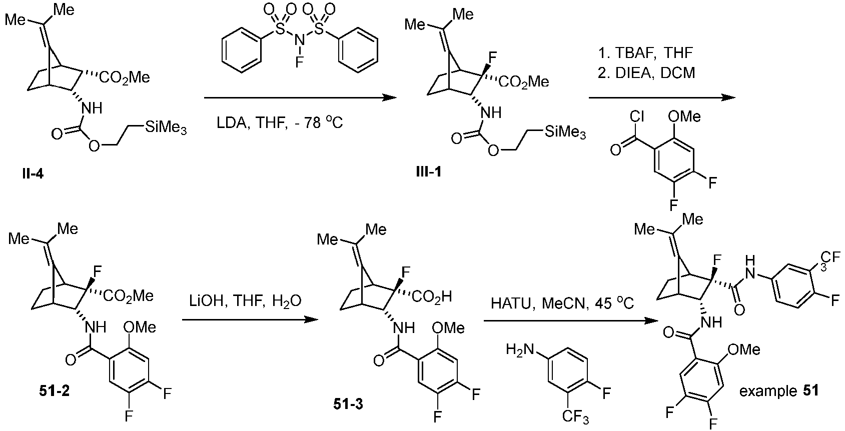

Intermediate **III-1:** Into the reaction vessel was added **II-4** (100 mg, 0.28 mmol) and THF (5 mL). After cooling to -78 °C, LDA (prepared from BuLi (0.53 mL, 0.85 mmol) and diisopropylamine (0.12 mL, 0.85 mmol) at 0 °C) was added and the reaction mixture was stirred at -78 °C for 15 min. Subsequently, N-fluoro-N-(phenylsulfonyl)benzenesulfonamide (223 mg, 0.710 mmol) was added at -78 °C. After stirring at -78 °C for 1 h, the reaction mixture was quenched by the addition of sat. NaHCO₃ and the aqueous portion extracted with EtOAc. The combined organic portion was dried over Na₂SO₄, concentrated under reduced pressure, and purified via silica gel chromatography to produce **III-1** (59.5 mg, 0.160 mmol, 57.0% yield) (1st peak) along with the *trans*-isomer (17.5 mg, 0.0500 mmol, 17.0% yield) (2nd peak). LC-MS RT = 1.21 min, (M+H) = 372.1; Method A.

Intermediate **51-2:** Into the reaction vessel was added **III-1** (20 mg, 0.050 mmol), THF (1 mL), and TBAF (0.270 mL, 0.270 mmol). The reaction mixture was stirred at 23 °C for 3 h, diluted with EtOAc, and the organic portion washed with sat. NaHCO₃. The organic phase was collected, dried over Na₂SO₄, concentrated under reduced pressure and redissolved in DCM (1 mL). DIEA (0.02 mL, 0.11 mmol) and 4,5-difluoro-2-methoxybenzoyl chloride (16.7 mg, 0.0800 mmol) were subsequently added. After stirring at 23 °C for 1 h, the reaction mixture was concentrated under reduced pressure and purified via silica gel chromatography to produce **51-2** (7.2 mg, 0.020 mmol, 34% yield). LC-MS RT = 1.11 min, (M+H) = 398.1; Method A.

Intermediate **51-3:** Into the reaction vessel was added **51-2** (7.5 mg, 0.020 mmol), THF (1 mL), water (0.5 mL), and lithium hydroxide monohydrate (4.0 mg, 0.090 mmol). The reaction mixture was stirred at 23 °C for 1 h, diluted with EtOAc (10 mL), and the organic portion washed with 10 mL sat. NH₄Cl containing 0.1 mmol HCl. The organic phase was dried over Na₂SO₄, filtered and concentrated under reduced pressure to provide **51-3** (7.5 mg, 0.020 mmol, 100% yield) that was used without further purification. LC-MS RT = 0.98 min, (M+H) = 384.1; Method A.

Procedure for **example 51:** Into the reaction vessel was added **51-3** (7.0 mg, 0.020 mmol), 4-fluoro-3-(trifluoromethyl)aniline (6.5 mg, 0.040 mmol), MeCN (1 mL), DIEA (6 µl, 0.04 mmol), and HATU (14 mg, 0.040 mmol). The reaction mixture was stirred at 50 °C for 1 h, allowed to cool to 23 °C, concentrated under reduced pressure, and purified via silica gel chromatography to produce **example 51** (4.7 mg, 8.3 µmol, 45% yield). ¹H NMR (500MHz, CDCl₃) δ 9.21 (d, J=8.3 Hz, 1H), 8.33 (d, J=8.8 Hz, 1H), 8.09 (dd, J=6.2, 2.6 Hz, 1H), 8.02 (dd, J=11.3, 9.4 Hz, 1H), 7.54 (dt, J=8.8, 3.4 Hz, 1H), 7.18 (t, J=9.2 Hz, 1H), 6.78 (dd, J=11.7, 6.2 Hz, 1H), 4.70 - 4.54 (m, 1H), 3.13 (t, J=3.9 Hz, 1H), 2.98 (dd, J=9.4, 3.9 Hz, 1H), 2.07 - 2.00 (m, 1H), 1.79 (s, 3H), 1.74 (s, 3H), 1.57 - 1.43 (m, 3H). LC-MS RT: 1.23 min; MS (ESI) m/z = 545.1 (M+H)+; Method A.

### Example 52

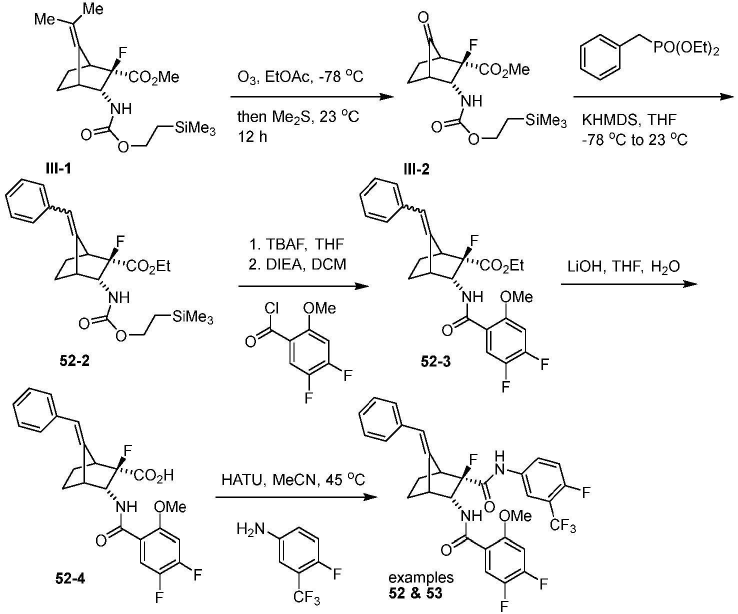

Intermediate **III-2**: Into the reaction vessel was added **III-1** (50 mg, 0.14 mmol) and EtOAc (3 mL). The reaction mixture was cooled to -78 °C and O₃ was bubbled through the solution for 10 min. Dimethyl disulfide (0.24 mL, 2.7 mmol) was subsequently added and the reaction mixture was allowed to warm to 23 °C and stirred for 12 h. After concentration under reduced pressure, the residue was dissolved in EtOAc and filtered through silica gel. Concentration of the filtrate under reduced pressure gave **III-2** (50.5 mg, 0.15) mmol, 100% yield) that was used without further purification. LCMS RT = 1.24 min, (M+H) = 346.0; Method A.

Intermediate **52-2:** Into the reaction vessel was added diethyl benzylphosphonate (0.14 mL, 0.65 mmol), THF (5 mL). The reaction mixture was cooled to -78 °C and KHMDS (0.65 mL, 0.65 mmol) was added. This mixture was stirred at -78 °C for 20 min and **III-2** (45 mg, 0.13 mmol) was added at -78 °C. After stirring at -78 °C for 5 min and at 23 °C for 1 h, the reaction mixture was quenched by the addition of sat. NaHCO₃ and the aqueous portion extracted with EtOAc. The organic portions were combined, dried over Na₂SO₄, concentrated under reduced pressure, and purified via silica gel chromatography to give **52-2** (17.4 mg, 0.0400 mmol, 31.0% yield, 1.45:1 mixture of olefin isomers). LC-MS RT = 1.27 min, (M+H-Et) = 406.0; Method A.

Intermediate **52-3:** Intermediate **52-3** was prepared utilizing the procedure described for the synthesis of intermediate **51-2.**

Intermediate **52-4:** Intermediate **52-4** was prepared utilizing the procedure described for the synthesis of intermediate **51-3.**

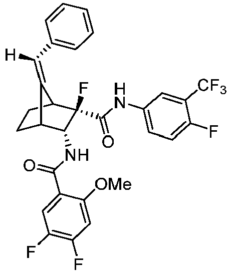

Procedure for **example 52: Example 52** was prepared from **52-4,** according to the method described for **example 51.** ¹H NMR (500MHz, CDCl₃) δ 9.25 (d, J=8.0 Hz, 1H), 8.33 (d, J=8.5 Hz, 1H), 8.10 (dd, J=6.2, 2.6 Hz, 1H), 8.04 (dd, J=11.1, 9.5 Hz, 1H), 7.58 - 7.50 (m, 1H), 7.39 - 7.31 (m, 4H), 7.18 (t, J=9.4 Hz, 1H), 6.79 (dd, J=11.6, 6.1 Hz, 1H), 6.56 (s, 1H), 4.92 - 4.73 (m, 1H), 4.00 (s, 3H), 3.41 (dd, J=8.7, 3.4 Hz, 1H), 3.06 (t, J=3.9 Hz, 1H), 2.24 - 2.13 (m, 1H), 1.79 - 1.72 (m, 1H), 1.71 - 1.57 (m, 3H). LC-MS RT: 1.27 min; MS (ESI) m/z = 593.0 (M+H)+; Method A.

### Example 53

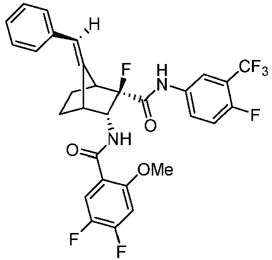

Procedure for **example 53: Example 53** was prepared from **52-4,** according to the method described for **example 51.** ¹H NMR (500MHz, CDCl₃) δ 9.28 (d, J=7.4 Hz, 1H), 8.33 (d, J=8.3 Hz, 1H), 8.11 (d, J=4.1 Hz, 1H), 8.02 (t, J=10.3 Hz, 1H), 7.55 (d, J=7.7 Hz, 1H), 7.40 - 7.31 (m, 4H), 7.19 (t, J=9.2 Hz, 1H), 6.79 (dd, J=11.6, 6.1 Hz, 1H), 6.45 (s, 1H), 4.89 - 4.72 (m, 1H), 4.01 (s, 3H), 3.61 (br. s., 1H), 2.85 (d, J=6.1 Hz, 1H), 2.25 - 2.13 (m, 1H), 1.85 - 1.76 (m, 1H), 1.75 - 1.58 (m, 3H). LC-MS RT: 1.27 min; MS (ESI) m/z = 593.0 (M+H)+; Method A.

### Example 65

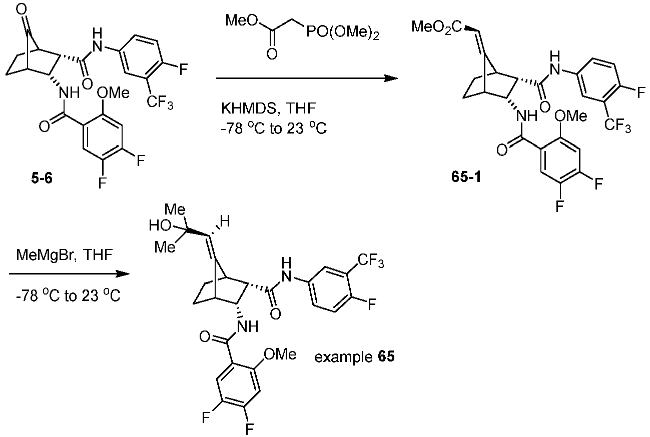

Intermediate **65-1:** Intermediate **65-1** was prepared from **5-6** and methyl 2-(dimethoxyphosphoryl)acetate in a similar manner to the Wittig reaction described in Example 5.

Procedure for **example 65:** Into the reaction vessel was added **65-1** (5.0 mg, 9.0 µmol) and THF (1 mL). After the reaction mixture was cooled to -78 °C, methylmagnesium chloride (0.06 mL, 0.2 mmol) was added. The reaction mixture was allowed to warm to 23 °C, stirred at 23 °C for 2 h. The reaction was quenched by the addition of sat. NaHCO₃ and the solution extracted with EtOAc. The organic layer was dried over Na₂SO₄, filtered, concentrated under reduced pressure, and purified via preparative RP-HPLC purification to produce **example 65** (3.2 mg, 5.3 µmol, 59% yield). ¹H NMR (500MHz, CDCl₃) δ 9.46 (d, J=7.7 Hz, 1H), 8.03 - 7.91 (m, 3H), 7.53 (dt, J=8.6, 3.5 Hz, 1H), 7.10 (t, J=9.4 Hz, 1H), 6.78 (dd, J=11.6, 6.3 Hz, 1H), 5.49 (s, 1H), 4.77 - 4.67 (m, 1H), 3.98 (s, 3H), 3.49 (t, J=4.0 Hz, 1H), 3.07 (dd, J=11.0, 4.1 Hz, 1H), 2.69 (t, J=3.9 Hz, 1H), 2.17 - 2.09 (m, 1H), 1.89 - 1.81 (m, 1H), 1.68 - 1.55 (m, 2H), 1.42 (s, 6H). LC-MS RT: 1.14 min; MS (ESI) m/z = 557.0 (M+H)+; Method C.

### Example 76

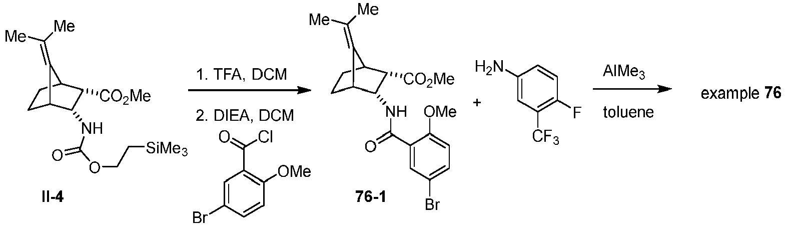

Intermediate **76-1:** To a 20 mL vial containing **II-4** (1.77 g, 5.00 mmol) was added DCM (20 mL). TFA (2.02 mL, 26.3 mmol) was then added and the reaction mixture was stirred at 23 °C for 48 h. The resulting solution was concentrated under reduced pressure and dried under high vacuum for 5 hours. The residue was carried forward to the acylation step without further purification. 5-bromo-2-methoxybenzoyl chloride was prepared in the following manner: To a 100 mL flask charged with 5-bromo-2-methoxybenzoic acid (1.39 g, 6.00 mmol) was added DCM (30 mL) followed by oxalyl chloride (0.6 mL, 7 mmol) and DMF (0.05, mL 0.6 mmol). The solution was stirred for 18h at 23 °C and was converted to the amide in the same manner described for intermediate **5-5** to produce **76-1** (878 mg, 2.10 mmol, 56.0% yield). ¹H NMR (500MHz, DMSO-d₆) δ 9.49 (d, *J*=7.0 Hz, 1H), 8.04 - 7.87 (m, 1H), 7.74 - 7.59 (m, 1H), 7.17 (d, *J*=8.8 Hz, 1H), 4.26 (br. s., 1H), 3.98 (s, 3H), 3.63 (s, 1H), 3.51 (br. s., 1H), 3.42 (d, *J*=18.1 Hz, 3H), 3.13 - 3.01 (m, 1H), 2.92 (d, *J*=13.5 Hz, 2H), 1.67 (s, 5H), 1.64 - 1.47 (m, 3H), 1.36 (br. s., 2H).

Procedure for **example 76: Example 76** was prepared from **76-1,** employing 4-fluoro-3-(trifluoromethyl)aniline, according to the method described for **example 56.** ¹H NMR (500MHz, DMSO-d6) δ 10.52 (s, 1H), 9.88 (d, J=7.0 Hz, 1H), 8.19 (d, J=4.3 Hz, 1H), 7.97 (d, J=2.7 Hz, 1H), 7.78 (d, J=8.5 Hz, 1H), 7.65 (dd, J=8.7, 2.6 Hz, 1H), 7.47 (t, J=9.8 Hz, 1H), 7.16 (d, J=8.9 Hz, 1H), 4.31 (br. s., 1H), 3.98 (s, 3H), 3.55 - 3.40 (m, 3H), 3.09 (dd, J=10.7, 4.0 Hz, 1H), 3.02 (br. s., 1H), 2.91 (br. s., 1H), 1.80 (t, J=8.9 Hz, 1H), 1.75 - 1.62 (m, 7H), 1.33 (d, J=6.1 Hz, 2H).

LC-MS RT: 2.69 min; MS (ESI) m/z = 569.1 (M-H)+; Method B.

### Example 77

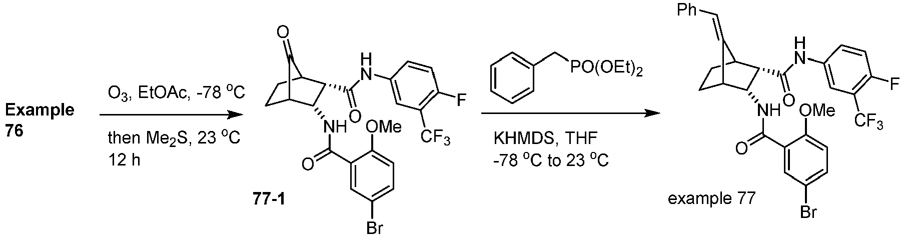

**Intermediate 77-1:** Intermediate **77-1** was prepared from example **76** in the same general manner described for intermediate **5-6.** LC-MS RT = 1.0 min; (M+H) = 544.0; Method A.

Procedure for **example 77: Example 77** was prepared from **77-1,** employing diethyl benzylphosphonate, according to the general method described for **example 5.** ¹H NMR (500 MHz, DMSO-d6) δ 10.66 - 10.50 (m, 1H), 10.06 - 9.88 (m, 1H), 8.27 - 8.13 (m, 1H), 8.06 - 7.94 (m, 1H), 7.86 - 7.73 (m, 1H), 7.71 - 7.59 (m, 1H), 7.53 - 7.43 (m, 1H), 7.43 - 7.31 (m, 4H), 7.31 - 7.21 (m, 1H), 7.21 - 7.09 (m, 1H), 6.47 - 6.22 (m, 1H), 4.55 - 4.37 (m, 1H), 4.09 - 3.95 (m, 3H), 3.33 - 3.19 (m, 1H), 2.90 - 2.76 (m, 1H), 2.02 - 1.88 (m, 1H), 1.87 - 1.71 (m, 1H), 1.64 - 1.42 (m, 2H), 1.06 - 0.91 (m, 1H). LC-MS RT: 2.83 min; MS (ESI) m/z = 617.20 (M-H)+; Method B.

### Example 78

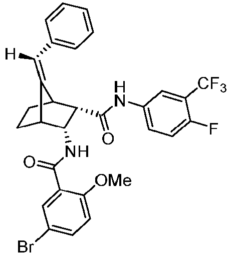

Procedure for **example 78: Example 78** was prepared as a byproduct in the production of Example 77. ¹H NMR (500 MHz, DMSO-d6) δ 10.66 - 10.50 (m, 1H), 10.06 - 9.88 (m, 1H), 8.27 - 8.13 (m, 1H), 8.06 - 7.94 (m, 1H), 7.86 - 7.73 (m, 1H), 7.71 - 7.59 (m, 1H), 7.53 - 7.43 (m, 1H), 7.43 - 7.31 (m, 4H), 7.31 - 7.21 (m, 1H), 7.21 - 7.09 (m, 1H), 6.47 - 6.22 (m, 1H), 4.55 - 4.37 (m, 1H), 4.09 - 3.95 (m, 3H), 3.33 - 3.19 (m, 1H), 2.90 - 2.76 (m, 1H), 2.02 - 1.88 (m, 1H), 1.87 - 1.71 (m, 1H), 1.64 - 1.42 (m, 2H), 1.06 - 0.91 (m, 1H). LC-MS RT: 2.82 min; MS (ESI) m/z = 617.35 (M-H)+; Method B.

### Example 79

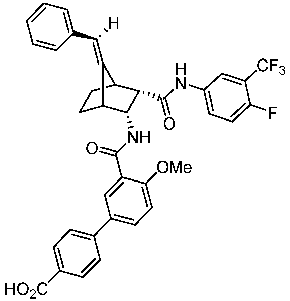

Procedure for **example 79:** To a 0.5 to 2.0 mL microwave reaction via charged with **example 77** (15 mg, 0.024 mmol) was added 4-boronobenzoic acid (6 mg, 0.04 mmol) followed by THF (490 µl) and a solution of K₃PO₄ (97 µl, 0.049 mmol) in water. Finally, XPhos-Pd-G2 (CAS 1310584-14-5) (2 mg, 0.002 mmol, small spatula tip) was added. The vial was capped and heated in the microwave to 100 °C for 30 min. The reaction was diluted with DMF to a total volume of 2 mL, filtered, and purified by preparative RP-HPLC to give **example 79** (5.2 mg, 0.01 mmol, 33% yield). ¹H NMR (500MHz, DMSO-d6) δ 9.95 (d, J=7.0 Hz, 1H), 8.26 (d, J=4.3 Hz, 1H), 7.94 (d, J=6.7 Hz, 1H), 7.81 (br. s., 1H), 7.50 (t, J=8.2 Hz, 3H), 7.44 - 7.32 (m, 6H), 7.25 (br. s., 2H), 7.18 (d, J=8.2 Hz, 1H), 7.04 (t, J=7.5 Hz, 1H), 6.39 (s, 1H), 4.52 (br. s., 1H), 3.28 (br. s., 1H), 2.93 (br. s., 1H), 2.02 - 1.79 (m, 3H), 1.53 (br. s., 3H). LC-MS RT: 2.2 min; MS (ESI) m/z = 659.4 (M-H)+; Method B.

### Example 107

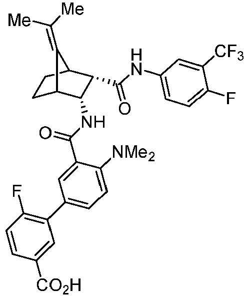

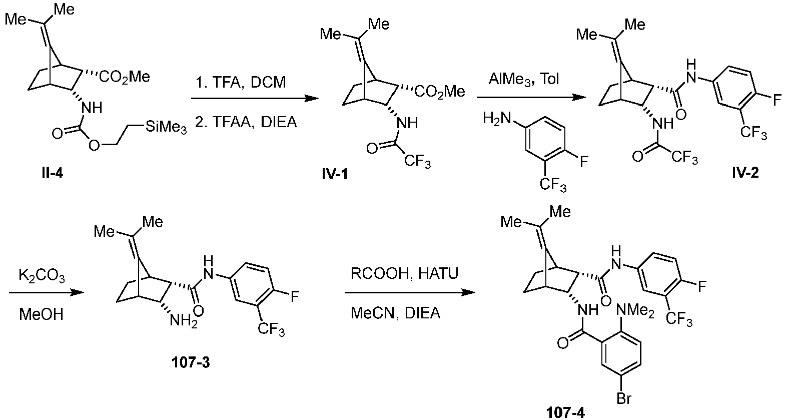

Intermediate **IV-1:** Deprotection of **II-4** to intermediate **IV-1** utilized the same conditions described in **76-1.** Installation of the trifluoroacetyl protecting group was conducted as follows: **II-4** was deprotected to the corresponding amine intermediate and the amine (1.7 g, 8.1 mmol) was added DCM (41 mL) and the flask was cooled to 0 °C via an ice bath. TFAA (1.26 mL, 8.90 mmol) and DIEA (5.7 mL, 33 mmol) were added. The reaction flask was removed from the ice bath after 5 min, and was stirred at 23 °C for 30 min. The reaction mixture was quenched with sat. NaHCO₃ (50 mL) and extracted with EtOAc (3 x 50 mL). The combined organic portions were dried over Na₂SO₄, filtered and concentrated under reduced pressure to afford **IV-1** (2.48 g, 8.12 mmol, 100% yield) that was used without further purification. LC-MS RT = 1.11 min; MS (ESI) m/z = 306.1 (M+H)⁺; Method A.

Intermediate **IV-2:** Intermediate **IV-2** was prepared from **IV-1** in the same manner as described for **5-6.** (2.5 g, 5.5 mmol, 63% yield); LC-MS RT = 1.20 min; MS (ESI) m/z = 453.0 (M+H)⁺; Method A.

Intermediate **107-3:** Intermediate **IV-2** (133 mg, 0.290 mmol) was dissolved in water (2.9 mL) and MeOH (2.9 mL). K₂CO₃ (2.03 g, 1.47 mmol) was added and the reaction mixture was stirred at 40 °C for 4 h. The reaction mixture was allowed to cool to room temperature then water (5 mL) was added. The resulting solution was extracted with EtOAc (3 x 10 mL). The combined organic extracts were dried over Na₂SO₄, filtered and concentrated under reduced pressure to afford **107-3** (105 mg, 0.290 mmol, 100% yield) that was used without further purification. LC-MS RT = 0.82 min; MS (ESI) m/z = 357.1 (M+H)⁺; Method A.

Intermediate **107-4** was prepared from **107-3** using the sample procedure employed for **76-1.**

Procedure for **example 107: Example 107** was prepared from **107-4,** employing 3-borono-4-fluorobenzoic acid, according to the method described for **example 79.** ¹H NMR (500 MHz, DMSO-d6) δ 10.64 (s, 1H), 10.34 (br d, J=7.3 Hz, 1H), 8.23 (dd, J=6.1, 1.8 Hz, 1H), 7.99 (br d, J=7.6 Hz, 1H), 7.93 (s, 1H), 7.91 - 7.86 (m, 1H), 7.80 (br d, J=8.2 Hz, 1H), 7.61 (br d, J=8.2 Hz, 1H), 7.44 (br t, J=9.8 Hz, 1H), 7.33 (d, J=8.5 Hz, 1H), 7.24 (br t, J=9.6 Hz, 1H), 4.46 - 4.38 (m, 1H), 3.13 (br dd, J=10.4, 4.0 Hz, 1H), 3.03 (br s, 1H), 2.93 (br s, 1H), 2.71 (s, 6H), 1.94 - 1.87 (m, 2H), 1.84 - 1.75 (m, 1H), 1.71 (s, 6H), 1.45 - 1.30 (m, 2H). LC-MS RT: 2.2 min; MS (ESI) m/z = 642.2 (M+H)+; Method B.

### Example 108

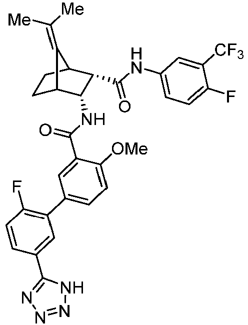

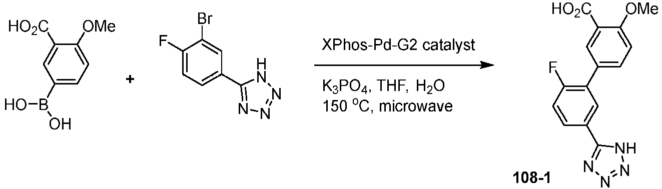

Intermediate **108-1:** To a vial was added 5-(3-bromo-4-fluorophenyl)-1H-tetrazole (50 mg, 0.21 mmol), 5-borono-2-methoxybenzoic acid (60.5 mg, 0.309 mmol), XPhos-Pd-G2 catalyst (32 mg, 0.042 mmol) and K₃PO₄ (131 mg, 0.617 mmol) followed by THF (1.8 mL) and water (257 µl). The reaction mixture was degassed with nitrogen for 2 min, then sealed and heated at 150 °C for 2.5 h with microwave irradiation. The reaction mixture was partitioned between 1 N HCl (5 mL) and extracted with EtOAc (3 x 5 mL). The combined organic portions were dried over Na₂SO₄, filtered and concentrated under reduced pressure. The resulting residue was purified by preparative RP-HPLC to give **108-1** (13 mg, 0.041 mmol, 20% yield). LC-MS RT = 0.77 min; MS (ESI) m/z = 315.1 (M+H)⁺; Method A.

Procedure for **example 108:** Into the reaction vessel was added **107-3** (10 mg, 0.03 mmol), **108-1** (13.2 mg, 0.0400 mmol), MeCN (1 mL), DIEA (0.02 mL, 0.1 mmol), and HATU (16 mg, 0.040 mmol). The reaction mixture was stirred at 23 °C for 3 h, concentrated under reduced pressure, and subjected to preparative RP-HPLC purification to afford **example 108** (12.3 mg, 0.0200 mmol, 65.0% yield). ¹H NMR (500 MHz, DMSO-d6) δ 10.54 (s, 1H), 9.93 (d, J=7.0 Hz, 1H), 8.26 - 8.19 (m, 2H), 8.17 (br d, J=6.4 Hz, 1H), 8.09 - 8.02 (m, 1H), 7.83 - 7.75 (m, 2H), 7.54 (dd, J=10.2, 9.0 Hz, 1H), 7.47 (t, J=9.8 Hz, 1H), 7.34 (d, J=8.5 Hz, 1H), 4.41 - 4.34 (m, 1H), 4.05 (s, 3H), 3.11 (dd, J=10.8, 4.1 Hz, 1H), 3.05 - 3.02 (m, 1H), 2.99 - 2.92 (m, 1H), 1.86 - 1.80 (m, 1H), 1.77 - 1.69 (m, 7H), 1.41 - 1.31 (m, 2H). LC-MS RT: 2.17 min; MS (ESI) m/z = 653.6 (M+H)+; Method B.

### Example 110

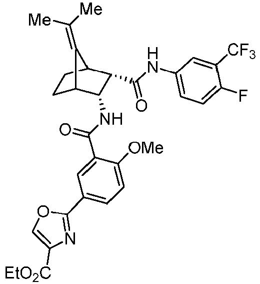

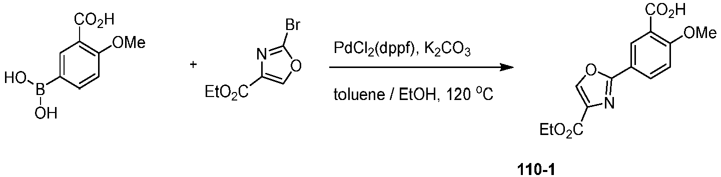

Intermediate **110-1:** To a vial was added 5-borono-2-methoxybenzoic acid (100 mg, 0.51 mmol), ethyl 2-bromooxazole-4-carboxylate (75 mg, 0.34 mmol), PdCl₂(dppf)-CH₂Cl₂ adduct (28 mg, 0.030 mmol), K₂CO₃ (470 mg, 3.40 mmol), toluene (1.7 mL), and ethanol (1.7 mL). The reaction mixture was heated at 120 °C for 3 h after which it became a gel. The reaction mixture was diluted with DMF, filtered, and purified by preparative RP-HPLC to afford **110-1** (24 mg, 0.080 mmol, 24% yield). RT = 0.73 min; MS (ESI) m/z = 292.1 (M+H)⁺; Method A.

Procedure for **example 110: Example 110** was prepared from **107-3,** employing **110-1,** according to the method described for **example 108.** ¹H NMR (500 MHz, DMSO-d6) δ 10.54 (s, 1H), 9.93 (d, J=7.0 Hz, 1H), 8.88 (s, 1H), 8.58 (d, J=2.4 Hz, 1H), 8.20 (dd, J=6.4, 2.1 Hz, 1H), 8.12 (dd, J=8.7, 2.3 Hz, 1H), 7.81 (br dd, J=8.4, 4.1 Hz, 1H), 7.48 (t, J=9.8 Hz, 1H), 7.37 (d, J=8.9 Hz, 1H), 4.41 - 4.35 (m, 1H), 4.31 (q, J=7.0 Hz, 2H), 4.08 (s, 3H), 3.12 (br dd, J=10.7, 4.0 Hz, 1H), 3.07 - 3.02 (m, 1H), 2.98 - 2.93 (m, 1H), 1.86 - 1.79 (m, 1H), 1.78 - 1.69 (m, 7H), 1.38 - 1.33 (m, 2H), 1.31 (t, J=7.0 Hz, 3H). LC-MS RT: 2.61 min; MS (ESI) m/z = 630.5 (M+H)+; Method B.

### Example 113

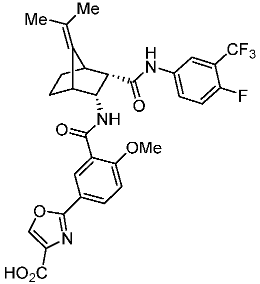

Procedure for **example 113:** To a vial containing **example 110** (11.5 mg, 0.02 mmol) in THF (180 µl) / water (90 µl) / MeOH (90 µl) was added a 1.5 M solution of lithium hydroxide (61 µl, 0.09 mmol), and the reaction was stirred at 23 °C for 5 min. The reaction mixture was quenched by the addition of 1N HCl (1 mL) and extracted with EtOAc (3 x 5 mL). The combined organics were dried over Na₂SO₄ and concentrated under reduced pressure. The resulting crude product was purified via preparative RP-HPLC to afford **example 113** (6.3 mg, 0.01 mmol, 56% yield). ¹H NMR (500 MHz, DMSO-d6) δ 10.57 (s, 1H), 9.93 (d, J=7.3 Hz, 1H), 8.57 (s, 1H), 8.54 (d, J=2.1 Hz, 1H), 8.18 (dd, J=6.1, 2.1 Hz, 1H), 8.10 (dd, J=8.7, 2.3 Hz, 1H), 7.79 (dd, J=8.1, 3.8 Hz, 1H), 7.46 (t, J=9.8 Hz, 1H), 7.35 (d, J=8.9 Hz, 1H), 4.41 - 4.30 (m, 1H), 4.06 (s, 3H), 3.10 (dd, J=10.7, 4.0 Hz, 1H), 3.05 - 2.99 (m, 1H), 2.98 - 2.91 (m, 1H), 1.81 (t, J=8.7 Hz, 1H), 1.76 - 1.65 (m, 7H), 1.41 - 1.28 (m, 2H). LC-MS RT: 1.92 min; MS (ESI) m/z = 601.9 (M+H)+; Method B.

Procedure for **example 114: Example 114** was prepared from **14-3,** employing 5-cyano-2-fluorobenzoic acid, according to the method described for **example 108.** ¹H NMR. LC-MS RT: 2.53 min; MS (ESI) m/z = 504.1 (M+H)+; Method C.

### Example 120

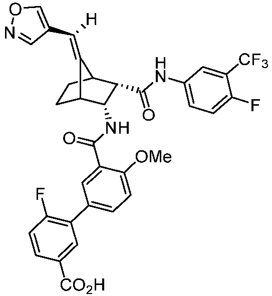

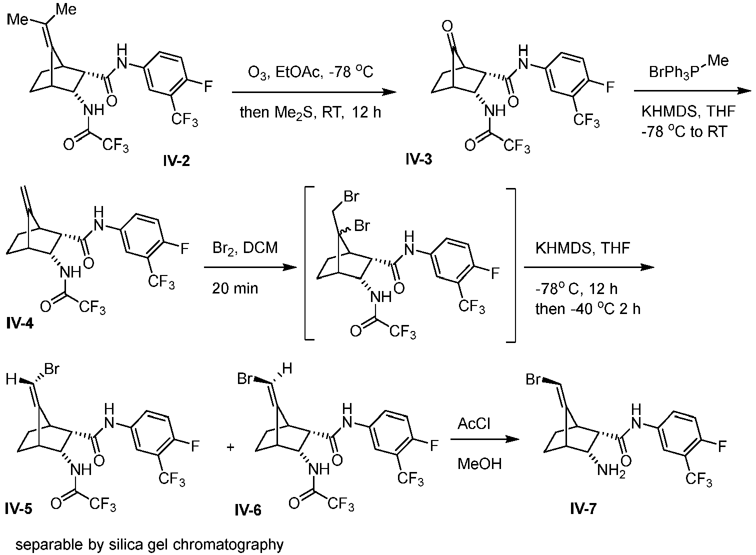

Intermediate **IV-3:** Intermediate **IV-3** was prepared from **IV-2** in the same manner as **5-6.** (101 mg, 0.240 mmol, 97.0% yield). ¹H NMR (500 MHz, CDCl₃) δ 9.61 (br d, J=6.3 Hz, 1H), 7.76 (dd, J=5.9, 2.6 Hz, 1H), 7.71 (dt, J=8.9, 3.4 Hz, 1H), 7.66 (s, 1H), 7.23 (t, J=9.4 Hz, 1H), 4.70 (dt, J=10.3, 5.3 Hz, 1H), 3.33 (dd, J=10.5, 4.4 Hz, 1H), 2.54 (t, J=4.3 Hz, 1H), 2.42 (t, J=4.1 Hz, 1H), 2.20 - 2.10 (m, 1H), 2.06 - 1.99 (m, 1H), 1.96 - 1.81 (m, 2H).

Intermediate **IV-4:** Into the reaction vessel was added bromo(methyl)triphenylphosphorane (419 mg, 1.17 mmol) (fine powder by grinding the commercial material) and THF (7 mL). The reaction mixture was cooled to -78 °C and KHMDS (1.2 mL, 1.17 mmol) was added. This reaction mixture was stirred vigorously at -78 °C for 30 min and **IV-3** (100 mg, 0.240 mmol) was added at -78 °C. After stirring at - 78 °C for a further 10 min, the reaction mixture was allowed to warm to 23 °C and stirred for 1.5 h. The reaction mixture was cooled to -40 °C and quenched by the addition of sat. NaHCO₃. The solution was extracted with EtOAc. The organic phase wasdried over Na₂SO₄, filtered, concentrated under reduced pressure, and purified via silica gel chromatography to produce **IV-4** (71 mg, 0.17 mmol, 71% yield). LCMS RT = 1.16 min; (M+H) = 425.0; Method A.

Intermediates **IV-5** and **IV-6:** Into the reaction vessel was added **IV-4** (71 mg, 0.17 mmol), DCM (3 mL), and Br₂ (0.03 mL, 0.6 mmol). The reaction mixture was stirred at 23 °C for 20 min and concentrated under reduced pressure with sat. Na₂S₂O₃ trap to quench excess Br₂. The resulting dibromide was dissolved in THF (3 mL). After cooling the flask to -78 °C, KHMDS (1.0 mL, 1.0 mmol) was added. The reaction mixture was kept at -78 °C for 12 h and -40 °C for 2 h, then quenched by the addition of sat. NaHCO₃ at -40 °C. The resulting solution was extracted with EtOAc. The organic phase was collected, dried over Na₂SO₄, filtered, concentrated under reduced pressure, and purified via silica gel chromatography to produce **IV-6** (27 mg, 0.050 mmol, 32% yield) (Z-isomer, peak2. LCMS RT = 1.19 min; (M+H) = 504.9; Method A. and the corresponding *E*-isomer **IV-5** (28 mg, 0.060 mmol, 33% yield) (peak 1). **IV-6** was produced as a racemate as outlined above and separated into individual enantiomers using chiral SFC. Preparative chromatographic conditions: Instrument: Thar 350 SFC; Column: Chiralcel OD-H, 5 x 50 cm, 5 micron; Mobile phase: 20% MeOH/80% CO₂; Flow conditions: 340 mL/min, 100 Bar, 35 °C; Detector wavelength: 220 nm; Injections details: 3.75 mL of 30 mg/mL in MeOH. Peak 1, RT = 7.81 min, >99% ee; Peak 2, RT = 10.97 min, >99% ee. Intermediate **IV-6** product peak #1 (1.9 grams) was collected and carried forward to produce chiral **IV-7.**

Intermediate **IV-7:** Into the reaction was added MeOH (3 mL) and AcCl (0.3 mL, 4.2 mmol). After stirring for 5 min, chiral **IV-6** (1^{st} eluting peak from chiral SFC, 75 mg, 0.15 mmol) was added and the reaction mixture was stirred at 40 °C for 48 h. The resulting solution was concentrated under reduced pressure to generate **IV-7** (67 mg, 0.16 mmol, 100%) that was used without further purification. LC-MS RT = 0.78 min; (M+H) = 408.9; Method A.

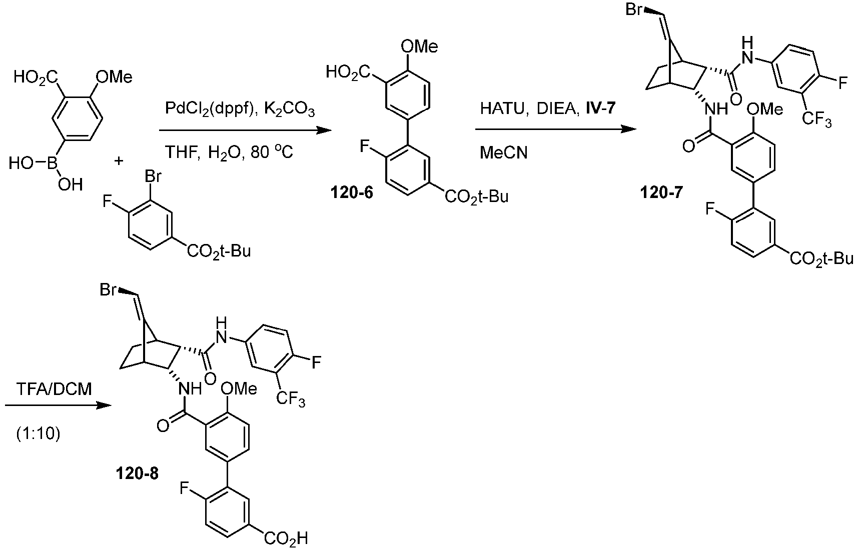

Intermediate **120-6:** To a vial was added 5-borono-2-methoxybenzoic acid (500 mg, 2.55 mmol), tert-butyl 3-bromo-4-fluorobenzoate (842 mg, 3.06 mmol), tert-butyl 3-bromo-4-fluorobenzoate (842 mg, 3.06 mmol), K₂CO₃ (1.76 g, 12.8 mmol), PdCl₂(dppf)-CH₂Cl₂ adduct (313 mg, 0.380 mmol), and THF (22.3 mL). The reaction mixture wasdegassed for 2 min with nitrogen, then heated at 80 °C for 18 h. After cooling to room temperature, the reaction mixture was diluted with 1N HCl (25 mL) and the solution extracted with EtOAc (3 x 25 mL). The combined organic portions were dried over Na₂SO₄, filtered, concentrated under reduced pressure, and the resulting residue was dissolved in DMF and purified by preparative RP-HPLC to afford **120-6** (586 mg, 1.69 mmol, 66.0% yield).

LC-MS RT = 1.02 min; (M+H) = 347.1; Method A. '

The Suzuki reaction may be performed with alternative aryl halides with the remainder of the steps conducted similarly to generate biaryl analogs.

Intermediate **120-7:** In the reaction vessel was added **IV-7** (7 mg, 0.02 mmol) and **120-6** (6.6 mg, 0.020 mmol), MeCN (1 mL), DIEA (9.64 uL, 0.0600 mmol) and HATU (12.0 mg, 0.0300 mmol). The reaction mixture was stirred at 23 °C for 3 h, concentrated under reduced pressure and purified via silica gel chromatography to produce **120-7** (10 mg, 0.014 mmol, 86% yield). LC-MS RT = 1.33 min; (M+H) = 735.2; Method A.

Intermediate **120-8:** Intermediate **120-8** was prepared from **120-7** in the same manner as intermediate **34-2** (5 mg, 0.07 mmol, 100% yield).. LC-MS RT = 1.15 min; (M+H) = 679.08; Method A.

Procedure for **example 120:** Into the reaction vessel containing **120-8** (10 mg, 0.01 mmol) was added 4-(4,4,5,5-tetramethyl-1,3,2-dioxaborolan-2-yl)isoxazole (13.3 mg, 0.07 mmol), PdCl₂(dppf)-CH₂Cl₂ adduct (3 mg, 0.004 mmol, small spatula tip), and Na₂CO₃ (0.5 mL, 1.0 mmol). The reaction mixture was degassed by bubbling N₂ for 10 min, sealed, and stirred at 60 °C for 2 h. After allowing to cool to 23 °C, the reaction mixture was concentrated under reduced pressure and purified via preparative RP-HPLC to produce the intermediate tert-butyl ester. Treatment of the ester with 10:1 DCM/TFA followed by purification by reverse phase HPLC produced **example 120** (7.0 mg, 0.01 mmol, 72% yield). ¹H NMR (500 MHz, CDCl₃) δ 10.03 (br d, J=6.3 Hz, 1H), 8.50 (br s, 1H), 8.43 (br s, 1H), 8.31 (br s, 1H), 8.21 (br d, J=5.2 Hz, 1H), 8.10 - 7.91 (m, 3H), 7.73 (br d, J=8.3 Hz, 1H), 7.53 (br d, J=3.9 Hz, 1H), 7.27 - 7.19 (m, 1H), 7.19 - 7.08 (m, 2H), 6.06 (s, 1H), 4.86 (br s, 1H), 4.11 (br s, 3H), 3.31 (br s, 1H), 3.22 (br d, J=7.2 Hz, 1H), 2.93 (br s, 1H), 2.37 - 2.25 (m, 1H), 2.03 (br d, J=11.8 Hz, 1H), 1.75 - 1.65 (m, 2H). LC-MS RT: 1.14 min; MS (ESI) m/z = 668.3 (M+H)+; Method A.

### Example 121

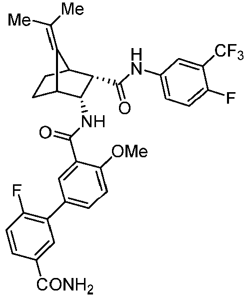

Procedure for **example 121:** Into the reaction vessel was added **example 87** (3 mg, 4.77 µmol), ethanesulfonamide (1.6 mg, 0.01 mmol), MeCN (1 mL), DIEA (3 µl, 0.017 mmol), and BOP-Cl (4 mg, 0.01 mmol). The reaction was stirred at 40 °C for 12 h, concentrated under reduced pressure, and purified via preparative RP-HPLC to produce only the primary amide by-**product 121** (3.0 mg, 0.0040 mmol, 93% yield). ¹H NMR (500 MHz, CDCl₃) δ 9.65 (br d, J=8.0 Hz, 1H), 8.41 (d, J=2.2 Hz, 1H), 7.96 (dd, J=7.2, 2.2 Hz, 2H), 7.90 - 7.83 (m, 2H), 7.73 (dt, J=8.5, 2.2 Hz, 1H), 7.56 (dt, J=8.7, 3.5 Hz, 1H), 7.25 (dd, J=9.9, 8.8 Hz, 1H), 7.16 - 7.06 (m, 2H), 6.69 - 6.68 (m, 1H), 4.72 (br t, J=11.0 Hz, 1H), 4.08 (s, 3H), 3.06 (br d, J=8.8 Hz, 3H), 2.21 - 2.14 (m, 1H), 1.84 (br t, J=8.7 Hz, 1H), 1.76 (s, 3H), 1.75 (s, 3H), 1.64 - 1.54 (m, 2H). LC-MS RT: 1.26 min; MS (ESI) m/z = 628.3 (M+H)+; Method A.

### Example 125

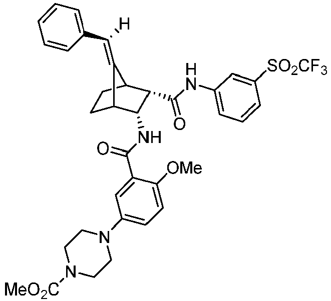

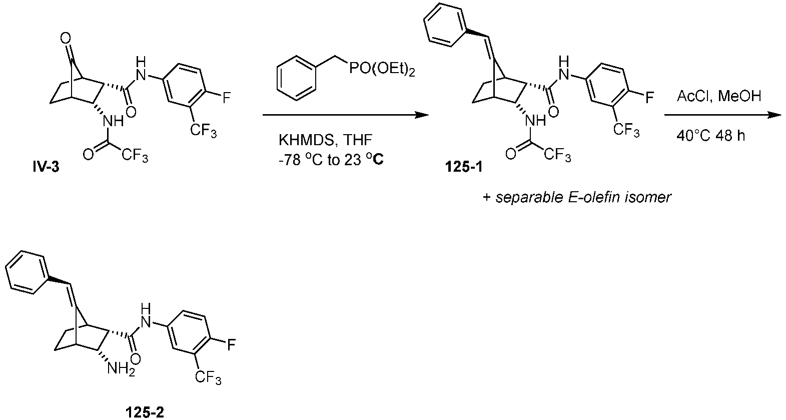

Intermediate **125-1:** Intermediate **125-1** was prepared from **IV-3** in the same manner as example 5 and purified via silica gel chromatography (49 mg, 0.10 mmol, 30% yield). RT = 1.23 min; MS (ESI) m/z = 501.1 (M+H)⁺; Method A.

Intermediate **125-2:** Intermediate **125-2** was prepared from **125-1** in the same manner as intermediate **IV-7** (73 mg, 0.18 mmol, 96% yield). RT = 0.87 min; MS (ESI) m/z = 405.1 (M+H)⁺; Method A.

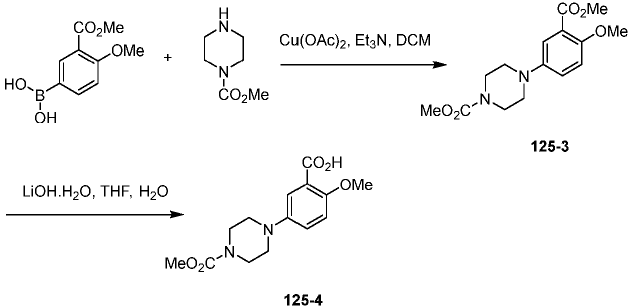

Intermediate **125-3:** Into the reaction vessel was added methyl piperazine-1-carboxylate (103 mg, 0.710 mmol), DCE (1 mL), MeCN (1 mL), copper (II) acetate (130 mg, 0.71 mmol), (4-methoxy-3-(methoxycarbonyl)phenyl)boronic acid (50 mg, 0.24 mmol), and 4Å molecular sieves (300 mg). The reaction mixture was stirred at 23 °C for 12 h (open to air), filtered, concentrated under reduced pressure, and purified via preparative RP-HPLC to produce **125-3** (37 mg, 0.12 mmol, 50% yield). LC-MS RT = 0.72 min; MS (ESI) m/z = 309.1 (M+H)⁺; Method A.

Intermediate **125-4:** Into the reaction vessel was added **125-3** (37 mg, 0.12 mmol), THF (1 mL), water (0.5 mL), and lithium hydroxide monohydrate (34.4 mg, 0.820 mmol). The reaction mixture was stirred at 23 °C for 2.5 h, diluted with EtOAc (10 mL), and washed with 10 mL sat. NH₄Cl containing 0.82 mmol HCl. The organic phased was dried over Na₂SO₄ and concentrated under reduced pressure to provide **125-4** (35.3 mg, 0.120 mmol, 100% yield) that was used without further purification. LC-MS RT = 0.62 min; MS (ESI) m/z = 295.0 (M+H)⁺; Method A.

Procedure for **example 125: Example 125** was prepared from **125-2,** employing **125-4,** according to the method described for **example 108.** ¹H NMR (500 MHz, CDCl₃) δ 9.63 (br d, J=7.7 Hz, 1H), 7.96 - 7.84 (m, 3H), 7.59 (dt, J=8.8, 3.4 Hz, 1H), 7.33 (d, J=4.1 Hz, 4H), 7.29 (dd, J=8.9, 3.2 Hz, 1H), 7.25 - 7.20 (m, 1H), 7.10 (t, J=9.4 Hz, 1H), 6.95 (d, J=9.1 Hz, 1H), 6.33 (s, 1H), 4.89 - 4.80 (m, 1H), 3.99 (s, 3H), 3.75 (s, 3H), 3.74 - 3.69 (m, 4H), 3.49 (t, J=3.3 Hz, 1H), 3.23 - 3.14 (m, 5H), 2.89 (m, 1H), 2.27 - 2.19 (m, 1H), 1.97 - 1.87 (m, 1H), 1.75 - 1.66 (m, 2H). LC-MS RT: 1.16 min; MS (ESI) m/z = 681.3 (M+H)+; Method A.

### Example 126

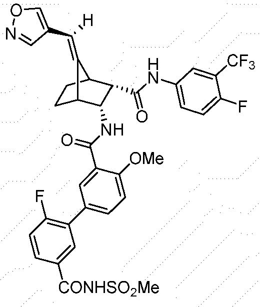

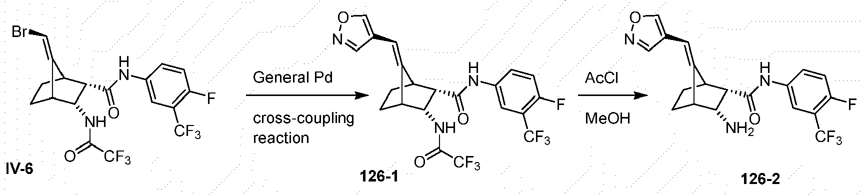

Intermediate **126-1:** Into the reaction vessel containing **IV-6** (125 mg, 0.25 mmol) was added 4-(4,4,5,5-tetramethyl-1,3,2-dioxaborolan-2-yl)isoxazole (125 mg, 0.610 mmol), PdCl₂(dppf)-CH₂Cl₂ adduct (50.7 mg, 0.0620 mmol), and Na₂CO₃ (1.5 mL, 3.0 mmol). The reaction mixture was degassed by bubbling nitrogen for 3 min, sealed, and stirred at 60 °C for 2 h. After allowing to cool to 23 °C, the reaction mixture was extracted with EtOAc, the combined organic portions dried over Na₂SO₄, filtered, concentrated under reduced pressure, and purified via silica gel chromatography to produce **126-1** (101 mg, 0.210 mmol, 83.0% yield). LC-MS RT = 1.07 min; MS (ESI) m/z = 492.1 (M+H)⁺; Method A.

Intermediate **126-2:** Intermediate **126-2** was prepared from **126-1** in the same manner as intermediate **IV-7** (67 mg, 0.16 mmol, 100% yield). RT = 0.76 min; MS (ESI) m/z = 396.0 (M+H)⁺; Method A.

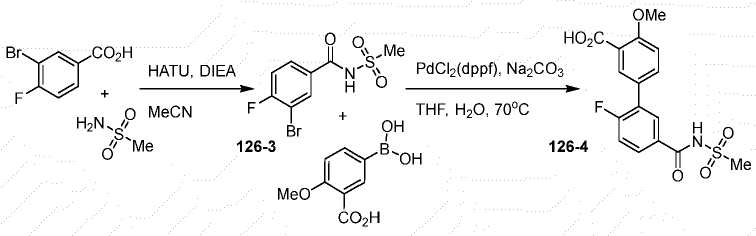

Intermediate **126-3:** Into the reaction vessel was added methanesulfonamide (521 mg, 5.48 mmol), 3-bromo-4-fluorobenzoic acid (400 mg, 1.83 mmol), MeCN (3.7 mL), DIEA (1.1 mL, 6.40 mmol), and HATU (833 mg, 2.19 mmol). The reaction mixture was stirred at 40 °C for 12 h, allowed to cool, concentrated under reduced pressure, and subjected to preparative RP-HPLC purification to produce **126-3** (450 mg, 1.52 mmol, 83% yield). LC-MS RT = 0.76 min; (M+H) = 297.7; Method A

Intermediate **126-4:** Into the reaction vessel containing **126-3** (200 mg, 0.68 mmol) was added 5-borono-2-methoxybenzoic acid (199 mg, 1.01 mmol), PdCl₂(dppf)-CH₂Cl₂ adduct (83 mg, 0.10 mmol), THF (6.7 mL) and 1 M Na₂CO₃ (4.0 mL, 4.1 mmol). The reaction mixture was degassed by bubbling nitrogen for 10 min, sealed, and stirred at 70 °C for 2 h. After allowing to cool to 23 °C, the reaction mixture was concentrated under reduced pressure and purified by preparative RP-HPLC to afford **126-4** (158 mg, 0.430 mmol, 64.0% yield). LC-MS RT = 0.70 min; MS (ESI) m/z = 368.1 (M+H)+; Method A.

Procedure for **example 126: Example 126** was prepared from **126-2,** employing **126-4,** according to the method described for **example 108.** ¹H NMR (500 MHz, CDCl₃) δ 10.32 (br s, 1H), 9.86 (br d, J=7.7 Hz, 1H), 8.41 (s, 1H), 8.34 (s, 1H), 8.25 (d, J=1.7 Hz, 1H), 8.10 (br s, 1H), 8.04 (dd, J=6.1, 2.5 Hz, 1H), 7.98 (dd, J=7.3, 2.1 Hz, 1H), 7.89 (ddd, J=8.5, 4.5, 2.2 Hz, 1H), 7.68 (br d, J=8.8 Hz, 1H), 7.58 (dt, J=8.6, 3.5 Hz, 1H), 7.19 - 7.06 (m, 3H), 5.95 (s, 1H), 4.72 - 4.63 (m, 1H), 4.08 (s, 3H), 3.45 (s, 3H), 3.25 - 3.20 (m, 1H), 3.15 (dd, J=10.7, 4.1 Hz, 1H), 2.89 - 2.84 (m, 1H), 2.28 - 2.23 (m, 1H), 2.01 - 1.96 (m, 1H), 1.71 - 1.62 (m, 2H). LC-MS RT: 1.09 min; MS (ESI) m/z = 745.2 (M+H)+; Method A.

### Example 127

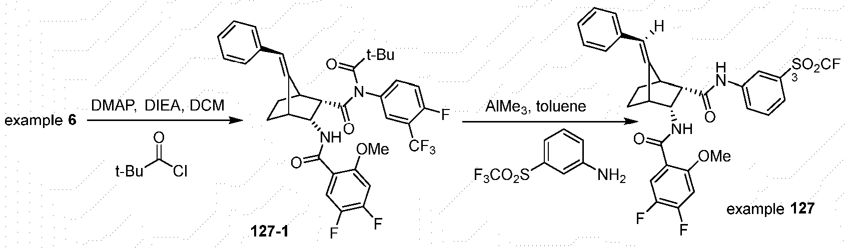

Intermediate **127-1:** Into the reaction vessel was added example **6** (10 mg, 0.017 mmol), DCM (1 mL), DIEA (0.015 mL, 0.087 mmol), and DMAP (1.06 mg, 8.70 µmol). After stirring at 23 °C for 12 h, the residue was purified via silica gel chromatography to produce **127-1** (10.5 mg, 0.0160 mmol, 92.0% yield). LC-MS RT = 1.31 min; MS (ESI) m/z = 659.3 (M+H)⁺; Method A.

Procedure for **example 127:** Into the reaction vessel was added 3-((trifluoromethyl)sulfonyl)aniline (24 mg, 0.11 mmol), toluene (0.5 mL) and trimethylaluminum (0.05 mL, 0.11 mmol). After stirring at 23 °C for 15 min, intermediate **127-1** (5 mg, 7.6 µmol) in toluene (0.5 mL) was added. The reaction was stirred at 23 °C for 1 h, quenched with sat. Rochelle salt and extracted with EtOAc. The organic phase was dried over Na₂SO₄, concentrated, and purified via preparative RP-HPLC to produce **example 127** (3.6 mg, 5.80 µmol, 76% yield). ¹H NMR (500 MHz, CDCl₃) δ 9.56 (br d, J=7.7 Hz, 1H), 8.56 - 8.47 (m, 1H), 8.06 - 7.98 (m, 2H), 7.78 - 7.71 (m, 2H), 7.58 (t, J=8.0 Hz, 1H), 7.37 - 7.30 (m, 4H), 7.25 - 7.21 (m, 1H), 6.79 (dd, J=11.7, 6.2 Hz, 1H), 6.33 (s, 1H), 4.88 - 4.81 (m, 1H), 4.03 (s, 3H), 3.52 - 3.47 (m, 1H), 3.20 (dd, J=10.7, 3.9 Hz, 1H), 2.91 - 2.87 (m, 1H), 2.26 - 2.18 (m, 1H), 1.95 - 1.88 (m, 1H), 1.74 - 1.70 (m, 2H). LC-MS RT: 1.25 min; MS (ESI) m/z = 621.2 (M+H)+; Method A.

### Example 130

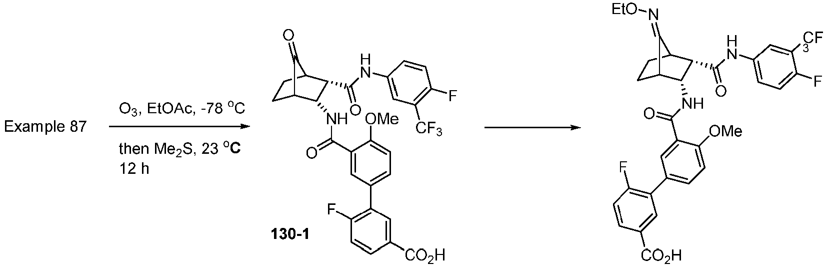

Intermediate **130-1:** Intermediate **130-1** was prepared from example **87** in the same manner as described for **77-1** (19 mg, 0.030 mmol, 100% yield). LC-MS RT = 1.06 min; MS (ESI) m/z = 603.1 (M+H)⁺; Method A.

Procedure for **example 130:** Into the reaction vessel containing **130-1** (17 mg, 0.03 mmol) was added DCE (1.5 mL), DIEA (0.09 mL, 0.51 mmol), and O-ethylhydroxylamine, HCl (41.3 mg, 0.42 mmol). The mixture was stirred 40 °C at 23 °C for 24 h, concentrated under reduced pressure, and subjected to preparative RP-HPLC purification to give **example 130** as a mixture of Z/E isomers (15 mg, 0.023 mmol, 82 % yield). LC-MS RT: 1.13 min; MS (ESI) m/z = 464.2 (M+H)+; Method A.

### Example 134

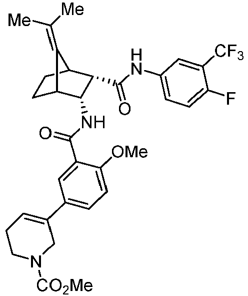

Procedure for **example 134:** Into the reaction vessel was added **example 140** (6 mg, 10 µmol), DCM (1 mL), DIEA (6 µl, 0.03 mmol), and methyl chloroformate (2 µl, 0.02 mmol). After stirring at 23 °C for 30 min, the reaction mixture was concentrated under reduced pressure and purified via preparative RP-HPLC to produce **example 134** (3.5 mg, 5.4 µmol, 51% yield). ¹H NMR (500 MHz, CDCl₃) δ 9.42 - 9.17 (m, 1H), 8.25 - 8.02 (m, 2H), 7.87 (dd, J=6.1, 2.4 Hz, 1H), 7.62 - 7.53 (m, 1H), 7.43 (br s, 1H), 7.07 (t, J=9.5 Hz, 1H), 6.92 (br d, J=8.6 Hz, 1H), 6.17 (br s, 1H), 4.78 - 4.66 (m, 1H), 4.38 - 4.23 (m, 2H), 3.98 (s, 3H), 3.75 (s, 3H), 3.66 - 3.52 (m, 2H), 3.08 - 2.97 (m, 3H), 2.37 - 2.26 (m, 2H), 2.23 - 2.11 (m, 1H), 1.83 - 1.75 (m, 1H), 1.74 - 1.72 (m, 3H), 1.72 (s, 3H), 1.62 - 1.53 (m, 2H). LC-MS RT: 1.25 min; MS (ESI) m/z = 630.3 (M+H)+; Method B.

### Example 136

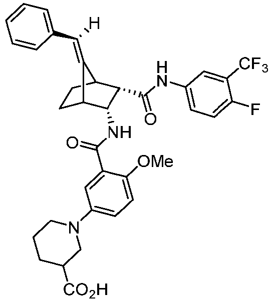

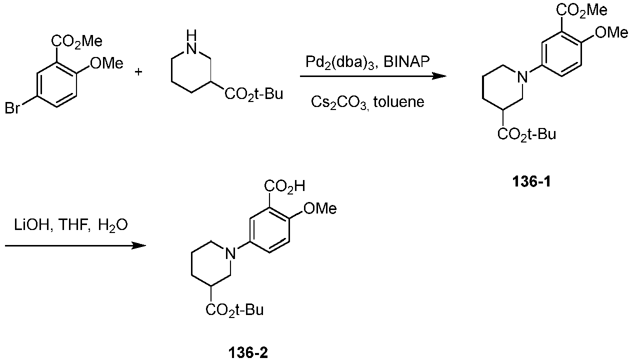

Intermediate **136-1:** Into the reaction vessel was added methyl 5-bromo-2-methoxybenzoate (33.1 mg, 0.135 mmol), tert-butyl piperidine-3-carboxylate (25 mg, 0.14 mmol), toluene (1 mL), tert-butyl piperidine-3-carboxylate (25 mg, 0.14 mmol), BINAP (10.5 mg, 0.0200 mmol) and Pd₂(dba)₃ (6 mg, 0.01 mmol). The reaction mixture was degassed with nitrogen for 3 min and was stirred at 100 °C for 12 h, allowed to cool to 23 °C, diluted with EtOAc, and the solution washed with sat. NaHCO₃ (2 x 10 mL). The organic layer was dried over Na₂SO₄, filtered, concentrated under reduced pressure, and purified via preparative RP-HPLC to produce **136-1** (39 mg, 0.084 mmol, 62% yield). LC-MS RT = 0.82 min; MS (ESI) m/z = 350.1 (M+H)⁺; Method A.

Intermediate **136-2:** Into the reaction vessel was added **136-1** (26 mg, 0.060 mmol), THF (1 mL), water (0.5 mL), and lithium hydroxide monohydrate (19.1 mg, 0.460 mmol). The reaction mixture was stirred at 23 °C for 3 h, diluted with EtOAc (10 mL), and washed with 10 mL sat. NH₄Cl containing 0.5 mmol HCl. The organic phase was dried over Na₂SO₄ filtered and concentrated under reduced pressure to provide **136-2** (19 mg, 0.060 mmol, 100% yield) which was used without further purification. LC-MS RT = 0.74 min; MS (ESI) m/z = 336.1 (M+H)⁺; Method A.

Procedure for **example 136: Example 136** was prepared from **125-2,** employing racemic **136-2,** according to the method described for **example 108.** A subsequent removal of the tert-butyl ester was accomplished as in the procedure to prepare **example 120. Example 136** (Peak 1) was separated from its diasteromer (Peak 2), **example 138,** via SFC chromatography. Peak 1, RT = 8.80 min, >99.5% ee; Peak 2, RT = 9.97 min, > 99.5% ee.

Preparative Chromatographic Conditions: Instrument: Berger MG II; Column: Chiralpak IA, 30 x 250 mm, 5 micron; Mobile Phase: 25% EtOH / 75% CO₂; Flow Conditions:;70 mL/min, 150 Bar, 40°C; Detector Wavelength: 220 nm; Injection Details: 0.5 mL of ~3 mg/mL in ACN. Analytical Chromatographic Conditions: Instrument: Berger Analytical SFC; Column: Chiralpak IA, 4.6 x 250 mm, 5 micron; Mobile Phase: 25% EtOH / 75% CO₂; Flow Conditions: 2.0 mL/min, 150 Bar, 40°C; Detector Wavelength: 220 nm; Injection Details: 10 µL of concentrated sample in EtOH. LC-MS RT: 1.07 min; MS (ESI) m/z = 666.3 (M+H)+; Method A.

### Example 140

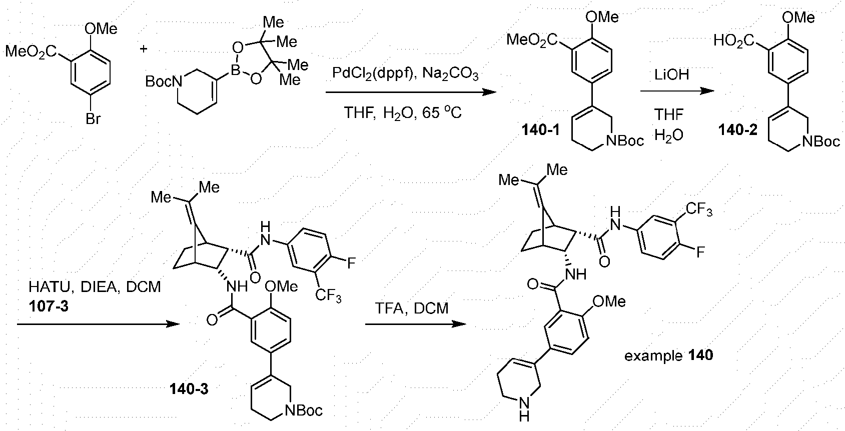

Intermediate **140-1:** Into the reaction vessel containing methyl 5-bromo-2-methoxybenzoate (47.6 mg, 0.190 mmol) was added tert-butyl 3-(4,4,5,5-tetramethyl-1,3,2-dioxaborolan-2-yl)-5,6-dihydropyridine-1(2H)-carboxylate (50 mg, 0.16 mmol), PdCl₂(dppf)-CH₂Cl₂ adduct (19.8 mg, 0.0240 mmol), and Na₂CO₃ (1 mL, 2 mmol). The reaction mixture was degassed by bubbling nitrogen for 3 min, sealed, and stirred at 65 °C for 2 h. After allowing to cool to 23 °C, the reaction mixture was extracted with EtOAc. The organic phase was dried over Na₂SO₄, filtered, concentrated under reduced pressure, and purified via silica gel chromatography to produce **140-1** (57.4 mg, 0.17 mmol, 100% yield). LC-MS RT = 1.04 min; MS (ESI) m/z = 348.0 (M+H)⁺; Method A.

Intermediate **140-2:** Into the reaction vessel was added **140-1** (28 mg, 0.081 mmol), THF (1 mL), water (0.5 mL), and lithium hydroxide monohydrate (16.9 mg, 0.400 mmol). The reaction mixture was stirred at 23 °C for 1 h, diluted with EtOAc (10 mL), and the resulting solution washed with 10 mL sat. NH₄Cl containing 0.5 mmol HCl. The organic phase was dried over Na₂SO₄ filtered and concentrated under reduced pressure to provide **140-2** (25 mg, 0.080 mmol, 93% yield) that was used without further purification.

Intermediate **140-3:** Intermediate **140-3** was prepared from **140-2** and **107-3** using the general amide coupling procedure employed in Example **108** (67 mg, 0.16 mmol, 100% yield). RT = 1.32 min; MS (ESI) m/z = 672.3 (M+H)⁺; Method A.

Procedure for **example 140:** Into the reaction vessel was added **140-3** (11.4 mg, 0.02 mmol), DCM (1 mL), and TFA (0.1 mL, 1.30 mmol). After stirring at 23 °C for 3 h, concentration of the reaction contents under reduction pressure provided **example 140** (3.7 mg, 5.13 µmol, 30% yield). ¹H NMR (500 MHz, CDCl₃) δ 9.29 (br d, J=7.9 Hz, 1H), 8.59 (br s, 1H), 8.08 (d, J=2.3 Hz, 1H), 7.94 (br d, J=4.5 Hz, 1H), 7.67 - 7.57 (m, 1H), 7.18 (br d, J=8.3 Hz, 1H), 7.06 (t, J=9.4 Hz, 1H), 6.82 (d, J=8.6 Hz, 1H), 6.16 (br s, 1H), 4.75 - 4.60 (m, 1H), 3.97 (s, 3H), 3.79 - 3.59 (m, 2H), 3.20 - 3.12 (m, 1H), 3.11 - 3.05 (m, 2H), 3.04 - 3.00 (m, 1H), 2.98 - 2.95 (m, 1H), 2.46 - 2.34 (m, 2H), 2.22 (br t, J=8.7 Hz, 1H), 1.77 (br t, J=8.7 Hz, 1H), 1.72 (s, 3H), 1.71 (s, 3H), 1.60 - 1.53 (m, 2H). LC-MS RT: 0.98 min; MS (ESI) m/z = 572.4 (M+H)+; Method B.

### Example 144

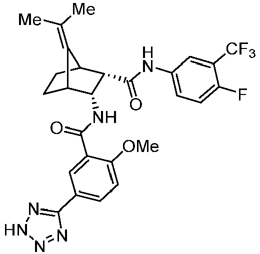

Procedure for **example 144:** Into the reaction vessel was added **example 114** (3.4 mg, 6.6 µmol), sodium azide (12.9 mg, 0.198 mmol), ammonium chloride (10.6 mg, 0.198 mmol), and DMF. The reaction mixture was stirred at 105 °C for 4 h, allowed to cool to 23 °C, diluted with MeOH, filtered, and purified via preparative RP-HPLC to produce **example 144** (2.3 mg, 4.0 µmol, 60% yield). ¹H NMR (500 MHz, CDCl₃) δ 10.01 (d, J=9.4 Hz, 1H), 9.30 (d, J=2.5 Hz, 1H), 8.51 (dd, J=8.8, 2.5 Hz, 1H), 8.44 (s, 1H), 8.11 (dd, J=6.3, 2.8 Hz, 1H), 7.41 (dt, J=8.7, 3.3 Hz, 1H), 7.25 - 7.22 (m, 1H), 7.08 - 6.99 (m, 1H), 4.96 (td, J=9.8, 4.3 Hz, 1H), 4.21 (s, 3H), 3.31 (dd, J=10.7, 3.9 Hz, 1H), 3.04 (t, J=3.7 Hz, 1H), 2.88 (t, J=4.0 Hz, 1H), 2.51 - 2.44 (m, 1H), 1.87 - 1.80 (m, 2H), 1.67 (s, 3H), 1.60 - 1.50 (m, 2H), 1.48 (s, 3H). LC-MS RT: 1.11 min; MS (ESI) m/z = 559.1 (M+H)+; Method A.

### Example 145

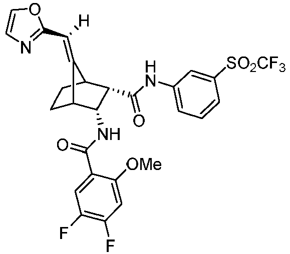

Procedure for **example 145: Example 145** was prepared from **5-6,** employing 2-methyloxazole: Into the reaction vessel was added 2-methyloxazole (24.9 mg, 0.300 mmol) and THF (1 mL). After the reaction mixture was cooled to -78 °C. KHMDS (0.30 mL, 0.30 mmol) was added. The mixture was stirred at -78 °C for 10 min and additional 2-methyloxazole (24.9 mg, 0.300 mmol) was added. The mixture was allowed to warm to 23 °C, stirred at 23 °C for 3 h, and quenched by the addition of sat. Na₂CO₃. The organic phase was dried over Na₂SO₄, filtered, concentrated, and purified via silica gel chromatography to produce the intermediate alcohol (17 mg, 0.029 mmol, 97% yield). The intermediate alcohol was dehydrated according to the method described for **example 33.** ¹H NMR (500 MHz, CDCl₃) δ 9.53 (br d, J=7.4 Hz, 1H), 8.06 - 7.99 (m, 2H), 7.97 (dd, J=6.2, 2.6 Hz, 1H), 7.63 (s, 1H), 7.53 (dt, J=8.9, 3.4 Hz, 1H), 7.18 - 7.09 (m, 2H), 6.80 (dd, J=11.6, 6.1 Hz, 1H), 6.28 (s, 1H), 4.88 - 4.80 (m, 1H), 4.00 (s, 3H), 3.93 (t, J=4.0 Hz, 1H), 3.19 (dd, J=10.9, 3.7 Hz, 1H), 2.99 - 2.92 (m, 1H), 2.35 - 2.27 (m, 1H), 2.02 - 1.94 (m, 1H), 1.78 - 1.71 (m, 2H). LC-MS RT: 1.17 min; MS (ESI) m/z = 566.0 (M+H)+; Method A.

### Example 147

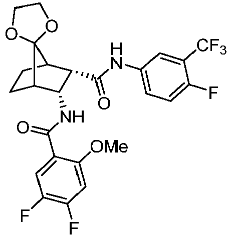

Procedure for **example 147:** Into the reaction vessel was added **5-6** (10 mg, 0.020 benzene (1 mL) ethane-1,2-diol (24.81 mg, 0.4000 mmol), MgSO₄ (200 mg, 1.66 mmol), and pTsOH monohydrate (3.8 mg, 0.020 mmol). After stirring at 50 °C for 12 h, the reaction mixture was filtered, concentrated under reduced pressure, and purified via preparative RP-HPLC to produce **example 147** (2.1 mg, 3.8 µmol, 19% yield). ¹H NMR (500 MHz, CDCl₃) δ 9.35 (br d, J=7.8 Hz, 1H), 8.04 (dd, J=11.4, 9.4 Hz, 1H), 7.93 (dd, J=6.3, 2.6 Hz, 1H), 7.77 (s, 1H), 7.52 (dt, J=8.7, 3.6 Hz, 1H), 7.12 (t, J=9.4 Hz, 1H), 6.79 (dd, J=11.6, 6.1 Hz, 1H), 5.05 - 4.97 (m, 1H), 4.08 - 4.01 (m, 4H), 3.99 (s, 3H), 3.49 - 3.41 (m, 1H), 2.23 (t, J=4.0 Hz, 1H), 2.20 - 2.11 (m, 2H), 1.93 - 1.81 (m, 2H), 1.75 - 1.67 (m, 1H). LC-MS RT: 1.14 min; MS (ESI) m/z = 545.1 (M+H)+; Method C.

### Example 150

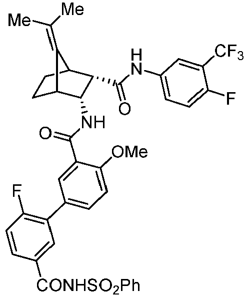

Procedure for **example 150:** Into the reaction vessel was added **example 87** (5 mg, 8 µmol), benzenesulfonamide (3.8 mg, 0.020 mmol), MeCN (1 mL), DIEA (5 µl, 0.03 mmol), and BOP-Cl (6.0 mg, 0.024 mmol). The reaction mixture was stirred at 40 °C for 12 h, concentrated under reduced pressure, and purified via preparative RP-HPLC to produce **example 150** (2.2 mg, 2.7 µmol, 34% yield). ¹H NMR (500 MHz, CDCl₃) δ 9.65 (br d, J=8.0 Hz, 1H), 8.41 (d, J=2.2 Hz, 1H), 7.96 (dd, J=7.2, 2.2 Hz, 2H), 7.90 - 7.84 (m, 2H), 7.73 (dt, J=8.5, 2.2 Hz, 1H), 7.56 (dt, J=8.7, 3.5 Hz, 1H), 7.25 (dd, J=9.9, 8.8 Hz, 1H), 7.16 - 7.03 (m, 2H), 4.75 - 4.68 (m, 1H), 4.08 (s, 3H), 3.06 (br d, J=8.8 Hz, 3H), 2.21 - 2.14 (m, 1H), 1.87 - 1.81 (m, 1H), 1.76 (s, 3H), 1.75 (s, 3H), 1.63 - 1.56 (m, 2H). LC-MS RT: 1.4 min; MS (ESI) m/z = 768.2 (M+H)+; Method C.

### Example 166

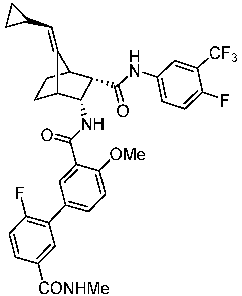

Intermediate **166-1:** Intermediate **166-1** was prepared from **IV-6** in the same manner as intermediate **126-1** (5.1 mg, 0.010 mmol, 23% yield). RT = 1.21 min; MS (ESI) m/z = 465.1 (M+H)⁺; Method A.

Intermediate **166-2:** Intermediate **166-2** was prepared from **166-1** in the same manner as intermediate **IV-7** (4.0 mg, 0.010 mmol, 100% yield). RT = 0.84 min; MS (ESI) m/z = 369.1 (M+H)⁺; Method A.

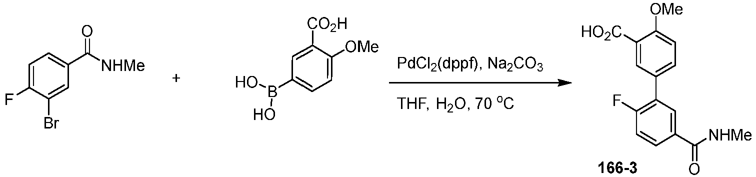

Intermediate **166-3:** Intermediate **166-3** was prepared from 3-bromo-4-fluoro-N-methylbenzamide and 5-borono-2-methoxybenzoic acid in the same manner as intermediate **140-1** (28 mg, 0.080 mmol, 41% yield). LC-MS RT = 0.99 min; MS (ESI) m/z = 304.9 (M+H)⁺; Method A.

Procedure for **example 166: Example 166** was prepared from **166-2,** employing **166-3,** according to the method described for **example 108.** ¹H NMR (500 MHz, CDCl₃) δ 9.73 (br d, J=7.7 Hz, 1H), 8.39 (d, J=1.9 Hz, 1H), 7.97 (dd, J=6.2, 2.3 Hz, 1H), 7.90 (s, 1H), 7.85 (dd, J=7.4, 2.2 Hz, 1H), 7.81 (ddd, J=8.5, 4.7, 2.2 Hz, 1H), 7.72 (dt, J=8.8, 2.2 Hz, 1H), 7.56 (dt, J=8.7, 3.4 Hz, 1H), 7.24 - 7.19 (m, 1H), 7.15 - 7.10 (m, 1H), 7.08 (d, J=8.8 Hz, 1H), 6.47 (br s, 1H), 4.85 - 4.76 (m, 1H), 4.66 (d, J=9.6 Hz, 1H), 4.09 (s, 3H), 3.22 (t, J=3.7 Hz, 1H), 3.10 (dd, J=10.7, 3.3 Hz, 1H), 3.05 (d, J=4.7 Hz, 3H), 2.77 - 2.67 (m, 1H), 2.19 - 2.12 (m, 1H), 1.94 - 1.86 (m, 1H), 1.71 - 1.61 (m, 2H), 1.53 - 1.46 (m, 1H), 0.81 - 0.71 (m, 2H), 0.41 - 0.30 (m, 2H). LC-MS RT: 1.18 min; MS (ESI) m/z = 654.2 (M+H)+; Method A.

### Example 168

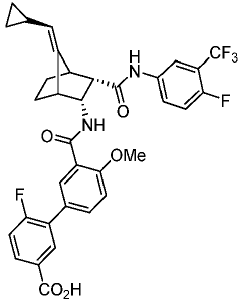

Procedure for **example 168: Example 168** was prepared from **166-2,** employing **120-6,** according to the method described for **example 108.** Cleavage of the tert-butyl ester was accomplished in DCM (1 mL) and stirring with ZnBr₂ (20 equiv.) at 23 °C for 12 h. After quenching the reaction by the addition of HCl (1.0 M) and extracting the resulting solution with ethyl acetate, the organic phase was dried over Na₂SO₄ filtered, concentrated under reduced pressure and the residue purified via preparative RP-HPLC to produce **example 168.** Analytical data for **example 168:** ¹H NMR (500 MHz, CDCl₃) δ 9.42 (br d, J=7.7 Hz, 1H), 8.43 (br s, 1H), 8.30 - 8.21 (m, 1H), 8.12 - 8.02 (m, 1H), 7.96 (br s, 2H), 7.71 (dt, J=8.7, 2.0 Hz, 1H), 7.54 - 7.47 (m, 1H), 7.26 - 7.21 (m, 1H), 7.13 - 7.06 (m, 2H), 4.95 - 4.85 (m, 1H), 4.66 (d, J=9.6 Hz, 1H), 4.07 (s, 3H), 3.27 - 3.19 (m, 1H), 3.14 (br dd, J=10.9, 3.2 Hz, 1H), 2.75 (t, J=3.9 Hz, 1H), 2.32 - 2.23 (m, 1H), 1.94 - 1.87 (m, 1H), 1.74 - 1.63 (m, 2H), 1.54 - 1.48 (m, 1H), 0.76 (m, 2H), 0.36 (m, 2H). LC-MS RT: 1.19 min; MS (ESI) m/z = 641.1 (M+H)+; Method A.

### Example 170

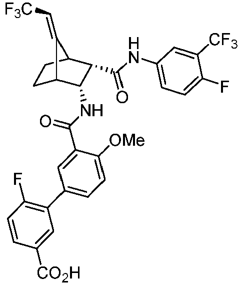

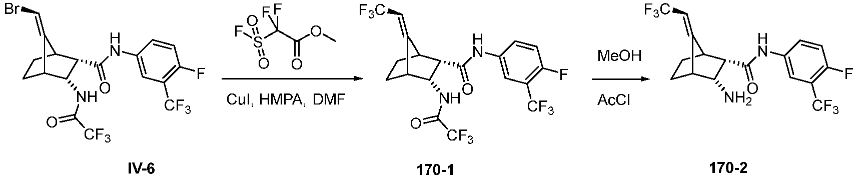

Intermediate **170-1:** To a 20 mL vial charged with methyl 2,2-difluoro-2-(fluorosulfonyl)acetate (volume ?, 0.15 mmol) in anhydrous DMF (0.5 mL) was added dropwise via syringe to a suspension of **IV-6** and CuI (mass?, 0.07 mmol) in anhydrous DMF (1 mL) and HMPA (0.5 mL) at 75 °C under a nitrogen atmosphere for 30 min. The resulting mixture was stirred at the same temperature for 12 h. The reaction mixture was allowed to cool and filtered via an HPLC filter and purified by RP-HPLC to produce **170-1** (24 mg, 81% yield). ¹H NMR (500 MHz, CDCl₃) δ 9.38 (br d, J=6.1 Hz, 1H), 7.77 - 7.69 (m, 2H), 7.46 (s, 1H), 7.24 (t, J=9.1 Hz, 1H), 5.62 (q, J=7.2 Hz, 1H), 4.50 (dt, J=10.5, 5.3 Hz, 1H), 3.50 - 3.42 (m, 1H), 3.13 - 3.04 (m, 1H), 2.89 (t, J=4.0 Hz, 1H), 2.02 - 1.90 (m, 2H), 1.76 - 1.60 (m, 2H).

Intermediate **170-2:** Intermediate **170-2** was prepared from **170-1.** MeOH (1.5 mL) and acetyl chloride (2.1 mmol) were charged into a 2 dram vial and stirred at 23 °C for 5 min. **170-1** was added to the reaction vial and the contents heated to 40 °C for 24 h. The reaction mixture was concentration with a stream of nitrogen gave **170-2** as the HCl salt which was used without further purification. LC-MS RT = 0.75 min; MS (ESI) m/z = 397.1 (M+H)⁺; Method A.

Procedure for **example 170: Example 170** was prepared from **170-2,** employing **120-6,** according to the method described **example 120.** Analytical data for **example 170:** ¹H NMR (500 MHz, CDCl₃) δ 9.28 (br d, J=6.6 Hz, 1H), 8.41 (br s, 1H), 8.37 (br s, 1H), 8.27 (br d, J=6.1 Hz, 1H), 8.08 (br s, 1H), 7.92 (br s, 1H), 7.80 - 7.70 (m, 1H), 7.47 (dt, J=8.6, 3.7 Hz, 1H), 7.27 - 7.20 (m, 1H), 7.13 - 7.02 (m, 2H), 5.60 (q, J=7.3 Hz, 1H), 5.05 - 4.92 (m, 1H), 4.06 (s, 3H), 3.42 (br s, 1H), 3.25 (br dd, J=10.6, 3.4 Hz, 1H), 2.95 (t, J=4.0 Hz, 1H), 2.61 - 2.50 (m, 1H), 2.05 - 1.97 (m, 1H), 1.82 - 1.72 (m, 2H). LC-MS RT: 1.15 min; MS (ESI) m/z = 669.2 (M+H)+; Method A.

### Example 171

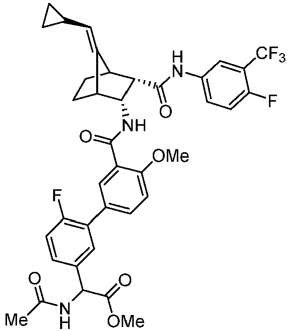

Procedure for **example 171: Example 171** was prepared from **example 186.** To a 1 dram vial charged with **example 186** (0.008 mmol), DCM (0.3 mL), and MeOH (0.1 mL) was added TMS-diazomethane (0.5 M in DCM, 0.34 mL, 0.17 mmol, 20 equiv.), and the reaction mixture stirred at 23 °C for 1 h. The reaction mixture was concentrated under reduced pressure and purified via silica gel normal phase chromatography to give 6.1 mg of example **171.** Analytical data for **example 171:** ¹H NMR (500 MHz, CDCl₃) δ 9.45 (br d, J=8.0 Hz, 1H), 8.38 - 8.35 (m, 1H), 8.00 - 7.92 (m, 2H), 7.64 (dt, J=8.7, 2.0 Hz, 1H), 7.54 (dt, J=8.7, 3.5 Hz, 1H), 7.43 (dd, J=7.3, 2.3 Hz, 1H), 7.32 (ddd, J=8.4, 4.5, 2.5 Hz, 1H), 7.17 - 7.03 (m, 3H), 6.59 (br d, J=6.9 Hz, 1H), 5.61 (d, J=6.9 Hz, 1H), 4.89 - 4.81 (m, 1H), 4.65 (d, J=9.6 Hz, 1H), 4.06 (s, 3H), 3.77 (s, 3H), 3.21 (t, J=4.1 Hz, 1H), 3.15 - 3.08 (m, 1H), 2.73 (t, J=4.0 Hz, 1H), 2.24 - 2.16 (m, 1H), 2.08 (s, 3H), 1.95 - 1.86 (m, 1H), 1.72 - 1.63 (m, 2H), 1.54 - 1.45 (m, 1H), 0.79 - 0.72 (m, 2H), 0.39 - 0.32 (m, 2H). LC-MS RT: 1.15 min; MS (ESI) m/z = 726.3 (M+H)+; Method A.

### Example 172

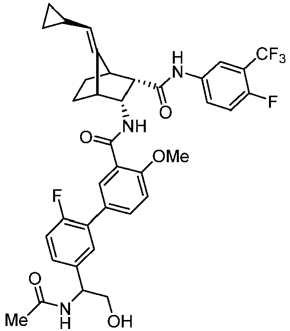

Procedure for **example 172: Example 172** was prepared from **example 171.** To ice bath cooled 1 dram vial charged with **example 171** (0.009 mmol) and THF (0.5 mL) was added LiBH₄ (0.027, 3.0 equiv.). The reaction mixture was stirred at 0 °C for 5 min and then allowed to warm to 23 °C and stirred for an additional 30 min. The reaction mixture was diluted with ethyl acetate (10 mL). The solution was washed with saturated aqueous ammonium chloride (20 mL). The aqueous phase was extracted with EtOAc, the combined organic portions dried over Na₂SO₄, filtered and concentrated under reduced pressure and the residue purified via preparative RP-HPLC to give **example 172.** Analytical data for **example 172:** ¹H NMR (500 MHz, CDCl₃) δ 9.54 (d, J=8.0 Hz, 1H), 8.29 (d, J=1.7 Hz, 1H), 8.21 (s, 1H), 7.96 (dd, J=6.1, 2.5 Hz, 1H), 7.57 - 7.47 (m, 2H), 7.32 - 7.29 (m, 1H), 7.23 (ddd, J=8.3, 4.6, 2.5 Hz, 1H), 7.12 - 7.00 (m, 3H), 6.45 (br d, J=6.9 Hz, 1H), 5.10 - 5.03 (m, 1H), 4.85 - 4.76 (m, 1H), 4.62 (d, J=9.4 Hz, 1H), 4.08 (s, 3H), 3.93 - 3.86 (m, 2H), 3.18 (t, J=4.1 Hz, 1H), 3.13 - 3.06 (m, 1H), 2.71 (t, J=4.0 Hz, 1H), 2.26 - 2.18 (m, 1H), 2.08 (s, 3H), 1.95 - 1.87 (m, 1H), 1.70 - 1.62 (m, 2H), 1.51 - 1.43 (m, 1H), 0.77 - 0.72 (m, 2H), 0.37 - 0.30 (m, 2H). LC-MS RT: 1.08 min; MS (ESI) m/z = 698.4 (M+H)+; Method A.

### Example 177

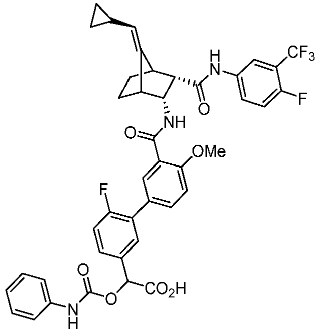

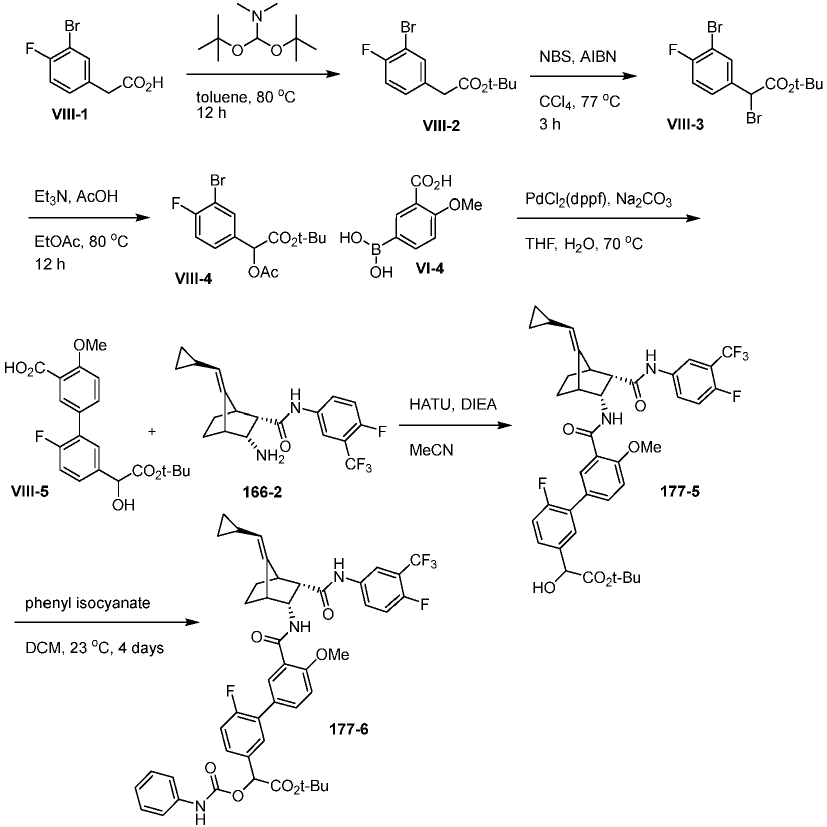

Intermediate **VIII-2:** Intermediate **VIII-2** was prepared employing known conditions for analogous substrates (Ludwig, J.; Lehr, M. Syn. Comm. 2004, 34, 3691-3695), except the reaction temperature was maintained at 80 °C for 12 h. ¹H NMR (500 MHz, CDCl₃) δ 7.49 (dd, J=6.6, 2.2 Hz, 1H), 7.20 (ddd, J=8.3, 4.6, 2.2 Hz, 1H), 7.13 - 7.03 (m, 1H), 3.49 (s, 2H), 1.46 (s, 9H).

Intermediate **VIII-3:** To a 20 mL reaction vial charged with intermediate **VIII-2** (266 mg, 0.920 mmol) was added NBS (196 mg, 1.10 mmol), carbon tetrachloride (10 mL), and AIBN (15 mg, 0.090 mmol). The solution was stirred at 77 °C, for 3 h. The solution was concentrated under reduced pressure and purified by normal phase silica gel chromatography to give intermediate **VIII-3** (308 mg, 0.840 mmol, 91.0% yield).

Intermediate **VIII-4:** To a 2 dram vial charged with intermediate **VIII-3** was added ethyl acetate (2 mL), triethyl amine (0.27 mL, 2.0 mmol), and acetic acid (0.1 mL, 2 mmol). The reaction mixture was stirred at 80 °C for 12 h. The reaction mixture was concentrated under reduced pressure and purified by normal phase silica gel chromatography to give intermediate **VIII-4.** ¹H NMR (500 MHz, CDCl₃) δ 7.70 (dd, J=6.6, 2.2 Hz, 1H), 7.41 (ddd, J=8.4, 4.7, 2.1 Hz, 1H), 7.15 (t, J=8.4 Hz, 1H), 5.77 (s, 1H), 2.22 (s, 3H), 1.43 (s, 9H).

Intermediate **VIII-5:** Intermediate **VIII-5** was prepared from intermediate **VIII-4,** employing 5-borono-2-methoxybenzoic acid as the same conditions that were used for intermediate **140-1.** Half of the material was isolated as the O-acetate (85 mg, 0.60 mmol, 34%); ¹H NMR (500 MHz, CDCl₃) δ 8.43 - 8.36 (m, 1H), 7.81 (dt, J=8.7, 2.0 Hz, 1H), 7.56 (dd, J=7.3, 2.3 Hz, 1H), 7.45 (ddd, J=8.5, 4.6, 2.3 Hz, 1H), 7.23 - 7.16 (m, 2H), 5.84 (s, 1H), 4.17 (s, 3H), 2.23 (s, 3H), 1.45 (s, 9H) while the other half was isolated as the free alcohol (70 mg, 0.19 mmol, 31%); ¹H NMR (500 MHz, CDCl₃) δ 8.40 (d, *J*=2.2 Hz, 1H), 7.82 (dt, *J*=8.6, 2.2 Hz, 1H), 7.54 (dd, *J*=7.4, 2.5 Hz, 1H), 7.41 (ddd, *J*=8.4, 4.8, 2.2 Hz, 1H), 7.19 - 7.14 (m, 2H), 5.09 (s, 1H), 4.16 (s, 3H), 1.47 (s, 9H). Racemic **VIII-5** was separated into individual enantiomers using chiral SFC. Preparative chromatographic conditions: Instrument: Berger MG II; Column: Chiralpak ID, 21 x 250 mm, 5 micron; Mobile phase: 25% IPA / 75% CO₂; Flow conditions; 45 mL/min, 120 Bar, 40 °C; Detector wavelength: 220 nm; Injection details: 8 injections of 0.36 mL of ~20 mg/mL in IPA. Analytical chromatrographic conditions: Instrument: Waters UPC2 analytical SFC; Column: Chiralpak ID 4.6 x 100 mm, 3 micron; Mobile phase: 25% IPA / 75% CO₂; Flow conditions: 2 mL/min, 150 Bar, 40 °C; Detector wavelength: 220 nm. Peak 1, RT = 3.89 min, >99.5% ee; Peak 2, RT = 5.44 min, >99.5% ee. Intermediate **VIII-5** product Peak #2 was collected and carried forward to produce chiral intermediate **177-5.**

Intermediate **177-5:** Intermediate **177-5** was prepared from **VIII-5** peak 2, according to the method described for **example 108.** Intermediate **177-5** (14.2 mg, 0.0200 mmol, 79.0% yield). LC-MS RT = 1.22 min; MS (ESI) m/z = 727.1 (M+H)⁺; Method A.

Intermediate **177-6:** To a 1 dram vial charged with **177-5** was added DCM (1 mL) and phenyl isocyanate (82 mg, 0.69 mmol). The solution stirred for 4 days at 23 °C, concentrated under reduced pressure and purified by RP-HPLC to give intermediate **177-6** (6.2 mg, 0.0070 mmol, 53% yield).

Procedure for **example 177: Example 177** was prepared from **177-6** by employing the tert-butyl ester cleavage method described for **example 168.** Analytical data for **example 177:** ¹H NMR (500 MHz, CDCl₃) δ 9.74 (br d, J=8.0 Hz, 1H), 8.22 (d, J=2.2 Hz, 1H), 8.06 - 7.97 (m, 2H), 7.73 (br s, 1H), 7.65 (td, J=8.7, 2.1 Hz, 2H), 7.46 (dt, J=8.8, 3.4 Hz, 1H), 7.41 - 7.33 (m, 3H), 7.24 (t, J=7.8 Hz, 2H), 7.09 - 6.98 (m, 4H), 6.15 (s, 1H), 4.84 - 4.74 (m, 1H), 4.59 (d, J=9.6 Hz, 1H), 4.04 (s, 3H), 3.16 (t, J=4.0 Hz, 1H), 3.09 (br dd, J=10.6, 3.7 Hz, 1H), 2.67 (br t, J=3.7 Hz, 1H), 2.21 - 2.14 (m, 1H), 1.91 - 1.82 (m, 1H), 1.68 - 1.52 (m, 2H), 1.51 - 1.41 (m, 1H), 0.79 - 0.69 (m, 2H), 0.36 - 0.29 (m, 2H). LC-MS RT: 1.26 min; MS (ESI) m/z = 790.4 (M+H)+; Method A.

### Example 178

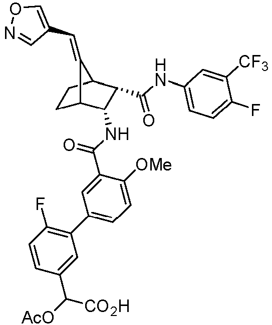

Procedure for **example 178: Example 178** was prepared from **34-1.** To a 2 dram vial charged with **34-1,** DCM (1.5 mL), and DIEA (0.12 mL, 0.70 mmol, 30 equiv.) was added acetyl chloride (0.03, 0.5 mmol, 20 equiv.) and stirred 1 h at 23 °C. The reaction was quenched by the addition of MeOH (1 mL) and the tert-butyl ester was removed according to the method described for **example 168.** Analytical data for **example 178:** ¹H NMR (500 MHz, CDCl₃) δ 9.82 (d, J=8.3 Hz, 1H), 8.45 (s, 1H), 8.36 (s, 1H), 8.29 (s, 1H), 8.26 (d, J=2.5 Hz, 1H), 8.00 (dd, J=6.3, 2.5 Hz, 1H), 7.70 (dt, J=8.5, 2.2 Hz, 1H), 7.62 (dd, J=7.4, 2.2 Hz, 1H), 7.46 (ddd, J=8.5, 4.3, 2.6 Hz, 2H), 7.14 (dd, J=10.0, 8.7 Hz, 1H), 7.08 - 7.00 (m, 2H), 5.98 (s, 1H), 5.98 (s, 1H), 4.88 - 4.79 (m, 1H), 4.06 (s, 3H), 3.25 - 3.19 (m, 2H), 2.91 - 2.86 (m, 1H), 2.40 - 2.33 (m, 1H), 2.19 (s, 3H), 2.00 - 1.93 (m, 1H), 1.72 - 1.60 (m, 2H) LC-MS RT: 1.11 min; MS (ESI) m/z = 740.1 (M+H)+; Method A.

### Example 179

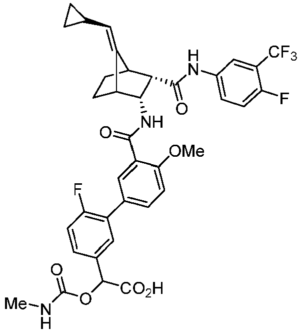

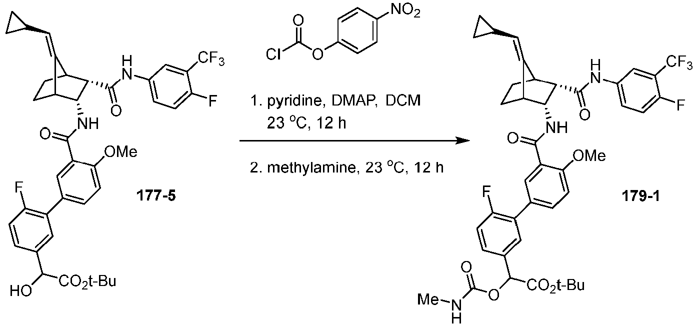

Intermediate **179-1:** To a 20 mL vial charged with **177-5** was added DCM (4 mL), 4-nitrophenyl carbonochloridate (volume or mass, 0.43 mmol) and DMAP (mass, 0.04 mmol). The reaction solution was stirred for at 23 °C for 12 h. Methylamine (0.85 mmol) was added and the reaction solution stirred for an additional 1 h. The reaction solution was concentrated under reduced pressure and purified by RP-HPLC to give intermediate **179-1** (65 mg, 0.083 mmol, 97%). LC-MS RT = 1.24 min; MS (ESI) m/z = 784.4 (M+H)⁺; Method A.

Procedure for **example 179: Example 179** was prepared from **179-1** according to the method described for the tert-butyl ester cleavage as in **example 168.** Analytical data for **example 179:** ¹H NMR (500 MHz, CDCl₃) δ 9.70 (br d, J=8.0 Hz, 1H), 8.28 (d, J=1.9 Hz, 1H), 8.19 (s, 1H), 8.01 (dd, J=6.2, 2.6 Hz, 1H), 7.68 (dt, J=8.6, 2.2 Hz, 1H), 7.60 (br d, J=5.5 Hz, 1H), 7.52 - 7.45 (m, 1H), 7.45 - 7.37 (m, 1H), 7.15 - 7.00 (m, 3H), 6.05 (s, 1H), 5.38 - 5.28 (m, 1H), 4.82 - 4.75 (m, 1H), 4.60 (d, J=9.4 Hz, 1H), 4.07 (s, 3H), 3.16 (t, J=4.1 Hz, 1H), 3.10 (dd, J=10.5, 3.3 Hz, 1H), 2.85 (br d, J=3.3 Hz, 3H), 2.71 - 2.67 (m, 1H), 2.25 - 2.20 (m, 1H), 1.92 - 1.86 (m, 1H), 1.68 - 1.53 (m, 2H), 1.49 - 1.42 (m, 1H), 0.78 - 0.69 (m, 2H), 0.36 - 0.30 (m, 2H). LC-MS RT: 1.13 min; MS (ESI) m/z = 728.3 (M+H)+; Method A.

### Example 182

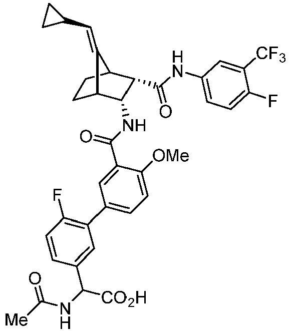

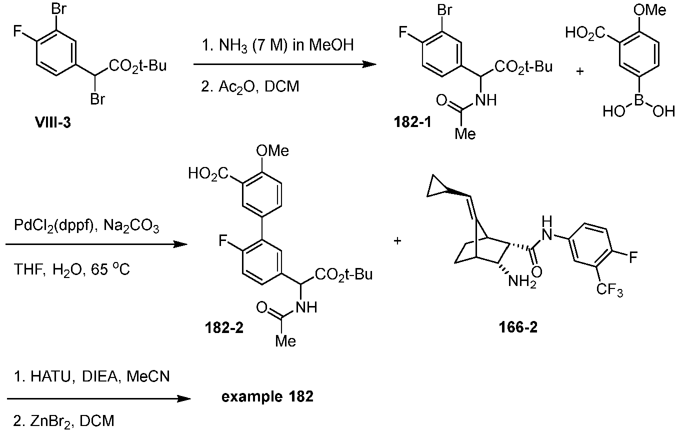

Intermediate **182-1:** To a 1 dram vial charged with intermediate **VIII-3** was added ammonia (0.5 mL, 4 mmol, 7 M in MeOH). The solution was stirred at 23 °C for 12 h. The solution was conentrated under reduced pressure and the residue, was treated with acetic anhydride (7.2 µL, 0.076 mmol) in DCM (1 mL) and stirred at 23 °C for 1 h. The resulting residue was purified by normal phase silica gel chromatography to give intermediate **182-1** (26 mg, 0.074 mmol, 97% yield). LC-MS RT = 0.92 min; MS (ESI) m/z = 346.1 (M+H)⁺; Method A.

Intermediate **182-2:** Intermediate **182-2** was prepared employing similar conditions described for intermediate **140-1,** except at a temperature of 65 °C for 18 h. ¹H NMR (500 MHz, CDCl₃) δ 8.37 (d, J=1.9 Hz, 1H), 7.81 (dt, J=8.5, 2.1 Hz, 1H), 7.45 (dd, J=7.3, 2.3 Hz, 1H), 7.35 (ddd, J=8.5, 4.6, 2.3 Hz, 1H), 7.21 - 7.13 (m, 2H), 6.74 (br d, J=6.9 Hz, 1H), 5.51 (d, J=6.9 Hz, 1H), 4.17 (s, 3H), 2.12 (s, 3H), 1.45 (s, 9H). Racemic **182-2** was separated into it's enantiomers using chiral SFC. Preparative chromatographic conditions: Instrument: Berger MG II; Column: Chiralpak ID, 21 x 250 mm, 5 micron; Mobile phase: 20% IPA / 80% CO₂; Flow conditions; 45 mL/min, 120 Bar, 40 °C; Detector wavelength: 215 nm; Injection details: 3 injections of 15 mg/mL in MeOH. Analytical chromatographic conditions: Instrument: Aurora Infinity analytical SFC; Column: Chiralpak AD-H, 4.6 x 100 mm, 3 micron; Mobile phase: 20% IPA / 80% CO₂; Flow conditions: 2 mL/min, 150 Bar, 40 °C; Detector wavelength: 220 nm. Peak 1, RT = 3.49 min, >99.5% ee; Peak 2, RT = 4.43 min, >99.5% ee. Intermediate **182-2** product Peak #2 was collected and carried forward to produce **example 182.**

Procedure for **example 182: Example 182** was prepared from **166-2,** employing **182-2** (peak 2, isomer 2), according to the method described for **example 108.** A subsequent removal of the tert-butyl ester was accomplished as in the procedure to prepare **example 168.** Analytical data for **example 182** (isomer 1): ¹H NMR (500 MHz, CDCl₃) δ 10.14 (d, J=7.7 Hz, 1H), 8.72 (br d, J=9.1 Hz, 1H), 8.46 (d, J=2.5 Hz, 1H), 8.00 (dd, J=6.1, 2.8 Hz, 1H), 7.80 - 7.70 (m, 2H), 7.61 (s, 1H), 7.46 - 7.38 (m, 2H), 7.11 - 7.01 (m, 2H), 6.98 (d, J=8.8 Hz, 1H), 5.96 (d, J=9.1 Hz, 1H), 4.73 - 4.65 (m, 2H), 4.04 (s, 3H), 3.18 (br t, J=3.7 Hz, 1H), 3.03 (dd, J=10.6, 4.0 Hz, 1H), 2.69 (br t, J=3.7 Hz, 1H), 2.13 (s, 3H), 2.06 - 1.98 (m, 1H), 1.88 - 1.80 (m, 1H), 1.64 - 1.49 (m, 3H), 0.89 - 0.76 (m, 2H), 0.44 - 0.34 (m, 2H). LC-MS RT: 1.11 min; MS (ESI) m/z = 712.2 (M+H)+; Method A.

### Example 183

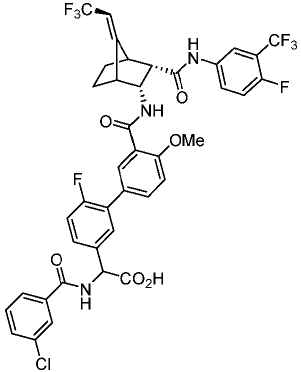

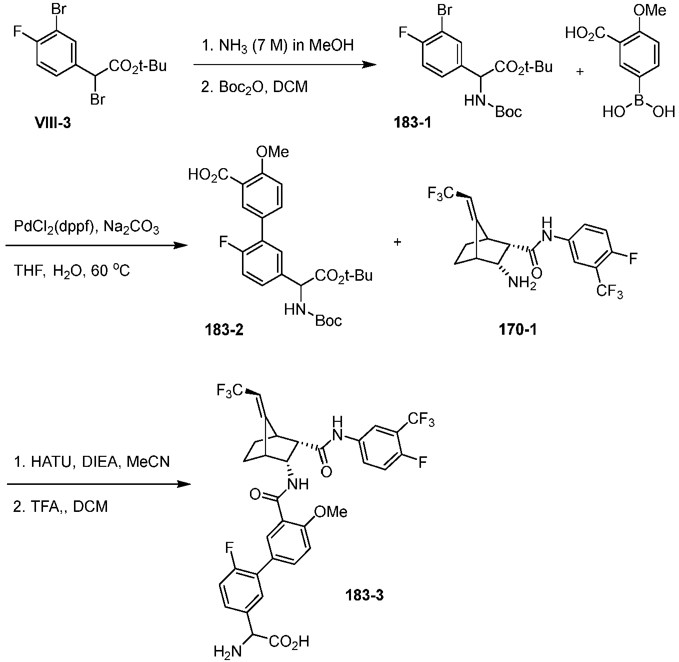

Intermediate **183-1:** Intermediate **183-1** was prepared from **VIII-3** according to the method described for intermediate **182-1** with the replacment of Ac₂O with Boc₂O. LC-MS RT = 1.14 min; MS (ESI) m/z = 406.0 (M+H)⁺; Method A.

Intermediate **183-2:** Intermediate **183-2** was prepared employing that same conditions that were used for intermediate **140-1,** except at a temperature of 60 °C for 18 h. ¹H NMR (500 MHz, CDCl₃) δ 8.38 (d, *J*=1.9 Hz, 1H), 7.80 (dt, *J*=8.7, 2.0 Hz, 1H), 7.46 (dd, *J*=7.4, 2.5 Hz, 1H), 7.36 (dddd, *J*=8.8, 4.4, 2.2, 1.1 Hz, 1H), 7.19 - 7.13 (m, 2H), 5.67 (br d, *J*=5.2 Hz, 1H), 5.25 (br d, *J*=6.3 Hz, 1H), 4.16 (s, 3H), 1.46 (br s, 9H), 1.44 (s, 9H). Racemic **183-2** was separated into individual enantiomers using chiral SFC. Preparative chromatographic conditions: Instrument: Berger MG II; Column: Chiralpak ID, 21 x 250 mm, 5 micron; Mobile phase: 20% MeOH / 80% CO₂; Flow conditions; 45 mL/min, 120 Bar, 40 °C; Detector wavelength: 209 nm; Injection details: 49 injections in MeOH. Analytical chromatographic conditions: Instrument: Waters UPC2 analytical SFC; Column: Chiralpak IC, 4.6 x 100 mm, 3 micron; Mobile phase: 25% MeOH / 75% CO₂; Flow conditions: 2 mL/min, 150 Bar, 40 °C; Detector wavelength: 220 nm. Peak 1, RT = 4.22 min, 95.7% ee; Peak 2, RT = 5.11 min, >99% ee. Intermediate **183-2** product Peak #2 was collected and carried forward to produce intermediate **183-3.**

Intermediate **183-3:** Intermediate **183-3** was prepared from **183-2** according to the method described for **example 108.** A subsequent removal of the tert-butyl ester was accomplished as in the procedure to prepare **example 120.** LC-MS RT = 0.99 min; MS (ESI) m/z = 698.3 (M+H)⁺; Method A.

Procedure for **example 183: Example 183** was prepared from **183-3.** A 2 dram vial was charged with **183-3,** DIEA (0.06 mmol, 5 equiv.) and 4-chlorobenzoyl chloride (0.035 mmol, 3.0 equiv.). The solution was stirred at 23 °C for 30 min and subsequently quenched with MeOH. The reaction contents were concentrated under reduced pressure to provide crude product that was purified via preparative RP-HPLC to give **example 183.** Analytical data for **example 183:** ¹H NMR (500 MHz, CDCl₃) δ 9.98 (br d, J=8.0 Hz, 1H), 8.77 - 8.68 (m, 1H), 8.49 (d, J=2.5 Hz, 1H), 7.96 (dd, J=6.3, 2.5 Hz, 1H), 7.91 - 7.84 (m, 2H), 7.77 - 7.71 (m, 1H), 7.69 (t, J=1.7 Hz, 1H), 7.60 (d, J=8.0 Hz, 1H), 7.55 (ddd, J=8.3, 4.3, 2.2 Hz, 1H), 7.43 - 7.39 (m, 1H), 7.37 - 7.31 (m, 1H), 7.27 - 7.24 (m, 1H), 7.08 (dd, J=10.6, 8.7 Hz, 1H), 7.01 - 6.95 (m, 2H), 6.21 (d, J=8.5 Hz, 1H), 5.61 (q, J=7.4 Hz, 1H), 4.83 - 4.74 (m, 1H), 4.04 (s, 3H), 3.41 (br s, 1H), 3.14 (dd, J=10.5, 4.1 Hz, 1H), 2.88 (t, J=3.9 Hz, 1H), 2.30 - 2.22 (m, 1H), 2.01 - 1.95 (m, 1H), 1.73 - 1.62 (m, 2H). LC-MS RT: 1.21 min; MS (ESI) m/z = 836.3 (M+H)+; Method A.

### Example 192

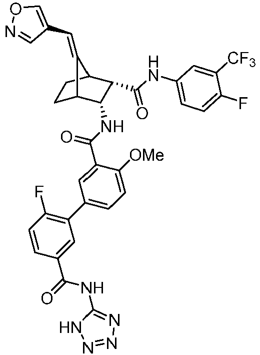

Procedure for **example 192: Example 192** was prepared from **example 120,** employing BHFFT as the coupling reagent. To a 2 dram pressure rated vail charged with **example 120** (0.043 mmol, 1.3 equiv.) was added BHFFT (0.049 mmol, 2.0 equiv.) followed by DCM (1 mL) and DIEA (0.15 mmol, 4.5 equiv.). The reaction mixture was stirred at 23 °C for 30 min, then heated to heated to 80 °C for 18 h. The reaction mixture was allowed to cooled to 23 °C, the vial contents were dissolved in DMF (1.5 mL), and the residue purified by RP-HPLC. Analytical data for **example 192:** LC-MS RT: 2.41 min; MS (ESI) m/z = 735.1 (M+H)+; Method C.

### Example 199

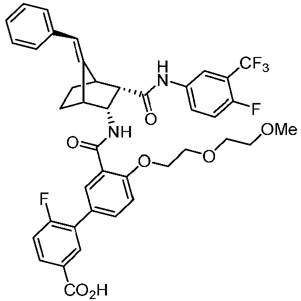

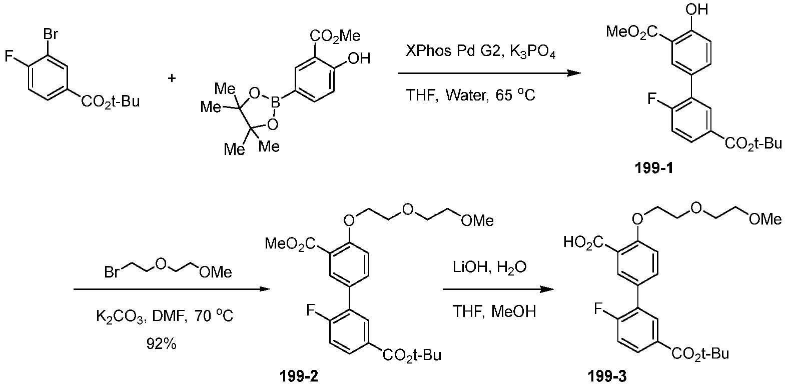

Intermediate **199-1:** Intermediate **199-1** was prepared employing that same conditions that were used for intermediate **140-1,** except at a temperature of 65 °C for 18 h. ¹H NMR (500 MHz, CDCl₃) δ 10.87 (s, 1H), 8.11 - 8.04 (m, 2H), 7.95 (ddd, J=8.5, 4.8, 2.3 Hz, 1H), 7.68 (dt, J=8.5, 1.9 Hz, 1H), 7.18 (dd, J=10.0, 8.7 Hz, 1H), 7.09 (d, J=8.5 Hz, 1H), 3.99 (s, 3H), 1.66 - 1.59 (m, 9H). LC-MS RT = 1.20 min; MS (ESI) m/z = 347.1 (M+H)⁺
Intermediate **199-2:** To a 1 dram vial charged with intermediate **199-1** was added potassium carbonate (53.5 mg, 0.39 mmol), DMF (0.4 mL), and 1-bromo-2-(2-methoxyethoxy)ethane (70.8 mg, 0.39 mmol). The reaction mixture was stirred at 23 °C for 18 h then heated to 40 °C for an additional 18 h. The reaction mixture was concentrated with a stream of nitrogen gas, the residue diluted with ethyl acetate and water and the resulting solution extracted with ethyl acetate (3 x 10 mL). The combined organic portions were dried over sodium sulfate, filtered and concentrated under reduced pressure to give **199-2** (80 mg, 0.18 mmol, 92% yield). ¹H NMR (500 MHz, CDCl₃) δ 8.08 (dd, J=7.7, 2.2 Hz, 1H), 8.00 (dd, J=2.2, 1.1 Hz, 1H), 7.94 (ddd, J=8.5, 4.8, 2.3 Hz, 1H), 7.66 (dt, J=8.7, 2.0 Hz, 1H), 7.17 (dd, J=10.0, 8.7 Hz, 1H), 7.09 (d, J=8.8 Hz, 1H), 4.28 (t, J=5.1 Hz, 2H), 3.97 - 3.93 (m, 2H), 3.91 (s, 3H), 3.81 - 3.77 (m, 2H), 3.61 - 3.57 (m, 2H), 3.44 - 3.39 (m, 3H), 1.61 (s, 9H). LC-MS RT = 1.11 min; MS (ESI) m/z = 449.1 (M+H)⁺; Method A.

Intermediate **199-3:** Intermediate **199-3** was prepared by lithium hydroxide hydrolysis of intermediate **199-2** in a manner similar to intermediate **3-3.** LC-MS RT = 1.02 min; MS (ESI) m/z = 348.1 (M+H)⁺; Method A.

Procedure for **example 199: Example 199** was prepared from **125-2,** employing **199-3,** according to the method described for **example 108.** A subsequent removal of the tert-butyl ester was accomplished as in the procedure to prepare **example 120.** Analytical data for **example 199:** LC-MS RT: 1.15 min; MS (ESI) m/z = 765.2 (M+H)+; Method A.

### Example 201

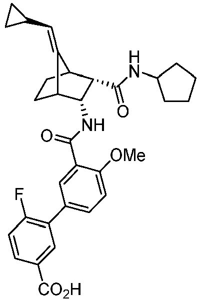

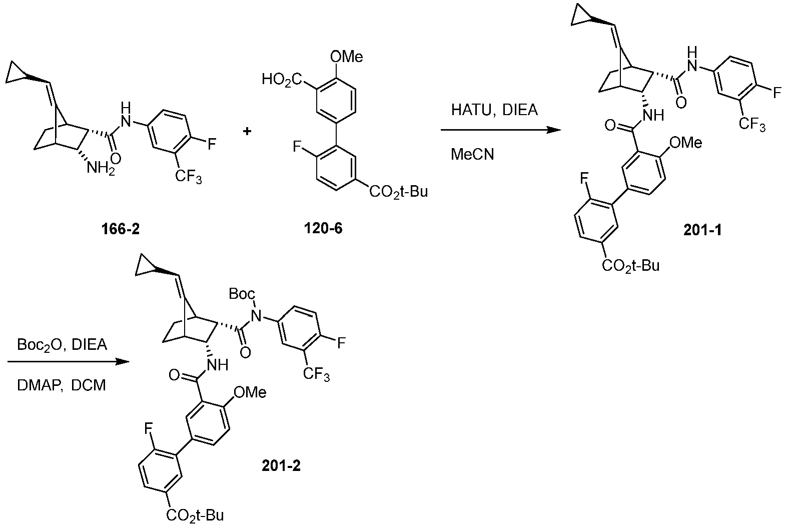

Intermediate **201-1:** Intermediate **201-1** was prepared from **166-2,** employing **120-6** according to the method described for **example 108.** ¹H NMR (500 MHz, CDCl₃) δ 9.40 (br d, J=8.0 Hz, 1H), 8.40 (d, J=1.1 Hz, 1H), 8.08 (dd, J=7.7, 2.2 Hz, 1H), 8.02 - 7.93 (m, 3H), 7.64 (dt, J=8.6, 1.9 Hz, 1H), 7.49 (dt, J=8.6, 3.5 Hz, 1H), 7.18 (dd, J=10.0, 8.7 Hz, 1H), 7.12 - 7.03 (m, 2H), 4.89 - 4.81 (m, 1H), 4.63 (d, J=9.4 Hz, 1H), 4.05 (s, 3H), 3.20 (t, J=3.9 Hz, 1H), 3.10 (dd, J=10.6, 3.2 Hz, 1H), 2.72 (t, J=3.9 Hz, 1H), 2.24 - 2.16 (m, 1H), 1.93 - 1.85 (m, 1H), 1.71 - 1.63 (m, 2H), 1.61 (s, 9H), 1.53 - 1.41 (m, 1H), 0.74 (dt, J=7.8, 3.7 Hz, 2H), 0.41 - 0.30 (m, 2H). LC-MS RT = 1.30 min; MS (ESI) m/z = 697.3 (M+H)⁺; Method A.

Intermediate **201-2:** To a 2 dram vial charged with intermediate **201-1** (88 mg, 0.126 mmol) was added DCM (1.25 mL) followed by Boc₂O (0.51 mmol), DMAP (0.06 mmol), and DIEA (0.51 mmol). The solution was stirred at 23 °C for 18 h and then concentrated under reduced pressure. The resulting crude material was purified by normal phase silica gel chromatography to give intermediate **201-2** (94 mg, 0.12 mmol, 93% yield). LC-MS RT = 1.34 min; MS (ESI) m/z = 797.5 (M+H)⁺; Method A.

Procedure for **example 201: Example 201** was prepared from **201-2.** To a 1 dram vial charged with **201-2** (0.013 mmol) was added DCM (0.3 mL) and cyclopentyl amine (0.125 mmol, 10 equiv.). The solution was stirred at 23 °C for 18 h and concentrated under reduced pressure to give the crude intermediate. A subsequent removal of the tert-butyl ester was accomplished as in the procedure to prepare **example 120.** Analytical data for **example 201:** ¹H NMR (500 MHz, DMSO-d6) δ 9.78 (br d, J=7.0 Hz, 1H), 7.85 - 7.75 (m, 3H), 7.74 - 7.67 (m, 1H), 7.48 (br d, J=8.5 Hz, 1H), 7.16 (br t, J=9.5 Hz, 1H), 7.05 (d, J=8.9 Hz, 1H), 4.36 (d, J=9.5 Hz, 1H), 4.04 (dt, J=10.0, 5.2 Hz, 1H), 3.81 - 3.73 (m, 4H), 2.83 - 2.76 (m, 1H), 2.66 - 2.60 (m, 1H), 1.69 - 1.45 (m, 4H), 1.40 - 1.29 (m, 2H), 1.28 - 1.16 (m, 3H), 1.15 - 1.01 (m, 4H), 0.53 - 0.39 (m, 2H), 0.06 (br d, J=3.1 Hz, 2H). LC-MS RT: 2.33 min; MS (ESI) m/z = 547.4 (M+H)+; Method C.

### Example 206

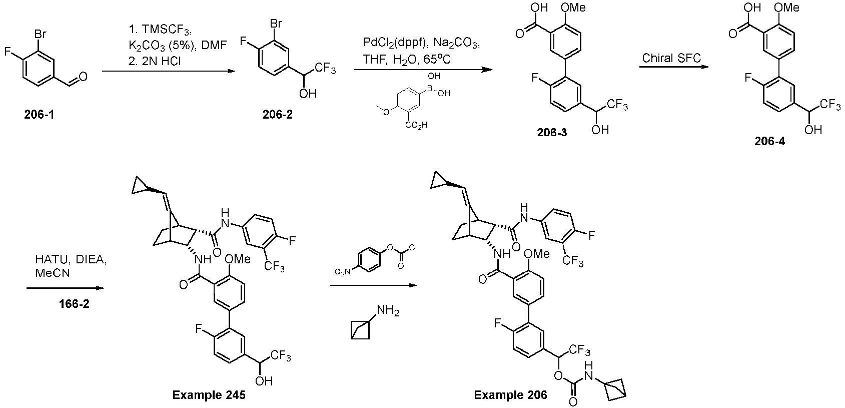

Intermediate **206-2:** Into the reaction vessel was added 3-bromo-4-fluorobenzaldehyde (**206-1**, 235 mg, 1.15 mmol), DMF (3.5 mL), (trifluoromethyl)trimethylsilane (0.34 mL, 2.3 mmol), and K₂CO₃ (8.0 mg, 0.058 mmol). The reaction mixture was stirred at rt for 60 min and 2N HCl (3 mL) was added. After stirring at rt for an additional 1h, the reaction mixture was diluted with EtOAc (15 mL), and the solution washed with sat NH₄Cl. The aqueous phase was extracted with addition al EtOAc (10 mL X2). The combined organic portions were dried over Na₂SO₄, filtered, concentrated, and purified by silica gel chromatography to produce **206-2** (205 mg, 0.751 mmol, 64.9 % yield). ¹H NMR (500 MHz, CDCl₃) d 7.74 (dd, *J*=6.5, 2.1 Hz, 1H), 7.43 (ddd, *J*=8.4, 4.8, 2.2 Hz, 1H), 7.19 (t, *J*=8.4 Hz, 1H), 5.11 - 4.98 (m, 1H), 2.69 (d, *J*=4.4 Hz, 1H).

Intermediate **206-3:** Into the reaction vessel containing **206-2** (100 mg, 0.366 mmol) was added 5-borono-2-methoxybenzoic acid (93 mg, 0.48 mmol), PdCl₂(dppf)-CH₂Cl₂ adduct (45 mg, 0.055 mmol), Na₂CO₃ (155 mg, 1.46 mmol), and H₂O (1 mL). The reaction mixture was degassed by bubbling N₂ for 10 min, sealed, and stirred at 65 °C for 3h. After allowing to cool to rt, the reaction mixture was quenched by the addition of 1N HCl, the solution extracted with EtOAc, dried over Na₂SO₄, filtered, concentrated and purification by HPLC to produce **206-3** (50.5 mg, 0.147 mmol, 40.1 % yield). ¹H NMR (500 MHz, CDCl₃) δ 8.39 (d, *J*=1.9 Hz, 1H), 7.83 (dt, *J*=8.7, 2.1 Hz, 1H), 7.59 (dd, *J*=7.3, 2.1 Hz, 1H), 7.53 - 7.45 (m, 1H), 7.23 (dd, *J*=10.2, 8.8 Hz, 1H), 7.18 (d, *J*=8.5 Hz, 1H), 5.11 (q, *J*=6.6 Hz, 1H), 4.17 (s, 3H).

Intermediate **206-4:** Racemic **206-3** was separated into individual enantiomers using chiral SFC. Preparative chromatographic conditions: Instrument: Berger MG II; Column: Kromasil 5-CelluCoat, 21 x 250 mm, 5 micron; Mobile phase: 15% IPA-ACN (0.1% DEA) / 85% CO₂; Flow conditions; 45 mL/min, 120 Bar, 40 °C; Detector wavelength: 220 nm; Injection details: 0.4 mL of ~15 mg/mL in ACN-IPA (1:1). Peak #2 was collected to afford intermediate **206-4.** Analytical chromatographic conditions: Instrument: Aurora Infinity Analytical SFC; Column: Kromasil 5-CelluCoat, 4.6 x 250 mm, 5 micron; Mobile phase: 20% IPA-ACN (0.1% DEA) / 80% CO₂; Flow conditions: 2 mL/min, 150 Bar, 40 °C; Detector wavelength: 220 nm. Peak 1, RT = 9.12 min, 99% ee; Peak 2, RT = 10.19 min, 98% ee.

Example **245:** Into the reaction vessel was added intermediate **166-2** (7.0 mg, 0.017 mm-ol), intermediate **206-4** (6.2 mg, 0.018 mmol), MeCN (1 mL), DIEA (9.1 µl, 0.052 mmol), and HATU (7.2 mg, 0.019 mmol). The reaction mixture was stirred at rt for 12h, concentrated under reduced pressure, and subjected to prep-HPLC purification to produce example **245** (9.5 mg, 0.014 mmol, 78 % yield). ¹H NMR (500 MHz, CDCl₃) δ 9.57 (d, *J*=7.7 Hz, 1H), 8.33 (dd, *J*=2.2, 0.8 Hz, 1H), 8.05 (s, 1H), 7.99 (dd, *J*=6.3, 2.5 Hz, 1H), 7.66 (dt, *J*=8.7, 2.0 Hz, 1H), 7.57 (dd, *J*=7.3, 2.1 Hz, 1H), 7.56 - 7.52 (m, 1H), 7.45 - 7.40 (m, 1H), 7.17 (dd, *J*=10.2, 8.5 Hz, 1H), 7.11 - 7.04 (m, 2H), 5.11 - 5.04 (m, 1H), 4.77 - 4.70 (m, 1H), 4.57 (d, *J*=9.4 Hz, 1H), 4.06 (s, 3H), 3.42 (br s, 1H), 3.19 (t, *J*=4.1 Hz, 1H), 3.08 (ddd, *J*=10.7, 4.1, 1.2 Hz, 1H), 2.67 (t, *J*=4.0 Hz, 1H), 2.18 - 2.07 (m, 1H), 1.92 - 1.82 (m, 1H), 1.67 - 1.58 (m, 2H), 1.53 - 1.45 (m, 1H), 0.79 - 0.68 (m, 2H), 0.38 - 0.27 (m, 2H). LC-MS RT: 1.38 min; MS (ESI) *m*/*z* 695.3 (M+H)⁺; Method A.

Example **246:** Prepared from intermediate **166-2** and the enantiomer of **206-4** (peak 1 from chiral SFC purification) following the procedure for the synthesis of example **246.**

¹H NMR (500 MHz, CDCl₃) δ 9.53 (d, *J*=7.7 Hz, 1H), 8.34 (dd, *J*=2.5, 0.8 Hz, 1H), 8.01 (s, 1H), 7.97 (dd, *J*=6.2, 2.6 Hz, 1H), 7.66 (dt, *J*=8.7, 2.0 Hz, 1H), 7.57 - 7.50 (m, 2H), 7.48 - 7.40 (m, 1H), 7.18 (dd, *J*=10.2, 8.5 Hz, 1H), 7.12 - 7.02 (m, 2H), 5.13 - 5.03 (m, 1H), 4.81 - 4.71 (m, 1H), 4.60 (d, *J*=9.6 Hz, 1H), 4.06 (s, 3H), 3.19 (t, *J*=3.7 Hz, 2H), 3.09 (ddd, *J*=10.8, 4.1, 1.1 Hz, 1H), 2.70 (t, *J*=4.0 Hz, 1H), 2.19 - 2.11 (m, 1H), 1.92 - 1.84 (m, 1H), 1.70 - 1.60 (m, 2H), 1.51 - 1.42 (m, 1H), 0.77 - 0.70 (m, 2H), 0.36 - 0.30 (m, 2H). LC-MS RT: 1.38 min; MS (ESI) *m*/*z* 695.3 (M+H)⁺; Method A.

Example **206:** Into the reaction vessel was added example **245** (6.0 mg, 8.6 µmol), DCM (1 mL), pyridine (7.0 µl, 0.086 mmol), 4-nitrophenyl carbonochloridate (8.7 mg, 0.043 mmol), and DMAP (1.0 mg, 8.6 µmol). After stirring at rt for 2 h, bicyclo[1.1.1]pentan-1-amine (7.2 mg, 0.086 mmol) was added. The reaction mixture was stirred at rt for 1h, concentrated under reduced pressure and subjected to prep-HPLC purification to produce 1-(3'-(((1R,2R,3S,4R,Z)-7-(cyclopropylmethylene)-3-((4-fluoro-3-(trifluoromethyl)phenyl)carbamoyl)bicyclo[2.2.1]heptan-2-yl)carbamoyl)-6-fluoro-4'-methoxy-[1,1'-biphenyl]-3-yl)-2,2,2-trifluoroethyl bicyclo[1.1.1]pentan-1-ylcarbamate (example **206**, 3.8 mg, 4.7 µmol, 54 % yield). ¹H NMR (500 MHz, DMSO-d6) *δ* 10.54 (s, 1H), 9.95 (br d, *J*=6.3 Hz, 1H), 8.55 (br s, 1H), 8.24 (br d, *J*=4.2 Hz, 1H), 8.13 (br s, 1H), 7.86 - 7.75 (m, 1H), 7.69 (br t, *J*=9.4 Hz, 2H), 7.58 - 7.38 (m, 3H), 7.33 (d, *J*=8.8 Hz, 1H), 6.43 - 6.30 (m, 1H), 4.69 (d, *J*=9.6 Hz, 1H), 4.51 - 4.41 (m, 1H), 4.06 (s, 3H), 3.16 (br dd, *J*=10.1, 3.8 Hz, 1H), 3.11 (br s, 1H), 2.72 (br s, 1H), 2.39 - 2.34 (m, 1H), 2.02 - 1.89 (m, 6H), 1.88 - 1.77 (m, 2H), 1.54 - 1.47 (m, 1H), 1.45 - 1.36 (m, 2H), 0.79 - 0.69 (m, 2H), 0.35 (br s, 2H). LC-MS RT: 1.27 min; MS (ESI) *m*/*z* 804.5 (M+H)⁺; Method A.

### Example 222

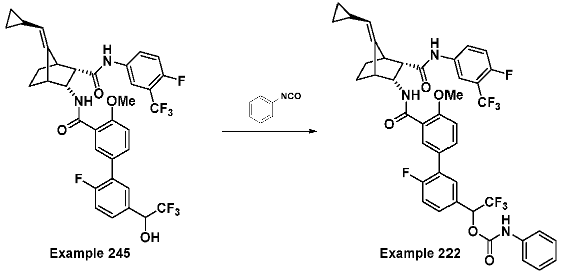

Example **222:** Into the reaction vessel was added example **246** (11 mg, 0.017 mmol), DCM (1 mL), pyridine (8.0 µl, 0.099 mmol), and isocyanatobenzene (9.9 mg, 0.083 mmol). After stirring at rt for 12h, the reaction mixture was concentrated and subjected to prep-HPLC purification to produce 1-(3'-(((1R,2R,3S,4R,Z)-7-(cyclopropylmethylene)-3-((4-fluoro-3-(trifluoromethyl)phenyl)carbamoyl)bicyclo[2.2.1]heptan-2-yl)carbamoyl)-6-fluoro-4'-methoxy-[1,1'-biphenyl]-3-yl)ethyl phenylcarbamate (example **222,** 11.8 mg, 0.0160 mmol, 94.0 % yield). ¹H NMR (500 MHz, DMSO-d6) *δ* 10.52 (s, 1H), 9.92 (br d, *J*=7.3 Hz, 1H), 9.72 (br s, 1H), 8.22 (br d, *J*=4.9 Hz, 1H), 8.14 (s, 1H), 7.81 - 7.75 (m, 1H), 7.70 (br d, *J*=8.2 Hz, 1H), 7.54 (br d, *J*=6.7 Hz, 1H), 7.50 - 7.38 (m, 4H), 7.35 - 7.27 (m, 2H), 7.25 (br t, *J*=7.8 Hz, 2H), 6.96 (t, *J*=7.5 Hz, 1H), 5.89 - 5.80 (m, 1H), 4.69 (d, J=9.5 Hz, 1H), 4.49 - 4.41 (m, 1H), 4.05 (s, 3H), 3.16 (br dd, *J*=10.8, 3.5 Hz, 1H), 3.11 (br s, 1H), 2.72 (br s, 1H), 1.92 - 1.74 (m, 2H), 1.56 (br d, *J*=6.1 Hz, 3H), 1.53 - 1.47 (m, 1H), 1.45 - 1.35 (m, 2H), 0.82 - 0.66 (m, 2H), 0.39 - 0.29 (m, 2H). LC-MS RT: 1.26 min; MS (ESI) *m*/*z* 760.5 (M+H)⁺; Method A.

### Example 230

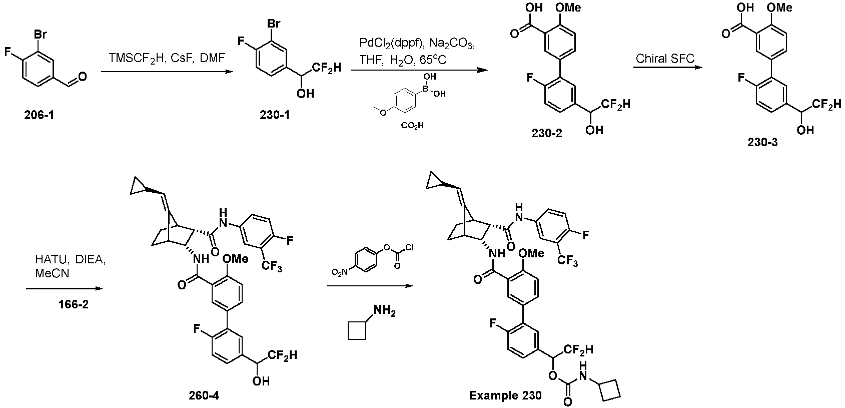

Intermediate **230-1:** Into the reaction vessel was added **206-1** (577 mg, 2.84 mmol), DMF (15 mL), (difluoromethyl)trimethylsilane (530 mg, 4.26 mmol), and CsF (216 mg, 1.42 mmol). After stirring at 50 °C for 12h, the reaction mixture was diluted with EtOAc (15 mL), and the solution washed with sat NH₄Cl. The aqueous phase was extracted with additional EtOAc (10 mLx2). The combined organic portions were dried over Na₂SO₄, filtered, concentrated, and purified by silica gel chromatography to produce 1-(3-bromo-4-fluorophenyl)-2,2-difluoroethan-1-ol (**230-1**, 98 mg, 0.38 mmol, 13 % yield). ¹H NMR (500 MHz, CDCl₃) δ 7.67 (dd, *J*=6.6, 2.1 Hz, 1H), 7.36 (ddd, *J*=8.4, 4.6, 2.1 Hz, 1H), 7.16 (t, *J*=8.4 Hz, 1H), 5.87 - 5.57 (m, 1H), 4.86 - 4.78 (m, 1H), 2.50 (br s, 1H). Intermediate **230-2:** Into the reaction vessel containing **230-1** (220 mg, 0.863 mmol) was added 5-borono-2-methoxybenzoic acid (220 mg, 1.12 mmol), PdCl₂(dppf)-CH₂Cl₂ adduct (106 mg, 0.129 mmol), Na₂CO₃ (366 mg, 3.45 mmol), and H₂O (3.5 mL). The reaction mixture was degassed by bubbling N₂ for 10 min, sealed, and stirred at 65 °C for 3h. After allowing to cool to rt, the reaction mixture was quenched by the addition of 1N HCl, the resulting solution extracted with EtOAc, dried over Na₂SO₄, filtered, concentrated and subjected to prep-HPLC purification to produce 5'-(2,2-difluoro-1-hydroxyethyl)-2'-fluoro-4-methoxy-[1,1'-biphenyl]-3-carboxylic acid (**230-2**, 186 mg, 0.570 mmol, 66.1 % yield). ¹H NMR (400 MHz, CDCl₃) δ 8.37 (d, *J*=1.8 Hz, 1H), 7.82 (dt, *J*=8.7, 2.0 Hz, 1H), 7.53 (dd, *J*=7.4, 2.1 Hz, 1H), 7.45 - 7.39 (m, 1H), 7.24 - 7.14 (m, 2H), 6.01 - 5.59 (m, 1H), 4.89 (td, *J*=10.1, 4.7 Hz, 1H), 4.15 (s, 3H).

Intermediate **230-3:** Racemic **230-2** was separated into individual enantiomers using chiral SFC. Preparative chromatographic conditions: Instrument: PIC Solution SFC Prep-200; Column: Chiralpak IC, 30 x 250 mm, 5 micron; Mobile phase: 10 % MeOH / 90% CO₂; Flow conditions; 85 mL/min, 150 Bar, 40 °C; Detector wavelength: 220 nm; Injection details: 10µL of ~1mg/mL in MeOH. Peak #2 was collected to afford intermediate **230-3.** Analytical chromatographic conditions: Instrument: Aurora Infinity Analytical SFC; Column: Chiralpak ID, 4.6 x 250 mm, 5 micron; Mobile phase: 10%MeOH / 90% CO₂; Flow conditions: 2 mL/min, 150 Bar, 40 °C; Detector wavelength: 220 nm. Peak 1, RT = 11.85 min, 96% ee; Peak 2, RT = 13.65 min, >99.5% ee.

Intermediate **230-4:** Into the reaction vessel was added intermediate **166-2** (20 mg, 0.054 mmol), intermediate **230-3** (18 mg, 0.057 mmol), MeCN (1 mL), DIEA (0.028 mL, 0.16 mmol), and HATU (23 mg, 0.060 mmol). The reaction mixture was stirred at rt for 12h, concentrated under reduced pressure, and subjected to silica gel chromatography purification to produce (1R,2S,3R,4R,Z)-7-(cyclopropylmethylene)-3-(5'-(2,2-difluoro-1-hydroxyethyl)-2'-fluoro-4-methoxy-[1,1'-biphenyl]-3-carboxamido)-N-(4-fluoro-3-(trifluoromethyl)phenyl)bicyclo[2.2.1]heptane-2-carboxamide (**230-4**, 25 mg, 0.037 mmol, 68 % yield). ¹H NMR (400 MHz, CDCl₃) δ 9.49 (br d, *J*=7.7 Hz, 1H), 8.40 - 8.33 (m, 1H), 8.01 (s, 1H), 7.98 - 7.91 (m, 1H), 7.66 (dt, *J*=8.7, 2.0 Hz, 1H), 7.57 - 7.48 (m, 2H), 7.38 (dq, *J*=6.4, 4.2 Hz, 1H), 7.18 (ddd, *J*=10.2, 8.6, 1.2 Hz, 1H), 7.11 - 7.01 (m, 2H), 6.03 - 5.59 (m, 1H), 4.91 - 4.83 (m, 1H), 4.81 - 4.73 (m, 1H), 4.61 (d, *J*=9.5 Hz, 1H), 4.06 (s, 3H), 3.19 (t, *J*=3.7 Hz, 1H), 3.09 (dd, *J*=10.8, 3.3 Hz, 1H), 2.82 (br d, *J*=12.5 Hz, 1H), 2.70 (t, *J*=3.9 Hz, 1H), 2.22 - 2.12 (m, 1H), 1.95 - 1.85 (m, 1H), 1.72 - 1.61 (m, 2H), 1.50 - 1.41 (m, 1H), 0.79 - 0.69 (m, 2H), 0.39 - 0.28 (m, 2H).

Example **230:** Into the reaction vessel was added intermediate **230-4** (6.0 mg, 8.9 µmol), DCM (1 mL), pyridine (7.2 µl, 0.089 mmol), 4-nitrophenyl carbonochloridate (8.9 mg, 0.044 mmol), and DMAP (1.1 mg, 8.9 µmol). After stirring at rt for 2h, cyclobutanamine (6.3 mg, 0.089 mmol) was added. The reaction mixture was stirred at rt for 1h, concentrated under reduced pressure and subjected to prep-HPLC purification to produce 1-(3'-(((1R,2R,3S,4R,Z)-7-(cyclopropylmethylene)-3-((4-fluoro-3-(trifluoromethyl)phenyl)carbamoyl)bicyclo[2.2.1]heptan-2-yl)carbamoyl)-6-fluoro-4'-methoxy-[1,1'-biphenyl]-3-yl)-2,2-difluoroethyl cyclobutylcarbamate (example **230, 4.5** mg, 5.8 µmol, 66 % yield). ¹H NMR (500 MHz, DMSO-d6) *δ* 10.55 (s, 1H), 9.94 (br d, *J*=7.2 Hz, 1H), 8.19 (br d, *J*=5.1 Hz, 1H), 8.09 (s, 1H), 7.94 (br d, *J*=7.8 Hz, 1H), 7.80 - 7.71 (m, 1H), 7.68 (br d, *J*=8.8 Hz, 1H), 7.53 (br d, *J*=6.7 Hz, 1H), 7.48 - 7.39 (m, 2H), 7.38 - 7.27 (m, 2H), 6.49 - 6.13 (m, 1H), 5.93 - 5.81 (m, 1H), 4.67 (d, *J*=9.6 Hz, 1H), 4.48 - 4.38 (m, 1H), 4.03 (s, 3H), 3.94 - 3.85 (m, 1H), 3.19 - 3.11 (m, 1H), 3.08 (br s, 1H), 2.70 (br s, 1H), 2.15 - 2.01 (m, 2H), 1.92 - 1.73 (m, 4H), 1.58 - 1.45 (m, 3H), 1.43 - 1.35 (m, 2H), 0.77 - 0.66 (m, 2H), 0.37 - 0.28 (m, 2H). LC-MS RT: 1.22 min; MS (ESI) *m*/*z* 774.3 (M+H)⁺; Method A.

### Example 233

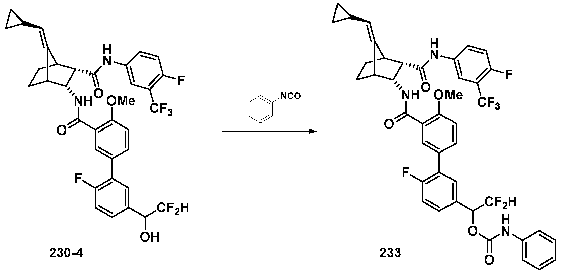

Example **233:** Into the reaction vessel was added **230-4** (6.0 mg, 8.9 µmol), DCM (1 mL), pyridine (0.014 mL, 0.17 mmol), and isocyanatobenzene (5.3 mg, 0.044 mmol). After stirring at rt for 12h, the mixture mixture was concentrated under reduced pressure and subjected to prep-HPLC purification to produce 1-(3'-(((1R,2R,3S,4R,Z)-7-(cyclopropylmethylene)-3-((4-fluoro-3-(trifluoromethyl)phenyl)carbamoyl)bicyclo[2.2.1]heptan-2-yl)carbamoyl)-6-fluoro-4'-methoxy-[1,1'-biphenyl]-3-yl)-2,2-difluoroethyl phenylcarbamate (example **233, 4.9** mg, 5.9 µmol, 67 % yield). ¹H NMR (500 MHz, DMSO-d6) *δ* 10.54 (s, 1H), 10.07 (br s, 1H), 9.93 (br d, *J*=7.0 Hz, 1H), 8.17 (br d, *J*=4.6 Hz, 1H), 8.10 (s, 1H), 7.79 - 7.59 (m, 3H), 7.50 (br s, 1H), 7.47 - 7.34 (m, 4H), 7.34 - 7.24 (m, 3H), 7.01 (br t, *J*=7.2 Hz, 1H), 6.55 - 6.25 (m, 1H), 6.08 - 5.98 (m, 1H), 4.68 (d, *J*=9.5 Hz, 1H), 4.48 - 4.39 (m, 1H), 4.02 (s, 3H), 3.19 - 3.10 (m, 1H), 3.08 (br s, 1H), 2.72 - 2.67 (m, 1H), 1.87 - 1.72 (m, 2H), 1.53 - 1.45 (m, 1H), 1.44 - 1.34 (m, 2H), 0.77 - 0.65 (m, 2H), 0.37 - 0.26 (m, 2H). LC-MS RT: 1.23 min; MS (ESI) *m*/*z* 796.2 (M+H)⁺; Method A.

### Example 238:

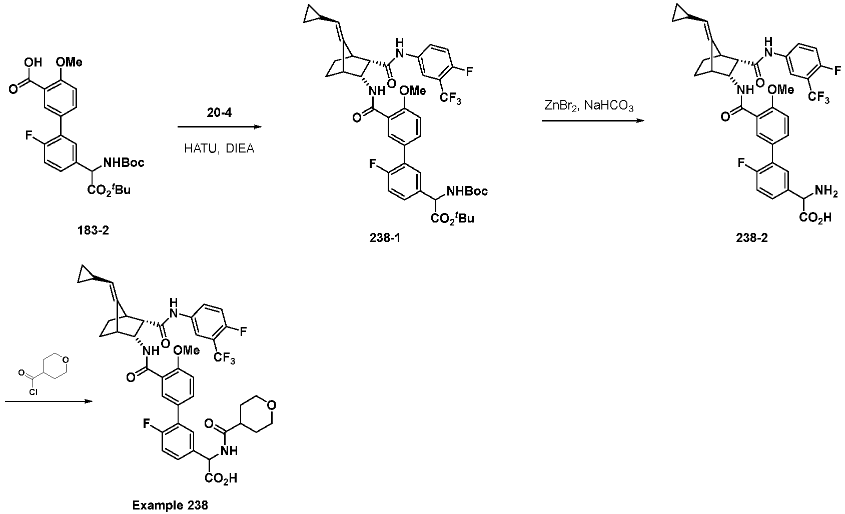

Intermediate **238-1:** Into the reaction vessel was added **166-2** (75 mg, 0.19 mmol), **183-2** (92 mg, 0.20 mmol), MeCN (5 mL), DIEA (0.097 mL, 0.56 mmol), and HATU (77 mg, 0.200 mmol). The reaction mixture was stirred at rt for 12h, concentrated under reduced pressure, and the residue subjected to silica gel chromatography purification to produce tert-butyl 2-((tert-butoxycarbonyl)amino)-2-(3'-(((1R,2R,3S,4R,Z)-7-(cyclopropylmethylene)-3-((4-fluoro-3-(trifluoromethyl)phenyl)carbamoyl)bicyclo[2.2.1]heptan-2-yl)carbamoyl)-6-fluoro-4'-methoxy-[1,1'-biphenyl]-3-yl)acetate (**238-1,** 147 mg, 0.178 mmol, 96.0 % yield). ¹H NMR (400 MHz, CDCl₃) δ 9.42 (br d, *J*=7.9 Hz, 1H), 8.41 (dd, *J*=2.2, 1.3 Hz, 1H), 8.09 - 8.04 (m, 1H), 8.06 (s, 1H), 8.00 (dd, *J*=6.3, 2.5 Hz, 1H), 7.67 - 7.61 (m, 1H), 7.55 - 7.48 (m, 1H), 7.44 (dd, *J*=7.3, 2.4 Hz, 1H), 7.36 - 7.31 (m, 1H), 7.20 - 7.04 (m, 3H), 5.66 (br d, *J*=6.6 Hz, 1H), 5.24 (br d, *J*=7.0 Hz, 1H), 4.92 - 4.82 (m, 1H), 4.65 (d, *J*=9.5 Hz, 1H), 4.07 (s, 3H), 3.22 (t, *J*=3.9 Hz, 1H), 3.13 (dd, *J*=10.5, 3.6 Hz, 1H), 2.74 (t, *J*=3.7 Hz, 1H), 2.27 - 2.16 (m, 1H), 1.95 - 1.86 (m, 1H), 1.74 - 1.66 (m, 2H), 1.46 (br s, 9H), 1.43 (s, 9H), 1.39 - 1.34 (m, 1H), 0.81 - 0.72 (m, 2H), 0.43 - 0.33 (m, 2H).

Intermediate **238-2:** Into the reaction vessel was added **238-1** (147 mg, 0.178 mmol), DCM (10 mL), sodium bicarbonate (112 mg, 1.33 mmol) and zinc bromide (1200 mg, 5.34 mmol). After stirring at for 24h, the reaction mixture was quenched by the addition of 1N HCl and the solution extracted with EtOAc. The combined organic portion was dried over Na₂SO₄, filtered, concentrated, and subjected to prep-HPLC purification to produce 2-amino-2-(3'-(((1R,2R,3S,4R,Z)-7-(cyclopropylmethylene)-3-((4-fluoro-3-(trifluoromethyl)phenyl)carbamoyl)bicyclo[2.2.1]heptan-2-yl)carbamoyl)-6-fluoro-4'-methoxy-[1,1'-biphenyl]-3-yl)acetic acid, TFA (**238-2**, 62 mg, 0.079 mmol, 44 % yield). MS (ESI) *m*/*z* 670.4 (M+H).

Example **238:** Into the reaction vessel was added **238-2** (9 mg, 0.01 mmol), MeCN (1 mL), pyridine (2.8 µl, 0.034 mmol), and tetrahydro-2*H*-pyran-4-carbonyl chloride (1.7 mg, 0.012 mmol) were added. After stirring at rt for 30min, the reaction mixture was quenched by the addition of MeOH, concentrated under reduced pressure, and the residue subjected to prep-HPLC purification to produce 2-(3'-(((1R,2R,3S,4R,Z)-7-(cyclopropylmethylene)-3-((4-fluoro-3-(trifluoromethyl)phenyl)carbamoyl)bicyclo[2.2.1]heptan-2-yl)carbamoyl)-6-fluoro-4'-methoxy-[1,1'-biphenyl]-3-yl)-2-(tetrahydro-2H-pyran-4-carboxamido)acetic acid (example **238, 8.9** mg, 0.011 mmol, 99 % yield). ¹H NMR (500 MHz, CDCl₃) δ 9.89 (br d, *J*=7.7 Hz, 1H), 8.26 (d, *J*=2.2 Hz, 1H), 7.99 (dd, *J*=6.2, 2.3 Hz, 1H), 7.84 (s, 1H), 7.74 - 7.62 (m, 2H), 7.54 (dd, *J*=7.3, 2.3 Hz, 1H), 7.49 - 7.42 (m, 1H), 7.37 - 7.31 (m, 1H), 7.12 - 7.05 (m, 1H), 7.02 - 6.94 (m, 2H), 5.80 (d, *J*=8.0 Hz, 1H), 4.77 - 4.69 (m, 1H), 4.64 (d, *J*=9.4 Hz, 1H), 4.06 (s, 3H), 4.03 - 3.90 (m, 2H), 3.49 - 3.36 (m, 2H), 3.14 - 3.09 (m, 1H), 3.06 (dd, *J*=10.6, 4.0 Hz, 1H), 2.73 - 2.66 (m, 1H), 2.57 - 2.48 (m, 1H), 2.16 - 2.10 (m, 1H), 1.92 - 1.84 (m, 2H), 1.82 - 1.74 (m, 4H), 1.67 - 1.54 (m, 2H), 1.54 - 1.46 (m, 1H), 0.86 - 0.74 (m, 2H), 0.41 - 0.32 (m, 2H). LC-MS RT: 1.26 min; MS (ESI) *m*/*z* 782.5 (M+H)⁺; Method A.

### Example 249:

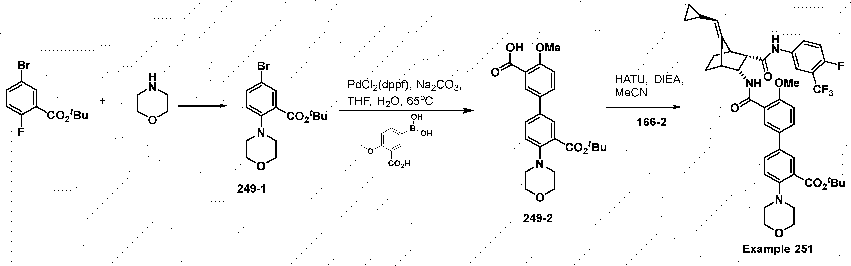

Intermediate **249-1:** Into the reaction vessel was added tert-butyl 5-bromo-2-fluorobenzoate (120 mg, 0.436 mmol), morpholine (0.19 mL, 2.2 mmol), and toluene (2 mL). After stirring at 90 °C for 12h, the reaction mixture was concentrated under reduced pressure and the residue subjected to silica gel chromatography purification to produce tert-butyl 5-bromo-2-morpholinobenzoate (**249-1,** 117 mg, 0.342 mmol, 78.0 % yield). ¹H NMR (400 MHz, CDCl₃) δ 7.69 (d, *J*=2.4 Hz, 1H), 7.49 (dd, *J*=8.7, 2.5 Hz, 1H), 6.91 (d, *J*=8.8 Hz, 1H), 3.89 - 3.85 (m, 4H), 3.07 - 3.03 (m, 4H), 1.62 (s, 9H).

Intermediate **249-2:** Into the reaction vessel containing **249-1(30** mg, 0.088 mmol) was added 5-borono-2-methoxybenzoic acid (25.8 mg, 0.131 mmol), PdCl₂(dppf)-CH₂Cl₂ adduct (14 mg, 0.018 mmol), and Na₂CO₃ (46 mg, 0.44 mmol). The reaction mixture was degassed by bubbling N₂ for 10 min, sealed, and stirred at 65°C for 2h. After cooling to rt, the reaction mixture was concentrated under reduced pressure and the residue subjected to prep-HPLC purification to produce 3'-(tert-butoxycarbonyl)-4-methoxy-4'-morpholino-[1,1'-biphenyl]-3-carboxylic acid (**249-2,** 40 mg, 0.097 mmol, 110 % yield,). MS (ESI) *m*/*z* 414.0 (M+H).

Example **251:** Into the reaction vessel was added intermediate **166-2** (15 mg, 0.037 mmol), **249-2** (20 mg, 0.048 mmol), MeCN (1 mL), DIEA (0.02 mL, 0.1 mmol), and HATU (18 mg, 0.048 mmol). The reaction mixture was stirred at rt for 12h, concentrated under reduced pressure, and the residue subjected to silica gel chromatography purification to produce tert-butyl 3'-(((1R,2R,3S,4R,Z)-7-(cyclopropylmethylene)-3-((4-fluoro-3-(trifluoromethyl)phenyl)carbamoyl)bicyclo[2.2.1]heptan-2-yl)carbamoyl)-4'-methoxy-4-morpholino-[1,1'-biphenyl]-3-carboxylate (example **251**,12 mg, 0.016 mmol, 42 % yield). ¹H NMR (500 MHz, CDCl₃) δ 9.87 (br d, *J*=7.7 Hz, 1H), 8.31 (d, *J*=2.2 Hz, 1H), 8.22 (s, 1H), 8.04 (dd, *J*=6.3, 2.5 Hz, 1H), 7.94 (d, *J*=2.2 Hz, 1H), 7.76 (br dd, *J*=8.3, 1.9 Hz, 1H), 7.60 (dd, *J*=8.7, 2.3 Hz, 1H), 7.56 (dt, *J*=8.7, 3.4 Hz, 1H), 7.47 (br d, *J*=8.0 Hz, 1H), 7.11 (t, *J*=9.4 Hz, 1H), 7.03 (d, *J*=8.8 Hz, 1H), 4.62 (d, *J*=9.6 Hz, 2H), 4.07 (s, 3H), 4.07 - 4.04 (m, 4H), 3.50 - 3.38 (m, 4H), 3.17 (t, *J*=3.9 Hz, 1H), 2.97 (dd, *J*=10.7, 3.9 Hz, 1H), 2.69 (t, *J*=3.9 Hz, 1H), 2.12 - 2.05 (m, 1H), 1.90 - 1.81 (m, 1H), 1.62 (s, 9H), 1.61 - 1.54 (m, 2H), 1.50 - 1.43 (m, 1H), 0.78 - 0.69 (m, 2H), 0.37 - 0.28 (m, 2H). LC-MS RT: 1.23 min; MS (ESI) *m*/*z* 764.3 (M+H)⁺; Method A.

Example **249:** Into the reaction vessel was added example **251** (12 mg, 0.016 mmol), CH₂Cl₂ (2 mL), sodium bicarbonate (13.2 mg, 0.157 mmol) and zinc bromide (142 mg, 0.628 mmol). After stirring at 35 °C for 3h, the reaction mixture was quenched by the addition of 1N HCl and the solution extracted with EtOAc. The combined organic portion was dried over Na₂SO₄, filtered, concentrated, and subjected to prep-HPLC purification to produce 3'-(((1R,2R,3S,4R,Z)-7-(cyclopropylmethylene)-3-((4-fluoro-3-(trifluoromethyl)phenyl)carbamoyl)bicyclo[2.2.1]heptan-2-yl)carbamoyl)-4'-methoxy-4-morpholino-[1,1'-biphenyl]-3-carboxylic acid, TFA (example **249**, 5.2 mg, 6.2 µmol, 40 % yield). ¹H NMR (500 MHz, CDCl₃) *δ* 9.71 (br d, *J*=7.7 Hz, 1H), 8.53 (d, *J*=2.2 Hz, 1H), 8.41 (d, *J*=2.5 Hz, 1H), 7.96 (dd, *J*=6.1, 2.5 Hz, 1H), 7.91 - 7.84 (m, 2H), 7.76 (dd, *J*=8.7, 2.6 Hz, 1H), 7.59 (dt, *J*=8.7, 3.5 Hz, 1H), 7.54 (d, *J*=8.3 Hz, 1H), 7.13 (t, *J*=9.4 Hz, 1H), 7.09 (d, *J*=8.8 Hz, 1H), 4.83 - 4.74 (m, 1H), 4.67 (d, *J*=9.6 Hz, 1H), 4.09 (s, 3H), 4.02 (br s, 4H), 3.23 (br t, *J*=4.0 Hz, 1H), 3.17 (br s, 4H), 3.10 (br dd, *J*=10.9, 3.4 Hz, 1H), 2.74 (t, *J*=3.9 Hz, 1H), 2.22 - 2.15 (m, 1H), 1.93 - 1.86 (m, 1H), 1.73 - 1.59 (m, 2H), 1.55 - 1.47 (m, 1H), 0.80 - 0.73 (m, 2H), 0.39 - 0.34 (m, 2H). LC-MS RT: 1.15 min; MS (ESI) *m*/*z* 708.4 (M+H)⁺; Method A.

### Example 253:

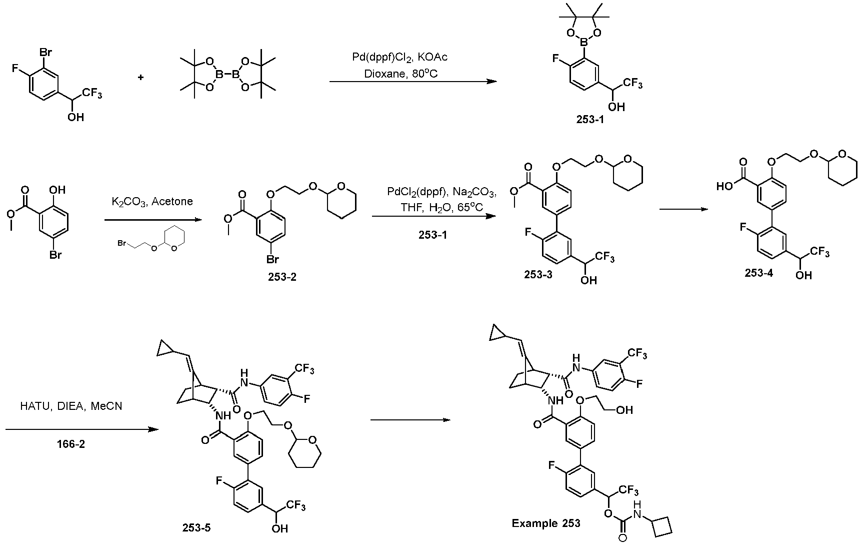

Intermediate **253-1:** Into the reaction vessel was added 1-(3-bromo-4-fluorophenyl)-2,2,2-trifluoroethan-1-ol (100 mg, 0.366 mmol), 4,4,4',4',5,5,5',5'-octamethyl-2,2'-bi(1,3,2-dioxaborolane) (126 mg, 0.494 mmol), and 1,4-dioxane (3 mL). PdCl₂(dppf)-CH₂Cl₂ adduct (29.9 mg, 0.037 mmol) and potassium acetate (90 mg, 0.91 mmol) were subsequently added and the reaction mixture was degassed by bubbling N₂ for 10 min. The reaction mixture was stirred at 65 °C for 5h, allowed to cool to rt and the solution extracted with EtOAc. The combined organic portions were dried over Na₂SO₄, filtered and concentrated. The resulting material (**253-1**)was used for next step without further purification.

Intermediate **253-2:** Into the reaction vessel was added methyl 5-bromo-2-hydroxybenzoate (200 mg, 0.866 mmol), 2-(2-bromoethoxy)tetrahydro-2H-pyran (217 mg, 1.039 mmol), acetone (3 mL), and K₂CO₃ (239 mg, 1.73 mmol). After stirring at 50 °C for 12h, the reaction mixture was concentrated under reduced pressure and the residue subjected to silica gel chromatography purification to produce methyl 5-bromo-2-(2-((tetrahydro-2H-pyran-2-yl)oxy)ethoxy)benzoate (**253-2,** 112 mg, 0.312 mmol, 36.0 % yield). ¹H NMR (500 MHz, CDCl₃) δ 7.90 (d, *J*=2.6 Hz, 1H), 7.55 (dd, *J*=8.9, 2.6 Hz, 1H), 6.94 (d, *J*=8.9 Hz, 1H), 4.76 (t, *J*=3.5 Hz, 1H), 4.30 - 4.16 (m, 2H), 4.08 (dt, *J*=11.5, 4.6 Hz, 1H), 3.94 - 3.84 (m, 5H), 3.59 - 3.52 (m, 1H), 1.88 - 1.79 (m, 1H), 1.79 - 1.71 (m, 1H), 1.67 - 1.60 (m, 2H), 1.58 - 1.50 (m, 2H).

Intermediate **253-3:** Into the reaction vessel containing **253-2** (80 mg, 0.22 mmol) was added **253-1** (93 mg, 0.29 mmol), PdCl₂(dppf)-CH₂Cl₂ adduct (27 mg, 0.033 mmol), Na₂CO₃ (94 mg, 0.89 mmol), and H₂O (0.5 mL). The reaction mixture was degassed by bubbling N₂ for 10 min, sealed, and stirred at 65 °C for 3h. After cooling to rt, the reaction mixture was quenched by the addition of water, and the solution extracted with EtOAc. The combined EtOAc portions were dried over Na₂SO₄, filtered, concentrated and subjected to silica gel chromatography purification to produce methyl 2'-fluoro-4-(2-((tetrahydro-2H-pyran-2-yl)oxy)ethoxy)-5'-(2,2,2-trifluoro-1-hydroxyethyl)-[1,1'-biphenyl]-3-carboxylate (**253-3,** 66 mg, 0.14 mmol, 62 % yield). ¹H NMR (500 MHz, CDCl₃) δ 7.95 (dd, *J*=2.4, 1.0 Hz, 1H), 7.64 (dt, *J*=8.7, 1.8 Hz, 1H), 7.52 (dd, *J*=7.3, 2.1 Hz, 1H), 7.47 - 7.40 (m, 1H), 7.19 (dd, *J*=10.2, 8.5 Hz, 1H), 7.09 (d, *J*=8.7 Hz, 1H), 5.10 - 5.03 (m, 1H), 4.77 (t, *J*=3.5 Hz, 1H), 4.31 - 4.25 (m, 2H), 4.15 - 4.08 (m, 1H), 3.95 - 3.87 (m, 5H), 3.60 - 3.52 (m, 1H), 2.98 (br d, *J*=3.5 Hz, 1H), 1.89 - 1.81 (m, 1H), 1.79 - 1.71 (m, 1H), 1.67 - 1.61 (m, 2H), 1.59 - 1.51 (m, 2H).

Intermediate **253-4: 253-3** (66 mg, 0.14 mmol) was dissolved in THF (4 mL) and a solution of lithium hydroxide monohydrate (31.7 mg, 0.754 mmol) in water (2 mL) was added. The reaction mixture was stirred at rt for 12 h, diluted with EtOAc (10 mL), and quenched by the addition of 1.0 eq of 1N HCl. The organic phase was dried over Na₂SO₄, filtered and concentrated under reduced pressure to yield 2'-fluoro-4-(2-((tetrahydro-2H-pyran-2-yl)oxy)ethoxy)-5'-(2,2,2-trifluoro-1-hydroxyethyl)-[1,1'-biphenyl]-3-carboxylic acid (**253-4,** 64 mg, 0.14 mmol, 100 % yield) which was used for next step without further purification.

Intermediate **253-5:** Into the reaction vessel was added intermediate **166-2** (25 mg, 0.068 mmol), **253-4** (31 mg, 0.068 mmol), MeCN (1 mL), DIEA (0.036 mL, 0.20 mmol), and HATU (28.4 mg, 0.0750 mmol). The reaction mixture was stirred at rt for 12h, concentrated under reduced pressure and the residue subjected to prep-HPLC purification to produce (1R,2S,3R,4R,Z)-7-(cyclopropylmethylene)-N-(4-fluoro-3-(trifluoromethyl)phenyl)-3-(2'-fluoro-4-(2-((tetrahydro-2H-pyran-2-yl)oxy)ethoxy)-5'-(2,2,2-trifluoro-1-hydroxyethyl)-[1,1'-biphenyl]-3-carboxamido)bicyclo[2.2.1]heptane-2-carboxamide (**253-5,** 39 mg, 0.049 mmol, 72 % yield). MS (ESI) *m*/*z* 809.2 (M+H).

Example **253:** Into the reaction vessel was added **253-5** (15 mg, 0.019 mmol), DCM (1 mL), pyridine (0.015 mL, 0.19 mmol), 4-nitrophenyl carbonochloridate (19 mg, 0.093 mmol), and DMAP (2.3 mg, 0.019 mmol). After stirring at rt for 2h, cyclobutanamine (13.2 mg, 0.185 mmol) was added. The reaction mixture was stirred at rt for 1h and concentrated under reduced pressure. The residue was subjected to prep-HPLC purification to produce the corresponding carbamate. This product was not stable due to the presence of TFA. Standing at rt for 12h followed by concentration and prep-HPLC purification produced 1-(3'-(((1R,2R,3S,4R,Z)-7-(cyclopropylmethylene)-3-((4-fluoro-3-(trifluoromethyl)phenyl)carbamoyl)bicyclo[2.2.1]heptan-2-yl)carbamoyl)-6-fluoro-4'-(2-hydroxyethoxy)-[1,1'-biphenyl]-3-yl)-2,2,2-trifluoroethyl cyclobutylcarbamate (example **253,** 11.0 mg, 0.0130 mmol, 70.0 % yield). ¹H NMR (500 MHz, CDCl₃) *δ* 9.54 (br d, *J*=8.5 Hz, 1H), 8.29 (d, *J*=2.0 Hz, 1H), 7.76 - 7.67 (m, 2H), 7.57 - 7.47 (m, 3H), 7.43 - 7.36 (m, 1H), 7.21 - 7.14 (m, 2H), 7.10 (br d, *J*=8.9 Hz, 1H), 6.11 - 6.05 (m, 1H), 5.32 (br d, *J*=8.2 Hz, 1H), 4.93 - 4.85 (m, 1H), 4.69 (d, *J*=9.6 Hz, 1H), 4.49 - 4.43 (m, 1H), 4.32 - 4.24 (m, 2H), 4.17 - 4.09 (m, 2H), 3.17 (t, *J*=4.1 Hz, 1H), 3.13 (dd, *J*=10.5, 3.8 Hz, 1H), 2.75 (t, *J*=4.0 Hz, 1H), 2.42 - 2.24 (m, 2H), 2.20 - 2.14 (m, 1H), 1.98 - 1.85 (m, 3H), 1.80 - 1.61 (m, 4H), 1.53 - 1.46 (m, 1H), 0.82 - 0.73 (m, 2H), 0.40 - 0.33 (m, 2H). LC-MS RT: 1.33 min; MS (ESI) *m*/*z* 822.1 (M+H)⁺; Method A.

### Example 256:

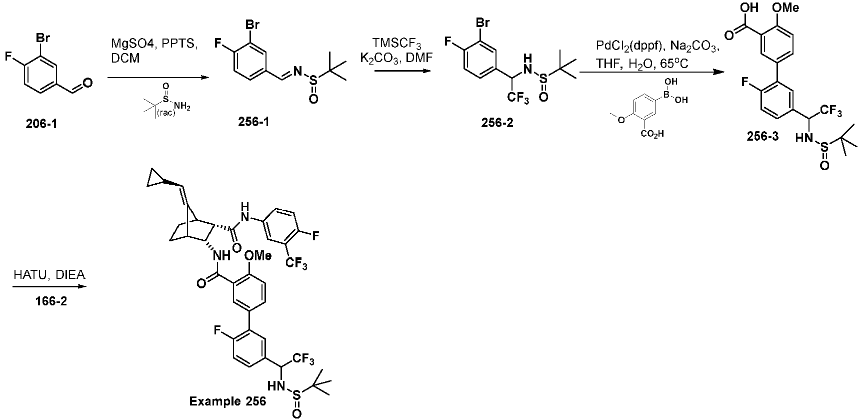

Intermediate **256-1:** Into the reaction vessel was added 3-bromo-4-fluorobenzaldehyde (1670 mg, 8.25 mmol), 2-methylpropane-2-sulfinamide (500. mg, 4.13 mmol), DCM (2 mL), MgSO₄ (2483 mg, 20.63 mmol), and PPTS (52 mg, 0.21 mmol). The reaction mixture was stirred at rt for 24h, loaded to silica cartridge, and subjected to silica gel chromatograph purification to produce (*E*)-N-(3-bromo-4-fluorobenzylidene)-2-methylpropane-2-sulfinamide (**256-1,** 1220 mg, 3.98 mmol, 97 % yield). ¹H NMR (500 MHz, CDCl₃) δ 8.51 (s, 1H), 8.11 (dd, *J*=6.6, 2.2 Hz, 1H), 7.77 (ddd, *J*=8.5, 4.7, 1.9 Hz, 1H), 7.24 (t, *J*=8.4 Hz, 1H), 1.28 (s, 9H).

Intermediate **256-2:** Into the reaction vessel was added **256-1** (200 mg, 0.653 mmol), DMF (3 mL), (trifluoromethyl)trimethylsilane (0.19 mL, 1.3 mmol), and K₂CO₃ (45 mg, 0.33 mmol). The reaction mixture was stirred at rt for 60 min and 2N HCl (15 mL) was added. After string at rt for 1h, the reaction mixture was diluted with EtOAc (30 mL), and the organic portion washed with sat NH₄Cl. The aqueous phase was extracted with addition al EtOAc (10 mLx2). The combined organic portion was dried over Na₂SO₄, concentrated, filtered, and purified by silica gel chromatography to produce N-(1-(3-bromo-4-fluorophenyl)-2,2,2-trifluoroethyl)-2-methylpropane-2-sulfinamide (**256-2,** 163 mg, 0.433 mmol, 66 % yield). ¹H NMR (500 MHz, CDCl₃) δ 7.65 (dd, *J*=6.3, 2.1 Hz, 1H), 7.42 - 7.37 (m, 1H), 7.18 (t, *J*=8.4 Hz, 1H), 4.81 (quin, *J*=7.1 Hz, 1H), 3.58 (br d, *J*=6.6 Hz, 1H), 1.27 (s, 9H).

Intermediate **256-3:** Into the reaction vessel containing **256-2** (50. mg, 0.13 mmol) was added 5-borono-2-methoxybenzoic acid (31 mg, 0.16 mmol), PdCl₂(dppf)-CH₂Cl₂ adduct (16 mg, 0.020 mmol), Na₂CO₃ (56 mg, 0.53 mmol), and H₂O (0.5 mL). The reaction mixture was degassed by bubbling N₂ for 10 min, sealed, and stirred at 65 °C for 3h. After cooling to rt, the reaction mixture was quenched by the addition of 1N HCl, the solution extracted with EtOAc, the combined organic portions dried over Na₂SO₄, filtered, concentrated and subjected to prep-HPLC purification to produce 5'-(1-((tert-butylsulfinyl)amino)-2,2,2-trifluoroethyl)-2'-fluoro-4-methoxy-[1,1'-biphenyl]-3-carboxylic acid (**256-3,** 47 mg, 0.10 mmol, 79 % yield). MS (ESI) *m*/*z* 448.1 (M+H). Example **256:** Into the reaction vessel was added **166-2** (10 mg, 0.027 mmol), **256-3** (12 mg, 0.027 mmol), MeCN (1 mL), DIEA (0.014 mL, 0.081 mmol), and HATU (11 mg, 0.030 mmol). The reaction mixture was stirred at rt for 12h, concentrated under reduced pressure and the residue subjected to prep-HPLC purification to produce (1R,2S,3R,4R,Z)-3-(5'-(1-((tert-butylsulfinyl)amino)-2,2,2-trifluoroethyl)-2'-fluoro-4-methoxy-[1,1'-biphenyl]-3-carboxamido)-7-(cyclopropylmethylene)-N-(4-fluoro-3-(trifluoromethyl)phenyl)bicyclo[2.2.1]heptane-2-carboxamide (example 256, 7.5 mg, 9.3 µmol, 34 % yield). ¹H NMR (500 MHz, DMSO-d6) δ 10.55 (s, 1H), 9.95 (br t, *J*=6.4 Hz, 1H), 8.27 - 8.19 (m, 1H), 8.16 (s, 1H), 7.87 - 7.74 (m, 2H), 7.70 (br d, *J*=8.8 Hz, 1H), 7.66 - 7.58 (m, 1H), 7.48 (br t, *J*=9.7 Hz, 1H), 7.40 - 7.29 (m, 2H), 6.51 (d, *J*=9.6 Hz, 1H), 5.39 - 5.27 (m, 1H), 4.68 (d, *J*=9.7 Hz, 1H), 4.51 - 4.41 (m, 1H), 4.05 (s, 3H), 3.19 - 3.14 (m, 1H), 3.11 (br s, 1H), 1.88 - 1.75 (m, 2H), 1.56 - 1.46 (m, 1H), 1.44 - 1.35 (m, 2H), 1.14 (s, 9H), 0.79 - 0.68 (m, 2H), 0.39 - 0.30 (m, 2H). LC-MS RT: 1.25 min; MS (ESI) *m*/*z* 798.1 (M+H)⁺; Method A.

### Example 258:

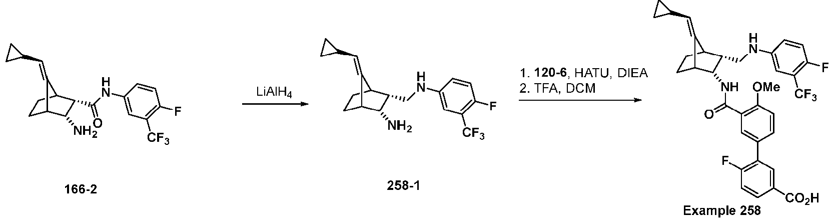

Intermediate **258-1:** Into the reaction vessel was added intermediate **166-2** (15 mg, 0.041 mmol) and THF (1 mL). After cooling to 0 °C, LiAlH₄ (0.5 mL, 0.500 mmol) was added. After stirring at 0 °C for 5min, the reaction mixture was allowed to warm to rt and stir at rt for 20 min. The reaction mixture was diluted with EtOAc. After washing the organic solution with sat NaHCO₃, the organic phase was dried over Na₂SO₄ andoncentrated under reduced pressure to provide (1R,2R,3R,4R,Z)-7-(cyclopropylmethylene)-3-(((4-fluoro-3-(trifluoromethyl)phenyl)amino)methyl)bicyclo[2.2.1]heptan-2-amine **(258-1,** 7.0 mg, 0.020 mmol, 49 % yield). This material was used for next step without further purification. MS (ESI) *m*/*z* 355.3 (M+H).

Example **258:** Into the reaction vessel was added **258-1** (7.0 mg, 0.020 mmol), **120-6** (6.5 mg, 0.019 mmol), MeCN (1 mL), DIEA (9.4 µl, 0.054 mmol), and HATU (7.5 mg, 0.020 mmol). The reaction mixture was stirred at rt for 12h, concentrated under reduced pressure and the residue subjected to silica gel chromatography purification to yield a residue that was treated with 2:1 DCM/TFA at rt for 30min. The resuting solution was concentrated and the residue purified by HPLC to produce 3'-(((1R,2R,3R,4R,Z)-7-(cyclopropylmethylene)-3-(((4-fluoro-3-(trifluoromethyl)phenyl)amino)methyl)bicyclo[2.2.1]heptan-2-yl)carbamoyl)-6-fluoro-4'-methoxy-[1,1'-biphenyl]-3-carboxylic acid, example **258,** 6.5 mg, 8.4 µmol, 47 % yield). ¹H NMR (500 MHz, CDCl₃) *δ* 8.59 (br d, *J*=7.4 Hz, 1H), 8.41 (d, *J*=1.4 Hz, 1H), 8.23 (dd, *J*=7.6, 2.1 Hz, 1H), 8.09 (ddd, *J*=8.5, 4.6, 2.1 Hz, 1H), 7.71 (br d, *J*=8.8 Hz, 1H), 7.26 - 7.22 (m, 1H), 7.08 - 7.02 (m, 2H), 6.99 - 6.94 (m, 2H), 4.65 (d, *J*=9.6 Hz, 1H), 4.63 - 4.57 (m, 1H), 4.03 (s, 3H), 3.32 (dd, *J*=11.4*,* 2.9 Hz, 1H), 3.09 (t, *J=*4.1 Hz, 1H), 3.04 - 2.97 (m, 1H), 2.59 - 2.52 (m, 2H), 1.83 - 1.74 (m, 1H), 1.73 - 1.67 (m, 1H), 1.64 - 1.55 (m, 2H), 1.47 - 1.39 (m, 1H), 0.76 - 0.69 (m, 2H), 0.41 - 0.31 (m, 2H). LC-MS RT: 1.31 min; MS (ESI) *m*/*z* 683.5 (M+H)⁺; Method A.

### Example 259

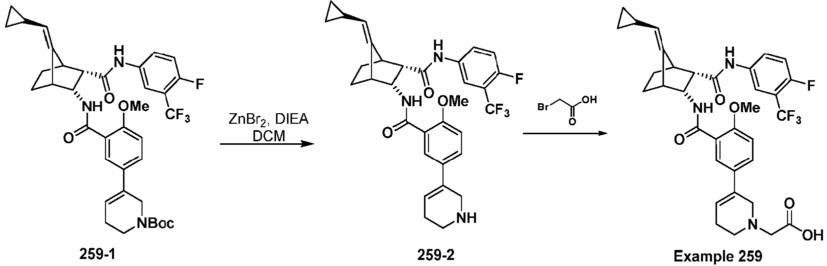

Intermediate **259-1: 259-1** was prepared from intermediate **166-2** and **140-2** following the procedure described for **Example 168.** ¹H NMR (500 MHz, CDCl₃) δ 9.58 - 9.15 (br. s, 1H), 8.20 (d, J=2.2 Hz, 1H), 8.17 - 7.93 (m, 1H), 7.90 (dd, J=6.1, 2.5 Hz, 1H), 7.56 (dt, *J*=8.9, 3.4 Hz, 1H), 7.41 (dd, *J*=8.5, 2.5 Hz, 1H), 7.08 (t, *J*=9.4 Hz, 1H), 6.92 (br d, *J*=7.7 Hz, 1H), 6.16 (dt, *J*=4.0, 2.1 Hz, 1H), 4.87 - 4.79 (m, 1H), 4.63 (d, *J*=9.6 Hz, 1H), 4.32 - 4.18 (m, 2H), 3.99 (s, 3H), 3.55 (br s, 2H), 3.18 (t, *J*=3.7 Hz, 1H), 3.11 - 3.06 (m, 1H), 2.71 (t, *J*=3.7 Hz, 1H), 2.31 (br d, *J*=2.8 Hz, 2H), 2.23 - 2.12 (m, 1H), 1.91 - 1.80 (m, 1H), 1.71 - 1.61 (m, 2H), 1.50 (s, 9H), 1.49 - 1.42 (m, 1H), 0.77 - 0.70 (m, 2H), 0.40 - 0.30 (m, 2H).

Intermediate **259-2:** Into the reaction vessel was added **259-1** (13 mg, 0.019 mmol), DCM (1.5 mL), DIEA (0.012 mL, 0.067 mmol) and zinc bromide (150 mg, 0.665 mmol). After stirring at for 12h, the reaction mixture was quenched with the addition of sat NaHCO₃ and the solution extracted with EtOAc. The combined organic portion was dried over Na₂SO₄ filtered and concentrated to generate (1R,2S,3R,4R,Z)-7-(cyclopropylmethylene)-N-(4-fluoro-3-(trifluoromethyl)phenyl)-3-(2-methoxy-5-(1,2,5,6-tetrahydropyridin-3-yl)benzamido)bicyclo[2.2.1]heptane-2-carboxamide **(259-2,** 12 mg, 0.021 mmol, 110 % yield). This intermediate was used for next step without further purification. MS (ESI) *m*/*z* 584.4 (M+H).

Example **259:** Into the reaction vessel was added **259-2** (11 mg, 0.019 mmol), MeCN (1 mL), 2-bromoacetic acid (1.5 mg, 0.011 mmol), and DIEA (9.9 µl, 0.057 mmol). The reaction mixture was stirred at rt for 1h and concentrated under reduced pressure. Preparative HPLC of the resulting residue, followed by SFC purification produced 2-(5-(3-(((1R,2R,3S,4R,Z)-7-(cyclopropylmethylene)-3-((4-fluoro-3-(trifluoromethyl)phenyl)carbamoyl)bicyclo[2.2.1]heptan-2-yl)carbamoyl)-4-methoxyphenyl)-3,6-dihydropyridin-1(2H)-yl)acetic acid example **259, 4.1** mg, 5.4 µmol, 28 % yield). ¹H NMR (500 MHz, CD₃OD) δ 10.31 (br d, *J*=7.2 Hz, 1H), 10.12 (s, 1H), 8.15 (dd, *J*=6.2, 2.6 Hz, 1H), 8.05 (d, *J*=2.5 Hz, 1H), 7.78 - 7.68 (m, 1H), 7.59 (dd, *J*=8.8, 2.5 Hz, 1H), 7.28 (t, *J*=9.6 Hz, 1H), 7.23 - 7.17 (m, 1H), 6.38 - 6.32 (m, 1H), 4.74 (d, J=9.4 Hz, 1H), 4.60 - 4.52 (m, 1H), 4.25 (br s, 4H), 4.09 (s, 3H), 3.25 - 3.19 (m, 1H), 3.17 - 3.11 (m, 1H), 2.77 - 2.68 (m, 3H), 2.01 - 1.89 (m, 2H), 1.59 - 1.47 (m, 3H), 0.80 - 0.71 (m, 2H), 0.41 - 0.29 (m, 2H). LC-MS RT: 0.94 min; MS (ESI) *m*/*z* 642.3 (M+H)⁺; Method A.

### Example 265

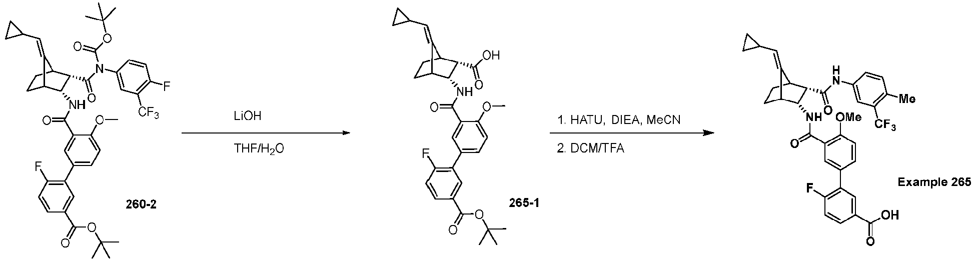

**Intermediate 265-1:** To a vial containing **260-2** (10 mg, 0.013 mmol) in THF (1.3 mL) was added LiOH (63 µl, 0.063 mmol) as a 1M solution in water. The reaction mixture was stirred at room temperature for 18 h, then diluted with 1N HCl. The resulting mixture was extracted with EtOAc (3 x 5 mL). The combined organics were dried over Na₂SO₄ filtered and concentrated to afford (1R,2S,3R,4R,Z)-3-(5'-(tert-butoxycarbonyl)-2'-fluoro-4-methoxy-[1,1'-biphenyl]-3-carboxamido)-7-(cyclopropylmethylene)bicyclo[2.2.1]heptane-2-carboxylic acid which was used without further purification, (7.0 mg, 0.013 mmol, 100 % yield). ¹H-NMR (500 MHz, DMSO-d6) δ 10.05 (br s, 1H), 8.12 (s, 1H), 8.01 - 7.97 (m, 1H), 7.96 - 7.92 (m, 1H), 7.74 (d, J=8.9 Hz, 1H), 7.45 (t, J=9.5 Hz, 1H), 7.33 (d, J=8.9 Hz, 1H), 4.66 (d, J=9.5 Hz, 1H), 4.34 - 4.25 (m, 1H), 4.04 (s, 3H), 3.15 - 3.09 (m, 1H), 2.99 (dd, J=10.8, 3.8 Hz, 1H), 2.68 - 2.61 (m, 1H), 1.76 - 1.63 (m, 2H), 1.56 (s, 9H), 1.47 (dt, J=8.7, 4.2 Hz, 1H), 1.42 (s, 2H), 0.84 - 0.60 (m, 2H), 0.44 - 0.23 (m, 2H). LC-MS RT: 1.17 min; MS (ESI) *m*/*z* 536 (M+H)⁺; Method D.

**Example 265:** Into the reaction vessel was added **265-1** (4.0 mg, 0.022 mmol), MeCN (1 mL), DIEA (10 µl, 0.060 mmol), and HATU (6.8 mg, 0.018 mmol). The reaction mixture was stirred at room temperature for 12h then concentrated under reduced pressure and the residue dissolved in 1:2 TFA/DCM and stirred for 30 min. The reaction mixture was concentrated under reduced pressure, dissolved in DMSO and purified by HPLC to afford 3'-(((1R,2R,3S,4R,Z)-7-(cyclopropylmethylene)-3-((4-methyl-3-(trifluoromethyl)phenyl)carbamoyl)bicyclo[2.2.1]heptan-2-yl)carbamoyl)-6-fluoro-4'-methoxy-[1,1'-biphenyl]-3-carboxylic acid (3.4 mg, 5.3 µmol, 35 % yield). ¹H-NMR (500 MHz, DMSO-d6) δ 10.41 (s, 1H), 9.98 (d, J=6.7 Hz, 1H), 8.21 - 8.09 (m, 2H), 8.05 - 7.99 (m, 1H), 7.99 - 7.90 (m, 1H), 7.77 - 7.70 (m, 1H), 7.64 (dd, J=7.8, 1.1 Hz, 1H), 7.45 - 7.36 (m, 2H), 7.33 (d, J=8.5 Hz, 1H), 4.69 (d, J=9.5 Hz, 1H), 4.55 - 4.36 (m, 1H), 4.06 (s, 3H), 3.19 - 3.13 (m, 1H), 3.13 - 3.08 (m, 1H), 2.78 - 2.66 (m, 1H), 2.37 (s, 3H), 1.90 - 1.84 (m, 1H), 1.83 - 1.76 (m, 1H), 1.55 - 1.47 (m, 1H), 1.47 - 1.37 (m, 2H), 0.84 - 0.60 (m, 2H), 0.42 - 0.23 (m, 2H). LC-MS RT: 2.21 min; MS (ESI) *m*/*z* 653 (M+H)⁺; Method A.

### Example 310

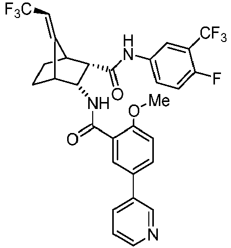

### Intermediate 310-1

A solution of 5-borono-2-methoxybenzoic acid (0.200 g, 1.02 mmol) in EtOAc (10ml) was treated with pinacol (0.121 g, 1.02 mmol) and the resulting solution stirred at rt overnight. The reaction mixture was then concentrated and the resulting solid used without further manipulation as 2-methoxy-5-(4,4,5,5-tetramethyl-1,3,2-dioxaborolan-2-yl)benzoic acid (0.284 g, 1.02 mmol, 100 % yield). This solid was coupled to intermediate **170-2** according to the same procedure as **Example 108** to furnish intermediate **310-1.**

The reaction mixture of **310-1** (50 mg, 0.076 mmol), PdCl₂(dppf) (5.6 mg, 7.6 µmol), 3-bromopyridine (0.1 mL) and K₃PO₄ (48.5 mg, 0.229 mmol) was heated to 80 °C. The reaction mixture was cooled to rt, and partitioned between water and EtOAc. The organic layer was concentrated and the residue purified by reverse phase HPLC to furnish (1R,2S,3R,4R,Z)-N-(4-fluoro-3-(trifluoromethyl)phenyl)-3-(2-methoxy-5-(pyridin-3-yl)benzamido)-7-(2,2,2-trifluoroethylidene)bicyclo[2.2.1]heptane-2-carboxamide (11.4 mg, 0.019 mmol, 24 % yield). ¹H NMR (500 MHz, DMSO-d6) δ 10.68 (s, 1H), 9.99 (br d, J=6.8 Hz, 1H), 8.85 (s, 1H), 8.55 (br d, J=3.4 Hz, 1H), 8.24 (br d, J=2.3 Hz, 2H), 8.07 - 8.00 (m, 1H), 7.90 (dd, J=8.6, 2.4 Hz, 1H), 7.83 - 7.73 (m, 1H), 7.56 - 7.45 (m, 2H), 7.34 (d, J=8.8 Hz, 1H), 6.05 - 5.92 (m, 1H), 4.60 - 4.51 (m, 1H), 4.06 (s, 3H), 3.47 (s, 1H), 3.00 (br s, 1H), 2.74 (s, 1H), 2.02 - 1.95 (m, 1H), 1.94 - 1.87 (m, 1H), 1.51 (br d, J=6.6 Hz, 2H). LC-MS RT 2.47 min; MS (ESI) m/z = 608.3 (M+H)+; Method C.

**Example 320** was prepared analoglously to **Example 253** via the following intermediates.

### Example 320

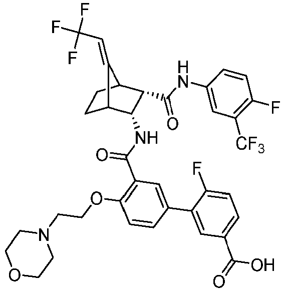

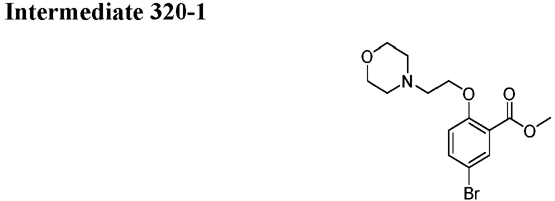

To a solution of methyl 5-bromo-2-hydroxybenzoate (750 mg, 3.25 mmol) and 4-(2-bromoethyl)morpholine (756 mg, 3.90 mmol) in DMF (12 mL) was added K₂CO₃ (1346 mg, 9.74 mmol) heated at 70 °C for 4 h. The reaction mixture was diluted with EtOAc, and the solution washed with water and brine solution. The separated organic layer was dried over Na₂SO₄, filtered and concentrated under reduced pressure. The residue was purified by silica column to furnish methyl 5-bromo-2-(2-morpholinoethoxy)benzoate (320-1, 0.800 g, 2.32 mmol, 71.6 % yield). MS, *m*/z: 343.9 (M+2H).

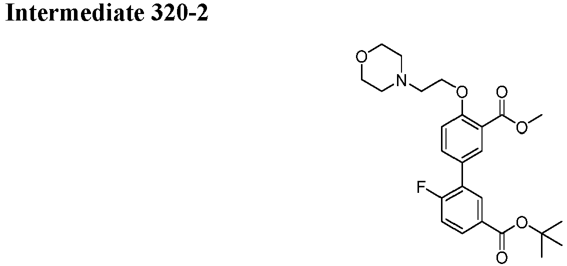

To a solution of **320**-1(300 mg, 0.872 mmol) and tert-butyl 4-fluoro-3-(4,4,5,5-tetramethyl-1,3,2-dioxaborolan-2-yl)benzoate (309 mg, 0.959 mmol) in 1,4-dioxane (10 mL) and water (1 mL) was added tripotassium phosphate (555 mg, 2.61 mmol) and the resulting mixture purged with nitrogen for 5 min. PdCl₂(dppf)-CH₂Cl₂ adduct (71 mg, 0.087 mmol) was added and the reaction mixture purged for 2 min with nitrogen then heated in a sealed tube at 85 °C for 16h. The reaction mixture was filtered through celite. The filtrate was diluted with EtOAc and the organic phase washed with water and brine solutions. The organic layer was dried over Na₂SO₄, filtered and concentrated under reduced pressure. The residue was purified by silica column chromatography to furnish 3'-(tert-butyl) 3-methyl 6'-fluoro-4-(2-morpholinoethoxy)-[1,1'-biphenyl]-3,3'-dicarboxylate **(320-2,** 0.310 g, 0.675 mmol, 77 % yield). MS, *m*/z: 460.2 (M+H).

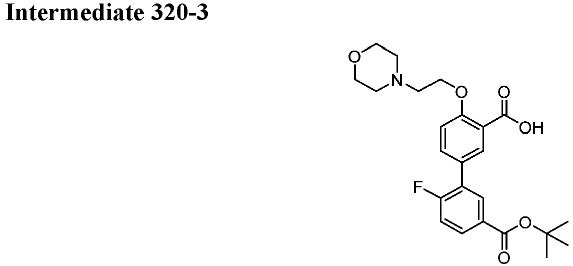

To a solution of **320-2** (100 mg, 0.218 mmol) in THF (2 mL) was added NaOH (0.87 mL, 2.2 mmol) solution and stirred at 50 °C for 30 min. THF was removed under vacuum, 1 ml of water was added and acidified with 1.5N HCl to pH 4. The aqueous layer was extracted with EtOAc (2x20ml). The combined organic layers were washed with water and brine solution, dried over Na₂SO₄, filtered and concentrated under reduced pressure to afford 5'-(tert-butoxycarbonyl)-2'-fluoro-4-(2-morpholinoethoxy)-[1,1'-biphenyl]-3-carboxylic acid (40 mg, 0.090 mmol, 41 % yield). MS, *m*/z: 446.2 (M+H).

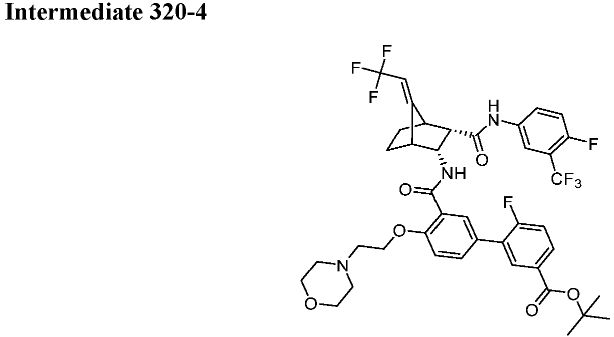

To a solution of 320-3 (30 mg, 0.076 mmol) and 170-2 (334 mg, 0.0760 mmol) in DMF (2 mL) were added DIPEA (0.07 mL, 0.4 mmol) and HATU (57.6 mg, 0.151 mmol), stirred for at room temperature for 12h. The reaction mixture was diluted EtOAc, washed with water and brine solution. The separated organic layer was dried over Na₂SO₄, filtered and concentrated. The residue product was purified by silica gel chromatography to furnish tert-butyl 6-fluoro-3'-(((1R,2R,3 S,4R,Z)-3-((4-fluoro-3-(trifluoromethyl)phenyl)carbamoyl)-7-(2,2,2-trifluoroethylidene)bicyclo[2.2.1]heptan-2-yl)carbamoyl)-4'-(2-morpholinoethoxy)-[1,1'-biphenyl]-3-carboxylate **(320-4,** 50 mg, 0.061 mmol, 80 % yield). MS, *m*/z: 824.3 (M+H).

To a solution of **320-4** (50 mg, 0.061 mmol) in DCM (2 mL) was added TFA (0.094 mL, 1.2 mmol) at 0 °C, stirred for 4 h at room temperature . The reaction mixture was concentrated under reduced pressure and the residue was purified reverse phase HPLC to furnish 6-fluoro-3'-(((1R,2R,3S,4R,Z)-3-((4-fluoro-3-(trifluoromethyl)phenyl)carbamoyl)-7-(2,2,2-trifluoroethylidene)bicyclo[2.2.1]heptan-2-yl)carbamoyl)-4'-(2-morpholinoethoxy)-[1,1'-biphenyl]-3-carboxylic acid (20 mg, 0.025 mmol, 42 % yield) as a white solid. ¹H NMR (400MHz, DMSO-d6) δ ppm 13.27 - 13.08 (m, 1H), 10.47 - 10.37 (m, 1H), 10.02 - 9.81 (m, 1H), 8.91 - 8.75 (m, 1H), 8.16 - 7.94 (m, 3H), 7.89 - 7.79 (m, 1H), 7.76 - 7.60 (m, 2H), 5.82 - 5.67 (m, 1H), 4.68 - 4.61 (m, 1H), 4.61 - 4.43 (m, 1H), 4.01 - 3.83 (m, 2H), 3.75 - 3.61 (m, 2H), 3.58 - 3.48 (m, 3H), 2.84 - 2.78 (m, 2H), 2.70 - 2.63 (m, 5H), 2.02 - 1.85 (m, 2H), 1.81 - 1.65 (m, 2H), 1.62 - 1.45 (m, 2H). MS, *m*/z: 768.2 (M+H).

### Example 323

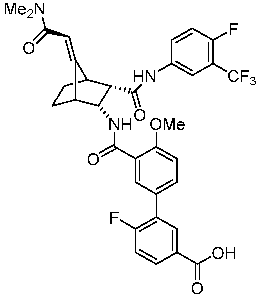

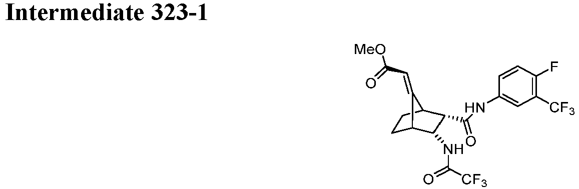

To 120-4 (0.05 g, 0.1 mmol) dissolved in MeOH (0.5 mL) and THF (0.5 mL) was added Hunig's Base (0.021 mL, 0.12 mmol), triphenylphosphine (0.8 mg, 3 µmol), and bis(triphenylphosphine)palladium (II) chloride (2 mg, 3 µmol). The vessel was pressurized with carbon monoxide at 60 psi and heated at 70 °C for 36 h. The reaction solution was concentrated under vacuum and purified via flash chromatography to furnish methyl (Z)-2-((1R,2S,3R,4R)-2-((4-fluoro-3-(trifluoromethyl)phenyl)carbamoyl)-3-(2,2,2-trifluoroacetamido)bicyclo[2.2.1]heptan-7-ylidene)acetate 323-1. ¹H NMR (500 MHz, CDCl₃) δ 9.49 (br d, *J*=6.9 Hz, 1H), 7.96 (s, 1H), 7.86 - 7.72 (m, 2H), 7.23 (t, *J*=9.4 Hz, 1H), 5.76 (s, 1H), 4.51 (dt, *J*=10.5*,* 5.3 Hz, 1H), 3.93 (t, *J*=4.1 Hz, 1H), 3.86 - 3.75 (m, 3H), 3.18 - 3.05 (m, 1H), 2.89 (t, *J=*4.*0* Hz, 1H), 2.06 - 1.87 (m, 2H), 1.78 - 1.64 (m, 2H).

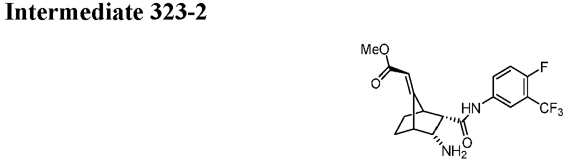

To MeOH (0.8 mL) was added AcCl (0.080 mL, 1.1 mmol) and stirred for 5 minutes and 323-1 added (0.029 g, 0.060 mmol) and the reaction mixture was stirred 32 h. The reaction mixture was concentrated under vacuum to furnish methyl (Z)-2-((1R,2R,3S,4R)-2-amino-3-((4-fluoro-3-(trifluoromethyl)phenyl)carbamoyl)bicyclo[2.2.1]heptan-7-ylidene)acetate, hydrogen chloride salt (323-2, 0.025 g, 0.060 mmol, 100 % yield) which was used without further purification. MS (ESI) *m*/*z* 387.0 (M+H).

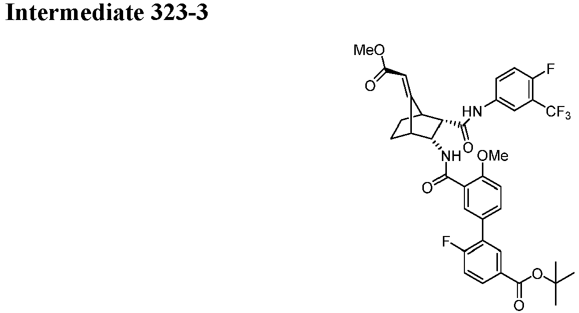

To **323-2** and **120-6** (0.025 g, 0.072 mmol) dissolved in MeCN (0.6 mL) was added DIEA (0.03 mL, 0.2 mmol) followed by HATU (0.034 g, 0.090 mmol). The reaction mixture was stirred 16 h,concentrated under vacuum and purified via flash chromatography to furnish tert-butyl 6-fluoro-3'-(((1R,2R,3S,4R,Z)-3-((4-fluoro-3-(trifluoromethyl)phenyl)carbamoyl)-7-(2-methoxy-2-oxoethylidene)bicyclo[2.2.1]heptan-2-yl)carbamoyl)-4'-methoxy-[1,1'-biphenyl]-3-carboxylate **(323-3,** 0.028 g, 0.039 mmol, 65 % yield). MS (ESI) *m*/*z* 715.3 (M+H).

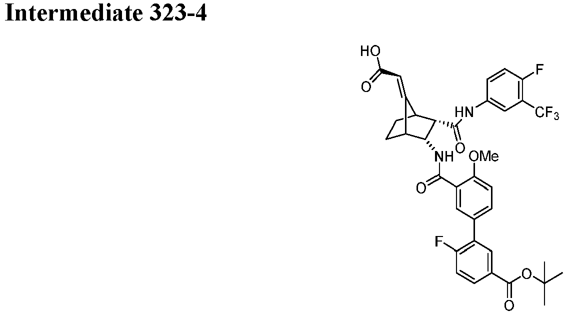

To **323-3** (0.028 g, 0.040 mmol) dissolved in THF (1 mL) was added water (0.5 mL) and lithium hydroxide monohydrate (2 mg, 0.05 mmol) and stirred 16 h. The reaction mixture was diluted with water, neutralized with 1M HCl, and extracted into EtOAc. The organic layer was separated and dried over Na₂SO₄ and concentrated under reduced pressure to furnish (Z)-2-((1R,2R,3S,4R)-2-(5'-(tert-butoxycarbonyl)-2'-fluoro-4-methoxy-[1,1'-biphenyl]-3-carboxamido)-3-((4-fluoro-3-(trifluoromethyl)phenyl)carbamoyl)bicyclo[2.2.1]heptan-7-ylidene)acetic acid **(323-4,** 0.025 g, 0.036 mmol, 90 % yield). ¹H NMR (500 MHz, CDCl₃) δ 8.43 (s, 1H), 8.30 - 8.18 (m, 1H), 8.16 - 8.06 (m, 1H), 8.02 - 7.94 (m, 1H), 7.71 (br d, *J*=8.5 Hz, 1H), 7.25 - 7.13 (m, 2H), 7.00 (br d, *J*=8.8 Hz, 1H), 5.82 (s, 1H), 4.85 - 4.65 (m, 1H), 4.29 (br s, 1H), 3.97 (s, 3H), 3.16 - 2.96 (m, 2H), 2.22 - 2.09 (m, 1H), 2.04 - 1.86 (m, 2H), 1.75 - 1.58 (m, 9H) MS (ESI) *m*/*z* 701.3 (M+H).

**Example 323** was prepared from **Intermediate 323-4** by first making the amide according to the procedure for **Example 34** followed by removal of the t-butyl group according to the procedure for **Example 120.** ¹H NMR (500 MHz, DMSO-d6) δ 10.67 (s, 1H), 9.85 (br d, J=7.0 Hz, 1H), 8.24 (br d, J=4.3 Hz, 1H), 8.11 (br s, 1H), 8.01 (br d, J=7.0 Hz, 1H), 7.93 (br s, 1H), 7.80 (br d, J=8.2 Hz, 1H), 7.72 (br d, J=8.2 Hz, 1H), 7.48 (br t, J=9.5 Hz, 1H), 7.37 (br t, J=9.5 Hz, 1H), 7.31 (br d, J=8.9 Hz, 1H), 6.14 (s, 1H), 4.58 - 4.44 (m, 1H), 4.06 (s, 3H), 3.54 (br s, 1H), 3.05 (s, 3H), 2.99 (s, 1H), 2.88 (s, 4H), 2.03 - 1.95 (m, 1H), 1.90 - 1.73 (m, 1H), 1.45 (br s, 2H). LC-MS RT: 2.19 min; MS (ESI) m/z = 627.14 (M-H)+; Method C.

### Example 325

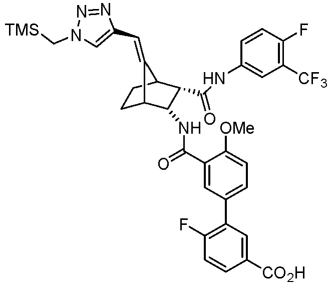

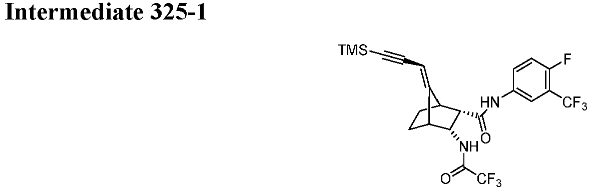

To **120-4** (0.05 g, 0.1 mmol) slurried in triethylamine (0.2 mL) was added ethynyltrimethylsilane (0.02 ml, 0.1 mmol), bis(triphenylphosphine)palladium (II) chloride (3 mg, 5 µmol), and copper(I) iodide (2 mg, 10 µmol). The reaction mixture was heated at 90 °C for 16 h. The reaction mixture was partitioned between EtOAc and pH 7.4 buffer and extracted in to EtOAc. The organic layer was separated and dried over Na₂SO₄, decanted and concentrated under vacuum, and the residue purified via flash chromatography to furnish (1R,2S,3R,4R,Z)-N-(4-fluoro-3-(trifluoromethyl)phenyl)-3-(2,2,2-trifluoroacetamido)-7-(3-(trimethylsilyl)prop-2-yn-1-ylidene)bicyclo[2.2.1]heptane-2-carboxamide **(325-1,** 40 mg, 0.077 mmol, 77 % yield). ¹H NMR (500 MHz, CDCl₃) δ 9.39 (br d, *J*=6.9 Hz, 1H), 7.78 - 7.67 (m, 2H), 7.33 (s, 1H), 7.26 - 7.18 (m, 1H), 5.45 (s, 1H), 4.60 - 4.41 (m, 1H), 3.32 (t, *J*=4.1 Hz, 1H), 3.05 (ddd, *J*=10.5*,* 4.4, 1.4 Hz, 1H), 2.81 (t, *J*=4.1 Hz, 1H), 1.98 - 1.90 (m, 1H), 1.90 - 1.81 (m, 1H), 1.76 - 1.59 (m, 2H), 0.31 - 0.17 (m, 9H). MS (ESI) *m*/*z* 521.0 (M+H).

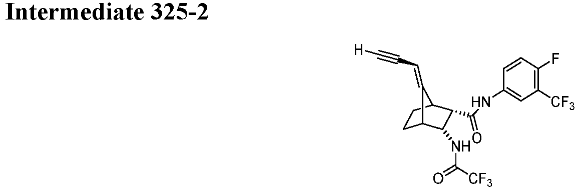

To 325-1 (40 mg, 0.077 mmol) dissolved in THF (0.8 mL) was added 1 M TBAF in THF (0.2 mL, 0.2 mmol) and the reaction was stirred 16 h. The reaction mixture was concentrated under reduced pressure and purified via flash chromatography to furnish (1R,2S,3R,4R,Z)-N-(4-fluoro-3-(trifluoromethyl)phenyl)-7-(prop-2-yn-1-ylidene)-3-(2,2,2-trifluoroacetamido)bicyclo[2.2.1]heptane-2-carboxamide **(325-2,** 38 mg, 0.084 mmol, quantitative yield) MS (ESI) *m*/*z* 499.0 (M+H).

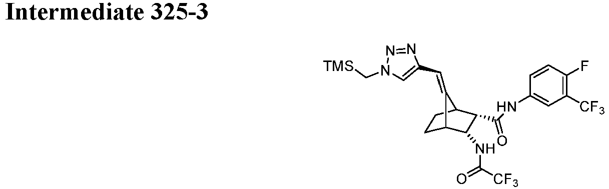

To a solution of **325-2** (0.017 g, 0.038 mmol), (azidomethyl)trimethylsilane (0.011 mL, 0.076 mmol) dissolved in DMF (0.3 mL) and water (0.1 mL) was added copper (II) sulfate pentahydrate (7 mg, 0.03 mmol), and sodium ascorbate (8 mg, 0.04 mmol) and stirred for 3 h. The reaction mixture was partitioned between EtOAc and water, and the organic layer was washed 2x with EtOAc, dried over MgSO₄, filtered and concentrated under vacuum to furnish (1R,2S,3R,4R,Z)-N-(4-fluoro-3-(trifluoromethyl)phenyl)-3-(2,2,2-trifluoroacetamido)-7-((1-((trimethylsilyl)methyl)-1H-1,2,3-triazol-4-yl)methylene)bicyclo[2.2.1]heptane-2-carboxamide **325-3,** which was used without further purification. MS (ESI) *m*/*z* 578.1 (M+H).

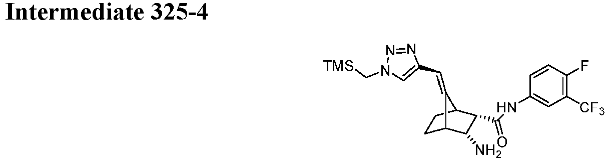

To MeOH (0.5 ml) was added AcCl (0.050 ml, 0.70 mmol) and the reaction mixture stirred for 5 min. 325-3(0.022 g, 0.038 mmol) was added and the reaction mixture was stirred at 40 °C for 48 h. The reaction mixture was concentrated under reduced pressure and residual solvent removed under high vacuum to generate (1R,2S,3R,4R,Z)-3-amino-N-(4-fluoro-3-(trifluoromethyl)phenyl)-7-((1-((trimethylsilyl)methyl)-1H-1,2,3-triazol-4-yl)methylene)bicyclo[2.2.1]heptane-2-carboxamide **(325-4,** 0.018 g, 0.038 mmol, 100 % yield) which was used without further purification. MS (ESI) *m*/*z* 482.2 (M+H).

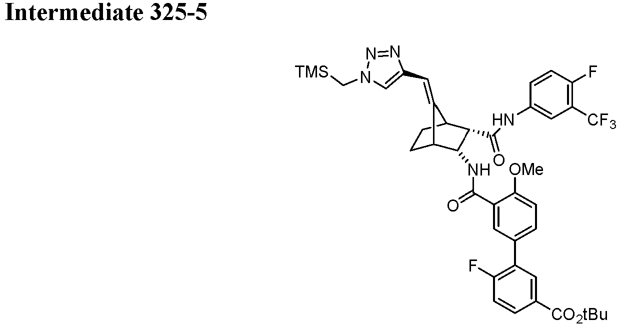

**Intermediate 325-5** was prepared from **325-4** and **120-6** according to the procedure for **Example 108.**

**Example 325** was prepared from **325-5** according to the procedure for **Example 120.** ¹H NMR (500 MHz, DMSO-d6) δ 10.53 (s, 1H), 9.85 (br d, J=7.0 Hz, 1H), 8.15 (br d, J=4.6 Hz, 1H), 8.05 (br s, 1H), 7.99 - 7.81 (m, 3H), 7.71 (br s, 1H), 7.65 (br d, J=8.2 Hz, 1H), 7.40 (br t, J=9.6 Hz, 1H), 7.33 (br t, J=9.6 Hz, 1H), 7.24 (br d, J=8.5 Hz, 1H), 6.18 (s, 1H), 4.44 (br s, 1H), 3.98 (s, 3H), 3.90 (s, 2H), 3.48 (br s, 1H), 3.27 - 3.09 (m, 1H), 2.83 (br s, 1H), 1.96 - 1.72 (m, 2H), 1.41 (br d, J=5.8 Hz, 2H), 0.00 (s, 9H). LC-MS RT: 2.54 min; MS (ESI) m/z = 754.36 (M-H)+; Method C.

### Example 329

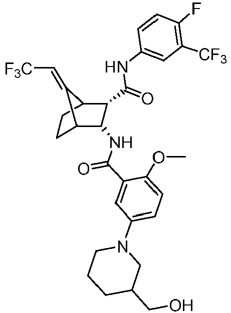

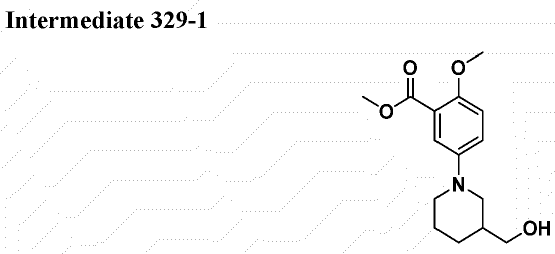

To a solution of methyl 5-iodo-2-methoxybenzoate (500 mg, 1.71 mmol) and piperidin-3-ylmethanol (394 mg, 3.42 mmol) in DMSO (10 mL) was added K₂CO₃ (710 mg, 5.14 mmol), CuI (98 mg, 0.51 mmol) and L-proline (59 mg, 0.51 mmol). The resulting solution was degassed with N₂ for 10 min followed by heating at 90 °C for 12h. The reaction mixture was diluted with ethyl acetate, washed with water, brine, dried over Na₂SO₄, and concentrated under reduced pressure. The residue was purified by silica gel chromatography to furnish methyl 5-(3-(hydroxymethyl)piperidin-1-yl)-2-methoxybenzoate **(329-1,** 350 mg, 1.25 mmol, 73.2 % yield). MS (ESI) *m*/*z* 280.2 (M+H).

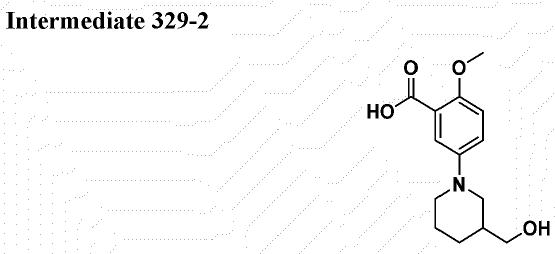

To a solution of **329-1** (350 mg, 1.253 mmol) in MeOH (5 mL), THF (5 mL) and water (3 mL) was added LiOH (150 mg, 6.26 mmol) and stirred at rt for 3h. The reaction mass was concentrated under reduced pressure, the aqueous layer was acidified to pH ~ 4-5 with HCl, and the resulting precipitate was filtered and dried to furnish 5-(3-(hydroxymethyl)piperidin-1-yl)-2-methoxybenzoic acid (300 mg, 1.13 mmol, 90 % yield) as white solid. MS (ESI) *m*/*z* 266.2 (M+H).

**Example 329** was prepared from **Intermediates 166-2** and **329-2** according to the proceudre for **Example 108.** The stereoisomers were separated by Prep HPLC column Chiralcel OD-H(250 X 4.6)mm,5u to furnish (1R,2R,3R,4R,Z)-N-(4-fluoro-3-(trifluoromethyl)phenyl)-3-(5-(3-(hydroxymethyl)piperidin-1-yl)-2-methoxybenzamido)-7-(2,2,2-trifluoroethylidene)bicyclo[2.2.1]heptane-2-carboxamide (2.1 mg, 3.231 µmol, 3.51 % yield) MS (ESI) *m*/*z* 644.2 (M+H). ¹H NMR (400 MHz, DMSO-d6) δ ppm 10.43 (s, 1H), 8.41 (d, *J =* 6.5 Hz, 1H), 8.12 (dd, *J* = 2.5, 6.5 Hz, 1H), 7.88 - 7.75 (m, 1H), 7.48 (t, *J =* 9.8 Hz, 1H), 7.20 (d, *J* = 2.5 Hz, 1H), 7.11 - 6.95 (m, 2H), 5.82 - 5.64 (m, 1H), 4.65 - 4.56 (m, 1H), 4.52 (t, *J* = 5.3 Hz, 1H), 3.83 (s, 3H), 3.51 (br s, 1H), 3.44 - 3.41 (m, 1H), 3.25 - 3.20 (m, 2H), 2.78 (d, *J* = 4.0 Hz, 1H), 2.58 - 2.55 (m, 3H), 2.32 - 2.27 (m, 1H), 1.92 (td, *J* = 4.7, 12.2 Hz, 1H), 1.80 - 1.65 (m, 6H), 1.56 (br s, 2H), 1.09 - 0.94 (m, 1H).

### Example 346

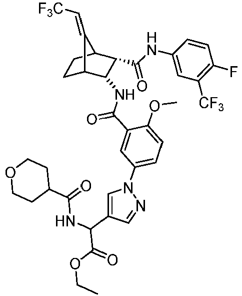

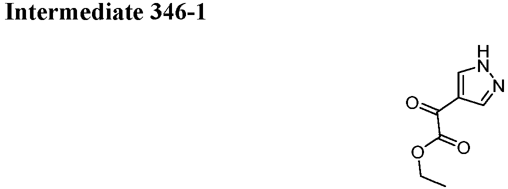

To a solution of 4-bromo-1H-pyrazole (2.00 g, 13.6 mmol) in THF (100 mL) at -78 °C was added dropwise n-butyllithium (25.5 mL, 40.8 mmol). After completion of addition, the reaction mixture was allowed to raise to room temperature and stirred at room temperature for 1.5 hours. The mixture was then cooled back to -78 °C and a solution of diethyl oxalate (2.8 mL, 20 mmol) in THF (2.5 mL) was added and allowed to stir for 20 minutes. The reaction mixture was quenched by the addition of saturated ammonium chloride and the solution extracted with ethyl acetate. The organic layers were combined, concentrated under reduced pressure and purified using silica gel chromatography to yield **346-1** (496 mg, 20.6%). MS (ESI) *m*/*z:* 168.9 (M+H).

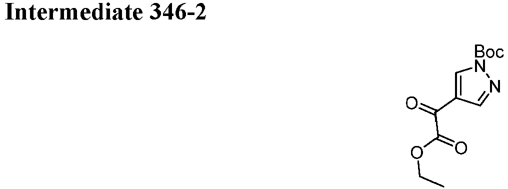

To a solution of **346-1** (150 mg, 0.892 mmol) in acetonitrile (5 mL) was added DMAP (10.90 mg, 0.089 mmol), Di-*tert*-butyl dicarbonate (0.249 mL, 1.07 mmol) followed by TEA (0.149 mL, 1.07 mmol). The reaction mixture was then stirred at room temperature for 18h. The reaction mixture was then concentrated under reduced pressure and purified using silica gel chromatography to yield **346-2** (185 mg, 73.4%). MS (ESI) *m*/*z*: 269.1 (M+H).

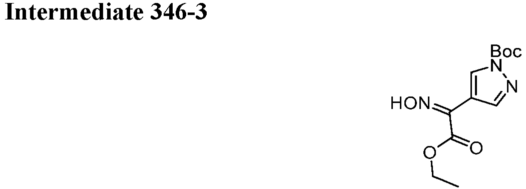

A solution of **346-2** (185 mg, 0.690 mmol), sodium acetate (62.2 mg, 0.759 mmol) and hydroxylamine hydrochloride (86 mg, 1.241 mmol) in ethanol (3 mL) was heated at reflux for 1 hour. The reaction mixture was then concentrated under vacuum and diluted with ethyl acetate. The organic layer was washed with 5% HCl solution to give **346-3** (190 mg, 88%) which was used without further purification. MS (ESI) *m*/*z:* 183.9 (M+H-Boc).

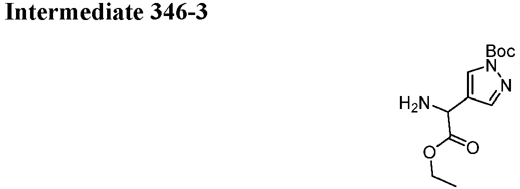

To a degassed solution of **346-3** (190 mg, 0.671 mmol) in ethanol (5 mL) was added palladium on carbon (143 mg, 0.134 mmol) and degassed with nitrogen. The reaction mixture was stirred under a hydrogen balloon for 1.5 hours. The reaction mixture was filtered over a pad of celite to yield **346-4** (181 mg, 100%) MS (ESI) *m*/*z:* 270.1 (M+H).

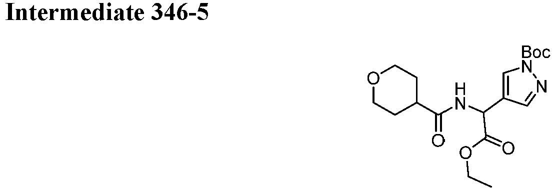

To a solution of **346-4** (181 mg, 0.672 mmol) and tetrahydro-2H-pyran-4-carboxylic acid (87 mg, 0.672 mmol) in anhydrous DMF (2 mL), was added DIEA (0.587 mL, 3.36 mmol) followed by BOP (327 mg, 0.739 mmol). The reaction mixture was stirred at room temperature for 1 hour and filtered. The residue was concentrated under reduced pressure and purified using silica gel chromatography to yield **346-5** (120 mg, 44.5%). MS (ESI) *m*/*z:* 382.3 (M+H).

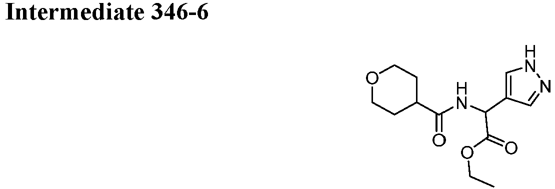

To a solution of **346-5** (120 mg, 0.315 mmol) in DCM (4 mL) was added TFA (1.5 mL, 19.47 mmol) and the reaction mixture stirred at room temperature for 1 hour. The reaction mixture was concentrated under reduced pressure to yield **346-6** (125 mg, 90%). MS (ESI) *m*/*z:* 282.2 (M+H).

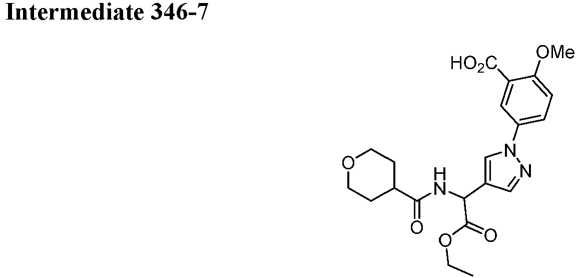

To a solution degassed under N₂ of 5-borono-2-methoxybenzoic acid (87 mg, 0.444 mmol), **346-6** (125 mg, 0.444 mmol) and boric acid (82 mg, 1.3 mmol) was added copper (II) acetate (81 mg, 0.44 mmol) and the reaction mixture stirred at room temperature for 18h. The reaction mixture was concentrated under reduced pressure and purified using silica gel chromatography. MS (ESI) *m*/*z:* 432.3 (M+H).

**Example 346** was prepared in a similar way as **Example 108** from **170-2** and **346-7.** ¹H NMR (500MHz, DMSO-d₆) δ 10.66 (s, 1H), 10.03 (d, *J*=6.7 Hz, 1H), 8.59 - 8.51 (m, 1H), 8.46 (br. s., 1H), 8.32 (br. s., 1H), 8.24 (d, *J*=4.6 Hz, 1H), 7.98 - 7.88 (m, 1H), 7.79 (br. s., 1H), 7.72 (s, 1H), 7.50 (t, *J*=9.8 Hz, 1H), 7.33 (d, *J*=8.8 Hz, 1H), 6.00 - 5.88 (m, 1H), 5.38 (d, *J*=6.4 Hz, 1H), 4.54 (br. s., 1H), 4.17 - 4.09 (m, 2H), 4.05 (s, 3H), 3.36 - 3.20 (m, 2H), 3.00 (br. s., 1H), 2.01 - 1.82 (m, 2H), 1.69 - 1.55 (m, 5H), 1.50 (d, *J*=6.1 Hz, 2H), 1.17 (t, *J*=7.0 Hz, 3H); LC-MS (M+H) = 810.1; HPLC RT = 2.44 min; Method B.

### Example 348

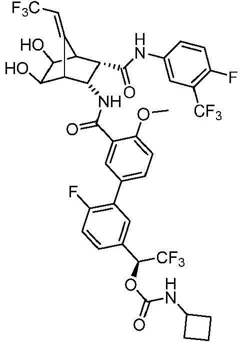

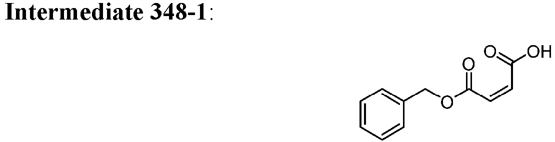

A mixture of furan-2,5-dione (10 g, 102 mmol) and phenylmethanol (31.7 mL, 306 mmol) in toluene (50 mL) was heated to 80 °C for 24 hours. The reaction mixture was then concentrated under reduced pressure and purified using silica gel chromatography to yield **348-1** (15.5 g, 73%). MS (ESI) *m*/*z:* 206.9 (M+H).

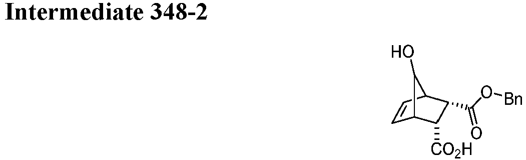

To a solution of **348-1** (3.6 g, 17 mmol) in MeCN (40 mL) and water (0.400 mL) was added ferrocenium hexafluorophosphate (11.6 g, 34.9 mmol) and stirred in open atmoshphere for 18 hours. The reaction mixture was concentrated under reduced pressure and diluted with DCM. The reaction mixture was treated with 1N HCl (40 mL) for 30 minutes. The organic layer was then separated and the aqueous layer was washed with DCM and separated. The organic layers were combined and washed with brine. The organic layer was concentrated under reduced pressure and purified using silica gel chromatography to yield **348-2** (1.8 g, 35%). MS (ESI) *m*/*z:* 289.1 (M+H).

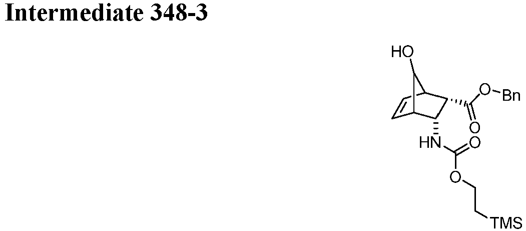

Into a 3 necked round bottom flask was added **348-2** (1.99 g, 6.90 mmol) and toluene (45 mL) followed by TEA (2.1 mL, 15mmol) and diphenylphosphoryl azide (1.26 mL, 5.87 mmol). The reaction mixture was stirred for 2.5 hours at room temperature. To this reaction mixture was added 2-(trimethylsilyl)ethan-1-ol (3.94 mL, 28.3 mmol) and the resulting reaction mixture was heated at 80 °C for 28 hours. The reaction mixture was allowed to cool to room temperature, concentrated under reduced pressure and purified using silica gel chromatography to yield **348-3** (1.52 g, 51.8%). MS (ESI) *m*/*z:* 403.9 (M+H).

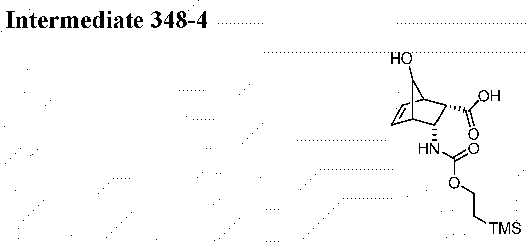

To a solution of **348-3** (1.52 g, 3.77 mmol) in THF (24 mL) and water (8.0 mL) was added LiOH (5.65 mL, 11.3 mmol) and the solution was stirred at room temperature for 1 hour. The reaction mixture was acidified and extracted with ethyl acetate. The organic layers were combined and concentrated under reduced pressure to yield **348-4** (1.1 g, 92%). MS (ESI) *m*/*z:* 313.9 (M+H).

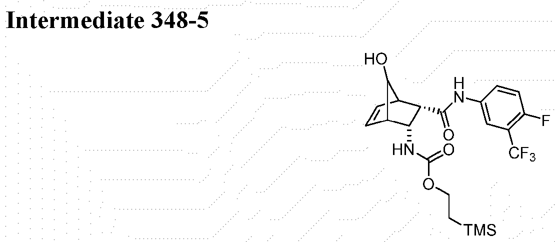

To a solution of **348-4** ( 680 mg, 2.17 mmol) in anhydrous DMF (12 mL) was added 4-fluoro-3-(trifluoromethyl)aniline (0.28 mL, 2.2 mmol), 1-hydroxybenzotriazole hydrate (515 mg, 3.36 mmol) and 1-(3-dimethylaminopropyl)-3-ethylcarbodiimide hydrochloride (624 mg, 3.25 mmol). The reaction mixture was stirred at rt for 18h and concentrated under reduced pressure. The residue was purified using silica gel chromatography to yield **348-5** (260 mg, 25%). MS (ESI) *m*/*z:* 474.9 (M+H).

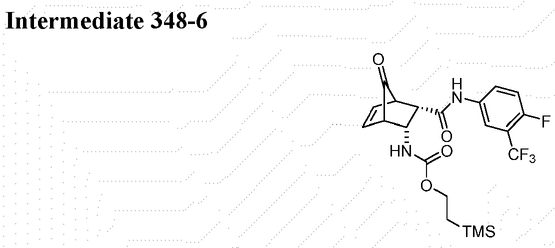

To a flask, under N₂ was added a solution of DMSO (4 mL) and pyridine sulfur trioxide (279 mg, 1.75 mmol) to a solution of **348-5** (260 mg, 0.548 mmol) and TEA (0.61 mL, 4.4 mmol) in DMSO (4 mL) at 0 °C. The reaction mixture was stirred for 1 hour anddiluted with EtOAc and the organic phase washed with brine. The organic layer was concentrated under reduced pressure and the residue purified using silica gel chromatography to yield **348-6** (280 mg, 100%). MS (ESI) *m*/*z:* 473.0 (M+H).

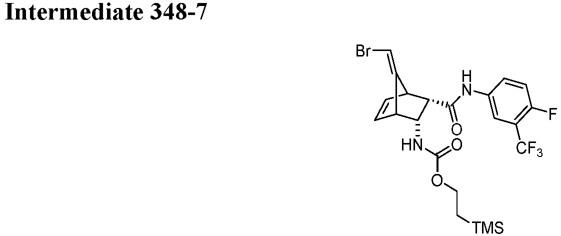

To a round bottom flask was added (bromomethyl)triphenylphosphonium bromide (388 mg, 0.889 mmol) and THF (5.0 mL). The reaction mixture was cooled to -78 °C and followed by addition of 1M NaHMDS (0.89 mL, 0.89 mmol) solution in THF dropwise over 2 minutes while keeping the internal temperature below -70 °C. The resulting bright yellow suspension was stirred at -78 °C for 1 hour. To this reaction mixture was added to a solution of **348-6** (280 mg, 0.593 mmol) in anhydrous THF (1.0 mL) that was previously treated with NaHMDS (1.12 mL, 1.12 mmol) over 2 minutes while keeping the internal temperature below -70 °C. The resulting reaction mixture was stirred at -78 °C for 3 hours. The reaction mixture was then quenched with slow addition of water (6 mL) followed by ethyl acetate (6 mL). The resulting reaction mixture was stirred for 5 minutes and then diluted with EtOAc. The combined organic portion was washed with brine and purifed using silica gel chromatography. The residue was subjected to chiral separation using Chiralcel OD-H, 21 x 250 mm, 5 micron column with a mobile phase of 5% MeOH/CAN/95% CO₂ at a flow rate of 45 mL/min and 150 Bar. The separation was carried out at 40 °C and measured at a wavelength of 240 nm. Chiral separation yielded four peaks with retention times of 9.29 mins (>99.9% ee), 11.16 mins (>99.9% ee), 13.98 mins (>99.9% ee) and 15.30 mins (>81.0% ee). The desired product was found at 11.16 mins and had an ee of >99.9%. (peak 2 from chiral SFC) which was confirmed by 2D NMR analysis to yield **348-7** (82 mg, 25.16%). MS (ESI) *m*/*z:* 473.1 (M+H).

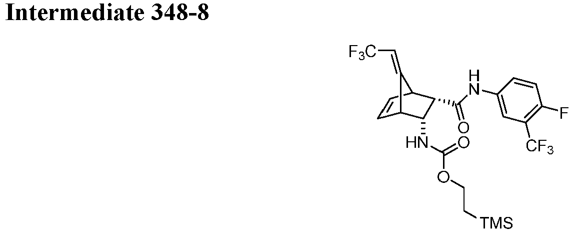

To a suspension of **348-7** (83 mg, 0.15 mmol) and CuI (43.2 mg, 0.227 mmol) in anhydrous DMF (1 mL) and HMPA (1.2 mL, 7.0 mmol) at 75°C under N₂ was added methyl 2,2-difluoro-2-(fluorosulfonyl)acetate (0.048 mL, 0.38 mmol) in anhydrous DMF (0.5 mL) dropwise over a period of 10 min. The resulting suspension was stirred at 75 °C under nitrogen for 12 hours. The reaction mixture was allowed to cool to room temperature, quenched by the addition of NaHCO₃ (20 mL) and the solution, extracted with EtOAc. The organic layer was concentrated and subjected to silica gel chromatography to provide **348-8** (52 mg, 61%). MS (ESI) *m*/*z:* 539.1 (M+H).

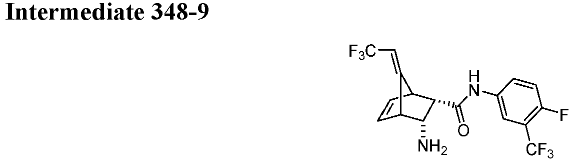

To a solution of **348-8** (52 mg, 0.097 mmol) in 1,4-Dioxane (1.5 mL) was added DCM (1.6 mL) and TFA (0.4 mL). The reaction mixture was stirred at room temperature for 30 minutes and concentrated under reduced pressure to yield **348-9** which was used without further purification (49 mg, 95%). MS (ESI) *m*/*z:* 394.9 (M+H).

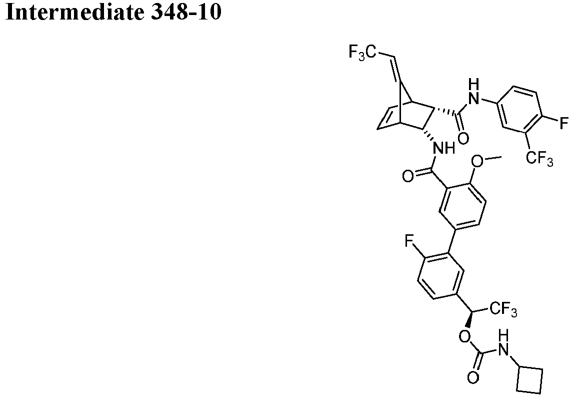

**348-10** was prepared according to the procedure for **Example 230.** MS (ESI) *m*/*z:* 818.2 (M+H).

### Example 348

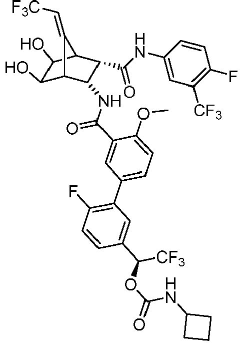

To a solution of **348-10** (35 mg, 0.043 mmol) in acetone (1 mL) was added *N-*methylmorpholine N-oxide (10 mg, 0.086 mmol) followed by OsO₄ in t-butanol (0.054 mL, 4.2 µmol). The reaction mixture was stirred at rt for 18h. The reaction mixture was diluted with EtOAc and the solution washed with sodium thiosulfate. The organic layer was separated and concentrated under reduced pressure and the residue purified using preparative reverse phase HPLC to yield example **Example 348** (14.6 mg, 38.0%). ¹H NMR (400MHz, CD₃OD) δ 10.41 (s, 1H), 10.15 (d, *J*=7.3 Hz, 1H), 8.27 (d, *J*=1.3 Hz, 1H), 8.19 (dd, *J*=6.3, 2.5 Hz, 1H), 7.84 - 7.72 (m, 2H), 7.69 - 7.61 (m, 1H), 7.56 - 7.48 (m, 1H), 7.38 - 7.24 (m, 3H), 6.18 (q, *J*=7.0 Hz, 1H), 5.94 (q, *J*=7.5 Hz, 1H), 4.70 (ddd, *J*=10.9*,* 7.1, 4.2 Hz, 1H), 4.55 (d, *J*=6.4 Hz, 1H), 4.45 (d, *J*=6.4 Hz, 1H), 4.13 (s, 3H), 4.12 - 3.99 (m, 1H), 3.42 (d, *J*=1.5 Hz, 1H), 3.38 (s, 1H), 2.90 (d, *J*=4.0 Hz, 1H), 2.39 - 2.19 (m, 2H), 2.09 - 1.90 (m, 2H), 1.78 - 1.63 (m, 2H); LC-MS (M+H) = 852.1; HPLC RT = 11.48 min; Method C.

A solution of **351-5** (120 mg, 0.176 mmol) and LiOH (21.05 mg, 0.879 mmol) in MeOH (2 mL), THF (2 mL) and water (1 mL) was stirred at ambient temperature for 12h. The reaction mass was concentrated and acidified with 1.5N HCl. The reaction was extracted with DCM and the organic layer was concentrated. The residue was purified by preparative reverse phase HPLC to get 4-fluoro-3'-(1R,2R,3R,4R,Z)-3-((4-fluoro-3-(trifluoromethyl)phenyl)carbamoyl)-7-(2,2,2- trifluoroethylidene)bicyclo[2.2.1]heptan-2-yl)carbamoyl)-4'-methoxy-[1,1'-biphenyl]-3-carboxylic acid (11.5 mg, 0.016 mmol, 9 % yield). ¹H NMR. MS (E⁻) *m*/*z:* 669.2 (M+H).

### Example 352

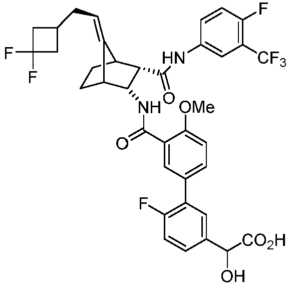

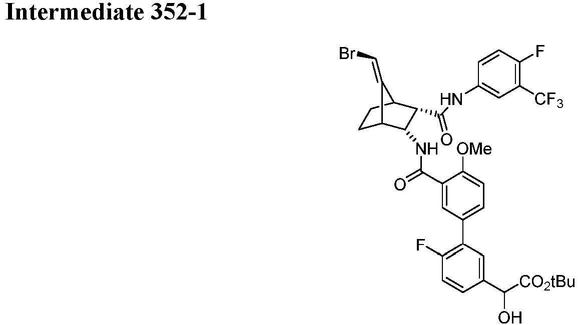

**Intermediate 352-1** was prepared from **120-5** and **177-4** according to the methods described for **Example 108.** LC-MS (M+H) = 767.1; HPLC RT = 1.25 min; Method A.

A slurry of **352-1** (0.038 g, 0.050 mmol), Na₂CO₃ (5.30 mg, 0.0500 mmol), (4,4'-di-t-butyl-2,2'-bipyridine)bis[3,5-difluoro-2-[5-trifluoromethyl-2-pyridinyl-κ_{N})phenyl-κ_{C}]iridium(III) PF₆ (0.515 mg, 0.500 µmol), NiCl₂-ethylene glycol dimethyl ether complex (0.549 mg, 2.50 µmol), 4,4'-di-t-butyl-2,2'-bipyridine (0.551 mg, 2.50 µmol), (TMS)₃SiH (0.03 mL) and 3-(bromomethyl)-1,1-difluorocyclobutane (0.019 g, 0.10 mmol) in DME was degassed with N₂ and irradiated with blue LED for 96 hours. The reaction mixture was diluted with EtOAc, filtered through silica gel and concentrated under reduced pressure. The residue was dissolved in DCM (0.4 mL) was treated with TFA (0.08 mL). After 15 min, the solution was diluted with toluene and concentrated under reduced pressure. The residue was purified by preparative reverse phase HPLC to furnish 2-(3'-(((1R,2R,3 S,4R,Z)-7-(2-(3,3-difluorocyclobutyl)ethylidene)-3-((4-fluoro-3-(trifluoromethyl)phenyl)carbamoyl)bicyclo[2.2.1]heptan-2-yl)carbamoyl)-6-fluoro-4'-methoxy-[1,1'-biphenyl]-3-yl)-2-hydroxyacetic acid (2.6 mg, 3.2 µmol, 6.5 % yield). ¹H NMR (500 MHz, DMSO-d6) δ 10.56 (br s, 1H), 9.92 (br dd, J=15.4, 7.2 Hz, 1H), 8.18 (br t, J=4.9 Hz, 1H), 8.08 (br d, J=11.0 Hz, 1H), 7.81 - 7.61 (m, 2H), 7.52 - 7.35 (m, 3H), 7.32 - 7.13 (m, 2H), 5.26 - 5.13 (m, 1H), 4.92 (br d, J=1.8 Hz, 1H), 4.38 (br d, J=4.3 Hz, 1H), 4.02 (s, 1H), 3.89 - 3.71 (m, 3H), 3.19 - 3.09 (m, 1H), 2.88 (s, 1H), 2.72 (s, 2H), 2.64 (br s, 2H), 2.32 - 2.06 (m, 5H), 1.89 - 1.66 (m, 2H), 1.38 (br s, 2H). LC-MS (M+H) = 734.24; HPLC RT = 2.48 min; Method C.

### Example 360

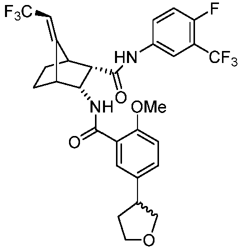

A slurry of **Example 292** (0.025 g, 0.041 mmol), Na₂CO₃ (4.35 mg, 0.0410 mmol), (4,4'-di-t-butyl-2,2'-bipyridine)bis[3,5-difluoro-2-[5-trifluoromethyl-2-pyridinyl-κ_{N})phenyl-κ_{C}]Ir(III) PF₆ (0.423 mg, 0.410 µmol), NiCl₂ ethyleneglycol dimethylether complex (0.451 mg, 2.05 µmol), 4,4'-di-t-butyl-2,2'-bipyridine (0.551 mg, 2.50 µmol), (TMS)₃SiH (0.03 mL) and 3-bromotetrahydrofuran (0.012 g, 0.082 mmol) in DME (1.641 ml) was degassed, blanketed under N₂ and irradiated with blue LED. After 96 h the reaction mixture was diluted with EtOAc, filtered through silica gel and concentrated under reduced pressure. The residue was purified by preparative reverse phase HPLC to furnish (1R,2S,3R,4R,Z)-N-(4-fluoro-3-(trifluoromethyl)phenyl)-3-(2-methoxy-5-(tetrahydrofuran-3-yl)benzamido)-7-(2,2,2-trifluoroethylidene)bicyclo[2.2.1]heptane-2-carboxamide (3.5 mg, 5.5 µmol, 13 % yield) as a mixture of diastereomers. ¹H NMR (500 MHz, DMSO-d6) δ 10.55 (s, 1H), 9.78 (br d, J=5.8 Hz, 1H), 8.14 (br d, J=4.6 Hz, 1H), 7.81 - 7.65 (m, 2H), 7.49 - 7.28 (m, 2H), 7.04 (br d, J=8.5 Hz, 1H), 5.84 (q, J=7.9 Hz, 1H), 4.43 (br s, 1H), 3.95 - 3.78 (m, 5H), 3.74 - 3.64 (m, 1H), 3.37 - 3.07 (m, 2H), 2.89 (br s, 1H), 2.81 (s, 1H), 2.71 - 2.62 (m, 1H), 2.24 - 2.11 (m, 1H), 1.99 - 1.87 (m, 1H), 1.83 - 1.71 (m, 2H), 1.50 - 1.26 (m, 2H). LC-MS (M+H) = 601.16; HPLC RT = 2.58 min; Method C.

### Example 378

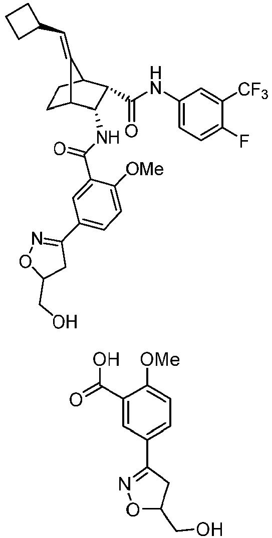

Intermediate **378-1:** Preparation of methyl (E)-5-((hydroxyimino)methyl)-2-methoxybenzoate. Commercially available methyl 5-formyl-2-methoxybenzoate (1.16 g, 5.97 mmol) was dissolved in DCM (5 mL), and to this solution was added hydroxylamine.HCl (415 mg, 5.97 mmol) followed by TEA (1 mL) and the reaction mixture was stirred at r.t. for 18h. Water (100 mL) was added and the solution extracted with EtOAc (2 x 25 mL), the combined organic portions dried (MgSO₄), filtered and evaporated under reduced pressure to generate **378-1,** 1.19 g, 95 % yield. ¹H NMR (400 MHz, CDCl₃) δ 8.13 (s, 1H), 8.03 (d, *J*=2.4 Hz, 1H), 7.78 - 7.67 (m, 1H), 7.03 (d, *J*=8.8 Hz, 1H), 3.97 (s, 3H), 3.93(s, 3H). MS (ESI) *m*/*z* = 210.1 (M+H).

Intermediate **378-2:** Preparation of methyl 5-(5-(hydroxymethyl)-4,5-dihydroisoxazol-3-yl)-2-methoxybenzoate. Intermediate **378-1** (55 mg, 0.26 mmol) was dissolved in DMF (2 mL), and to this solution was added NCS (35 mg, 0.26 mmol) and the reaction mixture was stirred at rt for 4h. Water was added and the solution extracted with EtOAc (2 x 25 mL), the combined organic portions were dried (MgSO₄), filtered, concentrated under reduced pressure and the residue immediately re-dissolved in DCM (5 mL). Allyl alcohol (61 mg, 1.05 mmol) was added to the solution followed by TEA (0.5 mL) and the resulting reaction mixture stirred at rt for 18h. Water was added (20 mL) and the solution extracted with EtOAc (2 x 20 mL), the combined organic portions dried (MgSO₄), filtered and purified by normal phase chromatography eluting with hexanes/EtOAc to yield **378-2, 58** mg, 85 % yield. ¹H NMR (500 MHz, CDCl₃) δ 8.05 (d, *J*=2.4 Hz, 1H), 7.89 (dd, *J*=8.8, 2.4 Hz, 1H), 7.05 (d, *J*=8.9 Hz, 1H), 4.90 (dddd, *J*=10.8, 7.7, 4.6, 3.2 Hz, 1H), 4.08 - 3.85 (ss, 6H), 3.81 - 3.68 (m, 1H), 3.46 - 3.36 (m, 1H), 1.89 (br t, *J*=6.2 Hz, 1H), 1.57 (s, 2H). MS (ESI) *m*/*z* = 266.1 (M+H).

Intermediate **378-3: 378-2** (58 mg, 0.22 mmol) was dissolved in THF (2 mL) and to this was added LiOH (6.3 g, 0.26 mmol) followed by water (2 mL) and methanol (1 mL) and stirred at r.t. for 4h. Quenched to pH 7 with dil HCl (1N) and the solution extracted with EtOAc (2 x 25 mL), the combined organic portions dried (MgSO₄), filtered and evaporated to **378-3.** ¹H NMR (500 MHz, CDCl₃) δ 8.28 (d, *J*=2.3 Hz, 1H), 8.14 (dd, *J*=8.8, 2.4 Hz, 1H), 7.28 - 7.14 (m, 1H), 4.92 (dddd, *J*=10.8, 7.7, 4.6, 3.1 Hz, 1H), 4.16 (s, 3H), 4.09 - 3.89 (m, 1H), 3.72 (dd, *J*=12.4, 4.6 Hz, 1H), 3.48 - 3.39 (m, 1H), 3.38 - 3.29 (m, 1H), 1.94 - 1.72 (m, 1H), 1.60 (br s, 1H). MS (ESI) *m*/*z* = 252.3 (M+H). Intermediate **378-4** and **378-5. 378-3** was subjected to chiral SFC separation according to the following preparative method: Instrument: Berger MG II, Column: Chiralpak IC, 21 x 250 mm, 5 micron Mobile Phase: 20 % Methanol / 80 % CO₂ Flow Conditions: 2 mL/min, 150 Bar, 40°C Detector Wavelength: 220 nm Injection Details: 0.7 mL of ~35mg/mL in MeOH to afford **378-4** (Peak 1, > 99 % de, Analytical RT = 5.6 min) and **378-5** (Peak 2, 99% de, Analytical RT = 6.6 min), Analytical Chromatographic Conditions: Instrument: Shimadzu Nexera SFC (CTR-L410-SFC3), Column: Chiralpak IC, 4.6 x 100 mm, 3 micron, Mobile Phase: 20% Methanol / 80% CO2Flow Conditions: 2.0 mL/min, 150 Bar, 40°C, Detector Wavelength: 220 nm Injection Details: 5 µL of ~1 mg/mL in MeOH

(1R,2S,3R,4R,Z)-7-(cyclobutylmethylene)-N-(4-fluoro-3-(trifluoromethyl)phenyl)-3-(5-(5-(hydroxymethyl)-4,5-dihydroisoxazol-3-yl)-2-methoxybenzamido)bicyclo[2.2.1]heptane-2-carboxamide **378** (diasteromeric mixture) was prepared by the coupling of intermediate **378-3** ( 4.6 mg, 0.018 mmol) with the cyclobutyl norbornyl intermediate **369-1** (7 mg, 0.02 mmol), BOP reagent (8.1 mg, 0.018 mmol) and Hunig's base (0.05 ml) in DMF. Purification via reverse phase HPLC afforded **378** as a solid (5 mg, 44% yield). ¹H NMR (500 MHz, DMSO-d6) δ 10.55 (s, 1H), 9.89 (dd, J=7.1, 2.8 Hz, 1H), 8.26 - 8.17 (m, 2H), 7.84 - 7.73 (m, 2H), 7.48 (br t, J=9.7 Hz, 1H), 7.26 (d, J=8.8 Hz, 1H), 5.37 (d, J=8.4 Hz, 1H), 4.78 - 4.65 (m, 1H), 4.35 (br s, 1H), 4.03 (s, 3H), 3.63 (br s, 1H), 3.22 - 3.05 (m, 3H), 2.96 (br s, 1H), 2.70 (br s, 1H), 2.23 - 2.06 (m, 3H), 1.91 - 1.70 (m, 7H), 1.43 - 1.22 (m, 2H). MS (ESI) m/z =616.1 (M+H). HPLC Purity: 100 %; Retention Time: 2.54 min; Method C.

### Example 379

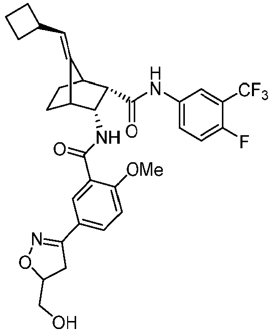

**Example 379.** (1R,2S,3R,4R,Z)-7-(cyclobutylmethylene)-N-(4-fluoro-3-(trifluoromethyl)phenyl)-3-(5-(5-(hydroxymethyl)-4,5-dihydroisoxazol-3-yl)-2-methoxybenzamido)bicyclo[2.2.1]heptane-2-carboxamide (homochiral isomer-2) was prepared (49 % yield) by the coupling method described for example **378** using the cyclobutyl norbornyl intermediate **369-1** and intermediate **378-5.** ¹H NMR (500 MHz, DMSO-d6) δ 10.54 (s, 1H), 9.88 (br d, *J*=7.0 Hz, 1H), 8.22 (s, 1H), 8.23 (d, *J*=7.0 Hz, 1H), 7.79 (br d, *J*=8.2 Hz, 2H), 7.49 (br t, *J*=9.6 Hz, 1H), 7.27 (d, *J*=8.5 Hz, 1H), 5.38 (br d, *J*=8.5 Hz, 1H), 4.70 (br d, *J*=3.1 Hz, 2H), 4.36 (br s, 1H), 4.04 (s, 3H), 3.51 (br s, 1H), 3.37 (br s, 2H), 3.22 - 3.04 (m, 2H), 2.97 (br s, 1H), 2.71 (br s, 1H), 2.19 (br d, *J*=5.8 Hz, 1H), 2.14 (br s, 1H), 1.92 - 1.71 (m, 6H), 1.37 (br s, 2H). MS (ESI) *m*/*z* = 616.1 (M+H). HPLC Purity: 100 %; Retention Time: 2.54 min; Method C.

### Example 384

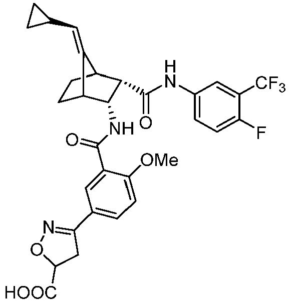

### Intermediate 384-1 (racemate) and 384-2 (homochiral peak-1) and 384-3 (homochiral peak-2)

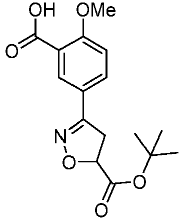

Intermediate **384-1:** The intermediate 5-(5-(tert-butoxycarbonyl)-4,5-dihydroisoxazol-3-yl)-2-methoxybenzoic acid was prepared from the product from **378-1** vial hydrolysis of the ester and treatment with NCS in DMF as described for **378-2** to afford 5-(chloro(hydroxyimino)methyl)-2-methoxybenzoic acid which on treatment with excess t-butyl acrylate afforded the desired intermediate 5-(5-(tert-butoxycarbonyl)-4,5-dihydroisoxazol-3-yl)-2-methoxybenzoic acid **(384-1)** in 76 % yield. ¹H NMR (500 MHz, CDCl₃) δ 8.25 (d, *J*=2.3 Hz, 1H), 8.19 (dd, *J*=8.8, 2.4 Hz, 1H), 7.16 (d, *J*=8.9 Hz, 1H), 5.10 (dd, *J*=9.9, 8.7 Hz, 1H), 4.16 (s, 3H), 3.67 - 3.60 (m, 2H), 1.74 - 1.51 (m, 9H). MS (ESI) *m*/*z* = 322.1 (M+H).

Intermediate **384-2** and **384-3:** The **384-1** chiral intermediates were separated by chiral SFC by the following preparative chromatographic methods: Instrument: Berger MG II, Column: Chiralpak IC, 21 x 250 mm, 5 micron, Mobile Phase: 20 % Methanol / 80 % CO₂, Flow Conditions: 2 mL/min, 150 Bar, 40°C, Detector Wavelength: 220 nm, Injection Details: 0.7 mL of ~35mg/mL in MeOH to afford **384-2** (Peak 1, > 99% de, Analytical RT = 7.93 min) and **384-3** (Peak 2, > 99% de, Analytical RT = 9.65 min). Analytical Chromatographic Conditions: Instrument: Shimadzu Nexera SFC (CTR-L410-SFC3), Column: Chiralpak IC, 4.6 x 100 mm, 3 micronMobile Phase: 20% Methanol / 80% CO₂, Flow Conditions: 2.0 mL/min, 150 Bar, 40°C, Detector Wavelength: 220 nm, Injection Details: 5 µL of ~1 mg/mL in Methanol.

3-(3-(((1R,2R,3S,4R,Z)-7-(cyclopropylmethylene)-3-((4-fluoro-3-(trifluoromethyl)phenyl)carbamoyl)bicyclo[2.2.1]heptan-2-yl)carbamoyl)-4-methoxyphenyl)-4,5-dihydroisoxazole-5-carboxylic acid (diasteromeric mixture) was prepared (7 % yield) by the coupling method described for example **378** using the norbornyl intermediate **166-2** and intermediate **384-1.** ¹H NMR (500 MHz, DMSO-d6) δ 10.56 (s, 1H), 9.92 (d, *J*=7.0 Hz, 1H), 8.32 - 8.20 (m, 2H), 7.87 - 7.75 (m, 2H), 7.49 (t, *J*=9.8 Hz, 1H), 7.34 - 7.22 (m, 1H), 5.15 (dd, *J*=11.6*,* 6.7 Hz, 1H), 4.70 (d, *J*=9.5 Hz, 1H), 4.45 (br s, 1H), 4.05 (s, 3H), 3.74 (dd, *J*=17.1, 11.6 Hz, 1H), 3.23 - 3.13 (m, 2H), 3.11 (br s, 2H), 2.86 - 2.64 (m, 1H), 1.88 - 1.68 (m, 2H), 1.62 - 1.46 (m, 1H), 1.42 (br s, 2H), 0.88 - 0.68 (m, 2H), 0.36 (br s, 2H). MS (ESI) *m*/*z* = 616.3 (M+H). HPLC Purity: 100 %; Retention Time: 2.38 min. Method C.

### Example 385

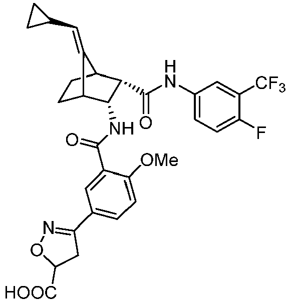

3-(3-(((1R,2R,3S,4R,Z)-7-(cyclopropylmethylene)-3-((4-fluoro-3-(trifluoromethyl)phenyl)carbamoyl)bicyclo[2.2.1]heptan-2-yl)carbamoyl)-4-methoxyphenyl)-4,5-dihydroisoxazole-5-carboxylic acid, homochiral isomer-1 was prepared by the coupling of intermediate **384-2** (13.9 mg, 0.04 mmol) with intermediate **166-2** (16 mg, 0.04 mmol) in the presence of BOP reagent (19 mg, 0.04 mmol) and Hunig's base (0.05 mL) in DMF. The reaction mixture was concentrated under reduced pressure and water added (25 mL) and the solution was extracted with EtOAc (2 x 25 mL), the combined organic portions dried (MgSO₄), filtered and concentrated under reduced pressure. The residue was dissolved in DCM (1ml) and to this was added TFA (0.2 mL) and stirred at rt for 15 min. The solution was concentrated under reduced pressure and redissolved with DMF (1 mL) and purified via reverse phase HPLC to afford **385,** 3-(3-(((1R,2R,3S,4R,Z)-7-(cyclopropylmethylene)-3-((4-fluoro-3-(trifluoromethyl)phenyl)carbamoyl)bicyclo [2.2.1] heptan-2-yl)carbamoyl)-4-methoxyphenyl)-4,5-dihydroisoxazole-5-carboxylic acid (homochiral) as a solid (12 mg, 99 % yield). ¹H NMR (500 MHz, DMSO-d₆) δ 10.62 (s, 1H), 9.92 (br d, *J*=7.0 Hz, 1H), 8.24 (br s, 2H), 7.88 - 7.76 (m, 2H), 7.49 (br t, *J*=9.5 Hz, 1H), 7.27 (d, *J*=8.9 Hz, 1H), 5.01 - 4.84 (m, 1H), 4.69 (d, *J*=9.5 Hz, 1H), 4.45 (br s, 1H), 4.05 (s, 3H), 3.67 - 3.43 (m, 1H), 3.18 (br d, *J*=7.3 Hz, 1H), 3.12 (br s, 1H), 2.73 (br s, 1H), 1.92 (s, 1H), 1.88 - 1.66 (m, 2H), 1.51 (br d, *J*=4.3 Hz, 1H), 1.42 (br s, 2H), 0.89 - 0.68 (m, 2H), 0.35 (br s, 2H). HPLC purity 100 %. Analytical LC-MS: 2.33 min; (ESI) *m*/*z* = 616.28 (M+H)+, Method C.

### Example 390

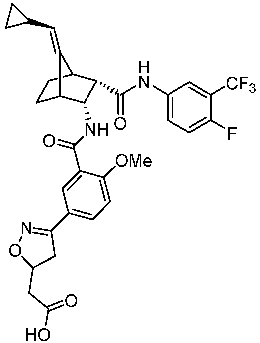

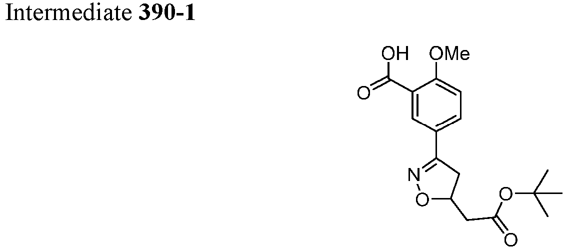

Intermediate **390-1** was prepared in an identical fashion (71 % yield) described for intermediate **378-3** which in this case by substituting allyl alcohol with tert-butyl but-3-ynoate. ¹H NMR (400 MHz, CDCl₃) δ 10.40 (br s, 1H), 8.29 - 8.25 (m, 1H), 8.18 (dd, *J*=8.8, 2.4 Hz, 1H), 7.16 (d, *J*=8.8 Hz, 1H), 5.21 - 5.09 (m, 1H), 4.23 - 4.12 (m, 3H), 3.58 (dd, *J*=16.8, 10.5 Hz, 1H), 3.17 (dd, *J*=16.7, 7.5 Hz, 1H), 2.82 (dd, *J*=15.8, 5.9 Hz, 1H), 2.61 (dd, *J*=15.8, 7.5 Hz, 1H), 1.52 - 1.43 (m, 9H). MS (ESI) *m*/*z* = 336.1 (M+H). Intermediate **390-2** and **390-3:** The chiral intermediates of **390-1** were separated by chiral SFC by the following preparative chromatographic methods: Instrument: PIC Solution SFC Prep-200, Column: Chiralpak IC, 30 x 250 mm, 5 micron Mobile Phase: 15 % MeOH / 85 % CO₂ Flow Conditions: 85 mL/min, 150 Bar, 40°C Detector Wavelength: 227 nm, Injection Details: 0.5 mL of ~53mg/mL in MeOH to obtain **390-2** (Peak 1, 100 % de, Analytical RT = 11.3 min) and **390-3** (Peak 2, 93.8 % de, Analytical RT = 12.6 min). Analytical Chromatographic Conditions: Instrument: Aurora Infinity SFC. Column: Chiralpak IC, 4.6 x 250 mm, 3 micron, Mobile Phase: 20% MeOH / 80% CO₂, Flow Conditions: 2.0 mL/min, 150 Bar, 40°C, Detector Wavelength: 220 nm, Injection Details: 5 µL of ~1 mg/mL in MeOH.

2-(3-(3-(((1R,2R,3S,4R,Z)-7-(cyclopropylmethylene)-3-((4-fluoro-3-(trifluoromethyl)phenyl)carbamoyl)bicyclo[2.2.1]heptan-2-yl)carbamoyl)-4-methoxyphenyl)-4,5-dihydroisoxazol-5-yl)acetic acid, homochiral isomer-2, **390** was prepared (47 % yield) by the coupling method described for example **378** using the cyclopropyl norbornyl intermediate **166-2** and intermediate **390-3** followed by deprotection with TFA. ¹H NMR (500 MHz, DMSO-d6) δ 10.55 (s, 1H), 9.92 (br d, *J*=7.2 Hz, 1H), 8.26 - 8.19 (m, 2H), 7.84 - 7.77 (m, 2H), 7.49 (t, *J*=9.6 Hz, 1H), 7.28 (d, *J*=8.9 Hz, 1H), 5.04 - 4.90 (m, 1H), 4.70 (d, *J*=9.5 Hz, 1H), 4.46 (br s, 1H), 4.05 (s, 3H), 3.22 - 3.09 (m, 2H), 2.73 (br s, 1H), 2.70 - 2.59 (m, 2H), 2.55 (s, 2H), 1.89 - 1.71 (m, 2H), 1.51 (br d, J=4.9 Hz, 1H), 1.42 (br s, 2H), 0.87 - 0.69 (m, 2H), 0.36 (br s, 2H). MS (ESI) *m*/*z* = 630.3 (M+H). HPLC Purity: 100 %; Retention Time: 2 min. Method B.

### Example 397

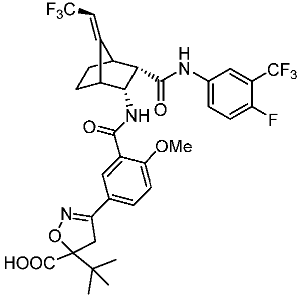

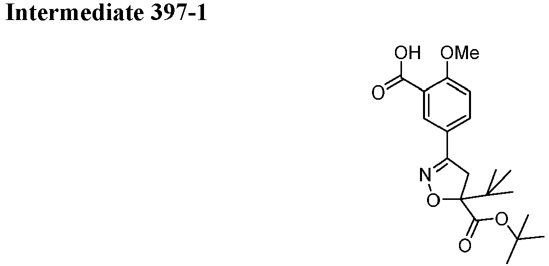

Intermediate **397-1** was prepared in an identical fashion (81 % yield) described for intermediate **378-3** which in this case by substituting allyl alcohol with tert-butyl 3,3-dimethyl-2-methylenebutanoate. ¹H NMR (500 MHz, CD₃OD) δ 8.12 (d, *J*=2.3 Hz, 1H), 7.88 (d, *J*=8.5 Hz, 1H), 7.22 (d, *J*=8.9 Hz, 1H), 4.06 - 3.88 (s, 3H), 3.62 (q, *J=*18.0 Hz, 2H), 1.61 - 1.39 (m, 9H). MS (ESI) m/z = 378.3 (M+H).

5-(tert-butyl)-3-(3-(((1R,2R,3S,4R,Z)-3-((4-fluoro-3-(trifluoromethyl)phenyl)carbamoyl)-7-(2,2,2-trifluoroethylidene)bicyclo[2.2.1]heptan-2-yl)carbamoyl)-4-methoxyphenyl)-4,5-dihydroisoxazole-5-carboxylic acid diasteromeric mixture, **397** was prepared (54 % yield) by the coupling method described for example **378** using the trifluoromethyl norbornyl intermediate **170-2** and intermediate **397-1** followed by treatment with TFA. ¹H NMR (500 MHz, DMSO-d6) δ 10.74 - 10.63 (m, 1H), 9.98 - 9.88 (m, 1H), 8.21 (br d, *J*=5.2 Hz, 1H), 7.79 (br s, 1H), 7.50 (br t, *J*=9.2 Hz, 1H), 7.26 (br s, 1H), 7.08 (br s, 1H), 5.99 - 5.86 (m, 1H), 4.50 (br s, 1H), 4.03 (s, 3H), 3.51 (br s, 3H), 3.24 (br s, 1H), 2.99 (s, 1H), 2.11 - 1.90 (m, 1H), 1.86 (br s, 1H), 1.49 (br s, 1H), 0.99 (br s, 9H). MS (ESI) *m*/*z* = 700.3 (M+H). HPLC Purity: 98.8 %; Retention Time: 2.07 min. Method B.

### Example 406

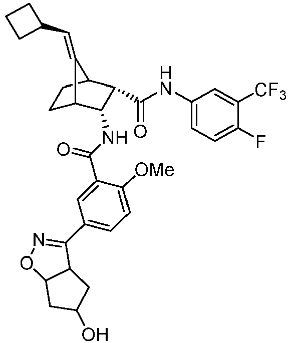

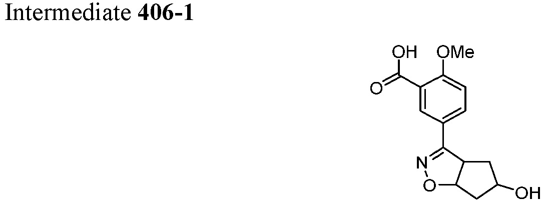

Intermediate **406-1** was prepared in an identical fashion (31 % yield) described for intermediate **378-3** which in this case by substituting allyl alcohol with cyclopent-3-en-1-ol as mixture of diasteromers. ¹H NMR (600 MHz, CDCl₃) δ 8.04 (d, J=2.3 Hz, 1H), 7.85 (dd, J=8.8, 2.3 Hz, 1H), 7.03 (d, J=8.8 Hz, 1H), 5.30 (ddd, J=9.4, 6.2, 2.9 Hz, 1H), 4.50 (quin, J=5.9 Hz, 1H), 4.19 (td, J=9.3, 4.7 Hz, 1H), 3.92 (s, 3H), 2.33 - 2.27 (m, 1H), 2.18 - 2.06 (m, 3H). MS (ESI) *m*/*z* = 292.0 (M+H).

Intermediate **406-2** through **406-5 (chiral).** The chiral intermediates of **406-1** were separated by chiral SFC by the following preparative chromatographic methods: Instrument: Berger SFC (LVL-L4021 Lab) Column: IC 25 X 3 cm ID, 5µm, Temperature: 40C, Flow rate: 85 mL/min, Mobile Phase: gradient 75/25 CO₂/MeOH for 12min then to 45 % MeOH, Detector Wavelength: 235 nm, Injection Volume: 1000 µL to afford chiral **406-2** Peak-1, > 99 % de, Analytical RT = 8.80 min), chiral **406-3** (Peak-2, >95 % de, Analytical RT = 9.86 min), chiral **406-4** (Peak-3, > 99 % de, Analytical RT = 13.53 min), chiral **406-5** (Peak-4, > 99 % de, Analytical RT = 16.67 min). Analytical Chromatographic Conditions: Instrument: Agilent SFC (LVL-L4021 Lab), Column: IC 250 X 4.6 mm ID, 5 µm, Temperature: Ambient, Flow rate: 2.0 mL/min, Mobile Phase: gradient 75/25 CO₂/MeOH 12 min then to 45%MeOH.

(1R,2S,3R,4R,Z)-7-(cyclobutylmethylene)-N-(4-fluoro-3-(trifluoromethyl)phenyl)-3-(5-(5-hydroxy-3a,5,6,6a-tetrahydro-4H-cyclopenta[d]isoxazol-3-yl)-2-methoxybenzamido)bicyclo[2.2.1]heptane-2-carboxamide diateromeric mixture, **406** was prepared (74 % yield) by the coupling method described for example **378** using the cyclobutyl norbornyl intermediate **369-1** and intermediate **406-1.** ¹H NMR (500 MHz, DMSO-d6) δ 10.56 (s, 1H), 9.89 (d, *J*=7.3 Hz, 1H), 8.21 (br s, 2H), 7.78 (br d, *J*=8.7 Hz, 2H), 7.48 (br t, *J*=9.6 Hz, 1H), 7.26 (br d, *J*=8.8 Hz, 1H), 5.37 (d, *J*=8.3 Hz, 1H), 5.10 (br t, *J*=7.2 Hz, 1H), 4.34 (br s, 1H), 4.15 (br s, 1H), 4.12 - 4.05 (m, 1H), 4.03 (s, 3H), 3.72 - 3.56 (m, 3H), 3.20 - 3.02 (m, 2H), 2.95 (br s, 1H), 2.70 (br s, 1H), 2.16 (br s, 1H), 2.13 - 2.01 (m, 2H), 1.92 - 1.70 (m, 6H), 1.36 (br s, 2H). MS (ESI) *m*/*z* = 642.1 (M+H). HPLC Purity: 100 %; Retention Time: 2.49 min. Method C.

### Example 413

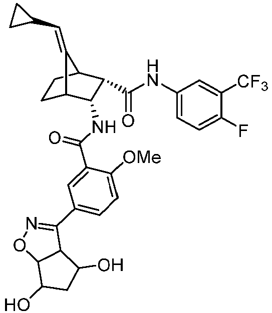

### Intermediate 413-1 (diasteromeric mixture)

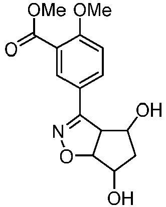

Intermediate **413-1** was prepared in an identical fashion (10 % yield) described for intermediate **378-3** which in this case by substituting allyl alcohol with (1R,3S)-cyclopent-4-ene-1,3-diol as a mixture of diastereomers. ¹H NMR (400 MHz, CDCl₃) δ 8.09 - 7.91 (m, 1H), 7.30 (s, 1H), 7.11 - 7.01 (m, 1H), 5.46 - 5.21 (m, 1H), 4.45 - 4.23 (m, 1H), 4.04 - 3.88 (ss, 6H), 3.02 - 2.98 (m, 1H), 2.92 (d, *J*=0.7 Hz, 1H), 2.45 - 2.35 (m, 1H), 2.02 (s, 2H). MS (ESI) *m*/*z* = 294.1 (M+H).

### Intermediate 413-2 (diasteromeric mixture)

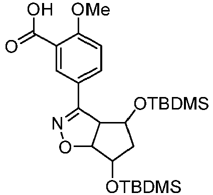

**413-2** was obtained from intermediate **413-1** via a two step sequence by the protection with excess TBDMS triflate (2.64 g, 9.99 mmol) and 2,6-lutidine (1.61 g, 14.9 mmol) in DCM (5 mL) followed by the hydrolysis of the ester with LiOH in THF/MeOH/water (1:1:1, 5 mL). ¹H NMR (500 MHz, CDCl₃) δ 8.48 - 8.46 (m, 1H), 8.04 - 8.00 (m, 1H), 7.10 - 7.05 (m, 1H), 5.06 - 5.02 (m, 1H), 4.33 - 4.29 (m, 1H), 4.23 - 4.18 (s, 3H), 4.15 - 4.13 (m, 1H), 4.12 - 4.10 (m, 1H), 4.00 - 3.94 (m, 1H), 1.29 - 1.24 (m, 1H), 0.93 (ss, 18H), 0.12 (s, 3H), 0.12 - 0.03 (m, 3H), 0.03 (s, 1H), 0.02 (s, 3H), -0.05 - 0.06 (m, 3H). MS (ESI) m/z 522.5 (M+H).

(1R,2S,3R,4R,Z)-7-(cyclopropylmethylene)-3-(5-(4,6-dihydroxy-3a,5,6,6a-tetrahydro-4H-cyclopenta[d]isoxazol-3-yl)-2-methoxybenzamido)-N-(4-fluoro-3-(trifluoromethyl)phenyl)bicyclo[2.2.1]heptane-2-carboxamide diasteromeric mixture, **413** was prepared (36 % yield) by the coupling method described for example **378** using the cyclopropyl norbornyl intermediate **166-2** and intermediate **413-2** followed by deprotection with tetrabutylammonium fluoride (1M in THF, 1 mL). ¹H NMR (500 MHz, DMSO-d6) δ 10.55 (s, 1H), 9.90 (br d, *J*=7.3 Hz, 1H), 8.43 - 8.37 (m, 1H), 8.21 (br d, *J*=6.1 Hz, 1H), 7.89 (dd, *J*=8.7, 2.3 Hz, 1H), 7.83 - 7.66 (m, 1H), 7.48 (t, *J*=9.6 Hz, 1H), 7.29 (d, *J*=8.9 Hz, 1H), 4.96 (dd, *J*=10.2, 2.0 Hz, 1H), 4.70 (d, *J*=9.5 Hz, 1H), 4.45 (br s, 1H), 4.05 (s, 3H), 3.54 (br s, 1H), 3.21 - 3.07 (m, 2H), 3.00 (s, 1H), 2.54 (s, 1H), 2.85 - 2.64 (m, 1H), 1.92 - 1.76 (m, 3H), 1.76 - 1.62 (m, 1H), 1.52 (br s, 1H), 1.42 (br s, 2H), 0.85 - 0.68 (m, 2H), 0.36 (br s, 2H). MS (ESI) *m*/*z* = 644.4 (M+H). HPLC Purity: 100 %; Retention Time: 2.36 min. Method C.

### Example 414

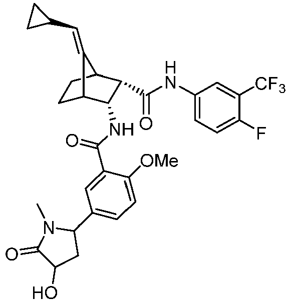

### Intermediate 414-3 (Racemate) and chiral 414-4 (chiral peak-1), chiral 414-5 (chiral peak-2), chiral 414-6 (chiral peak-3), chiral 414-7 (chiral peak-4).

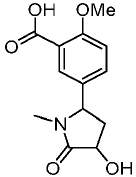

Intermediate **414-1:** Commercially available methyl 5-formyl-2-methoxybenzoate (948 mg, 4.88 mmol) was dissolved in EtOH (10 mL) and to this solution was added NMeNHOH.HCl (408 mg, 4.88 mmol) followed by K₂CO₃ (675 mg, 4.88 mmol) and the reaction mixture was stirred at rt for 1h. with water was added (100 mL) and the solution was extracted with EtOAc (2 x 25 mL), the combined organic portions dried (MgSO₄) and evaporated under reduced pressure to a solid. The solid was transferred to a vial and toluene (7 mL) was added followed by methyl acrylate (3 mL) and the vial sealed. The reaction mixture was heated at 95 °C for 18h. The cooled reaction mixture was concentrated under reduced pressure and the residue purified by silica gel chromatography. **414-3** was isolated as an oil (200 mg, 13 %). ¹H NMR (400 MHz, CDCl₃) δ 7.96 (m, 1H), 7.88 (m, 1), 7.02 (d, *J*=8.8 Hz, 1H), 4.01 - 3.84 (mss, 8H), 3.79 - 3.71 (m, 3H), 3.17 - 3.01 (m, 3H), 2.92 - 2.67 (m, 1H), 2.07 - 1.81 (m, 2H). MS (ESI) *m*/*z* = 310.0 (M+H).

Intermediate **414-2:** The product **414-1** (49 mg, 0.158 mmol) was dissolved in methanol (5 mL) in a Parr flask and to this was added Pd/C 10 % (20 mg) and hydrogenated at 60 psi for 5h. The reaction mixture was filtered over a celite pad and evaporated under reduced pressure to afford methyl 5-(4-hydroxy-1-methyl-5-oxopyrrolidin-2-yl)-2-methoxybenzoate as an oil (35 mg, 79 %). ¹H NMR (500 MHz, CD₃OD) δ 7.71 (d, *J*=2.4 Hz, 1H), 7.50 (dd, *J*=8.7*,* 2.4 Hz, 1H), 7.18 (d, *J*=8.7 Hz, 1H), 4.51 - 4.42 (m, 1H), 4.38 (t, *J*=8.5 Hz, 1H), 3.90 (s, 3H), 3.86 (s, 3H), 2.84 (ddd, *J*=13.1*,* 8.4, 6.9 Hz, 1H), 2.58 (s, 3H), 1.74 (dt, *J*=13.0, 8.5 Hz, 1H). MS (ESI) *m*/*z* = 280.2 (M+H).

Intermediate **414-3:** The product **414-2** (30 mg) was dissolved in MeOH (1 mL), and to this solution was added LiOH followed by water (1 mL) and stirred at rt for 5h. dil. HCl was added and the resulting solution concentrated under reduced pressure to a gummy solid. Methanol was added and the reaction mixture filtered and concentrated under reduced pressure to to generate (20 mg, 71 % yield) **414-3.** MS m/z = 266.08 (M+H). Chiral Intermediate **414-(4-7): 414-3** was separated by SFC under the following preparative conditions: Instrument: Berger SFC (LVL-L4021 Lab), Column: IG 25 X 3 cm ID, 5µm, Temperature: 40C, Flow rate: 85 mL/min, Mobile Phase: 82/18 CO₂/MeOH-0.1 % DEA, Detector Wavelength: 220 nm, Injection Volume: 1200 µL to afford chiral **414-4** (Peak-1, > 99 % de, Analytical RT = 15.56 min), chiral **414-5** (Peak-2 > 95 % de, Analytical RT = 18.09 min), chiral **414-6** (Peak-3, >99 % de, Analytical RT = 26.38 min) and chiral **414-7** (Peak-4, > 95% de, Analytical RT = 29.29 min). Analytical Chromatographic Conditions: Instrument: Agilent SFC (LVL-L4021 Lab), Column: IG 250 X 4.6 mm ID, 5 µm, Temperature: Ambient, Flow rate: 2.0 mL/min, Mobile Phase: 80/20 CO₂/MeOH-0.1%DEA

(1R,2S,3R,4R,Z)-7-(cyclopropylmethylene)-N-(4-fluoro-3-(trifluoromethyl)phenyl)-3-(5-(4-hydroxy-1-methyl-5-oxopyrrolidin-2-yl)-2-methoxybenzamido)bicyclo[2.2.1]heptane-2-carboxamide homochiral isomer-1, **414** was prepared (48 % yield) by the coupling method described for example **378** using the cyclopropyl norbornyl intermediate **166-2** and intermediate **414-4.** ¹H NMR (500 MHz, DMSO-d6) δ 10.54 (s, 1H), 9.89 (d, *J*=7.3 Hz, 1H), 8.23 (dd, *J*=6.6, 2.3 Hz, 1H), 7.89 (d, *J*=2.1 Hz, 1H), 7.84 - 7.67 (m, 1H), 7.49 (t, *J*=9.2 Hz, 1H), 7.44 (d, *J*=8.3 Hz, 1H), 7.24 (d, *J*=8.5 Hz, 1H), 4.70 (d, *J*=9.8 Hz, 1H), 4.34 (m, 1H), 4.20 (br s, 1H), 4.02 (s, 3H), 3.16 (br dd, *J*=10.7, 4.0 Hz, 1H), 3.09 (br s, 1H), 2.80 - 2.63 (m, 2H), 2.50 - 2.39 (m, 2H), 1.94 - 1.74 (m, 2H), 1.65 - 1.45 (m, 1H), 1.45 - 1.24 (m, 2H), 0.88 - 0.67 (m, 2H), 0.36 (br s, 2H). MS (ESI) *m*/*z* = 616.2 (M+H). HPLC Purity: 100 %; Retention Time: 2.12 min. Method C.

### Example 416

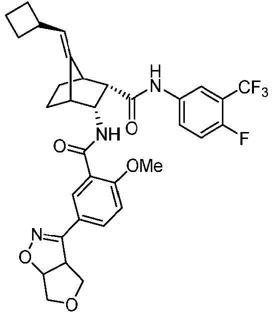

### Intermediate 416-1: (Racemate) and chiral 416-2 (chiral peak-1), 416-3 (Chiral peak-2).

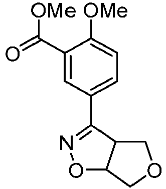

Intermediate **416-1** was prepared in an identical fashion (50 % yield) described for intermediate **378-1.** ¹H NMR (500 MHz, CDCl₃) δ 7.98 (d, J=2.3 Hz, 1H), 7.86 (dd, J=8.8, 2.4 Hz, 1H), 7.04 (d, J=8.7 Hz, 1H), 5.38 (dd, J=9.2, 3.9 Hz, 1H), 4.34 - 4.26 (m, 2H), 4.20 - 4.09 (m, 1H), 3.96 (s, 3H), 3.91 (s, 3H), 3.83 - 3.76 (m, 1H), 2.92 - 2.70 (m, 1H). MS (ESI) *m*/*z* = 278.3 (M+H).

**416-2 & 416-3:** The following chiral intermediates were separated by chiral SFC by the following preparative chromatographic methods from racemate DP39-1: Instrument: Berger MG II Column: Chiralpak IA, 21 x 250 mm, 5 micron, Mobile Phase: 20 % MeOH / 80 % CO₂, Flow Conditions: 45 mL/min, 150 Bar, 40°C, Detector Wavelength: 220 nm to afford chiral **416-2** (Peak-1, > 99 % de, Analytical RT = 3.80 min) and chiral **416-3** (Peak-2, > 98 % de, Analytical RT = 7.43 min). Analytical Chromatographic Conditions: Instrument: Shimadzu Nexera SFC, Column: Chiralpak IA, 4.6 x 100 mm, 3 micron, Mobile Phase: 20% MeOH / 80% CO₂, Flow Conditions: 2.0 mL/min, 150 Bar, 40°C, Detector Wavelength: 220 nm, Injection Details: 5 µL of ~1mg/mL in MeOH.

(1R,2S,3R,4R,Z)-7-(cyclobutylmethylene)-N-(4-fluoro-3-(trifluoromethyl)phenyl)-3-(2-methoxy-5-(3a,4,6,6a-tetrahydrofuro[3,4-d]isoxazol-3-yl)benzamido)bicyclo[2.2.1]heptane-2-carboxamide homochiral isomer-2, **416** was prepared (62 % yield) by the method described for example **378** using the cyclobutyl norbornyl intermediate **369-1** and intermediate **416-2.** ¹H NMR (500 MHz, DMSO-d6) δ 10.56 (s, 1H), 9.93 (dd, *J*=10.8, 7.2 Hz, 1H), 8.25 - 8.20 (m, 2H), 7.84 - 7.76 (m, 2H), 7.48 (t, *J*=9.8 Hz, 1H), 7.28 (d, *J*=8.9 Hz, 1H), 5.35 (dd, *J*=9.0, 3.2 Hz, 1H), 4.70 (d, *J*=9.5 Hz, 1H), 4.54 - 4.41 (m, 2H), 4.12 - 4.02 (m, 3H), 3.90 (br d, *J*=9.5 Hz, 1H), 3.84 - 3.73 (m, 1H), 3.50 (br s, 1H), 3.16 (br dd, *J*=10.8, 4.4 Hz, 1H), 3.11 (br s, 1H), 2.73 (br s, 1H), 2.56 (s, 4H), 1.91 - 1.71 (m, 2H), 1.50 (br s, 1H), 1.42 (br s, 2H), 0.87 - 0.68 (m, 2H), 0.35 (br s, 2H). MS (ESI) *m*/*z* = 614.2 (M+H). HPLC Purity: 100 %; Retention Time: 2.42 min. Method C.

### Example 419

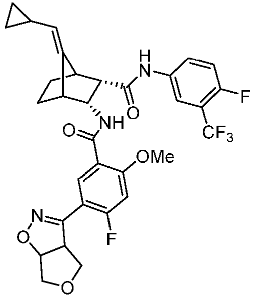

### Intermediate 419-5 (chiral peak-1) and 419-6 (chiral peak-2)

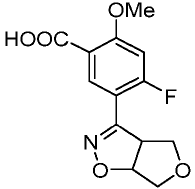

Intermediate **419-1:** To methyl 4-fluoro-5-formyl-2-methoxybenzoate (0.15 g, 0.68 mmol) (prepared as described in Chen, Xiao-Yang, Sorensen, Eric, J. JACS, 2018, 140, 2789-2792) and NH₂OH HCl (48 mg, 0.68 mmol) in DCM (10 mL) was added DIEA (0.12 mL, 0.68 mmol). After 24h, the reaction mixture was diluted with water and white solid (0.15 g, 96%), methyl (E)-4-fluoro-5-((hydroxyimino)methyl)-2-methoxybenzoate, was collected by filtration, dried and used as is. ¹H NMR (400 MHz, CDCl₃) δ 8.53 - 8.37 (m, 1H), 8.33 - 8.22 (m, 2H), 6.79 - 6.61 (m, 1H), 3.94 (s, 3H), 3.91 (s, 3H). MS (ESI) *m*/*z* 228.2 (M+H).⁺

Intermediate **419-2:** To intermediate **419-1** (0.15 g, 0.66 mmol) and DMF (1 mL) was added NCS (88 mg, 0.66 mmol). After 24h, the reaction mixture was partitioned with water (20 mL) and ethyl acetate (50 mL). The aqueous layer was extracted with ethyl acetate (2 x 20 mL). The combined organic layers were washed with brine (15 mL) and dried (Na₂SO₄), to afford a solid To the solid in DCM ( 3 mL), was added 2,5-dihydrofuran (0.46 g, 6.6 mmol) and TEA (0.1 mL, 0.66 mmol). After 24h, the reaction mixture was quenched with water (20 mL) and extracted with DCM (3 x 30 mL). The combined organic layers were washed with brine (15 mL) and dried (MgSO₄). The residue was purified via silica gel chromatography using hexanes/EtOAc as eluents to afford methyl 4-fluoro-2-methoxy-5-(3a,4,6,6a-tetrahydrofuro[3,4-d]isoxazol-3-yl)benzoate (0.13 g, 66%) as a tan solid. MS (ESI) *m*/*z* = 296.2 (M+H).⁺

Chiral Intermediate **419-3 and 419-4:** Intermediate **419-2** was separated on a Jasco SFC Prep with a Chiralpak IA, 21 x 250 mm column eluted with 20% MeOH / 80% CO₂ at 45 mL/min, 150 Bar, 40°C, detector wavelength 267 nm to afford **419-3** (33 mg, 0.11 mmol, 17 % yield) (Peak-1, 99% ee, Analytical RT = 1.693 min.); ¹H NMR (400 MHz, CDCl₃) δ 8.45 (d, *J*=8.8 Hz, 1H), 6.75 (d, *J*=13.2 Hz, 1H), 5.39 (dd, *J*=9.1, 3.9 Hz, 1H), 4.40 (dt, *J*=4.6, 2.3 Hz, 1H), 4.34 (d, *J*=10.8 Hz, 1H), 4.11 (br d, *J*=9.7 Hz, 1H), 3.97 (s, 3H), 3.91 (s, 3H), 3.86 (dd, *J*=9.7, 6.8 Hz, 1H), 3.79 (dd, J=10.8, 4.0 Hz, 1H); **419-4** (32 mg, 0.11 mmol, 16 % yield) (Peak-2, 99% ee,

Analytical RT = 5.463 min.); ¹H NMR (400 MHz,CDCl₃) δ 8.45 (d, *J*=8.6 Hz, 1H), 6.75 (d, *J=*13.4 Hz, 1H), 5.39 (dd, *J*=9.5, 4.0 Hz, 1H), 4.46 - 4.38 (m, 1H), 4.34 (d, *J*=11.0 Hz, 1H), 4.16 - 4.09 (m, 1H), 3.97 (s, 3H), 3.91 (s, 3H), 3.86 (dd, *J*=9.7, 6.8 Hz, 1H), 3.79 (dd, *J*=10.8, 4.0 Hz, 1H). Analytical Chromatographic Conditions: Instrument: Shimadzu Nexera SFC, Column: Chiralpak IC, 4.6 x 100 mm, 3 micron, Mobile Phase: 20% Methanol / 80% CO₂ Flow Conditions: 2.0 mL/min, 150 Bar, 40°C, Detector Wavelength: 220 nm

Intermediate **419-5:** To **419-3** (33 mg, 0.11 mmol) in THF (2 mL)/MeOH (0.1 mL), cooled to 0°C, was added a 2 M aqueous solution of LiOH (0.17 ml, 0.34 mmol). After stirring 18h, the reaction was quenched with dil HCl (10 mL) and extracted with EtOAc (3 x 30 mL). The combined organic layers were washed with brine (15 mL), dried (MgSO₄), filtered and concentrated to afford **419-5** (31 mg, 0.11 mmol, 99 % yield) as a white solid. ¹H NMR (600 MHz, DMSO-d6) δ 12.90 (br s, 1H), 8.07 (d, *J*=8.7 Hz, 1H), 7.17 (d, *J*=13.8 Hz, 1H), 5.34 (dd, *J*=9.2, 3.7 Hz, 1H), 4.49 - 4.42 (m, 1H), 4.09 (d, *J*=10.7 Hz, 1H), 3.90 (br d, *J*=9.7 Hz, 1H), 3.88 (s, 3H), 3.73 (dd, *J*=9.5, 6.9 Hz, 1H), 3.64 (dd, *J*=10.8, 3.7 Hz, 1H). LCMS(ESI) *m*/*z* = 282.2 (M+H).⁺

Intermediate **419-6: 419-6** (30 mg, 0.11 mmol, 96 % yield) was prepared in a similar manner as **419-5** substituting **419-4** for **419-3.** ¹H NMR (400 MHz, CDCl₃) δ 8.62 (d, *J*=8.6 Hz, 1H), 6.86 (d, *J*=12.5 Hz, 1H), 5.40 (dd, *J*=9.2, 4.0 Hz, 1H), 4.55 - 4.28 (m, 2H), 4.11 (s, 3H), 4.08 (s, 1H), 3.87 (dd, *J*=9.7, 6.8 Hz, 1H), 3.79 (dd, *J=*10.8, 4.0 Hz, 1H).

LCMS(ESI) *m*/*z* = 282.2 (M+H).⁺

**Example 419.** (1R,2S,3R,4R,Z)-7-(cyclopropylmethylene)-3-(4-fluoro-2-methoxy-5-(3a,4,6,6a-tetrahydrofuro[3,4-d]isoxazol-3-yl)benzamido)-*N*-(4-fluoro-3-(trifluoromethyl)phenyl)bicyclo[2.2.1]heptane-2-carboxamide, **419** was prepared (5.9 mg, 67 % yield) in a similar manner as example **378,** by using the cyclopropyl norbornyl intermediate **20-4** and intermediate **419-5.** ¹H NMR (500 MHz, DMSO-d₆) δ 10.67 - 10.38 (m, 1H), 9.89 (br d, *J*=7.0 Hz, 1H), 8.33 (br d, *J*=8.9 Hz, 1H), 8.20 (br d, *J=*4.0 Hz, 1H), 7.85 - 7.71 (m, 1H), 7.48 (br t, *J*=9.8 Hz, 1H), 7.22 (br d, *J*=13.1 Hz, 1H), 5.35 (br dd, *J*=9.5, 3.4 Hz, 1H), 4.69 (br d, *J*=9.5 Hz, 1H), 4.52 - 4.36 (m, 2H), 4.10 (br d, *J*=10.7 Hz, 1H), 4.05 (s, 3H), 3.77 - 3.66 (m, 1H), 3.66 - 3.54 (m, 2H), 3.22 - 3.12 (m, 1H), 3.09 (br s, 1H), 2.72 (br s, 1H), 1.90 - 1.79 (m, 1H), 1.79 - 1.66 (m, 1H), 1.50 (br dd, *J*=8.5, 4.3 Hz, 1H), 1.45 - 1.32 (m, 2H), 0.87 - 0.61 (m, 2H), 0.35 (br d, *J*=2.7 Hz, 2H). HPLC purity 98 %. Analytical LC-MS: 2.48 min; MS (ESI) *m*/*z* = 631.9 (M+H).⁺ Method B.

### Example 423

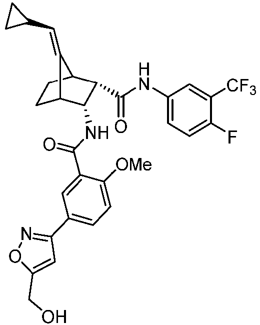

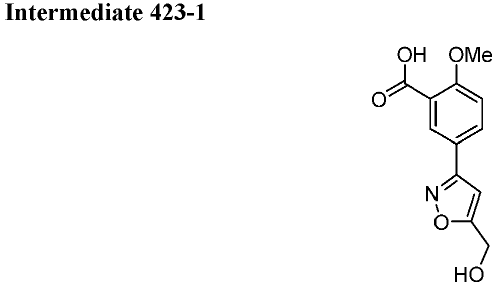

Intermediate **423-1** was prepared in an identical fashion described for intermediate **378-3** which in this case by substituting allyl alcohol with propargyl alcohol. ¹H NMR (500 MHz, CD₃OD) δ 8.28 (d, J=2.3 Hz, 1H), 8.01 (dd, J=8.7, 2.3 Hz, 1H), 7.27 (d, J=8.7 Hz, 1H), 6.75 (s, 1H), 4.91 - 4.82 (m, 5H), 4.73 (s, 2H), 4.00 - 3.96 (m, 3H). MS (ESI) *m*/*z* = 250.3 (M+H).

(1R,2S,3R,4R,Z)-7-(cyclopropylmethylene)-N-(4-fluoro-3-(trifluoromethyl)phenyl)-3-(5-(5-(hydroxymethyl)isoxazol-3-yl)-2-methoxybenzamido)bicyclo [2.2.1]heptane-2-carboxamide, **423** was prepared (77 % yield) by the coupling method described for example **378** using the norbornyl intermediate **20-4** and intermediate **423-1.** ¹H NMR (500 MHz, DMSO-d6) δ 10.56 (s, 1H), 9.95 (br d, *J*=7.0 Hz, 1H), 8.40 (d, *J*=1.8 Hz, 1H), 8.19 (br d, *J*=4.3 Hz, 1H), 7.97 (dd, *J*=8.5, 2.1 Hz, 1H), 7.77 (br d, *J*=8.9 Hz, 1H), 7.46 (br t, *J*=9.8 Hz, 1H), 7.32 (d, *J*=8.5 Hz, 1H), 6.84 (s, 1H), 4.69 (d, *J*=9.8 Hz, 1H), 4.61 (d, *J*=5.8 Hz, 2H), 4.45 (br s, 1H), 4.05 (s, 3H), 3.21 - 3.06 (m, 2H), 2.72 (br s, 1H), 1.92 - 1.73 (m, 2H), 1.62 - 1.45 (m, 1H), 1.41 (br s, 2H), 0.84 - 0.67 (m, 2H), 0.35 (br d, *J*=4.3 Hz, 2H). MS (ESI) *m*/*z* = 600.1 (M+H). HPLC Purity: 100 %; Retention Time: 2.39 min; Method B.

### Example 427

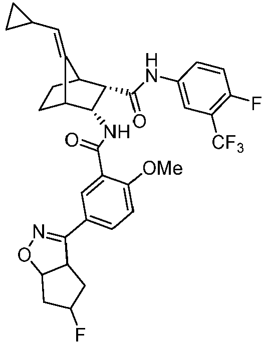

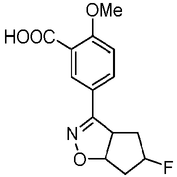

Preparation of methyl 5-(5-fluoro-3a,5,6,6a-tetrahydro-4*H*-cyclopenta[d]isoxazol-3-yl)-2-methoxybenzoate (diastereomer mixture). To **406-1** ester (0.1 g, 0.3 mmol) in DCM (2 mL) was added DAST (0.05 mL, 0.412 mmol). After 24 h, the reaction mixture was concentrated under reduced pressure and purified by silica gel chromatography to afford the corresponding fluoride (66 mg, 0.23 mmol, 66 % yield) as a clear film. ¹H NMR (400 MHz, CDCl₃) δ 8.06 (d, *J*=2.4 Hz, 1H), 7.88 (dd, *J*=8.7, 2.3 Hz, 1H), 7.06 (d, J=8.8 Hz, 1H), 5.53 - 5.38 (m, 1H), 4.25 (dd, *J*=9.5, 2.0 Hz, 1H), 4.01 - 3.97 (m, 4H), 3.94 - 3.92 (m, 3H), 2.76 - 2.47 (m, 2H), 2.33 - 2.12 (m, 1H), 2.10 - 1.90 (m, 1H). LCMS(ESI) *m*/*z* = 294.2 (M+H).⁺

Intermediate **427-2:** Preparation of 5-(5-fluoro-3a, 5,6,6a-tetrahydro-4H-cyclopenta[d]isoxazol-3-yl)-2-methoxybenzoic acid. To intermediate **427-1** (14 mg, 0.048 mmol) in THF (1 mL) was added a 2M aqueous solution of LiOH (72 µl, 0.14 mmol). After 24 h, dil HCl (10 mL) was added and the solution extracted with EtOAc (3 x 30 mL). The combined organic layers were washed with brine (15 mL), dried (MgSO₄), filtered and concentrated under reduced pressure to afford **427-2** (13 mg, 0.047 mmol, 98 % yield). ¹H NMR (400 MHz, CDCl₃) δ 8.28 (d, *J*=2.2 Hz, 1H), 8.13 (dd, *J*=8.8, 2.4 Hz, 1H), 7.20 - 7.12 (m, 1H), 5.95 - 5.83 (m, 1H), 5.45 (ddd, *J*=10.0, 6.8, 4.7 Hz, 1H), 5.38 - 5.18 (m, 1H), 4.28 (td, *J*=9.4, 7.5 Hz, 1H), 4.16 (s, 3H), 2.75 - 2.55 (m, 2H), 2.32 - 2.17 (m, 1H), 2.06 - 1.92 (m, 1H). LCMS(ESI) *m*/*z* = 280.2 (M+H).⁺

(1R,2S,3R,4R,Z)-7-(cyclopropylmethylene)-N-(4-fluoro-3-(trifluoromethyl)phenyl)-3-(5-(5-fluoro-3a,5,6,6a-tetrahydro-4H-cyclopenta[d]isoxazol-3-yl)-2-methoxybenzamido)bicyclo[2.2.1]heptane-2-carboxamide, diasteromeric mixture, **427** was prepared (5.7 mg, 9.1 µmol, 67 % yield) using the cyclopropyl norbornyl intermediate **20-4** in a similar manner as Example **378,** by using the cyclopropyl norbornyl intermediate **20-4** and intermediate **427-2.** ¹H NMR (500 MHz, DMSO-d₆) δ 10.69 - 10.39 (m, 1H), 9.92 (br t, *J*=7.0 Hz, 1H), 8.49 - 8.08 (m, 2H), 7.91 - 7.71 (m, 2H), 7.50 (br t, *J*=9.6 Hz, 1H), 7.29 (d, J=8.9 Hz, 1H), 5.45 - 5.26 (m, 1H), 4.71 (br d, *J*=9.2 Hz, 1H), 4.46 (br s, 1H), 4.39 - 4.30 (m, 1H), 4.06 (d, *J*=2.4 Hz, 3H), 3.41 (br s, 1H), 3.18 (br dd, *J*=10.8, 3.5 Hz, 1H), 3.12 (br s, 1H), 2.74 (br s, 1H), 2.51 - 2.35 (m, 2H), 2.17 - 2.06 (m, 1H), 2.06 - 2.00 (m, 1H), 1.92 - 1.84 (m, 1H), 1.80 (br d, *J*=11.3 Hz, 1H), 1.61 - 1.50 (m, 1H), 1.49 - 1.36 (m, 2H), 0.86 - 0.68 (m, 2H), 0.37 (br s, 2H). HPLC purity 100 %. Analytical LC-MS: 2.65 min; MS (ESI) *m*/*z* = 630.3 (M+H). ⁺Method B.

**428** was prepared (6.1 mg, 69 % yield) in a similar manner as example **379,** by using the cyclopropyl norbornyl intermediate **20-4** and intermediate **428-1.** ¹H NMR. HPLC purity 100 %. Analytical LC-MS: 2.84 min; MS (ESI) *m*/*z* = 638.2 (M+H). ⁺Method B.

### Example 429

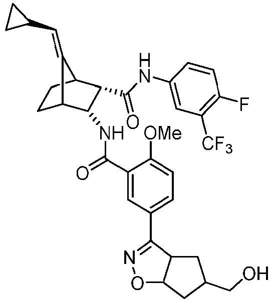

### Intermediate 429-1: Preparation of methyl 5-(5-(hydroxymethyl)-3a,5,6,6a-tetrahydro-4H-cyclopenta[d]isoxazol-3-yl)-2-methoxybenzoate.

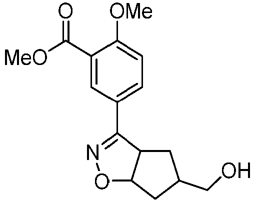

Intermediate **429-1** was prepared in an identical fashion (75 % yield) described for intermediate **378-3** which in this case by substituting allyl alcohol with cyclopent-3-en-1-ylmethanol.

### Intermediate 429-2: Preparation of 5-(5-(hydroxymethyl)-3a,5,6,6a-tetrahydro-4H-cyclopenta[d]isoxazol-3-yl)-2-methoxybenzoic acid.

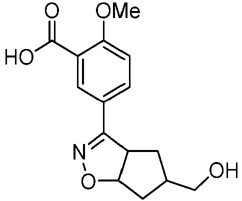

Methyl 5-(5-(hydroxymethyl)-3a,5,6,6a-tetrahydro-4H-cyclopenta[d]isoxazol-3-yl)-2-methoxybenzoate (58 mg, 0.22 mmol) was dissolved in THF (1 mL)/MeOH (1 mL) was treated with LiOH monohydrate (36 mg, 0.86 mmol) in H₂O (1 mL) at rt. After 3h, the reaction mixture was diluted with H₂O (5 mL) and liberated of organics. The pH of the remaining aq. layer was adjusted to pH 7 with 1M HCl solution, extracted with EtOAc (2 x 25 mL), washed with brine, dried (Na₂SO₄), filtered, and evaporated to give intermediate **429-2** (62 mg, 74.2%). The carboxlic acid was carried forward to the next reaction without further purification. MS (ESI) *m*/*z* = 292.3 (M+H).

**Example 429** was prepared by the coupling of intermediate **429-2** (3.95 mg, 0.014 mmol) with intermediate **166-2** (5 mg, 0.014 mmol) dissolved in anhydrous DMF (2 mL) the presence of DIEA (0.012 mL, 0.068 mmol) and BOP (6.60 mg, 0.015 mmol). After 3 h, the reaction mixture was filtered and purified by reverse phase preparative HPLC to give desired product (1R,2S,3R,4R,Z)-7-(cyclopropylmethylene)-N-(4-fluoro-3-(trifluoromethyl)phenyl)-3-(5-(5-(hydroxymethyl)-3a,5,6,6a-tetrahydro-4H-cyclopenta[d]isoxazol-3-yl)-2-methoxybenzamido)bicyclo[2.2.1]heptane-2-carboxamide, diasteromeric mixture, **429** (5.1 mg, 0.0079 mmol, 58 % yield). ¹H NMR (500 MHz, DMSO-d6) δ 10.53 (s, 1H), 9.90 (br d, *J*=6.4 Hz, 1H), 8.23 - 8.17 (m, 2H), 7.81 - 7.74 (m, 2H), 7.46 (br t, *J*=9.8 Hz, 1H), 7.26 (d, *J*=8.9 Hz, 1H), 5.15 - 5.07 (m, 1H), 4.68 (br d, *J*=9.5 Hz, 1H), 4.42 (br s, 1H), 4.20 - 4.14 (m, 1H), 4.02 (s, 3H), 3.58 - 3.47 (m, 2H), 3.39 - 3.18 (m, 2H), 3.17 - 3.06 (m, 2H), 2.73 - 2.68 (m, 1H), 1.99 - 1.73 (m, 5H), 1.66 - 1.59 (m, 1H), 1.56 - 1.37 (m, 4H), 0.78 - 0.68 (m, 2H), 0.38 - 0.29 (m, 2H). HPLC purity: 99.2 %. Analytical LC-MS: 2.53 min; MS (ESI) *m*/*z* = 642.2 (M+H); Method B.

### Example 430

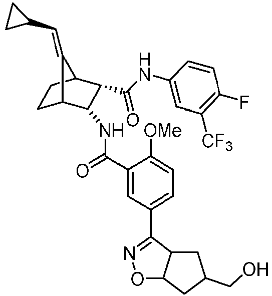

### Intermediate 429-4 (chiral peak-1), 429-6 (chiral peak-2), 429-8 (chiral peak-3), and 429-10 (chiral peak-4)

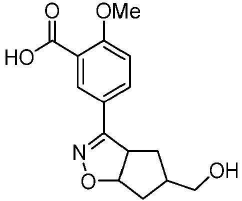

Individual chiral diastereomer ester intermediates **429-4A, 429-6A, 429-8A,** and **429-10A** were obtained by chiral SFC of diasteremeric mixture intermediate **429** (524.9 mg, 1.72 mmol). Chiral SFC Preparative chromatographic conditions: Instrument: Berger MG II (SFC); Column: Chiralpak AD-H, 21 x 250 mm, 5 micron; Mobile phase: 15% MeOH / 85% CO₂; Flow conditions: 45 mL/min, 150 Bar, 40°C; Detector wavelength: 210 nm; Injections details: 0.5 mL of ~35mg/mL in MeOH. Analytical chromatographic conditions: Instrument: Shimadzu Nexera SFC; Column: Chiralpak AD-H, 4.6 x 100 mm, 3 micron; Mobile phase: 15% MeOH / 85% CO2; Flow conditions: 2.0 mL/min, 150 Bar, 40°C; Detector wavelength: 220 nm; Injection details: 5 µL of ~1mg/mL in MeOH.

Intermediate **429-4A** (Peak-1, >99% de, analytical RT = 4.02 min) was obtained as a film (152.8 mg, 29.1%). ¹H NMR (600 MHz, CDCl₃) δ 8.04 (d, *J*=2.3 Hz, 1H), 7.87 (dd, *J*=8.7, 2.3 Hz, 1H), 7.01 (d, J=8.8 Hz, 1H), 5.23 (dd, *J*=8.8, 5.1 Hz, 1H), 4.10 (t, *J*=8.7 Hz, 1H), 3.94 (s, 3H), 3.90 (s, 3H), 3.72 - 3.66 (m, 1H), 3.61 (dt, *J*=10.5*,* 5.2 Hz, 1H), 2.30 - 2.16 (m, 2H), 2.05 (dd, *J*=13.0, 6.1 Hz, 1H), 1.76 (ddd, *J*=12.9*,* 11.5, 9.4 Hz, 1H), 1.68 - 1.62 (m, 1H), 1.39 (br t, *J*=4.8 Hz, 1H).

Intermediate **429-4** (104.4 mg, 78%) was prepared in a similar manner as intermediate **429-2** with the hydrolysis of intermediate **429-4A.** MS (ESI) *m*/*z* = 292.3 (M+H).

Intermediate **429-6A** (Peak-2, >99% de, analytical RT = 4.56 min) was obtained as a film (33.2 mg, 6.3%). ¹H NMR (600 MHz, CDCl₃) δ 8.05 (d, *J*=2.3 Hz, 1H), 7.87 (dd, J=8.8, 2.3 Hz, 1H), 7.02 (d, *J*=8.8 Hz, 1H), 5.25 (ddd, *J*=10.1, 6.2, 4.2 Hz, 1H), 4.04 - 3.98 (m, 1H), 3.94 (s, 3H), 3.90 (s, 3H), 3.63 - 3.57 (m, 1H), 3.56 - 3.50 (m, 1H), 2.38 - 2.26 (m, 3H), 1.92 - 1.85 (m, 1H), 1.73 - 1.66 (m, 1H), 1.51 (t, *J*=5.3 Hz, 1H). Intermediate **429-6** (20.2 mg, 92%) was prepared in a similar manner as intermediate **429-2** with the hydrolysis of intermediate **429-6a.** MS (ESI) *m*/*z =* 292.3 (M+H).

Intermediate **429-8A** (Peak-3, >99% de, analytical RT = 5.67 min) was obtained as a film (160.8 mg, 30.6%). ¹H NMR: (600 MHz, CDCl₃) δ 8.05 - 8.03 (m, 1H), 7.86 (dd, J=8.7, 2.3 Hz, 1H), 7.01 (d, *J*=8.8 Hz, 1H), 5.23 (dd, *J*=8.7, 5.2 Hz, 1H), 4.10 (t, *J*=8.7 Hz, 1H), 3.94 (s, 3H), 3.90 (s, 3H), 3.69 (br dd, *J*=10.6, 5.2 Hz, 1H), 3.63 - 3.58 (m, 1H), 2.28 - 2.17 (m, 2H), 2.05 (br dd, *J*=12.9i*,* 6.2 Hz, 1H), 1.76 (ddd, *J*=13.0i*,* 11.5, 9.4 Hz, 1H), 1.64 - 1.60 (m, 1H), 1.49 (br s, 1H).

Intermediate **429-8** (121 mg, 85%) was prepared in a similar manner as intermediate **429-2** with the hydrolysis of intermediate **429-8a.** MS (ESI) *m*/*z* = 292.3 (M+H).

Intermediate **429-10A** (Peak-4, >99% de, analytical RT = 9.78 min) was obtained as a film (47.1 mg, 9.0%). ¹H NMR: (600 MHz, CDCl₃) δ 8.04 (d, J=2.3 Hz, 1H), 7.87 (dd, J=8.7, 2.3 Hz, 1H), 7.02 (d, J=8.8 Hz, 1H), 5.24 (ddd, J=10.1, 6.2, 4.2 Hz, 1H), 4.03 - 3.98 (m, 1H), 3.94 (s, 3H), 3.90 (s, 3H), 3.63 - 3.57 (m, 1H), 3.56 - 3.49 (m, 1H), 2.38 - 2.25 (m, 3H), 1.91 - 1.85 (m, 1H), 1.72 - 1.66 (m, 1H), 1.55 (br s, 1H).

Intermediate **429-10** ( 18.2 mg, 51.6%) was prepared in a similar manner as intermediate **429-2** with the hydrolysis of intermediate **429-10A.** MS (ESI) *m*/*z* = 292.3 (M+H).

**Example 430** was prepared in a similar manner as example **429** with intermediate **429-4** (Peak-1 from SFC). (1R,2S,3R,4R,Z)-7-(cyclopropylmethylene)-N-(4-fluoro-3-(trifluoromethyl)phenyl)-3-(5-(5-(hydroxymethyl)-3a,5,6,6a-tetrahydro-4H-cyclopenta[d] isoxazol-3-yl)-2-methoxybenzamido)bicyclo[2.2.1]heptane-2-carboxamide homochiral isomer-1 (10.5 mg, 0.016 mmol, 60.3 % yield). ¹H NMR (500 MHz, DMSO-d6) δ 10.53 (s, 1H), 9.90 (br d, *J*=7.0 Hz, 1H), 8.23 - 8.18 (m, 2H), 7.81 - 7.75 (m, 2H), 7.47 (br t, *J*=9.5 Hz, 1H), 7.26 (d, *J*=8.5 Hz, 1H), 5.11 (br dd, J*=*8.2, 5.5 Hz, 1H), 4.68 (d, *J*=9.8 Hz, 1H), 4.46 - 4.39 (m, 1H), 4.22 - 4.13 (m, 1H), 4.03 (s, 3H), 3.49 - 3.28 (m, 1H), 3.19 - 3.05 (m, 2H), 2.73 - 2.68 (m, 1H), 1.98 (br dd, *J*=13.6, 5.0 Hz, 1H), 1.93 - 1.74 (m, 4H), 1.69 - 1.60 (m, 1H), 1.58 - 1.36 (m, 4H), 0.77 - 0.68 (m, 2H), 0.37 - 0.30 (m, 2H). HPLC purity: 100 %. Analytical LC-MS: 2.3 min; MS (ESI) *m*/*z* = 642.3 (M+H); Method B.

### Example 434

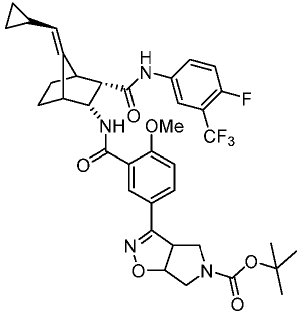

### Intermediate 434-2 (diastereomeric mixture)

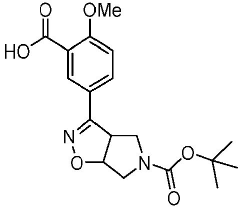

### Intermediate 434-1: Preparation of tert-butyl 3-(4-methoxy-3-(methoxycarbonyl)phenyl)-3a,4,6,6a-tetrahydro-5H-pyrrolo[3,4-d]isoxazole-5-carboxylate.

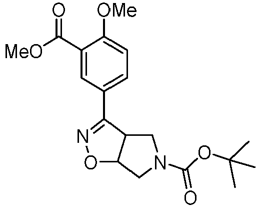

Intermediate **434-1** (499.5 mg, 46%) was prepared by the method described for intermediate **429-1** which in this case by substituting cyclopent-3-en-1-ylmethanol with tert-butyl 2,5-dihydro-1H-pyrrole-1-carboxylate. ¹H NMR: (400 MHz, CDCl₃) δ 7.99 (d, *J*=2.4 Hz, 1H), 7.84 (dd, *J*=8.7, 2.3 Hz, 1H), 7.04 (d, *J*=8.8 Hz, 1H), 5.31 (ddd, *J*=9.2, 5.4, 1.2 Hz, 1H), 4.21 (br dd, *J*=12.4, 9.1 Hz, 1H), 3.96 (s, 3H), 3.91 (s, 3H), 3.72 - 3.61 (m, 2H), 1.43 (s, 9H). MS (ESI) *m*/*z* = 377.4 (M+H).

Intermediate **434-2:** Preparation of 5-(5-(tert-butoxycarbonyl)-3a,5,6,6a-tetrahydro-4H-pyrrolo[3,4-d]isoxazol-3-yl)-2-methoxybenzoic acid. **434-2** (151.4 mg, 43.7 % over three steps) was prepared by the method described for intermediate **429-2** replacing intermediate **429-1** with intermediate **434-1.** MS (ESI) *m*/*z* = 363.4 (M+H).

### Intermediates 434-4 and 434-6 (homochiral)

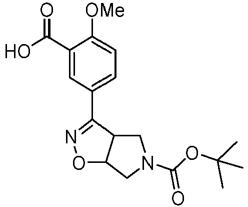

Intermediates **434-3** and **434-4** were obtained by chiral SFC of diastereomeric mixture intermediate **434-2** (499 mg, 1.33 mmol). Chiral SFC Preparative chromatographic conditions: Instrument: Berger MG II (SFC); Column: Regis Whelk-01, 21 x 250 mm, 5 micron; Mobile phase: 15 % MeOH / 85 % CO₂; Flow conditions: 45 mL/min, 150 Bar, 40°C; Detector wavelength: 220 nm; Injections details: 1.0 mL of ~31mg/mL in MeOH-ACN. Analytical chromatographic conditions: Instrument: Shimadzu Nexera SFC; Column: Regis Whelk-01, 4.6 x 100 mm, 3 micron; Mobile phase: 15% MeOH / 85% CO₂; Flow conditions: 2.0 mL/min, 150 Bar, 40°C; Detector wavelength: 220 nm; Injection details: 5 µL of ~1mg/mL in Acetonitrile.

Intermediate **434-3** (Peak-1, > 99 % de, analytical RT = 4.02 min) was obtained as a white solid (95.9 mg, 19.2 % yield). ¹H NMR: (600 MHz, CDCl₃) δ 7.99 (d, *J*=2.3 Hz, 1H), 7.86 - 7.82 (m, 1H), 7.04 (br d, *J*=8.7 Hz, 1H), 5.31 (ddd, *J*=9.2, 5.4, 1.3 Hz, 1H), 4.24 - 4.18 (m, 1H), 4.01 - 3.93 (m, 4H), 3.91 (s, 3H), 3.83 - 3.76 (m, 1H), 3.71 - 3.67 (m, 1H), 3.63 (br s, 1H), 1.43 (br s, 9H).

Intermediate **434-4.** Preparation of 5-(5-(tert-butoxycarbonyl)-3a,5,6,6a-tetrahydro-4H-pyrrolo[3,4-d]isoxazol-3-yl)-2-methoxybenzoic acid. Intermediate **434-4** (52 mg, 67.5 % yield) was prepared in a similar manner as intermediate **434-2** with the hydrolysis of intermediate **434-3.** MS (ESI) *m*/*z* = 363.1 (M+H).

Intermediate **434-5** (Peak-2, 99.6 % de, analytical RT = 4.56 min) was obtained as a white solid (96.7 mg, 19.4 % yield). 1H NMR (600 MHz, CDCl₃) δ 7.98 (d, *J*=2.3 Hz, 1H), 7.83 (dd, *J*=8.8, 2.2 Hz, 1H), 7.03 (d, *J*=8.7 Hz, 1H), 5.32 - 5.28(m, 1H), 4.21 (td, *J*=8.8, 4.0 Hz, 1H), 4.01 - 3.94 (m, 1H), 3.95 (s, 3H), 3.90 (s, 3H), 3.83 - 3.73 (m, 1H), 3.68 (dd, *J*=11.4, 8.9 Hz, 1H), 3.65 - 3.58 (m, 1H), 1.43 (s, 9H). Intermediate **434-6.** Preparation of 5-(5-(tert-butoxycarbonyl)-3a,5,6,6a-tetrahydro-4H-pyrrolo[3,4-d]isoxazol-3-yl)-2-methoxybenzoic acid. Intermediate **434-6** (48 mg, 62.3 % yield) was prepared in a similar manner as intermediate **434-2** with the hydrolysis of intermediate **434-5.** MS (ESI) *m*/*z* = 363.1 (M+H).

Example **434** was prepared in a similar manner as example **429** replacing intermediate **429-2** with intermediate **434-2.** tert-butyl 3-(3-(((1R,2R,3S,4R,Z)-7-(cyclopropylmethylene)-3-((4-fluoro-3-(trifluoromethyl)phenyl) carbamoyl) bicyclo[2.2.1]heptan-2-yl)carbamoyl)-4-methoxyphenyl)-3a,4,6,6a-tetrahydro-5H-pyrrolo[3,4-d]isoxazole-5-carboxylate diasteromeric mixture, **434** (7.1 mg, 0.0098 mmol, 72.3 % yield, diastereomeric mixture). ¹H NMR (500 MHz, DMSO-d6) δ 10.53 (s, 1H), 9.91 (br d, *J*=6.7 Hz, 1H), 8.21 (br s, 2H), 7.78 (br d, *J*=7.0 Hz, 2H), 7.47 (br t, *J*=9.5 Hz, 1H), 7.28 (d, J=8.5 Hz, 1H), 5.25 (br dd, J=8.9, 4.9 Hz, 1H), 4.68 (br d, J=9.5 Hz, 1H), 4.46 - 4.39 (m, 2H), 4.04 (d, J=3.1 Hz, 3H), 3.80 - 3.69 (m, 1H), 3.15 (br dd, J=11.0, 3.7 Hz, 1H), 3.10 (br d, J=3.4 Hz, 1H), 2.73 - 2.68 (m, 1H), 1.90 (s, 1H), 1.85 - 1.74 (m, 2H), 1.55 - 1.15 (m, 14H), 0.79 - 0.67 (m, 2H), 0.40 - 0.24 (m, 2H). HPLC purity: 98.5 %. Analytical LC-MS: 2.81 min; MS (ESI) *m*/*z* = 713.2 (M+H); Method B.

### Example 437

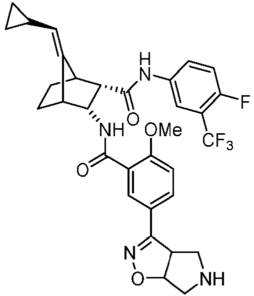

Prepared by the coupling of intermediate **434-2** (9.84 mg, 0.027 mmol) with intermediate **166-2** (10 mg, 0.027 mmol) dissolved in anhydrous THF (2 mL) the presence of DIEA (0.024 mL, 0.136 mmol) and BOP (13.21 mg, 0.030 mmol). After 1 h, the reaction mixture was concentrated, dissolved in DCM (1 mL), and treated with 50 % TFA/DCM (1 mL). After 1h, the reaction mixture was concentrated under reduced pressure and purified by reverse phase preparative HPLC to give **437** (1R,2S,3R,4R,Z)-7-(cyclopropylmethylene)-N-(4-fluoro-3-(trifluoromethyl)phenyl)-3-(2-methoxy-5-(3a,5,6,6a-tetrahydro-4H-pyrrolo[3,4-d]isoxazol-3-yl)benzamido)bicyclo[2.2.1]heptane-2-carboxamide diastereomeric mixture, (10.3 mg, 0.0140 mmol, 51.5 % yield). ¹H NMR (500 MHz, DMSO-d6) δ 10.58 - 10.55 (m, 1H), 9.94 (dd, *J*=18.8, 7.1 Hz, 1H), 8.25 (dd, *J*=10.2, 2.0 Hz, 1H), 8.22 - 8.17 (m, 1H), 7.83 - 7.78 (m, 1H), 7.78 - 7.74 (m, 1H), 7.46 (br t, *J*=9.5 Hz, 1H), 7.29 (d, *J*=8.7 Hz, 1H), 5.43 (dd, *J*=9.3, 4.6 Hz, 1H), 4.69 - 4.64 (m, 2H), 4.44 - 4.37 (m, 1H), 4.04 (d, *J*=1.7 Hz, 3H), 3.72 - 3.65 (m, 2H), 3.46 - 3.38 (m, 1H), 3.16 - 3.11 (m, 1H), 3.09 - 3.05 (m, 1H), 2.73 - 2.68 (m, 1H), 1.83 - 1.70 (m, 2H), 1.51 - 1.34 (m, 4H), 0.76 - 0.66 (m, 2H), 0.33 (br d, J=3.2 Hz, 2H). HPLC purity: 98.6 %. Analytical LC-MS: 2.32 min; MS (ESI) *m*/*z* = 613.2 (M+H); Method C.

### Example 438

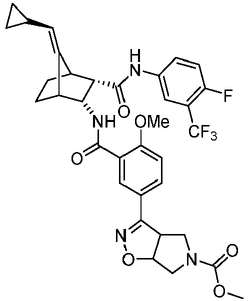

Intermediate **434-2** (9.84 mg, 0.027 mmol) and cyclopropyl norbornyl intermediate **166-2** (10 mg, 0.027 mmol) were dissolved in anhydrous THF (2.0 mL), then DIEA (0.024 mL, 0.136 mmol) and BOP (13.21 mg, 0.030 mmol) were added. After 2 h, the reaction mixture was concentrated, the resulting residue was re-dissolved in DCM (0.25 mL), and treated with 50% TFA/DCM (0.25 mL). After 1h, the reaction mixture was concentrated to dryness. The amine was dissolved in THF (2.0 mL) and treated with TEA (0.019 mL, 0.13 mmol) followed by methyl chloroformate (2.6 mg, 0.027 mmol) at 0°C. After stirring 2h at rt, the reaction mixture was concentrated under reduced pressure and purified by preparative RP-HPLC to give methyl 3-(3-(((1R,2R,3S,4R,Z)-7-(cyclopropylmethylene)-3-((4-fluoro-3-(trifluoromethyl)phenyl) carbamoyl)bicyclo[2.2.1]heptan-2-yl)carbamoyl)-4-methoxyphenyl)-3a,4,6,6a-tetrahydro-5H-pyrrolo[3,4-d]isoxazole-5-carboxylate (diastereomeric mixture), **438** (2.6 mg, 0.0036 mmol, 14.2 % yield). ¹H NMR (500 MHz, DMSO-d6) δ 10.54 (s, 1H), 9.92 (br t, *J*=6.1 Hz, 1H), 8.18 (br s, 2H), 7.81 - 7.74 (m, 2H), 7.45 (br t, *J*=9.8 Hz, 1H), 7.29 - 7.26 (m, 1H), 5.28 (br dd, *J*=8.5, 4.9 Hz, 1H), 4.68 (br d, *J*=9.5 Hz, 1H), 4.48 - 4.38 (m, 2H), 4.03 (d, *J*=3.1 Hz, 2H), 3.81 - 3.74 (m, 1H), 3.64 - 3.48 (m, 4H), 3.17 - 3.06 (m, 2H), 2.73 - 2.66 (m, 1H), 1.84 - 1.72 (m, 2H), 1.52 - 1.33 (m, 4H), 0.77 - 0.67 (m, 2H), 0.33 (br d, J=3.4 Hz, 2H). HPLC purity: 99.1 %. Analytical LC-MS: 2.48 min; MS (ESI) *m*/*z* = 671.1 (M+H); Method B.

### Example 439

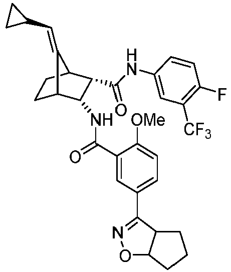

### Intermediate 439-1: Preparation of methyl 2-methoxy-5-(3a,5,6,6a-tetrahydro-4H-cyclopenta[d]isoxazol-3-yl)benzoate.

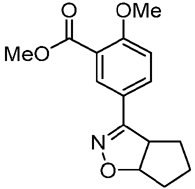

Intermediate **439-1** was prepared in an identical fashion (128 mg, 23% yield) described for intermediate **378-3** which in this case by substituting allyl alcohol with cyclopentene.

### Intermediate 439-2: Preparation of 2-methoxy-5-(3a,5,6,6a-tetrahydro-4H-cyclopenta[d]isoxazol-3-yl)benzoic acid.

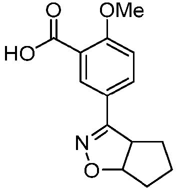

Intermediate **439-2** (45.2 mg, 60.3%) was prepared in a similar manner as intermediate **429-2** with the hydrolysis of intermediate **439-1.** MS (ESI) *m*/*z* = 262.2 (M+H).

Individual chiral diastereomer ester intermediates **439-4A (chiral peak-1)** and **439-6A (chiral peak-2)** were obtained by chiral SFC of diasteremeric mixture intermediate **439-1** (128 mg, 0.465 mmol). Chiral SFC Preparative chromatographic conditions: Instrument: Jasco SFC Prep; Column: Chiralpak OJ-H, 21 x 250 mm, 5 micron; Mobile phase: 5% IPA / 95% CO₂; Flow conditions: 45 mL/min, 150 Bar, 40°C; Detector wavelength: 220 nm; Injections details: 0.5 mL of ~35mg/mL in IPA-ACN. Analytical chromatographic conditions: Instrument: Shimadzu Nexera SFC; Column: Chiralpak OJ-H, 4.6 x 100 mm, 3 micron; Mobile phase: 10% IPA / 90% CO₂; Flow conditions: 2.0 mL/min, 150 Bar, 40°C; Detector wavelength: 220 nm; Injection details: 5 µL of ~1mg/mL in MeOH.

Intermediate **439-4A** (Peak-1, >99% de, analytical RT = 2.84 min) was obtained as a film (48.8 mg, 38.1%). ¹H NMR: (400 MHz, chloroform-d) δ 8.06 (d, *J*=2.4 Hz, 1H), 7.86 (dd, *J*=8.8, 2.2 Hz, 1H), 7.01 (d, *J*=8.8 Hz, 1H), 5.21 (dd, *J*=8.8, 4.6 Hz, 1H), 4.03 (td, *J*=8.4, 3.0 Hz, 1H), 3.94 (s, 3H), 3.90 (s, 3H), 2.21 - 2.14 (m, 1H), 1.94 - 1.85 (m, 2H), 1.83 - 1.72 (m, 2H), 1.60 - 1.47 (m, 1H).

Intermediate **439-4** ( 41.9 mg, 90%) was prepared in a similar manner as intermediate **429-2** with the hydrolysis of intermediate **439-4A.** MS (ESI) *m*/*z* = 262.3 (M+H).

Intermediate **439-6A** (Peak-2, >95%de, analytical RT = 3.60 min) was obtained as a film (51.5 mg, 40.2%). ¹H NMR: (400 MHz, CDCl₃) δ 8.06 (d, J=2.4 Hz, 1H), 7.86 (dd, J=8.8, 2.4 Hz, 1H), 7.01 (d, J=8.8 Hz, 1H), 5.21 (dd, J=8.8, 4.6 Hz, 1H), 4.07 - 4.00 (m, 1H), 3.94 (s, 3H), 3.90 (s, 3H), 2.21 - 2.15 (m, 1H), 1.94 - 1.87 (m, 2H), 1.83 - 1.71 (m, 2H), 1.60 - 1.49 (m, 1H).

Intermediate **439-6** (45.3 mg, 93%) was prepared in a similar manner as intermediate **429-2** with the hydrolysis of intermediate **439-6A.** MS (ESI) *m*/*z* = 262.3 (M+H).

Example **439** was prepared in a similar manner as example **429** replacing intermediate **429-2** with intermediate **439-2.** (1R,2S,3R,4R,Z)-7-(cyclopropylmethylene)-N-(4-fluoro-3-(trifluoromethyl)phenyl)-3-(2-methoxy-5-(3a,5,6,6a-tetrahydro-4H-cyclopenta[d]isoxazol-3-yl)benzamido)bicyclo[2.2.1]heptane-2-carboxamide diastereomeric mixture, **439** (6.2 mg, 0.010 mmol, 74.2 % yield). ¹H NMR (500 MHz, DMSO-d6) δ 10.57 - 10.50 (m, 1H), 9.93 - 9.85 (m, 1H), 8.23 - 8.17 (m, 2H), 7.80 - 7.73 (m, 2H), 7.49 - 7.44 (m, 1H), 7.28 - 7.24 (m, 1H), 5.16 - 5.09 (m, 1H), 4.71 - 4.66 (m, 1H), 4.46 - 4.37 (m, 1H), 4.18 - 4.12 (m, 1H), 4.05 - 3.99 (m, 3H), 3.17 - 3.06 (m, 2H), 2.73 - 2.67 (m, 1H), 1.99 - 1.90 (m, 1H), 1.86 - 1.63 (m, 6H), 1.52 - 1.26 (m, 4H), 0.78 - 0.68 (m, 2H), 0.39 - 0.29 (m, 2H). HPLC purity: 99.4 %. Analytical LC-MS: 2.82 min; MS (ESI) *m*/*z* 612.2 (M+H); Method B.

Prepared in a similar manner as example **429** replacing intermediate **429-2** with intermediate **439-6** (Peak-2 from SFC). ). (1R,2S,3R,4R,Z)-7-(cyclopropylmethylene)-N-(4-fluoro-3-(trifluoromethyl)phenyl)-3-(2-methoxy-5-(3a,5,6,6a-tetrahydro-4H-cyclopenta[d]isoxazol-3-yl)benzamido)bicyclo[2.2.1]heptane-2-carboxamide homochiral isomer-2, **441** (10.9 mg, 0.017 mmol, 63.1 % yield). ¹H NMR. HPLC purity: 100 %. Analytical LC-MS: 2.71 min; MS (ESI) *m*/*z* = 612.3 (M+H); Method B.

### Example 442

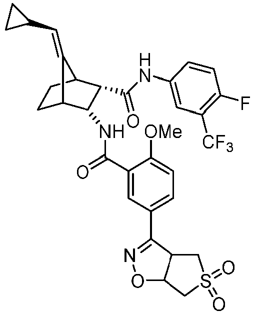

### Intermediate 442-1: Preparation of methyl 5-(5,5-dioxido-3a,4,6,6a-tetrahydrothieno[3,4-d]isoxazol-3-yl)-2-methoxybenzoate.

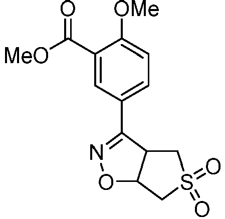

Intermediate **442-1** was prepared in an identical fashion (128 mg, 23% yield) described for intermediate **378-3** which in this case by substituting allyl alcohol with 2,5-dihydrothiophene 1,1-dioxide.

### Intermediate 442-2: Preparation of 5-(5,5-dioxido-3a,4,6,6a-tetrahydrothieno[3,4-d]isoxazol-3-yl)-2-methoxybenzoic acid.

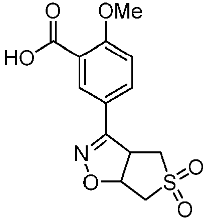

Intermediate **442-2** (59.0 mg, 39.6%) was prepared in a similar manner as intermediate **429-2** with the hydrolysis of intermediate **442-1.** MS (ESI) *m*/*z* = 312.2 (M+H).

Individual chiral diastereomer ester intermediates **442-4A** and **442-6A** were obtained by chiral SFC of diasteremeric mixture intermediate **441-1** (600 mg, 1.84 mmol). Chiral SFC Preparative chromatographic conditions: Instrument: PIC Solution SFC Prep-200; Column: Chiralcel OD-H, 21 x 250 mm, 5 micron; Mobile phase: 25% MeOH / 75% CO₂; Flow conditions: 45 mL/min, 150 Bar, 40°C; Detector wavelength: 271 nm; Injections details: 1.0 mL of ~50mg/mL in MeOH:ACN. Analytical chromatographic conditions: Instrument: Shimadzu Nexera SFC; Column: Chiralcel OD-H, 4.6 x 100 mm, 3 micron; Mobile phase: 15% MeOH / 85% CO2; Flow conditions: 2.0 mL/min, 150 Bar, 40°C; Detector wavelength: 220 nm; Injection details: 5 µL of ~1mg/mL in MeOH.

Intermediate **442-4A** (Peak-1, >99% de, analytical RT = 3.74 min.) was obtained as a white solid (108.1 mg, 18%). ¹H NMR: (400 MHz, chloroform-d) δ 7.96 (d, J=2.4 Hz, 1H), 7.85 (dd, J=8.8, 2.2 Hz, 1H), 7.07 (d, J=9.0 Hz, 1H), 5.43 (ddd, J=10.1, 7.2, 4.1 Hz, 1H), 4.52 - 4.44 (m, 1H), 3.97 (s, 3H), 3.92 (s, 3H), 3.66 - 3.47 (m, 3H), 3.14 (dd, J=13.6, 8.1 Hz, 1H).

Intermediate **442-4** (82 mg, 79%) was prepared in a similar manner as intermediate **429-2** with the hydrolysis of intermediate **442-4A.** MS (ESI) *m*/*z* = 312.3 (M+H).

Intermediate **442-6A** (Peak-2, >99% de, analytical RT = 5.44 min.) was obtained as a white solid (108.8 mg, 18%). ¹H NMR: (400 MHz, chloroform-d) δ 7.96 (d, J=2.4 Hz, 1H), 7.85 (dd, J=8.8, 2.2 Hz, 1H), 7.06 (d, J=8.8 Hz, 1H), 5.42 (ddd, J=10.1, 7.2, 4.1 Hz, 1H), 4.52 - 4.44 (m, 1H), 3.97 (s, 3H), 3.91 (s, 3H), 3.66 - 3.46 (m, 3H), 3.14 (dd, J=13.6, 8.4 Hz, 1H).

Intermediate **442-6** (89.6 mg, 87%) was prepared in a similar manner as intermediate **429-2** with the hydrolysis of intermediate **442-6A.** MS (ESI) *m*/*z* = 312.3 (M+H).

Example **442** was prepared in a similar manner as example **429** replacing intermediate **429-2** with intermediate **442-2.** (1R,2S,3R,4R,Z)-7-(cyclopropylmethylene)-3-(5-(5,5-dioxido-3a,4,6,6a-tetrahydrothieno[3,4-d]isoxazol-3-yl)-2-methoxybenzamido)-N-(4-fluoro-3-(trifluoromethyl)phenyl)bicyclo[2.2.1]heptane-2-carboxamide diastereomeric mixture, **442** (4.8 mg, 0.0072 mmol, 53.0 % yield). ¹H NMR (500 MHz, DMSO-d6) δ 10.54 (s, 1H), 9.91 (br t, *J*=8.4 Hz, 1H), 8.26 - 8.20 (m, 2H), 7.81 - 7.74 (m, 2H), 7.48 (br t, *J*=9.9 Hz, 1H), 7.28 (d, *J*=8.9 Hz, 1H), 5.43 - 5.38 (m, 1H), 4.78 - 4.72 (m, 1H), 4.69 (br d, *J*=9.8 Hz, 1H), 4.43 (br s, 1H), 4.05 (s, 3H), 3.65 (br dd, *J*=14.3, 6.7 Hz, 1H), 3.44 - 3.33 (m, 1H), 3.18 - 3.08 (m, 3H), 2.72 (br s, 1H), 1.85 - 1.74 (m, 2H), 1.52 - 1.46 (m, 1H), 1.45 - 1.34 (m, 2H), 0.79 - 0.68 (m, 2H), 0.34 (br s, 2H). HPLC purity: 99.2 %. Analytical LC-MS: 2.33 min; MS (ESI) *m*/*z* 662.2 (M+H); Method B.

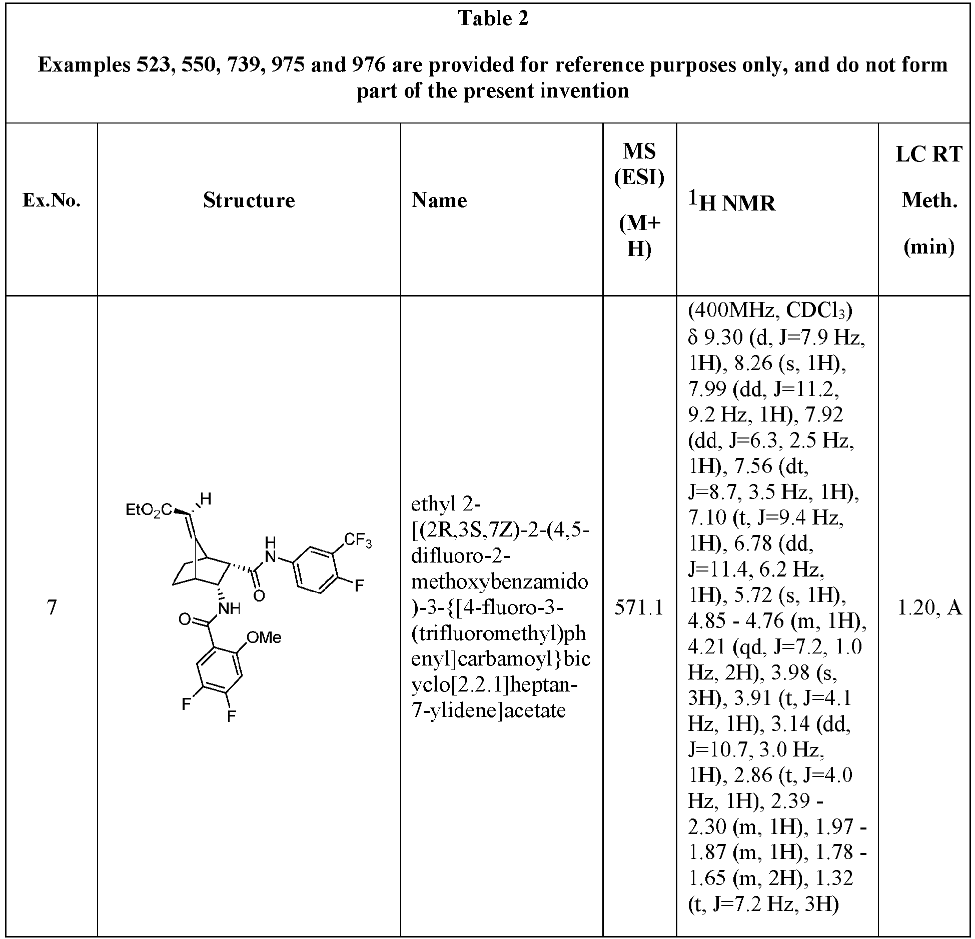

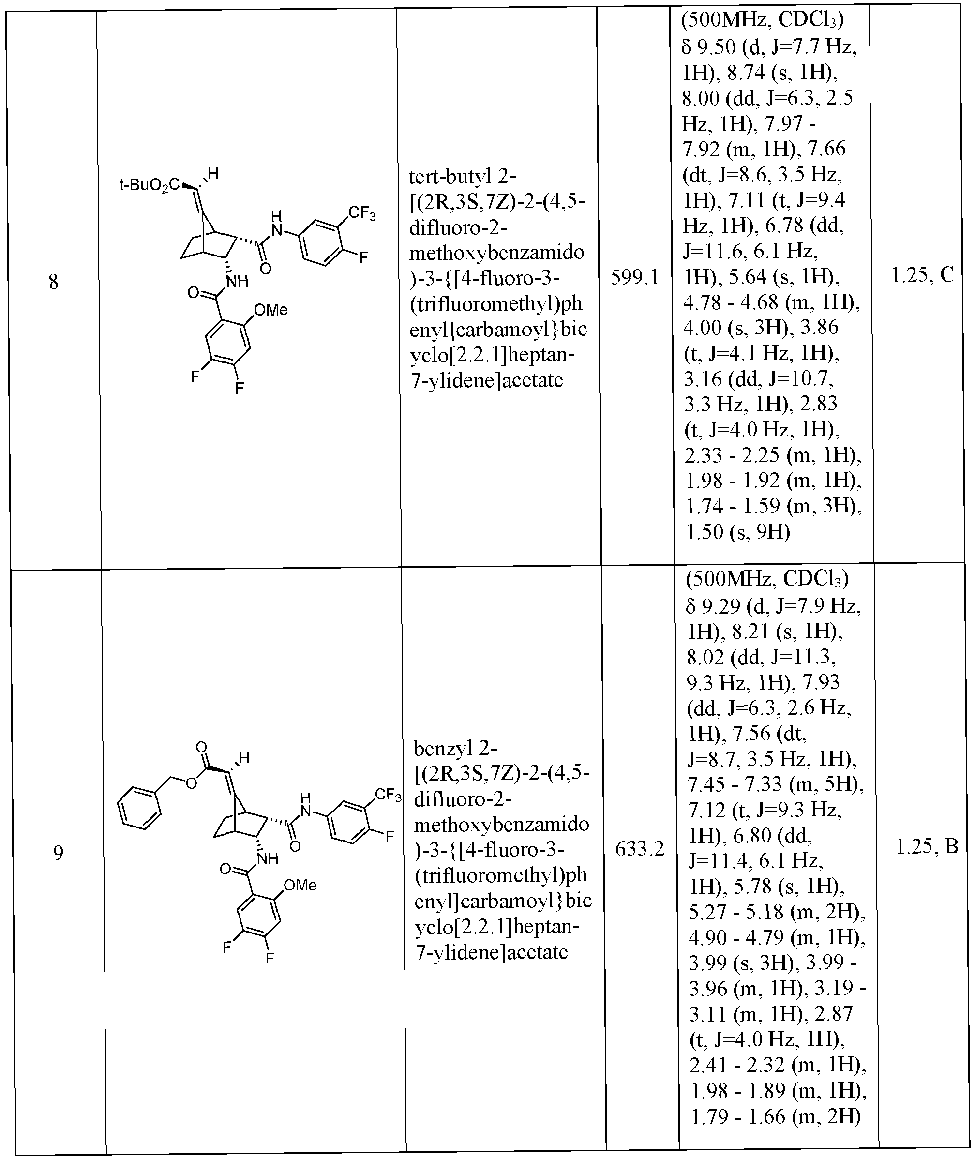

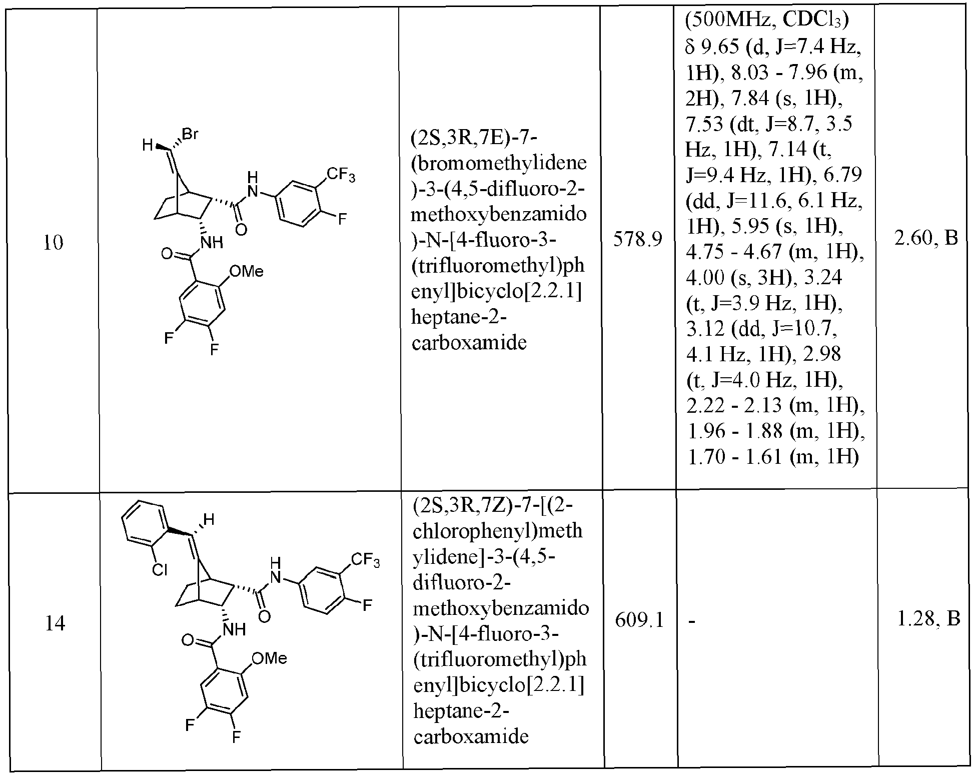

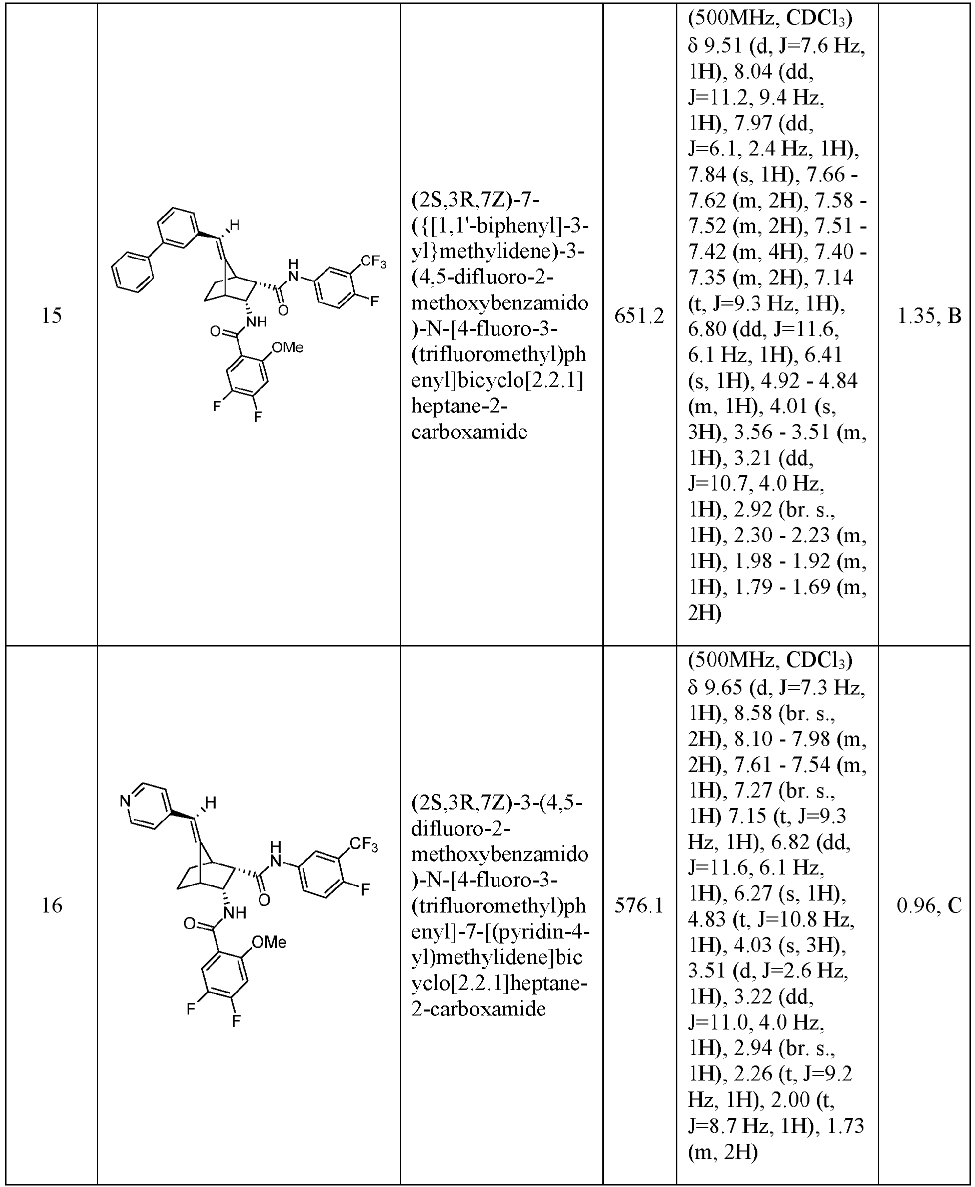

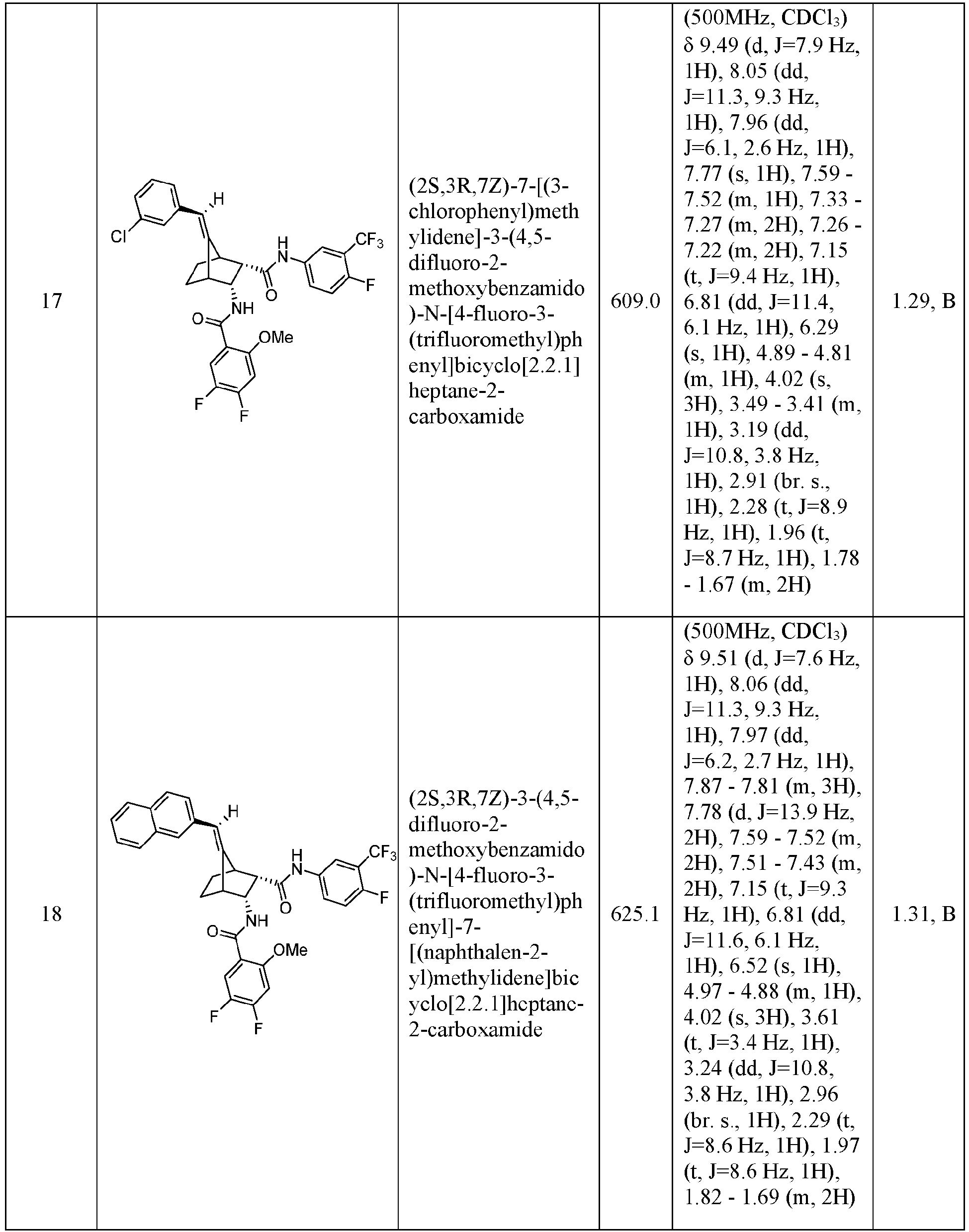

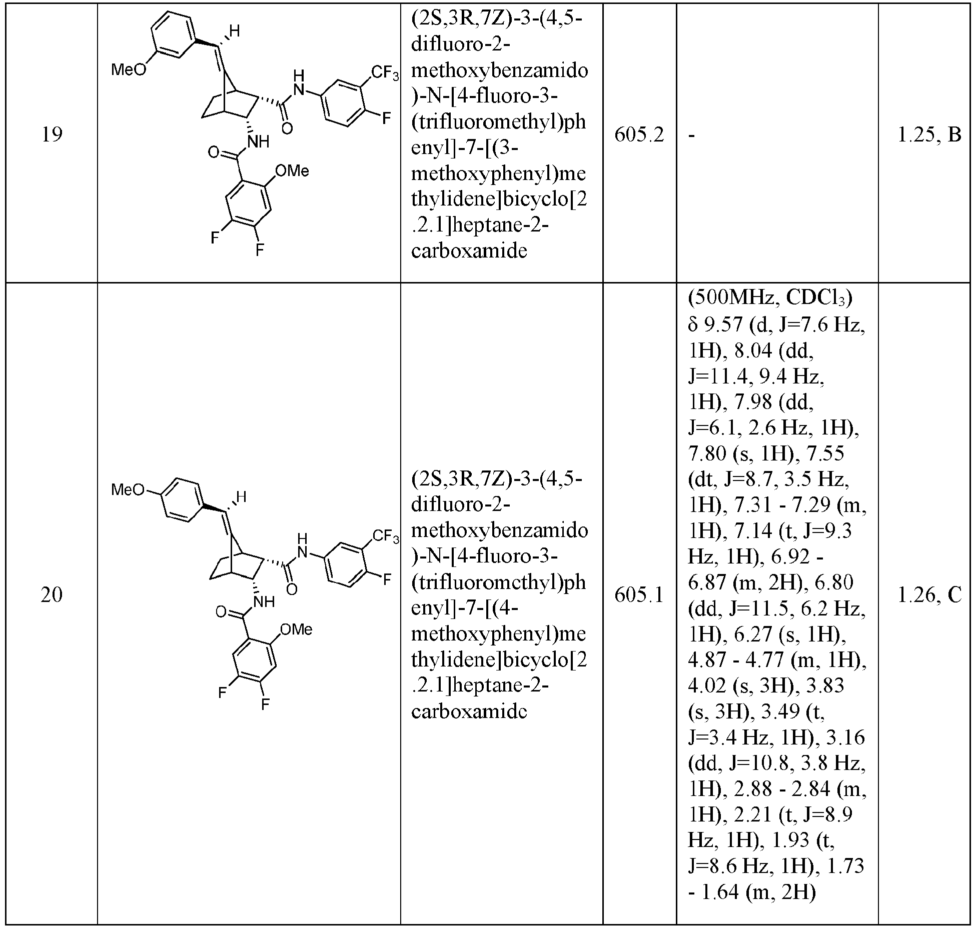

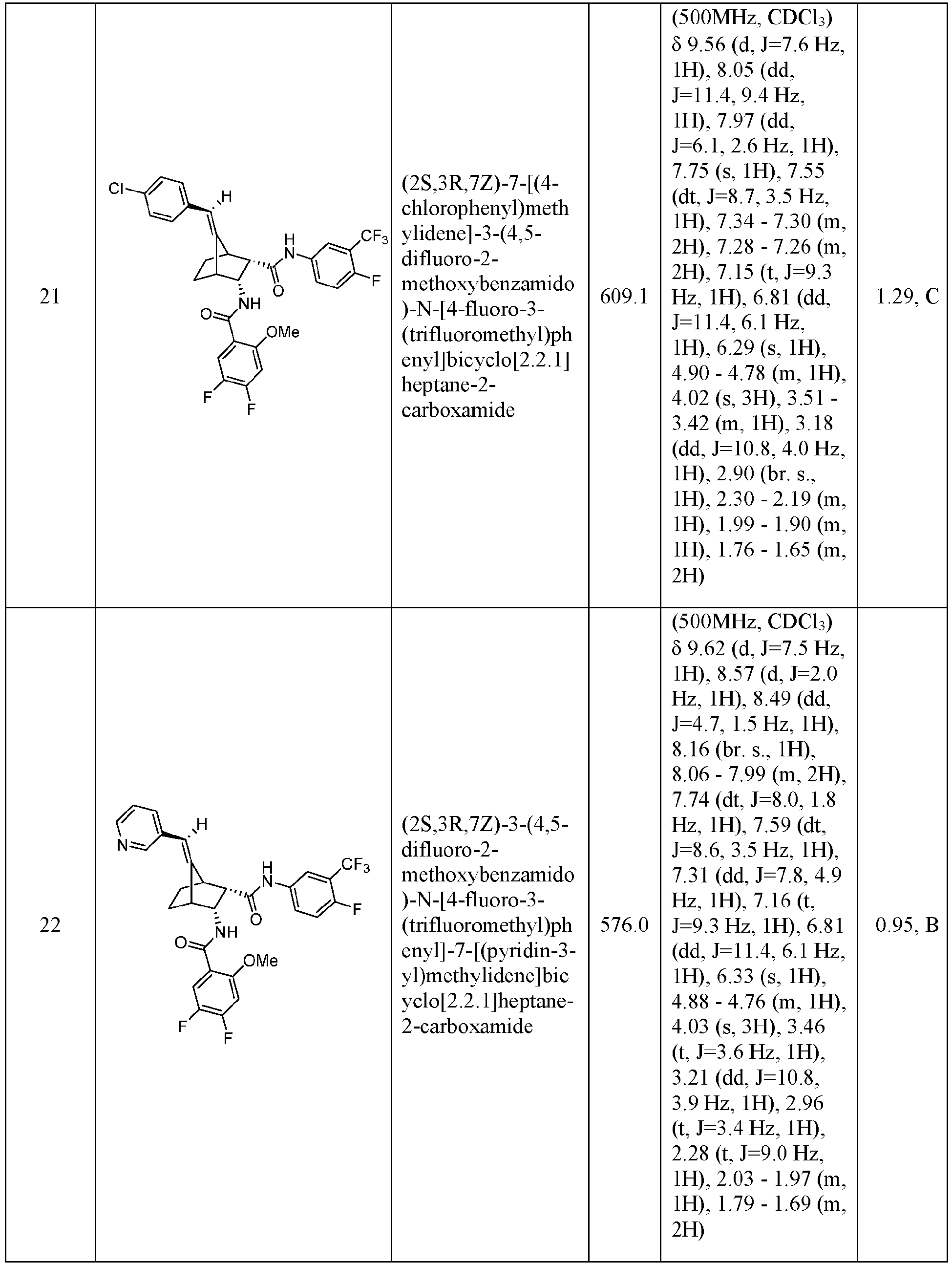

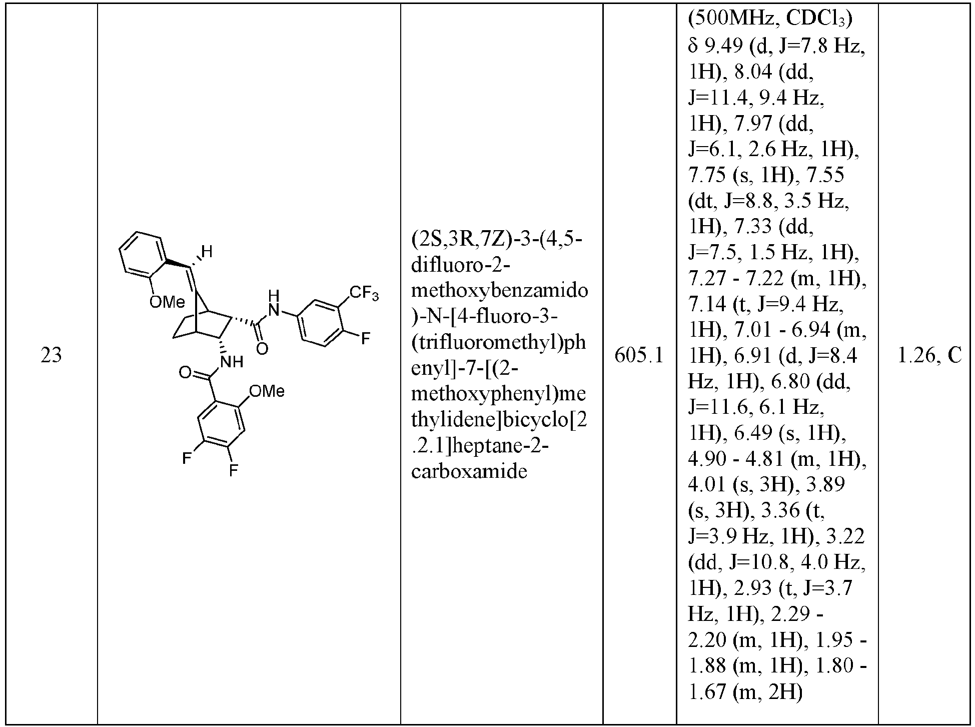

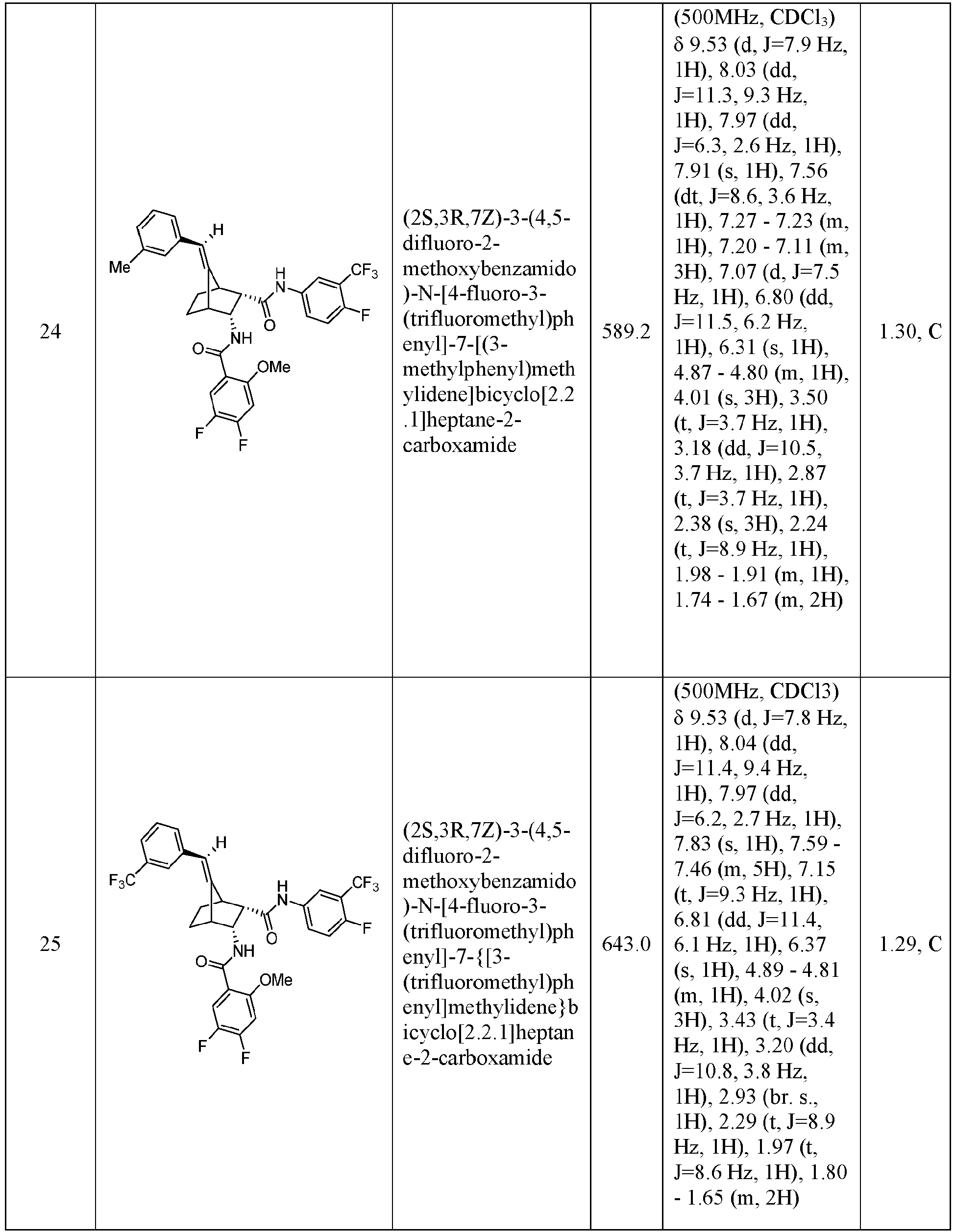

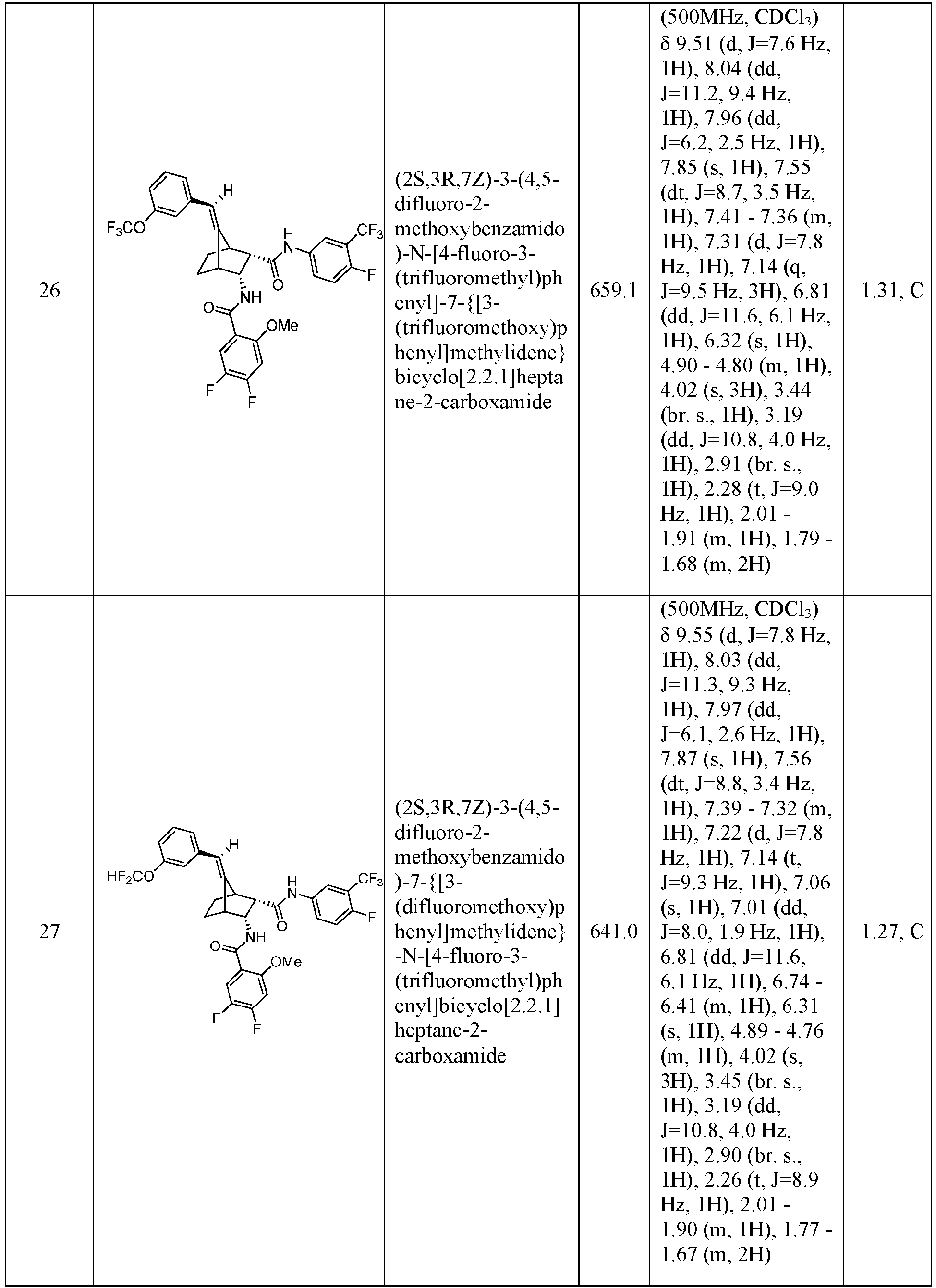

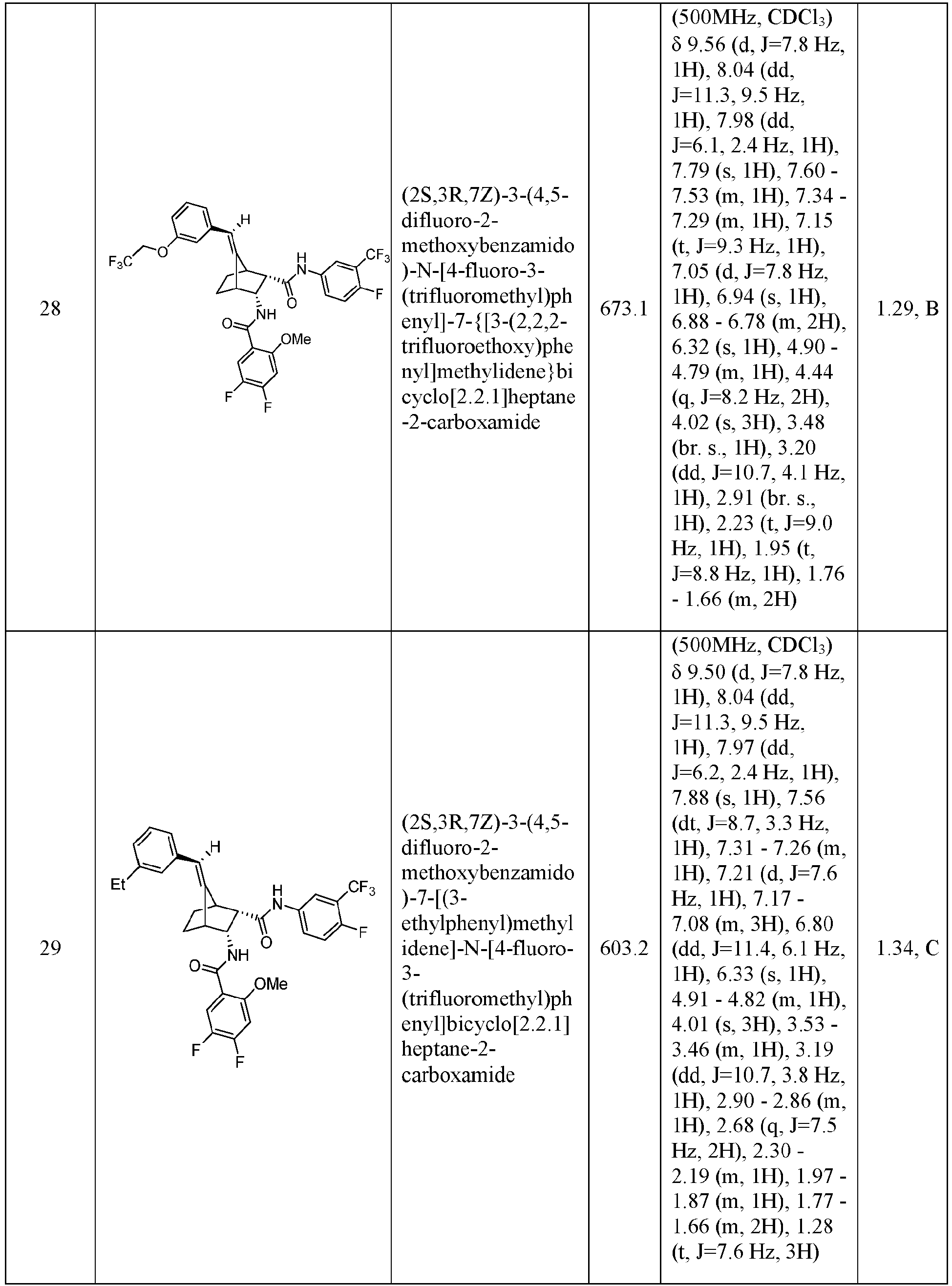

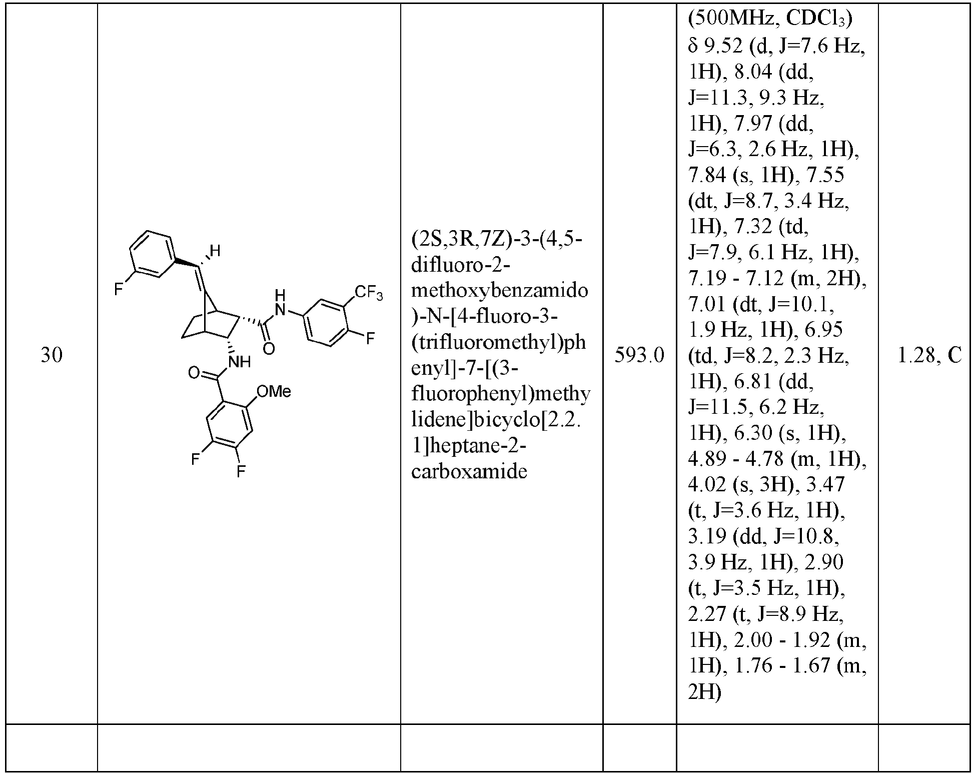

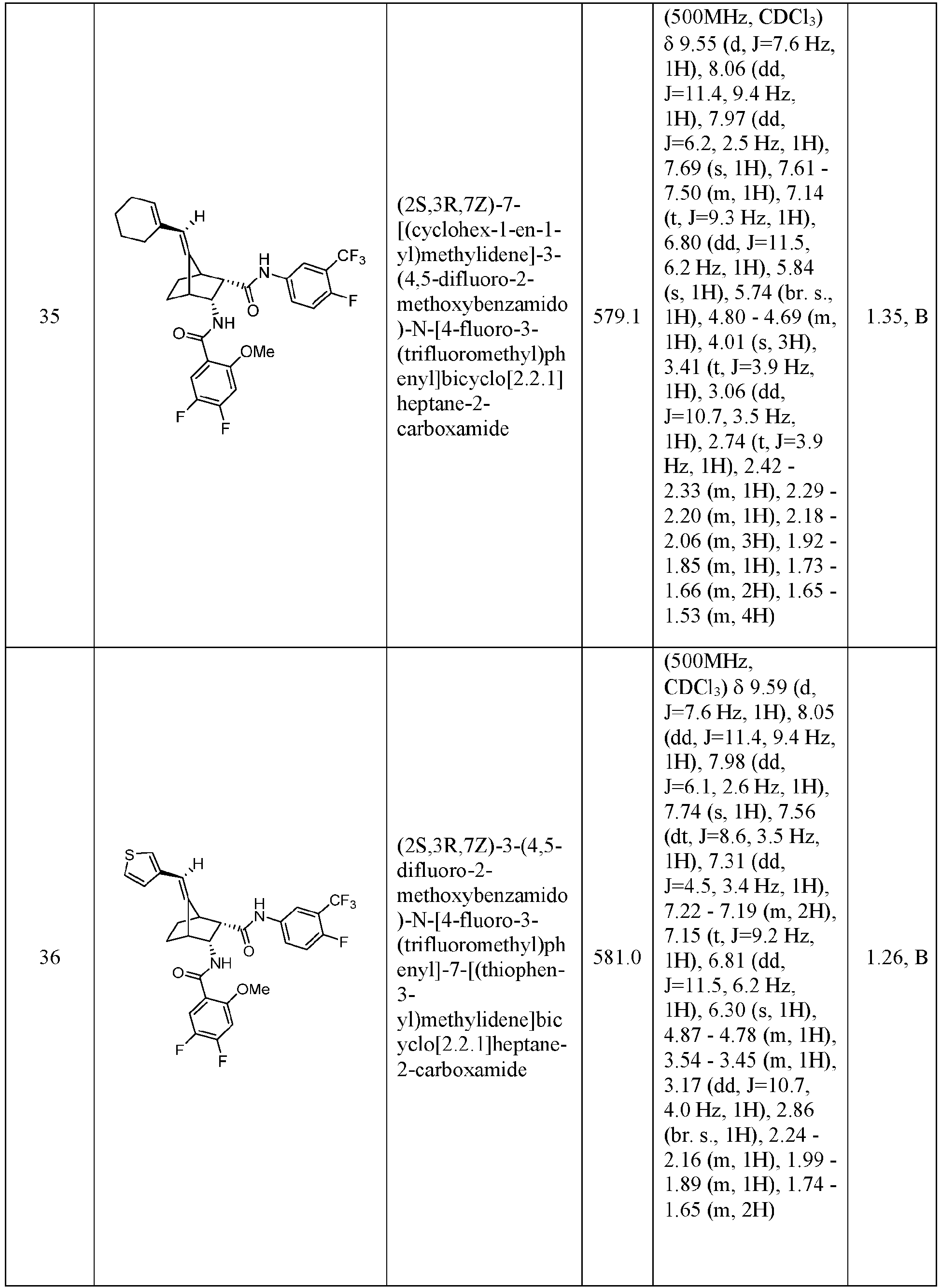

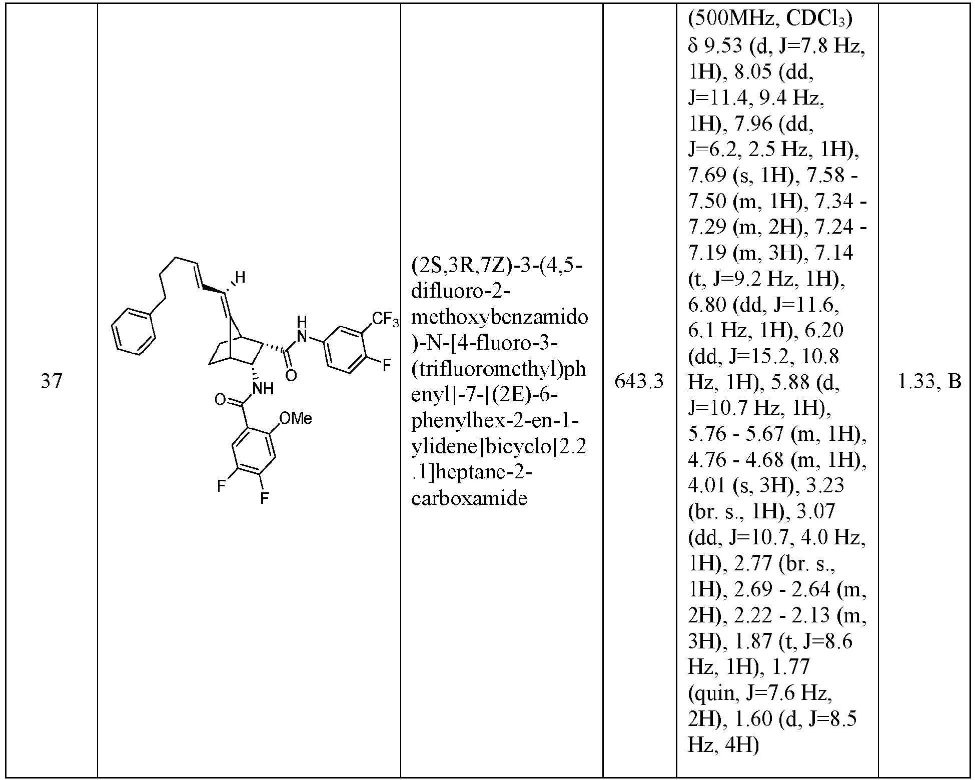

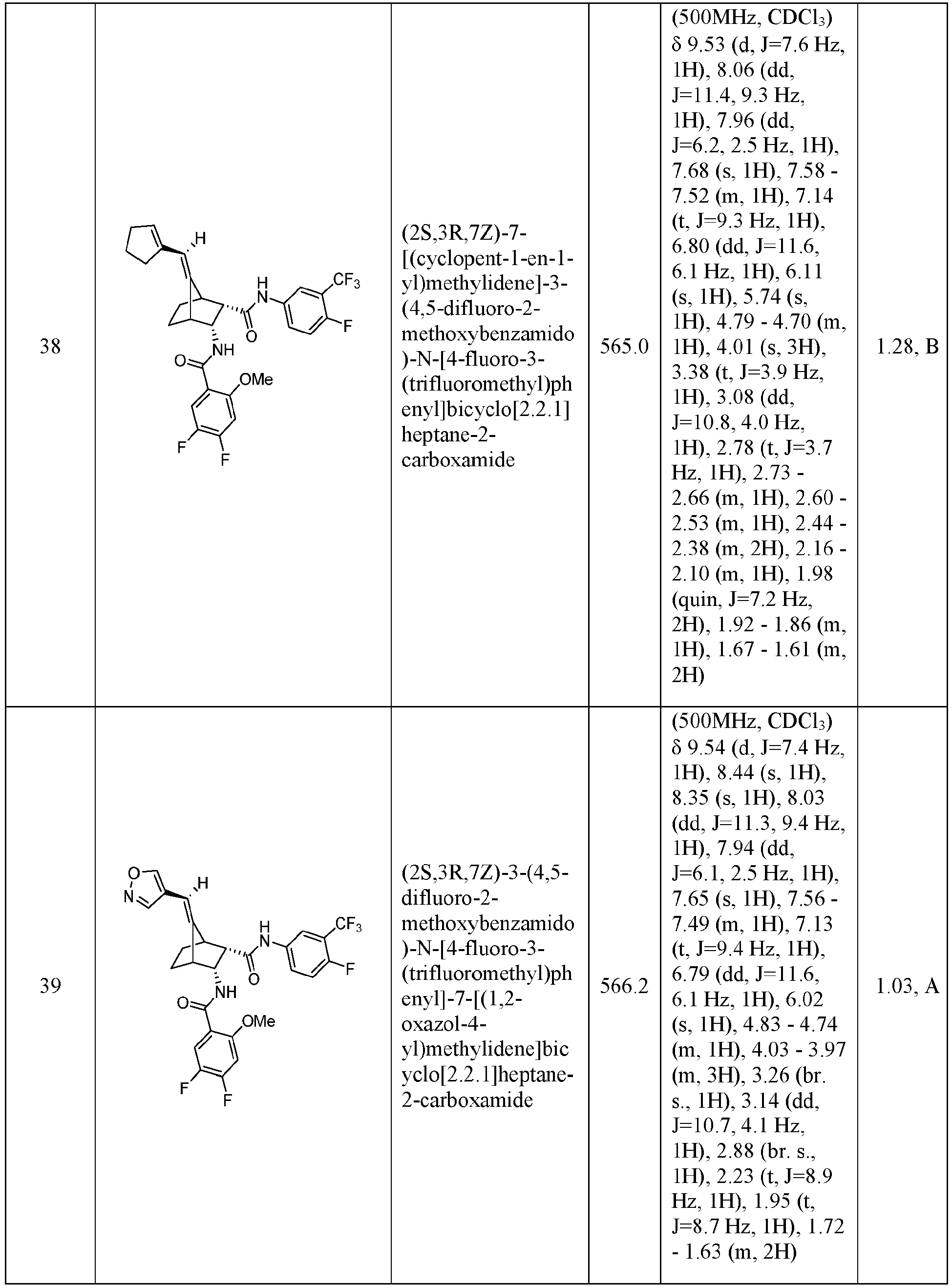

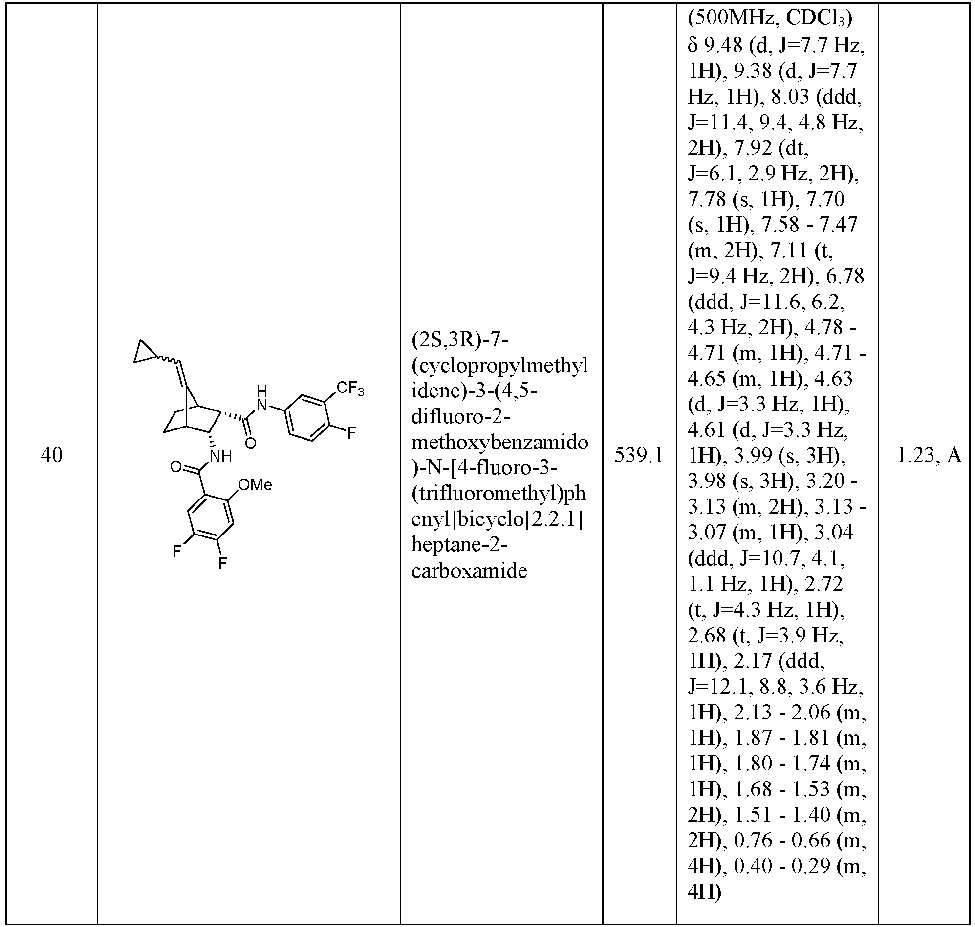

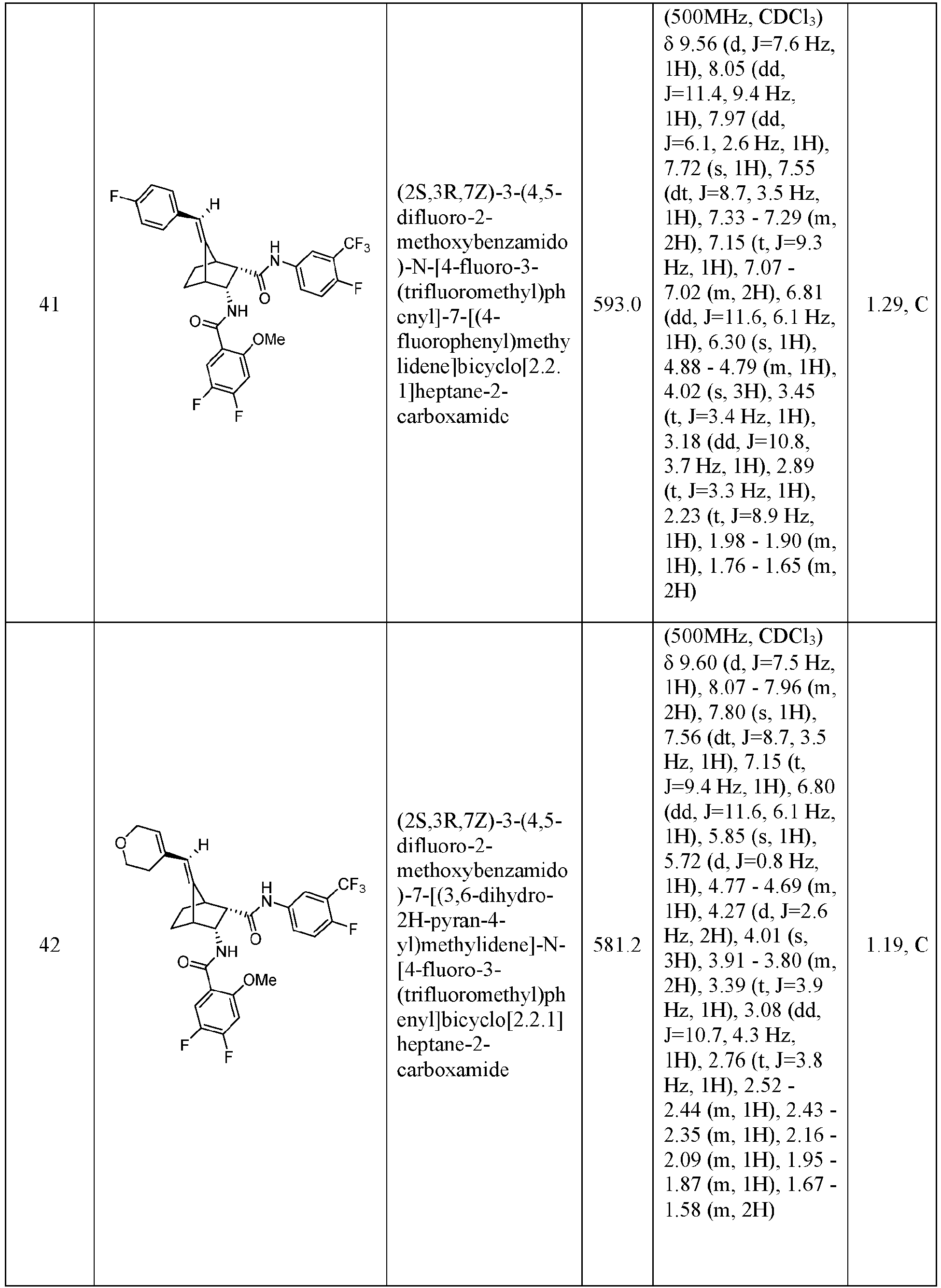

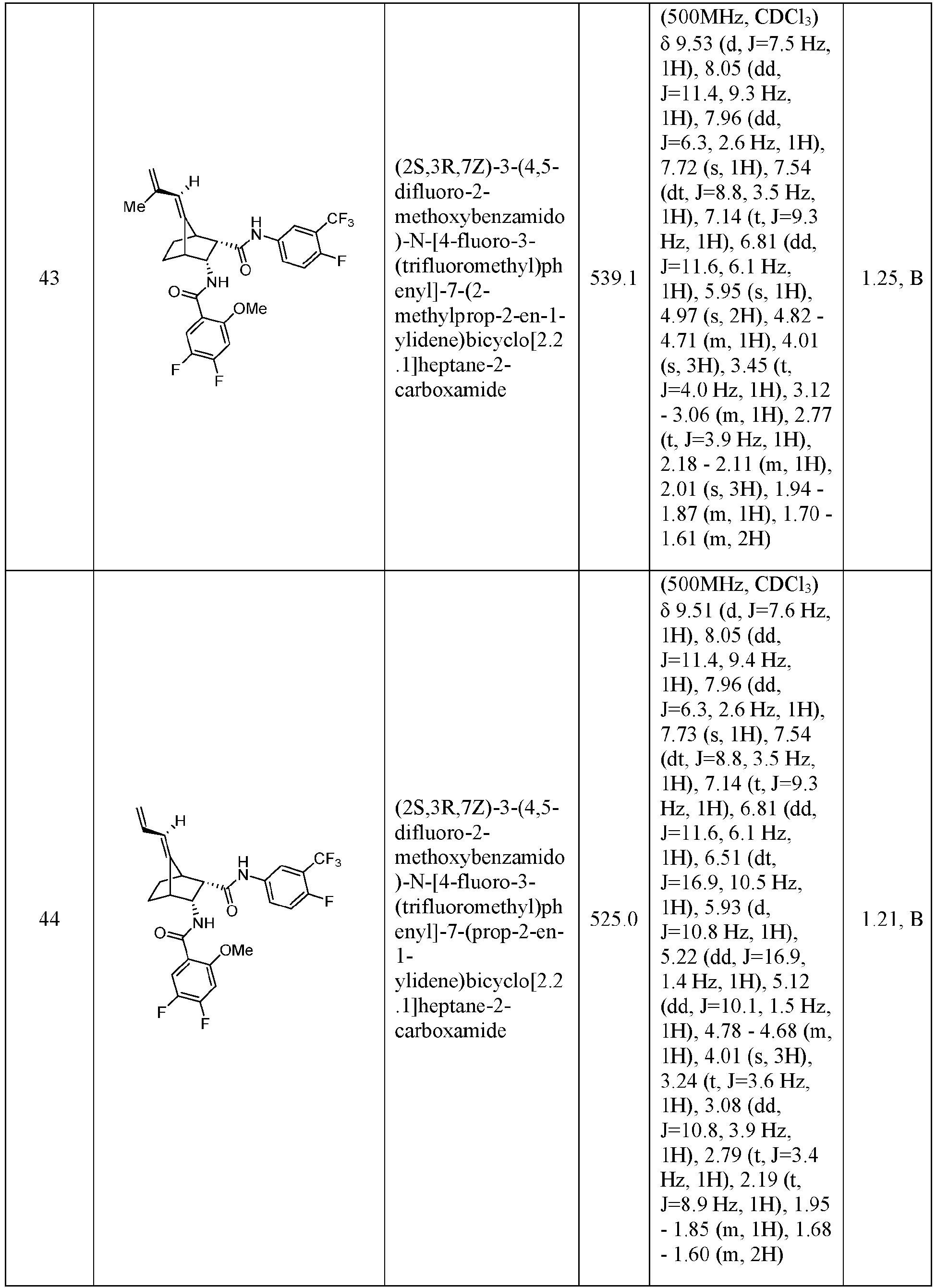

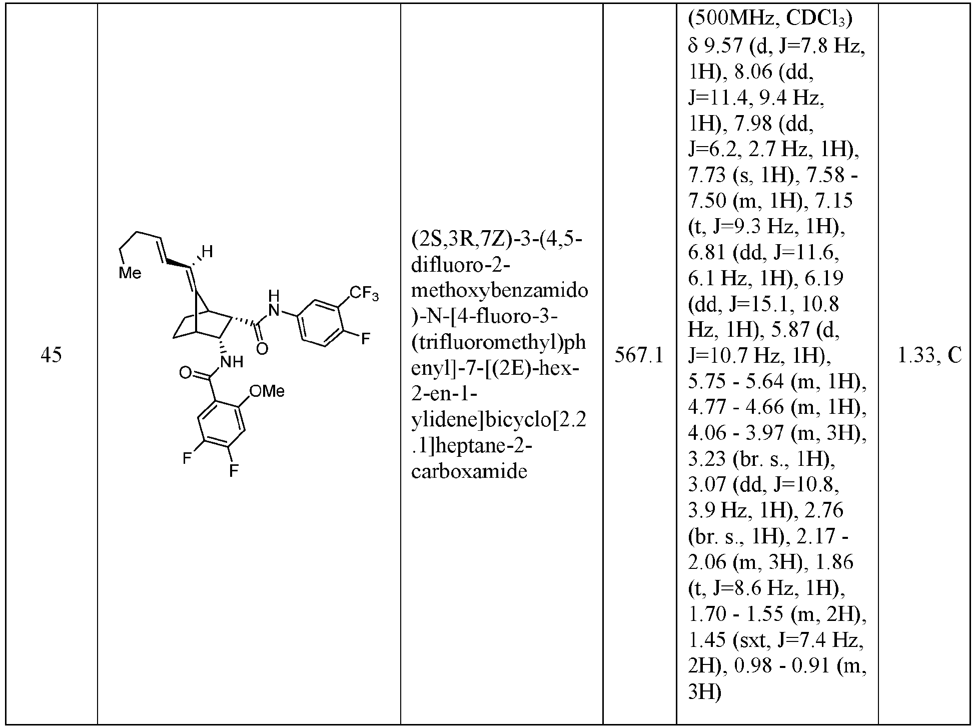

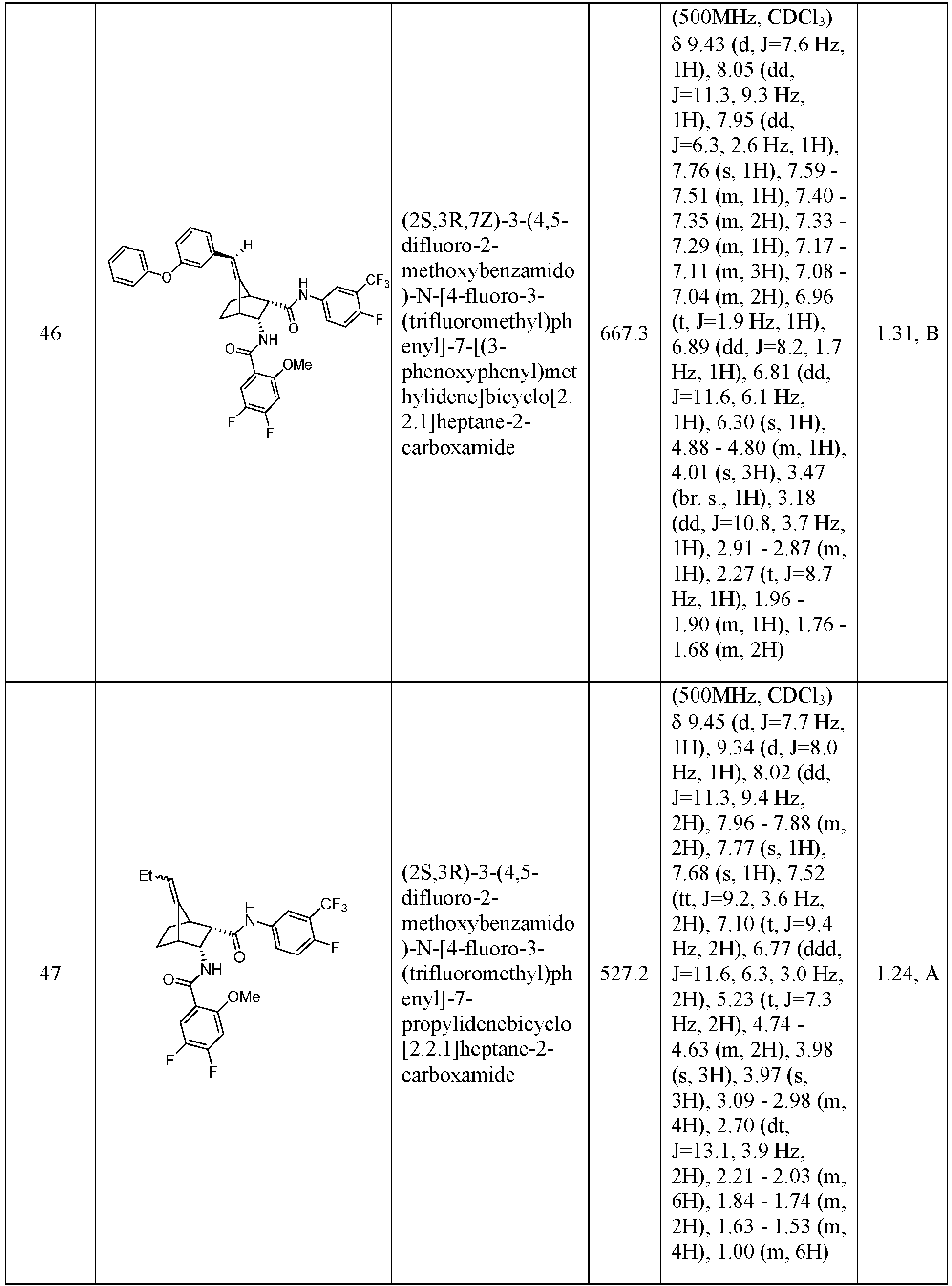

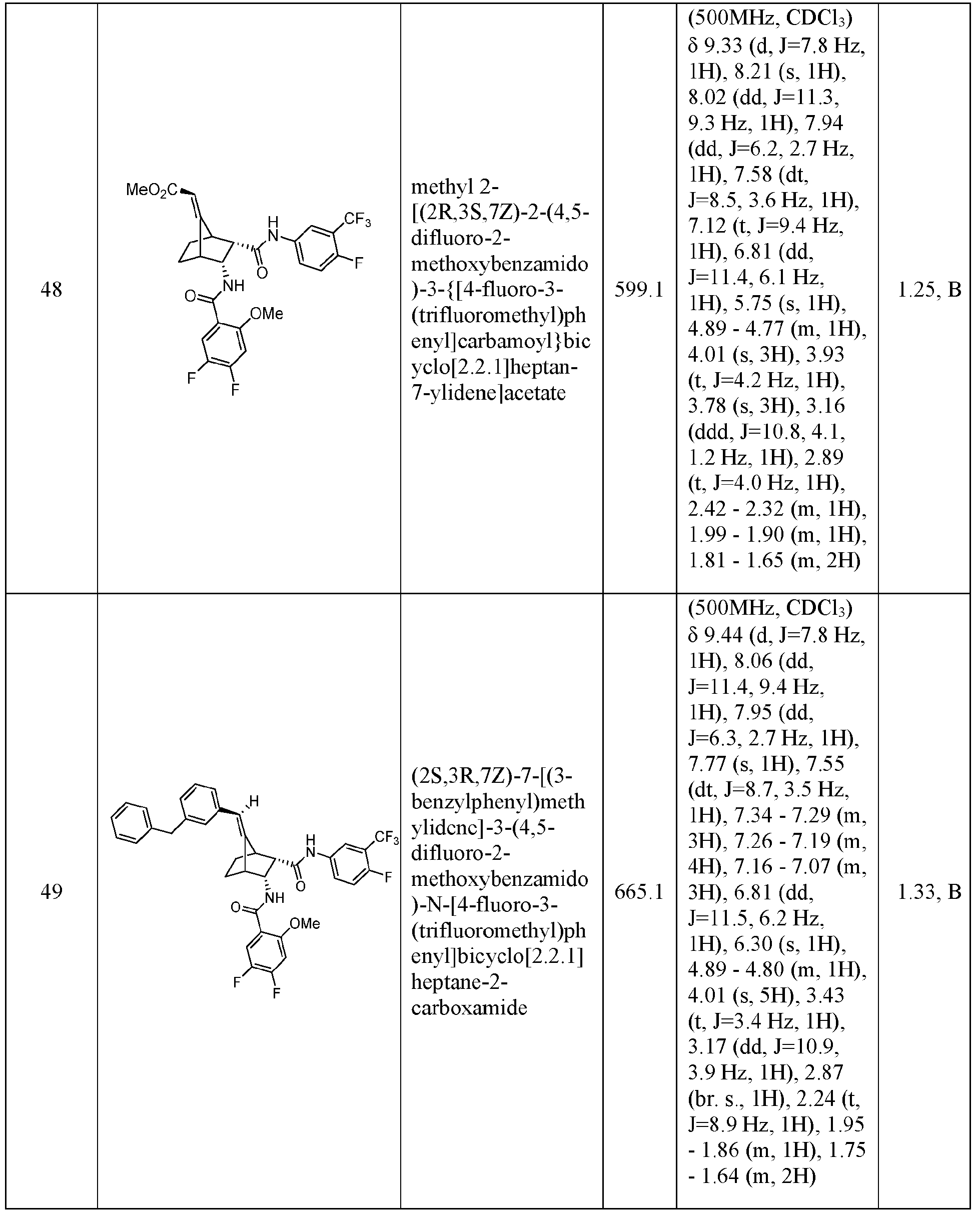

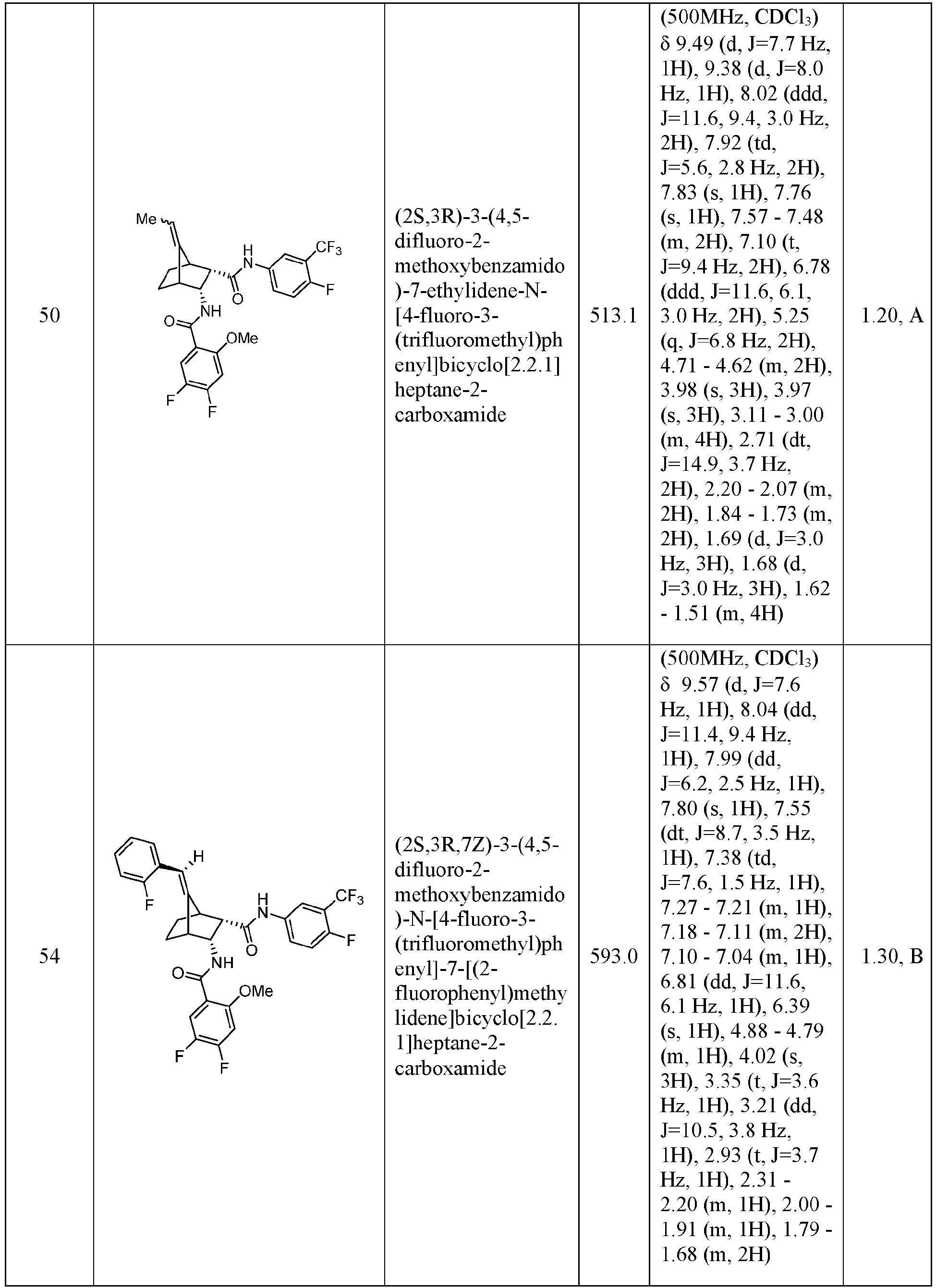

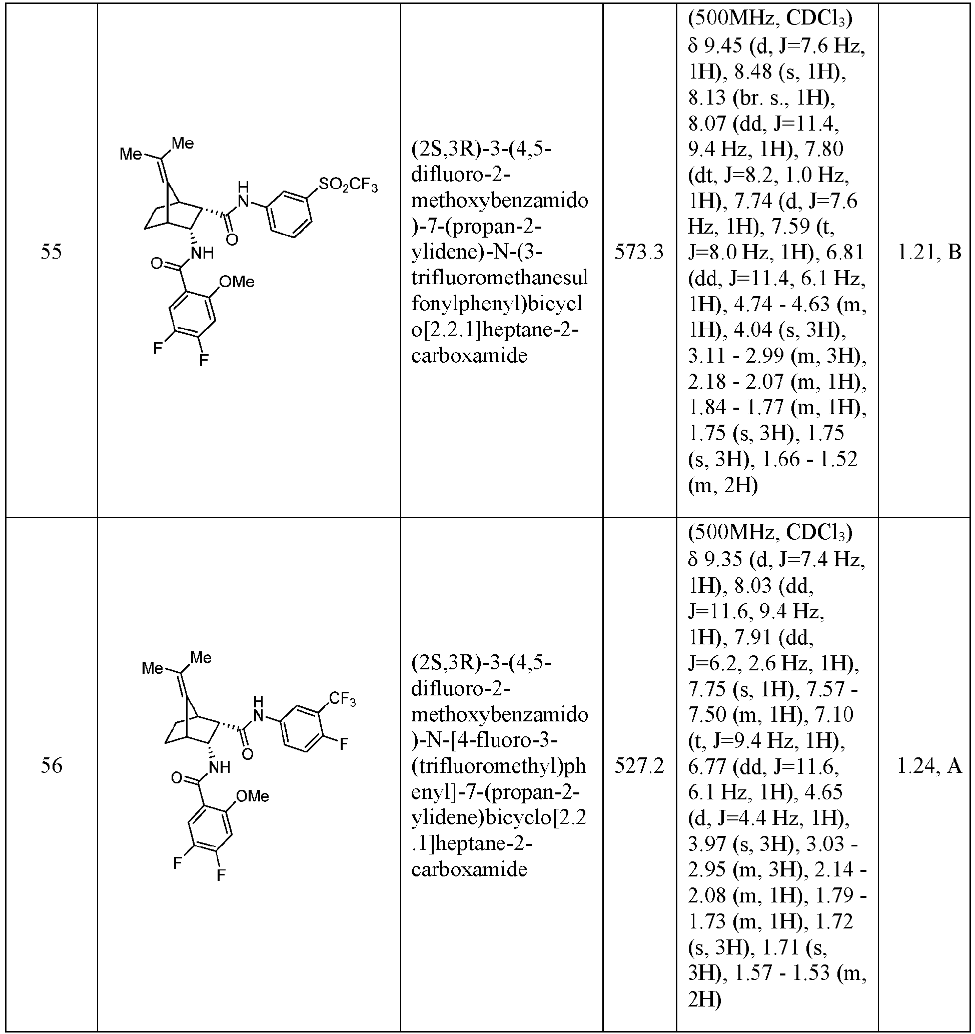

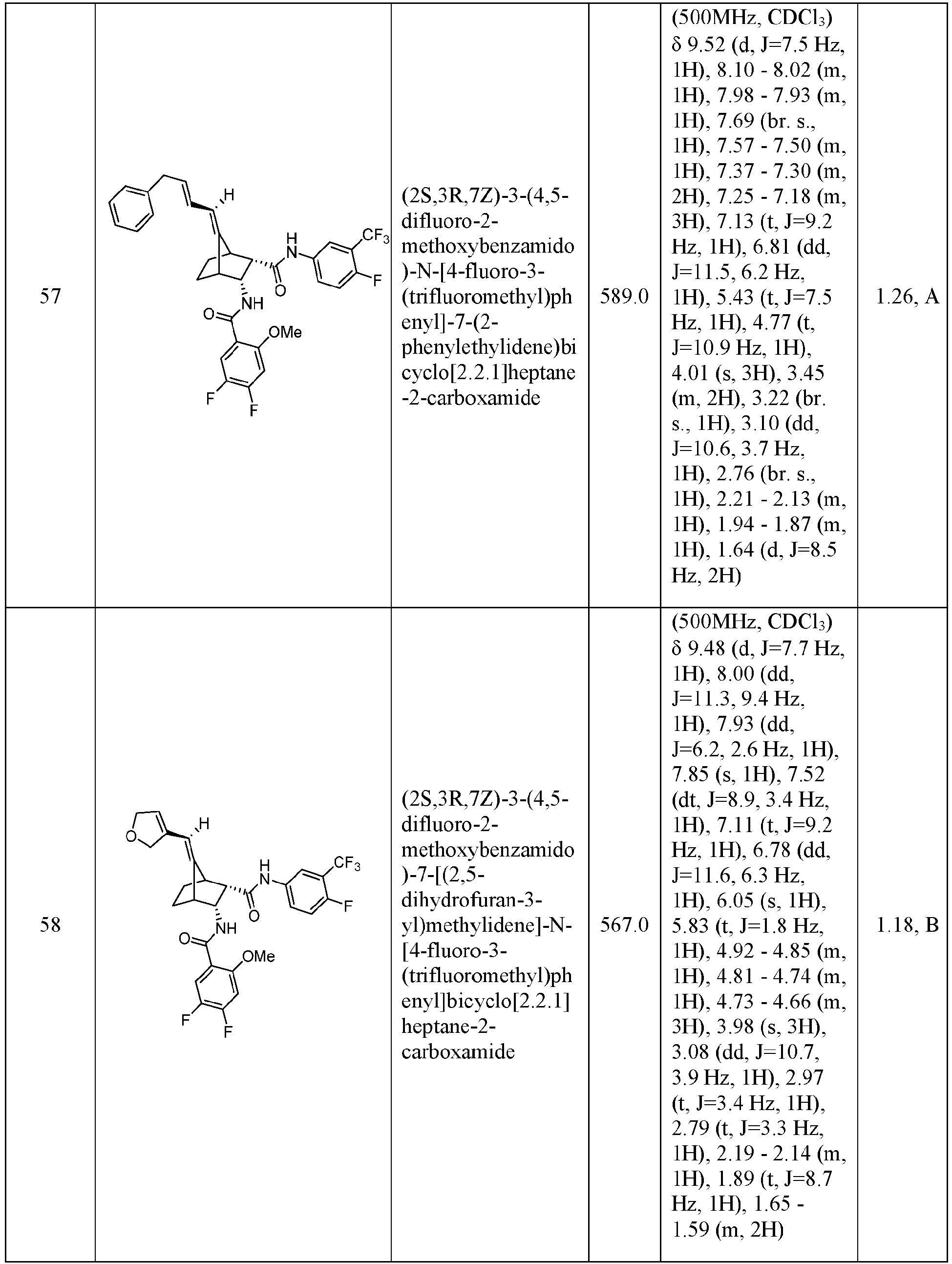

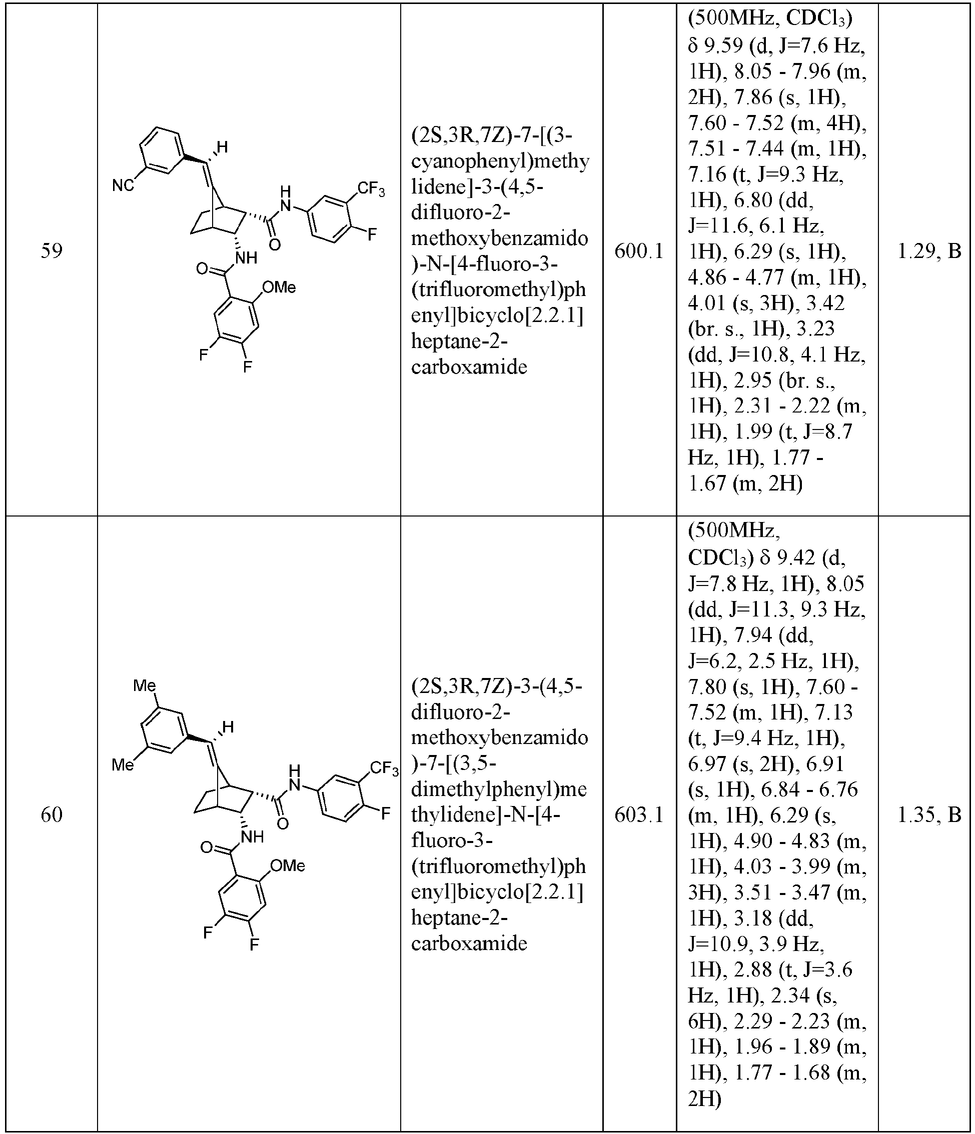

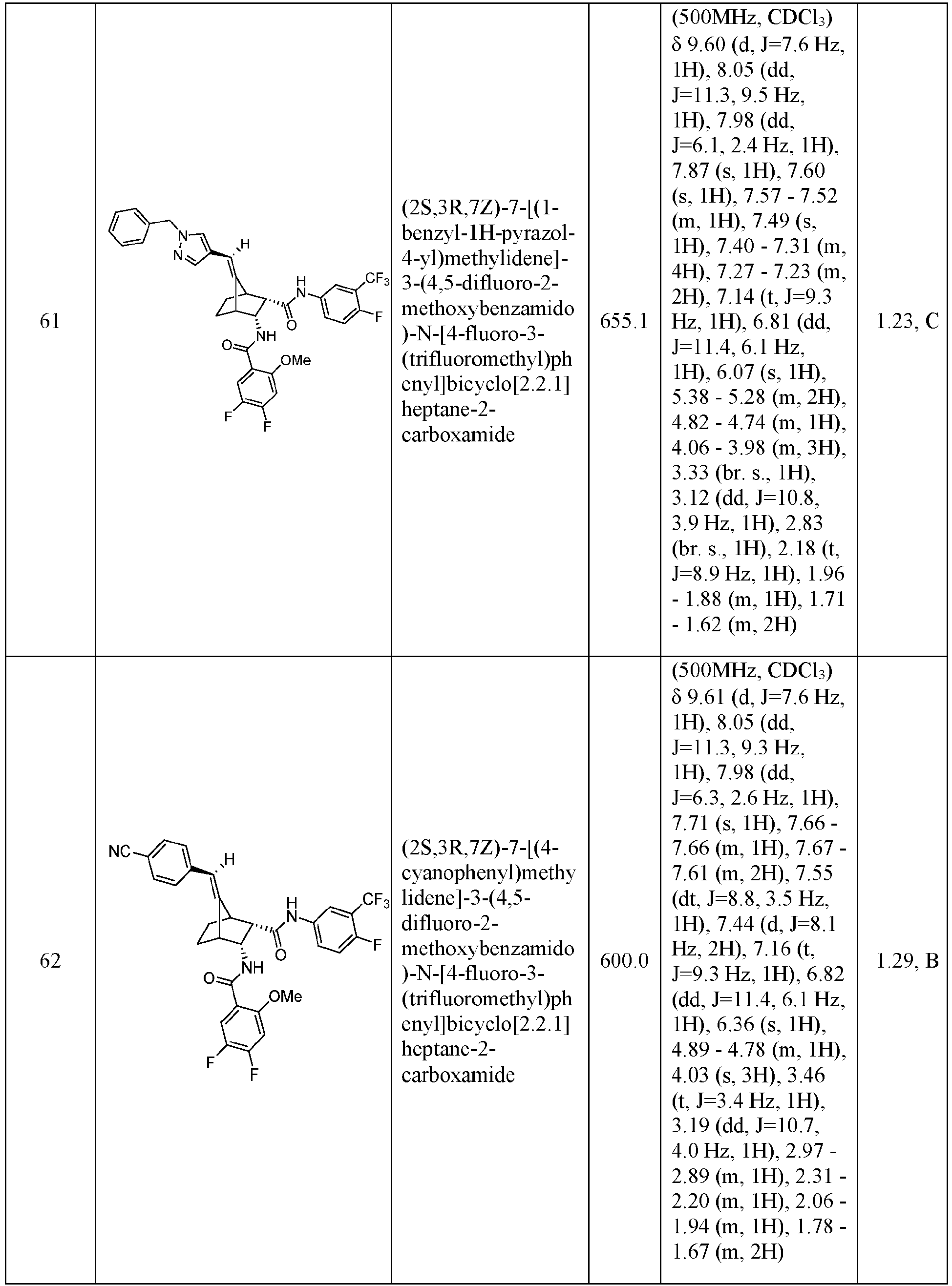

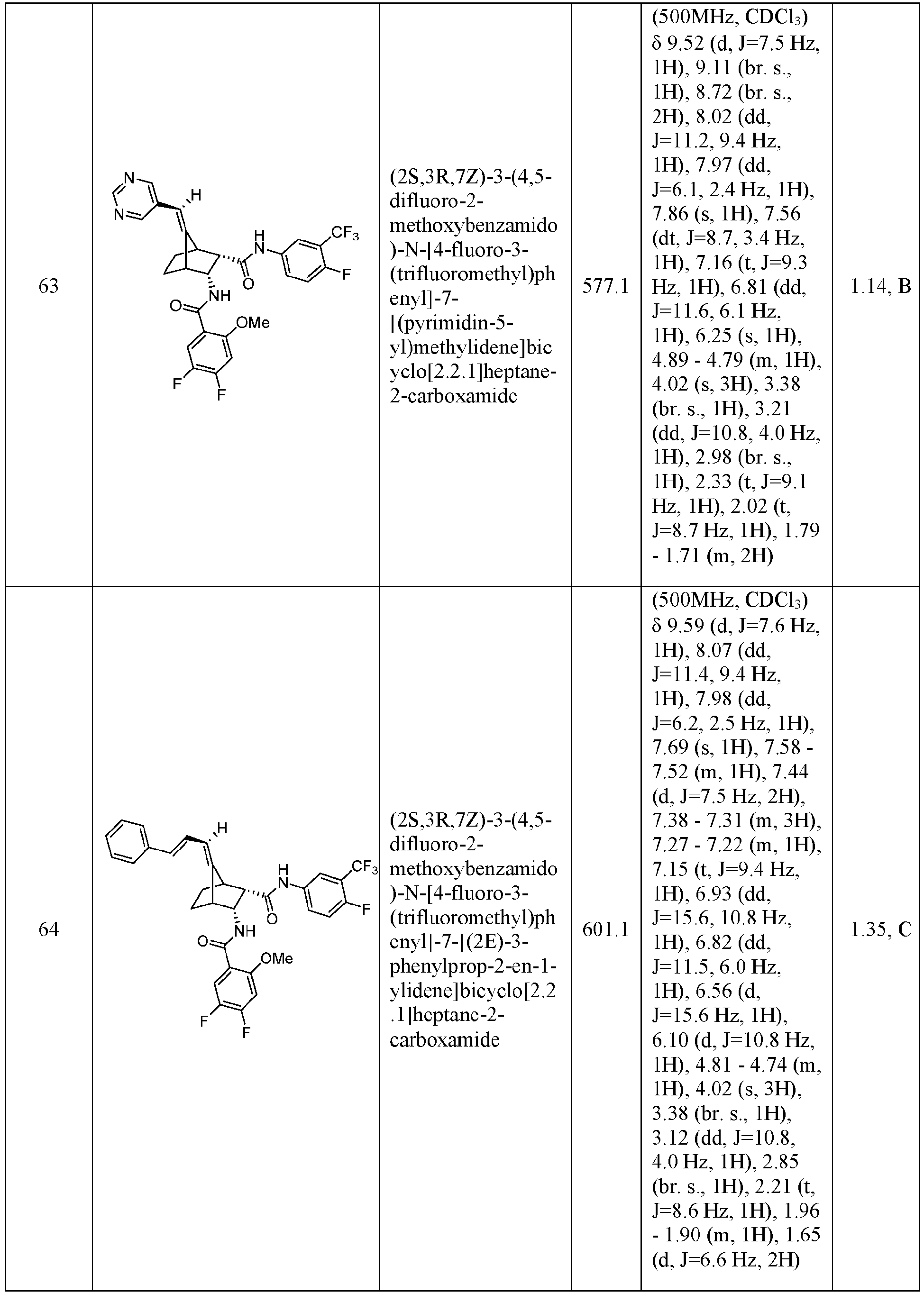

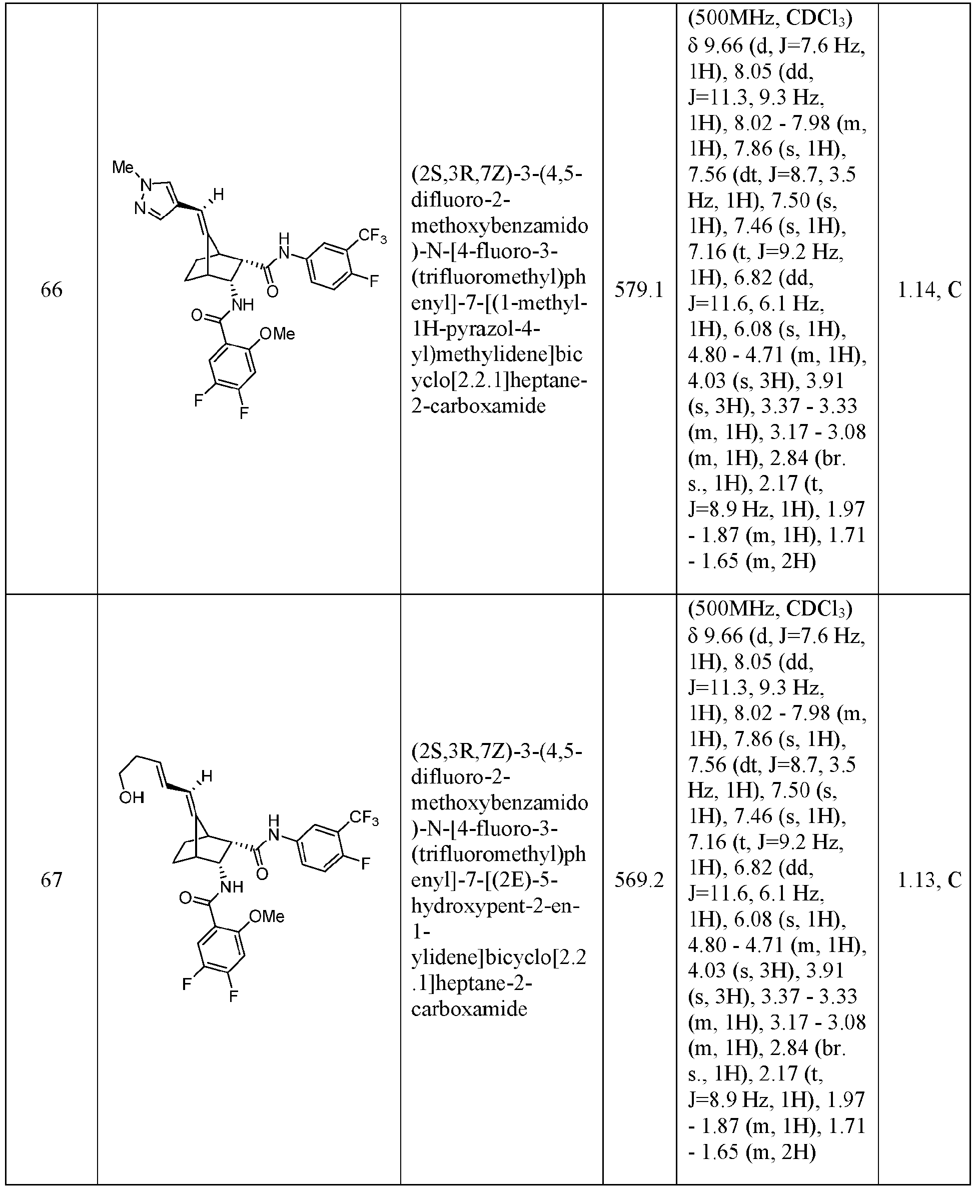

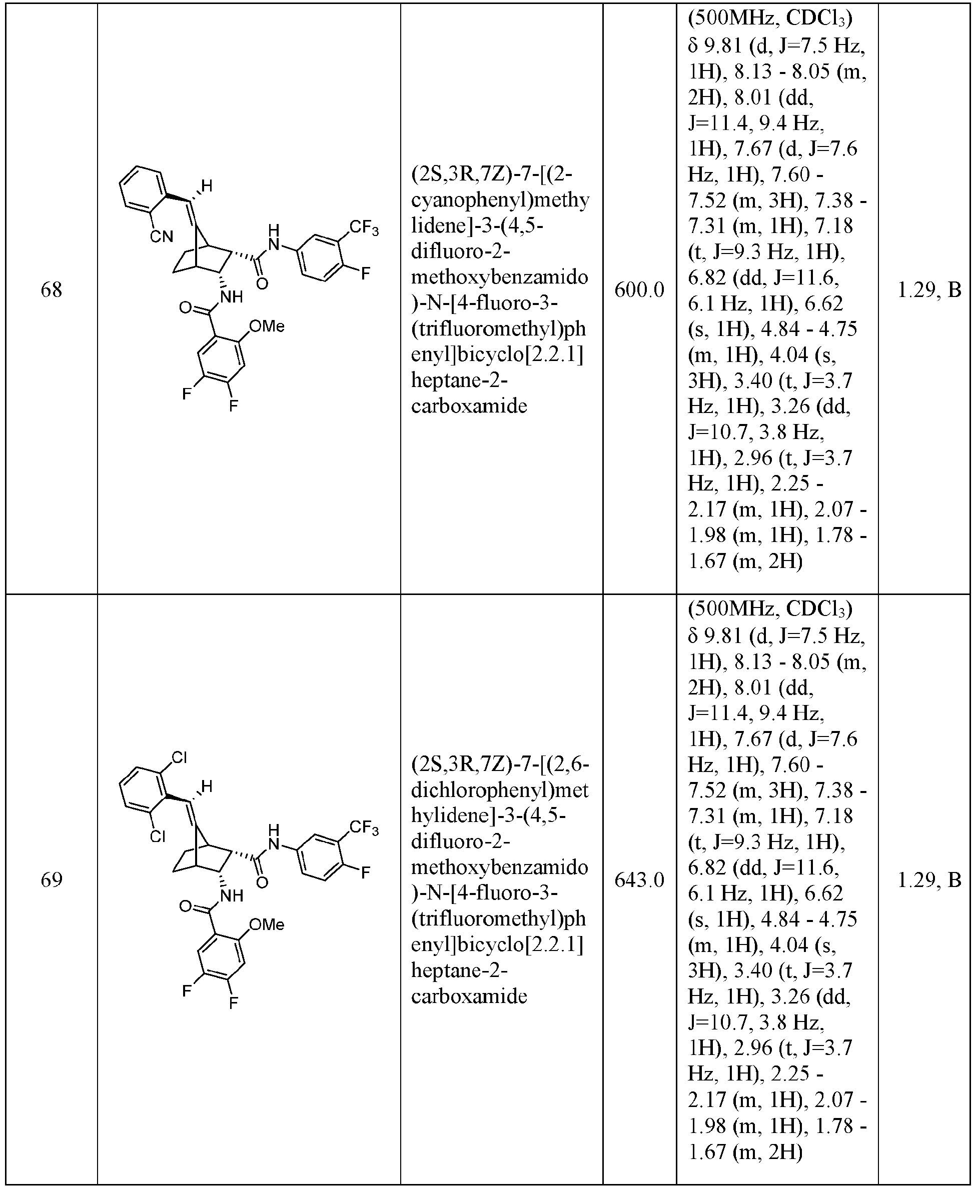

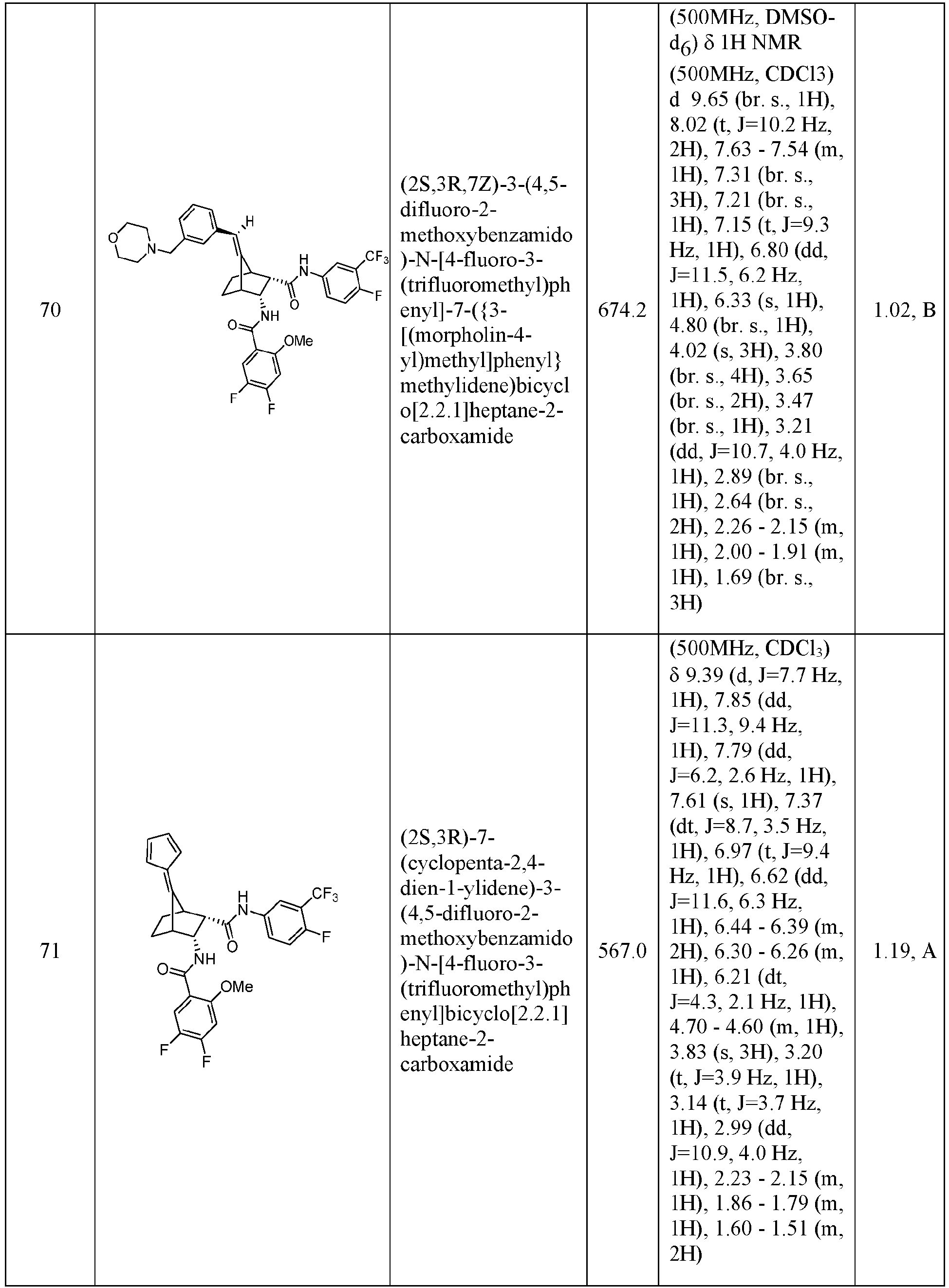

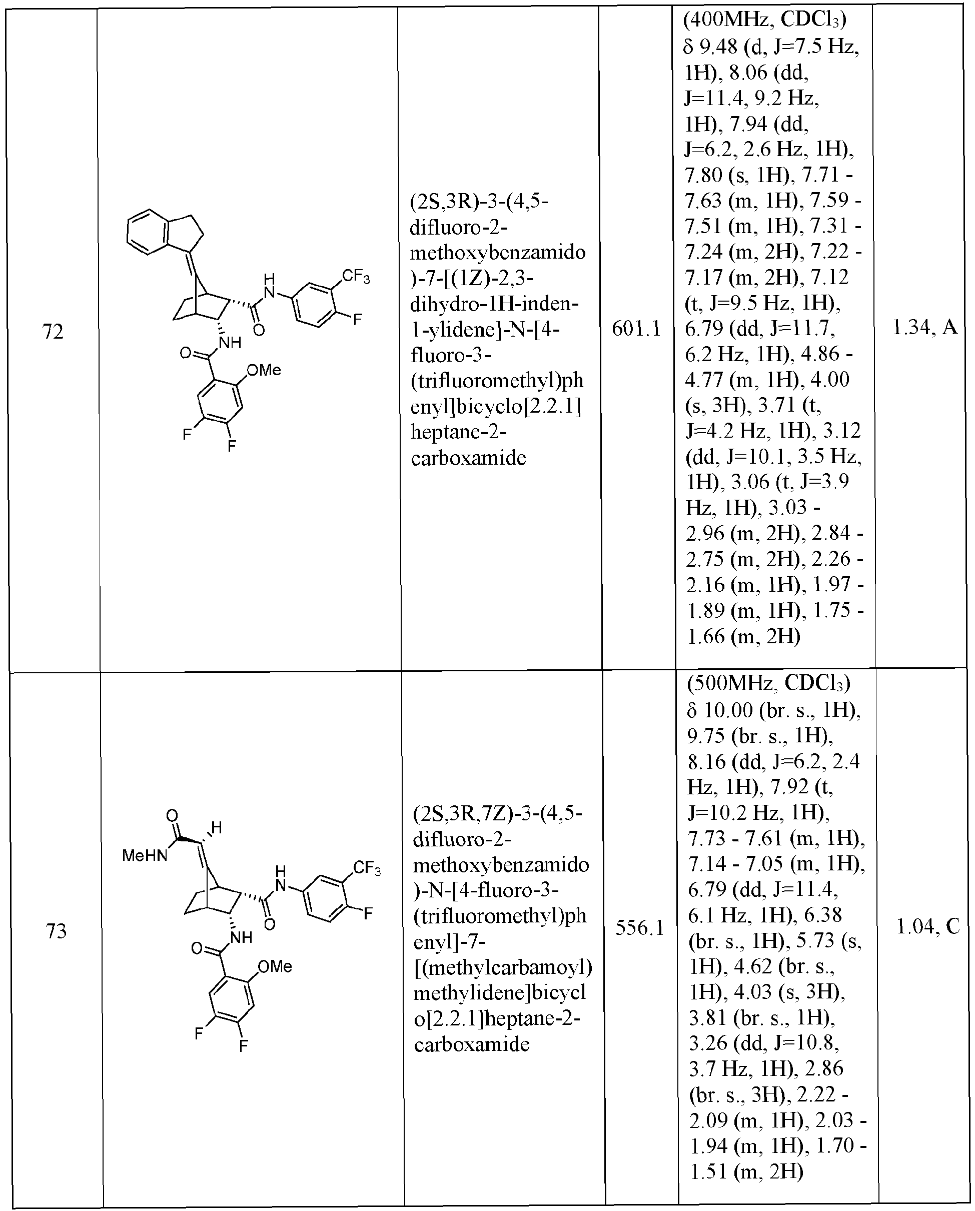

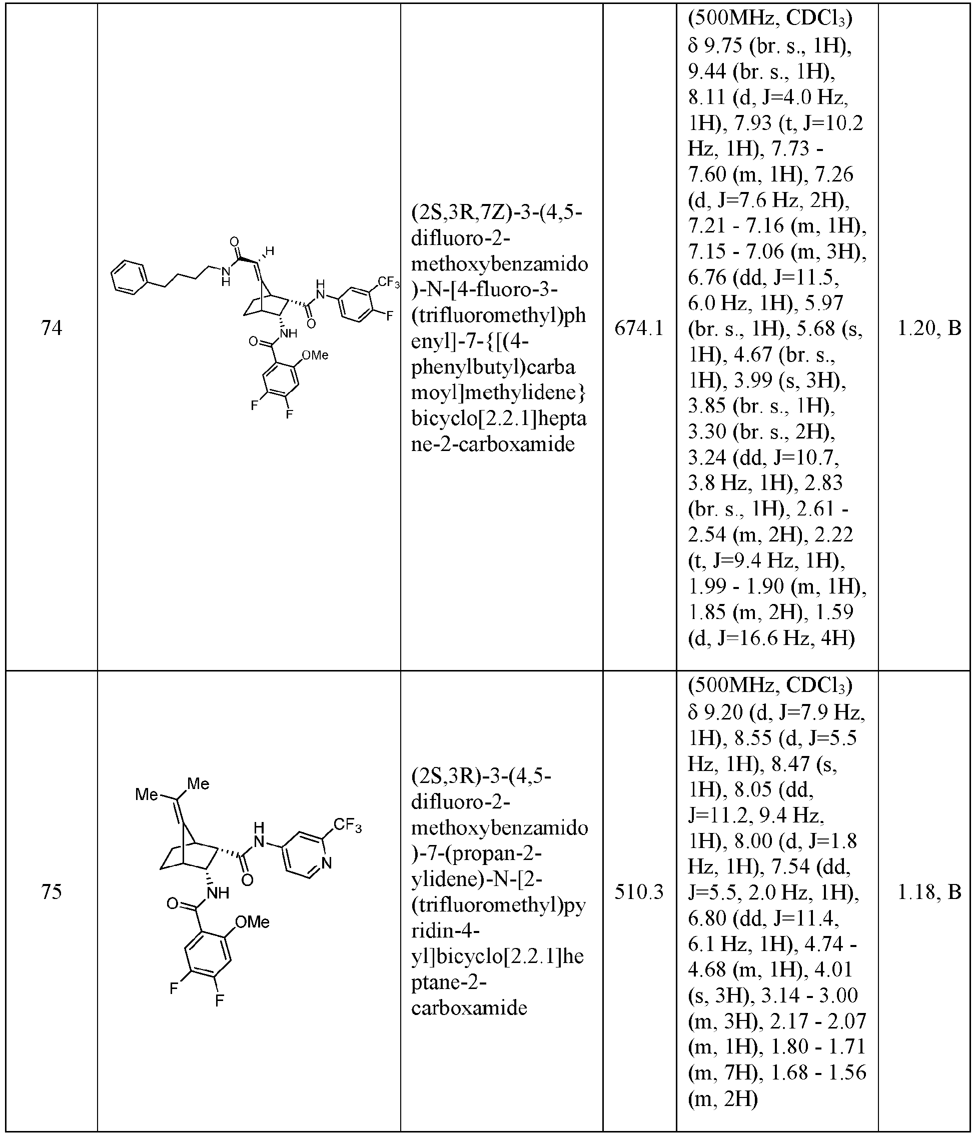

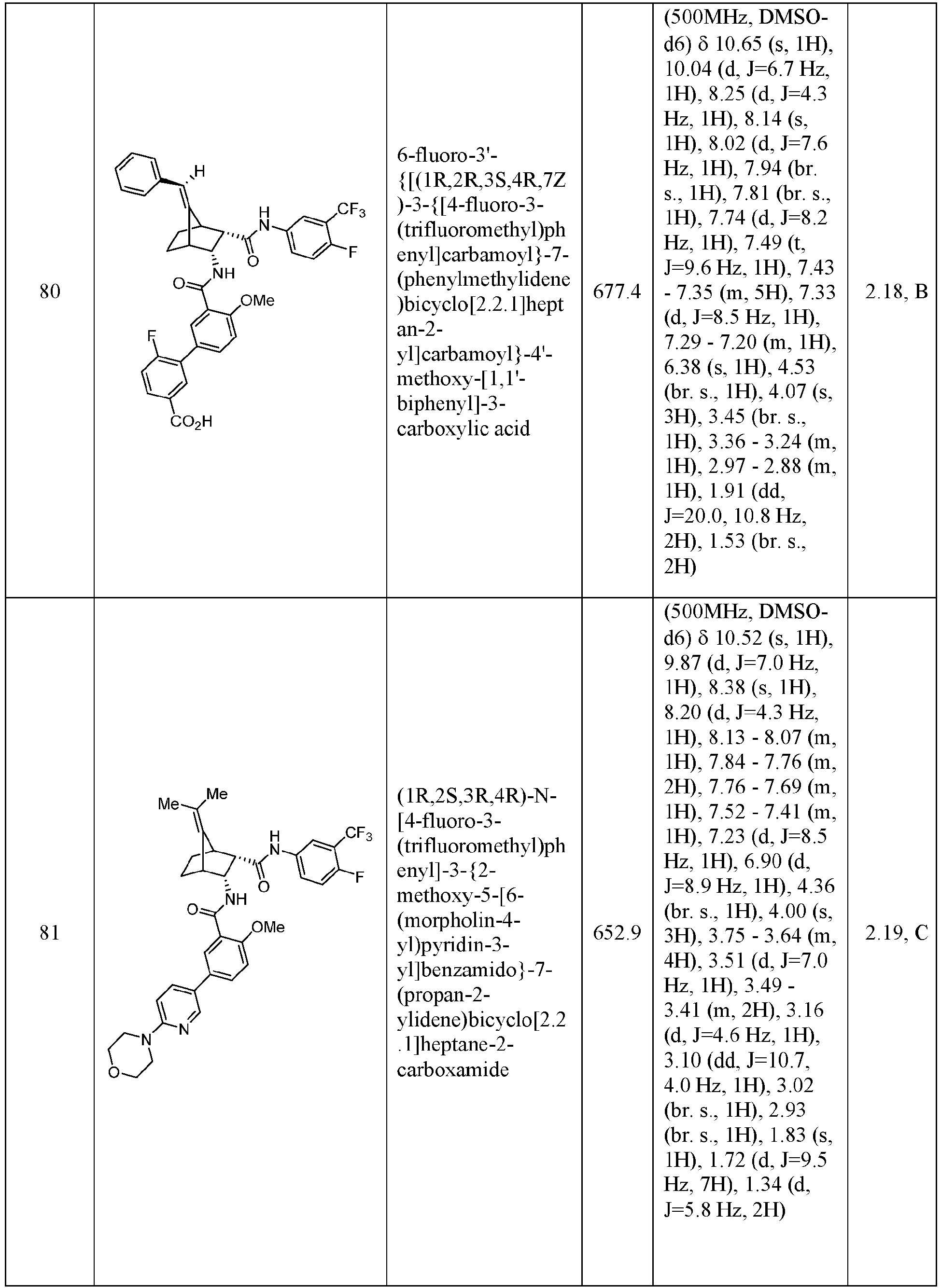

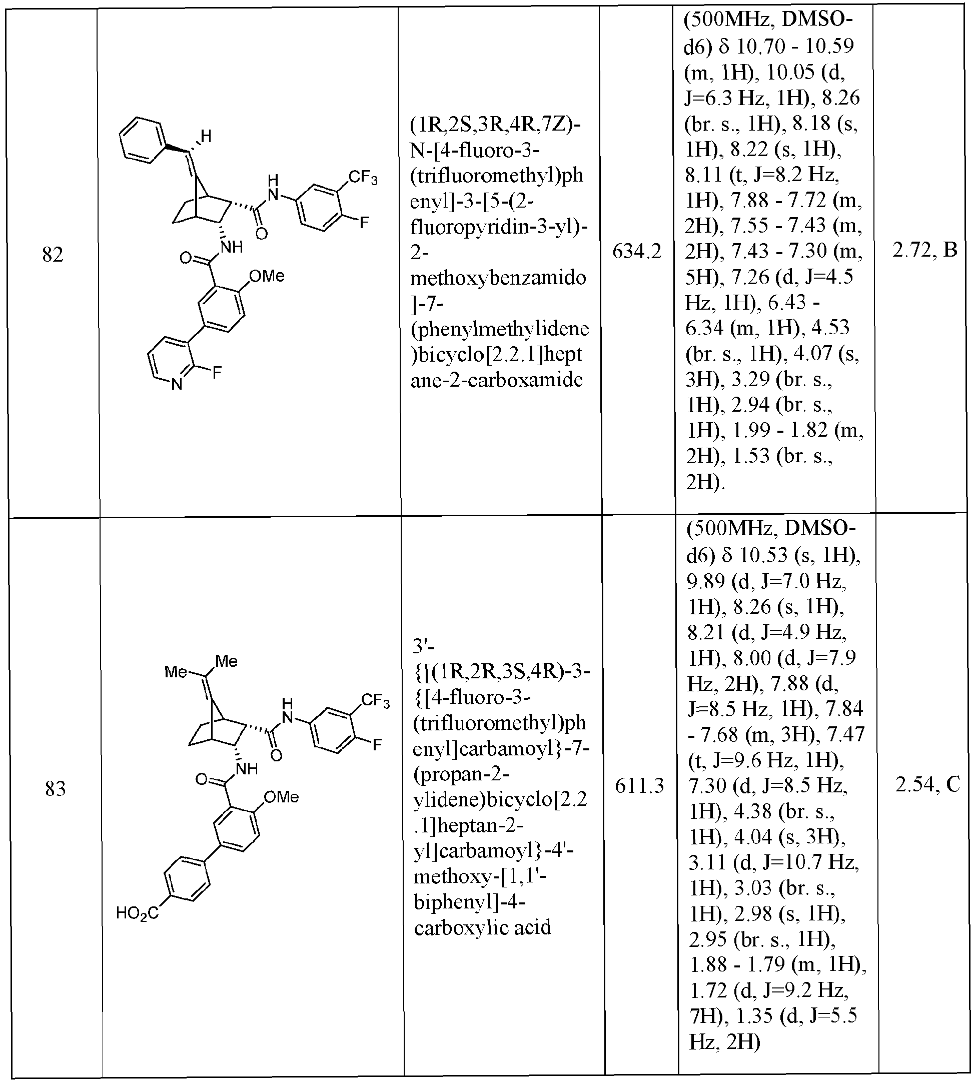

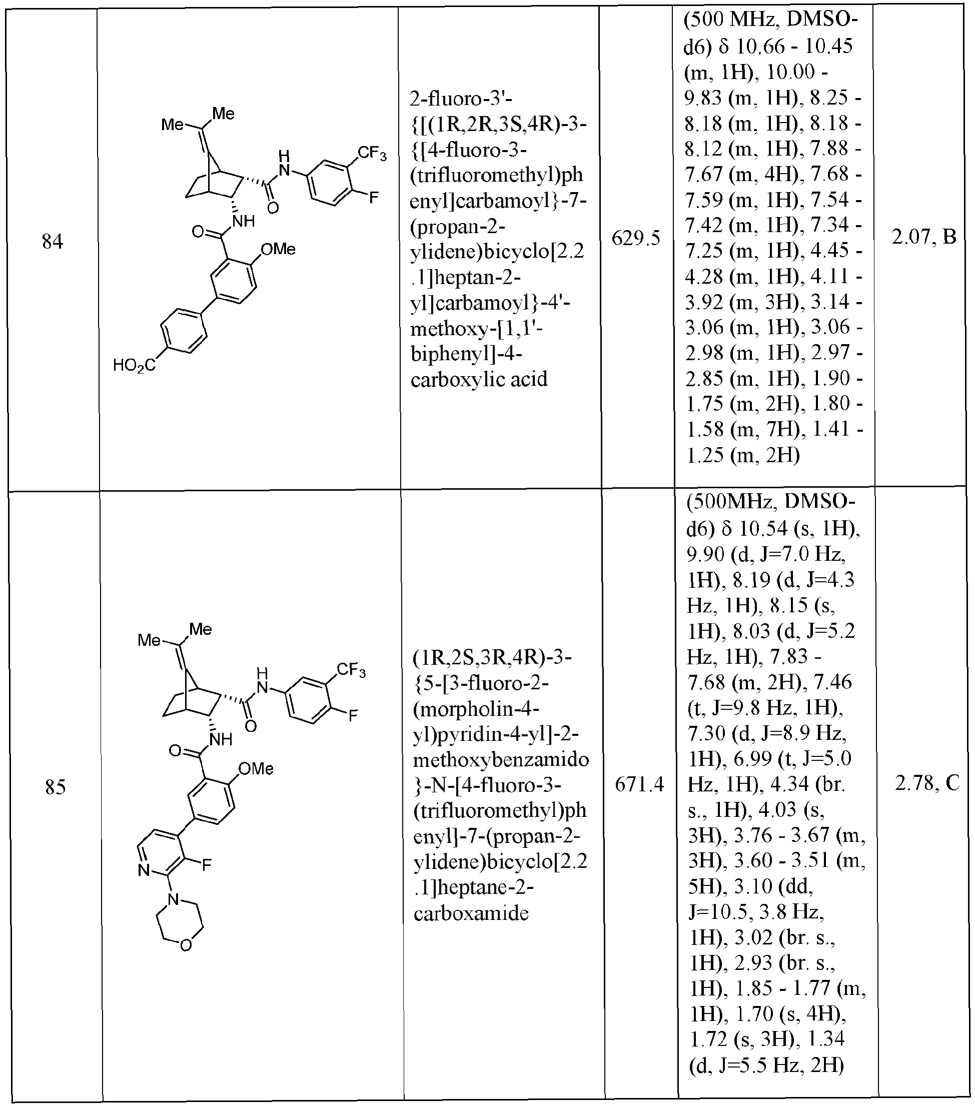

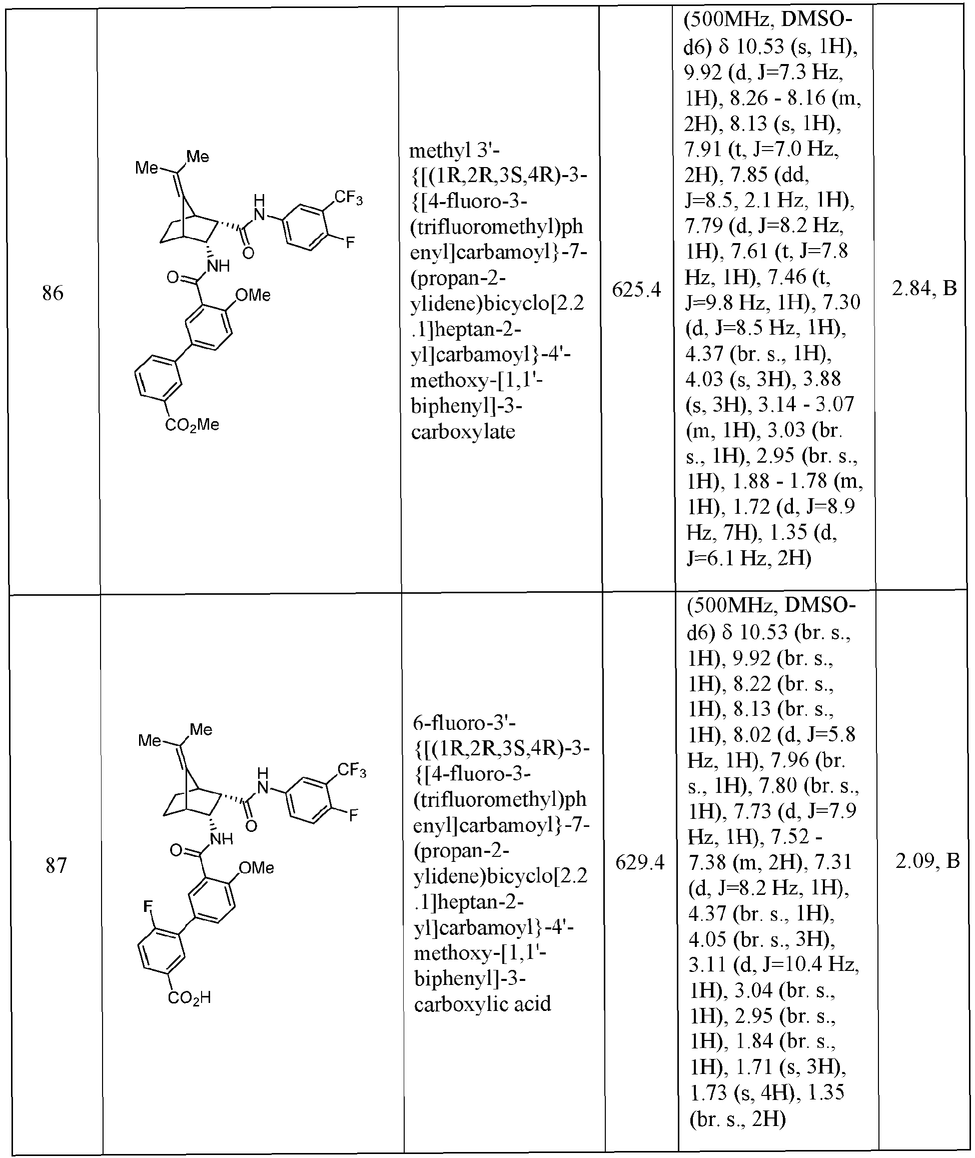

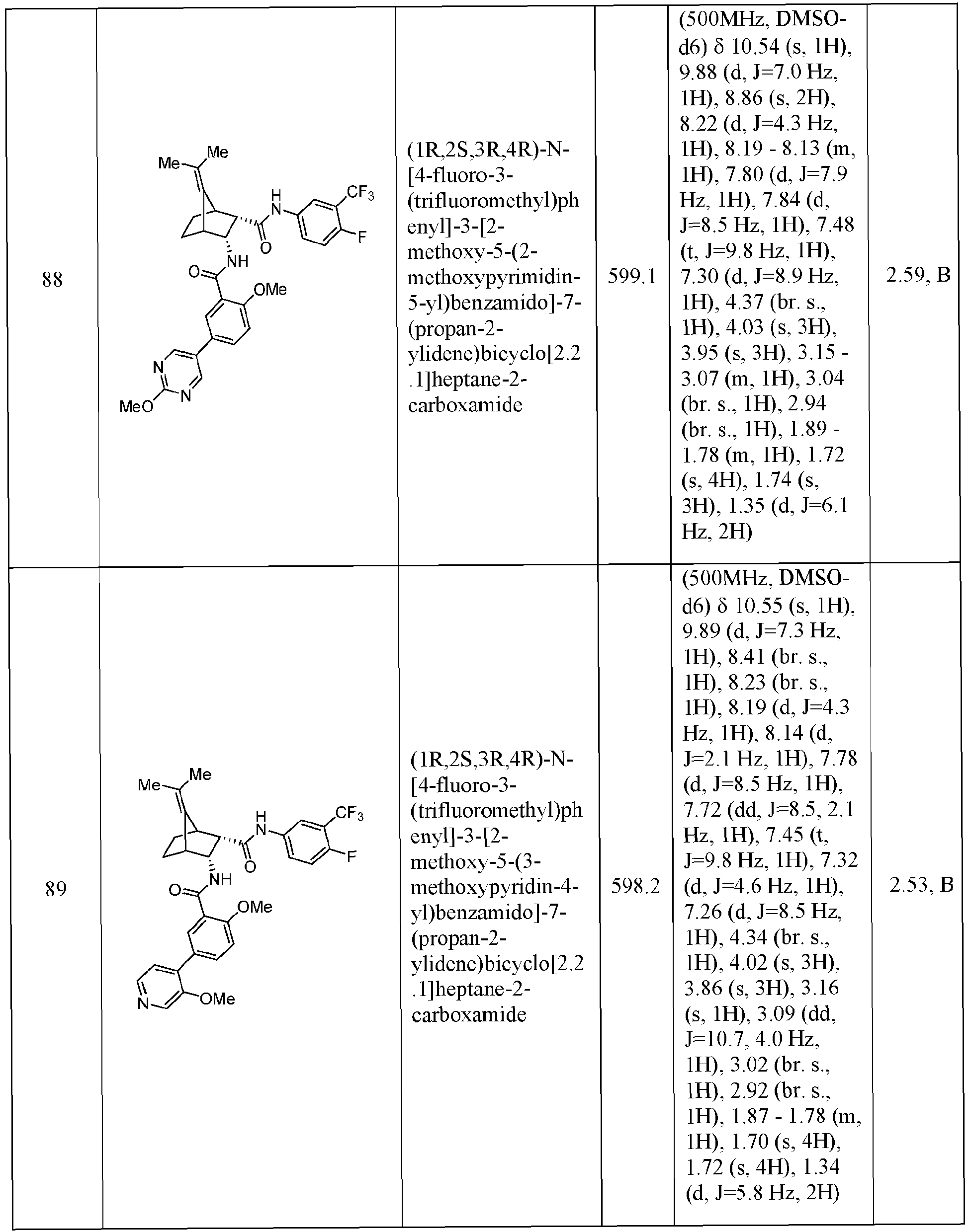

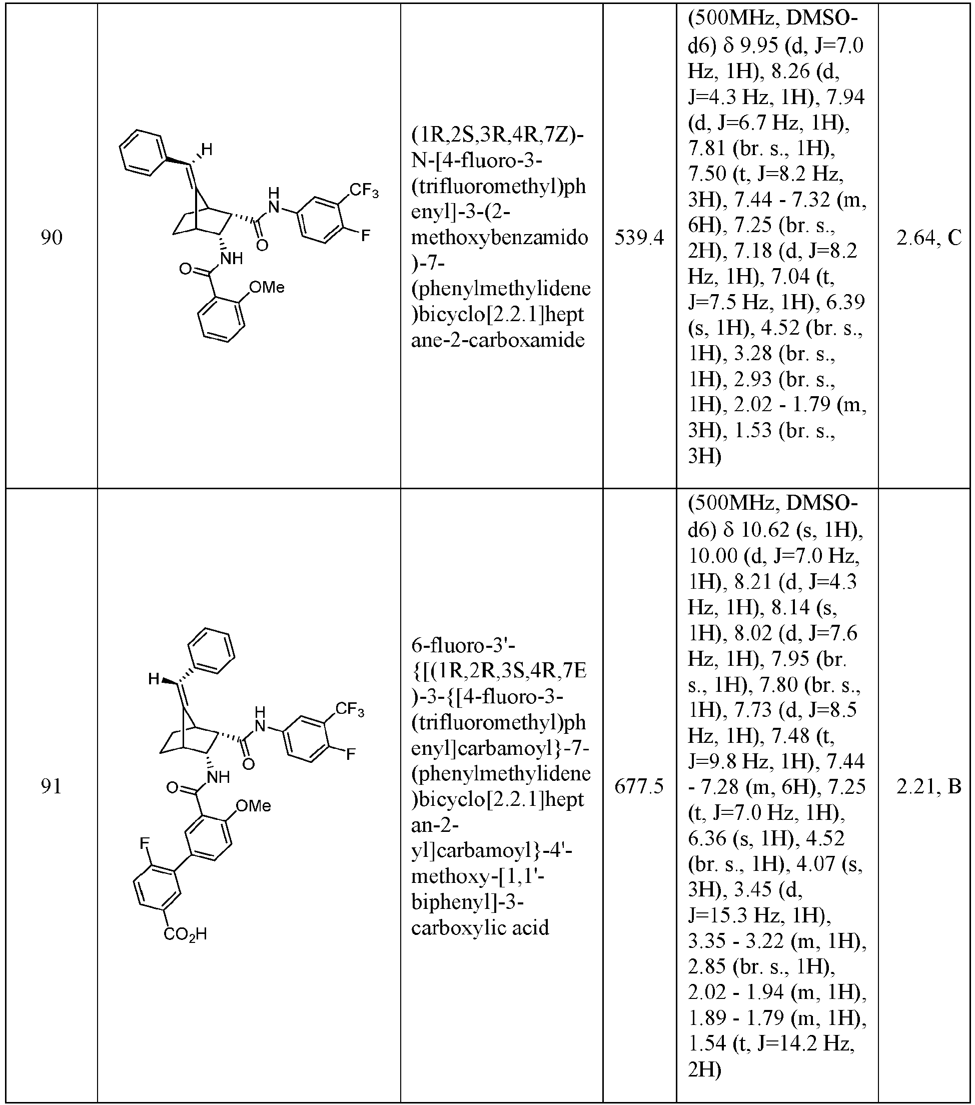

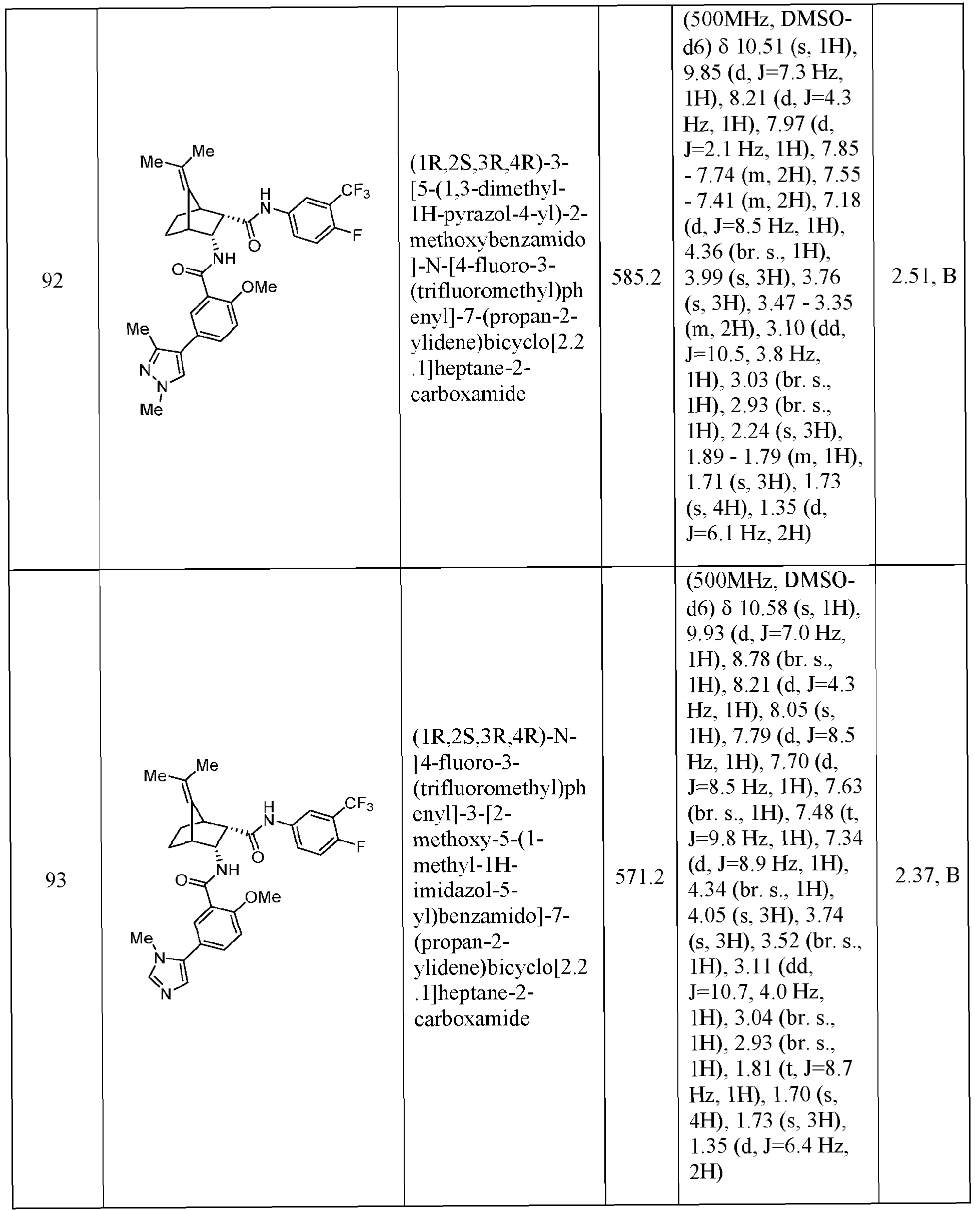

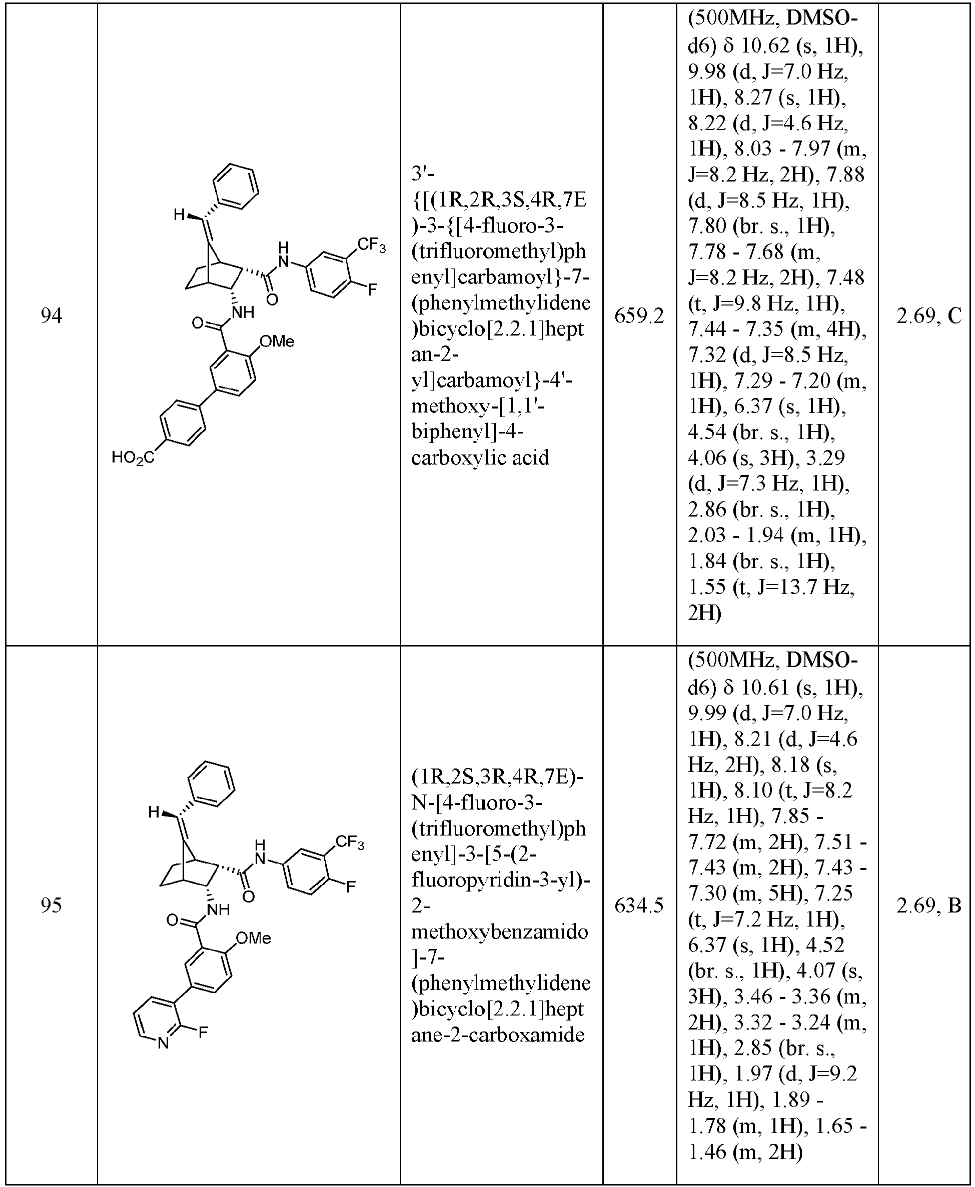

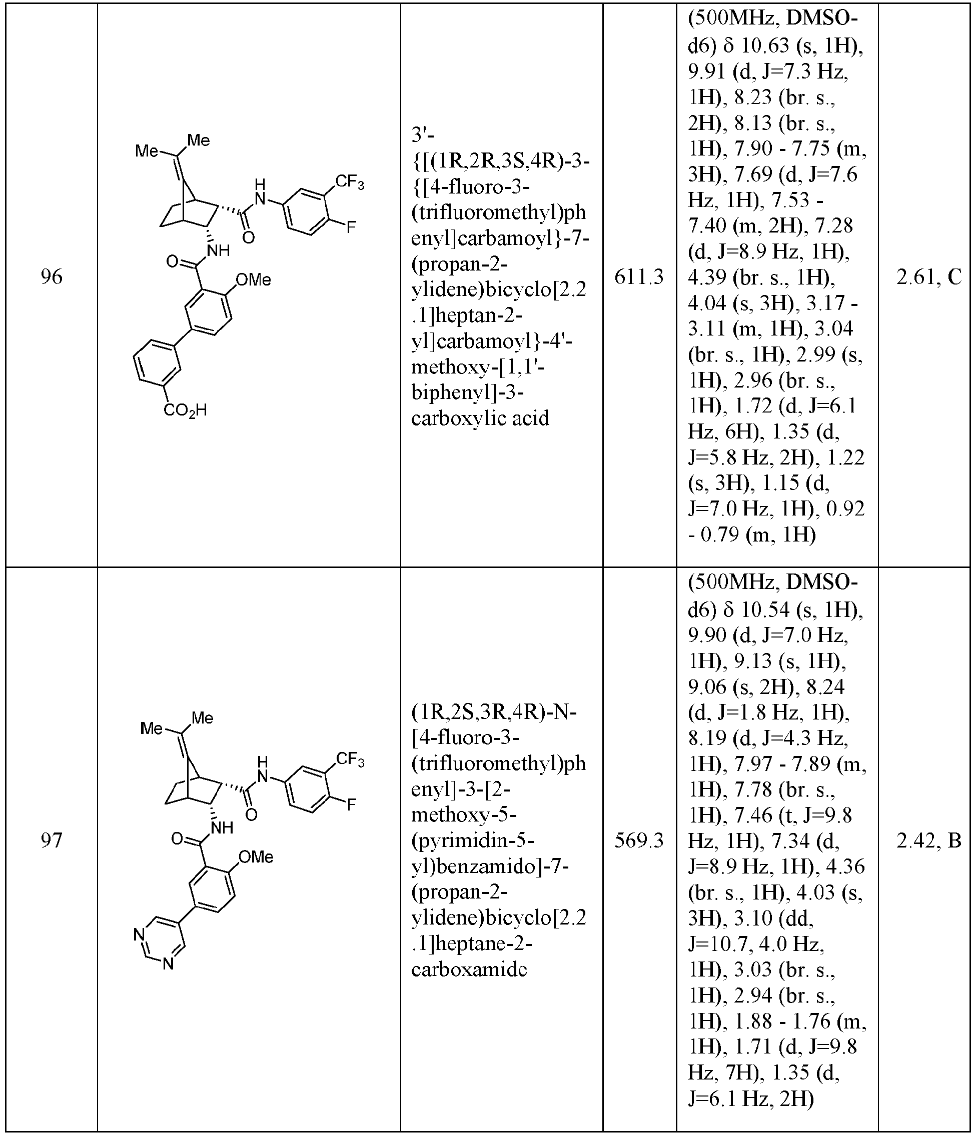

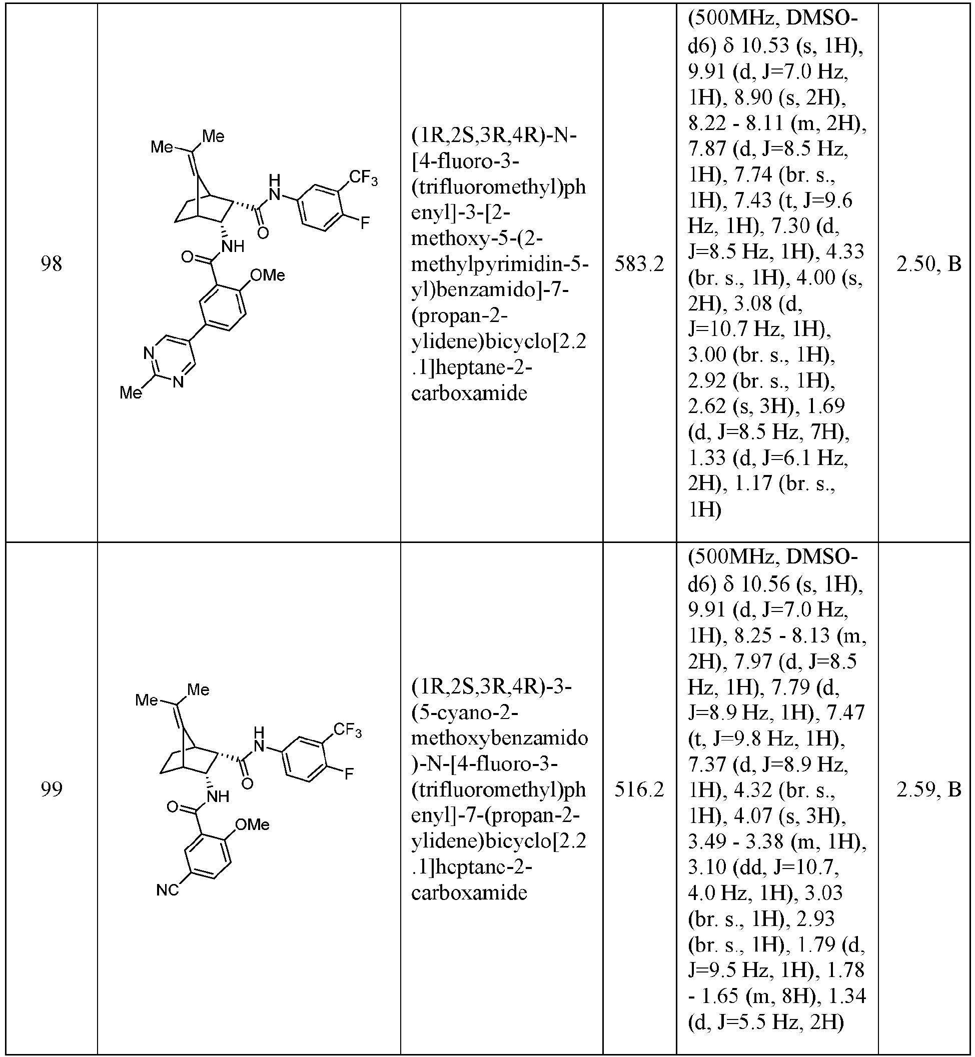

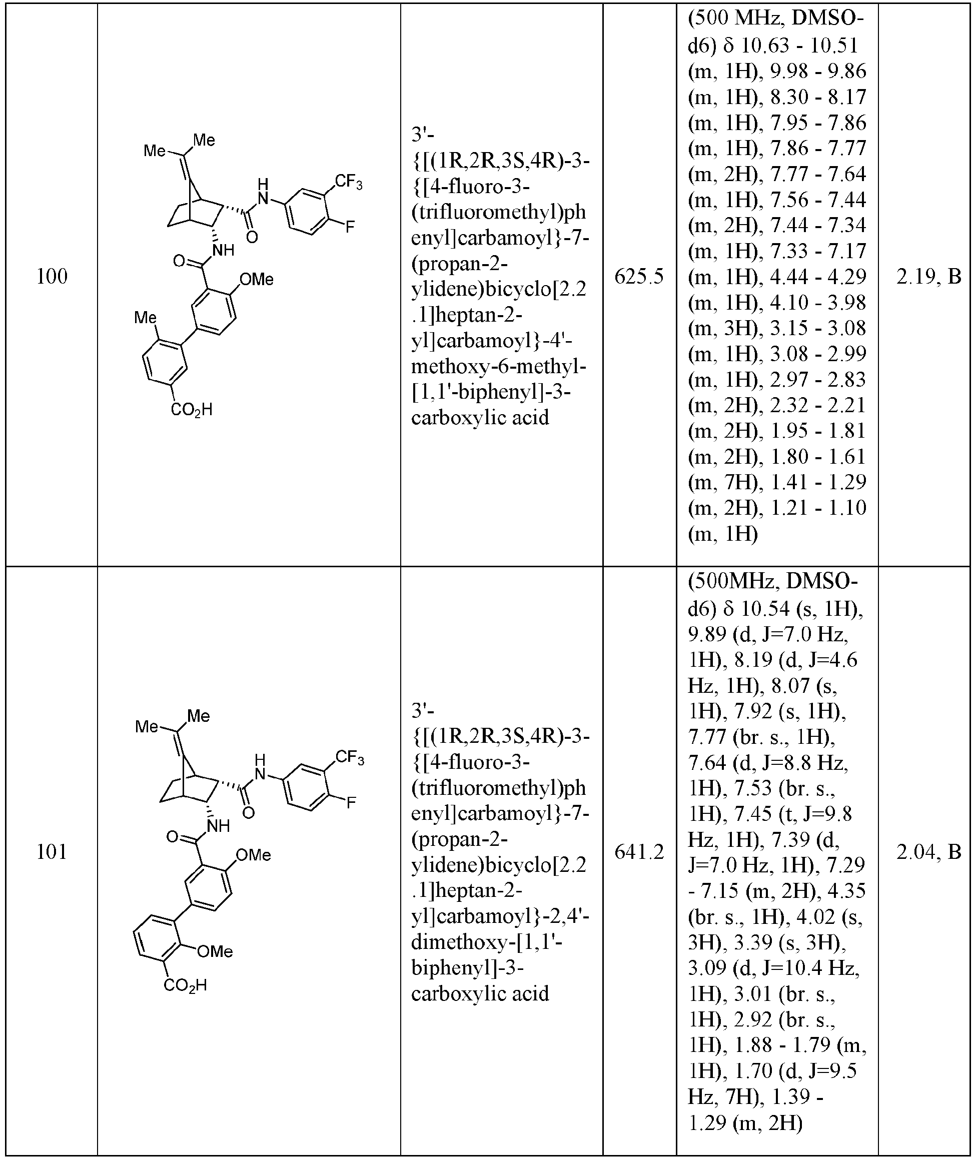

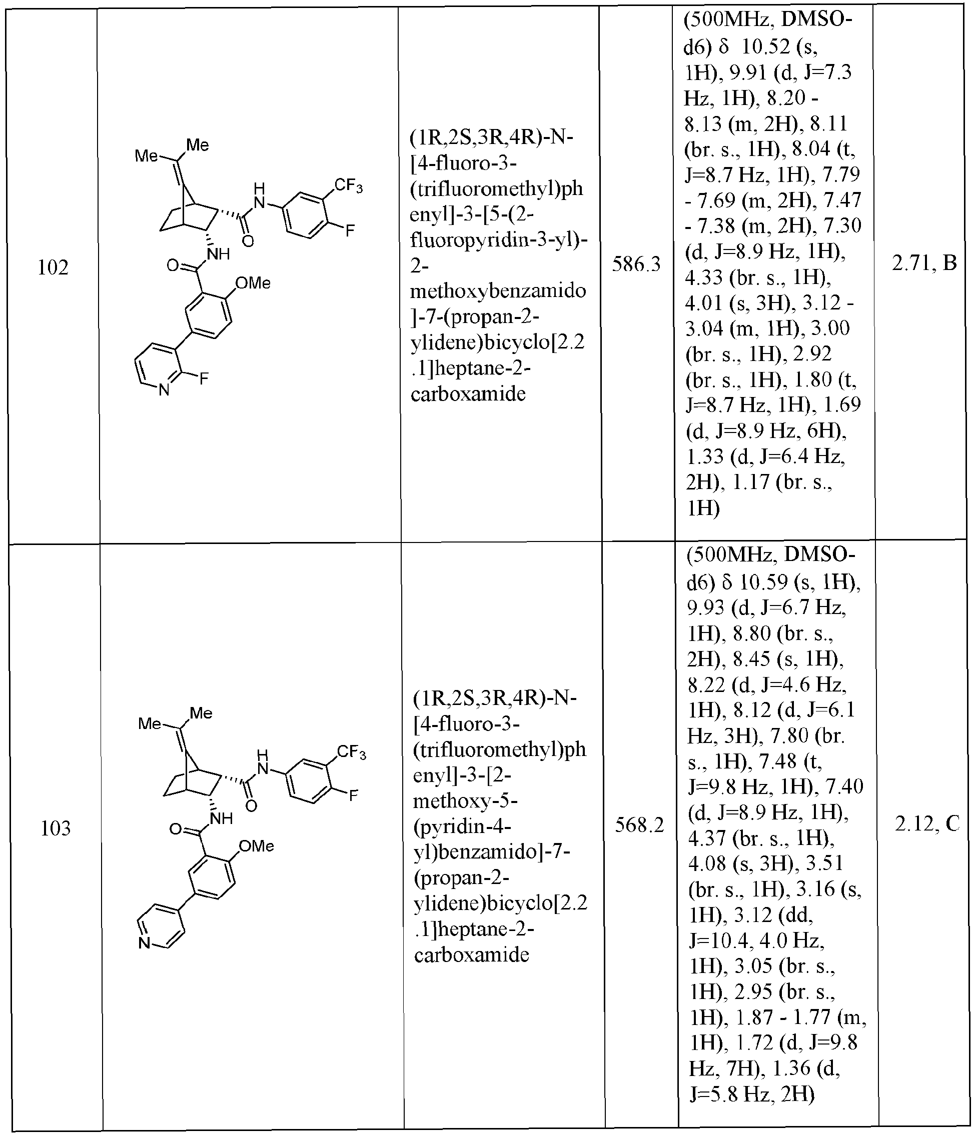

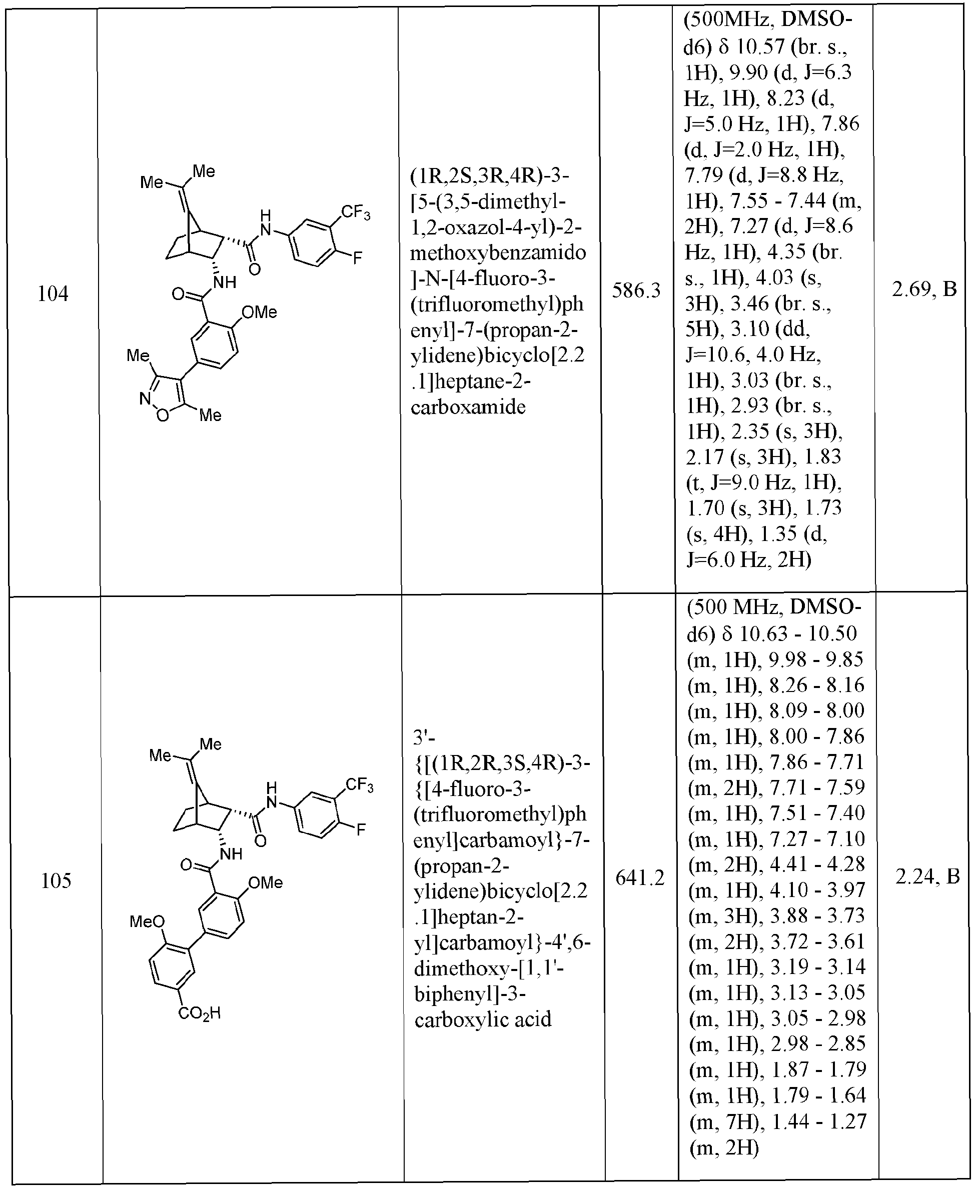

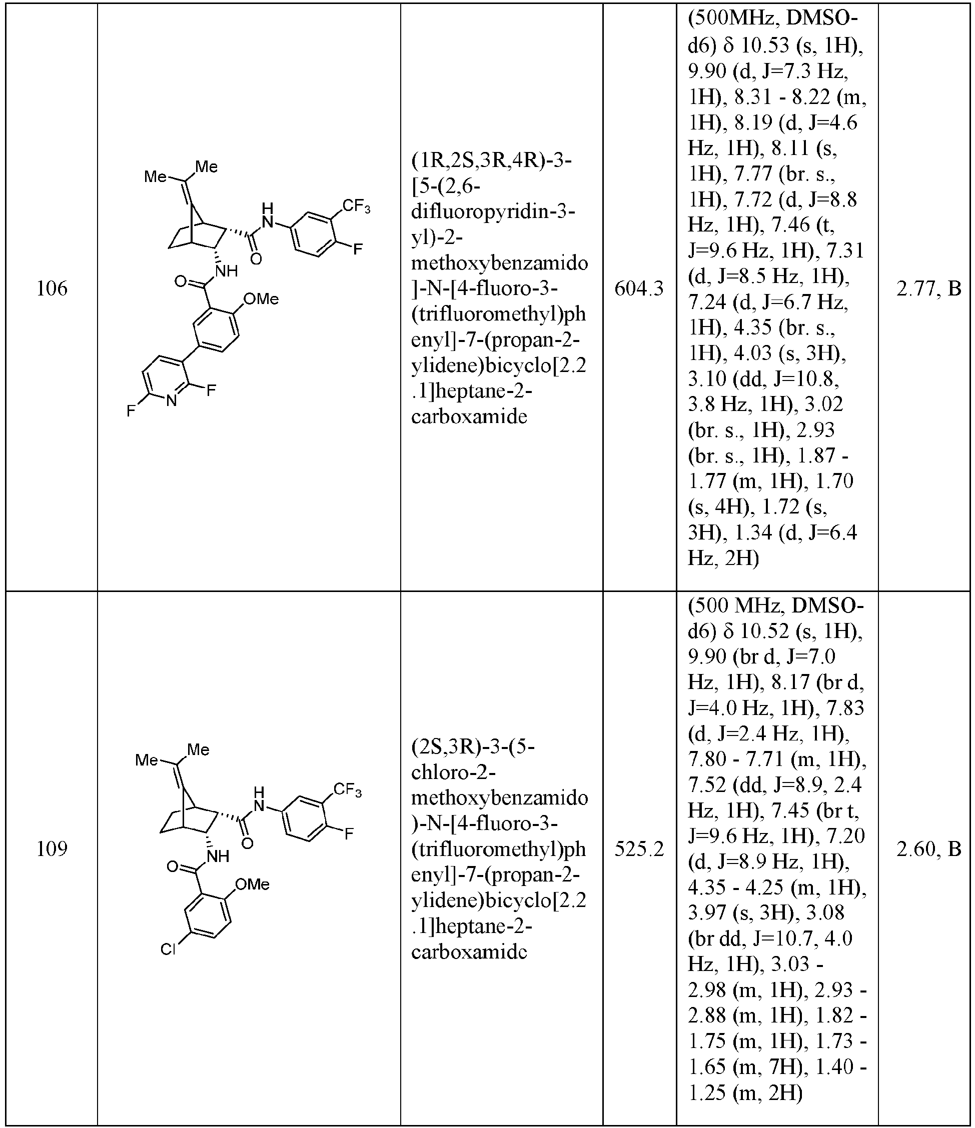

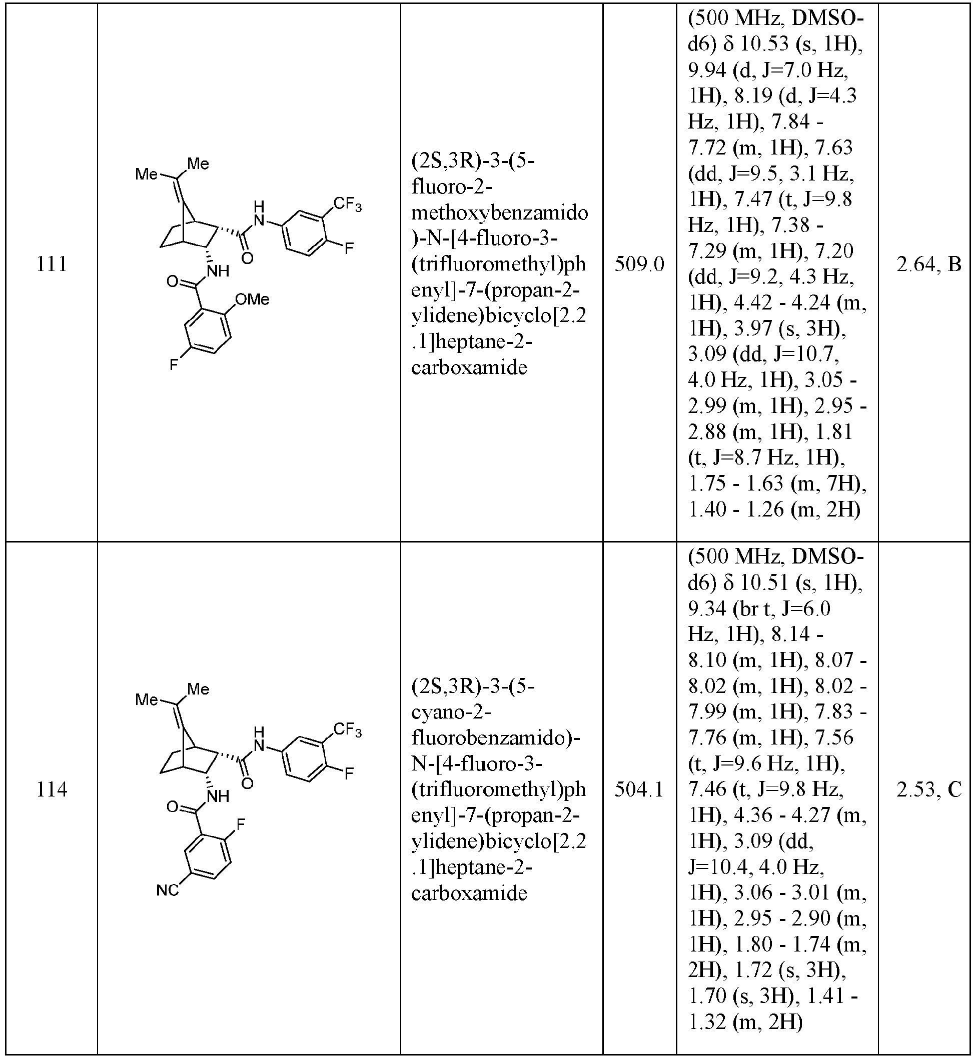

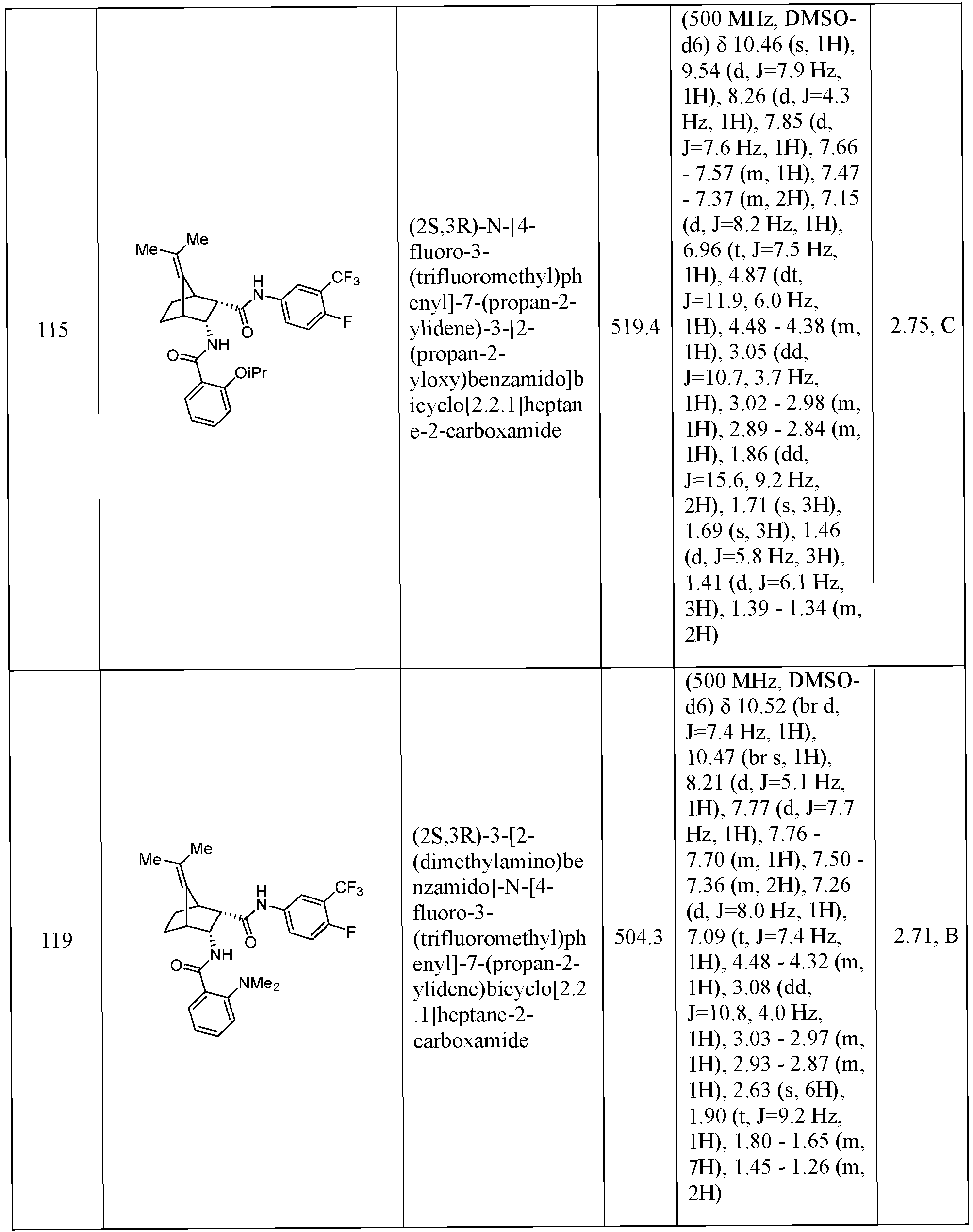

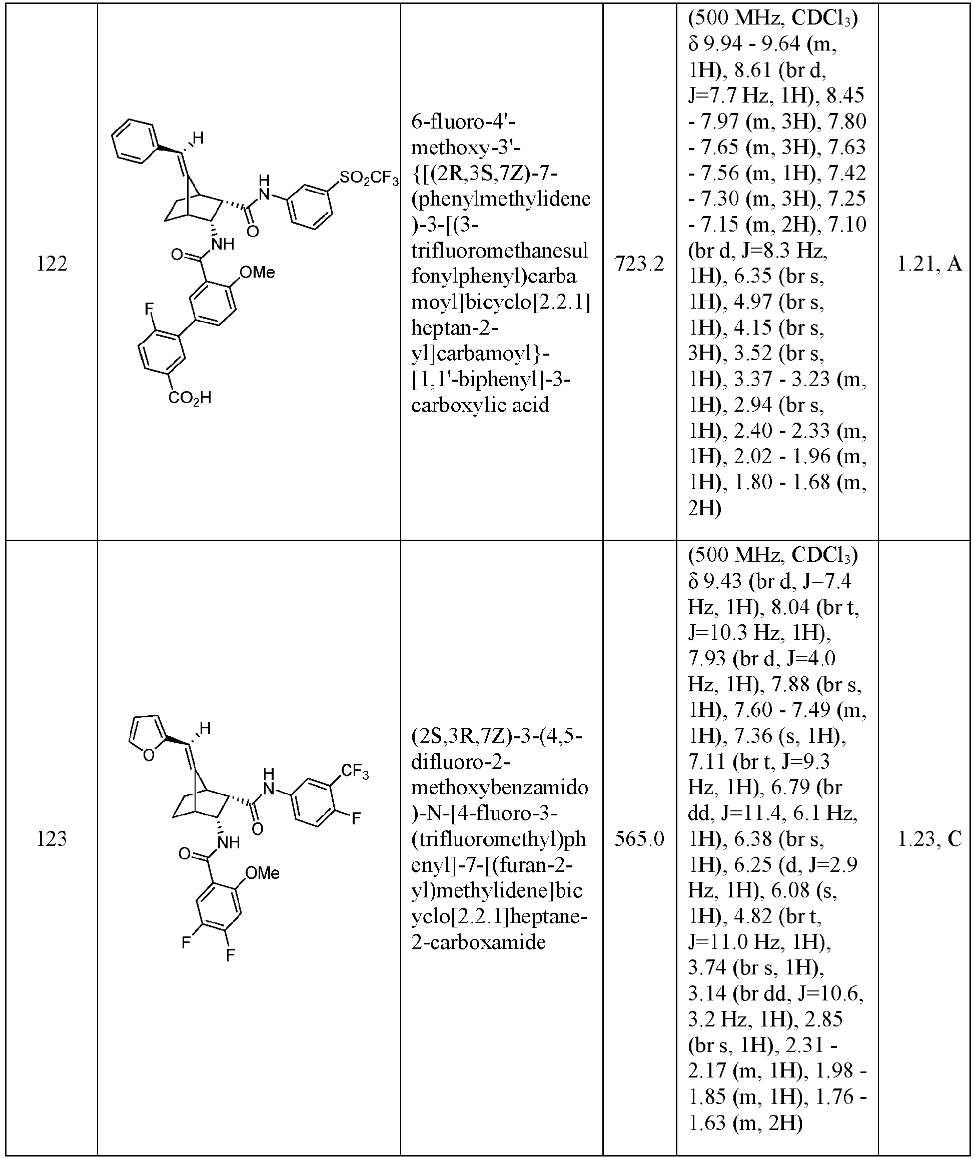

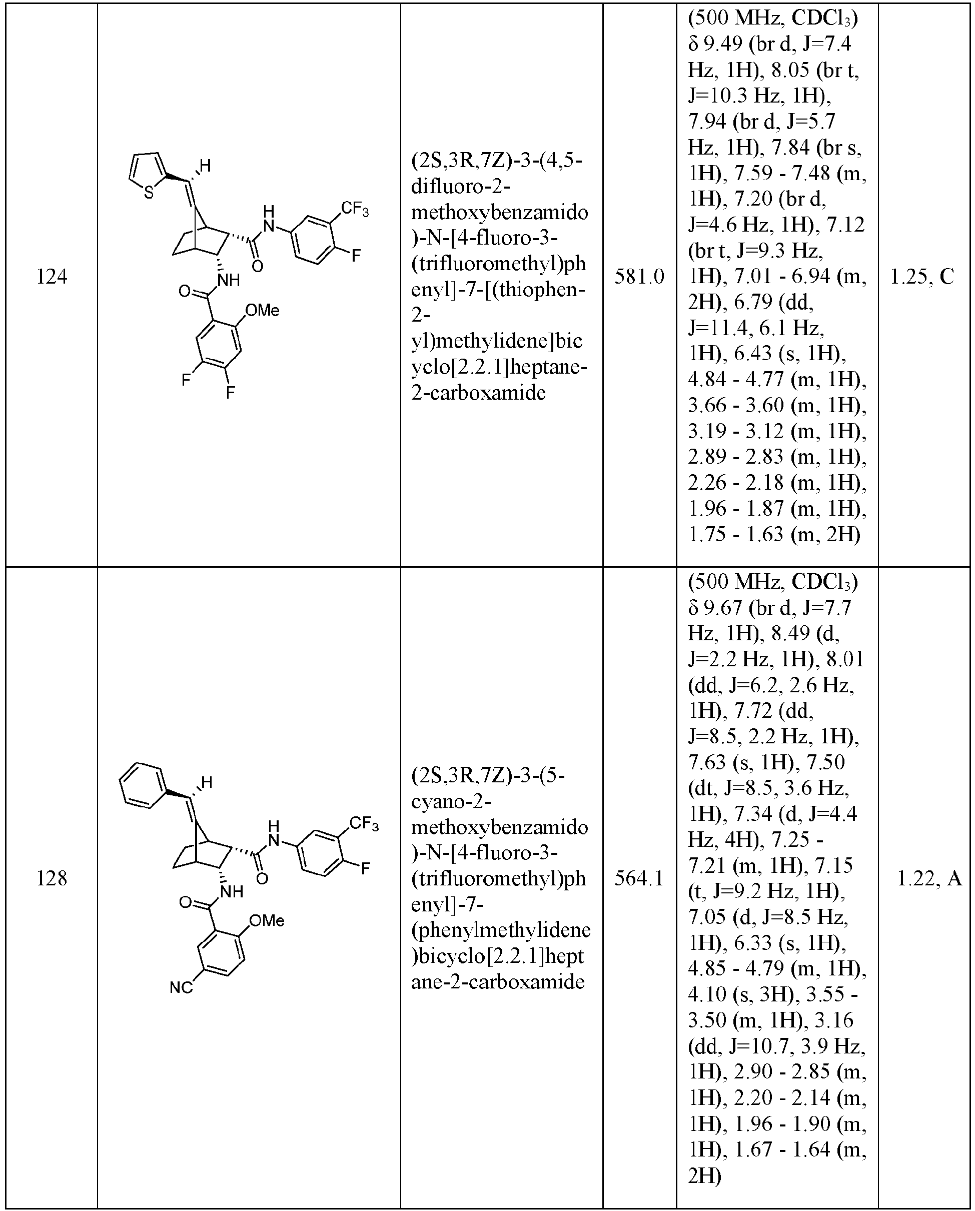

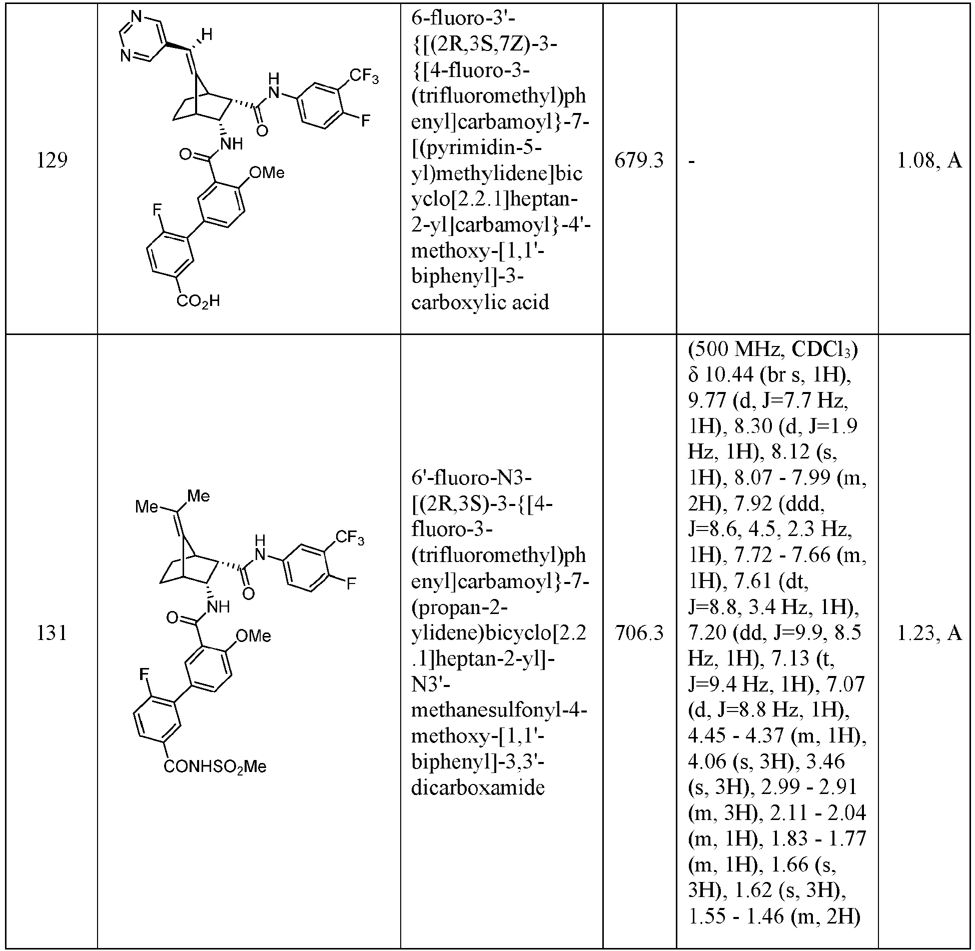

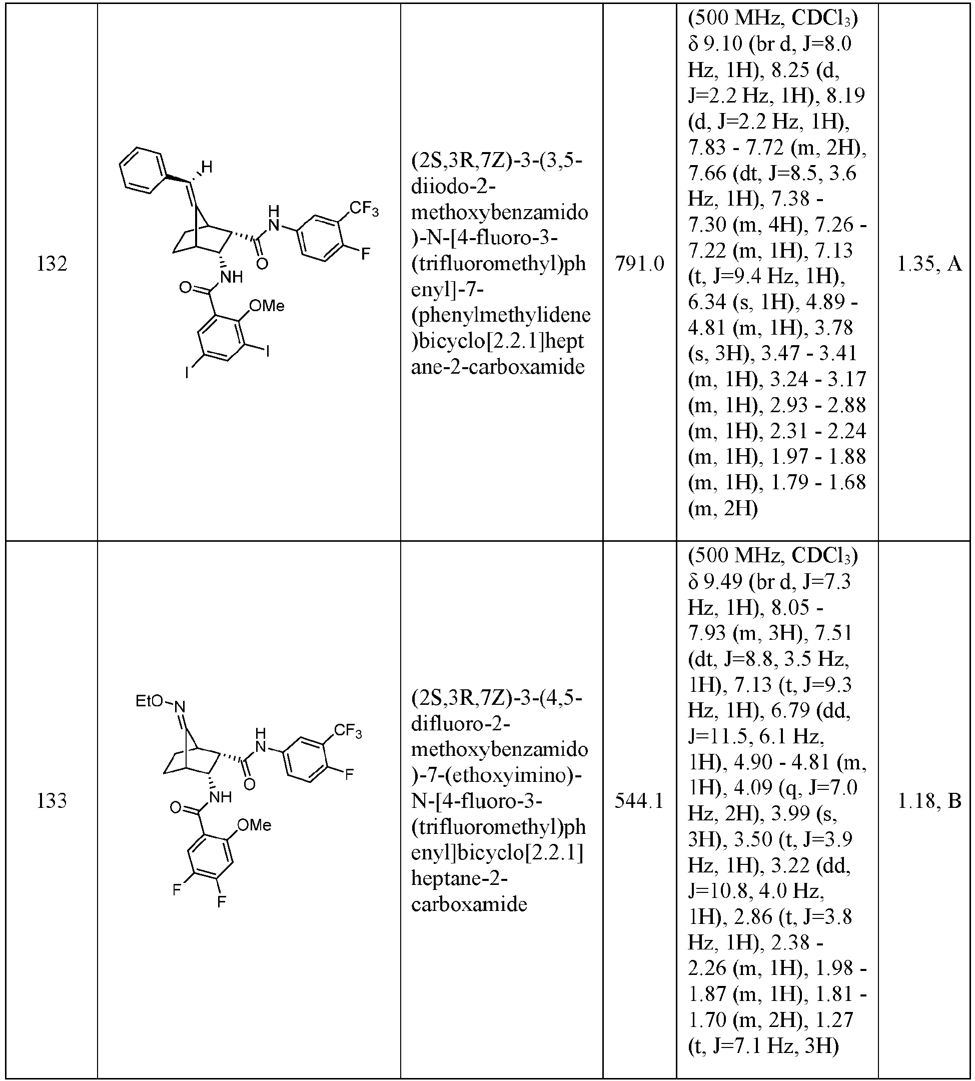

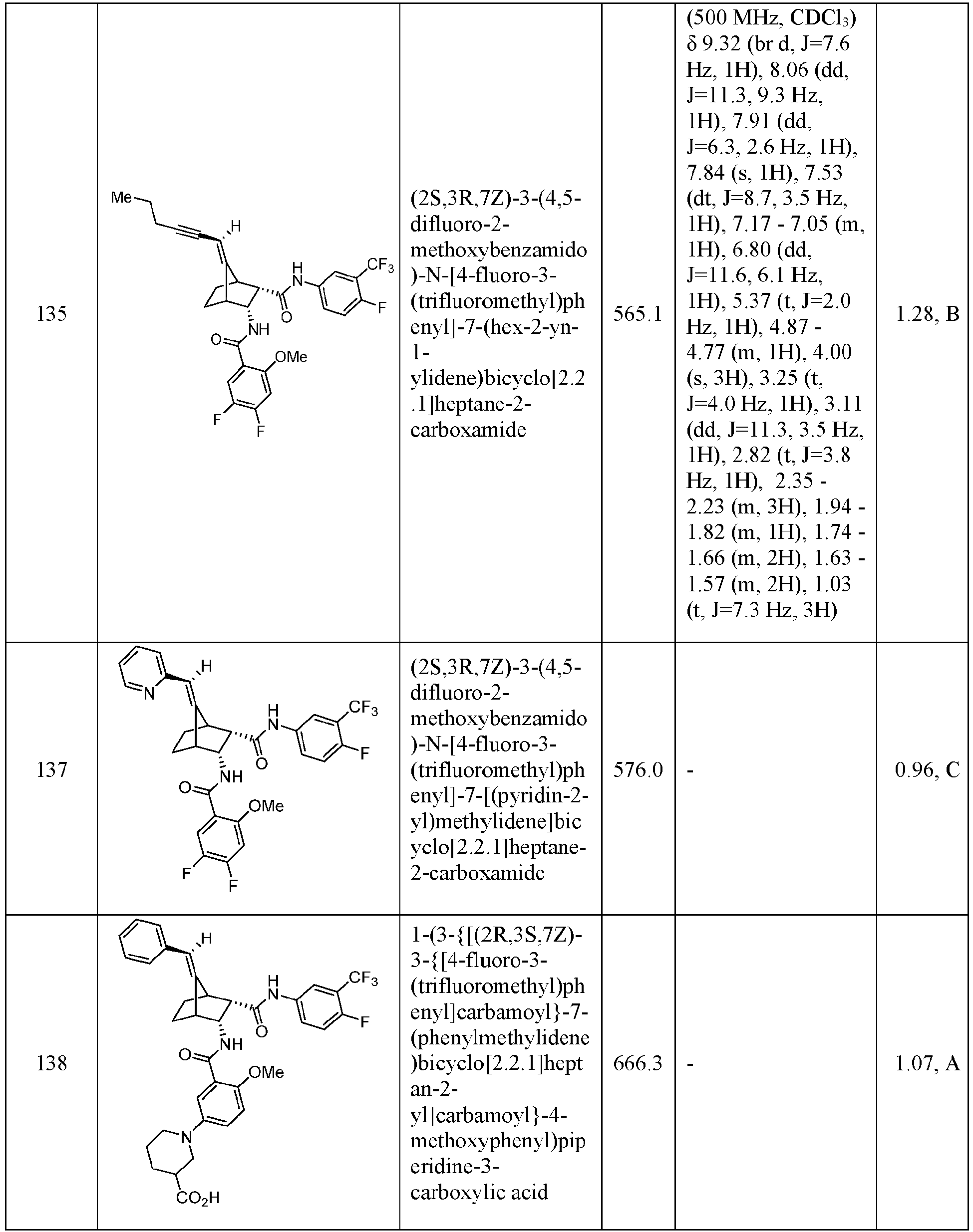

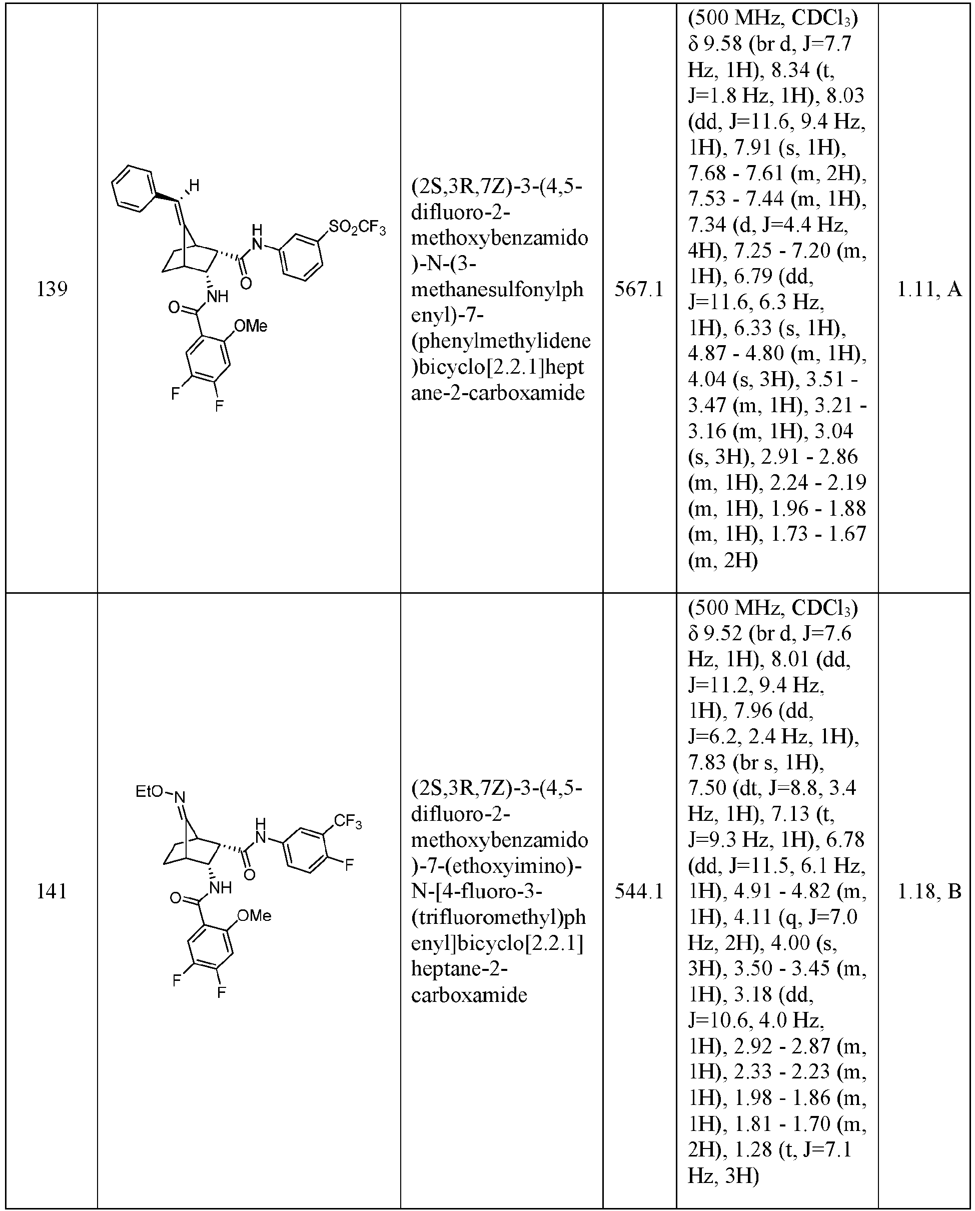

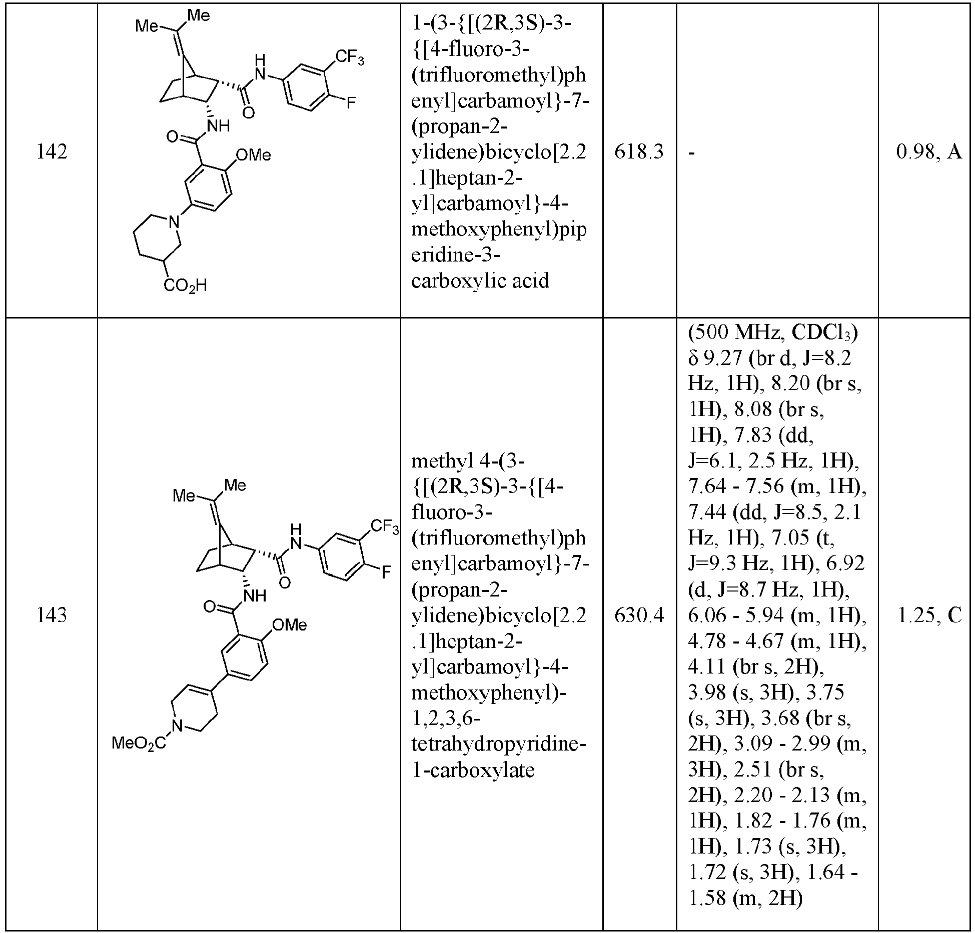

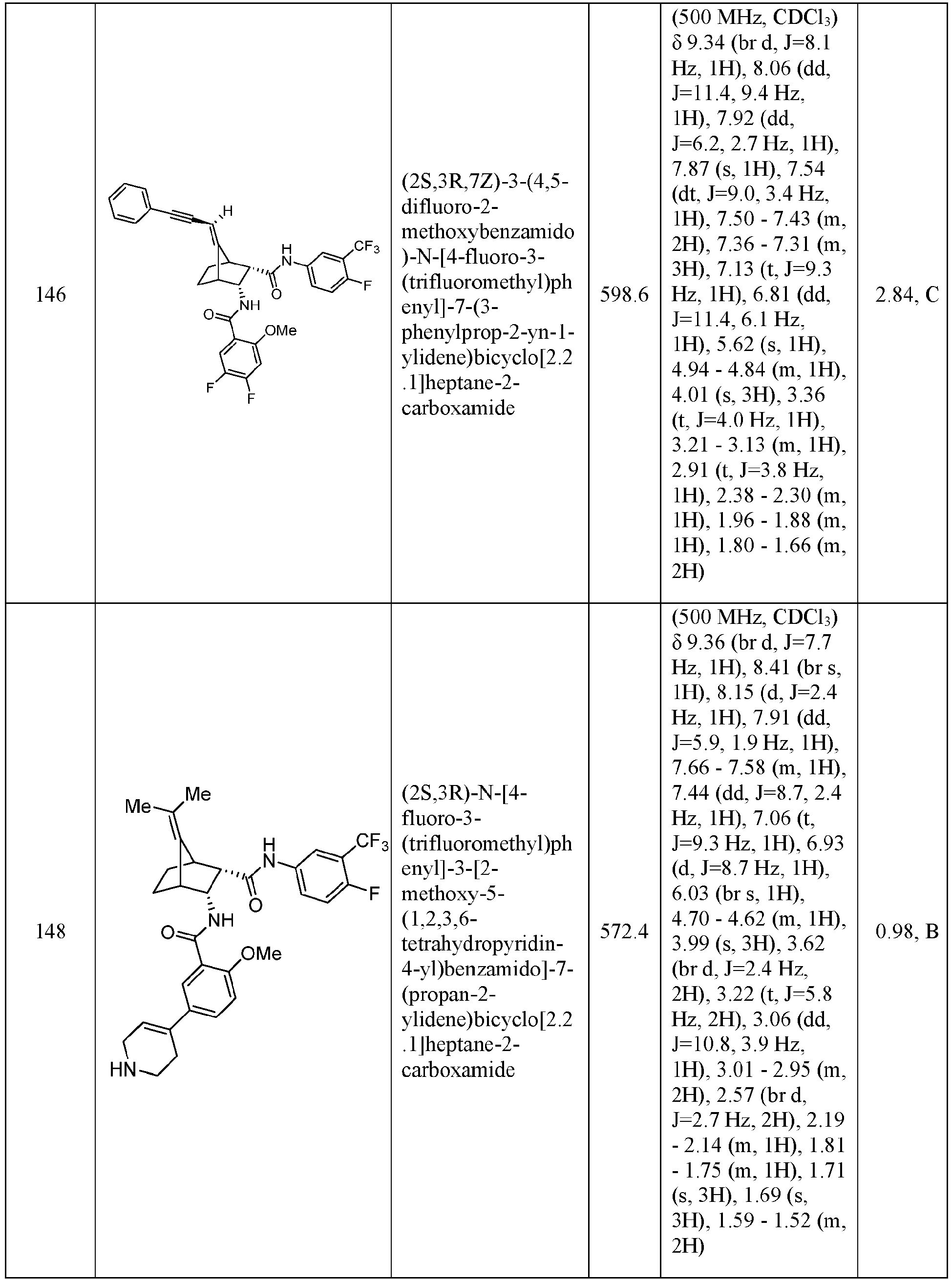

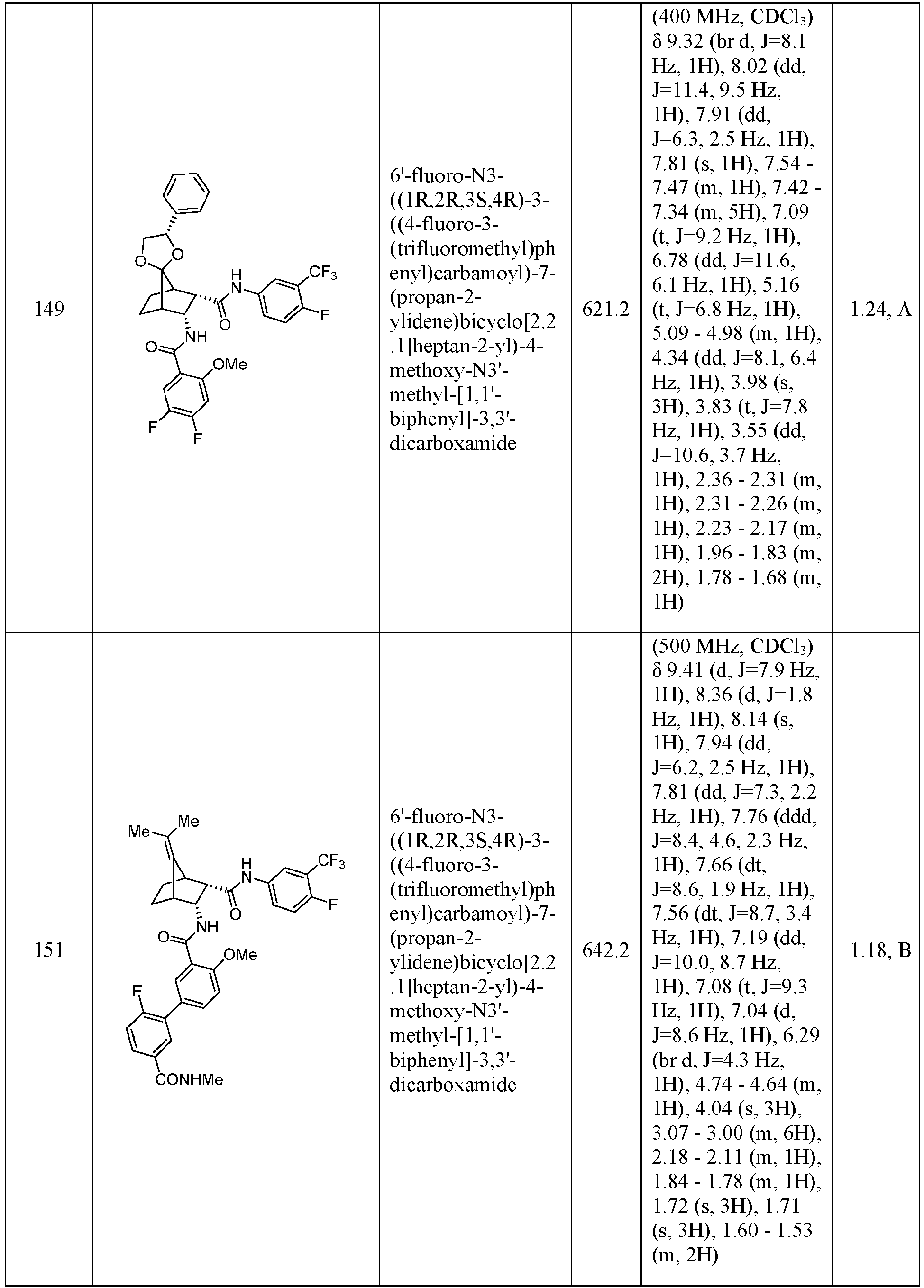

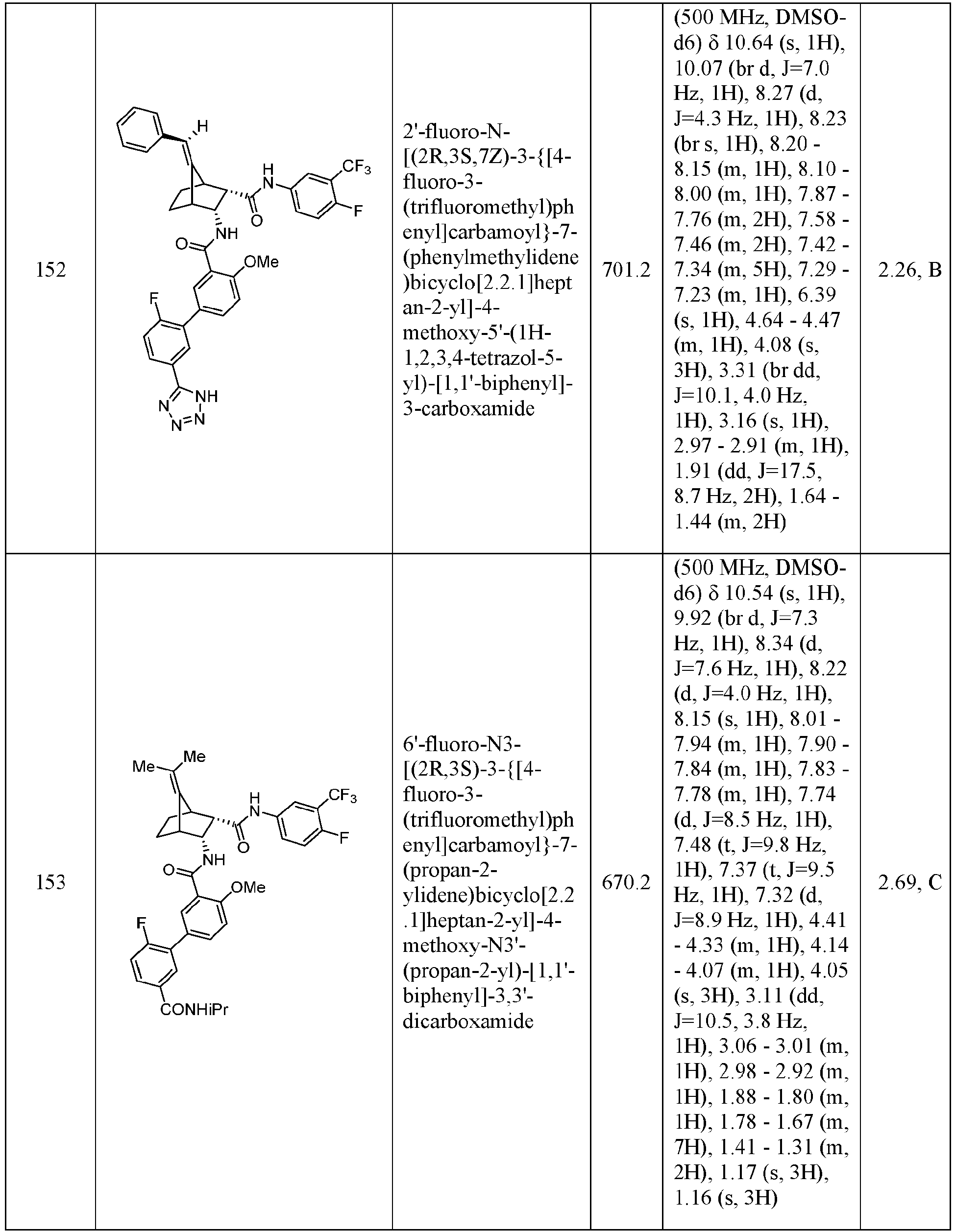

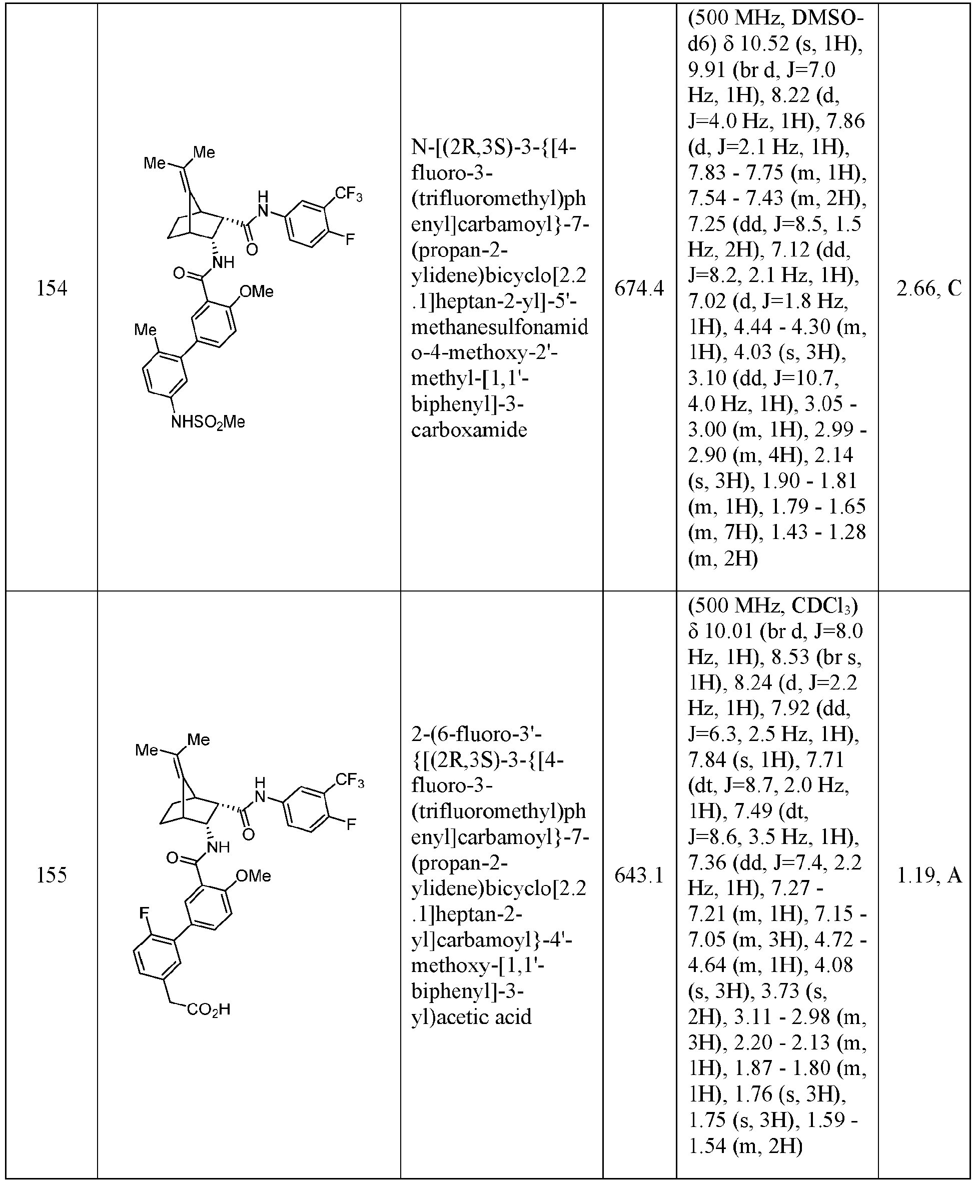

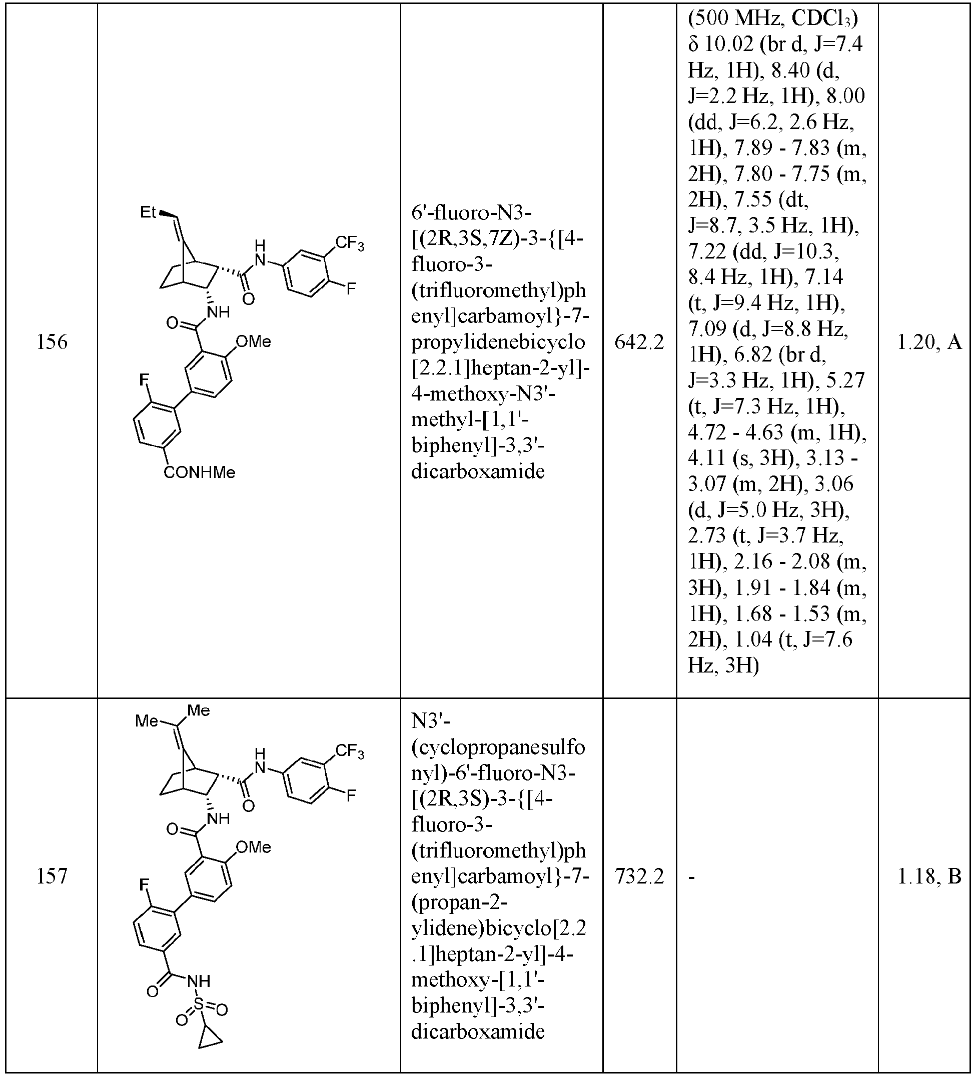

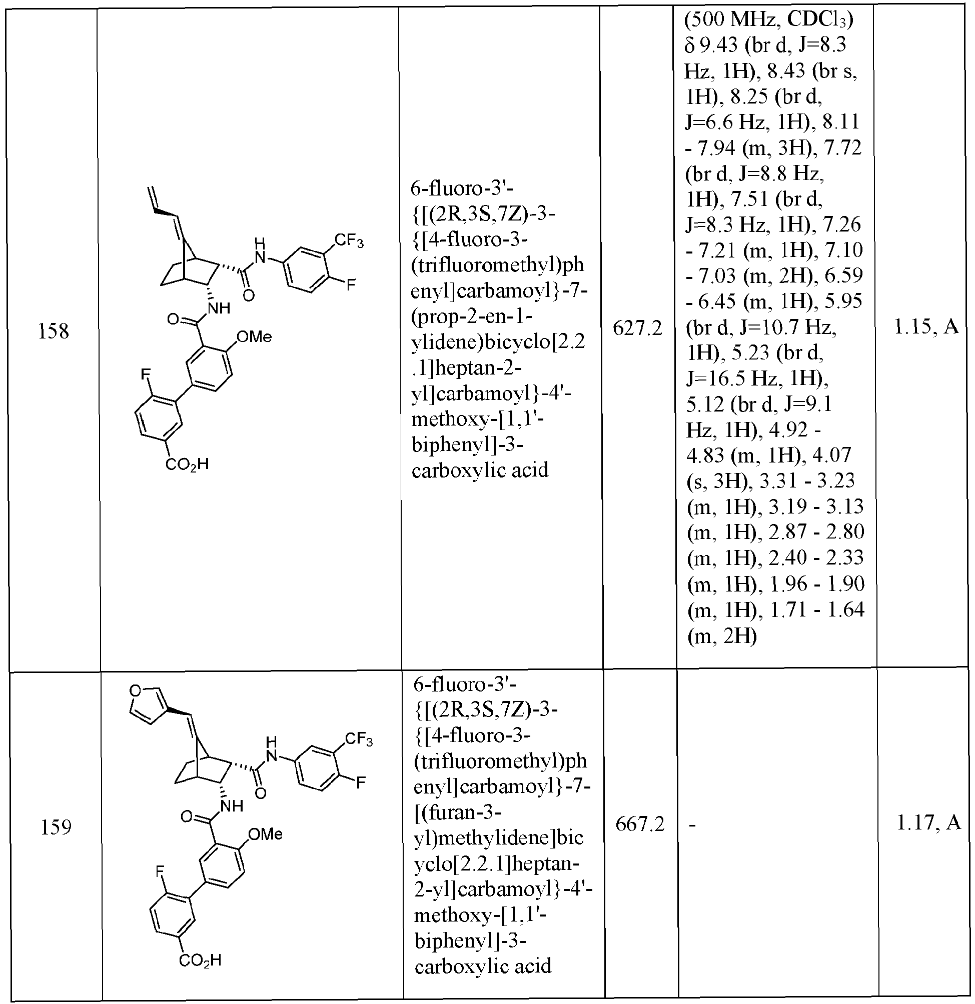

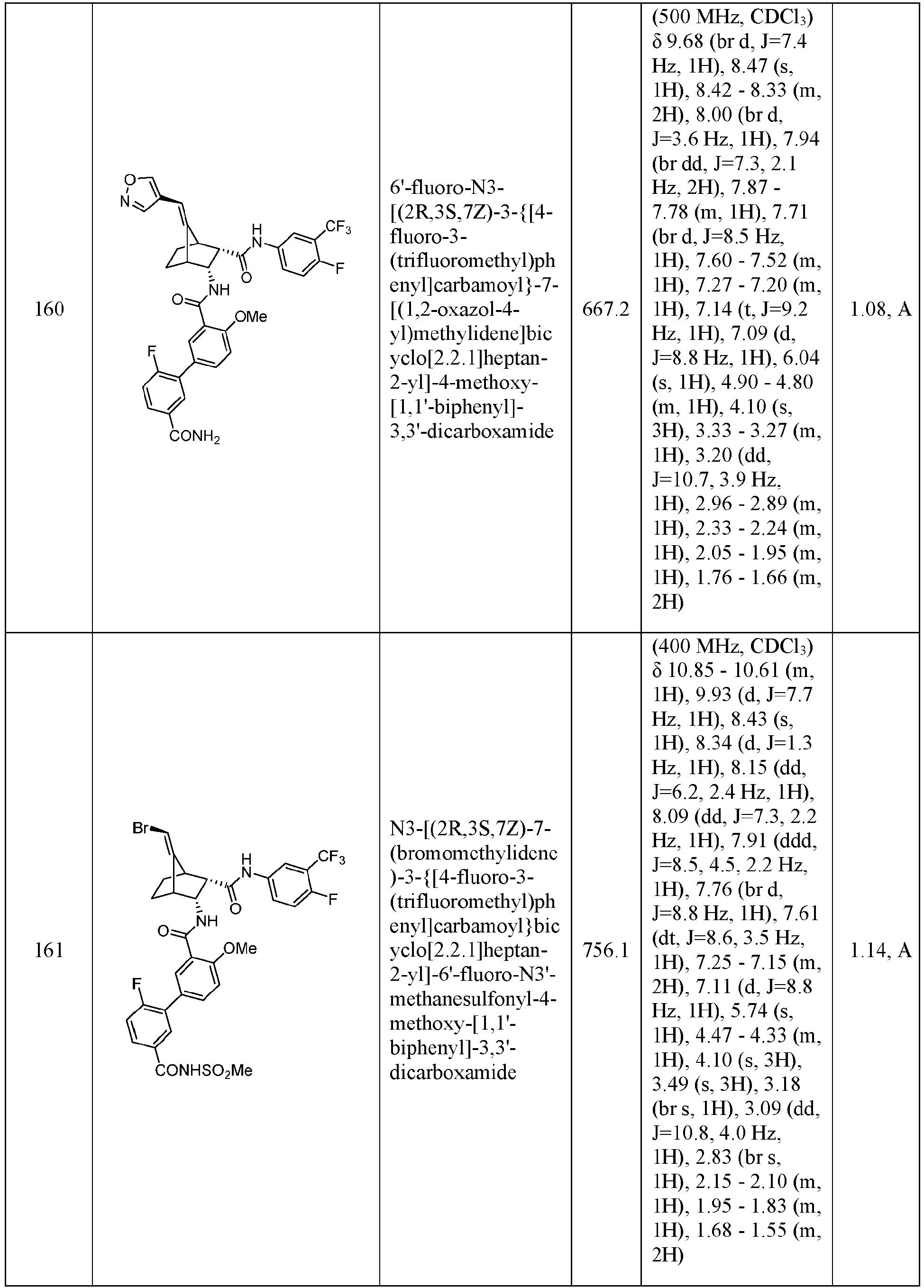

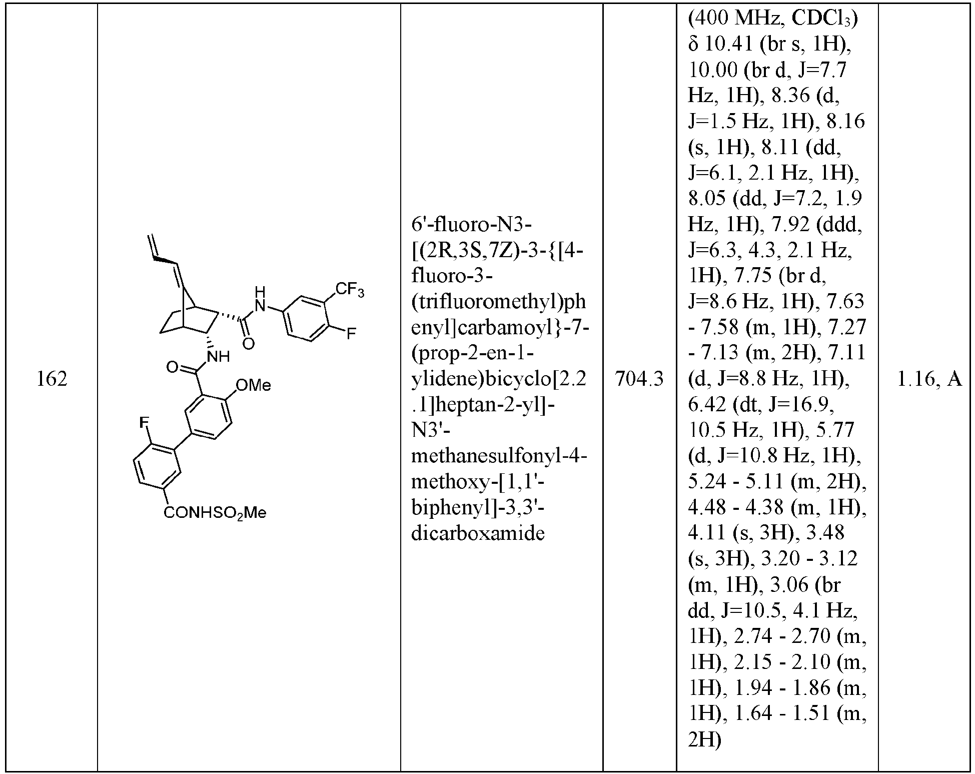

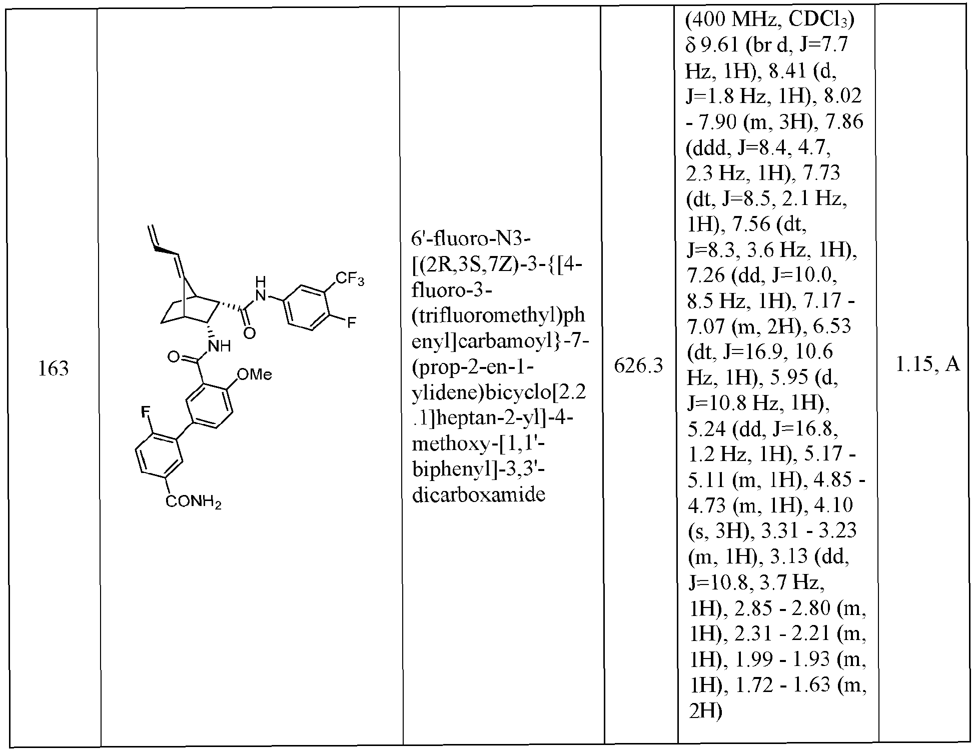

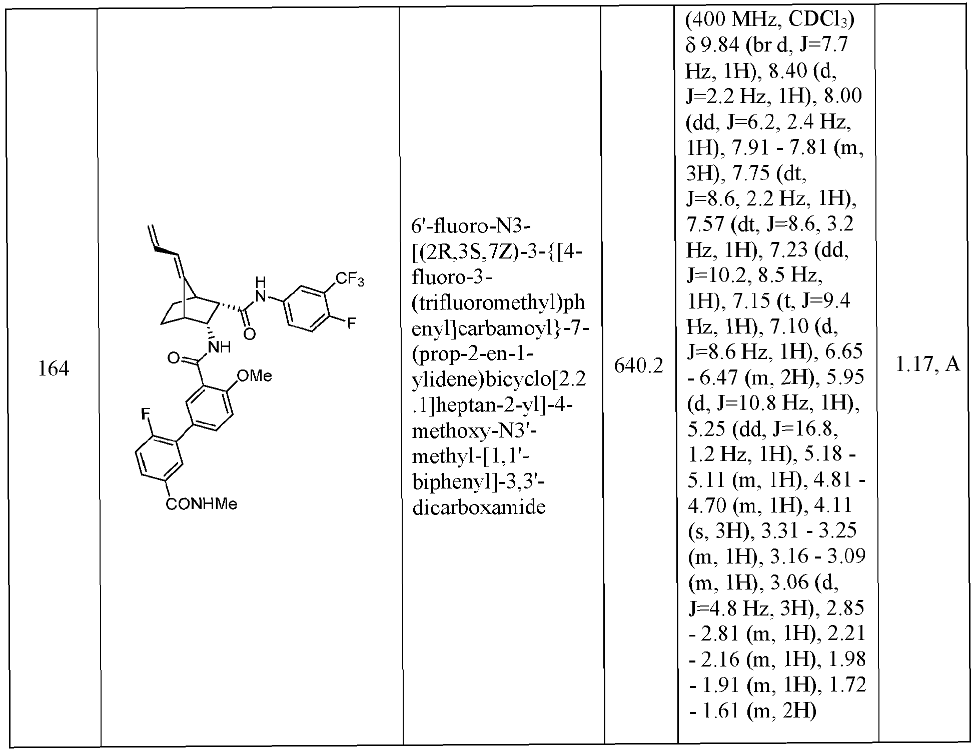

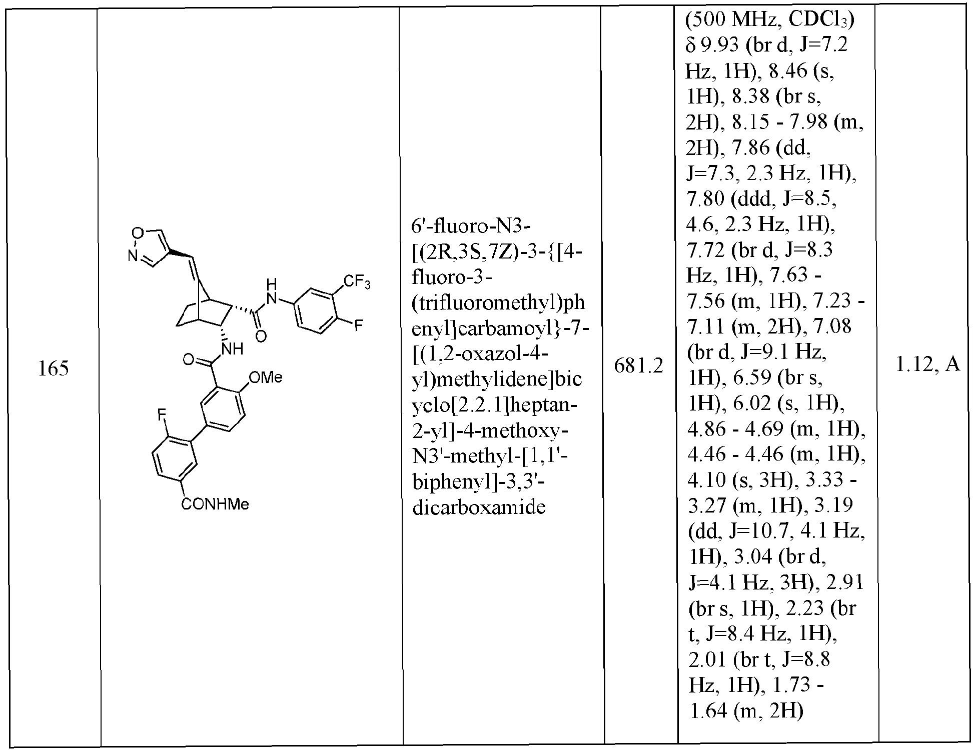

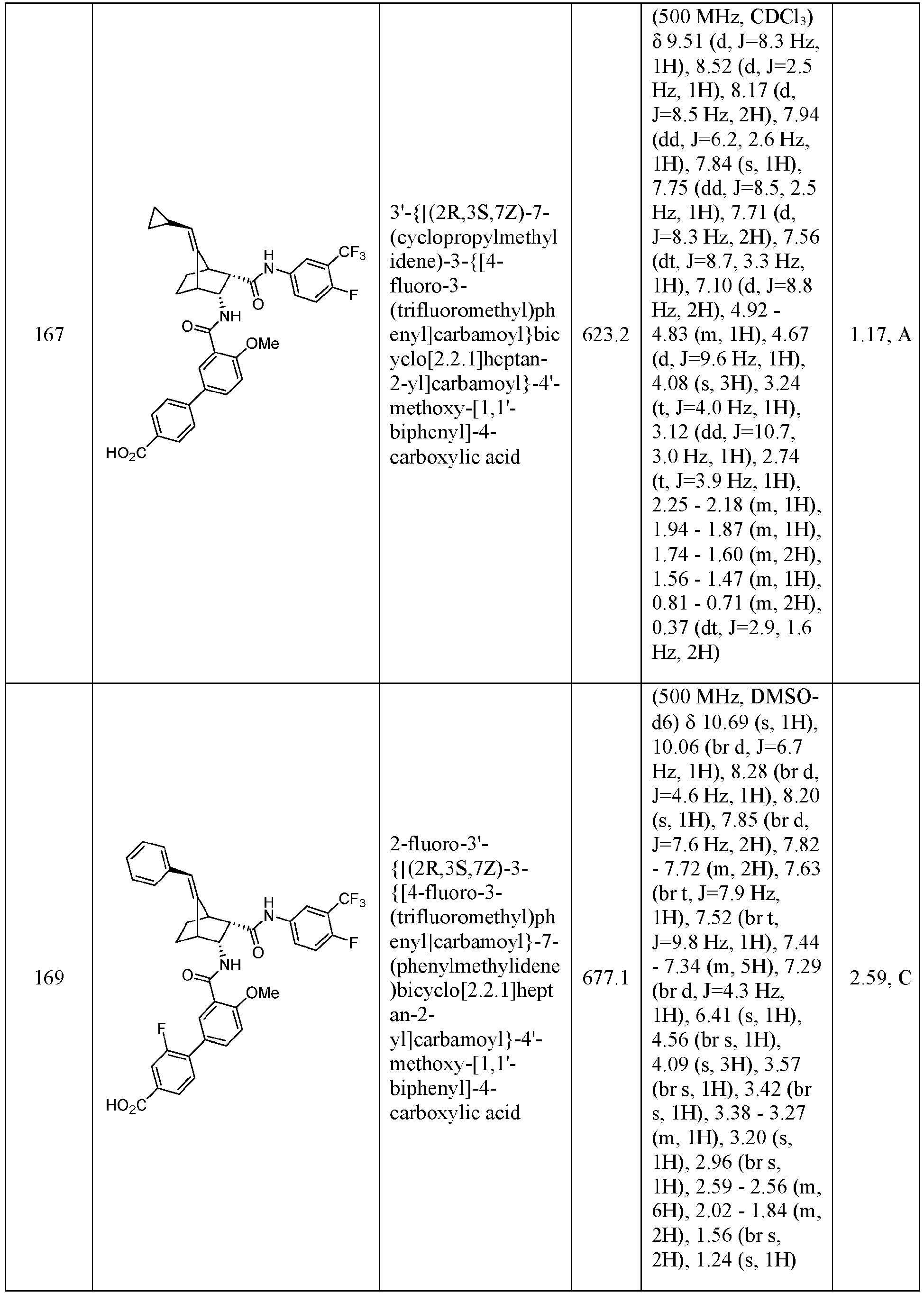

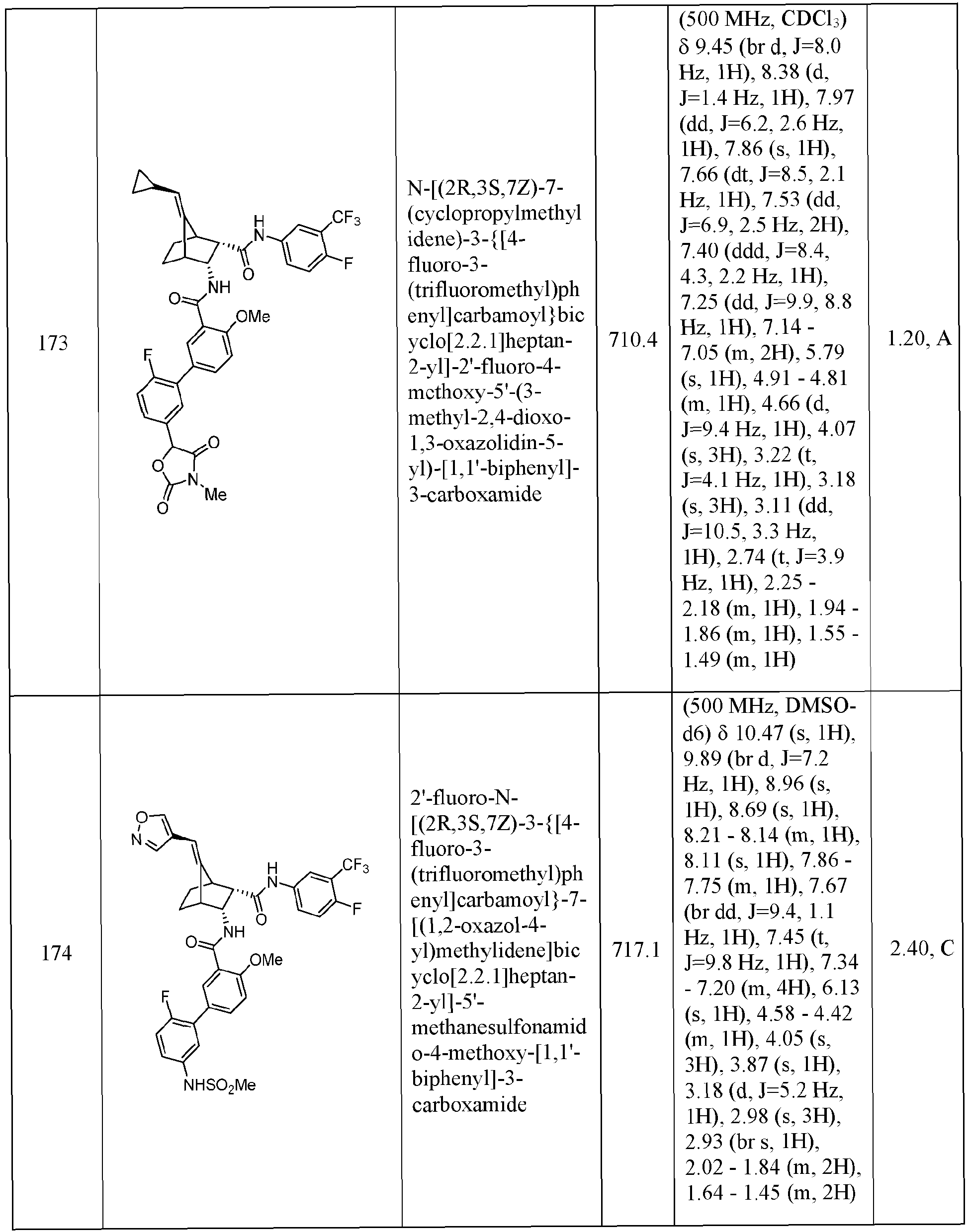

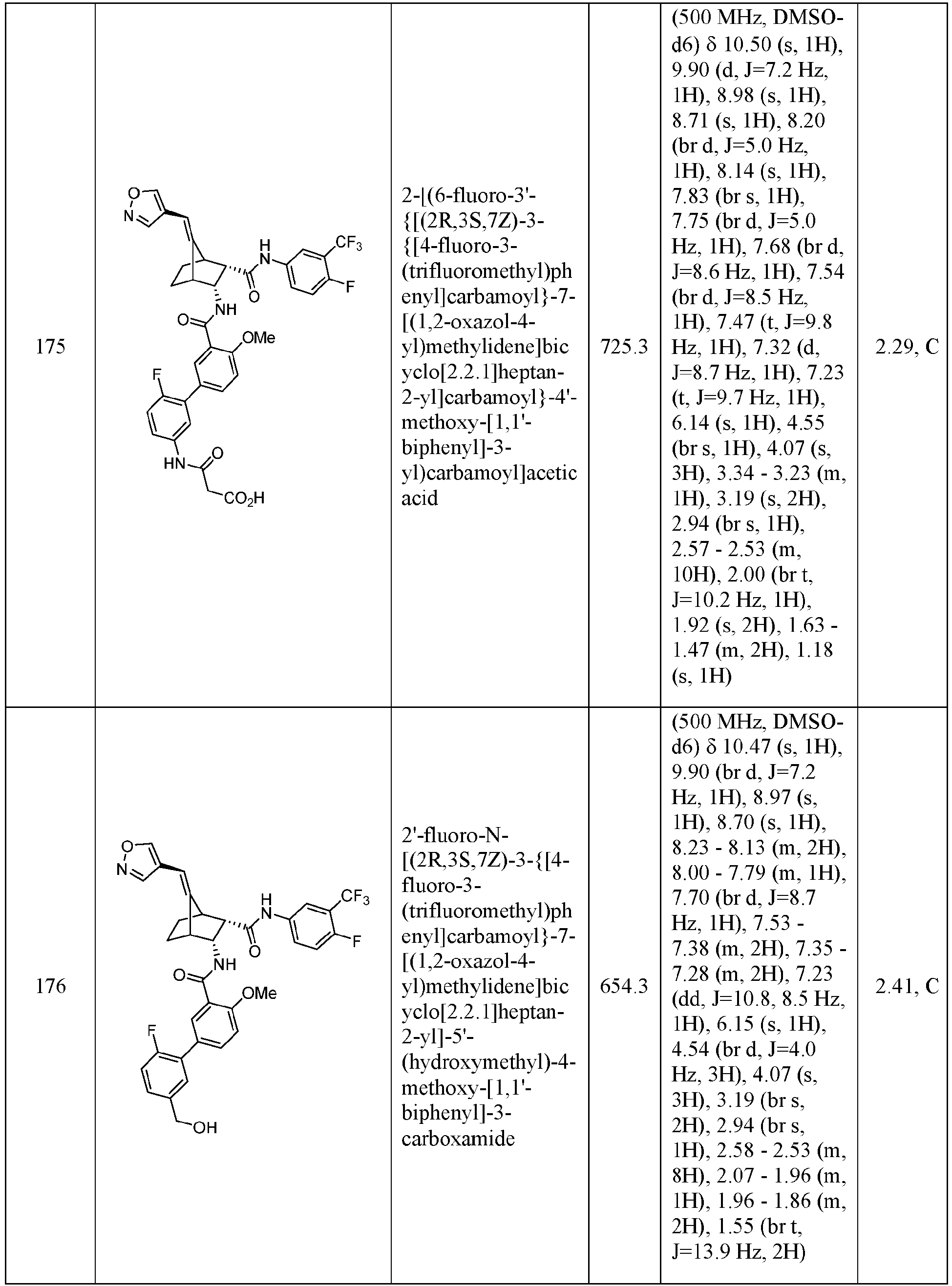

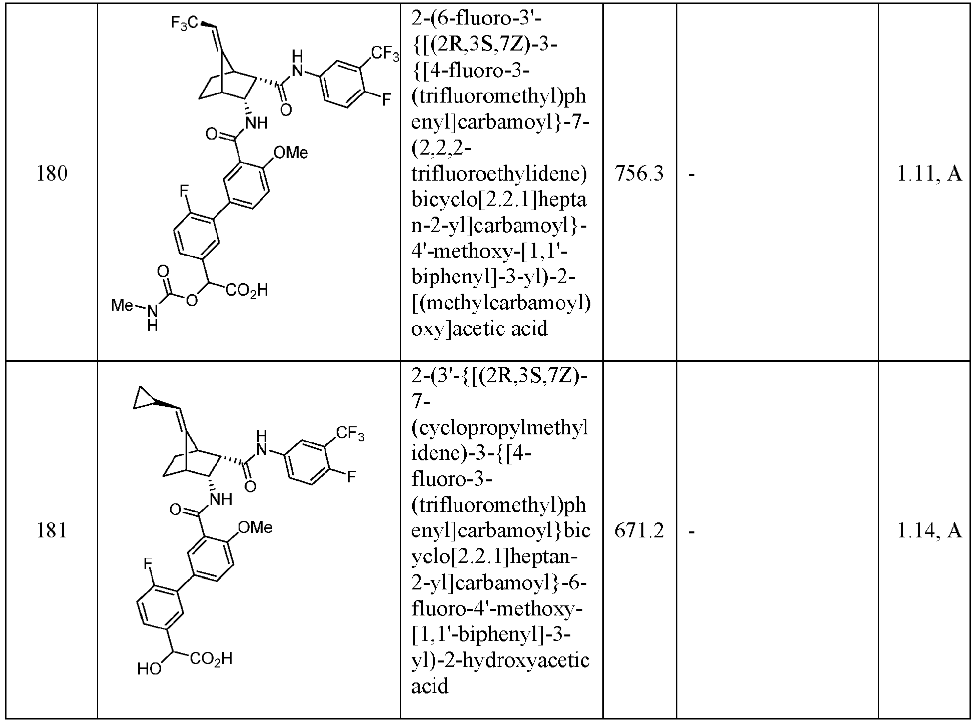

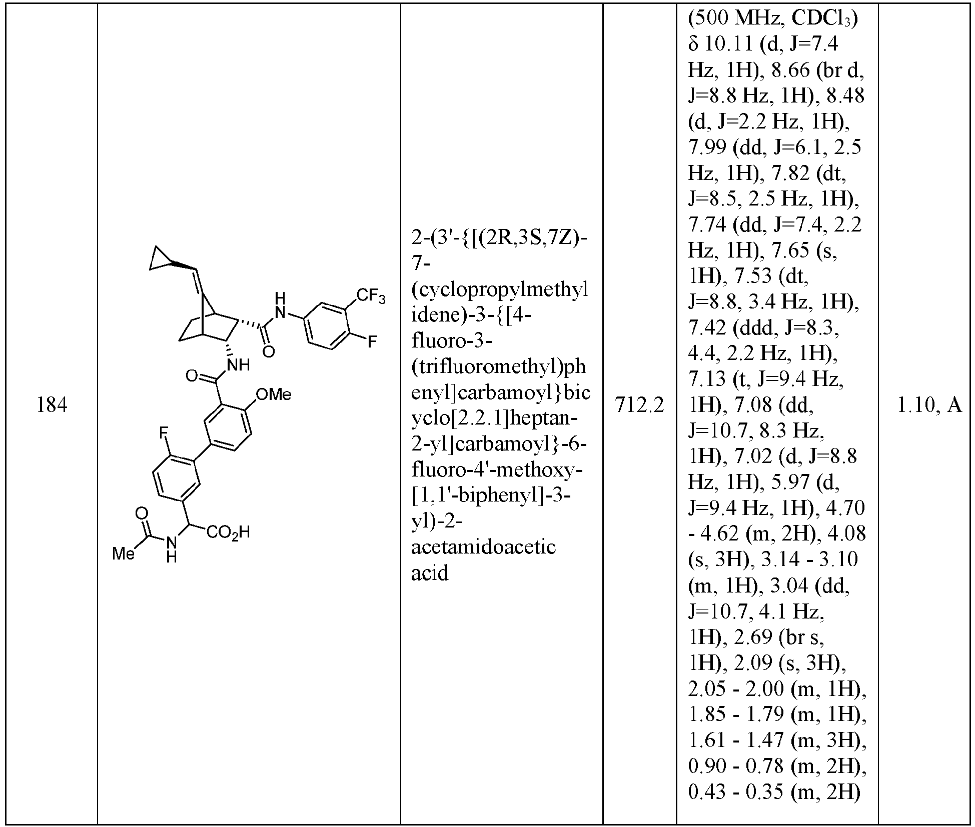

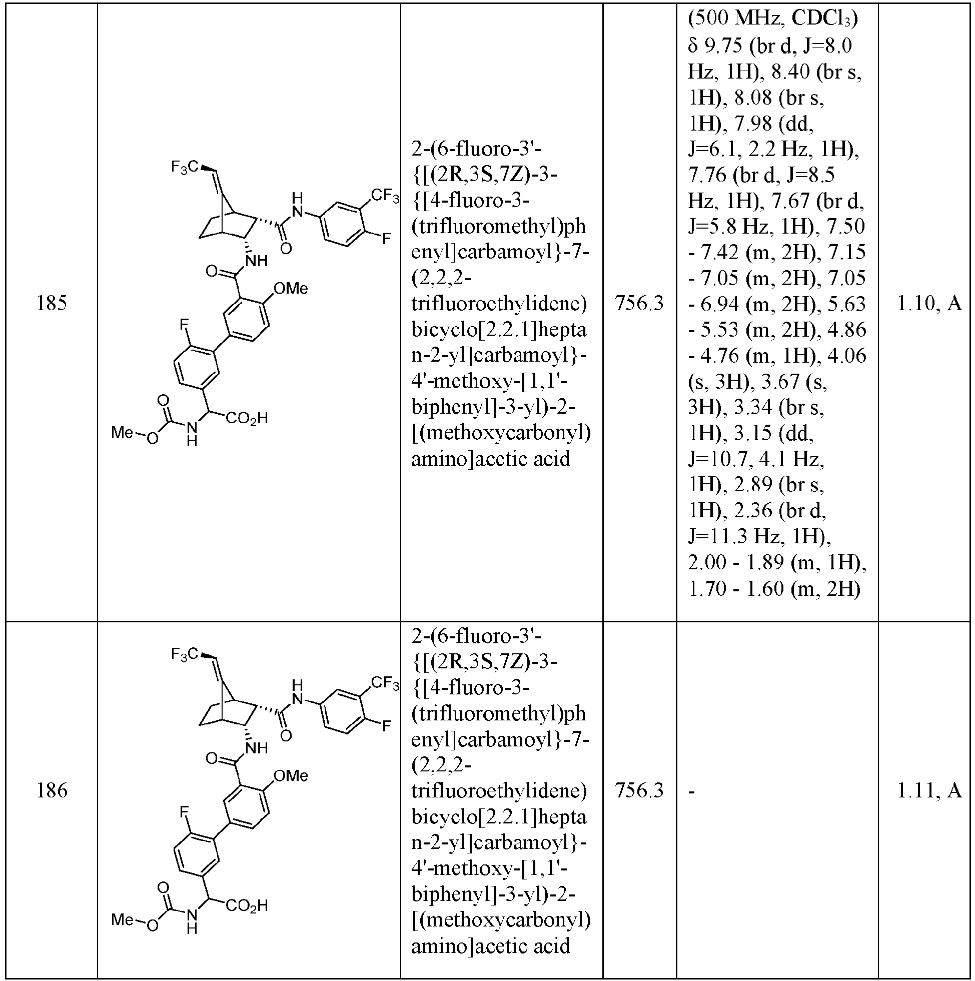

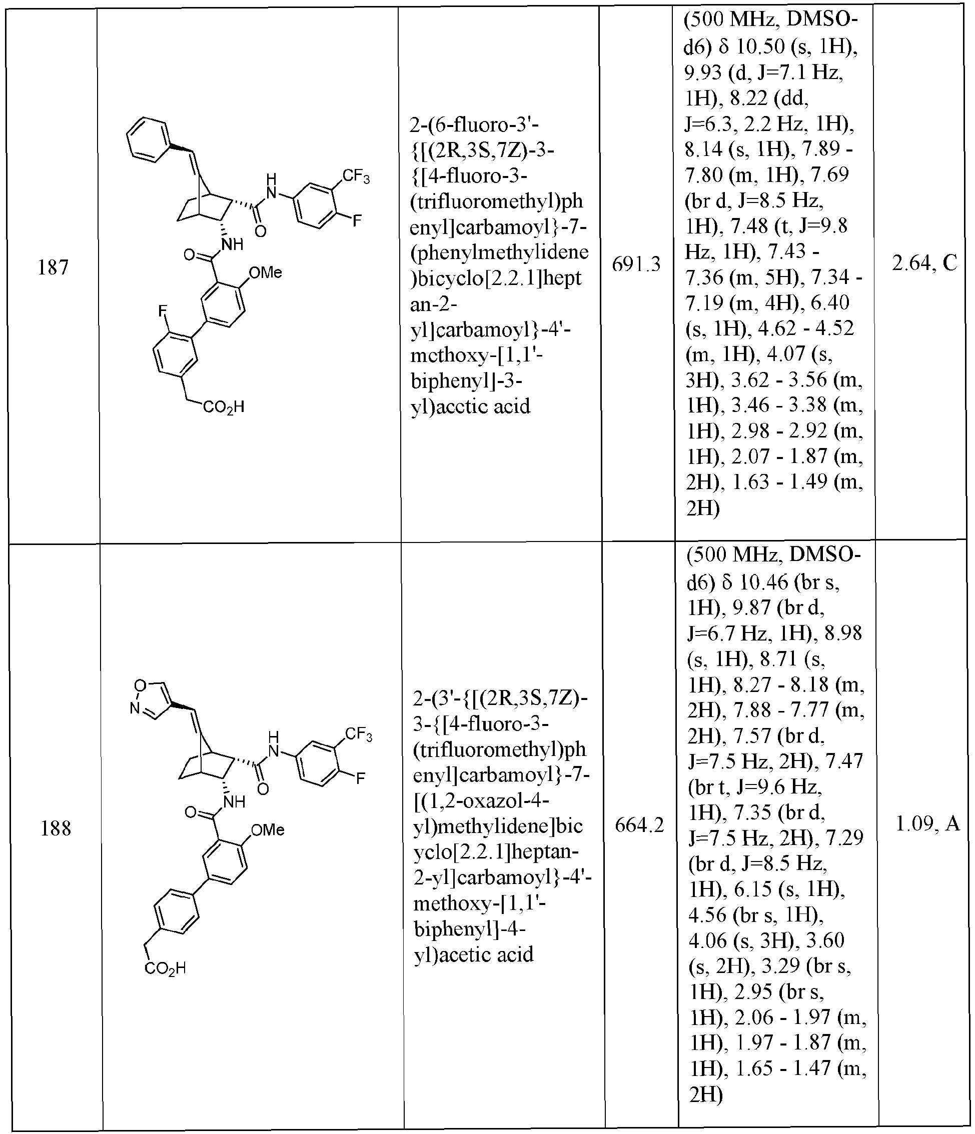

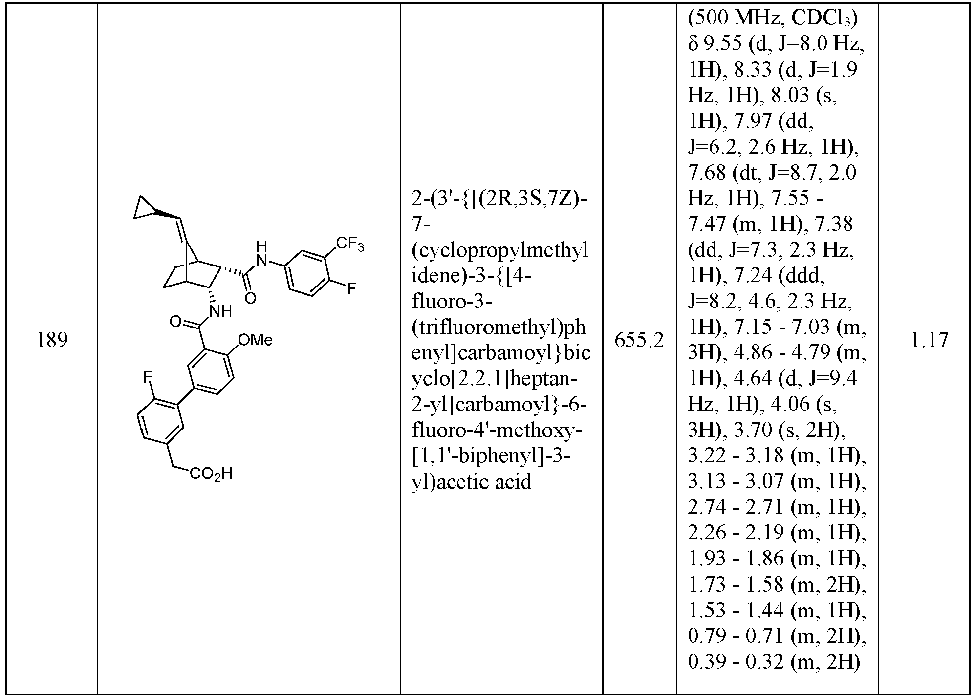

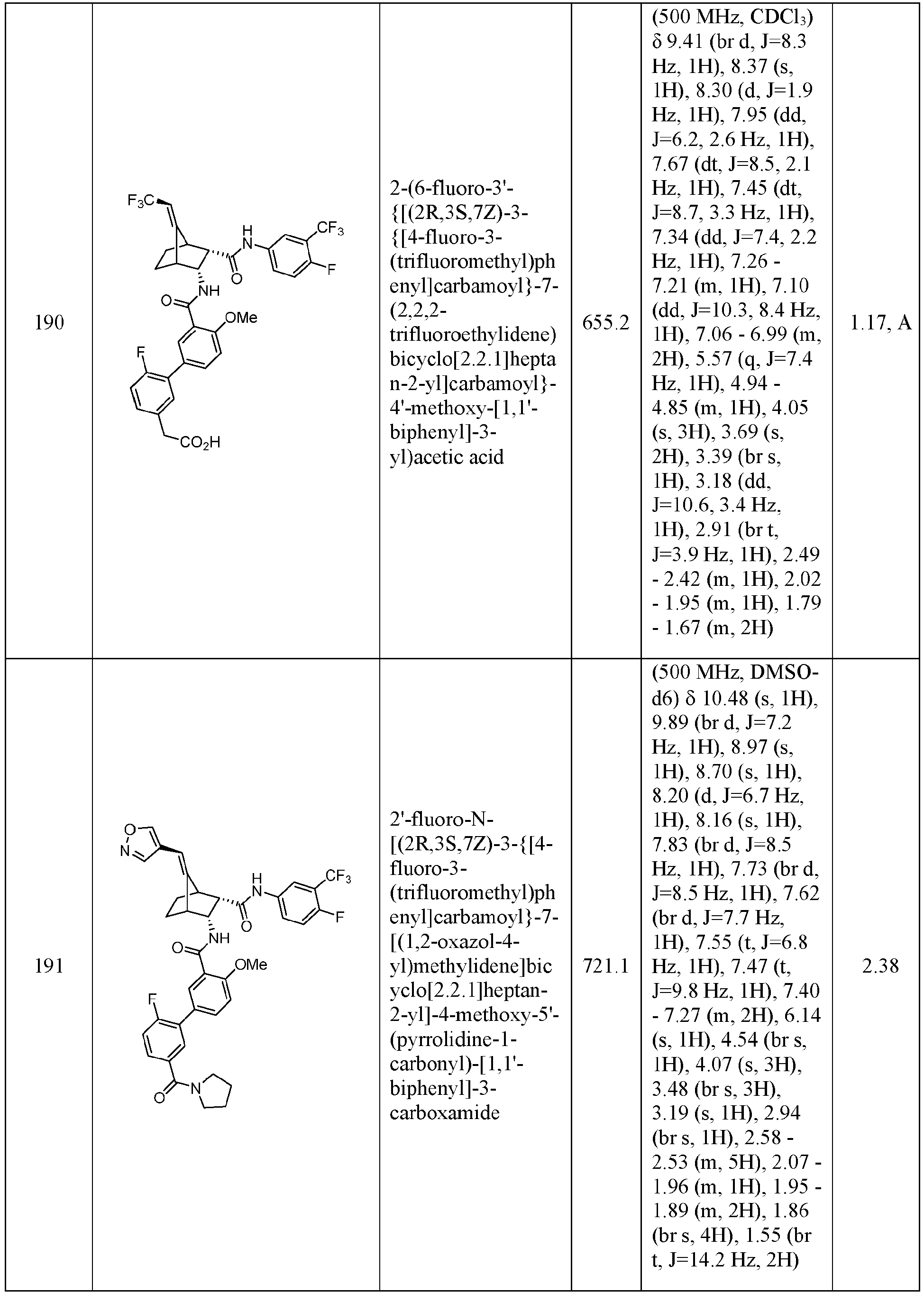

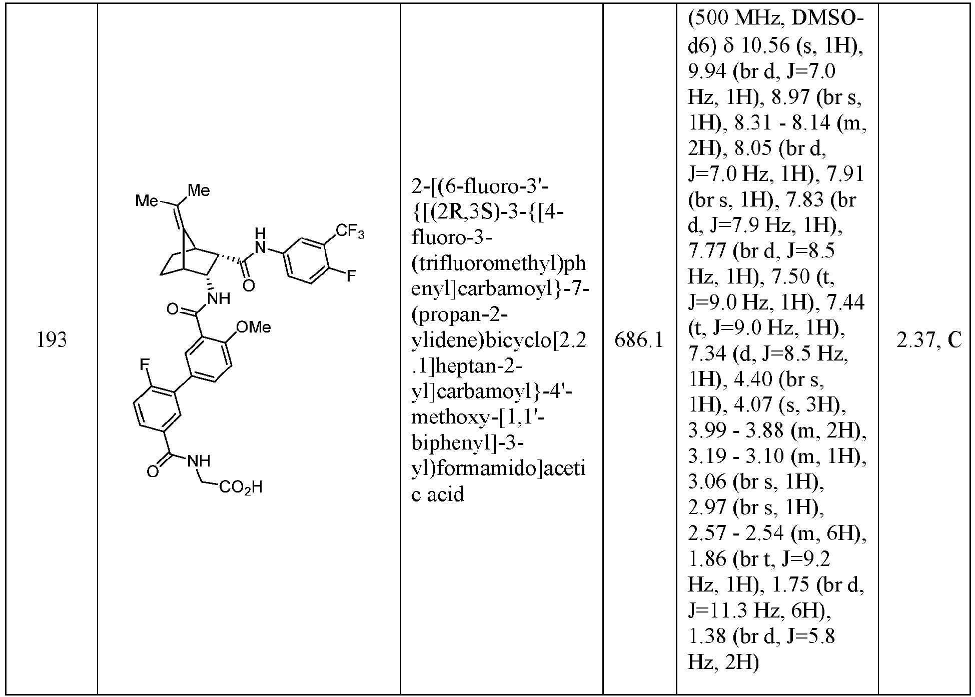

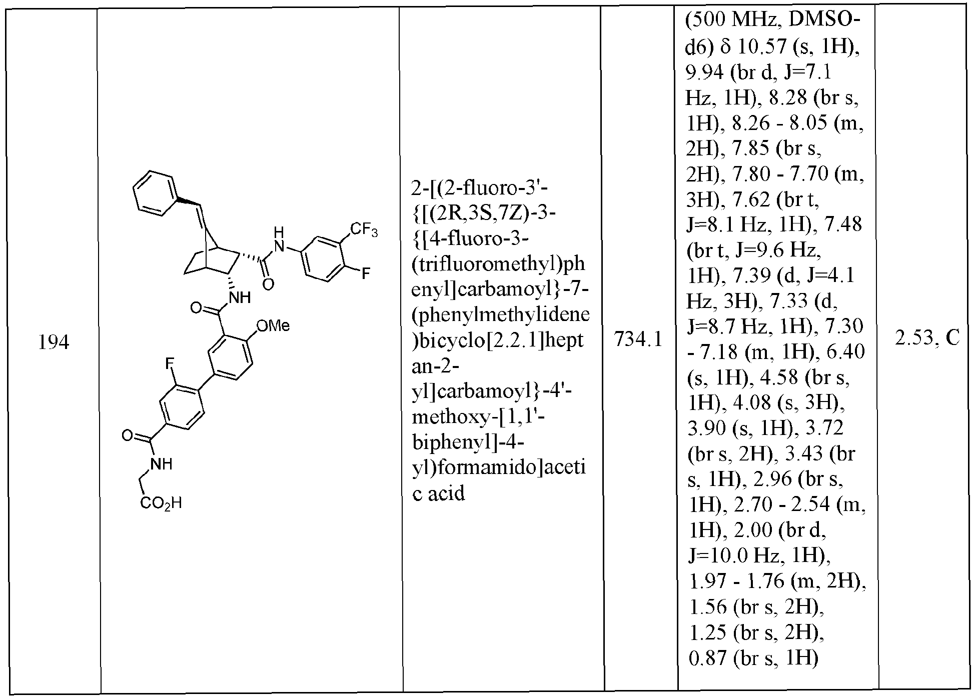

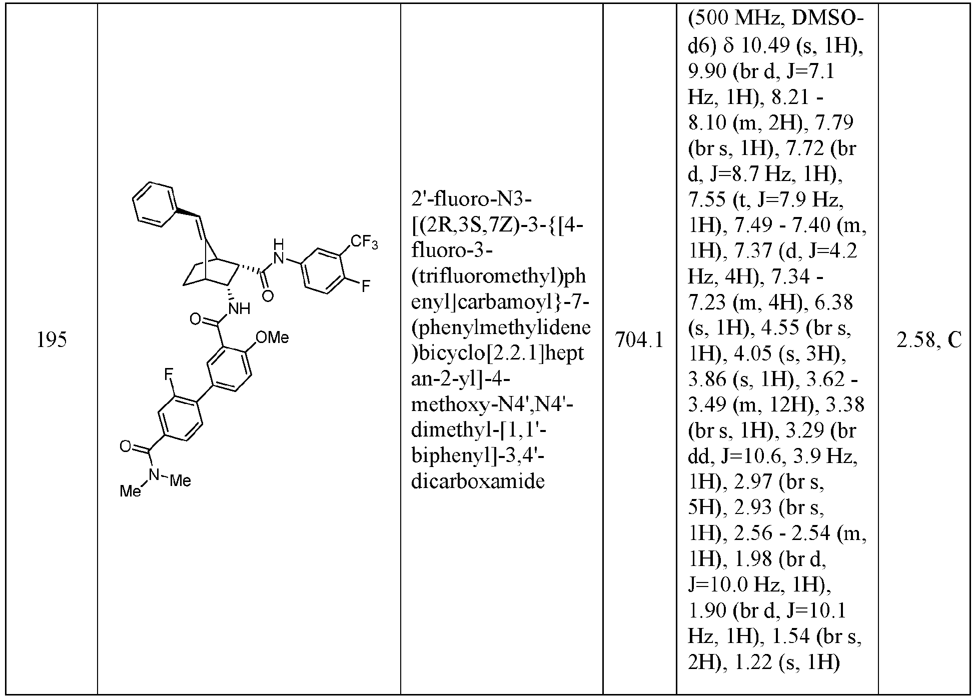

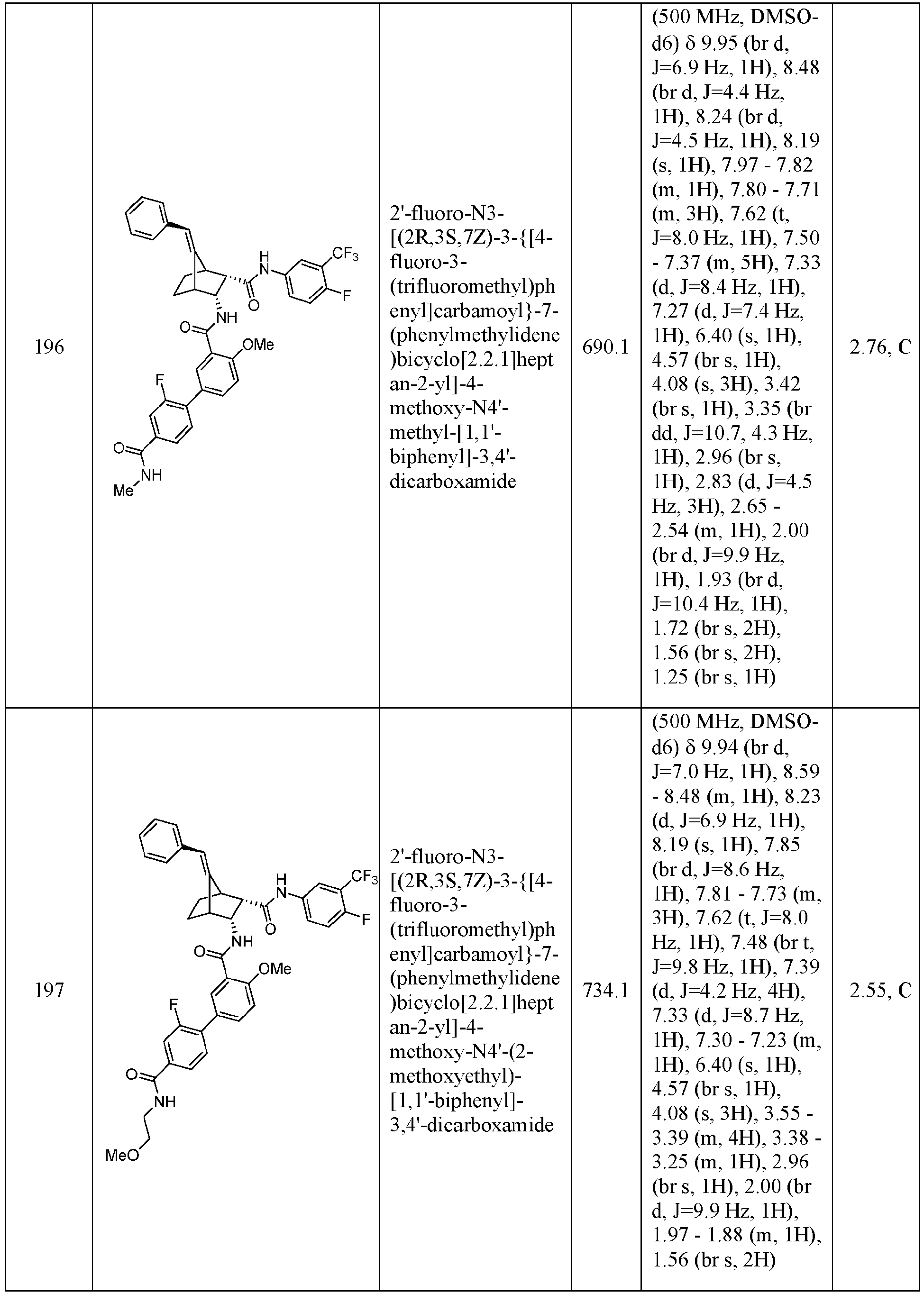

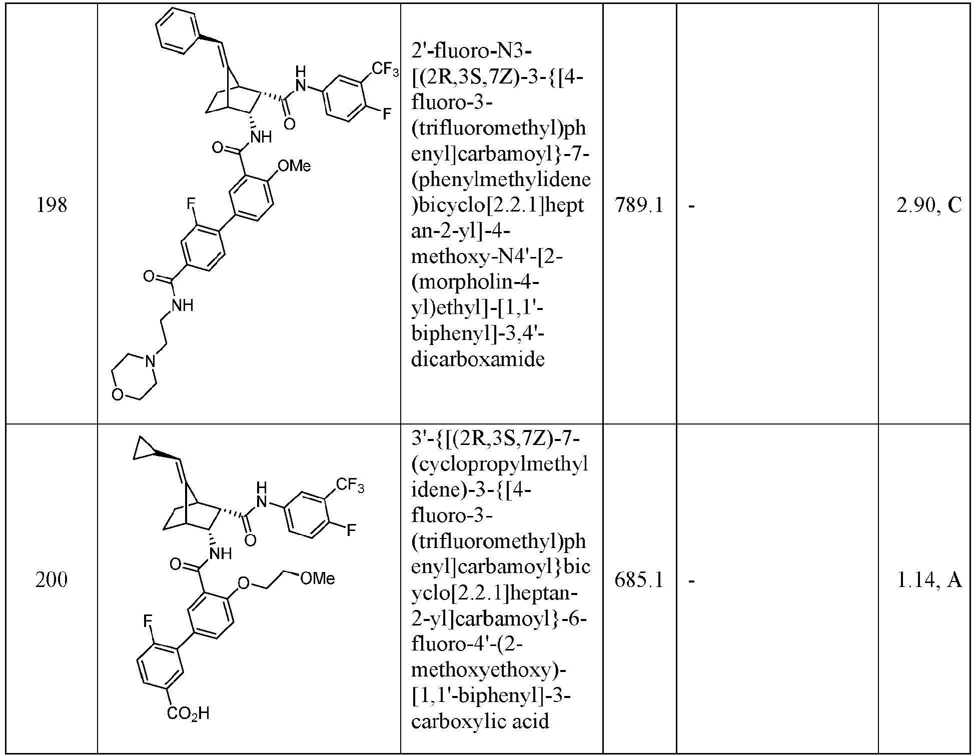

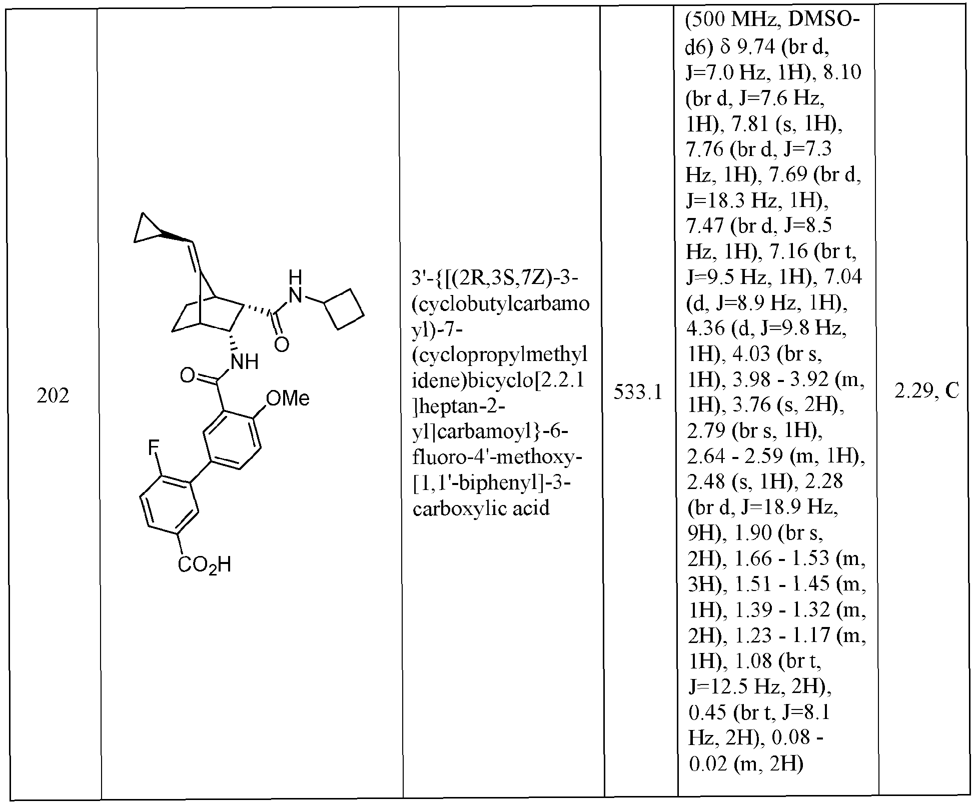

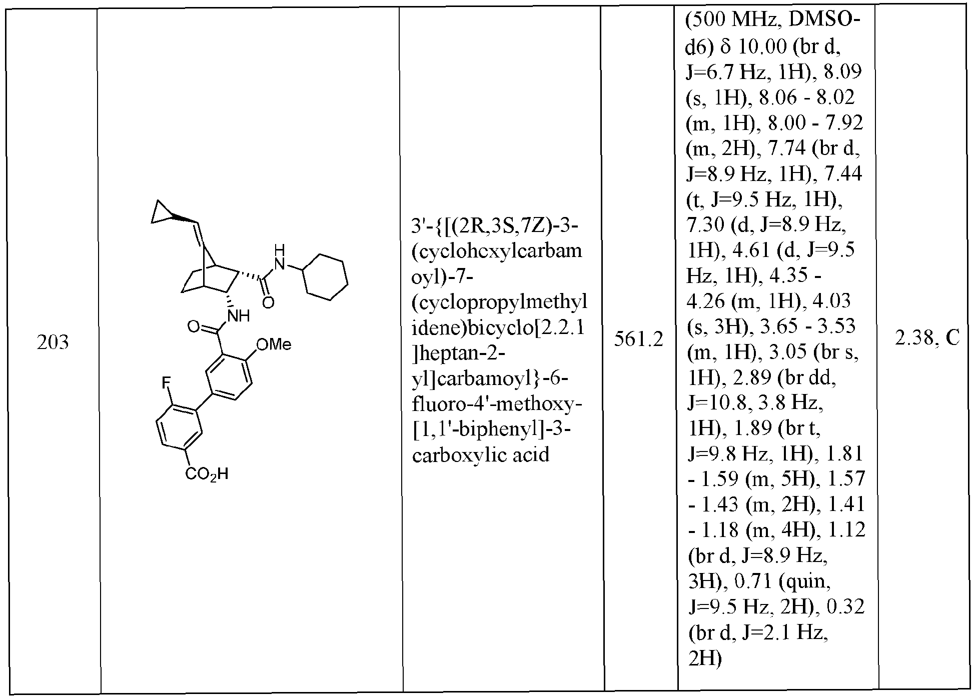

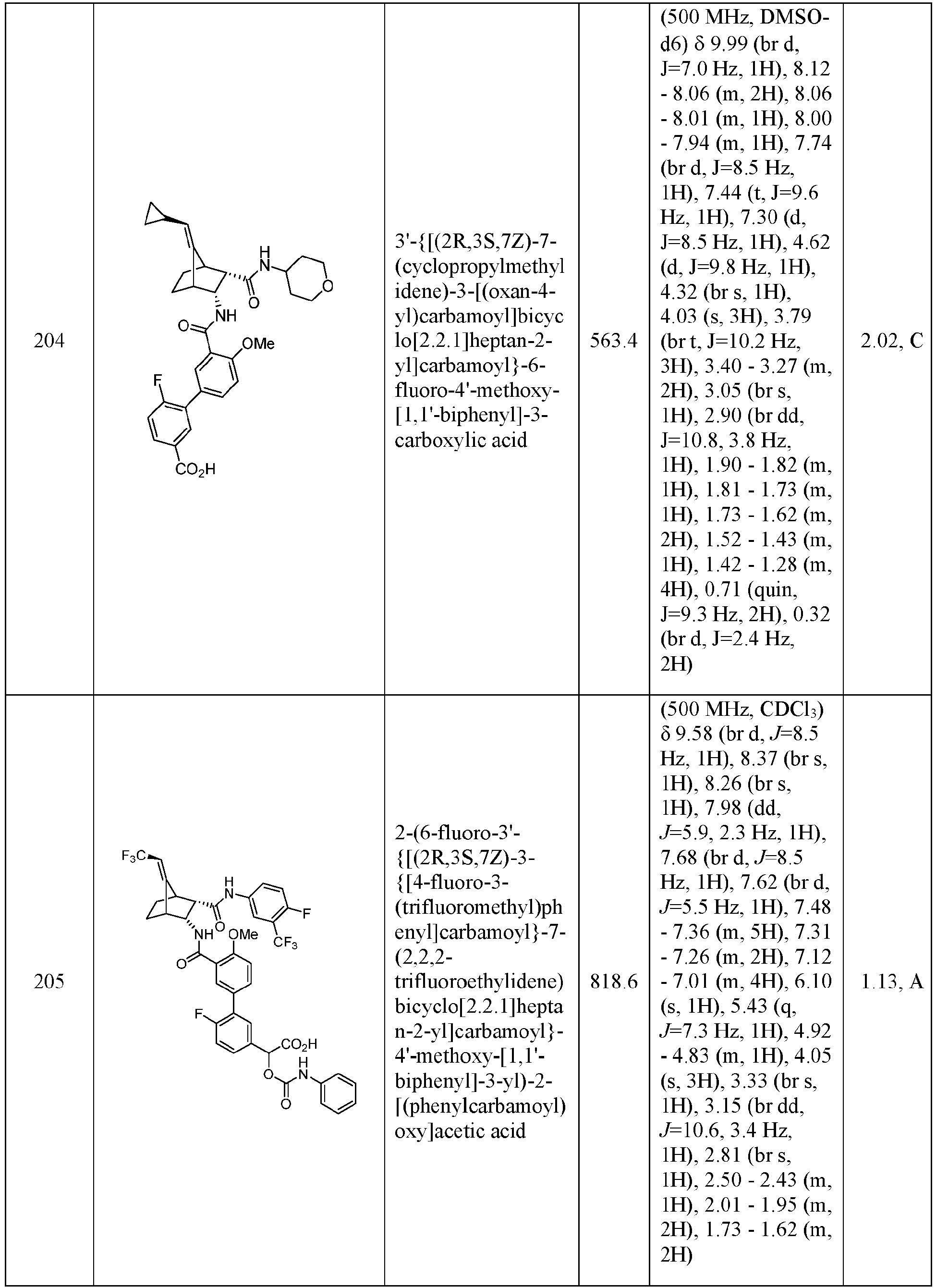

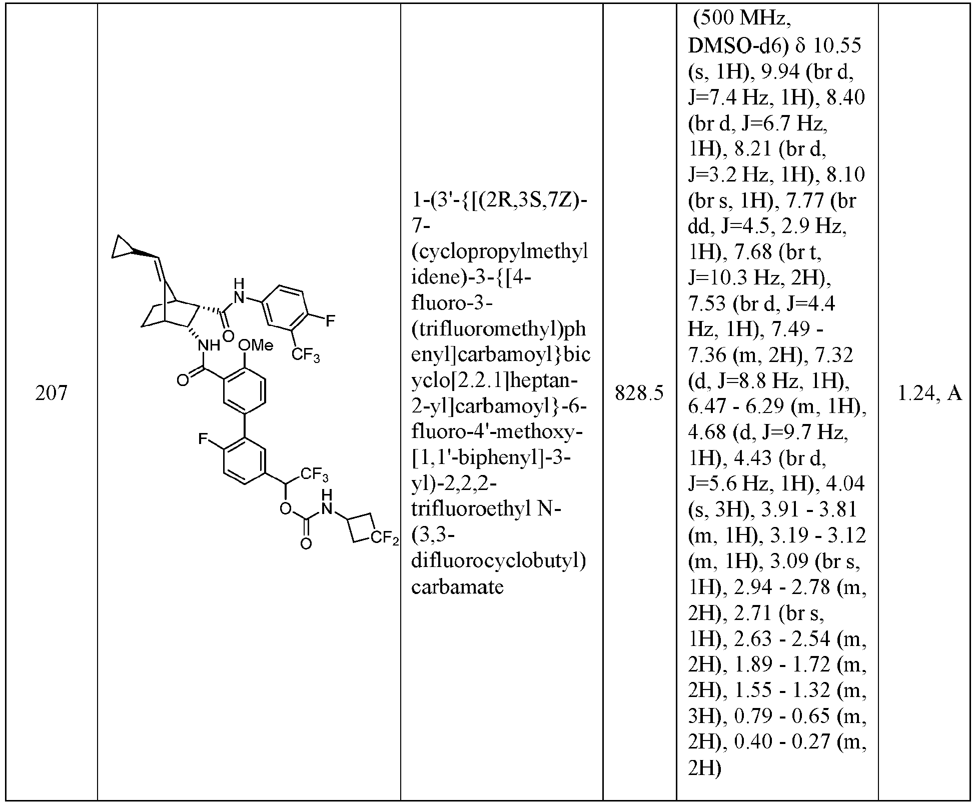

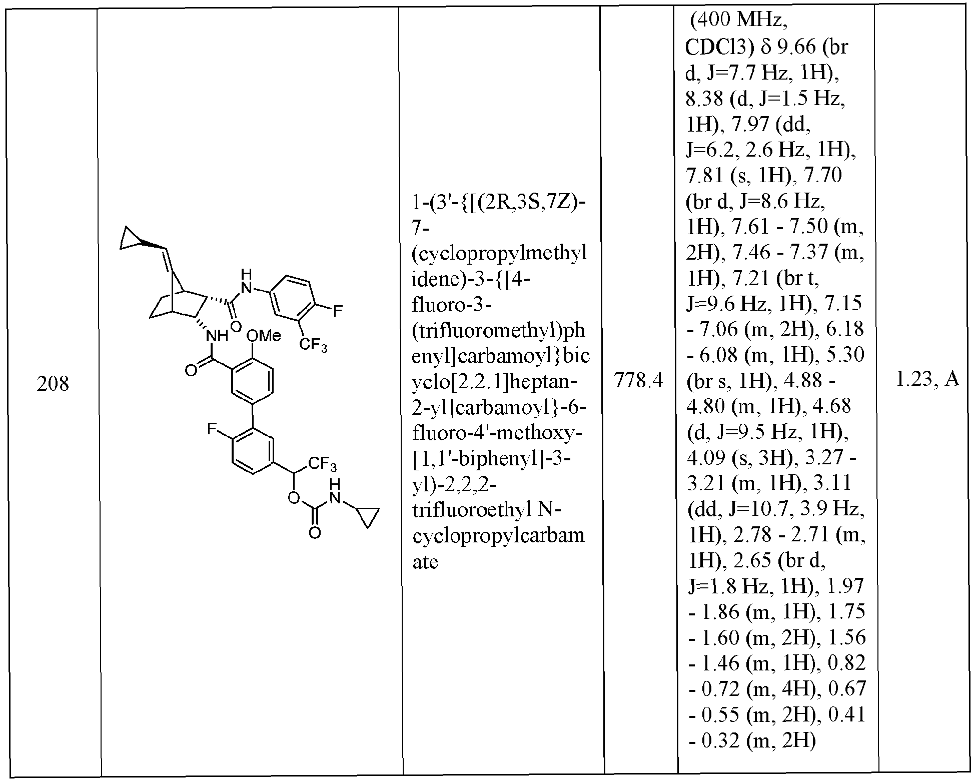

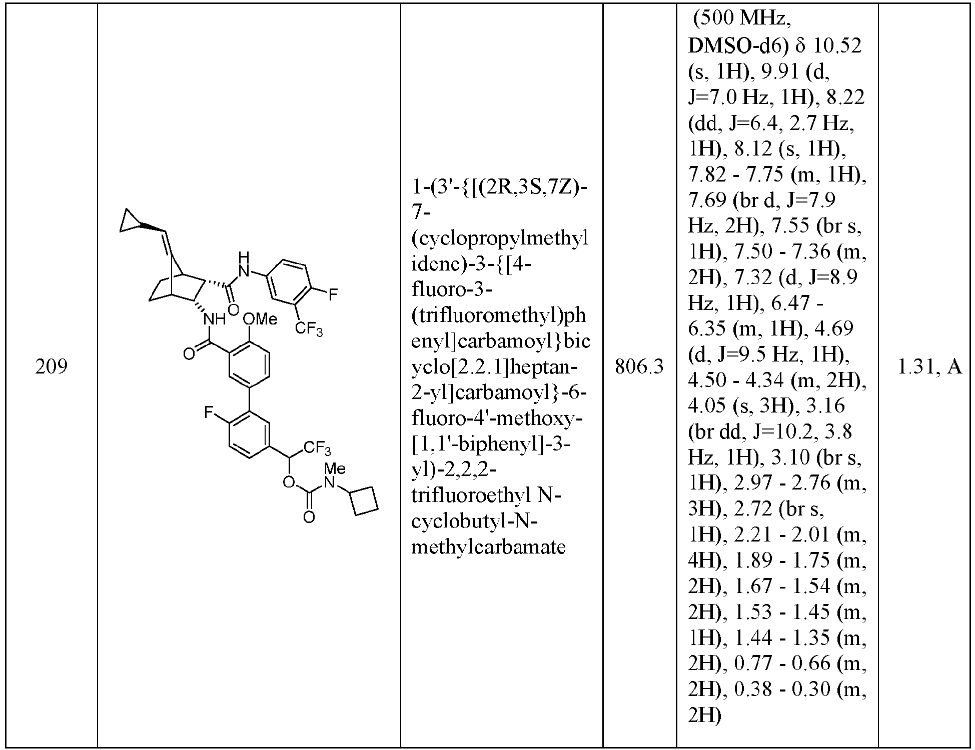

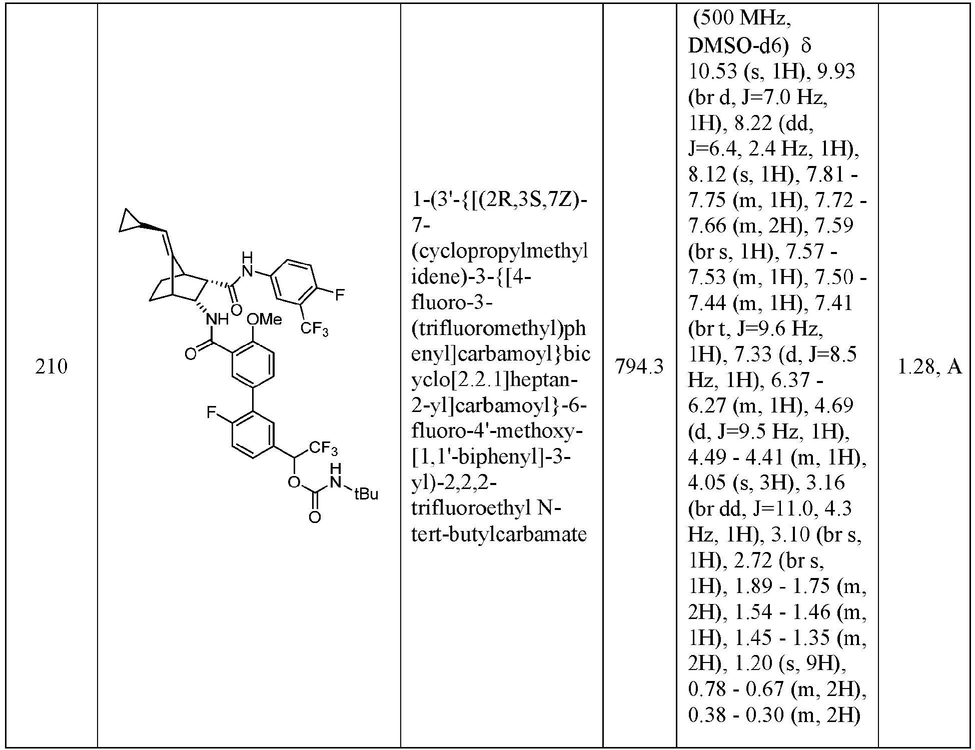

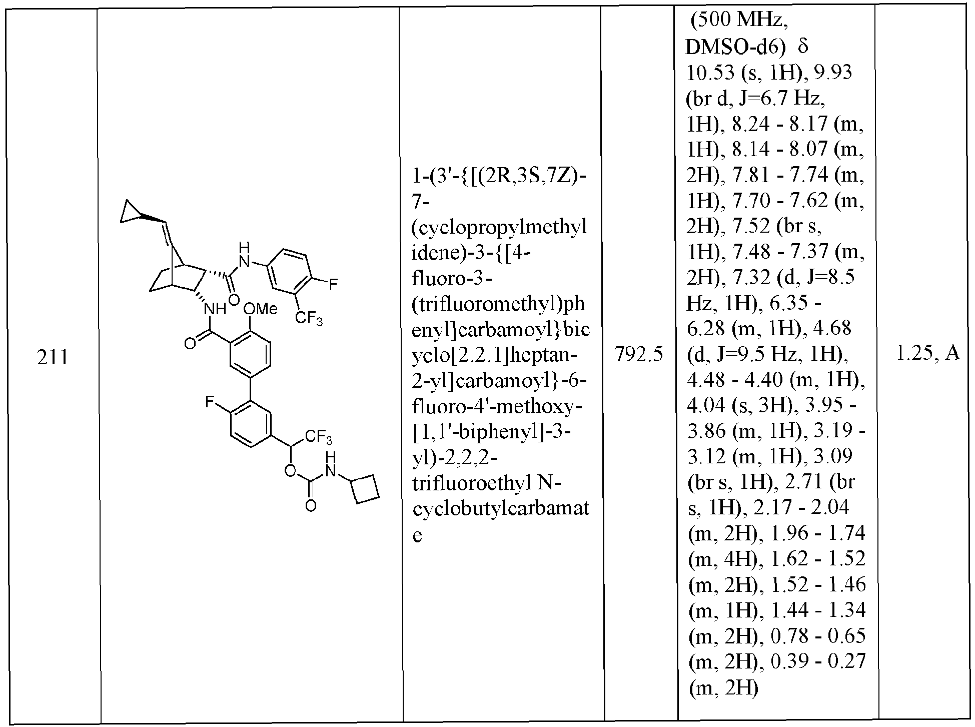

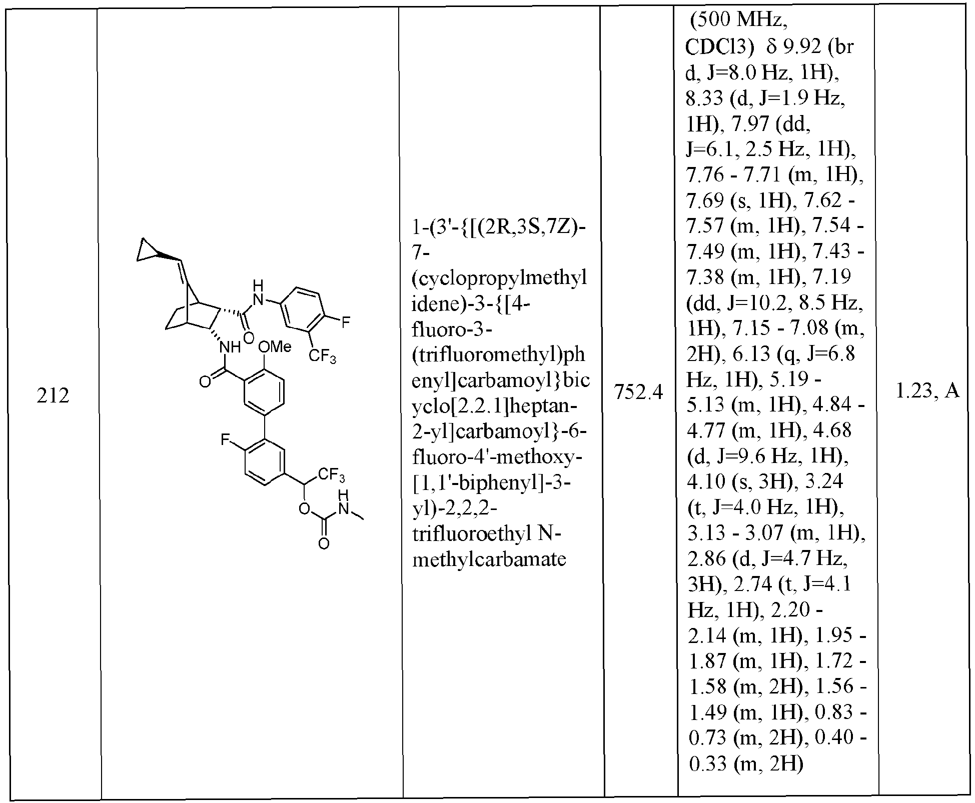

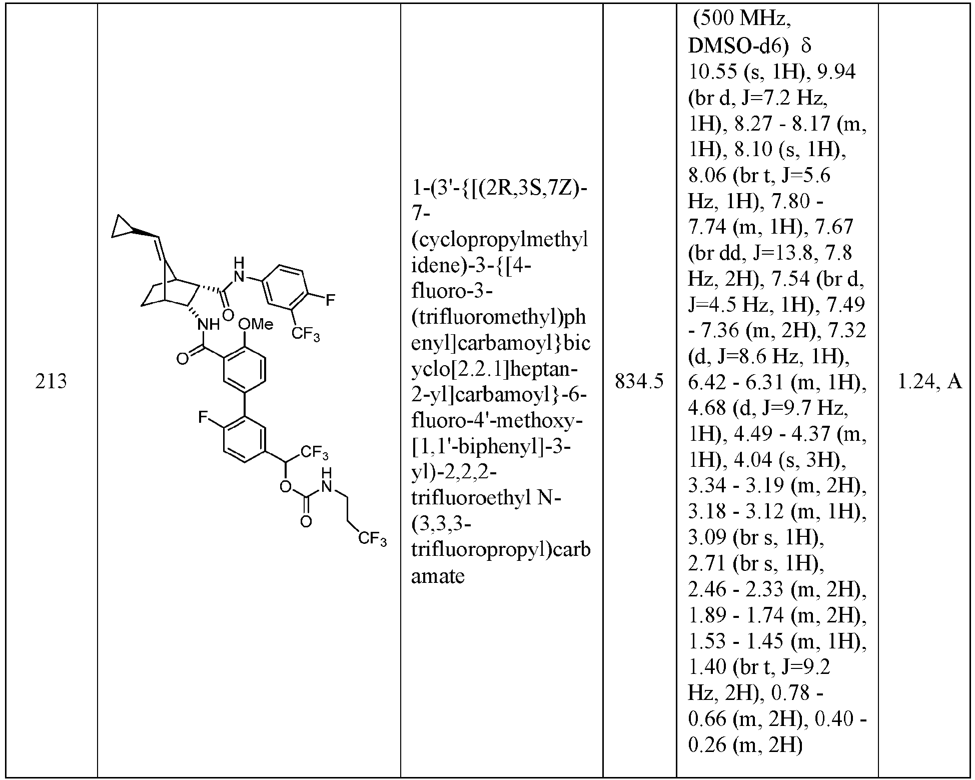

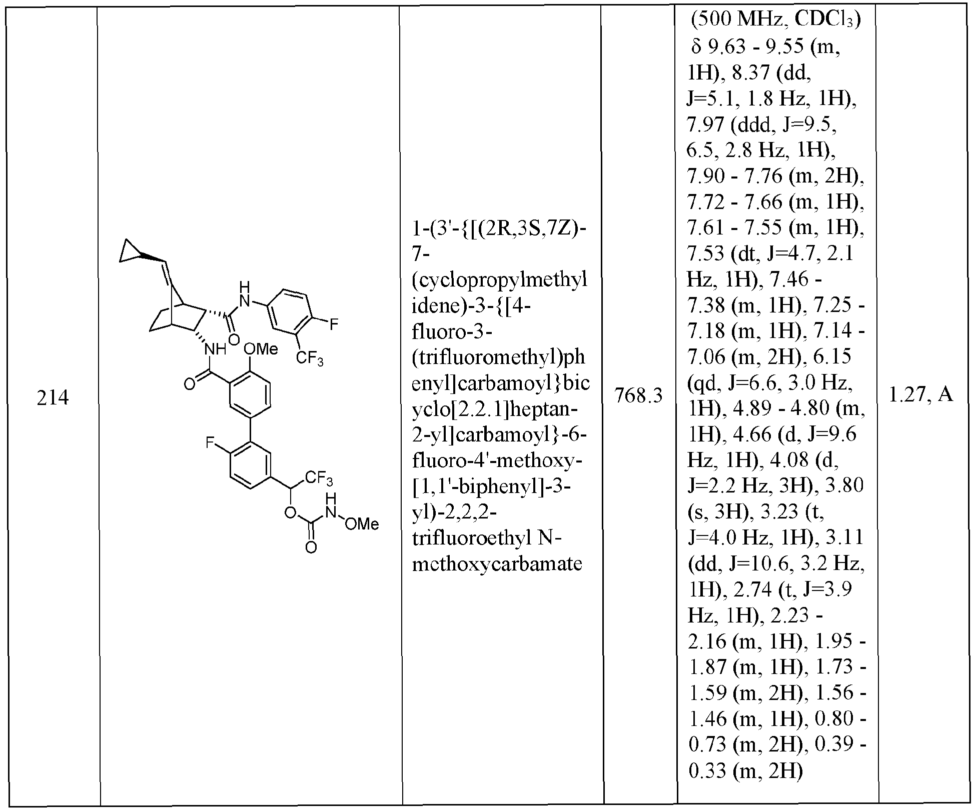

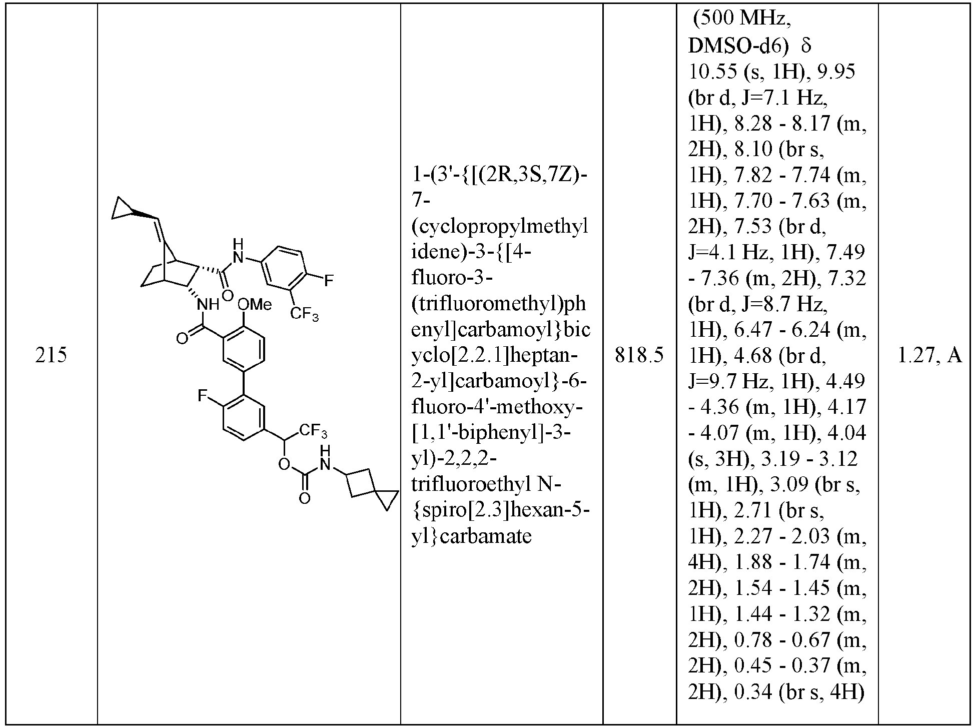

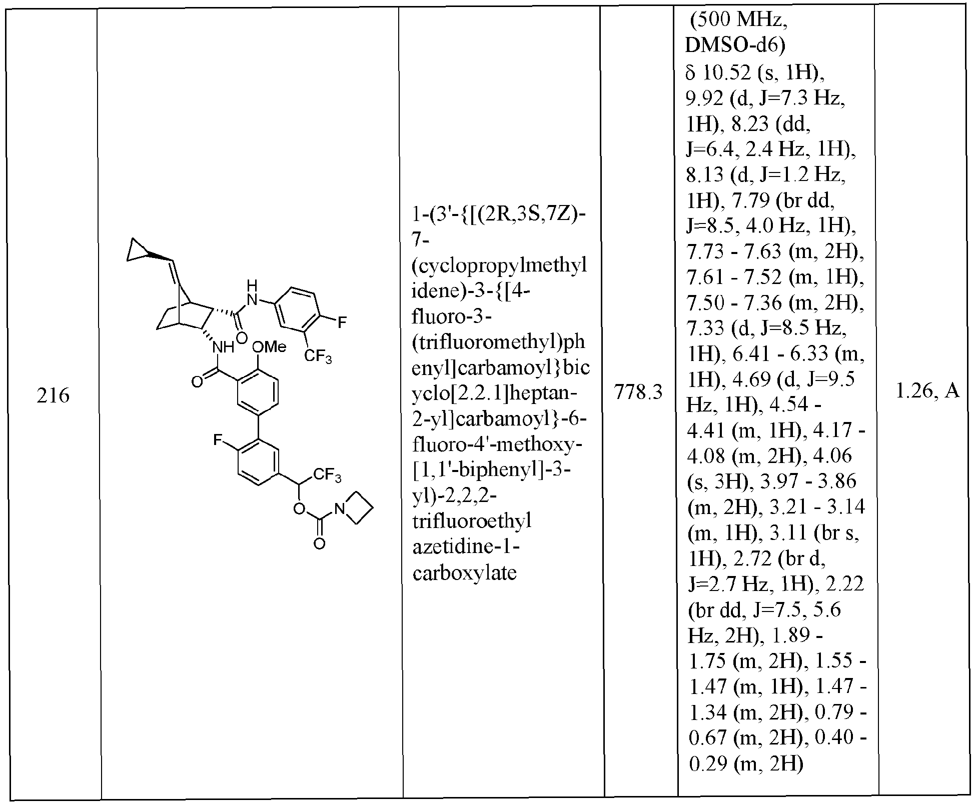

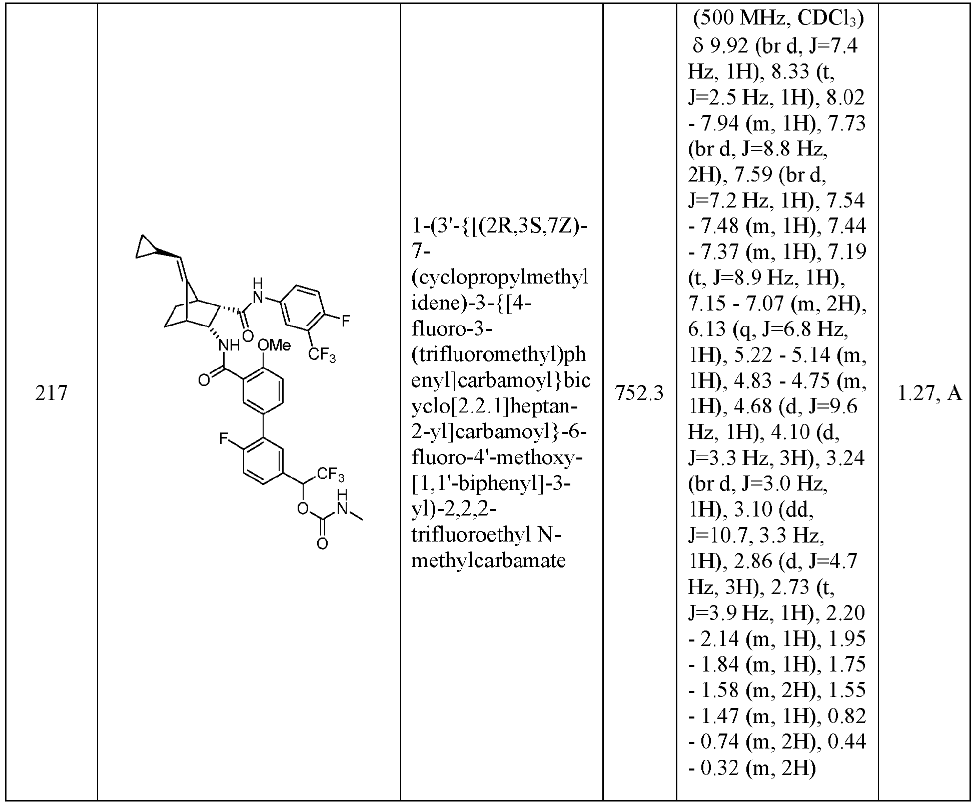

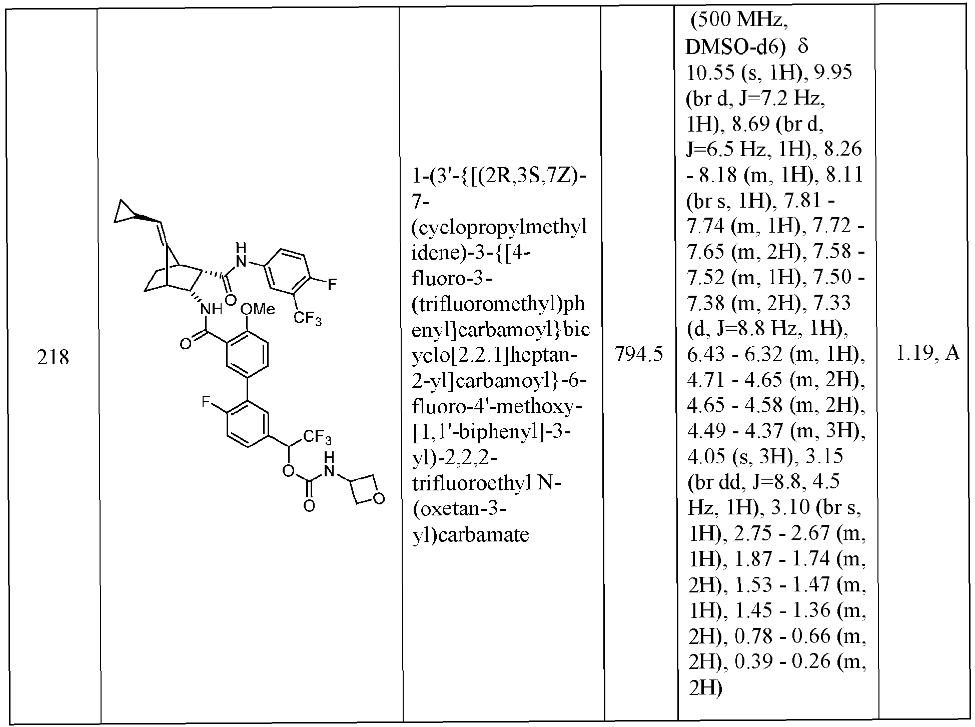

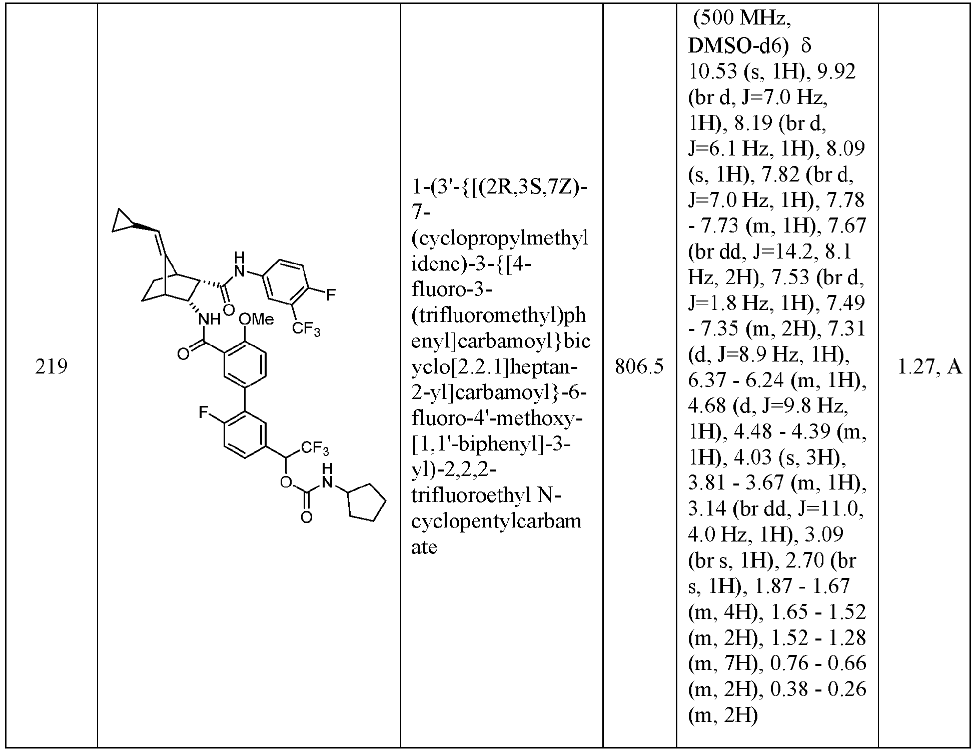

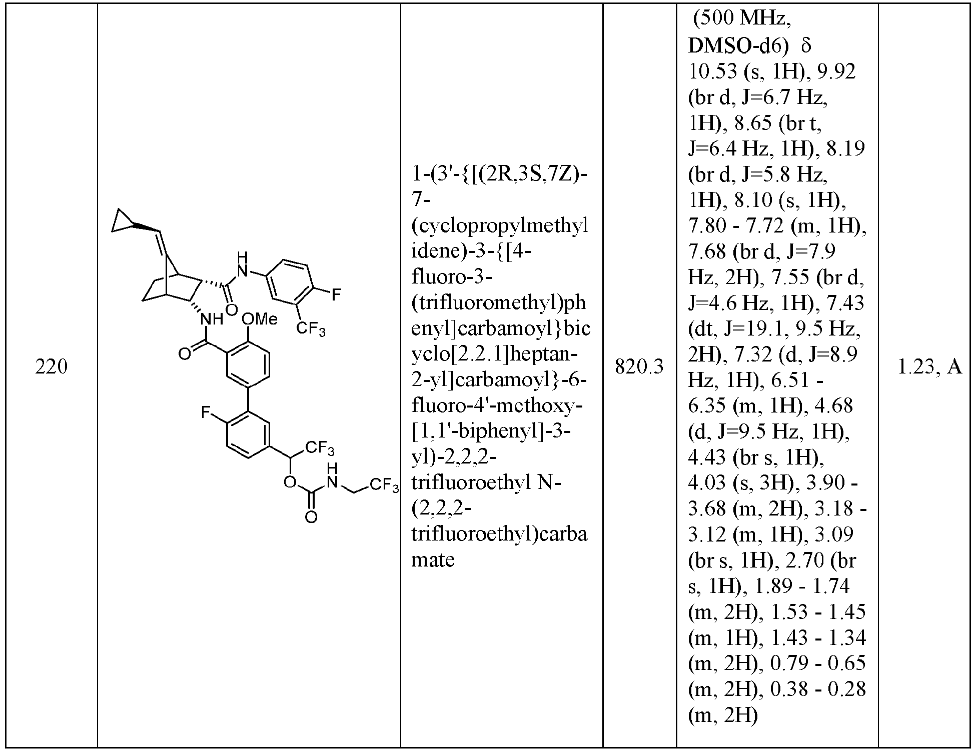

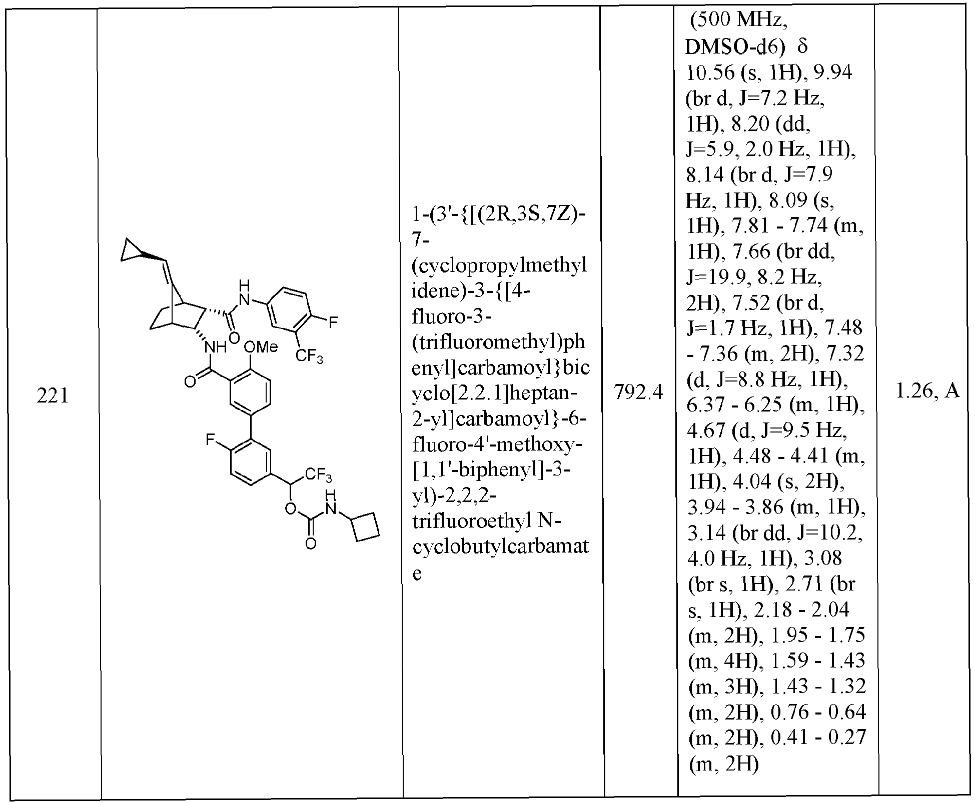

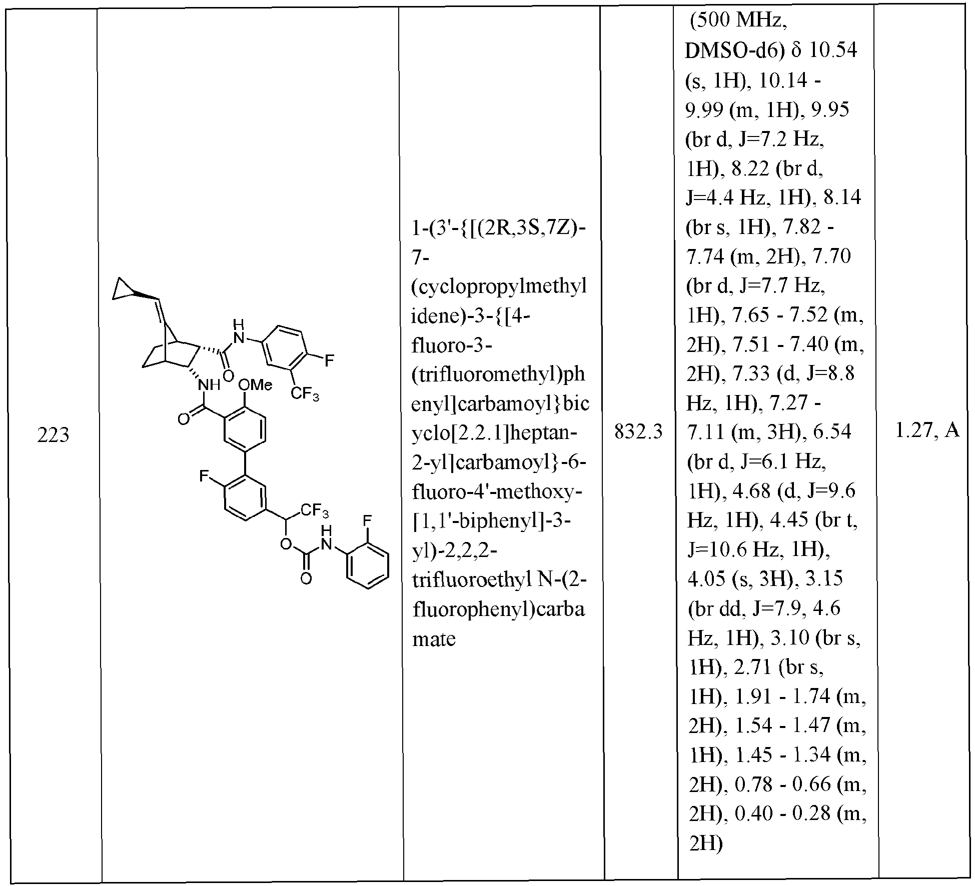

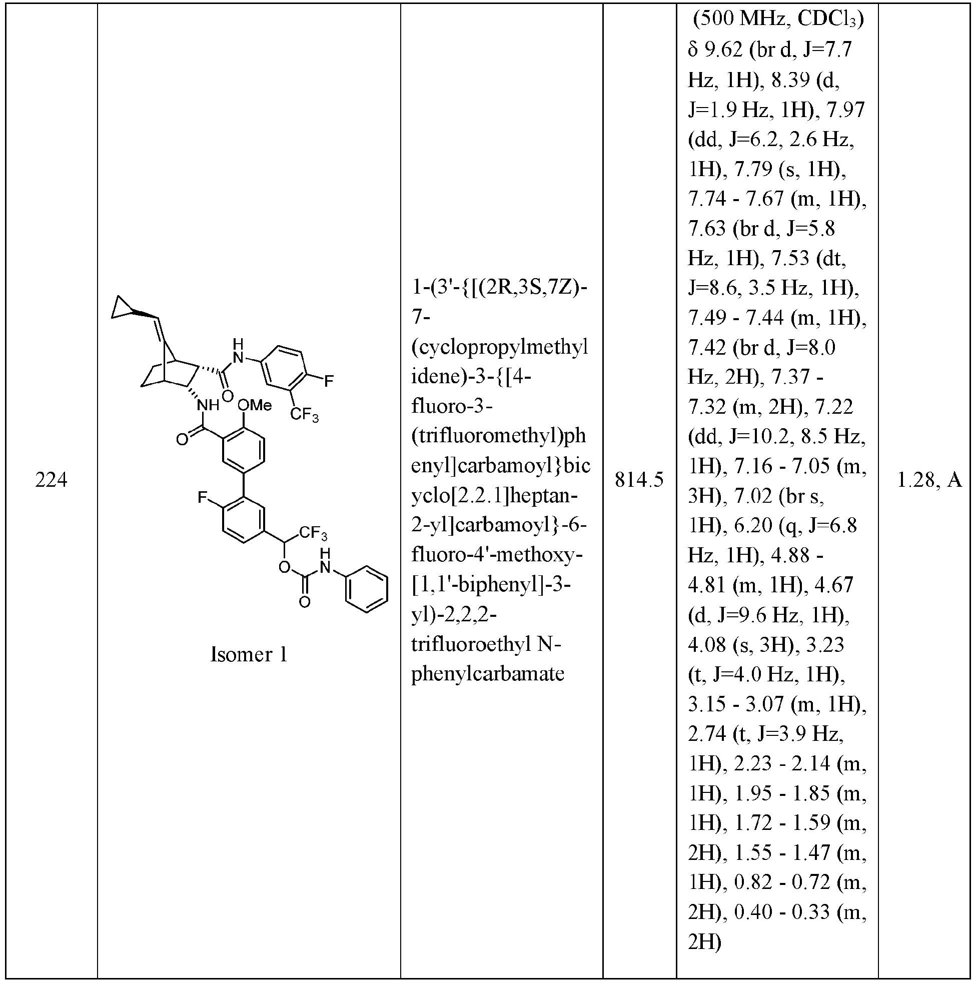

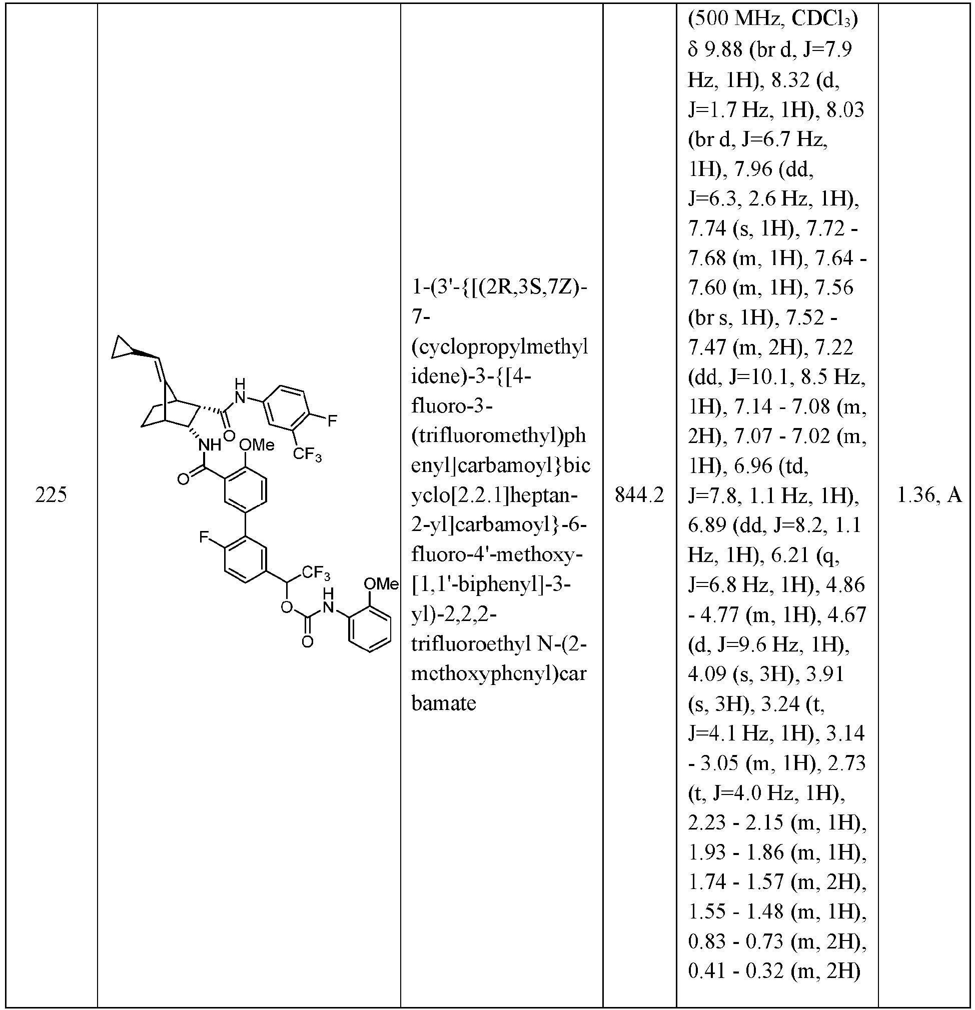

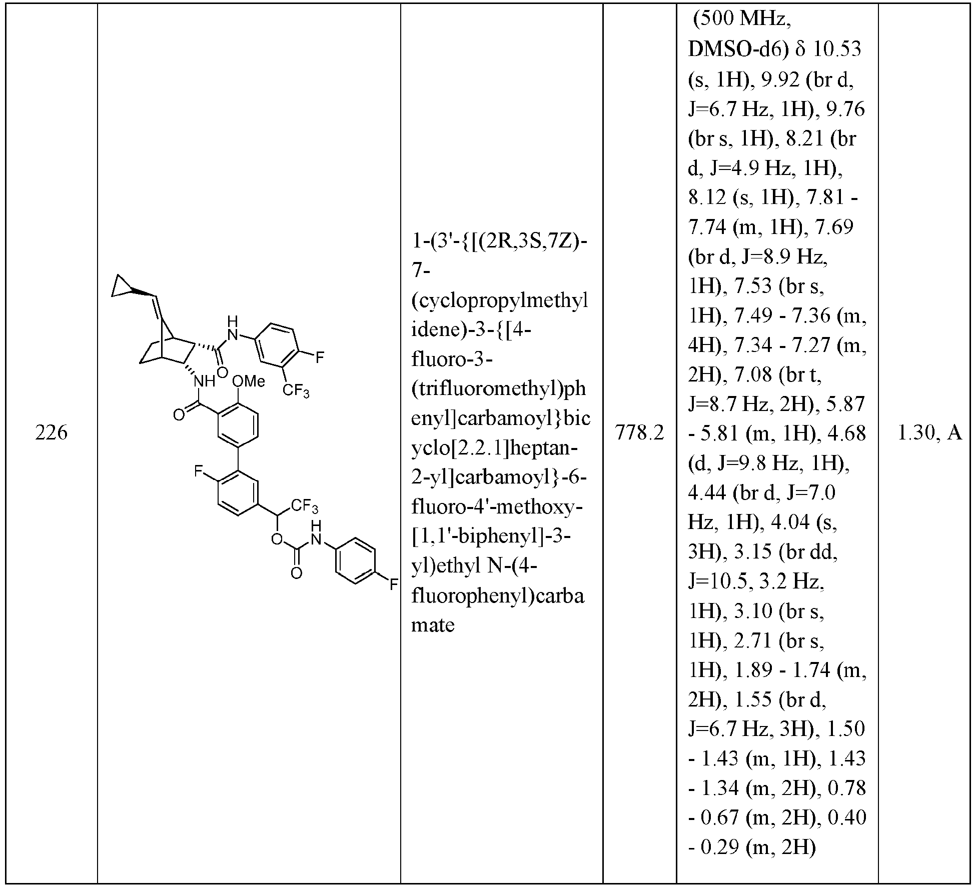

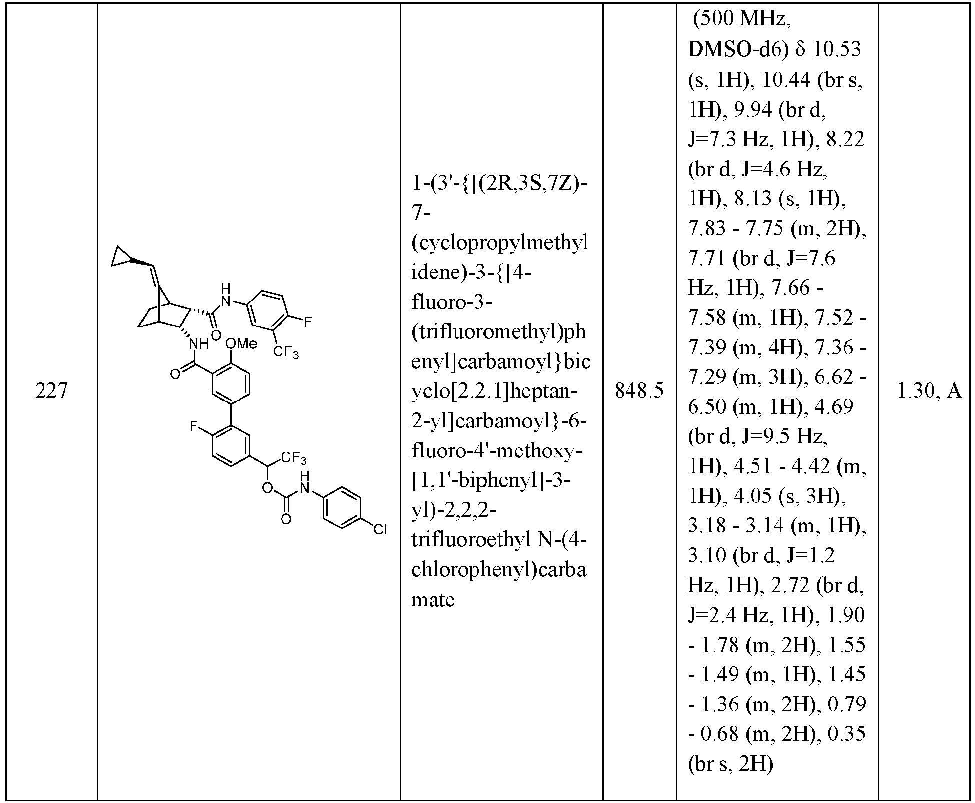

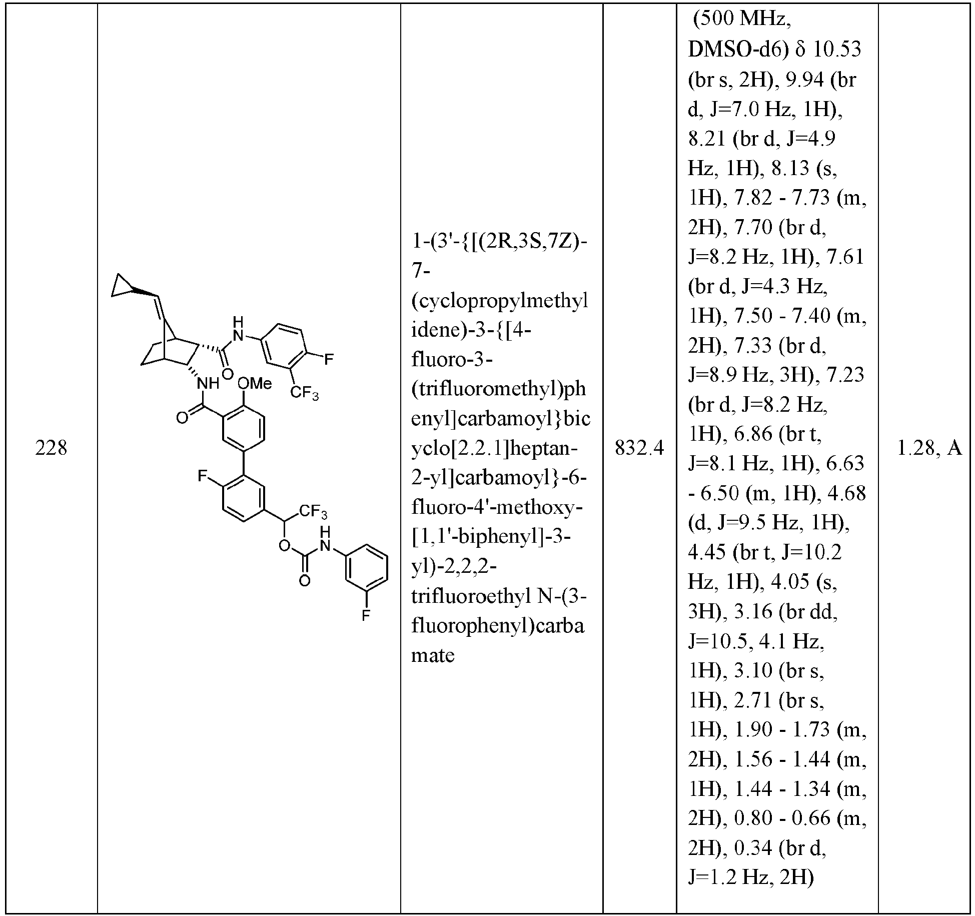

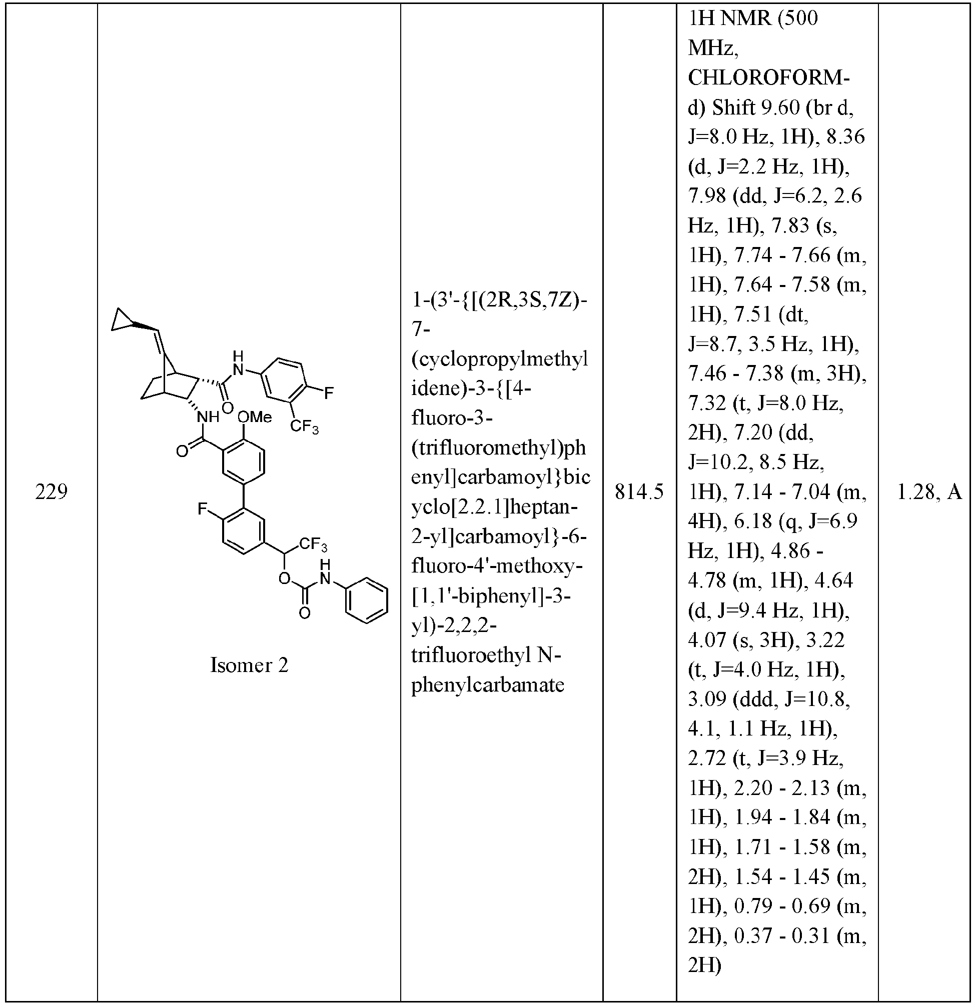

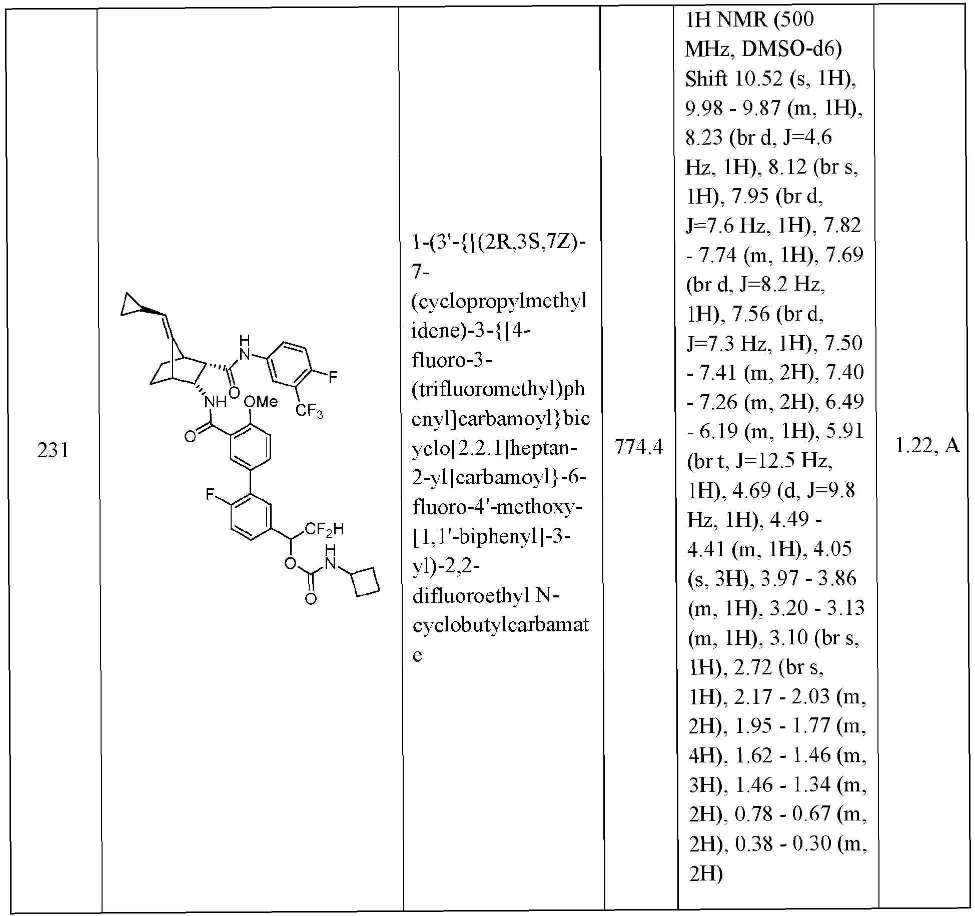

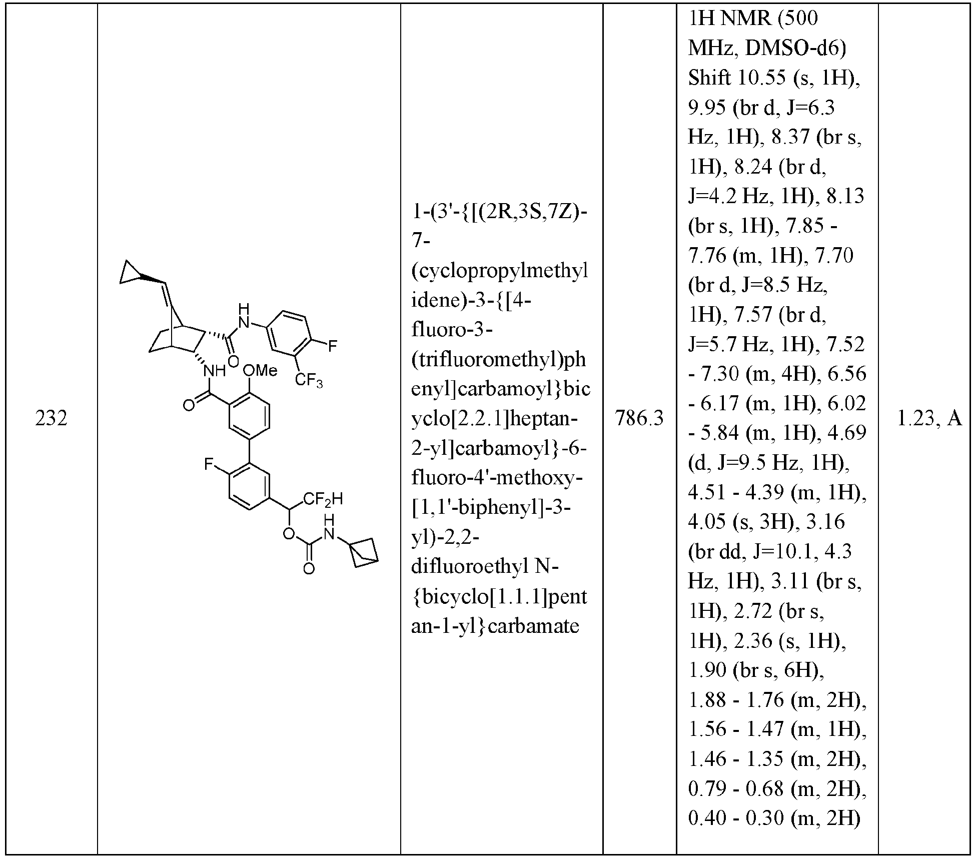

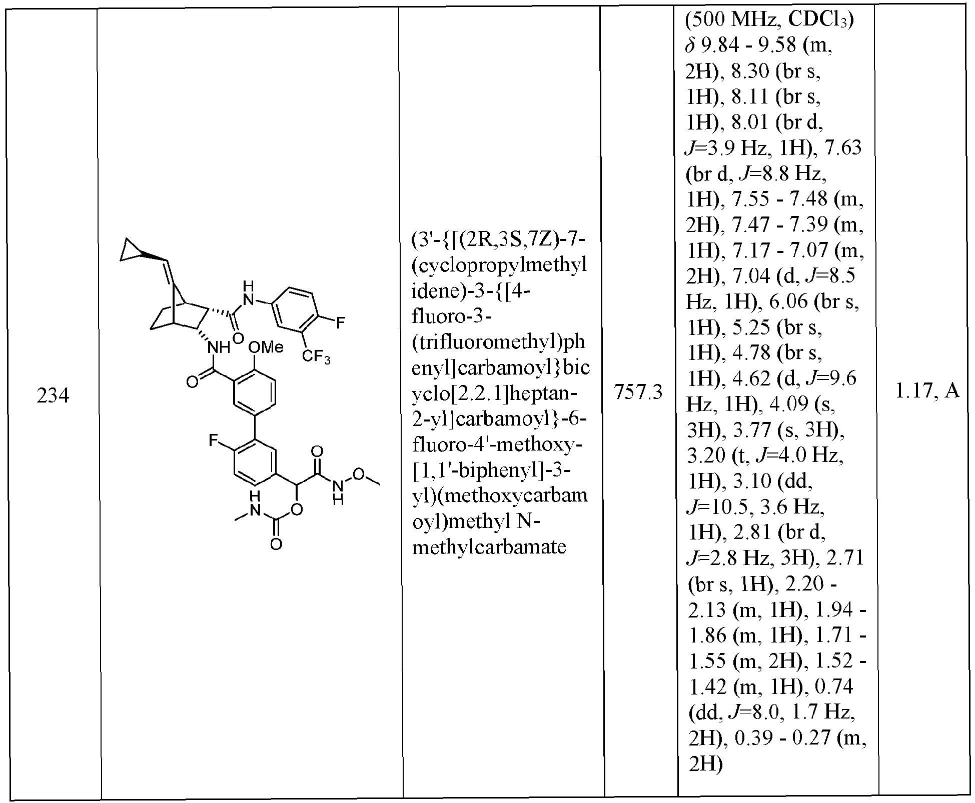

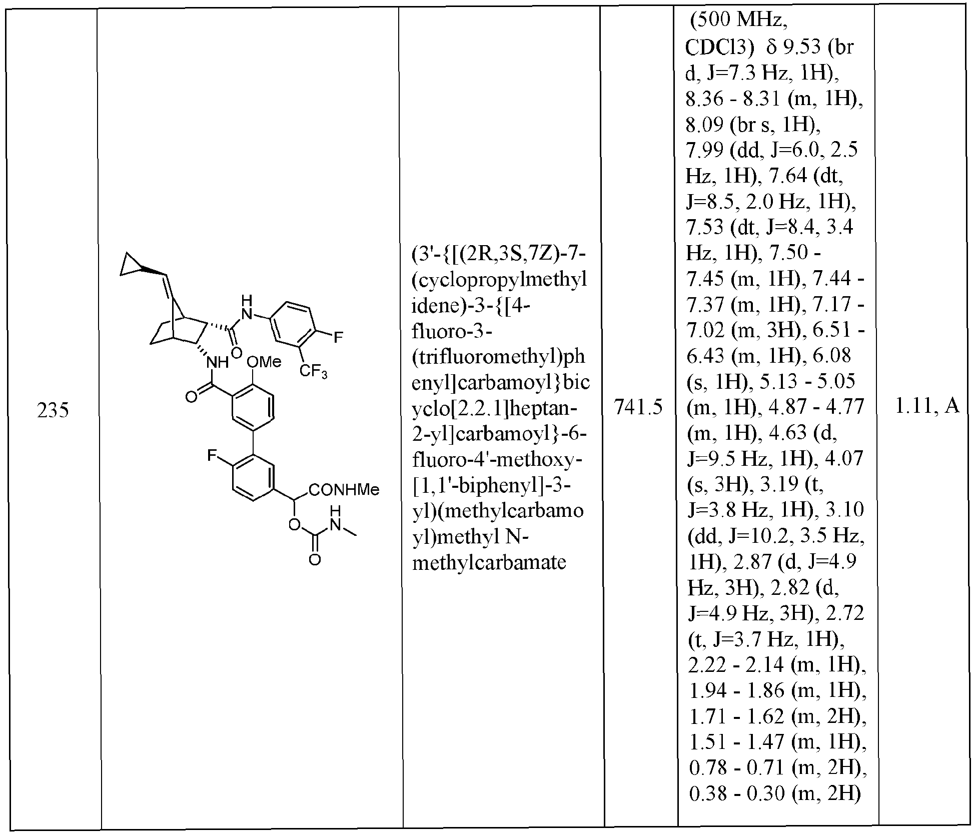

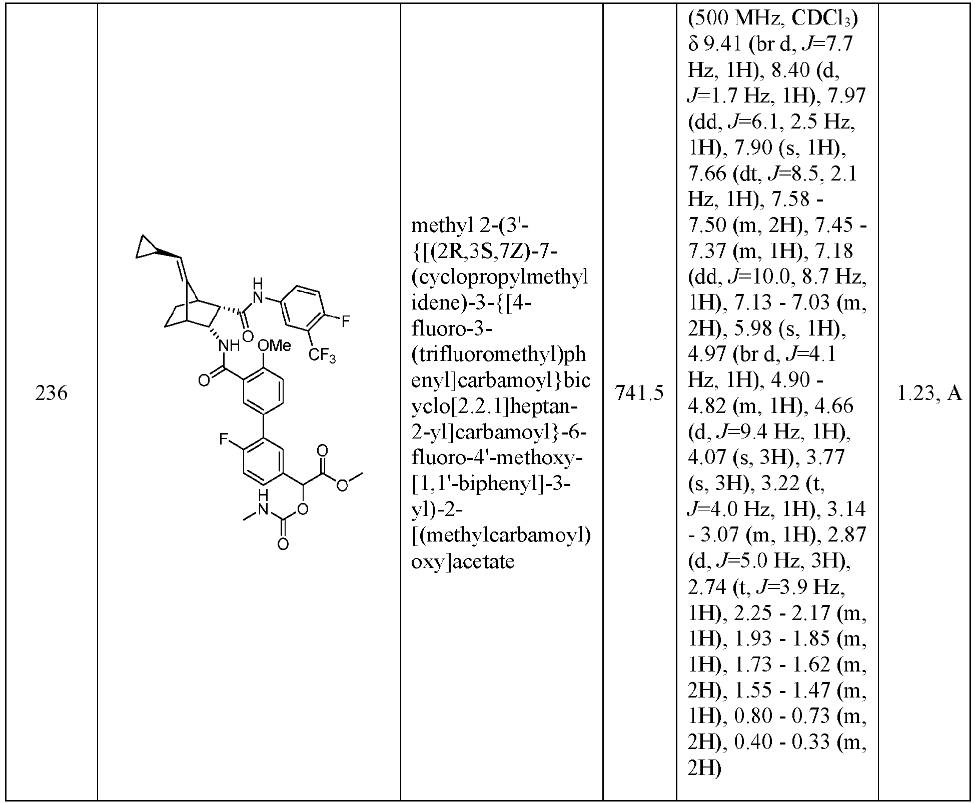

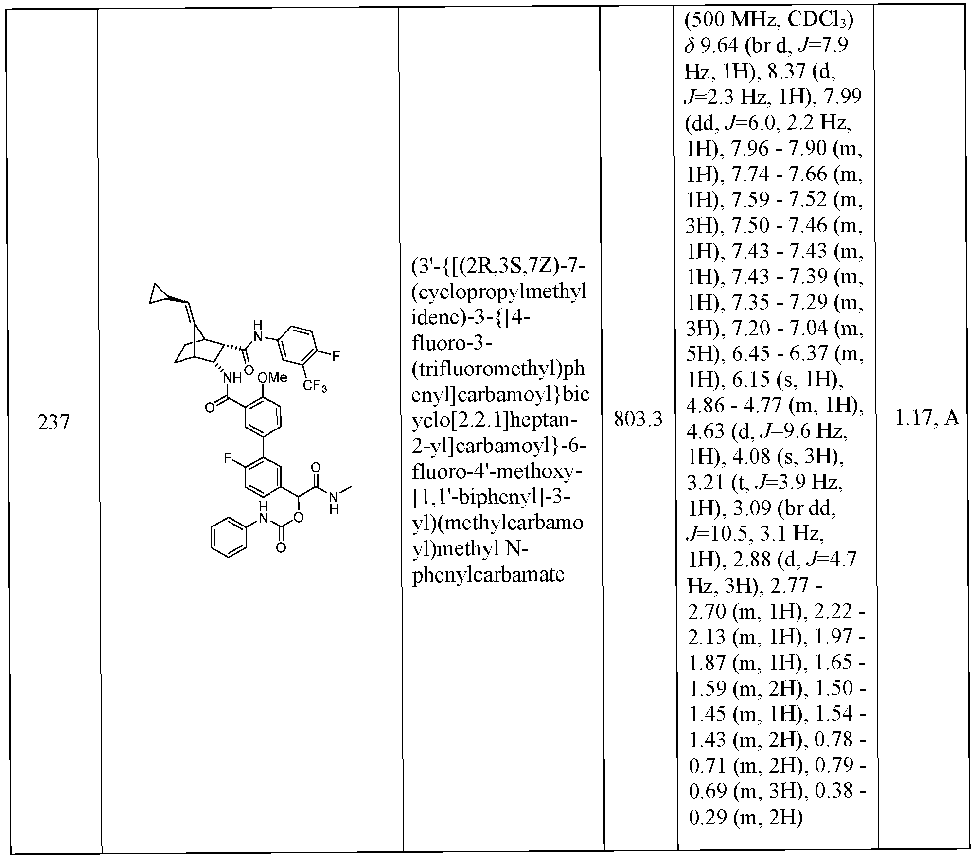

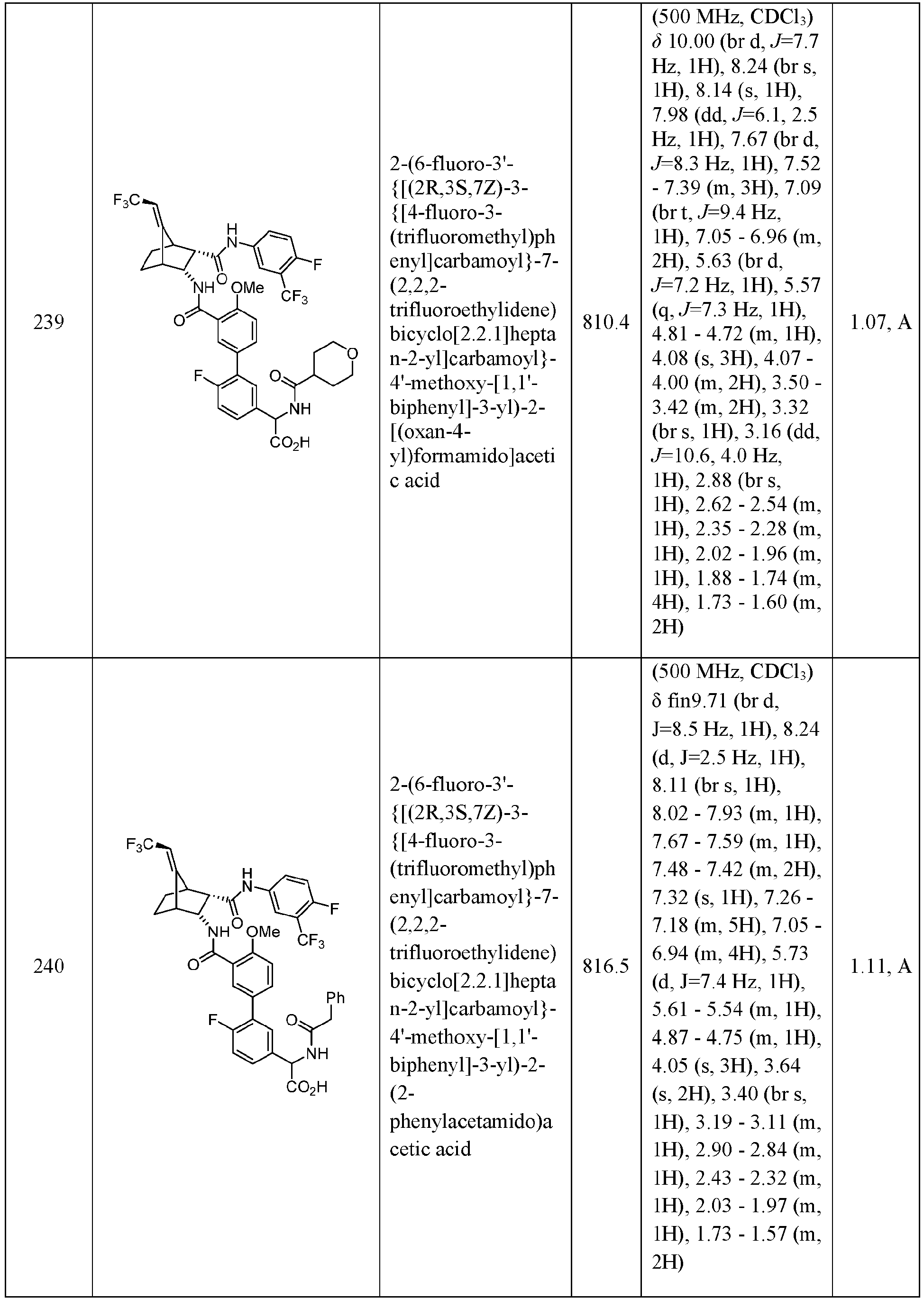

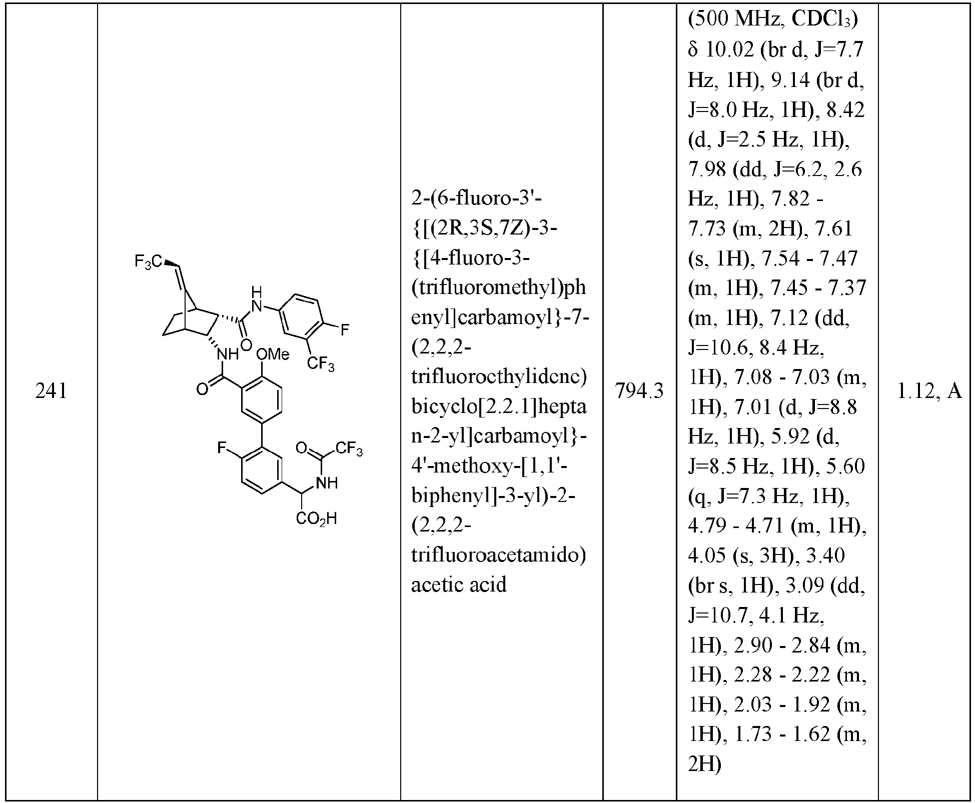

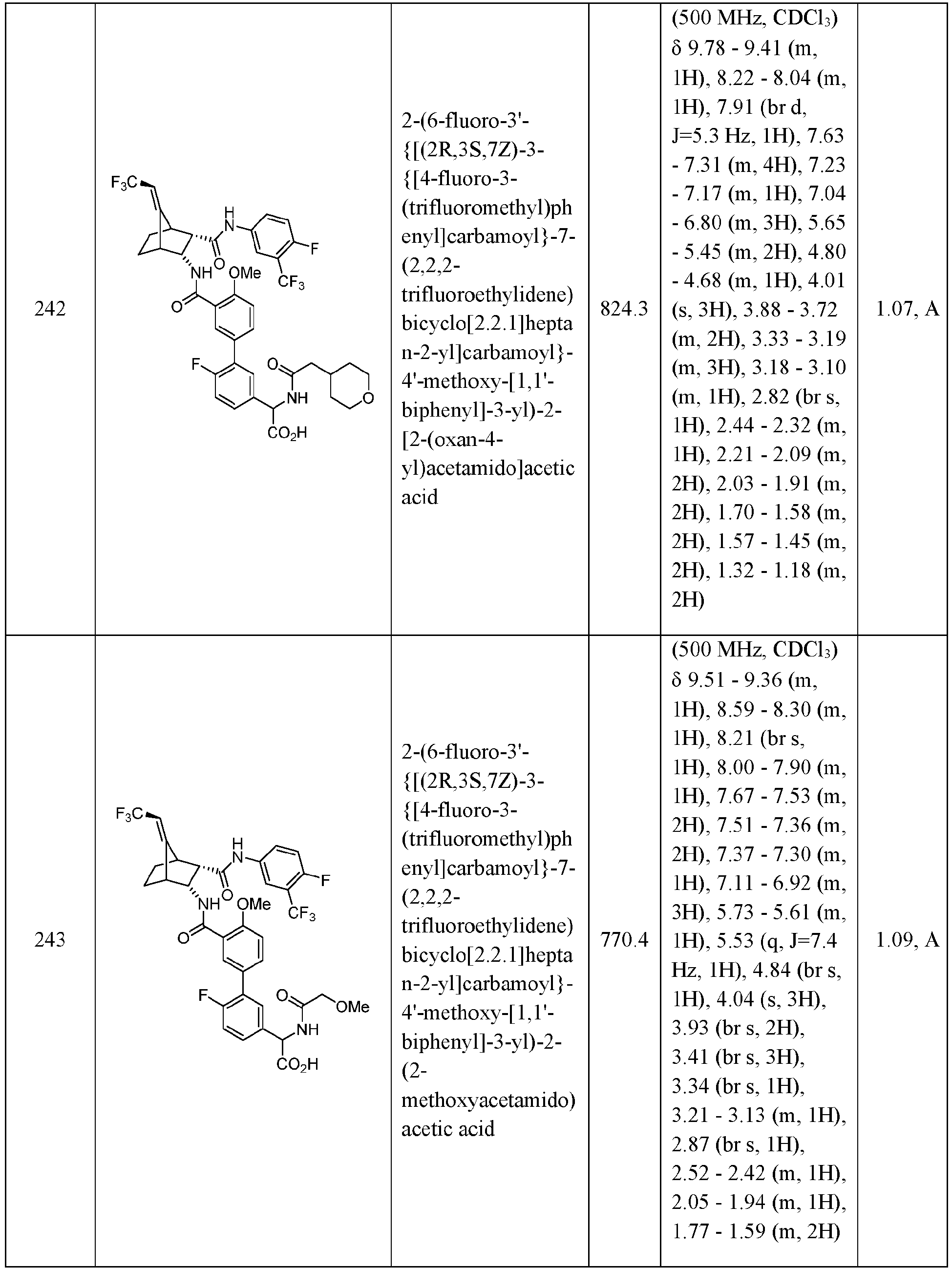

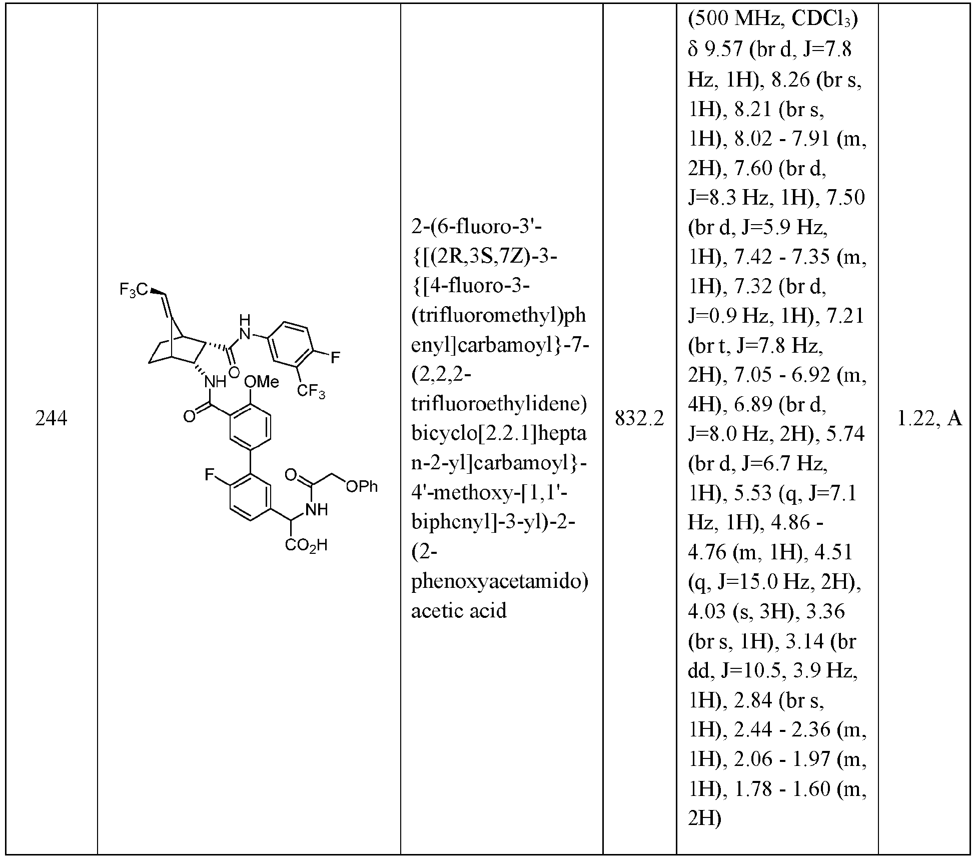

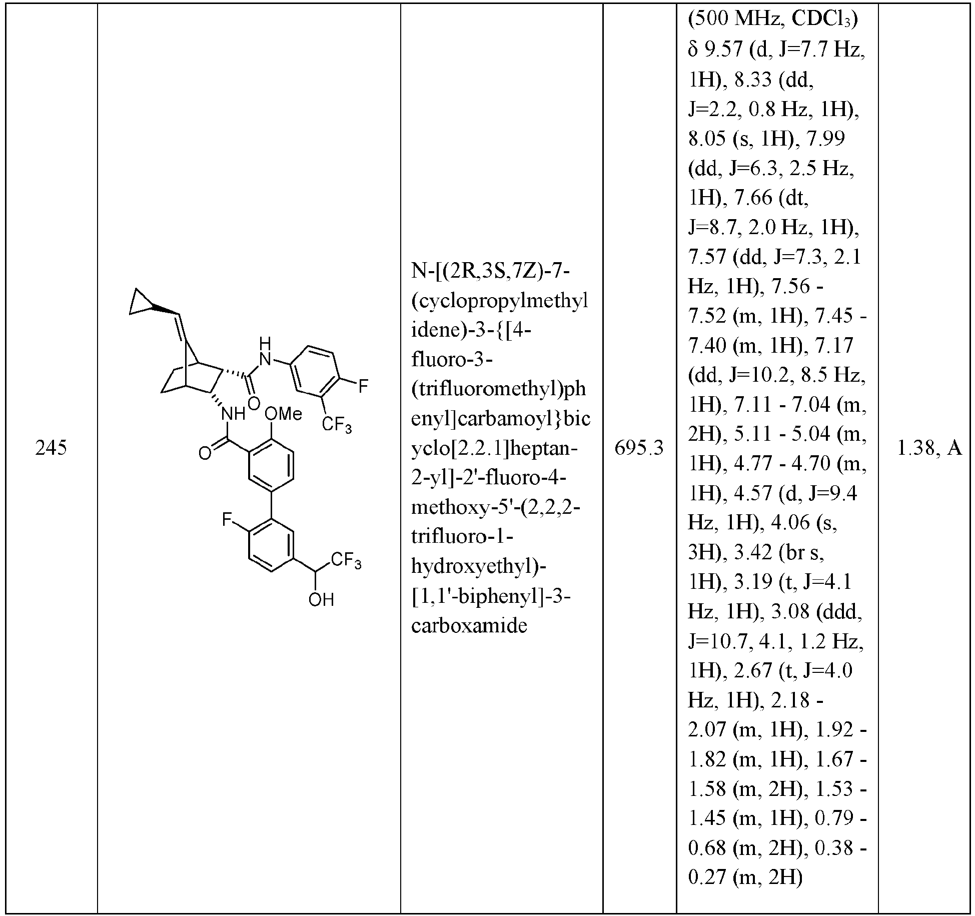

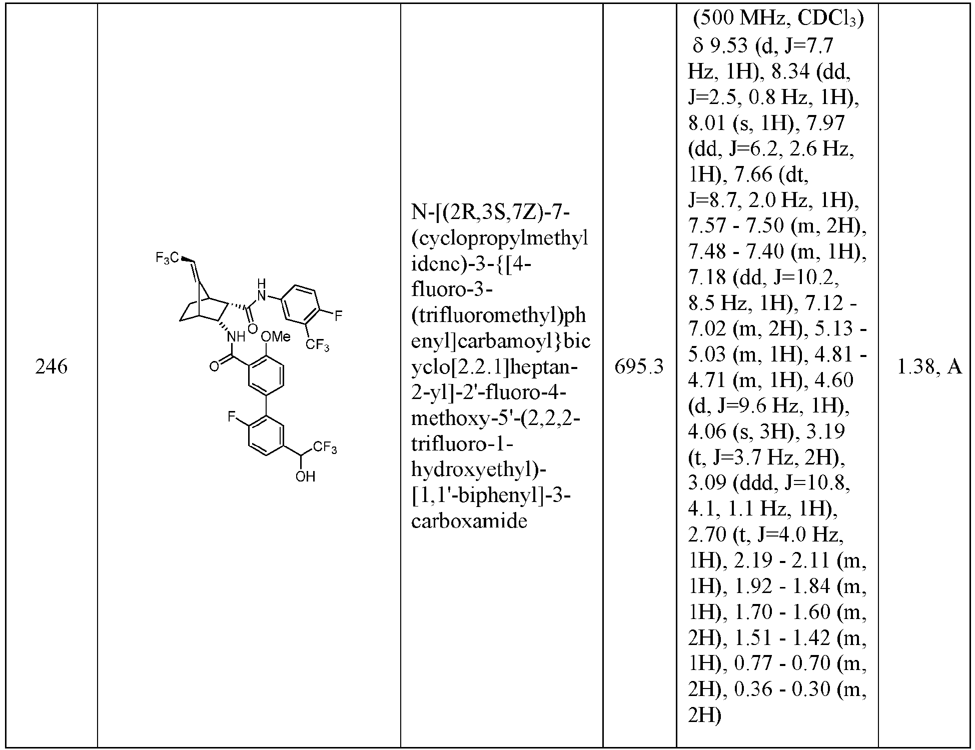

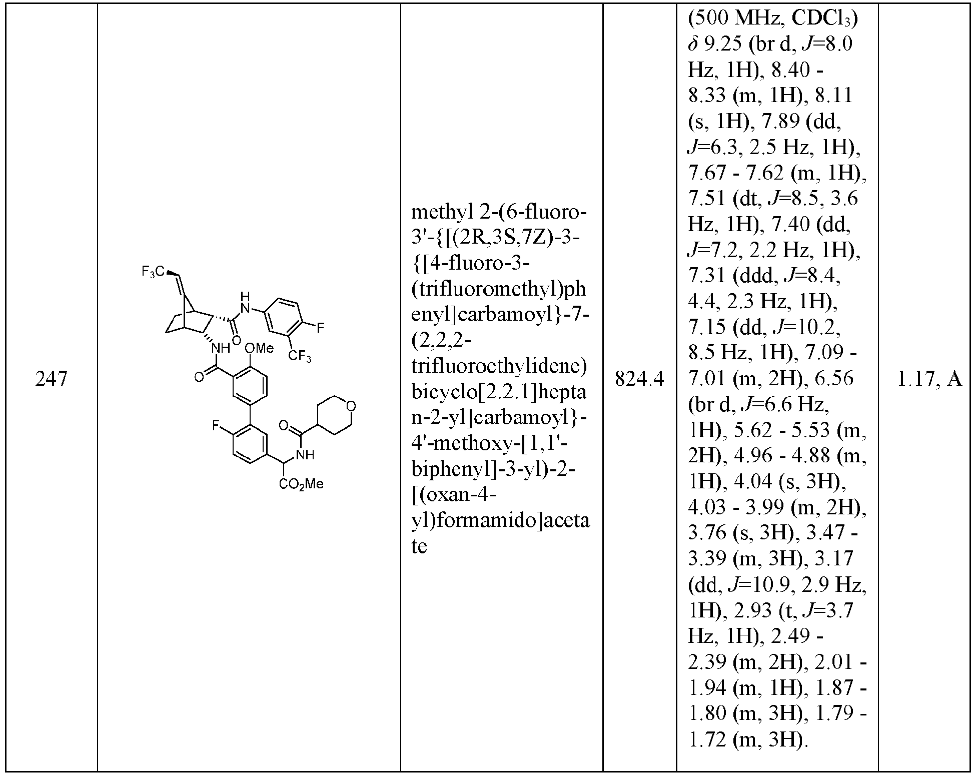

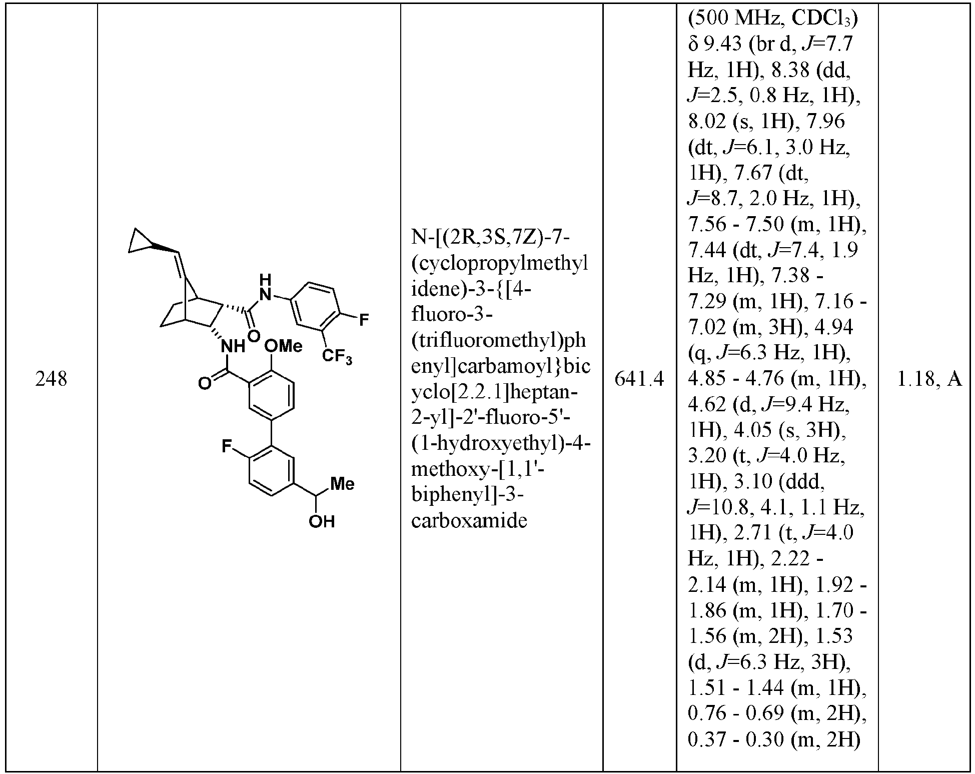

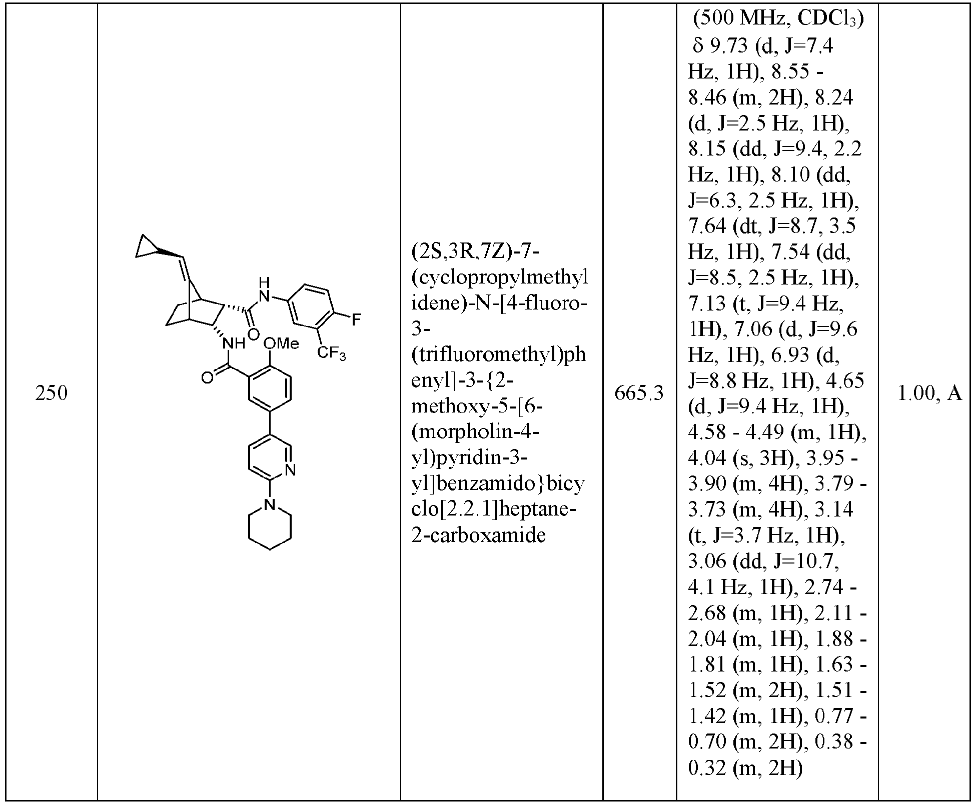

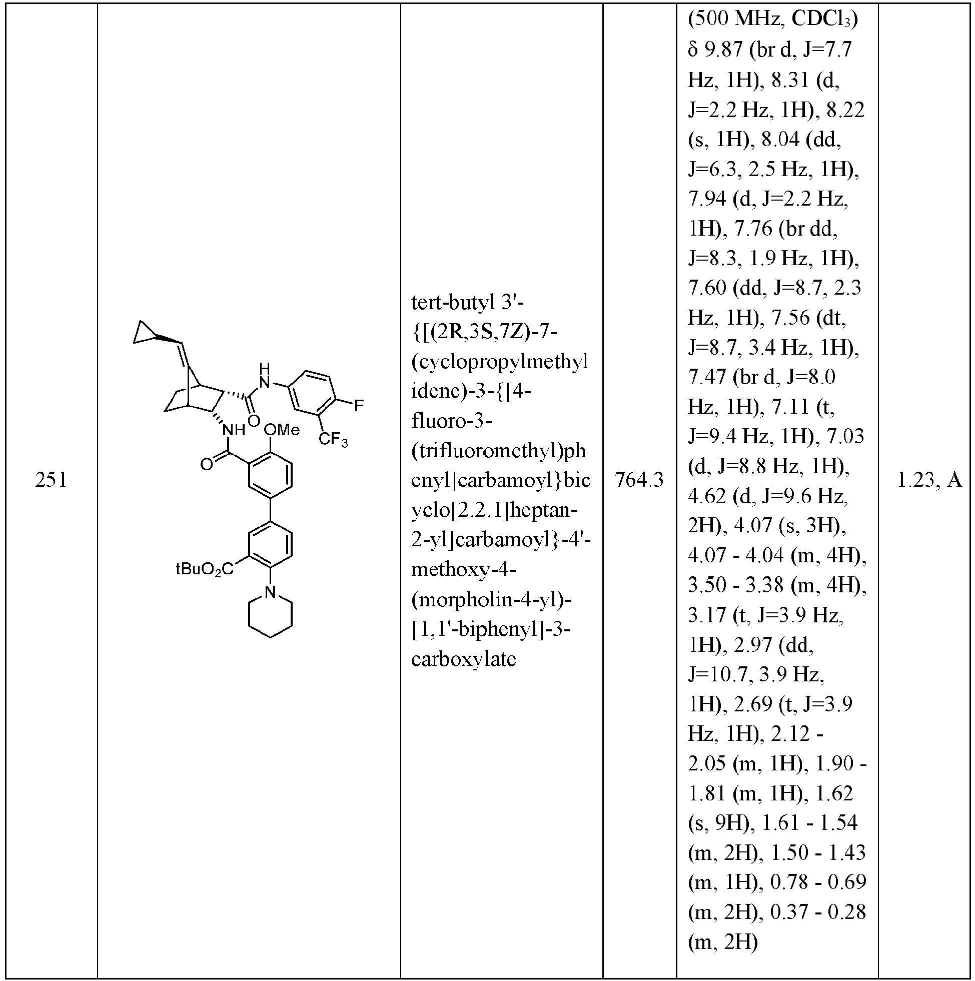

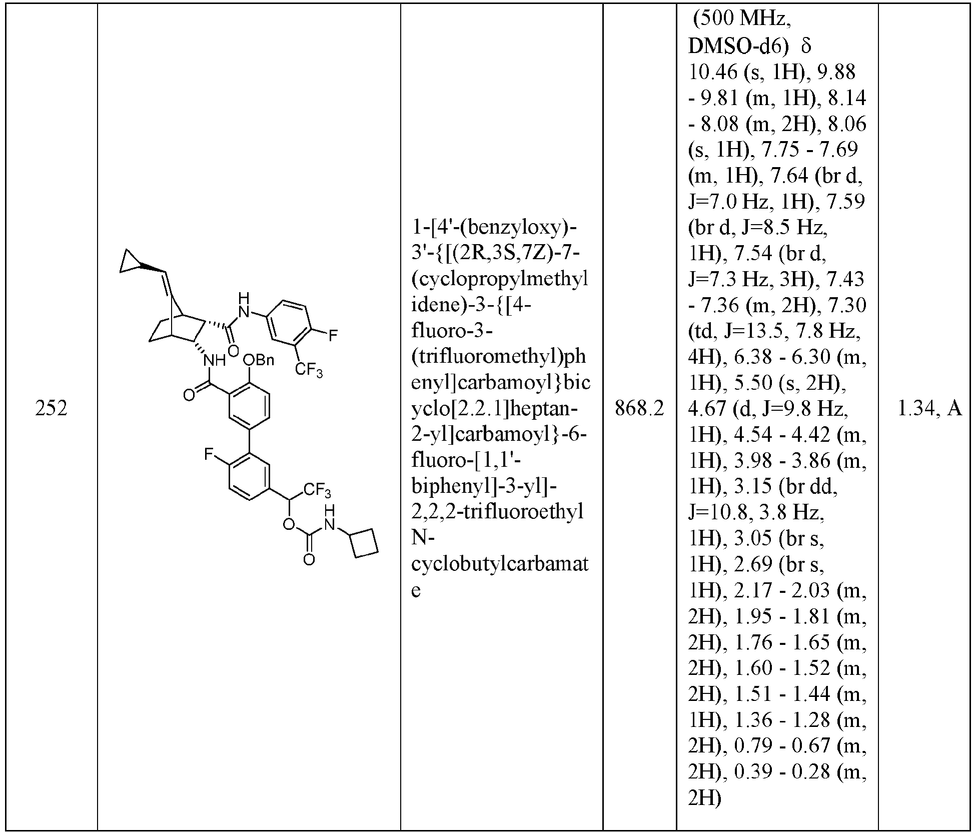

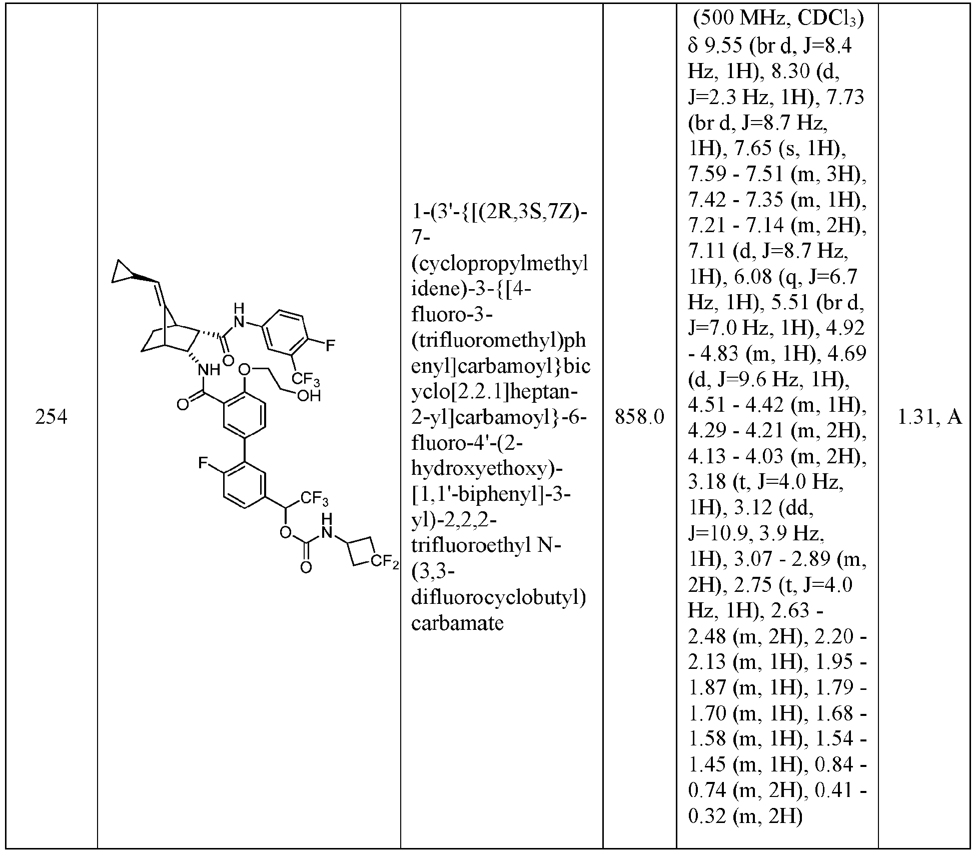

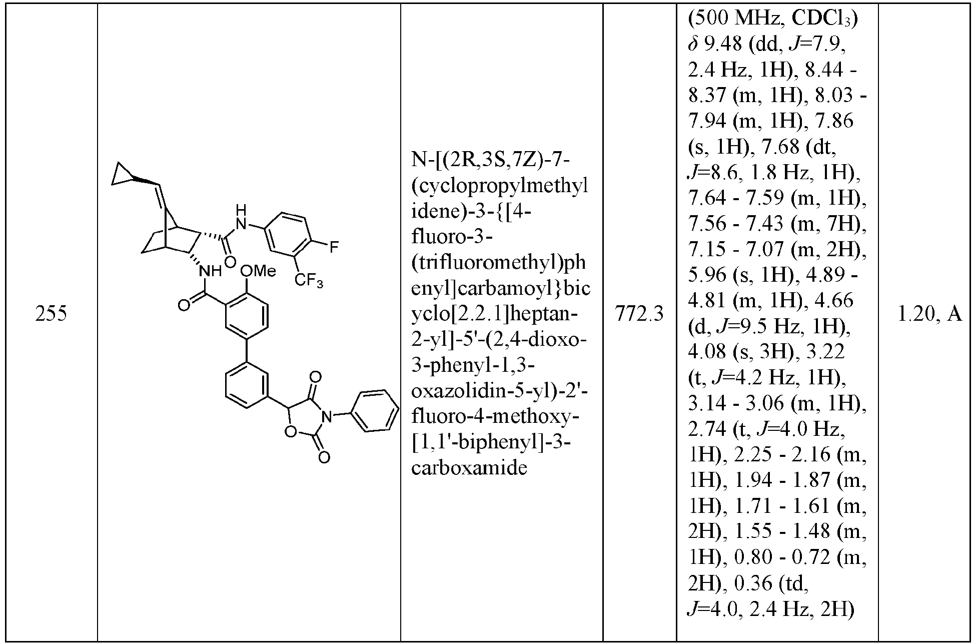

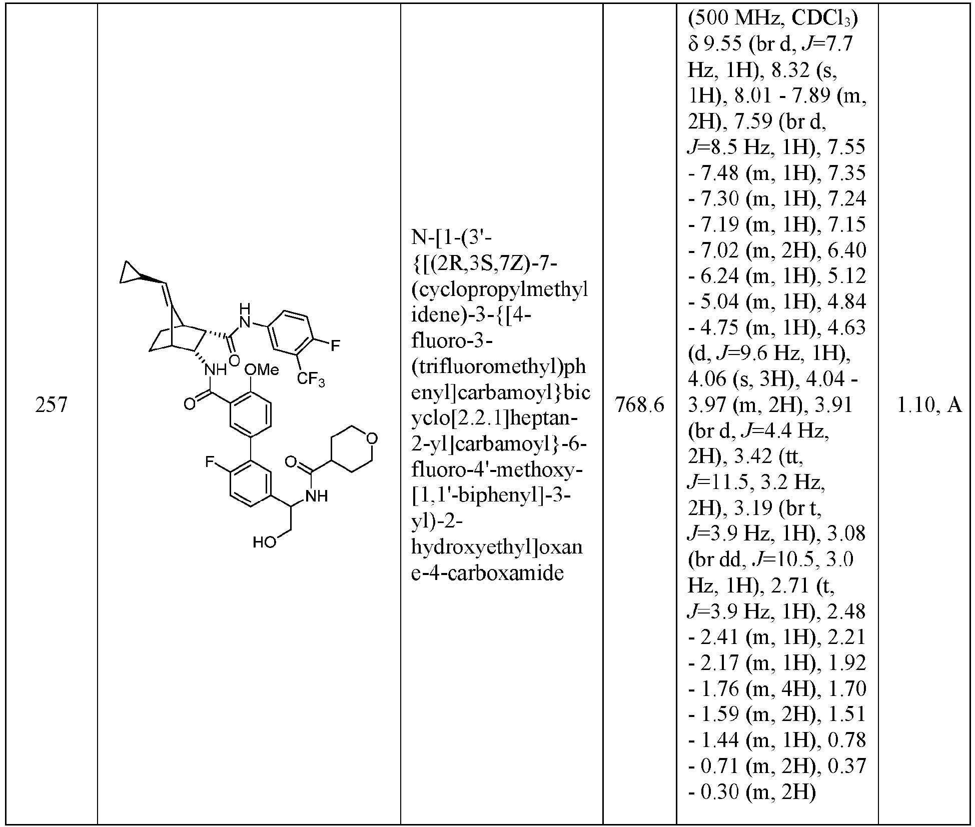

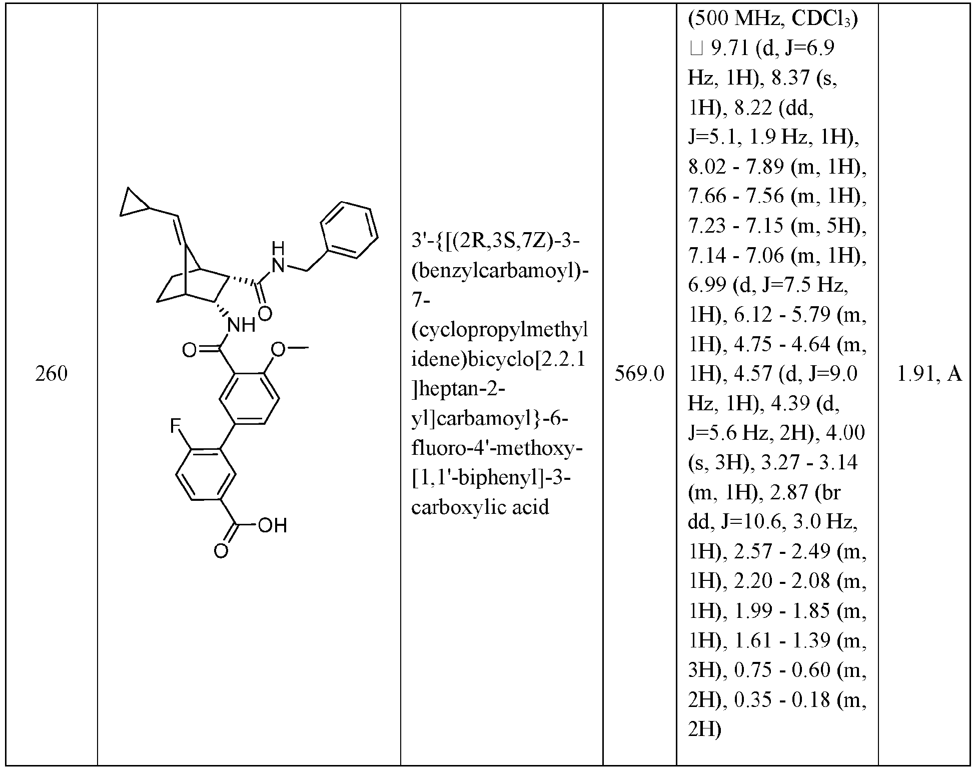

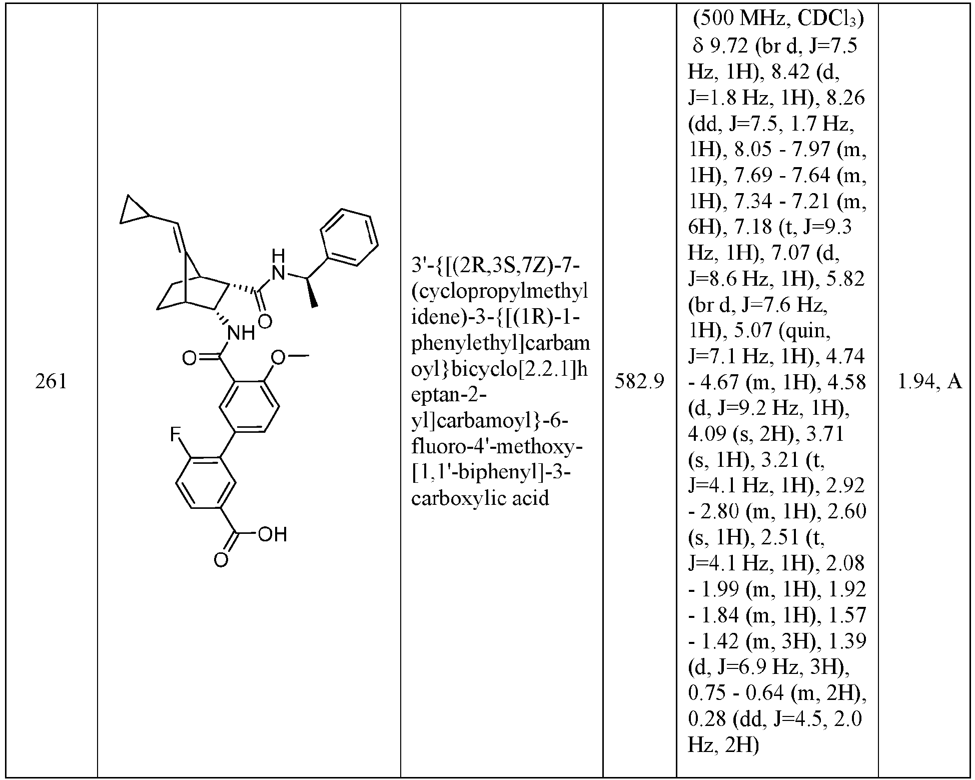

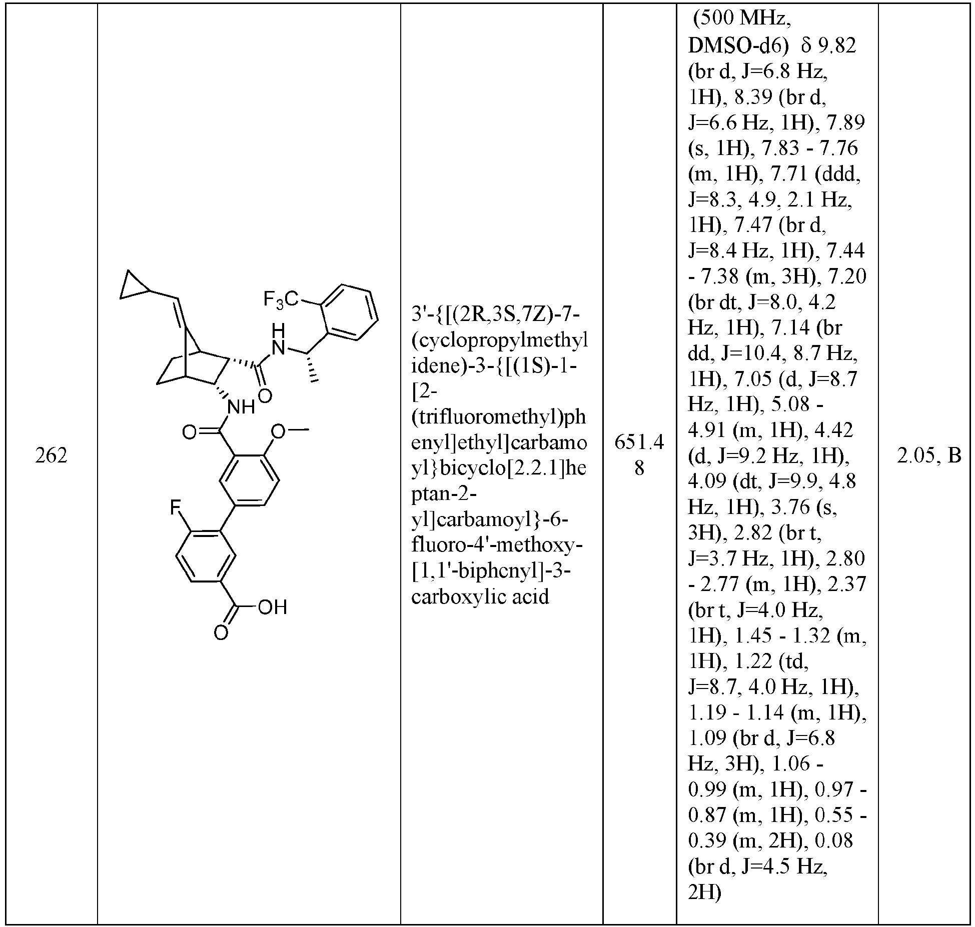

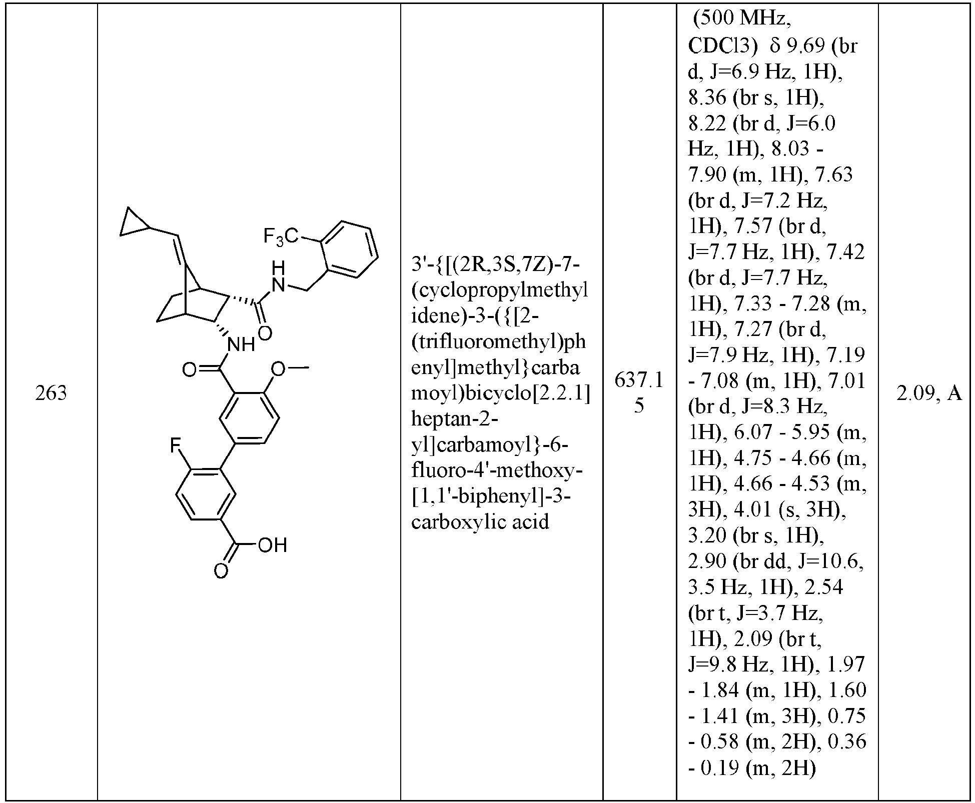

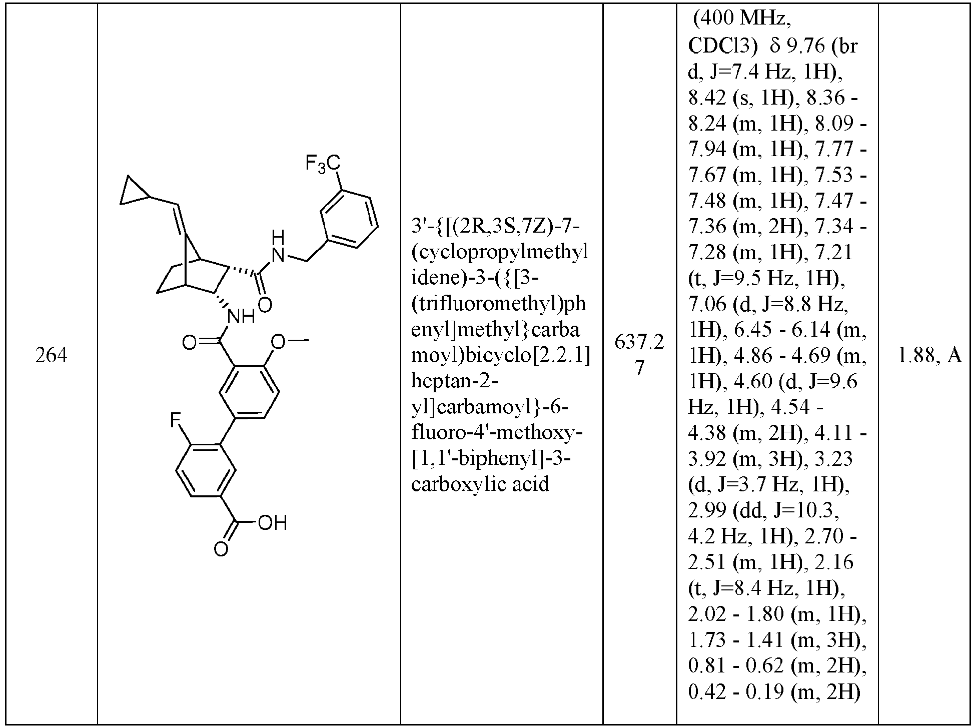

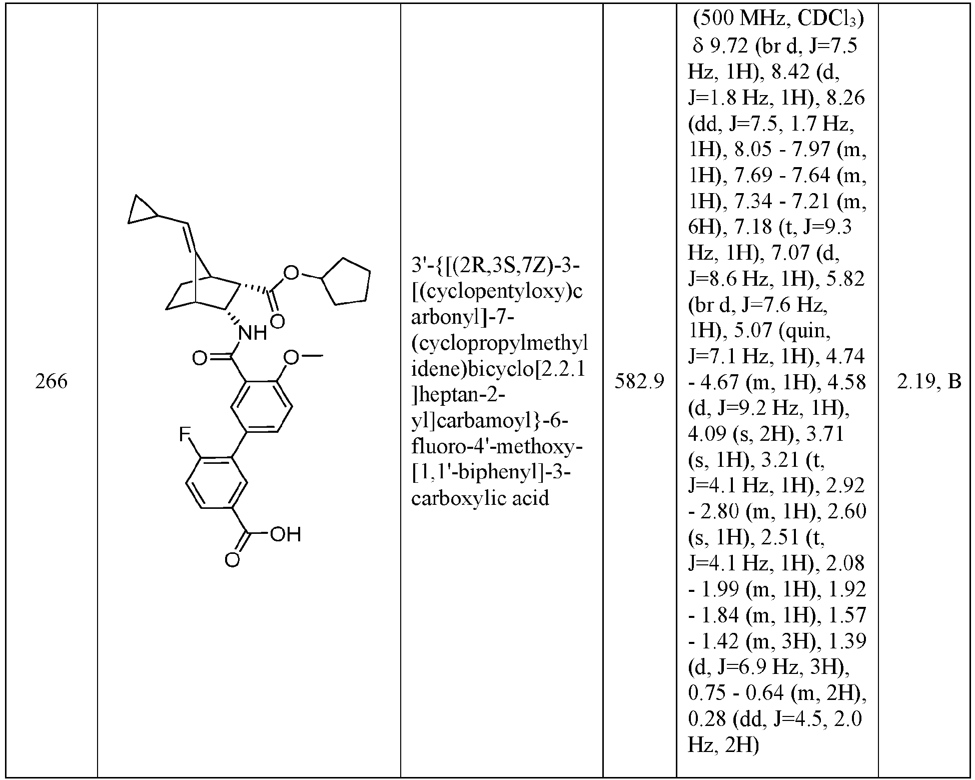

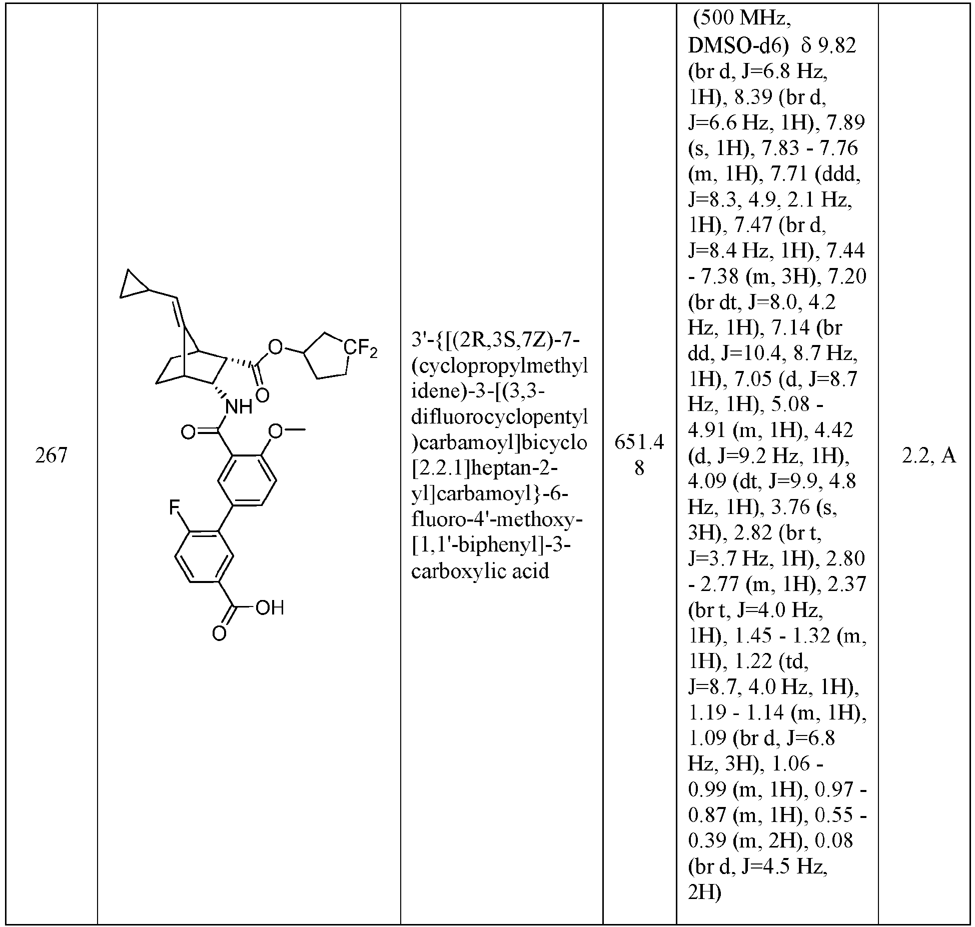

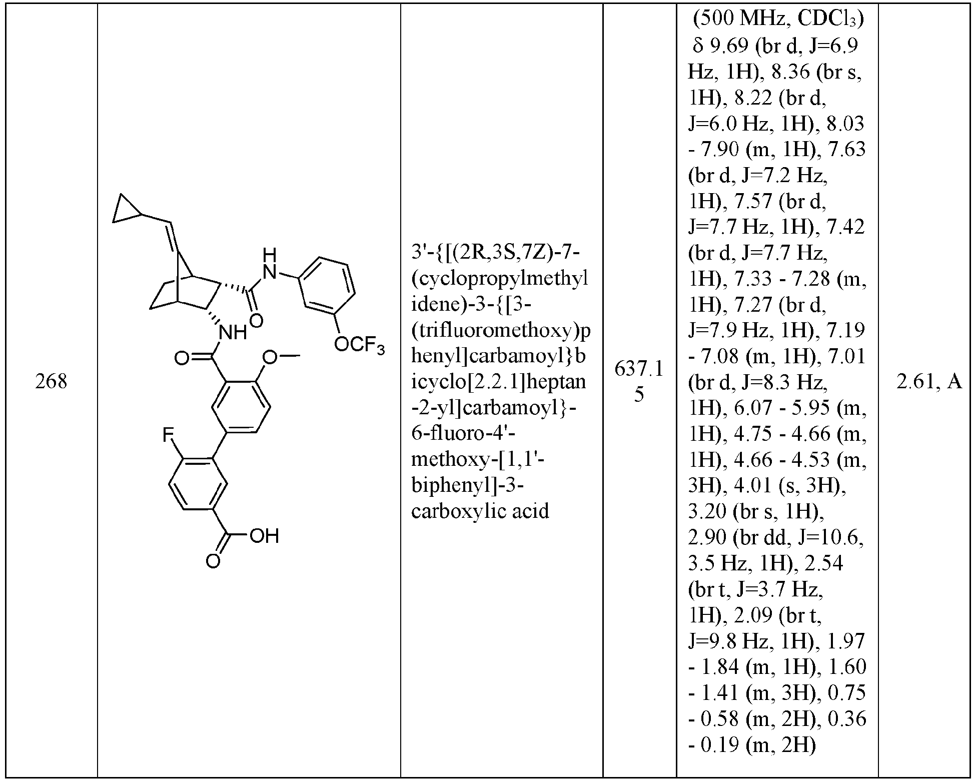

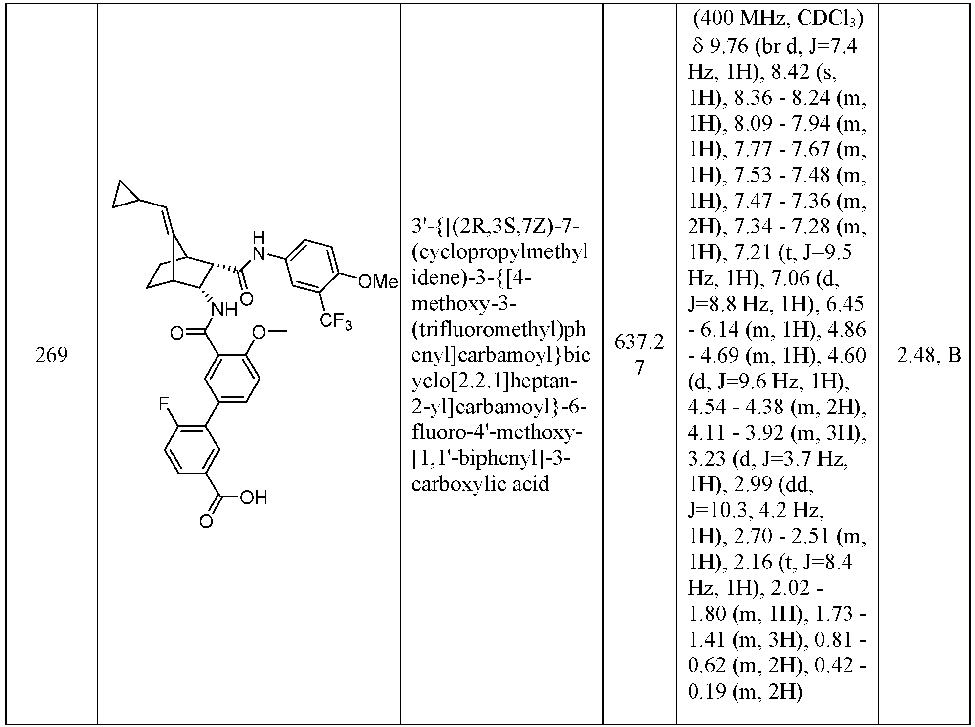

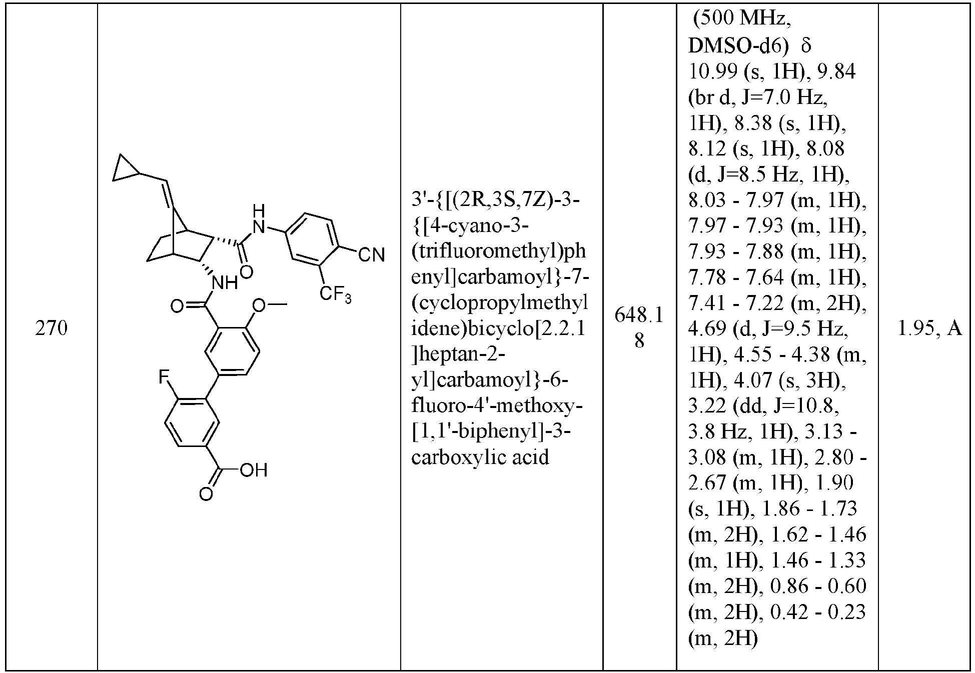

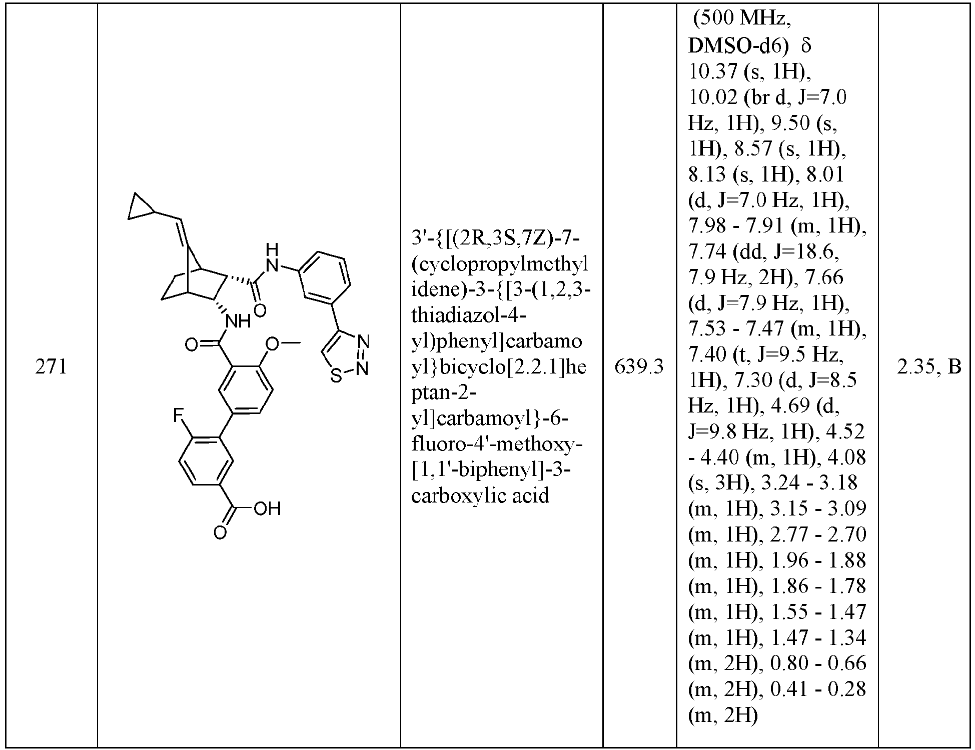

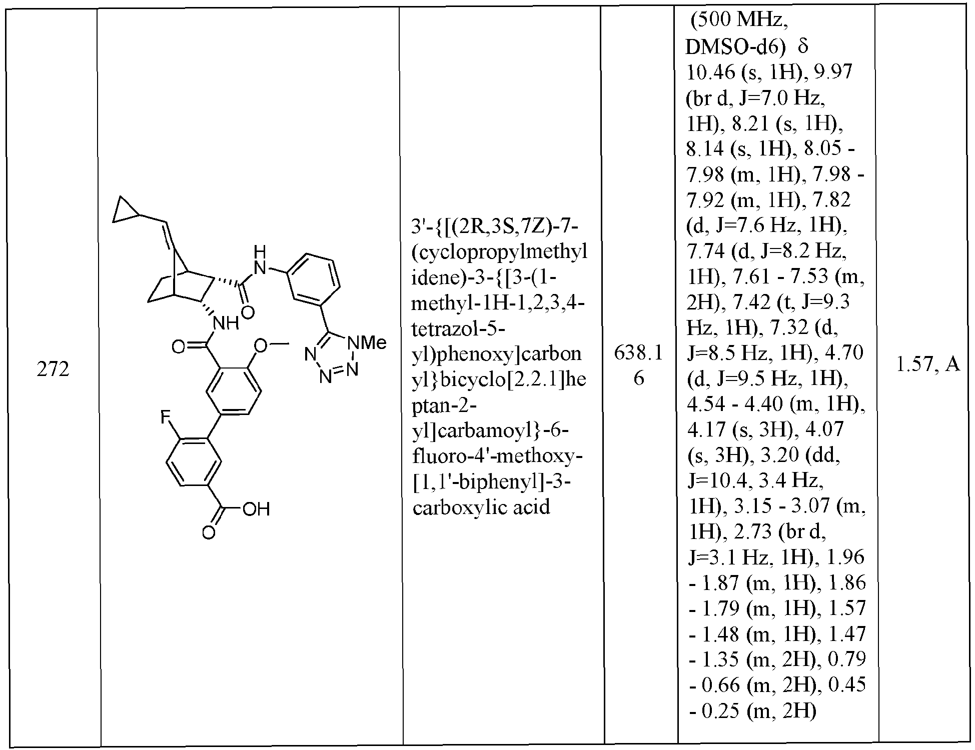

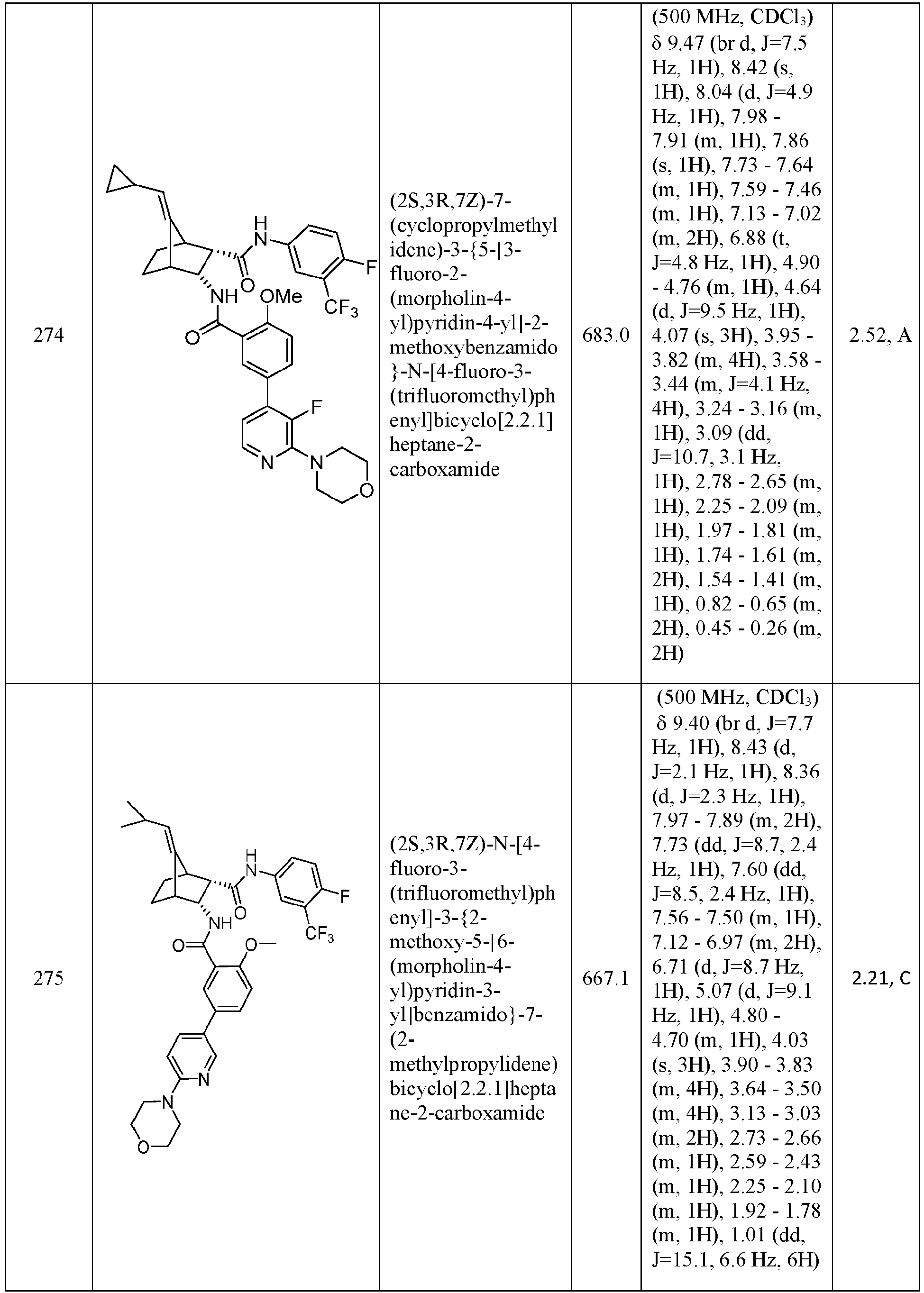

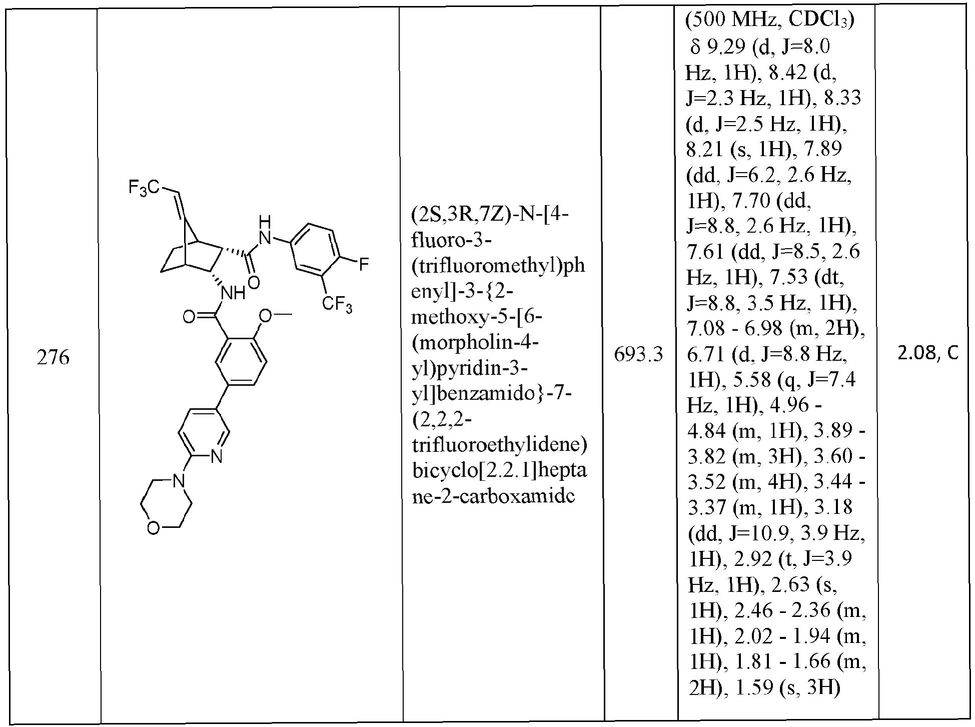

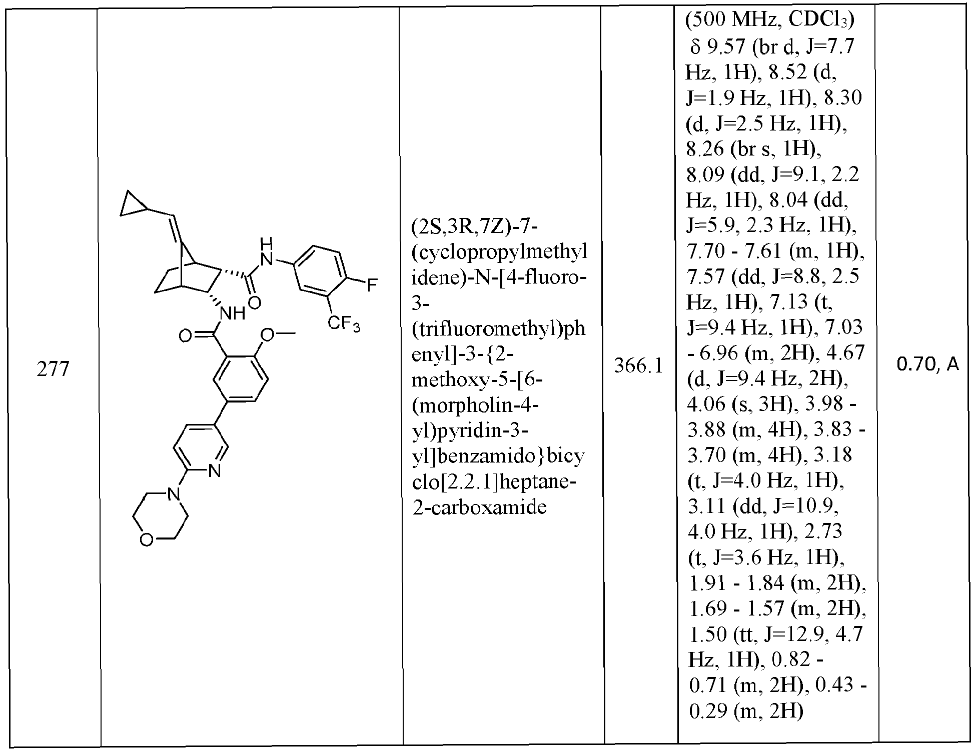

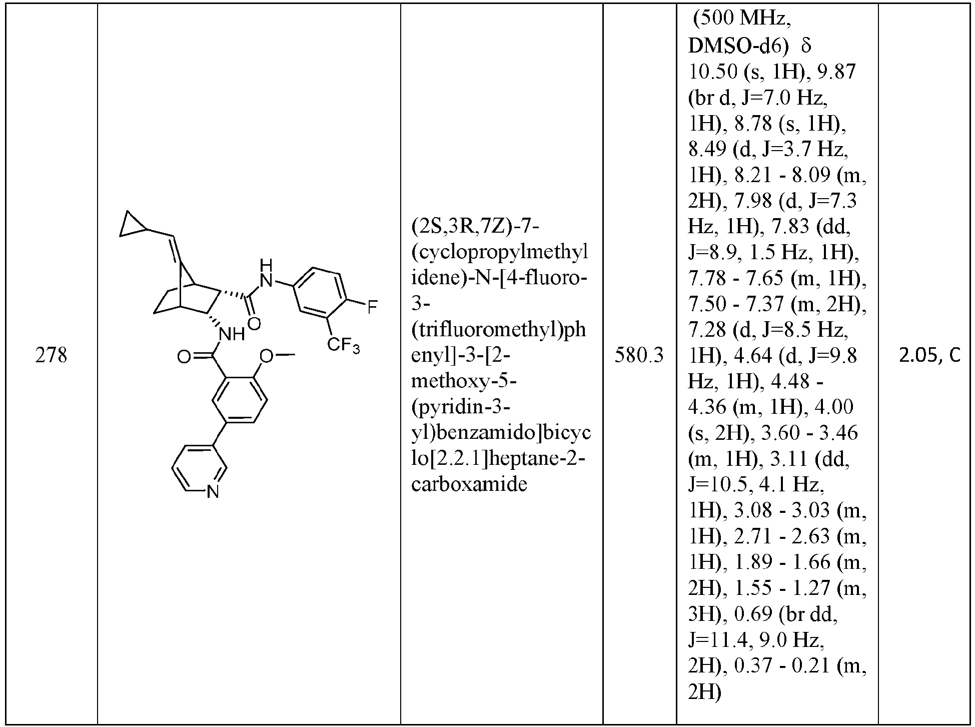

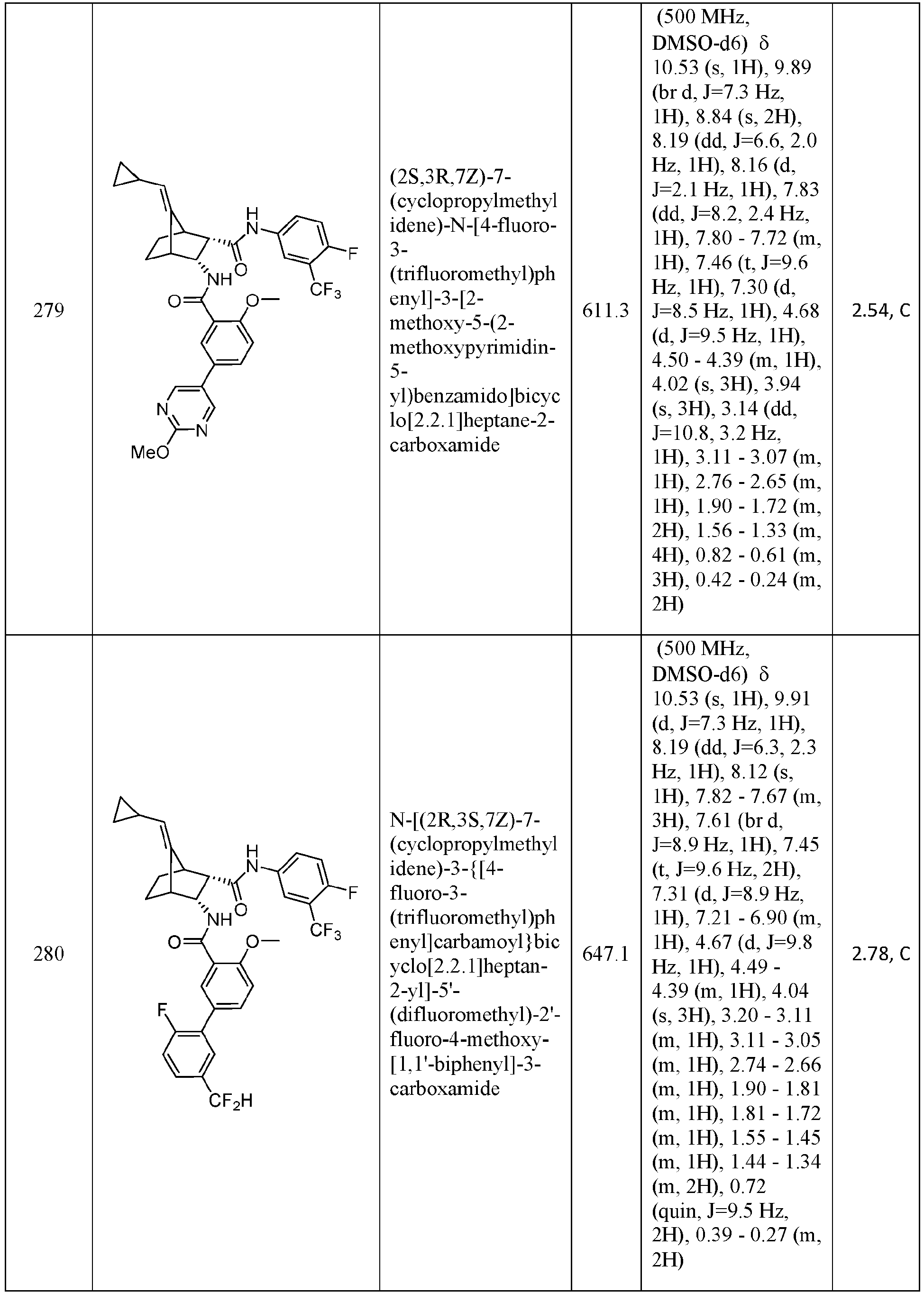

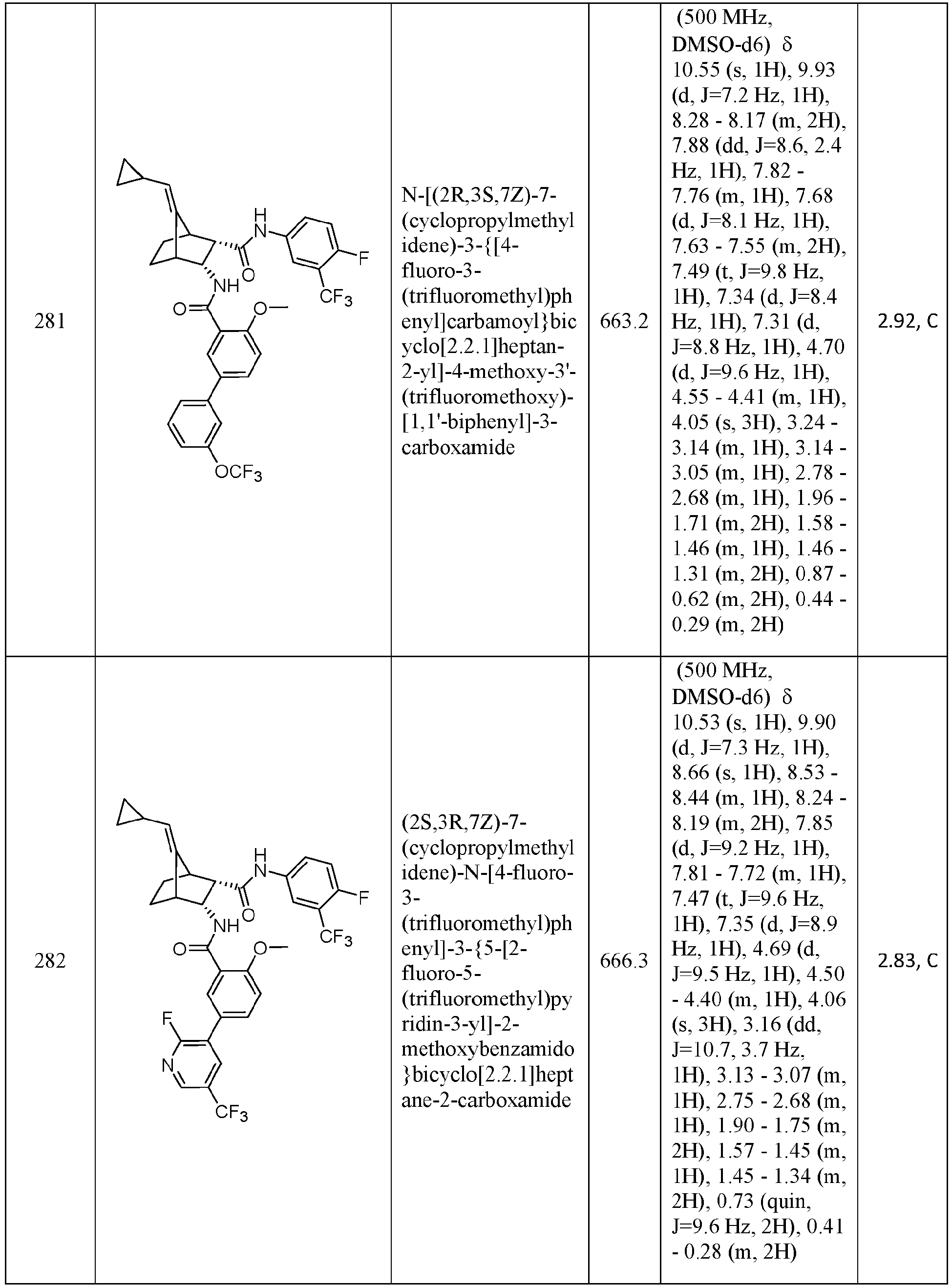

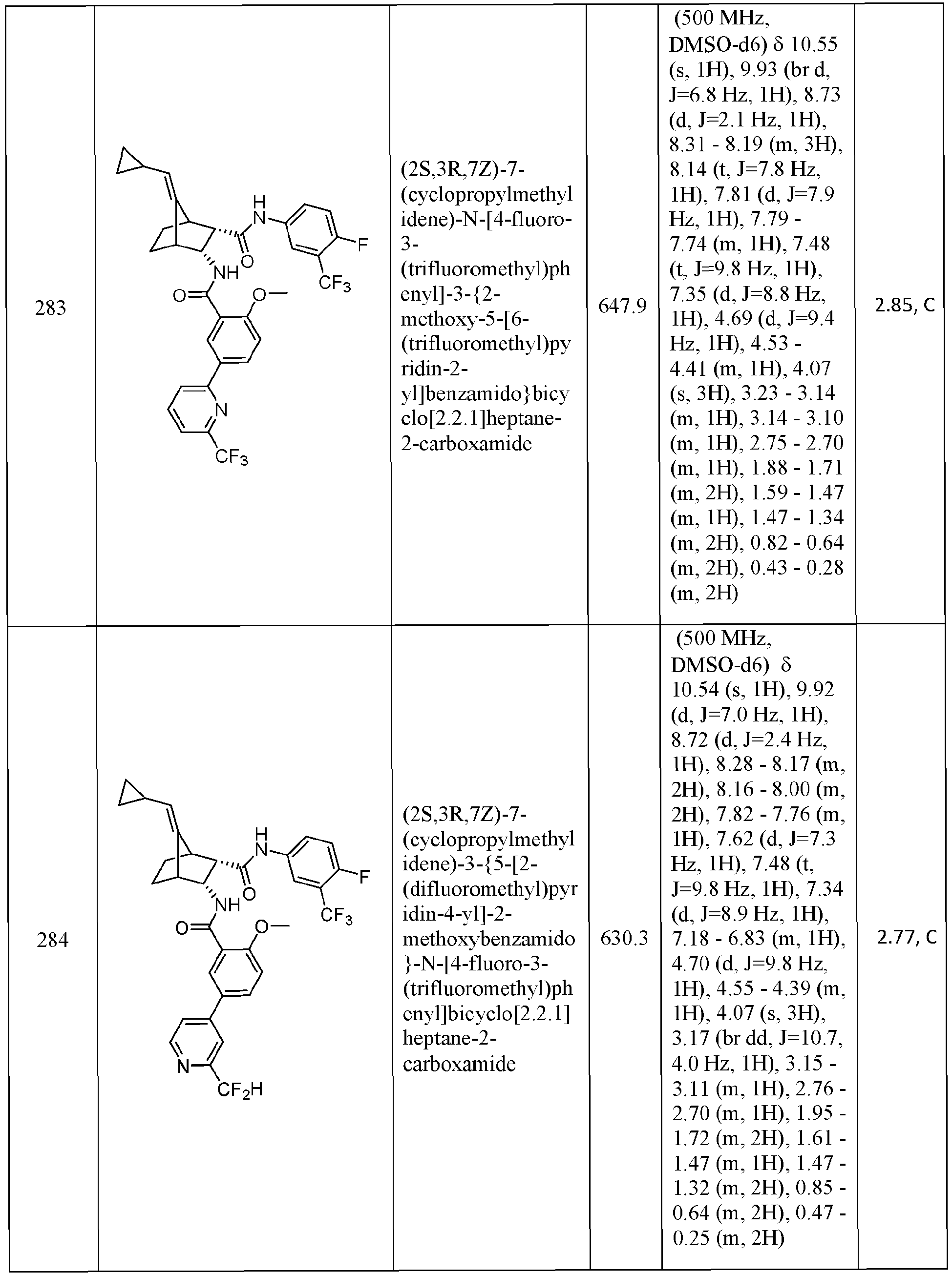

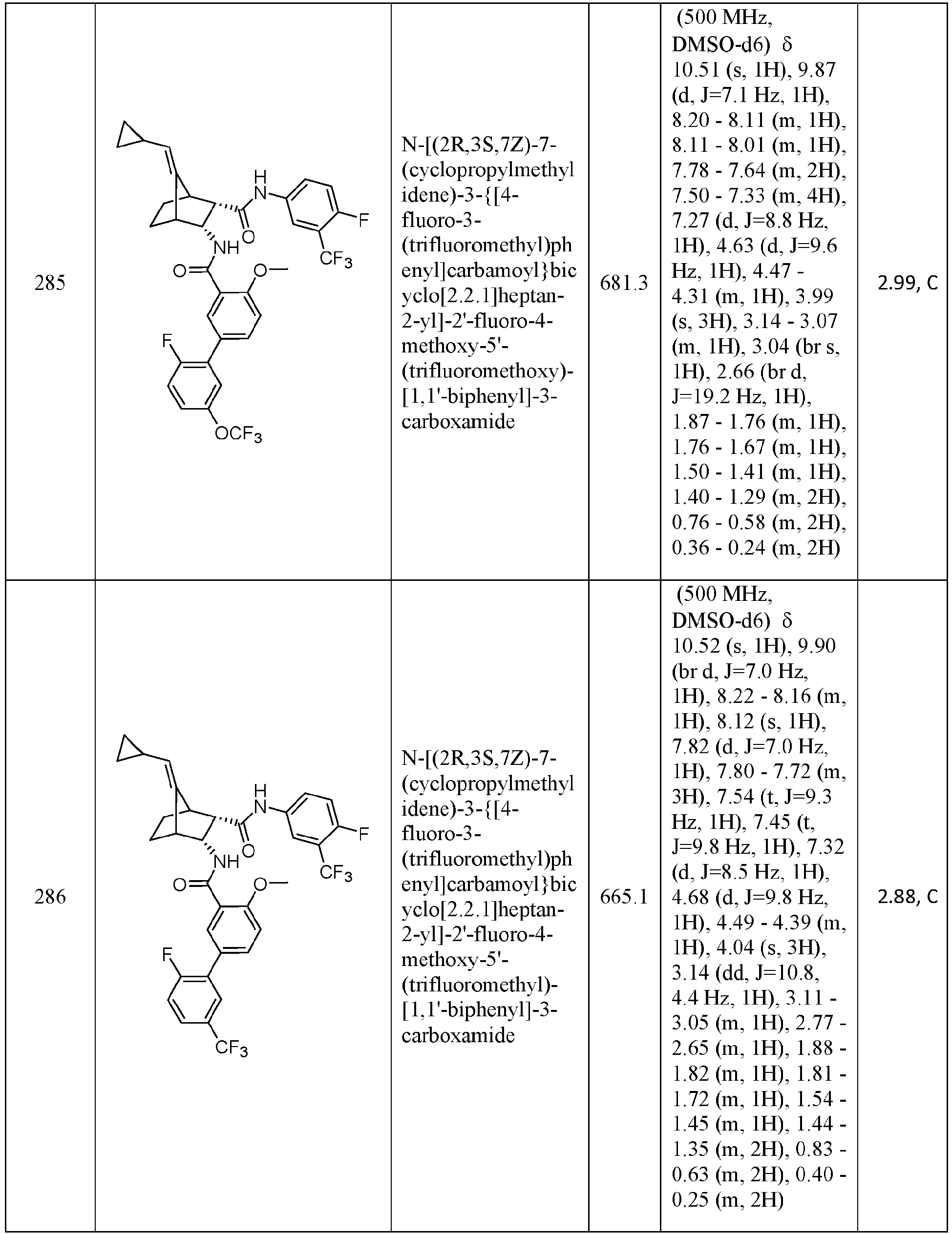

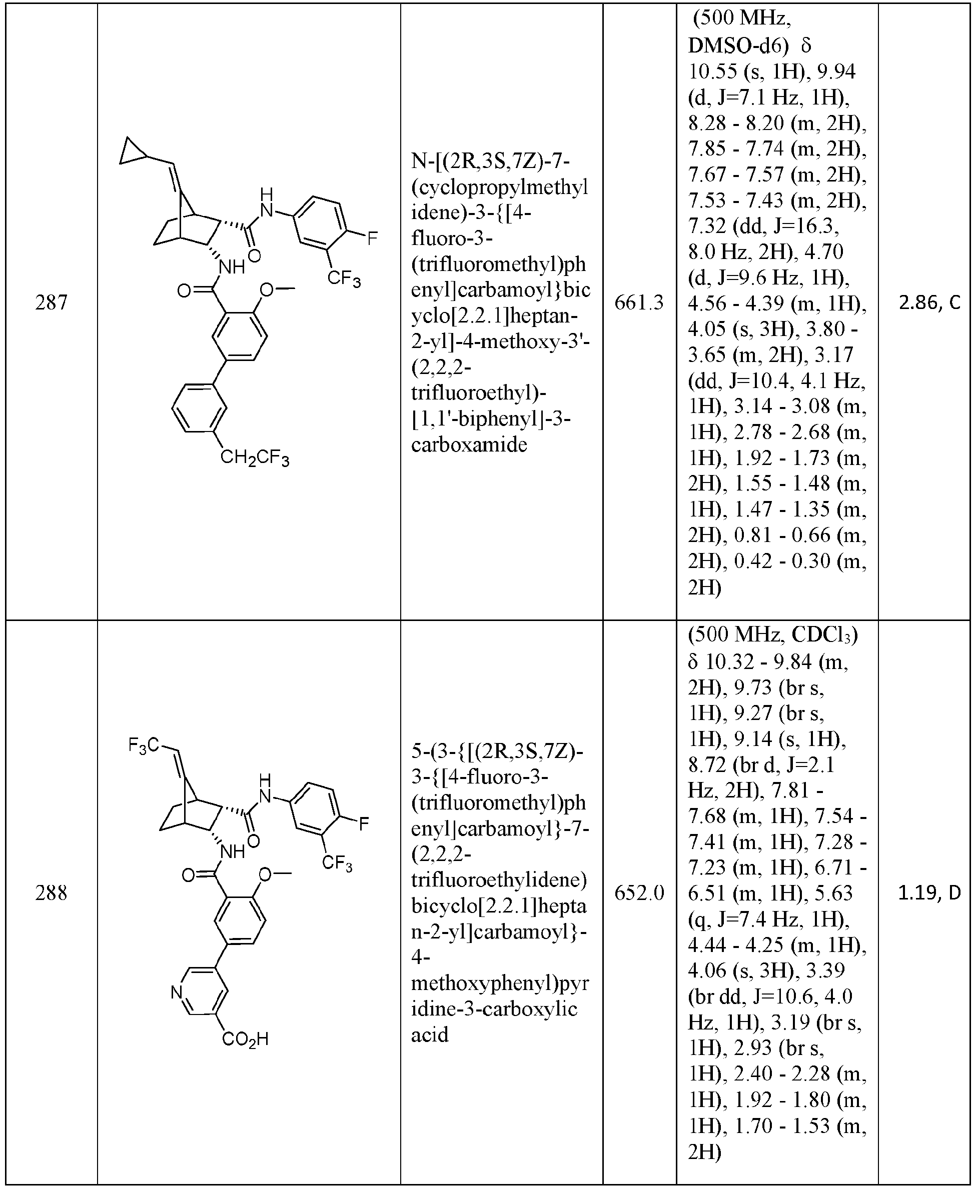

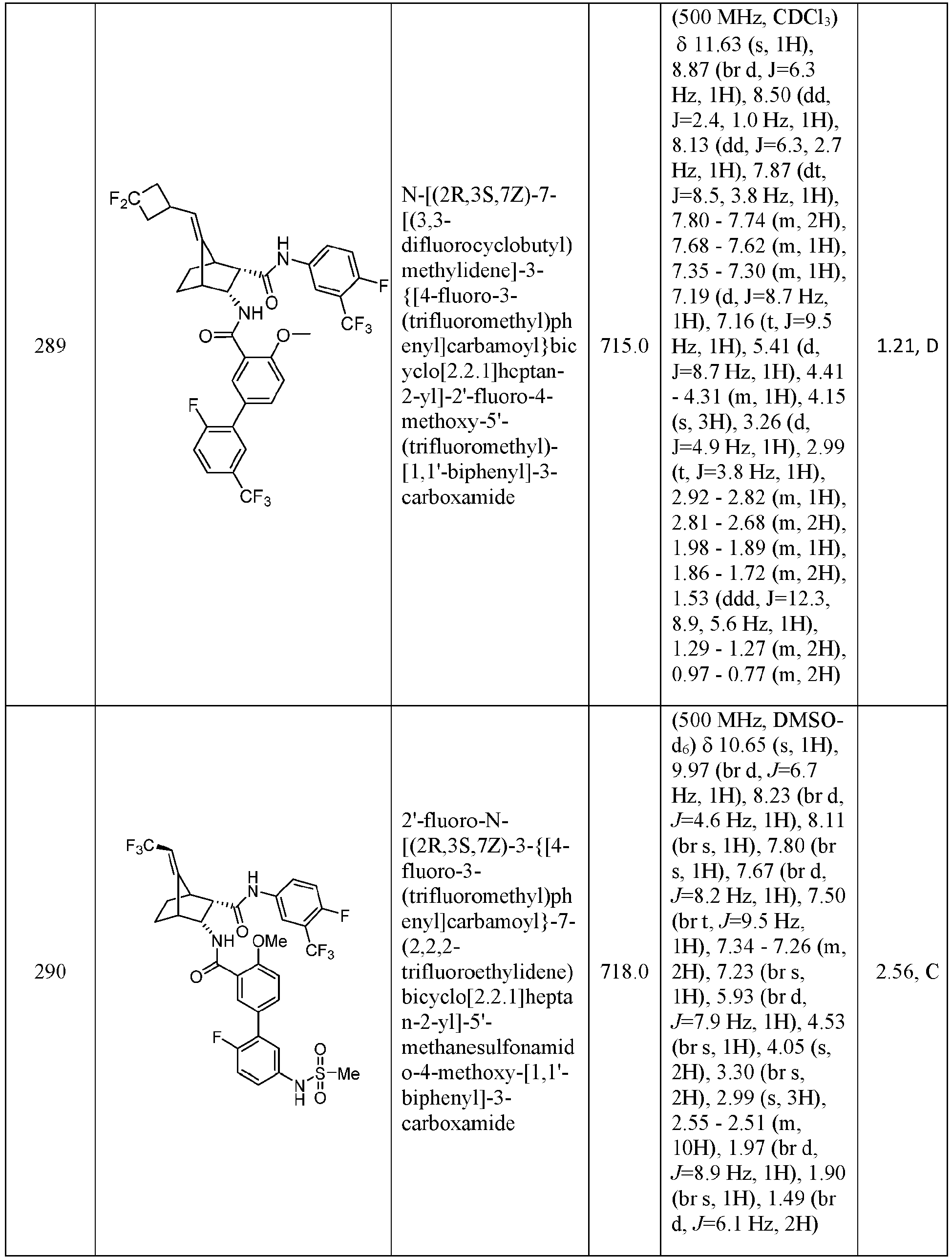

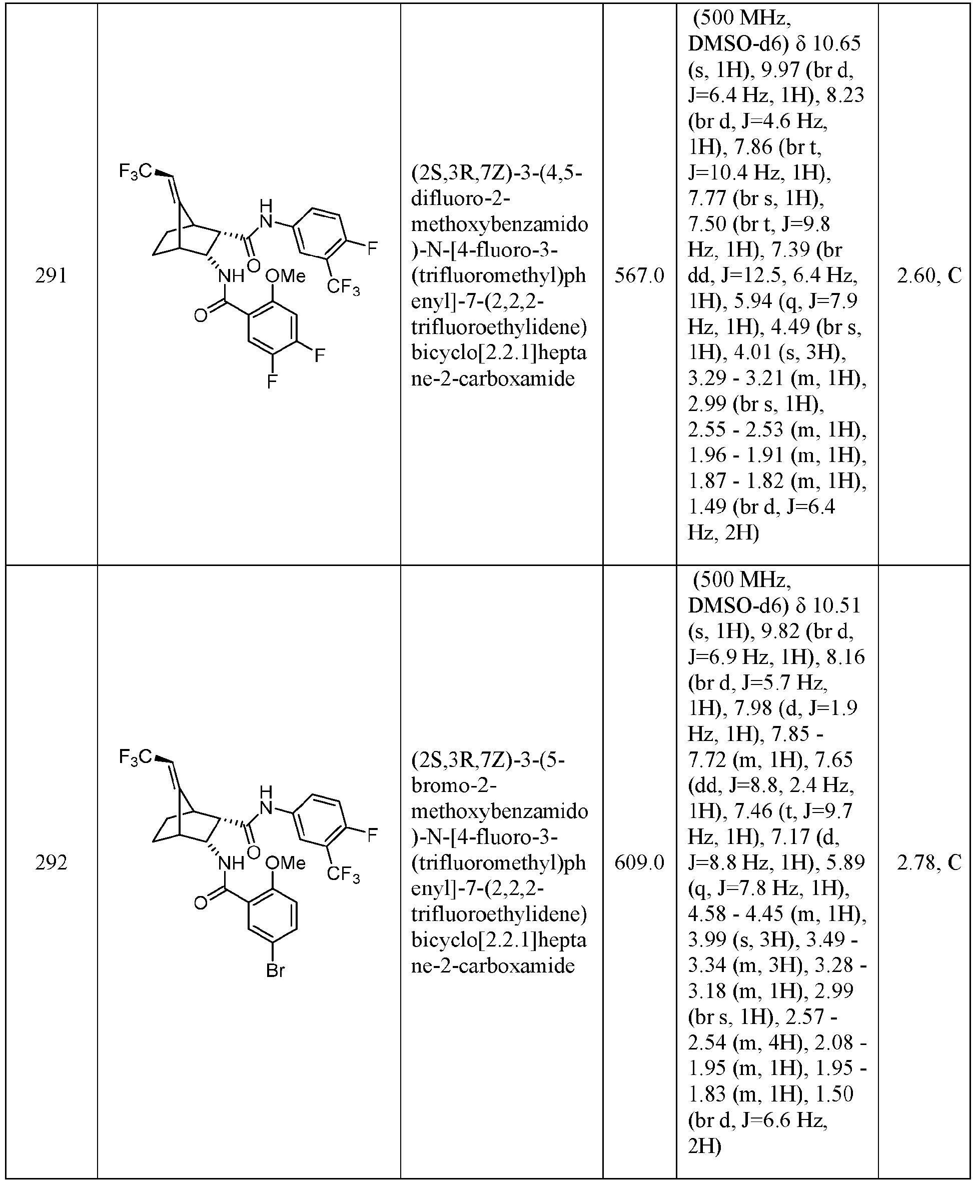

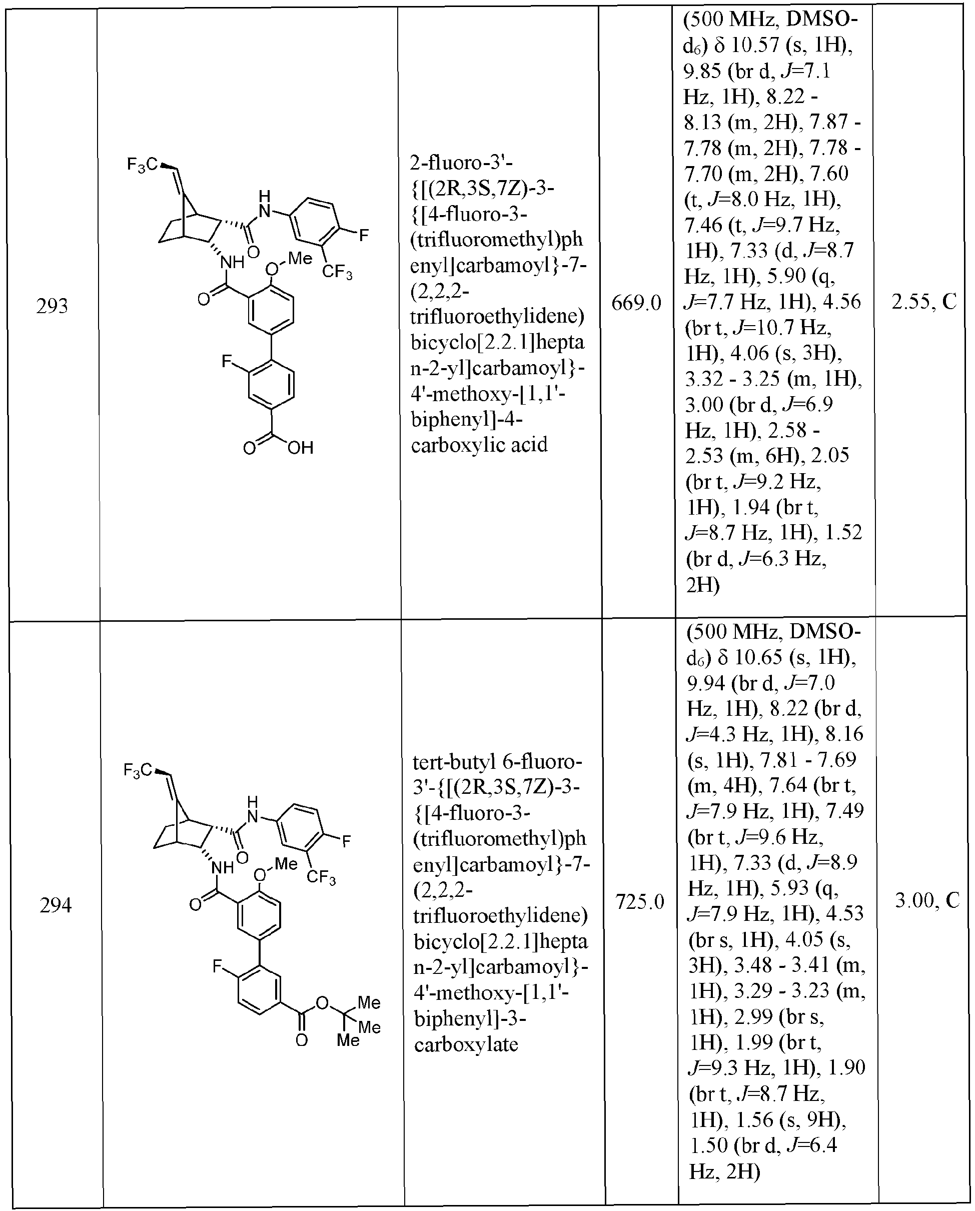

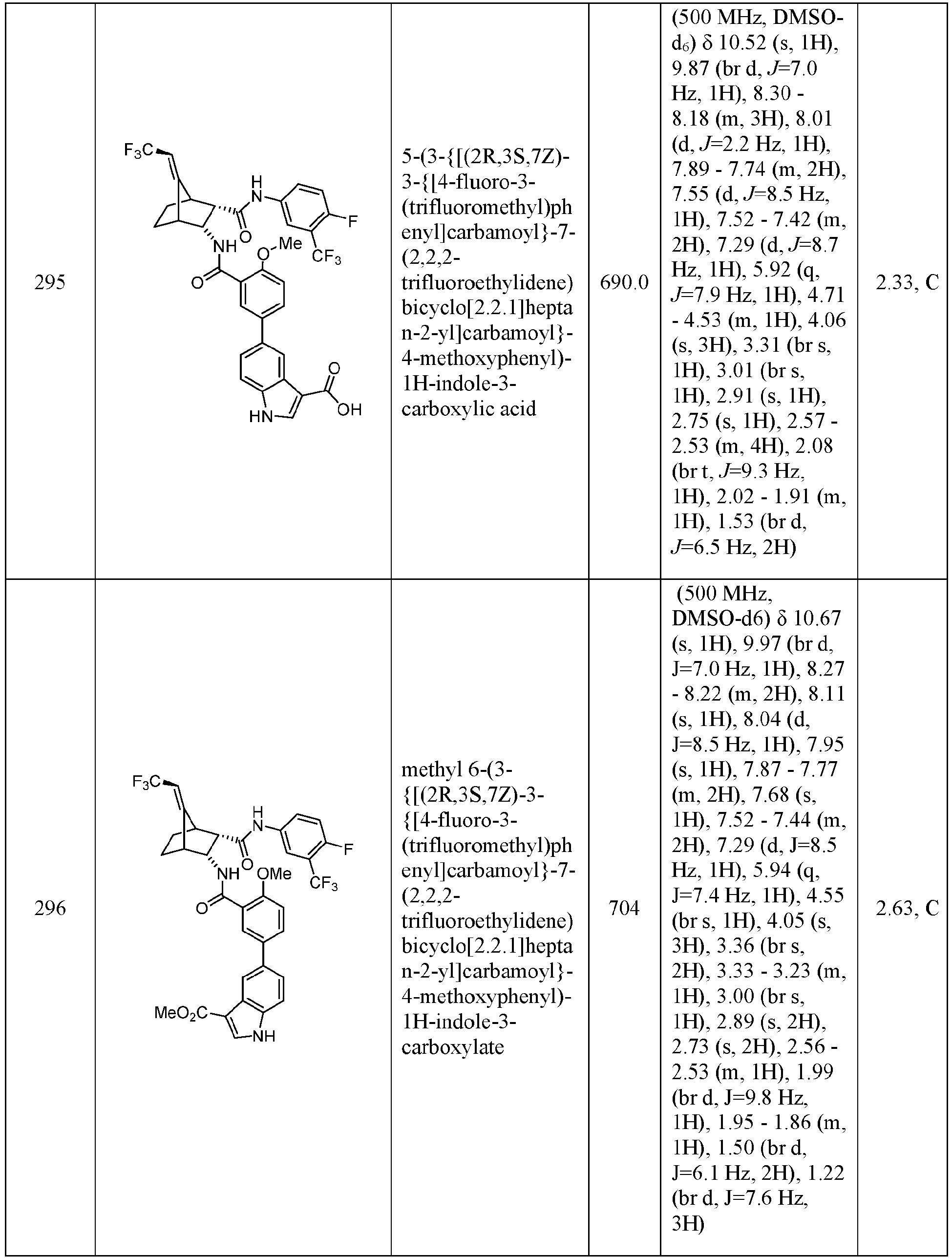

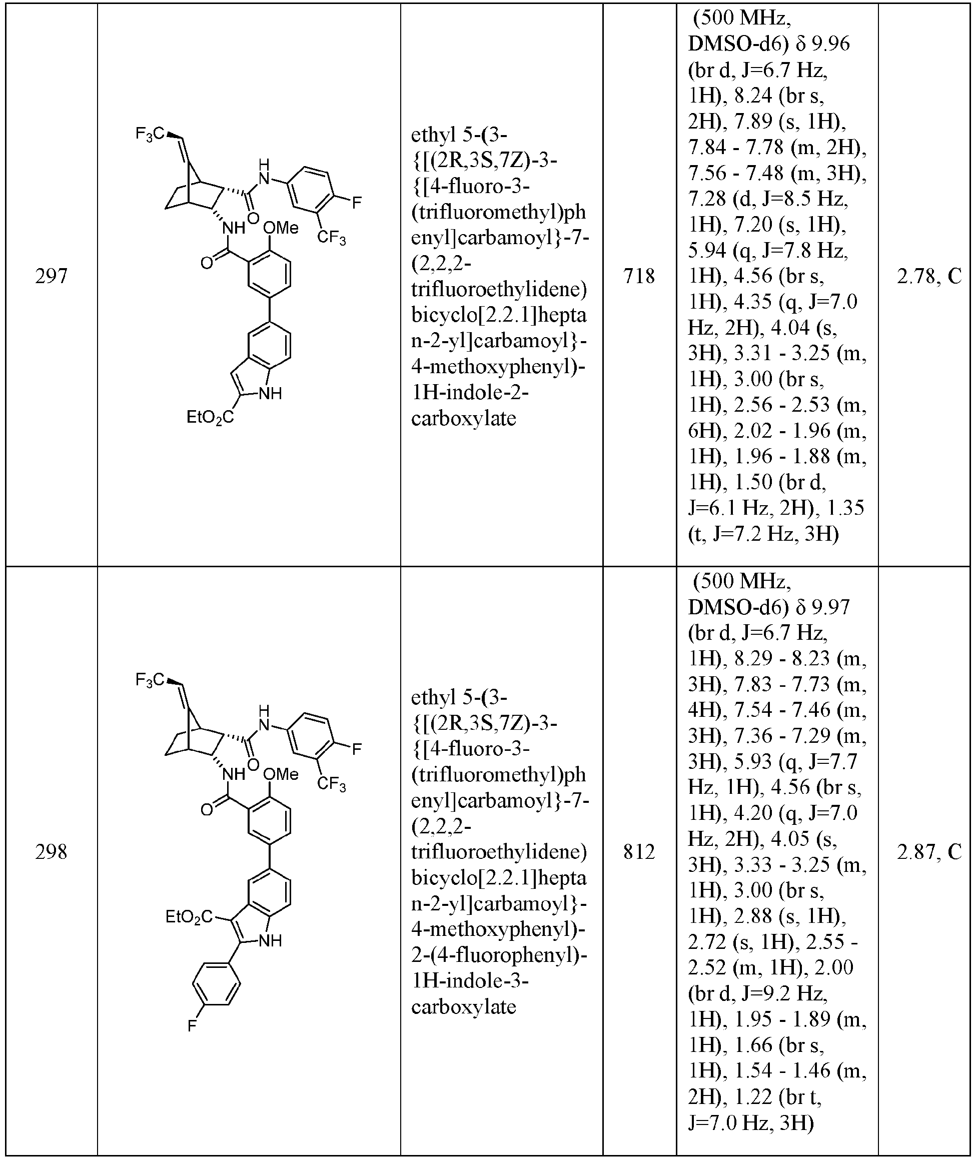

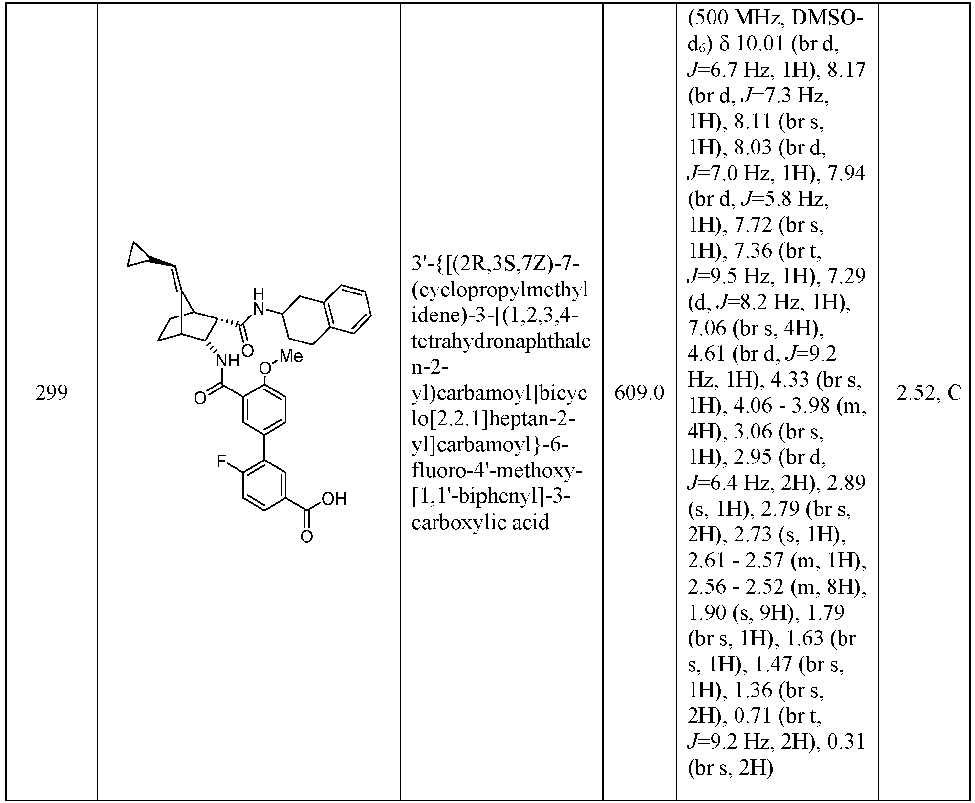

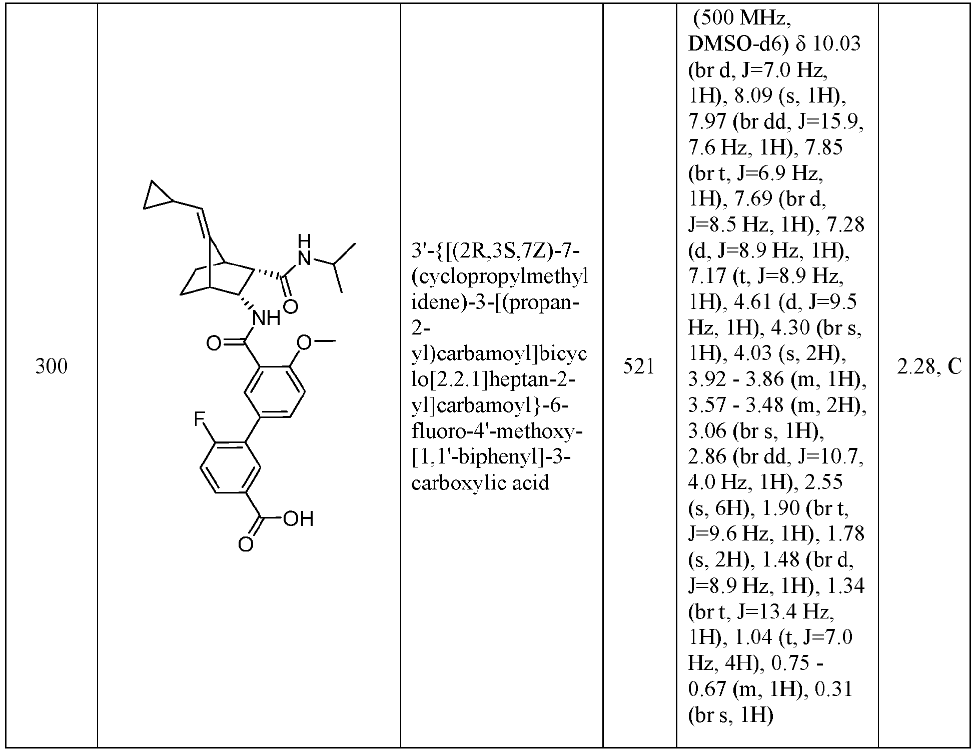

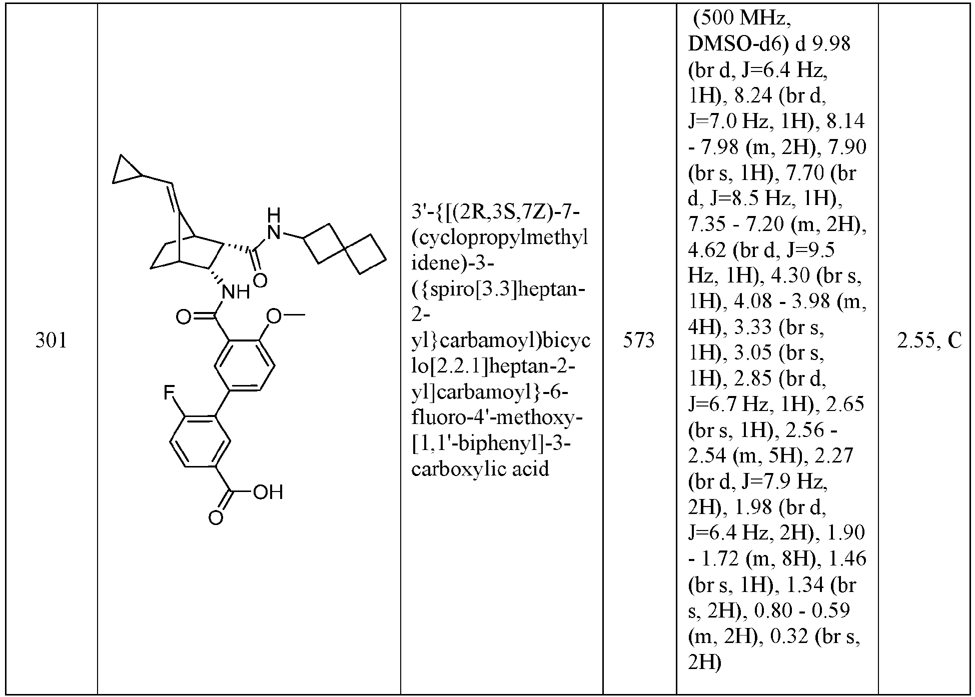

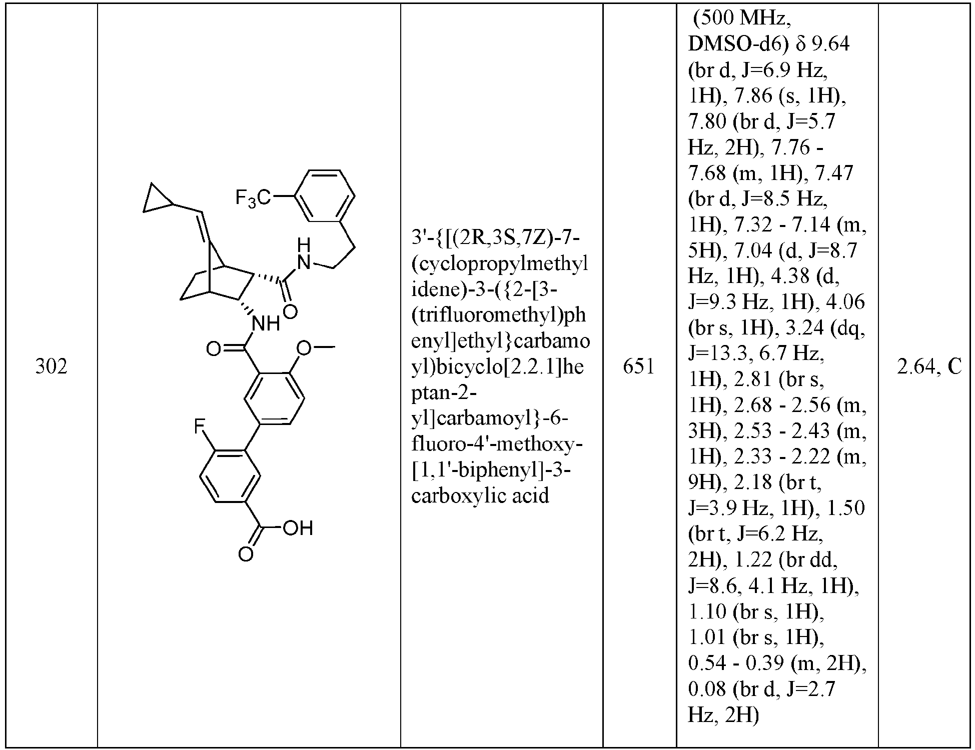

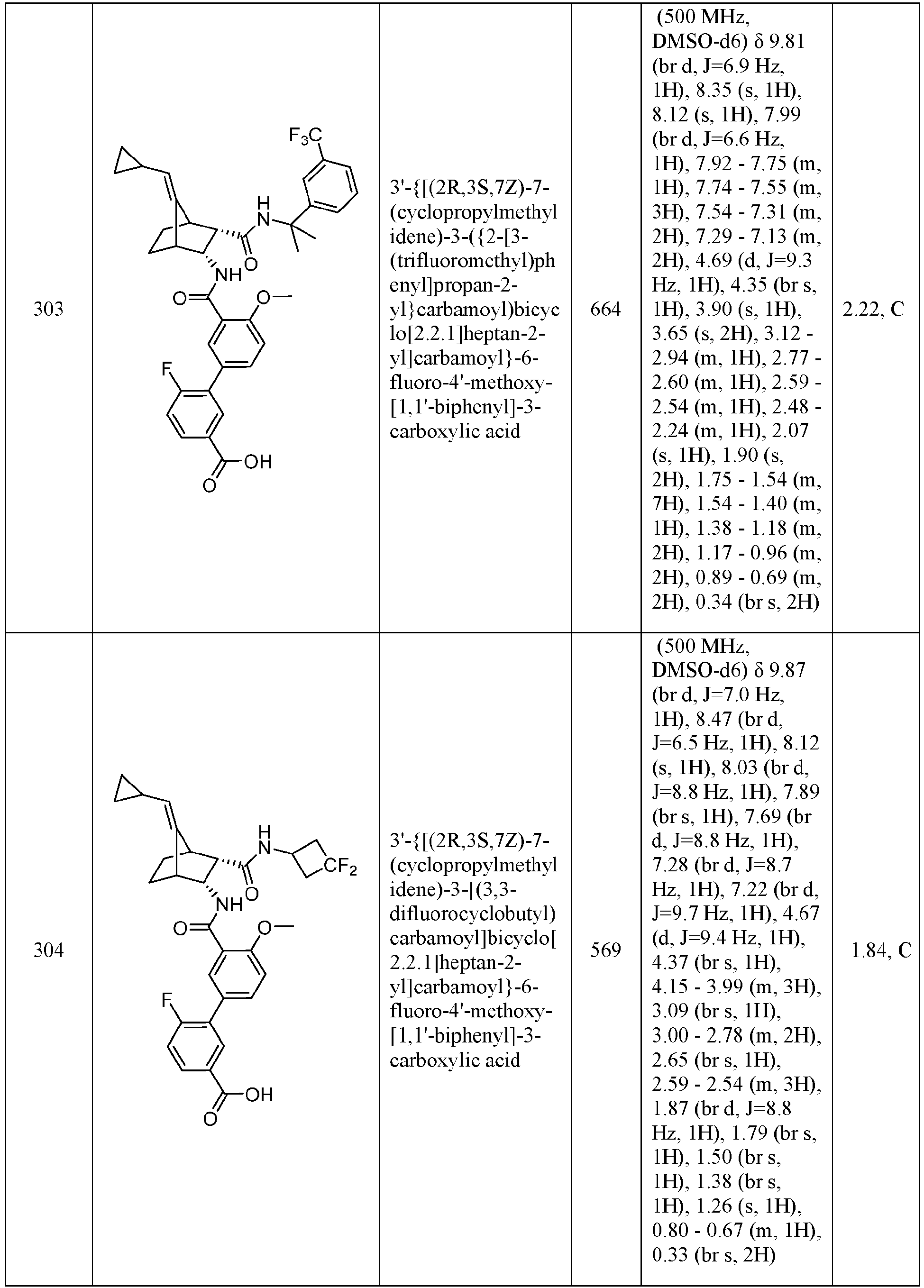

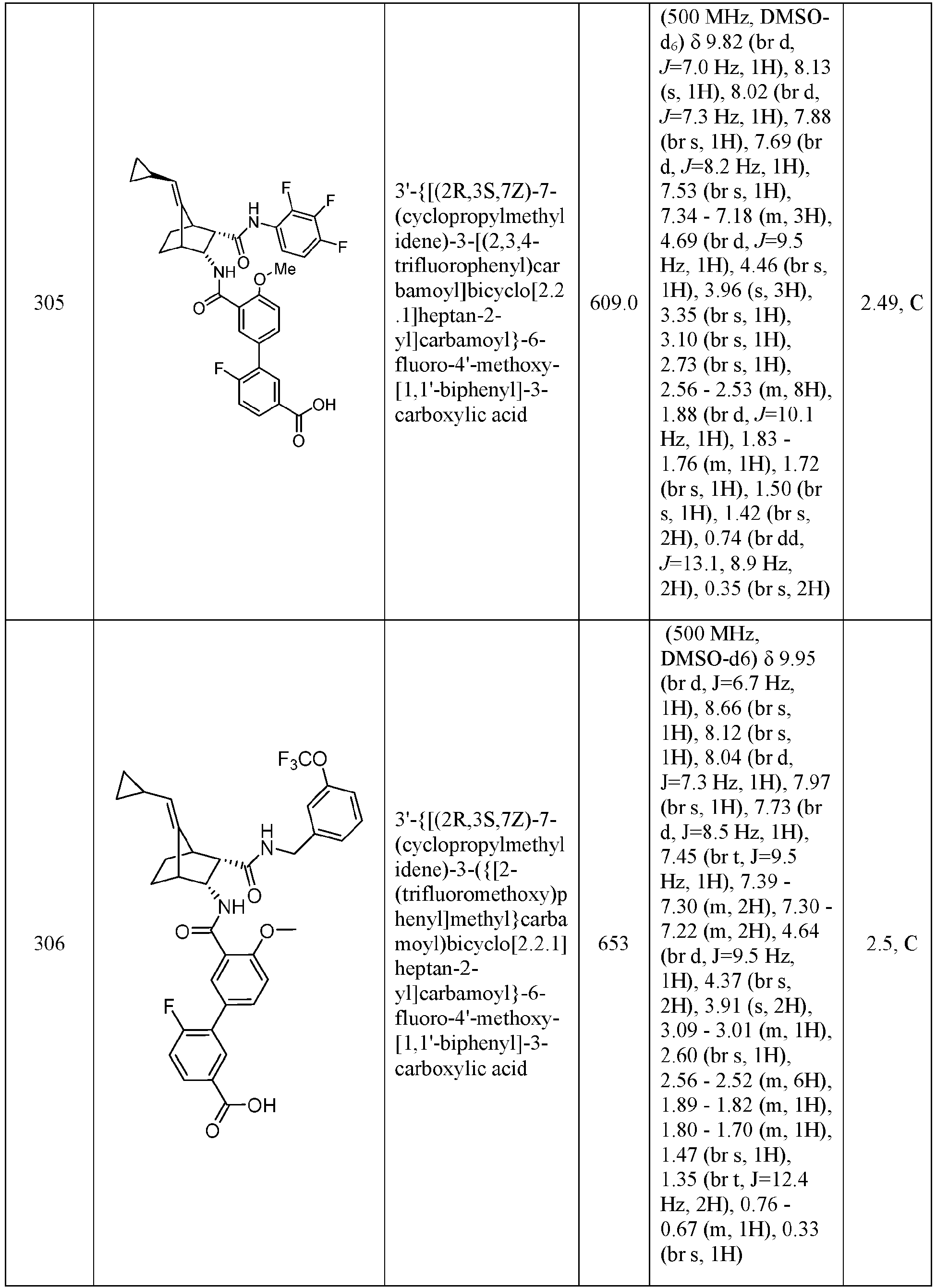

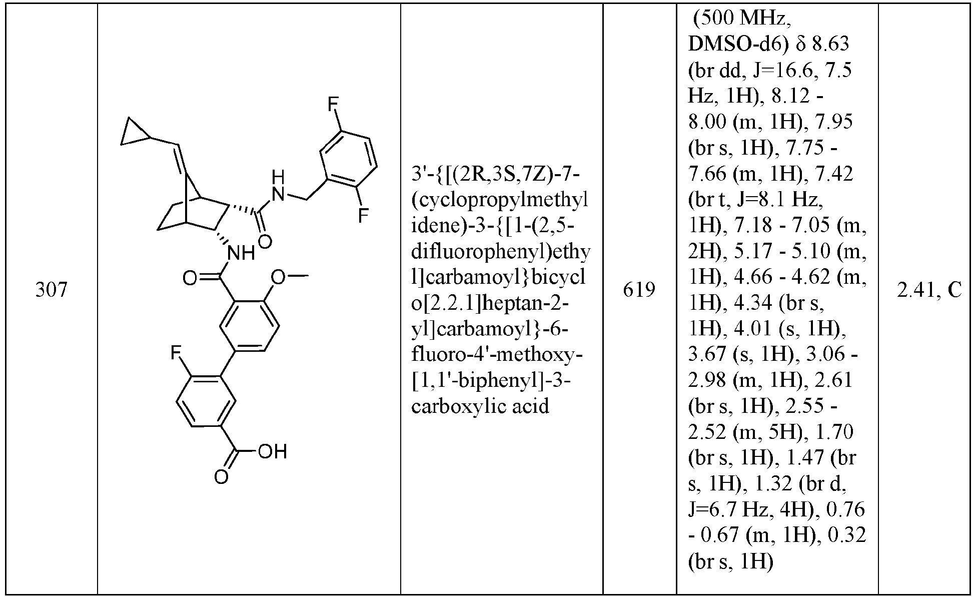

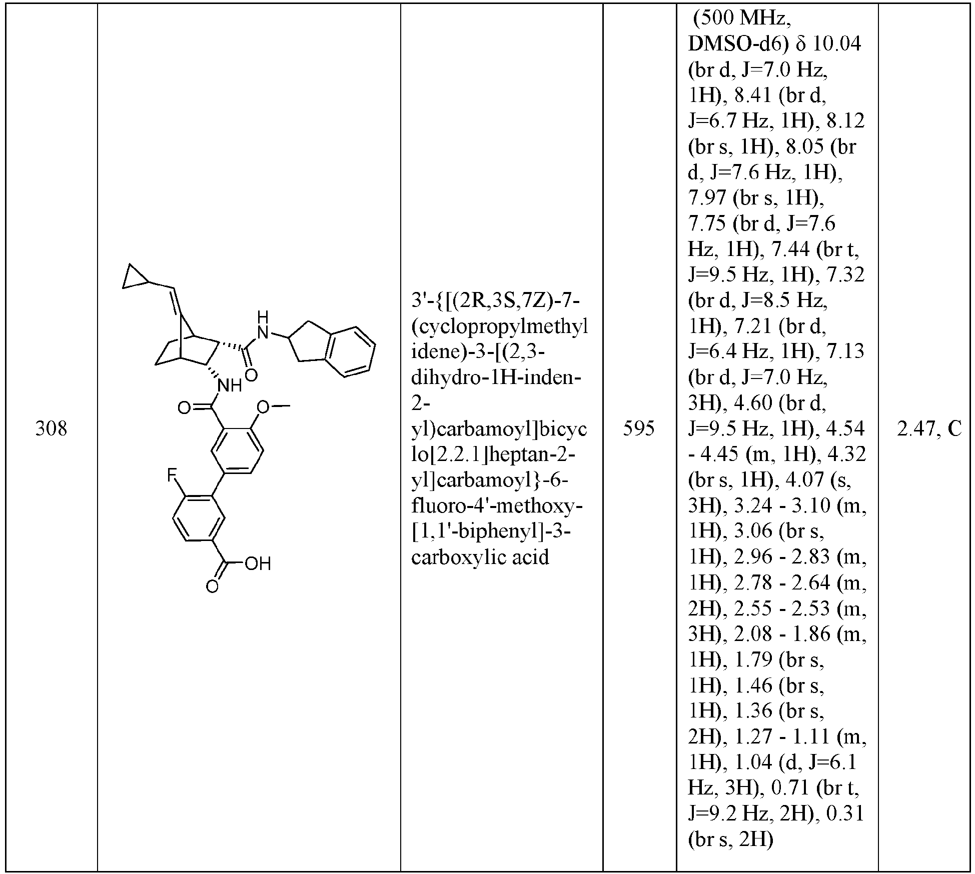

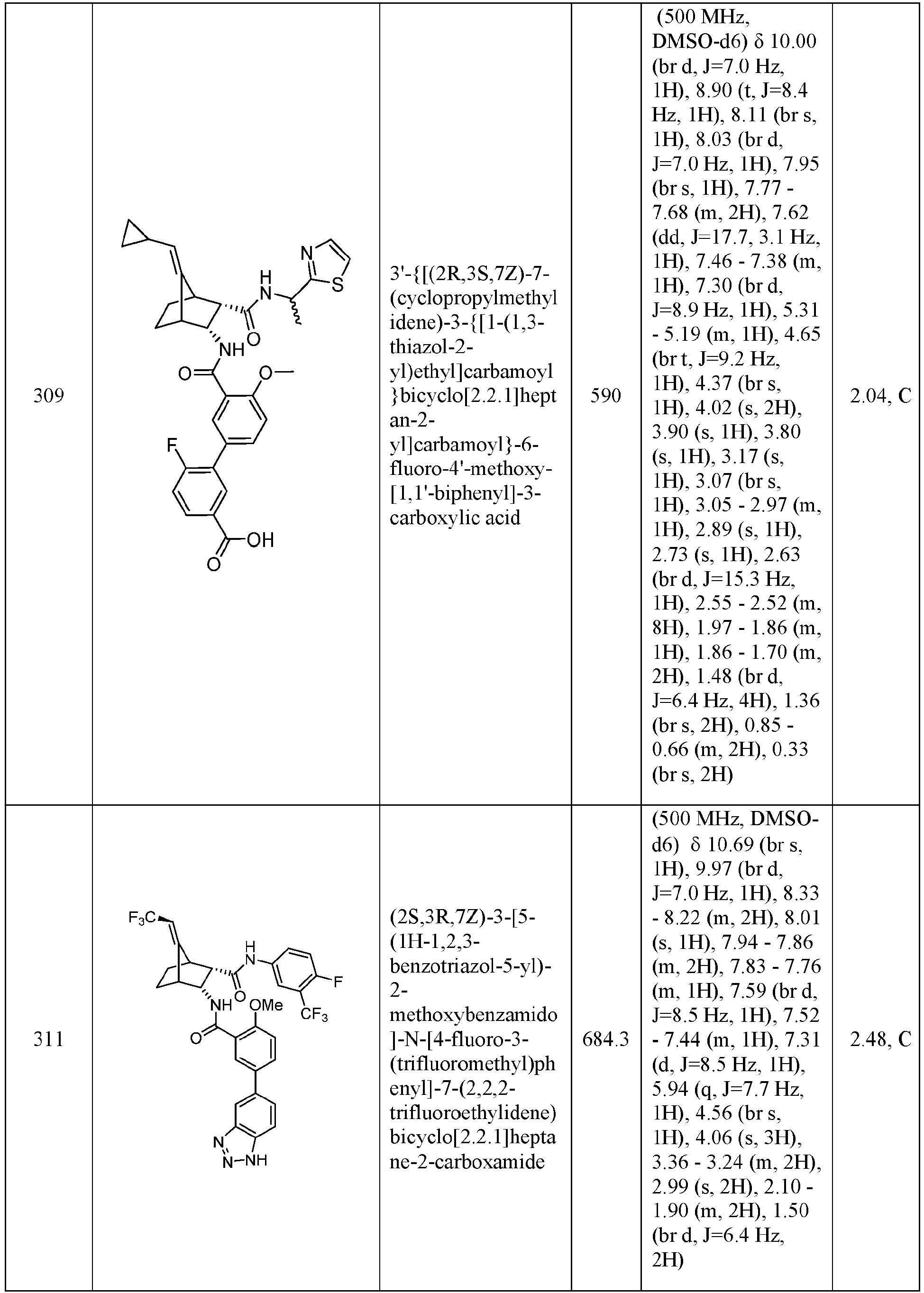

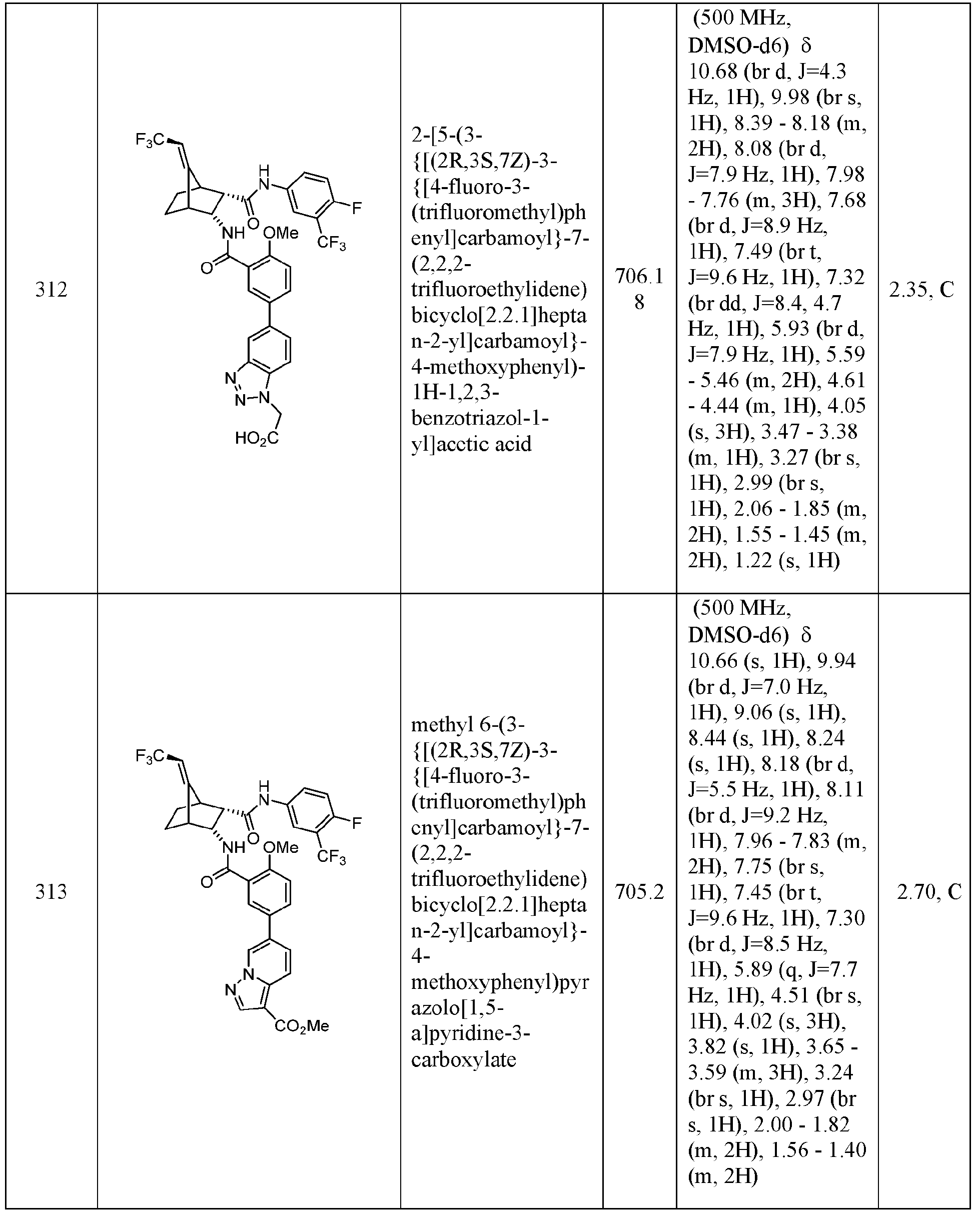

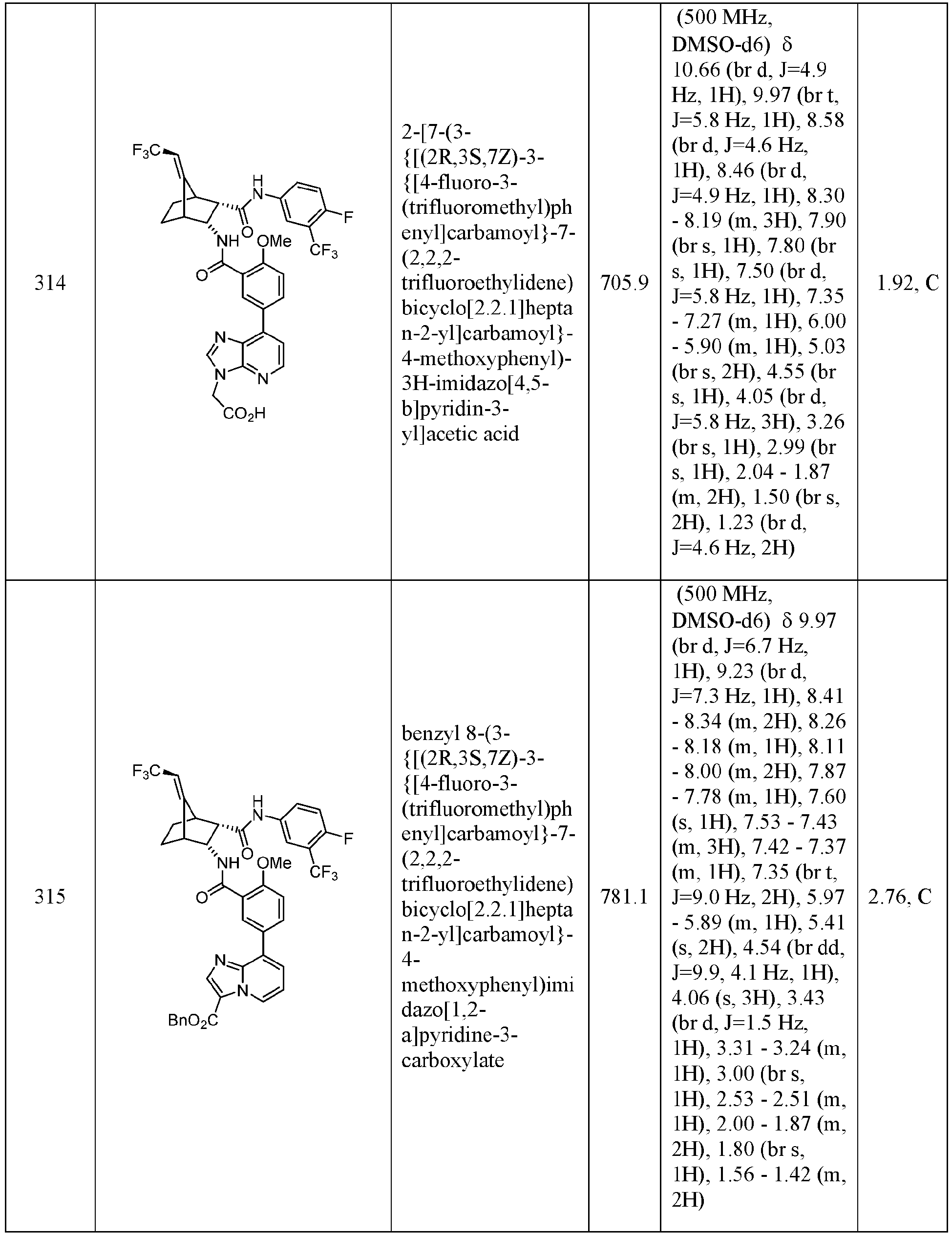

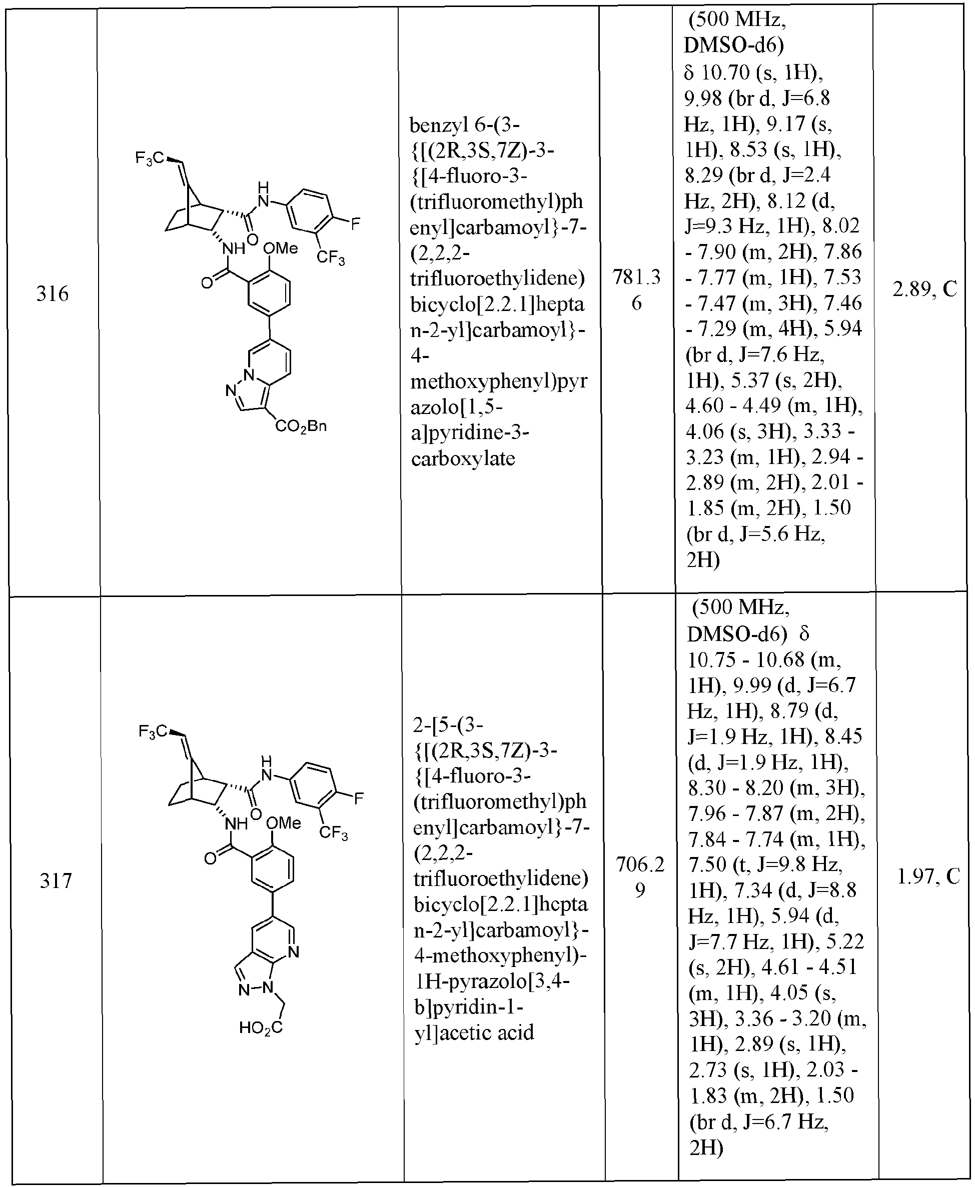

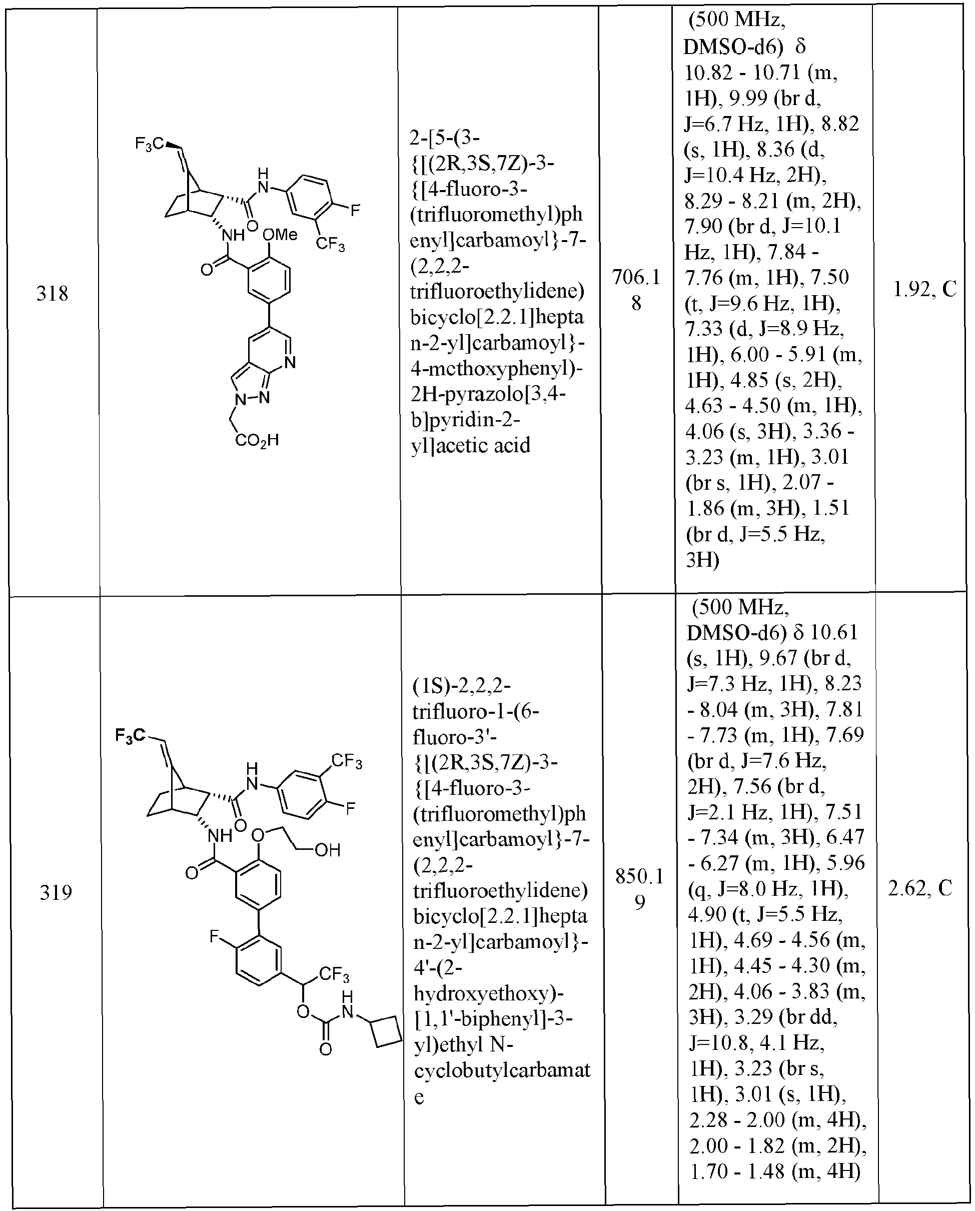

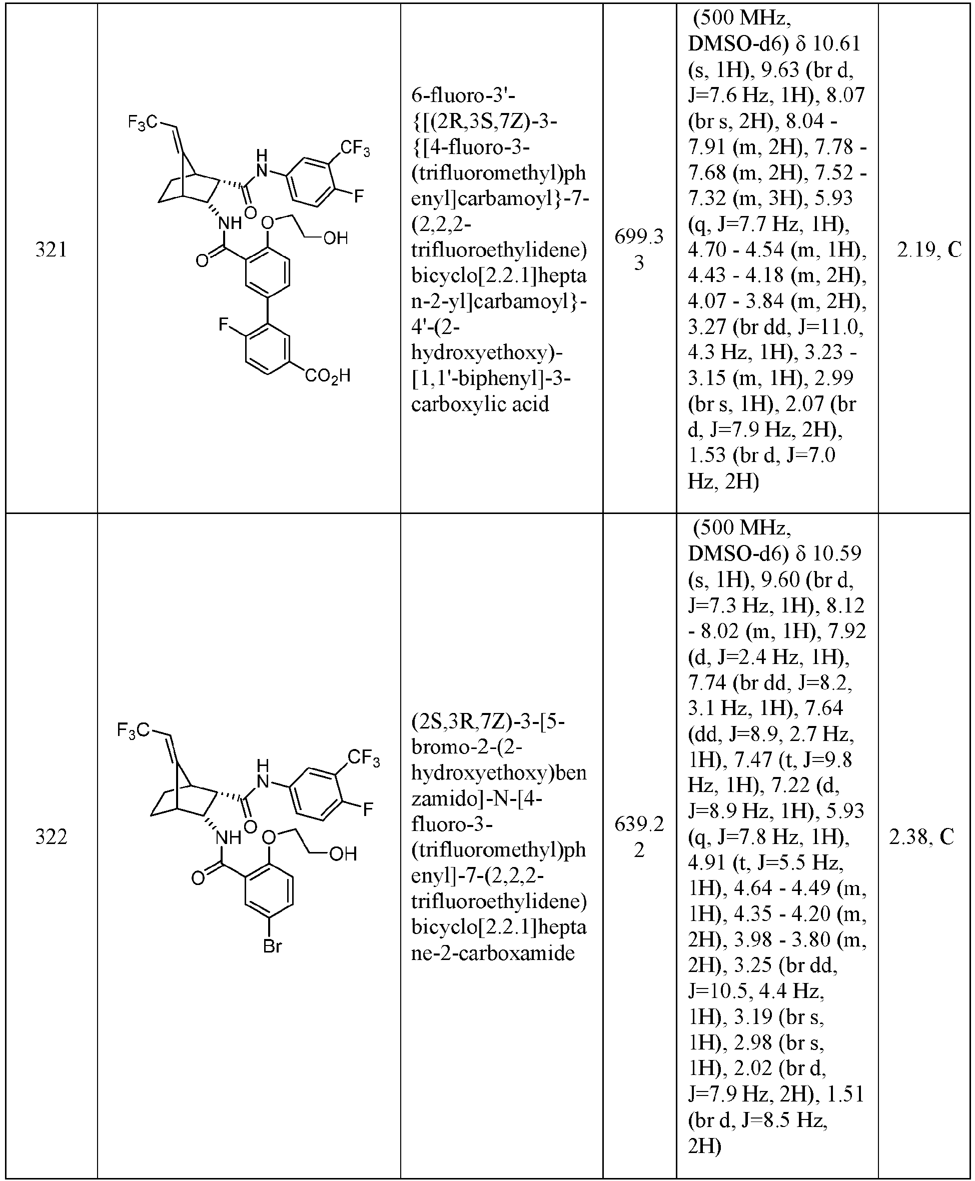

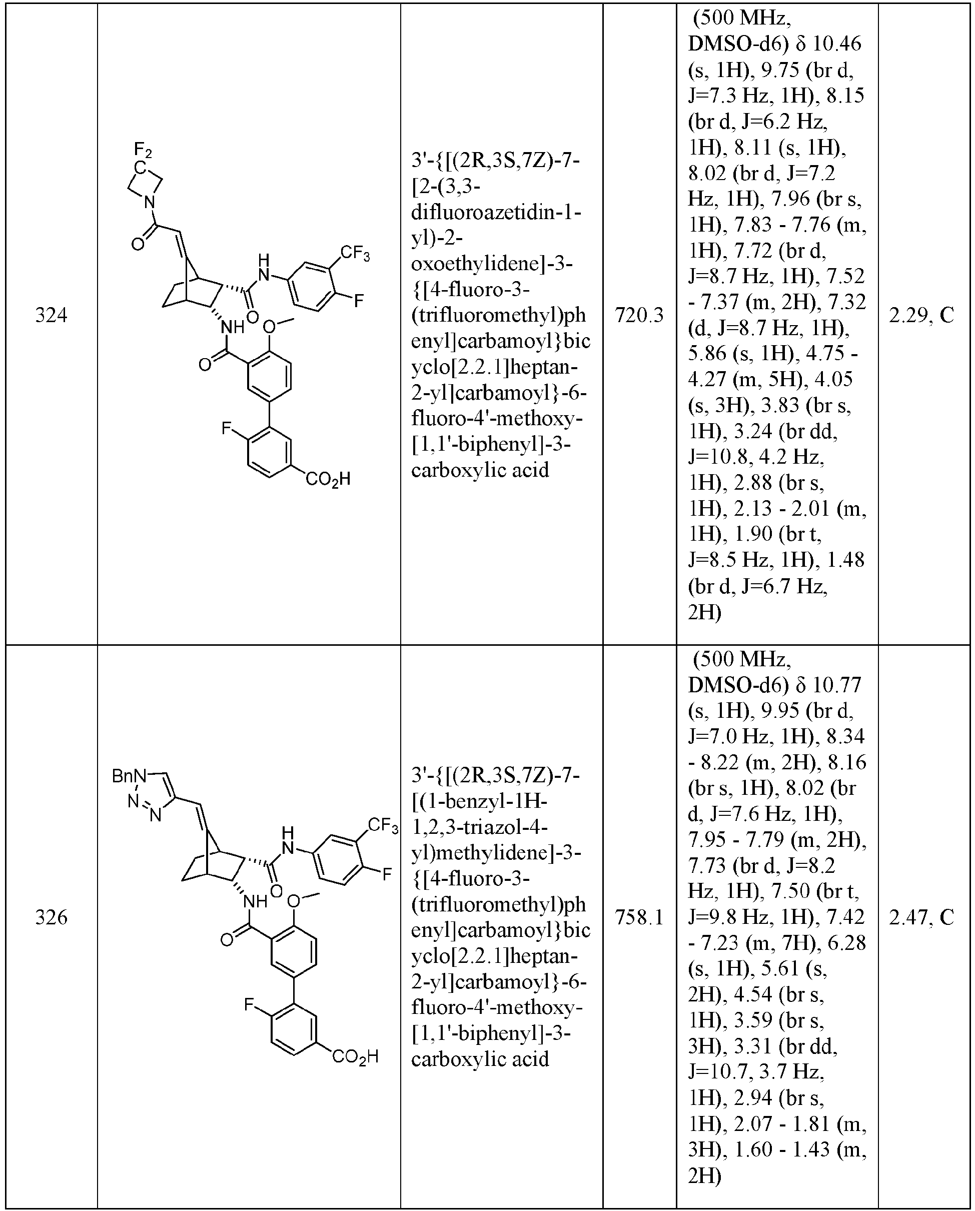

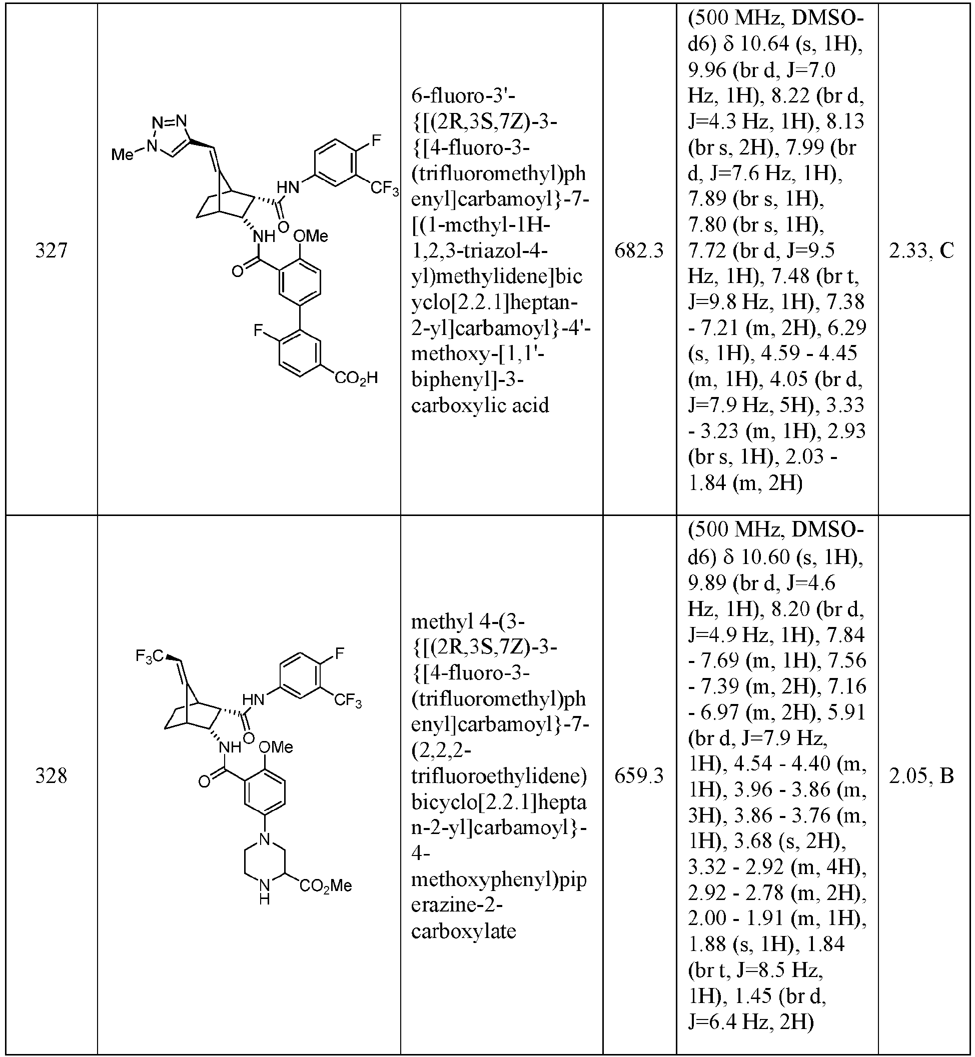

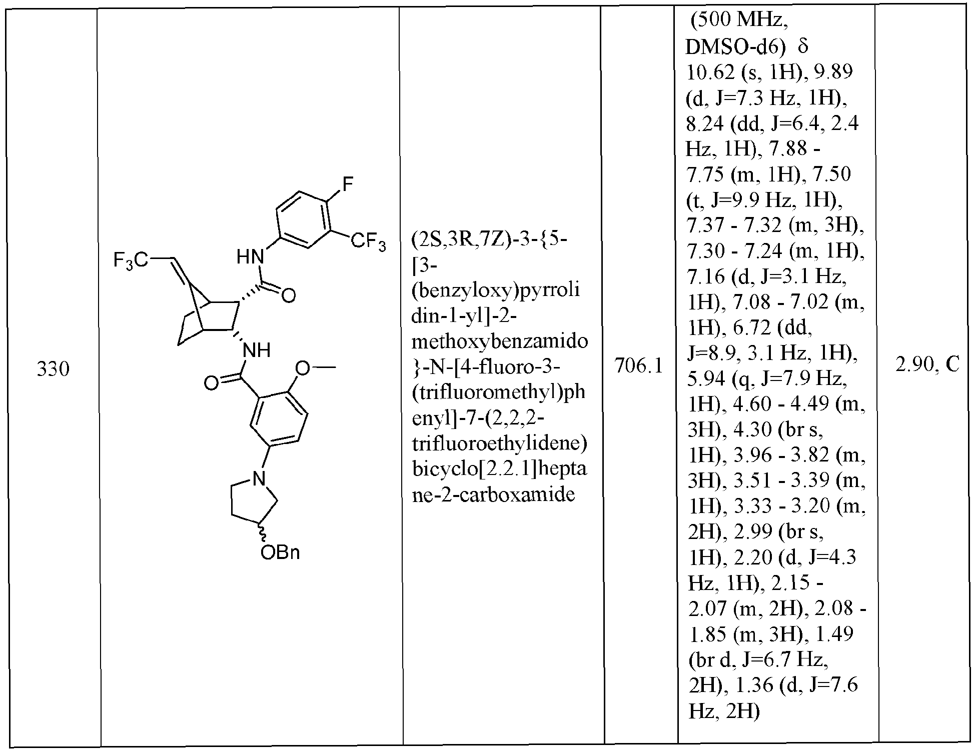

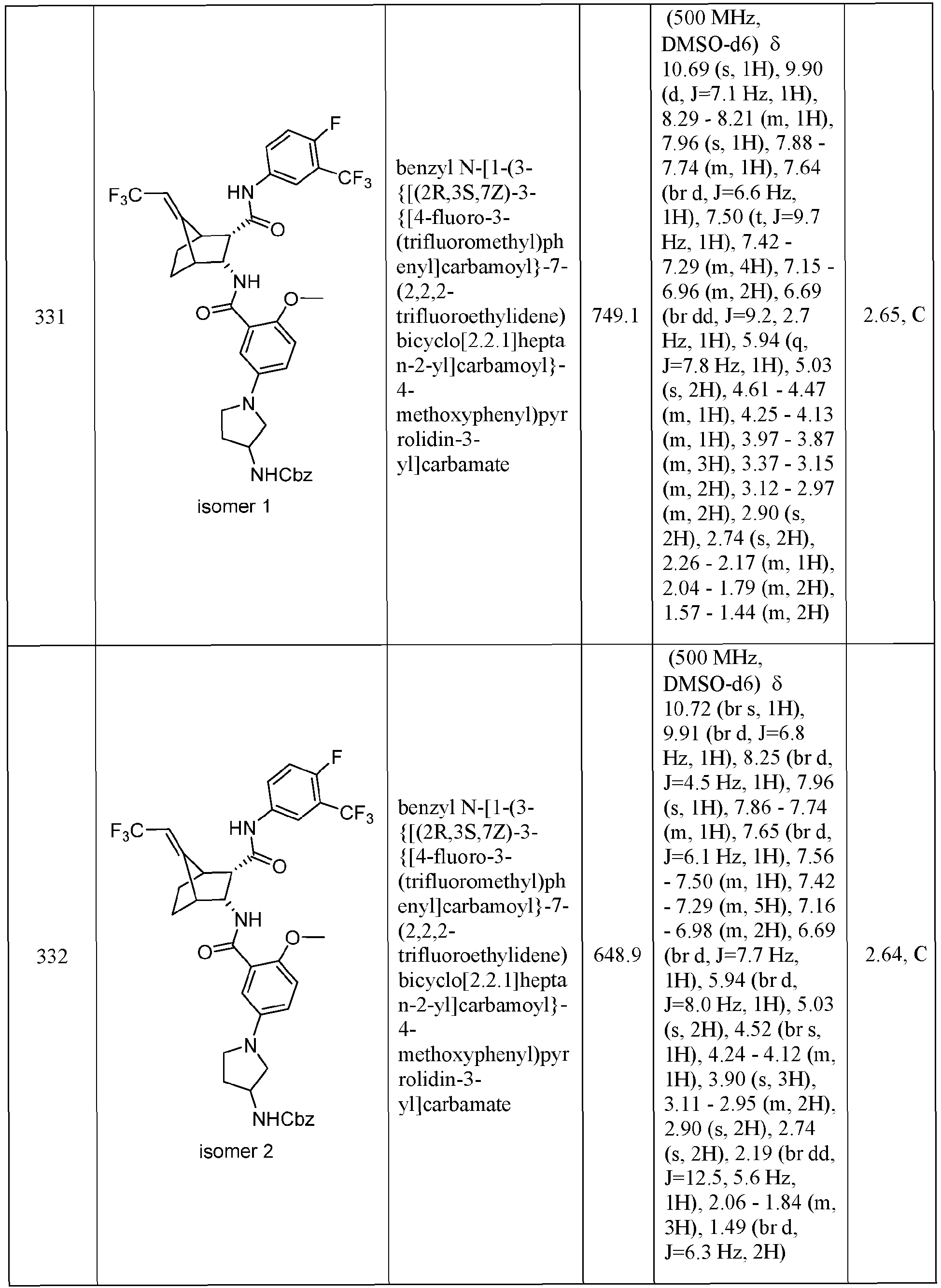

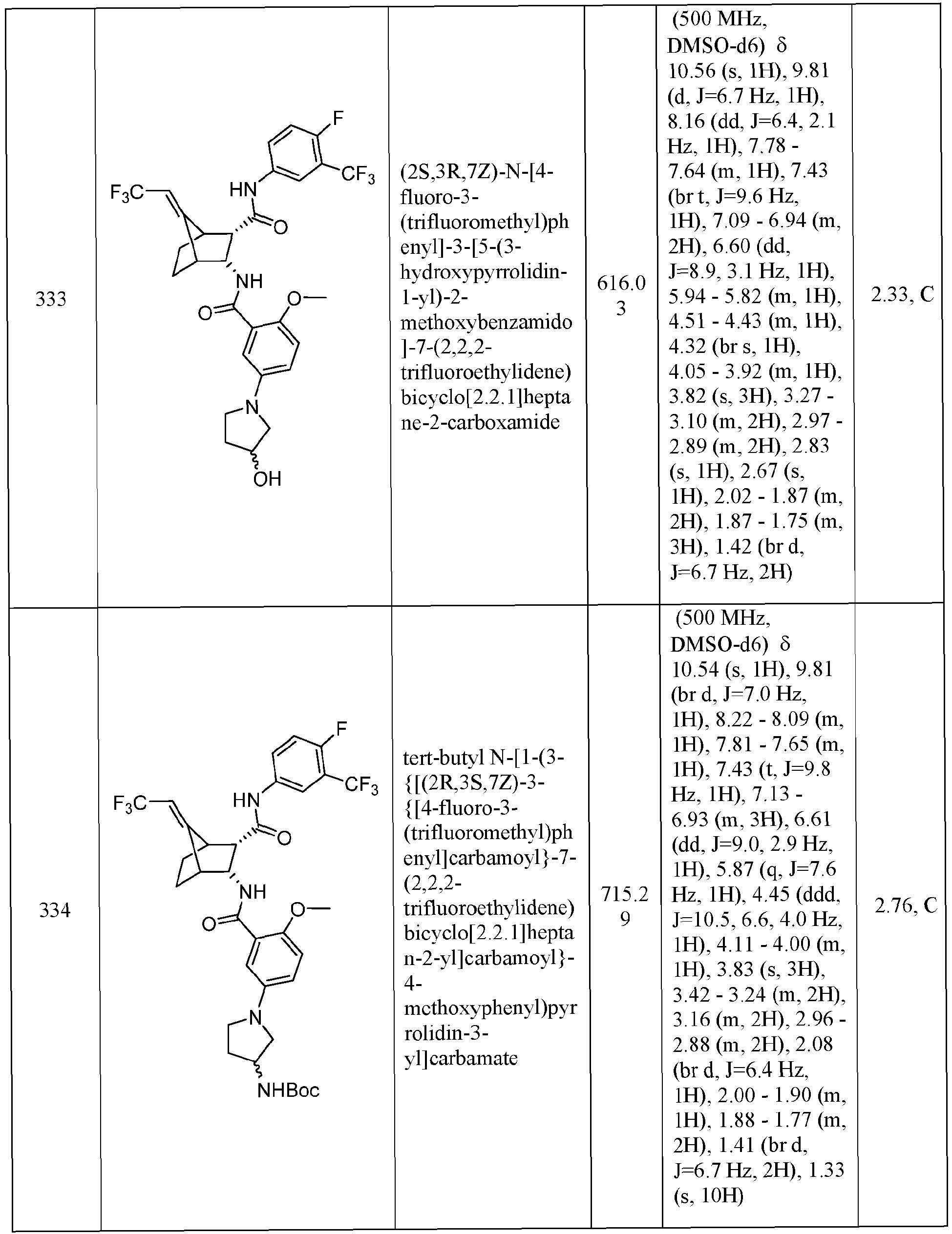

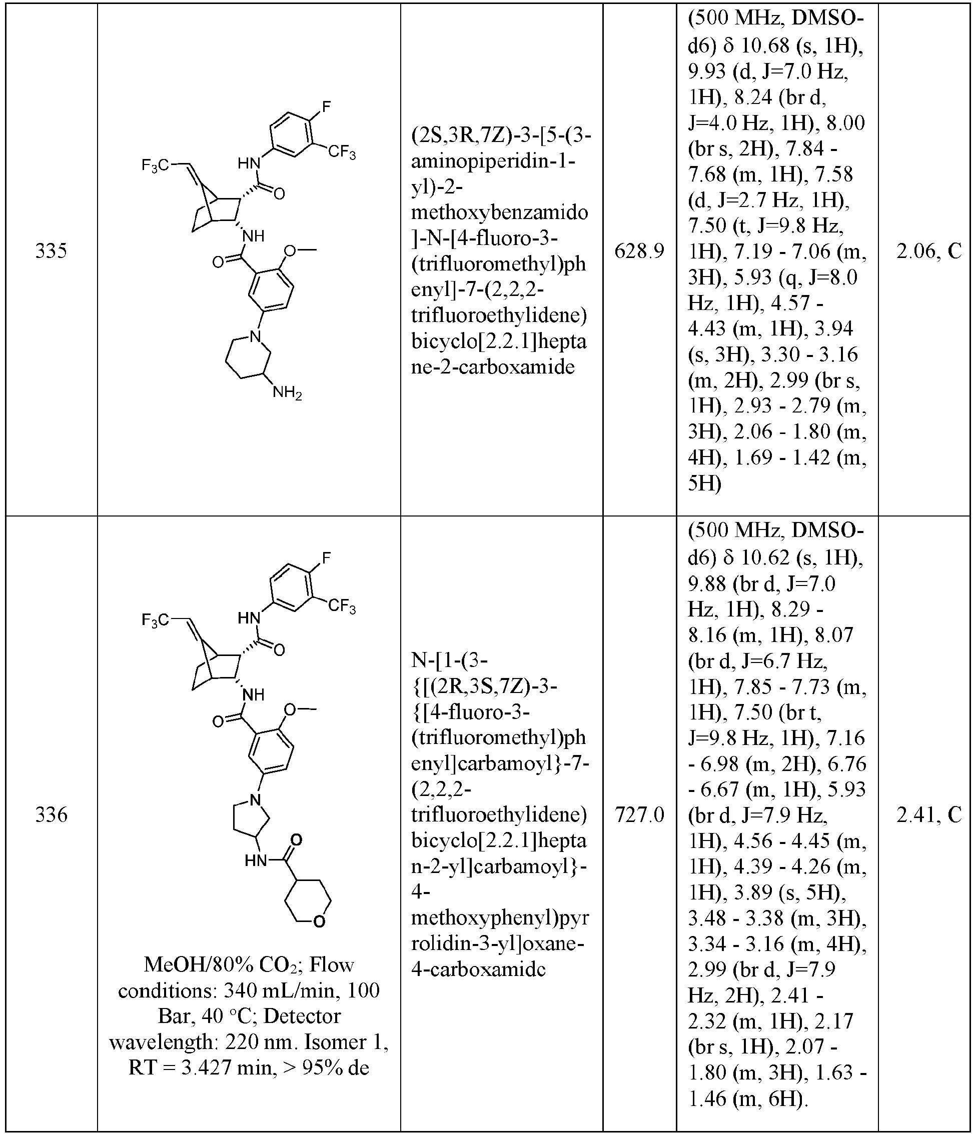

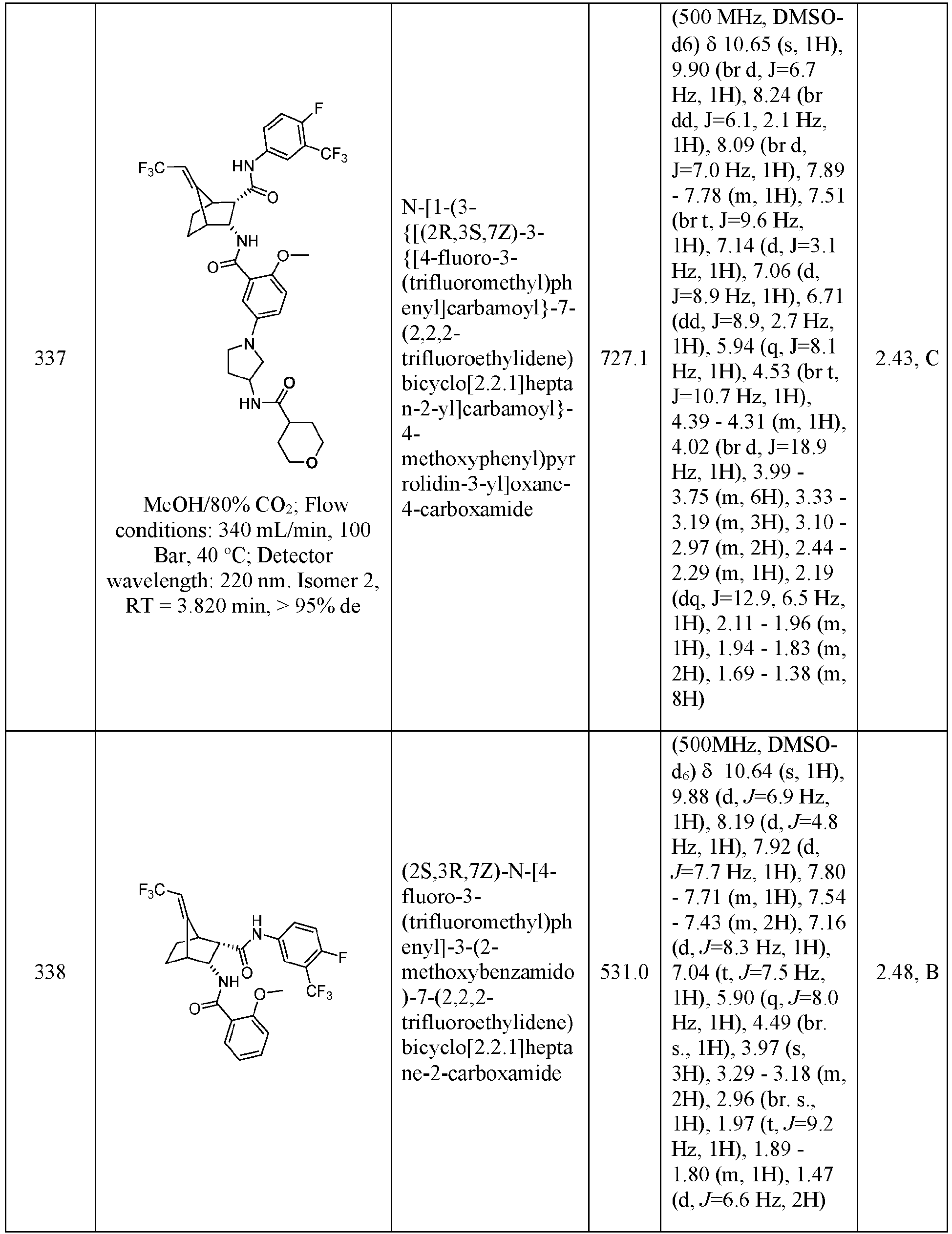

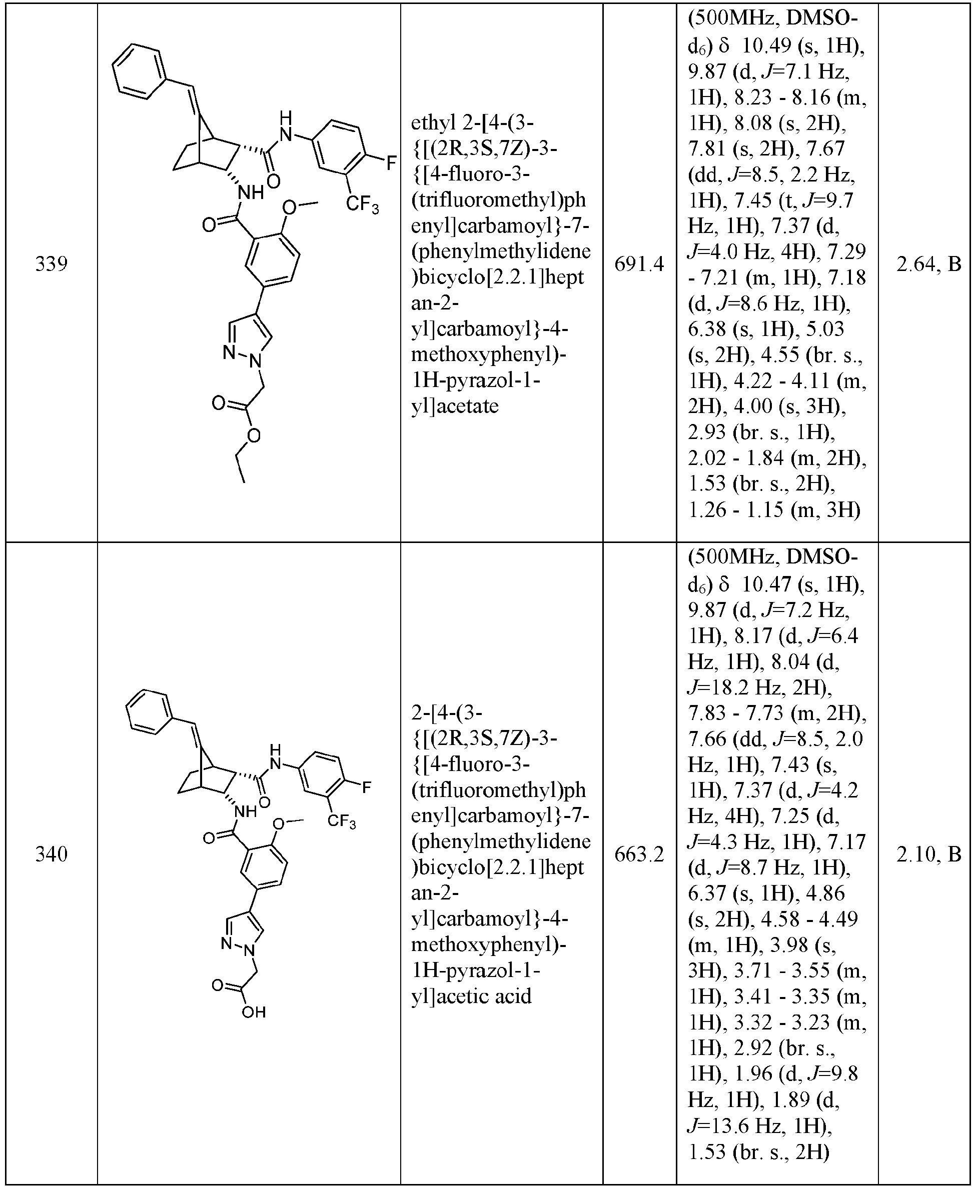

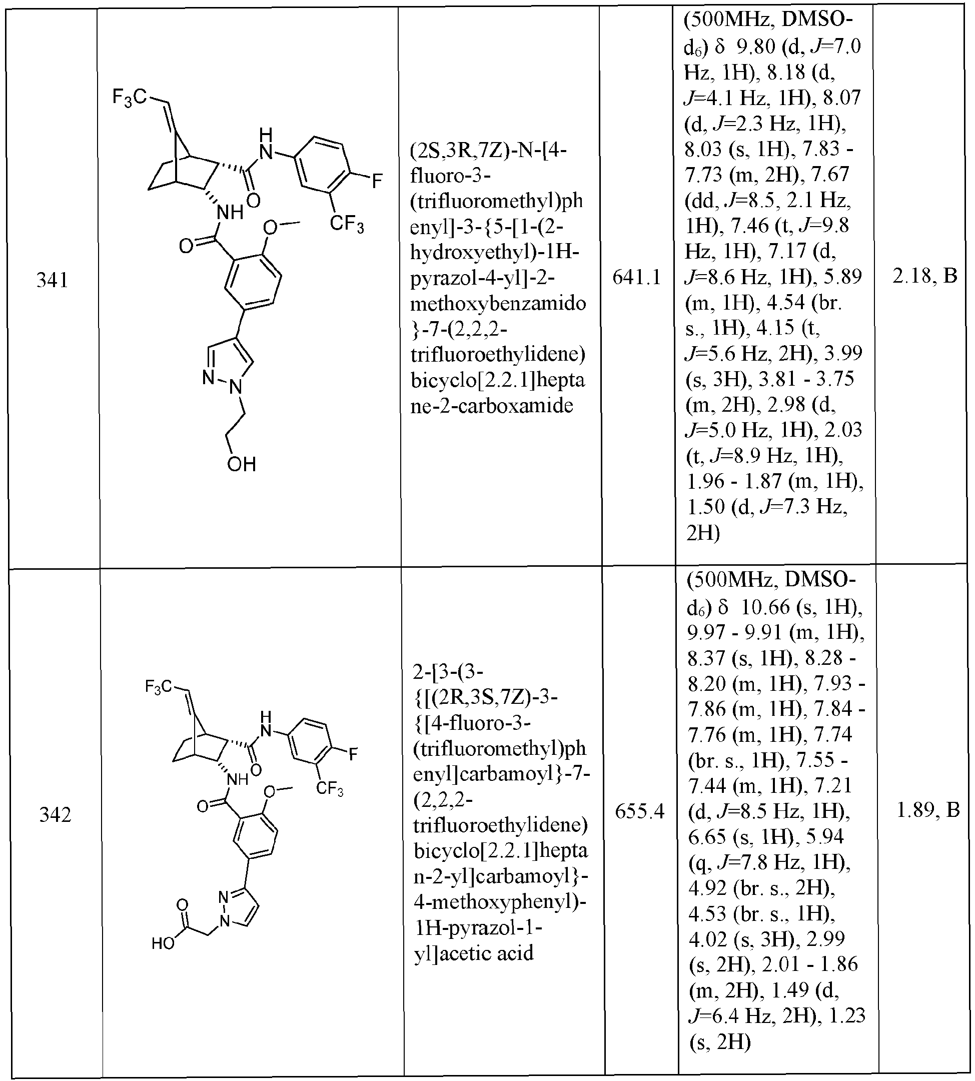

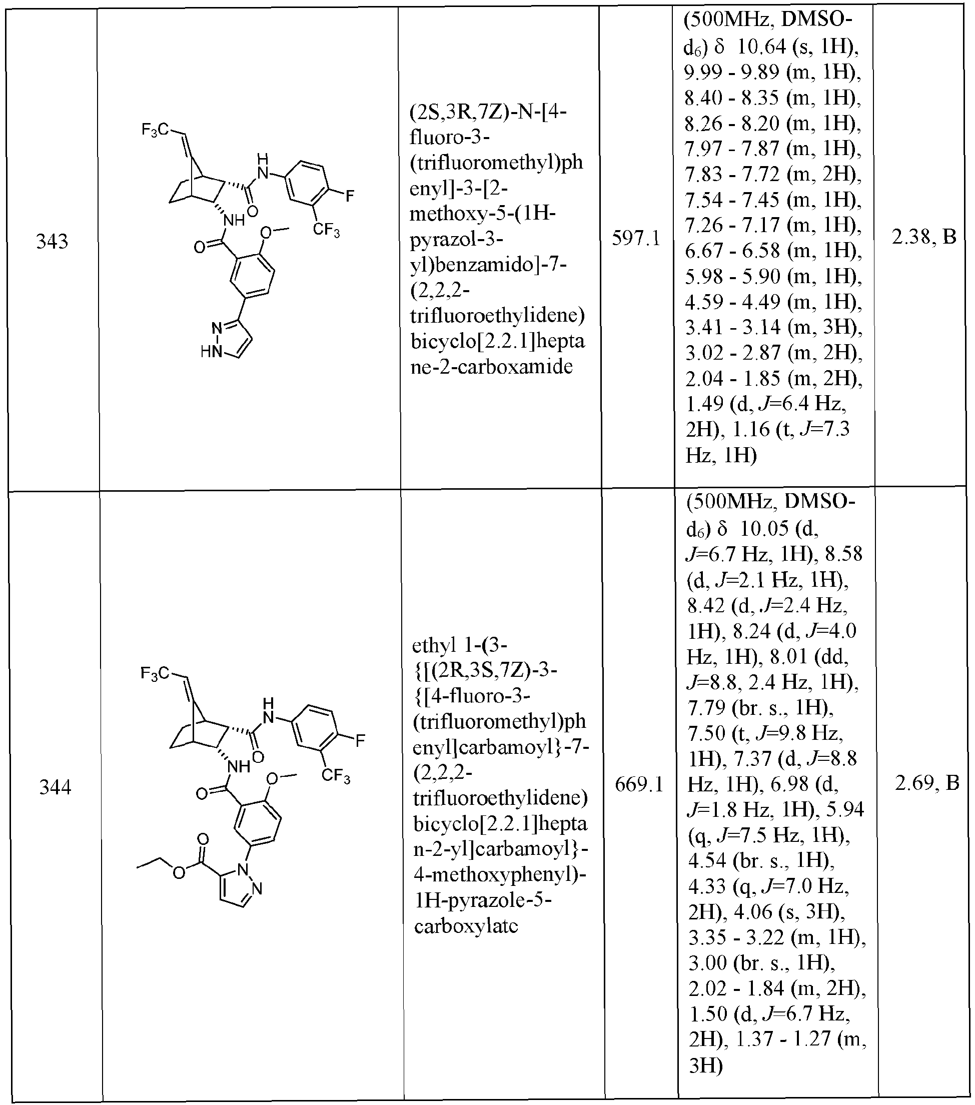

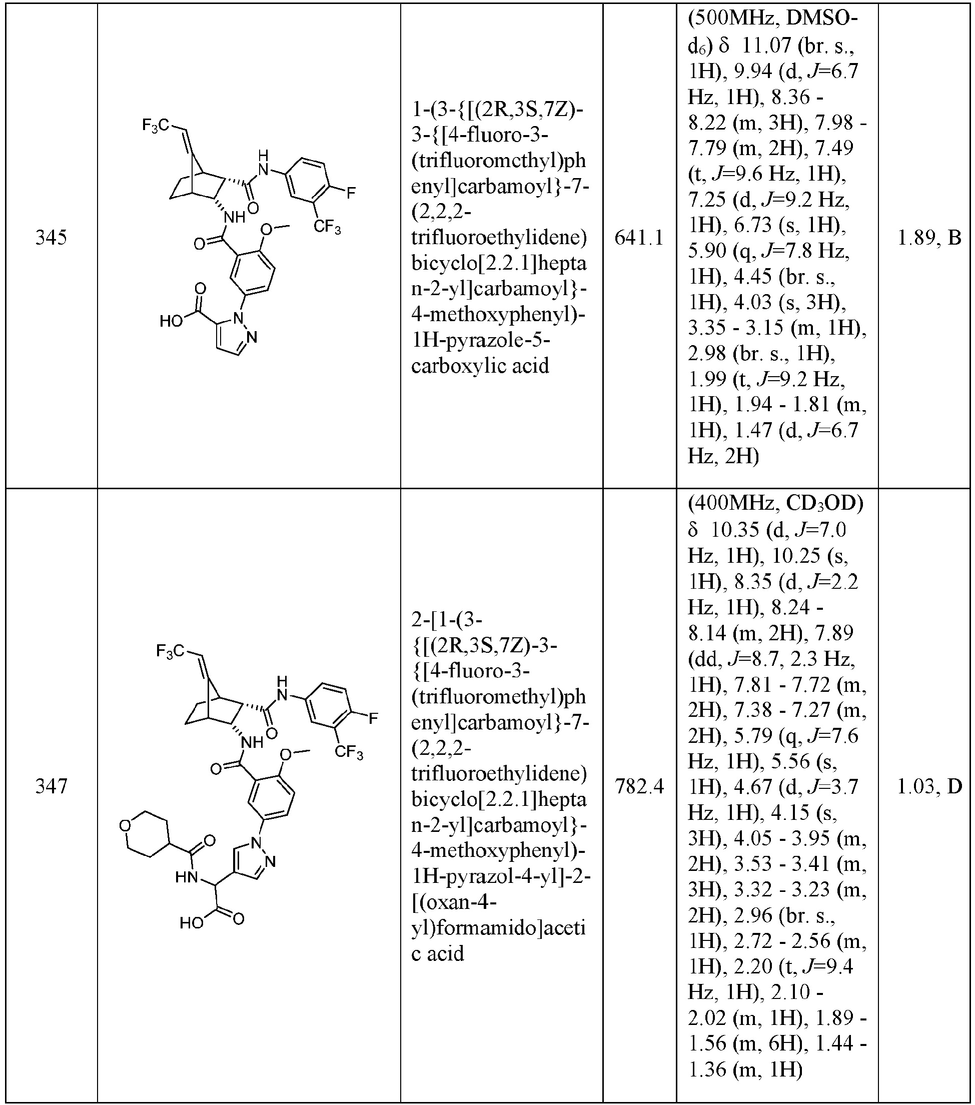

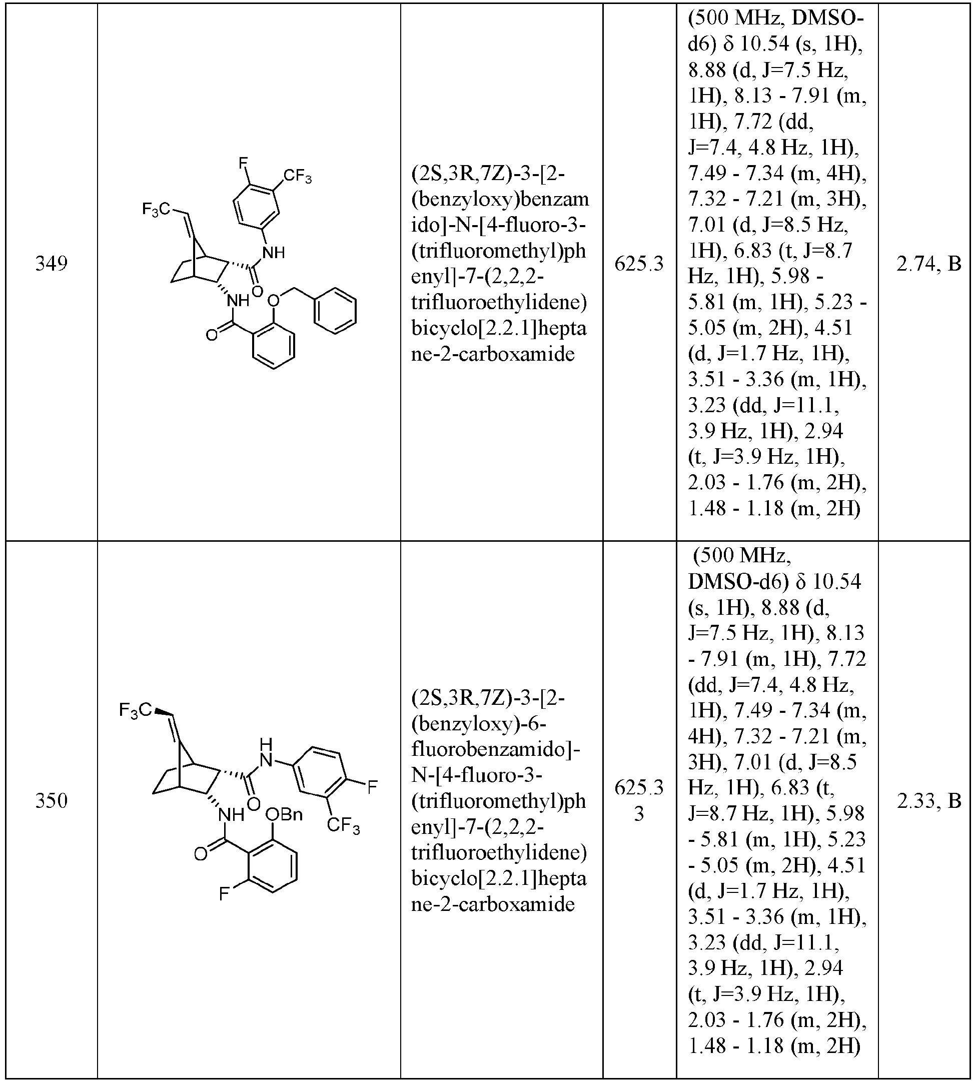

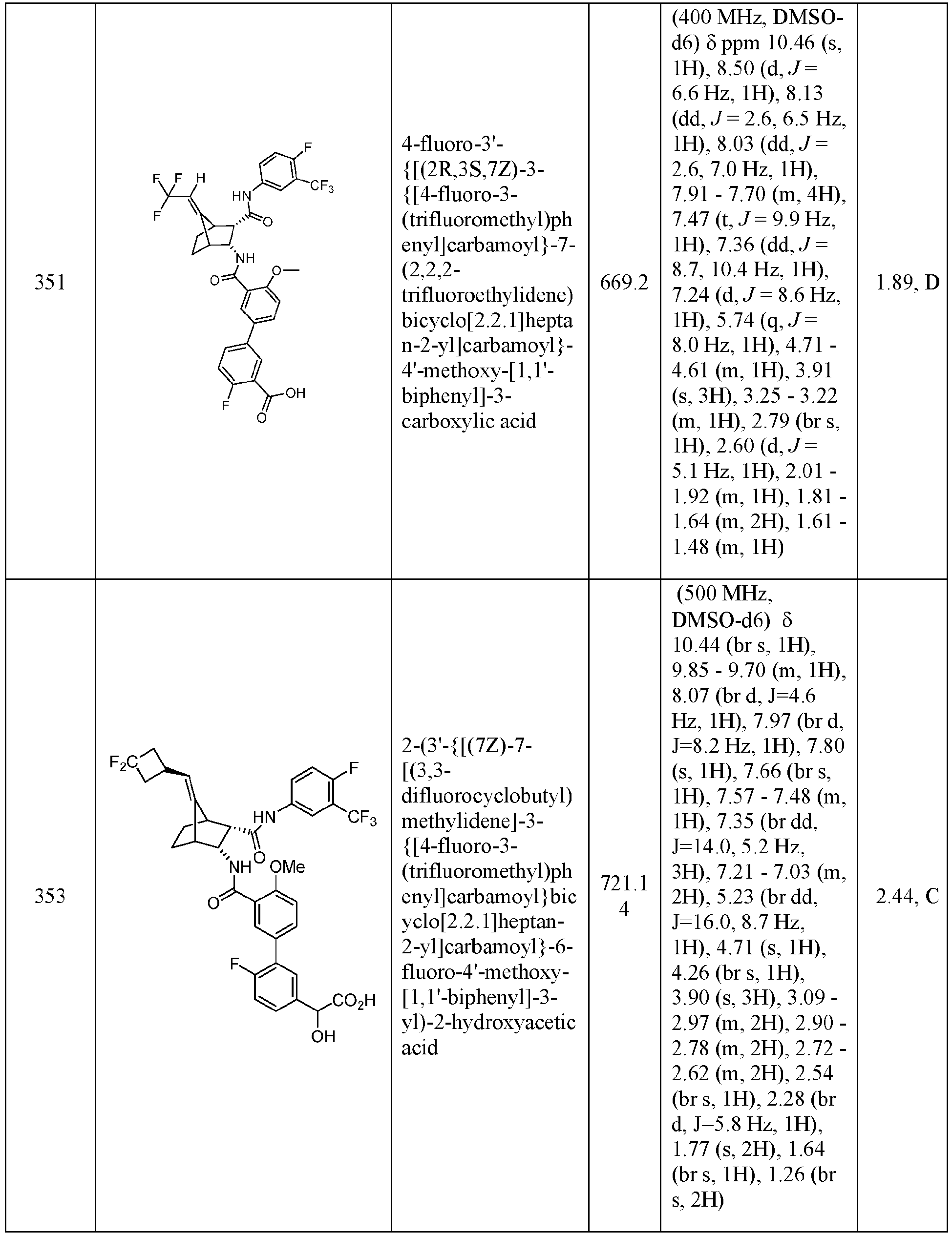

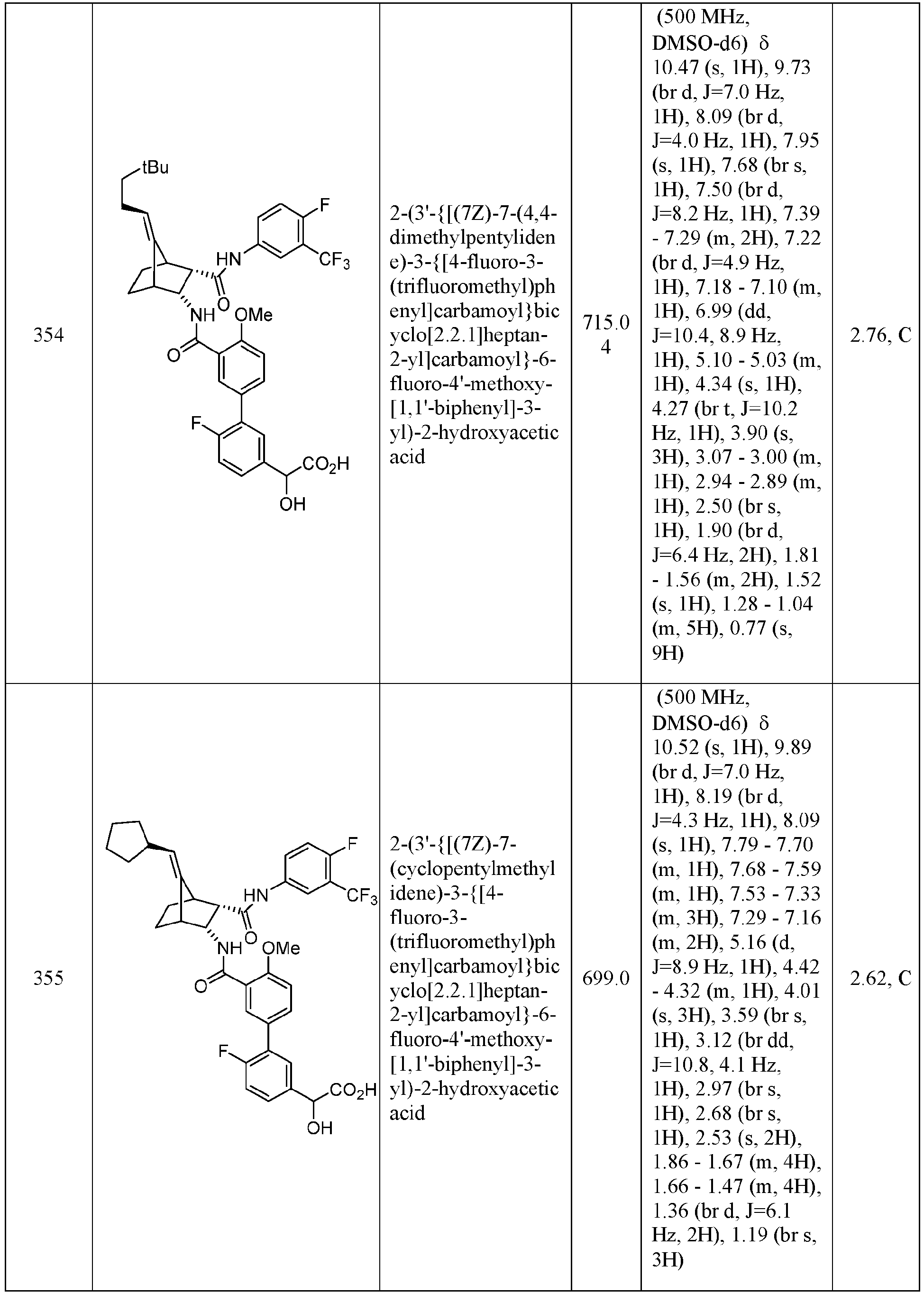

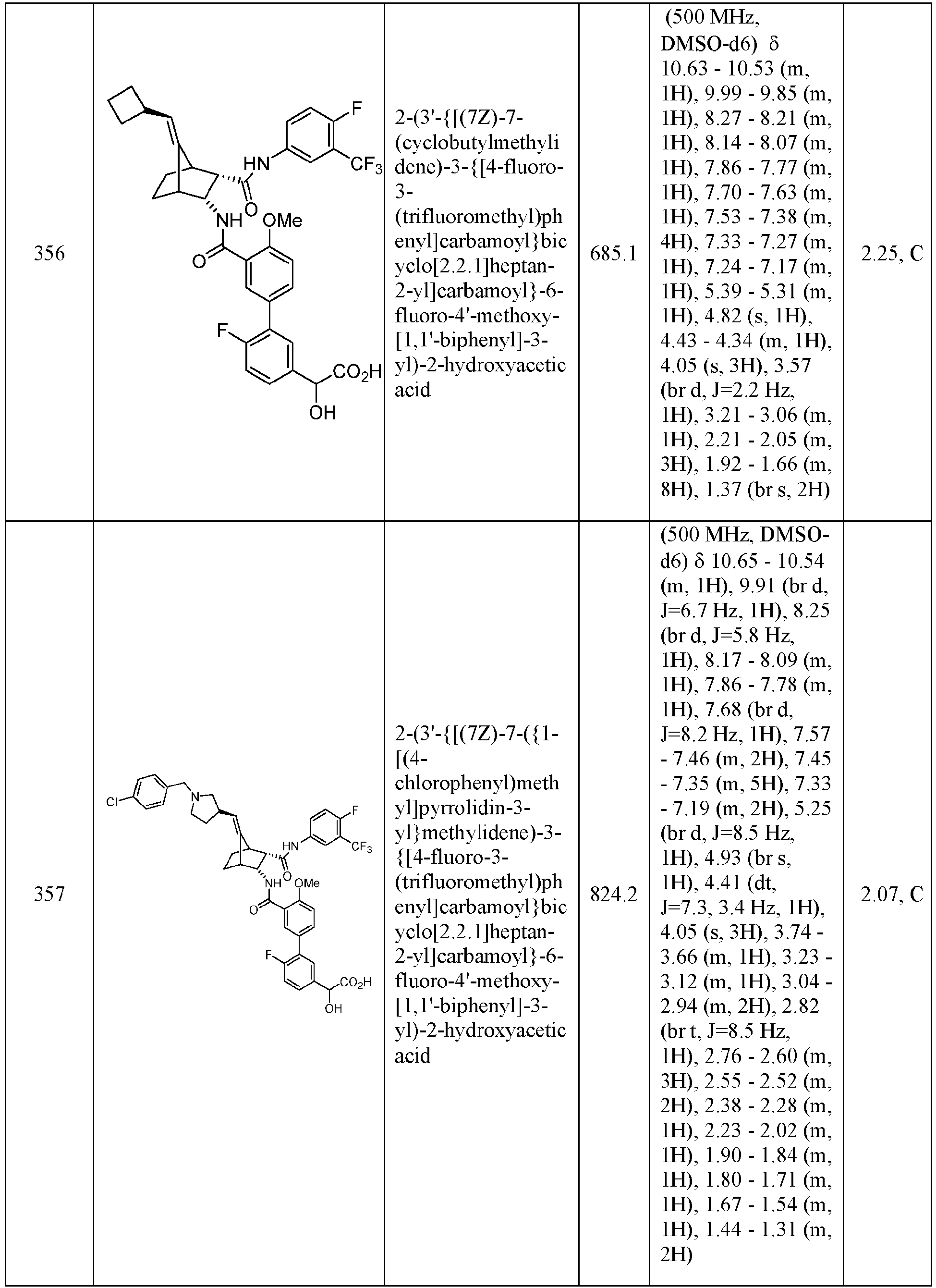

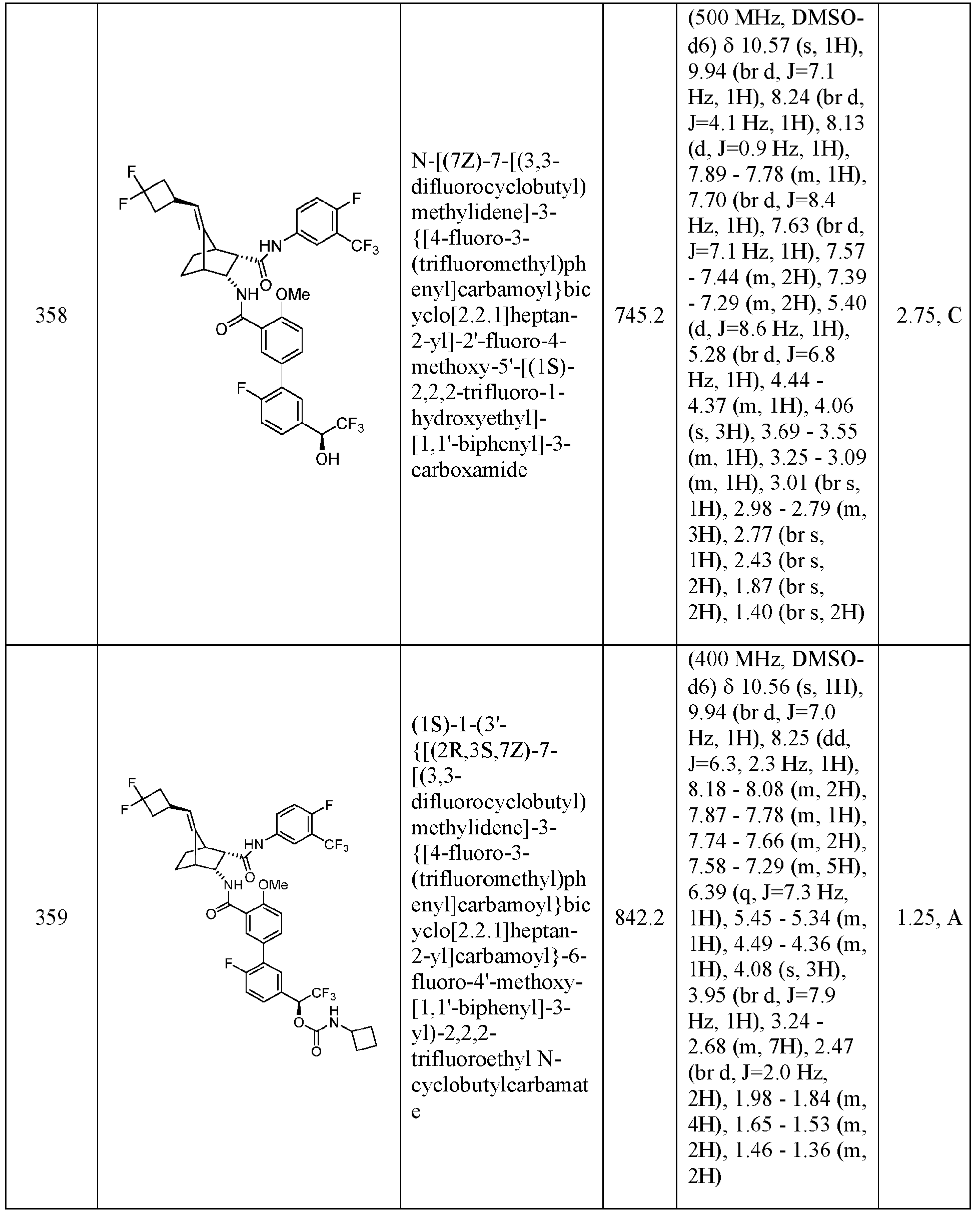

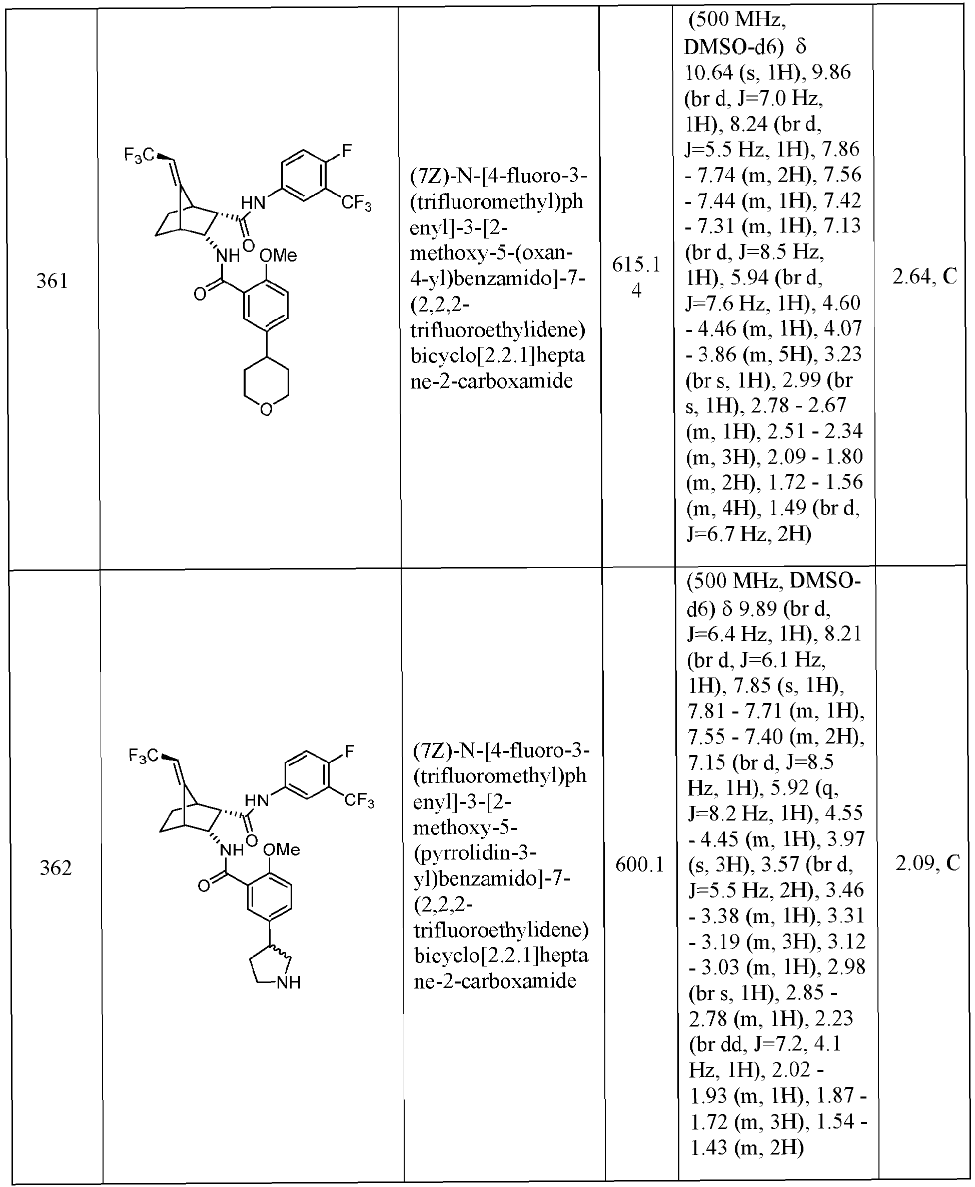

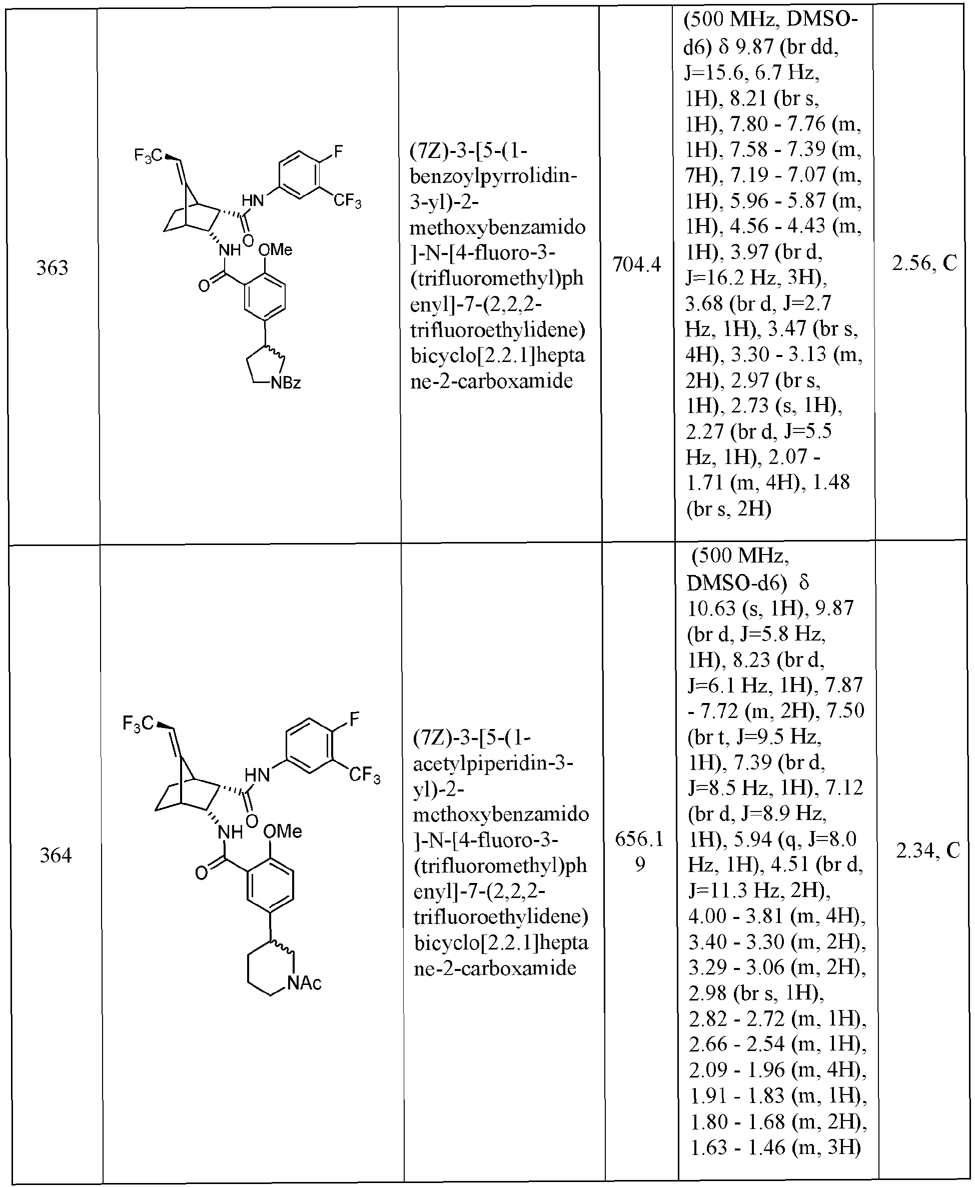

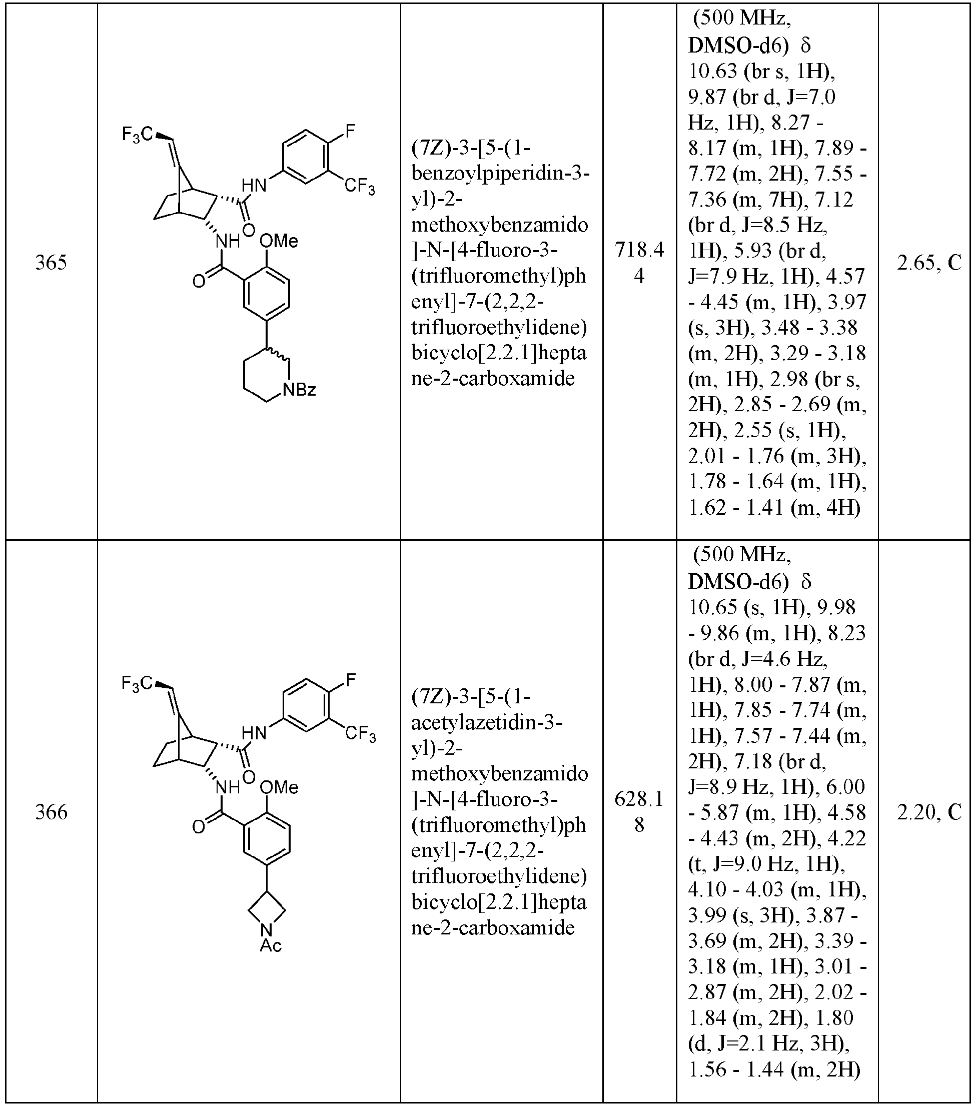

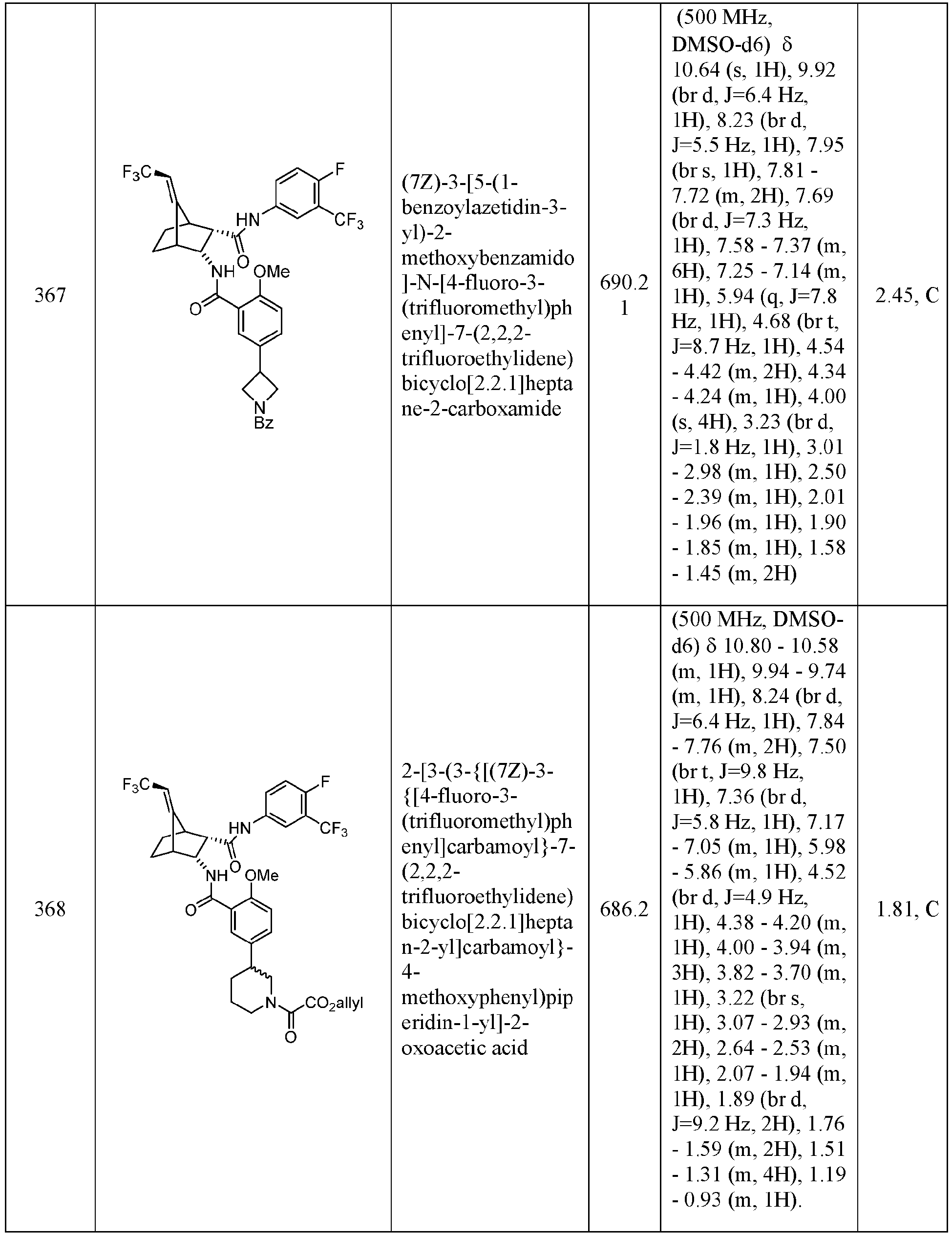

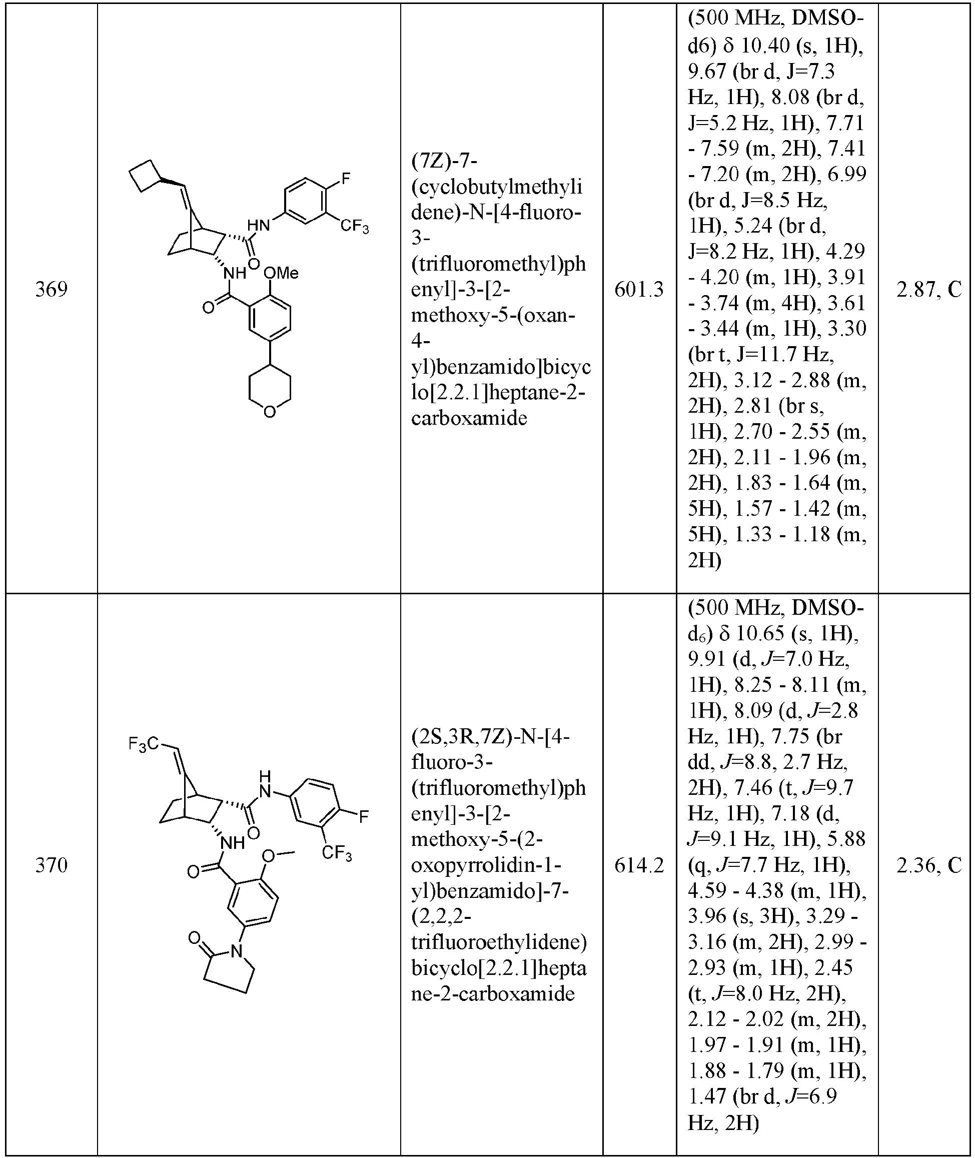

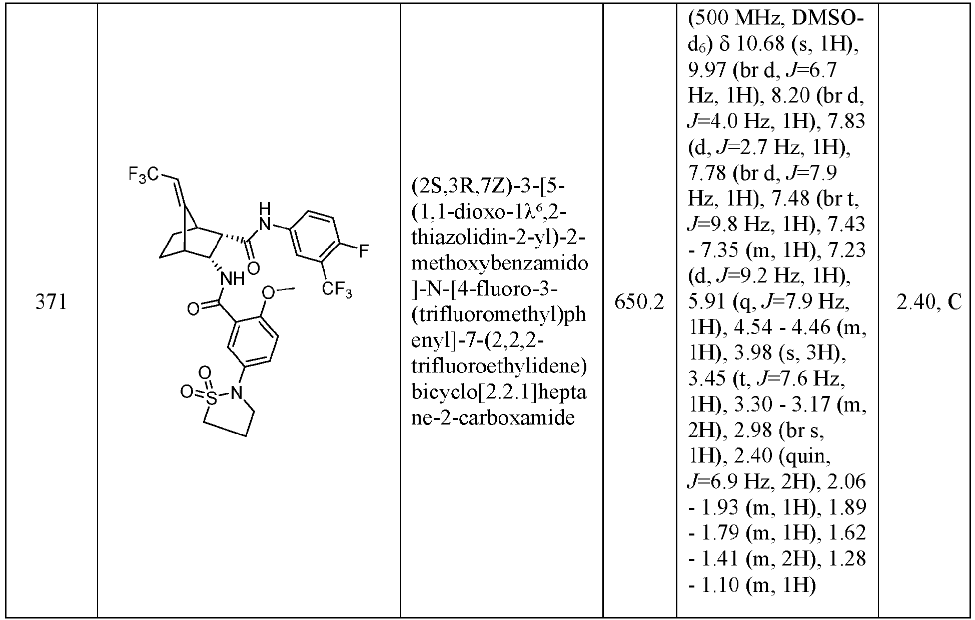

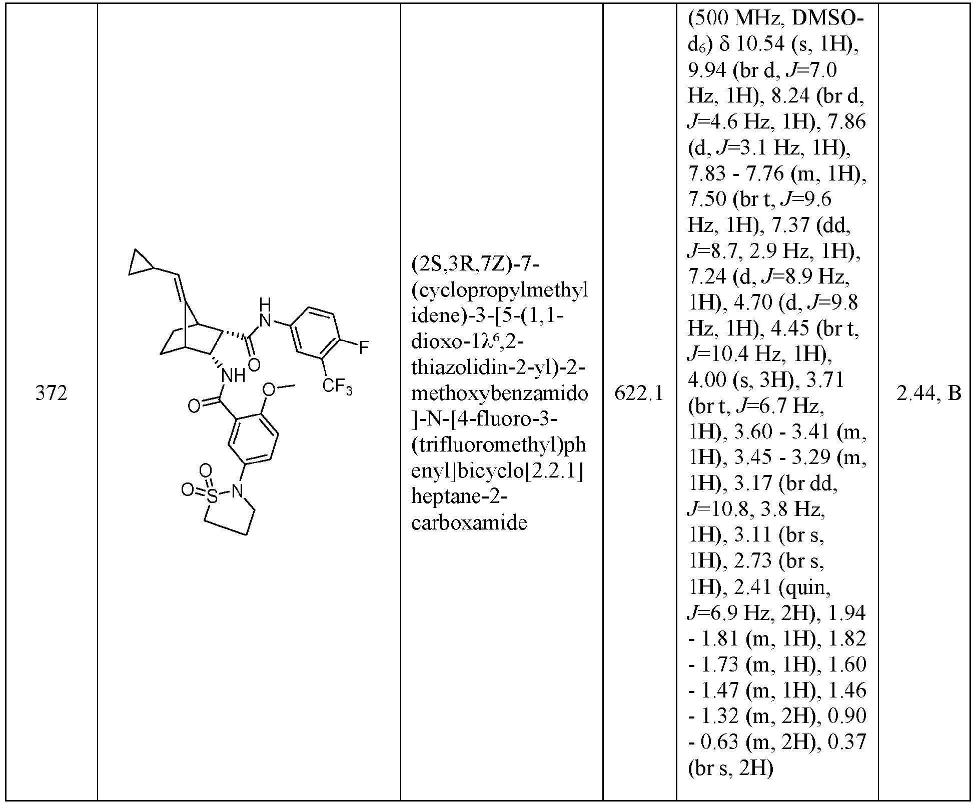

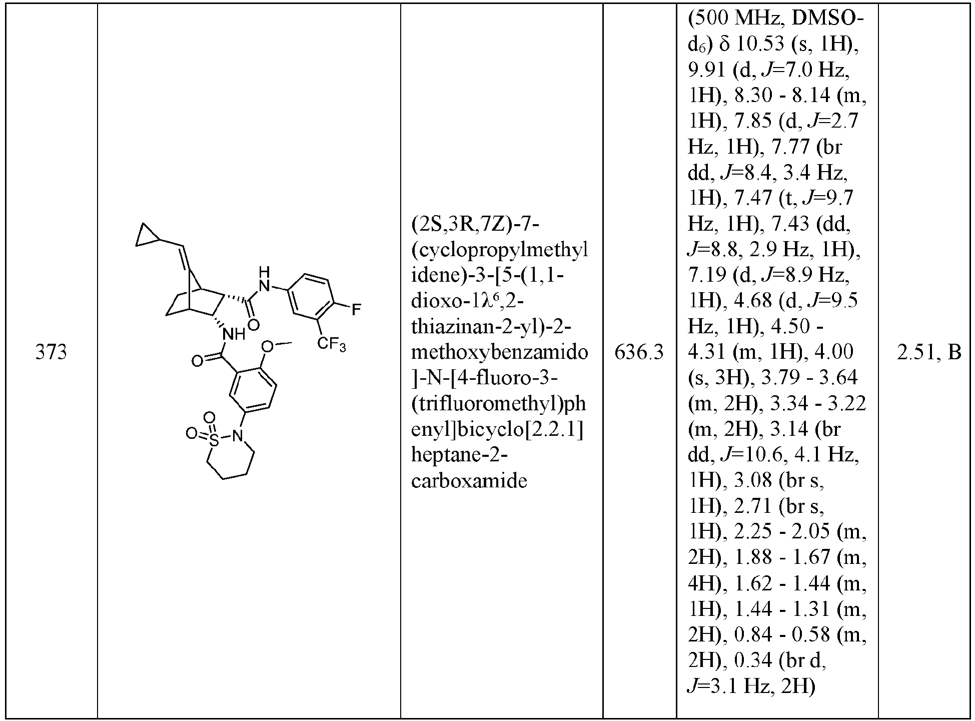

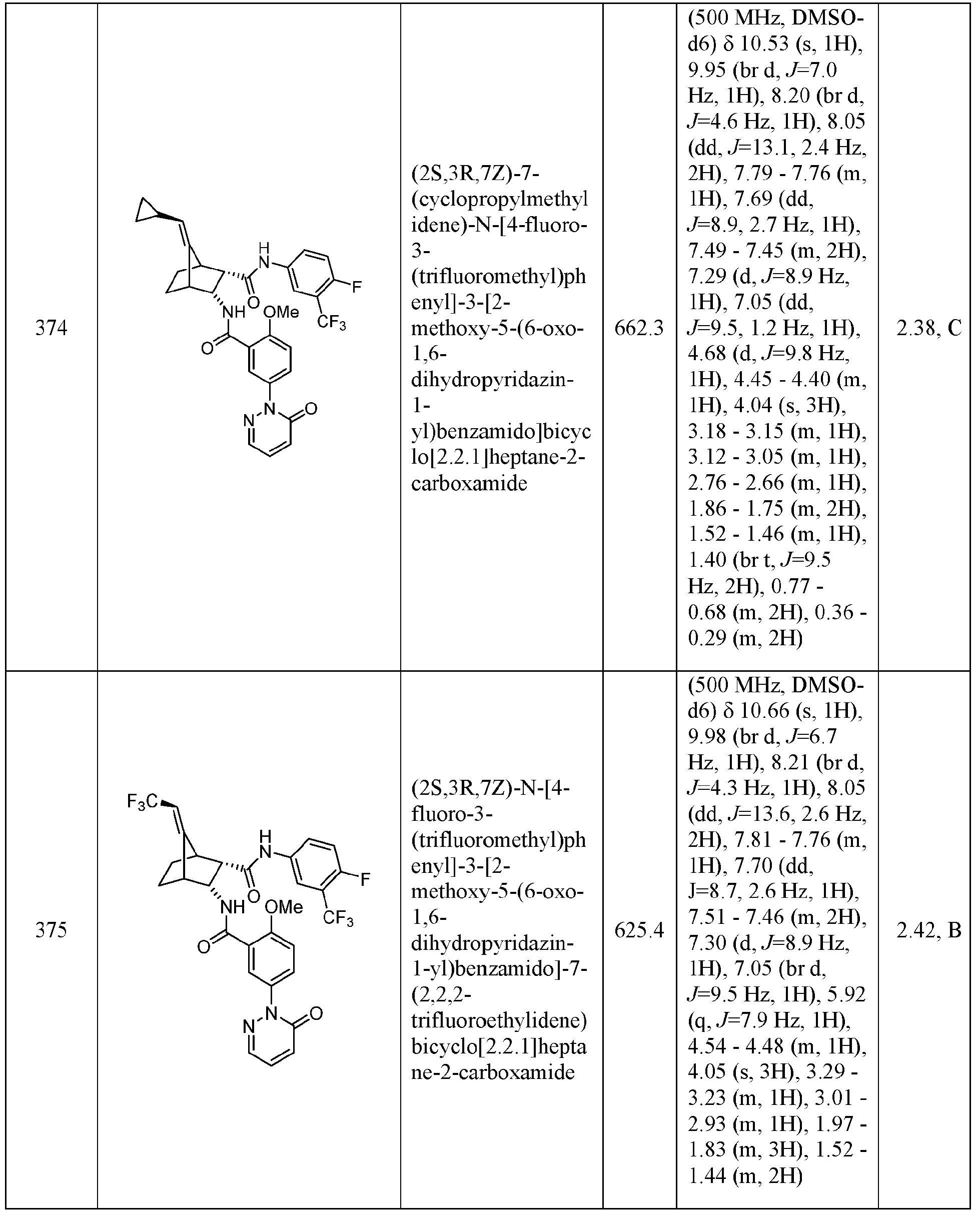

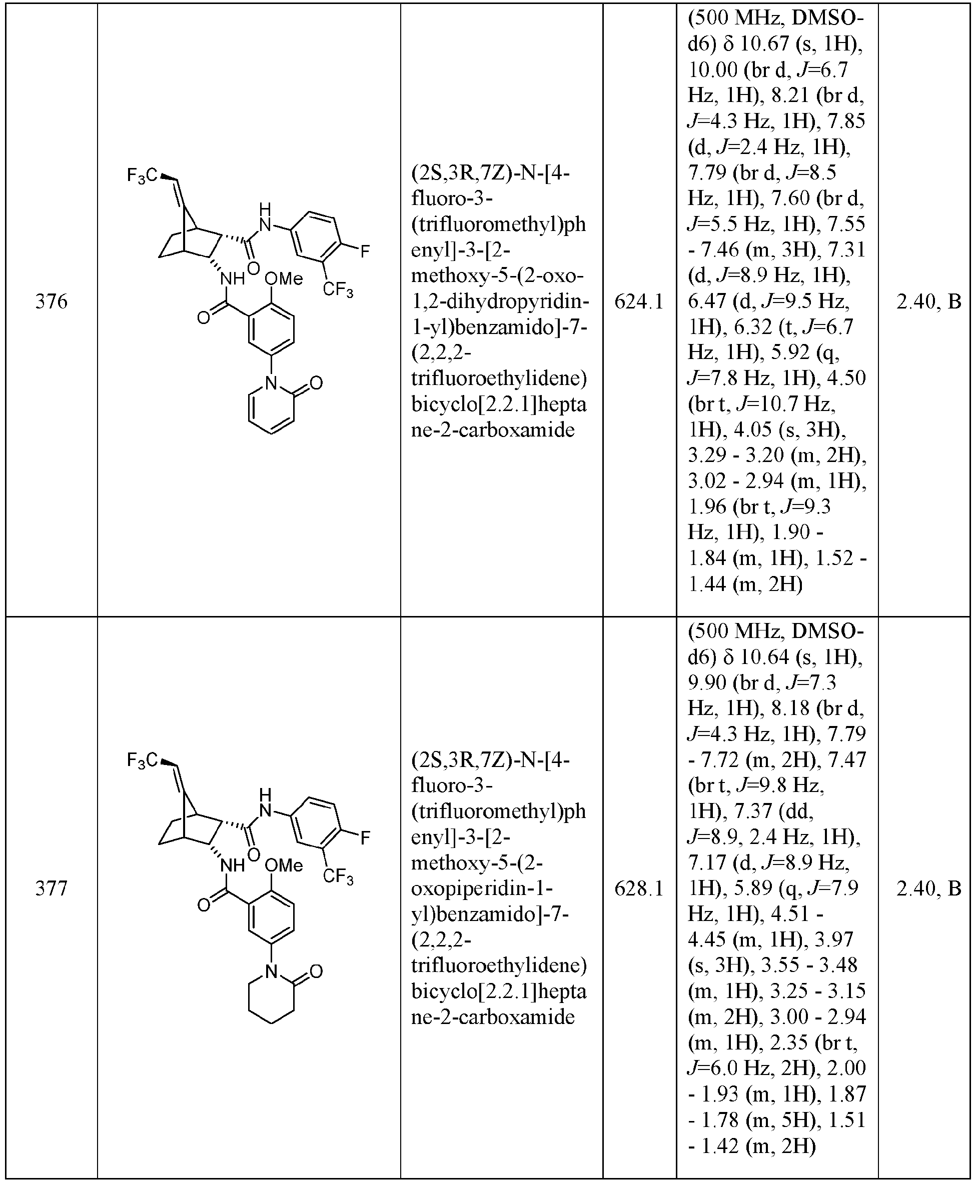

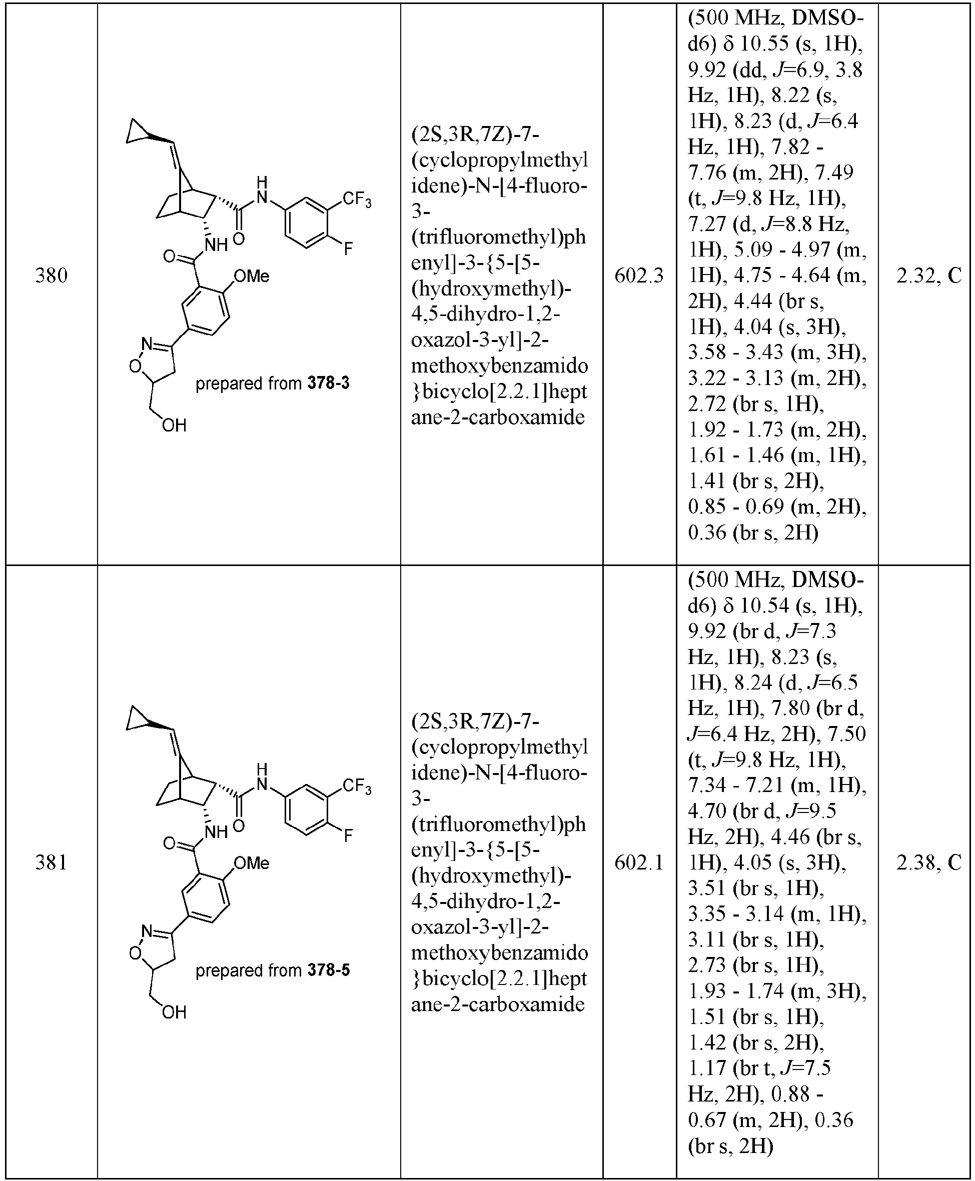

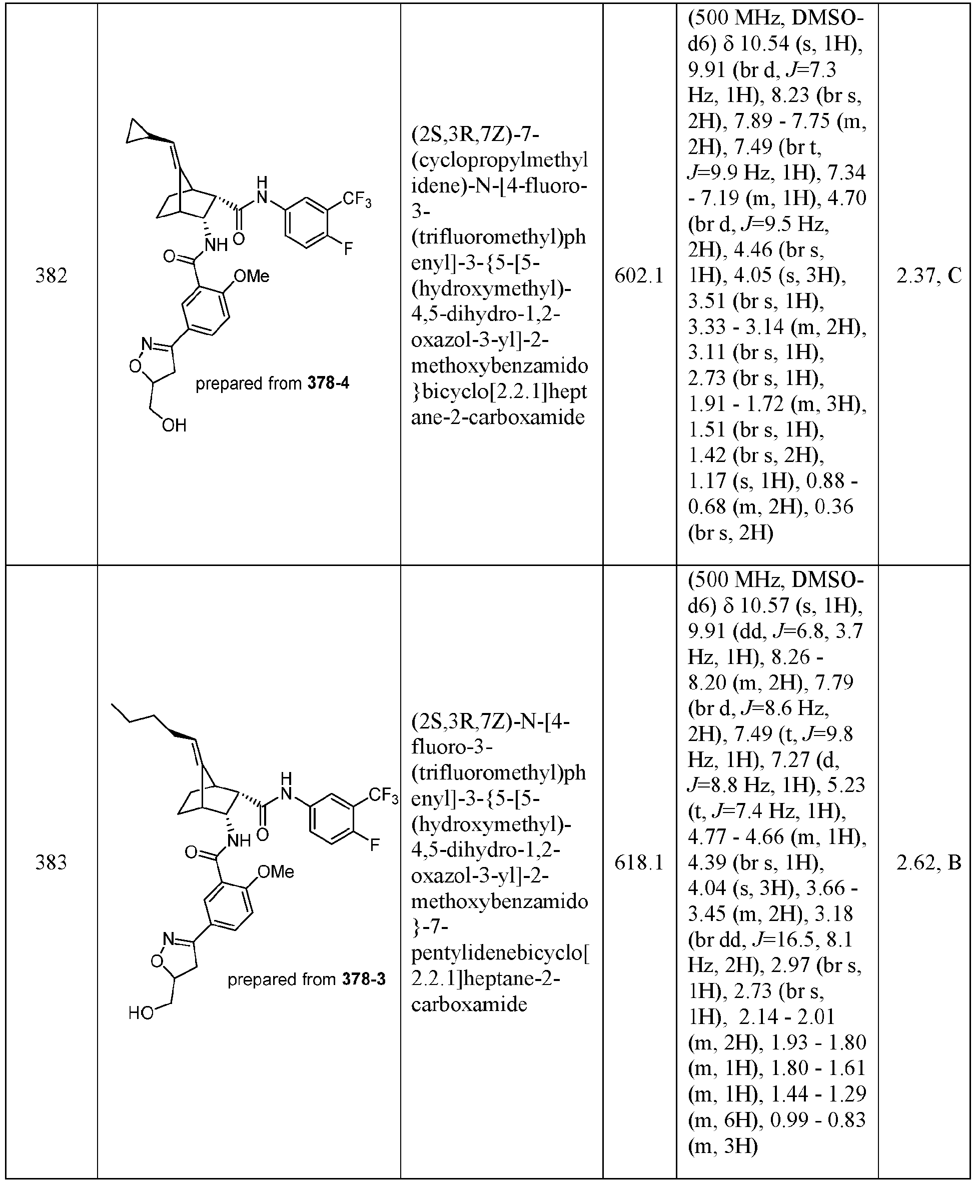

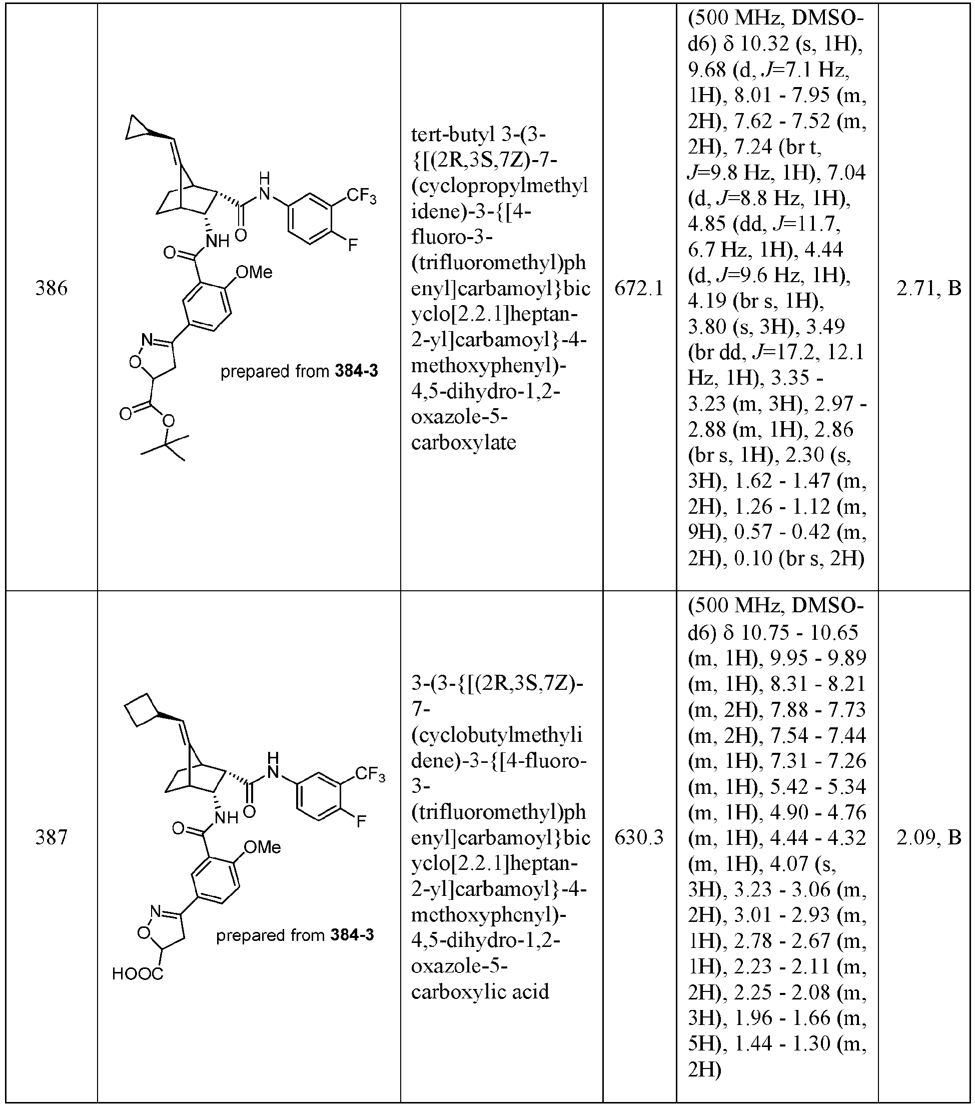

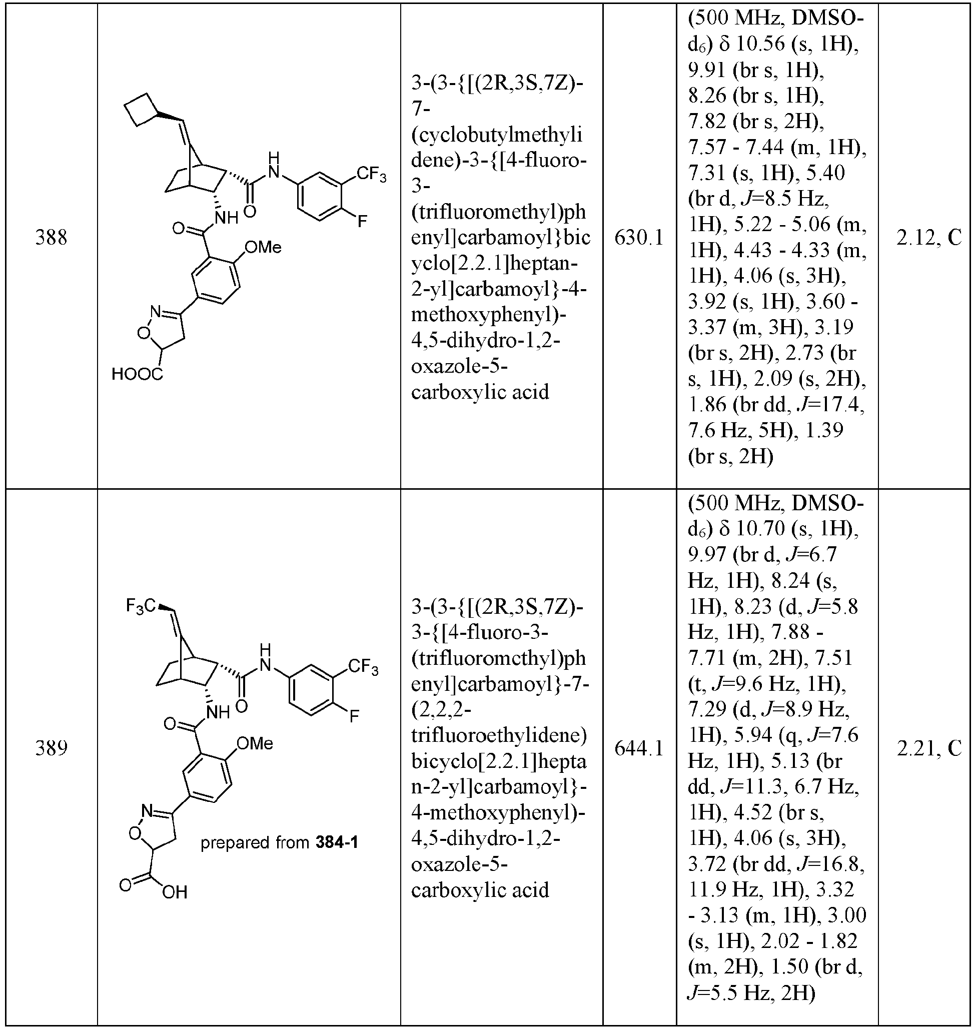

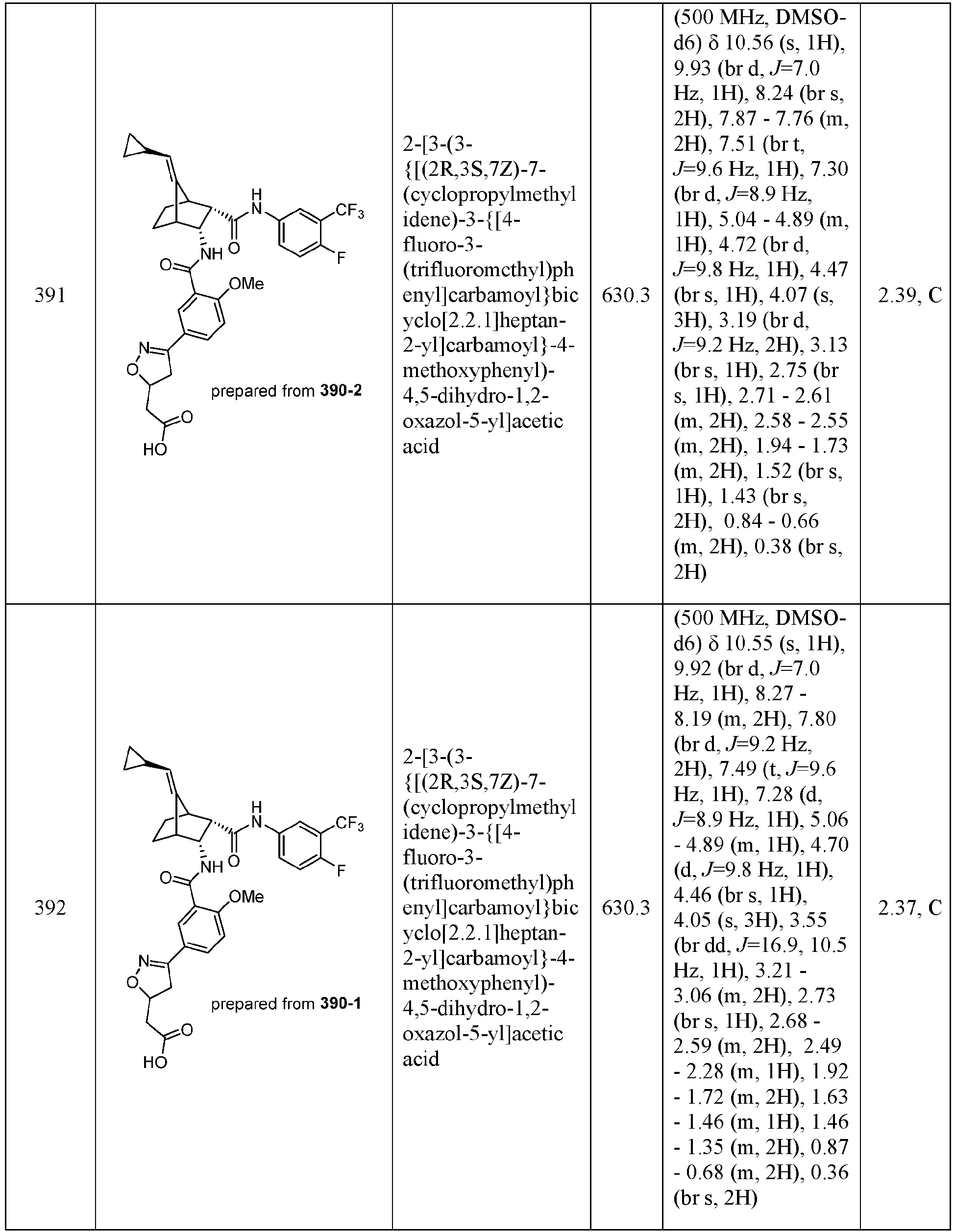

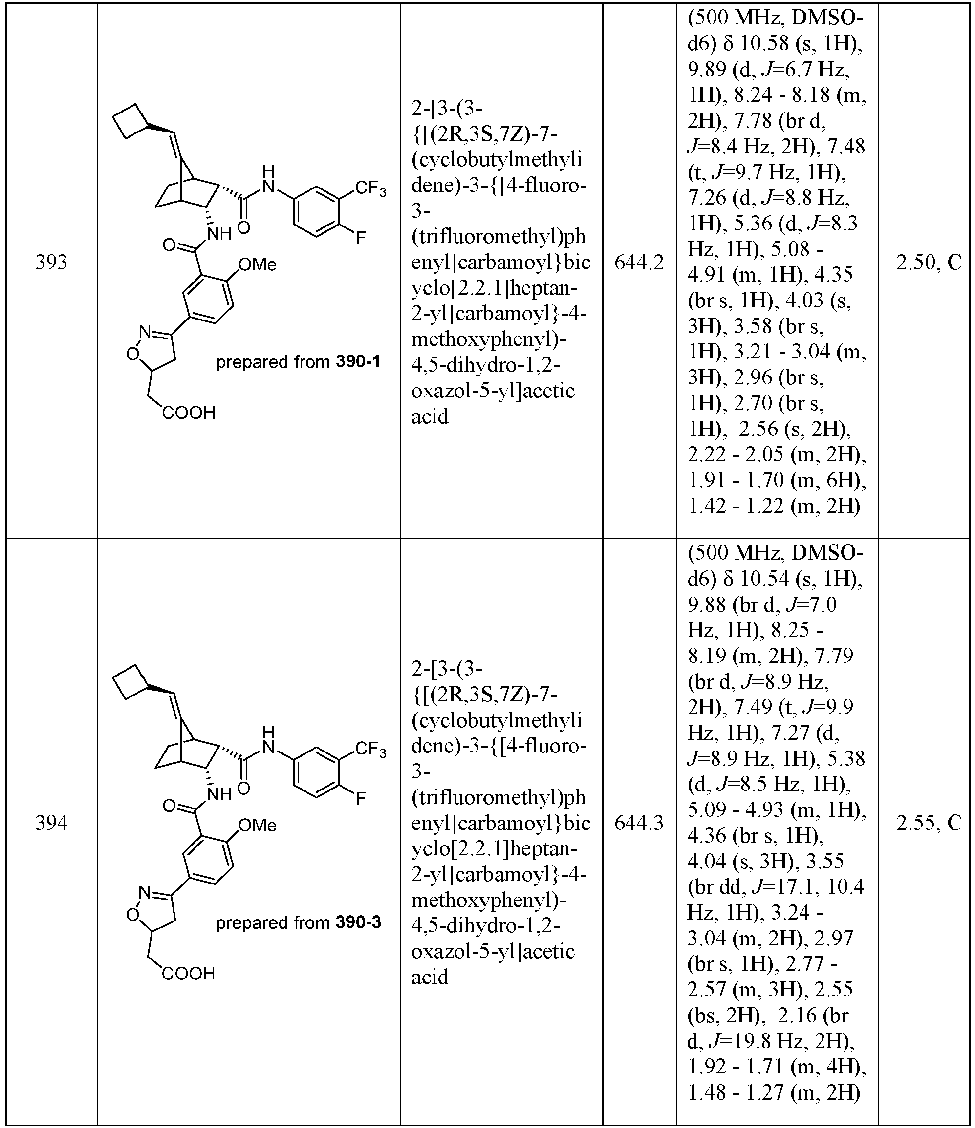

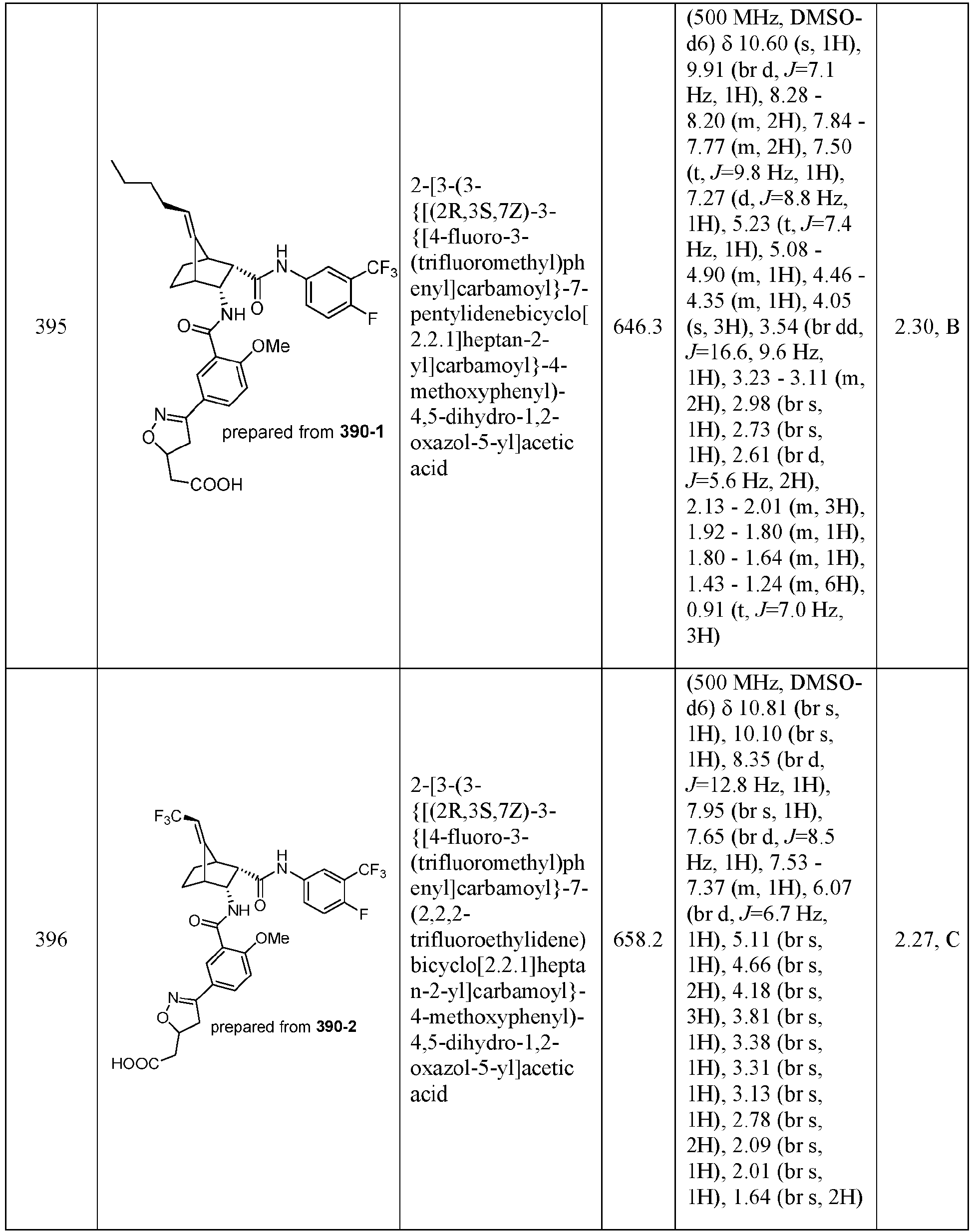

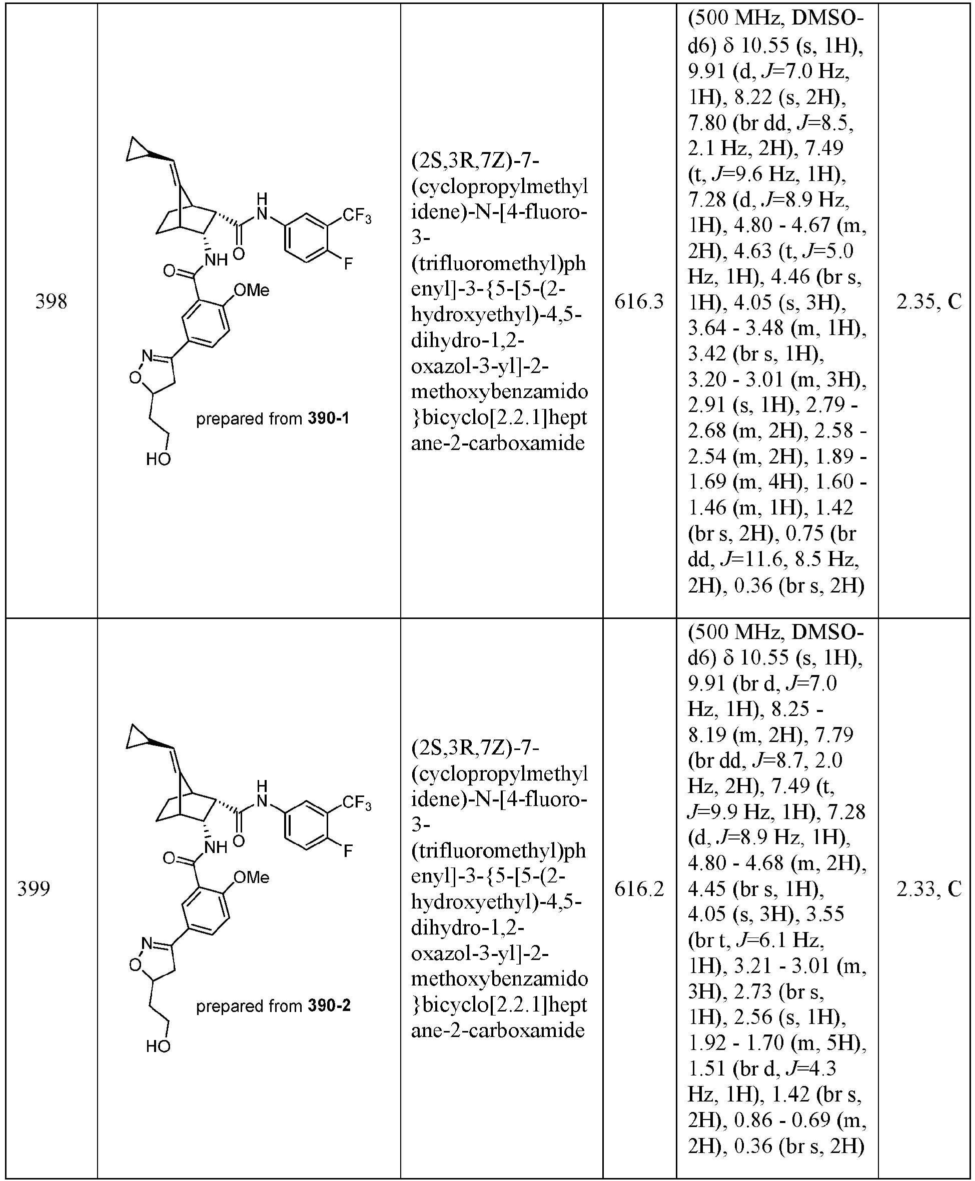

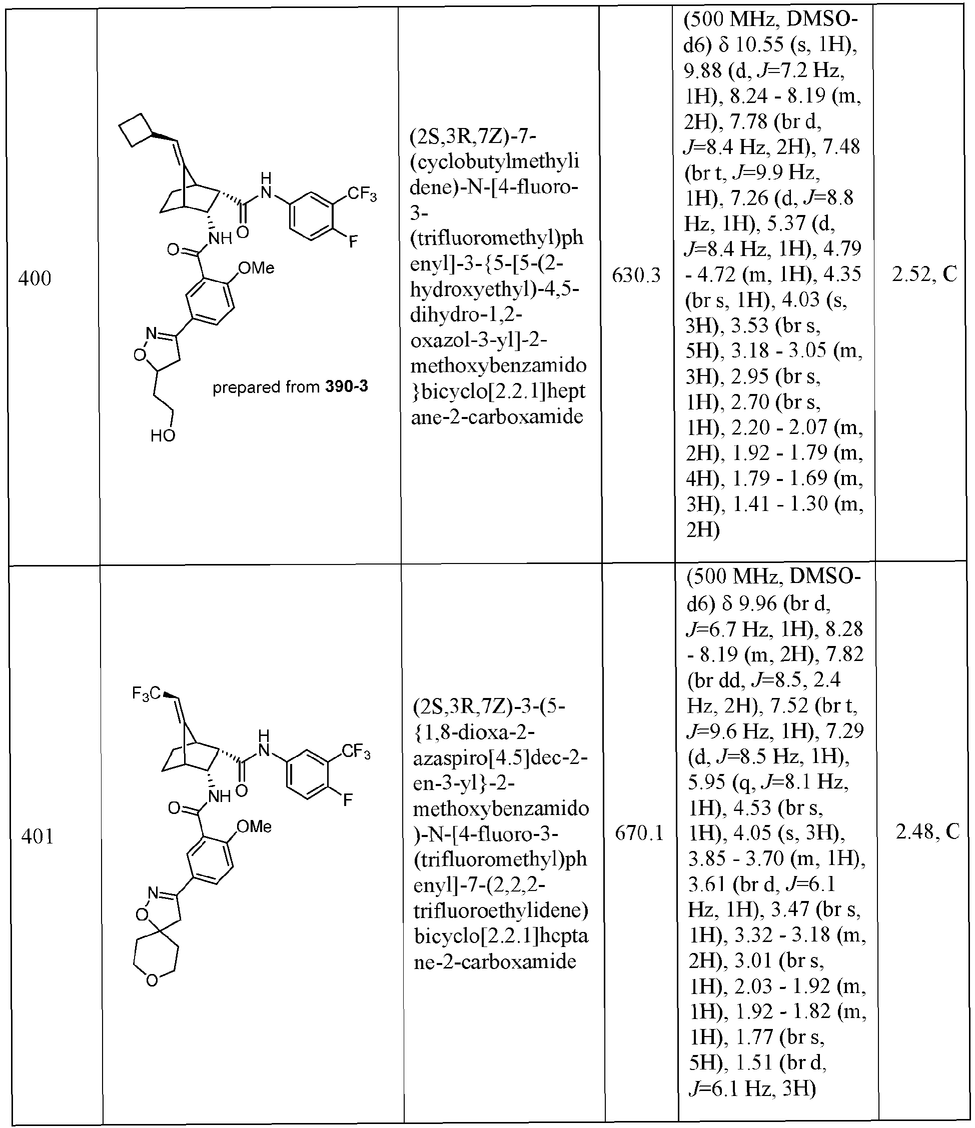

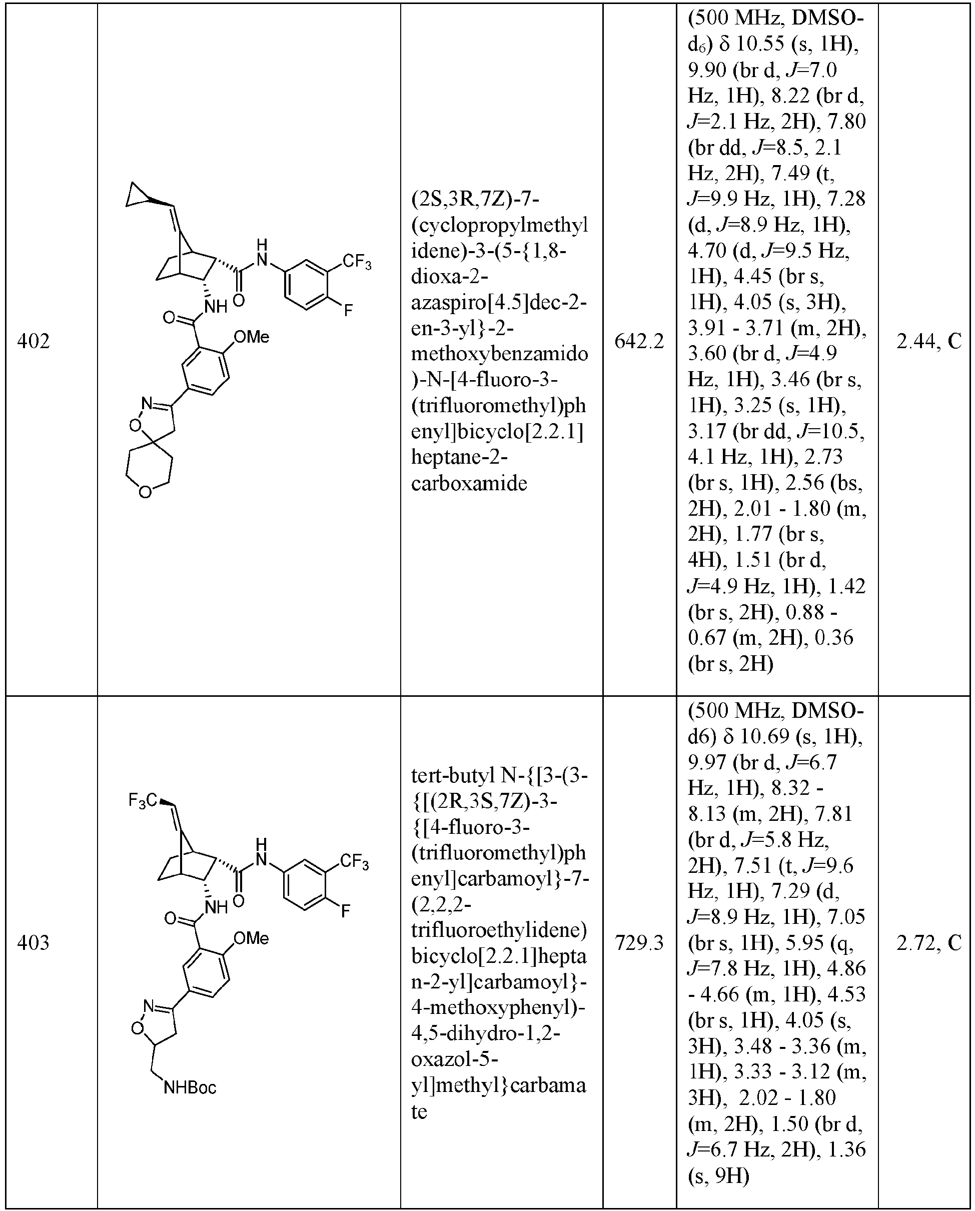

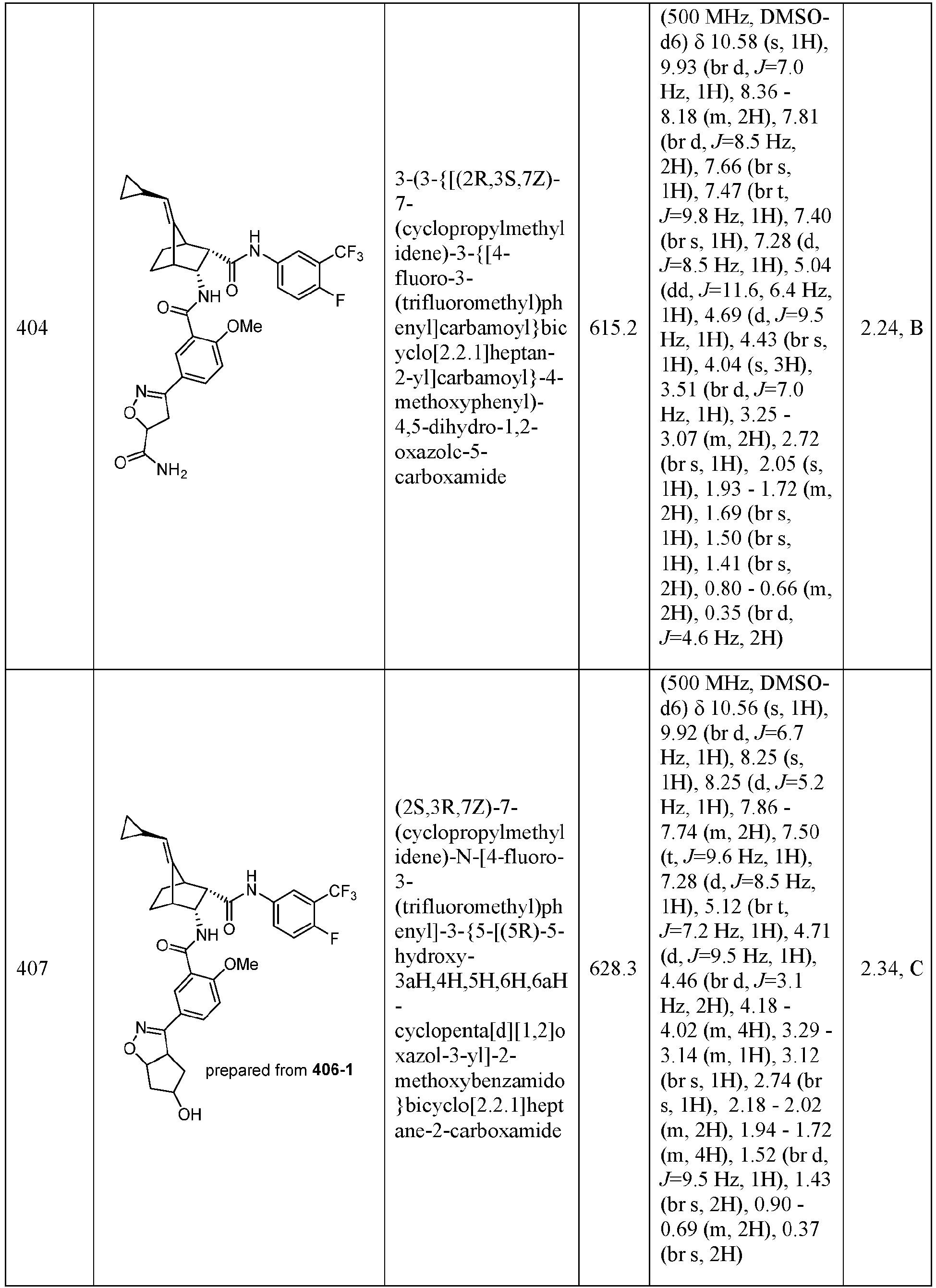

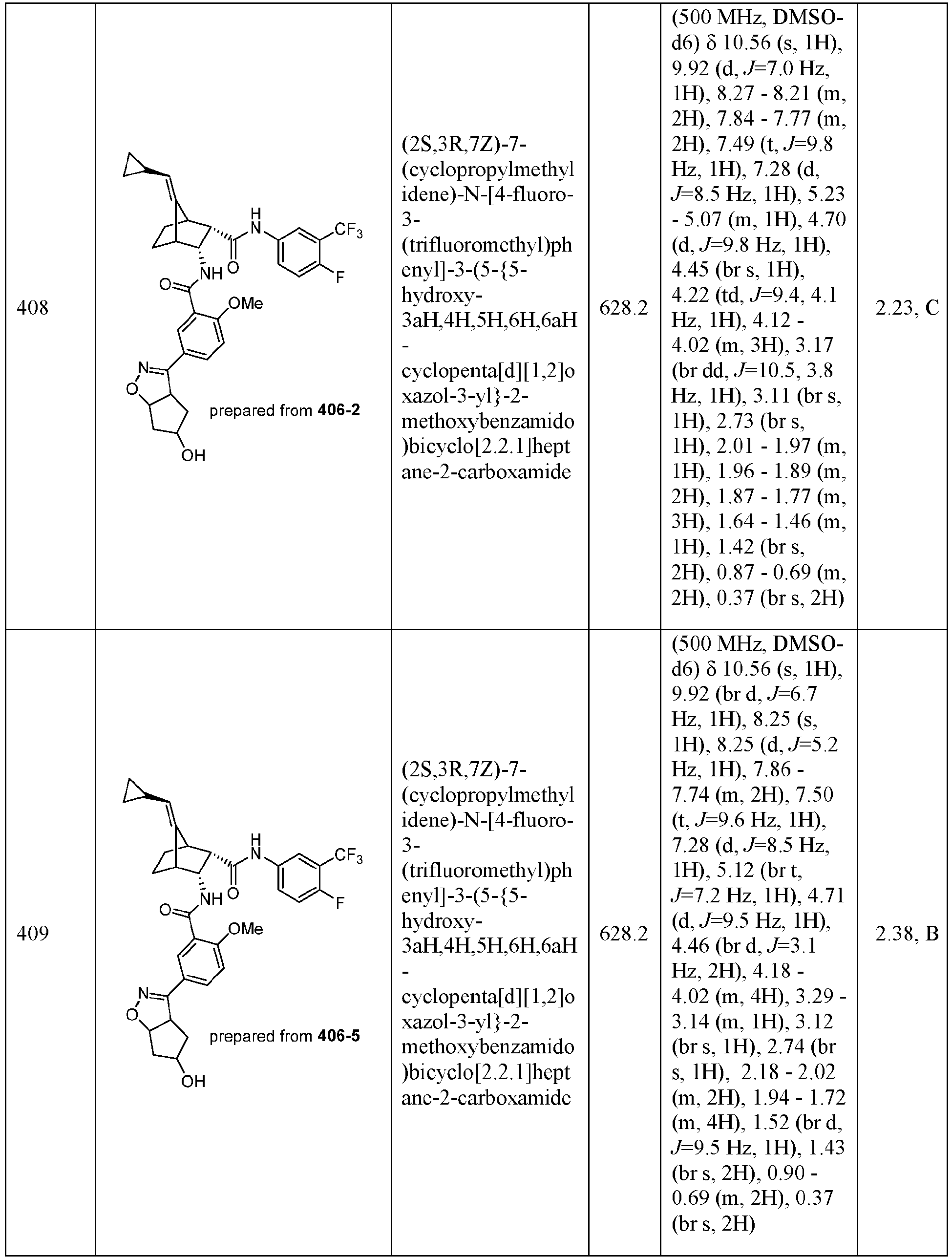

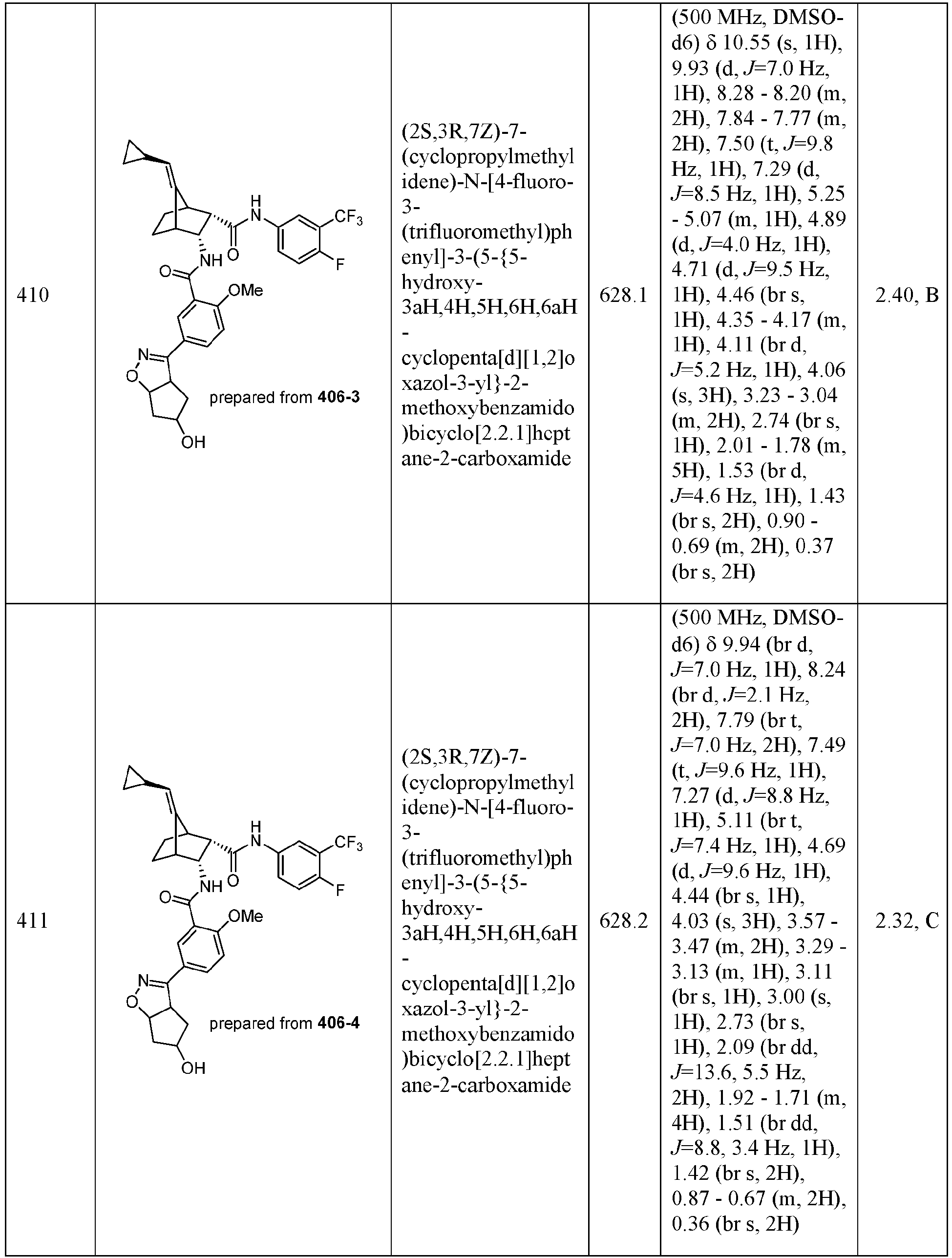

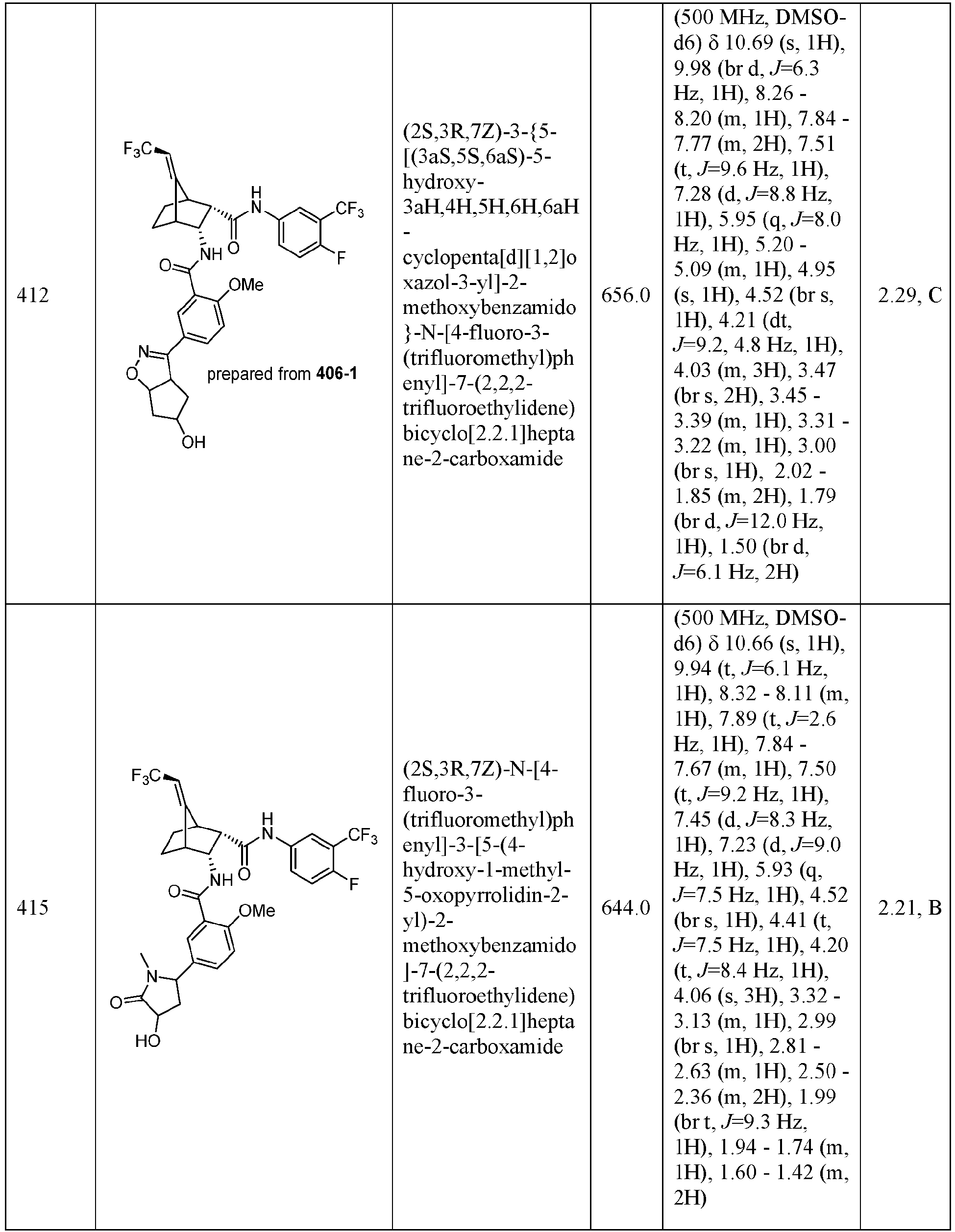

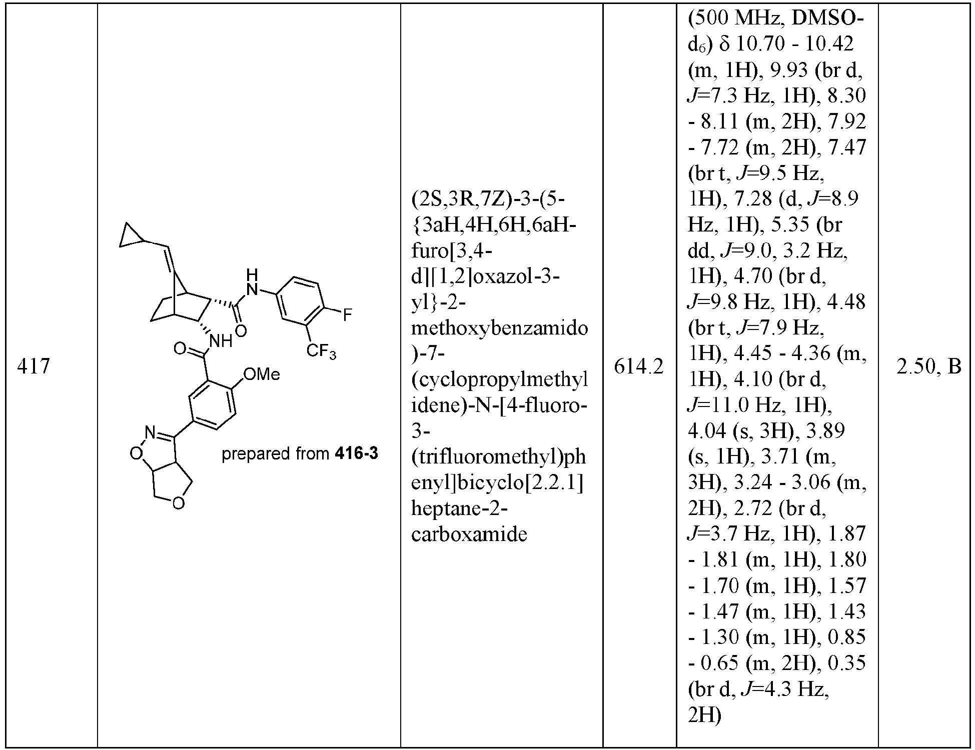

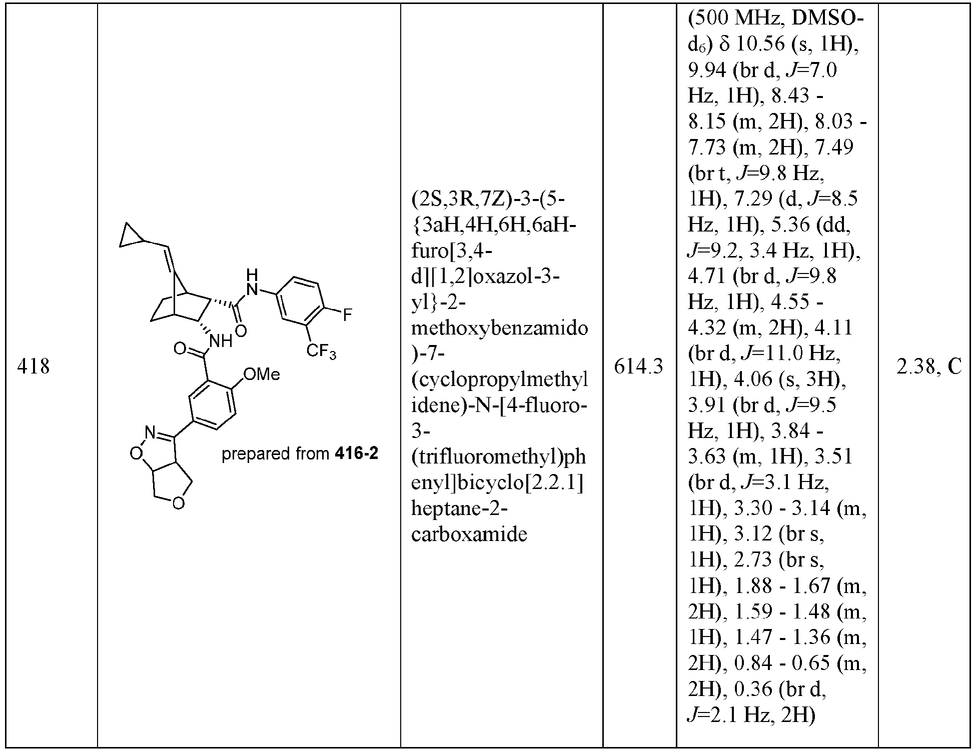

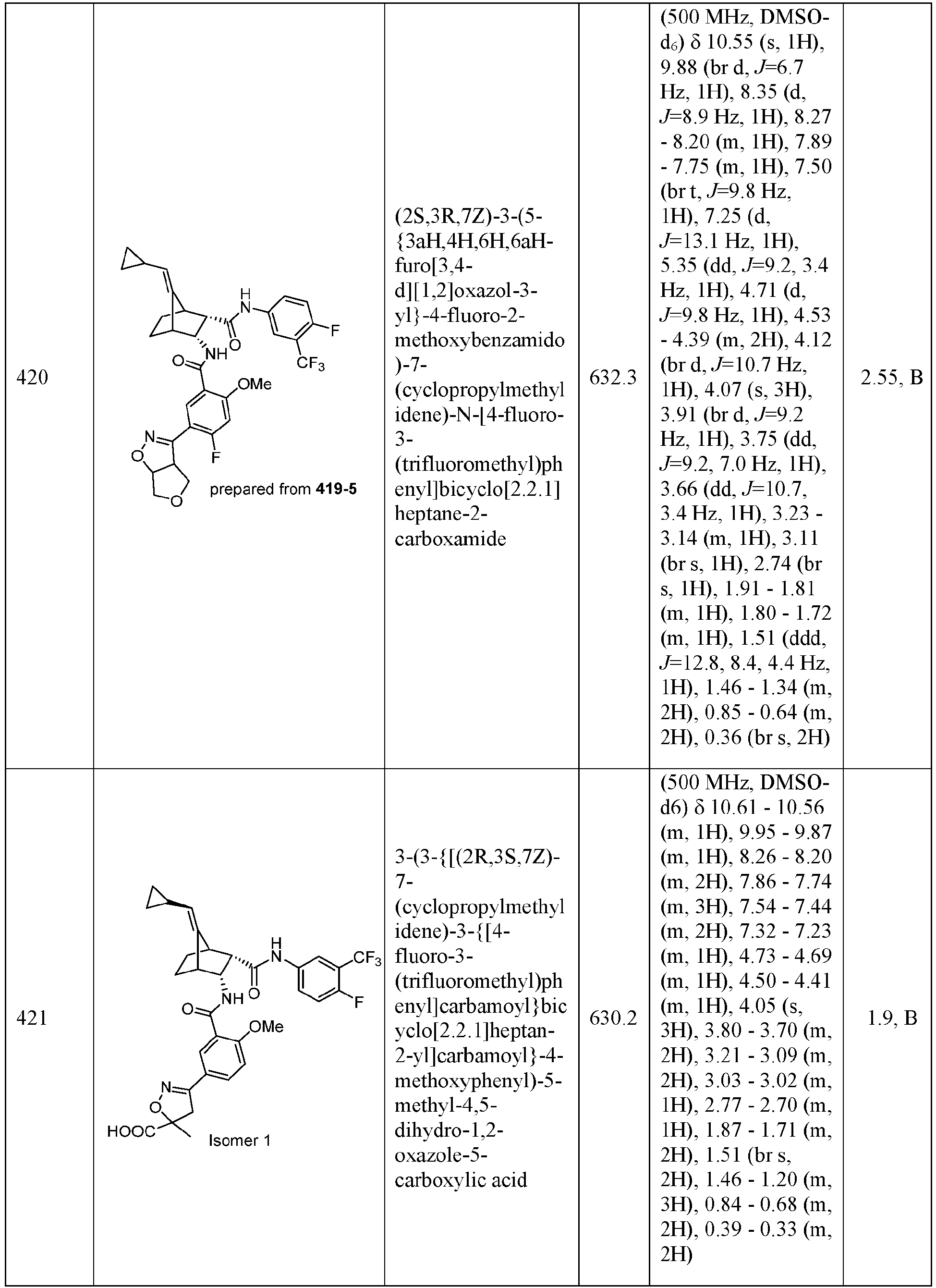

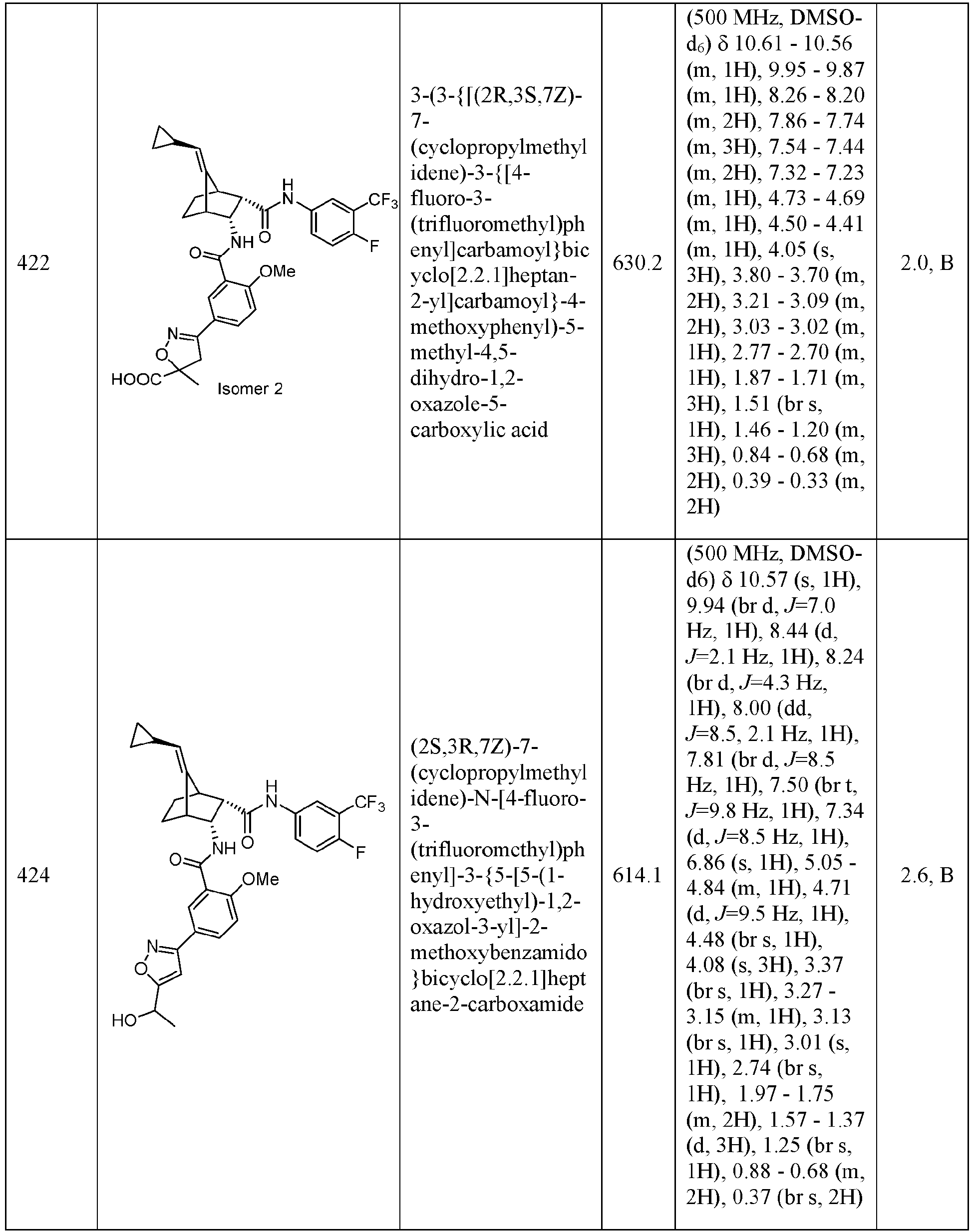

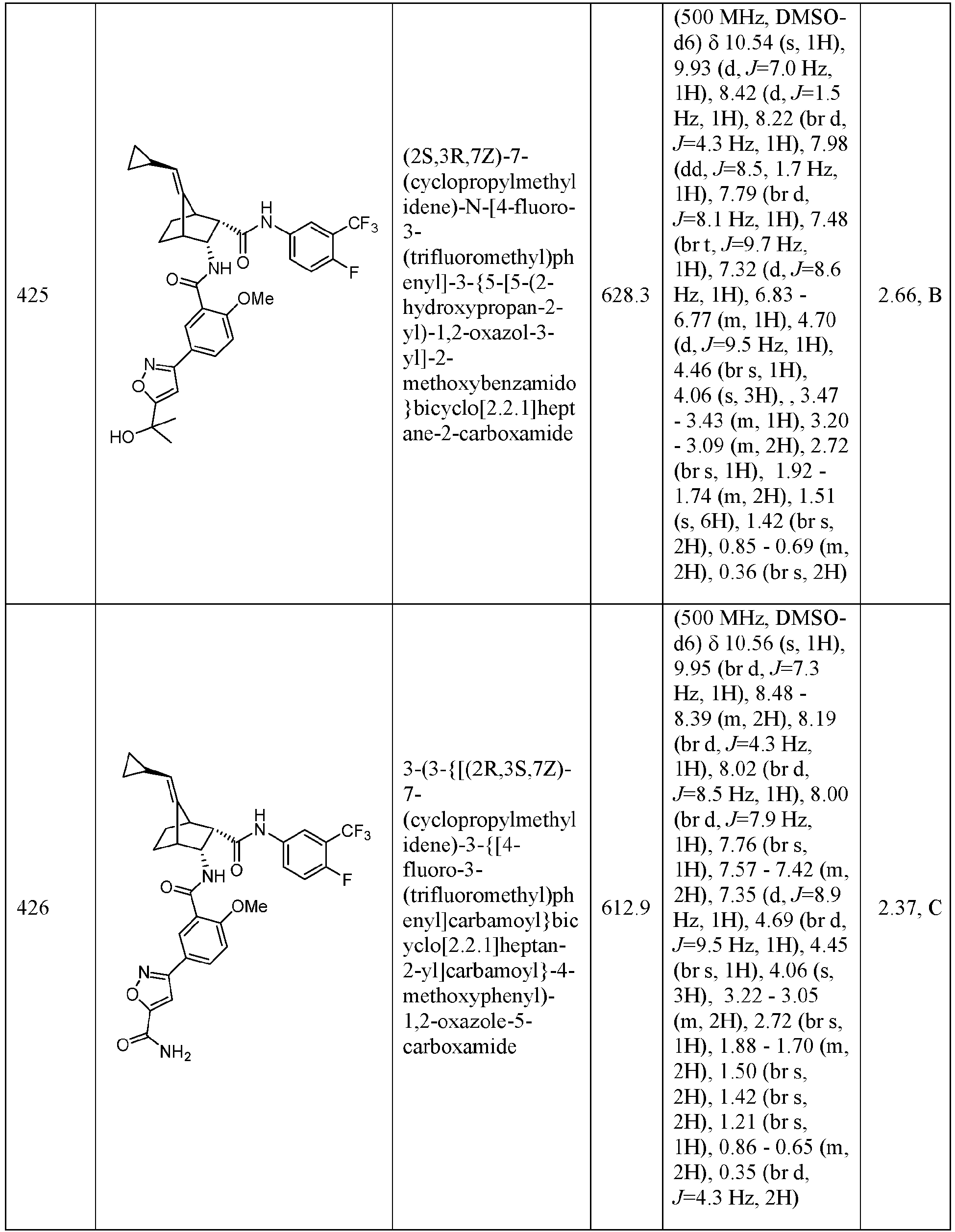

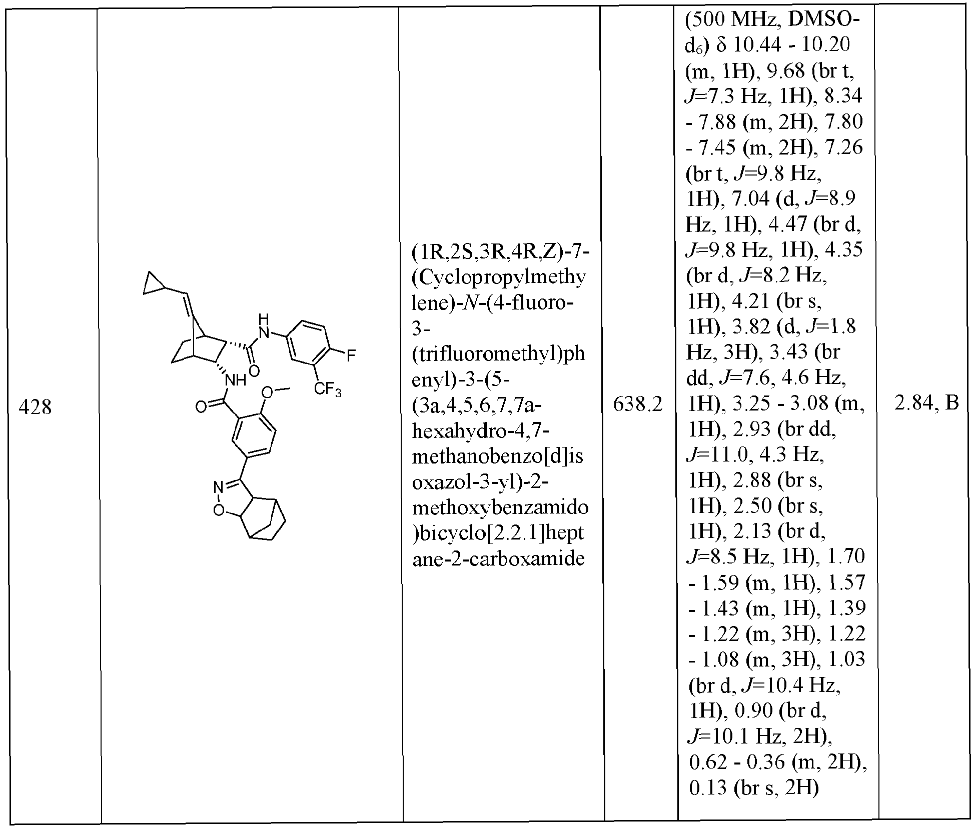

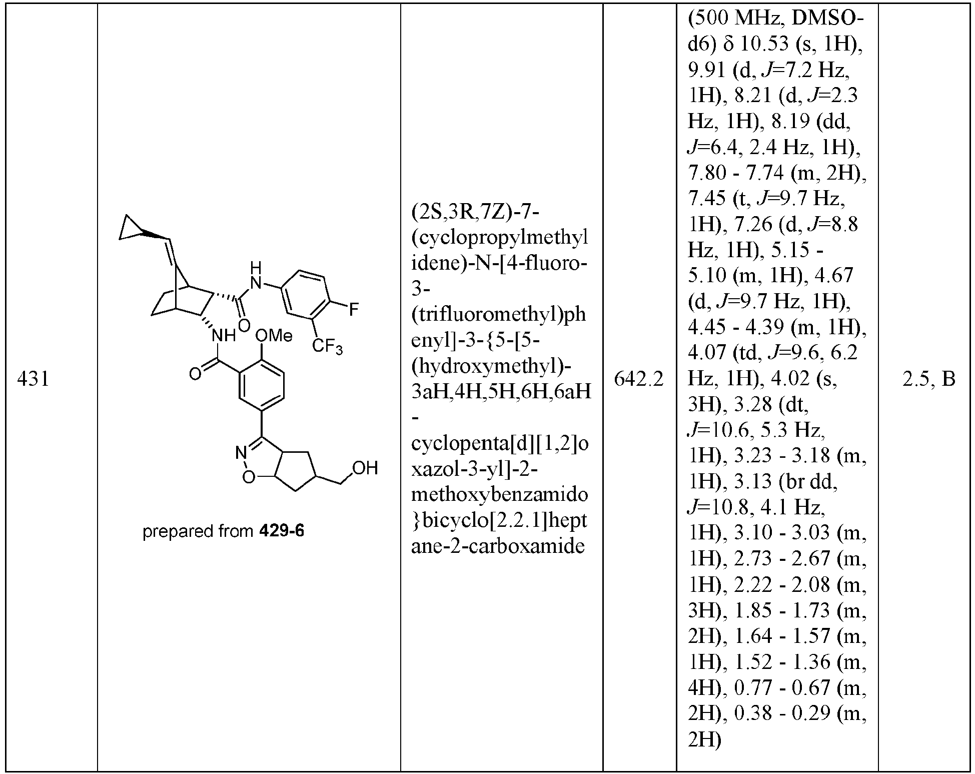

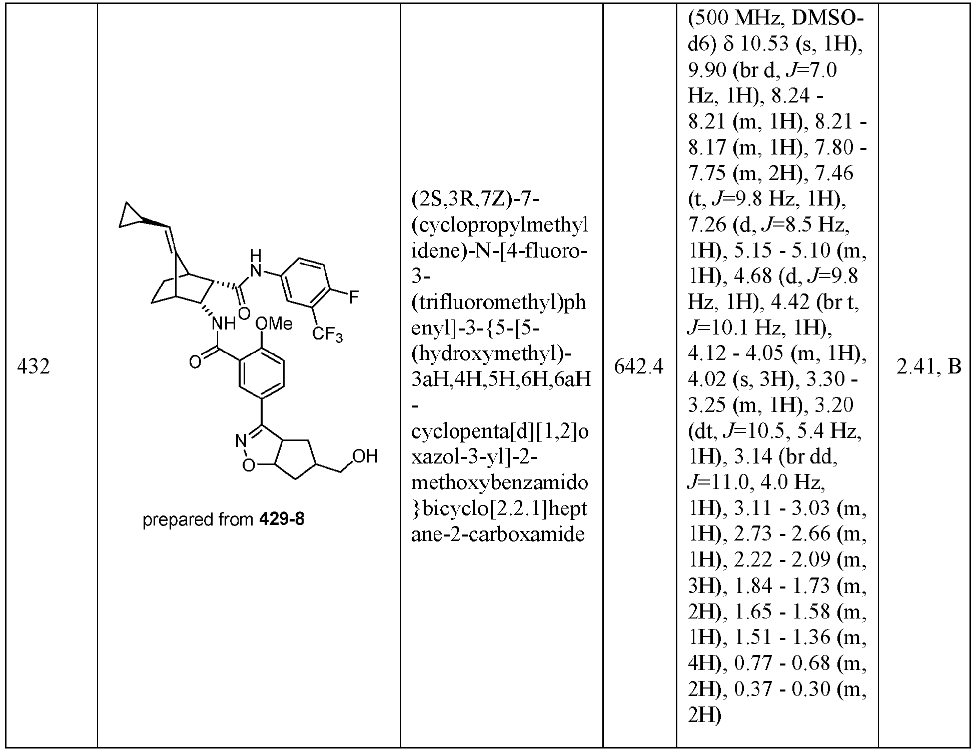

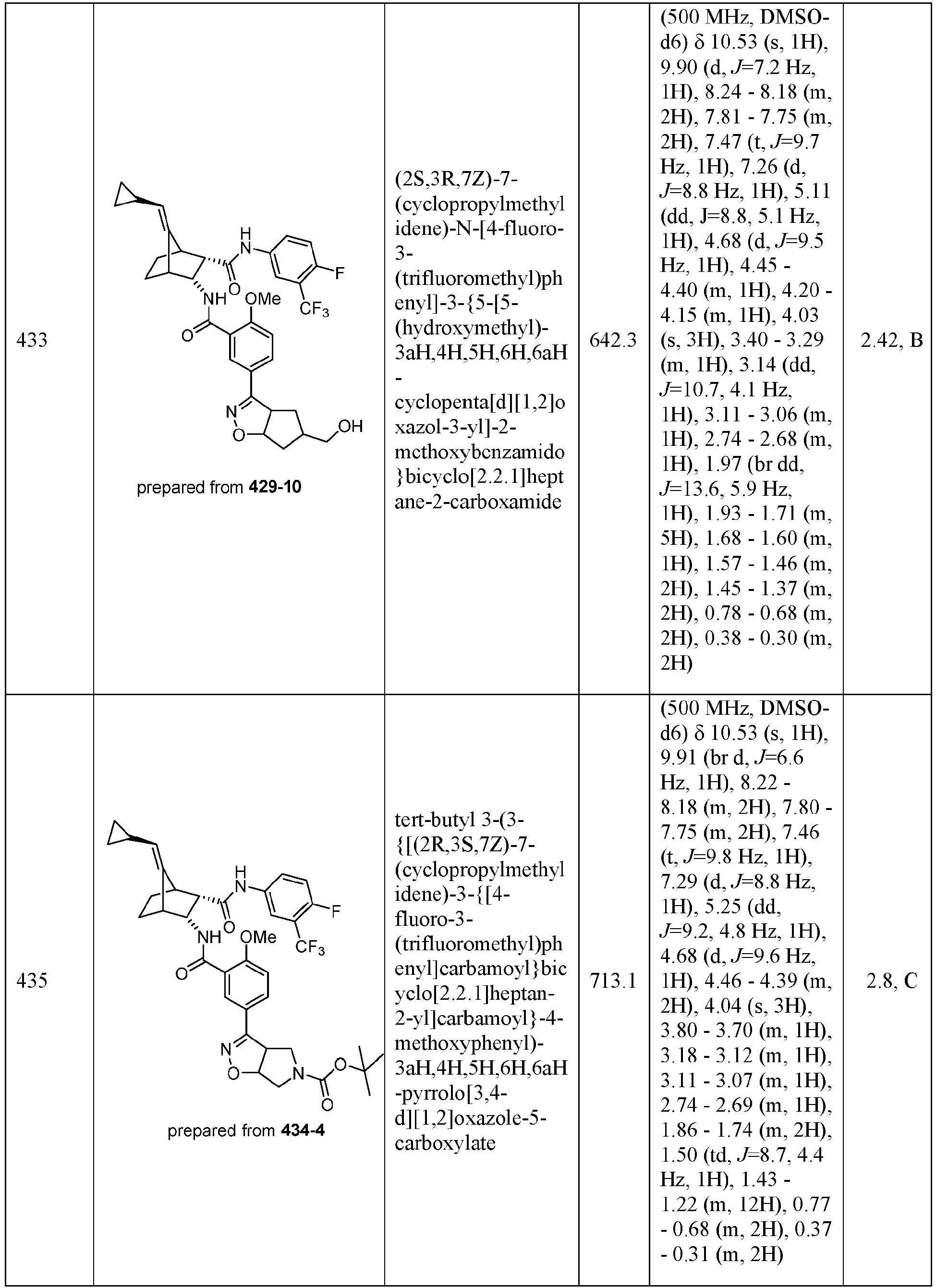

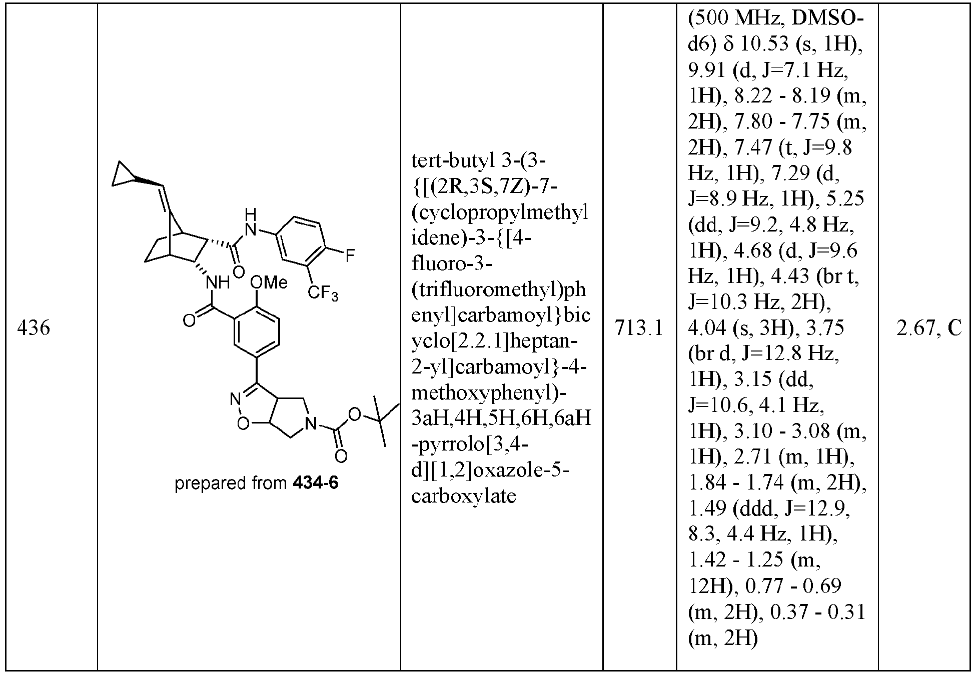

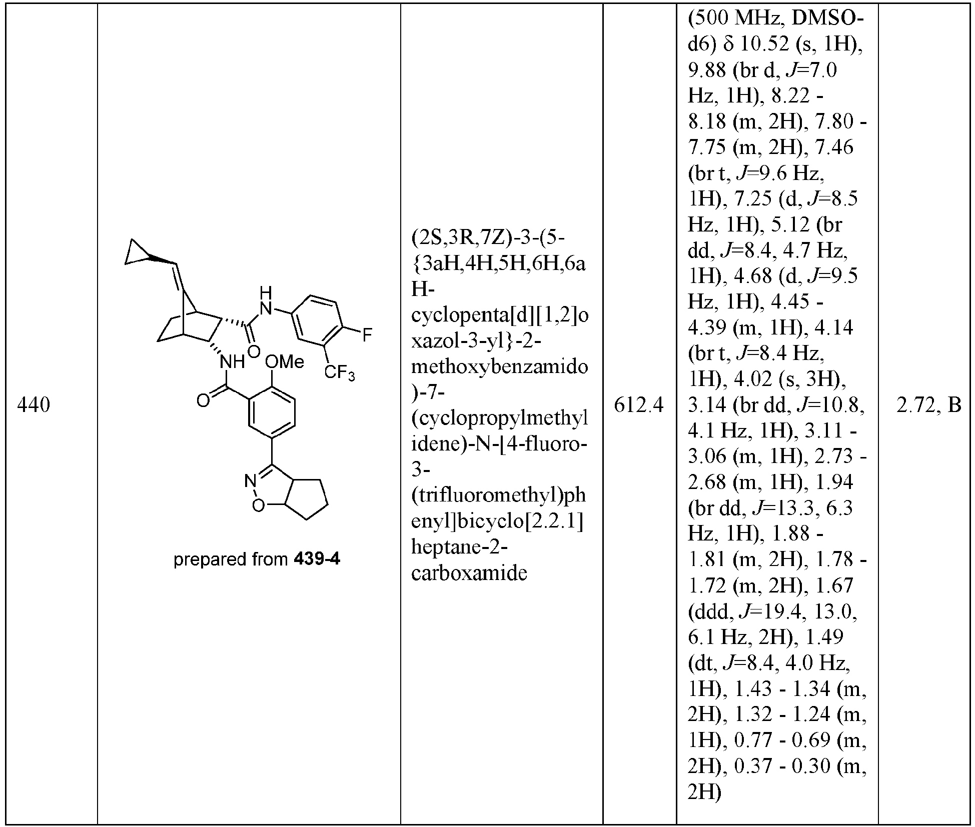

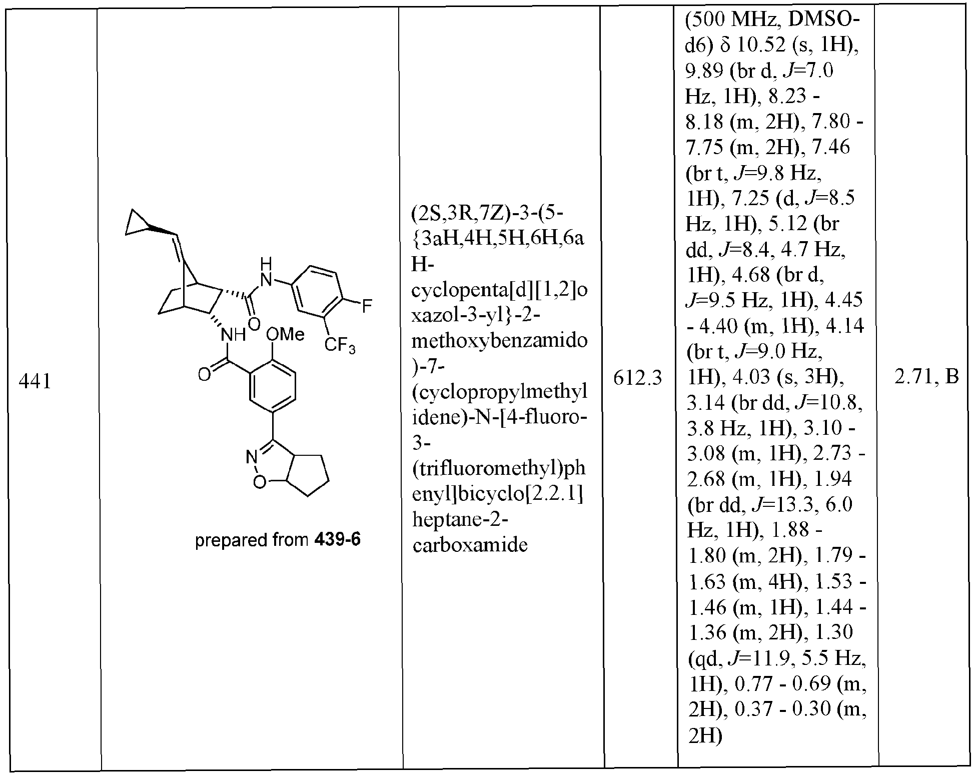

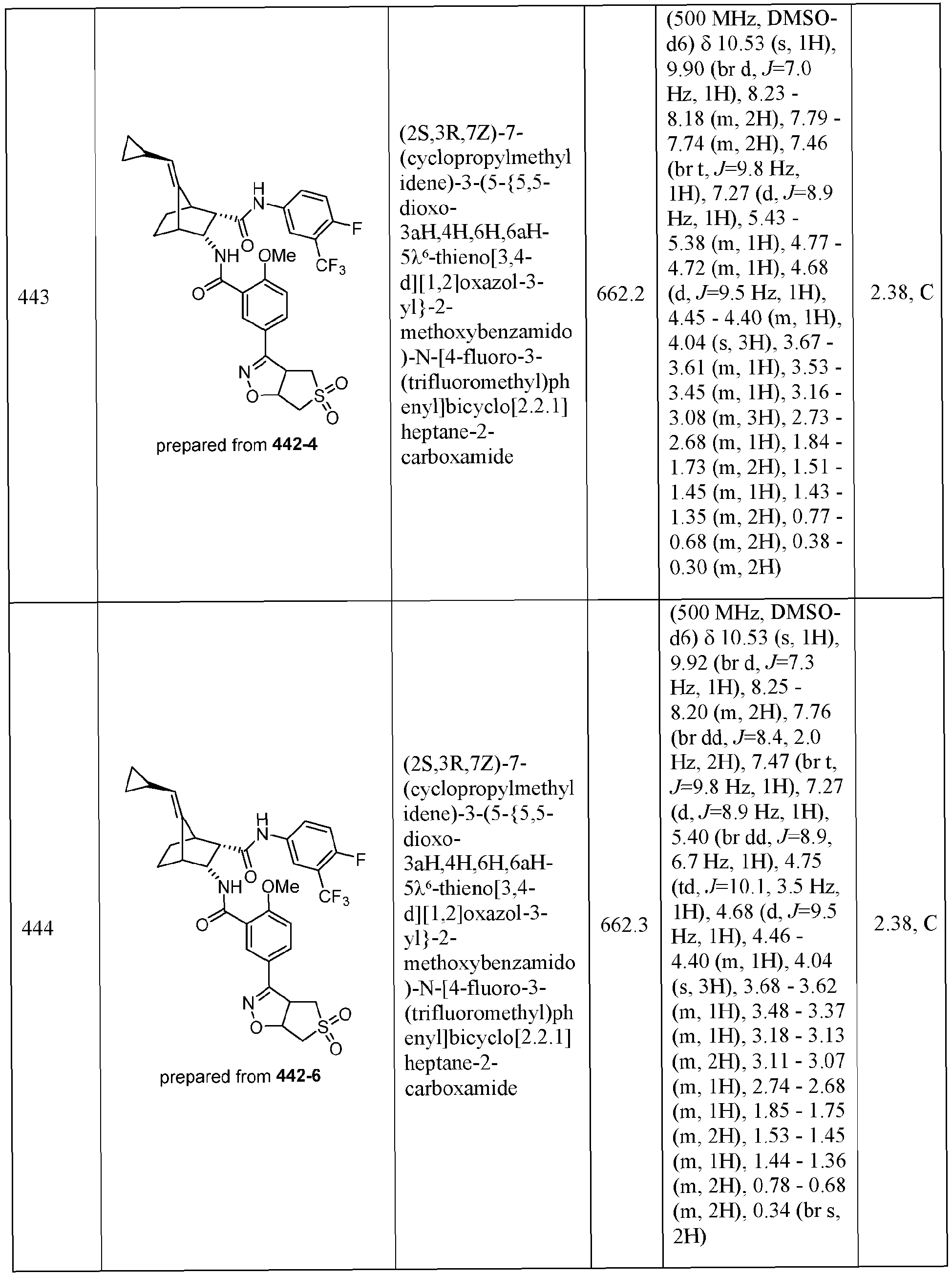

### Example 447

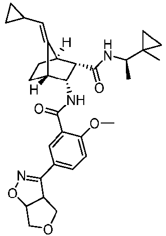

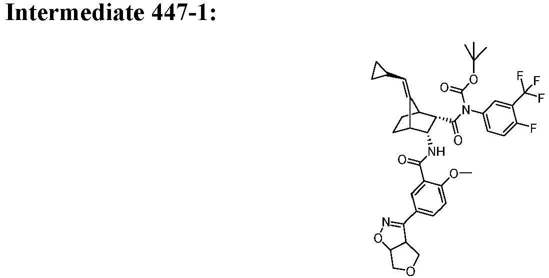

To a solution of (1R,2S,3R,4R,Z)-7-(cyclopropylmethylene)-N-(4-fluoro-3-(trifluoromethyl)phenyl)-3-(2-methoxy-5-(3a,4,6,6a-tetrahydrofuro[3,4-d]isoxazol-3-yl)benzamido)bicyclo[2.2.1]heptane-2-carboxamide (250 mg, 0.407 mmol, **Example 417)** in DCM (4.1 mL) was added Boc₂O (0.38 mL, 1.6 mmol), Hunig's base (0.28 µl, 1.6 mmol), and DMAP (25 mg, 0.20 mmol). The reaction mixture was stirred for 14 h, then concentrated in vacuo. The residue was purified via silica gel chromatography to furnish tert-butyl ((1R,2S,3R,4R,Z)-7-(cyclopropylmethylene)-3-(2-methoxy-5-(3a,4,6,6a-tetrahydrofuro[3,4-d]isoxazol-3-yl)benzamido)bicyclo[2.2.1]heptane-2-carbonyl)(4-fluoro-3-(trifluoromethyl)phenyl)carbamate (242 mg, 0.339 mmol, 83.0 % yield). LC-MS RT: 1.20 min; MS (ESI) *m*/*z* 736 (M+Na)⁺; Method E.

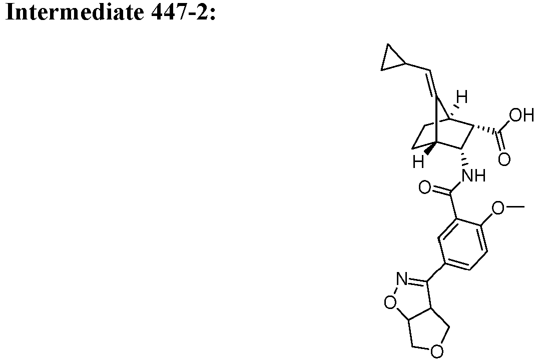

To a solution of **Intermediate 447-1** (533 mg, 0.747 mmol) in THF (14 mL) was added LiOH (1M aqueous) (3.7 mL, 3.7 mmol). After 3 hours, the reaction mixture was diluted with water and extracted twice with EtOAc. The organic layers were reextracted with 1M NaOH, then the aqueous layer was acidified to ca. pH 1 with 1M HCl. The precipitated solid was filtered off, then the filtrate was extracted twice with EtOAc. The organic fractions were combined with the solid and concentrated under reduced pressure to give (1R,2S,3R,4R,Z)-7-(cyclopropylmethylene)-3-(2-methoxy-5-(3a,4,6,6a-tetrahydrofuro[3,4-d]isoxazol-3-yl)benzamido)bicyclo[2.2.1]heptane-2-carboxylic acid **Example 447-2** (294 mg, 0.650 mmol, 87.0 % yield). LC-MS RT:0.75 min; MS (ESI) *m*/*z* 453 (M+H)⁺; Method E.

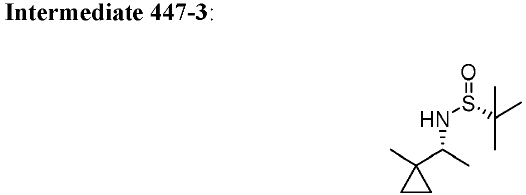

In each of two 40 mL pressure vials, 1-(1-methylcyclopropyl)ethan-1-one (0.500 g, 5.09 mmol) was dissolved in THF (10 ml). To this solution was added titanium(IV) ethoxide (2.1 ml, 10 mmol) and (R)-2-methylpropane-2-sulfinamide (0.617 g, 5.09 mmol). The reaction mixture was heated to 65 °C for 14 h then allowed to cool to room temperature. A suspension of sodium borohydride (1.54 g, 40.8 mmol) in 7 mL THF was cooled to -50 °C. The vials containing the reaction solutions were cooled to -50 °C. The reaction solutions were transferred to the sodium borohydride solution. After two hours, the reaction mixture was quenched with MeOH. After gas evolution ceased, the reaction mixture was poured into brine with stirring. The suspension was filtered through celite, rinsing with EtOAc. The filtrate was concentrated under reduced pressure. The residue was purified via silica gel chromatography to give **Intermediate 447-3** (982 mg, 47%). ¹H NMR (400 MHz, CDCl₃) δ 3.22 (br s, 1H), 2.61 (qd, *J*=6.5, 3.2 Hz, 1H), 1.26 - 1.21 (m, 12H), 1.01 (s, 3H), 0.54 - 0.46 (m, 1H), 0.42 - 0.32 (m, 3H).

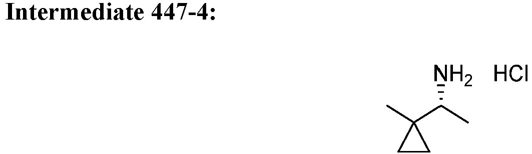

To a solution of **Intermediate 447-3** (100 mg, 0.492 mmol) in methanol (0.49 mL) was added HCl (4M in dioxane) (0.12 mL, 0.49 mmol). After 2.75 hours, the reaction mixture was concentrated in vacuo. The residue was sonicated with Et₂O and filtered. The solid was dried to give (R)-1-(1-methylcyclopropyl)ethan-1-amine, HCl (60 mg, 0.44 mmol, 90 % yield). ¹H NMR (400 MHz, CD₃OD) δ 2.66 - 2.52 (m, 1H), 1.38 - 1.27 (m, 3H), 1.09 (s, 3H), 0.65 - 0.55 (m, 1H), 0.55 - 0.41 (m, 3H).

### Example 447:

To a solution of **Intermediate 447-2** (20 mg, 0.044 mmol) in DMF (0.5 mL) was added **Intermediate 447-4** (24 mg, 0.18 mmol), HATU (22 mg, 0.057 mmol) and Hunig's base (0.046 mL, 0.27 mmol). After 1.5 hours, the reaction mixture was quenched with MeOH. The residue was purified via preparative HPLC to furnish **Example 447** (15.2 mg, 64.0%). ¹H NMR (400 MHz, DMSO-d₆) δ 10.07 (br d, *J*=6.7 Hz, 1H), 8.15 (d, *J*=2.4 Hz, 1H), 7.92 (d, *J*=8.6 Hz, 1H), 7.78 (dd, *J*=8.6, 2.4 Hz, 1H), 7.23 (d, *J*=8.8 Hz, 1H), 5.34 (dd, *J*=9.4, 3.5 Hz, 1H), 4.63 (d, *J*=9.4 Hz, 1H), 4.54 - 4.47 (m, 1H), 4.31 - 4.23 (m, 1H), 4.09 (d, *J*=10.9 Hz, 1H), 4.00 (s, 3H), 3.90 (d, *J*=10.0 Hz, 1H), 3.77 (dd, *J*=9.3, 6.7 Hz, 1H), 3.65 (dd, *J*=10.7, 3.6 Hz, 1H), 3.50 - 3.42 (m, 1H), 3.07 - 3.02 (m, 1H), 2.94 (dd, *J*=11.0, 4.0 Hz, 1H), 1.91 - 1.81 (m, 1H), 1.78 - 1.68 (m, 1H), 1.52 - 1.43 (m, 1H), 1.40 - 1.30 (m, 2H), 1.05 - 0.99 (m, 3H), 0.99 - 0.94 (m, 3H), 0.77 - 0.66 (m, 2H), 0.58 - 0.47 (m, 1H), 0.36 - 0.27 (m, 2H), 0.17 (br t, *J*=6.6 Hz, 2H). LC-MS RT: 2.26 min; MS (ESI) *m*/*z* 534.3 (M+H)⁺; Method B.

### Example 448

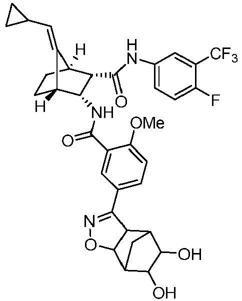

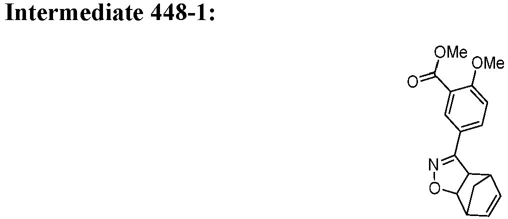

Methyl (Z)-5-(chloro(hydroxyimino)methyl)-2-methoxybenzoate (200 mg, 0.821 mmol) and bicyclo[2.2.1]hepta-2,5-diene (756 mg, 8.21 mmol) were dissolved in DCM (5 mL). To the solution was added TEA (1 mL) and stirred at r.t. for 14h. The reaction mixture was quenched with water (50 mL) and extracted with EtOAc (2x25 mL), which were then dried (MgSO₄), filtered and concentrated to an oil in vacuo. The residue was purified via silica gel chromatography and eluted with hex/EtOAc. Fractions containing pure product were isolated and concentrated under reduced pressure to methyl 2-methoxy-5-(3a,4,7,7a-tetrahydro-4,7-methanobenzo[d]isoxazol-3-yl)benzoate **448-1** (240 mg, 98% yield). ¹H NMR (500 MHz, CDCl₃) δ 8.09 (d, J=2.3 Hz, 1H), 7.88 (dd, J=8.9, 2.3 Hz, 1H), 7.02 (d, J=8.7 Hz, 1H), 6.36 (dd, J=5.7, 3.0 Hz, 1H), 6.10 (dd, J=5.8, 3.2 Hz, 1H), 4.97 (dt, J=8.2, 1.2 Hz, 1H), 3.94 (s, 3H), 3.91 (s, 3H), 3.29 - 3.25 (m, 1H), 1.71 (d, J=9.5 Hz, 1H), 1.62 (dt, J=9.4, 1.5 Hz, 1H). LCMS m/z = 300.2 (M+H)⁺.

### Preparation of intermediates homochiral isomers 448-2 and 448-3.

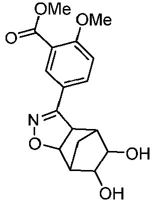

Intermediate **448-1** (180 mg, 0.60 mmol) was stirred as a slurry in t-BuOH (3 mL), and to the solution was added *N*-methylmorpholine oxide (146 mg, 1.20 mmol) followed by OsO₄ (380 mg, 0.060 mmol). The reaction turned black instantaneously and was stirred at r.t. for 14h. The reaction mixture was quenched with sat. soduim sulfite solution (20 mL), stirred for 5 min and extracted with EtOAc (2x25 mL), dried (MgSO₄), filtered and evaporated under reduced pressure to methyl 5-(5,6-dihydroxy-3a,4,5,6,7,7a-hexahydro-4,7-methanobenzo[d]isoxazol-3-yl)-2-methoxybenzoate as a foam (220 mg). The compound was separated into two homochiral isomers **448-2** and **448-3** via chiral SFC. Preparative Chromatographic Conditions: Instrument: Berger MG II (CTR-L409-PSFC1) Column: Chiralpak IF, 21 x 250 mm, 5 micron Mobile Phase: 30% MeOH / 70% CO₂ Flow Conditions: 45 mL/min, 150 Bar, 40°C Detector Wavelength: 220 nm. Injection Details: 1.0 mL of ~200 mg/3mL in MeOH-ACN. Analytical Chromatographic Conditions: Instrument: Shimadzu Nexera SFC (CTR-L410-SFC3) Column: Chiralpak IF, 4.6 x 100 mm, 3 micron. Mobile Phase: 30% MeOH / 70% CO₂. Flow Conditions: 2.0 mL/min, 150 Bar, 40°C. Detector Wavelength: 220 nm. Injection Details: 5 µL of ~1mg/mL in MeOH. Two homochiral isomers were isolated **448-2** Isomer-1 > 99% ee, RT = 2.89min (84 mg, 49 % yield) and **448-3** Isomer-2 > 99% ee, RT = 6.03 min (78 mg, 46 % yield). ¹H NMR (500 MHz, CDCl₃) δ 8.07 - 8.04 (m, 1H), 7.89 - 7.87 (m, 1H), 7.05 - 7.00 (m, 1H), 4.67 - 4.59 (m, 1H), 3.96 (s, 3H), 3.93 (s, 3H), 3.91 - 3.83 (m, 1H), 3.52 - 3.41 (m, 1H), 2.64 - 2.58 (m, 1H), 2.52 - 2.49 (m, 1H), 1.89 - 1.82 (m, 1H), 1.50 - 1.44 (m, 1H). LCMS m/z = 334.2 (M+H)⁺.

### Preparation of intermediate 448-4.

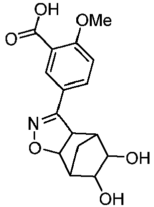

Methyl 5-(5,6-dihydroxy-3a,4,5,6,7,7a-hexahydro-4,7-methanobenzo[d]isoxazol-3-yl)-2-methoxybenzoate **448-3** (> 99% ee) was dissolved in methanol (3 mL) and to the solution was added LiOH (20 mg, 0.47 mmol) followed by the addition of water (2 mL). The reaction mixture was stirred at r.t. for 14 h, then quenched with water (25 mL). The organics were extracted with EtOAc (2x25 mL), dried (MgSO₄) and evaporated in vacuo to yield 5-(5,6-dihydroxy-3a,4,5,6,7,7a-hexahydro-4,7-methanobenzo[d]isoxazol-3-yl)-2-methoxybenzoic acid **448-4** (63 mg, 84 % yield). LCMS m/z = 320.3 (M+H). ¹H NMR (500 MHz, CD₃OD) δ 8.17 - 8.12 (m, 1H), 7.90 (dd, J=8.7, 2.3 Hz, 1H), 7.23 (d, J=8.9 Hz, 1H), 4.81 - 4.61 (m, 1H), 3.97 (s, 3H), 3.92 (m. 1H), 3.78 - 3.75 (m, 1H), 3.71 - 3.58 (m, 1H), 2.4 5 (s, 1H), 2.35 (s, 1H), 1.84 (br d, J=11.1 Hz, 1H), 1.36 - 1.15 (m, 1H).

**Example 448.** Intermediate **448-4** (17 mg, 0.050 mmol) was prepared using the general procedure described for example **378** to (1R,2S,3R,4R,Z)-3-amino-7-(cyclopropylmethylene)-N-(4-fluoro-3-(trifluoromethyl) phenyl) bicyclo[2.2.1]heptane-2-carboxamide intermediate **166-2** (15 mg, 0.050 mmol) with BOP (10 mg, 0.050 mmol) reagent,and Hunig's base (0.1 mL). **Example 448** was isolated as a solid (18 mg, 58 % yield) via HPLC purification. HPLC purity: 98.3 %; RT= 2.35 min, [method D ]. LCMS m/z = 670.3 (M+H)⁺. ¹H NMR (500 MHz, CD₃OD) δ 8.41 - 8.36 (m, 1H), 8.16 (dd, J=6.6, 2.4 Hz, 1H), 7.91 (dd, J=8.7, 2.4 Hz, 1H), 7.84 - 7.74 (m, 1H), 7.32 - 7.22 (m, 2H), 4.85 - 4.82 (m, 1H), 4.79 - 4.70 (m, 1H), 4.67 - 4.51 (m, 1H), 4.14 (s, 3H), 3.92 (br d, J=6.9 Hz, 1H), 3.76 (d, J=5.6 Hz, 1H), 3.65 (d, J=8.1 Hz, 1H), 3.56 - 3.42 (m, 1H), 3.31 - 3.22 (m, 2H), 3.21 - 3.06 (m, 2H), 2.73 (br d, J=4.3 Hz, 1H), 2.45 (s, 1H), 2.35 (s, 1H), 2.06 - 1.97 (m, 1H), 1.97 - 1.91 (m, 1H), 1.83 (br d, J=11.1 Hz, 1H), 1.62 - 1.48 (m, 2H), 1.38 - 1.21 (m, 1H), 0.77 (br d, J=4.4 Hz, 2H), 0.37 (br s, 2H).

### Example 449

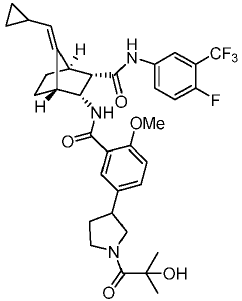

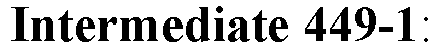

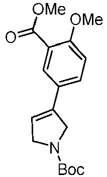

Methyl 5-bromo-2-methoxybenzoate (2.0 g, 8.2 mmol) and tert-butyl 3-(4,4,5,5-tetramethyl-1,3,2-dioxaborolan-2-yl)-2,5-dihydro-1H-pyrrole-1-carboxylate (2.65 g, 8.98 mmol) in dioxane (55 mL)/water (10 mL) was degassed 5 min, then PdCl₂(dppf)-CH₂Cl₂ adduct (0.666 g, 0.816 mmol) and K₃PO₄ (5.64 g, 24.5 mmol) were added and the reaction mixture again degassed for 5 min, then the slurry was stirred at 100 °C for 4 h in a sealed tube. The reaction mixture was diluted with EtOAc, the solid filtered, washed with excess EtOAc and the filtrate was collected and concentrated under reduced pressure. The residue was purified by silica gel chromatography to furnish tert-butyl 3-(4-methoxy-3-(methoxycarbonyl)phenyl)-2,5-dihydro-1H-pyrrole-1-carboxylate (2.2 g, 6.60 mmol, 81 % yield) compound as a light brown solid. MS (ES): m/z = 234.2 [M+H-Boc].

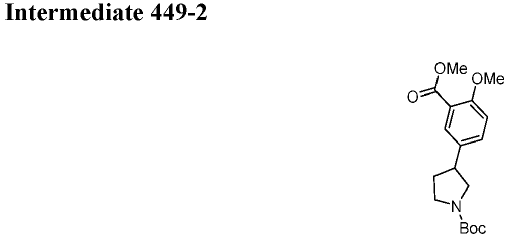

To a solution of **449-1** (2.2 g, 6.6 mmol) in MeOH (100 mL) was added Pd-C (1.756 g, 16.50 mmol) at rt, and the slurry was stirred under hydrogen atmosphere for 12 h. The reaction mixture was filtered through celite, the celite washed with excess methanol and THF, the filtrates were collected and concentrated under reduced pressure to yield tert-butyl 3-(4-methoxy-3-(methoxycarbonyl)phenyl) pyrrolidine-1-carboxylate (1.7 g, 5.1 mmol, 77 % yield) as a light brown semisolid. MS (ES): m/z = 353.2 [M+H₂O].

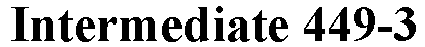

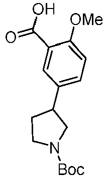

LiOH (1.43 g, 59.6 mmol) in water (5.0 mL) was added to a solution of **449-2** (2.0 g, 6.0 mmol) in methanol (10 mL), THF (10 mL), the resulting reaction mixture was stirred at rt for 5h. The volatiles were evaporated and dried under high vacuum. The residue was diluted with ice water (10 mL), then acidified using 0.1M HCl. A solid formed and was filtered, washed with water and dried under vacuum to yield 5-(1-(tert-butoxycarbonyl)pyrrolidin-3-yl)-2-methoxybenzoic acid (1.5 g, 4.7 mmol, 78 % yield) compound as a white solid. MS (ES): m/z = 320.2 [M-H].

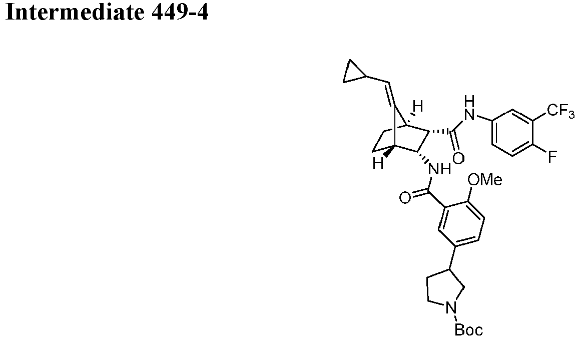

HATU (206 mg, 0.543 mmol) and TEA (0.38 mL, 2.7 mmol) were added to a solution of **449-2** (349 mg, 1.09 mmol) and **166-2** (200 mg, 0.543 mmol) in DMF (30 mL) at rt, the reaction mixture was stirred for 3h. The volatiles were removed under reduced pressure and the residue was purified by silica gel chromatography to furnish tert-butyl 3-(3-(((1R,2R,3R,4R,Z)-7-(cyclopropylmethylene)-3-((4-fluoro-3-(trifluoromethyl)phenyl) carbamoyl)bicyclo[2.2.1]heptan-2-yl)carbamoyl)-4-methoxyphenyl)pyrrolidine-1-carboxylate (190 mg) as an off white solid. The solid subjected to SFC purification: Column Name: Welk-01(R,R) (250*4.6)mm. 5µ, Co-Solvent : 20% Vial N° : LA8 Co-Solvent Name : 0.2% of Ammonia in Methanol Injected Volume : 30 µl FlowRate : 4 ml/min Outlet Pressure: 100 bar Temperature : 35 °C, to separatethe diastereomers.

After SFC purification, the fractions were collected, concentrated under reduced pressure and lyophilized to generate tert-butyl 3-(3-(((1R,2R,3S,4R,Z)-7-(cyclopropylmethylene)-3-((4-fluoro-3-(trifluoromethyl) phenyl)carbamoyl)bicyclo[2.2.1]heptan-2-yl)carbamoyl)-4-methoxyphenyl) pyrrolidine-1-carboxylate.

Peak - 1, **449-4,** (55 mg, 0.082 mmol, 14% yield) Chiral SFC RT - 9.9 min, ¹H NMR (400MHz, DMSO-d6) δ ppm 10.50 (s, 1H), 9.82 (d, J=7.0 Hz, 1H), 8.22 (dd, J=2.5, 6.0 Hz, 1H), 7.82 (d, J=2.5 Hz, 2H), 7.48 (t, J=10.0 Hz, 1H), 7.43 (dd, J=2.5, 8.5 Hz, 1H), 7.14 (d, J=8.5 Hz, 1H), 6.88 (s, 1H), 4.69 (d, J=10.0 Hz, 1H), 3.97 (s, 3H), 3.68 (dd, J=7.5, 10.0 Hz, 1H), 3.49 - 3.36 (m, 5H), 3.31 - 3.21 (m, 5H), 3.20 - 3.05 (m, 4H), 2.73 - 2.69 (m, 1H), 2.19 (s, 3H), 1.86 (br d, J=10.5 Hz, 3H), 1.46 - 1.33 (m, 20H), 0.74 (br t, J=8.3 Hz, 2H), 0.35 (br d, J=2.5 Hz, 2H); LCMS: RT = 1.565 min, MS (ES):m/z = 616.4 [M+H-tBu] method B; tert-butyl 3-(3-(((1R,2R,3S,4R,Z)-7-(cyclopropylmethylene)-3-((4-fluoro-3-(trifluoromethyl) phenyl) carbamoyl)bicyclo[2.2.1]heptan-2-yl)carbamoyl)-4-methoxyphenyl) pyrrolidine-1-carboxylate (55 mg, 0.082 mmol, 15 % yield) as a white solid.

Peak - 2, (60 mg, 0.089 mmol, 16% yield) Chiral SFC RT - 10.98 min, ¹H NMR (400MHz, DMSO-d6) δ ppm 10.49 (s, 1H), 9.81 (d, J=7.0 Hz, 1H), 8.22 (dd, J=2.5, 6.5 Hz, 1H), 7.82 (d, J=2.5 Hz, 1H), 7.76 (br s, 1H), 7.48 (t, J=9.8 Hz, 1H), 7.42 (dd, J=2.5, 8.5 Hz, 1H), 7.13 (d, J=8.5 Hz, 1H), 4.68 (d, J=9.5 Hz, 1H), 4.44 (br s, 1H), 3.97 (s, 3H), 3.67 (dd, J=7.5, 10.0 Hz, 1H), 3.49 - 3.34 (m, 2H), 3.29 - 3.06 (m, 4H), 2.72 - 2.67 (m, 1H), 2.56 - 2.52 (m, 2H), 2.18 (s, 2H), 1.86 (br t, J=9.3 Hz, 2H), 1.76 (br s, 1H), 1.50 (br d, J=4.5 Hz, 1H), 1.45 - 1.33 (m, 17H), 0.74 (br t, J=9.0 Hz, 2H), 0.34 (br s, 2H); LCMS: RT = 1.716 min, MS (ES):m/z = 616.3 [M+H-tBu] method B.

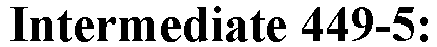

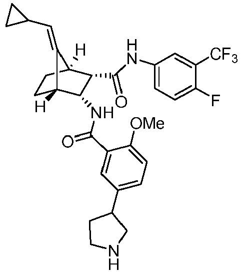

TFA (0.16 mL, 2.0 mmol) was added to a solution of **449-4** (55 mg, 0.082 mmol) in DCM (5.0 mL) at 0 °C, then the reaction mixture was stirred at rt for 2 h. The volatiles were removed under reduced pressure and the residue dried under vacuum to yield (1R,2S,3R,4R,Z)-7-(cyclopropylmethylene)-N-(4-fluoro-3-(trifluoromethyl)phenyl)-3-(2-methoxy-5-(pyrrolidin-3-yl)benzamido)bicyclo[2.2.1]heptane-2-carboxamide (35 mg) as a light brown semi solid which was taken to next step without further purification. MS (ES): m/z = 572.2 [M+H].

### Example 449

HATU (13 mg, 0.035 mmol), DIPEA (0.06, 0.04mmol) were added to a **449-5** (20 mg, 0.035 mmol) and 2-hydroxy-2-methylpropanoic acid (3.6 mg, 0.035 mmol) in DMF (2.0 mL) at rt. The reaction mixture was stirred at for 15h and purified by preparative reverse phase HPLC to furnish (1R,2S,3R,4R,Z)-7-(cyclopropylmethylene)-N-(4-fluoro-3-(trifluoromethyl)phenyl)-3-(5-(1-(2-hydroxy-2-methylpropanoyl)pyrrolidin-3-yl)-2-methoxybenzamido)bicyclo[2.2.1] heptane-2-carboxamide (13 mg, 49%) as a white solid. ¹H NMR (400 MHz, DMSO-d6) δ ppm 10.48 (s, 1H), 9.83 (dd, *J* = 3.3, 6.7 Hz, 1H), 8.25 - 8.17 (m, 1H), 7.85 (br s, 1H), 7.77 (td, *J* = 3.6, 8.7 Hz, 1H), 7.48 (t, *J* = 9.9 Hz, 1H), 7.43 (dd, *J* = 2.2, 8.6 Hz, 1H), 7.14 (dd, *J* = 1.8, 7.9 Hz, 1H), 5.19 - 5.12 (m, 1H), 4.68 (d, *J* = 9.5 Hz, 1H), 4.48 - 4.40 (m, 1H), 4.27 (br d, *J* =0.7 Hz, 1H), 4.08 (q, *J* = 5.5 Hz, 1H), 3.97 (s, 4H), 3.78 - 3.65 (m, 1H), 3.58 - 3.47 (m, 1H), 3.24 - 3.06 (m, 4H), 2.74 - 2.68 (m, 1H), 2.13 (br d, *J* = 0.7 Hz,1H), 1.93 - 1.72 (m, 3H), 1.53 - 1.36 (m, 3H), 1.32 - 1.26 (m, 7H), 0.80 - 0.65 (m, 2H), 0.35 (dd, *J* = 1.8, 4.8 Hz, 2H). LCMS: RT = 2.465 min, MS (ES):*m*/*z* = 658.3 [M+H⁺] method B.

### Example 452

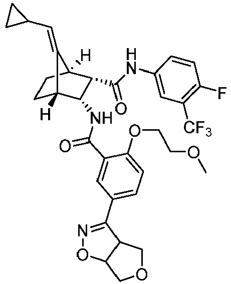

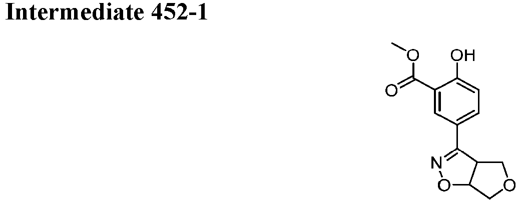

**452-1** (0.13 g, 0.49 mmol, 49 % yield) was prepared in a similar manner as example **416** substituting methyl 5-formyl-2-hydroxybenzoate for methyl 5-formyl-2-methoxybenzoate. ¹H NMR (400 MHz, CDCl₃) δ 10.98 (s, 1H), 8.04 (d, J=2.2 Hz, 1H), 7.82 (dd, J=8.8, 2.2 Hz, 1H), 7.04 (d, J=8.8 Hz, 1H), 5.38 (dd, J=9.1, 3.9 Hz, 1H), 4.36 - 4.30 (m, 1H), 4.30 - 4.25 (m, 1H), 4.14 (dd, J=9.2, 1.3 Hz, 1H), 3.99 (s, 3H), 3.89 (dd, J=9.4, 6.7 Hz, 1H), 3.79 (dd, J=10.8, 3.7 Hz, 1H). LCMS(ESI) *m*/*z:* 264 (M+H).⁺

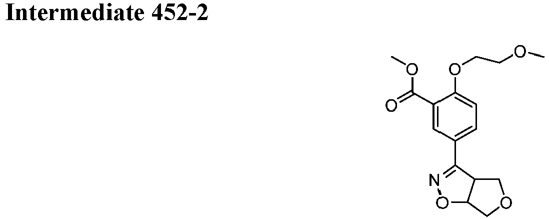

To **452-1** (0.1 g, 0.4 mmol) in DMF (2 mL) was added 1-chloro-2-methoxyethane (72 mg, 0.80 mmol), K₂CO₃ (0.16 g, 1.1 mmol), KI (63 mg, 0.38 mmol) and the reaction mixture was heated at 60 °C for 24h. The reaction mixture was partitioned with water (10 mL) and EtOAc (20 mL). The aqueous layer was extracted with EtOAc (2 x 20 mL), the combined organic layers were washed with brine (15 mL) and dried (MgSO₄), filtered and concentrated under reduced pressure. The residue was purified by normal phase silica gel chromatography to afford **452-2** (62 mg, 0.20 mmol, 51 % yield) clear oil. ¹H NMR (500 MHz, CDCl₃) δ 7.99 (d, *J*=2.3 Hz, 1H), 7.87 (dd, *J*=8.8, 2.4 Hz, 1H), 7.07 (d, *J*=8.7 Hz, 1H), 5.39 (dd, *J*=9.2, 3.9 Hz, 1H), 4.37 - 4.32 (m, 1H), 4.32 - 4.28 (m, 1H), 4.28 - 4.24 (m, 2H), 4.16 (dd, *J*=9.4, 1.3 Hz, 1H), 3.93 - 3.92 (m, 3H), 3.91 - 3.87 (m, 1H), 3.84 (dd, *J*=5.3, 4.3 Hz, 2H), 3.80 (dd, *J*=10.8, 3.9 Hz, 1H), 3.50 (s, 3H). LCMS(ESI) *m*/*z*: 322 (M+H).⁺

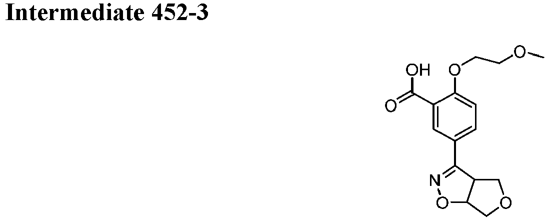

**452-3** (41 mg, 0.13 mmol, 72 % yield) was prepared by hydrolysis of **452-2** as described in **378-3.** ¹H NMR (500 MHz, CDCl₃) δ 8.23 - 8.07 (m, 2H), 7.20 - 7.07 (m, 1H), 5.42 (dd, *J*=9.2, 3.9 Hz, 1H), 5.32 (s, 1H), 4.46 - 4.39 (m, 2H), 4.36 - 4.30 (m, 2H), 4.14 (d, *J*=9.5 Hz, 1H), 3.93 - 3.88 (m, 1H), 3.85 - 3.82 (m, 2H), 3.78 (dd, *J*=10.9, 3.9 Hz, 1H), 3.49 (s, 3H).

**Example 452.** A mixture of isomers was prepared by BOP coupling as described in example **378** substituting cyclopropyl norbornyl intermediate **166-2** and intermediate **452-3** for intermediate **378-3** and the cyclobutyl norbornyl intermediate **369-1.** The isomeric mixture was separated using chiral SFC chromatography. Preparative chromatographic conditions Instrument: Waters 100 Prep SFC Column: Chiral OD, 30 x 250 mm, 5 micron, Mobile Phase: 25% MeOH / 75% CO2 w/0.1% DEA, Flow Conditions: 100 mL/min, Detector Wavelength: 220 nm; Analytical method:Intrument: Shimadzu Nexera SFC, Column Chiral OD, 4.6 x 100 mm, 5 micron, Mobile Phase: 25% MeOH / 75% CO2 w/0.1% DEA, Flow Conditions: 2 mL/min, Detector Wavelength: 220 nm, to afford chiral peak-1, example **452** (9.3 mg, 14 µmol, 23 % yield), RT=2.92 min., >95% de and peak-2 (9.3 mg, 14 µmol, 23 % yield), RT =3.6 min, >95% de. For example **452:** ¹H NMR (500 MHz, DMSO-d6) δ 10.49 (s, 1H), 9.60 (br d, J=7.9 Hz, 1H), 8.37 - 8.10 (m, 2H), 7.86 - 7.68 (m, 2H), 7.47 (t, J=9.9 Hz, 1H), 7.32 (d, J=8.9 Hz, 1H), 5.35 (dd, J=9.2, 3.7 Hz, 1H), 4.71 (d, J=9.5 Hz, 1H), 4.61 - 4.47 (m, 2H), 4.43 (br d, J=4.0 Hz, 2H), 4.11 (d, J=10.7 Hz, 1H), 4.07 - 3.98 (m, 1H), 3.95 - 3.86 (m, 1H), 3.83 - 3.74 (m, 1H), 3.66 (dd, J=10.7, 3.4 Hz, 1H), 3.28 (s, 1H), 3.21 - 3.13 (m, 1H), 3.09 - 2.99 (m, 1H), 2.74 (br s, 1H), 2.52 (br s, 3H), 2.03 - 1.84 (m, 2H), 1.63 - 1.51 (m, 1H), 1.48 - 1.31 (m, 1H), 0.89 - 0.67 (m, 2H), 0.46 - 0.27 (m, 2H). LCMS(ESI) *m*/*z*: 658.15(M+H).⁺ HPLC purity 100 % with retention time 2.42 min. [method C ]

### Example 461

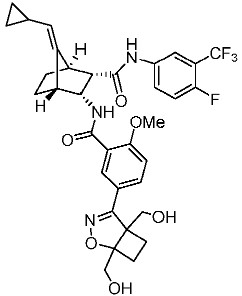

### Preparation of diasteromeric intermediate 461-1.

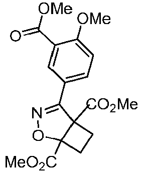

Diasteromeric intermediate **461-1** (200 mg, 80%) was prepared in a similar manner as described for **example 378** by the cycloadditon of dimethyl cyclobut-1-ene-1,2-dicarboxylate with Methyl (Z)-5-(chloro(hydroxyimino)methyl)-2-methoxybenzoate. LCMS m/z = 378.2 (M+H). ¹H NMR (500 MHz, CDCl₃) δ 8.12 (d, J=2.4 Hz, 1H), 7.75 (dd, J=8.9, 2.4 Hz, 1H), 7.00 (d, J=8.9 Hz, 1H), 3.96 (s, 3H), 3.92 (s, 3H), 3.87 (s, 3H), 3.68 (s, 3H), 3.21 - 3.09 (m, 1H), 2.79 - 2.67 (m, 1H), 2.62 - 2.52 (m, 2H).

### Preparation of homochiral intermediates 461-3 and 461-4.

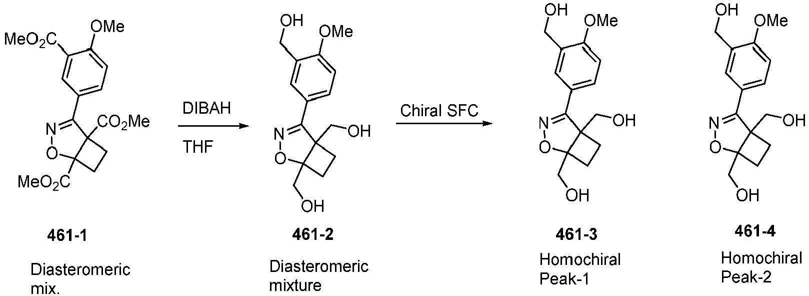

Diastermeric intermediate **461-1** (112 mg, 0.29 mmol) was dissolved in THF (10 mL) and to this was added DIBAH (1M, 2.08 mL, 2.08 mmol) solution. The reaction mixture was stirred at rt for 14 h and was subsequently quenched with dil HCl (5 mL) followed by extraction of the organic material with EtOAc (2 x 25 mL). The combined organic layers were dried (MgSO₄), filtered and concentrated under reduced pressure to afford the diasteromer (4-(3-(hydroxymethyl)-4-methoxyphenyl)-2-oxa-3-azabicyclo[3.2.0]hept-3-ene-1,5-diyl)dimethanol **461-2** as an oil (100 mg, 100% yield). ¹H NMR (500 MHz, CDCl₃) δ 7.69 - 7.49 (m, 2H), 6.92 - 6.86 (m, 1H), 4.73 - 4.60 (m, 2H), 3.90 (s, 3H), 3.88 (s, 3H), 3.74 - 3.65 (m, 1H), 3.05 - 2.82 (m, 1H), 2.54 - 2.32 (m, 2H), 2.19 - 1.97 (m, 2H), 1.95 - 1.37 (m, 1H), 1.33 - 0.84 (m, 1H). LCMS m/z = 294.2 (M+H)⁺. Intermediate **461-2** was chirally separated via chiral SFC conditions to afford homochiral intermediates **461-3** (Peak-1) and **461-4** (Peak-2). Preparative Chromatographic Conditions: Instrument: Berger MG II Column: Chiralpak IC, 21 x 250 mm, 5 micron Mobile Phase: 30% Methanol / 70% CO₂ Flow Conditions: 45 mL/min, 150 Bar, 40°C. Detector Wavelength: 220 nm Injection Details: 0.5 mL of ~55mg/mL in Methanol. Analytical Chromatographic Conditions: Instrument: Shimadzu Nexera SFC Column: Chiralpak IC, 4.6 x 100 mm, 3 micron Mobile Phase: 30% Methanol / 70% CO₂ Flow Conditions: 2.0 mL/min, 150 Bar, 40°C. Detector Wavelength: 220 nm Injection Details: 5 µL of ~1mg/mL in Methanol. Analytical data for **461-3** Homochiral Peak - 1 (57 mg, 15 % yield > 99 % ee, RT = 2.98 min); ¹H NMR (500 MHz, CDCl₃) δ 7.64 (s, 1H), 7.60 (d, J=8.4 Hz, 1H), 6.91 (d, J=8.7 Hz, 1H), 4.69 (br s, 2H), 4.05 (m, 2H), 3.91 (s, 3H), 3.81 (br s, 1H), 3.50 (s, 1H), 3.33 (br s, 1H), 2.56 (br s, 1H), 2.50 - 2.39 (m, 2H), 2.20 - 1.99 (m, 2H), 1.70 (br s, 1). Analytical data for **461-4** Homochiral Peak - 2 (60 mg 60 % yield, > 99 % ee, RT = 5.72 min). ¹H NMR (500 MHz, CDCl₃) δ 7.64 (s, 1H), 7.60 (d, J=8.4 Hz, 1H), 6.91 (d, J=8.5 Hz, 1H), 4.69 (d, J=6.3 Hz, 2H), 4.08 (m, 2H), 3.91 (s, 3H), 3.84 - 3.76 (m, 1H), 3.50 (d, J=4.7 Hz, 2H), 3.33 (br s, 1H), 2.56 (br t, J=6.5 Hz, 1H), 2.50 - 2.39 (m, 2H), 2.20 - 1.99 (m, 2H), 1.70 (s, 1H).

### Preparation of homochiral isomer-1intermediate 461-4.

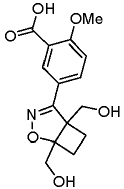

Homochiral isomer-1 intermediate **461-2** (4-(3-(hydroxymethyl)-4-methoxyphenyl)-2-oxa-3-azabicyclo[3.2.0]hept-3-ene-1,5-diyl)dimethanol (57 mg, 0.19 mmol) was dissolved in dry DCM (10 mL) and to the solution was added activated MnO₂ (847 mg, 9.72 mmol). The reaction mixture was stirred at rt for 14h. The reaction mixture was filtered and concentrated to an oil in vacuo. The oil was re-dissolved in t-BuOH (5 mL) and to the solution was added NaClO₂ (37 mg, 0.41 mmol) followed by an aq. solution of NaH₂PO₄ (5 mL) to pH ~3 and 2-methylbutene (10 mmol) was added. The reaction mixture was stirred at rt for 6 h then quenched with water (100 mL) and extracted with EtOAc (2x25 mL). The combined organic portion was dried (MgSO₄), filtered and evaporated in vacuo to yield isomer-1 intermediate **461-4** as an oil (50 mg, 55 % yield). ¹H NMR (500 MHz, CD₃OD) δ 8.15 (d, J=2.3 Hz, 1H), 7.88 (dd, J=8.8, 2.4 Hz, 1H), 7.17 (d, J=8.9 Hz, 1H), 4.79 - 4.58 (m, 4H), 3.97 (s, 3H), 3.95-3.88 (m, 2H), 3.51 - 3.34 (m, 2H), 2.63 - 1.96 (m, 2H). LCMS m/z = 308.2 (M+H)⁺.

**Example 461.** Homochiral isomer-1 intermediate **461-4** (4.3 mg, 0.02 mmol) was coupled to intermediate **166-2** (5.2 mg, 0.02 mmol) with BOP (6 mg, 0.02 mmol) reagent, and Hunig's base (0.1 mL) as described for example **378.** Example **461** was isolated as a solid after purification by revserse phase HPLC (4.3 mg, 45 % yield). HPLC purity: 100 %; RT= 2.43 min [method D]. LCMS m/z = 658.33 (M+H)⁺. ¹H NMR (500 MHz, DMSO-d6) δ 10.55 (s, 1H), 9.93 (br d, J=7.0 Hz, 1H), 8.30 - 8.21 (m, 2H), 7.85 - 7.75 (m, 2H), 7.50 (br t, J=9.8 Hz, 1H), 7.27 (br d, J=8.9 Hz, 1H), 5.38 (br t, J=5.3 Hz, 1H), 4.85 - 4.67 (m, 2H), 4.45 (br s, 1H), 4.06 (s, 3H), 3.92 - 3.78 (m, 3H), 3.71 (br dd, J=11.9, 7.0 Hz, 1H), 3.38 (br s, 1H), 3.17 (br d, J=7.3 Hz, 1H), 3.12 (br s, 1H), 2.74 (br s, 1H), 2.33 - 2.19 (m, 1H), 2.15 (br s, 3H), 2.09 (s, 1H), 1.93 (s, 1H), 1.89 - 1.68 (m, 2H), 1.52 (br d, J=8.5 Hz, 1H), 1.42 (br s, 2H), 0.87 - 0.67 (m, 2H), 0.36 (br s, 2H)

### Example 467

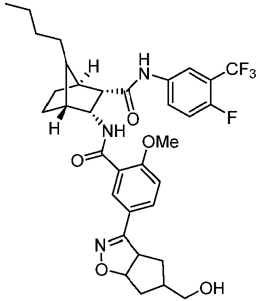

Compound **467** was prepared by reduction of **166-2** (6 mg, 0.02 mmol) with catlytic Pd/C (10%) followed by coupling of the resulting (1S,2S,3R,4R)-3-amino-7-butyl-N-(4-fluoro-3-(trifluoromethyl)phenyl)bicyclo[2.2.1]heptane-2-carboxamide with with **429-8** (4.72 mg, 0.0200 mmol) and BOP (7.16 g, 0.0200 mmol) and Hunig's base (0.1 mL) in DMF as described for Example **378.** The residue was purified via HPLC purification to generate **467** as a solid (0.7 mg, 7 % yield). HPLC purity: 100 %; RT= 2.72 min [method C]. LCMS m/z = 645.94 (M+H)+. ¹H NMR (500 MHz, DMSO-d₆) δ 10.64 (s, 1H), 8.37 (br d, *J*=7.3 Hz, 1H), 8.13 (br d, *J*=4.0 Hz, 1H), 7.94 (d, *J*=2.4 Hz, 1H), 7.87 (br s, 1H), 7.77 (br d, *J*=8.9 Hz, 1H), 7.50 (br t, *J*=9.6 Hz, 1H), 7.25 (d, *J*=8.9 Hz, 1H), 5.23 - 5.05 (m, 1H), 4.84 (br s, 1H), 4.23 (br t, *J*=9.2 Hz, 1H), 3.93 (s, 3H), 3.35 (br s, 1H), 2.77 - 2.54 (m, 2H), 2.43 - 2.27 (m, 2H), 2.17 - 1.95 (m, 2H), 1.95 - 1.86 (m, 1H), 1.80 (br d, *J*=12.8 Hz, 1H), 1.74 - 1.63 (m, 3H), 1.58 (br d, *J*=8.2 Hz, 1H), 1.54 - 1.44 (m, 2H), 1.36 (br d, *J*=6.7 Hz, 2H), 1.30 - 1.13 (m, 4H), 0.94 - 0.72 (m, 3H)

### Example 453

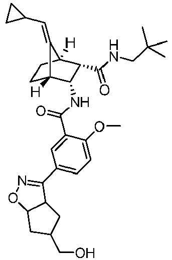

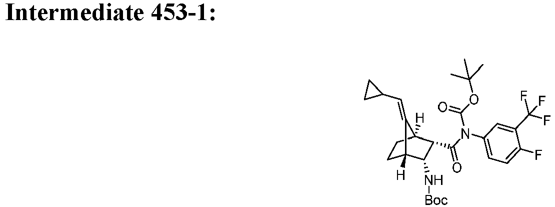

To a solution of (1R,2S,3R,4R,Z)-3-amino-7-(cyclopropylmethylene)-N-(4-fluoro-3-(trifluoromethyl)phenyl)bicyclo[2.2.1]heptane-2-carboxamide **IV-2a** (33 mg, 0.090 mmol) in DCM (0.9 mL) was added Boc₂O (0.10 mL, 0.45 mmol), Hunig'sbase (78 µl, 0.45 mmol) and DMAP (5.5 mg, 0.045 mmol). The reaction mixture was stirred for 14h, then concentrated under reduced pressure and purified via silica gel chromatography to furnish tert-butyl ((1R,2S,3R,4R,Z)-3-((tert-butoxycarbonyl)amino)-7-(cyclopropylmethylene)bicyclo[2.2.1]heptane-2-carbonyl)(4-fluoro-3-(trifluoromethyl)phenyl)carbamate **Intermediate 453-1** (43 mg, 0.076 mmol, 84 % yield) was obtained. LC-MS RT: 1.37 min; MS (ESI) *m*/*z* 591 (M+Na)⁺; Method A.

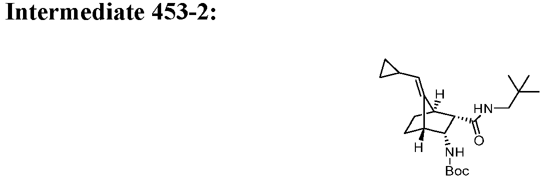

To a solution of tert-butyl ((1R,2S,3R,4R,Z)-3-((tert-butoxycarbonyl)amino)-7-(cyclopropylmethylene)bicyclo[2.2.1]heptane-2-carbonyl)(4-fluoro-3-(trifluoromethyl)phenyl)carbamate **Intermediate 453-1** (43 mg, 0.076 mmol) was added 2,2-dimethylpropan-1-amine (26.4 mg, 0.302 mmol). The reaction mixture was stirred for two days, then concentrated in vacuo and purified via silica gel chromatography to give tert-butyl ((1R,2R,3S,4R,Z)-7-(cyclopropylmethylene)-3-(neopentylcarbamoyl)bicyclo[2.2.1]heptan-2-yl)carbamate **Intermediate 453-2** (20 mg, 0.053 mmol, 70 % yield). LC-MS RT: 1.19 min; MS (ESI) *m*/*z* 377 (M+H)⁺; Method A.

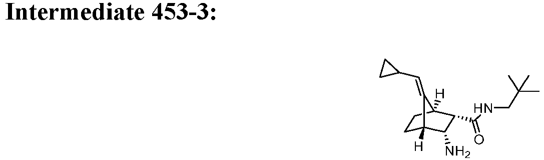

tert-butyl ((1R,2R,3S,4R,Z)-7-(cyclopropylmethylene)-3-(neopentylcarbamoyl)bicyclo[2.2.1]heptan-2-yl)carbamate (20 mg, 0.053 mmol) **453-2** was dissolved in THF (0.4 mL). HCl (4M in dioxane) (0.13 mL, 0.53 mmol) was added. After 1 hour, an additional 0.3 mL 4M HCl in dioxane was added. After 2 hours, the reaction mixture was concentrated in vacuo, then azeotroped with DCM/hexanes to give (1R,2S,3R,4R,Z)-3-amino-7-(cyclopropylmethylene)-N-neopentylbicyclo[2.2.1]heptane-2-carboxamide, HCl **Intermediate 453-3** (20 mg, 0.064 mmol, 120 % yield). LC-MS RT: 0.80 min; MS (ESI) *m*/*z* 277 (M+H)⁺; Method A.

**Example 453:** To a solution of **429-8** (7.87 mg, 0.0270 mmol) and **Intermediate 453-3** (8.45 mg, 0.0270 mmol) in DMF (0.4 mL) was added BOP (13 mg, 0.030 mmol) and Hunig's base (0.024 mL, 0.14 mmol). After 4 hours, the reaction mixture was diluted with MeOH, filtered through a syringe filter, and purified via preparative reverse phase HPLC to give **Example 453** (7.4, 49%). ¹H NMR (500 MHz, DMSO-d₆) δ 9.96 (d, *J*=6.8 Hz, 1H), 8.16 (d, *J*=2.3 Hz, 1H), 7.96 (br t, *J*=6.2 Hz, 1H), 7.77 (dd, *J*=8.7, 2.3 Hz, 1H), 7.22 (d, *J*=8.7 Hz, 1H), 5.11 (dd, *J*=8.7, 5.2 Hz, 1H), 4.62 (d, *J*=9.5 Hz, 1H), 4.53 - 4.48 (m, 1H), 4.34 - 4.25 (m, 1H), 4.19 (br t, *J*=8.6 Hz, 1H), 3.99 (s, 3H), 3.06 - 2.97 (m, 3H), 2.79 (dd, *J*=13.0, 5.7 Hz, 1H), 2.02 - 1.40 (m, 10H), 1.39 - 1.25 (m, 2H), 0.81 (s, 9H), 0.74 - 0.64 (m, 2H), 0.36 - 0.25 (m, 2H). LC-MS RT: 2.23 min; MS (ESI) *m*/*z* 550.1 (M+H)⁺; Method B.

### Example 475

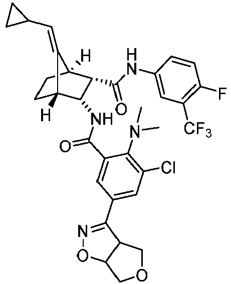

### Intermediate 475-1: Preparation of methyl 2-(dimethylamino)-5-formylbenzoate:

To a solution of methyl 2-fluoro-5-methylbenzoate (3.00 g, 17.8 mmol) in CCl₄ (100 mL) was added *N*-bromosuccinimide (6.99 g, 39.2 mmol) and benzoyl peroxide (0.475 g, 1.96 mmol). The reaction mixture was heated at reflux for 14h. The reaction mixture was allowed to cool to room temperature and the succinimide was removed by filtration. The filtrate was concentrated under reduced pressure to give an yellow liquid that was added to dimethylamine (40% aqeous) (90 mL, 711 mmol) and the contents were slowly heated to 56 °C for 15 mins, and the heat was then removed. The dark orange solution was poured in to DCM (2 x 75 mL) and the layers were separated. The organic layer was concentrated and purified using silica gel chromatography to yield intermediate **465-1** (2.2 g, 56% yield). MS (ESI) *m*/*z*: 208.2 (M+H).

### Intermediate 475-2: Preparation of methyl (E)-3-chloro-2-(dimethylamino)-5-(hydroxyimino)methyl) benzoate:

To a solution of intermediate **475-1** (2200 mg, 10.62 mmol) in DCM (25 mL) was added TEA (1.48 mL, 10.6 mmol). To this solution was then added hydroxylamine hydrochloride (885 mg, 12.7 mmol) and the reaction mixture was stirred at rtovernight. The reaction mixture was then concentrated to a solid and dissolved in EtOAc and washed with water. The organic layer was then dried over MgSO₄ and concentrated under vacuum to yield intermediate **475-2** (2.1 g, 73% yield). MS (ESI) *m*/*z*: 257.1 (M+H).

**Intermediate 475-3:** Preparation of methyl (Z)-3-chloro-5-(chloro(hydroxyimino)methyl)-2-(dimethylamino)benzoate: To intermediate **475-2** (770 mg, 3.00 mmol) in DMF (15 mL) was added *N*-chlorosuccinimide (441 mg, 3.30 mmol). After quenching the reaction mixture with water an off-white solid was collected by filtration which was dried in vacuo to afford intermediate **475-3** (480 mg, 52% yield). MS (ESI) *m*/*z*: 291.0 (M+H).

**475-4:** Preparation of methyl 3-chloro-2-(dimethylamino)-5-(3a,4,6,6a-tetrahydrofuro[3,4-d]isoxazol-3-yl)benzoate: To intermediate **475-3** (1.13 g, 3.88 mmol) and 2,5-dihydrofuran (2.72 g, 38.8 mmol) in DCM (12 mL) was added TEA (1.62 mL, 11.6 mmol). The reaction mixture was stirred at rt for 14h, concentrated under vacuum and the residue purified using silica gel chromatography to yield intermediate **475-4** (440 mg, 35% yield). MS (ESI) *m*/*z*: 325.2 (M+H).

### 475-5: Preparation of 3-chloro-2-(dimethylamino)-5-(3a,4,6,6a-tetrahydrofuro[3,4-d]isoxazol-3-yl)benzoic acid:

To a solution of intermediate **475-4** (100 mg, 0.308 mmol) in THF (3 mL) and water (1.000 mL) was added LiOH (0.462 mL, 0.924 mmol) and the reaction mixture stirred at rt for 2 h. The reaction mixture was concentrated under vacuum and used without further manipulation in the next step. MS (ESI) *m*/*z*: 311.1 (M+H).

**Example 475:** (1R,2S,3R,4R,Z)-3-(3-chloro-2-(dimethylamino)-5-(3a,4,6,6a-tetrahydrofuro[3,4-d]isoxazol-3-yl)benzamido)-N-(4-fluoro-3 -(trifluoromethyl)phenyl)-7-(2,2,2-trifluoroethylidene)bicyclo [2.2.1]heptane-2-carboxamide **475** was prepared using the general procedures described for **378** by using cyclopropyl norbornyl intermediate **166-2** (75 mg, 0.15 mmol) and intermediate **475-5** to yield **Exmple 475** (first eluting stereoisomer via reverse phase HPLC, 55 mg, 17% yield). ¹H NMR (500 MHz, DMSO-d₆) δ 10.45 (br d, *J*=5.2 Hz, 1H), 9.88 (br d, *J*=7.3 Hz, 1H), 8.12 (br d, *J*=4.6 Hz, 1H), 7.87 (s, 1H), 7.85 - 7.80 (m, 1H), 7.77 (br d, *J*=4.3 Hz, 2H), 7.45 (br t, *J*=9.8 Hz, 1H), 5.42 - 5.29 (m, 1H), 4.67 (br d, *J*=9.5 Hz, 1H), 4.55 - 4.37 (m, 2H), 4.09 (br dd, *J*=10.8, 5.0 Hz, 1H), 3.91 (br d, *J*=9.8 Hz, 1H), 3.70 - 3.57 (m, 2H), 3.15 (br d, *J*=11.0 Hz, 1H), 3.07 (br s, 1H), 2.82 (s, 6H), 2.72 (br s, 1H), 1.92 (br d, *J*=7.3 Hz, 2H), 1.60 - 1.47 (m, 1H), 1.42 (br d, *J*=18.3 Hz, 2H), 0.82 - 0.65 (m, 2H), 0.34 (br s, 2H). MS (ESI) m/z = 661.0 (M+H). HPLC Purity: 99 %; Retention Time: 2.96 min; Method B.

### Example 489 & 530

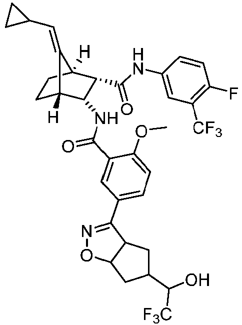

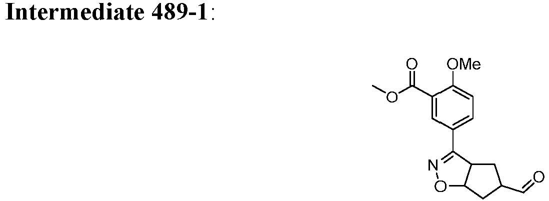

Dess-Martin periodinane (417 mg, 0.983 mmol) was added to a solution of methyl 5-(5-(hydroxymethyl)-3a,5,6,6a-tetrahydro-4H-cyclopenta[d]isoxazol-3-yl)-2-methoxybenzoate **429-8** (2.75 grams, 9.01mmol) in DCM (90 mL). After 3h, the reaction solution was transferred to a separatory funnel and washed successively with NH₄Cl solution and brine, dried over sodium sulfate, filtered, and concentrated under reduced pressure. The residue was purified by silica gel chromatography to give methyl 5-(5-formyl-3a,5,6,6a-tetrahydro-4H-cyclopenta[d]isoxazol-3-yl)-2-methoxybenzoate **489-1** (1.46 grams, 53%) as a white solid. LC-MS RT = 0.97 min; (M+H) = 304.1; Method A.

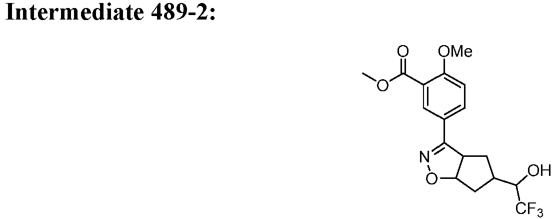

To a solution of **489-1** (100 mg, 0.330 mmol) in THF (3.3 mL) was added trimethyl(trifluoromethyl)silane (188 mg, 1.32 mmol) under a nitrogen atmosphere. The solution was cooled to 0 °C and TBAF (0.40 mL, 0.40 mmol) was added. After 10 min, the reaction mixture was allowed to come to rt and stirred for 14h. The reaction was quenched with MeOH, concentrated under reduced pressure onto Celite^{®}, and purified by silica gel chromatography to give methyl 2-methoxy-5-(5-(2,2,2-trifluoro-1-hydroxyethyl)-3a,5,6,6a-tetrahydro-4H-cyclopenta[d]isoxazol-3-yl)benzoate **489-2** (82.2 mg, 66.8%). ¹H NMR (500 MHz, CDCl₃) δ 8.05 - 8.01 (m, 1H), 7.84 (ddd, J=8.8, 4.7, 2.3 Hz, 1H), 7.04 - 6.98 (m, 1H), 5.24 - 5.17 (m, 1H), 4.08 (t, J=8.8 Hz, 1H), 3.94 (d, J=2.4 Hz, 3H), 3.89 (d, J=1.8 Hz, 3H), 2.38 - 2.23 (m, 2H), 2.10 - 1.96 (m, 2H), 1.89 - 1.81 (m, 1H). LC-MS RT = 0.834 min; (M+H) = 374; Method C.

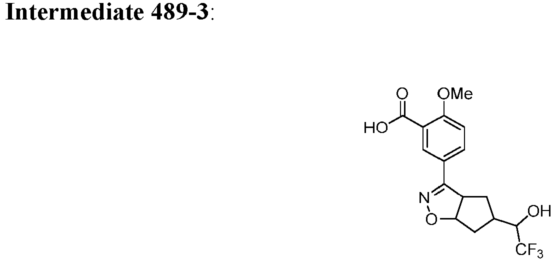

A solution of LiOH monohydrate (27.0 mg, 0.643 mmol) in H₂O (0.5 mL) was added to **489-2** (80 mg, 0.214 mmol) in THF (2.1 mL)/MeOH (2.1 mL). After 3h, the reaction mixture was concentrated under reduced pressure. The residue was suspended in water, acidified with 1.0M HCl solution, extracted with EtOAc, the extract washed with brine, dried over sodium sulfate, filtered, and concentrated under reduced pressure. Intermediate **489-3** (71.7 mg, 93%) was carried forward to the next reaction without further manipulation. LC-MS RT = 0.759 min; (M+H) = 360.0; Method C.

**Example 489** was prepared by the coupling intermediate **489-3** (3.95 mg, 0.014 mmol) with intermediate **166-2** (5 mg, 0.014 mmol) dissolved in anhydrous DMF (2 mL) with DIEA (0.012 mL, 0.068 mmol) and BOP (6.60 mg, 0.015 mmol). After 3 h, the reaction mixture was filtered and purified by reverse phase preparative HPLC to give (1R,2S,3R,4R,Z)-7-(cyclopropylmethylene)-N-(4-fluoro-3-(trifluoromethyl)phenyl)-3-(2-methoxy-5-(5-(2,2,2-trifluoro-1-hydroxyethyl)-3a,5,6,6a-tetrahydro-4H-cyclopenta[d]isoxazol-3-yl)benzamido)bicyclo[2.2.1]heptane-2-carboxamide
**Example 489** (52 mg, 35%) as the first eluting peak (RT = 10.87 min). ¹H NMR: (500 MHz, DMSO-d₆) δ 10.53 - 10.49 (m, 1H), 9.93 - 9.89 (m, 1H), 8.25 - 8.20 (m, 2H), 7.83 - 7.76 (m, 2H), 7.48 (t, *J*=9.8 Hz, 1H), 7.27 (d, *J*=8.9 Hz, 1H), 5.16 - 5.09 (m, 1H), 4.69 (d, *J*=9.5 Hz, 1H), 4.47 - 4.40 (m, 1H), 4.28 - 4.21 (m, 1H), 4.04 (s, 3H), 3.99 - 3.92 (m, 1H), 3.15 (dd, *J*=10.7, 4.1 Hz, 1H), 3.10 (br s, 1H), 2.74 - 2.68 (m, 1H), 2.06 - 1.96 (m, 2H), 1.94 - 1.87 (m, 1H), 1.86 - 1.72 (m, 4H), 1.54 - 1.35 (m, 3H), 0.78 - 0.69 (m, 2H), 0.38 - 0.31 (m, 2H). LC-MS RT = 2.379 min; (M+H) = 710.4; Method C.

**Example 530** (25 mg, 17%) was isolated as the second eluting peak (11.52 min) from reverse phase preparative HPLC of **Example 489.** ¹H NMR: (500 MHz, DMSO-d6) δ 10.53 (s, 1H), 9.92 (d, J=7.0 Hz, 1H), 8.27 - 8.21 (m, 2H), 7.84 - 7.77 (m, 2H), 7.49 (t, J=9.8 Hz, 1H), 7.28 (d, J=8.9 Hz, 1H), 5.15 (dd, J=8.9, 5.0 Hz, 1H), 4.70 (d, J=9.5 Hz, 1H), 4.45 (ddd, J=10.3, 6.4, 4.2 Hz, 1H), 4.25 (t, J=8.9 Hz, 1H), 4.05 (s, 3H), 3.99 - 3.92 (m, 1H), 3.17 (dd, J=10.8, 4.2 Hz, 1H), 3.11 (t, J=3.5 Hz, 1H), 2.73 (t, J=4.1 Hz, 1H), 2.04 - 1.89 (m, 3H), 1.86 - 1.76 (m, 4H), 1.54 - 1.47 (m, 1H), 1.45 - 1.38 (m, 2H), 0.79 - 0.70 (m, 2H), 0.39 - 0.33 (m, 2H). LC-MS RT = 2.382 min; (M+H) = 710.4; Method C.

### Example 511

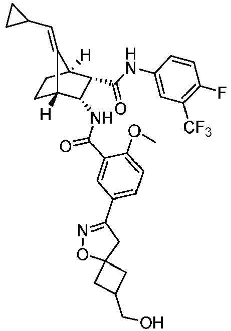

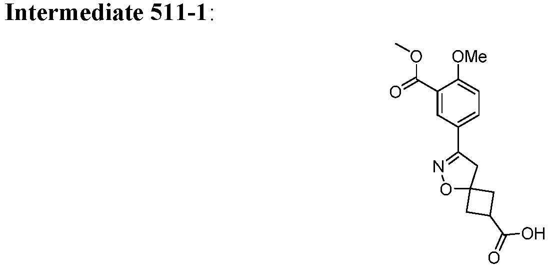

7-(4-methoxy-3-(methoxycarbonyl)phenyl)-5-oxa-6-azaspiro[3.4]oct-6-ene-2-carboxylic acid, **511-1** was prepared as described for intermediate **378-2** substituting 3-methylenecyclobutane-1-carboxylic acid for allyl alcohol to afford (1 g, 3 mmol, 200 % yield) as a tan solid which was carried onto the next step without purification.

LCMS(ESI) *m*/*z*: 320 (M+H).⁺

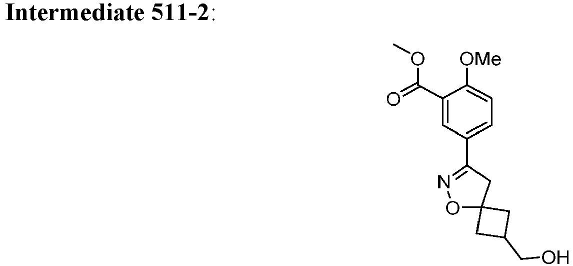

To intermediate **511-1** (0.6 g, 2 mmol) in THF (15 mL) was added BH₃.Me₂S (1.4 mL, 2.9 mmol). After 24h, an additional equivalent of BH₃.Me₂S was added and after a further 6h, the reaction mixture was quenched with ice and 1N HCl (10 mL) and the aqueous solution extracted with ethyl acetate (3 x 30 mL). The combined organic layers were washed with brine (15 mL) and dried (MgSO₄), filtered and concentrated under reduced pressure. The residue was purified by normal phase silica gel chromatography elueting with hexanes/EtOAc to afford intermediate **511-2** (170 mg, 0.56 mmol, 27 % yield) as a white solid.¹H NMR (500 MHz, CDCl₃) δ 8.05 - 7.93 (m, 1H), 7.90 - 7.82 (m, 1H), 7.06 - 6.97 (m, 1H), 3.97 - 3.93 (m, 3H), 3.90 (s, 3H), 3.70 (d, *J*=6.4 Hz, 2H), 2.73 - 2.65 (m, 2H), 2.62 - 2.54 (m, 1H), 2.52 - 2.44 (m, 1H), 2.40 - 2.31 (m, 1H), 2.22 - 2.14 (m, 2H), 1.87 (t, *J*=7.2 Hz, 1H). LCMS(ESI) *m*/*z*: 306.1 (M+H).⁺

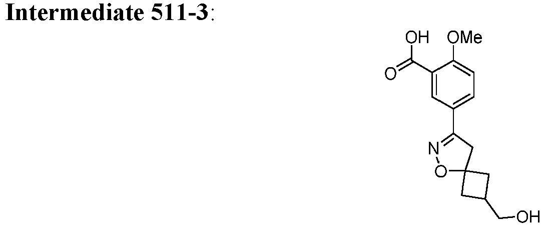

Preparation of 5-(2-(hydroxymethyl)-5-oxa-6-azaspiro[3.4]oct-6-en-7-yl)-2-methoxybenzoic acid. Intermediate **511-3** (135 mg, 0.460 mmol, 83.0 % yield) was prepared by hydrolysis of **511-2** as described in **378-3.** ¹H NMR (500 MHz, CDCl₃) δ 8.22 (dd, *J*=5.2, 2.3 Hz, 1H), 8.18 - 8.12 (m, 1H), 7.14 (dd, *J*=8.8, 3.6 Hz, 1H), 4.15 (d, *J*=1.8 Hz, 3H), 3.73 (d, *J*=6.3 Hz, 2H), 3.48 (s, 1H), 3.42 (s, 1H), 2.55 - 2.42 (m, 1H), 2.43 - 2.33 (m, 3H), 2.27 - 2.15 (m, 3H). LCMS(ESI) *m*/*z*: 291.2 (M+H).⁺

Example **511** (7.0 mg, 11 µmol, 40 % yield) was prepared by the procedure described for example **452,** substituting intermediate **511-3** for intermediate **452-3**.¹H NMR (500 MHz, DMSO-d6) δ 10.54 (s, 1H), 9.91 (br d, J=7.0 Hz, 1H), 8.36 - 8.15 (m, 2H), 7.86 - 7.73 (m, 2H), 7.50 (t, J=9.8 Hz, 1H), 7.35 - 7.23 (m, 1H), 4.71 (d, J=9.5 Hz, 1H), 4.53 - 4.36 (m, 1H), 4.05 (s, 3H), 3.52 (s, 1H), 3.49 - 3.42 (m, 1H), 3.18 (br dd, J=10.7, 3.4 Hz, 1H), 3.12 (br s, 1H), 2.74 (br s, 1H), 2.49 - 2.40 (m, 1H), 2.38 - 2.31 (m, 1H), 2.30 - 2.21 (m, 1H), 2.15 - 2.09 (m, 2H), 2.08 (br s, 1H), 1.94 - 1.83 (m, 1H), 1.83 - 1.73 (m, 1H), 1.62 - 1.48 (m, 1H), 1.47 - 1.33 (m, 2H), 0.85 - 0.65 (m, 2H), 0.37 (br s, 2H). LCMS(ESI) *m*/*z*: 642.91 (M+H).⁺ HPLC purity 100 % with retention time 2.42 min. [method B]

### Example 536

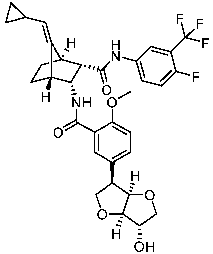

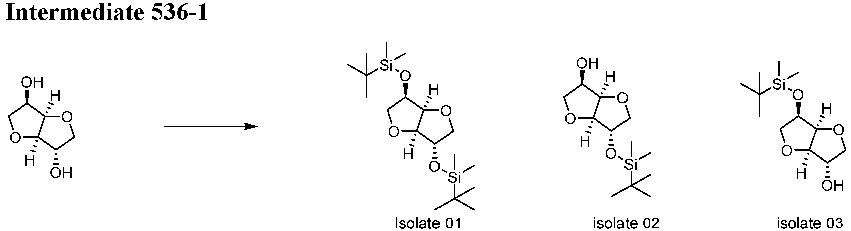

To a solution of dianhydro-D-glucitol (3.0 g, 21 mmol) dissolved in DCM (80 mL) was added imidazole (2.8 g, 41 mmol) and cooled to 0 °C. To this mixture was added TBSCl (3.9 g, 26 mmol) and the reaction mixture was allowed to warm to rt for 14h. The reaction mixture was washed with water and the organic portion was concentrated under reduced pressure, then purified by silica gel chromatography utilizing in-line light scattering detection to afford **Intermediate 536-1,** Isolate 02, (3R,3aR,6S,6aS)-6-((tertbutyldimethylsilyl)oxy)hexahydrofuro[3,2-b]furan-3-ol (1.8 g, 7.0 mmol, 34 % yield).¹H NMR (400 MHz, CDCl₃) δ 4.70 - 4.58 (m, 1H), 4.39 - 4.23 (m, 3H), 3.97 - 3.82 (m, 3H), 3.55 (dd, *J*=9.4, 6.1 Hz, 1H), 2.69 (d, *J*=7.7 Hz, 1H), 0.97 - 0.84 (m, 9H), 0.13 (d, *J*=2.0 Hz, 6H)

Peak 3, (3S,3aR,6R,6aS)-6-((tert-butyldimethylsilyl)oxy)hexahydrofuro[3,2-b]furan-3-ol (0.92 g, 3.5 mmol, 17 % yield). ¹H NMR (400 MHz, CDCl₃) δ 4.56 (t, *J*=4.7 Hz, 1H), 4.41 (d, *J*=4.4 Hz, 1H), 4.37 - 4.29 (m, 2H), 4.04 - 3.96 (m, 1H), 3.95 - 3.88 (m, 1H), 3.80 (dd, *J*=8.6, 5.9 Hz, 1H), 3.57 (dd, *J*=8.8, 6.8 Hz, 1H), 1.83 (d, *J*=5.3 Hz, 1H), 0.99 - 0.87 (m, 9H), 0.15 (d, *J*=5.7 Hz, 6H).

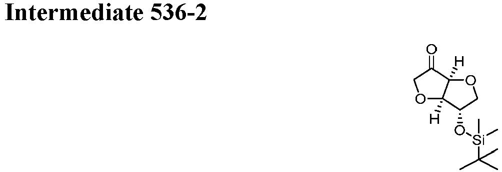

To a solution of **536-1** (1.9 g, 7.1 mmol) dissolved in DCM (24 mL) was added Dess-Martin periodinane (6.1 g, 14 mmol) and the reaction mixture was stirred for 14h. The reaction mixture was partitioned between DCM and pH 7.4 aqueous buffer, and extracted with DCM. The combined organic portions were concentrated under reduced pressure, then purified by silica gel chromatography utilizing in-line light scattering detection detection to afford (3aS,6S,6aS)-6-((tert-butyldimethylsilyl)oxy)tetrahydrofuro[3,2-b]furan-3(2H)-one (1.4 g, 5.4 mmol, 75 % yield). ¹H NMR (500 MHz, CDCL₃) δ 4.63 (d, *J*=4.0 Hz, 1H), 4.46 (d, *J*=3.2 Hz, 1H), 4.31 (d, *J*=4.0 Hz, 1H), 4.12 (d, *J*=17.4 Hz, 1H), 4.02 (dd, *J*=9.5, 3.4 Hz, 1H), 3.95 - 3.89 (m, 2H), 0.94 - 0.88 (m, 9H), 0.12 (d, *J*=4.4 Hz, 6H).

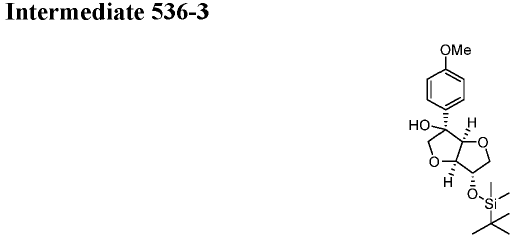

A solution of **536-2** (1.4 g, 5.4 mmol) dissolved in THF (5.4 mL) was cooled to 0 °C, (4-methoxyphenyl)magnesium bromide (11 mL, 5.4 mmol) was added and the reaction mixture was stirred for 48h. The reaction mixture was partitioned with sat NH₄Cl and extracted with EtOAc. The combined organic layers were concentrated under reduced pressure, then purified via silica gel chromatography to afford (3R,3aS,6S,6aS)-6-((tertbutyldimethylsilyl)oxy)-3-(4-methoxyphenyl)hexahydrofuro[3,2-b]furan-3-ol (1.5 g, 4.1 mmol, 76 % yield): ¹H NMR (500 MHz, CDCl₃) δ 7.49 (d, *J*=8.9 Hz, 2H), 6.93 (d, *J*=8.9 Hz, 2H), 4.45 - 4.38 (m, 3H), 4.12 (d, J=9.3 Hz, 1H), 4.05 - 4.00 (m, 1H), 3.98 - 3.93 (m, 1H), 3.83 (s, 3H), 3.79 (d, *J*=9.3 Hz, 1H), 3.48 (s, 1H), 0.91 - 0.85 (m, 9H), 0.11 (d, *J*=3.1 Hz, 6H).

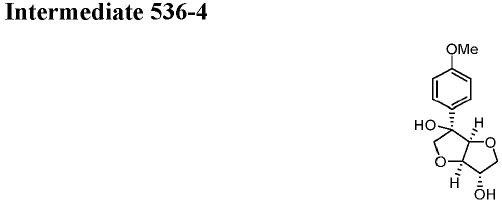

To **536-3** (0.50 g, 1.4 mmol) was added TBAF 1M in THF (1.4 mL, 1.4 mmol) and the reaction mixture was stirred for 14h. The reaction mixture was diluted with EtOAc, and washed successively with water, and brine. The organic portion was dried over Na₂SO₄ filtered and concentrated under reduced pressure, then purified by silica gel chromatography to afford (3R,3aS,6S,6aR)-3-(4-methoxyphenyl)hexahydrofuro[3,2-b]furan-3,6-diol (0.23 g, 0.92 mmol, 67 % yield). ¹H NMR (500 MHz, CDCl₃) δ 7.53 - 7.43 (m, 2H), 6.99 - 6.89 (m, 2H), 4.55 (d, *J*=4.1 Hz, 1H), 4.52 - 4.45 (m, 2H), 4.14 - 4.01 (m, 3H), 3.88 - 3.78 (m, 4H), 3.40 (s, 1H).

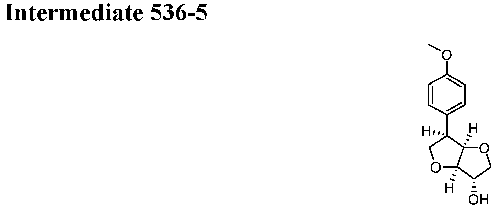

To a solution of **536-4** (0.080 g, 0.32 mmol) dissolved in DCM (1 mL) was added triethylsilane (0.15 mL, 0.95 mmol) and TFA (1 mL), and the reaction mixture was stirred for 14h. The reaction mixture was concentrated under reduced pressure, then purified by silica gel chromatography to afford (3S,3aR,6R,6aR)-6-(4-methoxyphenyl)hexahydrofuro[3,2-b]furan-3-ol (0.050 g, 0.21 mmol, 67 % yield). ¹H NMR (500 MHz, CDCl₃) δ 7.25 (d, *J*=8.4 Hz, 2H), 6.90 (d, *J*=8.7 Hz, 2H), 4.84 (t, *J*=3.8 Hz, 1H), 4.60 (d, *J*=3.7 Hz, 1H), 4.43 - 4.38 (m, 1H), 4.23 (t, *J*=8.1 Hz, 1H), 4.02 (dd, *J*=10.1, 3.8 Hz, 1H), 3.91 - 3.83 (m, 2H), 3.82 (s, 3H), 3.41 (ddd, *J*=11.6, 7.8, 4.0 Hz, 1H)

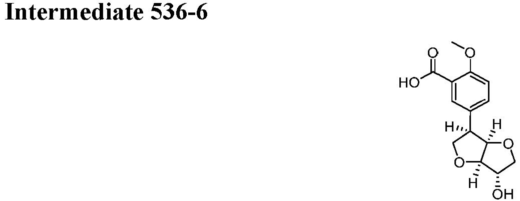

To a solution of **536-5** (0.050 g, 0.21 mmol) dissolved in acetone (2.1 mL) was added NBS (0.040 g, 0.22 mmol) followed by 1 drop of 1N HCl and stirred for 14h. The reaction mixture was diluted with EtOAc and washed with 1M pH 7.4 phosphate buffer. The organic portion was concentrated under reduced pressure, then purified by silica gel chromatography to afford (3S,3aR,6R,6aR)-6-(3-bromo-4-methoxyphenyl)hexahydrofuro[3,2-b]furan-3-ol (quantitative) which was used in the next step without futher manipulation.

To a slurry of (3S,3aR,6R,6aR)-6-(3-bromo-4-methoxyphenyl)hexahydrofuro[3,2-b]furan-3-ol (0.090 g, 0.21 mmol) dissolved in DMF (2.6 mL) was added Pd(OAc)₂ (0.019 g, 0.085 mmol), 1,3-bis(diphenylphosphino)propane (0.035 g, 0.085 mmol), TEA (0.12 mL, 0.85 mmol), and water (0.29 mL). The reaction mixture was blanketed under CO (100 psi) and heated to 100 °C for 14h. The reaction mixture was diluted with EtOAc, partitioned with 1 N HCl, and extracted with EtOAc. The combined organic portions were concentrated under reduced pressure, then used without further mainpulation in the next step as 5-((3R,3aR,6S,6aR)-6-hydroxyhexahydrofuro[3,2-b]furan-3-yl)-2-methoxybenzoic acid (0.046 g, 0.16 mmol, 785 yield, two steps).

### Example 536

To a solution of **166-2** (0.060 g, 0.16 mmol) dissolved in ACN (3.3 mL) was added **536-6** (0.046 g, 0.16 mmol), DIEA (0.085 mL, 0.49 mmol) and HATU (0.062 g, 0.16 mmol) and the reaction mixture stirred for 30 min. The reaction mixture was diluted with methanol and purified by preparative reverse phase HPLC to afford (1R,2S,3R,4R,Z)-7-(cyclopropylmethylene)-N-(4-fluoro-3-(trifluoromethyl)phenyl)-3-(5-((3R,3aR,6S,6aR)-6-hydroxyhexahydrofuro[3,2-b]furan-3-yl)-2-methoxybenzamido)bicyclo[2.2.1]heptane-2-carboxamide (13 mg, 0.021 mmol, 13 % yield). ¹H NMR (500 MHz, DMSO-d6) δ 10.53 (s, 1H), 9.84 (br d, J=7.0 Hz, 1H), 8.24 (br d, J=4.9 Hz, 1H), 7.88 (s, 1H), 7.83 - 7.71 (m, 1H), 7.50 (br t, J=9.5 Hz, 1H), 7.43 (br d, J=8.2 Hz, 1H), 7.12 (d, J=8.5 Hz, 1H), 5.26 (d, J=3.4 Hz, 1H), 4.76 - 4.61 (m, 2H), 4.52 - 4.39 (m, 2H), 4.17 - 4.06 (m, 2H), 3.99 (s, 3H), 3.81 (br dd, J=9.2, 3.1 Hz, 1H), 3.73 - 3.60 (m, 2H), 3.17 (br dd, J=10.8, 4.1 Hz, 1H), 3.10 (br s, 1H), 2.73 (br s, 1H), 1.91 - 1.84 (m, 1H), 1.83 - 1.71 (m, 1H), 1.51 (br dd, J=8.2, 4.6 Hz, 1H), 1.47 - 1.31 (m, 2H), 0.82 - 0.67 (m, 2H), 0.37 (br s, 2H). LC-MS RT: 2.34 min; MS (ESI) *m*/*z* 631.2 (M+H)⁺; Method A.

### Example 565

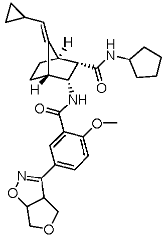

To a solution of intermediate **447-1** (20 mg, 0.028 mmol) in DCM (0.5 mL) was added cyclopentanamine (24 mg, 0.28 mmol). The reaction mixture was stirred for 14h, then concentrated under reduced pressure, dissolved in MeOH, and filtered through a syringe filter. The residue was purified via preparative reverse phase HPLC to furnish **Example 565** (12.2 mg, 84%). ¹H NMR (400 MHz, DMSO-d₆) δ 10.01 (d, *J*=6.8 Hz, 1H), 8.15 (d, *J*=2.4 Hz, 1H), 8.02 (d, *J*=7.6 Hz, 1H), 7.79 (dd, *J*=8.6, 2.4 Hz, 1H), 7.24 (d, *J*=8.7 Hz, 1H), 5.34 (dd, *J*=9.6, 3.2 Hz, 1H), 4.61 (d, *J*=9.7 Hz, 1H), 4.50 (dd, *J*=8.3, 6.6 Hz, 1H), 4.34 - 4.22 (m, 1H), 4.09 (d, *J*=10.8 Hz, 1H), 4.02 (s, 4H), 3.90 (d, *J*=9.3 Hz, 1H), 3.81 - 3.72 (m, 1H), 3.66 (dd, *J*=10.6, 3.9 Hz, 1H), 3.09 - 3.01 (m, 1H), 2.88 (dd, *J*=11.1, 4.4 Hz, 1H), 1.88 - 1.70 (m, 4H), 1.65 - 1.54 (m, 2H), 1.53 - 1.40 (m, 3H), 1.39 - 1.26 (m, 4H), 0.76 - 0.64 (m, 2H), 0.32 (br d, *J*=3.5 Hz, 2H). One proton is not visible in NMR, likely due to overlap with solvent peak. LC-MS RT: 2.14 min; MS (ESI) *m*/*z* 520.4 (M+H)⁺; Method B.

### Example 578

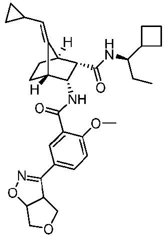

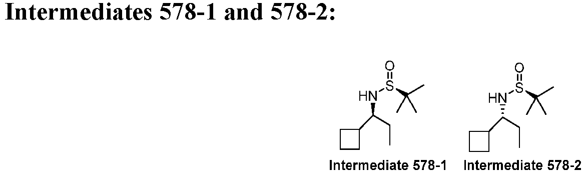

A solution of (S,E)-N-(cyclobutylmethylene)-2-methylpropane-2-sulfinamide (0.500 g, 2.67 mmol) was cooled in a dry ice/acetone bath. Ethylmagnesium bromide (3.6 M in 2Me-THF) (1.48 mL, 5.34 mmol) was added dropwise, and the reaction mixture was allowed to warm to rt for 14h. The reaction mixture was quenched with sat. ammonium chloride solution and extracted twice with DCM. The organic layers were concentrated under reduced pressure. The residue was purified via silica gel chromatography to furnish 533 mg of a mixture of diastereomers. The diastereomers were separated via preparative SFC with the following conditions: Instrument: PIC Solution SFC Prep-200; Column: Chiralpak AD-H, 21 x 250 mm, 5 micron; Mobile Phase 15% isopropanol-acetontrile, 85% CO₂; Flow Rate: 45 mL/min, 150 Bar; Column Temperature: 40 °C.

(S)-N-((S)-1-cyclobutylpropyl)-2-methylpropane-2-sulfinamide **Intermediate 578-1** Peak 1 RT: 6 min (70 mg, 0.32 mmol, 12 % yield). Analytical SFC conditions: Instrument: Shimadzu Nexera SFC; Column: Chiralpak AD-H, 4.6 x 100 mm, 3 micron; Mobile Phase 10% isopropanol-acetontrile, 90% CO₂; Flow Rate: 42 mL/min, 150 Bar; Column Temperature: 40 °C. RT: 2.09 min MS (ESI) *m*/*z* 218(M+H)⁺

(S)-N-((R)-1-cyclobutylpropyl)-2-methylpropane-2-sulfinamide **Intermediate 578-2** Peak 2 RT: 8.7 min. (350 mg, 1.61 mmol, 60.3 % yield). Analytical SFC conditions: Instrument: Shimadzu Nexera SFC; Column: Chiralpak AD-H, 4.6 x 100 mm, 3 micron; Mobile Phase 10% isopropanol-acetontrile, 90% CO₂; Flow Rate: 42 mL/min, 150 Bar; Column Temperature: 40 °C. RT: 2.26 min MS (ESI) *m*/*z* 218(M+H)⁺.

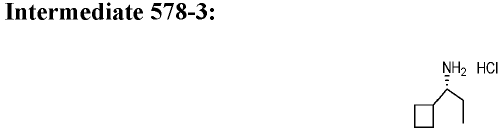

To a solution of **578-2** (350 mg, 1.61 mmol) in MeOH (0.8 mL) was added HCl (4M in dioxane) (0.81 mL, 3.2 mmol). After 45 minutes, the reaction mixture was concentrated in vacuo. Et₂O was added, then the solid was filtered off and washed with Et₂O/hexanes. The solid was collected and dried under vacuum to give (R)-1-cyclobutylpropan-1-amine **578-3** (175 mg, 1.55 mmol, 96 % yield). ¹H NMR (400 MHz, CD₃OD) δ 3.06 - 2.97 (m, 1H), 2.55 - 2.41 (m, 1H), 2.17 - 2.04 (m, 2H), 2.03 - 1.80 (m, 4H), 1.73 - 1.59 (m, 1H), 1.55 - 1.42 (m, 1H), 0.99 (t, J=7.6 Hz, 3H).

### Example 578

**Example 578** (4.8 mg, 49%) was prepared from **578-3** following the procedure given for **Example 378.** ¹H NMR (500 MHz, DMSO-d₆) δ 10.04 (d, *J*=6.8 Hz, 1H), 8.15 (d, *J*=2.4 Hz, 1H), 7.78 (dd, *J*=8.6, 2.4 Hz, 1H), 7.70 (br d, *J*=9.1 Hz, 1H), 7.23 (d, *J*=8.8 Hz, 1H), 5.33 (dd, *J*=9.2, 3.5 Hz, 1H), 4.62 (d, *J*=9.6 Hz, 1H), 4.49 (br t, *J*=7.9 Hz, 1H), 4.33 - 4.24 (m, 1H), 4.09 (d, *J*=10.7 Hz, 1H), 4.00 (s, 3H), 3.92 - 3.87 (m, 1H), 3.77 (dd, *J*=9.2, 6.7 Hz, 1H), 3.71 - 3.59 (m, 2H), 3.09 - 3.01 (m, 1H), 2.98 - 2.88 (m, 1H), 2.33 - 2.19 (m, 1H), 1.95 - 1.60 (m, 8H), 1.51 - 1.41 (m, 1H), 1.40 - 1.25 (m, 3H), 1.19 - 1.03 (m, 1H), 0.82 - 0.62 (m, 5H), 0.31 (dd, *J*=4.5, 2.2 Hz, 2H). One proton is not visible in NMR, likely due to overlap with solvent peak. LC-MS RT: 2.58 min; MS (ESI) *m*/*z* 548.2 (M+H)⁺; Method B.

### Example 589 and 629:

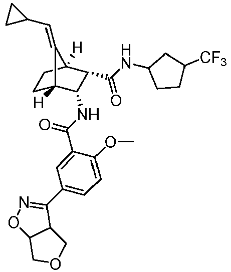

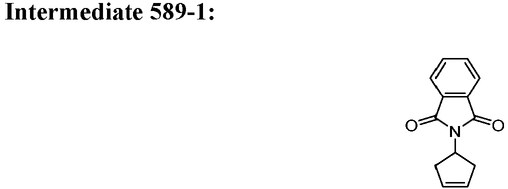

To a suspension of isobenzofuran-1,3-dione (372 mg, 2.51 mmol) and cyclopent-3-en-1-amine, HCl (300 mg, 2.51 mmol) in toluene (25 mL) was added Hunig's base (0.44 mL, 2.5 mmol). The reaction mixture was heated to 120 °C. After ca. 5.5 hours, the reaction mixture was concentrated under reduced pressure. The residue was purified via silica gel chromatography to furnish **Intermediate 589-1** (369 mg, 69%). ¹H NMR (400 MHz, CDCl₃) δ 7.88 - 7.80 (m, 2H), 7.76 - 7.67 (m, 2H), 5.81 (s, 2H), 5.02 (tt, J=9.6, 7.4 Hz, 1H), 2.93 - 2.82 (m, 2H), 2.74 - 2.63 (m, 2H).

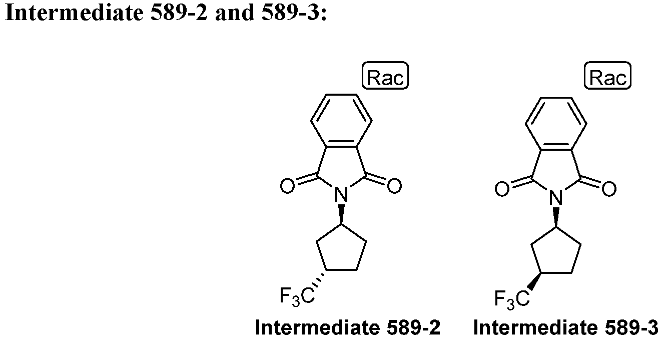

In each of four pressure vials, sodium trifluoromethanesulfinate (234 mg, 1.50 mmol), 9-mesityl-10-methylacridin-10-ium, tetrafluoroborate salt (15 mg, 0.038 mmol), and rac-2-((1R,3R)-3-(trifluoromethyl)cyclopentyl)isoindoline-1,3-dione (273 mg, 0.964 mmol) were suspended in CHCl₃ (3.4 mL) and trifluoroethanol (0.38 mL). Nitrogen was bubbled through the solution, then methyl 2-mercaptobenzoate (25 mg, 0.15 mmol) was added, and nitrogen was bubbled through the solution briefly. The vial was sealed and irradiated with a KSH 150B blue Kessil grow lamp, 34W, 461 nm lambda max for 48h. The reaction mixture was removed from the photoreactor and poured into saturated aq. NaHCO₃. The reaction mixture was extracted three times with DCM. The organic layers were dried with sodium sulfate and concentrated in vacuo. The residue was purified via silica gel chromatography.

The first eluting peak was trans product rac-2-((1R,3R)-3-(trifluoromethyl)cyclopentyl)isoindoline-1,3-dione **Intermediate 589-2** (273 mg, 0.964 mmol, 32.1 % yield). ¹H NMR (400 MHz, CDCl₃) δ 7.84 (dd, *J*=5.4, 3.1 Hz, 2H), 7.77 - 7.68 (m, 2H), 4.88 - 4.72 (m, 1H), 3.22 - 3.06 (m, 1H), 2.37 (ddd, *J*=14.0, 9.6, 6.9 Hz, 1H), 2.29 - 2.05 (m, 4H), 1.85 - 1.70 (m, 1H)

The second eluting peak was cis product **Intermediate 589-3** rac-2-((1R,3S)-3-(trifluoromethyl)cyclopentyl)isoindoline-1,3-dione (38 mg, 0.134 mmol, 4.47 % yield). ¹H NMR (400 MHz, CDCl₃) δ 7.90 - 7.79 (m, 2H), 7.77 - 7.65 (m, 2H), 4.73 - 4.58 (m, 1H), 2.83 - 2.59 (m, 1H), 2.55 - 2.30 (m, 2H), 2.22 - 2.08 (m, 2H), 2.06 - 1.84 (m, 2H).

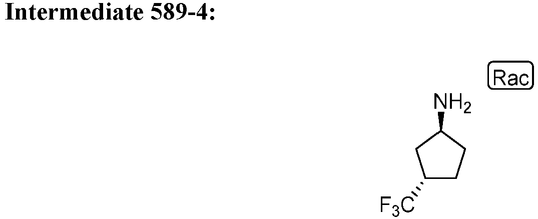

To a suspension of **Intermediate 589-2** in EtOH (1.8 mL) was added hydrazine hydrate (29 µl, 0.39 mmol). The reaction mixture was heated to 75 °C. After ca. 30 minutes, 1 mL EtOH was added. After 2.5 hours, the reaction mixture was allowed to cool, diluted with EtOH and filtered. 90 µL 4M HCl in dioxane was added to the filtrate and the solutionconcentrated in vacuo. The residue obtained contained ca. 0.25 equiv of phthalazidinone. The residue was suspended in EtOH and filtered. The filtrate was concentrated under reduced pressure to give **Intermediate 589-4** rac-(1R,3R)-3-(trifluoromethyl)cyclopentan-1-amine, HCl (83 mg, 0.44 mmol, 120 % yield) which contained ca. 0.1 equivalent of phthalazadinone. ¹H NMR (400 MHz, CD₃OD) δ 3.74 - 3.65 (m, 1H), 3.06 - 2.91 (m, 1H), 2.29 - 2.09 (m, 3H), 2.00 - 1.87 (m, 1H), 1.85 - 1.64 (m, 2H).

### Example 629 and 589:

The diastereomeric mixture of **Example 629 and 589** was prepared according to the procedure given for **Example 565.** The diastereomers were separate via preparative SFC with the following conditions: Column: Chiral OD, 30 x 250 mm, 5 micron; Mobile Phase 80% CO₂ / 20% IPA w/0.1%DEA; Flow Rate: 100 mL/min, 120 Bar; Column Temperature: 40 °C.

First eluting peak (RT=13.5 min) is **Example 629** (0.8 mg, 3.7%). ¹H NMR (500 MHz, DMSO-d₆) δ 9.95 (br d, *J*=7.0 Hz, 1H), 8.17 (br d, *J*=2.1 Hz, 2H), 7.79 (br d, *J*=8.2 Hz, 1H), 7.29 - 7.20 (m, 1H), 5.34 (br dd, *J*=9.2, 3.1 Hz, 1H), 4.62 (br d, *J*=9.5 Hz, 1H), 4.50 (br t, *J*=7.8 Hz, 1H), 4.35 - 4.25 (m, 1H), 4.18 - 4.07 (m, 2H), 4.02 (s, 3H), 3.94 - 3.86 (m, 1H), 3.82 - 3.72 (m, 1H), 3.69 - 3.61 (m, 1H), 3.05 (br s, 1H), 2.98 - 2.82 (m, 2H), 2.04 - 1.40 (m, 9H), 1.39 - 1.26 (m, 2H), 0.76 - 0.65 (m, 2H), 0.32 (br d, *J*=2.4 Hz, 2H). One proton is not visible in NMR, likely due to overlap with suppressed water peak. LC-MS RT: 2.30 min; MS (ESI) *m*/*z* 588.3 (M+H)⁺; Method B.

Second eluting peak (RT=16.0 min) is **Example 589** (0.7 mg, 3.4%). ¹H NMR (500 MHz, DMSO-d₆) δ 9.95 (br d, *J*=7.3 Hz, 1H), 8.17 (br d, *J*=8.5 Hz, 2H), 7.79 (br d, *J*=8.5 Hz, 1H), 7.29 - 7.18 (m, 1H), 5.40 - 5.27 (m, 1H), 4.62 (br d, *J*=9.5 Hz, 1H), 4.54 - 4.45 (m, 1H), 4.35 - 4.25 (m, 1H), 4.16 - 4.06 (m, 2H), 4.02 (s, 3H), 3.90 (br d, *J*=5.8 Hz, 1H), 3.82 - 3.74 (m, 1H), 3.66 (br dd, *J*=7.0, 3.4 Hz, 1H), 3.08 - 3.02 (m, 1H), 2.98 - 2.83 (m, 2H), 2.02 - 1.41 (m, 9H), 1.40 - 1.26 (m, 2H), 0.76 - 0.66 (m, 2H), 0.32 (br d, *J*=3.7 Hz, 2H). LC-MS RT: 2.23 min; MS (ESI) *m*/*z* 588.5 (M+H)⁺; Method B.

### Example 591

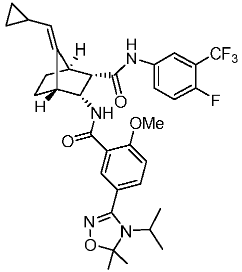

Preparation of intermediate **591-1.** 5-(4-isopropyl-5,5-dimethyl-4,5-dihydro-1,2,4-oxadiazol-3-yl)-2-methoxybenzoic acid. LDA was generated by the addition of nBuLi (2.7M, 2.3 mL, 6.3 mmol) to a THF (15 mL) solution of disopropylamine (0.64 g, 6.33 mmol) cooled to -78 °C. The reaction mixture was stirred cold for 0.5 h and methyl-3-cyclopentene carboxylate (0.4 g, 3.0 mmol) added followed by idodomethane (0.2 mL,3.0 mmol). The reaction mixture was stirred cold for 4 h and gradually allowed to warm up to r.t. for 14h. To the reaction mixture was then added methyl (E)-5-(chloro(hydroxyimino)methyl)-2-methoxybenzoate (1.05 g, 4.32 mmol) followed by the addtion of TEA (1.4 mL, 9.1 mmol) and the resulting solution was stirred at r.t. for 14h, The reaction was quenched by the addition of dil HCl (1N, 10 ml) and extracted with EtOAc (2 x 25 mL). The combined organic portion wasdried (MgSO₄), filtered and concentrated under reduced pressure to an oil. The oil was purified via silica gel chromatography using hexane/EtOAc as eluant to afford methyl 5-(4-isopropyl-5,5-dimethyl-4,5-dihydro-1,2,4-oxadiazol-3-yl)-2-methoxybenzoate intermediate by product (75 mg, 8 % yield) as a solid. The solid was dissolved in MeOH (2 mL) and to the solution was added LiOH (10 mg. 0.24 mmol) and water (2 mL). The reaction mixture was stirred for 14h at r.t. then concentrated under reduced pressure and quenched with dil HCl (1N, 5 mL). The solution was transferred to a separatory funnel and extracted the with EtOAc (2x25 mL), the organic portion dried (MgSO₄), filtered and concentrated under reduced pressure to a solid **591-1** (60 mg, 85 % yield) which was used without further manipulation. ¹H NMR (500 MHz, CDCl₃) δ 8.29 - 8.19 (m, 1H), 7.76 (dd, J=8.5, 2.3 Hz, 1H), 7.14 (d, J=8.3 Hz, 1H), 4.12 (s, 3H), 3.61 (spt, J=7.0 Hz, 1H), 1.63 (s, 6H), 1.84 - 0.82 (d, 6H). LCMS m/z = 293.3 (M+H)⁺.

**Example 591.** Intermediate **591-1** (9 mg, 0.03 mmol) was coupled to **166-2** (11.34 mg, 0.03 mmol) with BOP (13.62 mg, 0.03 mmol) reagent,and Hunig's base (0.1 mL) as described for example **378** to afford example **591** as a solid (8.4 mg, 41% yield) after purification by preparative reverse phase HPLC. HPLC purity: 96.5 %; RT= 2.67 min [Method B]. LCMS m/z = 643.18 (M+H)⁺. ¹H NMR (500 MHz, DMSO-d₆) δ 10.54 (s, 1H), 9.96 (br d, *J*=6.7 Hz, 1H), 8.23 (br d, *J*=4.0 Hz, 1H), 8.03 (d, *J*=2.1 Hz, 1H), 7.79 (br d, *J*=8.9 Hz, 1H), 7.61 (dd, *J*=8.5, 2.1 Hz, 1H), 7.49 (br t, *J*=9.8 Hz, 1H), 7.31 (d, *J*=8.5 Hz, 1H), 4.70 (d, *J*=9.5 Hz, 1H), 4.46 (br s, 1H), 4.07 (s, 3H), 3.68 - 3.48 (m, 1H), 3.41 (br s, 1H), 3.28 - 3.13 (m, 1H), 3.11 (br s, 1H), 2.73 (br s, 1H), 1.92 - 1.75 (m, 2H), 1.51 (s, 6H), 1.43 (br t, *J*=10.7 Hz, 2H), 1.06 (br d, *J*=6.7 Hz, 6H), 0.86 - 0.66 (m, 2H), 0.36 (br s, 2H)

### Example 594

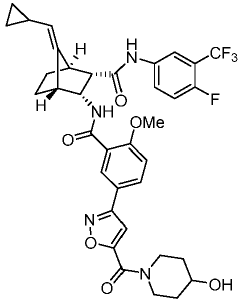

### Preparation of intermediate 594-1.

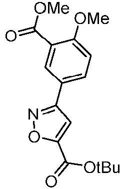

Intermediate **594-1** (434 mg, 60.0% yield) was prepared in a similar manner as described for example **378** by the cycloadditon of methyl (Z)-5-(chloro(hydroxyimino)methyl)-2-methoxybenzoate (530 mg, 2.18 mol) with t-butylpropiolate (274 mg, 2.17 mmol) and TEA (2 mL) in DCM as previously described. LCMS m/z = 334.3 (M+H)⁺.

### Preparation of intermediate 594-2.

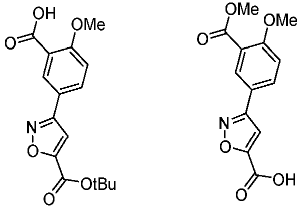

Intermediate **594-1** (100 mg, 0.30 mmol) was hydrolysed with LiOH (12 mg, 0.35 mmol) in methanol/water as previously described in Example 378 to afford **594-2** (75 mg) as a mixture of two products. LCMS m/z = 278.2 (M+H) for the desired product. The other by product was the cleavage of the t-butyl ester to the acid prduct. The crude mixture was carried on to the next step without further manipulation

### Preparation of intermediate 594-3.

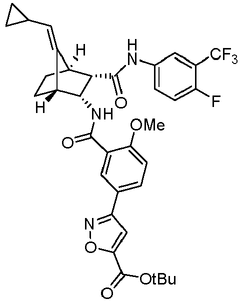

Intermediate **594-2** (60 mg, 0.22 mmol, part as a mixture) was dissolved in DMF (1 mL) and to this was added 4-OH-piperidine (22 mg, 0.22 mmol) followed by BOP (6 mg, 0.22 mmol) reagent and Hunig's base (0.1 mL). The reaction mixture was stirred at rt for 14 h, quenched with water (25 mL) and extractedwith EtOAc (2x25 mL). The combined organic portion was dried and concentrated in vacuo to an oil. LCMS m/z = 361.2 (M+H). The oil obtained was dissolved in methanol (1 mL) and to this was added LiOH (10 mg, 0.2 mmol) followed by water (1 mL) and the reaction mixture stirred for 14 h The reaction mixture was diluted with water (25 mL) and extracted with EtOAc (2x25 mL). The combined organic portion was dried (MgSO₄) and concentrated in vacuo to yield **594-3** as an oil (LCMS m/z = 347.3 (M+H) which was used without further purification in the next step.

**Example 594.** Intermediate **594-3** (8 mg, 0.02 mmol) was coupled to **166-2** (8.5 mg, 0.020 mmol) with BOP (10 mg, 0.02 mmol) reagent,and Hunig's base (0.1 mL) to afford Example **594** as a solid (3.6 mg, 22% yield) after purification by preparative LC/MS with the following conditions: Column: XBridge C18, 200 mm x 19 mm, 5-µm particles; Mobile Phase A: 5:95 acetonitrile: water with 0.05% trifluoroacetic acid; Mobile Phase B: 95:5 acetonitrile: water with 0.05% trifluoroacetic acid. HPLC purity: 98.1 %; RT= 2.30 min [Method B]. LCMS m/z = 697.01 (M+H)⁺. ¹H NMR (500 MHz, DMSO-d₆) δ 10.55 (s, 1H), 9.94 (br d, *J*=6.4 Hz, 1H), 9.26 - 9.19 (m, 1H), 8.27 (d, *J*=2.3 Hz, 1H), 8.22 (br d, *J*=4.6 Hz, 1H), 7.80 (br dd, *J*=8.6, 2.1 Hz, 2H), 7.48 (br t, *J*=9.7 Hz, 1H), 7.35 (d, *J*=8.8 Hz, 1H), 7.27 - 7.21 (bs, 1H), 7.17 - 7.13 (bs, 1H), 7.09 (bs, 1H)4.69 (d, *J*=9.5 Hz, 1H), 4.44 (br s, 1H), 4.06 (s, 3H), 3.67 (br s, 1H), 3.51 (m, 2H), 3.34 - 3.13 (m, 1H), 3.10 (br s, 1H), 2.80 - 2.63 (m, 1H), 1.83 (br d, *J*=9.8 Hz, 1H), 1.77 (br s, 2H), 1.50 (br s, 1H), 1.45 (br s, 1H), 1.41 (br s, 2H), 1.16 (t, *J*=7.3 Hz, 1H), 1.10 (br s, 1H), 0.86 - 0.68 (m, 2H), 0.35 (br s, 2H)

### Example 626

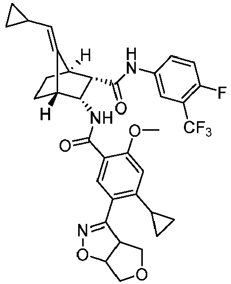

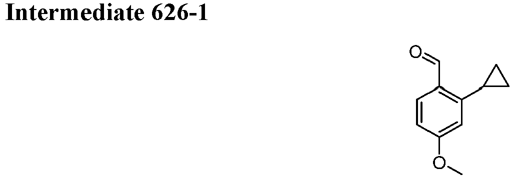

Preparation of 2-cyclopropyl-4-methoxybenzaldehyde. Intermediate **626-1** (0.30 g, 1.9 mmol, 84 % yield) was prepared in the manner described for example **12** substituting 2-bromo-4-methoxybenzaldehyde and cyclopropylboronic acid for example **11** and furan-3-ylboronic acid and dioxane for THF. ¹H NMR (400 MHz, CDCl₃) δ 10.46 (s, 1H), 7.83 (d, *J*=8.6 Hz, 1H), 6.84 (dd, *J*=8.7, 2.5 Hz, 1H), 6.62 (d, *J*=2.4 Hz, 1H), 3.89 (s, 3H), 2.70 (tt, *J*=8.5*,* 5.4 Hz, 1H), 1.15 - 1.07 (m, 2H), 0.86 - 0.76 (m, 2H). LCMS(ESI) *m*/*z*:177.1 (M+H).⁺

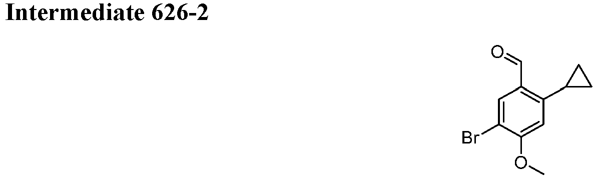

Preparation of 5-bromo-2-cyclopropyl-4-methoxybenzaldehyde. To **626-1** (0.30 g, 1.9 mmol) in MeOH (10 mL), cooled to 0 °C, was added pyridine hydrobromide perbromide (0.60 g, 1.9 mmol). After 24 h, the solvent was removed *in vacuo* and the residue was purified by normal phase silica gel chromatography elueting with hexanes/EtOAc to afford intermediate **626-2** (0.31 g, 1.2 mmol, 65 % yield) as a white solid. ¹H NMR (400 MHz, CDCl₃) δ 10.45 (s, 1H), 8.04 (s, 1H), 6.63 (s, 1H), 3.98 (s, 3H), 2.68 - 2.55 (m, 1H), 1.20 - 1.11 (m, 2H), 0.88 - 0.78 (m, 2H).

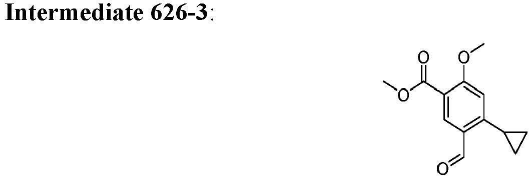

Preparation of methyl 4-cyclopropyl-5-formyl-2-methoxybenzoate. Intermediate **626-3** (0.16 g, 0.70 mmol, 58% yield) was prepared in a similar manner as intermediate **323-1** with the exception of using PdOAc₂, dppf and DMSO/ MeOH. ¹H NMR (400 MHz, CDCl₃) δ 10.40 (s, 1H), 8.33 (s, 1H), 6.64 (s, 1H), 3.99 (s, 3H), 3.93 (s, 3H), 2.96 - 2.71 (m, 1H), 1.25 - 1.13 (m, 2H), 0.87 (dd, *J*=5.3, 1.8 Hz, 2H). LCMS(ESI) *m*/*z*:235.2 (M+H).⁺

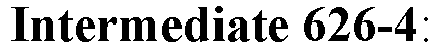

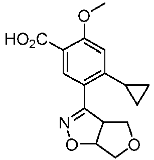

Preparation of 4-cyclopropyl-2-methoxy-5-(3a,4,6,6a-tetrahydrofuro[3,4-d]isoxazol-3-yl)benzoic acid. Following the general procedures for example **378** and example **416,** but substituting **626-3** for methyl 5-formyl-2-methoxybenzoate afforded **626-4** (46 mg, 0.15 mmol, 91 % yield). ¹H NMR (400 MHz, CDCl₃) δ 8.10 (s, 1H), 6.68 (s, 1H), 5.38 (dd, *J*=9.2, 3.7 Hz, 1H), 4.48 (t, *J*=7.5 Hz, 1H), 4.37 (d, *J*=10.6 Hz, 1H), 4.12 (s, 3H), 4.03 (d, *J*=9.5 Hz, 1H), 3.86 - 3.75 (m, 2H), 2.77 - 2.63 (m, 1H), 1.28 - 1.20 (m, 1H), 1.18 - 1.10 (m, 1H), 0.85 - 0.72 (m, 2H). LCMS(ESI) *m*/*z*: 304.3 (M+H).⁺

Example **626.** A mixture of diastereomers was prepared by BOP coupling as described in example **378** substituting cyclopropyl norbornyl intermediate **166-2** and intermediate **626-4** for intermediate **378-3** and the cyclobutyl norbornyl intermediate **369-1.** The mixture of diastereomers was separated into individual isomers using chiral SFC Instrument: Waters 100 Prep SFC Column: Chiral AD, 30 x 250 mm, 5 micron, Mobile Phase: 25% MeOH / 75% CO2 w/0.1% DEA, Flow Conditions: 100 mL/min, Detector Wavelength: 220 nm; Analytical method:Intrument: Shimadzu Nexera SFC, Column Chiral AD, 4.6 x 100 mm, 5 micron, Mobile Phase: 25% MeOH / 75% CO2 w/0.1% DEA, Flow Conditions: 2 mL/min, Detector Wavelength: 220 nm, to afford chiral peak-1 (example **626)** (8.2 mg, 12 µmol, 16 % yield), RT=2.6 min., >95% de and chiral peak-2 (8.1 mg, 12 µmol, 16 % yield), RT=3.3 min., >95% de. For example **626:** ¹H NMR (500 MHz, DMSO-d₆) δ 10.53 (s, 1H), 9.85 (br d, *J*=7.0 Hz, 1H), 8.31 - 8.18 (m, 1H), 7.90 (s, 1H), 7.82 - 7.74 (m, 1H), 7.48 (t, *J*=9.6 Hz, 1H), 6.68 (s, 1H), 5.32 (dd, *J*=9.0, 3.5 Hz, 1H), 4.69 (d, *J*=9.5 Hz, 1H), 4.56 (br t, *J*=7.6 Hz, 1H), 4.47 - 4.39 (m, 1H), 4.11 (br d, *J*=10.7 Hz, 1H), 4.04 (s, 3H), 3.76 (br d, *J*=8.9 Hz, 1H), 3.65 (br dd, *J*=10.5, 4.4 Hz, 1H), 3.48 (br s, 1H), 3.15 (br dd, *J*=10.8, 3.2 Hz, 1H), 3.09 (br s, 1H), 2.78 - 2.67 (m, 1H), 2.41 - 2.32 (m, 1H), 1.92 - 1.81 (m, 1H), 1.79 - 1.72 (m, 1H), 1.56 - 1.46 (m, 1H), 1.45 - 1.33 (m, 2H), 1.06 (br dd, *J*=8.7, 5.6 Hz, 1H), 1.02 - 0.94 (m, 1H), 0.93 - 0.87 (m, 1H), 0.87 - 0.81 (m, 1H), 0.79 - 0.67 (m, 2H), 0.35 (br s, 2H). LCMS(ESI) *m*/*z*: 653.93 (M+H).⁺ HPLC purity 95% with retention time 2.65 min. [method C].

### Example 664 and Example 702

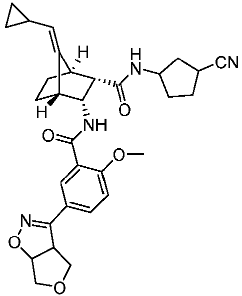

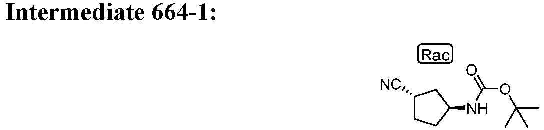

rac-tert-butyl ((1R,3S)-3-hydroxycyclopentyl)carbamate (260 mg, 1.29 mmol) was dissolved in in DCM (15 mL). Hunig's base (1.13 mL, 6.46 mmol) and MsCl (0.101 mL, 1.29 mmol) were added. After 2 hours, the reaction mixture was concentrated in vacuo. The residue was diluted with acetonitrile (20 mL), and tetrabutylammonium cyanide (347 mg, 1.29 mmol) was added. After 1 hour, the reaction mixture was concentrated in vacuo. The residue was purified via silica gel chromatography to furnish **664-1** (135 mg, 50%). ¹H NMR (400 MHz, CDCl₃) δ 4.48 (br d, J=4.3 Hz, 1H), 4.18 - 4.05 (m, 1H), 2.99 - 2.85 (m, 1H), 2.30 - 2.13 (m, 3H), 2.06 - 1.85 (m, 2H), 1.58 - 1.49 (m, 1H), 1.44 (s, 9H).

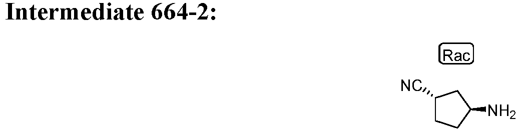

rac-tert-butyl ((1R,3R)-3-cyanocyclopentyl)carbamate (65 mg, 0.31 mmol) was dissolved in DCM (1.9 mL). TFA (0.19 mL, 2.5 mmol) was added. After 3 hours, TFA (0.19 mL, 2.5 mmol) was added. After a further 1.5 hours, the reaction mixture was concentrated in vacuo, then azeotroped with DCM and hexanes. rac-(1R,3R)-3-aminocyclopentane-1-carbonitrile, TFA (131 mg, 0.584 mmol, 189 % yield) was obtained and taken forward without further purification. ¹H NMR (400 MHz, CD₃OD) δ 3.99 - 3.84 (m, 1H), 2.53 - 2.34 (m, 3H), 2.31 - 2.19 (m, 1H), 2.11 - 2.00 (m, 1H), 1.85 (dt, *J*=14.1, 7.0 Hz, 1H).

### Example 642 and Example 702:

A mixture of **Example 642** and **Example 702** was prepared from **Intermediate 642-2** according to the procedure described for **Example 447.** The material obtained was purified via preparative reverse phas HPLC with the following conditions: Column: XBridge C18, 200 mm x 19 mm, 5-µm particles; Mobile Phase A: 5:95 acetonitrile: water with ammonium acetate; Mobile Phase B: 95:5 acetonitrile: water with ammonium acetate; Gradient: a 0-minute hold at 25% B, 25-70% B over 30 minutes, then a 0-minute hold at 100% B; Flow Rate: 20 mL/min; Column Temperature: 25 °C. Fraction collection was triggered by MS signals. Fractions containing the desired product were combined and dried via centrifugal evaporation.

Peak 1 is **Example 664** (6.3 mg, 41%). ¹H NMR (500 MHz, DMSO-d₆) δ 9.95 (br d, *J*=7.0 Hz, 1H), 8.21 - 8.14 (m, 2H), 7.79 (dd, *J*=8.5, 2.1 Hz, 1H), 7.24 (d, *J*=8.5 Hz, 1H), 5.34 (dd, *J*=9.0, 3.2 Hz, 1H), 4.61 (br d, *J*=9.5 Hz, 1H), 4.50 (br t, *J*=7.3 Hz, 1H), 4.27 (br dd, *J*=6.3, 4.1 Hz, 1H), 4.22 - 4.15 (m, 1H), 4.09 (br d, *J*=10.7 Hz, 1H), 4.02 (s, 3H), 3.90 (br d, *J*=9.2 Hz, 1H), 3.77 (br dd, *J*=9.2, 7.0 Hz, 1H), 3.67 - 3.60 (m, 1H), 3.13 - 3.01 (m, 2H), 2.86 (br dd, *J*=11.0, 4.3 Hz, 1H), 2.16 - 2.01 (m, 2H), 2.00 - 1.92 (m, 1H), 1.87 - 1.78 (m, 2H), 1.78 - 1.66 (m, 2H), 1.50 - 1.40 (m, 2H), 1.39 - 1.24 (m, 2H), 0.77 - 0.64 (m, 2H), 0.31 (br d, *J*=2.4 Hz, 2H). One proton is not visible in NMR, likely due to overlap with solvent peak. LC-MS RT: 2.02 min; MS (ESI) *m*/*z* 545.2 (M+H)⁺; Method B.

Peak 2 is **Example 702** (5.1, 32%). ¹H NMR (500 MHz, DMSO-d₆) δ 9.97 (d, *J*=6.8 Hz, 1H), 8.20 - 8.12 (m, 2H), 7.79 (dd, *J*=8.6, 2.3 Hz, 1H), 7.25 (d, *J*=8.8 Hz, 1H), 5.33 (dd, *J*=9.2, 3.5 Hz, 1H), 4.61 (d, *J*=9.6 Hz, 1H), 4.49 (br t, *J*=7.8 Hz, 1H), 4.32 - 4.24 (m, 1H), 4.23 - 4.14 (m, 1H), 4.13 - 4.06 (m, 1H), 4.03 (s, 3H), 3.90 (br d, *J*=8.9 Hz, 1H), 3.77 (dd, *J*=9.4, 7.0 Hz, 1H), 3.65 (dd, *J*=10.7, 3.5 Hz, 1H), 3.12 - 3.01 (m, 2H), 2.85 (dd, *J*=10.9, 4.2 Hz, 1H), 2.18 - 1.95 (m, 3H), 1.88 - 1.68 (m, 4H), 1.52 - 1.40 (m, 2H), 1.39 - 1.24 (m, 2H), 0.70 (quin, *J*=9.4 Hz, 2H), 0.40 - 0.21 (m, 2H). One proton is not visible in NMR, likely due to overlap with solvent peak. LC-MS RT: 1.86 min; MS (ESI) *m*/*z* 545.2 (M+H)⁺; Method B.

### Example 699

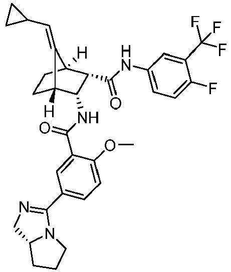

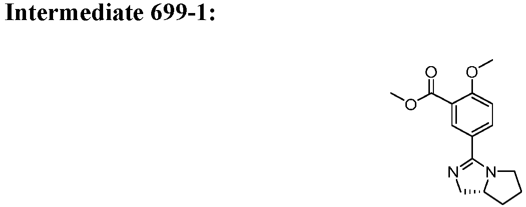

A solution of (R)-pyrrolidin-2-ylmethanamine, 2 HCl (70.2 mg, 0.406 mmol) in DCM (1.9 mL) was cooled in an ice bath. Hunig's base (0.14 mL, 0.81 mmol) and methyl 5-formyl-2-methoxybenzoate (75 mg, 0.39 mmol) were added. The reaction mixture was allowed to warm to rt. After 1 hour, NBS (72.2 mg, 0.406 mmol) was added and the reaction mixture was stirred for 14h. The reaction mixture was concentrated under reduced pressure. The residue was purified via silica gel chromatograph to furnish **Intermediate 699-1** (49 mg, 46%). ¹H NMR (400 MHz, CDCl₃) δ 8.18 (d, *J*=2.0 Hz, 1H), 7.96 (dd, *J*=8.8, 2.0 Hz, 1H), 7.02 (d, *J*=8.7 Hz, 1H), 4.09 - 4.02 (m, 2H), 3.92 (s, 3H), 3.86 (s, 3H), 3.75 (q, *J*=9.4 Hz, 1H), 3.42 - 3.27 (m, 1H), 3.24 - 3.14 (m, 1H), 2.05 - 1.91 (m, 1H), 1.90 - 1.72 (m, 2H), 1.52 - 1.40 (m, 1H).

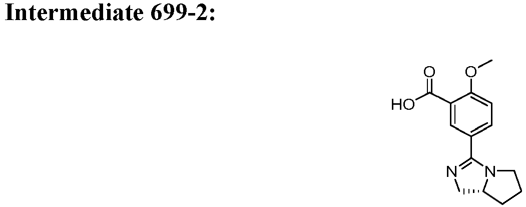

To a solution of **Intermediate 699-1** (49 mg, 0.18 mmol) in THF (1.9 mL) and MeOH (0.37 mL) was added LiOH (2M aqueous) (0.27 mL, 0.54 mmol). After 4 hours, the reaction mixture was acidfied to pH 3 with 1M HCl and concentrated under reduced pressure to give methyl (R)-2-methoxy-5-(5,6,7,7a-tetrahydro-1H-pyrrolo[1,2-c]imidazol-3-yl)benzoate **Intermediate 699-2** (49 mg, 0.18 mmol) which was used without further purification.

**Example 699** was prepared from **Intermediate 699-2** by the general procedure described for **Example 378** (3.8 mg, 31%). ¹H NMR (500 MHz, DMSO-d₆) δ 10.61 (s, 1H), 9.98 (d, *J*=6.8 Hz, 1H), 8.38 (d, *J*=2.4 Hz, 1H), 8.21 (dd, *J*=6.3, 2.2 Hz, 1H), 7.98 (dd, *J*=8.8, 2.4 Hz, 1H), 7.83 - 7.73 (m, 1H), 7.52 - 7.43 (m, 2H), 4.69 (d, *J*=9.6 Hz, 1H), 4.55 - 4.39 (m, 2H), 4.13 (s, 3H), 4.08 (t, *J=*11.5 Hz, 1H), 3.81 (dd, *J*=11.9*,* 7.1 Hz, 1H), 3.74 - 3.64 (m, 1H), 3.17 (dd, *J*=10.2, 4.9 Hz, 1H), 3.10 (br s, 1H), 2.73 (br s, 1H), 2.17 - 2.08 (m, 1H), 2.07 - 1.96 (m, 2H), 1.88 - 1.72 (m, 2H), 1.72 - 1.60 (m, 1H), 1.52 - 1.35 (m, 3H), 0.79 - 0.66 (m, 2H), 0.39 - 0.27 (m, 2H). One proton is not visible in NMR, likely due to overlap with suppressed water peak. LC-MS RT: 2.20 min; MS (ESI) *m*/*z* 611.2 (M+H)⁺; Method B.

### Example 724

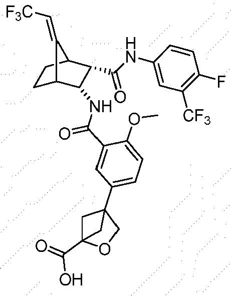

**Intermediate 724-1:** Preparation of tert-butyl 5-bromo-2-methoxybenzoate: To a solution of 5-bromo-2-methoxybenzoic acid (3.67 g, 15.9 mmol) in THF (50 mL) was added Boc-anhydride (7.38 mL, 31.8 mmol) and DMAP (0.194 g, 1.59 mmol). To this mixture was then added *t*-BuOH (50 mL) and the reaction mixture was then heated for 14h at 75 °C. The reaction mixture was then concentrated under vacuum and the residue was subjected to silica gel chromatography to yield intermediate **724-1** (4.1 g, 81% yield). ¹H NMR (500 MHz, CDCl₃) δ 7.81 (d, *J*=2.6 Hz, 1H), 7.52 (dd, *J*=8.9, 2.6 Hz, 1H), 6.85 (d, *J*=8.9 Hz, 1H), 3.88 (s, 3H), 1.61 - 1.57 (m, 9H)

**Intermediate 724-2:** Preparation of tert-butyl 5-(3-hydroxyprop-1-en-2-yl)-2-methoxybenzoate: To a solution of prop-2-en-1-ol (1.42 mL, 20.9 mmol) and intermediate **724-1** (1200 mg, 4.18 mmol) in DMSO (3 mL) was added TEA (1.05 mL, 7.52 mmol) and 1,3-bis(diphenylphosphino)propane (345 mg, 0.836 mmol) under argon. To the reaction mixture was added Pd(OAc)₂ (94 mg, 0.42 mmol) and the mixture was purged with argon for 10 mins. The reaction mixture was then sealed and stirred for 14h at 60 °C. The reaction mixture was allowed to cool to room temperature, diluted with EtOAc and the organic portion washed with brine. The organic portion was dried over MgSO₄, filtered and purified using silica gel chromatography to yield intermediate **724-2** (222 mg, 19.0% yield). ¹H NMR (500 MHz, CDCl₃) δ 7.82 (d, *J*=2.4 Hz, 1H), 7.53 (dd, *J*=8.6, 2.5 Hz, 1H), 6.95 (d, *J*=8.7 Hz, 1H), 5.43 (d, *J*=0.8 Hz, 1H), 5.32 (q, *J*=1.2 Hz, 1H), 4.53 (d, *J*=5.3 Hz, 2H), 3.92 - 3.89 (s, 3H), 1.62 - 1.59 (m, 9H)

**Intermediate 724-3:** Preparation of dimethyl 2-((2-(3-(tert-butoxycarbonyl)-4-methoxyphenyl)allyl) oxy)malonate: To a solution of intermediate **724-2** (222 mg, 0.840 mmol) in toluene (5 mL) was added Rh₂(OAc)₄ (19 mg, 0.042 mmol). The reaction mixture was then flushed with nitrogen before heating to reflux. To this refluxing solution was then added dimethyl 2-diazomalonate (133 mg, 0.840 mmol) in toluene (1 mL) over ~5 minutes. After continued relux for 30 minutes, the reaction mixture was allowed to cool to room temperature and concentrated under vacuum. The residue was purified using silica gel chromatography to yield intermediate **724-3** (192 mg, 58.0% yield). ¹H NMR (500 MHz, CDCl₃) δ 7.85 (d, *J*=2.4 Hz, 1H), 7.60 (dd, *J*=8.7, 2.4 Hz, 1H), 6.93 (d, *J*=8.7 Hz, 1H), 5.59 - 5.56 (m, 1H), 5.34 (d, *J*=0.9 Hz, 1H), 4.64 (s, 1H), 4.55 (d, *J*=0.8 Hz, 2H), 3.91 (s, 3H), 3.79 (s, 6H), 1.60 (s, 9H).

**Intermediate 724-4:** Preparation of dimethyl 2-((2-(3-(tert-butoxycarbonyl)-4-methoxyphenyl)allyl)oxy)-2-((dimethylamino)methyl)malonate: To a solution of intermediate **724-3** (192 mg, 0.487 mmol) in DCM (7 mL) was added Eschenmoser's salt (135 mg, 0.730 mmol) followed by TEA (0.10 mL, 0.73 mmol). The reaction mixture was stirred at rt for 14h. The reaction mixture was allowed to cool to room temperature and concentrated under vacuum. The residue was purified using silica gel chromatography to yield intermediate **724-4** (100 mg, 45% yield). MS (ESI) m/z = 452.5 (M+H).

**Intermediate 724-5:** Preparation of 2-((2-(3-(tert-butoxycarbonyl)-4-methoxyphenyl)allyl)oxy)-3-methoxy-2-(methoxycarbonyl)-N,N,N-trimethyl-3-oxopropan-1-aminium: To a solution of intermediate **724-4** (100 mg, 0.221 mmol) in acetone (5 mL) was added methyl iodide (0.021 mL, 0.33 mmol) and the reaction mixture stirred at room temperature for 14h. The reaction mixture was concentrated under vacuum to yield intermediate **724-5** which was used without further purification, (100 mg, 87% yield). MS (ESI) m/z = 466.5 (M+H).

**Intermediate 724-6:** Preparation of tert-butyl 2-methoxy-5-(3-((3-methoxy-3-oxoprop-1-en-2-yl)oxy)prop-1-en-2-yl)benzoate: To a solution of intermediate **724-5** (100 mg, 0.214 mmol) in DMSO (4 mL) was added NaOH (2M, 0.13 mL, 0.26 mmol) and the reaction mixture was stirred at room temperature for 3 h. The reaction mixture was concentrated under vacuum and purified using silica gel chromatography to yield intermediate **724-6** (50 mg, 64% yield). MS (ESI) m/z = 349.0 (M+H).

**Intermediate 724-7:** Preparation of methyl 4-(3-(tert-butoxycarbonyl)-4-methoxyphenyl)-2-oxabicyclo[2.1.1]hexane-1-carboxylate: To a solution of intermediate **724-6** (50 mg, 0.14 mmol) in DMSO (30 mL) was added (Ir[dF(CF₃)ppy]₂(dtbpy))PF₆ (1.6 mg, 1.4 µmol) and the reaction mixture degassed three times under N₂. The reaction mixture wasstirred for 48h in the presence of blue LED lights. The reaction mixture was diluted with EtOAc and the organic portion washed with brine. The organic portion was dried over MgSO₄, filtered and purified using silica gel chromatography to yield intermediate **724-7** (13 mg, 23% yield). ¹H NMR (500 MHz, CDCl₃) δ 7.75 (d, *J*=2.4 Hz, 1H), 7.45 (dd, *J*=8.6, 2.5 Hz, 1H), 6.93 (d, *J*=8.7 Hz, 1H), 5.28 (s, 1H), 5.07 (d, *J*=0.9 Hz, 1H), 3.90 (s, 3H), 3.86 (s, 3H), 3.04 - 2.98 (m, 2H), 2.88 - 2.79 (m, 2H), 1.62 - 1.59 (m, 9H)

**Intermediate 724-8:** Preparation of 2-methoxy-5-(1-(methoxycarbonyl)-2-oxabicyclo[2.1.1]hexan-4-yl)benzoic acid, TFA: To a solution of intermediate **724-7** (13 mg, 0.037 mmol) in DCM (0.8 mL) was added TFA (0.20 mL, 2.6 mmol) and the reaction mixture stirred at rt for 30 mins. The reaction mixture was then concentrated under vacuum to yield intermediate **724-8** which was used without further purification, (11 mg, 95% yield). MS (ESI) m/z = 293.2 (M+H).

**Intermediate 724-9:** Preparation of Methyl 4-(3-(((1R,2R,3S,4R,Z)-3-((4-fluoro-3-(trifluoromethyl)phenyl)carbamoyl)-7-(2,2,2-trifluoroethylidene)bicyclo[2.2.1]heptan-2-yl)carbamoyl)-4-methoxyphenyl)-2-oxabicyclo[2.1.1]hexane-1-carboxylate: Intermediate **724-9** was prepared by the general procedures described for **378** by using trifluoromethyl norbornyl intermediate **170-2** (25 mg, 0.049 mmol) and intermediate **724-8** to yield intermediate **724-9** (6 mg, 20% yield). MS (ESI) m/z = 671.1 (M+H).

**Example 724:** 4-(3-(((1R,2R,3S,4R,Z)-3-((4-fluoro-3-(trifluoromethyl)phenyl)carbamoyl)-7-(2,2,2-trifluoroethylidene)bicyclo[2.2.1]heptan-2-yl)carbamoyl)-4-methoxyphenyl)-2-oxabicyclo[2.1.1]hexane-1-carboxylic acid **724** was prepared by dissolving intermediate **724-9** (6 mg, 9 µmol) in THF (2 mL) and water (0.67 mL) and adding LiOH (2 M, 0.013 mL, 0.027 mmol). The reaction mixture was then stirred at rt for 2 h. The reaction mixture was concentrated under vacuum and purified using reverse phase preparative HPLC to yield **724** (4.8 mg, 75% yield). ¹H NMR (500 MHz, CD₃OD) δ 10.26 - 10.17 (m, 1H), 8.14 (dd, *J*=6.3, 2.6 Hz, 1H), 8.00 - 7.92 (m, 1H), 7.80 - 7.72 (m, 1H), 7.50 - 7.42 (m, 1H), 7.29 (t, *J=*9.6 Hz, 1H), 7.17 (d, *J*=8.5 Hz, 1H), 5.78 - 5.71 (m, 1H), 4.67 - 4.60 (m, 1H), 4.07 (s, 3H), 4.01 - 3.96 (m, 2H), 3.47 - 3.41 (m, 1H), 3.40 - 3.32 (m, 1H), 3.30 - 3.16 (m, 2H), 2.95 - 2.91 (m, 1H), 2.76 - 2.70 (m, 1H), 2.44 - 2.35 (m, 2H), 2.21 - 2.13 (m, 3H), 1.63 - 1.54 (m, 2H). MS (ESI) m/z = 657.4 (M+H). HPLC Purity: 92 %; Retention Time: 1.35 min; Method A.

### Examples 725-728

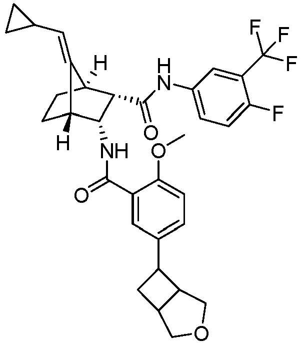

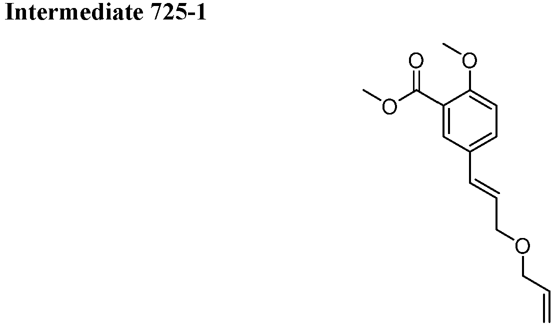

Methyl (E)-5-(3-hydroxyprop-1-en-1-yl)-2-methoxybenzoate (0.30 g, 1.3 mmol) in DMF (14 mL) was cooled to 0 °C and treated with NaH (60% in mineral oil) (0.059 g, 1.5 mmol). After 15 minutes, the reaction mixture was treated with allyl bromide (0.13 mL, 1.5 mmol) and the solution was allowed to warm to rt 14h. The reaction mixture was diluted with saturated ammonium chloride solution and extracted with EtOAc (2X). The organic portions were combined, dried over sodium sulfate, filtered and concentrated under reduced pressure. The residue was purified by silica gel chromatography to furnish methyl (E)-5-(3-(allyloxy)prop-1-en-1-yl)-2-methoxybenzoate (0.20 g, 0.76 mmol, 57 % yield). MS (ESI) *m*/*z* 263.0 (M+H)⁺.

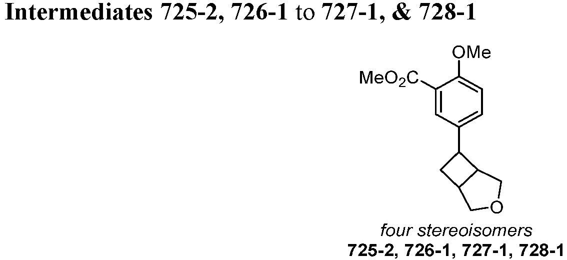

To a solution of Intermediate **725-1** (0.20 g, 0.76 mmol) dissolved in acetonitrile (75 mL) was added (Ir[dF(CF₃)ppy]₂(dtbpy))-PF₆, (9 mg, 8 µmol) and the solution irradiated with Blue LED for 60 h. The reaction mixture was concentrated under reduced pressure and the residue purified by SFC using two successive columns. First column: (A5-5 250 X 4.6 mm ID, 5µm Temperature: Ambient, Flow rate: 2.0 mL/min, Mobile Phase: 85/15 CO₂/MeOH) followed by (AD 250 X 4.6 mm ID, 5µm, Temperature: Ambient, Flow rate: 2.0 mL/min, Mobile Phase: 90/10 CO₂/MeOH) to afford **726-2** (Peak 1: 33 mg, 0.13 mmol. 17 % yield) MS (ESI) *m*/*z* 263.0 (M+H)⁺, RT = 7.2 min, AD 250 X 4.6 mm ID, 5µm, Temperature: Ambient, Flow rate: 2.0 mL/min, Mobile Phase: 90/10 CO₂/MeOH ¹H NMR (500 MHz, CDCl₃) δ 7.67 (d, *J*=2.3 Hz, 1H), 7.34 (dd, *J*=8.5, 2.4 Hz, 1H), 6.94 (d, *J*=8.5 Hz, 1H), 4.02 - 3.96 (m, 2H), 3.90 (s, 3H), 3.89 (s, 3H), 3.65 - 3.60 (m, 1H), 3.53 - 3.50 (m, 1H), 3.23 - 3.17 (m, 1H), 3.01 - 2.93 (m, 2H), 2.31 - 2.22 (m, 1H), 2.21 - 2.13 (m, 1H).

**727-1** (Peak 2: 20 mg, 0.076 mmol. 10 % yield) MS (ESI) *m*/*z* 263.0 (M+H)⁺, RT = 13.2 min, A5-5 250 X 4.6 mm ID, 5µm Temperature: Ambient, Flow rate: 2.0 mL/min, Mobile Phase: 85/15 CO₂/MeOH: ¹H NMR (500 MHz, CDCl₃) δ 7.70 (d, *J*=2.4 Hz, 1H), 7.36 (dd, *J*=8.5, 2.4 Hz, 1H), 6.96 (d, *J*=8.7 Hz, 1H), 4.00 (dd, *J*=9.2, 7.6 Hz, 2H), 3.93 (s, 3H), 3.91 (s, 3H), 3.63 (dd, *J*=9.2, 5.4 Hz, 1H), 3.53 (dd, *J*=9.4, 4.0 Hz, 1H), 3.26 - 3.19 (m, 1H), 3.04 - 2.95 (m, 2H), 2.33 - 2.24 (m, 1H), 2.22 - 2.16 (m, 1H).

**728-1** (Peak 3: 5.3 mg, 0.020 mmol. 2.7 % yield), MS (ESI) *m*/*z* 263.0 (M+H)⁺, RT = 6.11 min, AD 250 X 4.6 mm ID, 5µm, Temperature: Ambient, Flow rate: 2.0 mL/min, Mobile Phase: 90/10 CO₂/MeOH ¹H NMR (500 MHz, CDCl₃) δ 7.58 (d, *J*=2.3 Hz, 1H), 7.40 (dd, J=8.5, 2.2 Hz, 1H), 6.97 (d, *J*=8.7 Hz, 1H), 3.91 (s, 3H), 3.91 (s, 3H), 3.86 (d, *J=*9.0 Hz, 1H), 3.73 - 3.64 (m, 2H), 3.44 (dd, *J*=9.0, 4.4 Hz, 1H), 3.37 (dd, *J*=10.0*,* 6.6 Hz, 1H), 3.20 (q, *J*=7.5 Hz, 1H), 3.08 - 2.99 (m, 1H), 2.46 (dddd, *J*=12.3, 10.3, 8.2, 2.3 Hz, 1H), 2.13 (ddd, *J*=12.1*,* 9.6, 6.6 Hz, 1H)

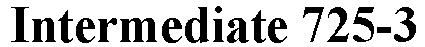

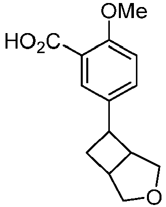

Intermediate **725-3** was prepared from **725-2** by the general procedure used for Intermediate **4-2.** LC-MS RT: 0.77 min; MS (ESI) m/z 249.0 (M+H)⁺; Method A.

**Example 725:** Prepared from intermediate **725-3** and **IV-2a** according to the general procedure for **Example 1** to afford (1R,2S,3R,4R,Z)-3-(5-(3-oxabicyclo[3.2.0]heptan-6-yl)-2-methoxybenzamido)-7-(cyclopropylmethylene)-N-(4-fluoro-3-(trifluoromethyl)phenyl)bicyclo[2.2.1]heptane-2-carboxamide (3.2 mg, 10 % yield). ¹H NMR (500 MHz, DMSO-d6) δ 10.49 (s, 1H), 9.81 (d, J=7.2 Hz, 1H), 8.23 (dd, J=6.6, 2.6 Hz, 1H), 7.84 (d, J=2.4 Hz, 1H), 7.78 (dt, J=8.5, 3.8 Hz, 1H), 7.49 (t, J=9.8 Hz, 1H), 7.41 (dd, J=8.5, 2.4 Hz, 1H), 7.13 (d, J=8.7 Hz, 1H), 4.69 (d, J=9.6 Hz, 1H), 4.49 - 4.41 (m, 1H), 3.97 (s, 3H), 3.91 - 3.84 (m, 2H), 3.48 (dd, J=9.2, 5.8 Hz, 1H), 3.39 (dd, J=9.2, 4.7 Hz, 1H), 3.15 (dd, J=10.7, 4.3 Hz, 1H), 3.13 - 3.06 (m, 2H), 3.00 - 2.90 (m, 1H), 2.89 - 2.83 (m, 1H), 2.72 (t, J=3.7 Hz, 1H), 2.16 (dt, J=12.3, 8.1 Hz, 1H), 2.09 - 2.01 (m, 1H), 1.87 (br t, J=8.7 Hz, 1H), 1.81 - 1.74 (m, 1H), 1.55 - 1.47 (m, 1H), 1.46 - 1.34 (m, 2H), 0.82 - 0.70 (m, 2H), 0.36 (dd, J=4.5, 2.5 Hz, 2H). LC-MS RT: 2.7 min; MS (ESI) *m*/*z* 599.0 (M+H)⁺; Method A.

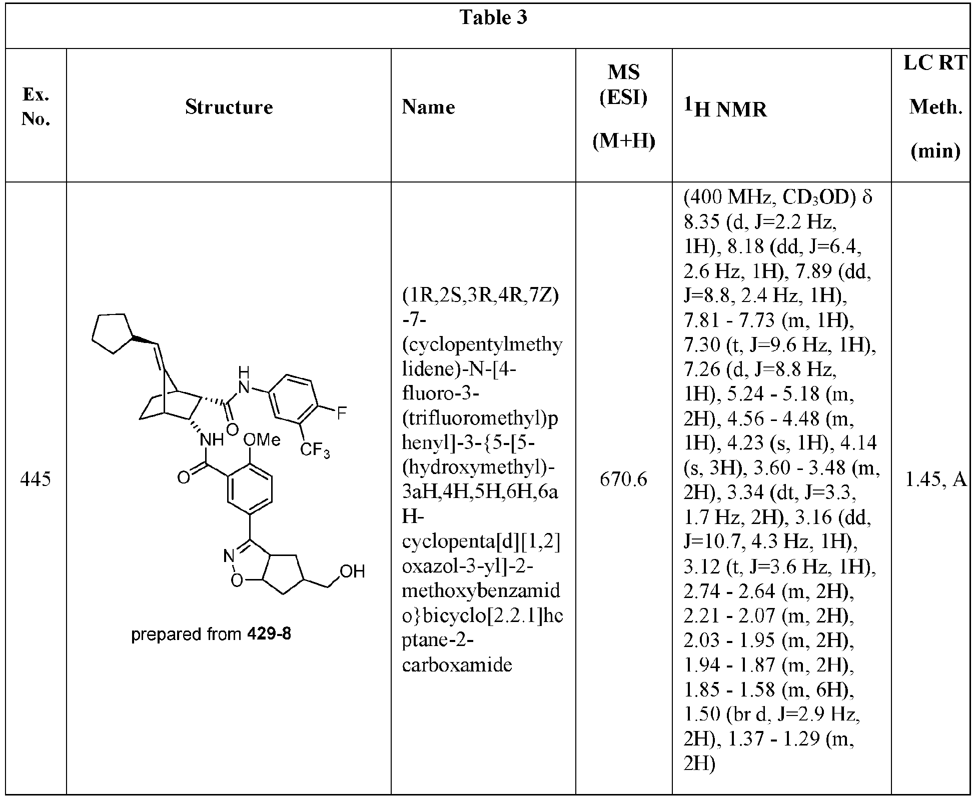

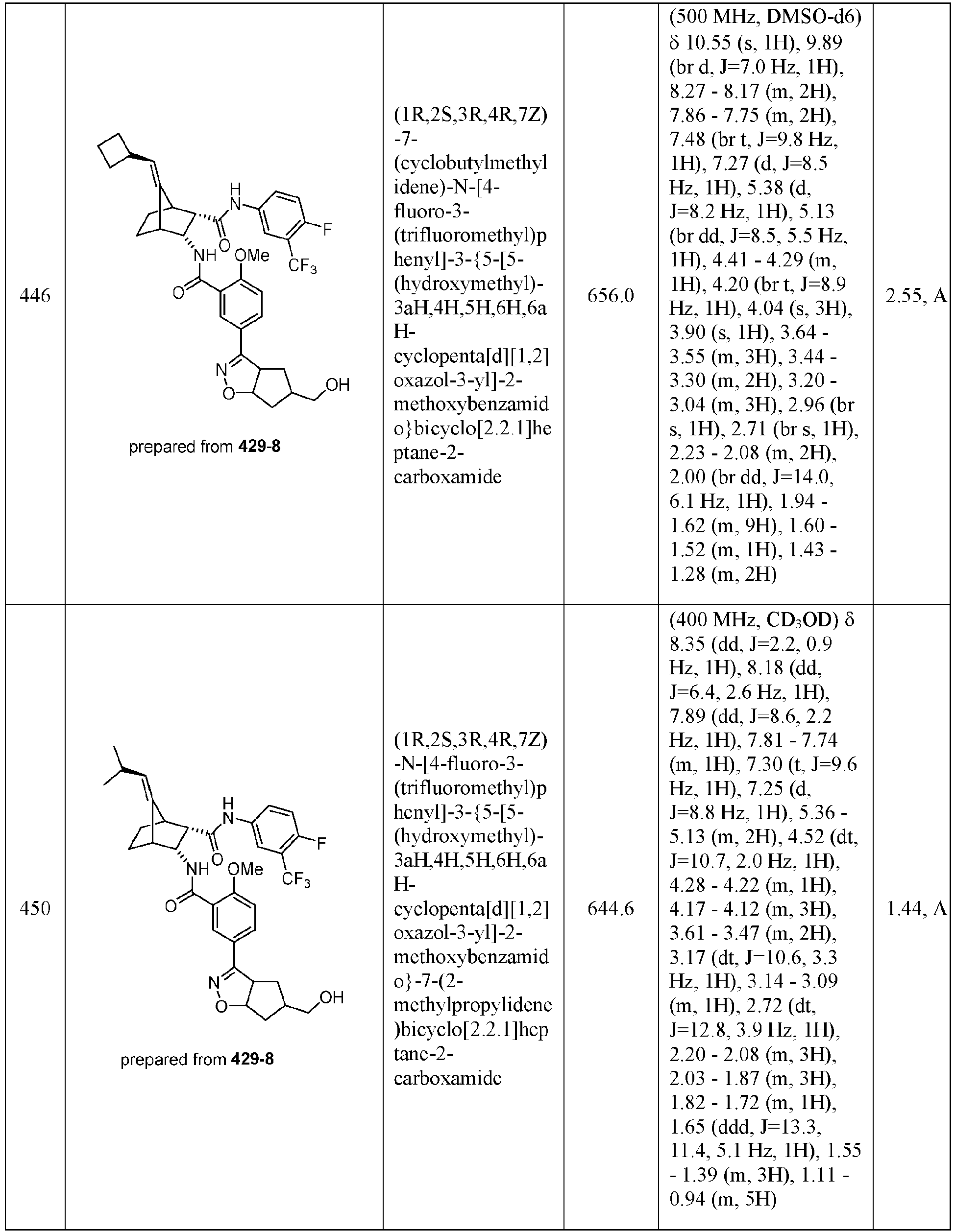

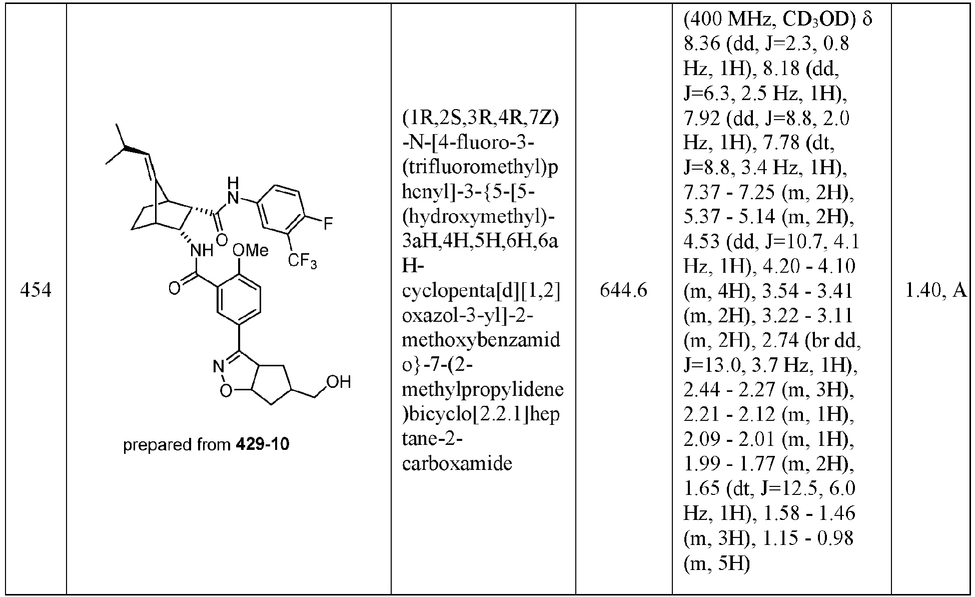

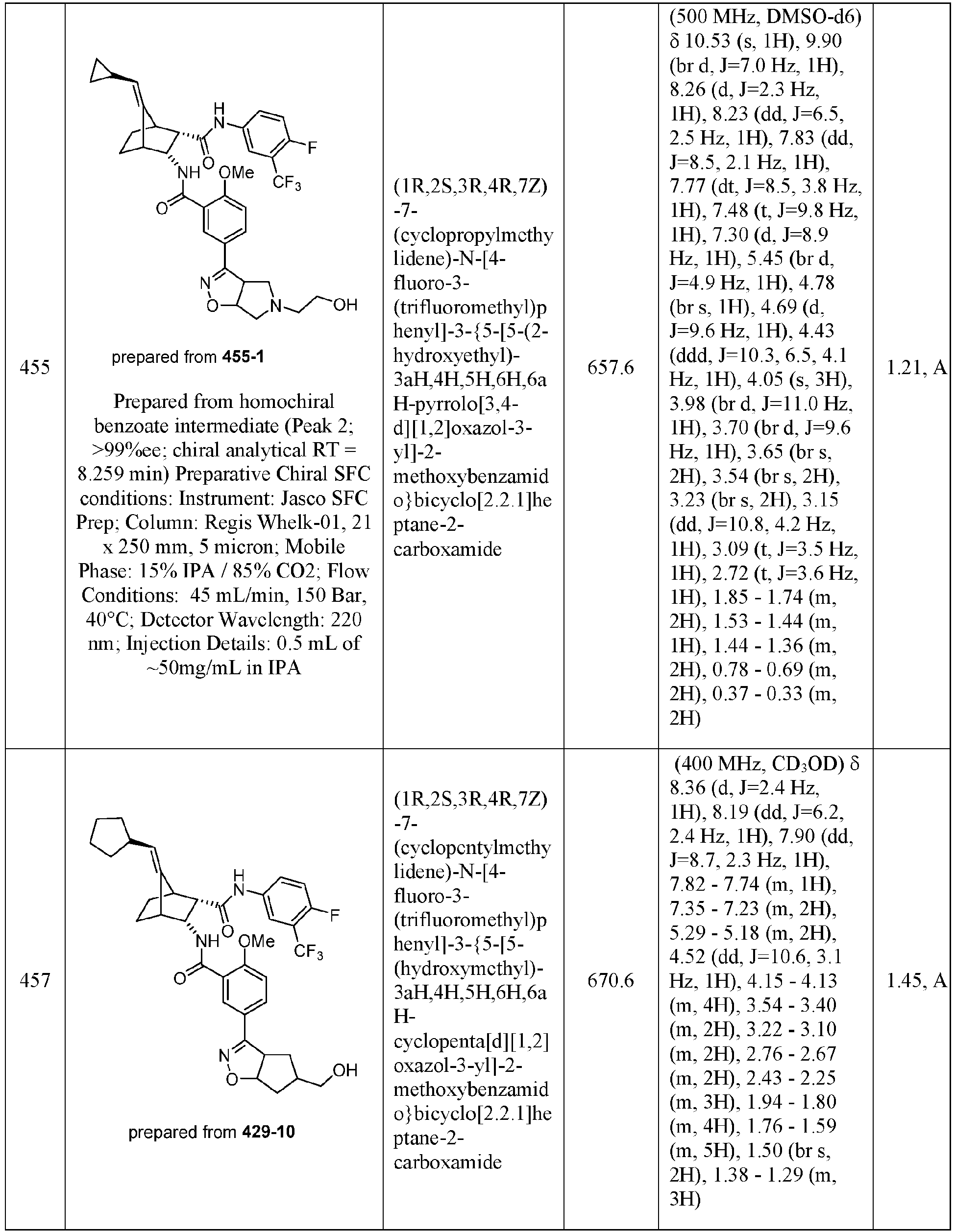

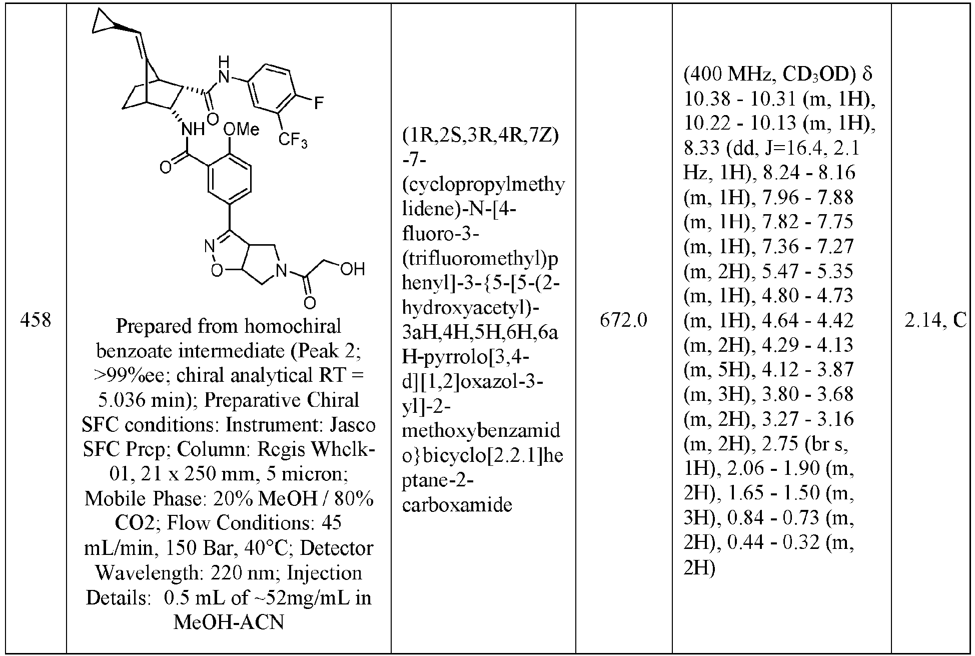

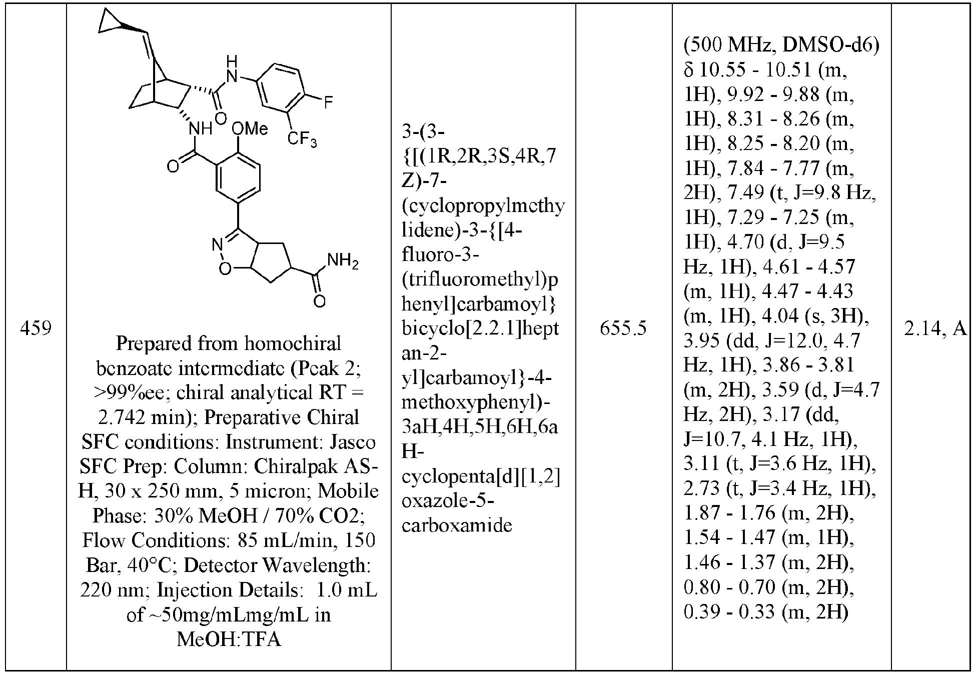

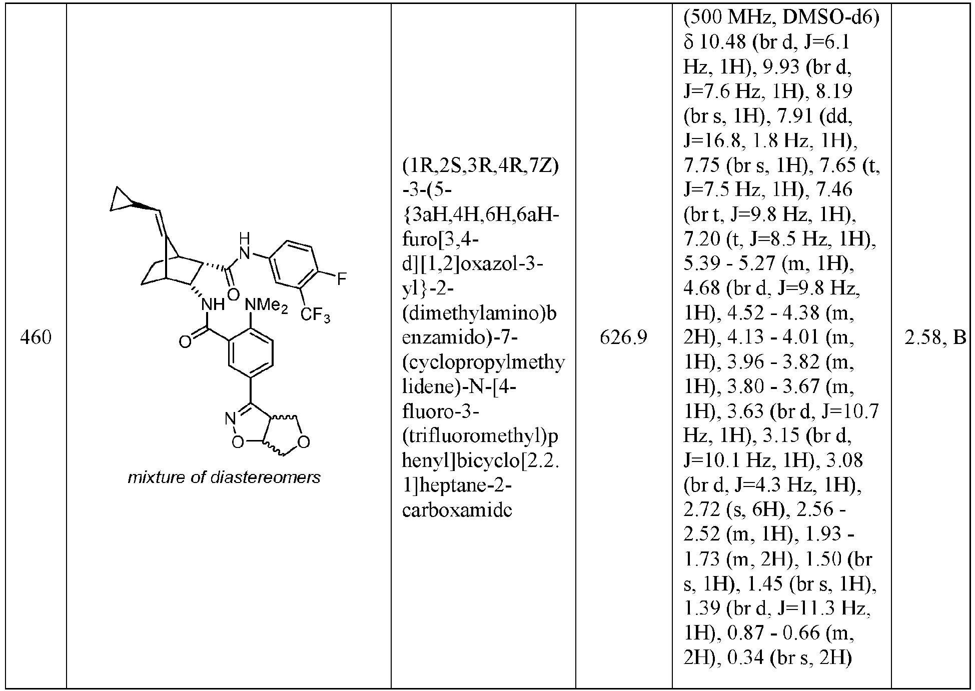

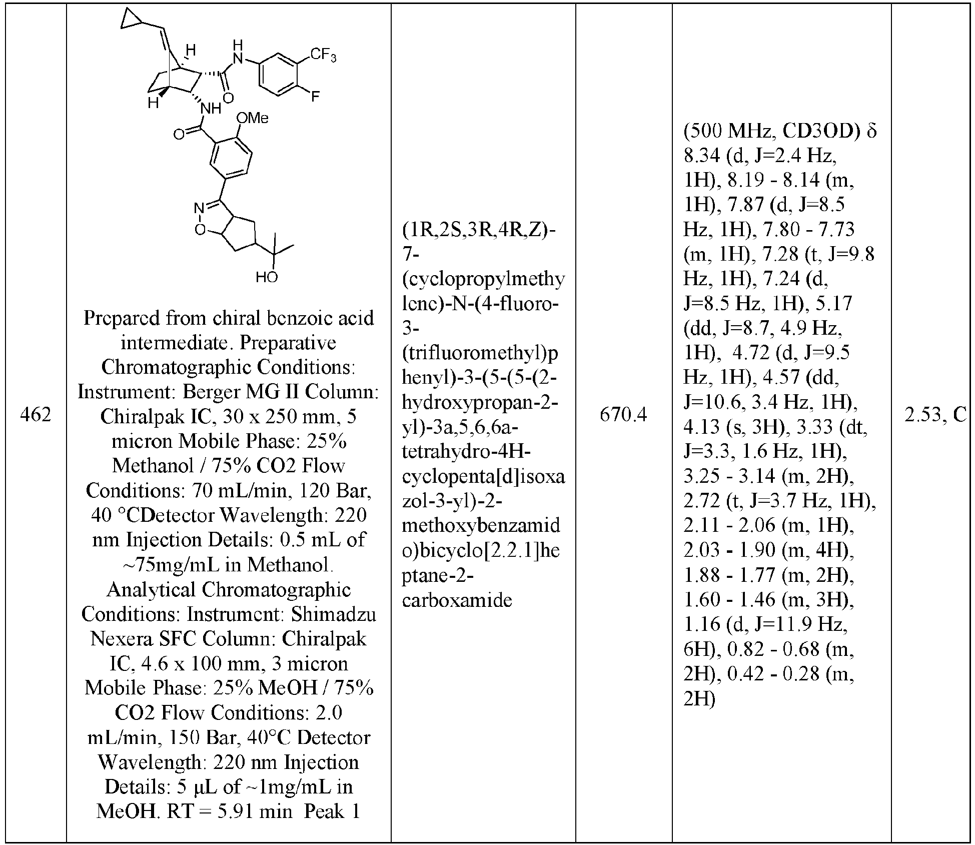

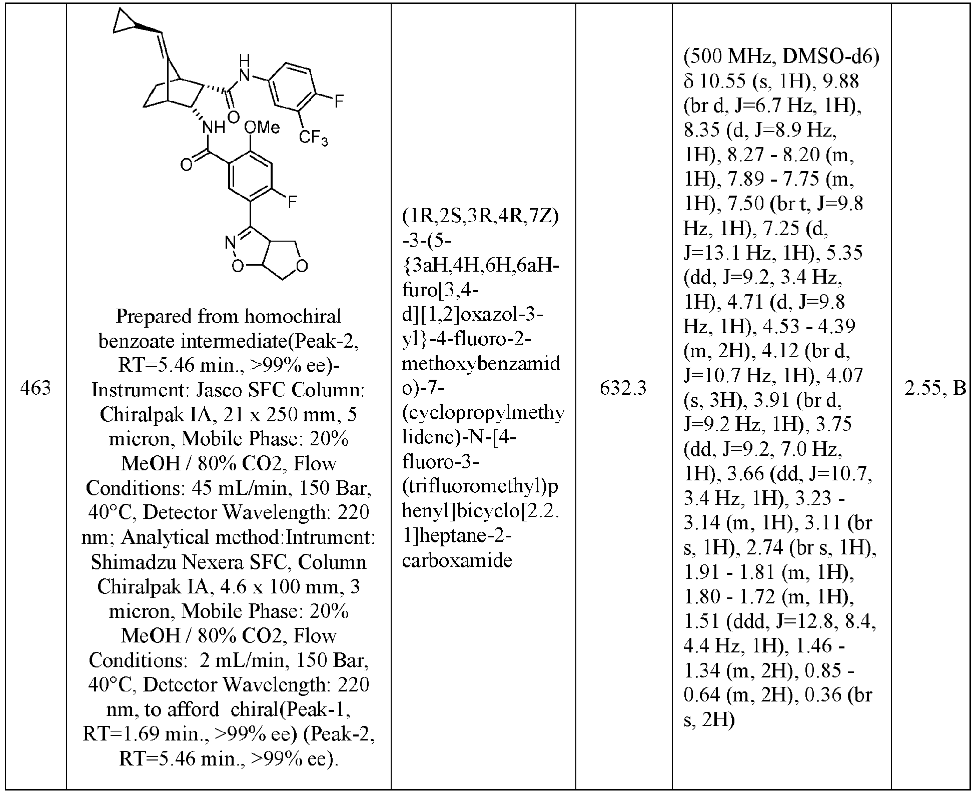

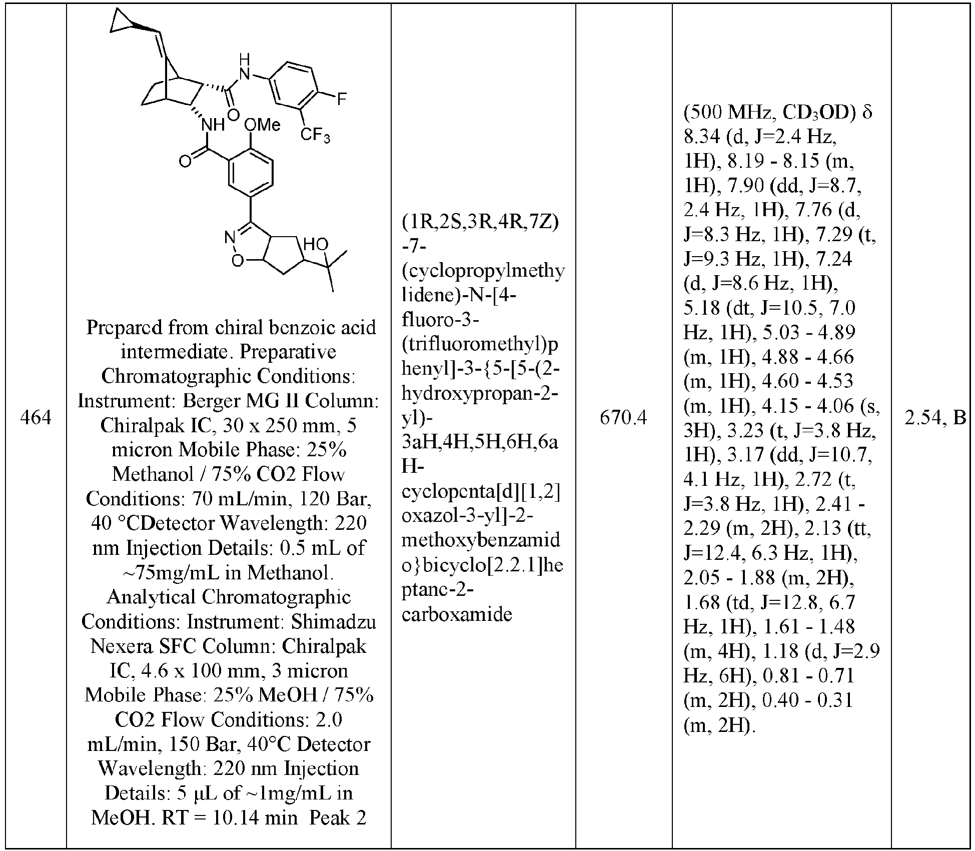

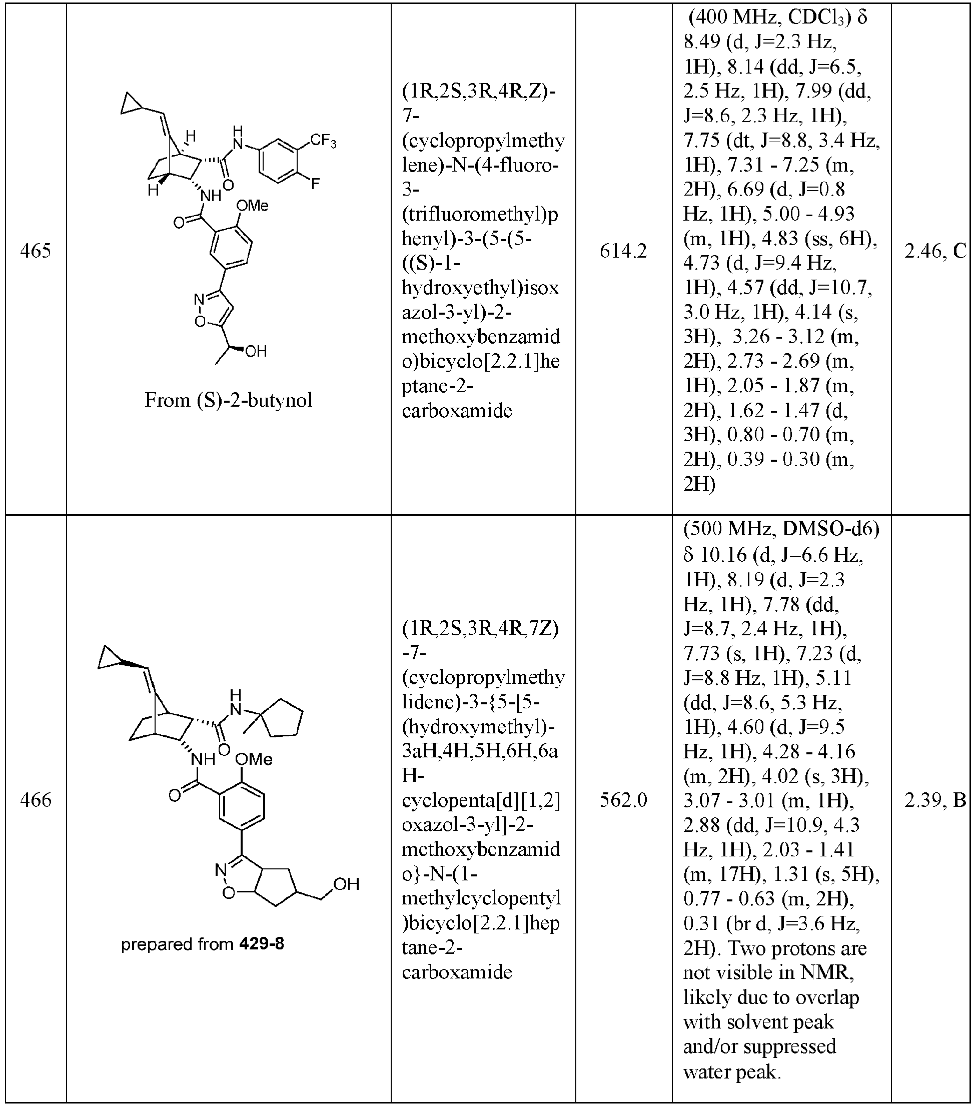

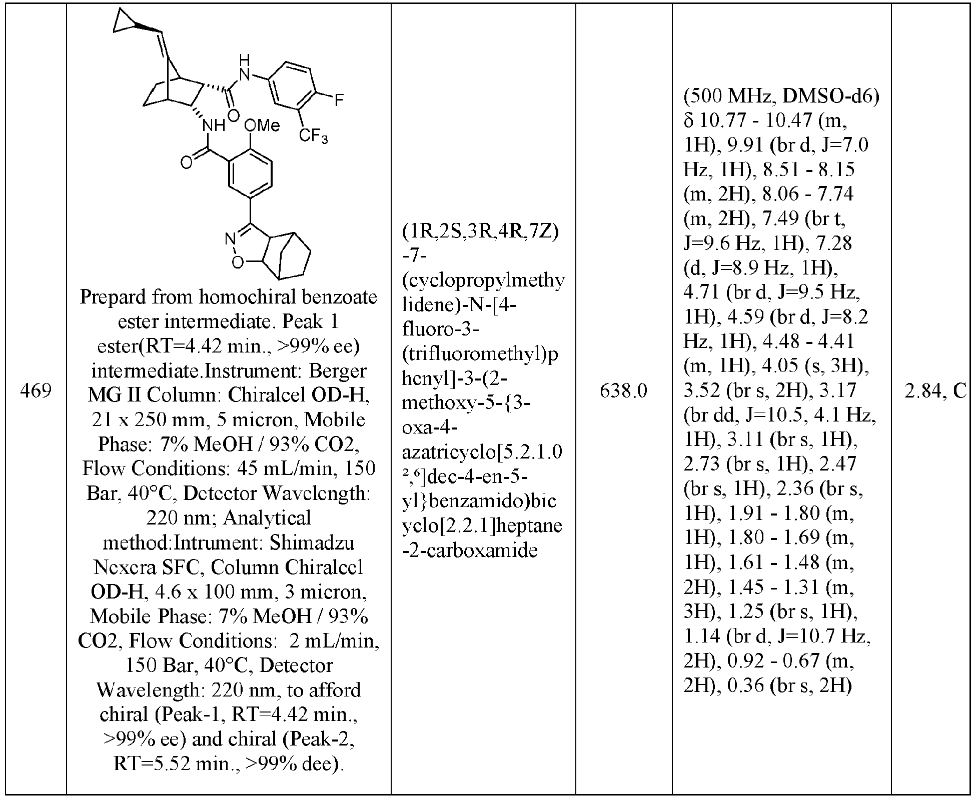

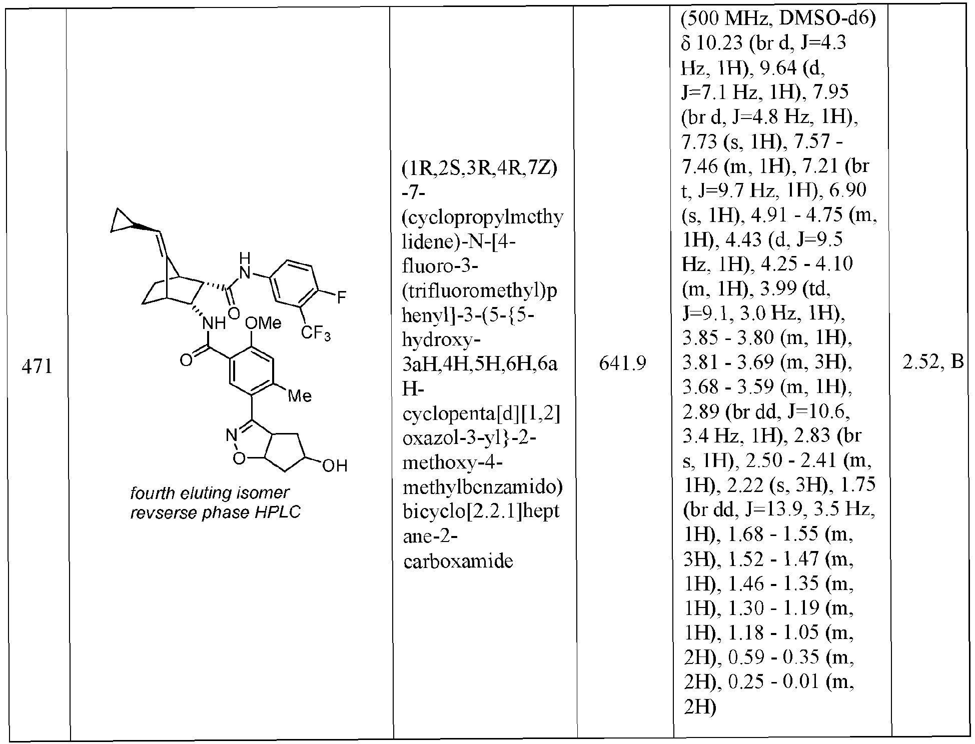

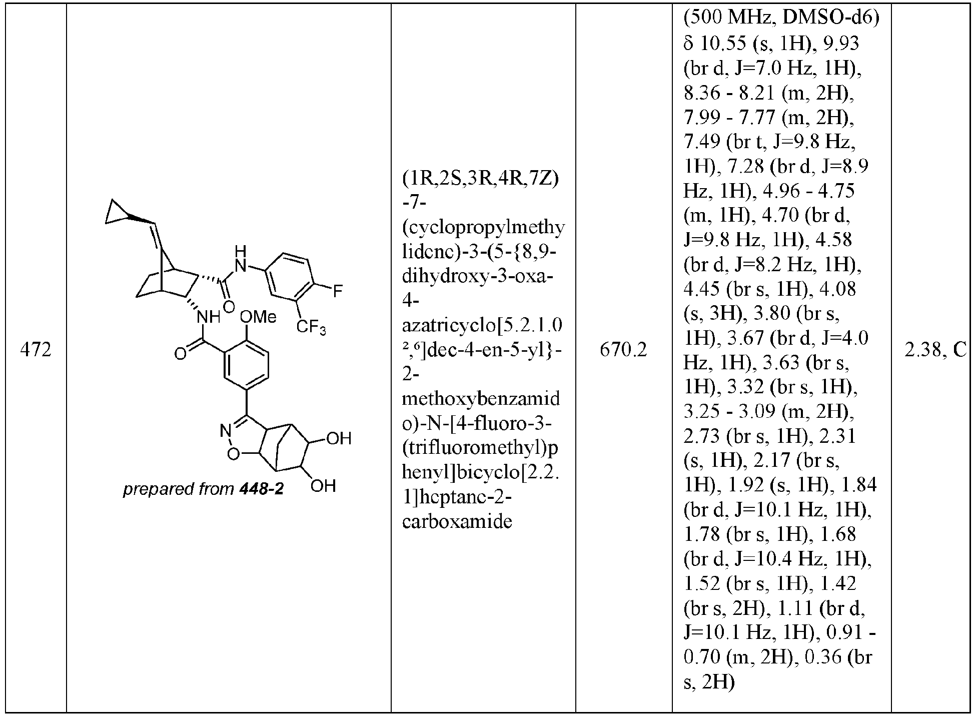

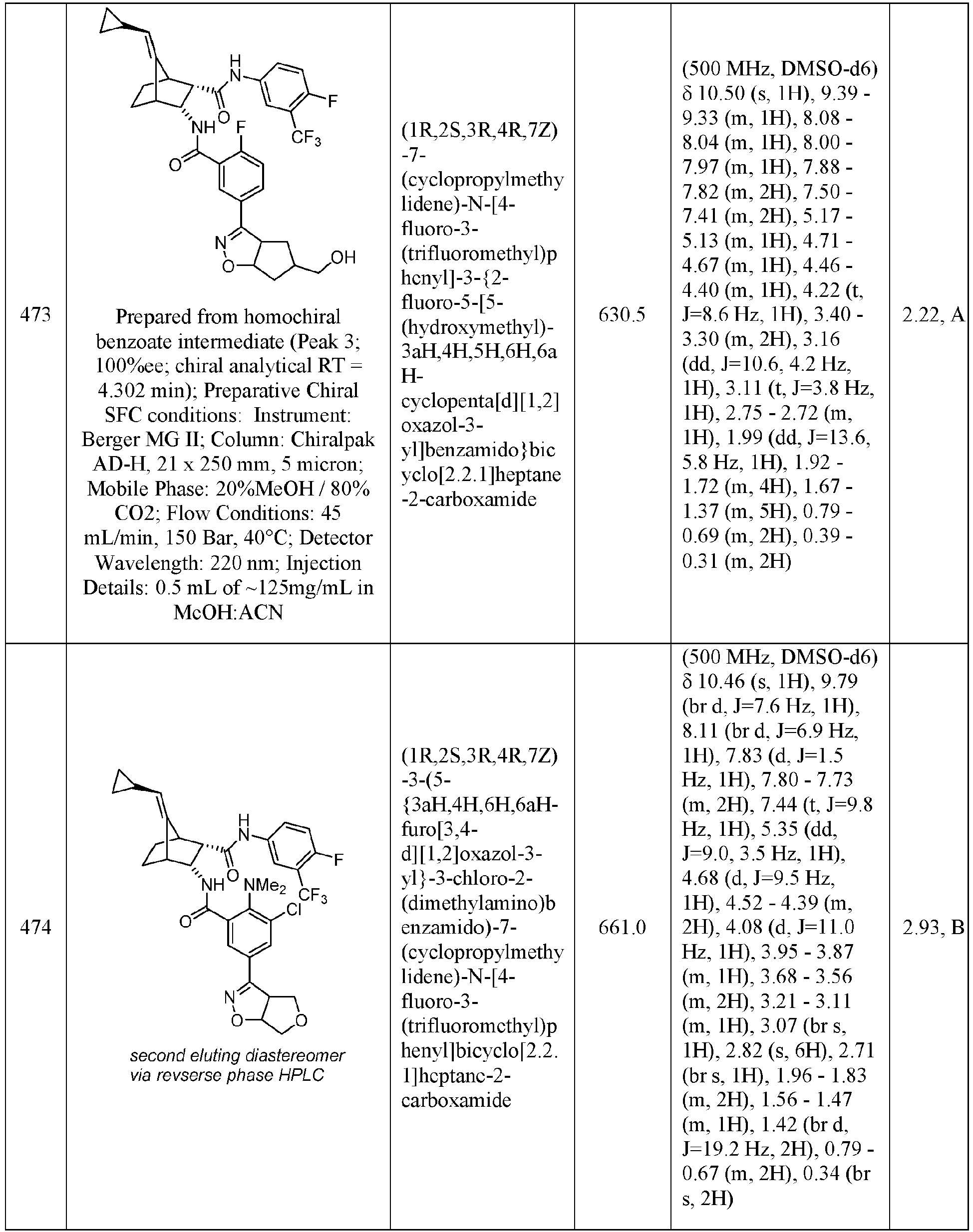

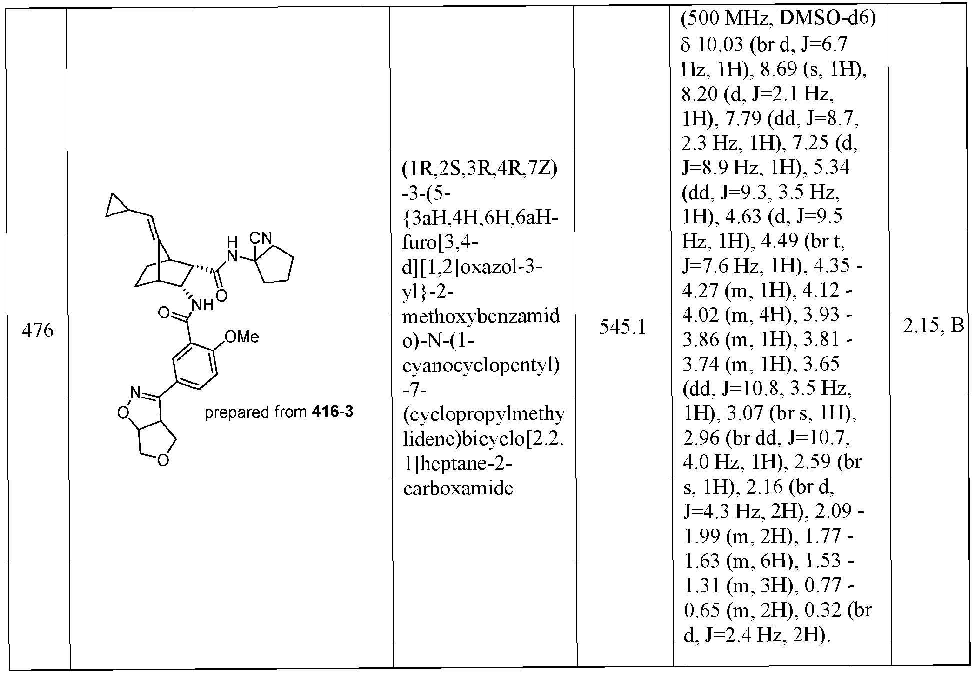

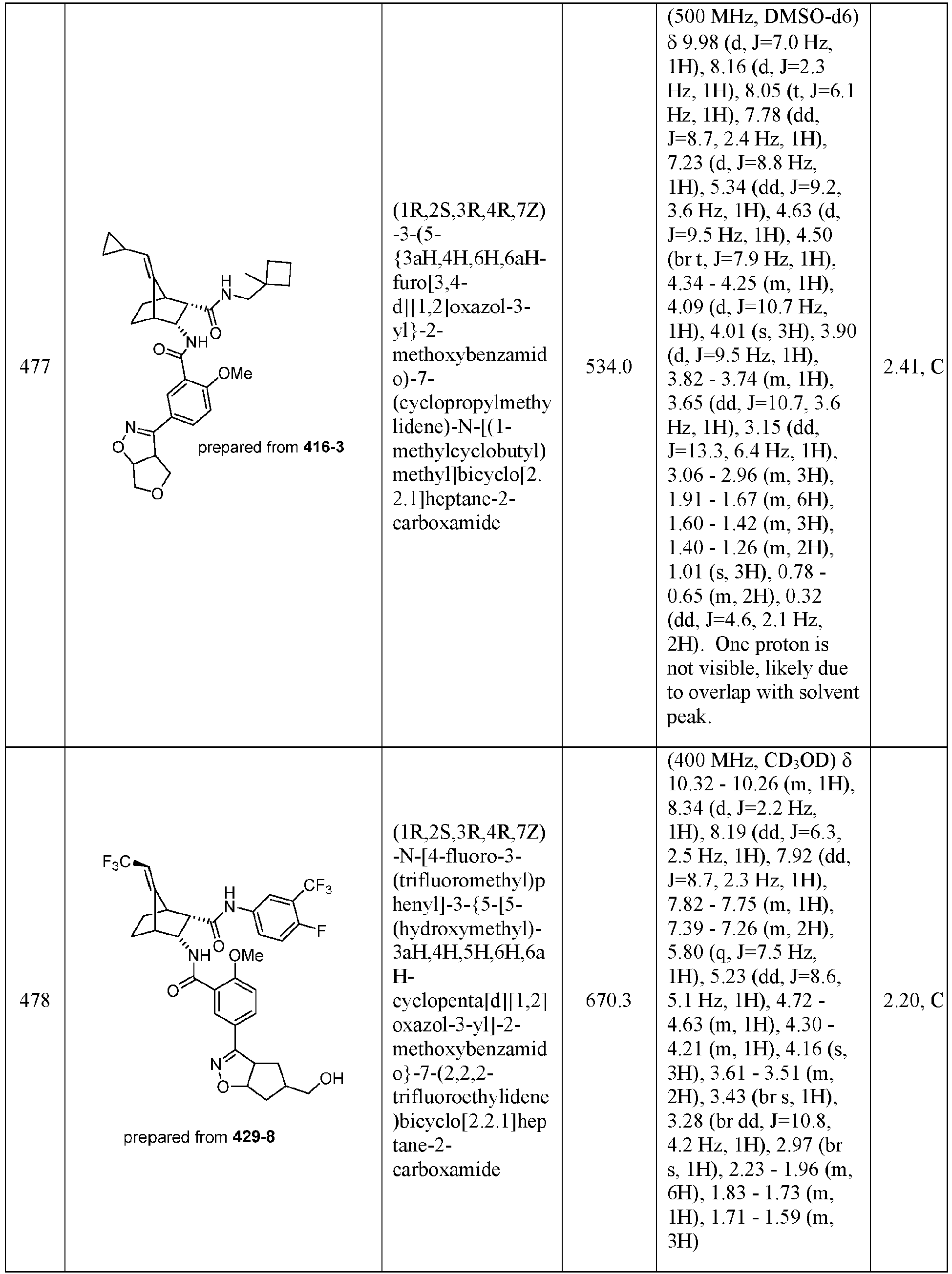

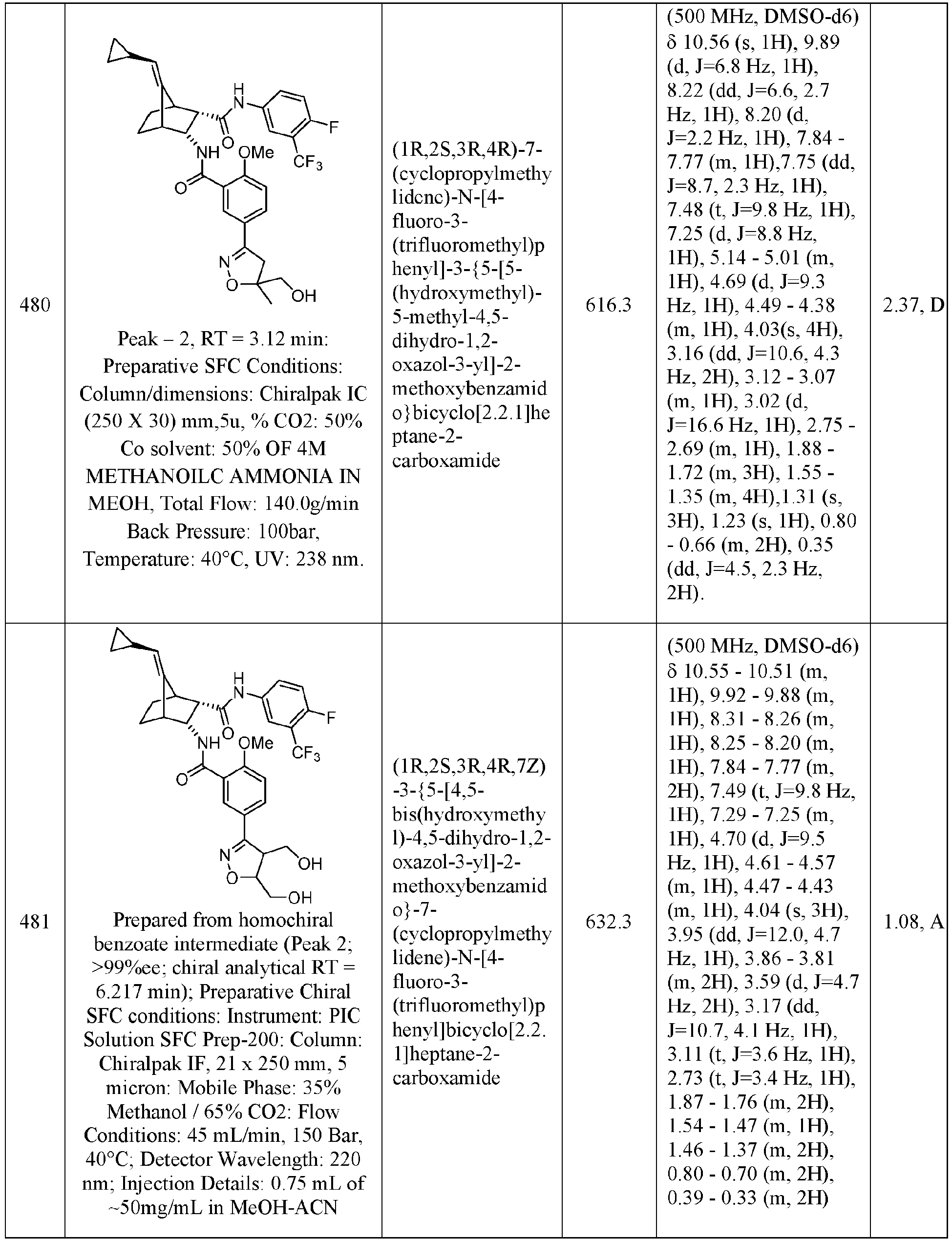

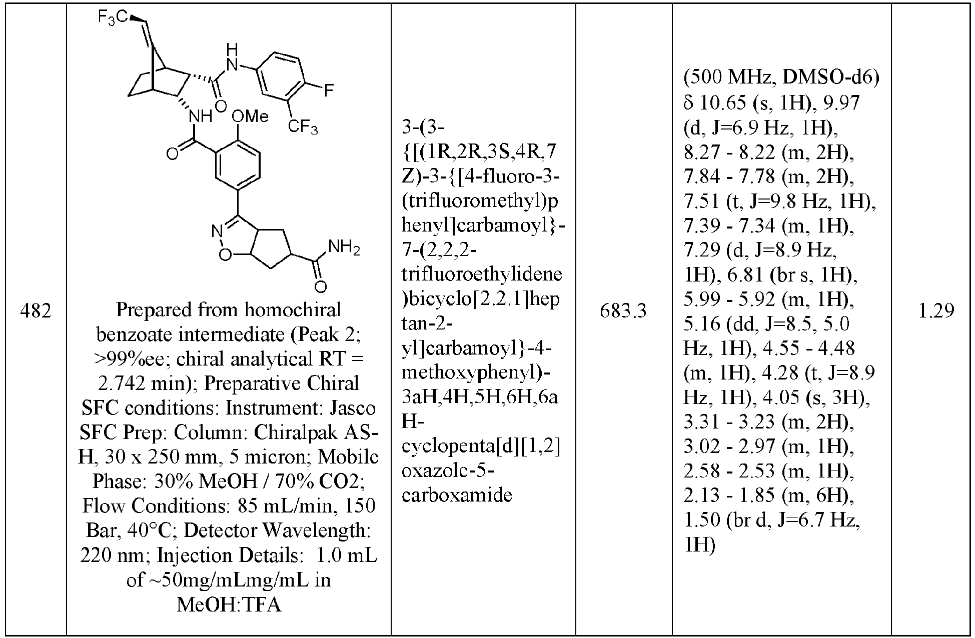

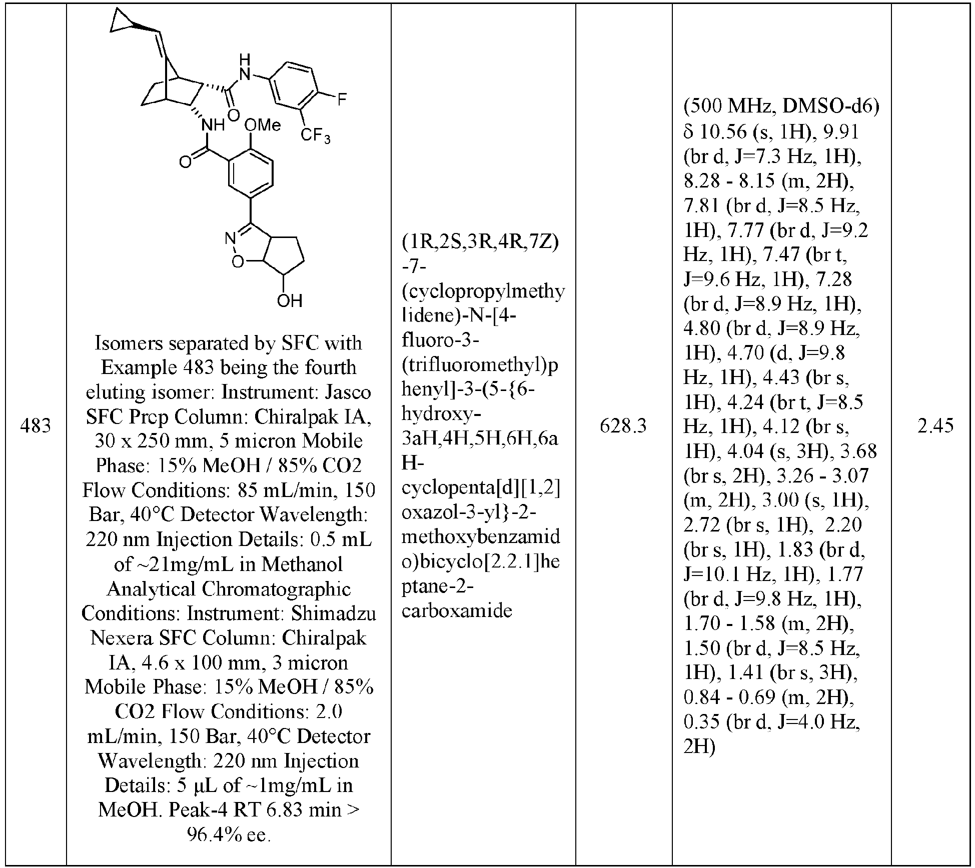

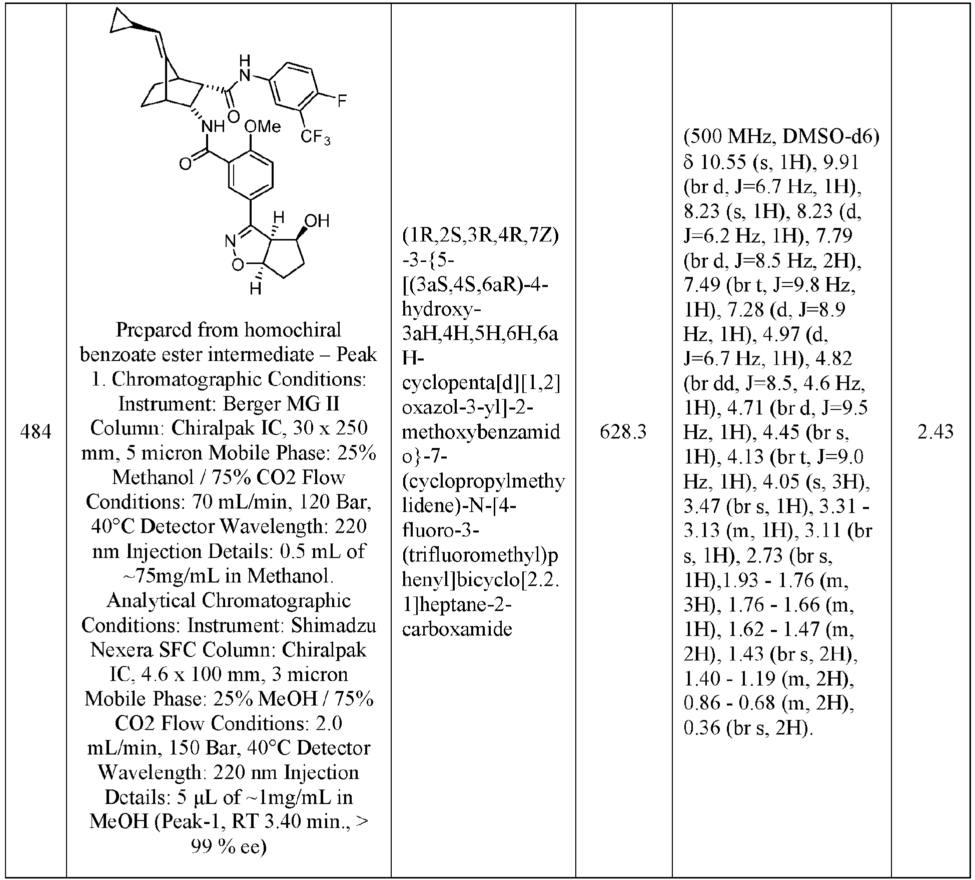

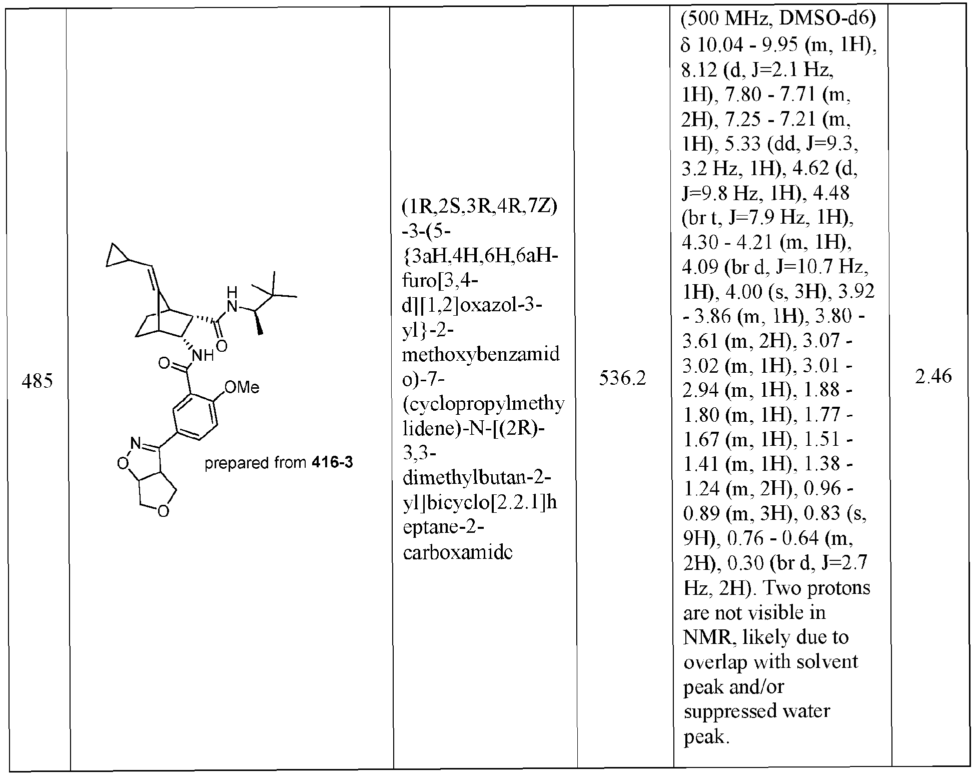

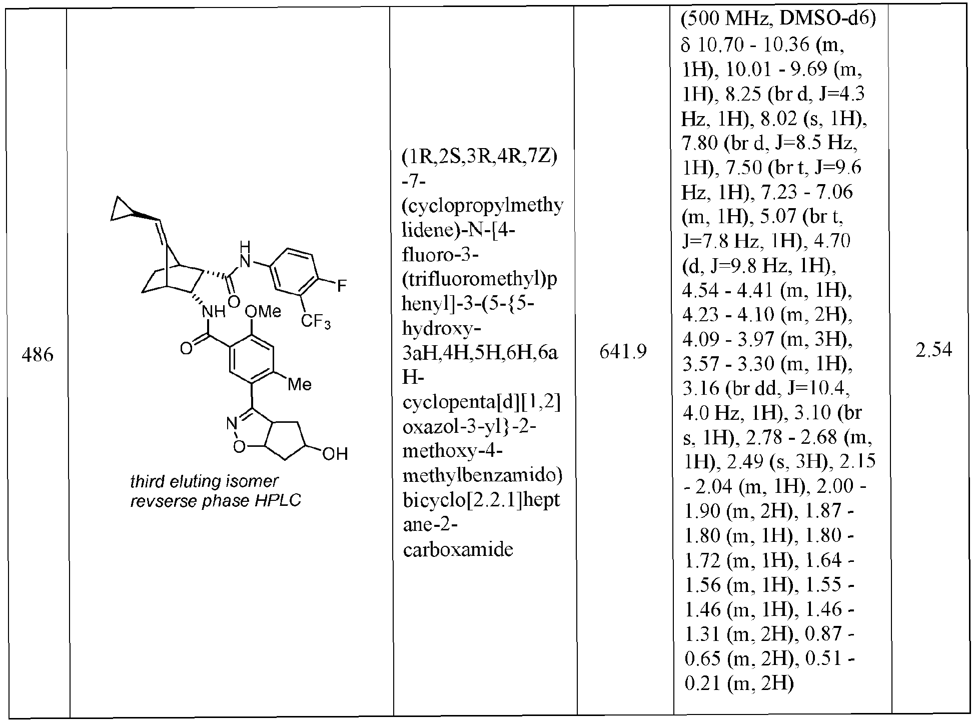

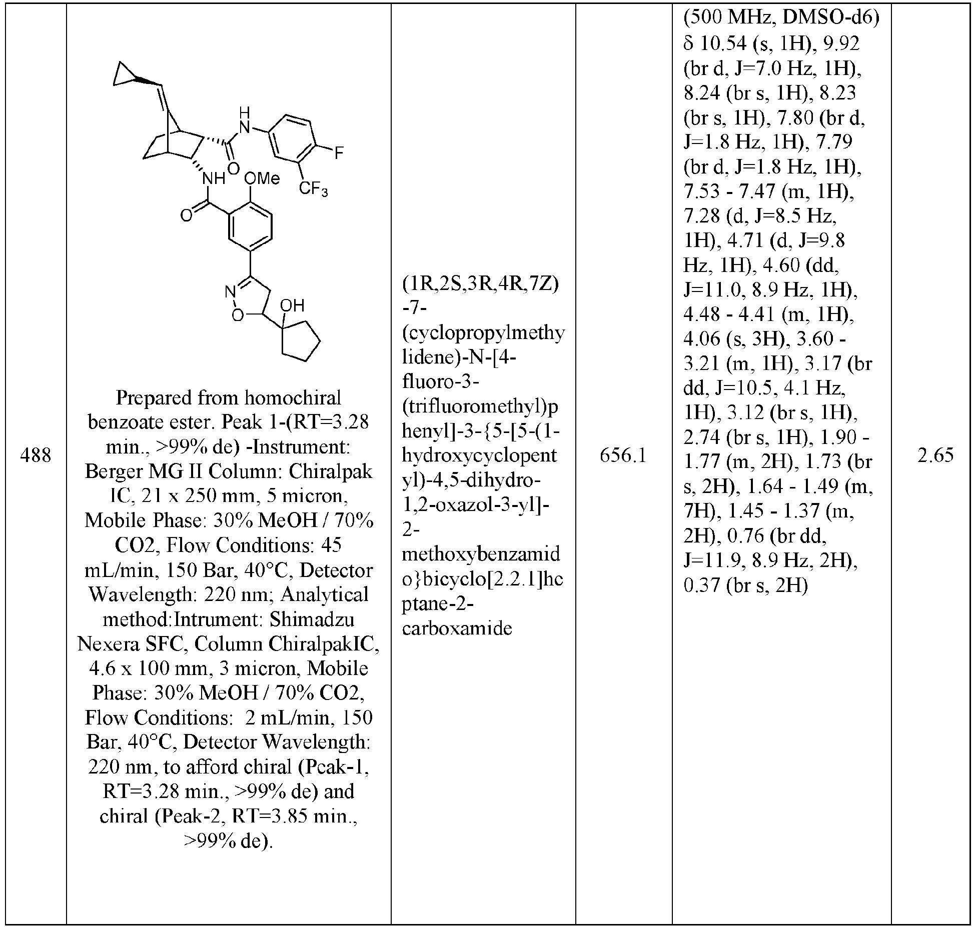

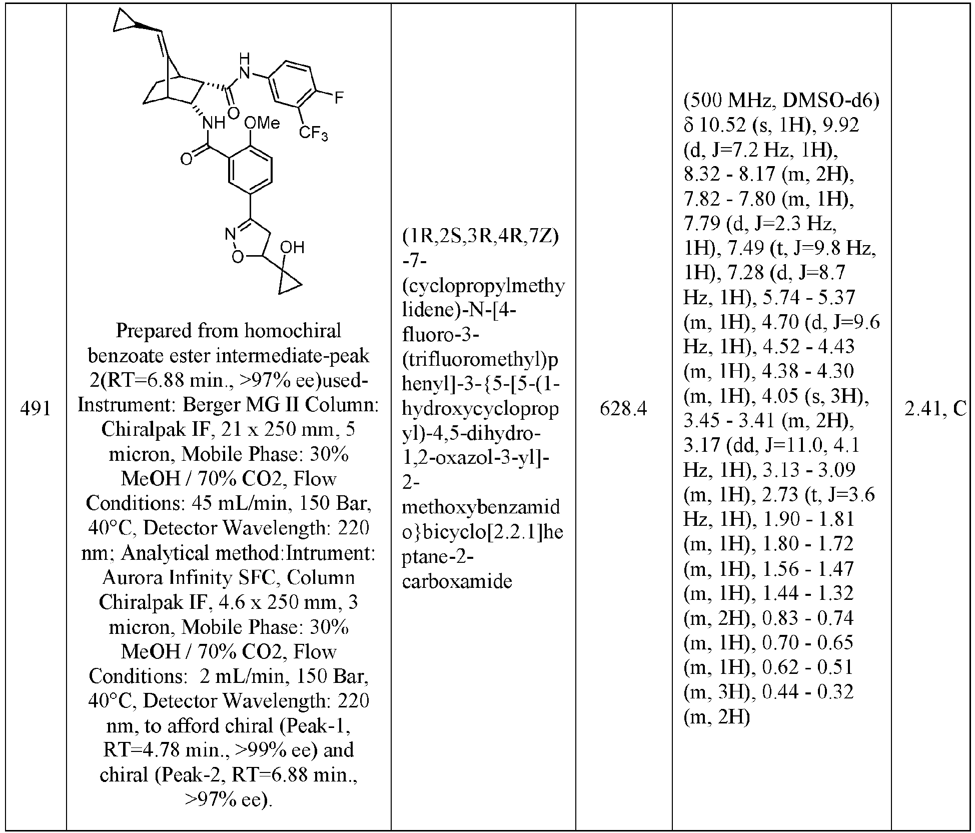

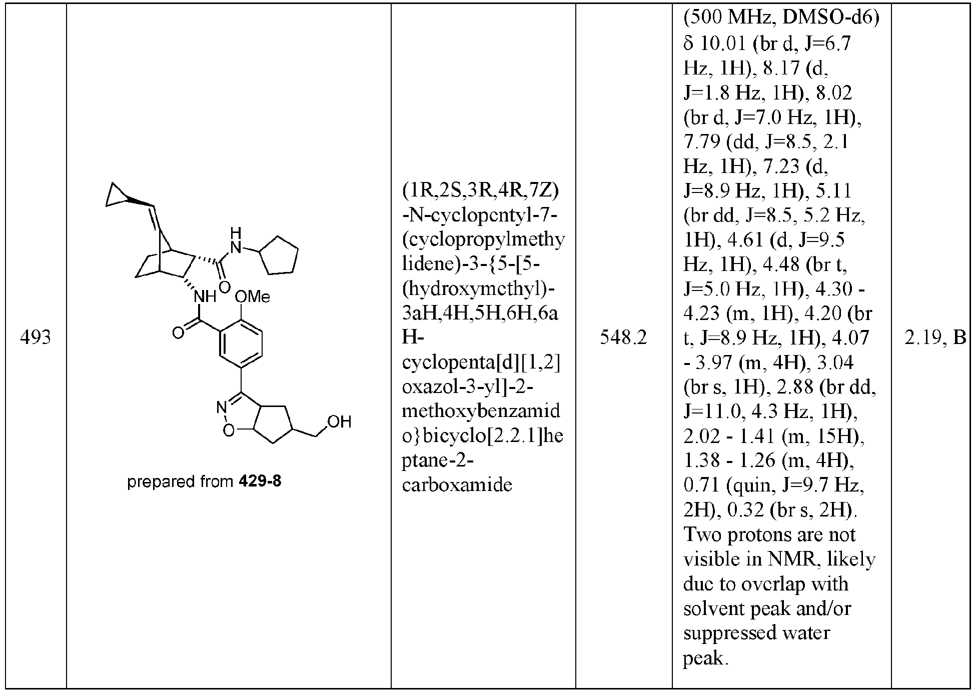

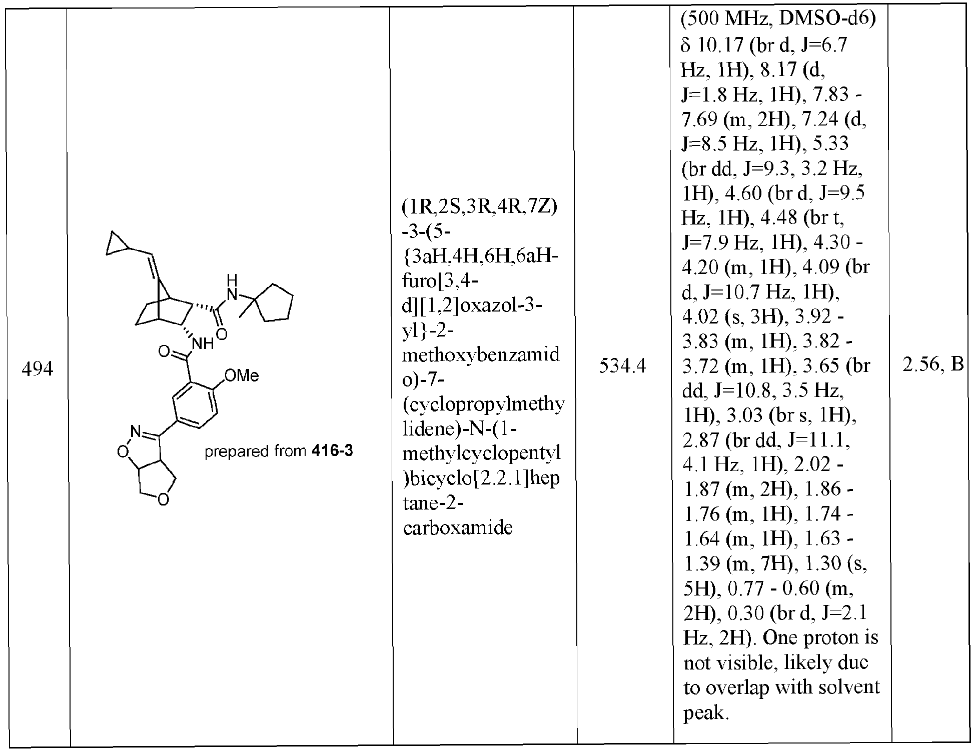

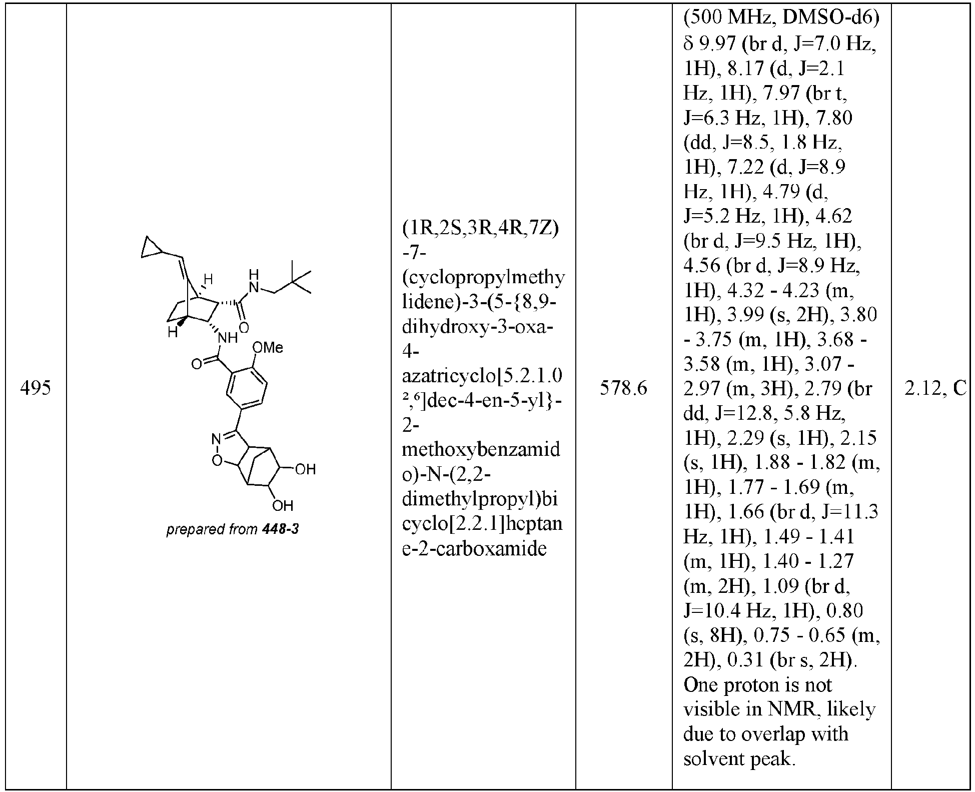

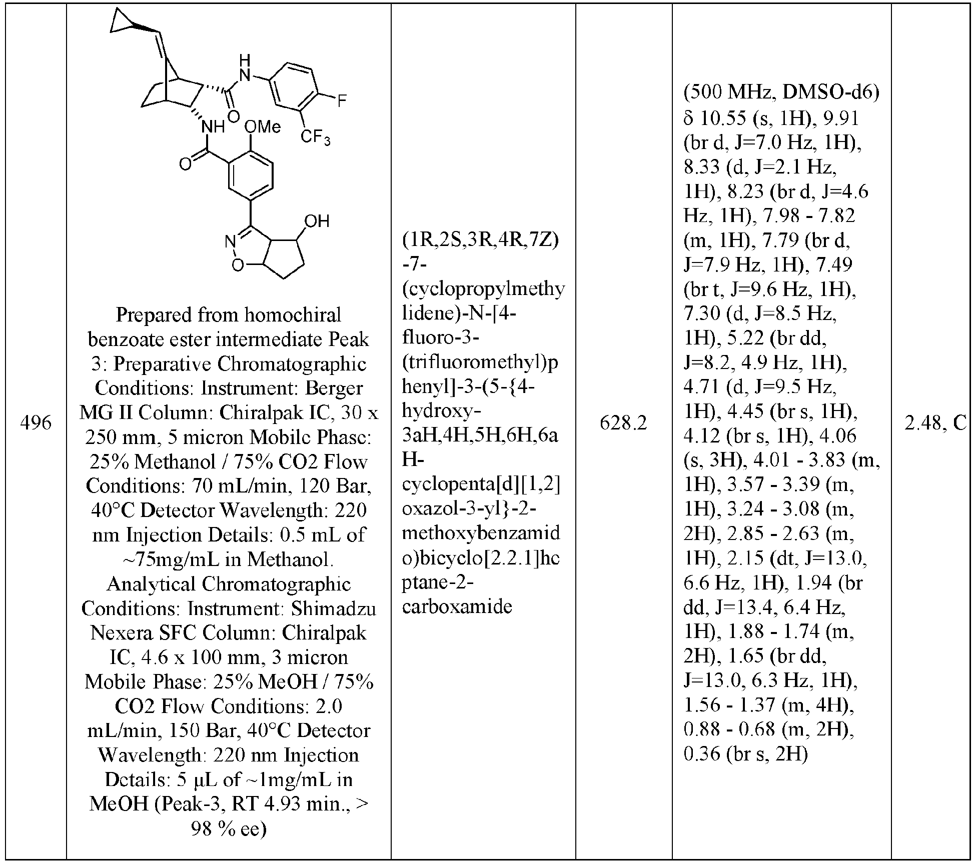

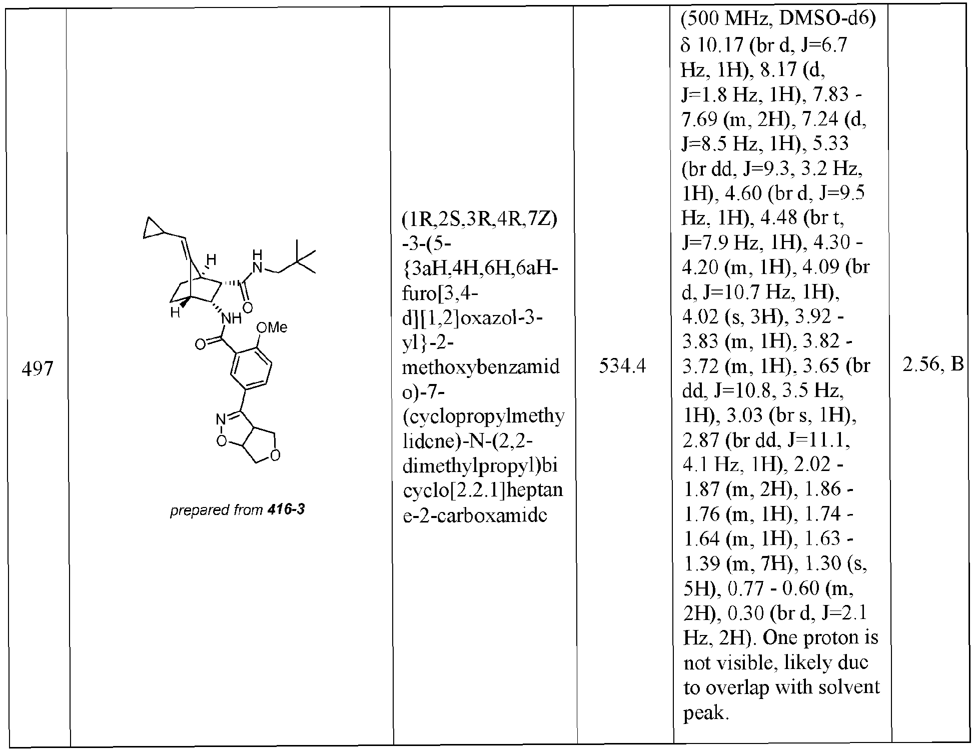

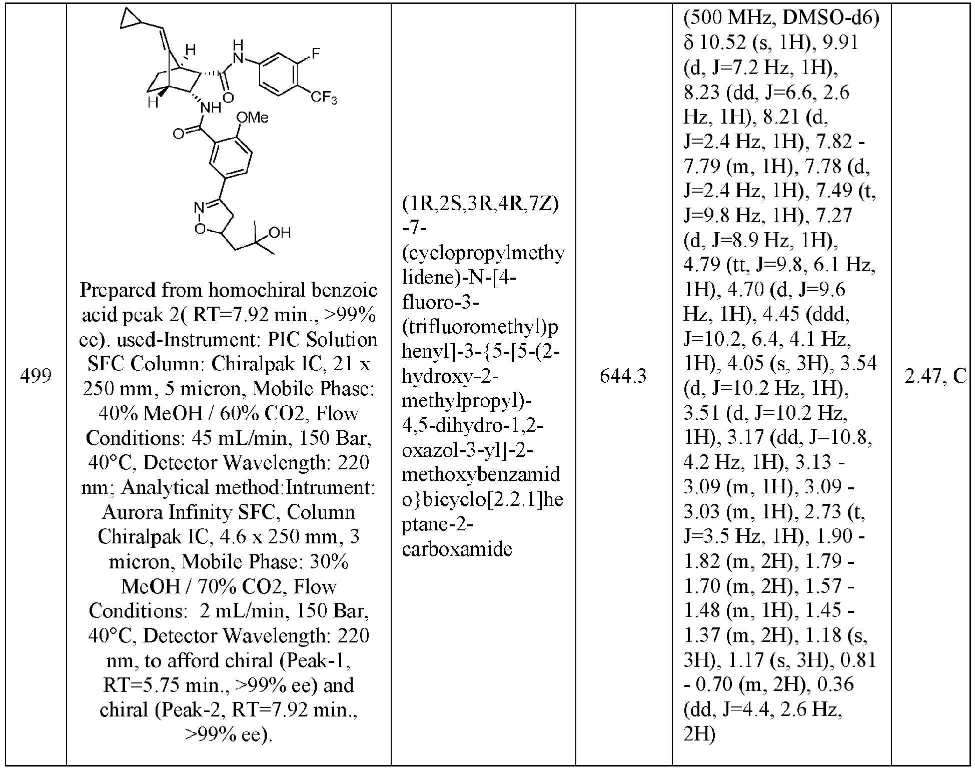

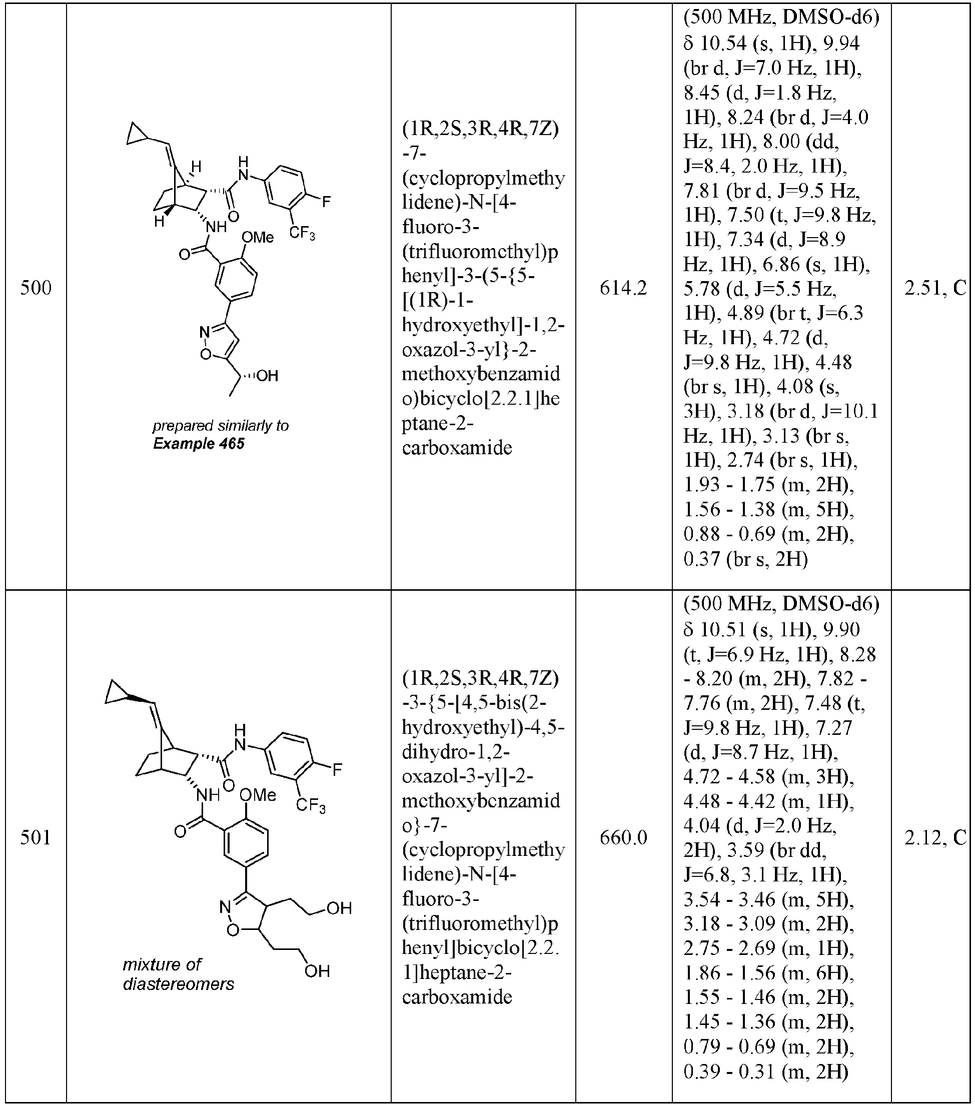

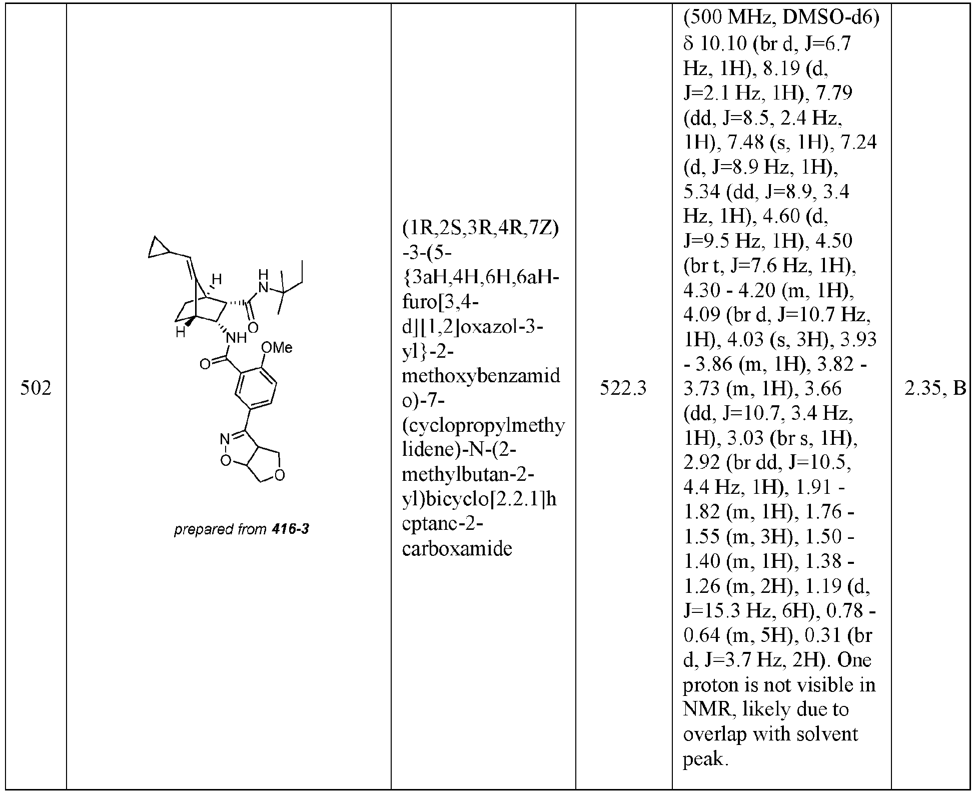

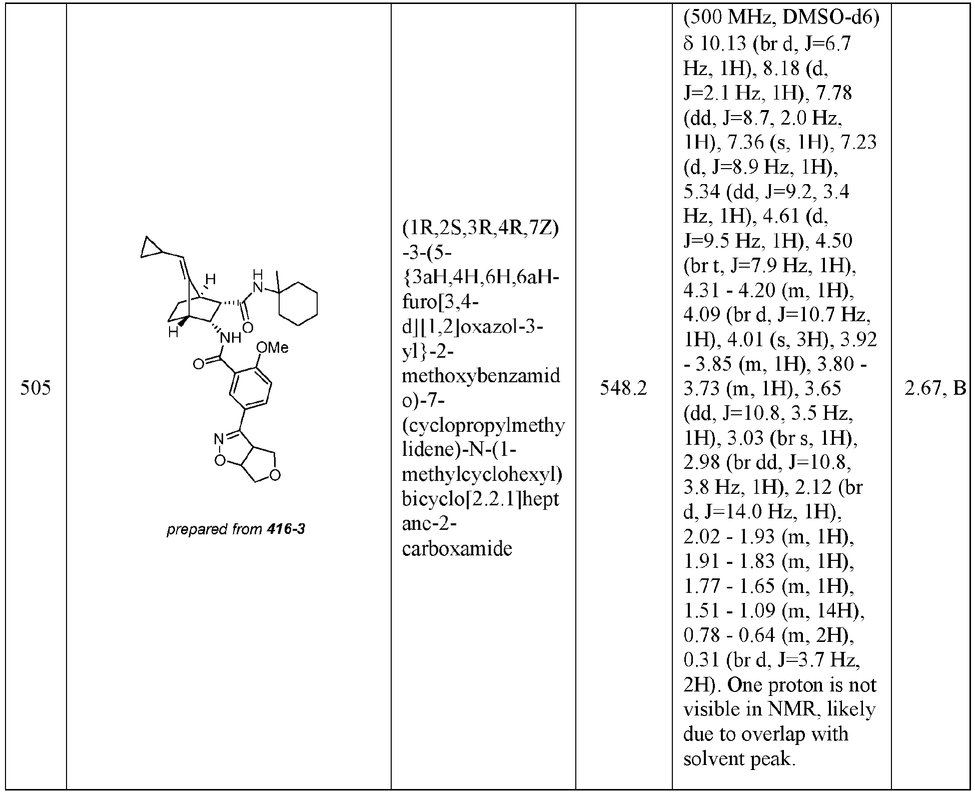

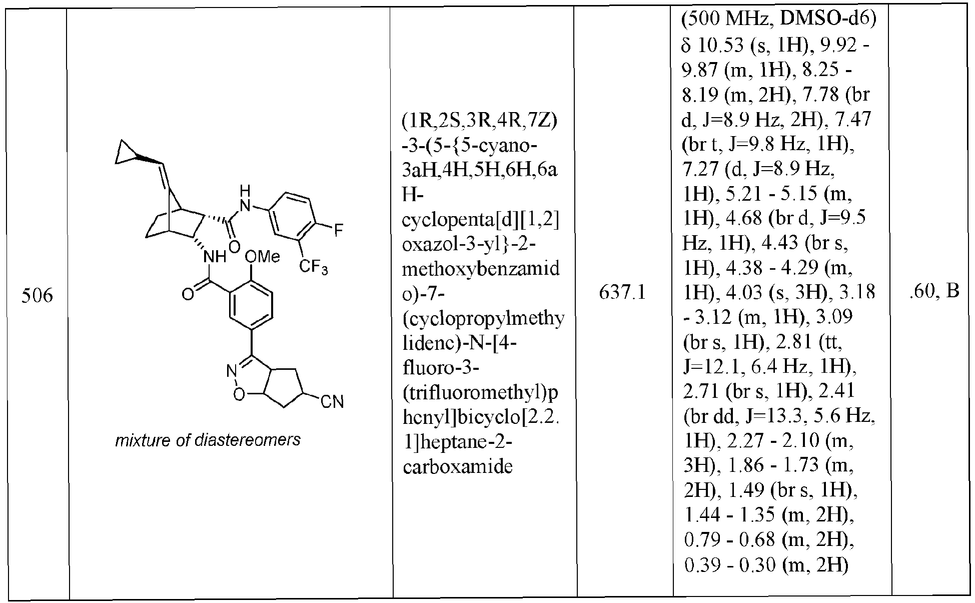

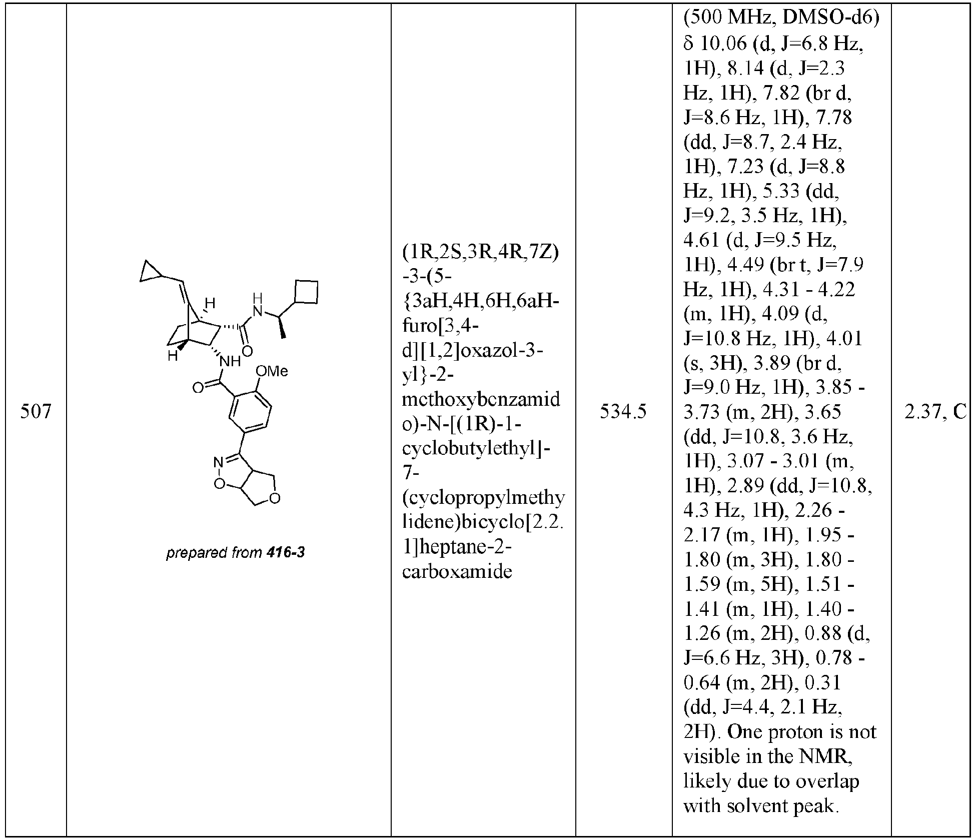

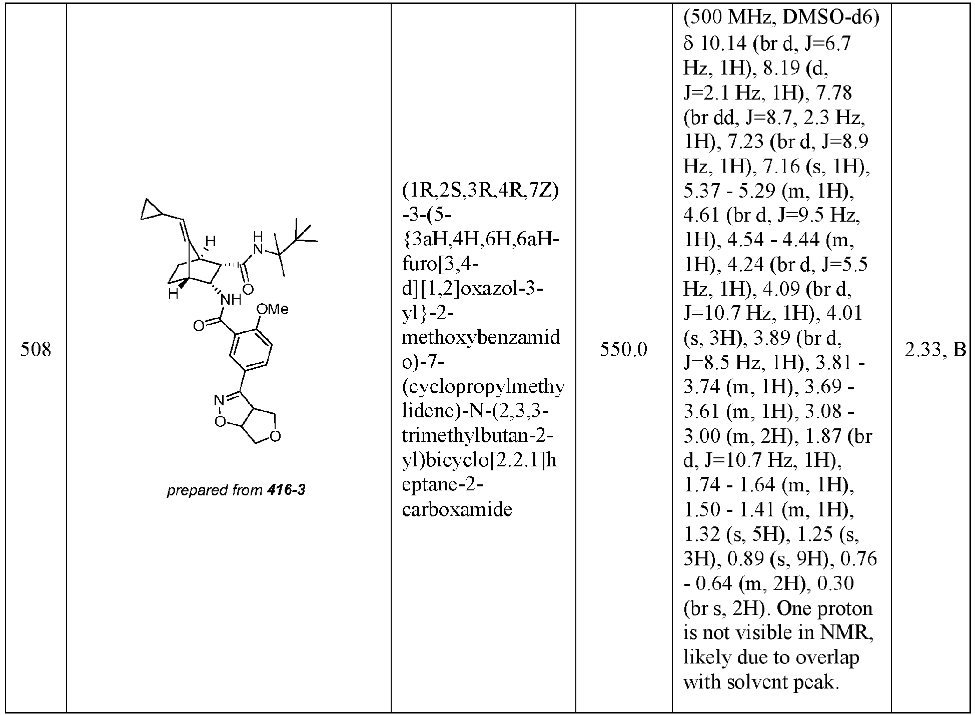

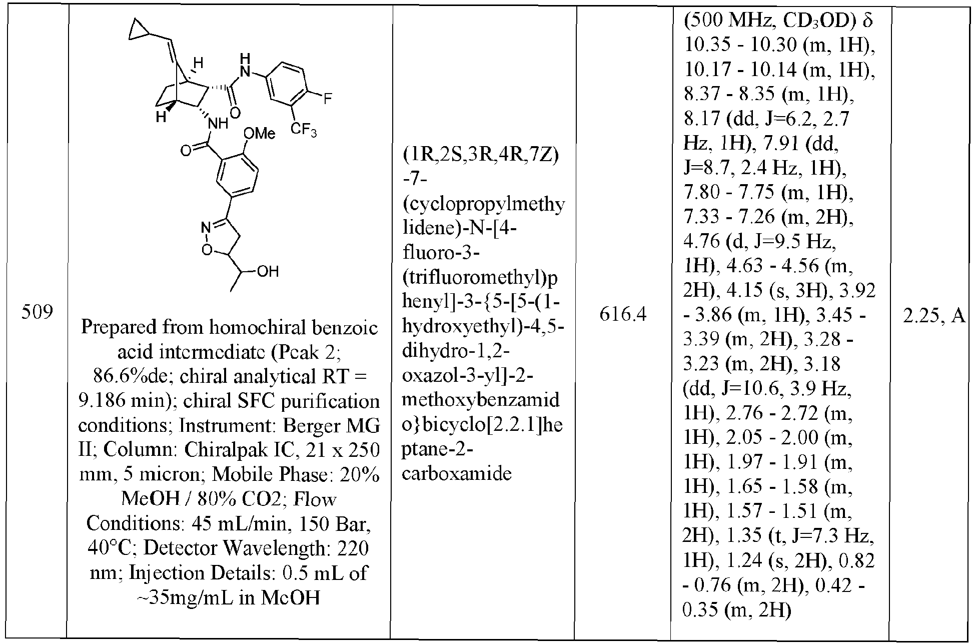

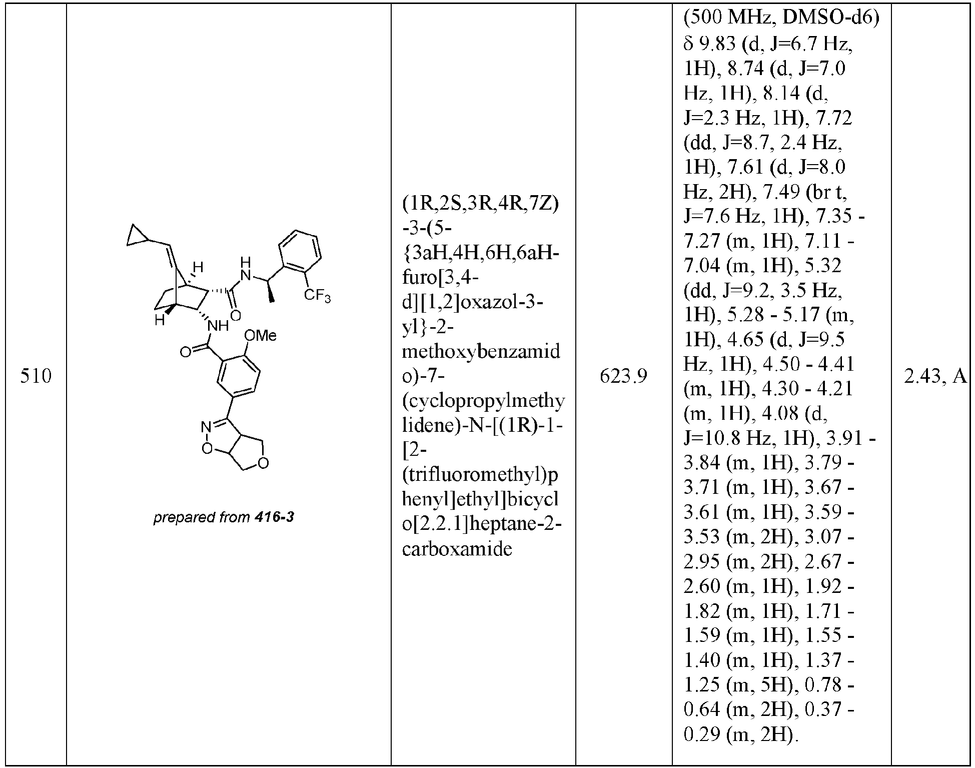

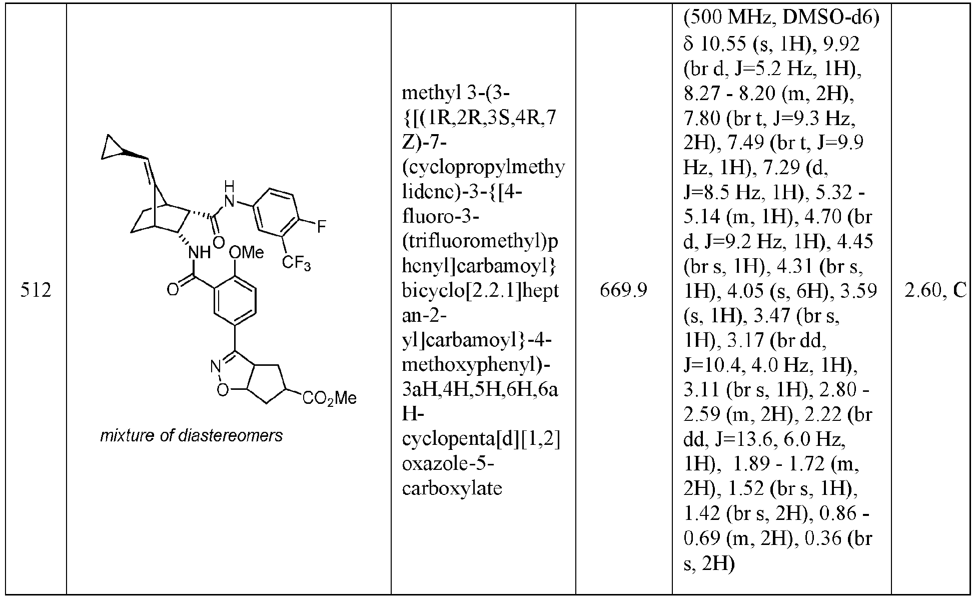

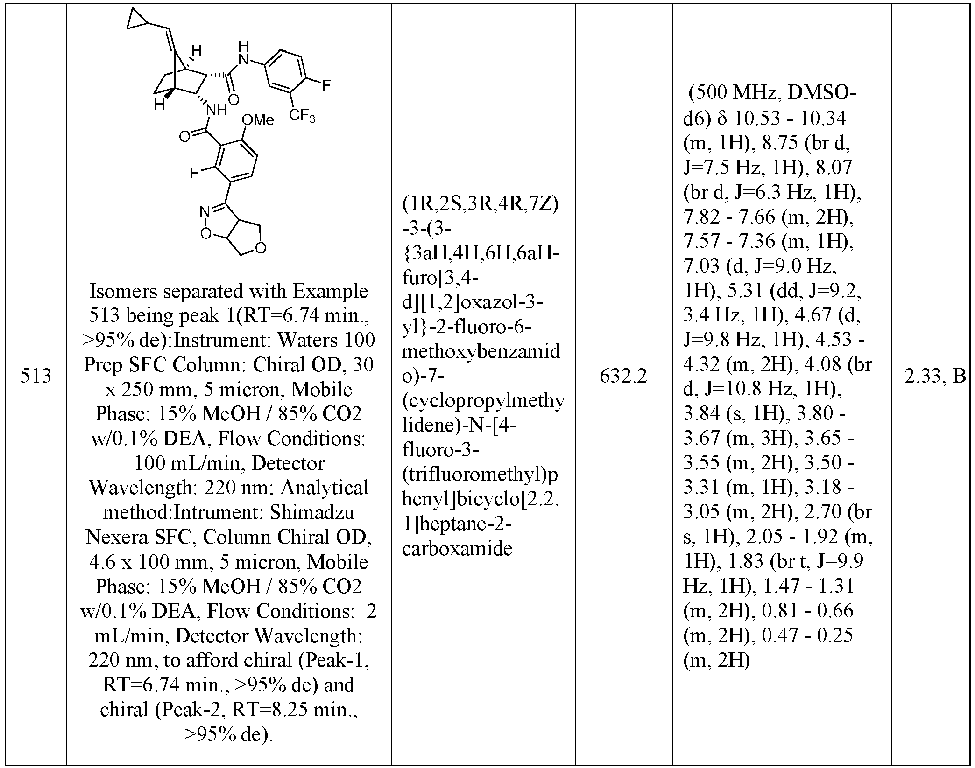

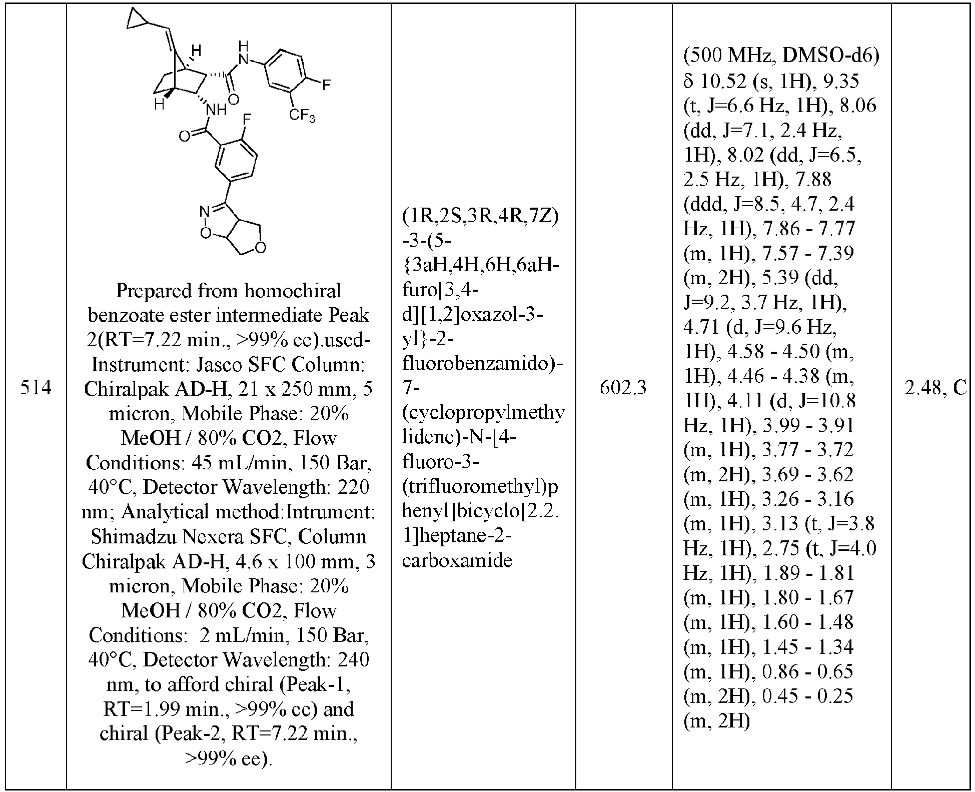

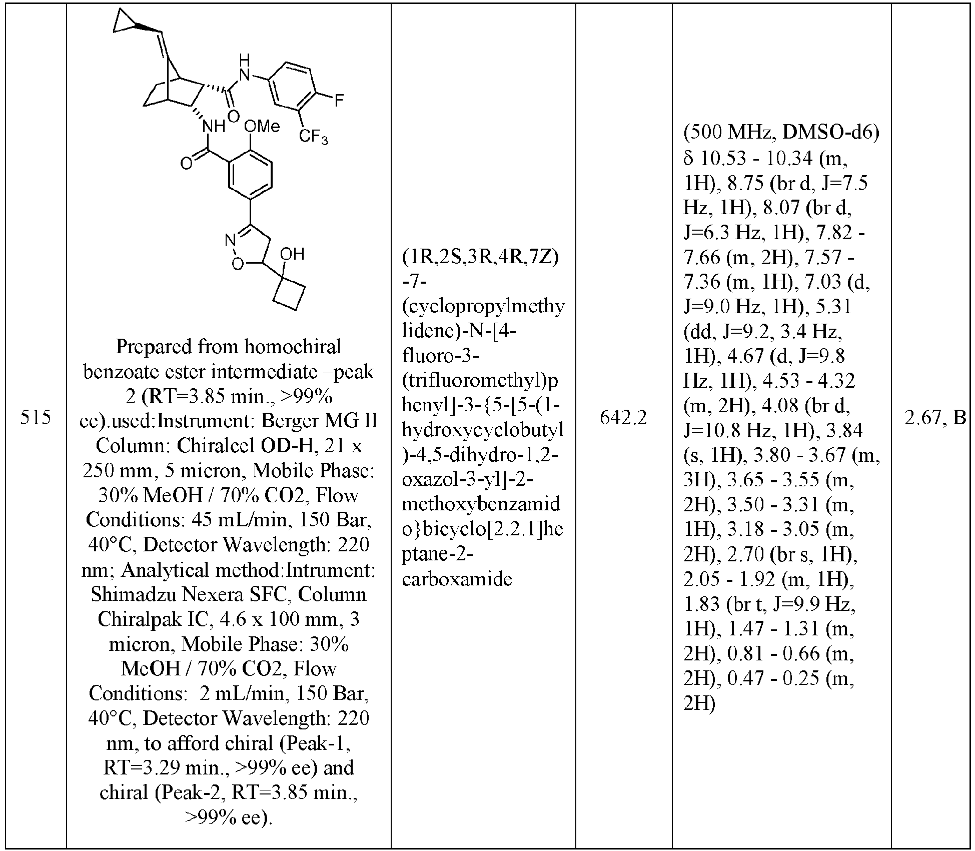

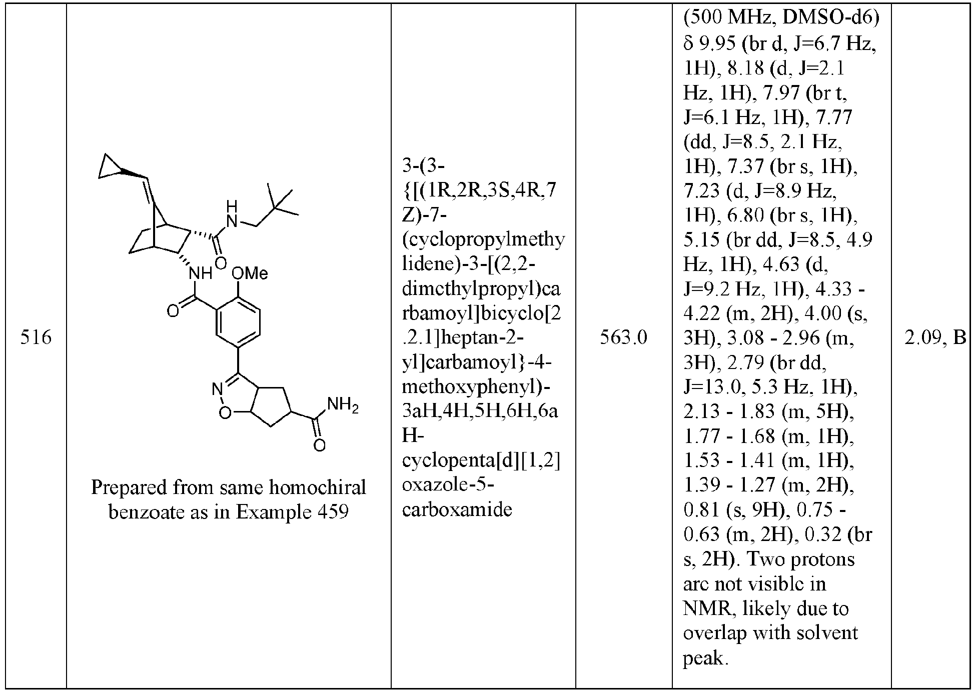

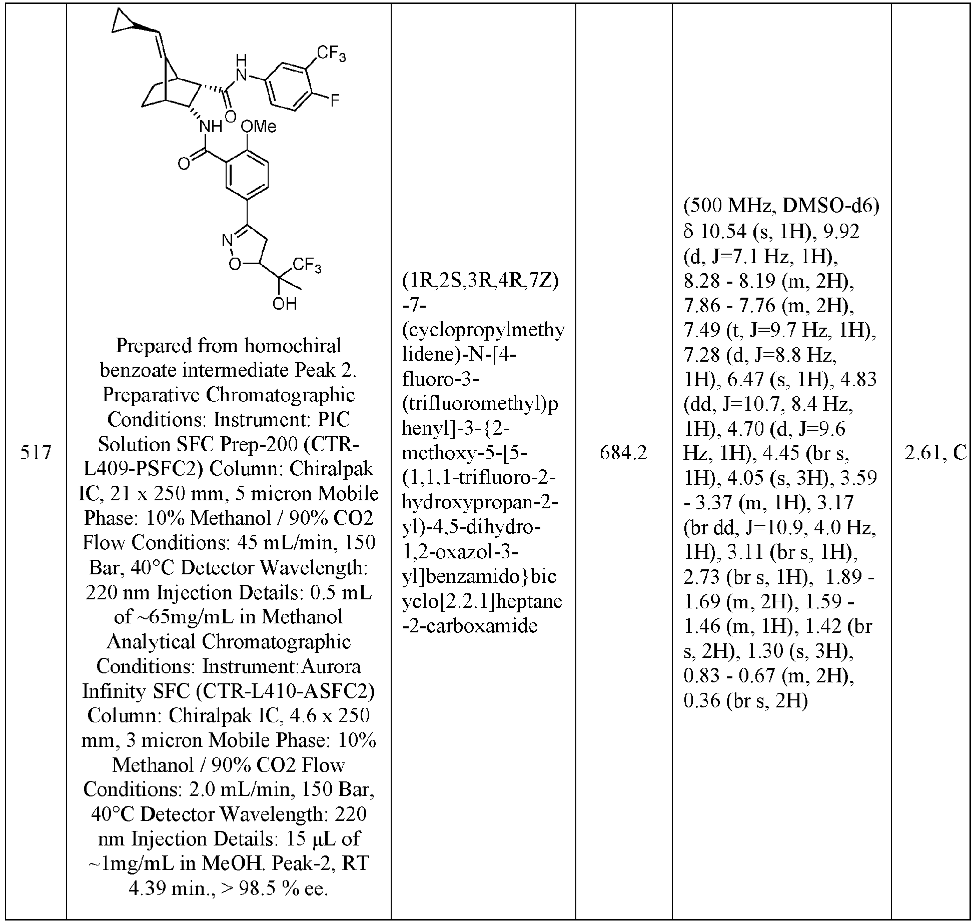

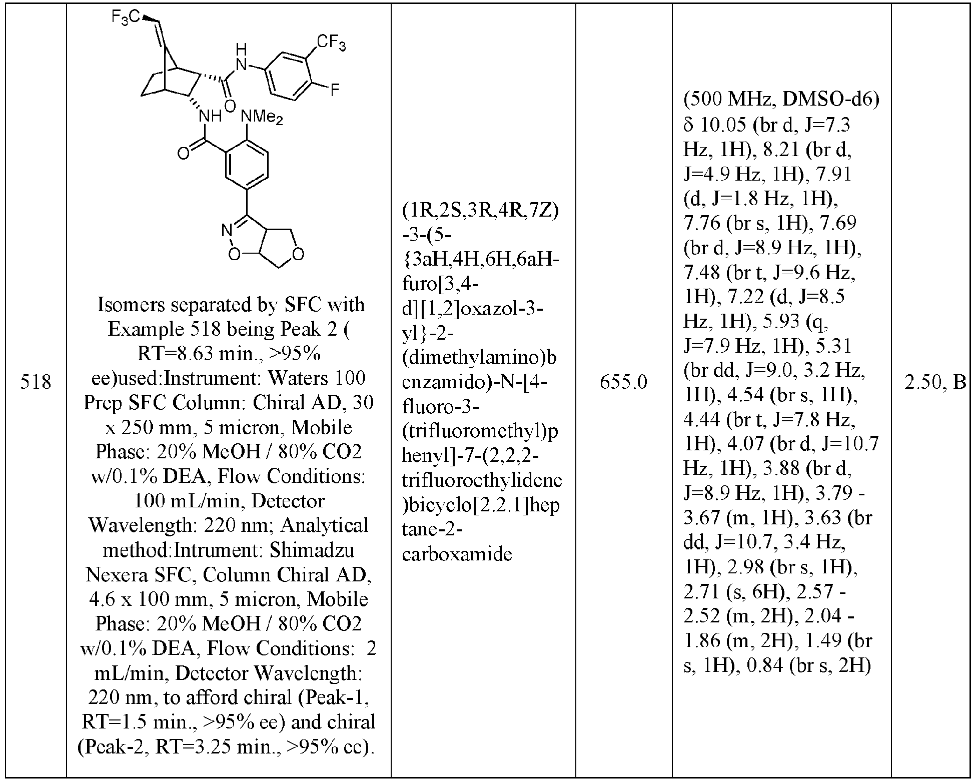

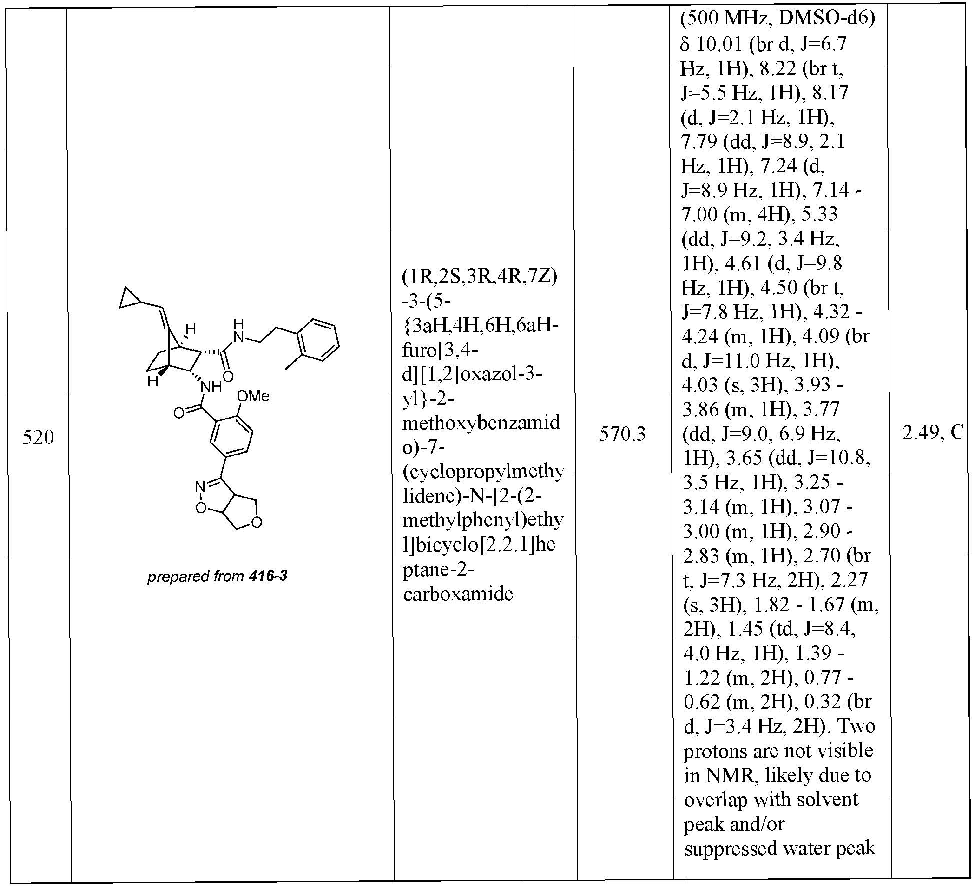

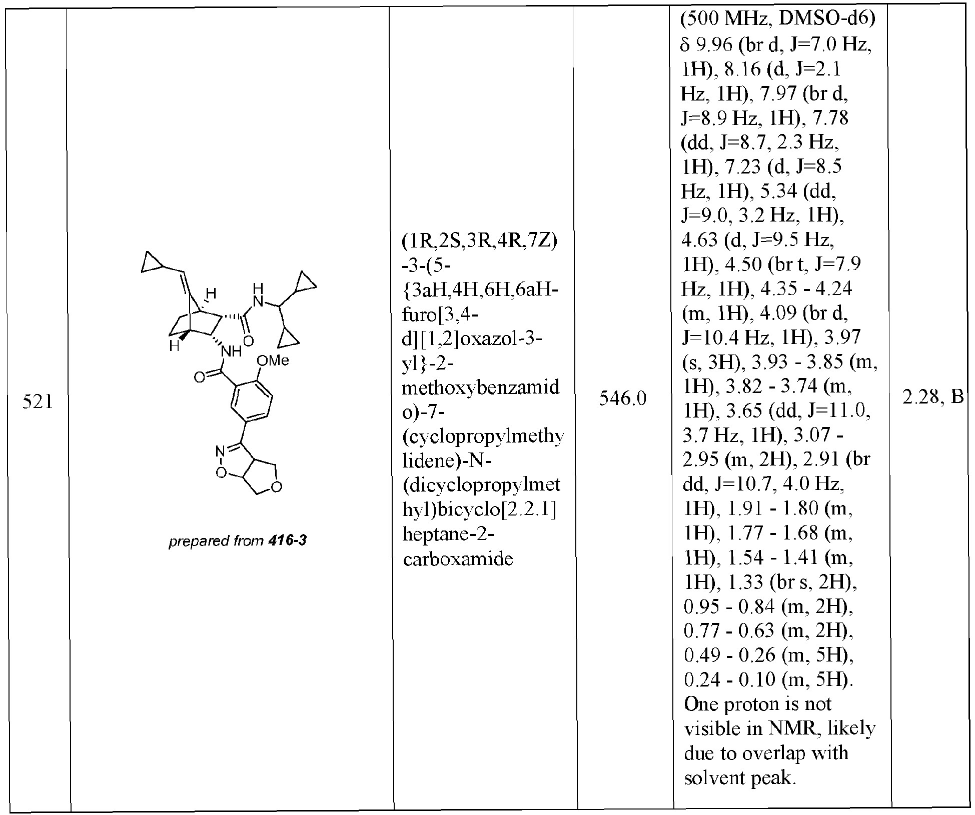

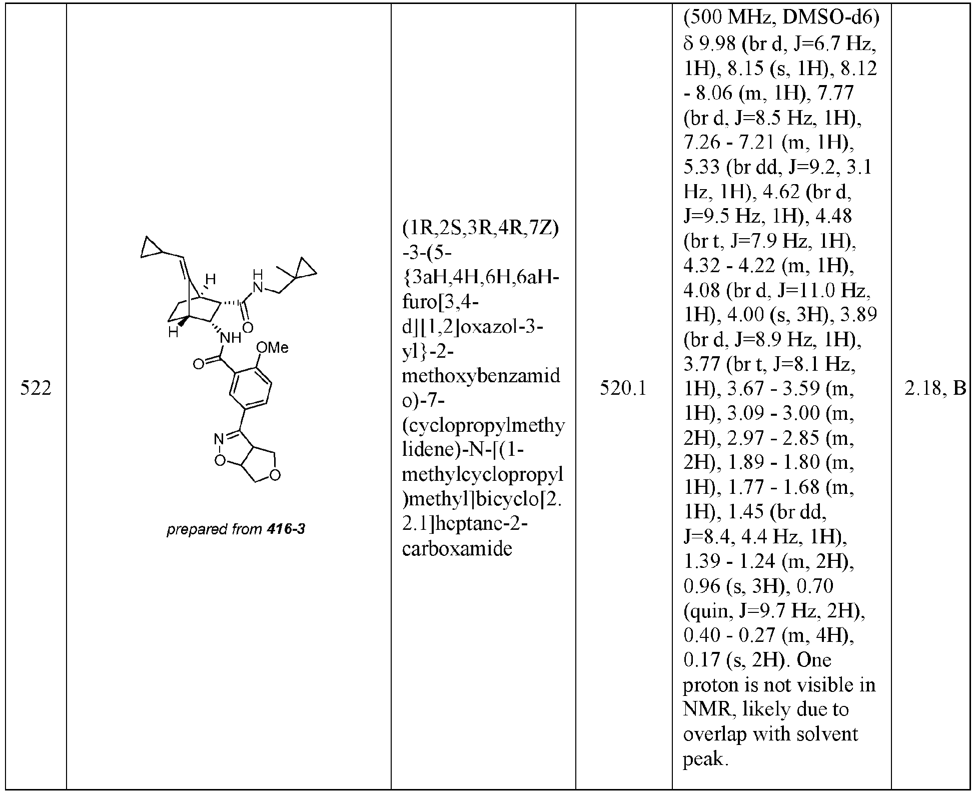

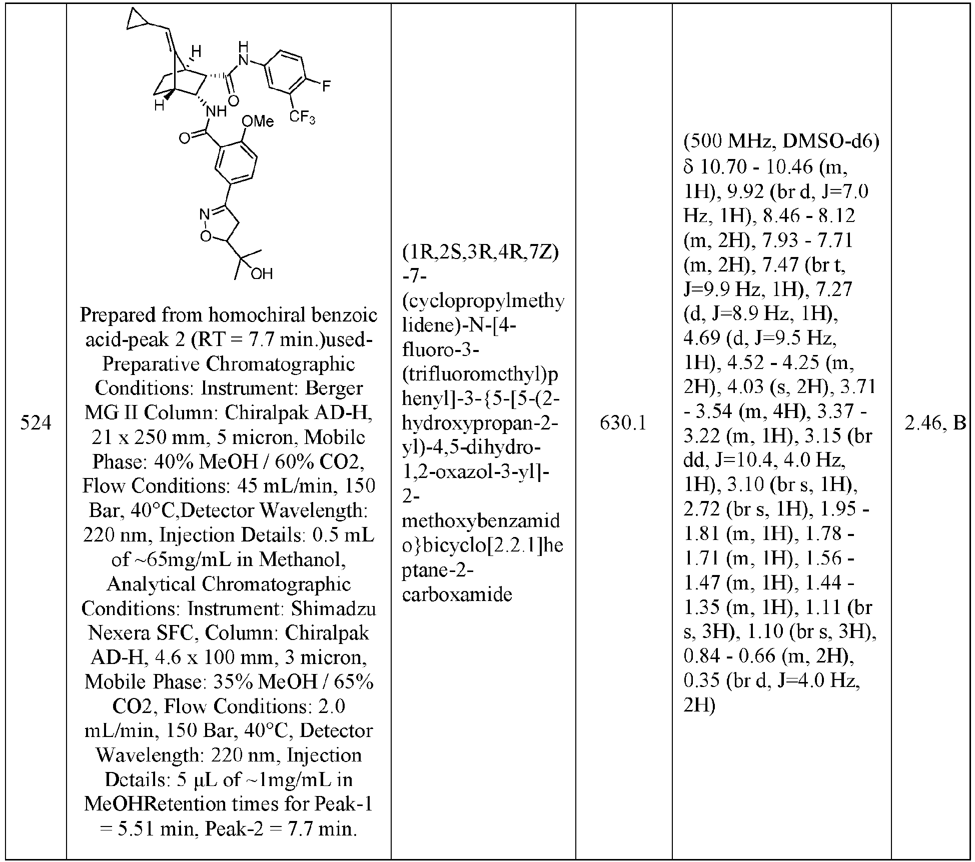

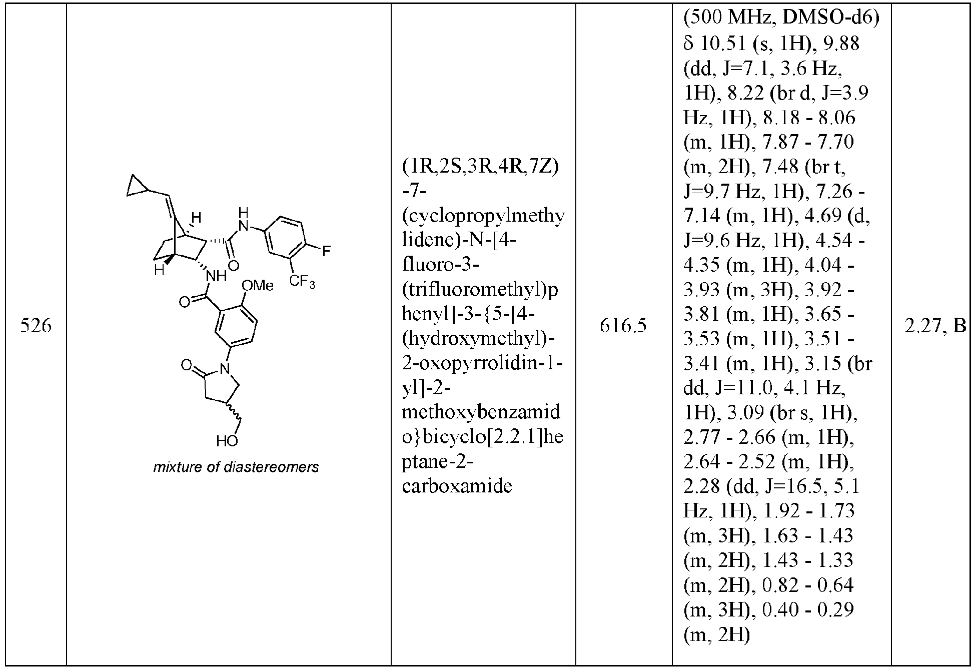

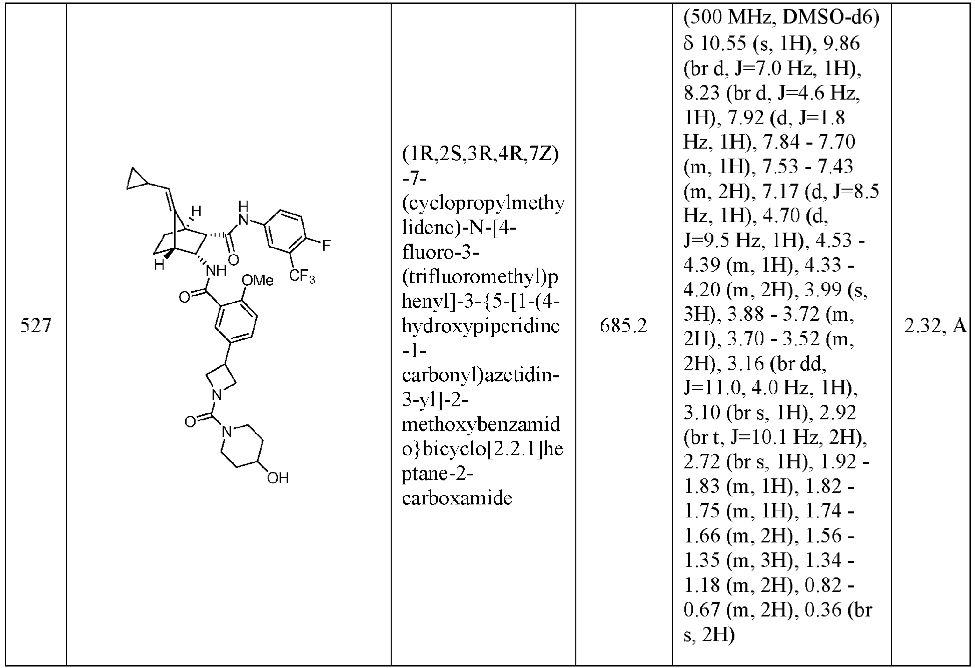

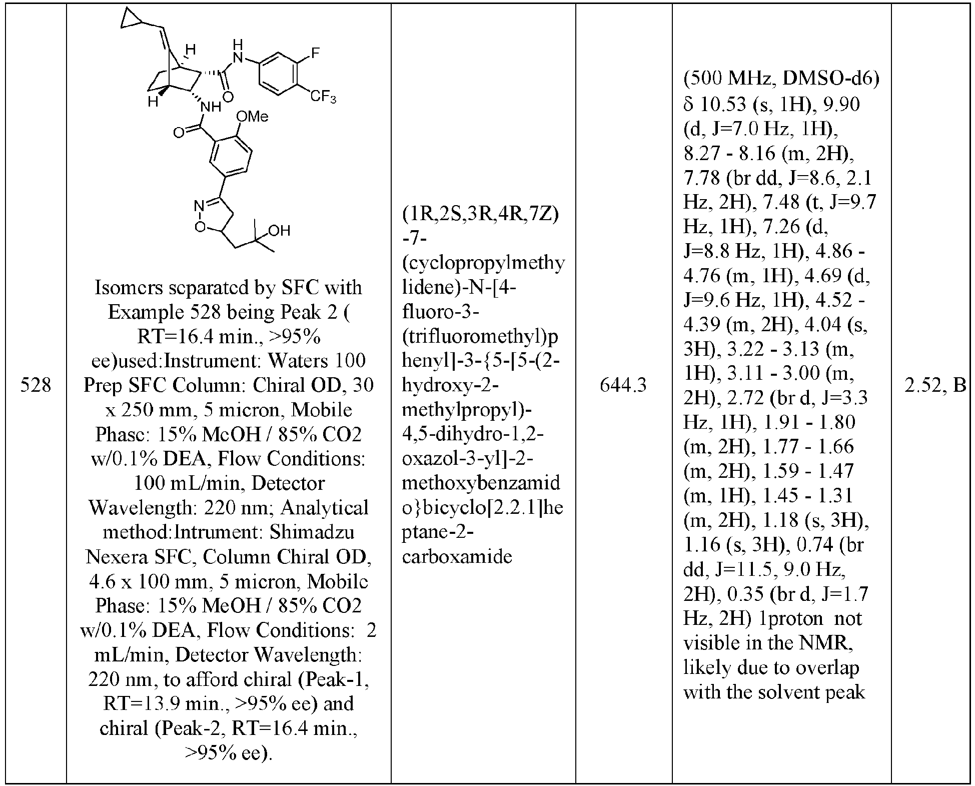

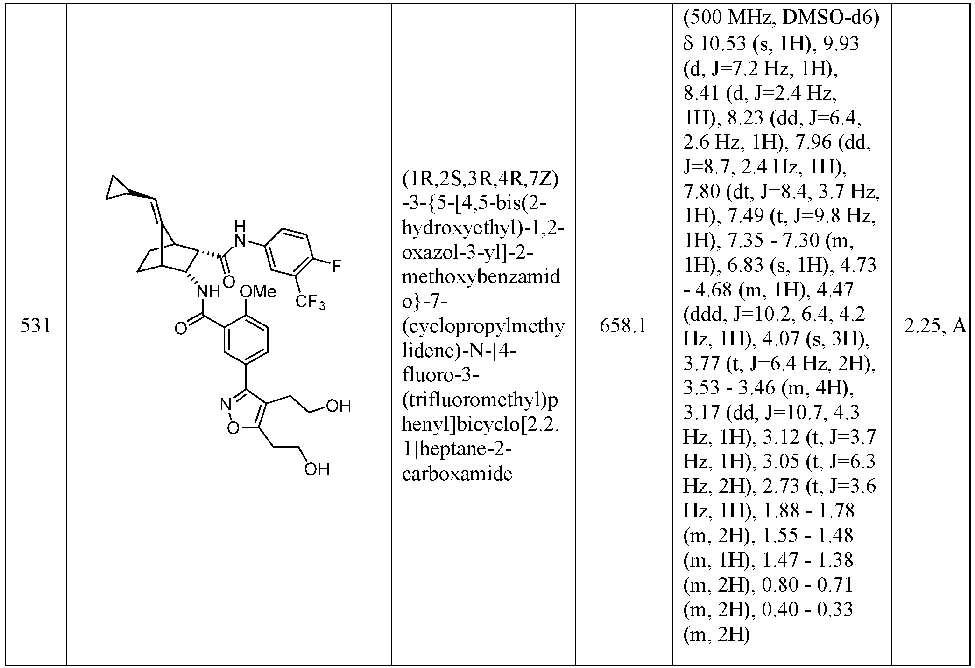

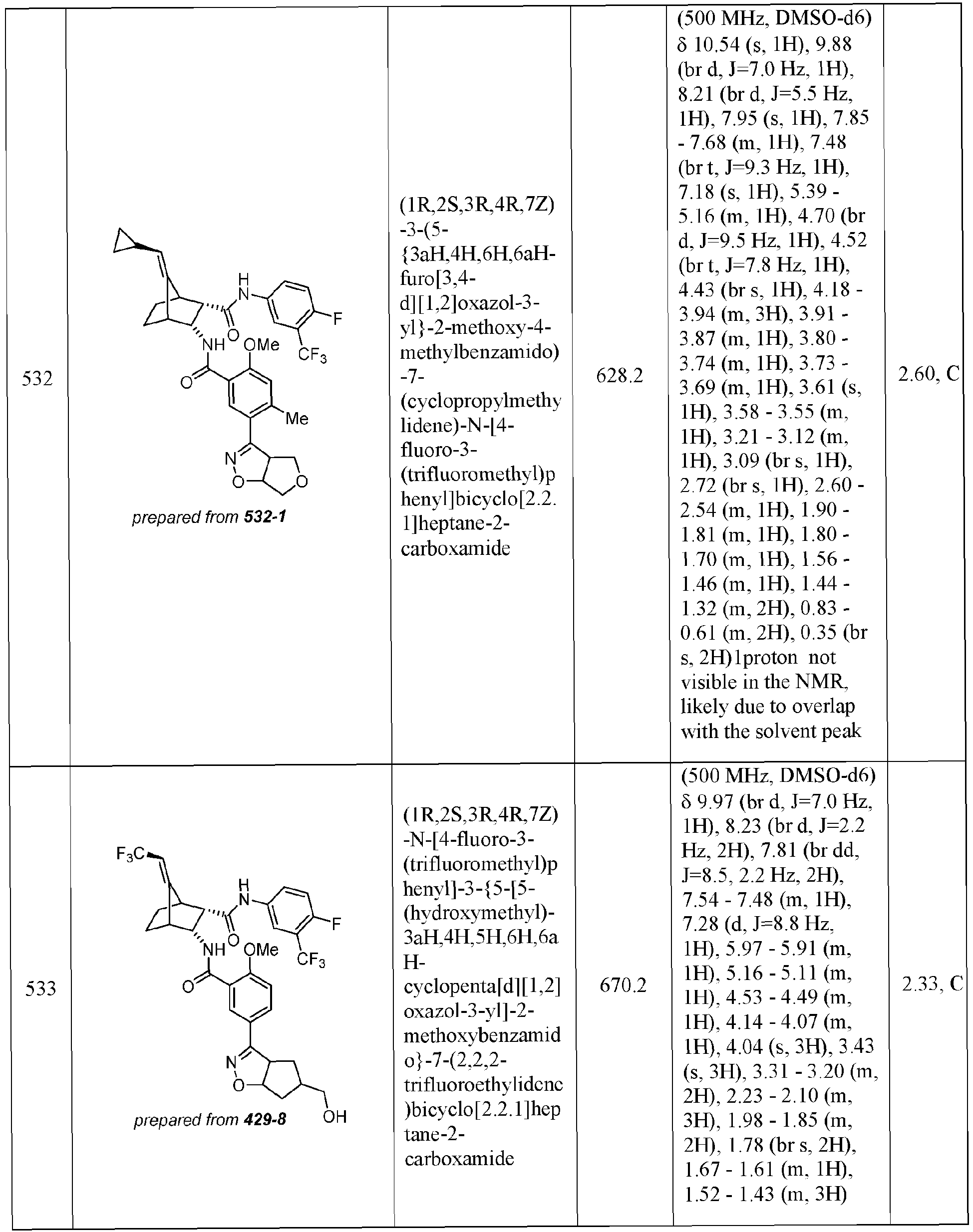

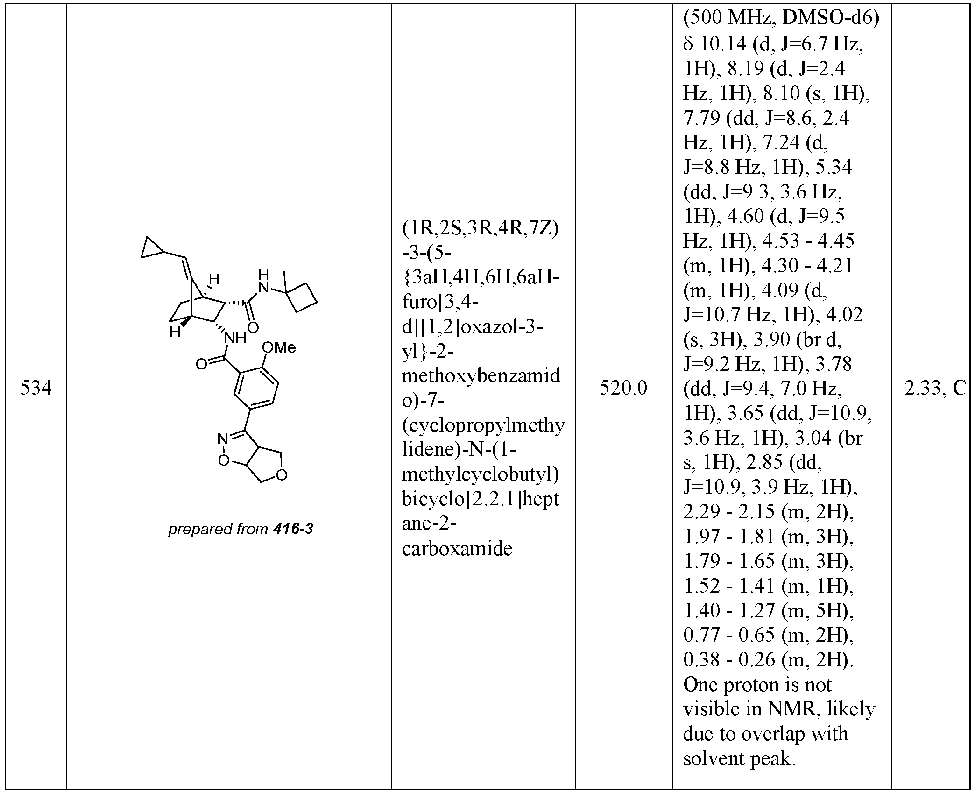

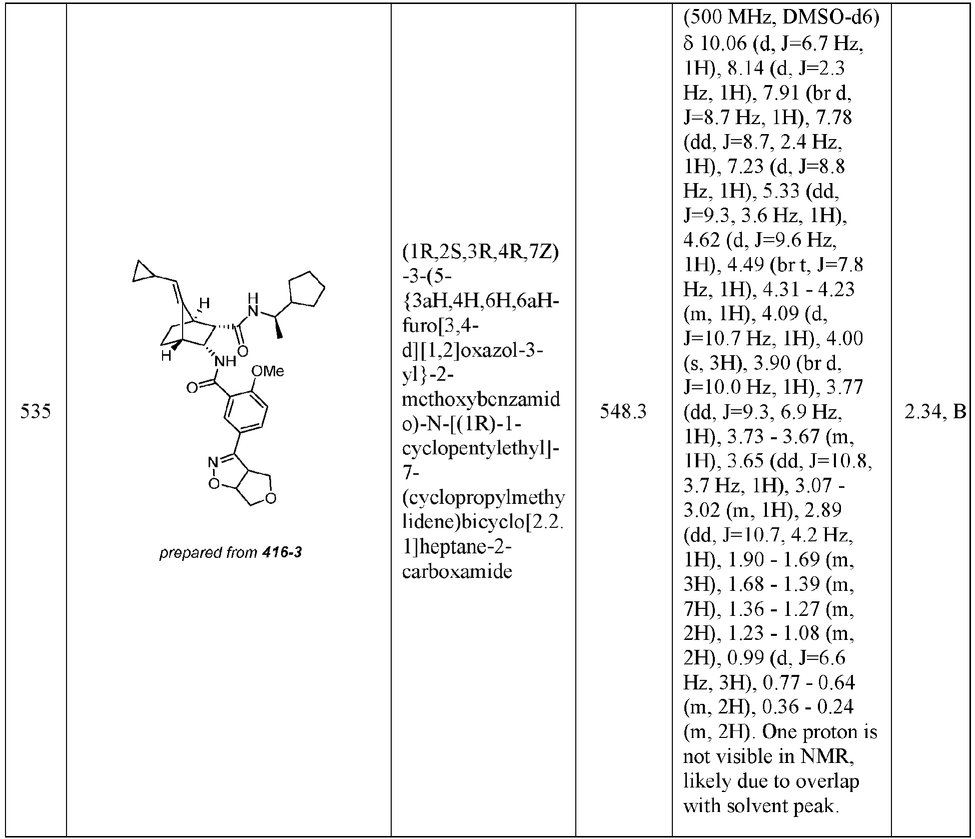

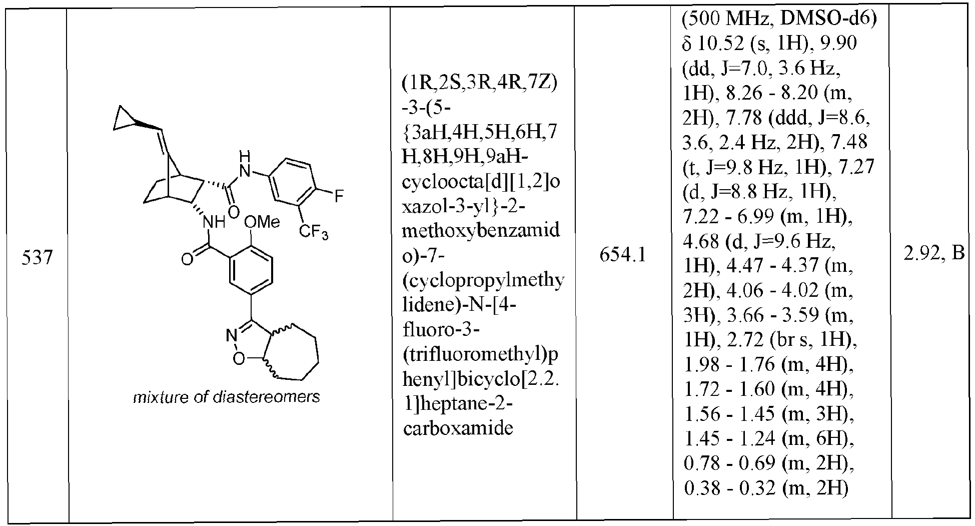

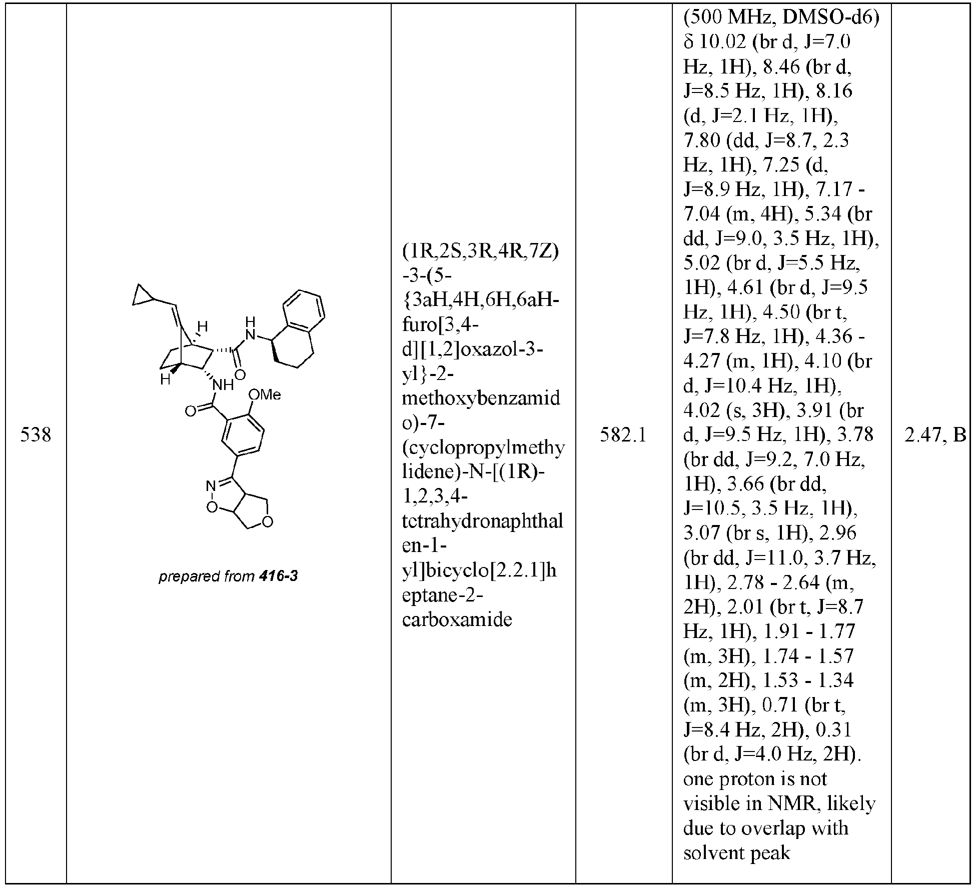

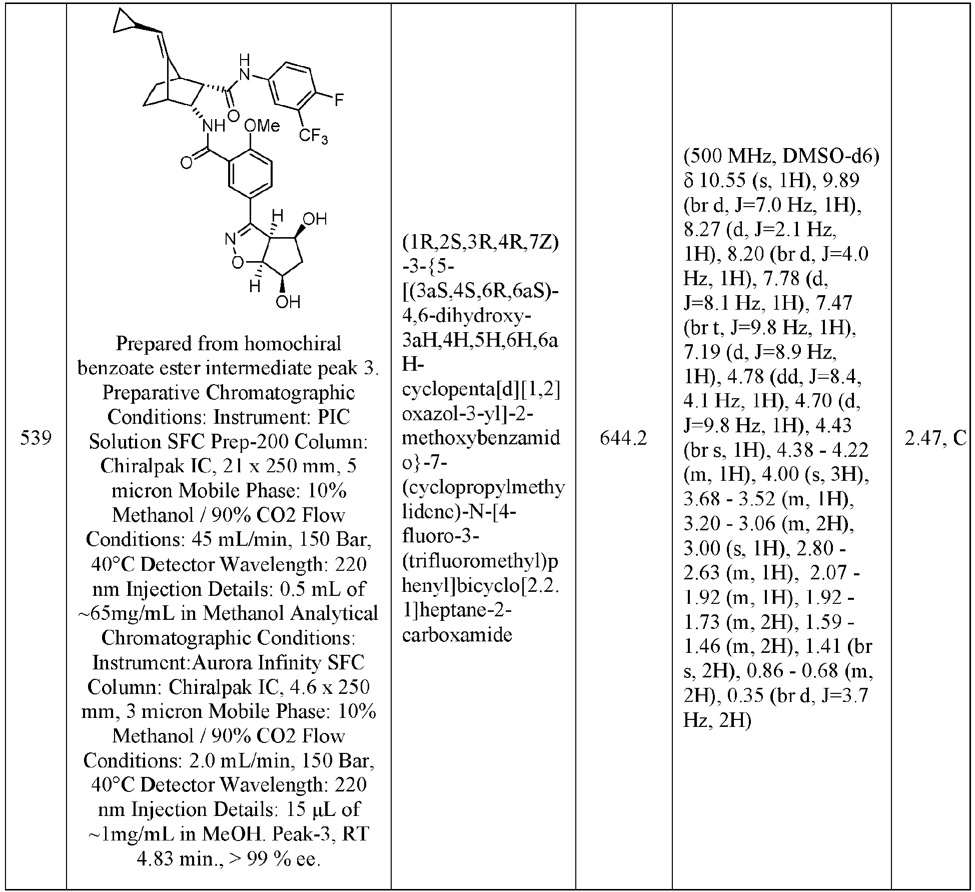

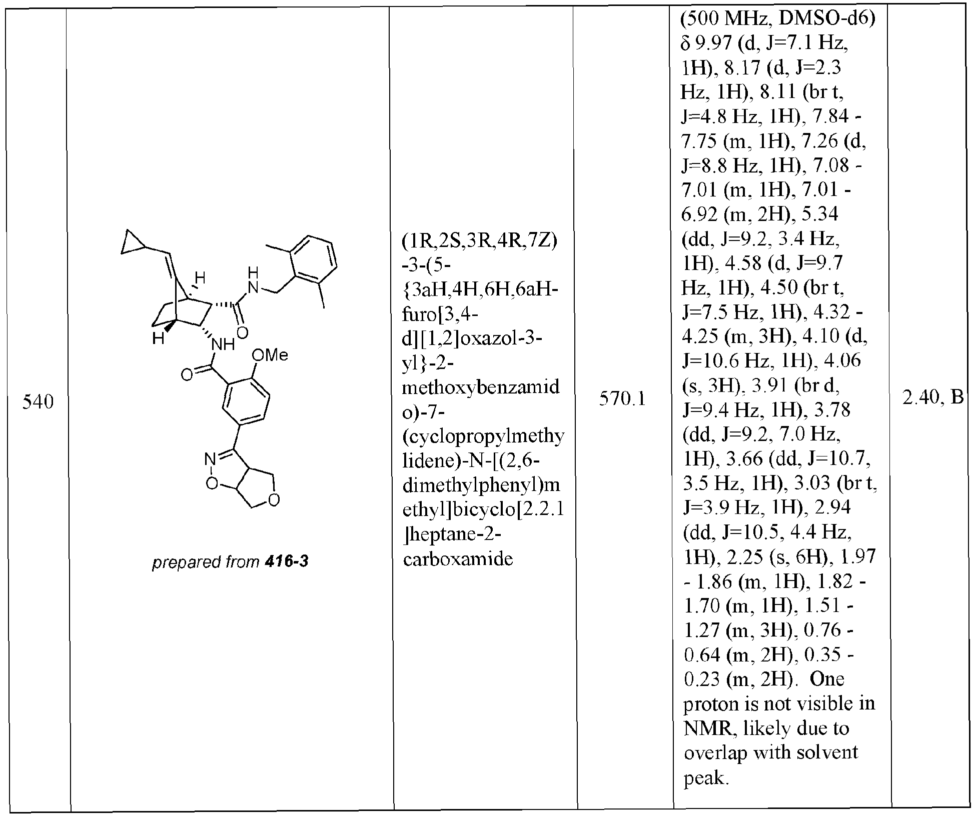

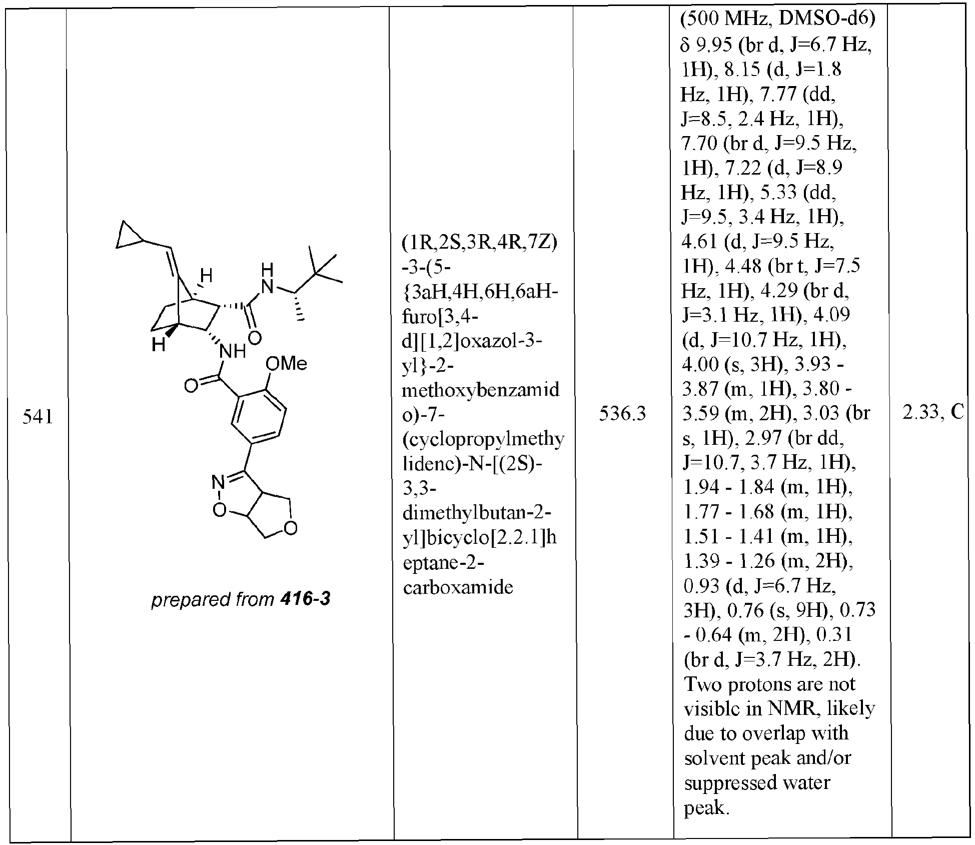

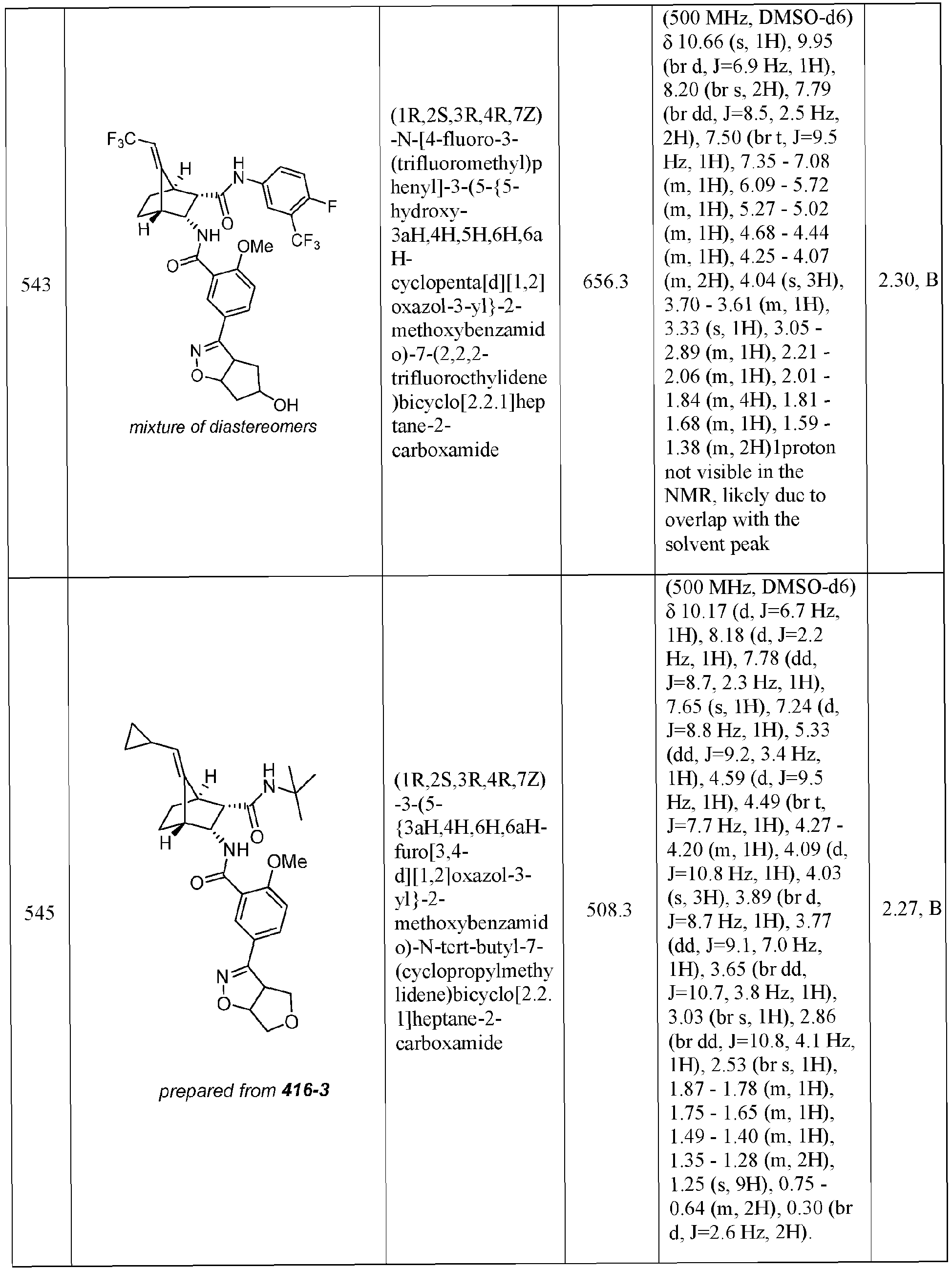

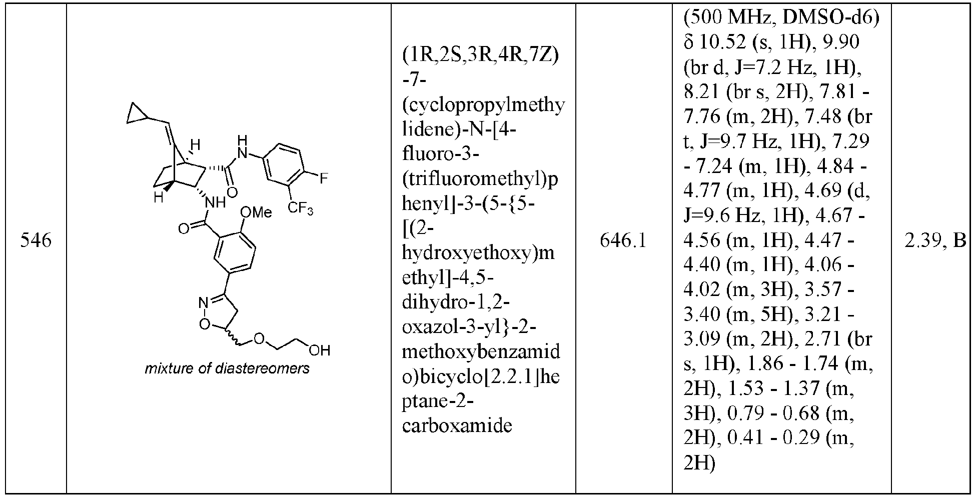

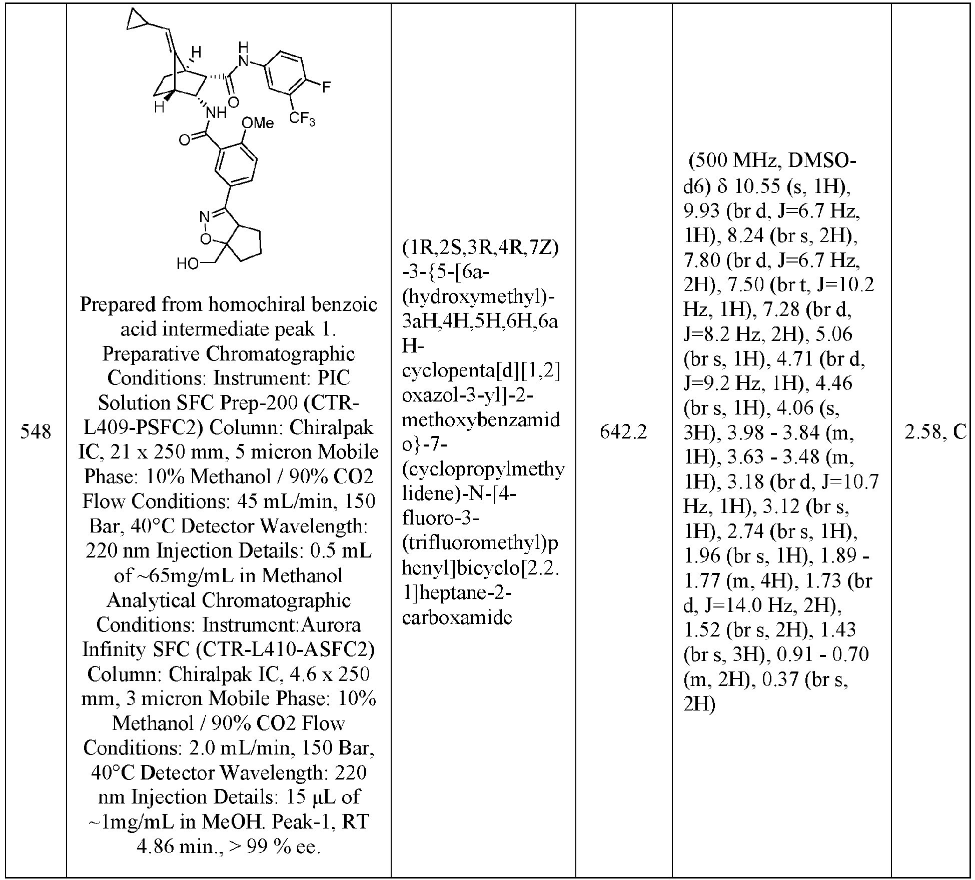

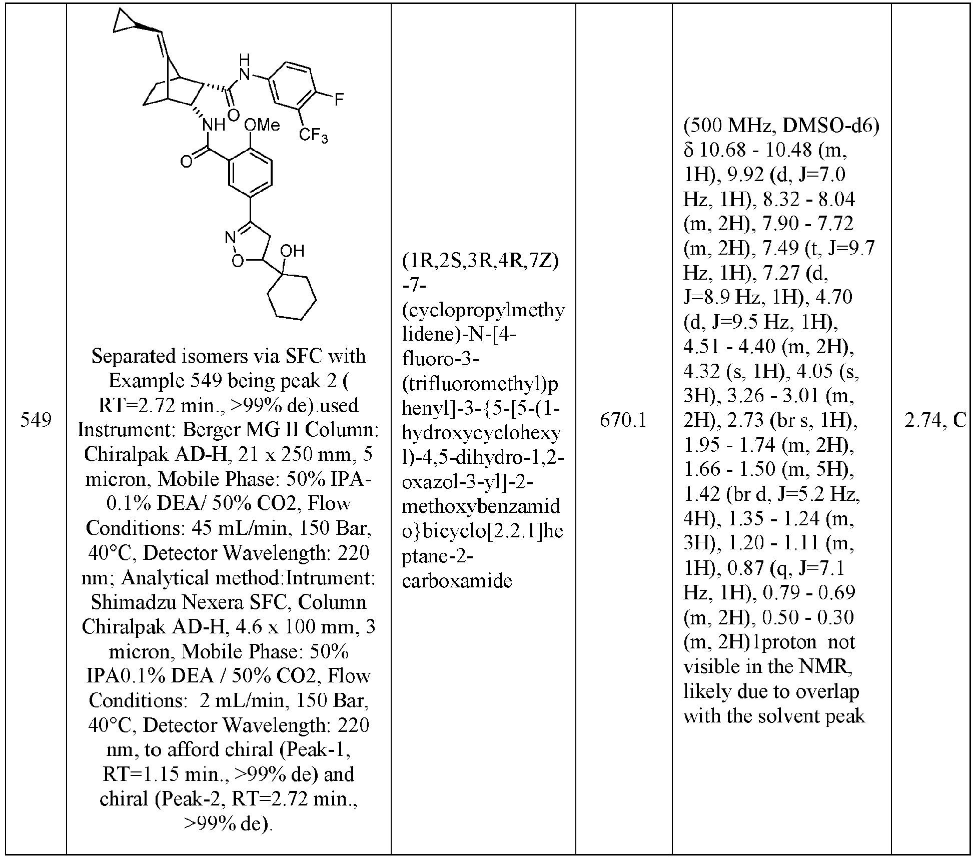

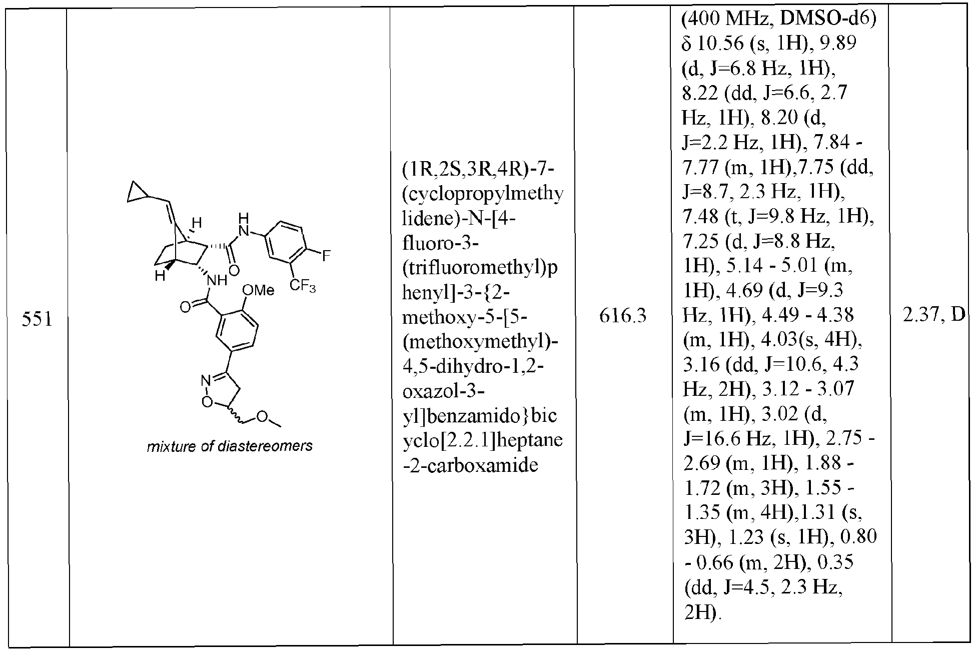

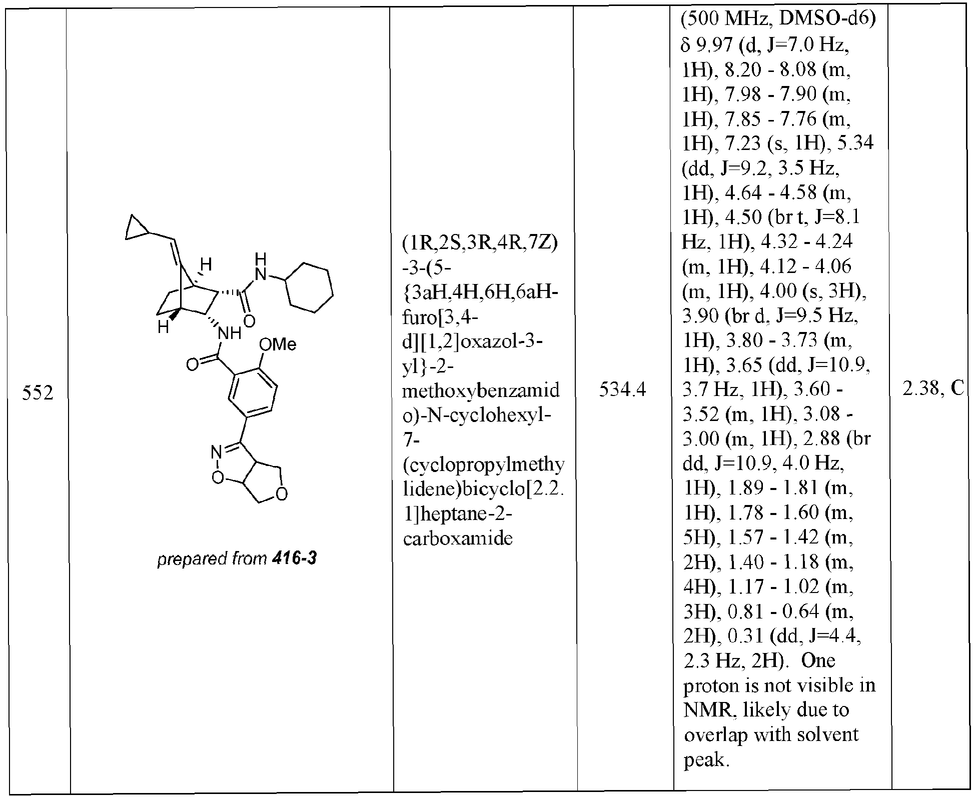

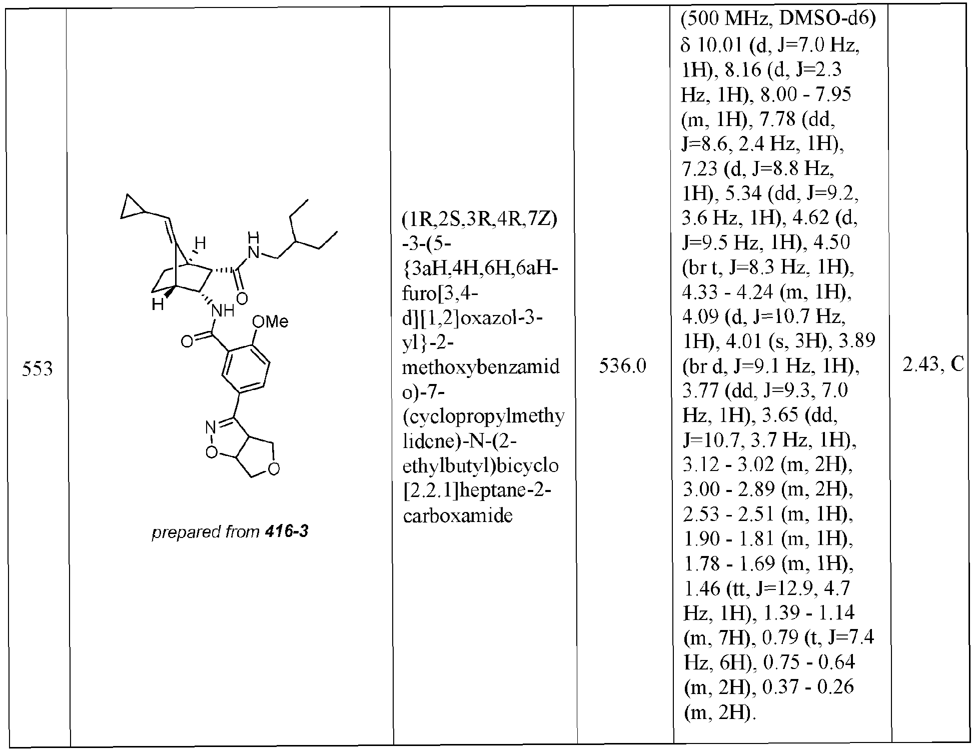

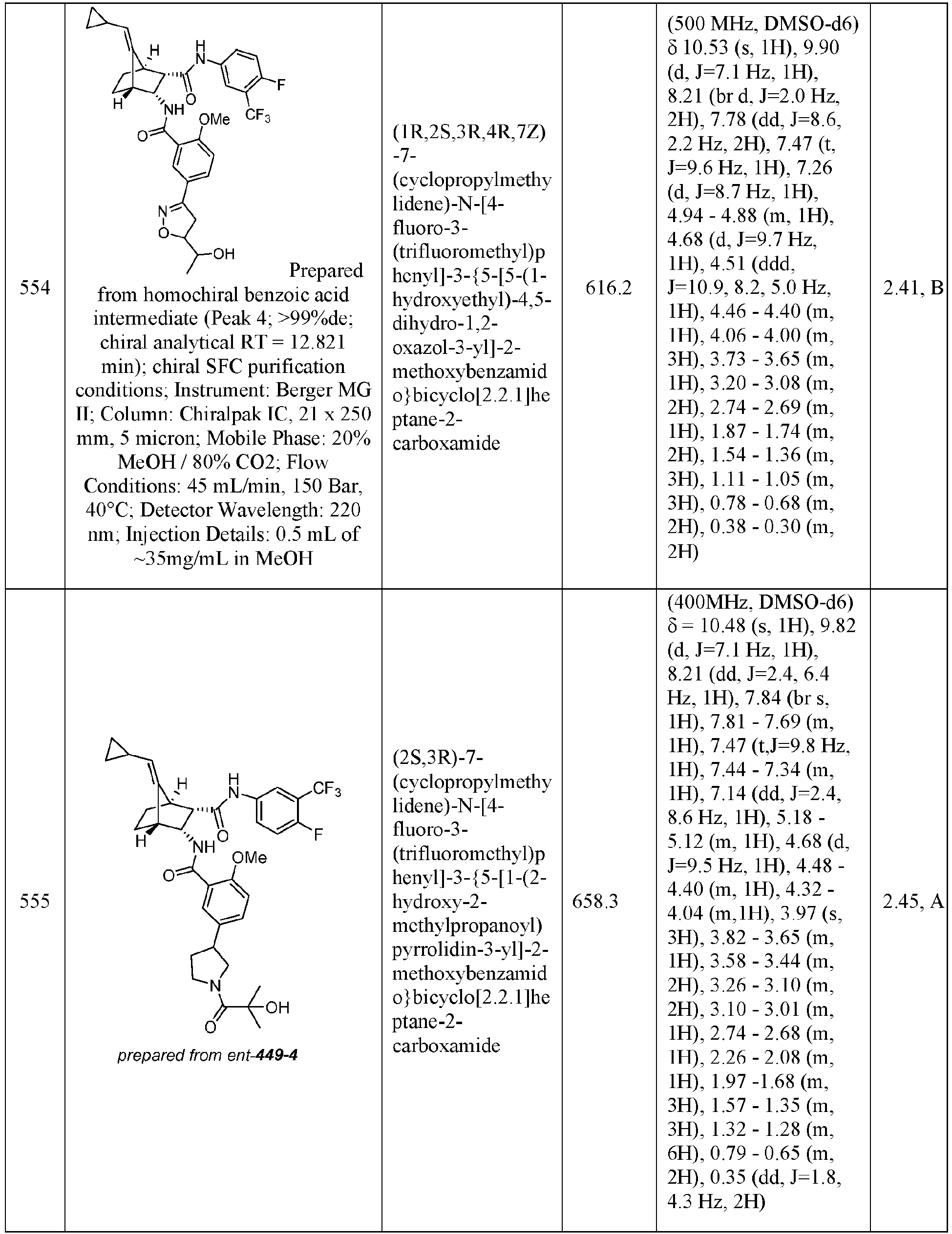

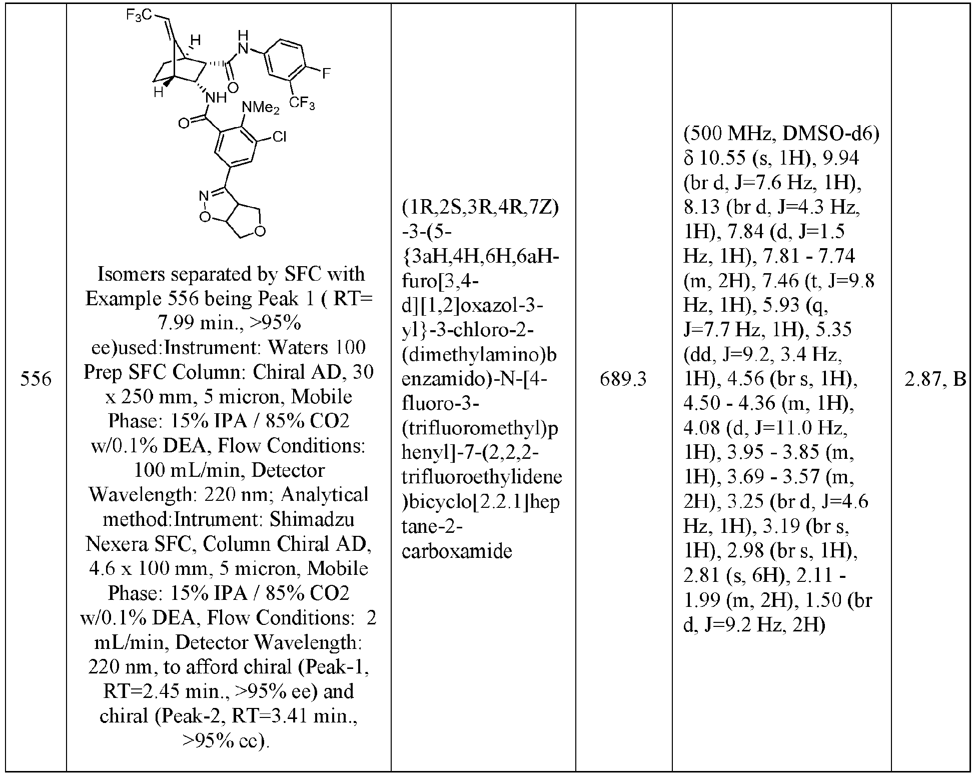

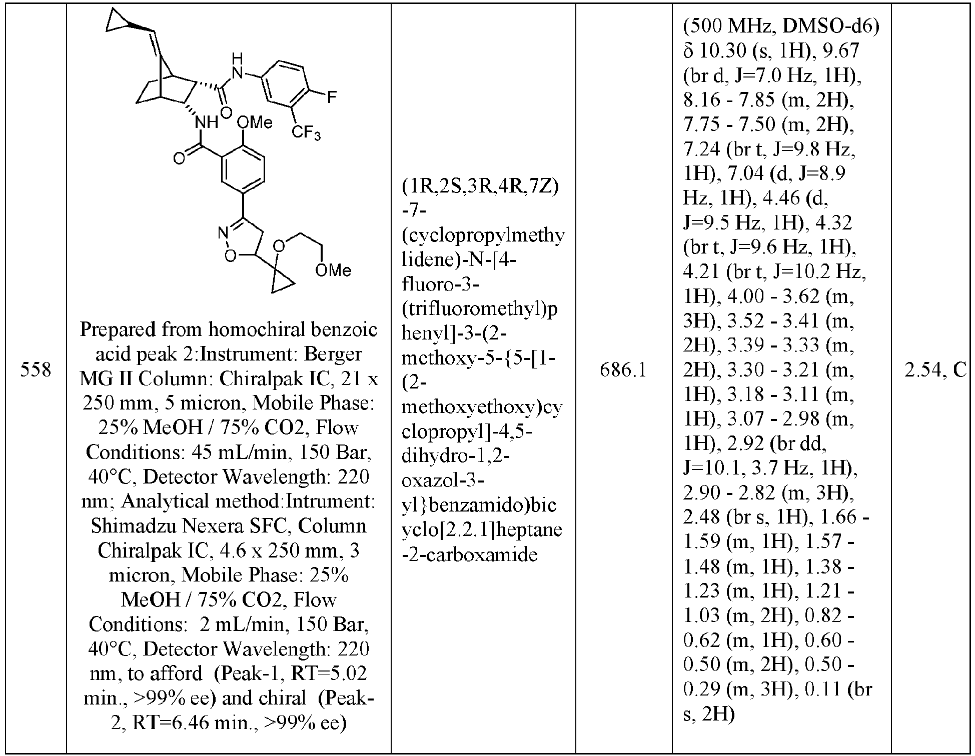

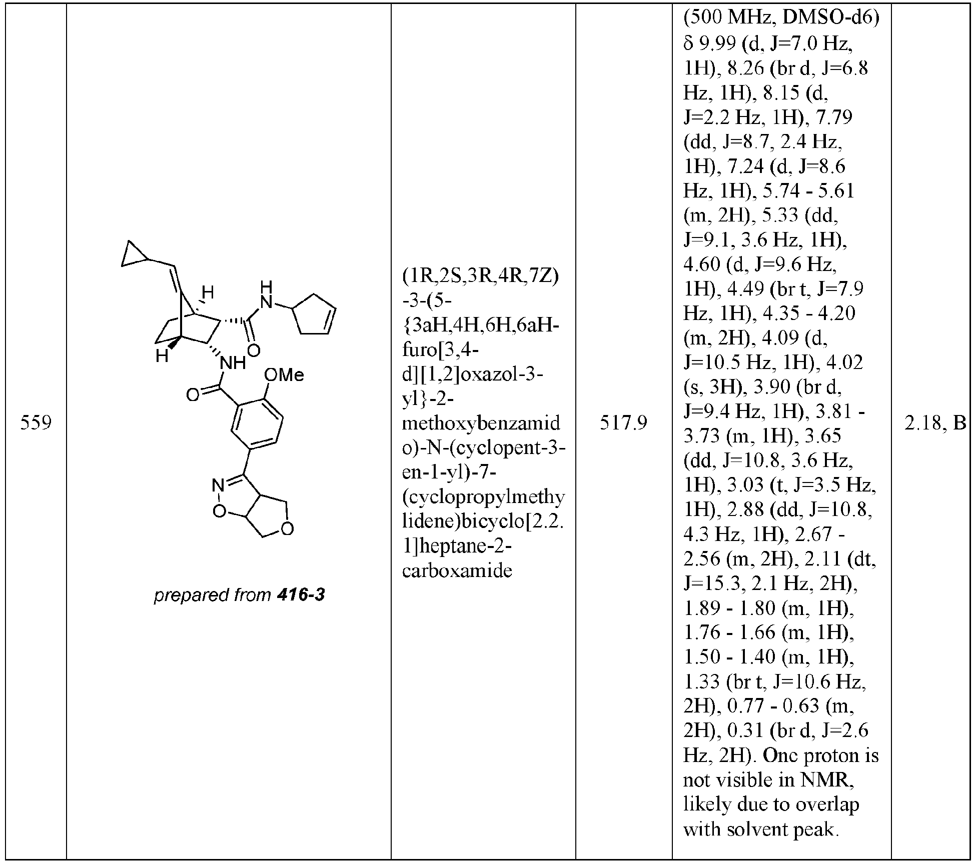

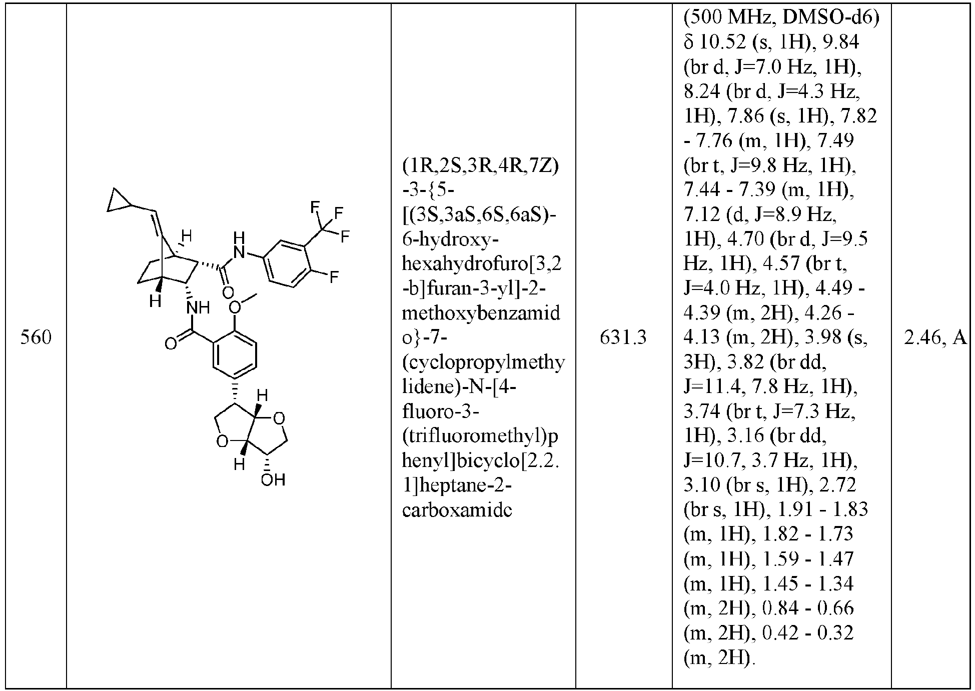

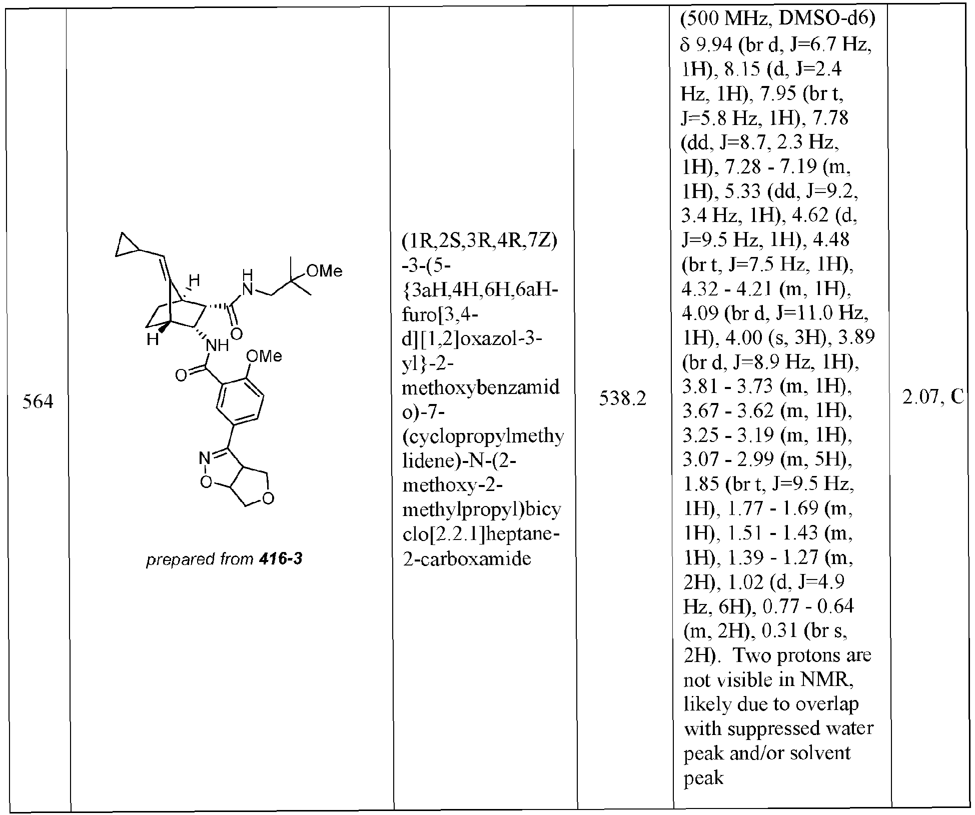

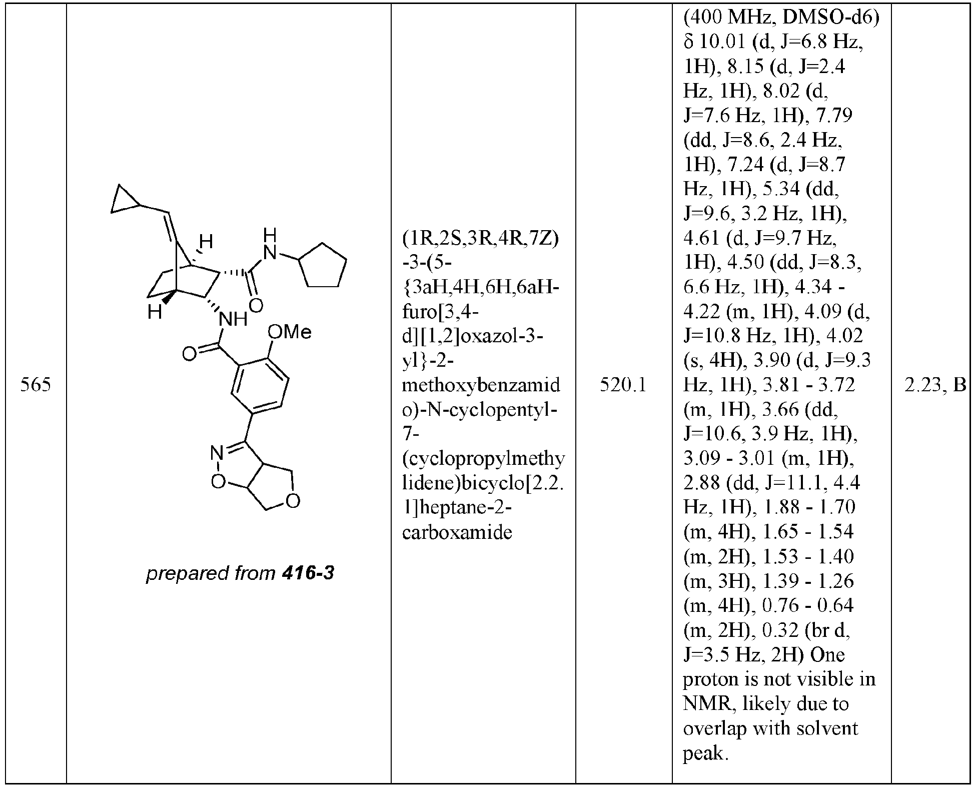

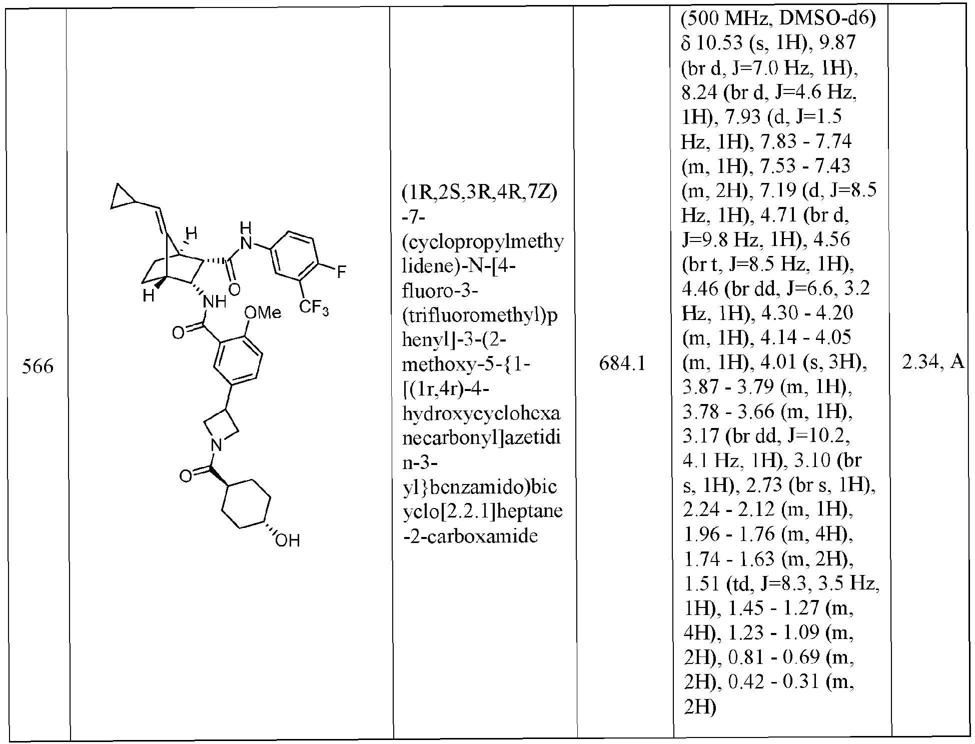

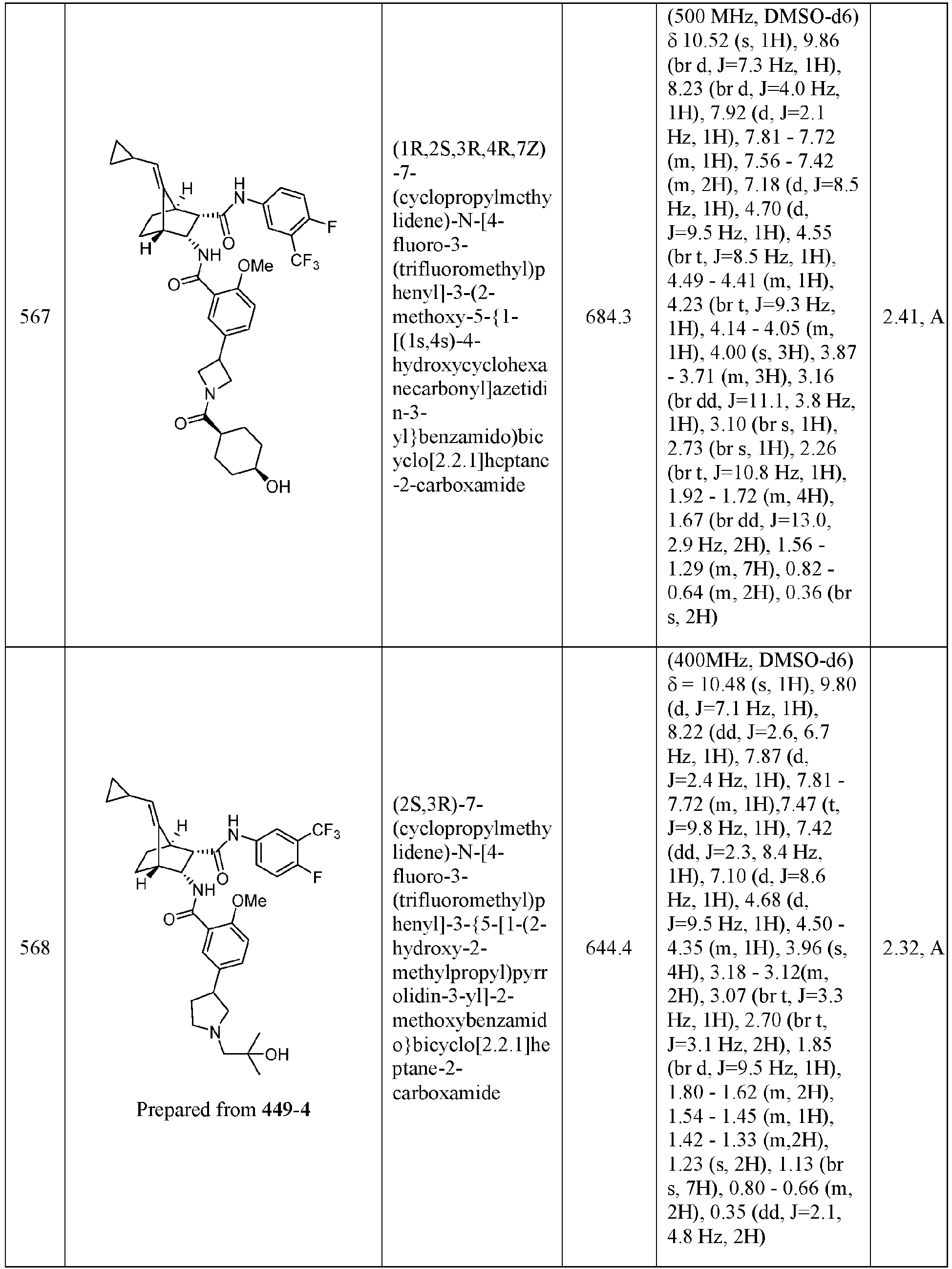

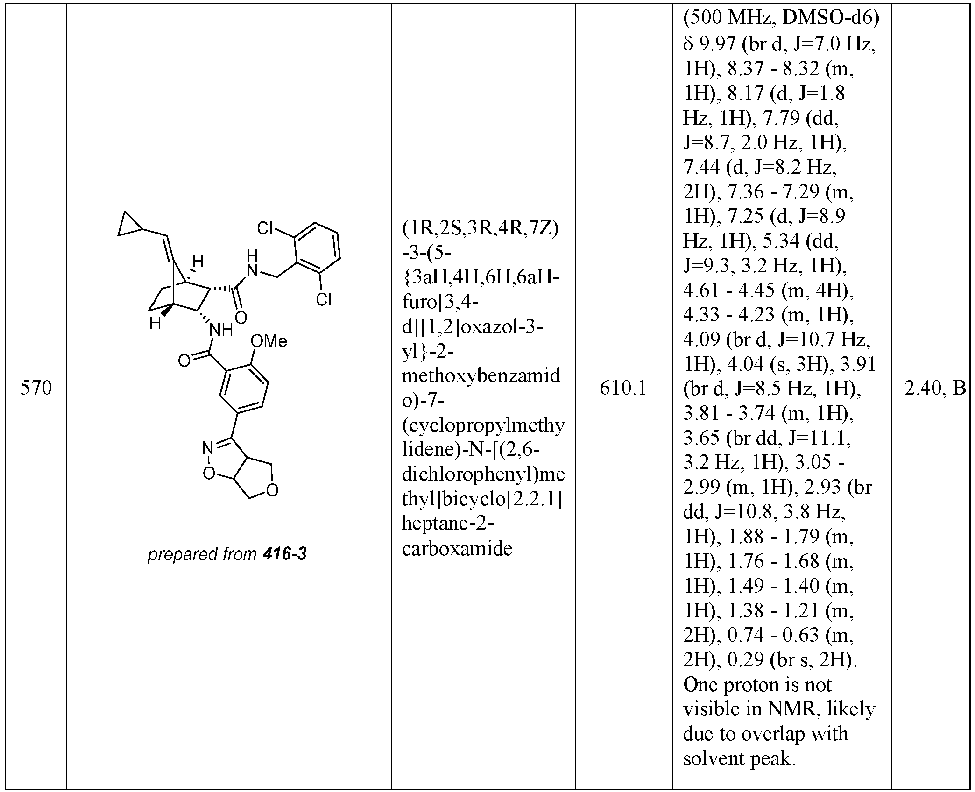

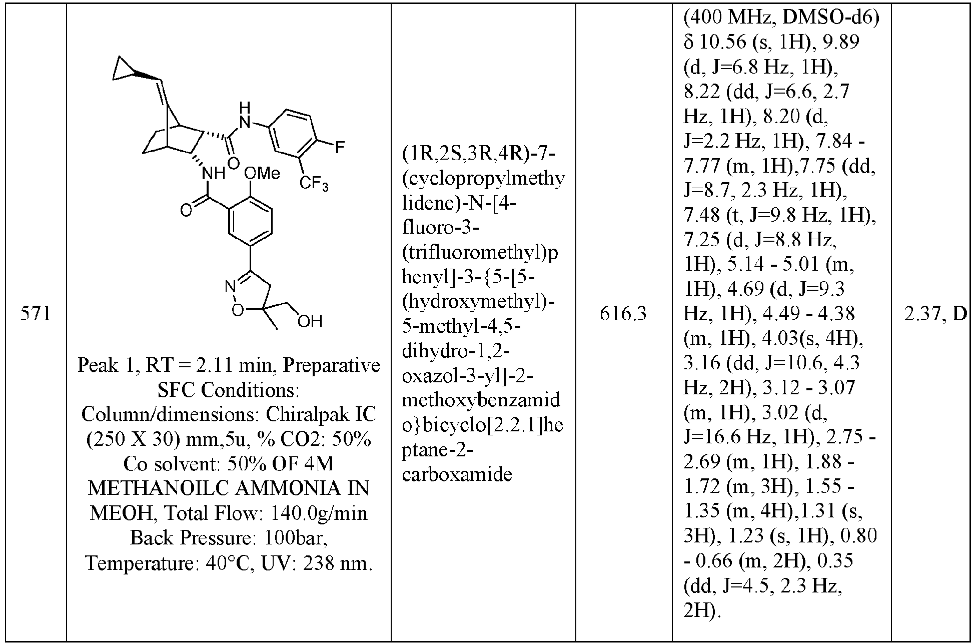

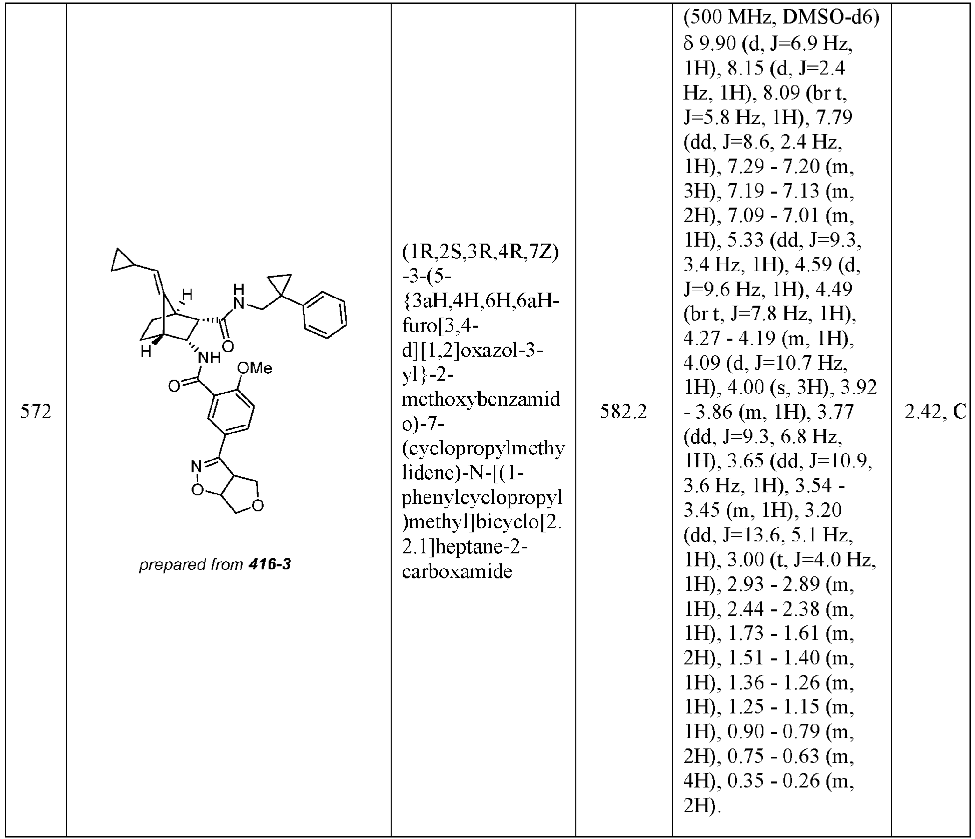

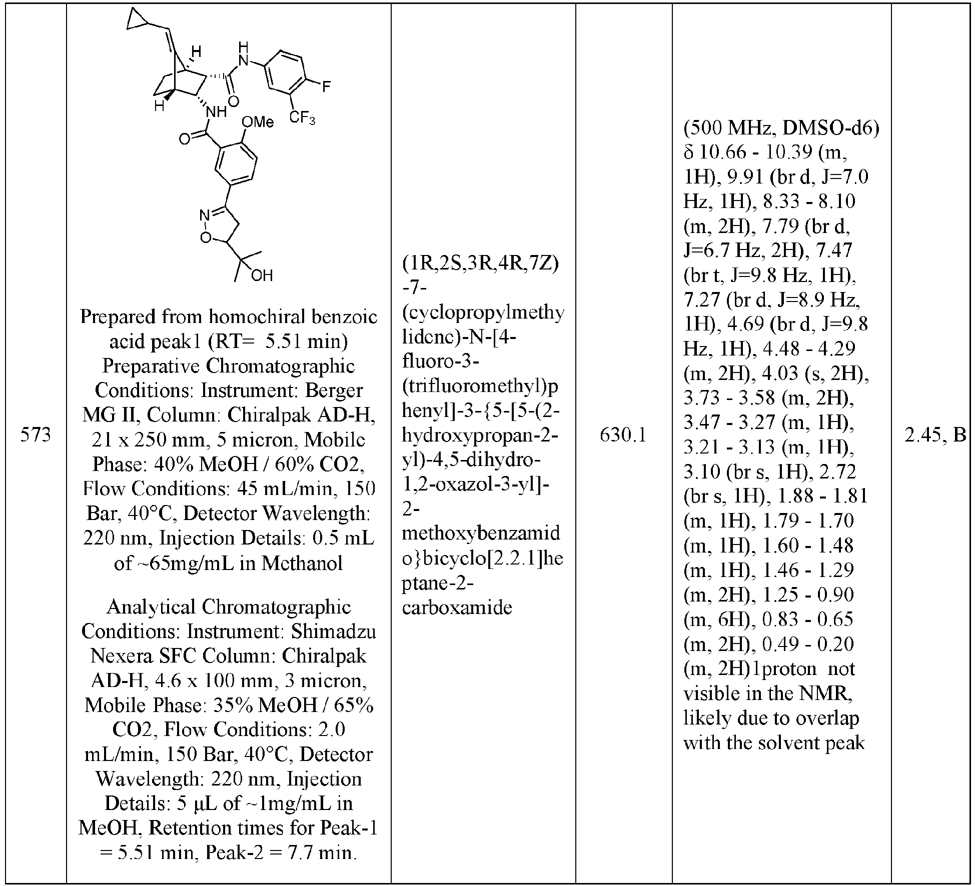

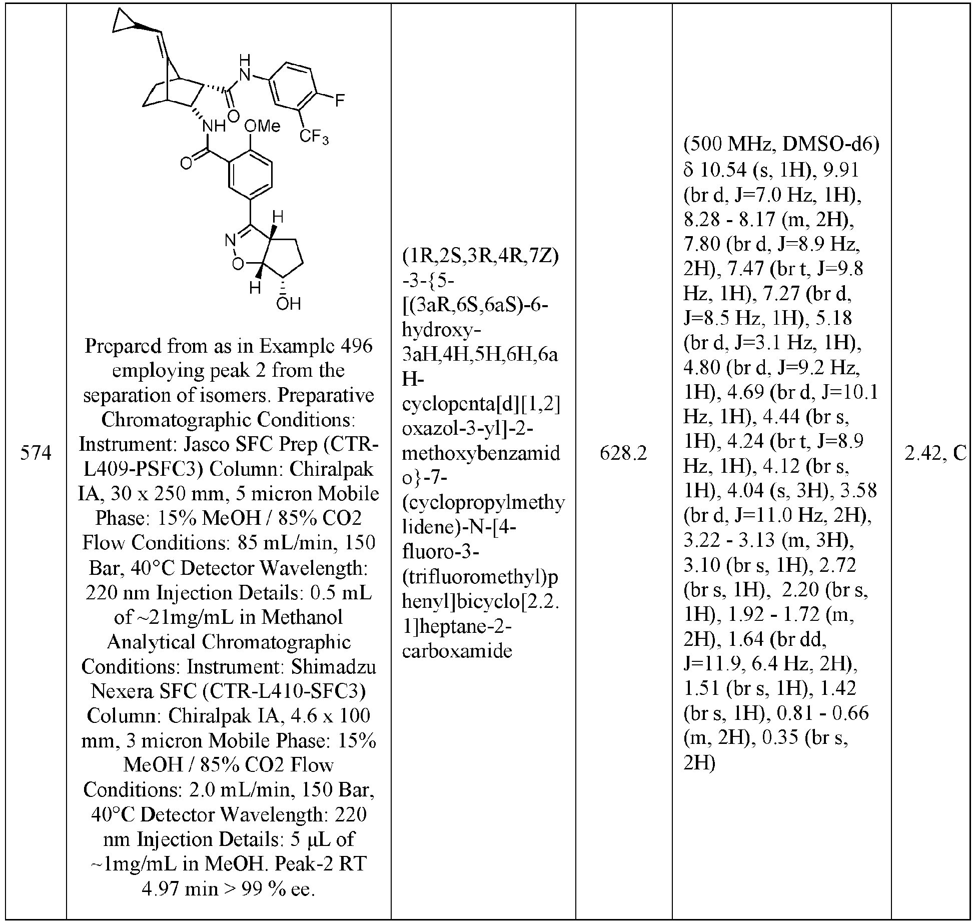

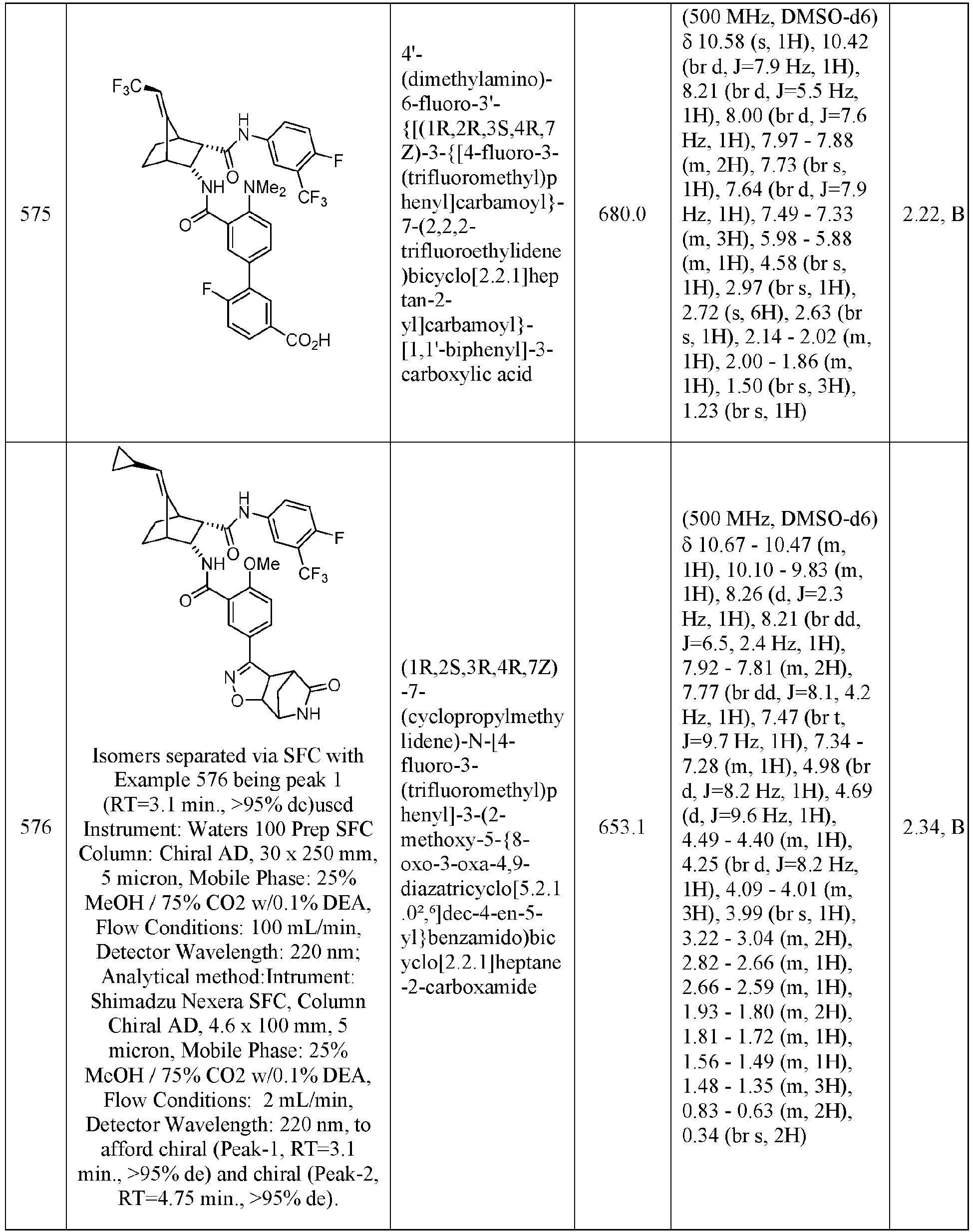

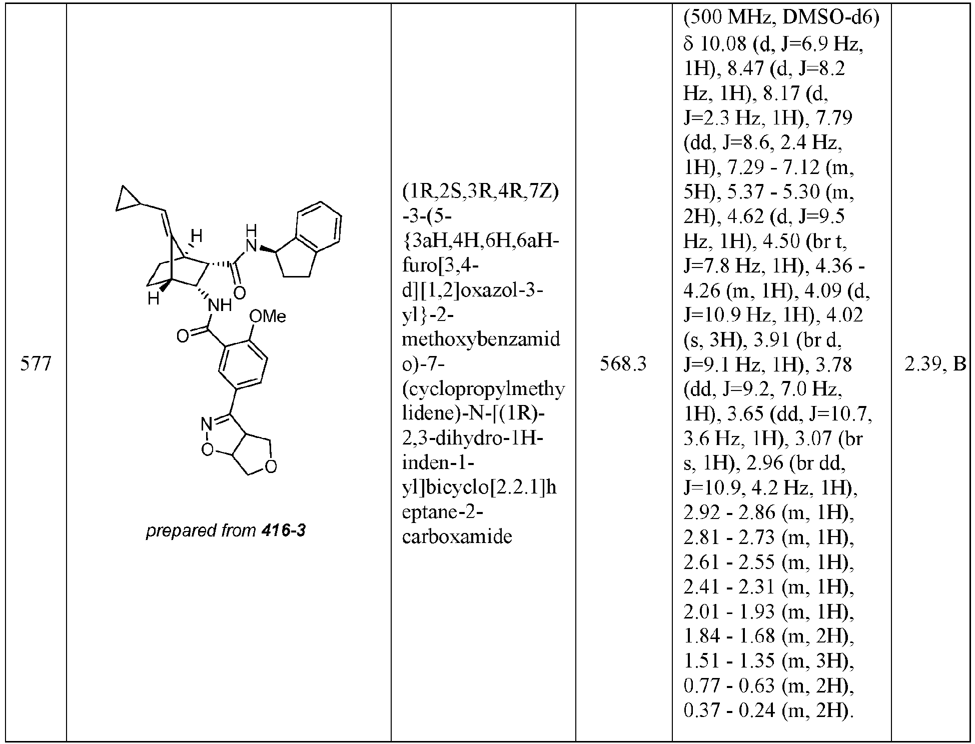

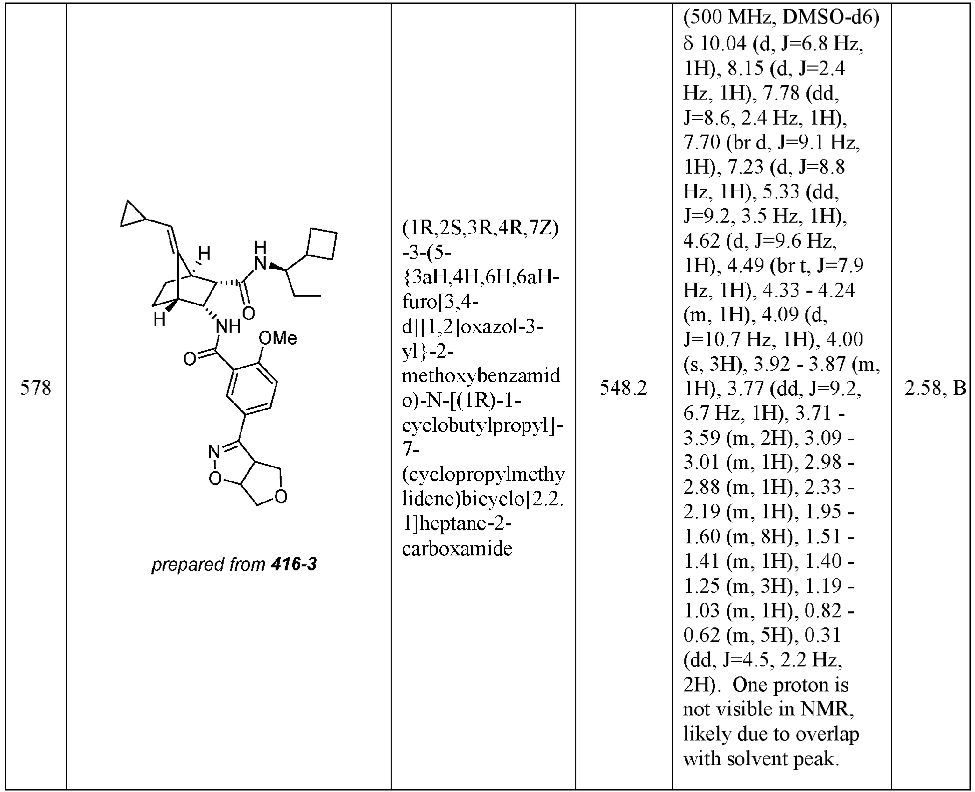

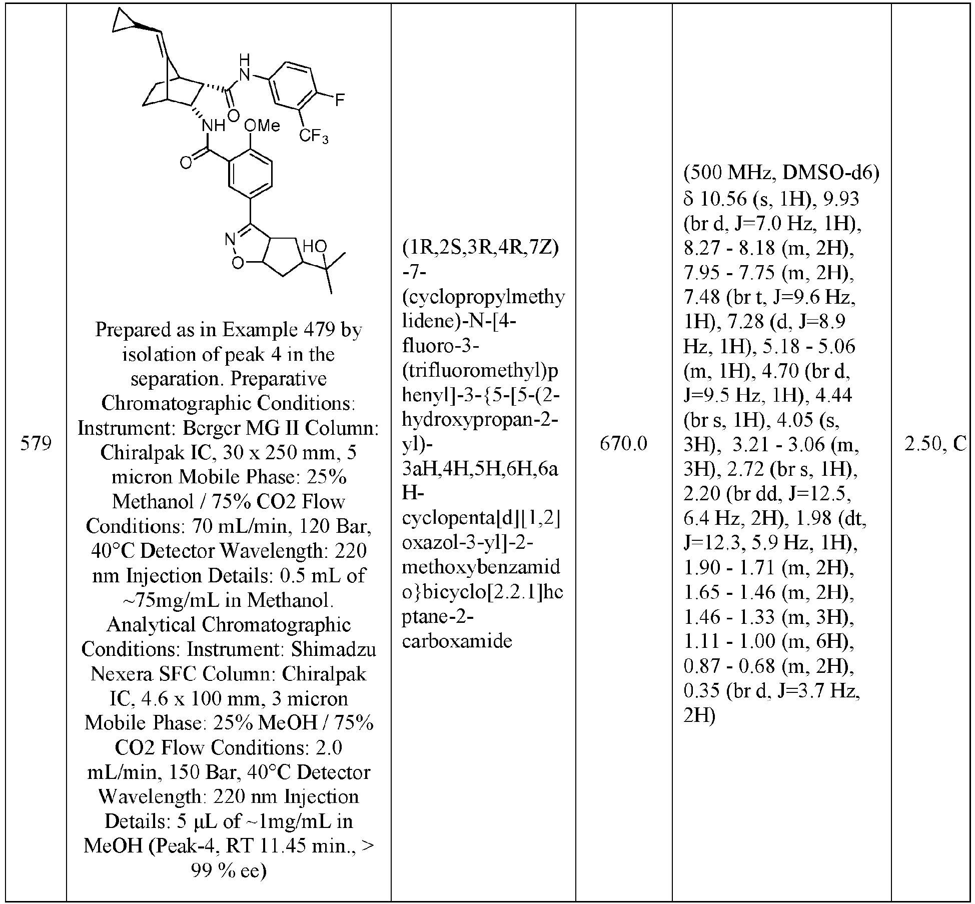

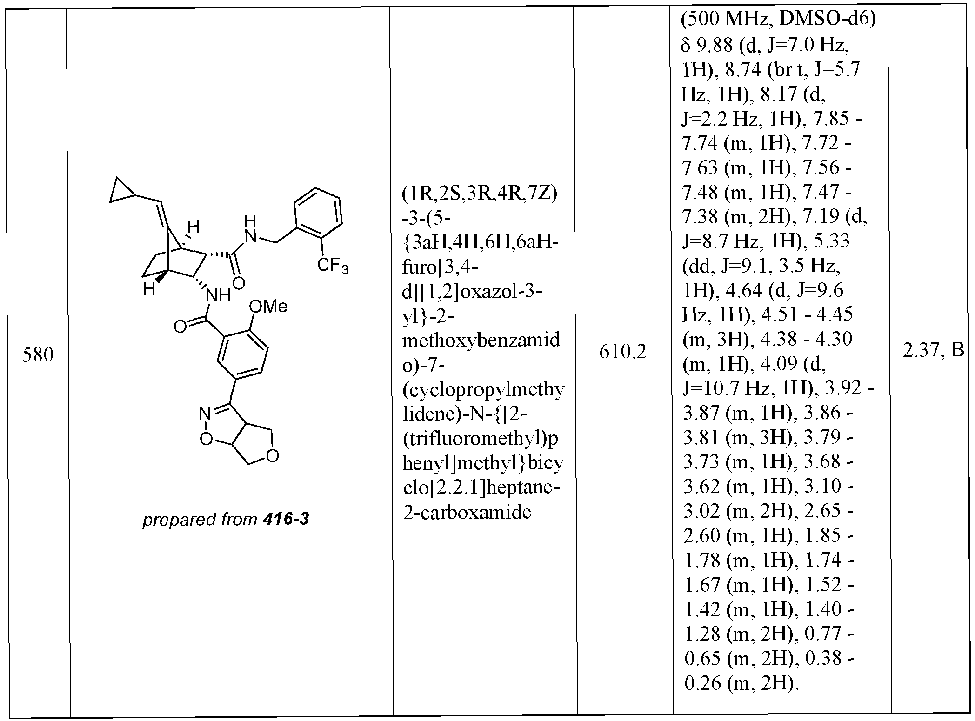

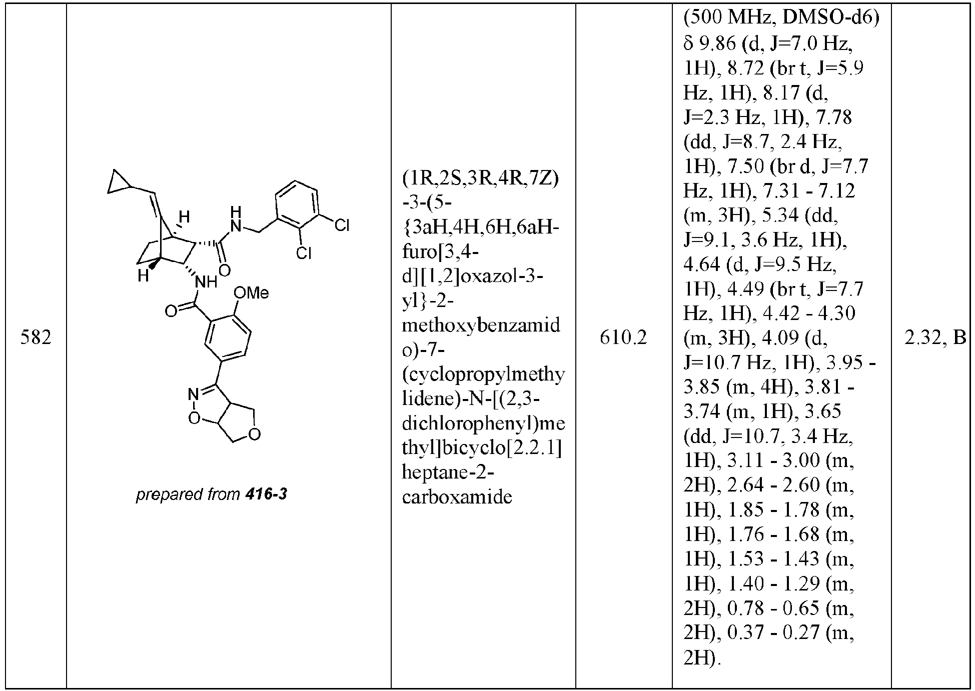

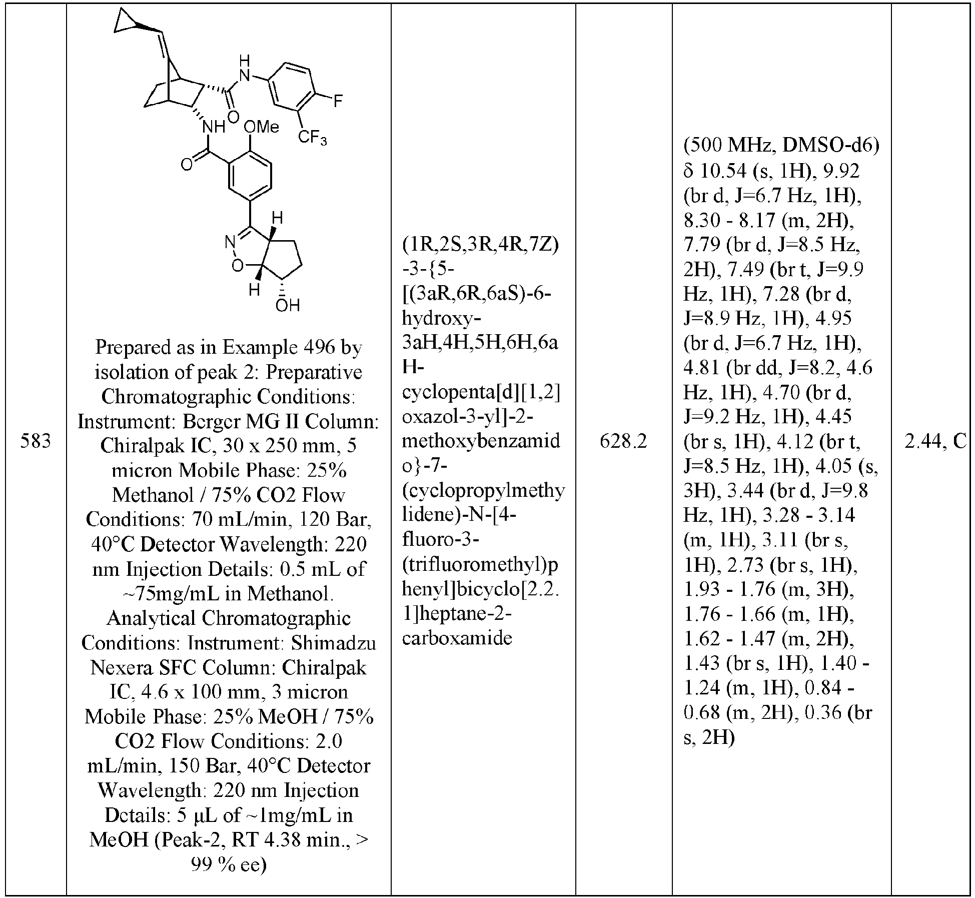

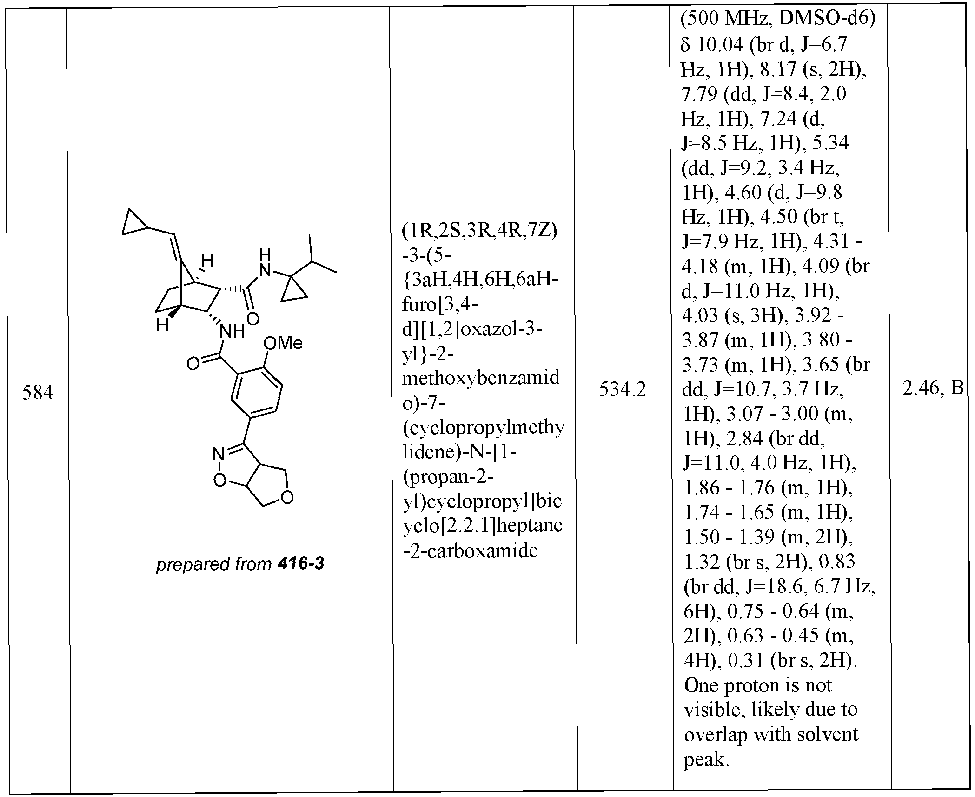

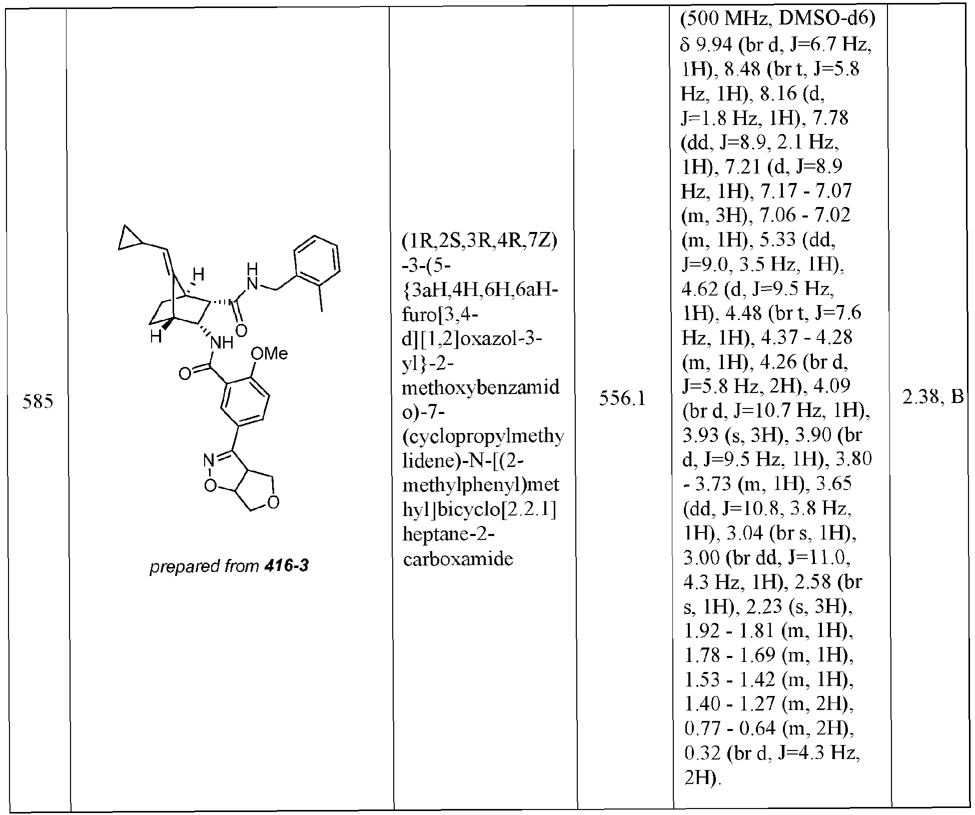

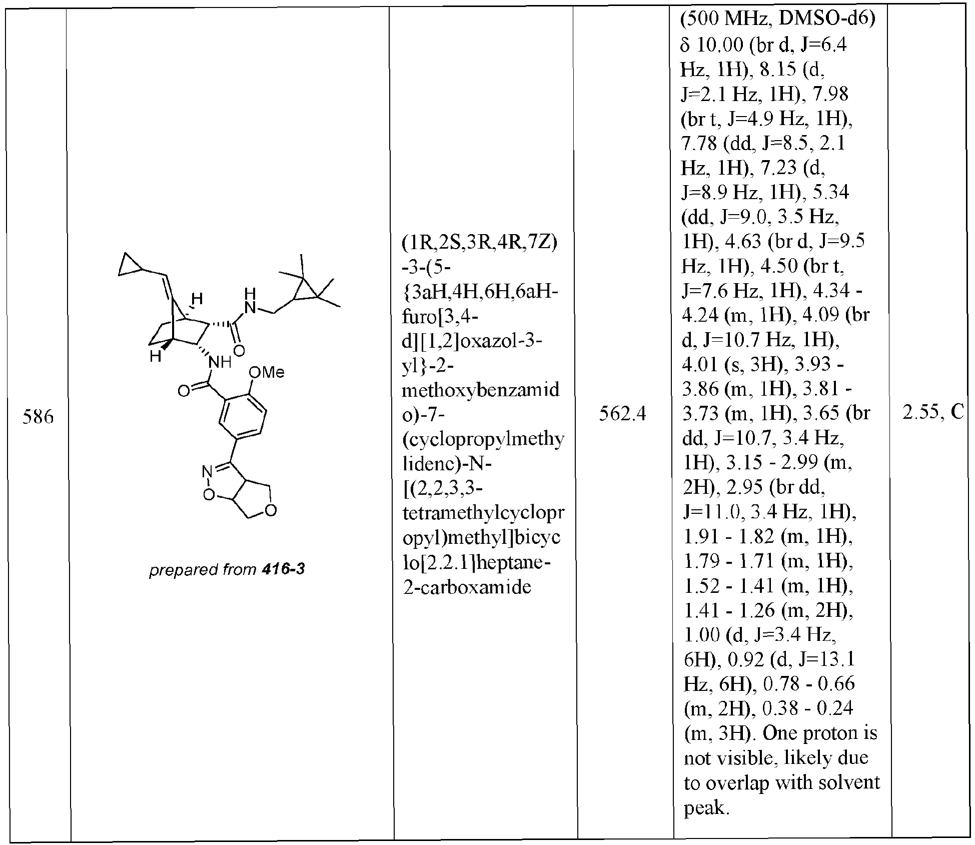

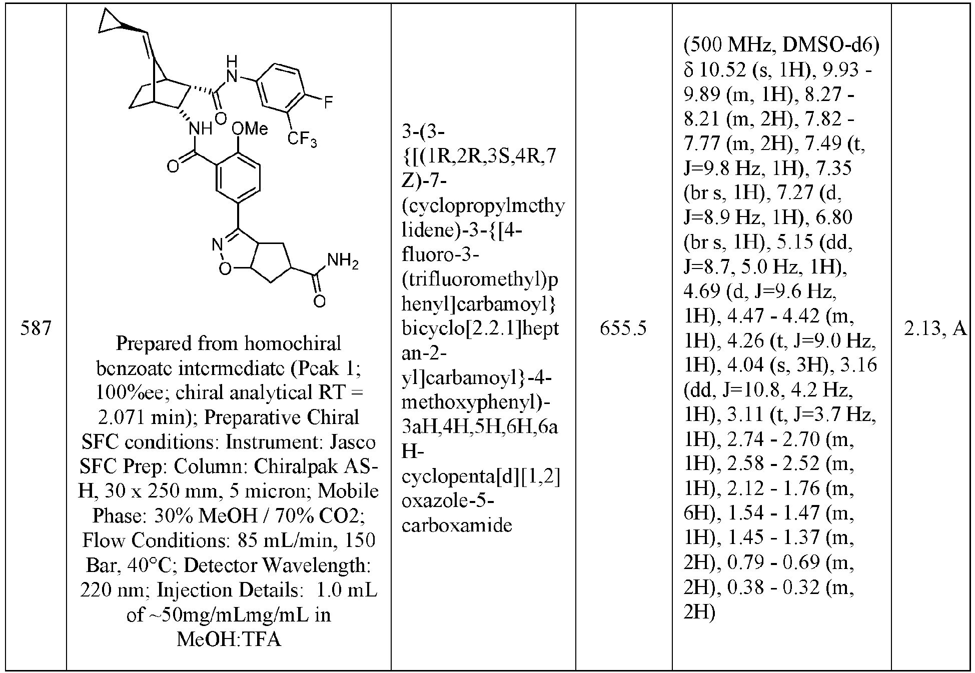

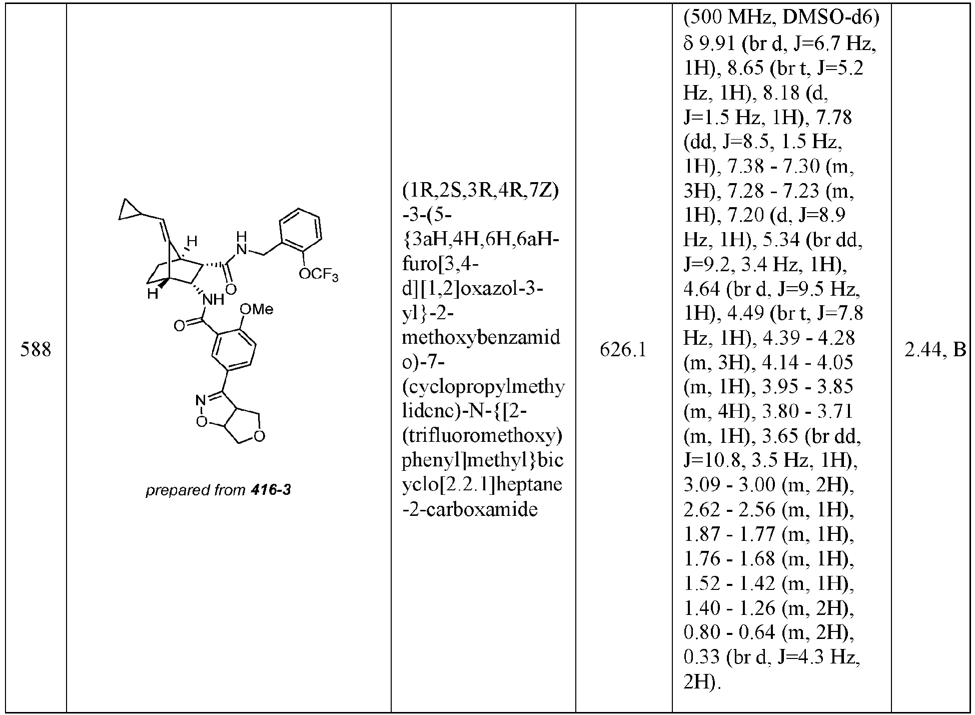

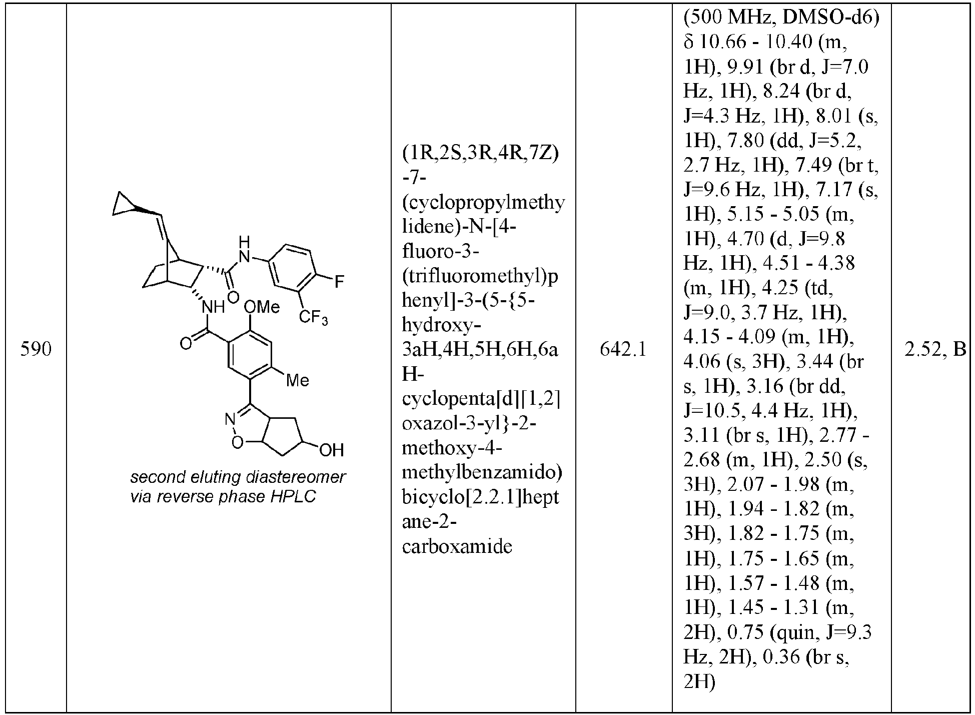

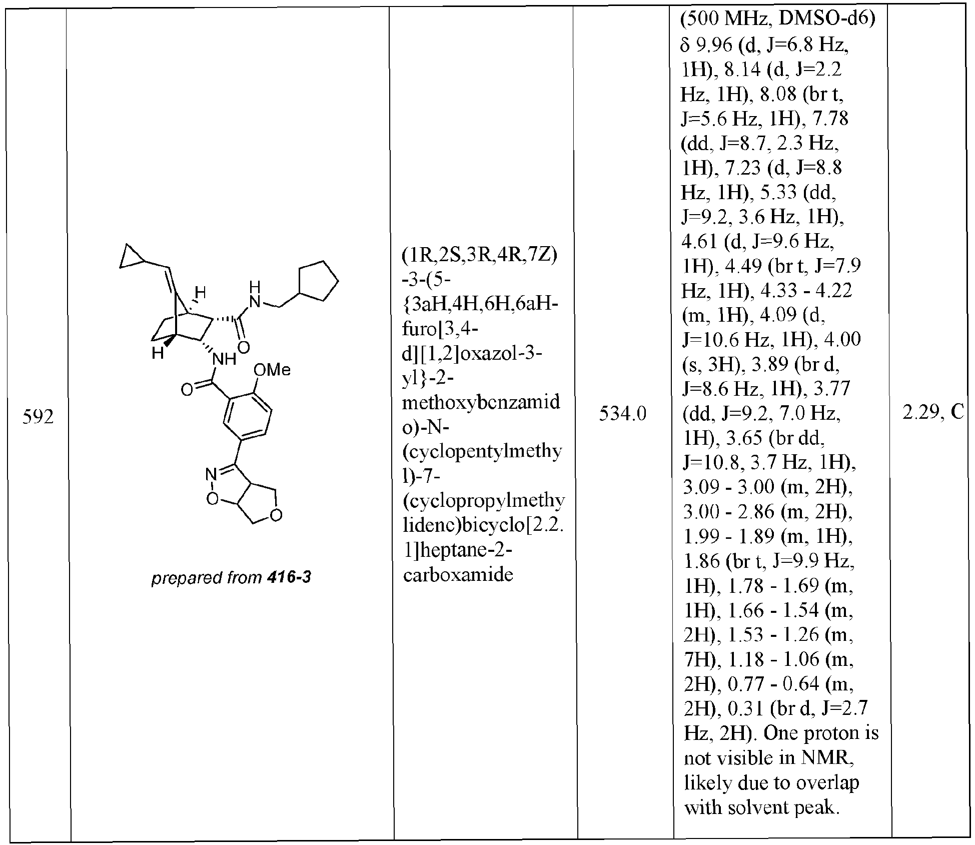

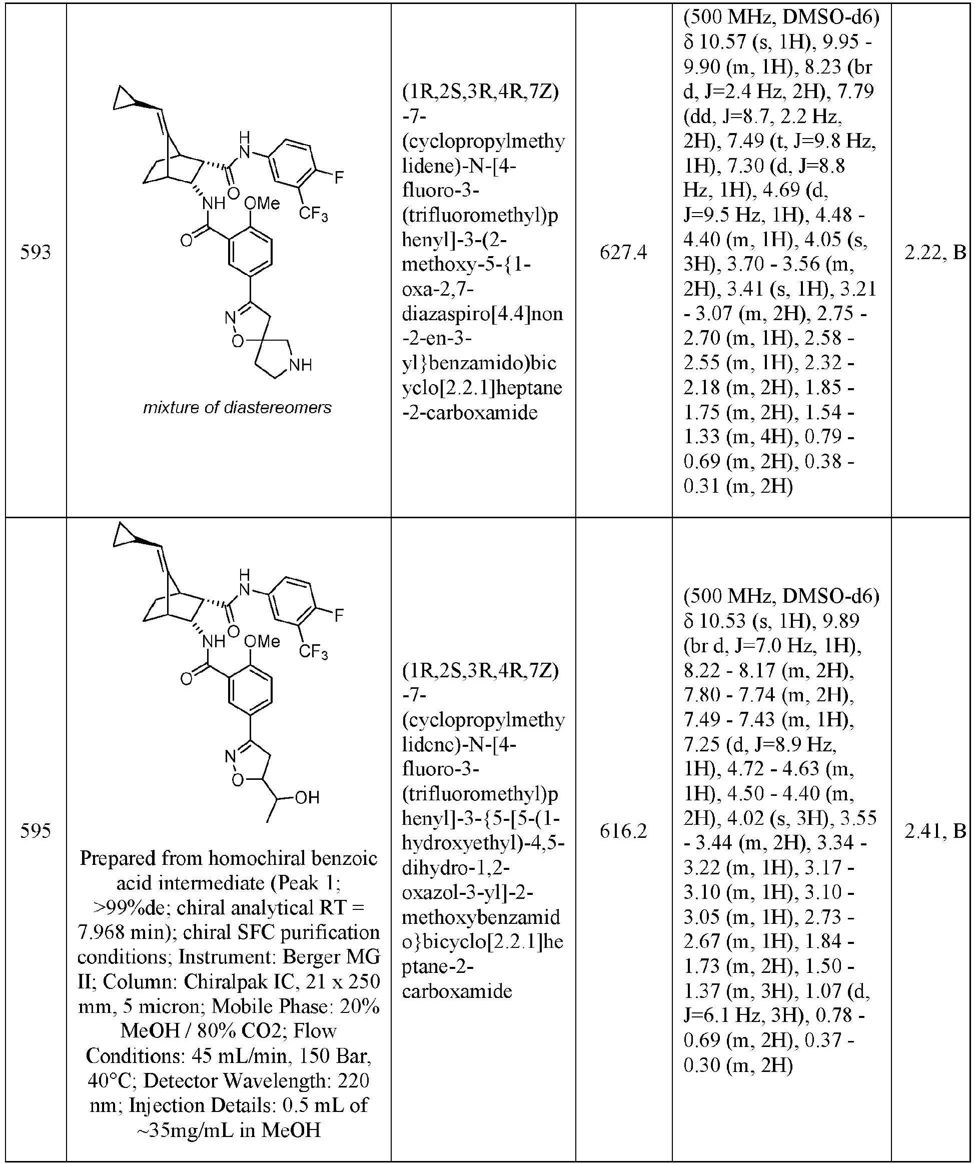

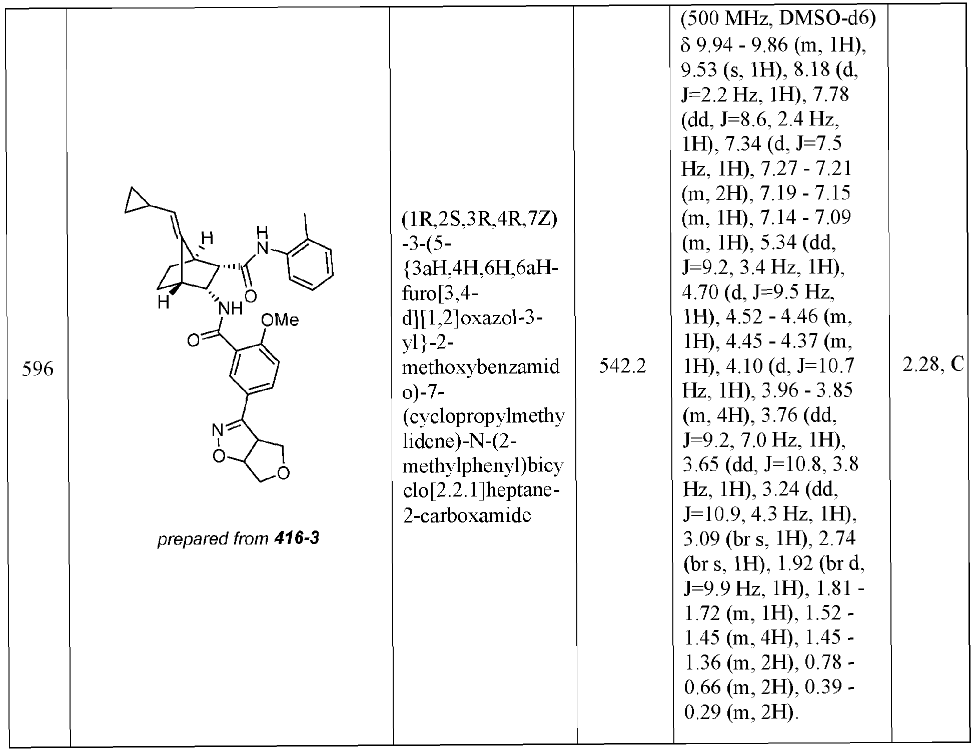

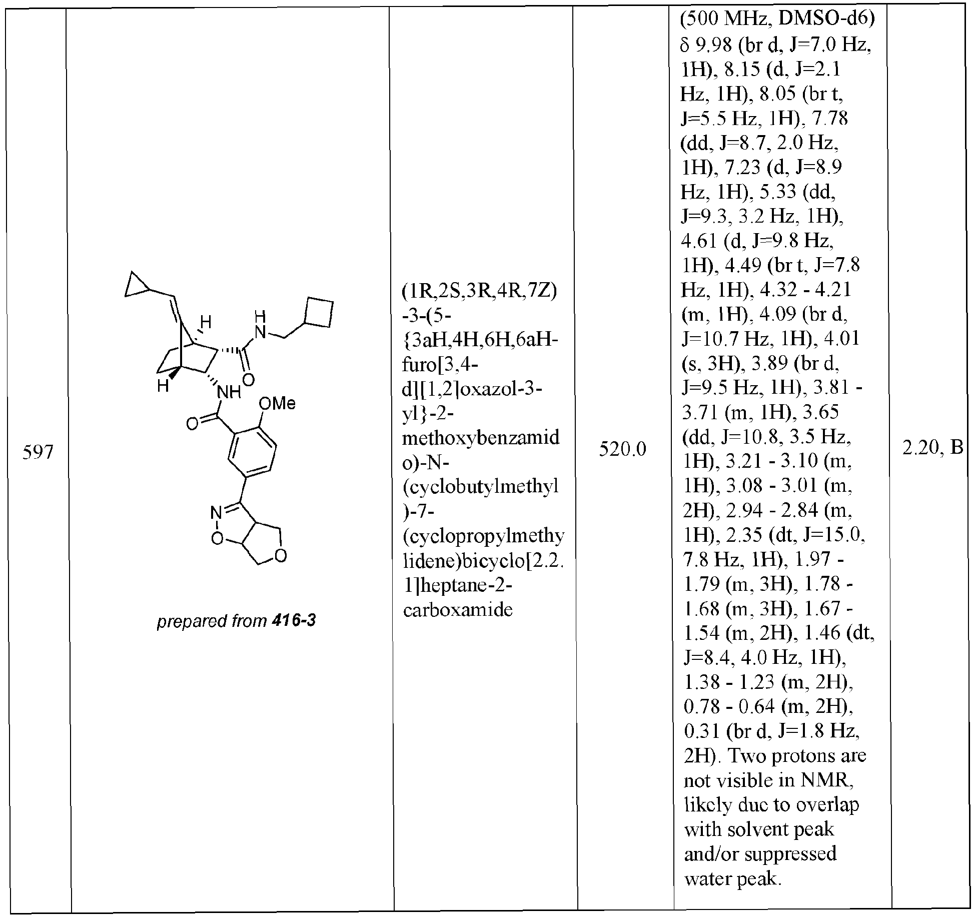

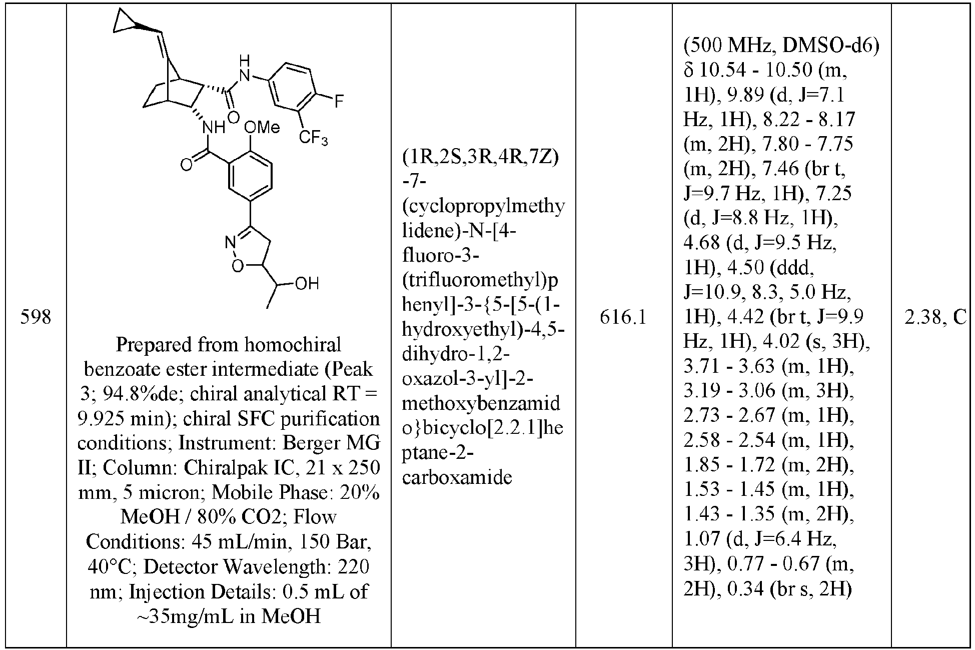

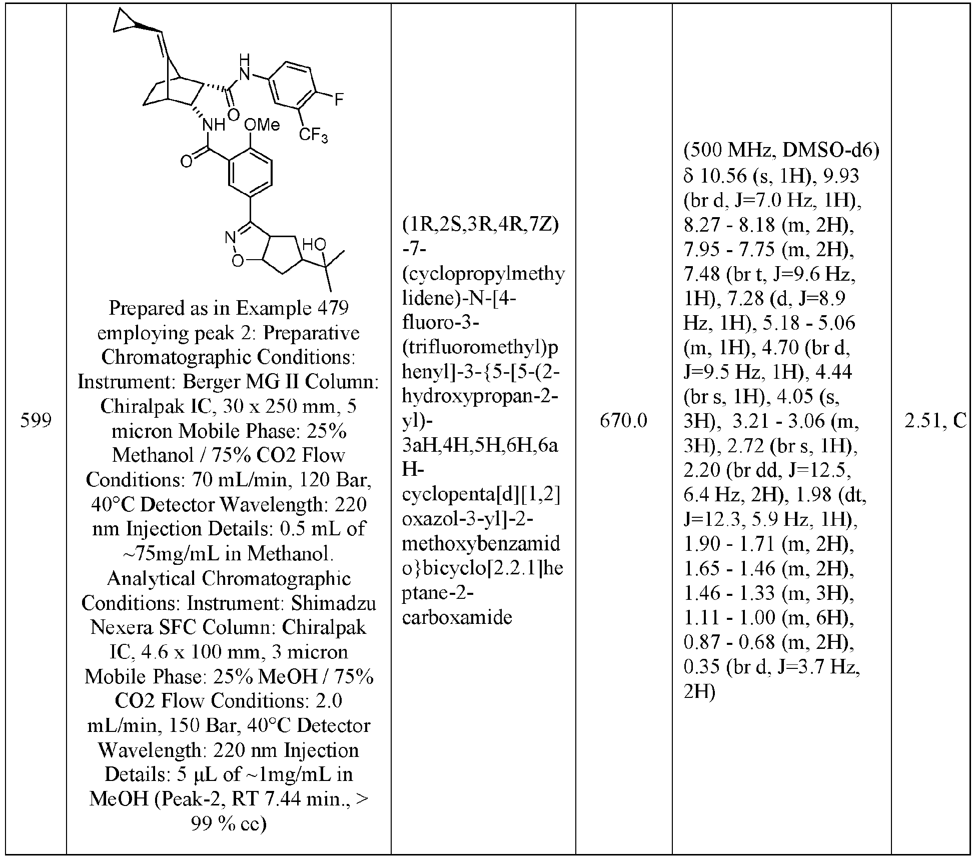

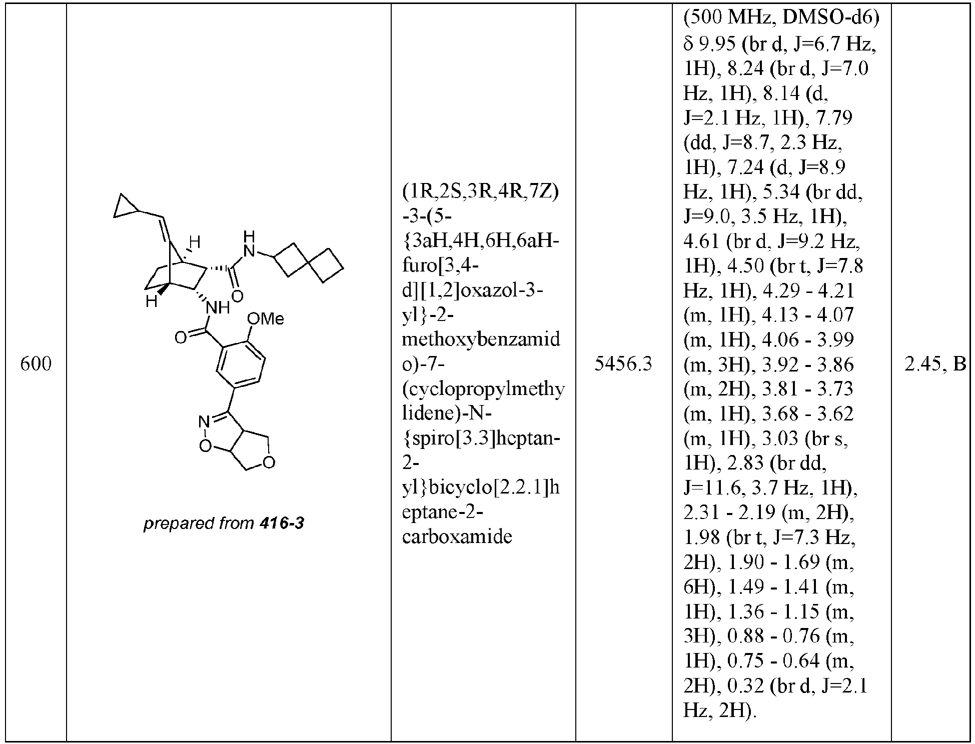

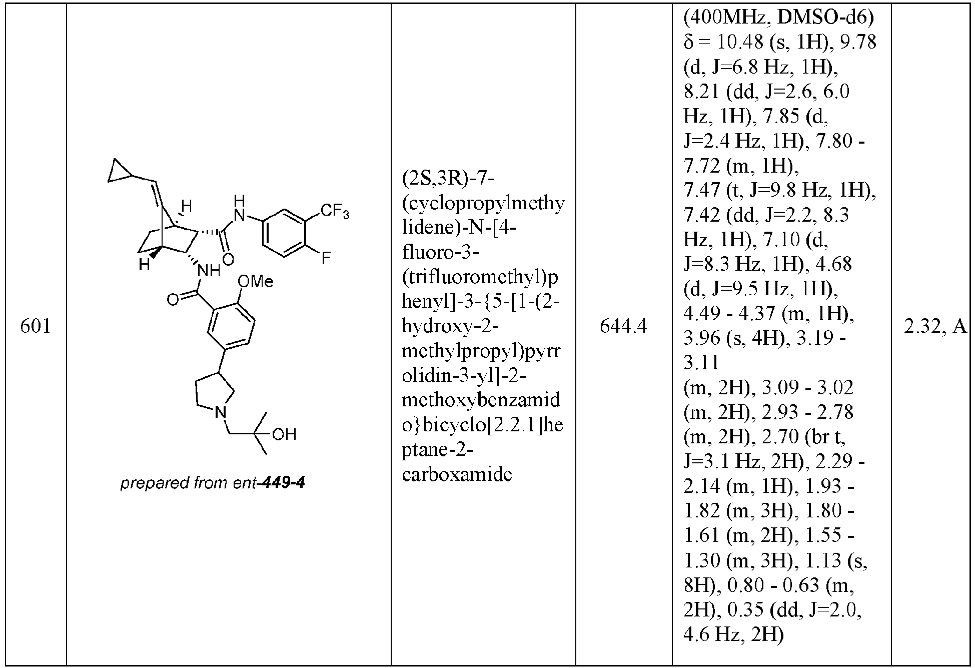

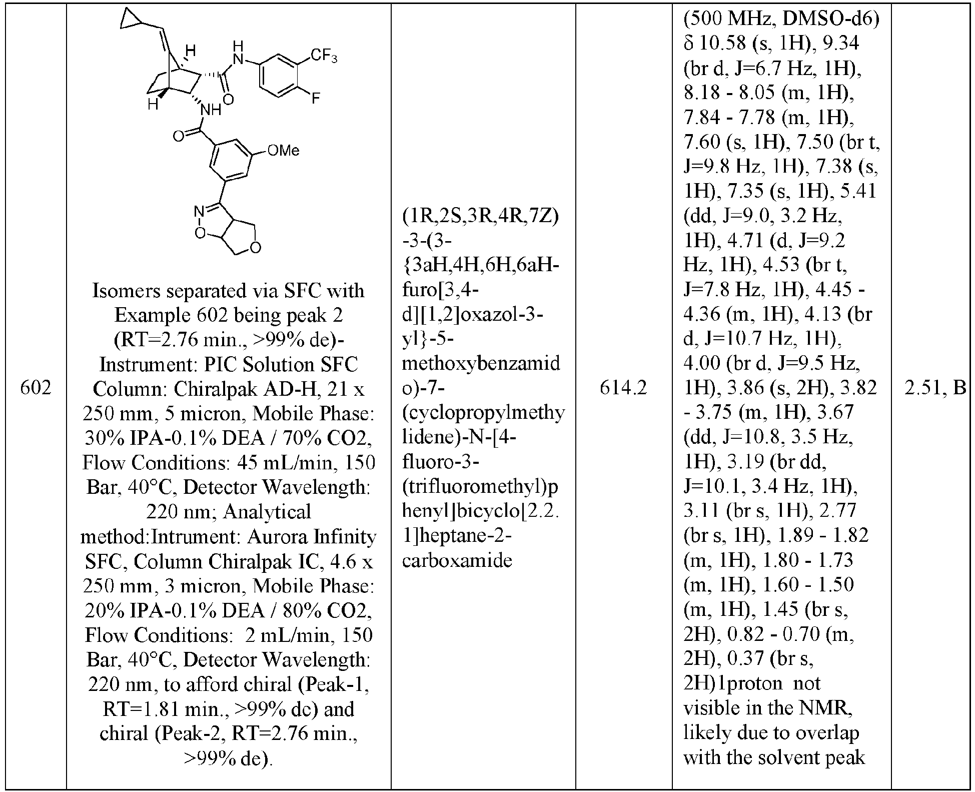

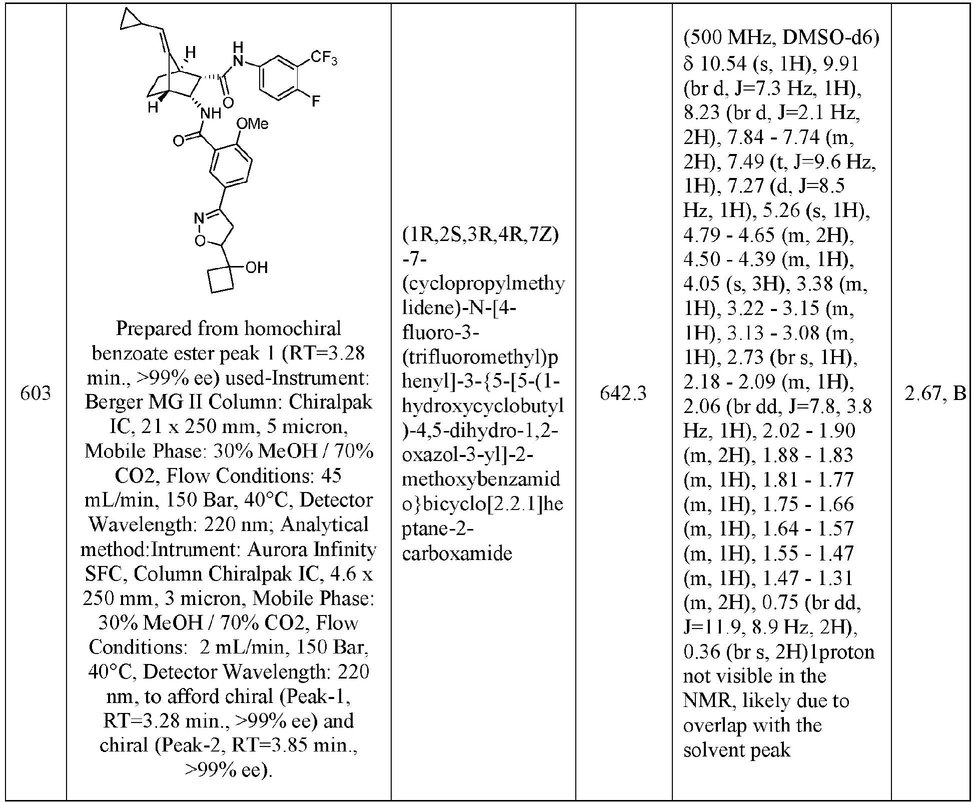

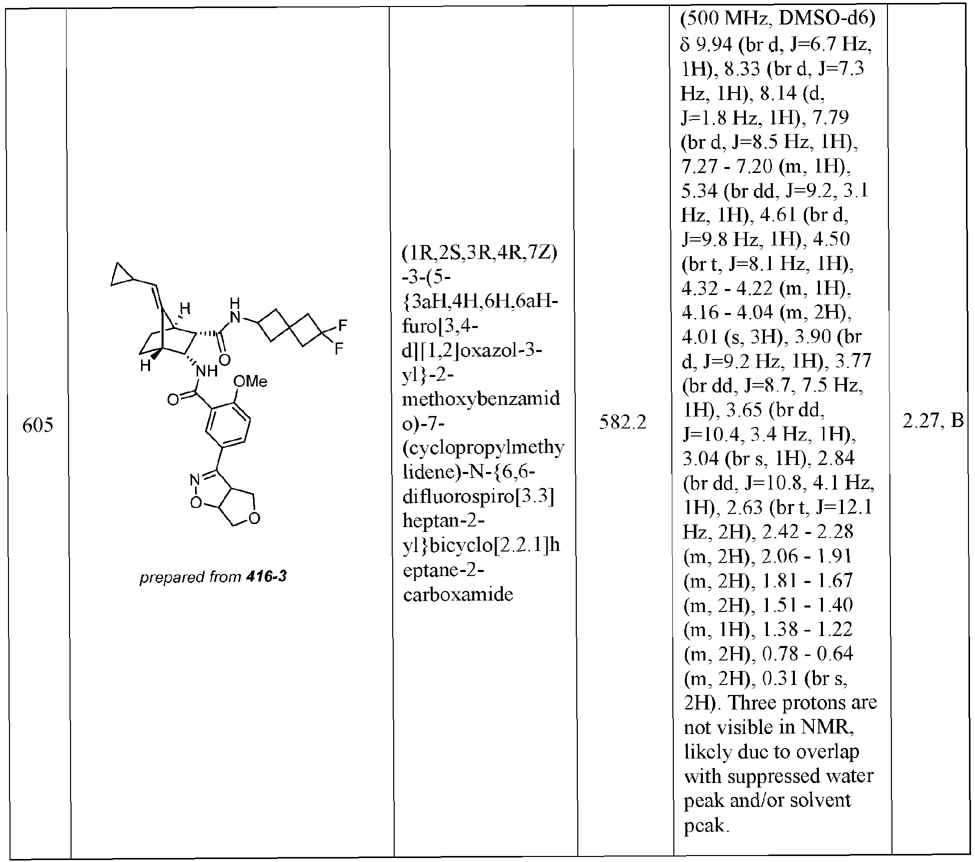

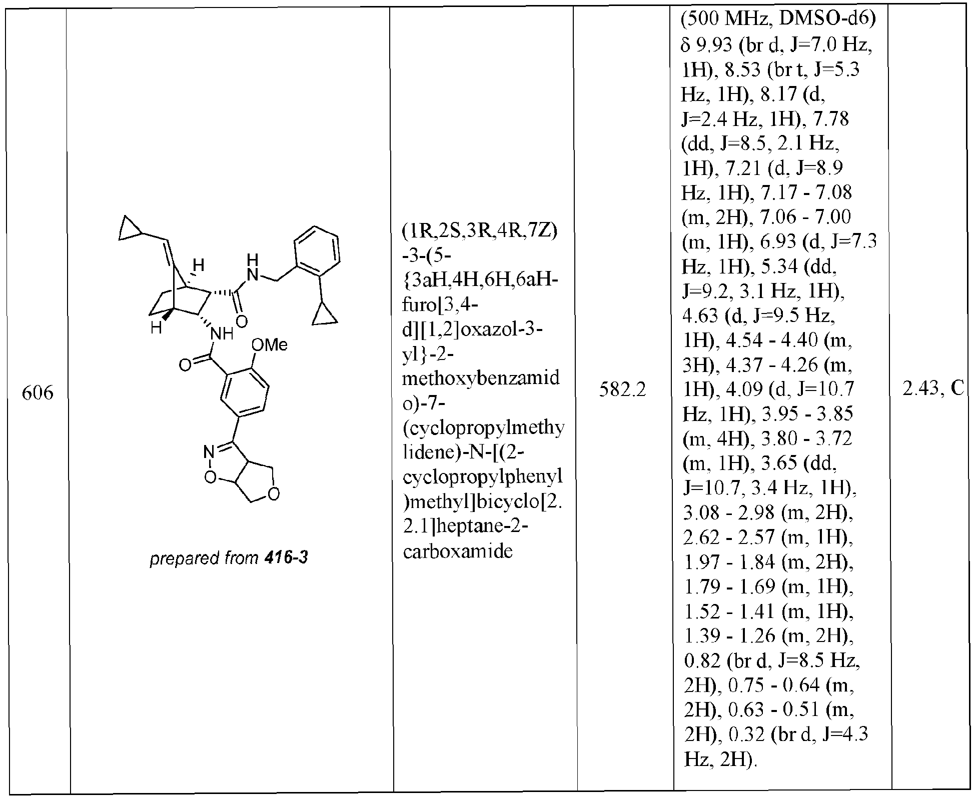

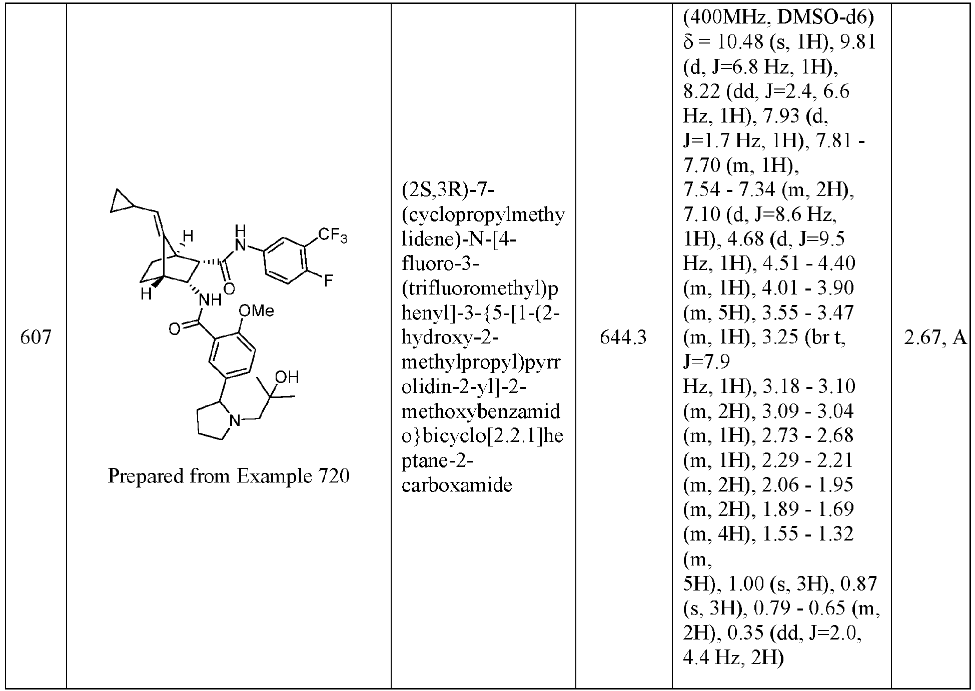

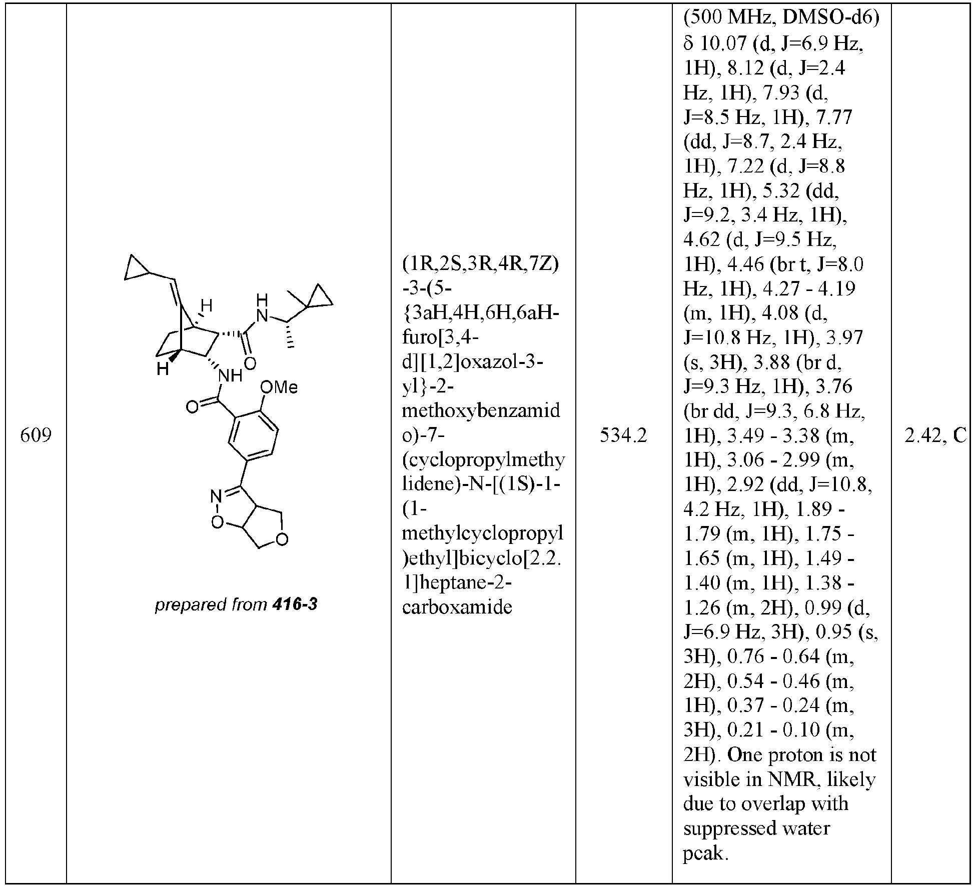

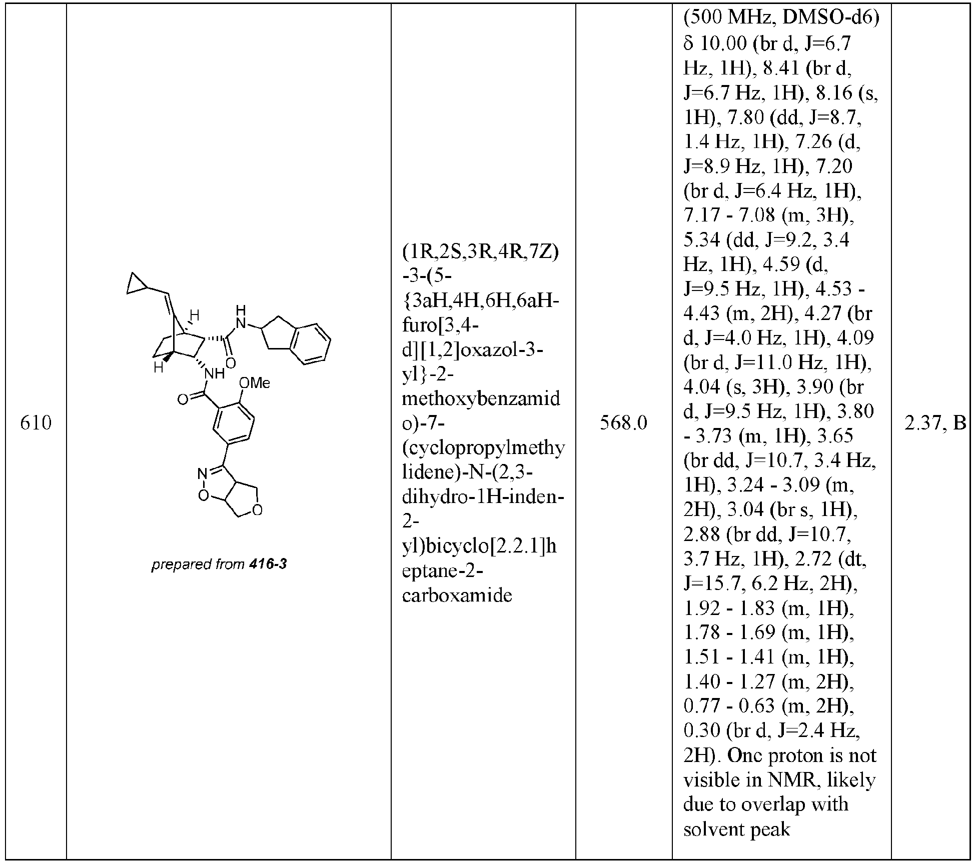

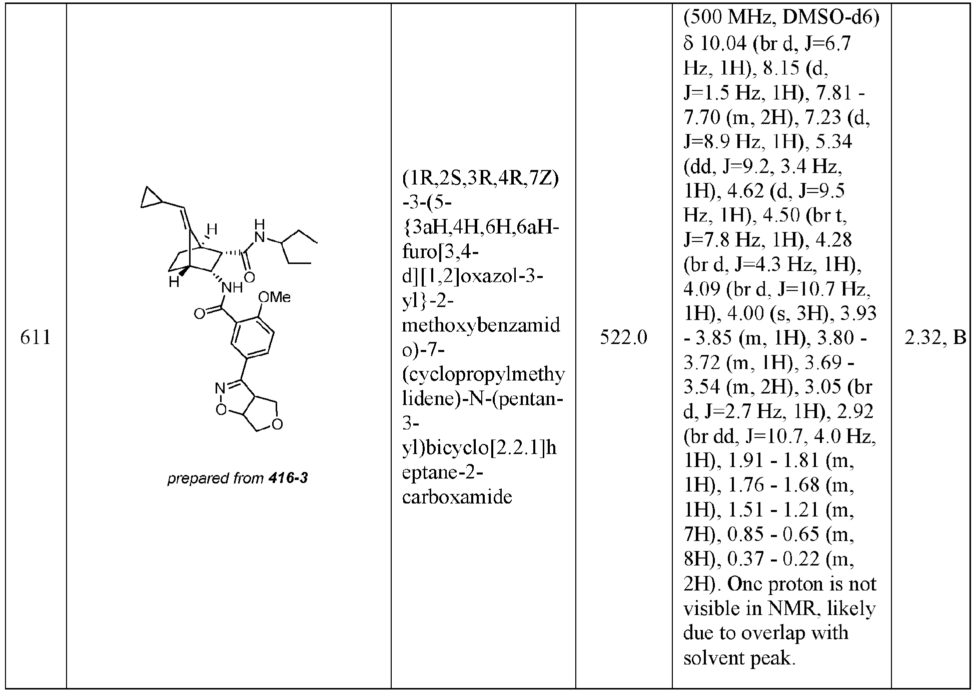

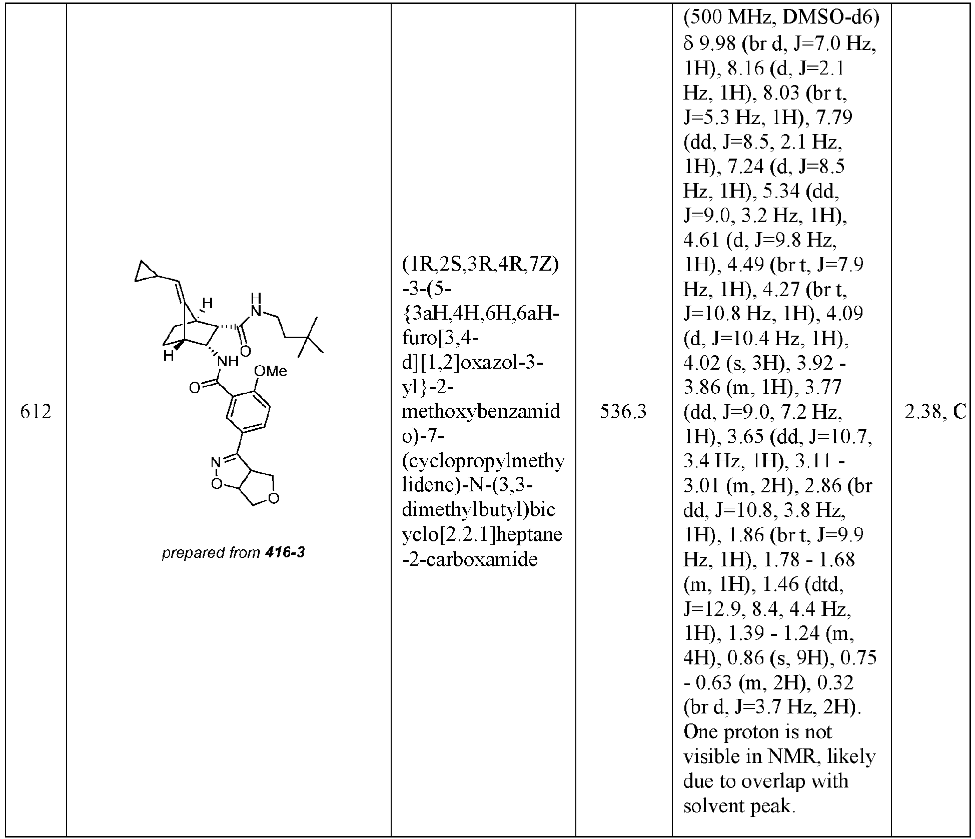

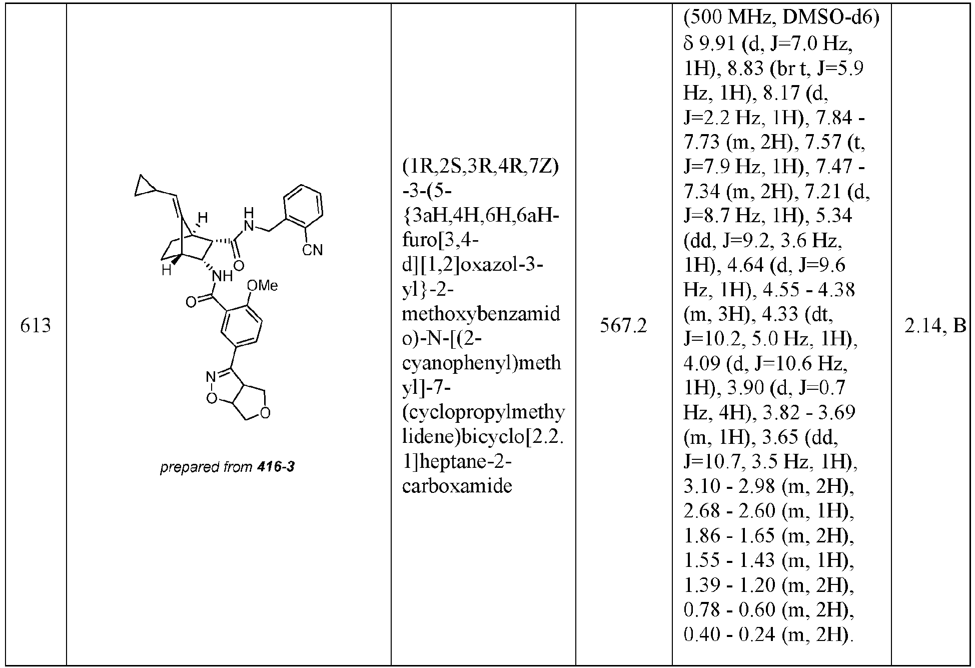

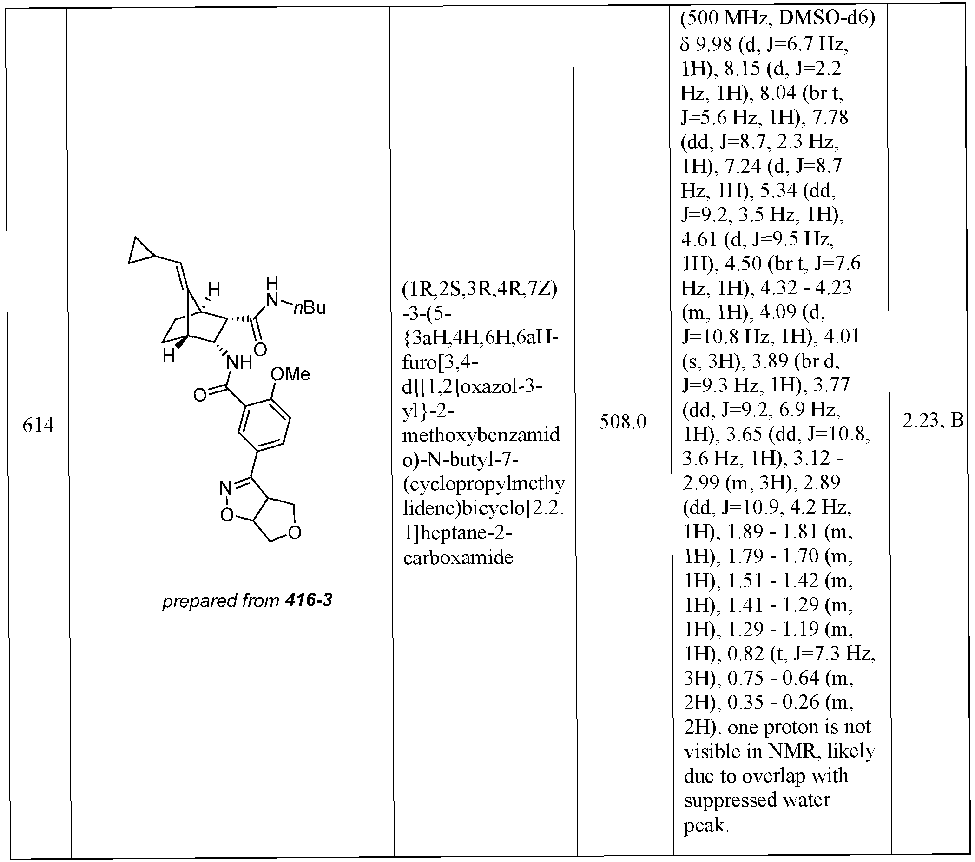

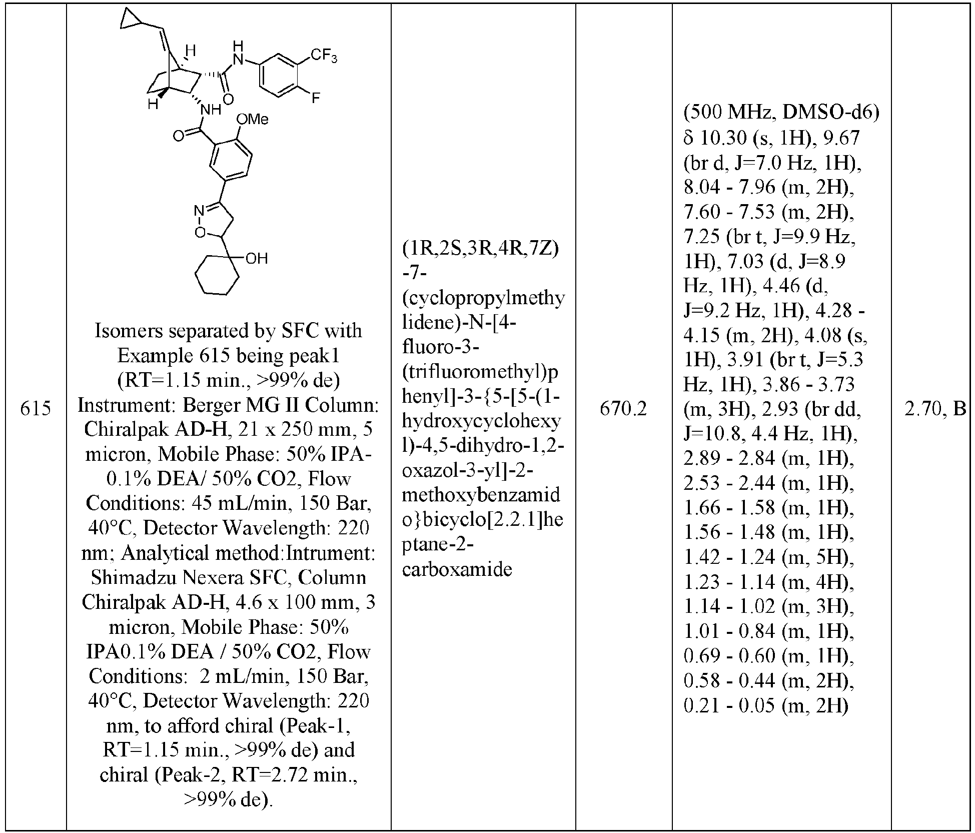

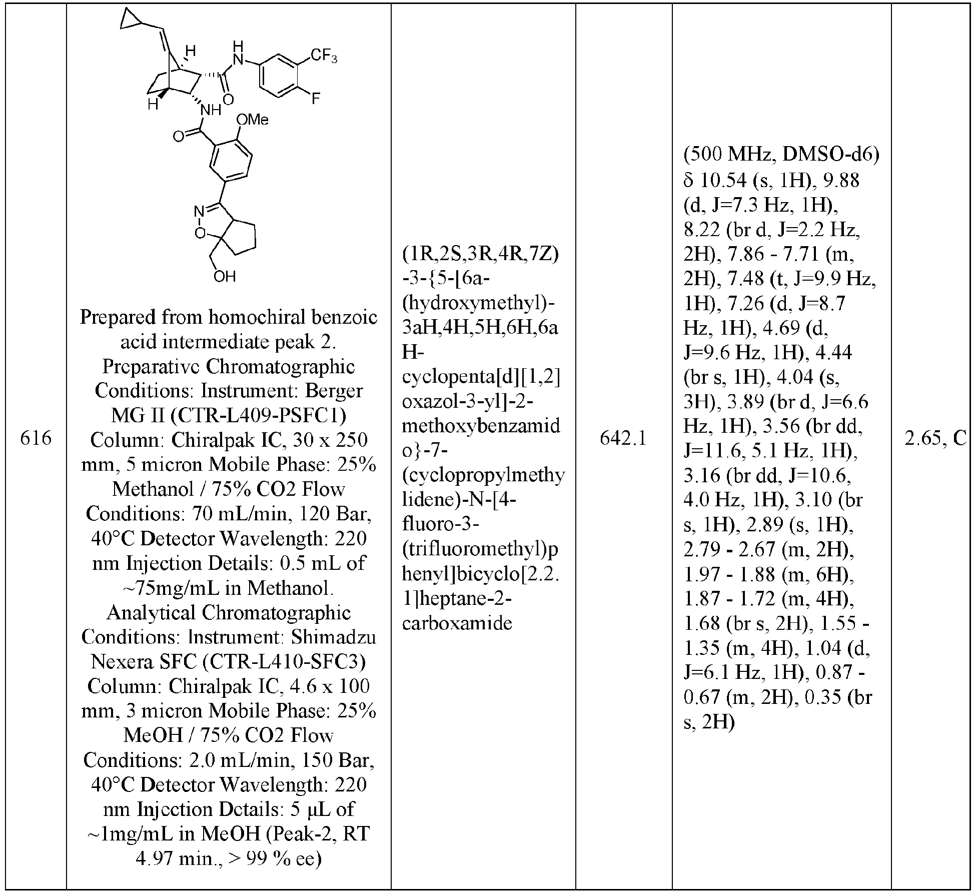

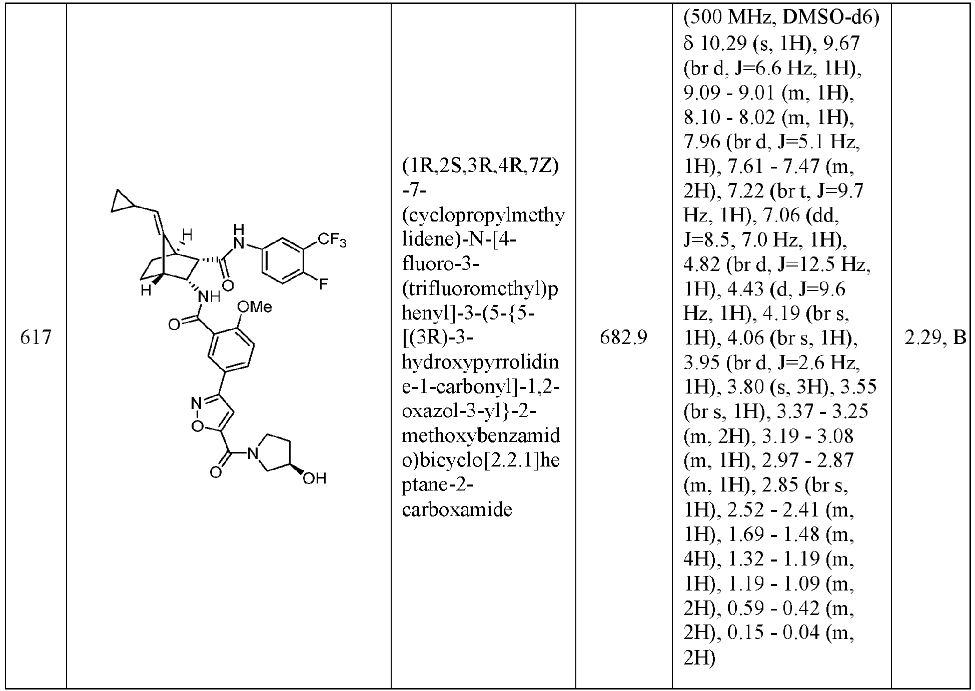

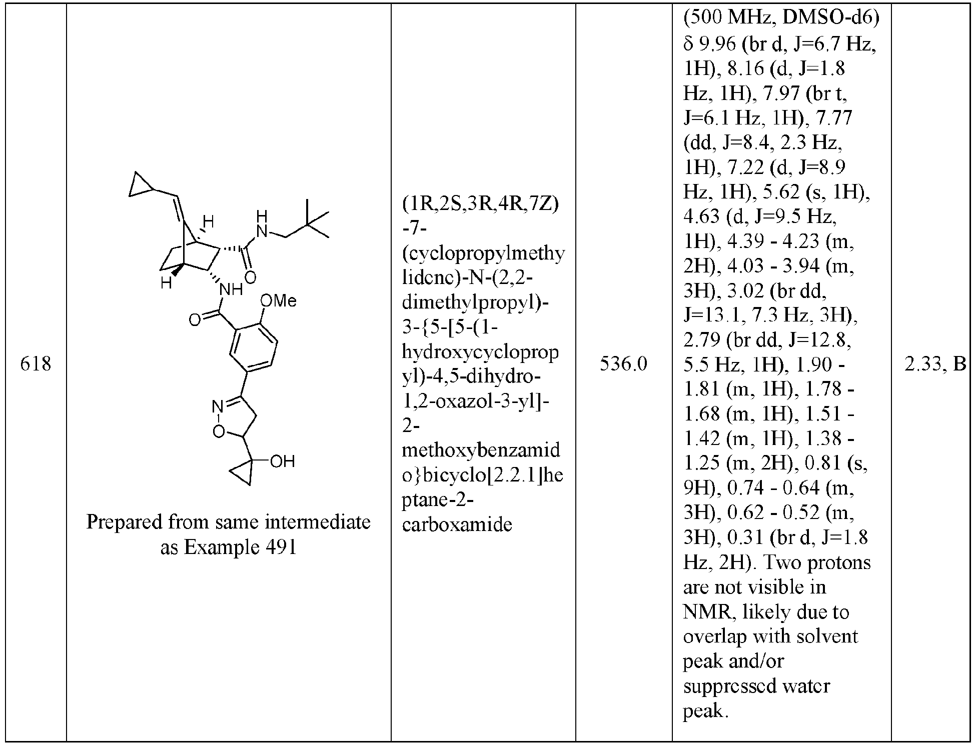

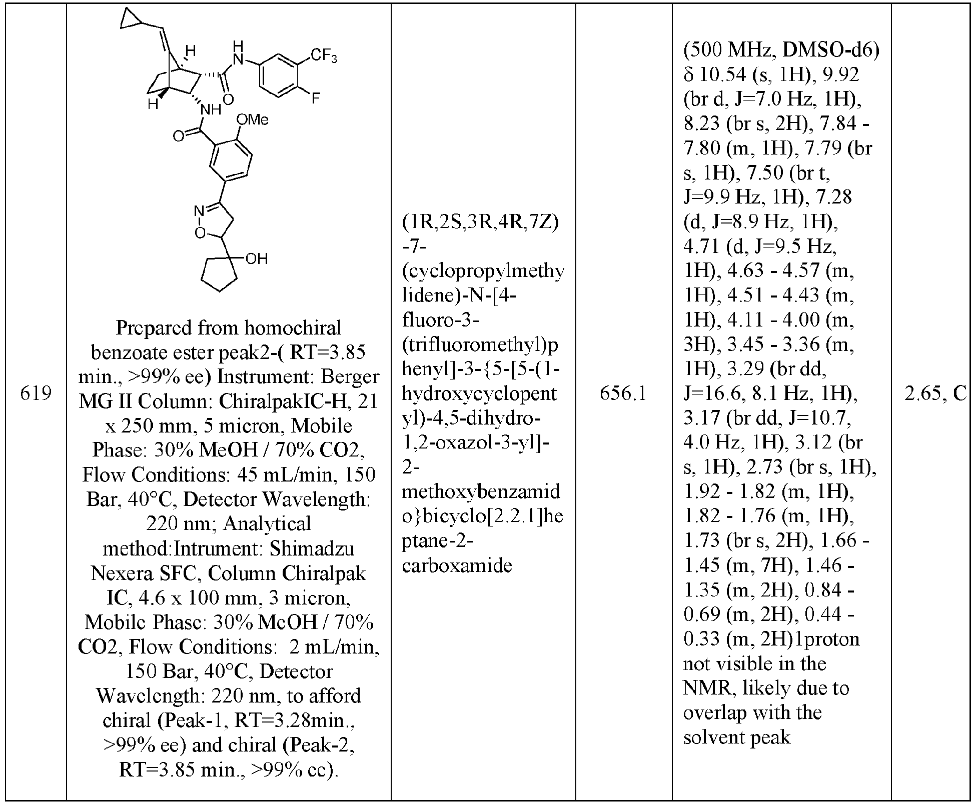

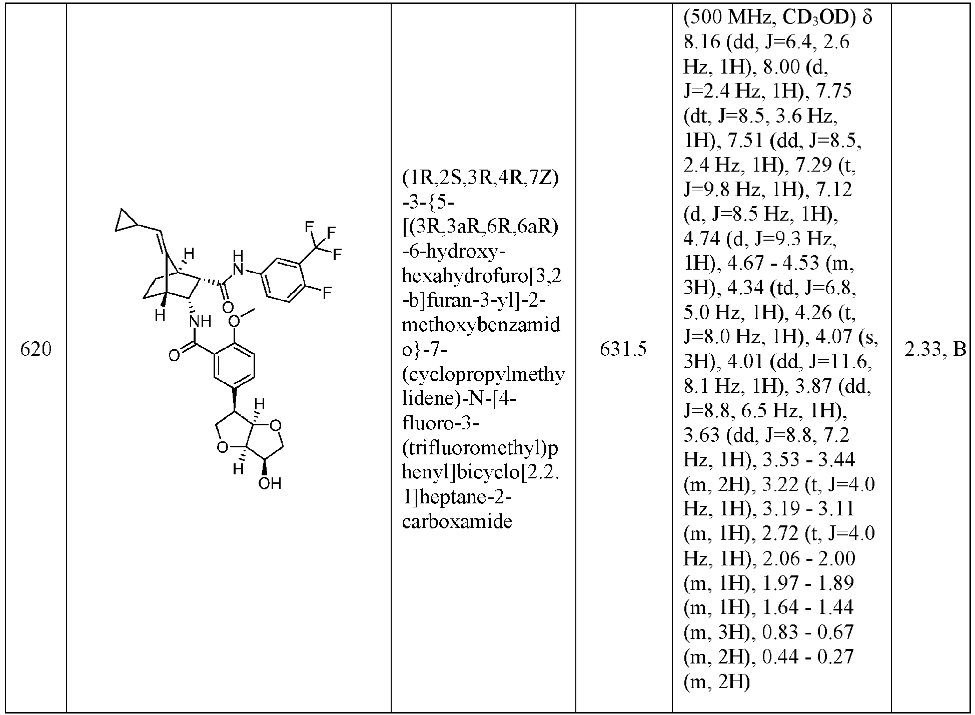

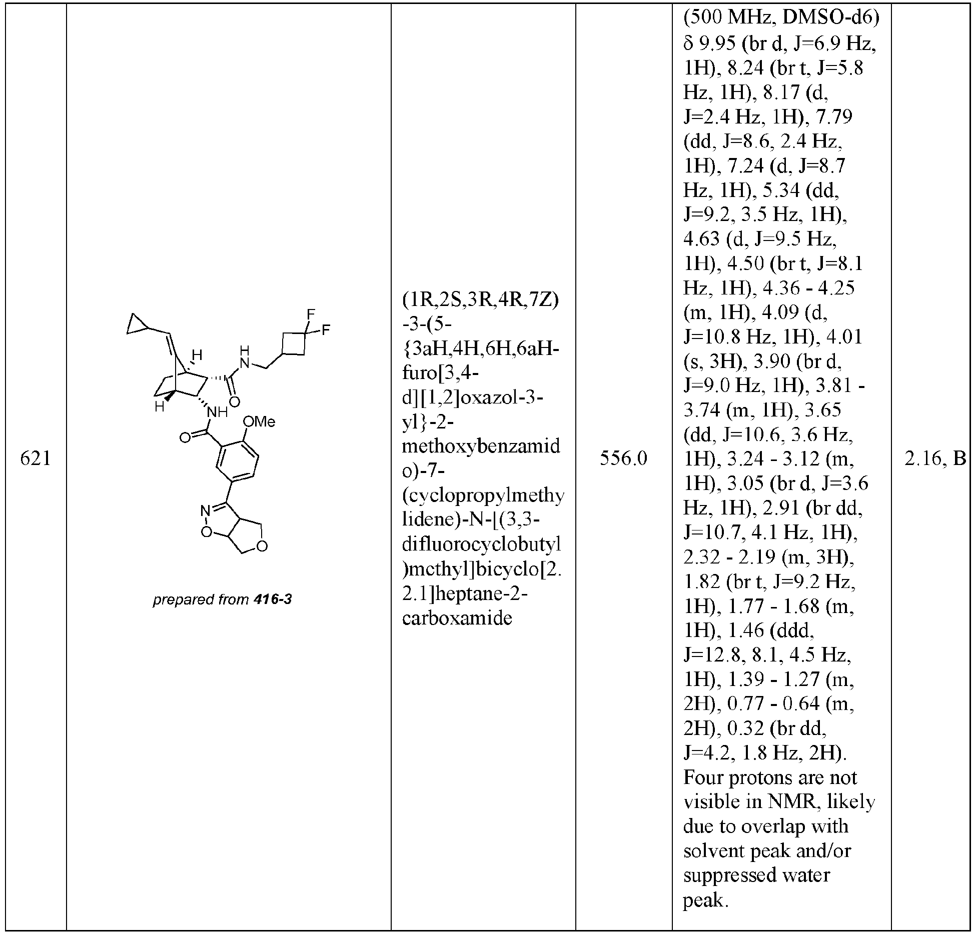

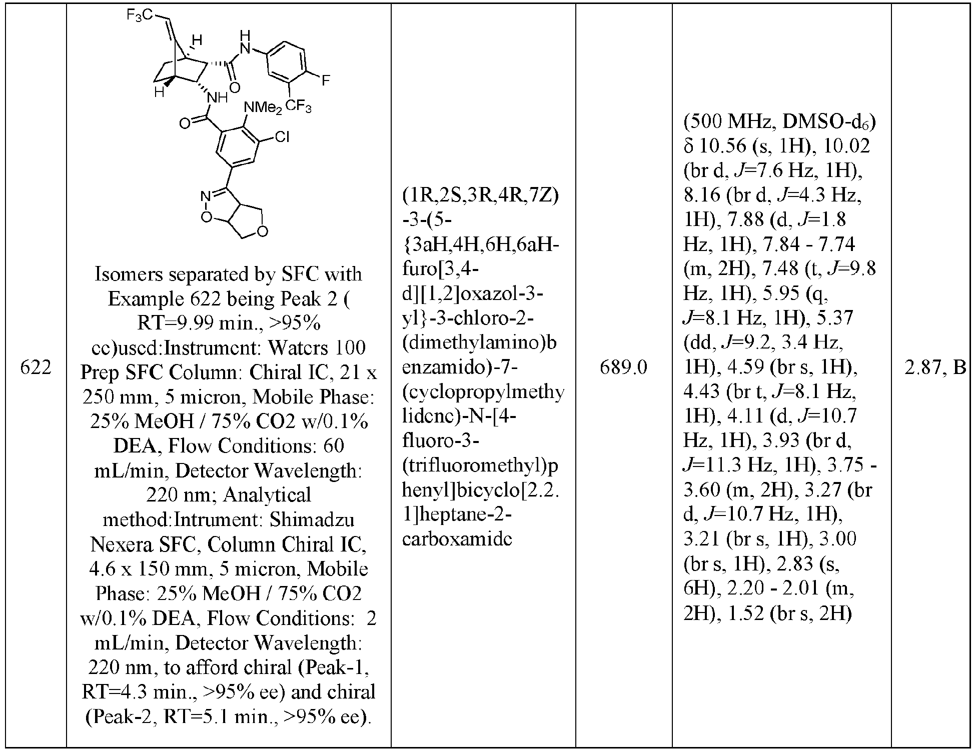

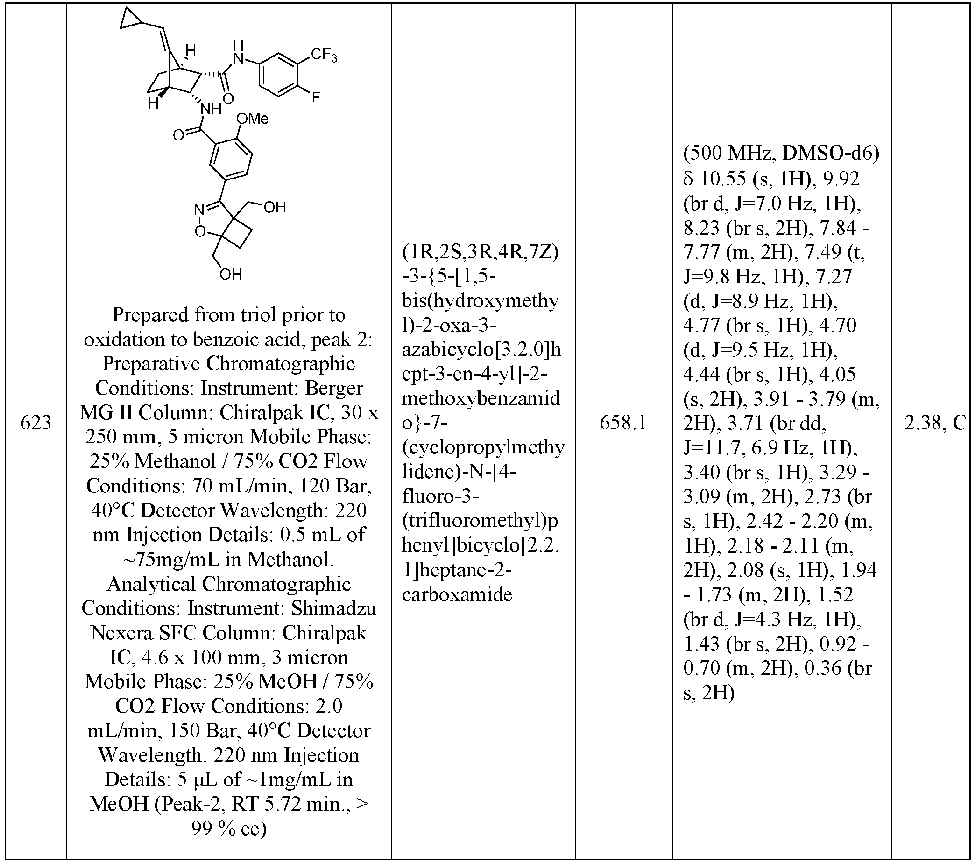

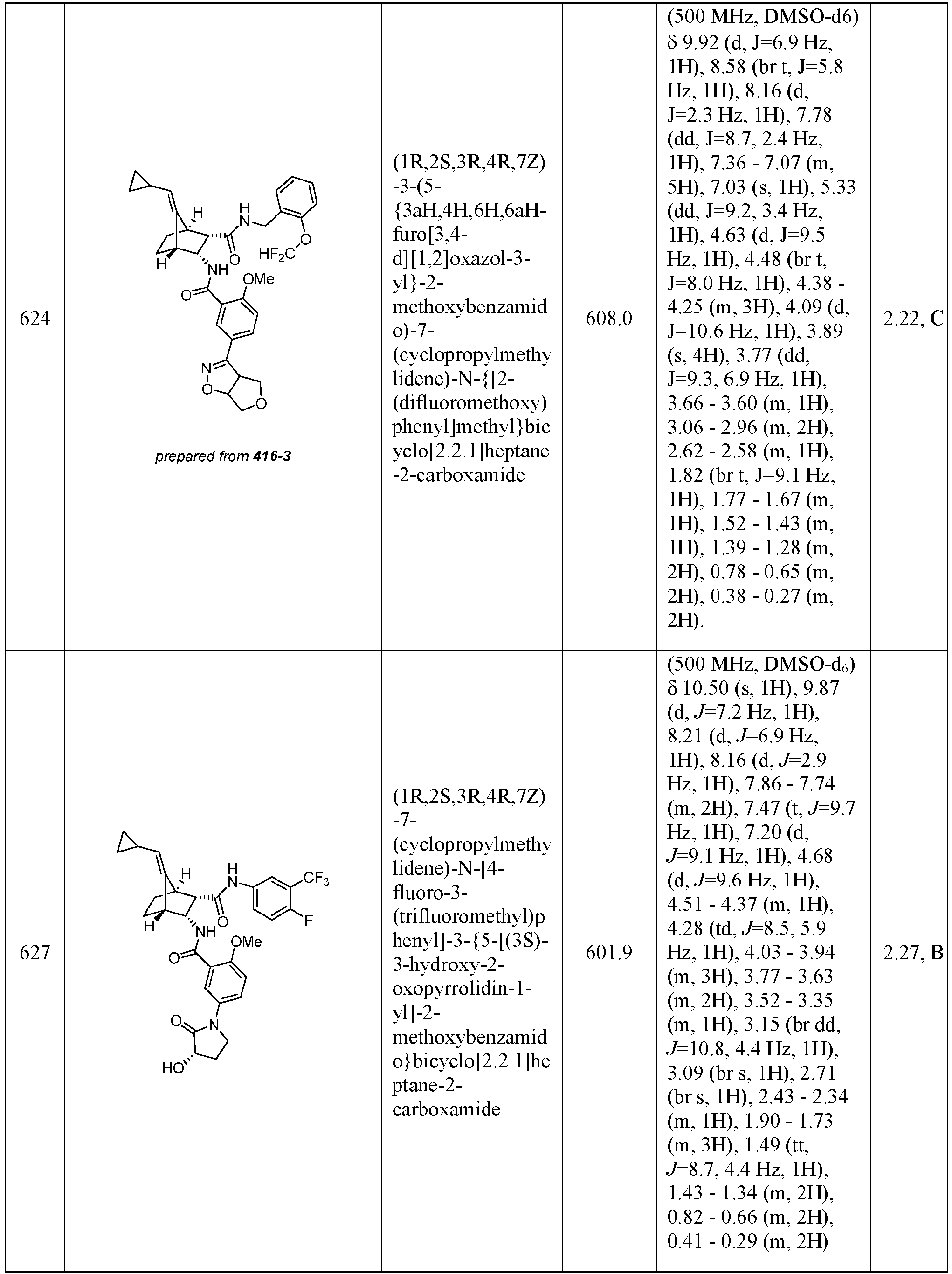

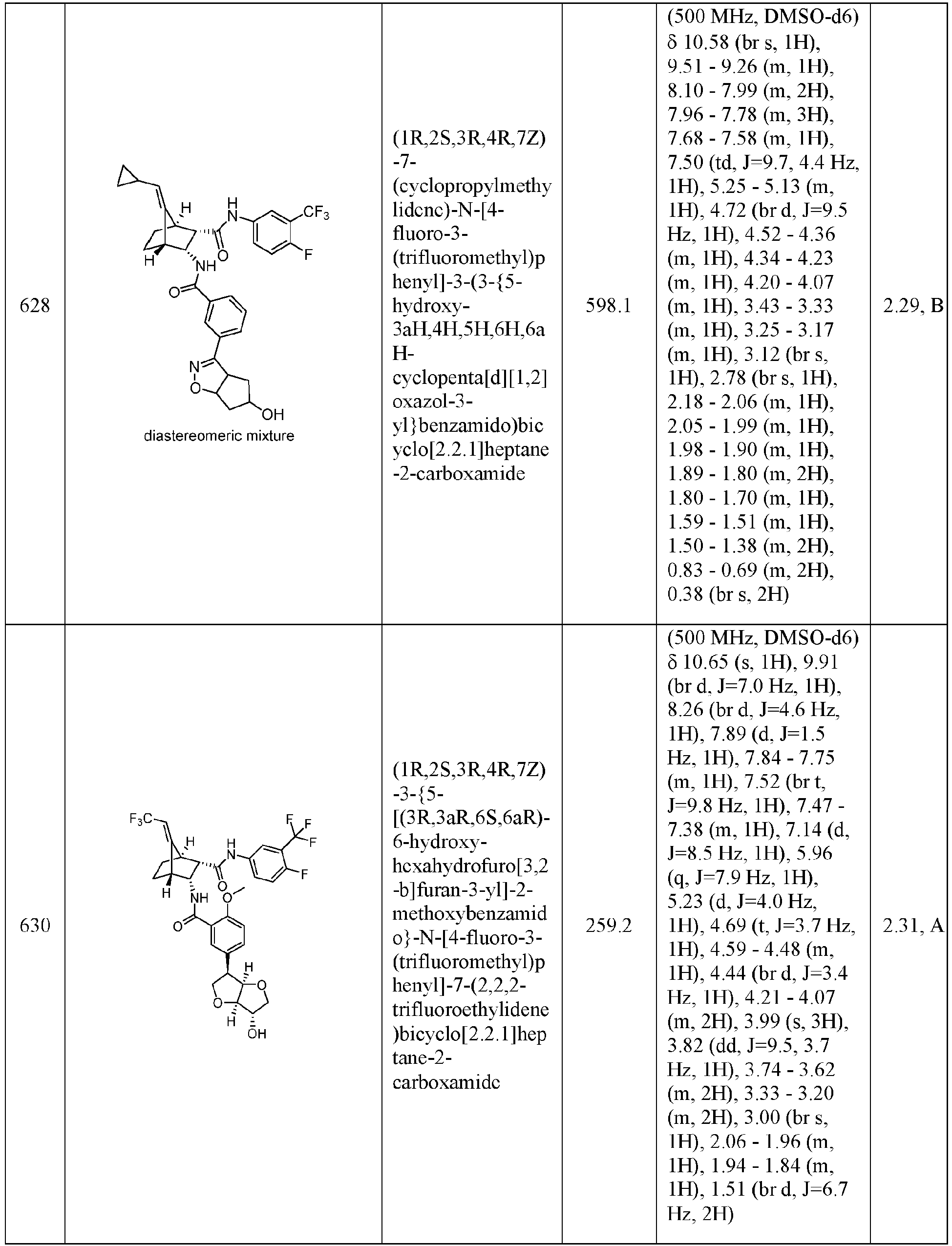

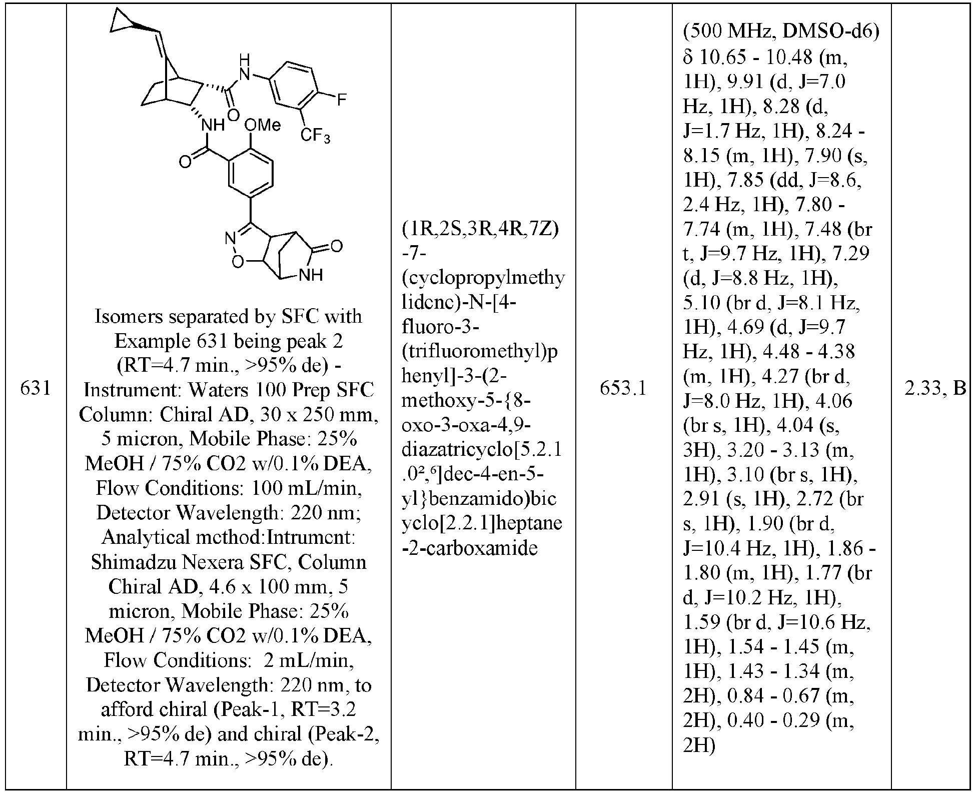

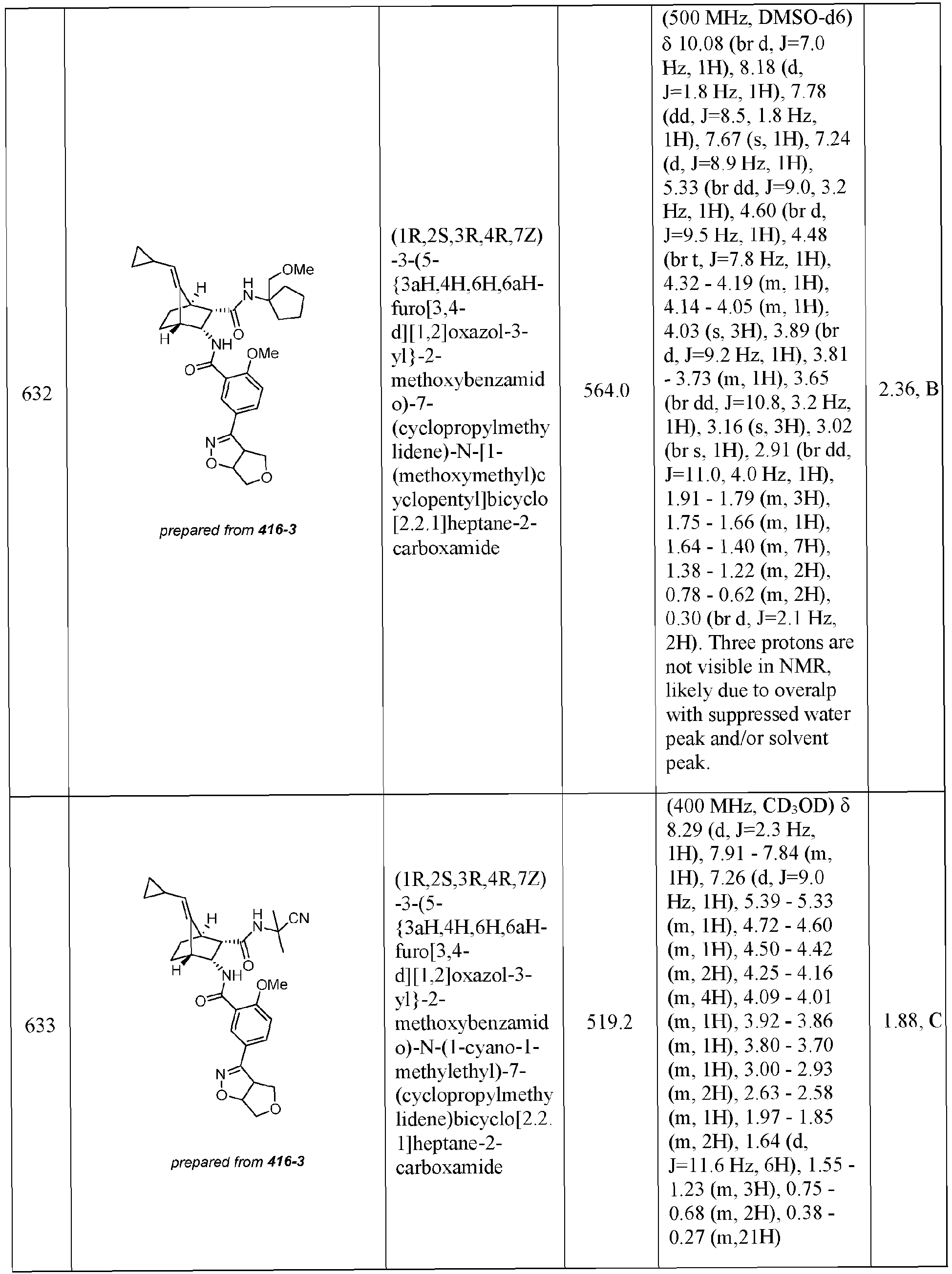

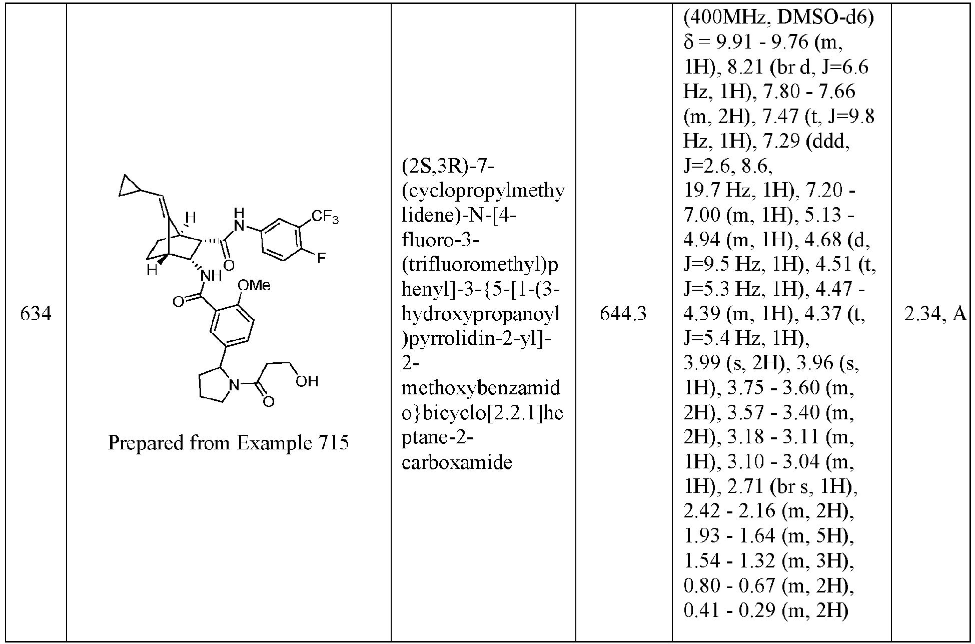

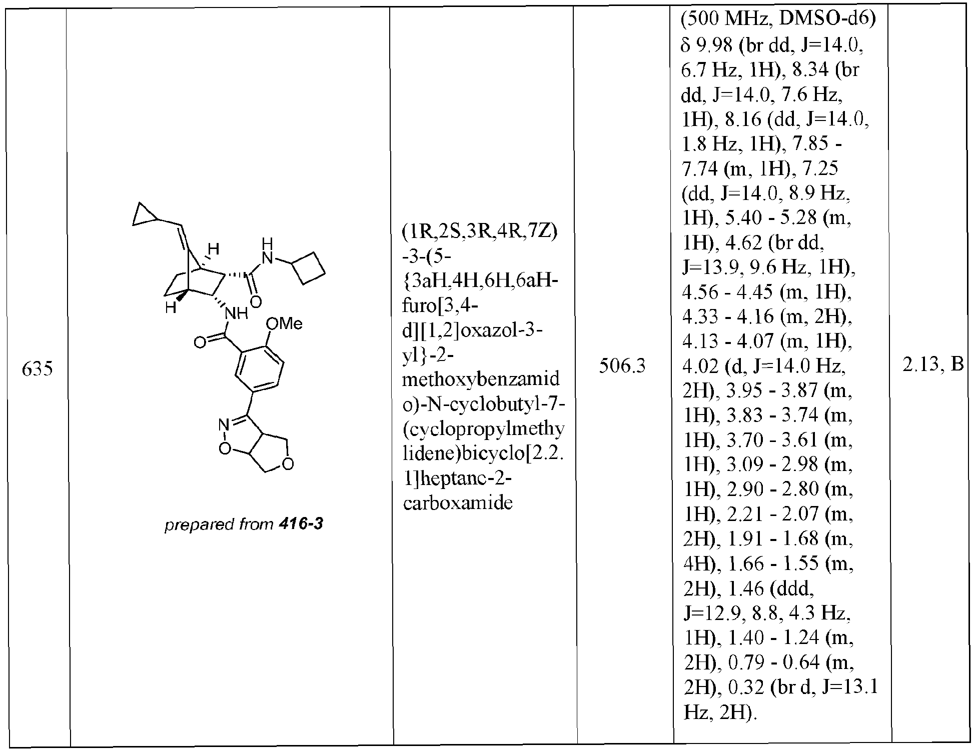

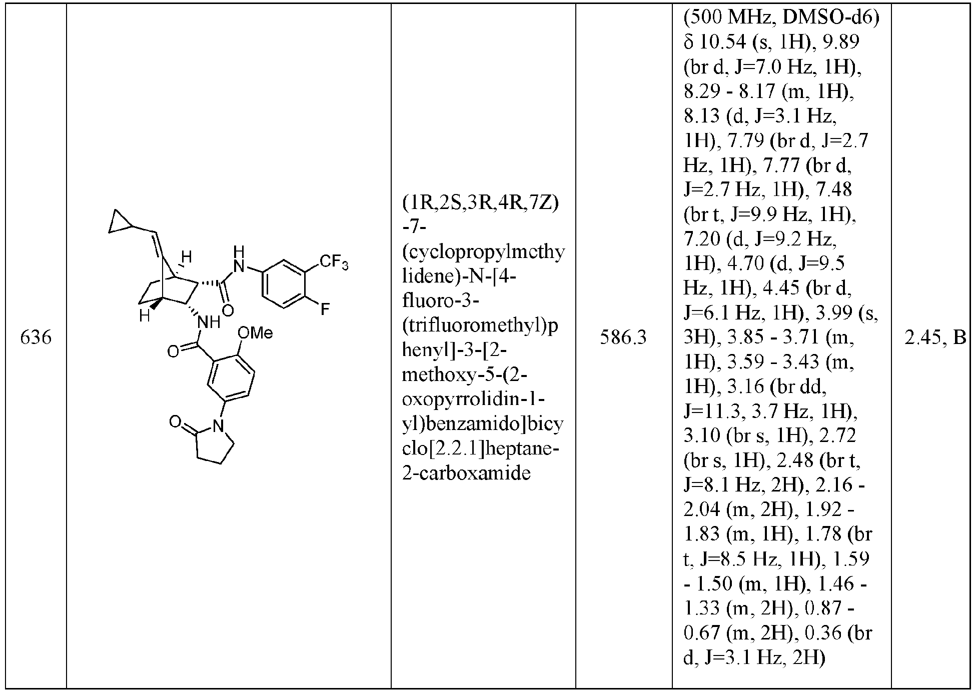

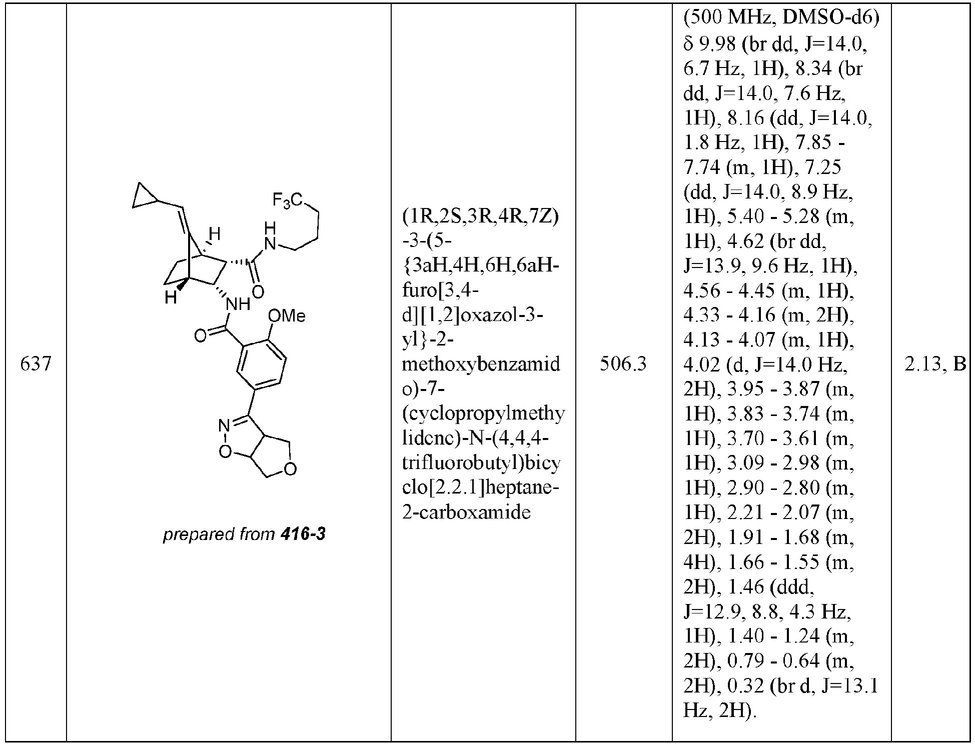

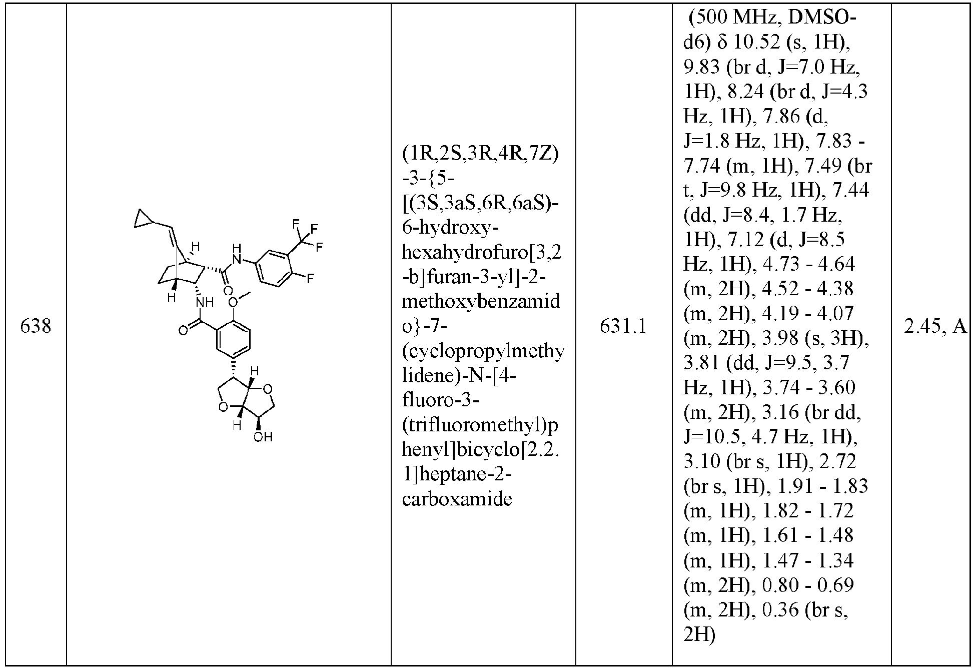

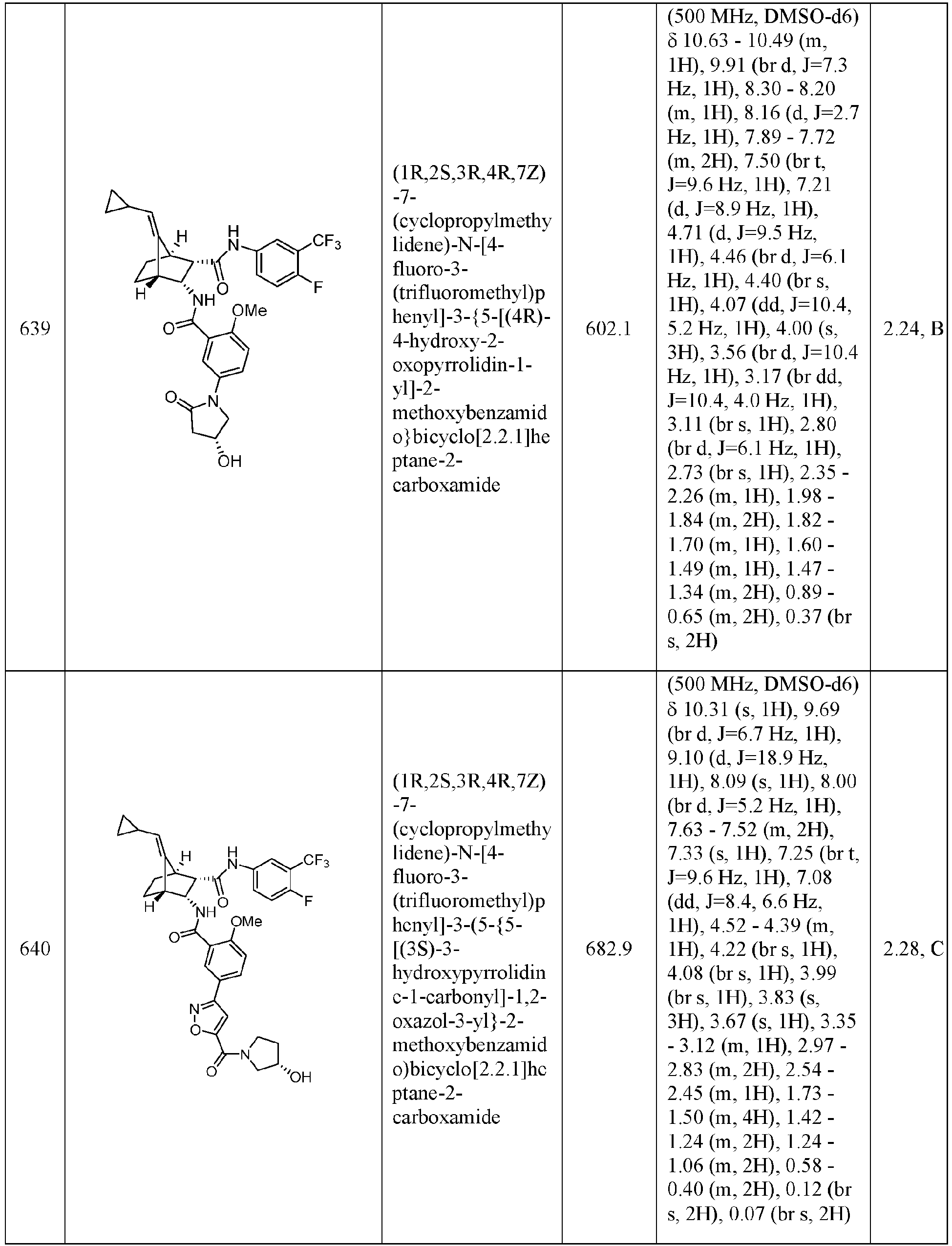

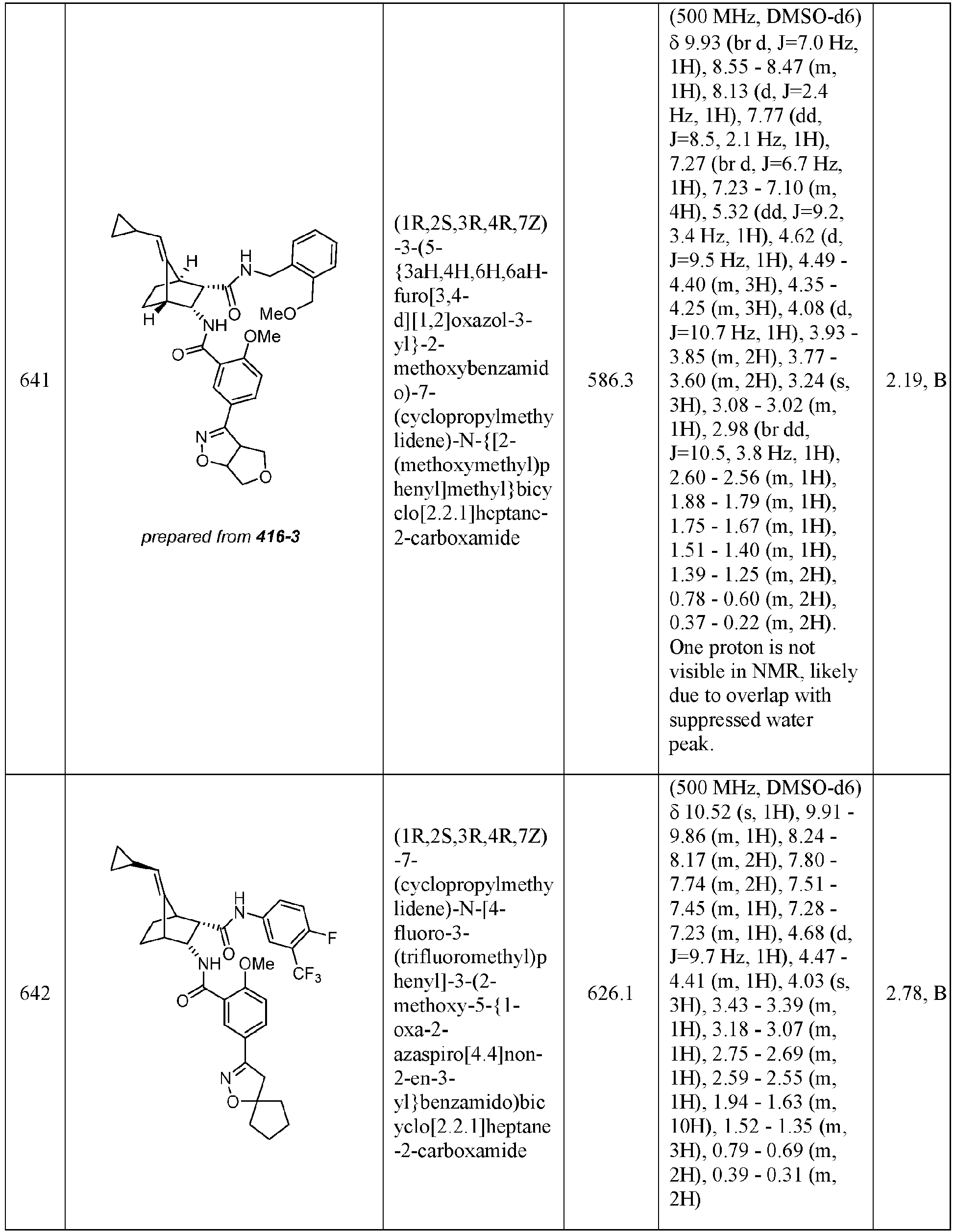

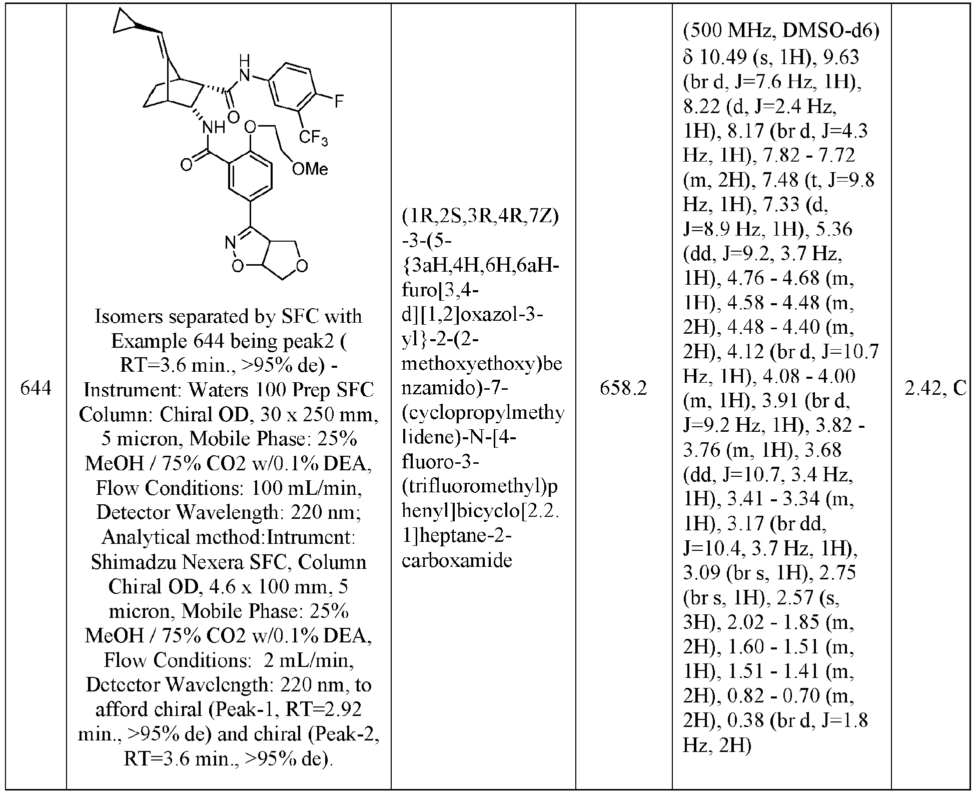

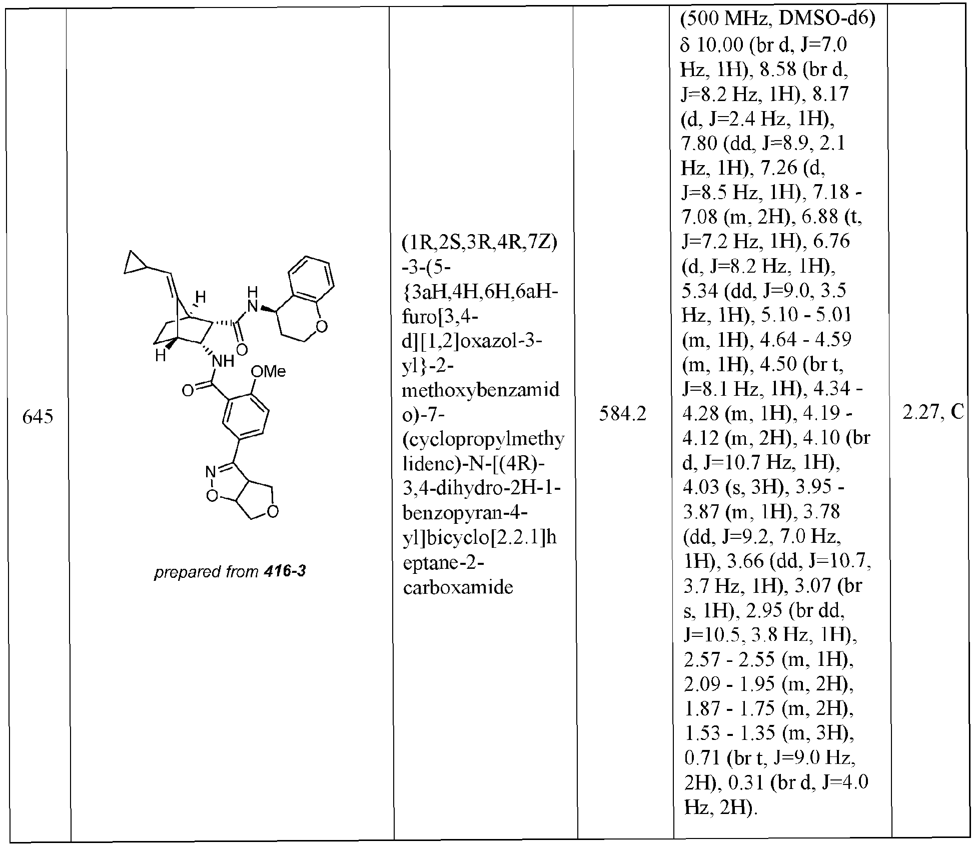

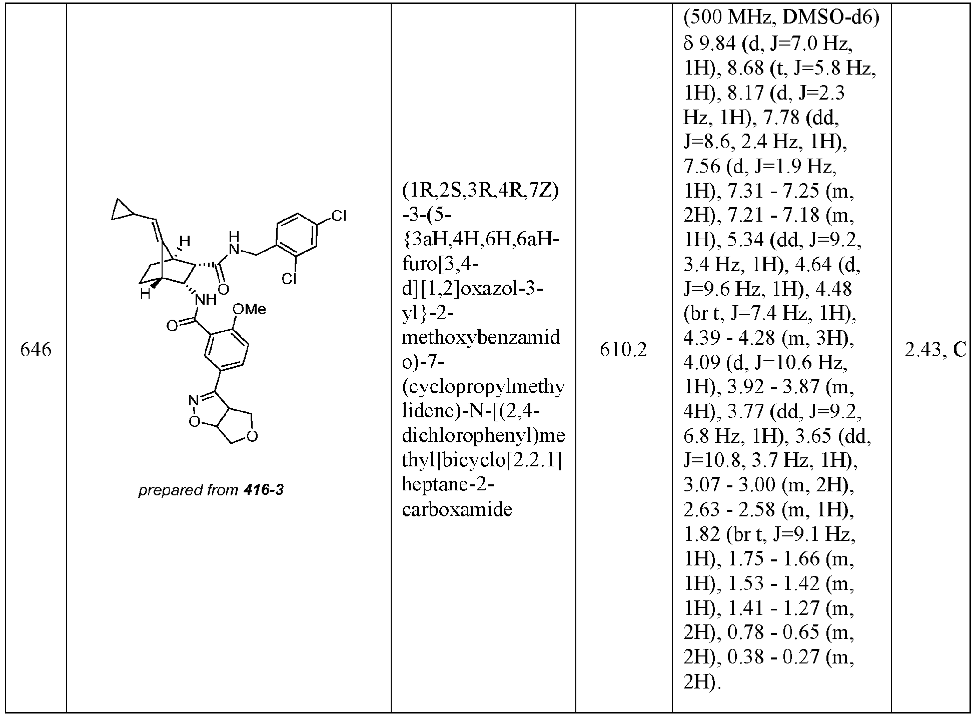

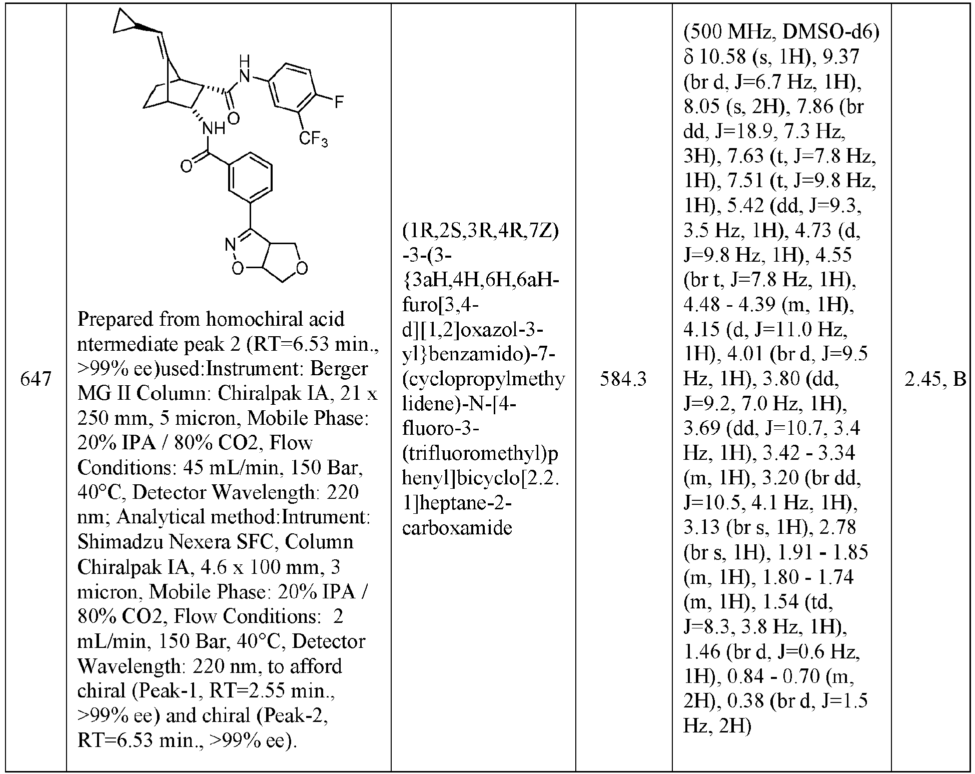

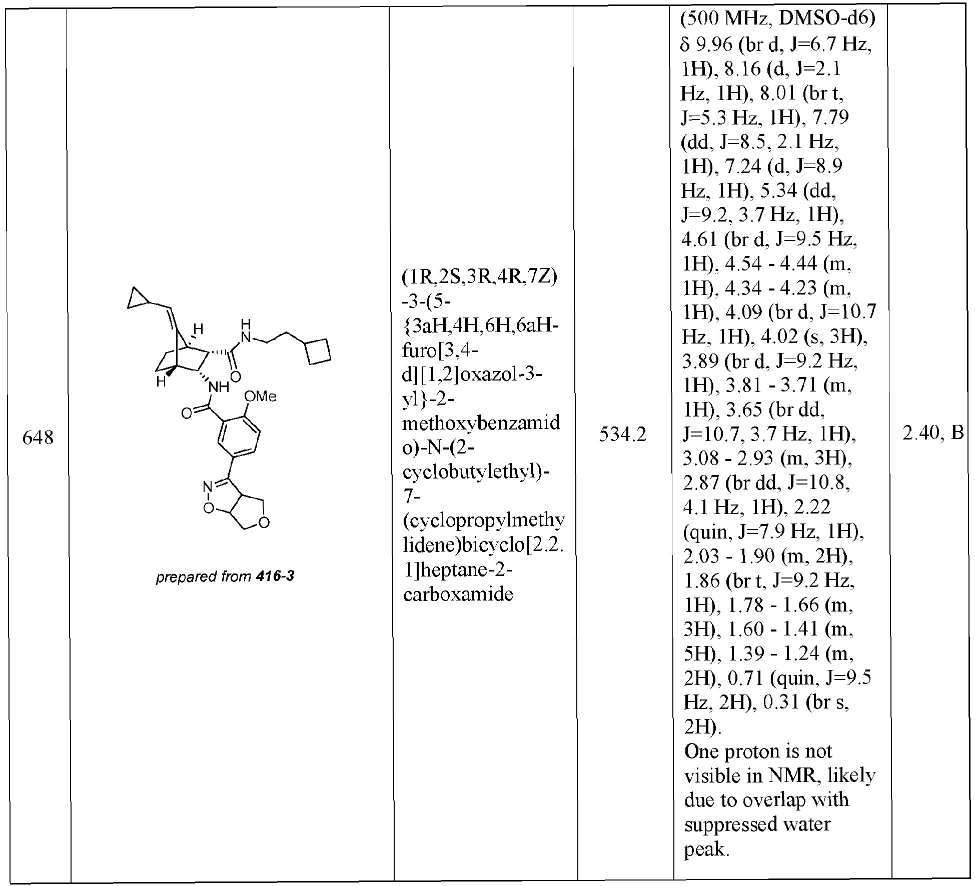

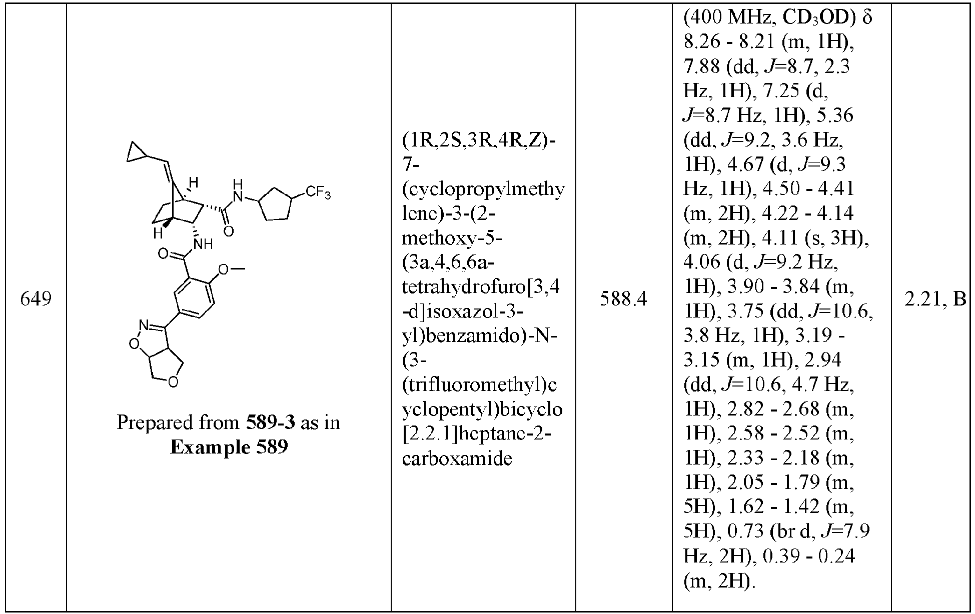

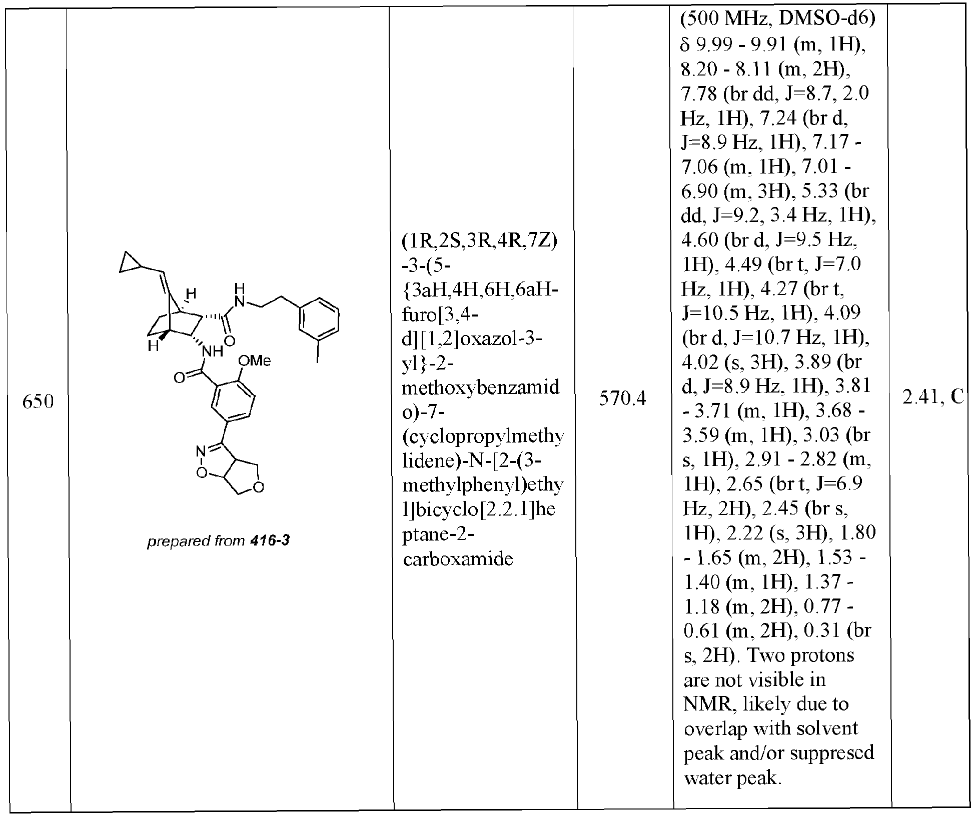

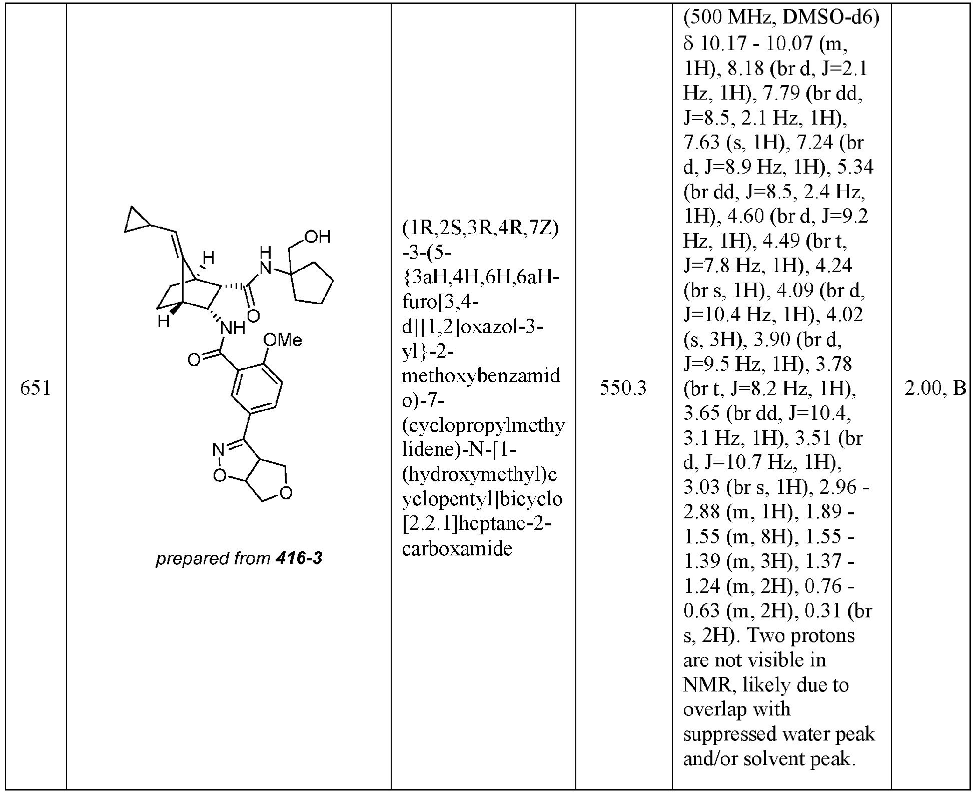

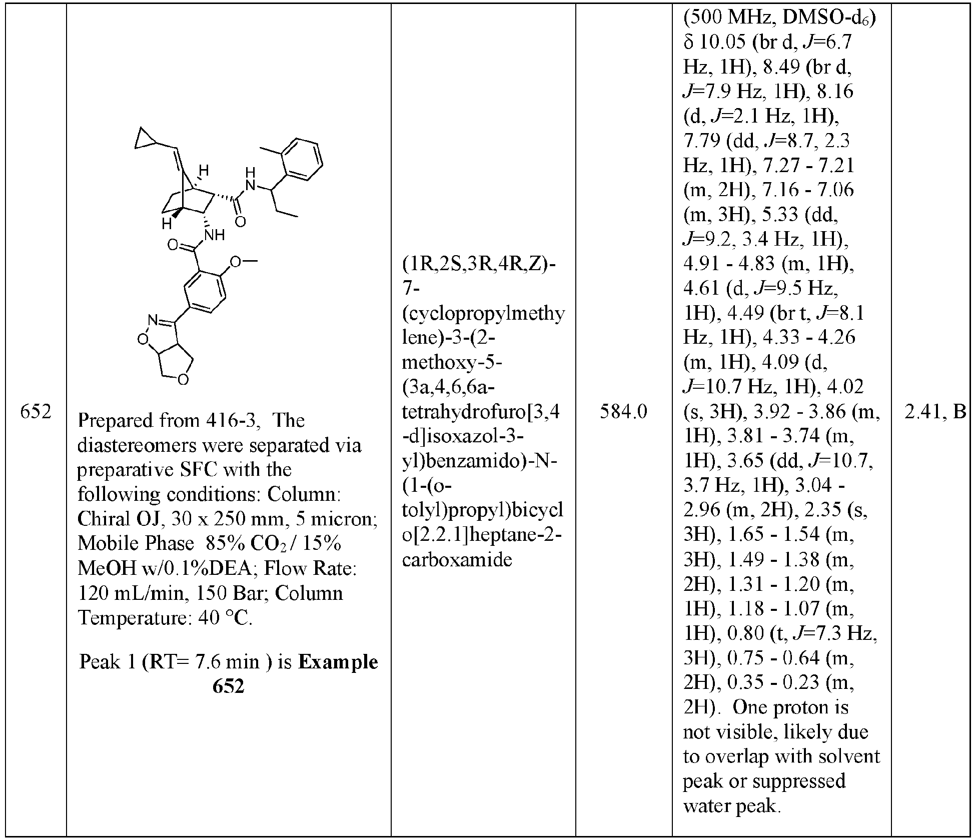

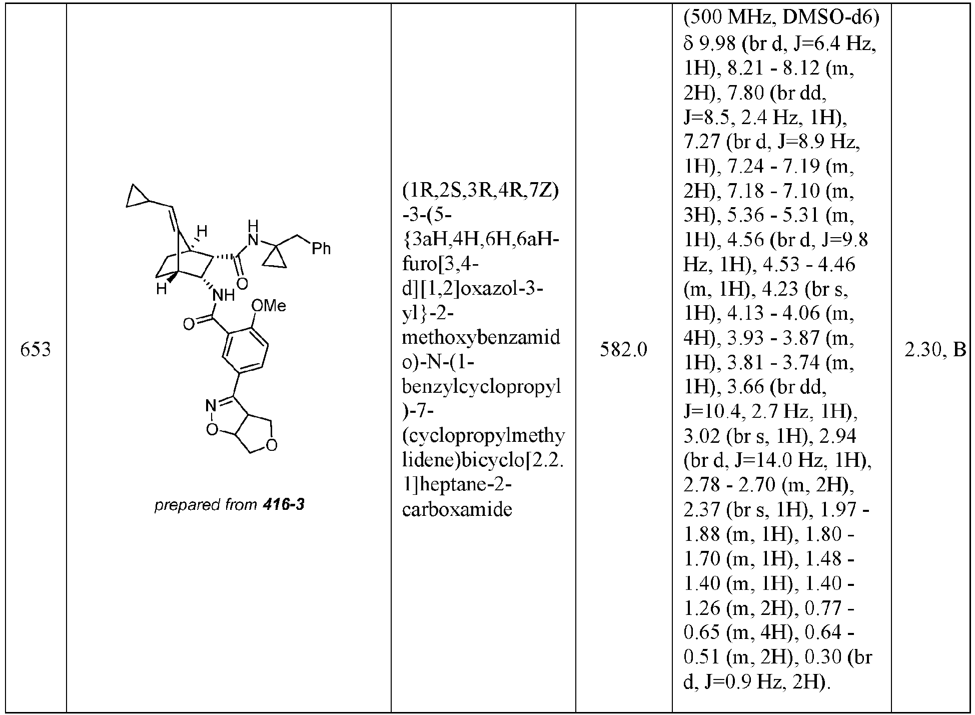

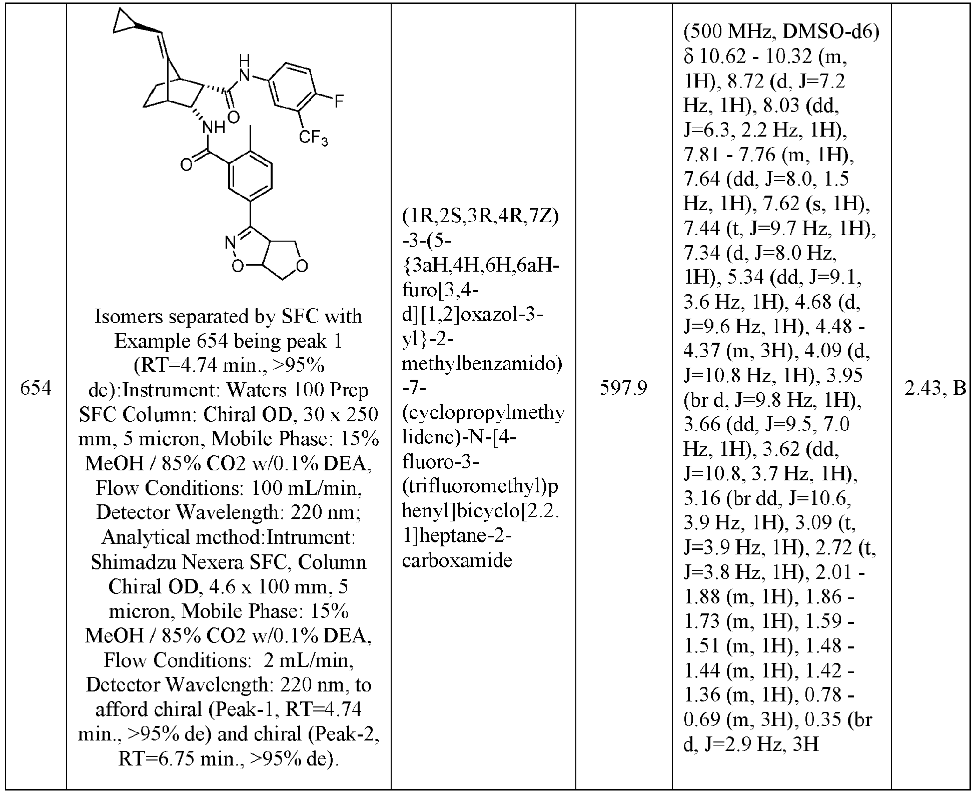

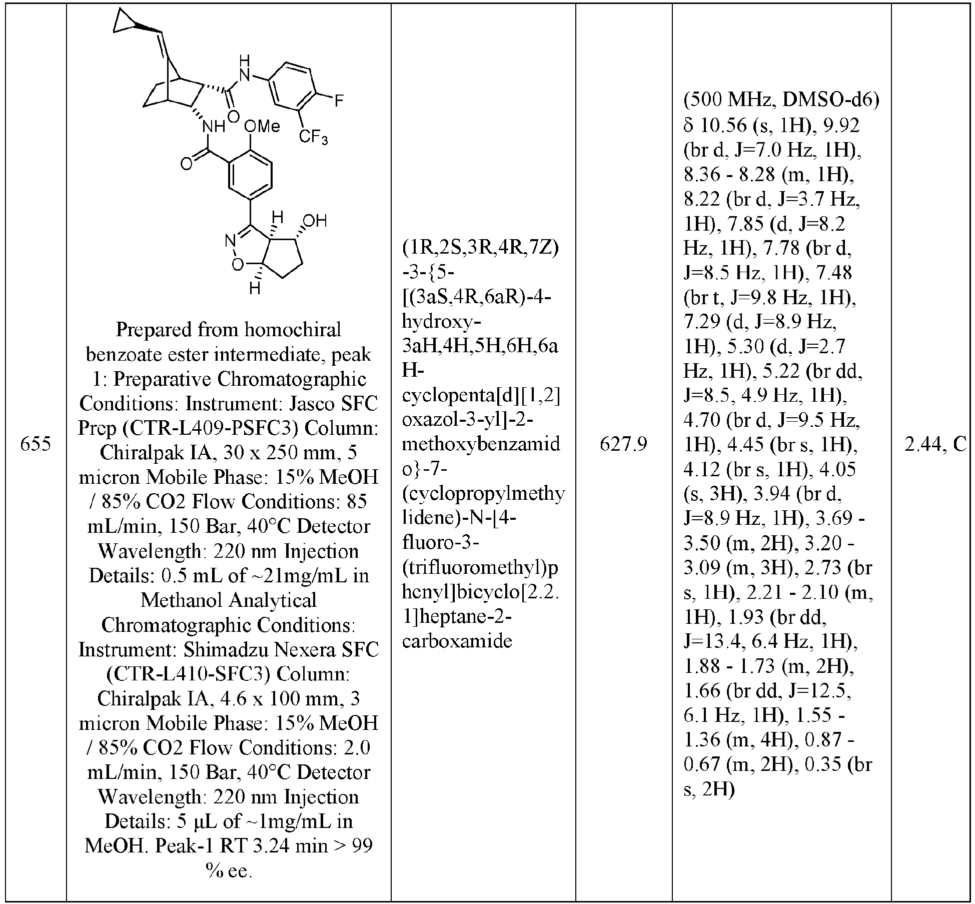

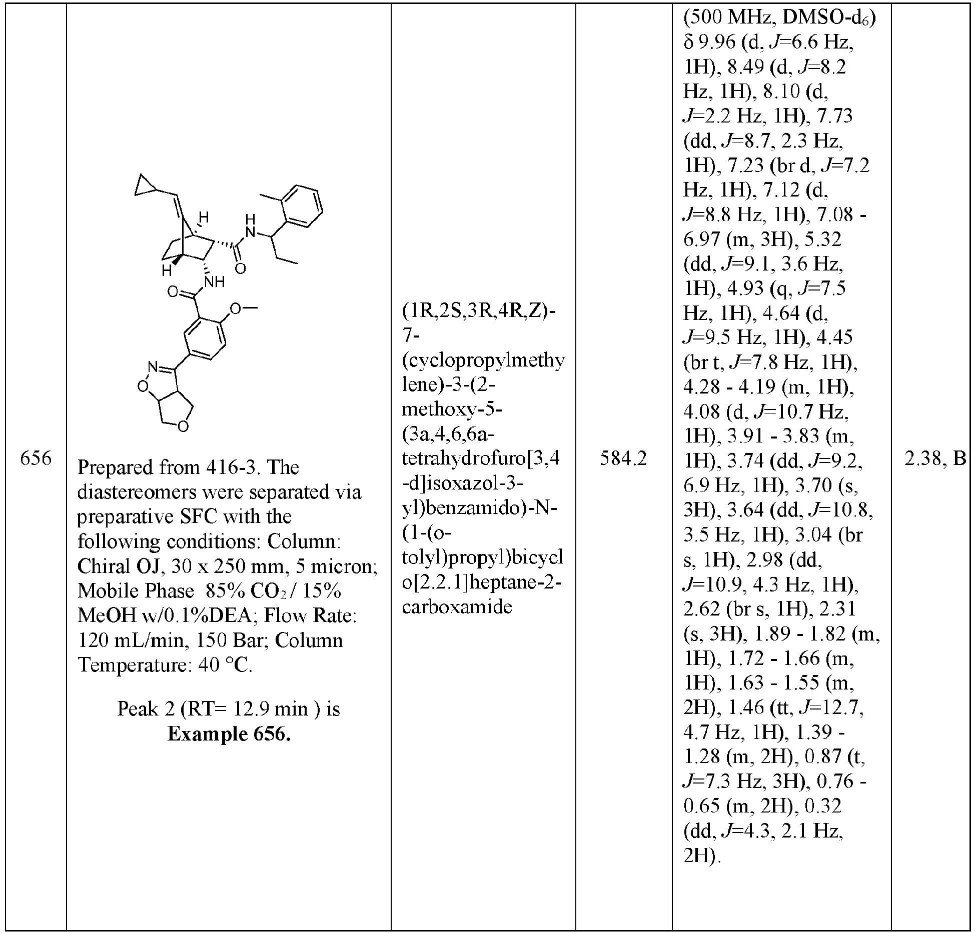

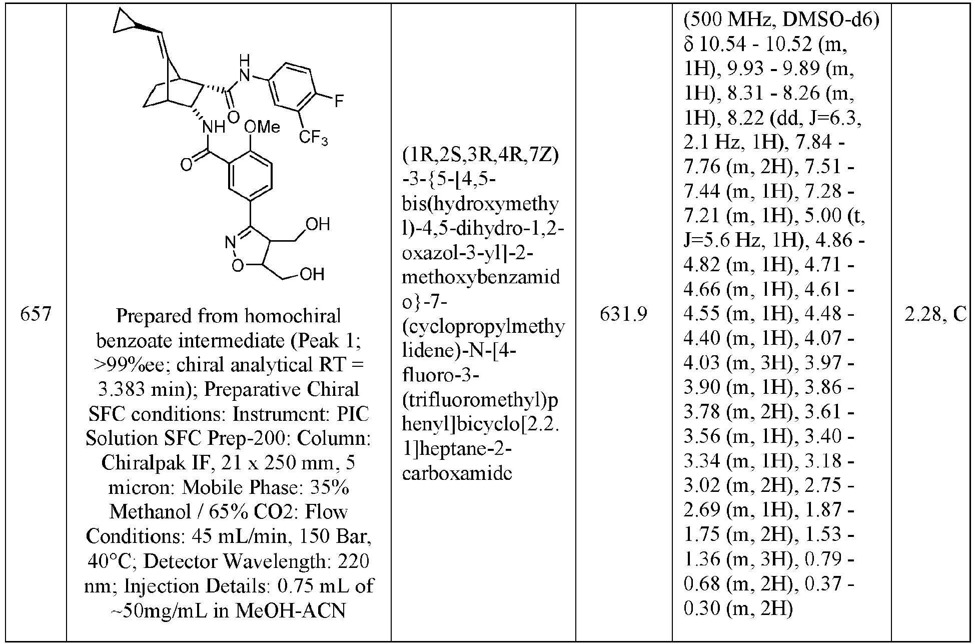

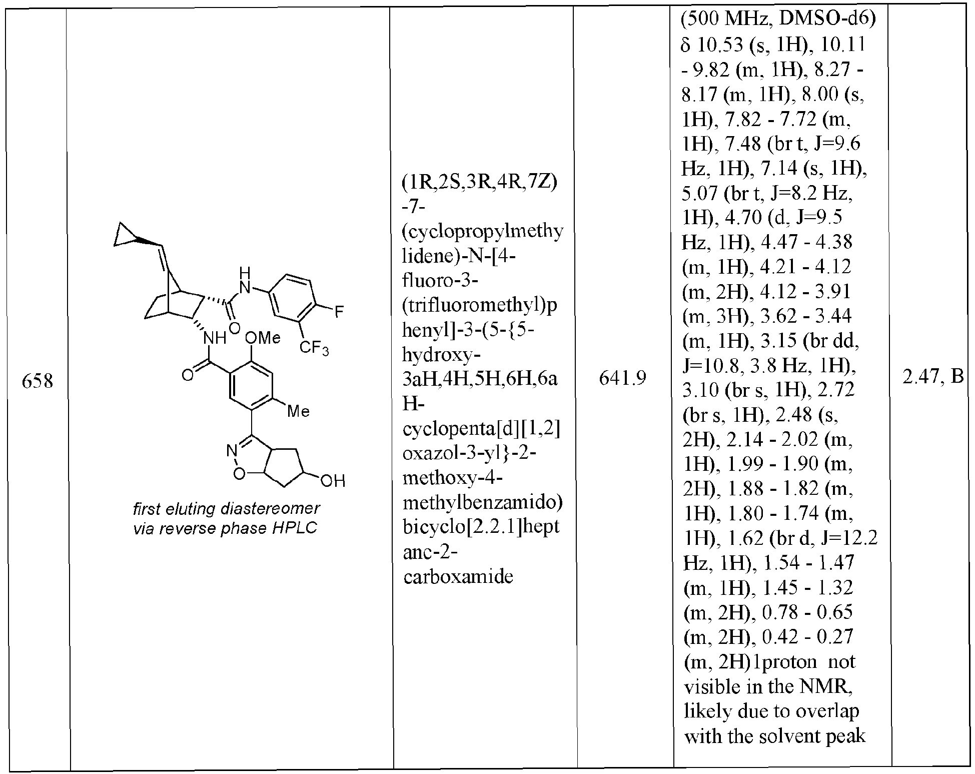

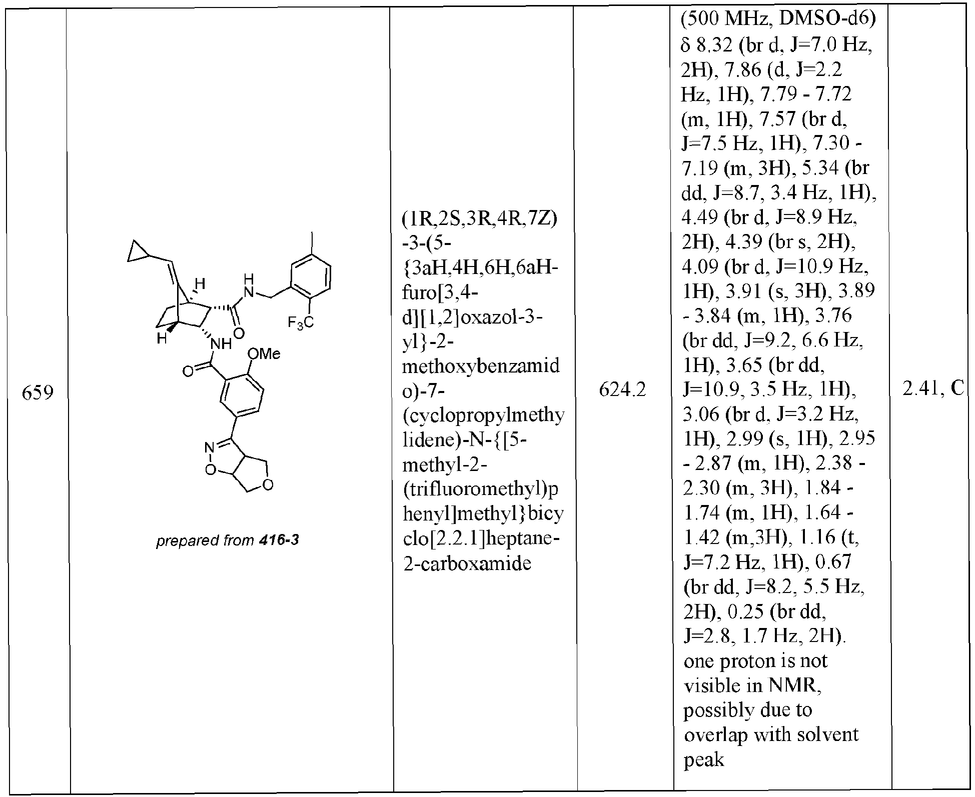

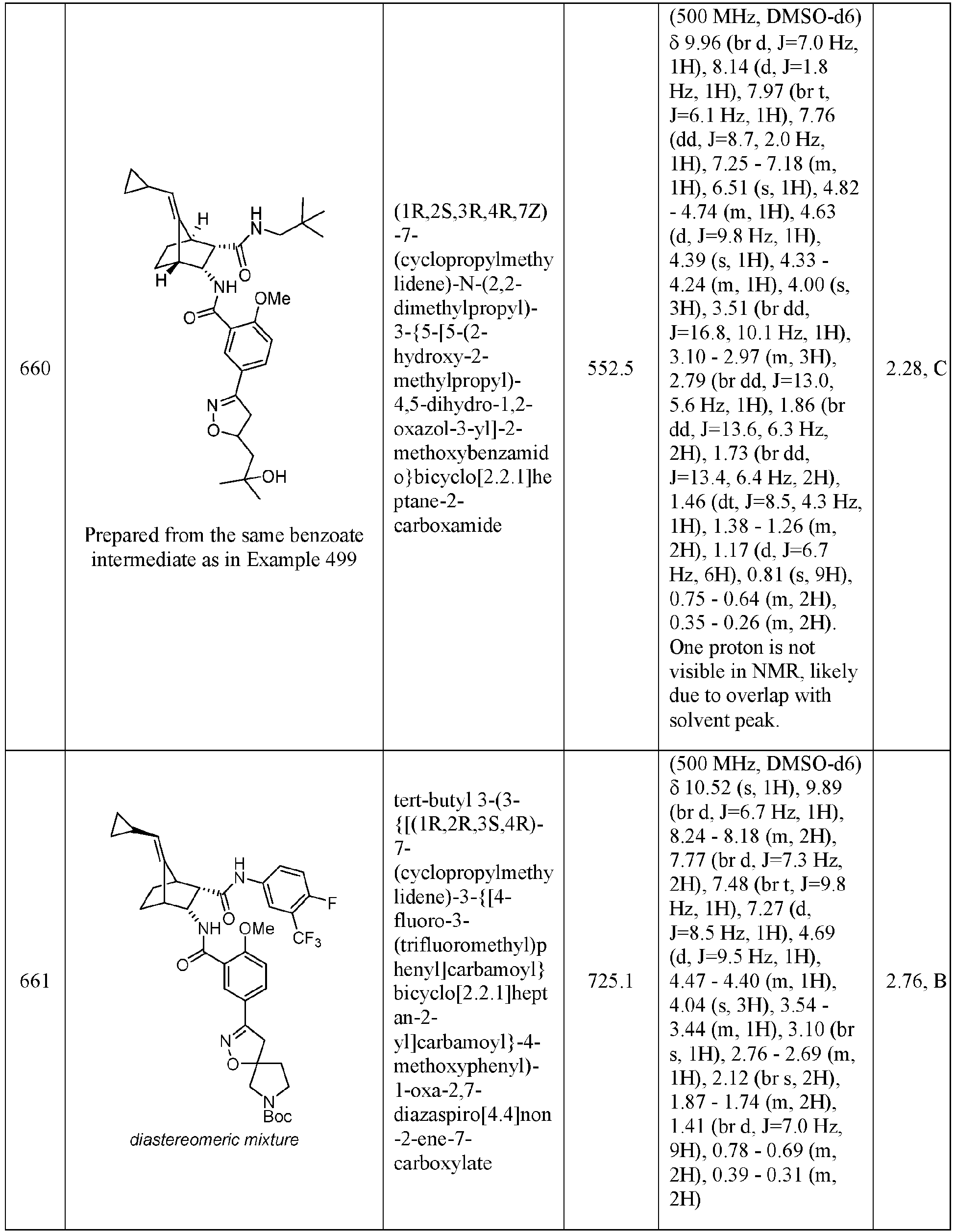

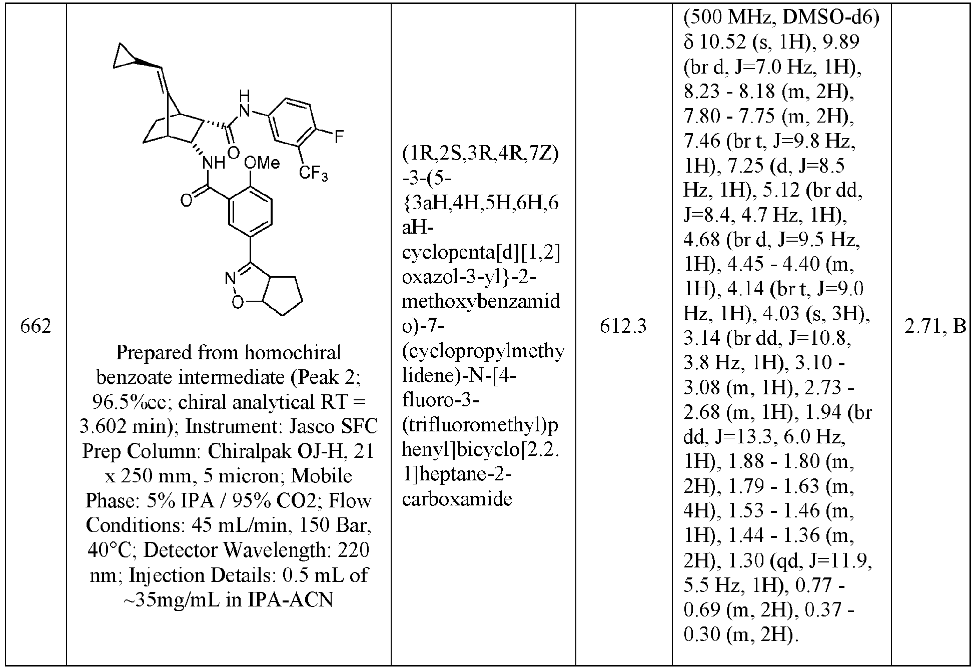

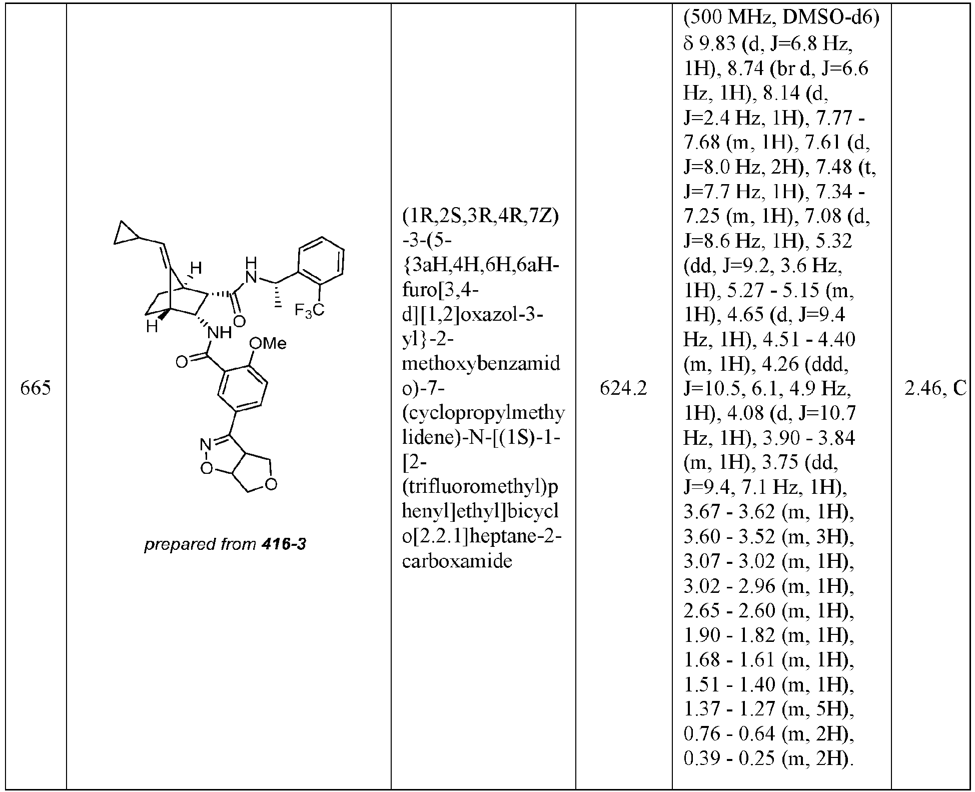

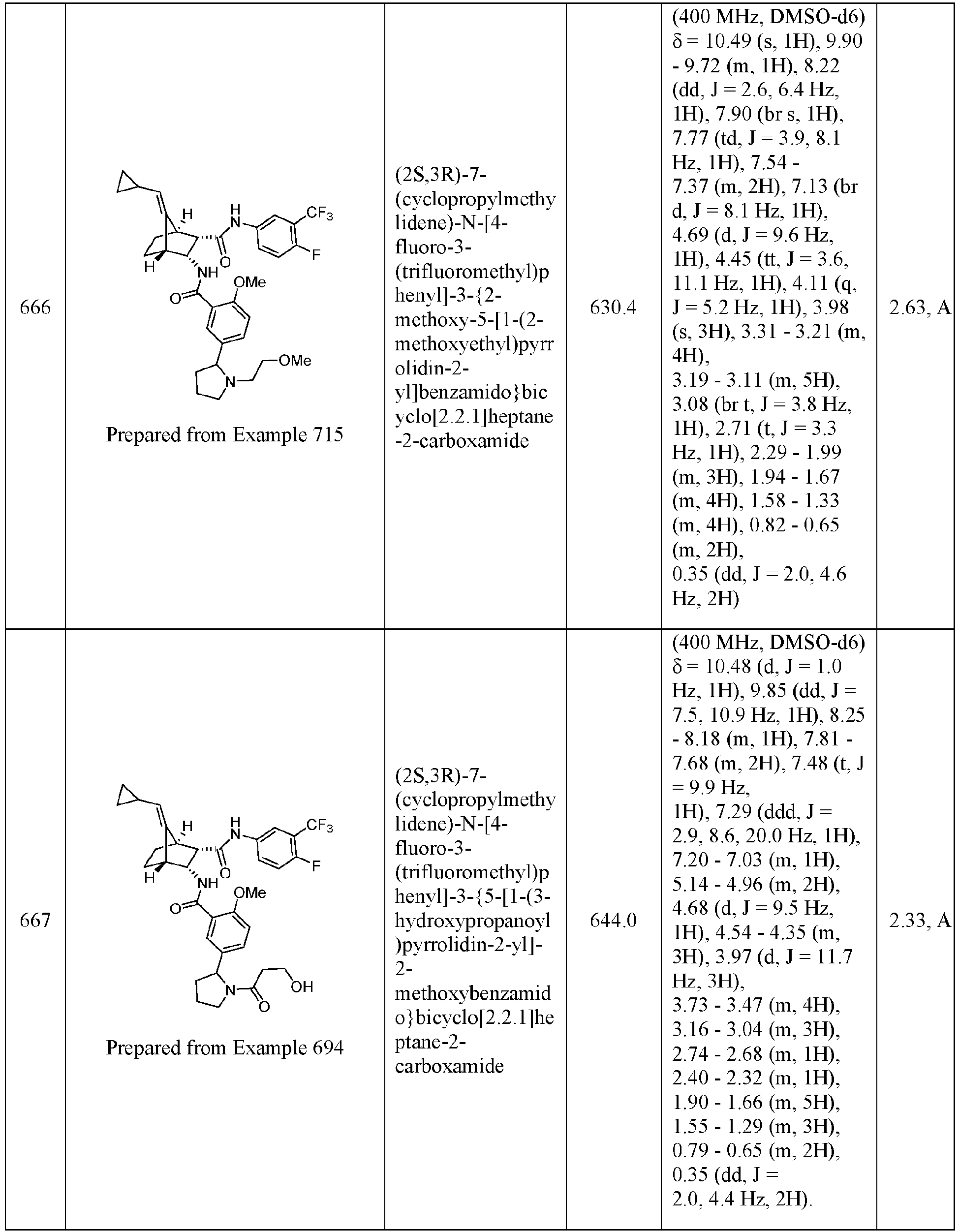

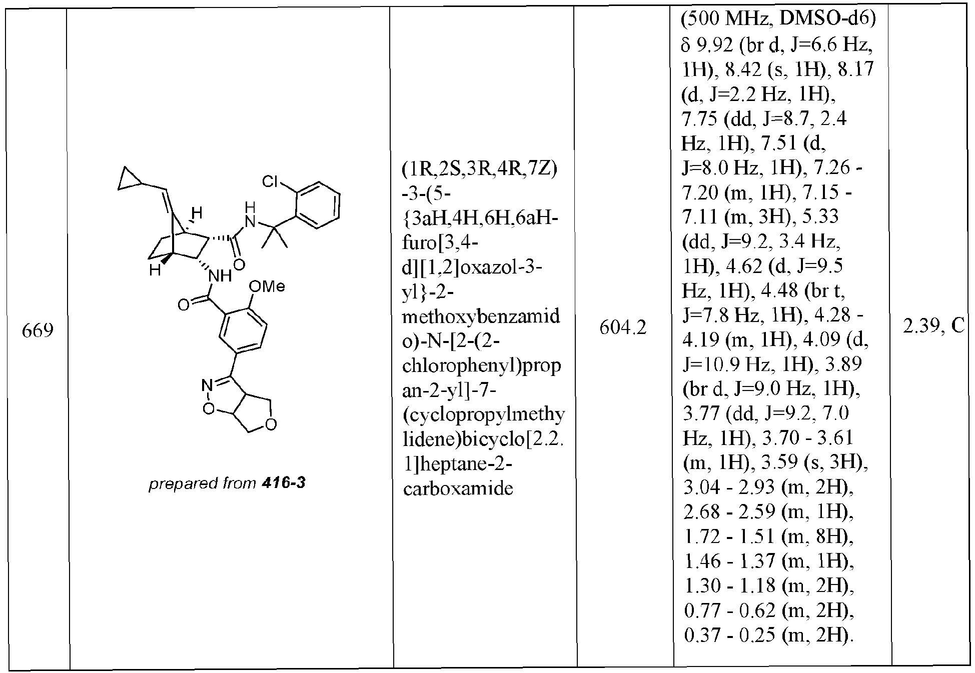

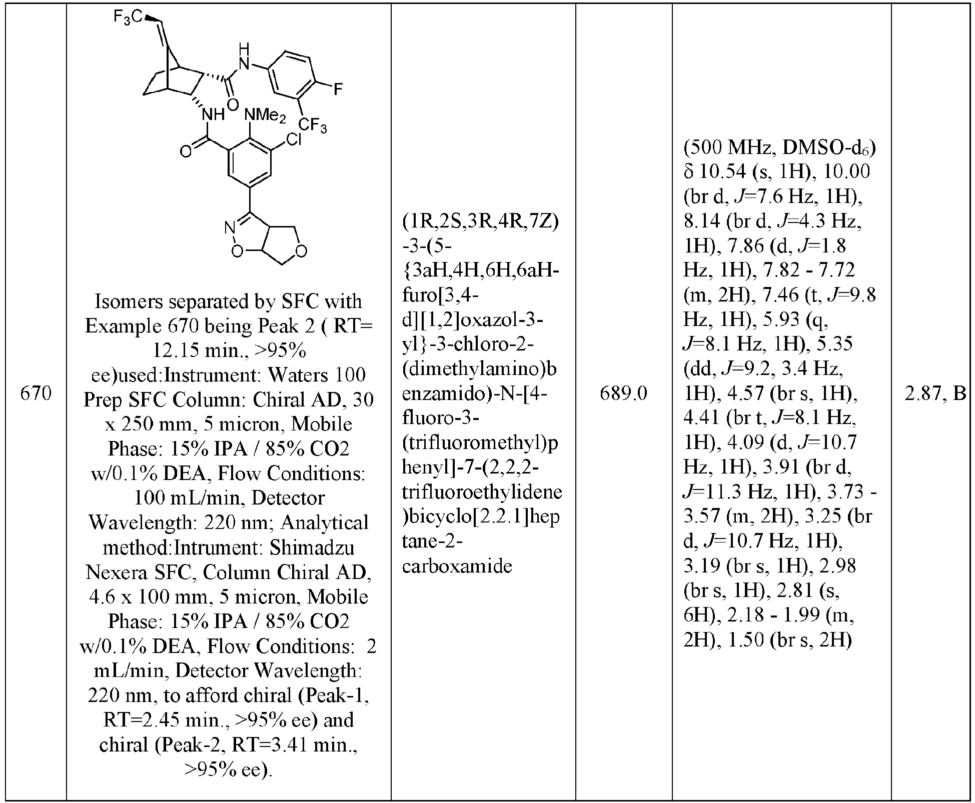

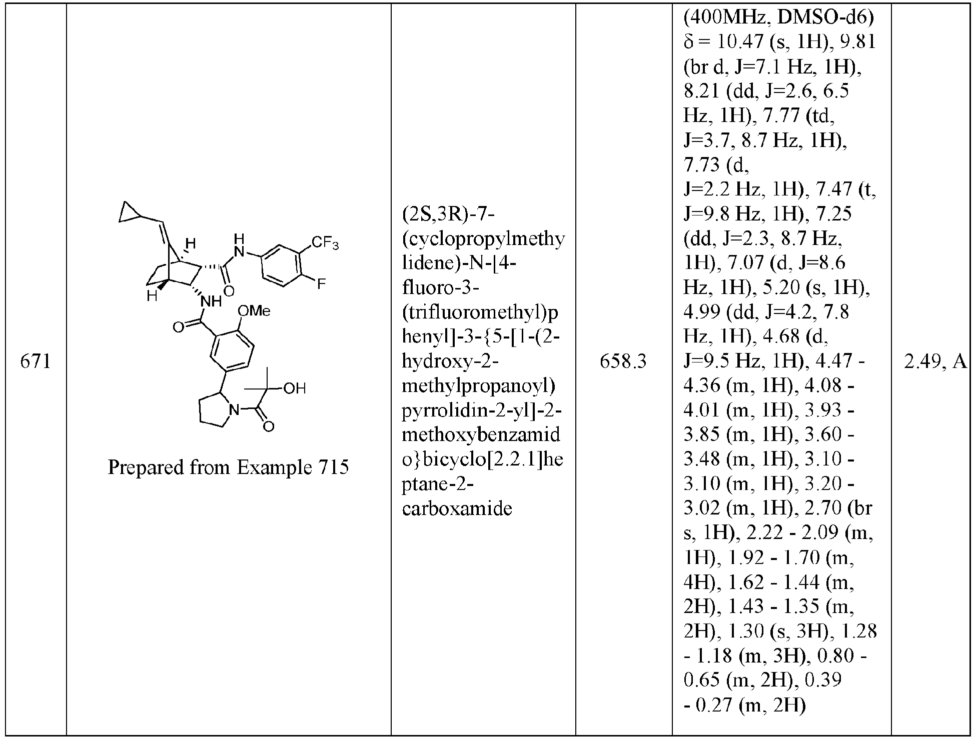

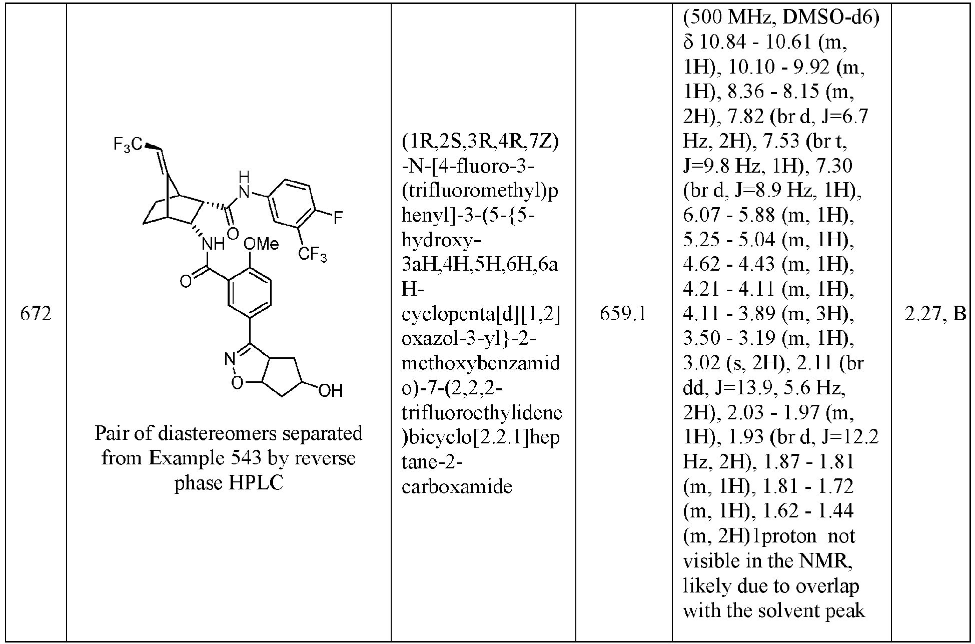

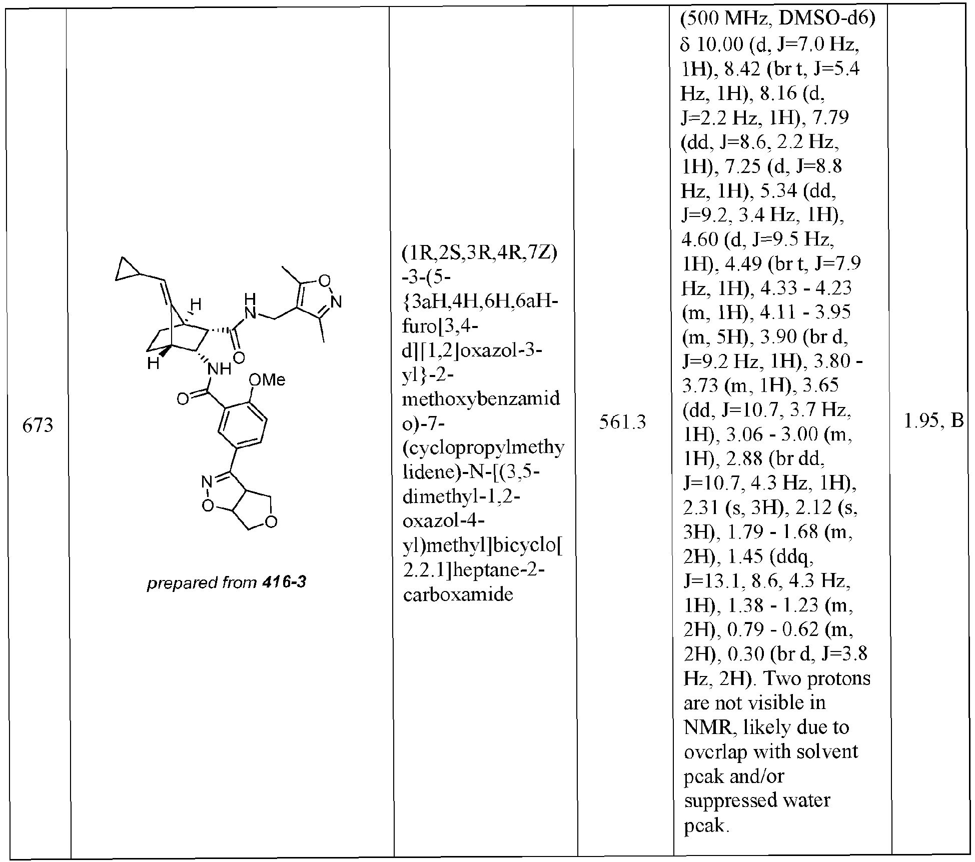

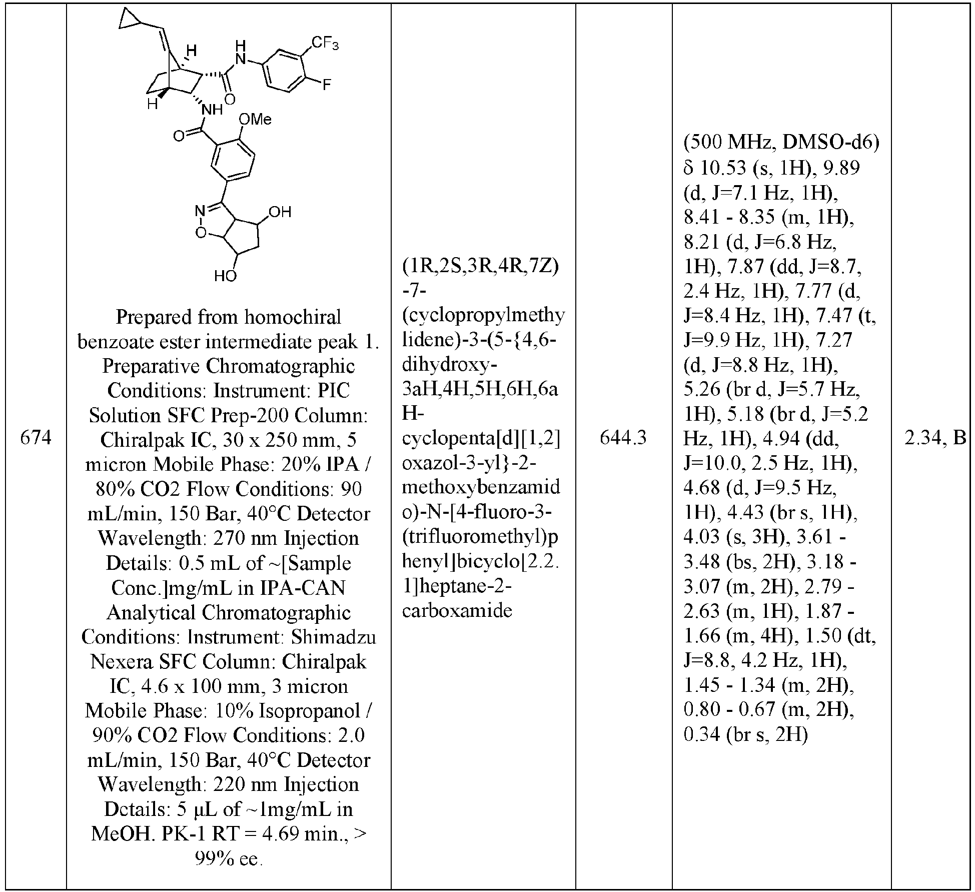

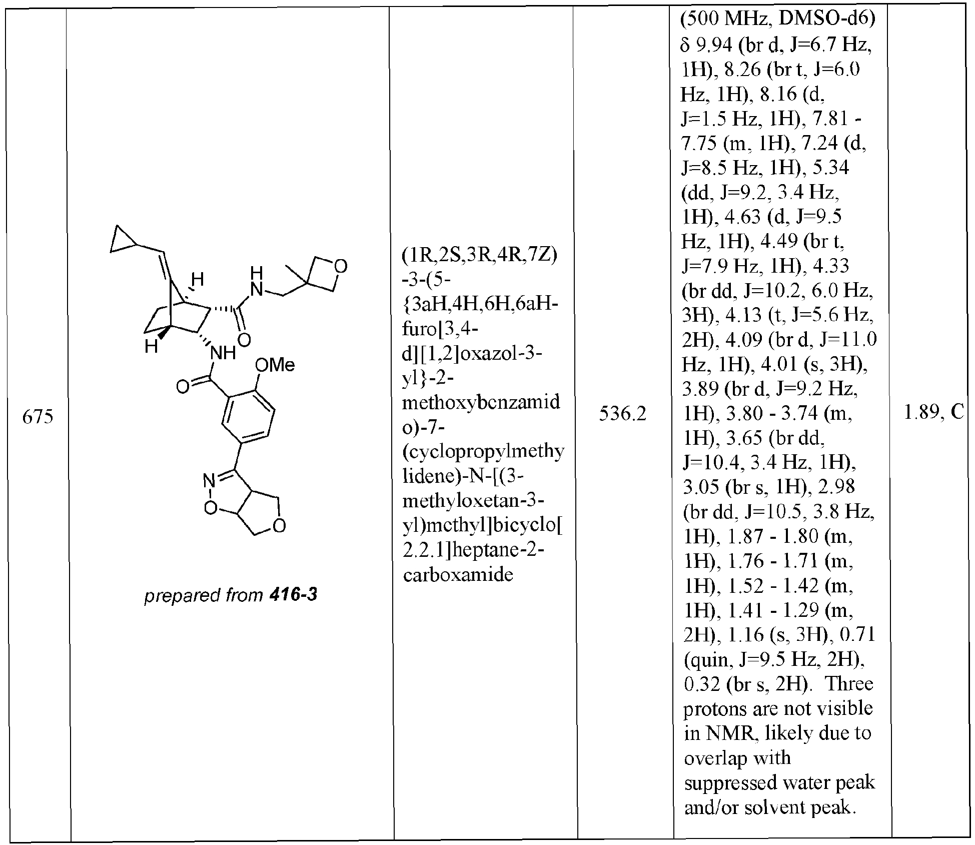

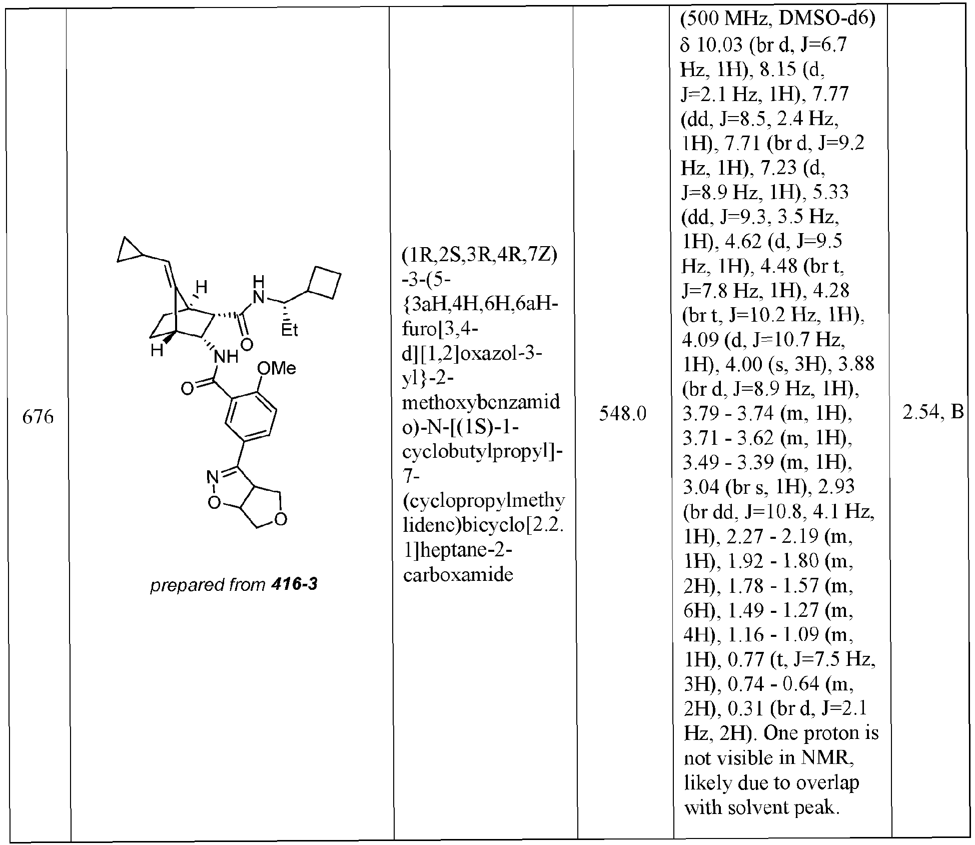

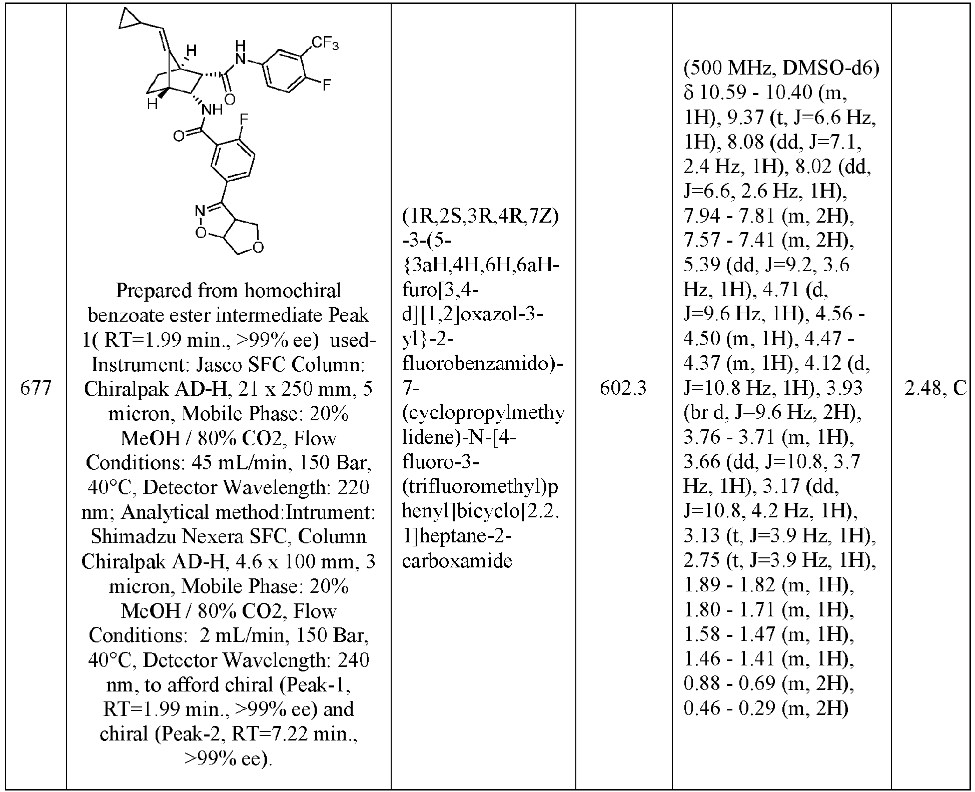

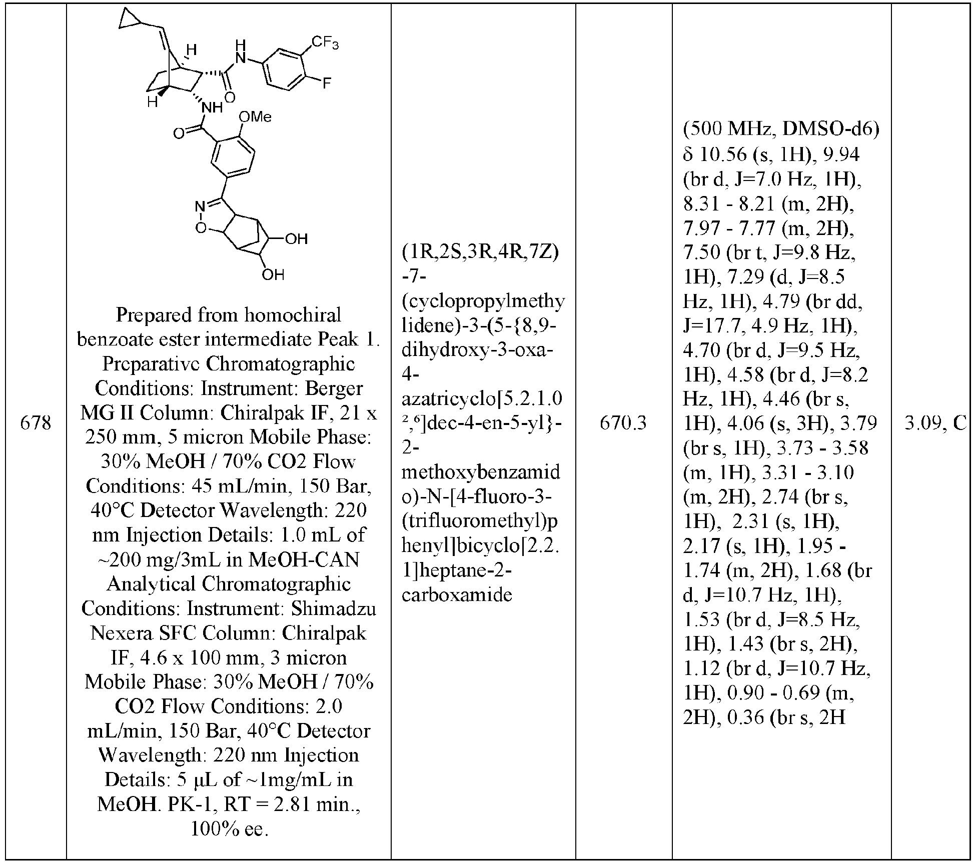

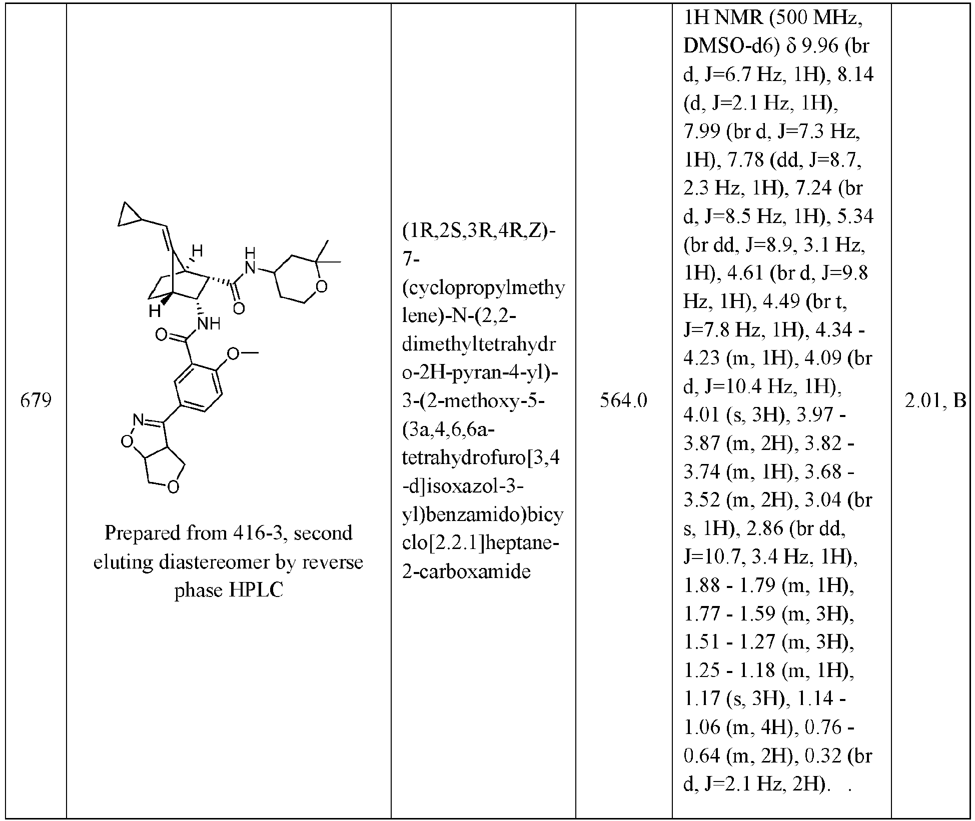

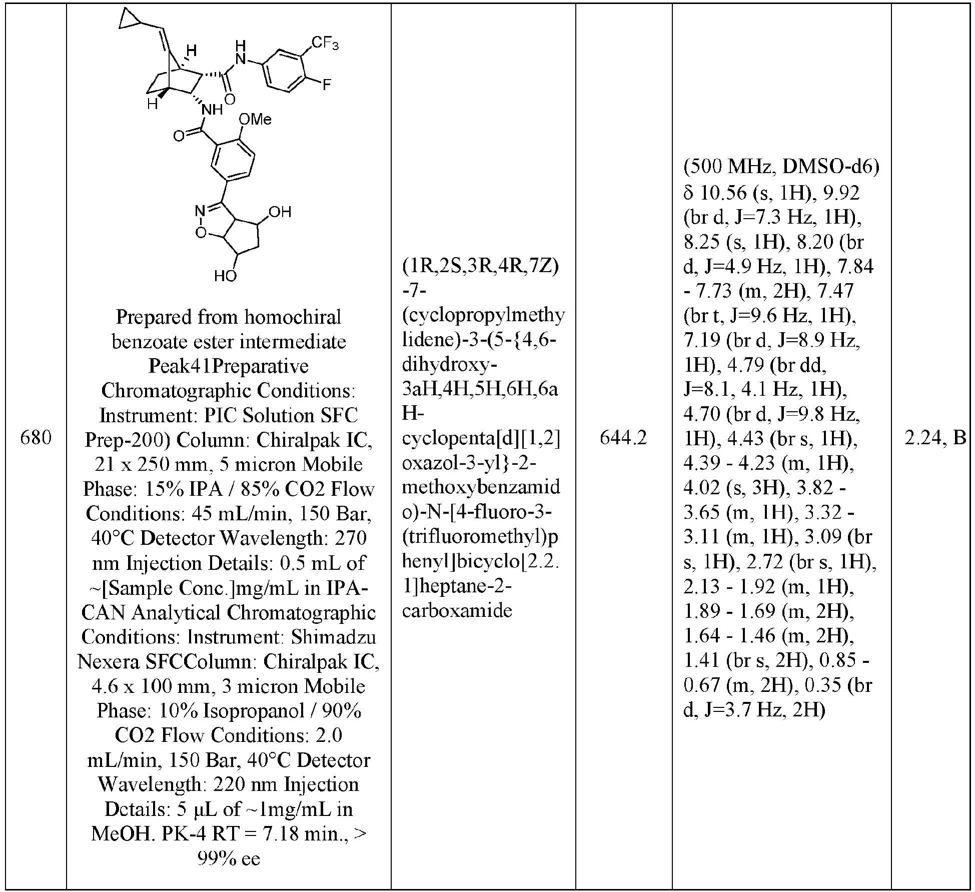

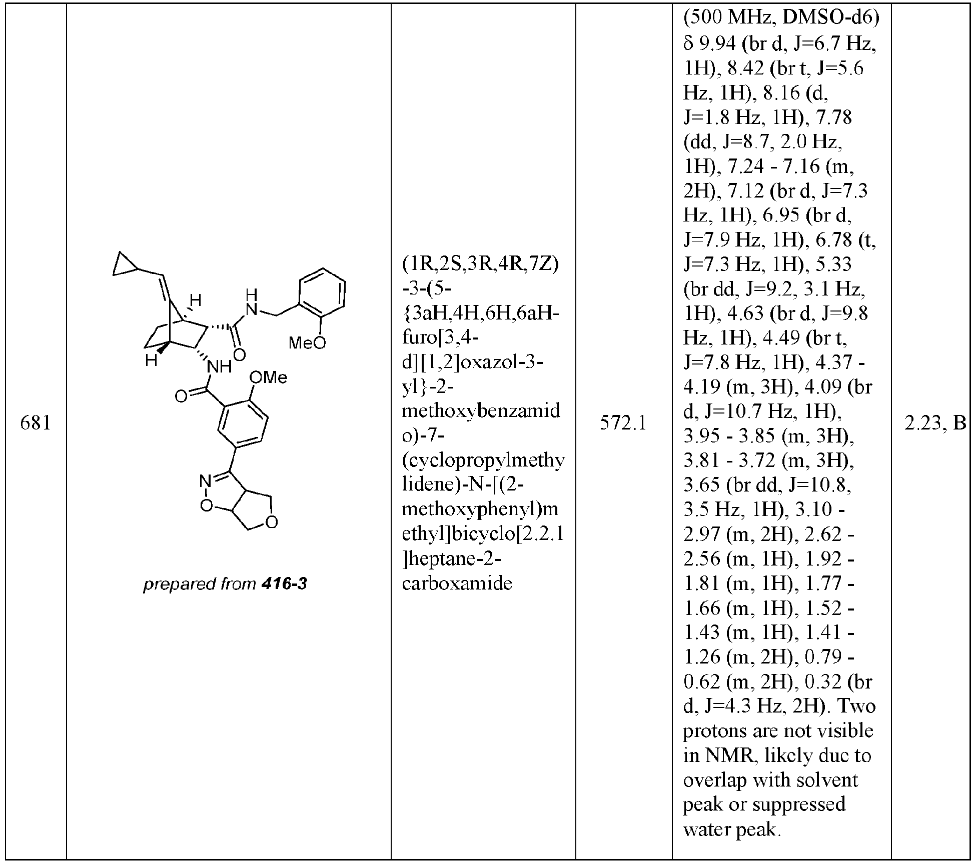

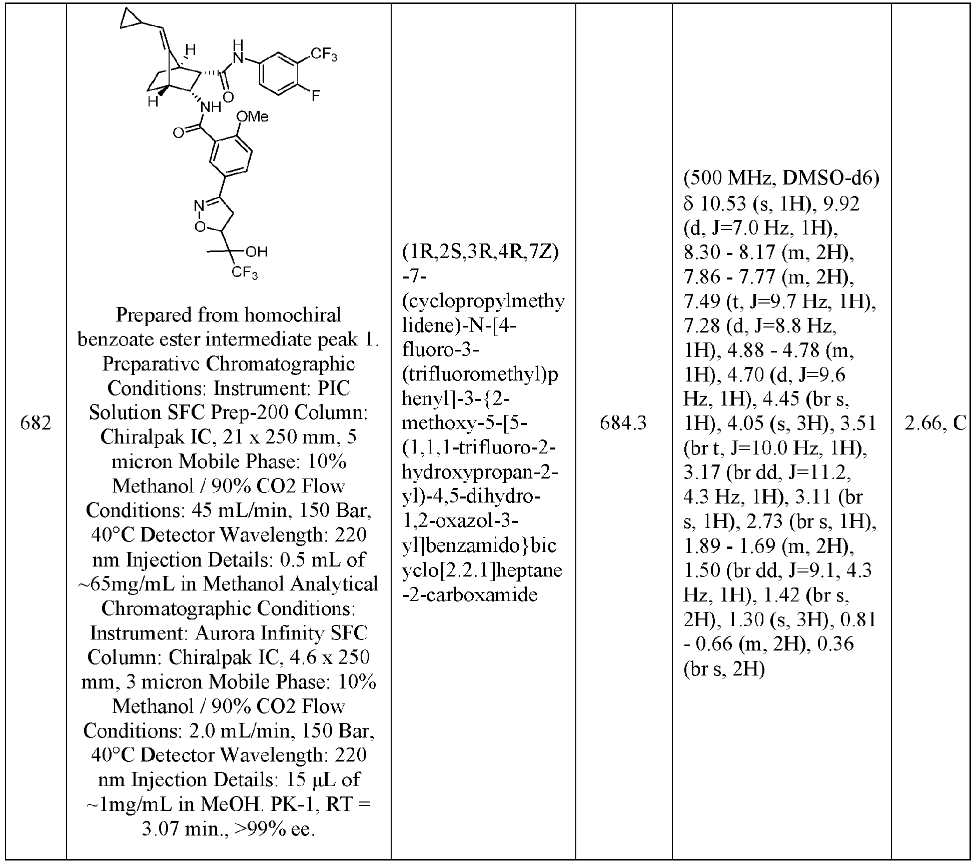

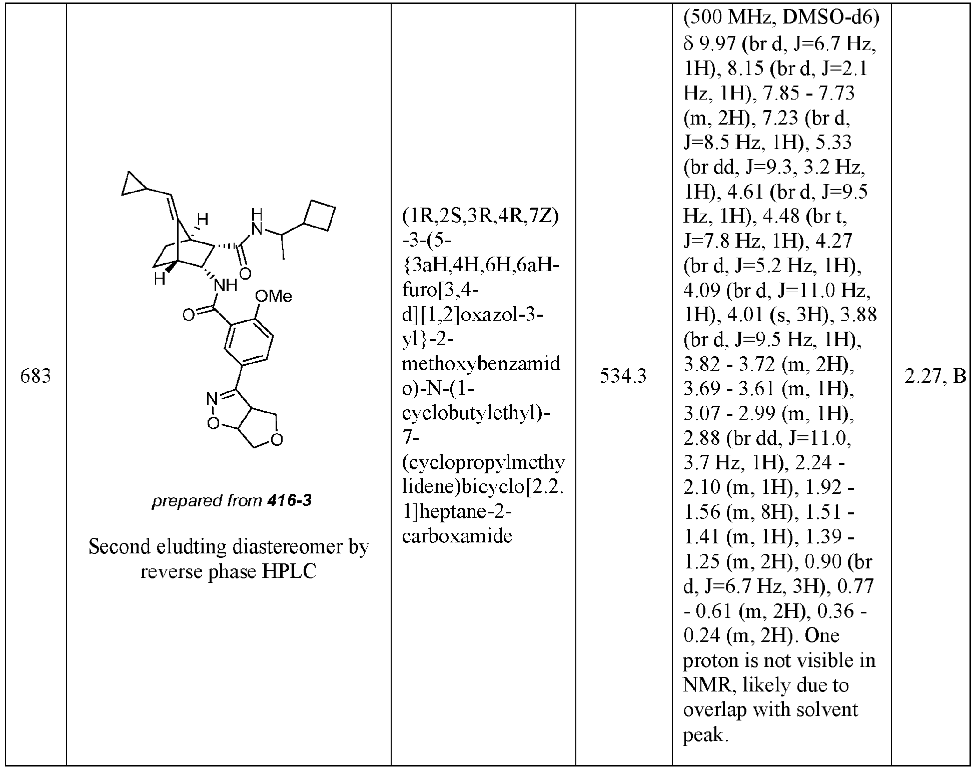

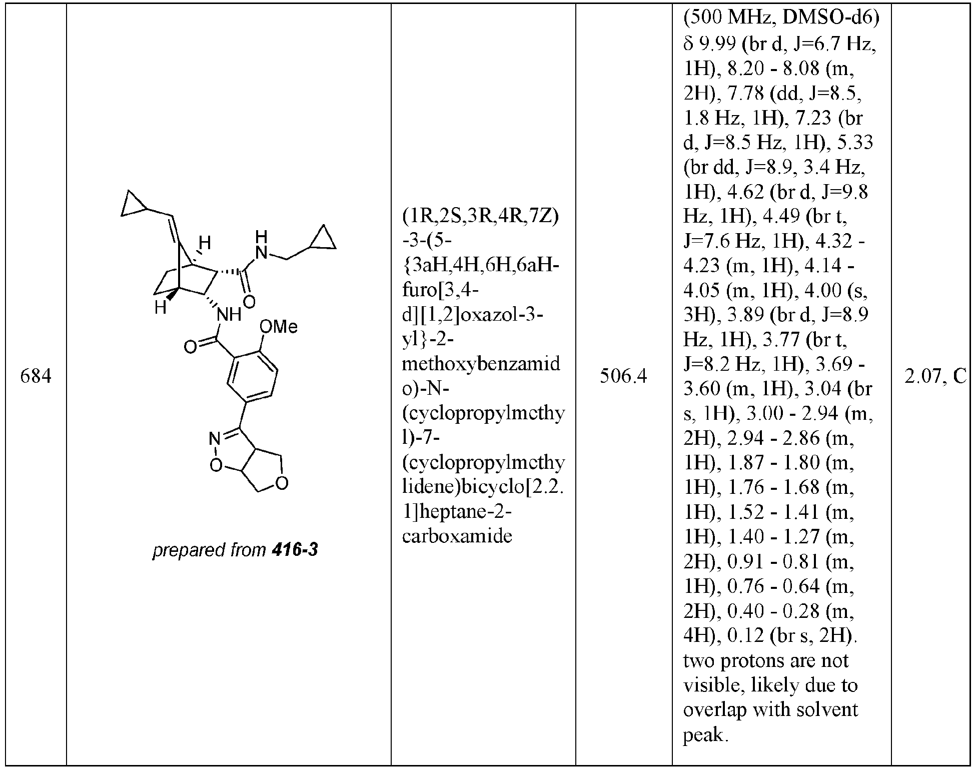

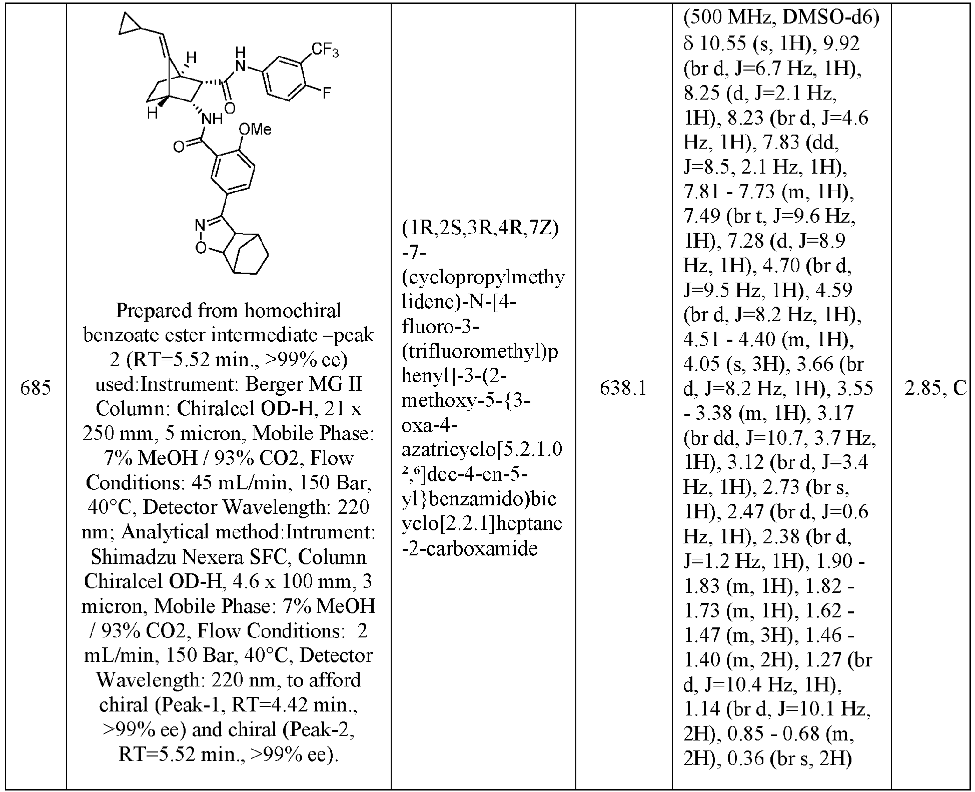

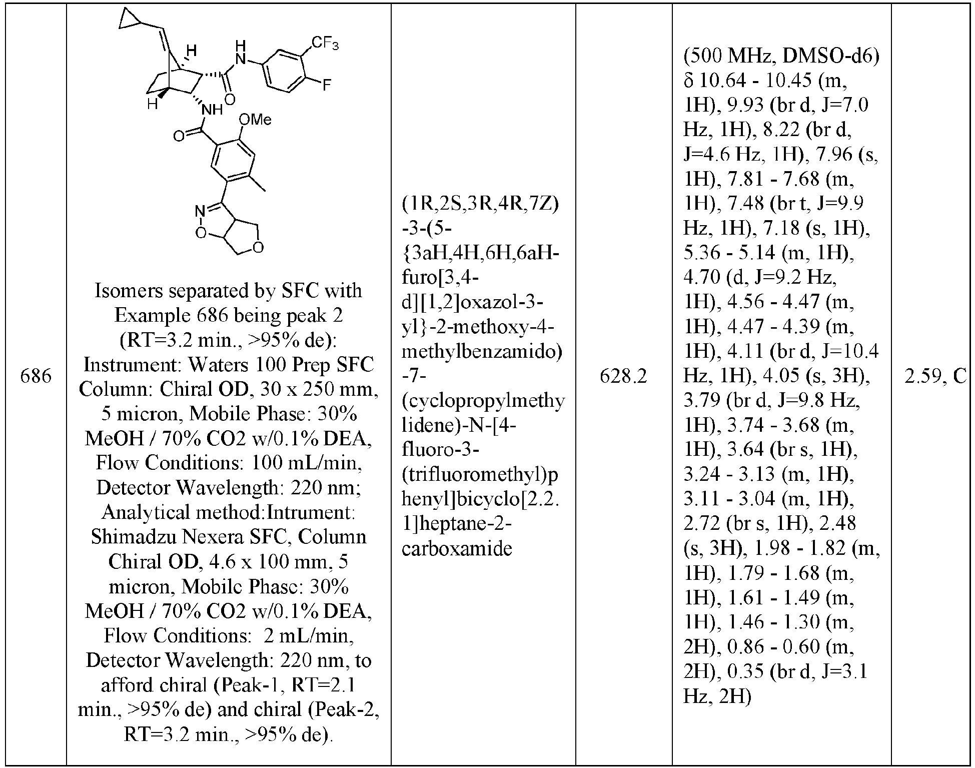

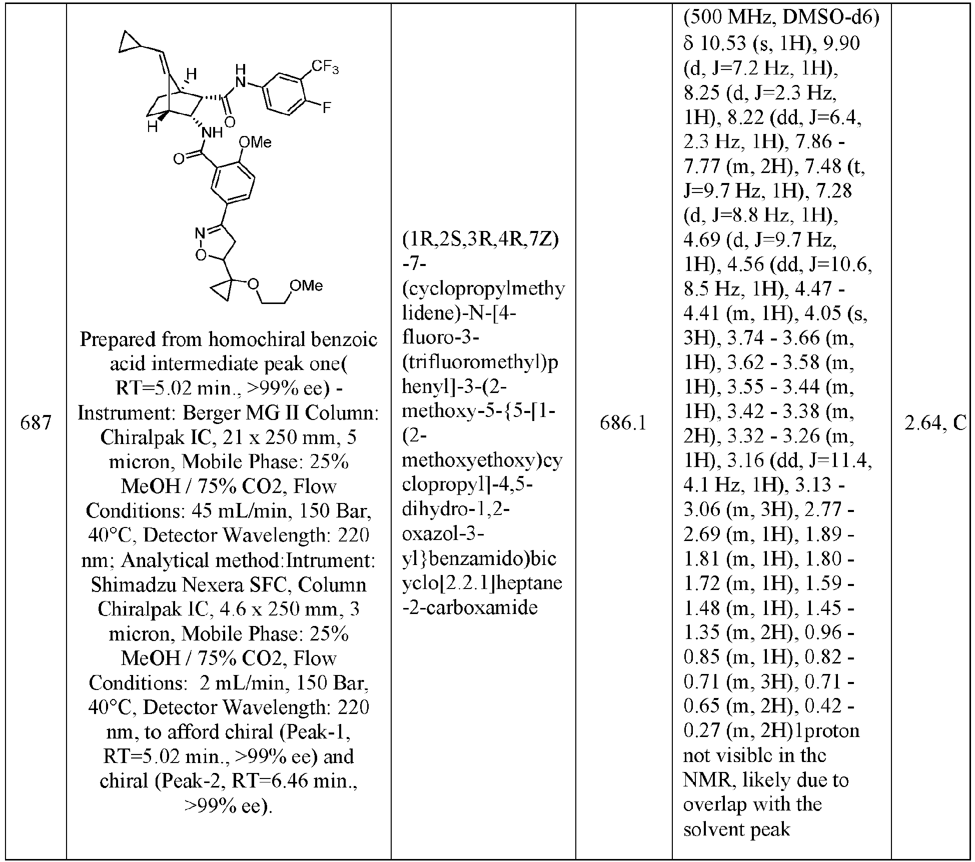

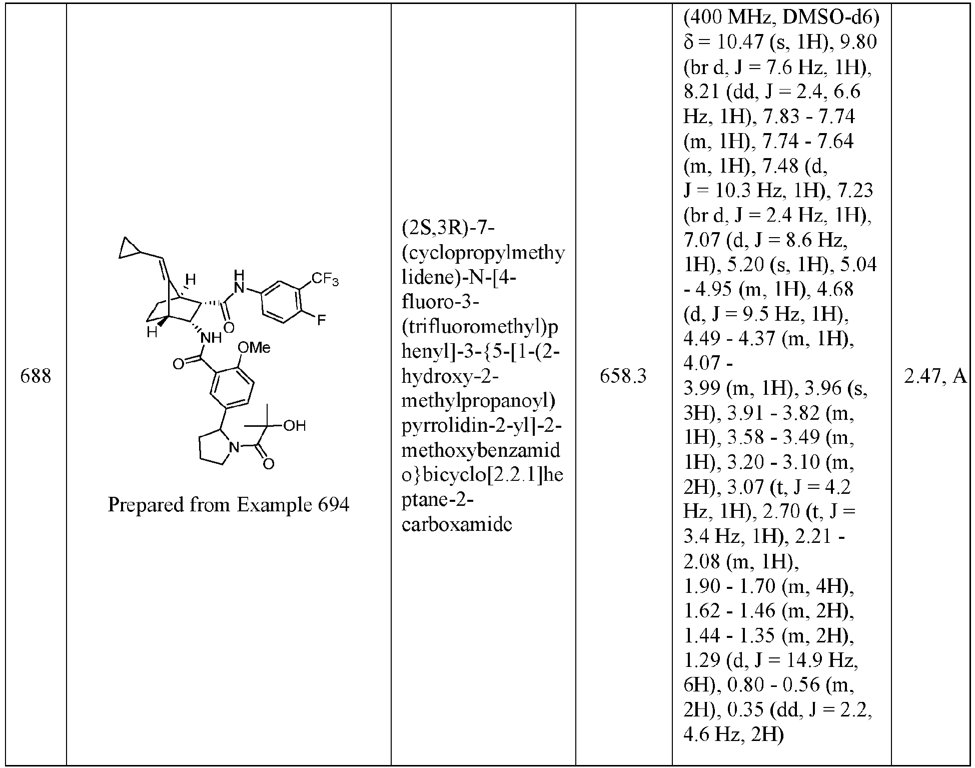

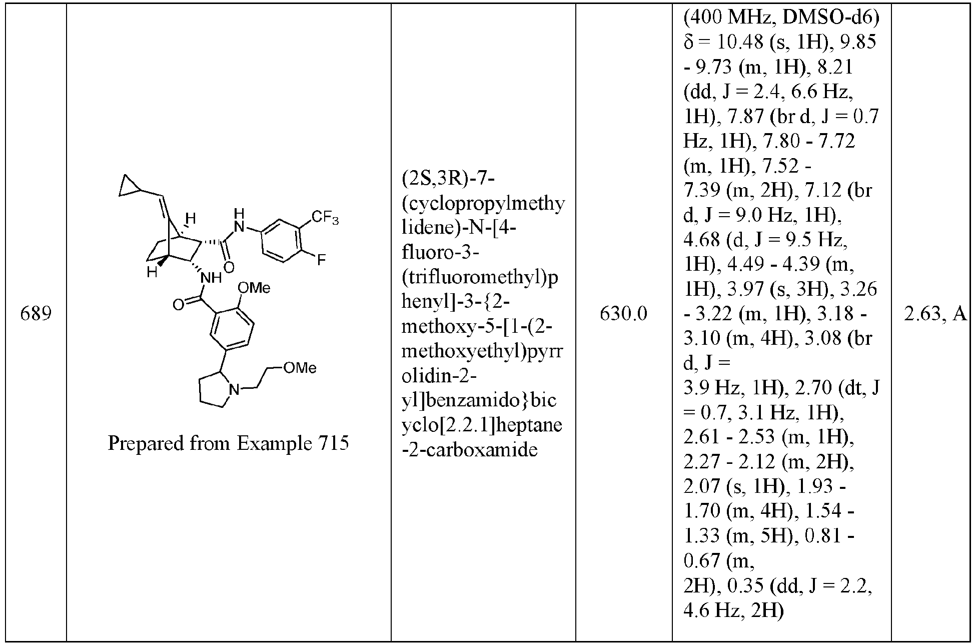

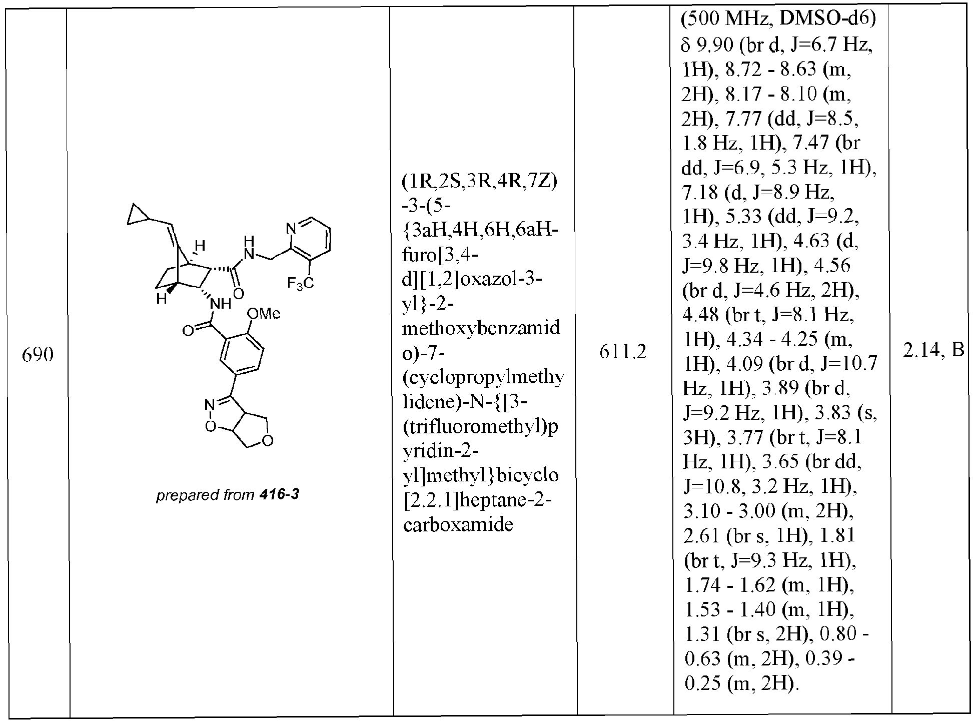

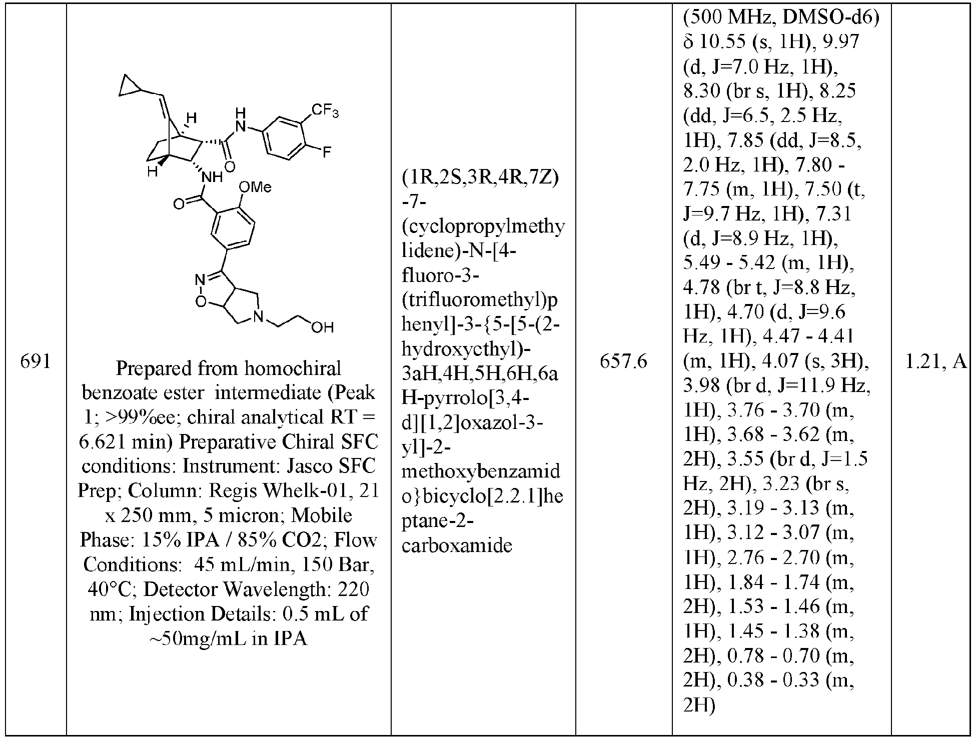

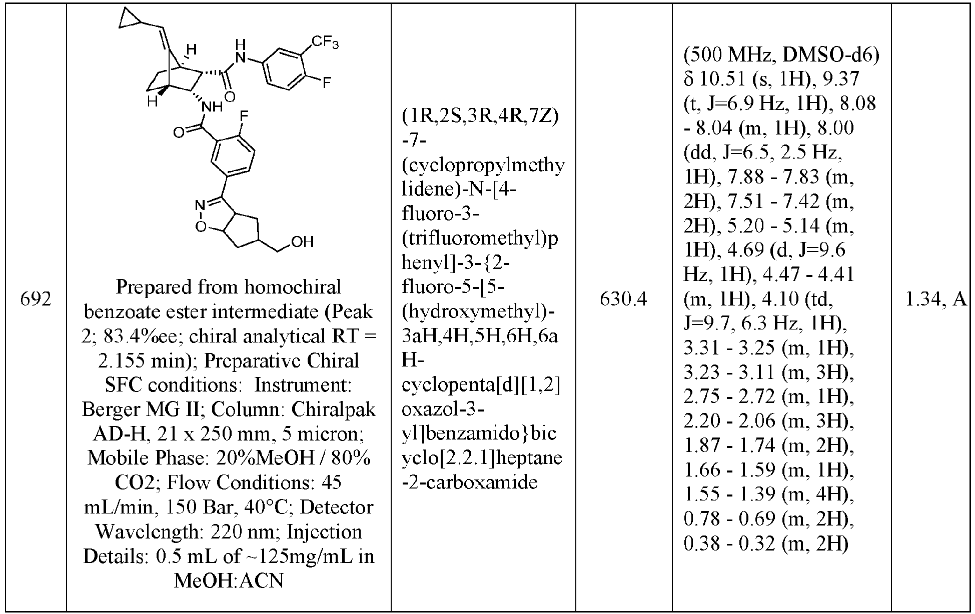

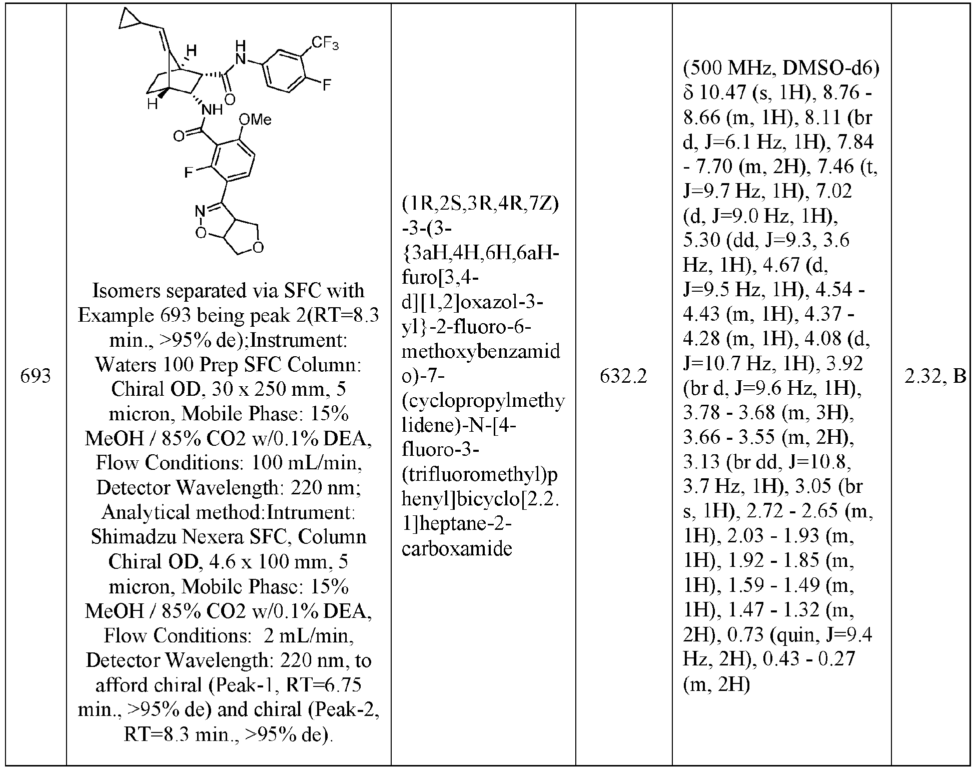

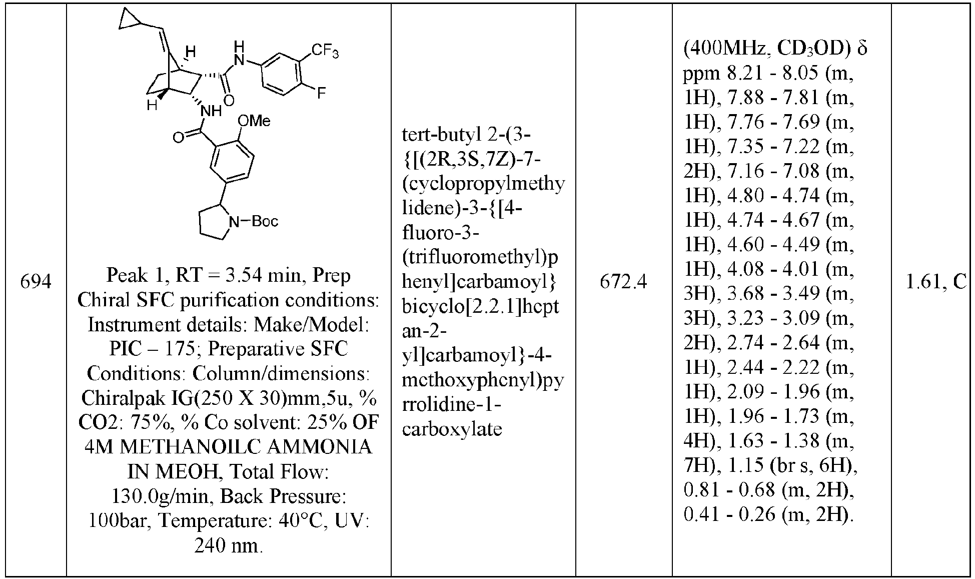

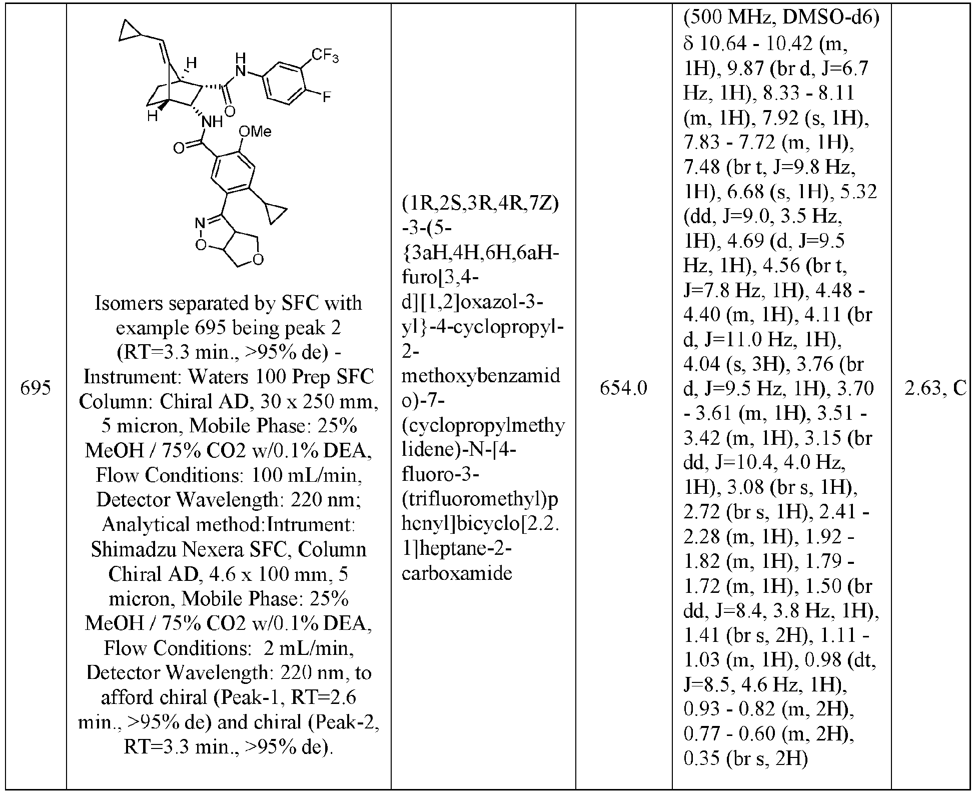

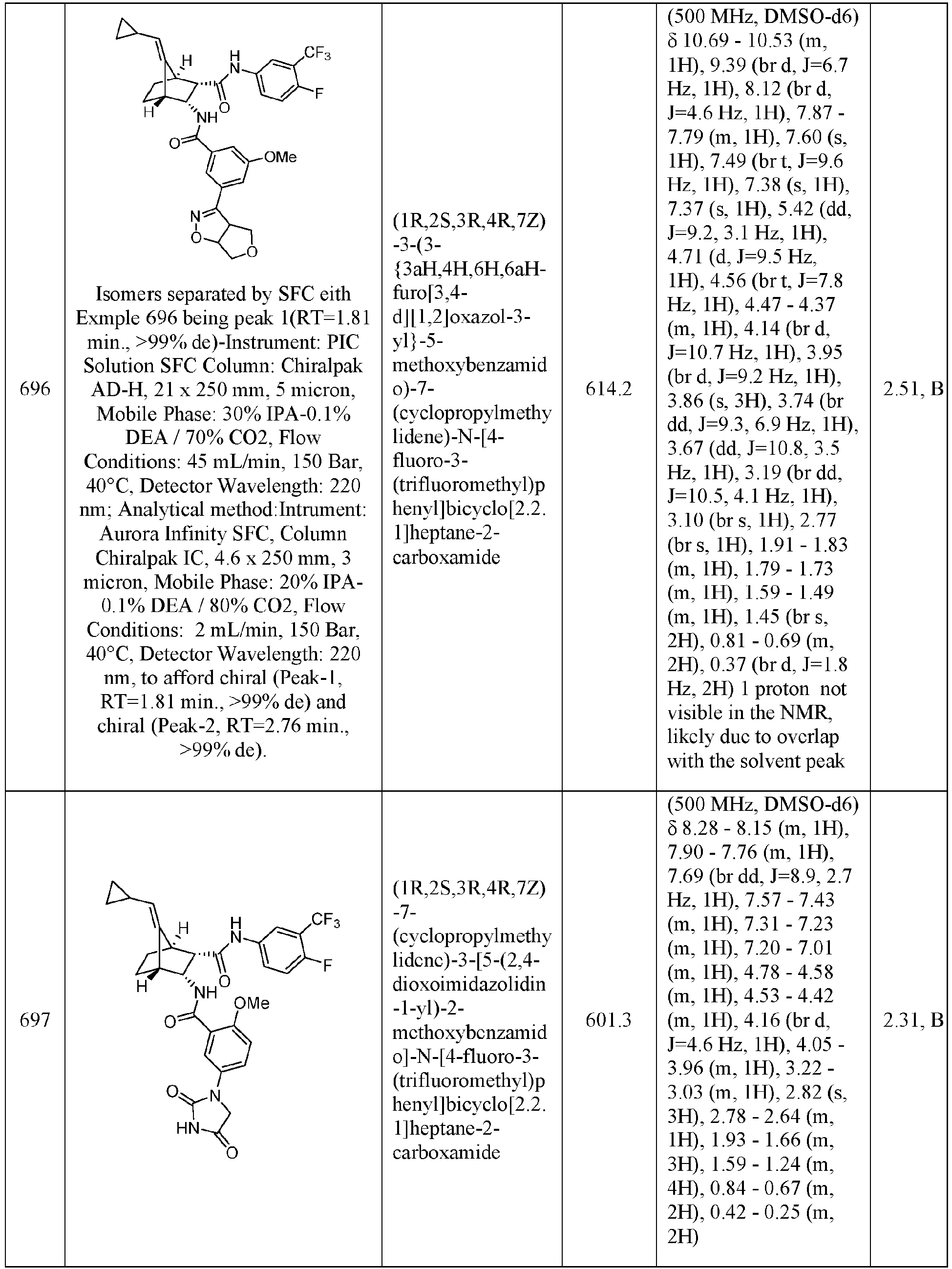

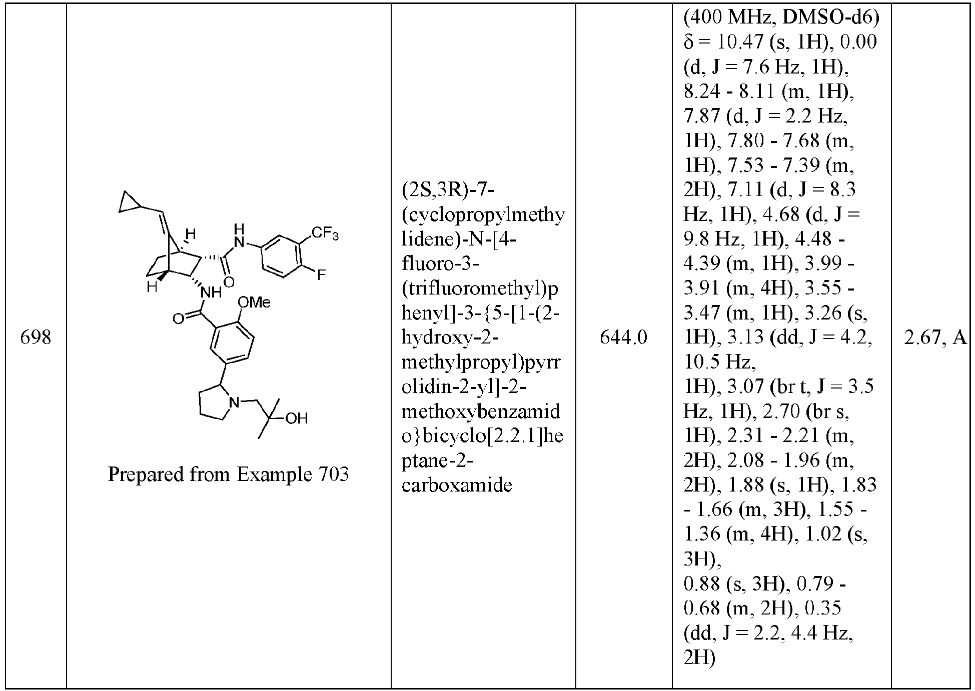

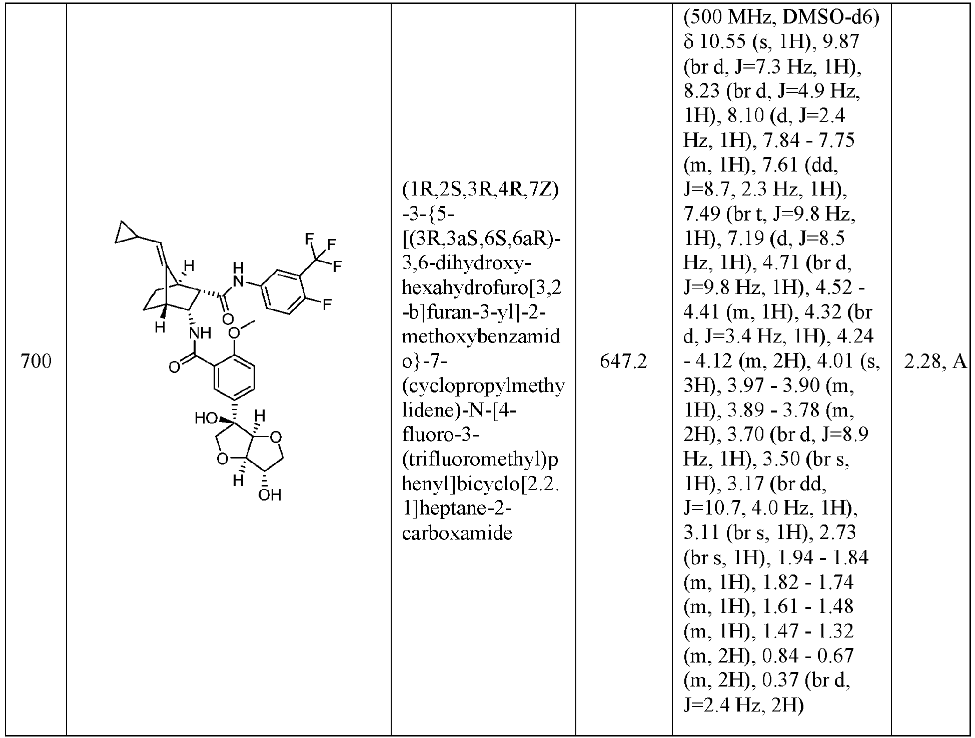

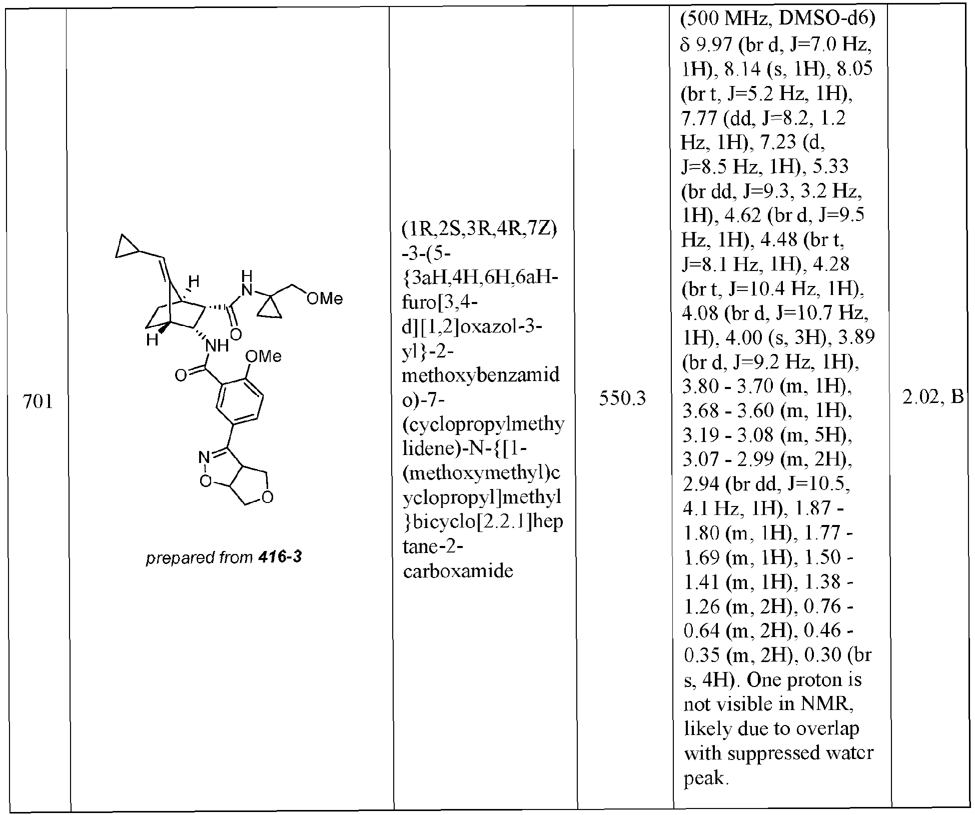

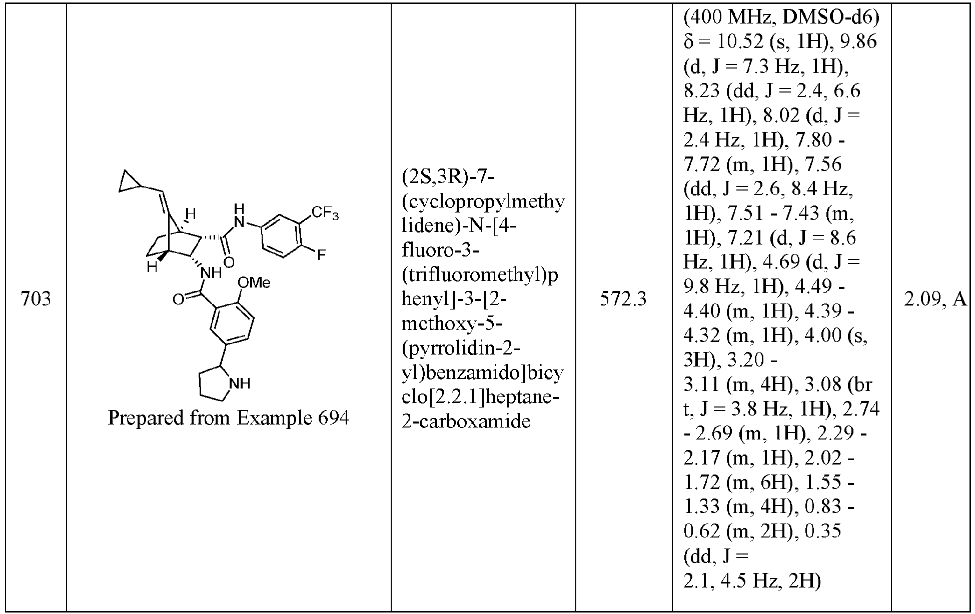

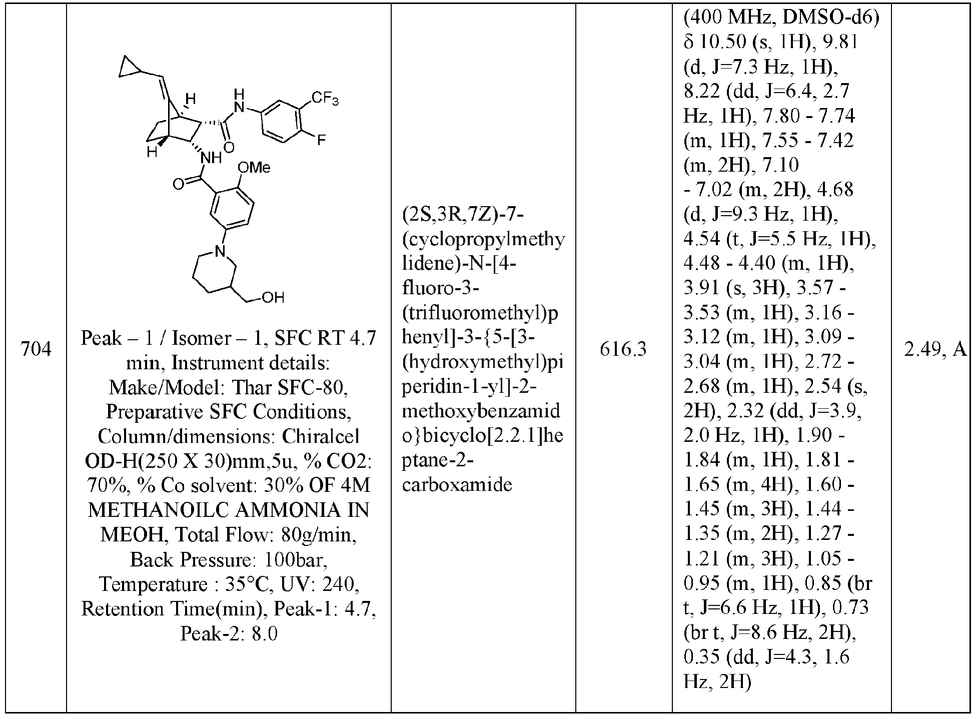

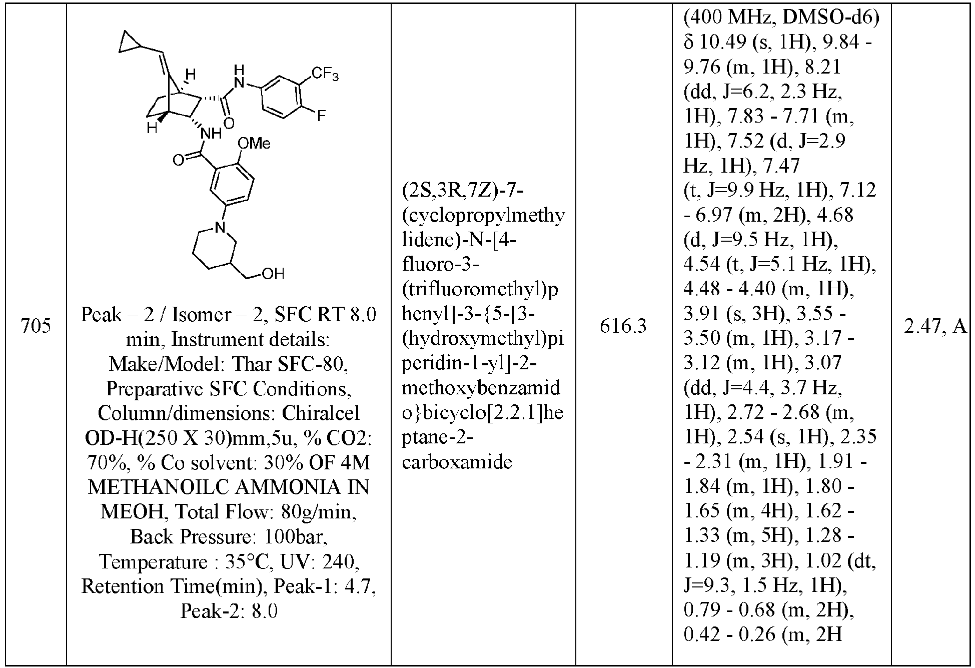

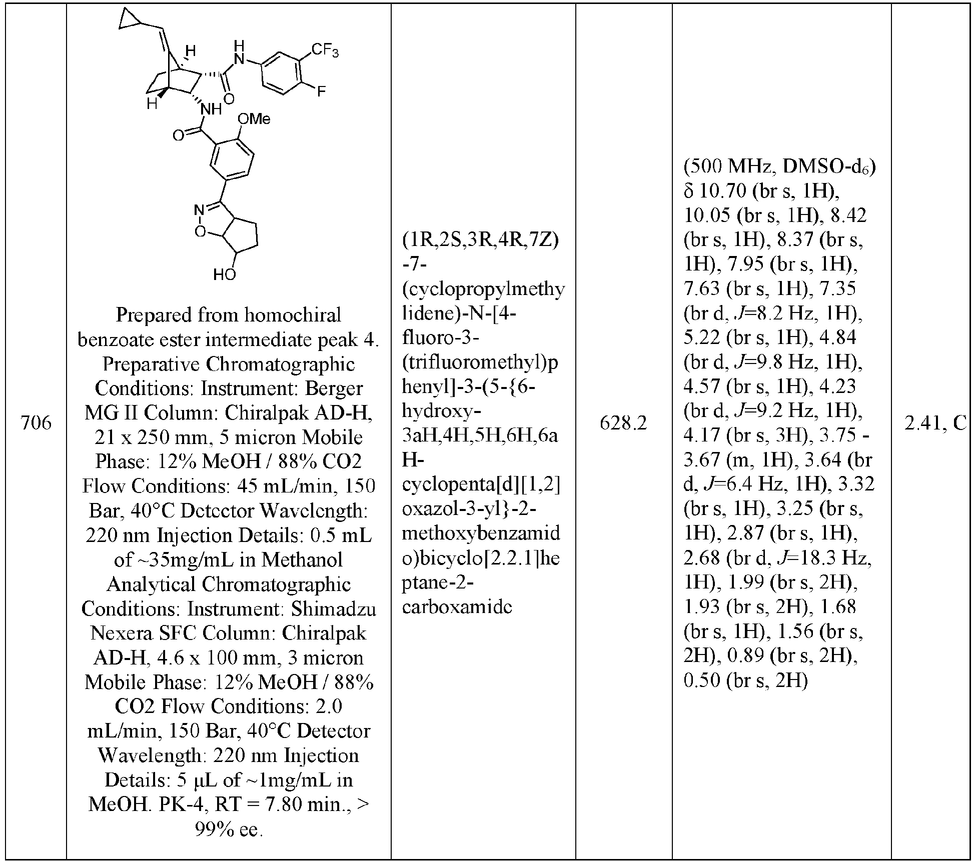

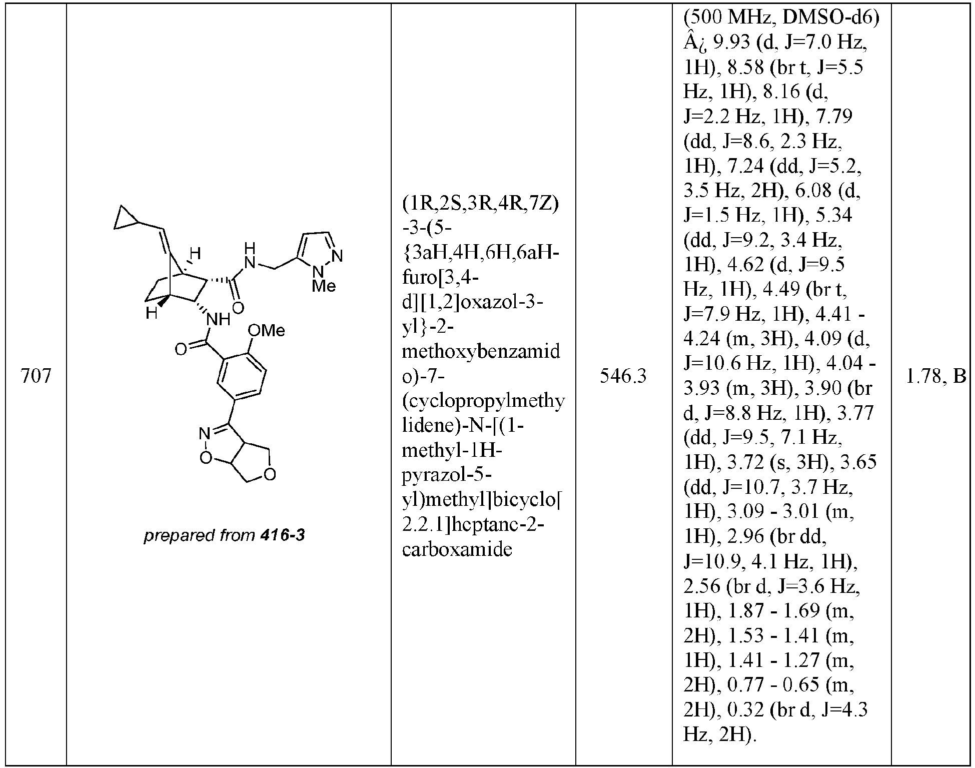

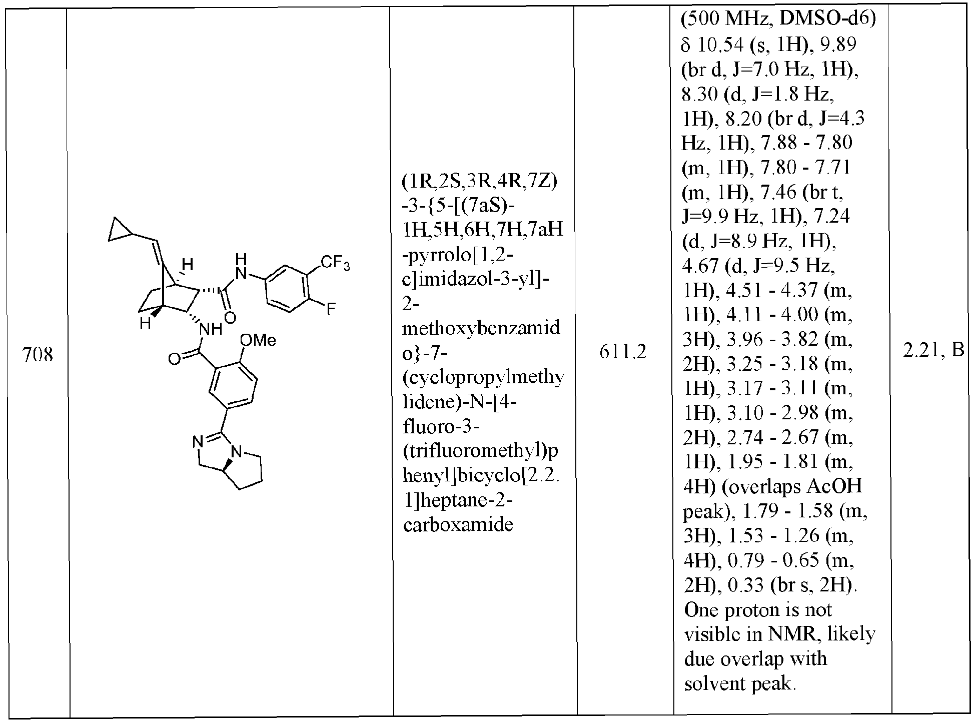

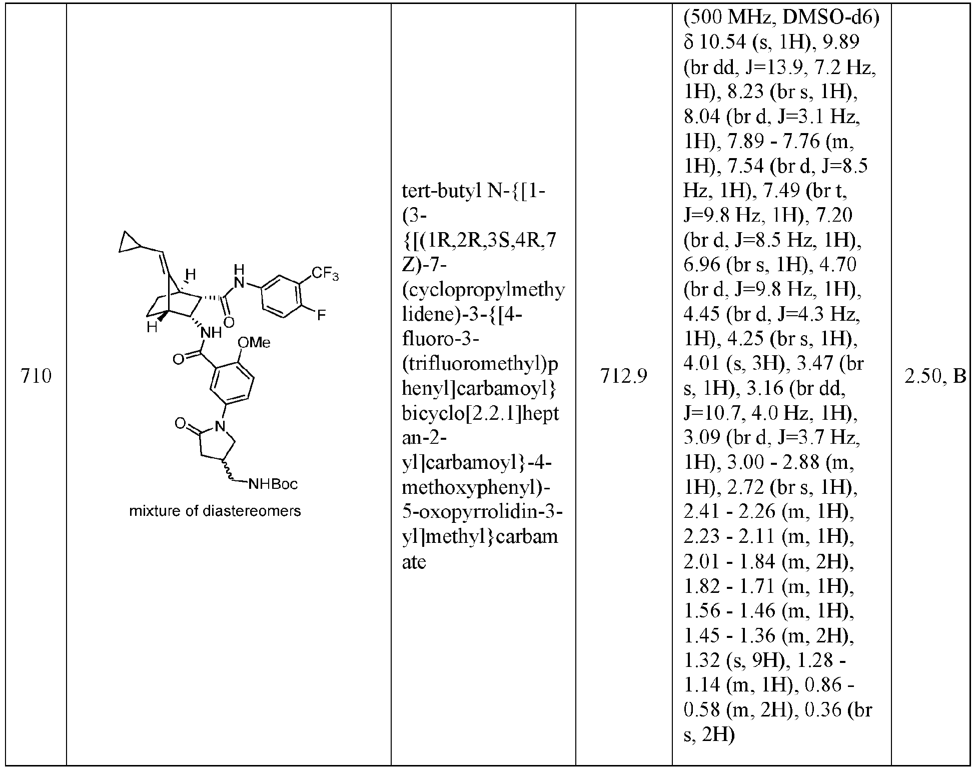

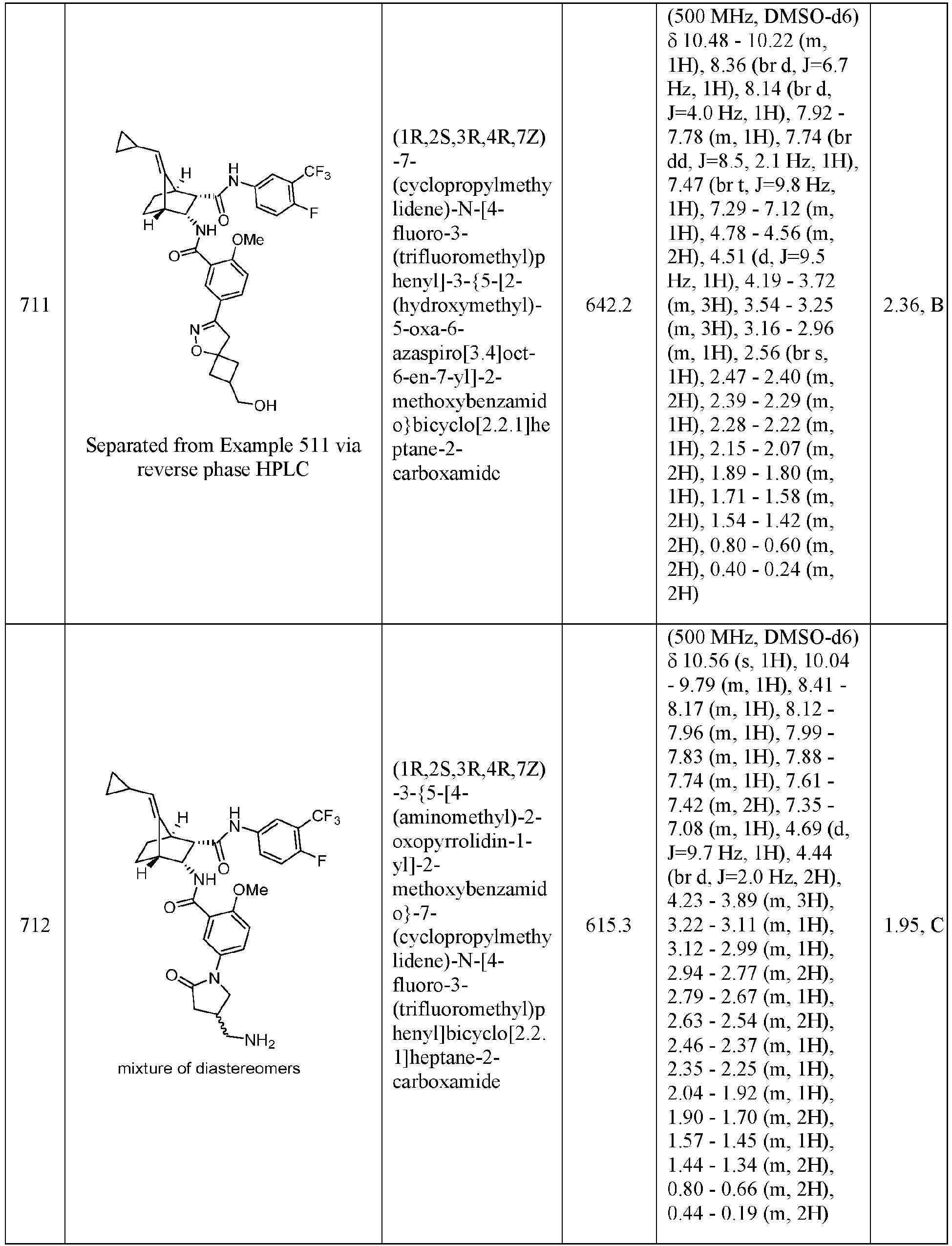

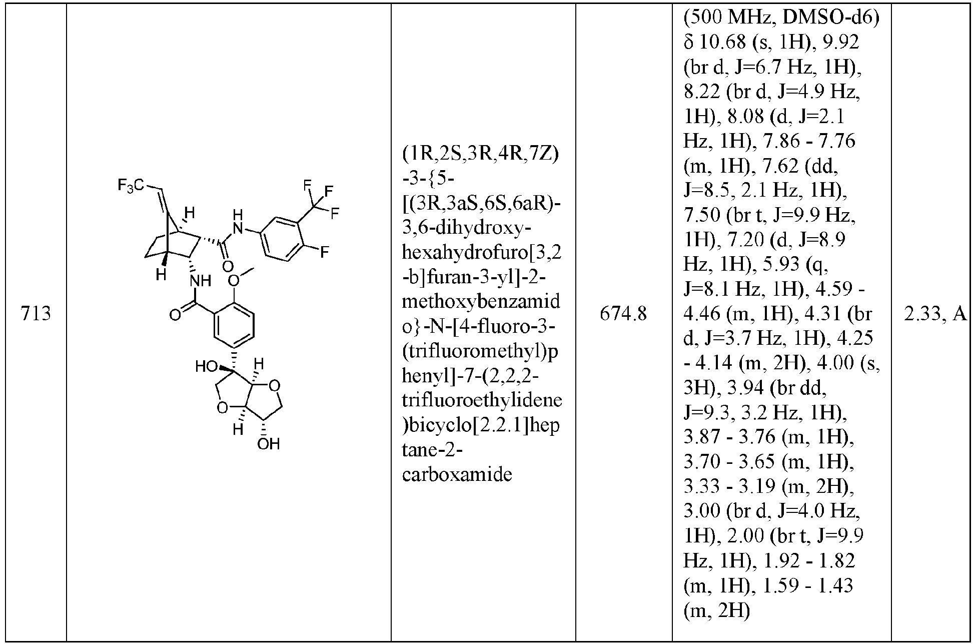

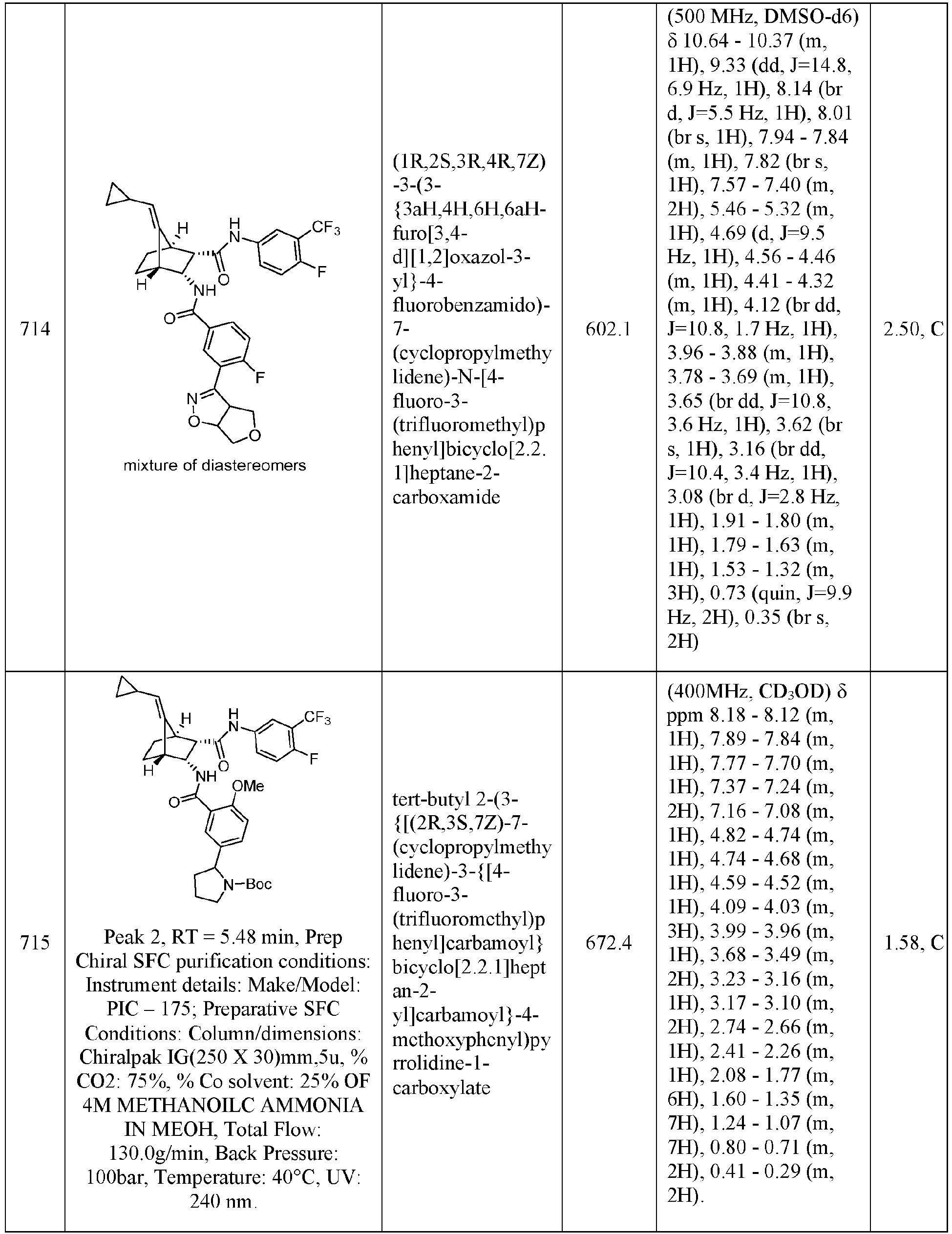

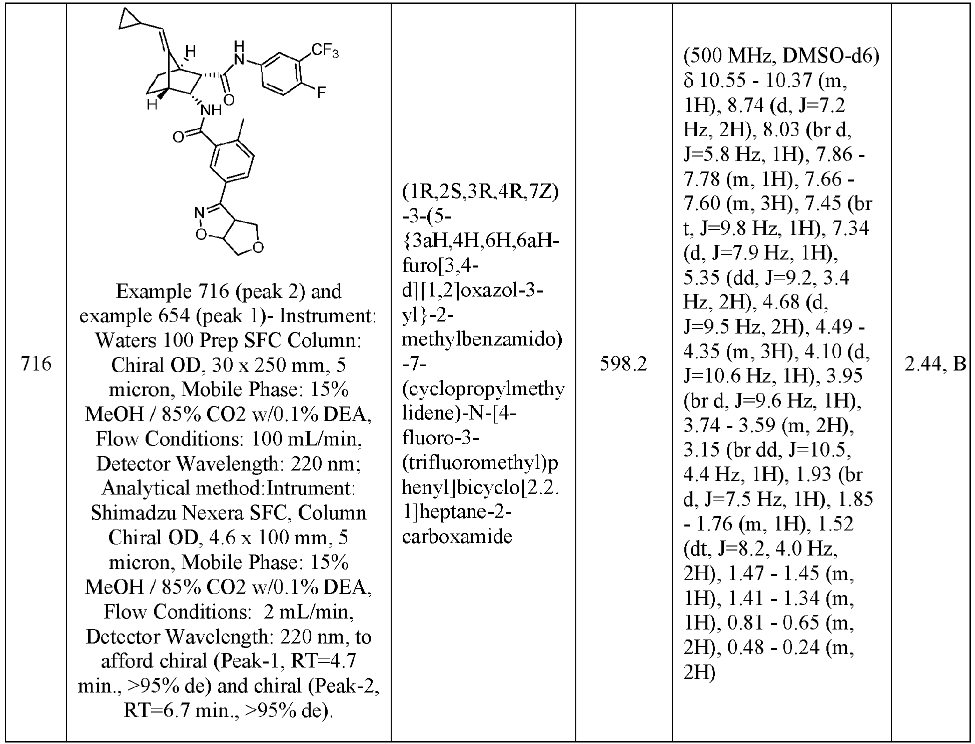

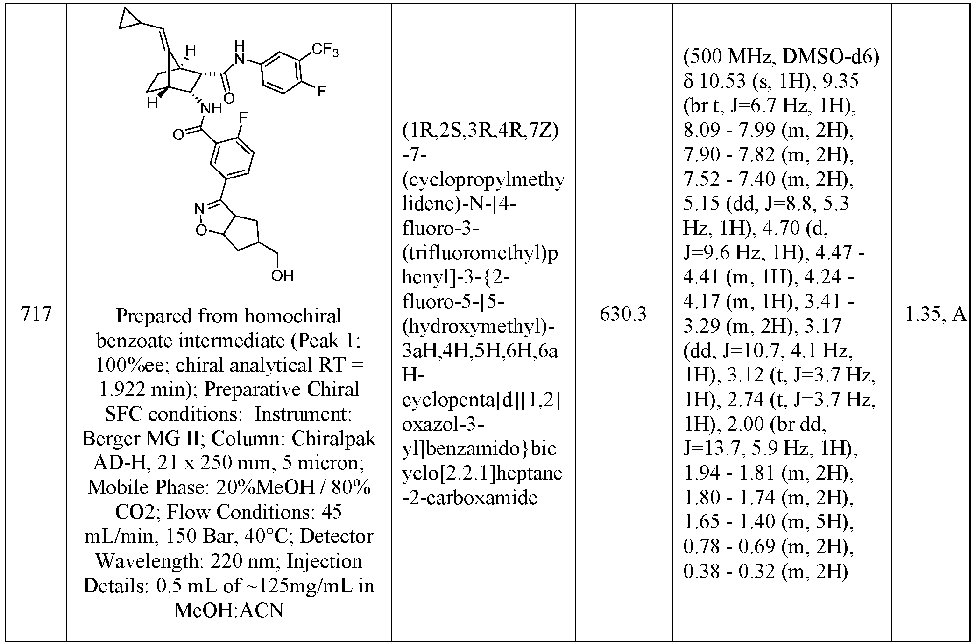

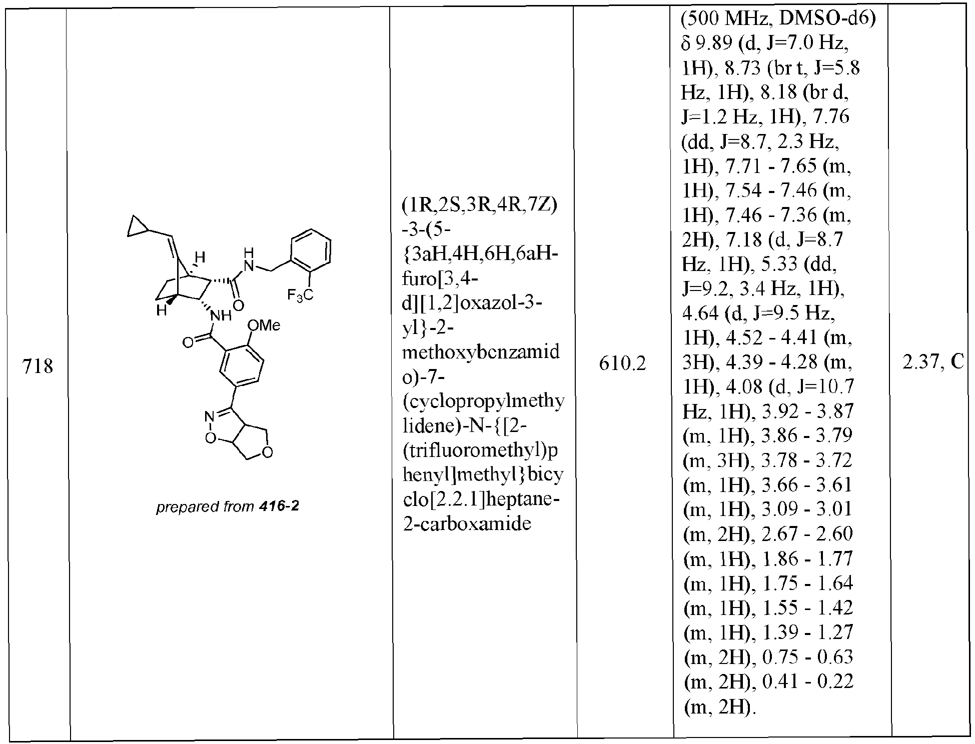

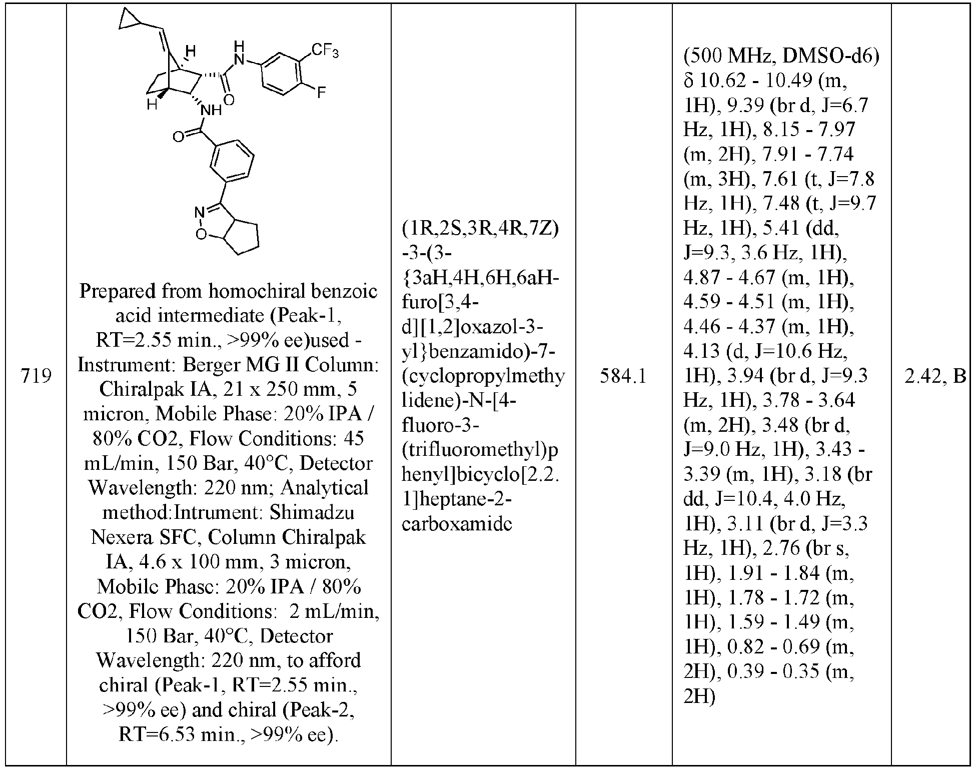

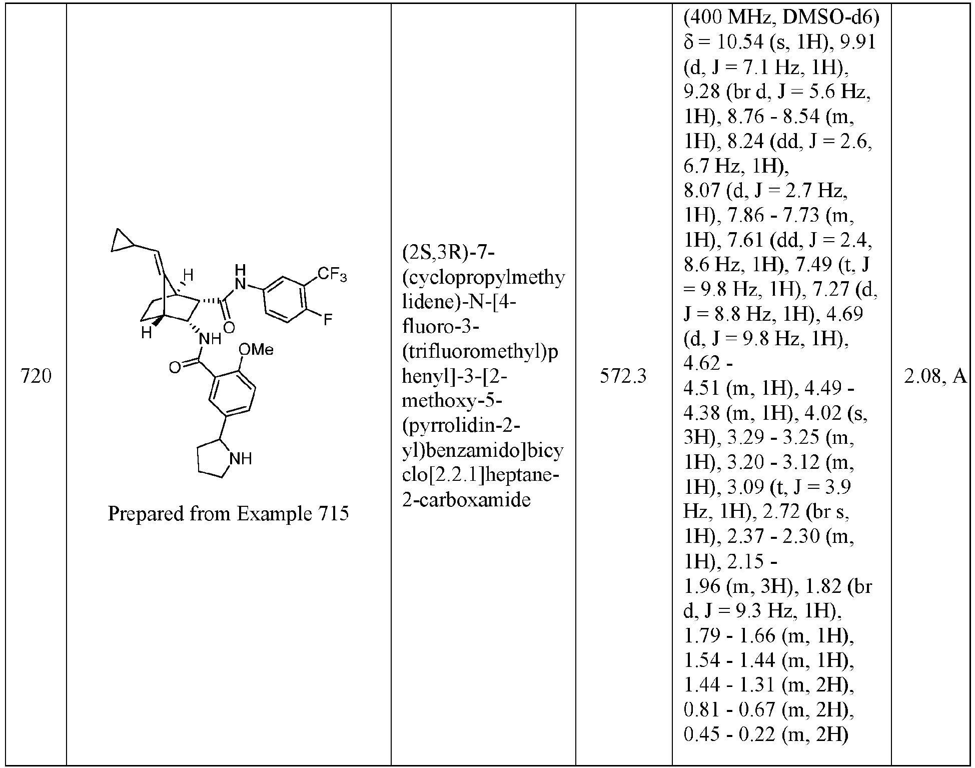

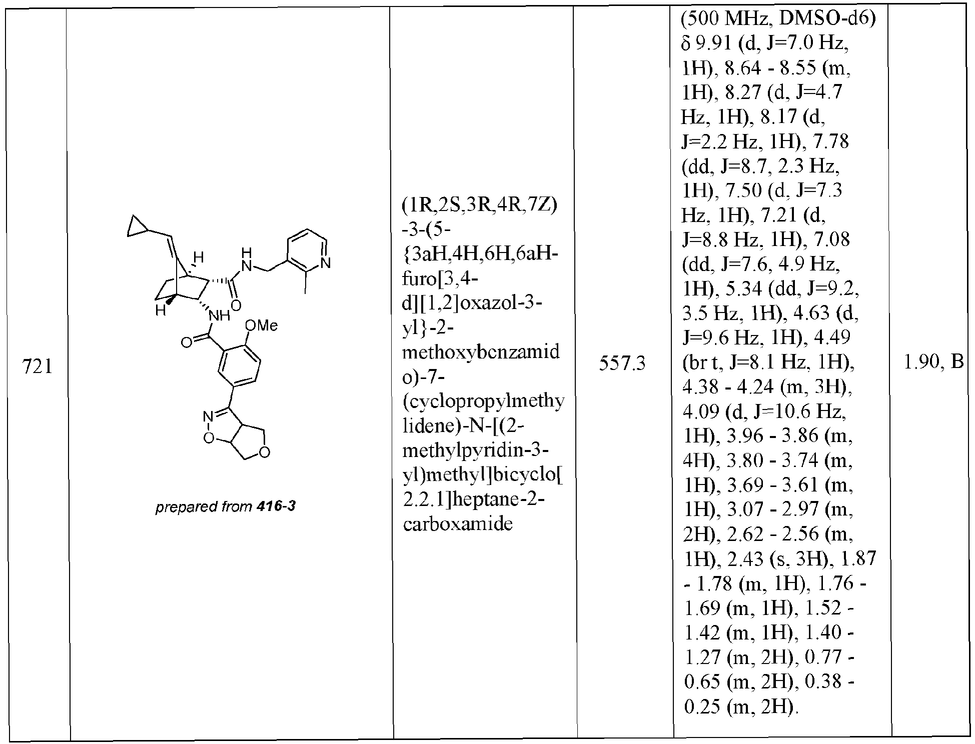

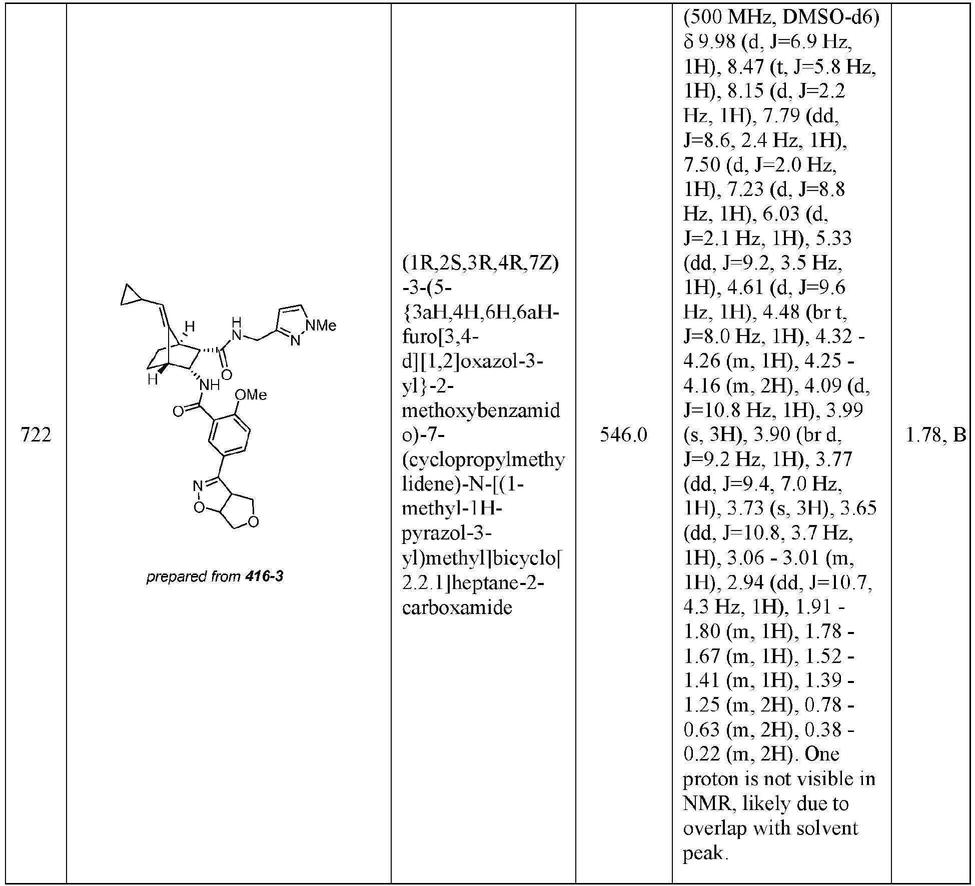

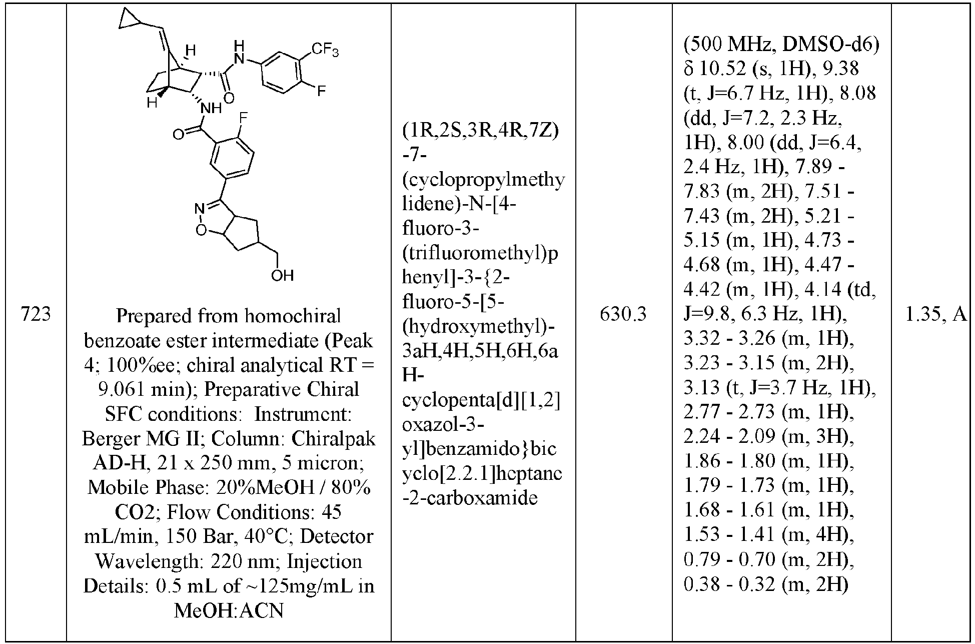

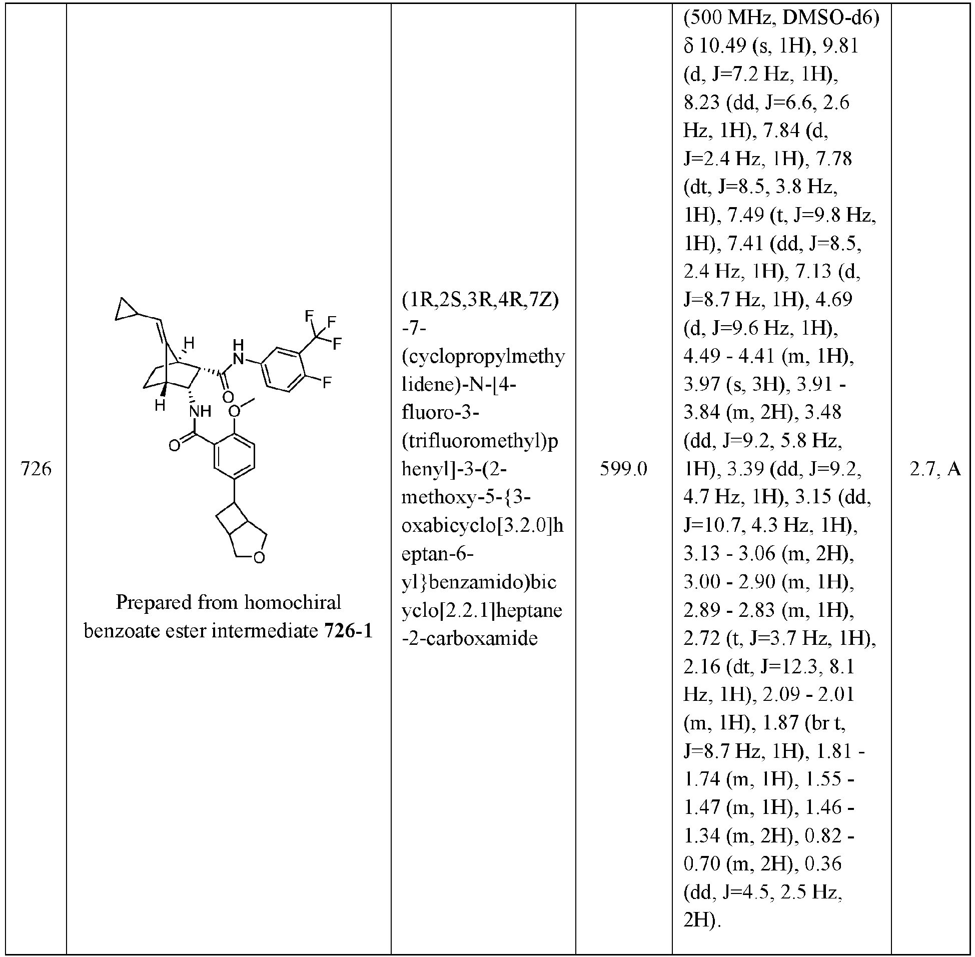

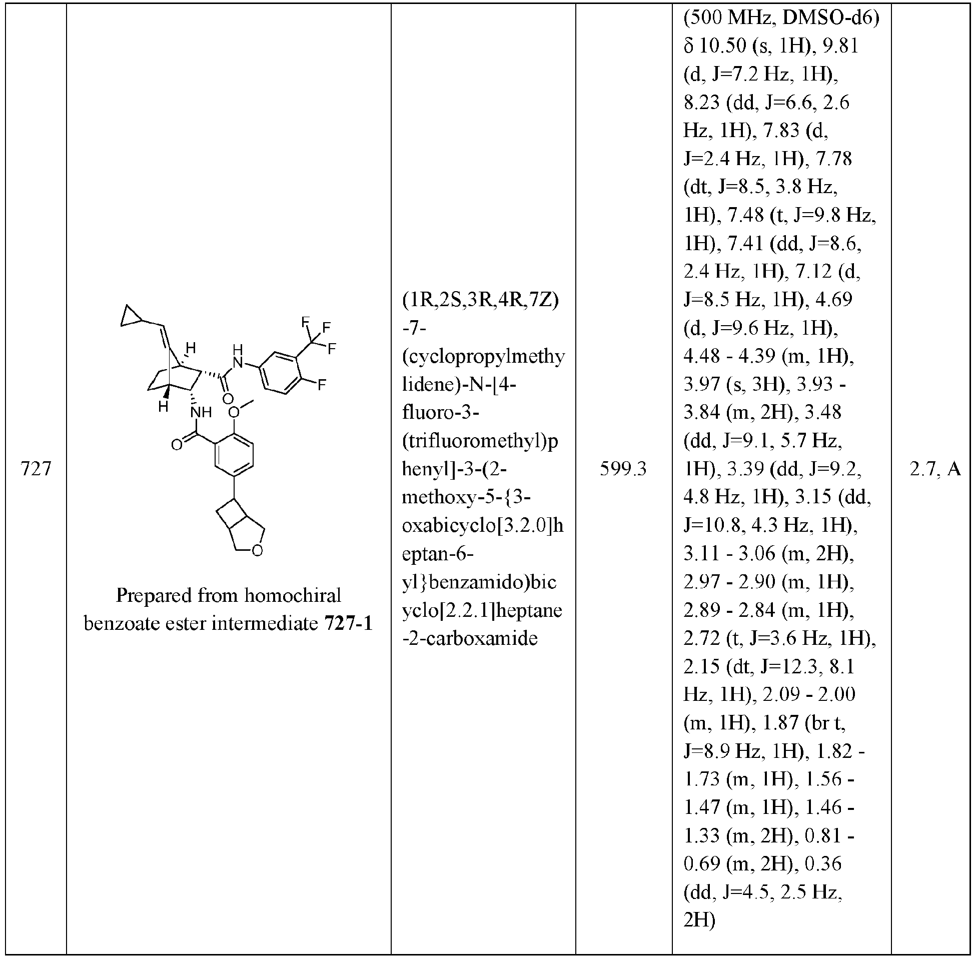

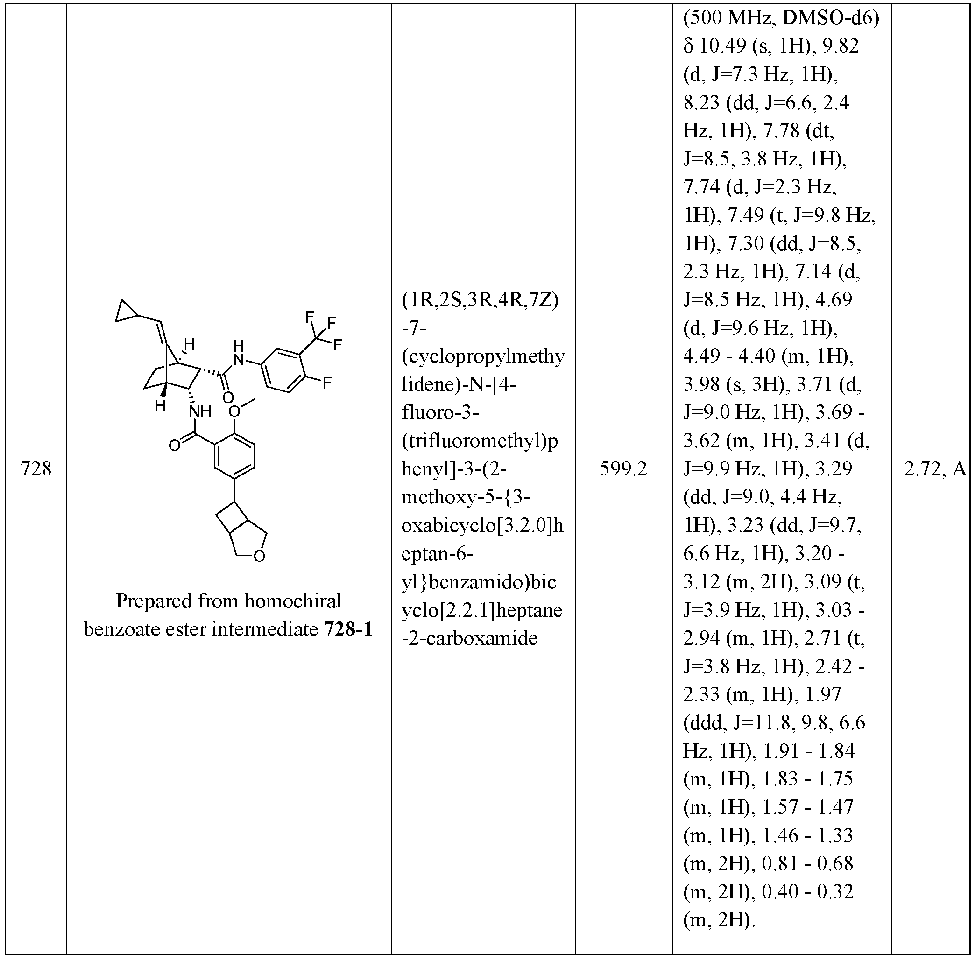

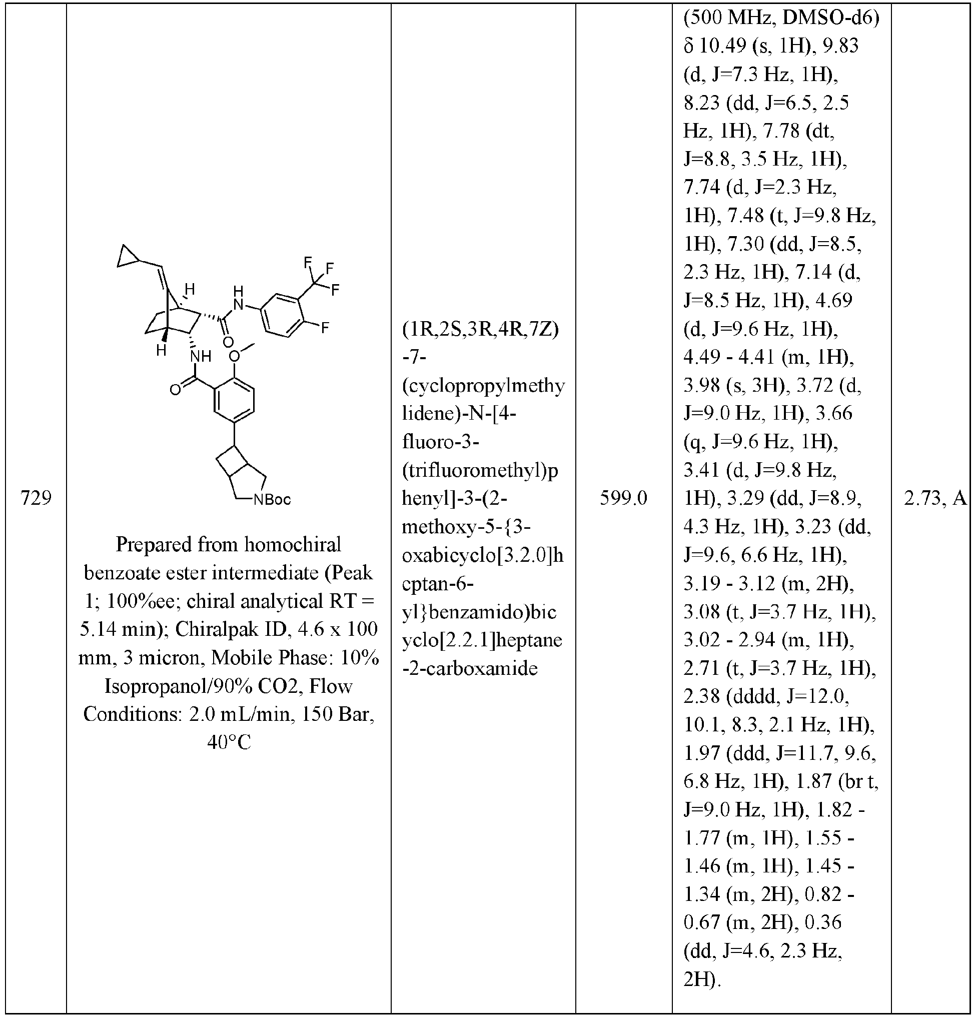

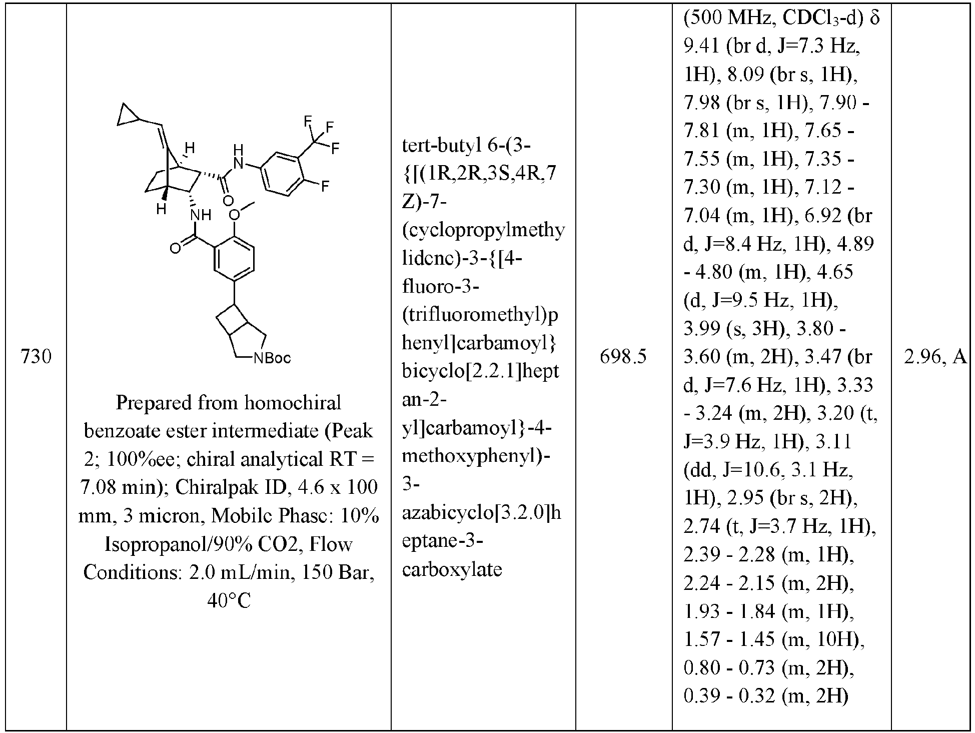

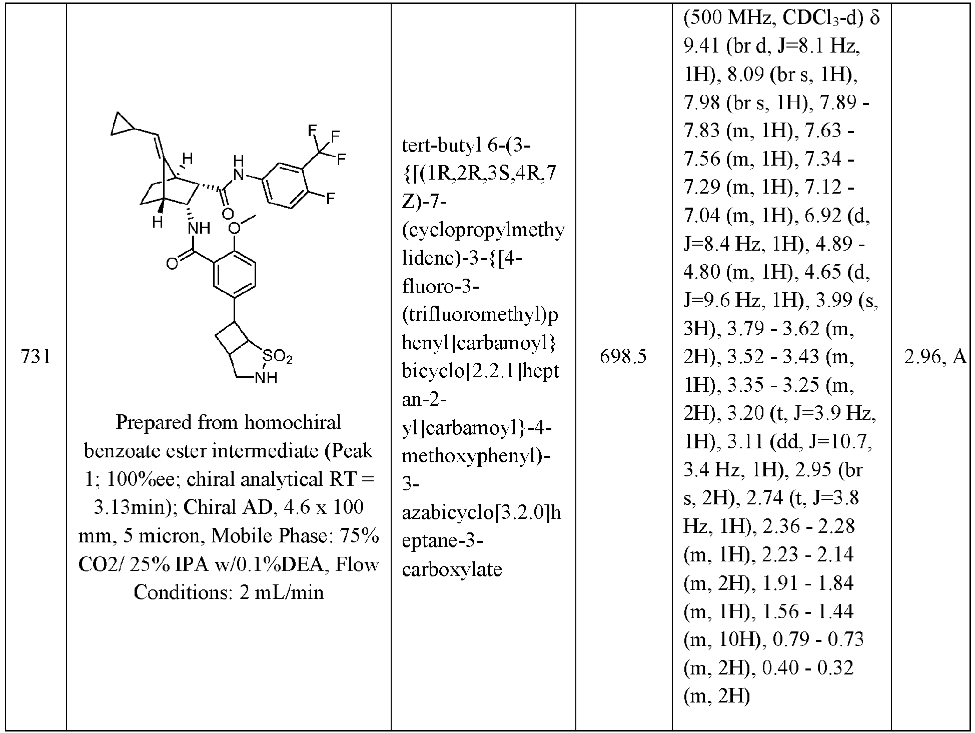

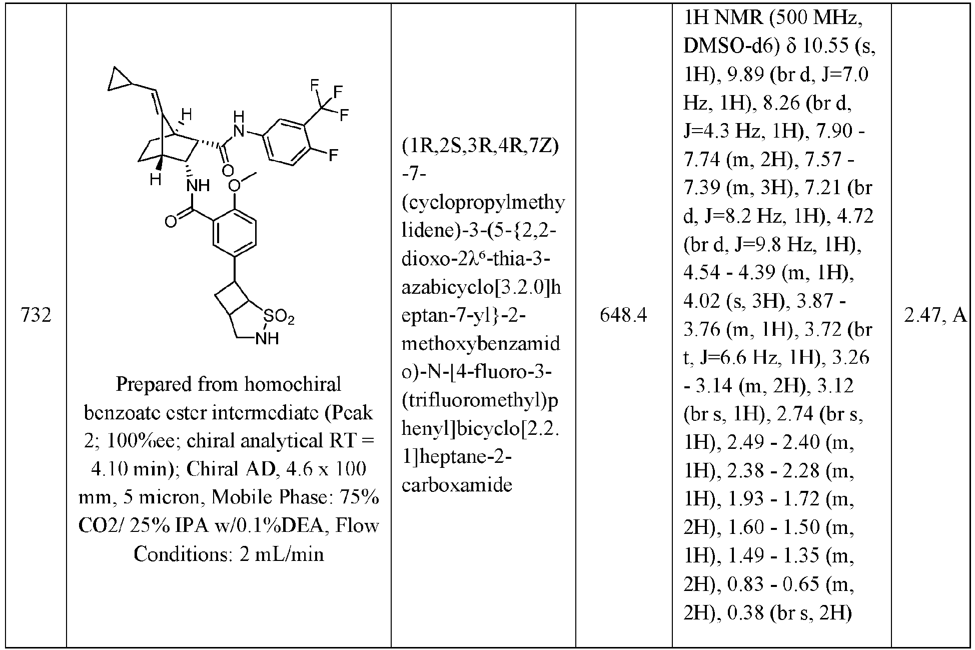

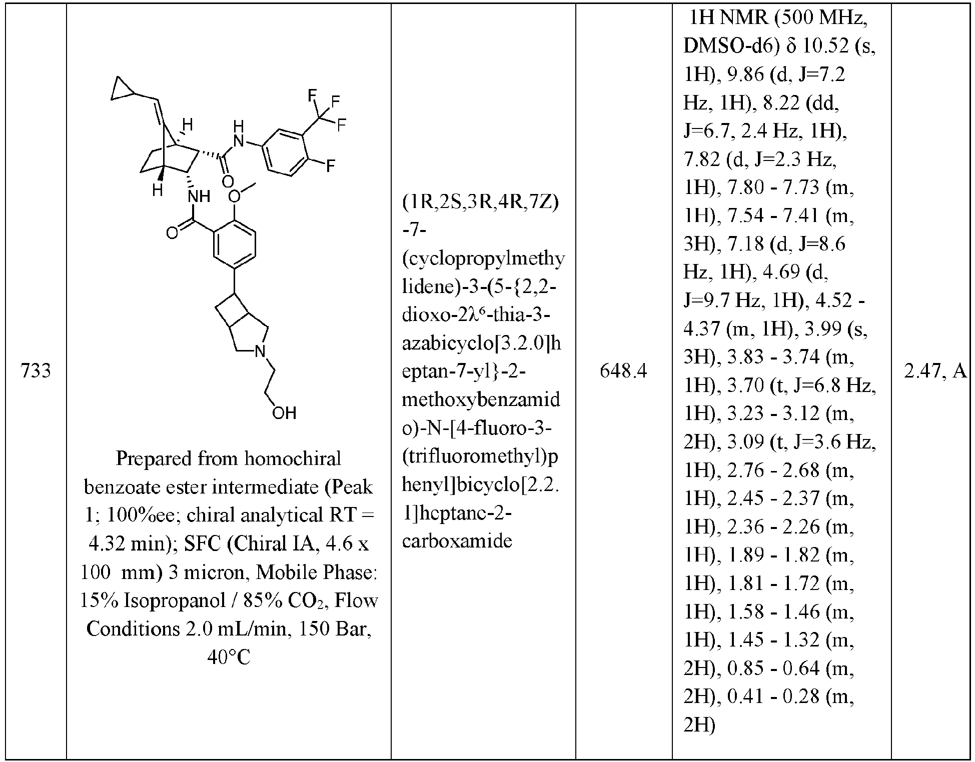

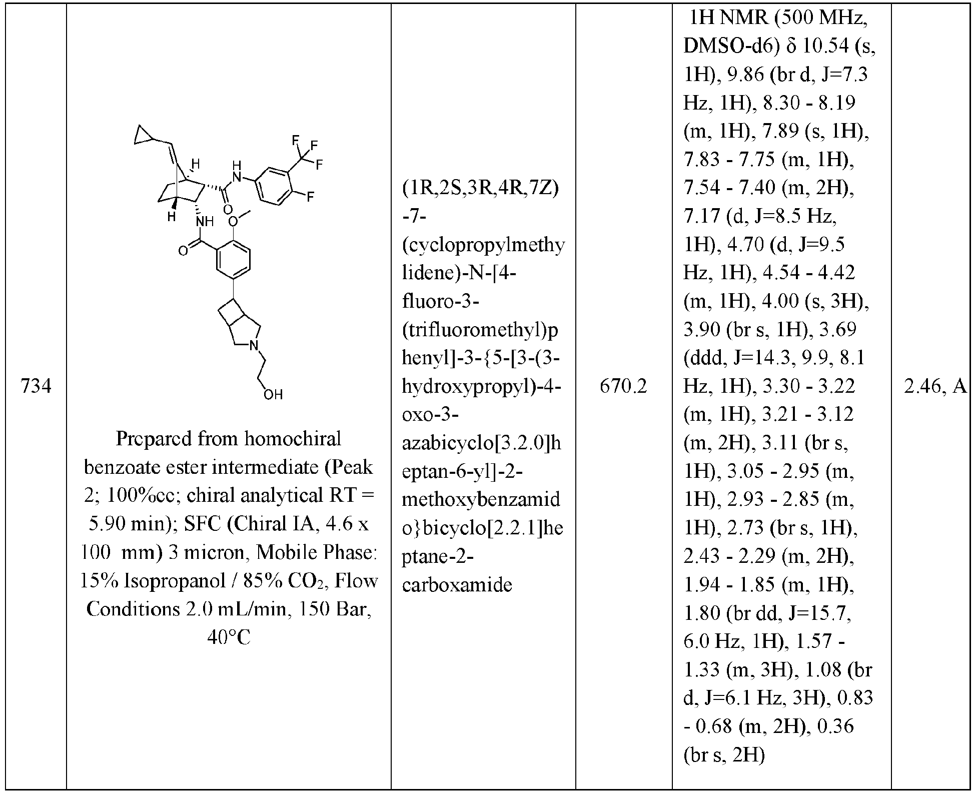

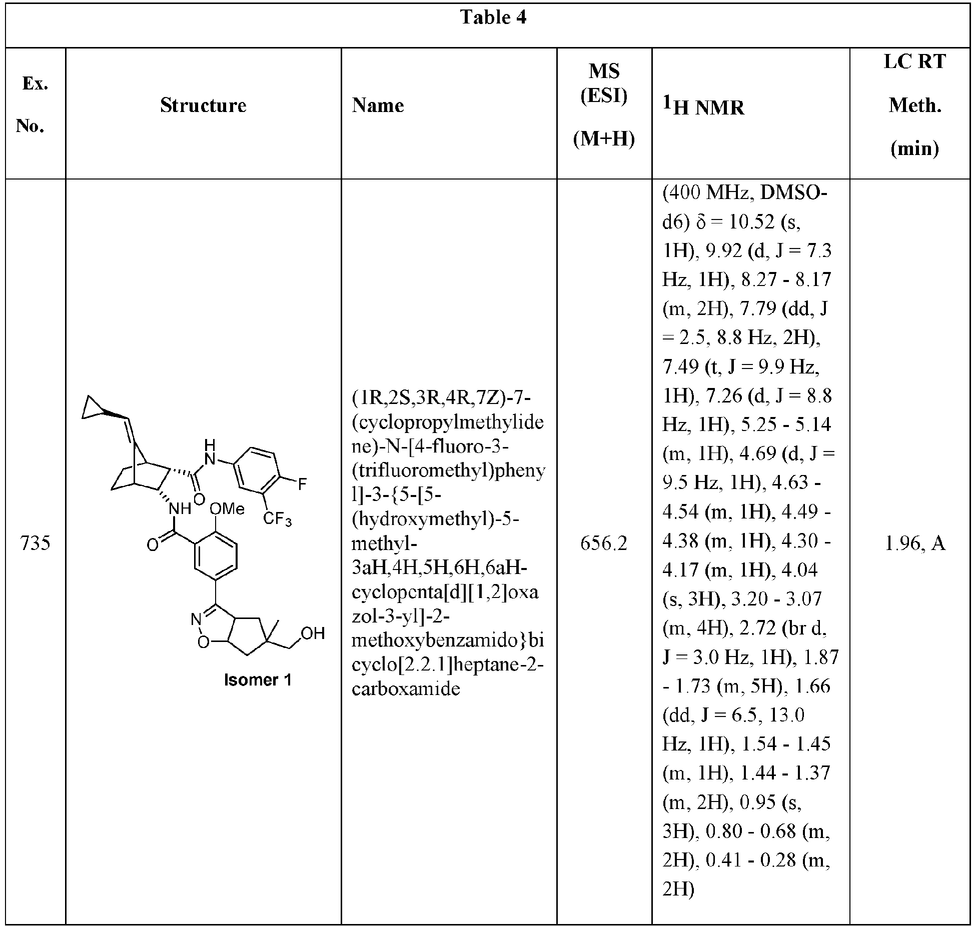

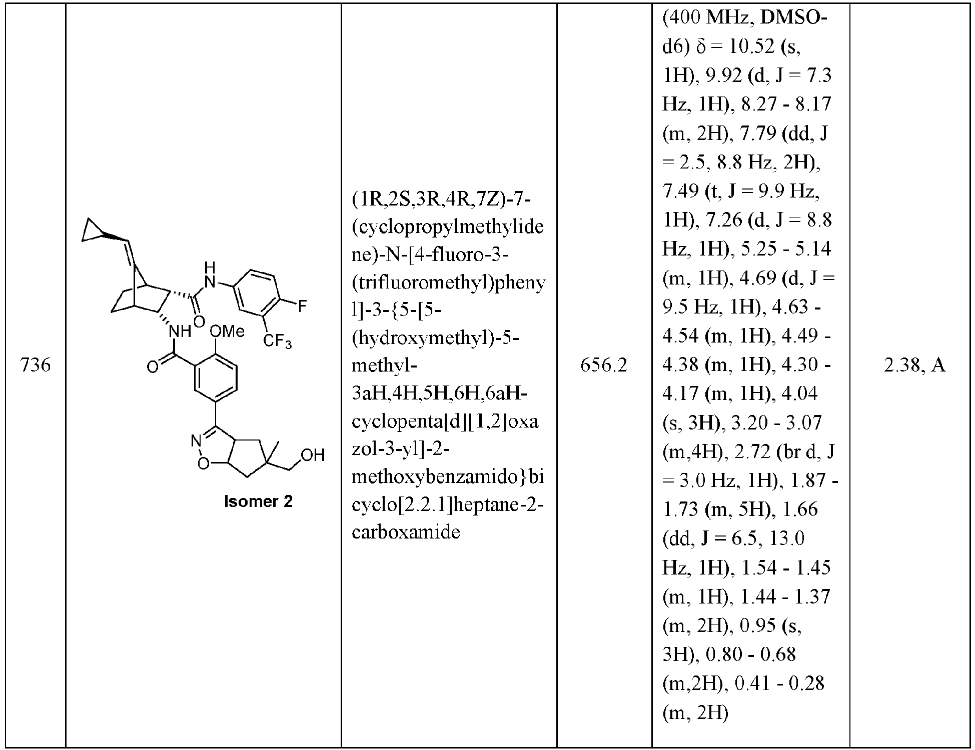

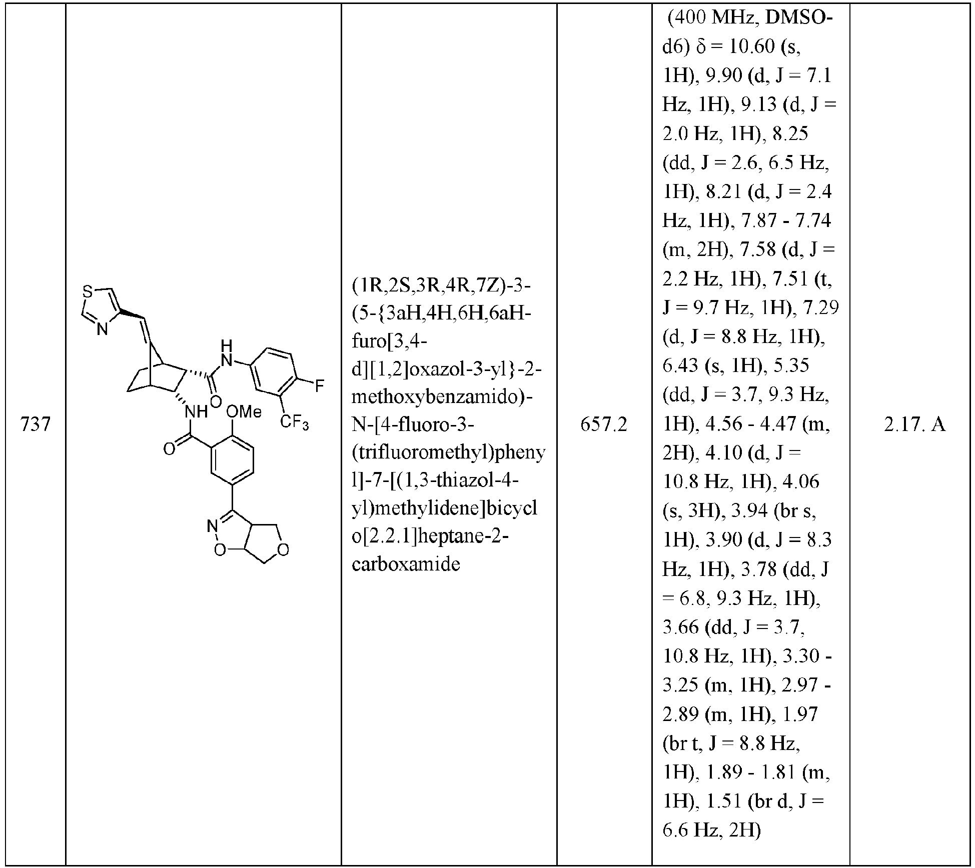

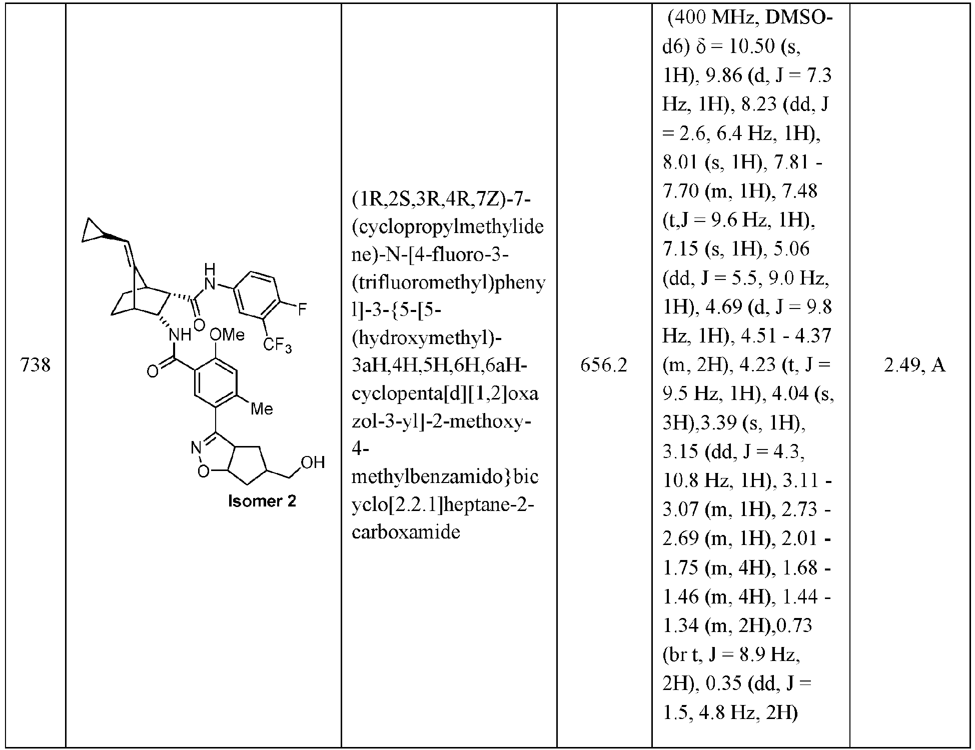

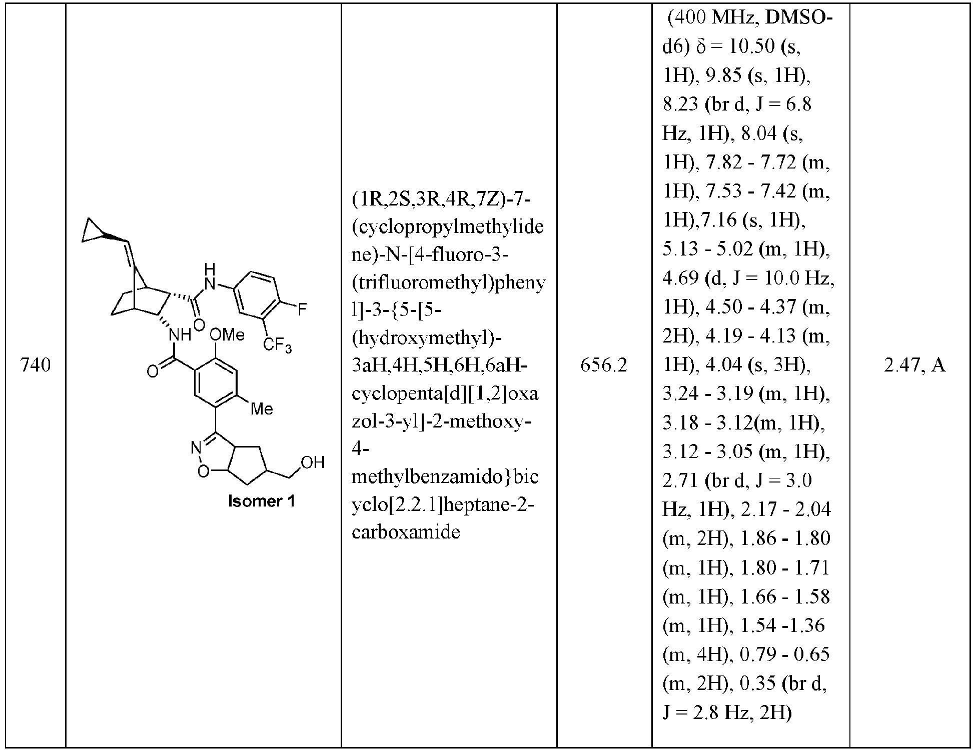

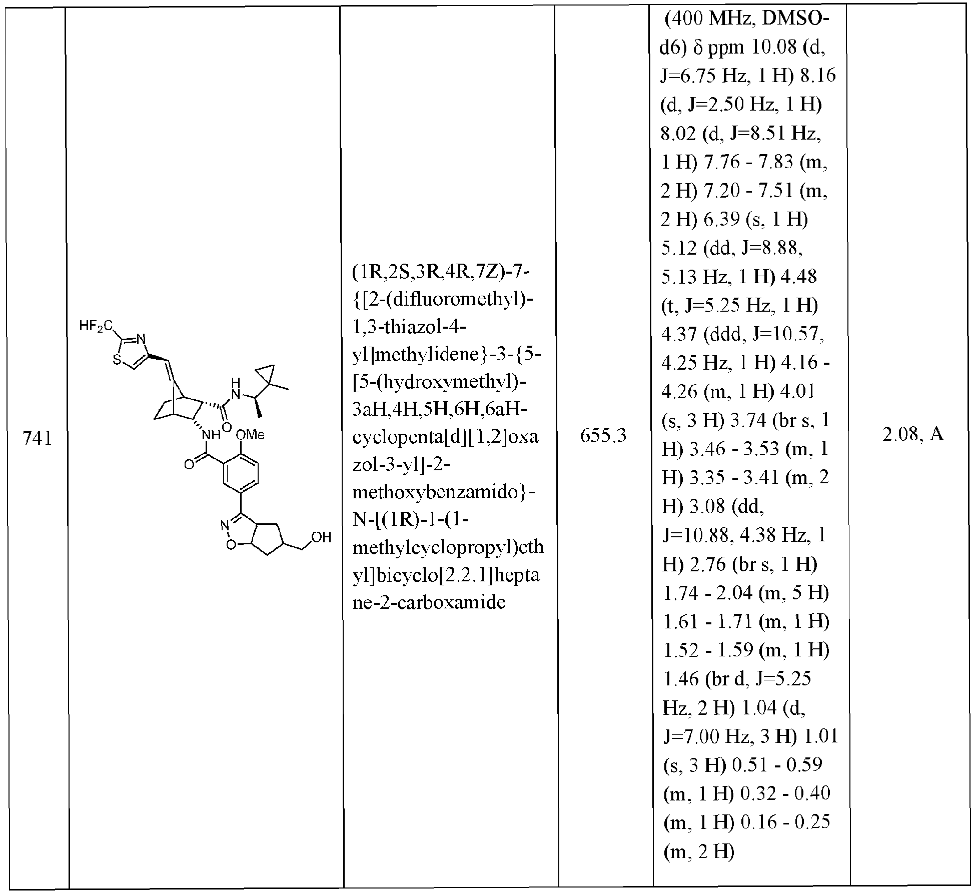

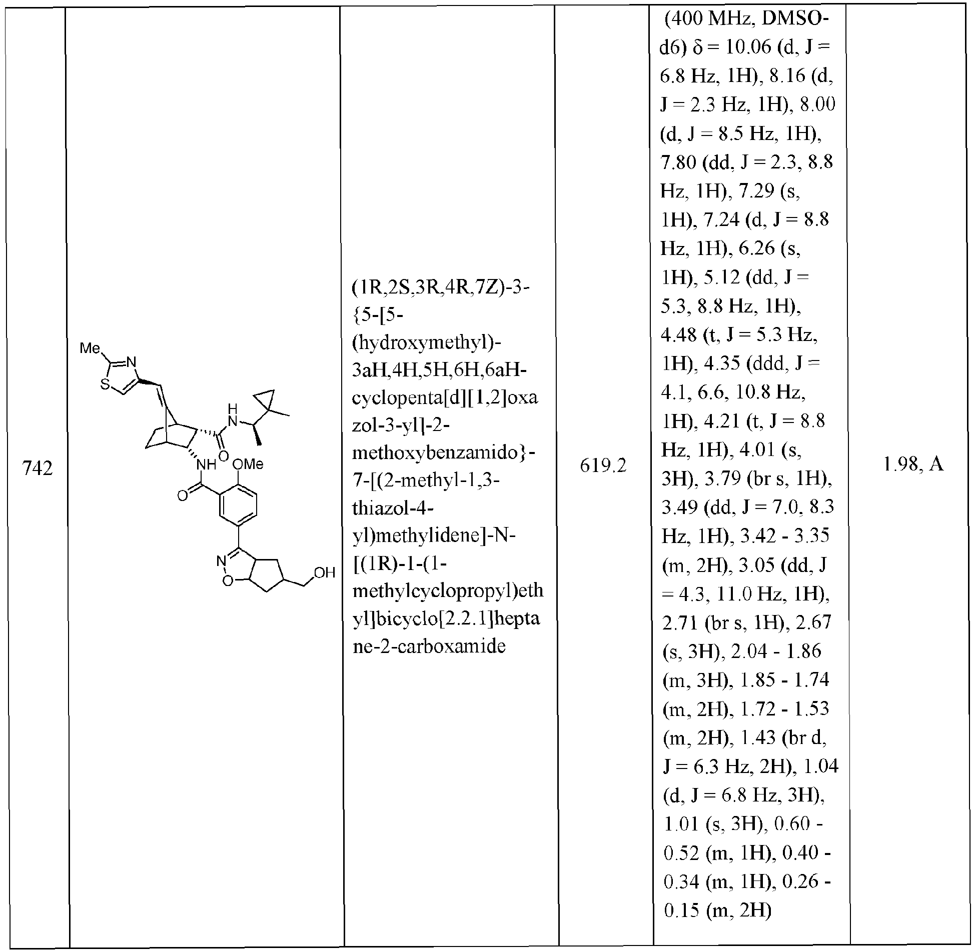

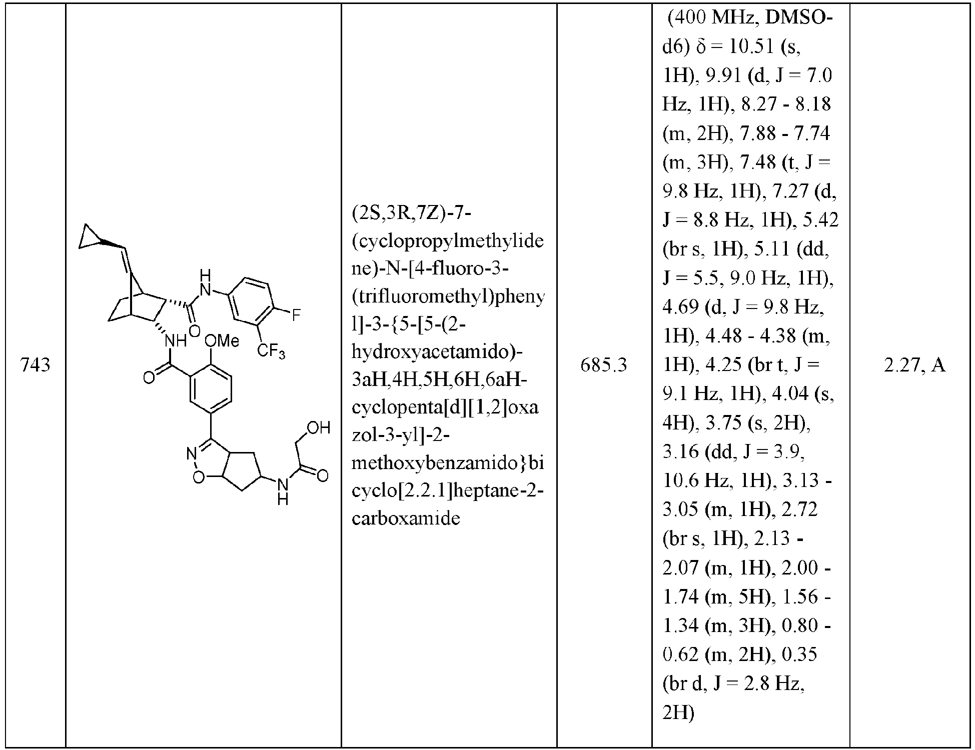

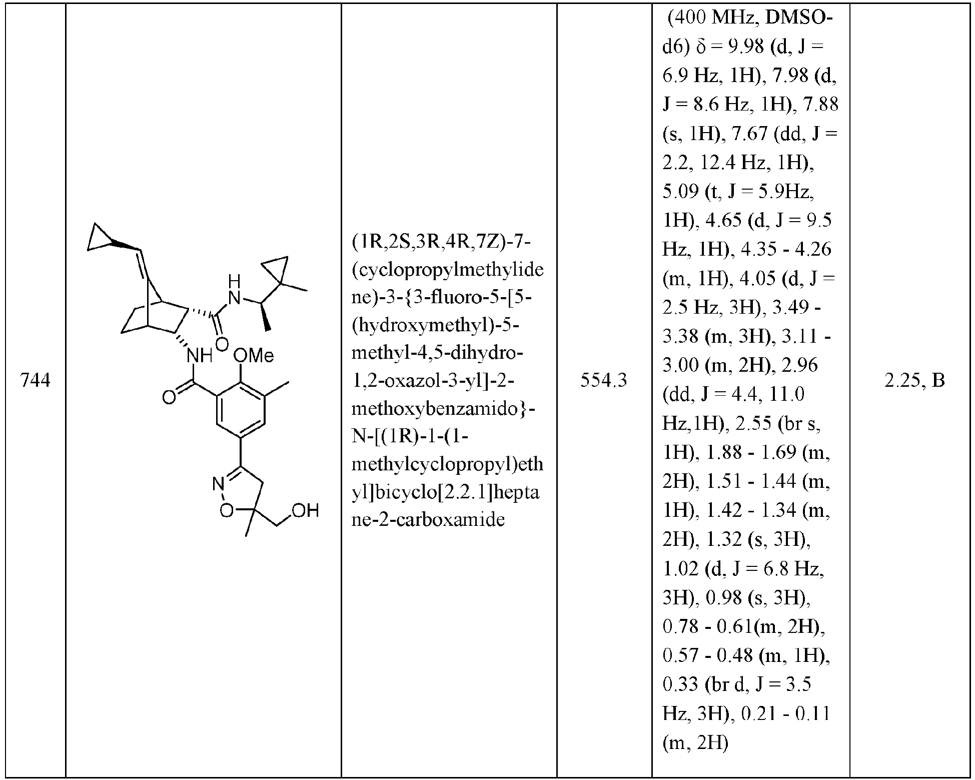

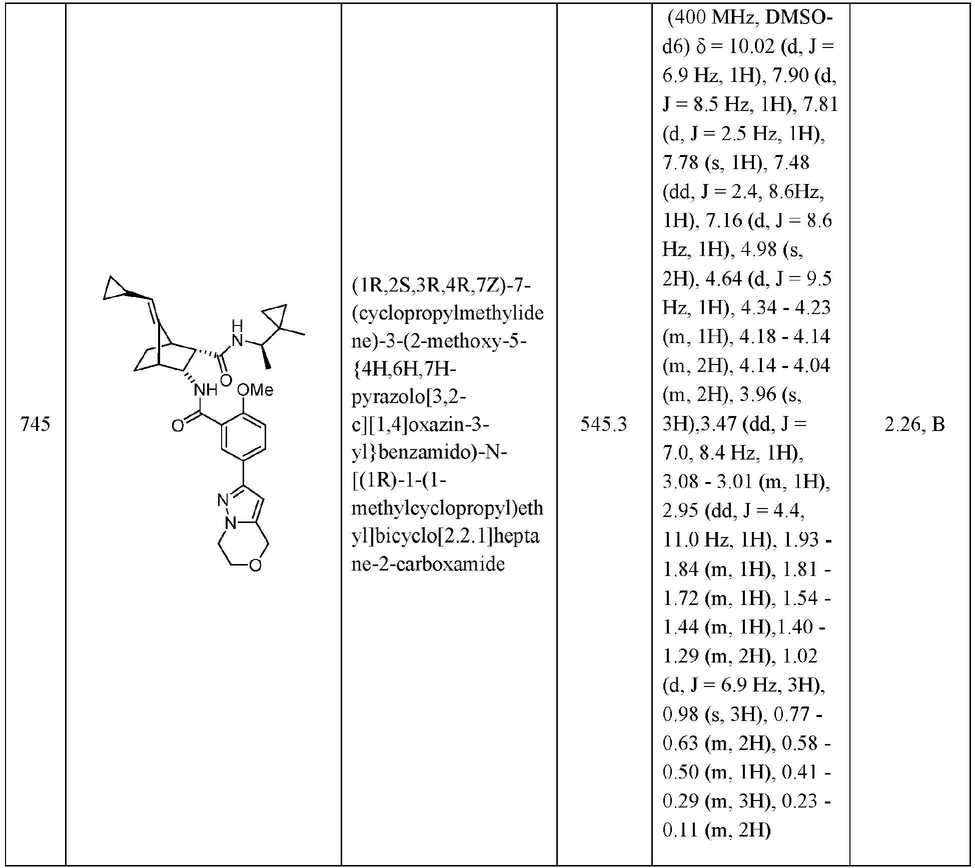

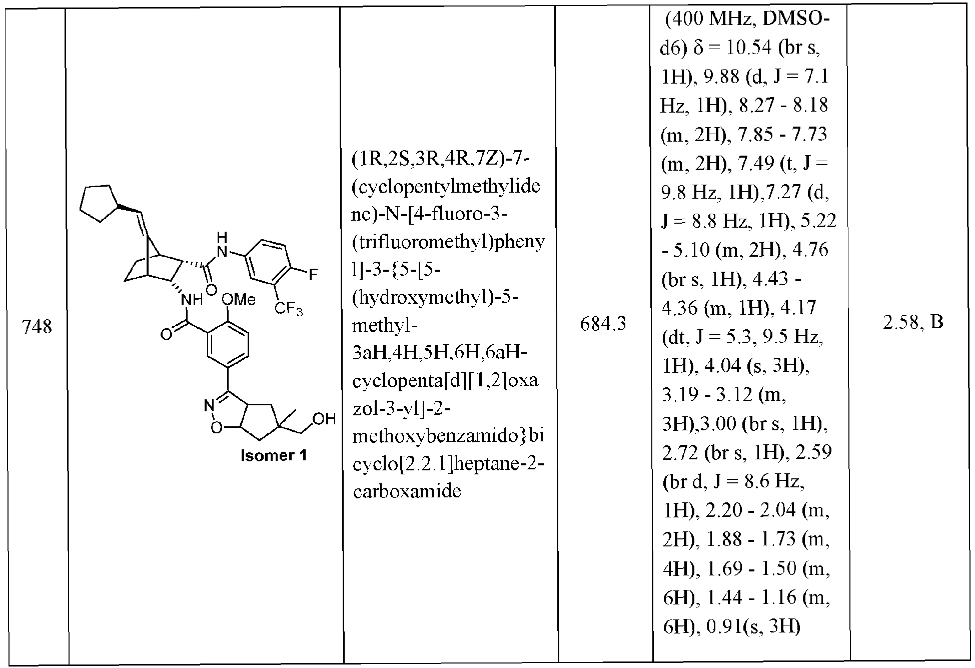

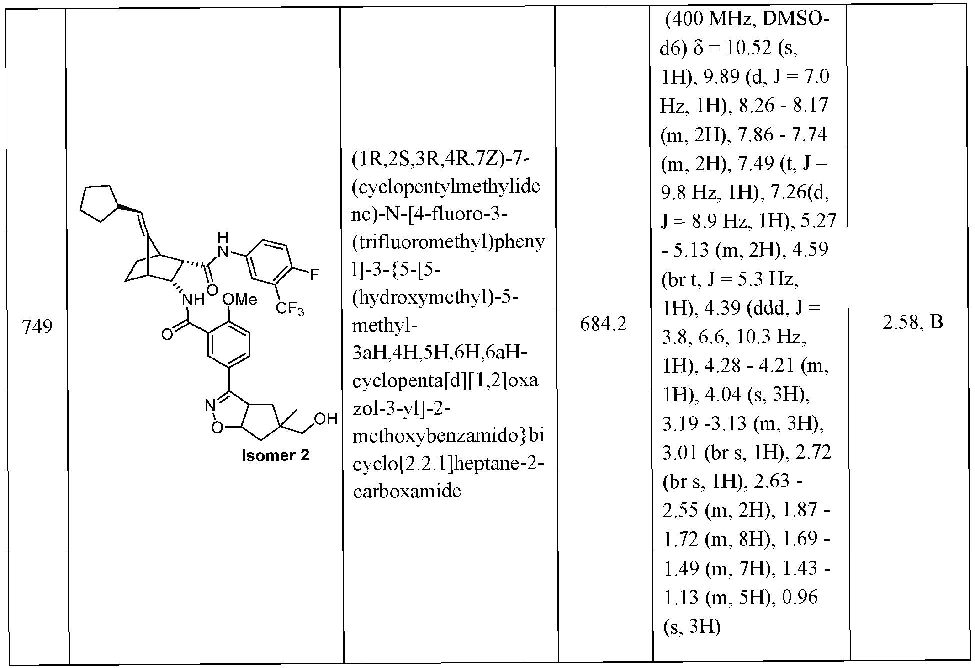

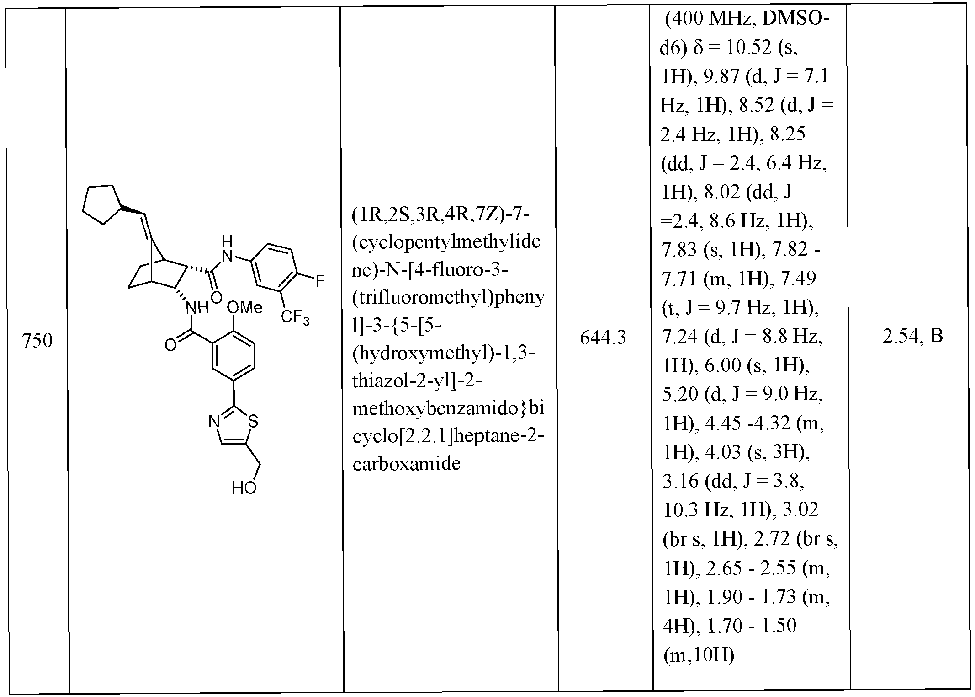

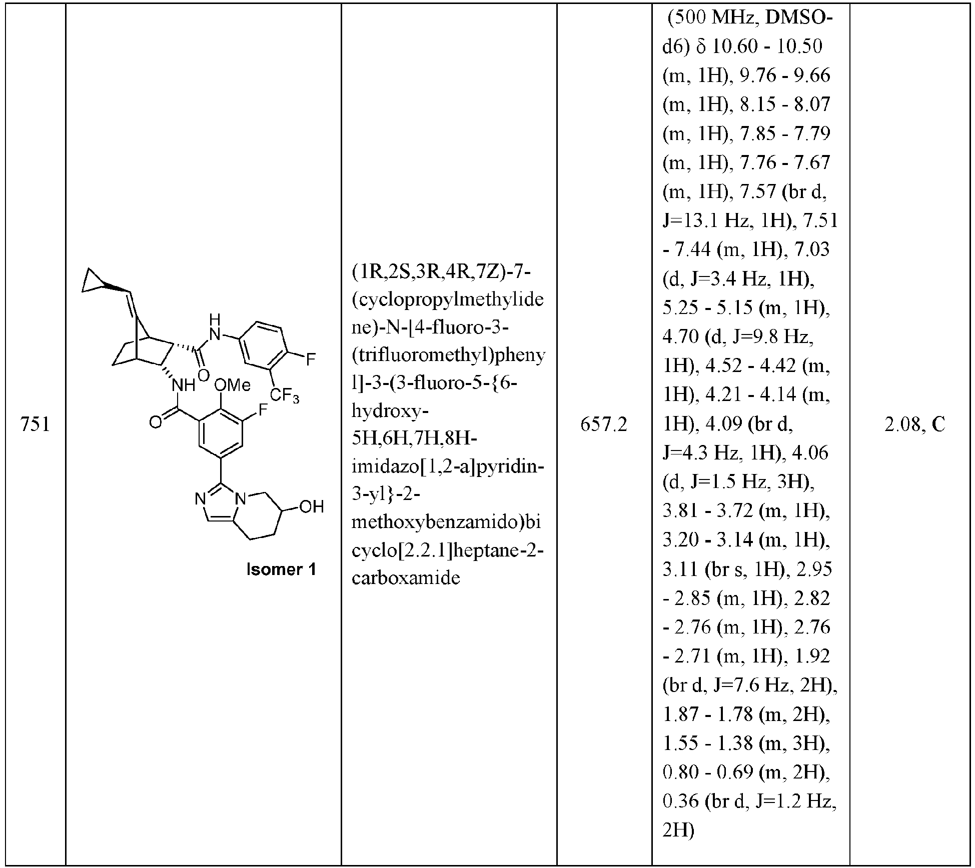

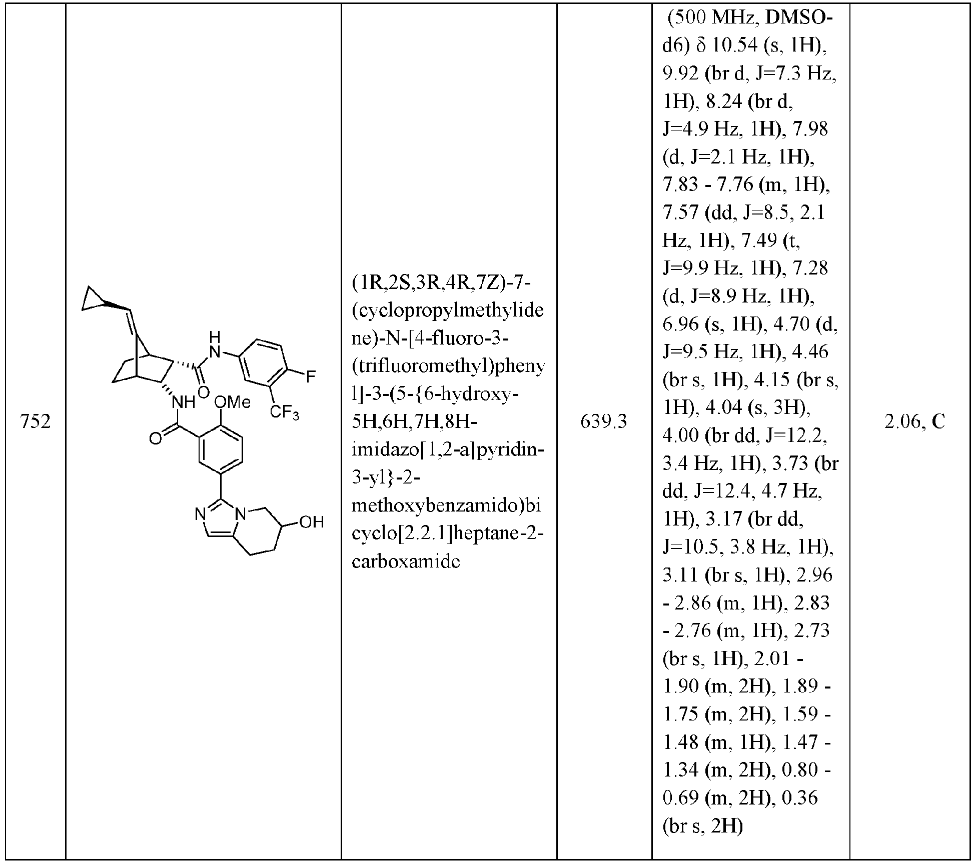

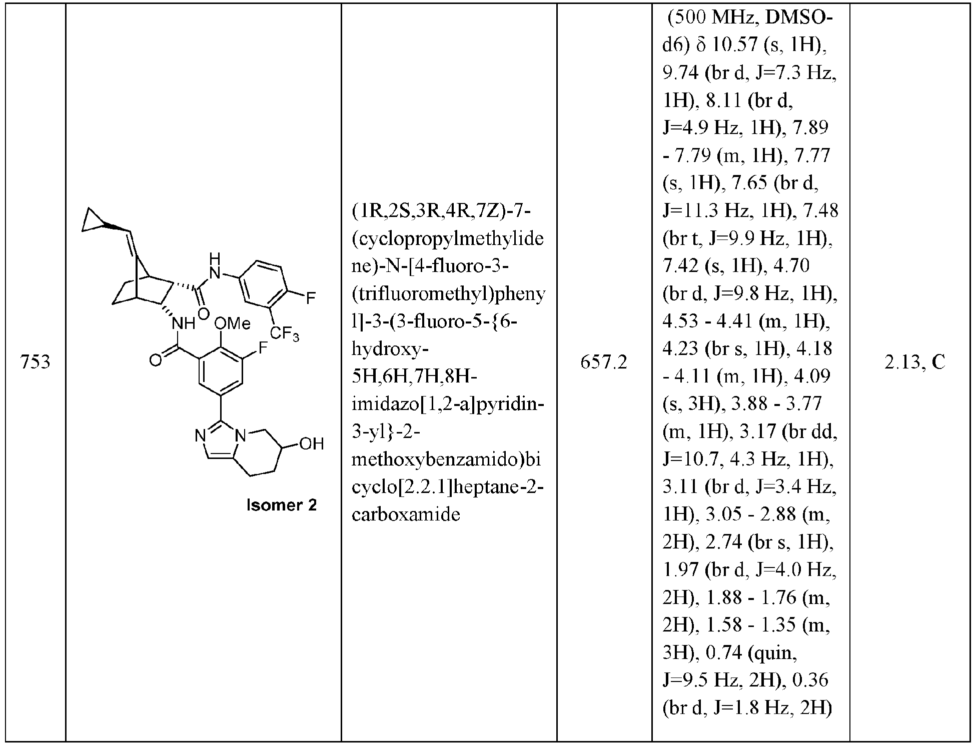

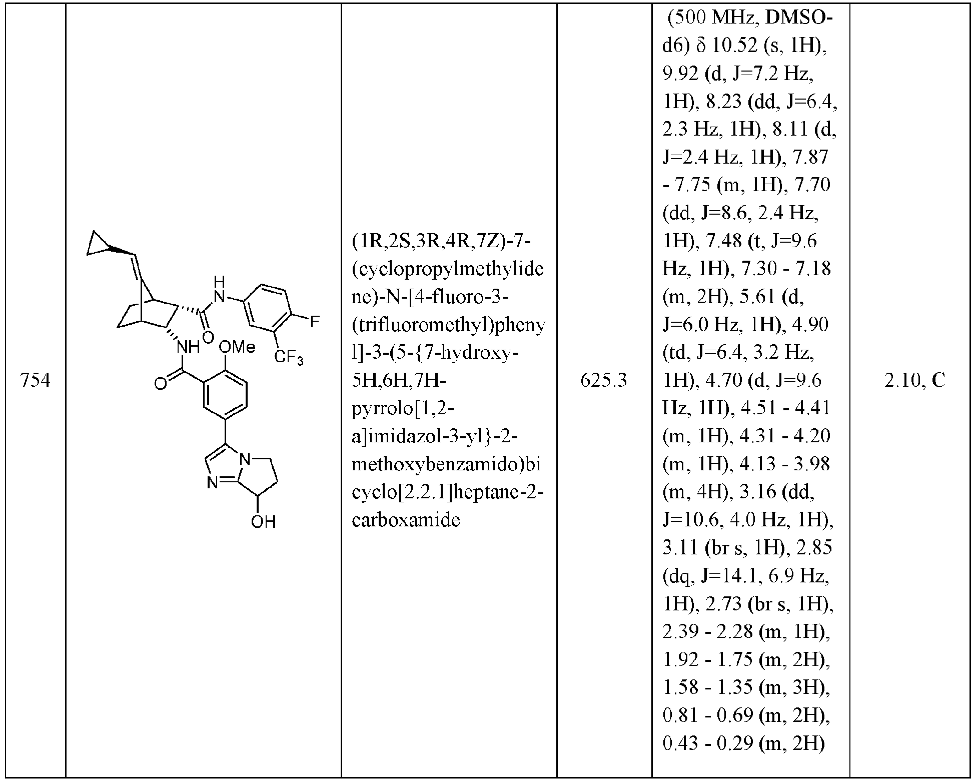

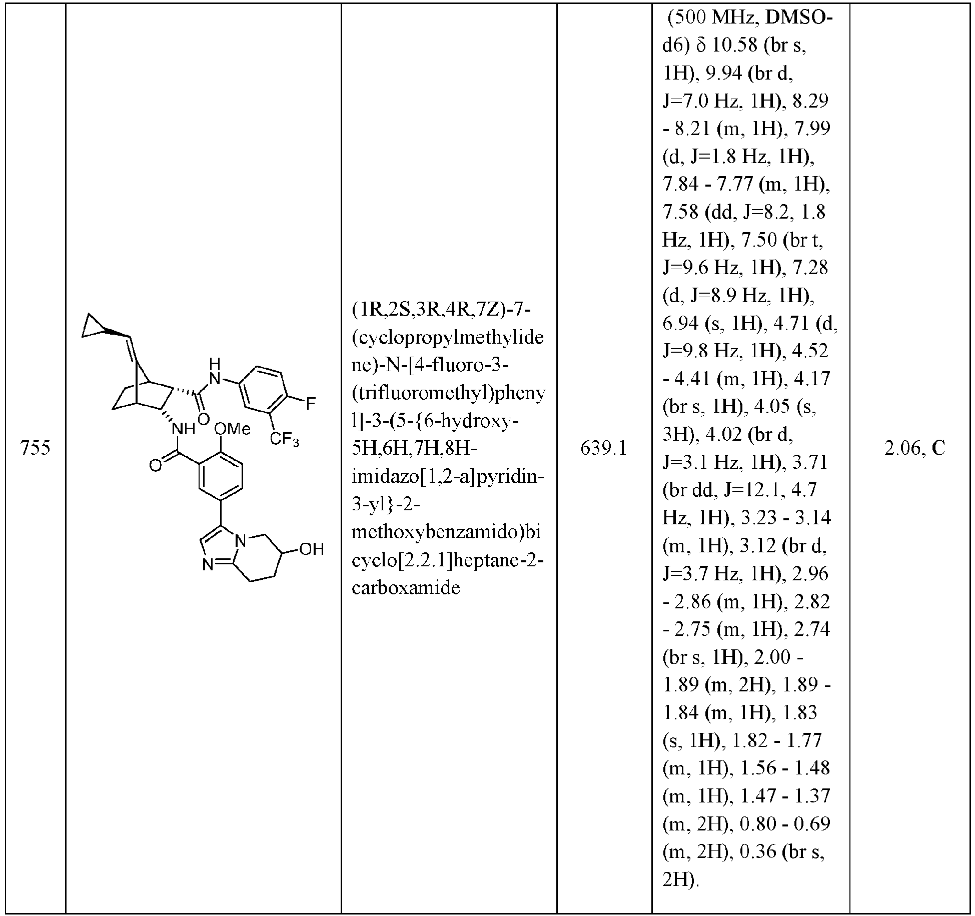

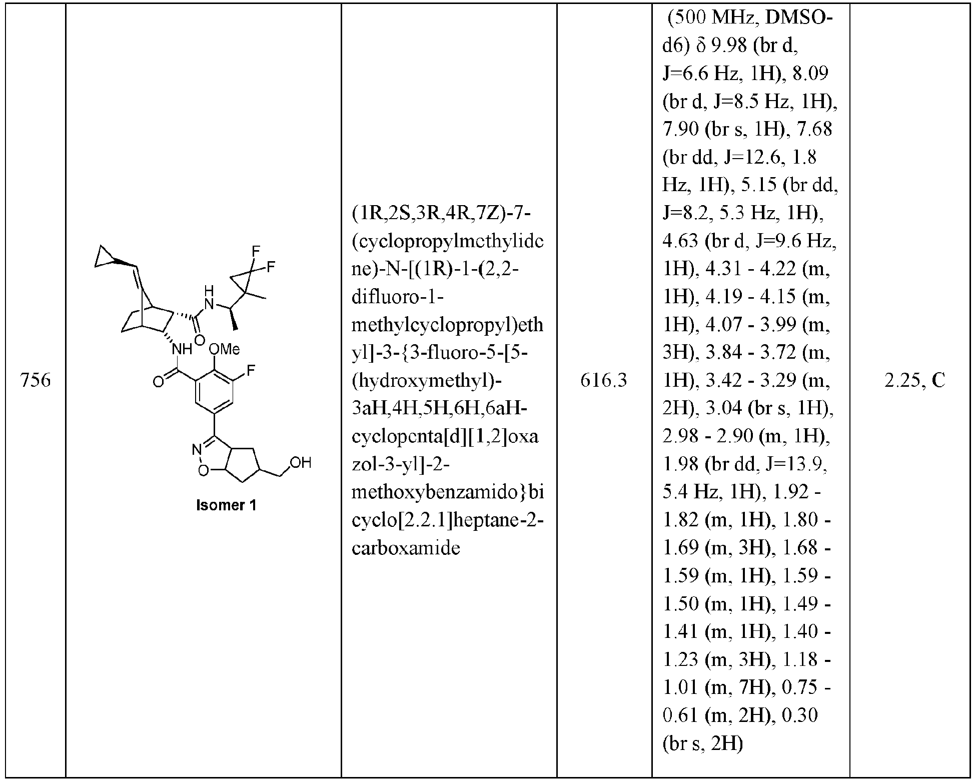

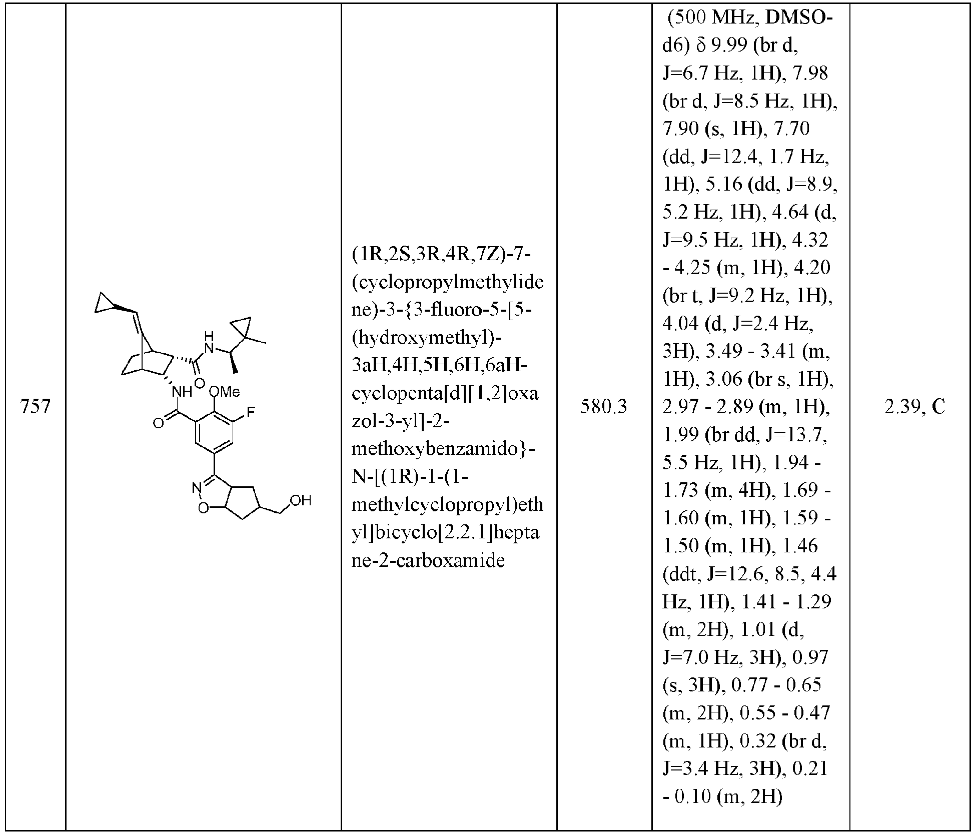

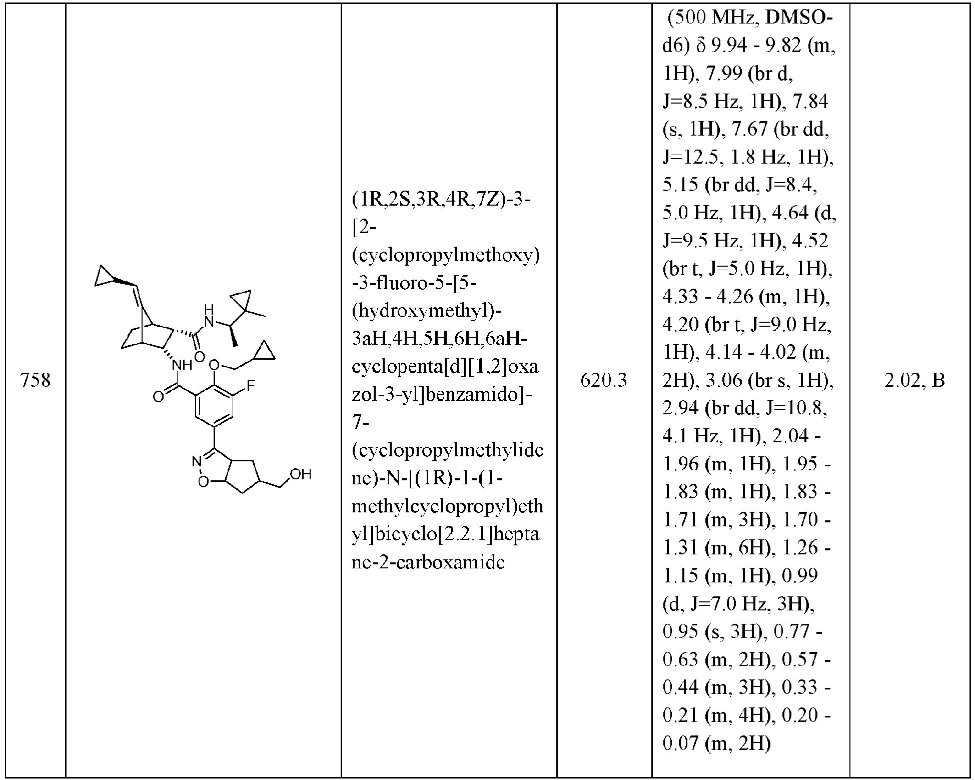

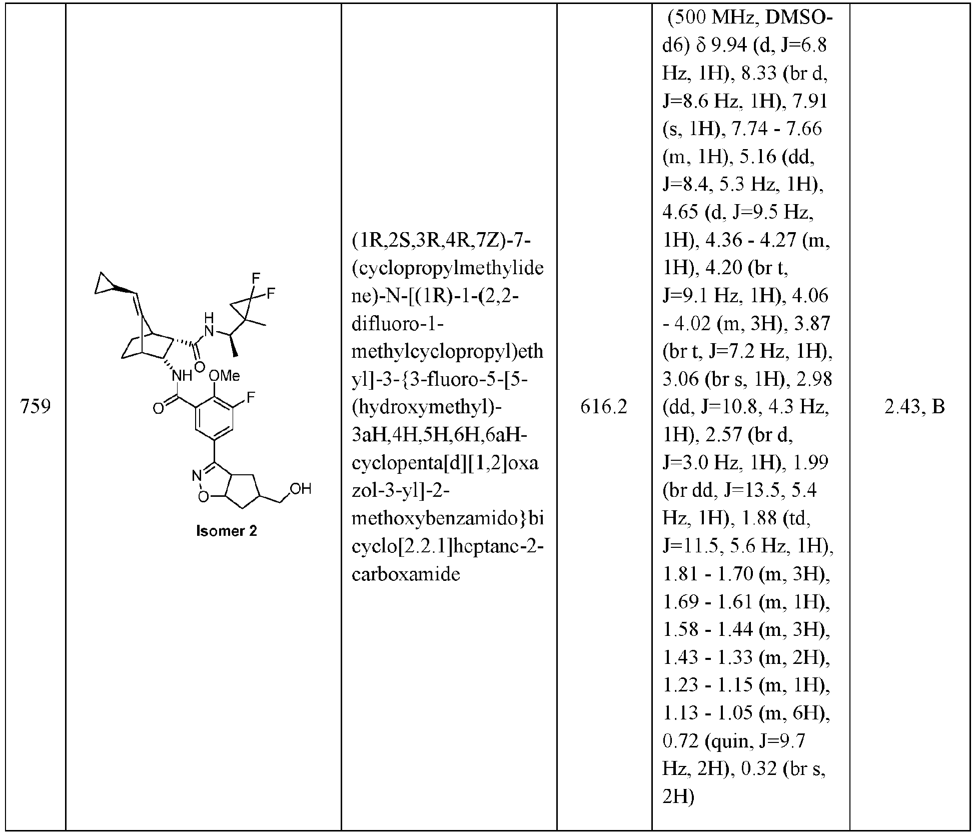

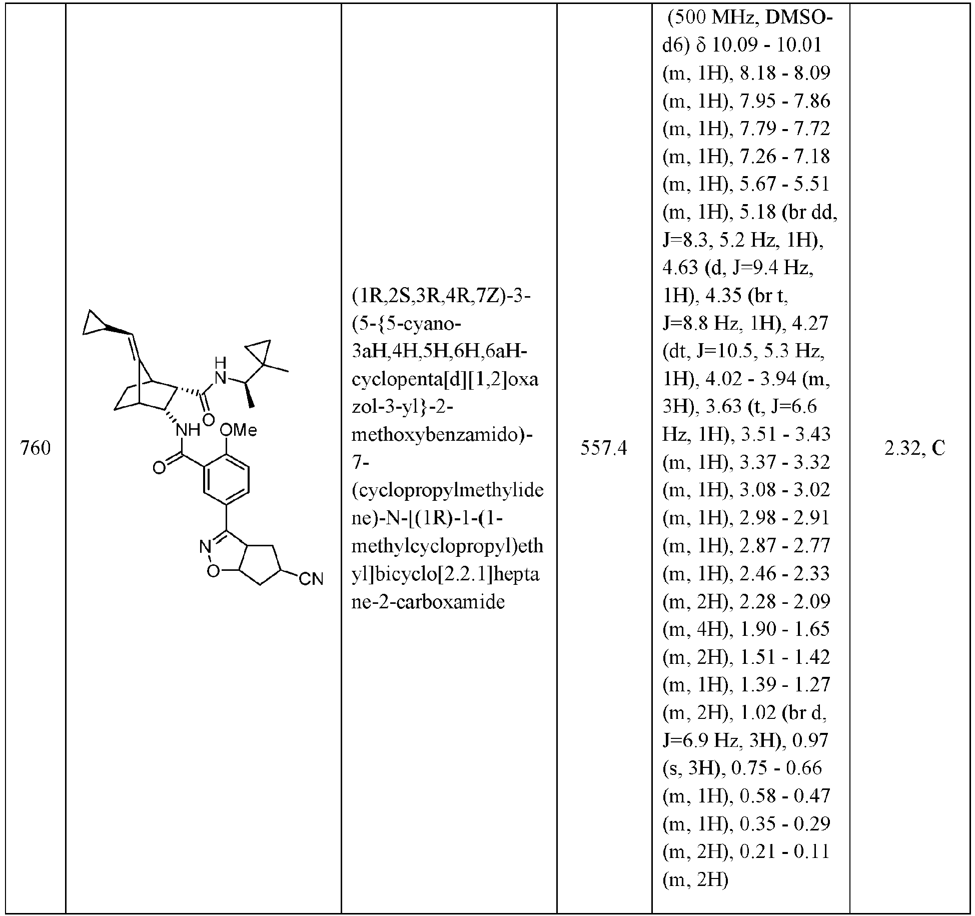

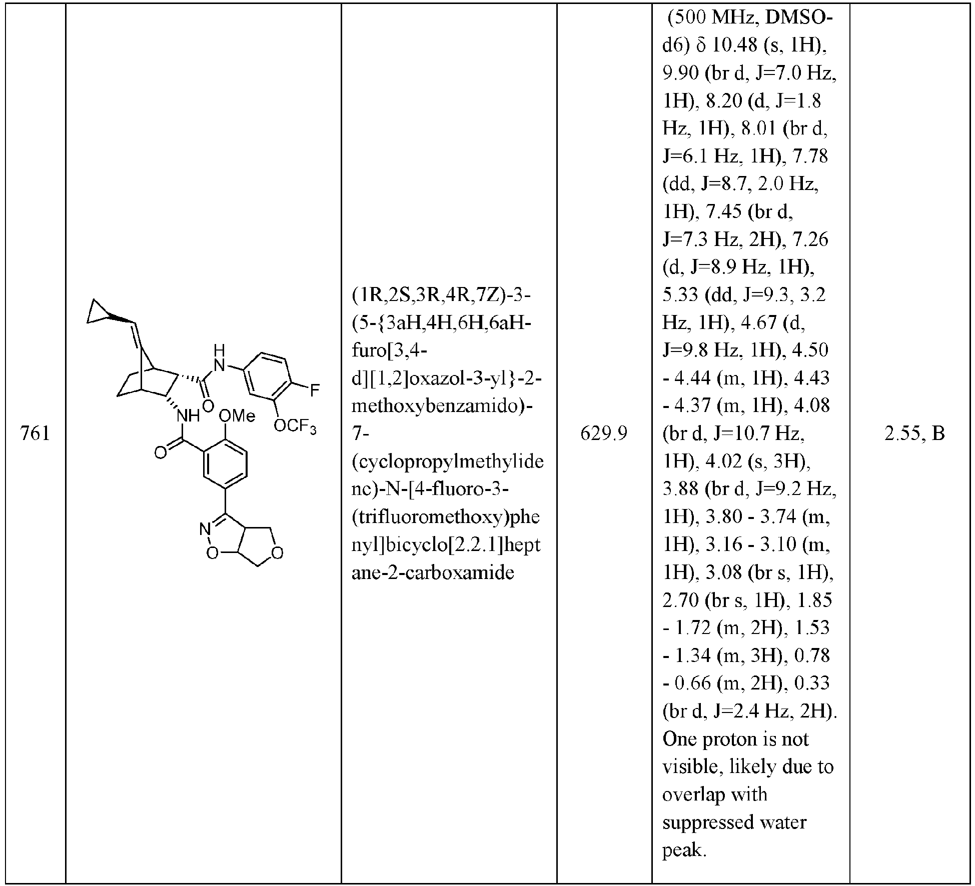

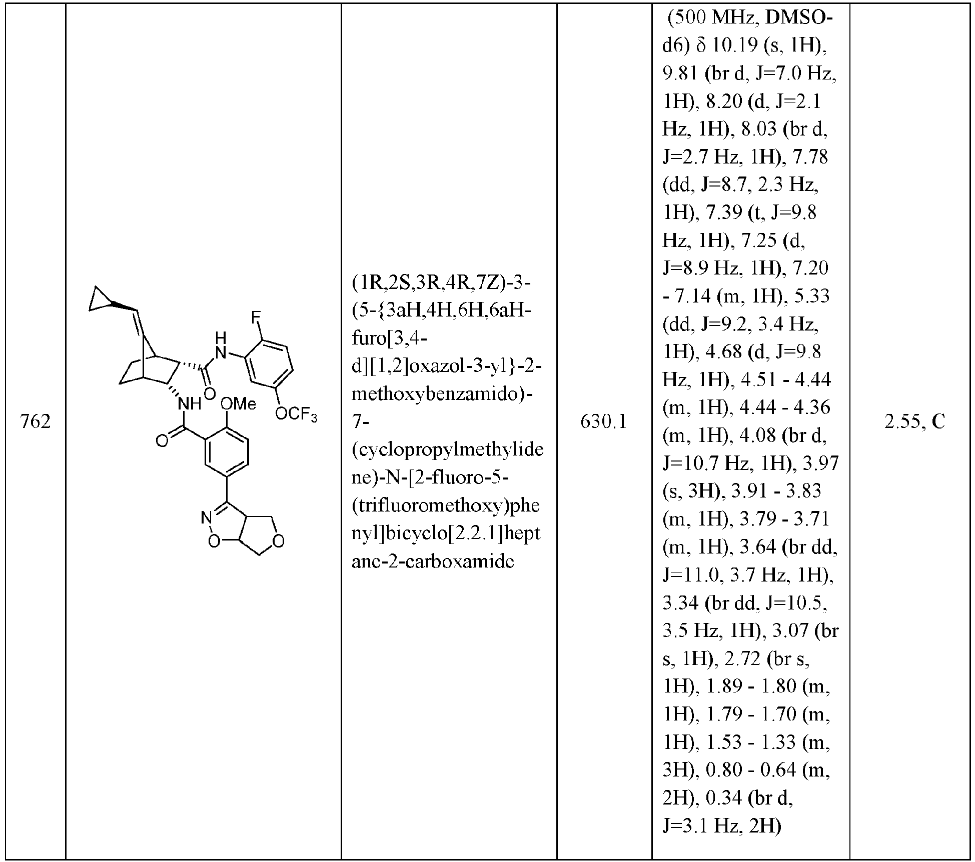

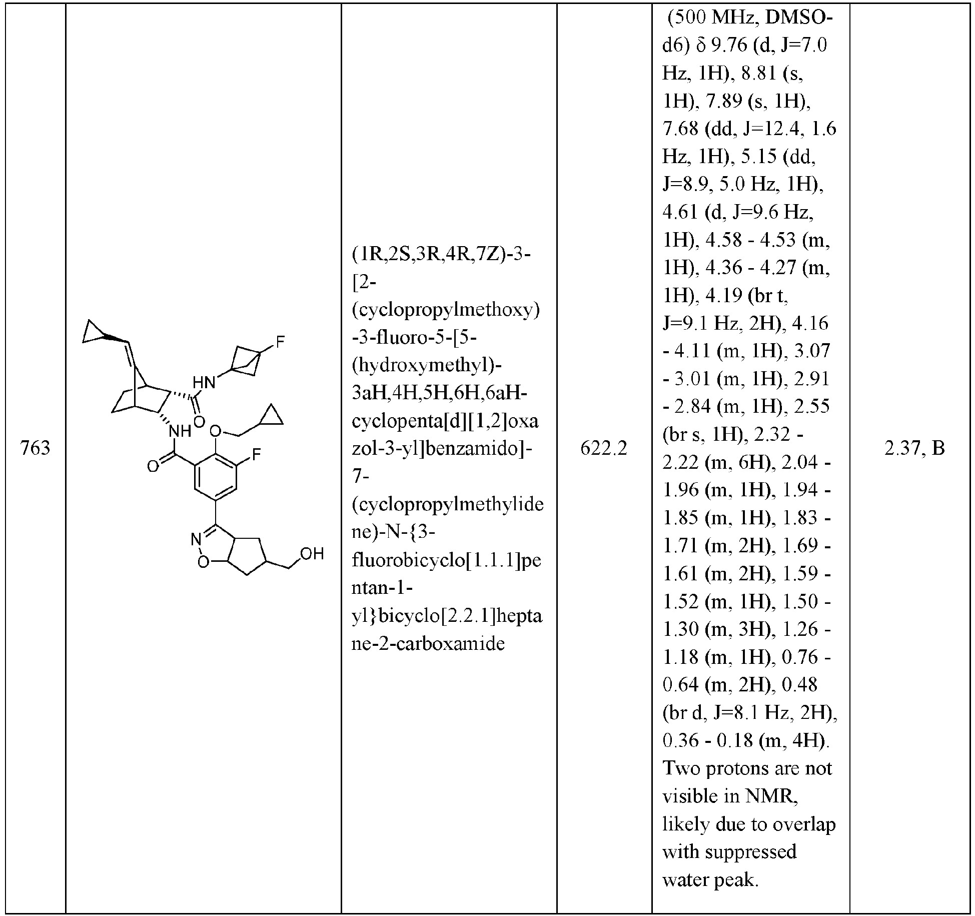

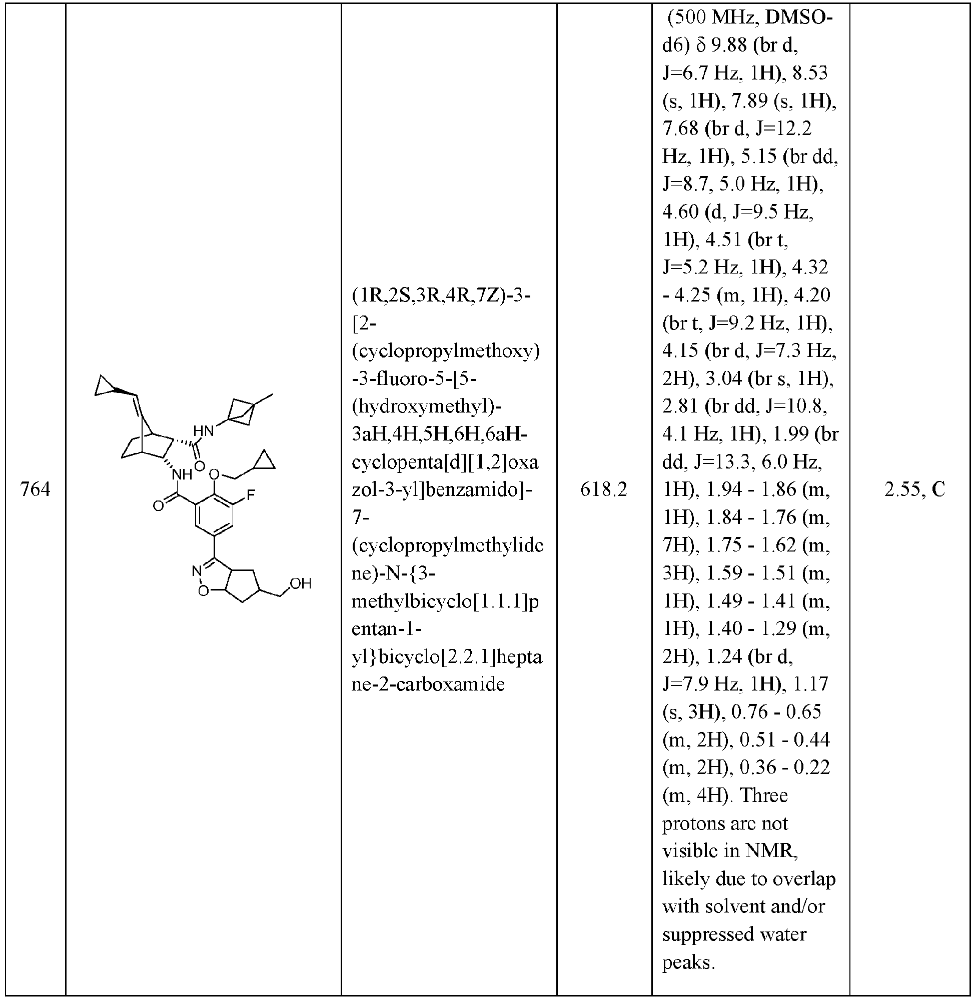

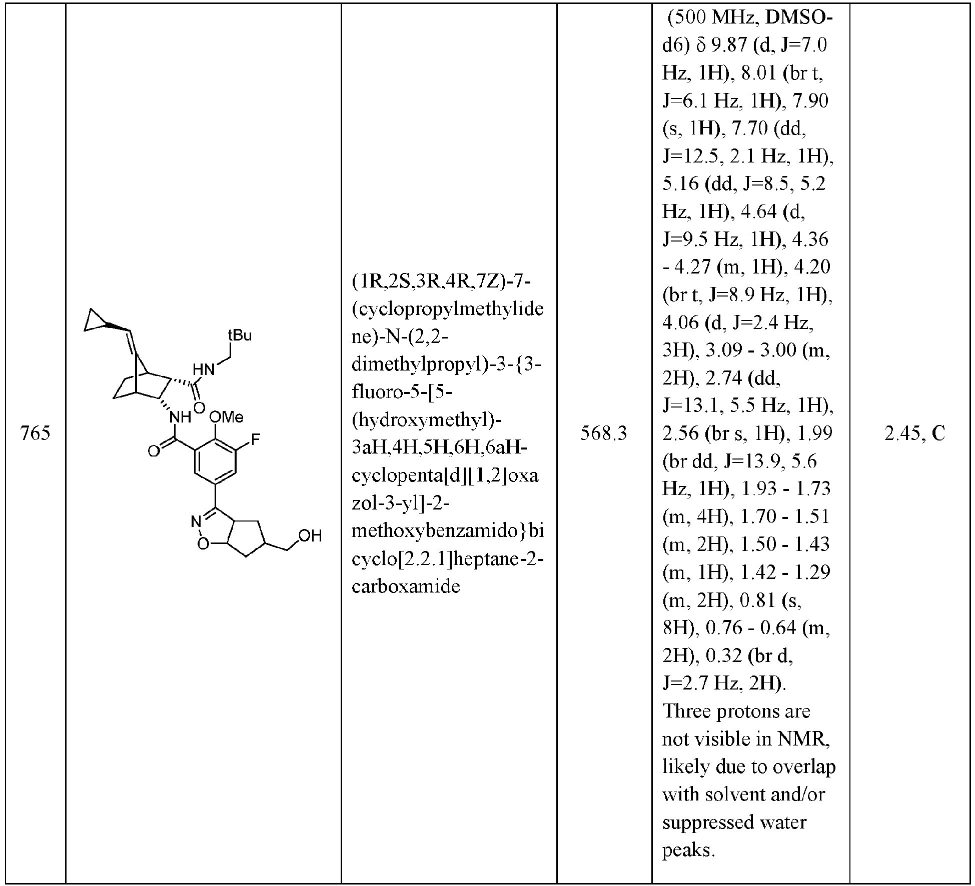

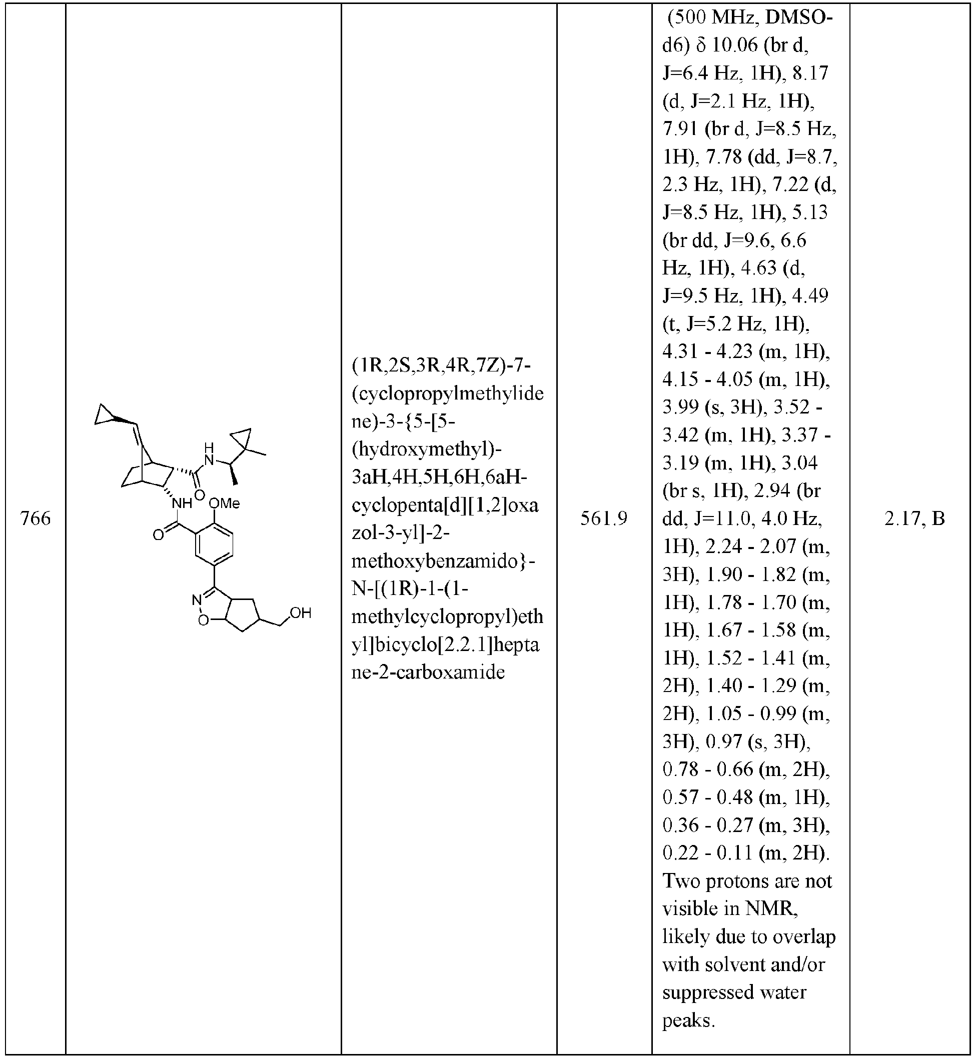

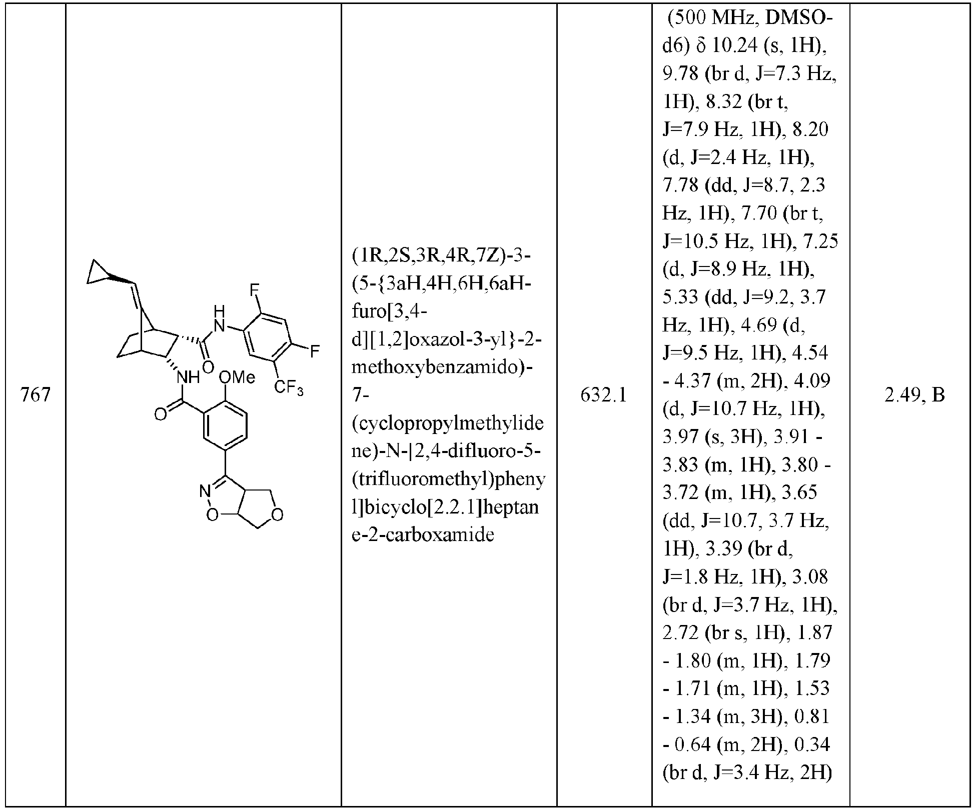

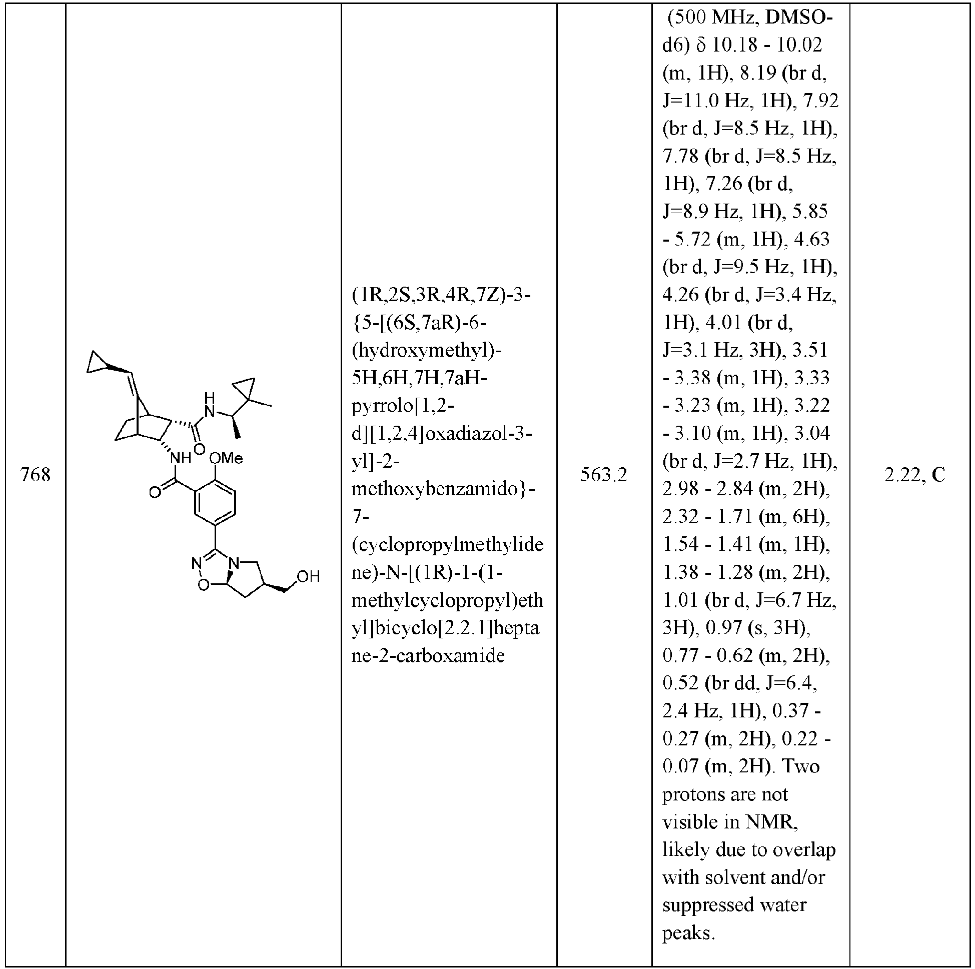

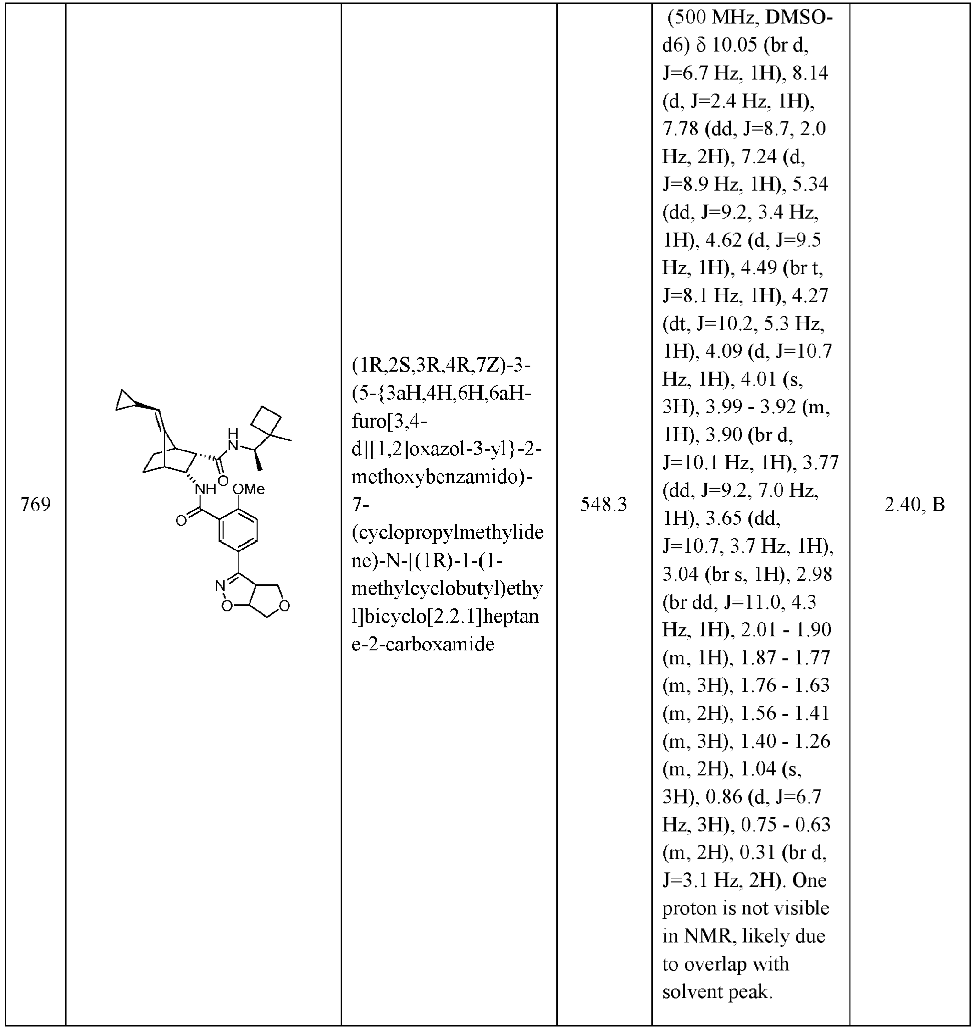

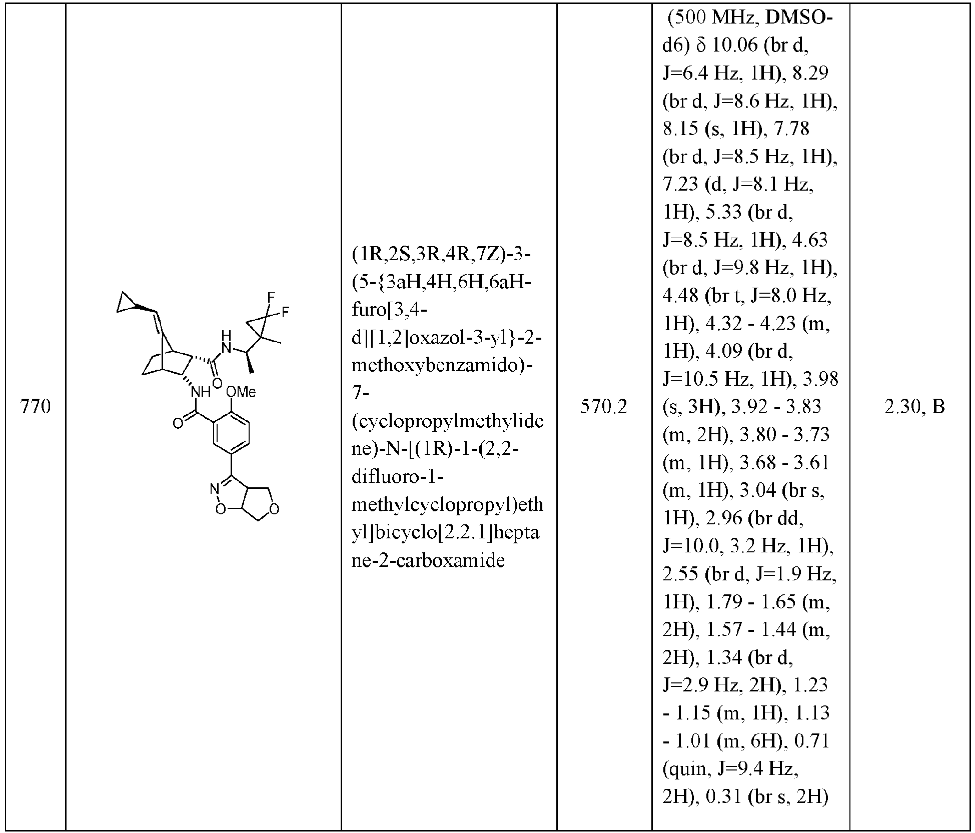

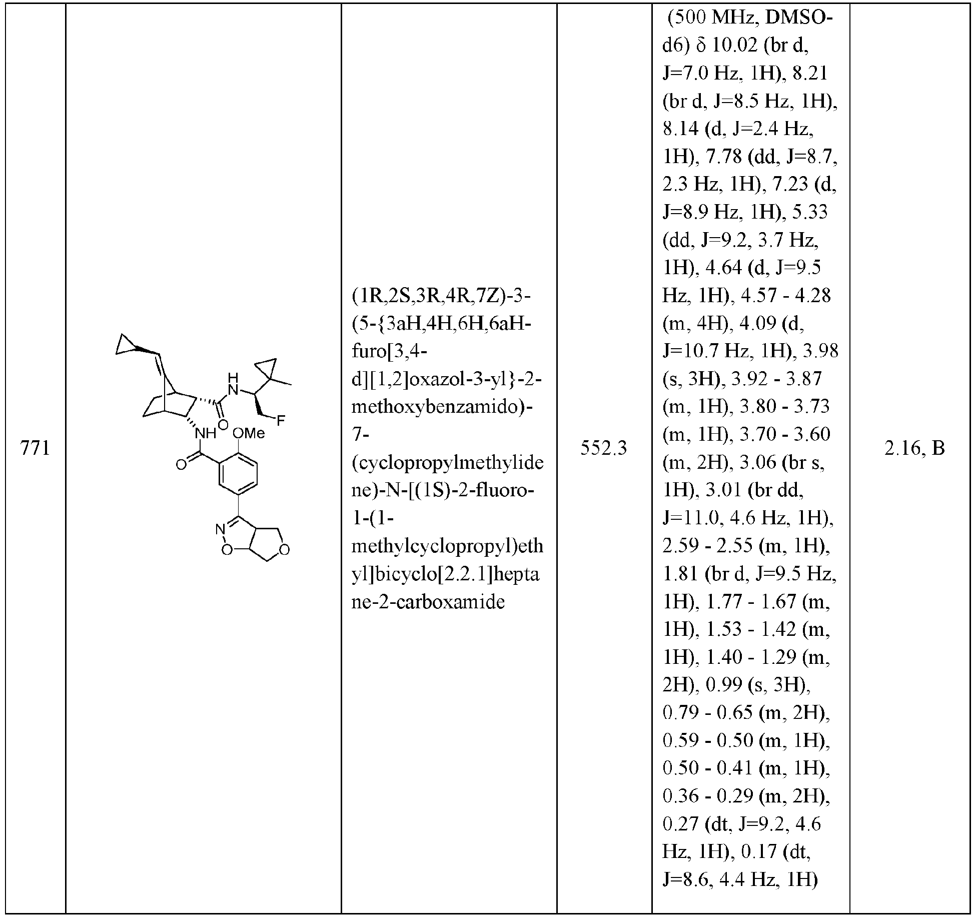

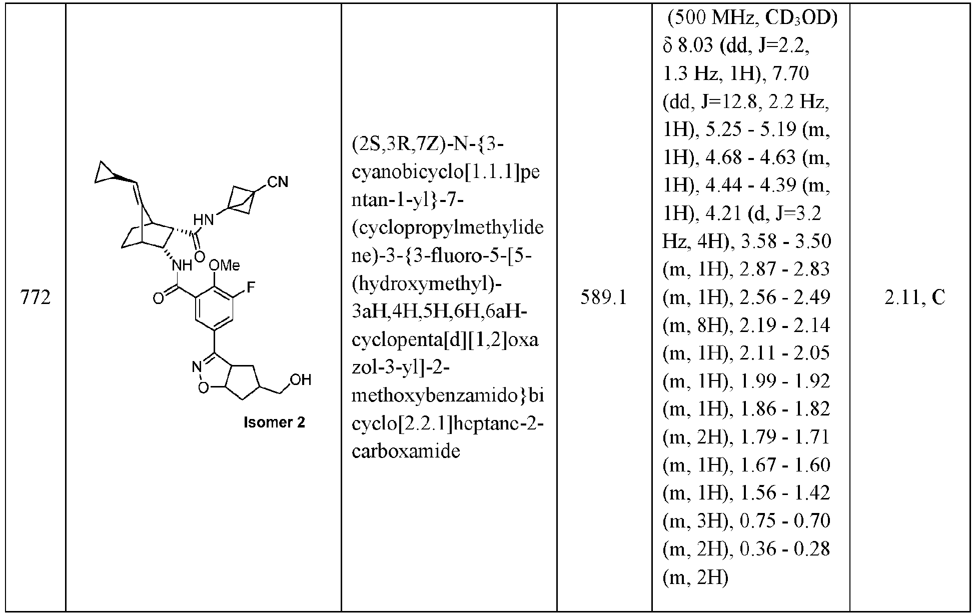

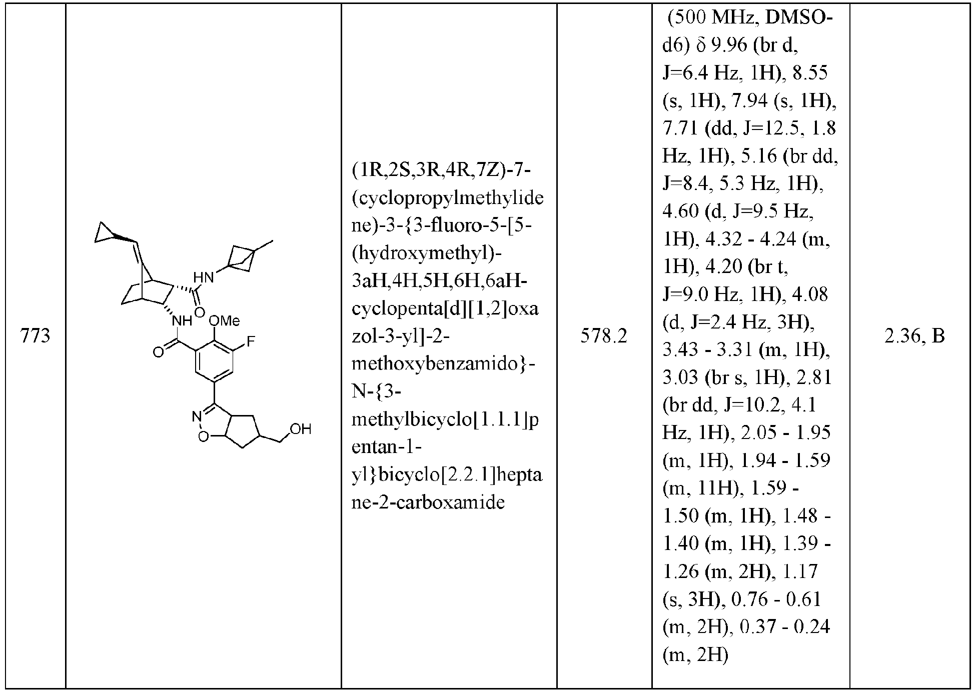

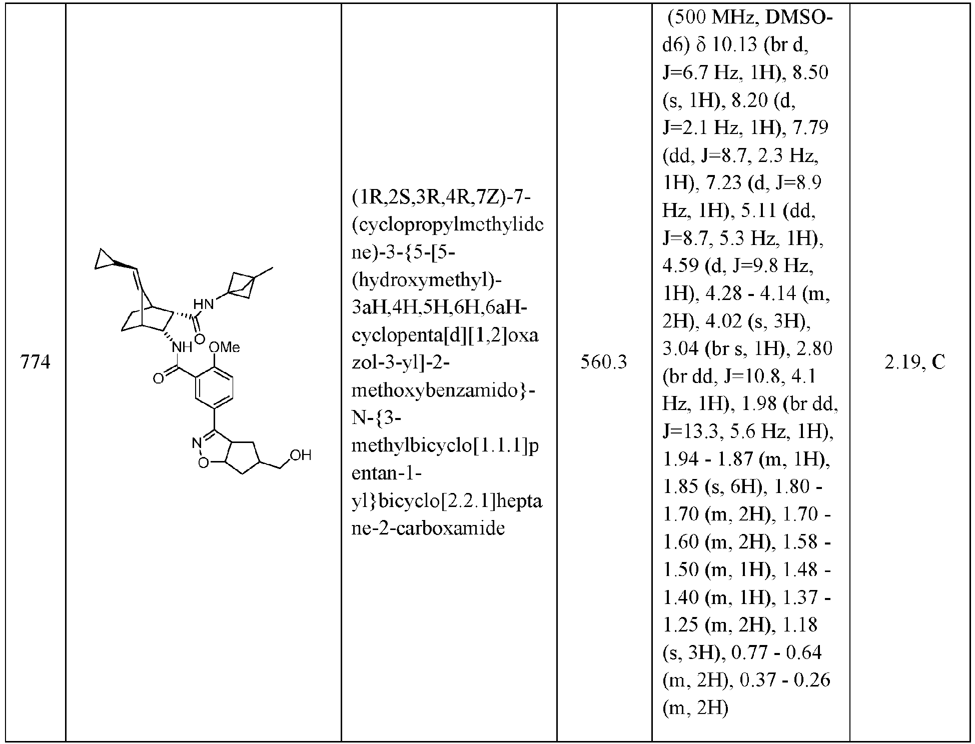

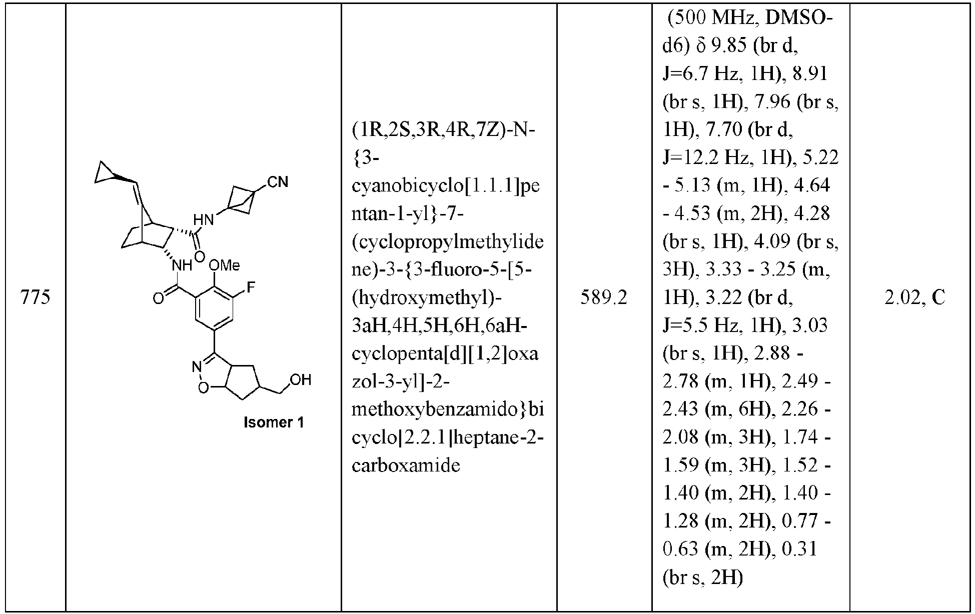

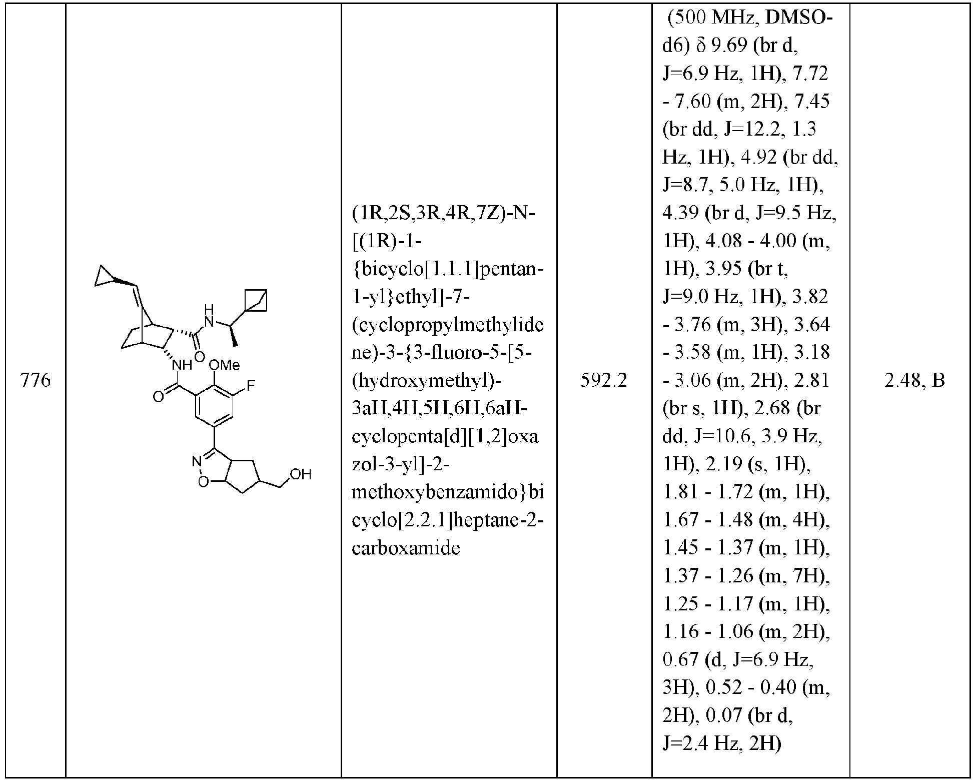

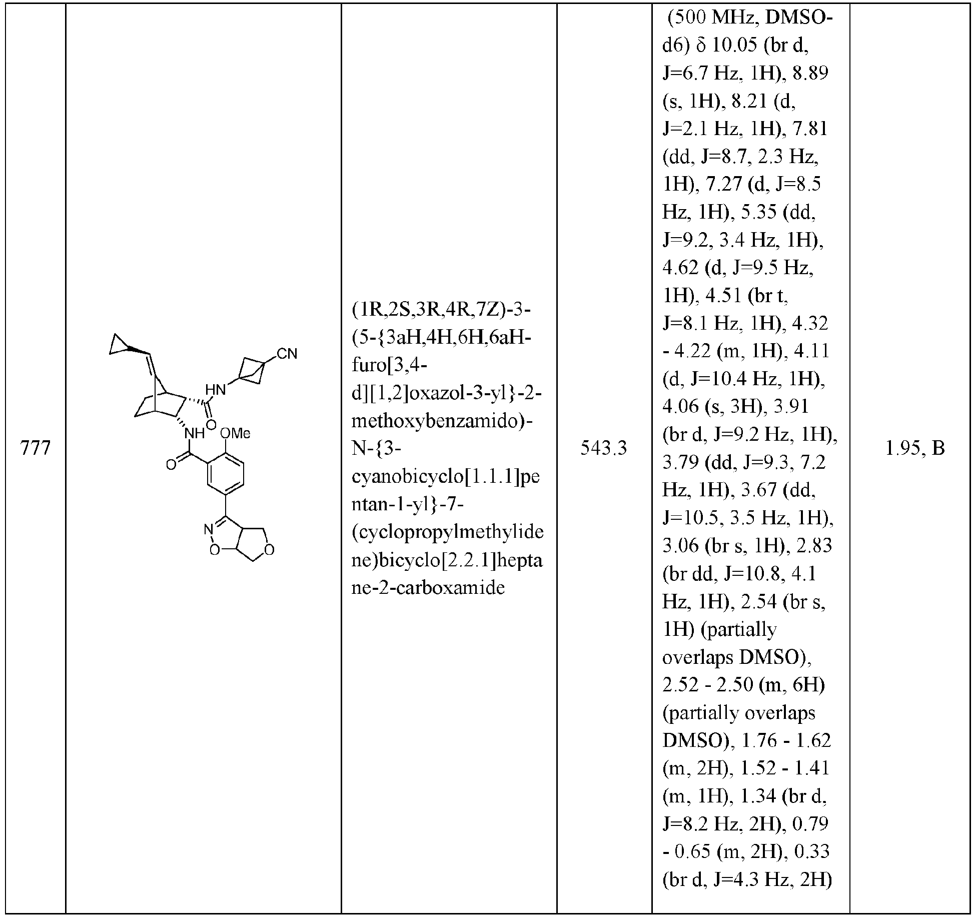

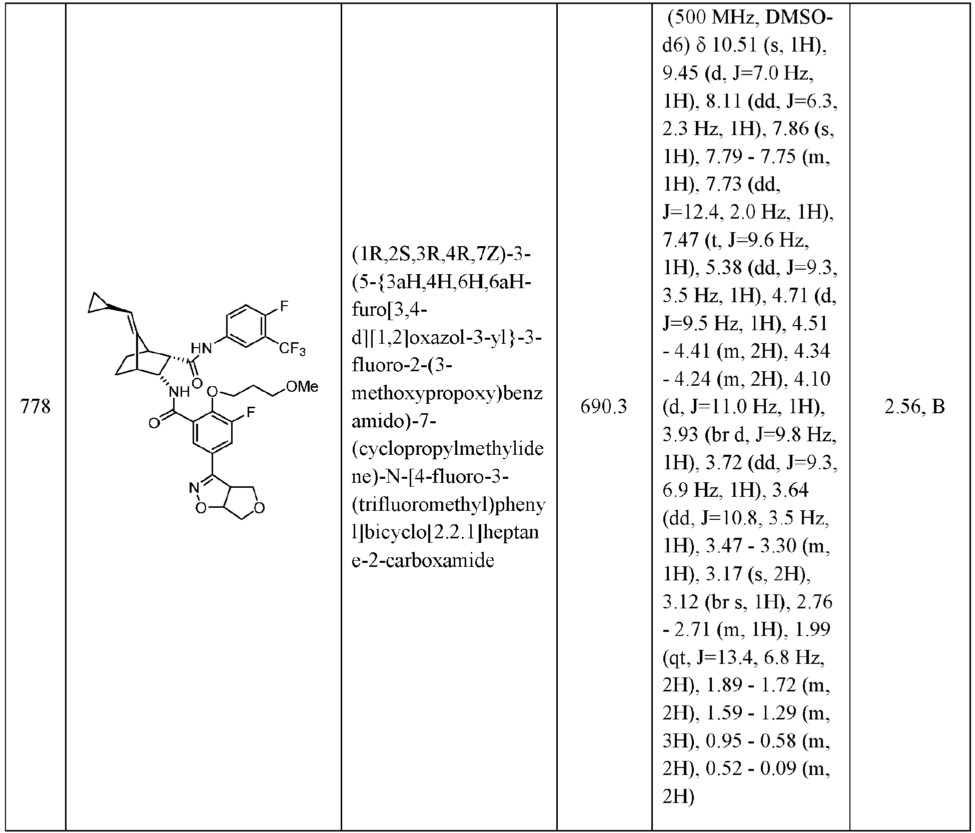

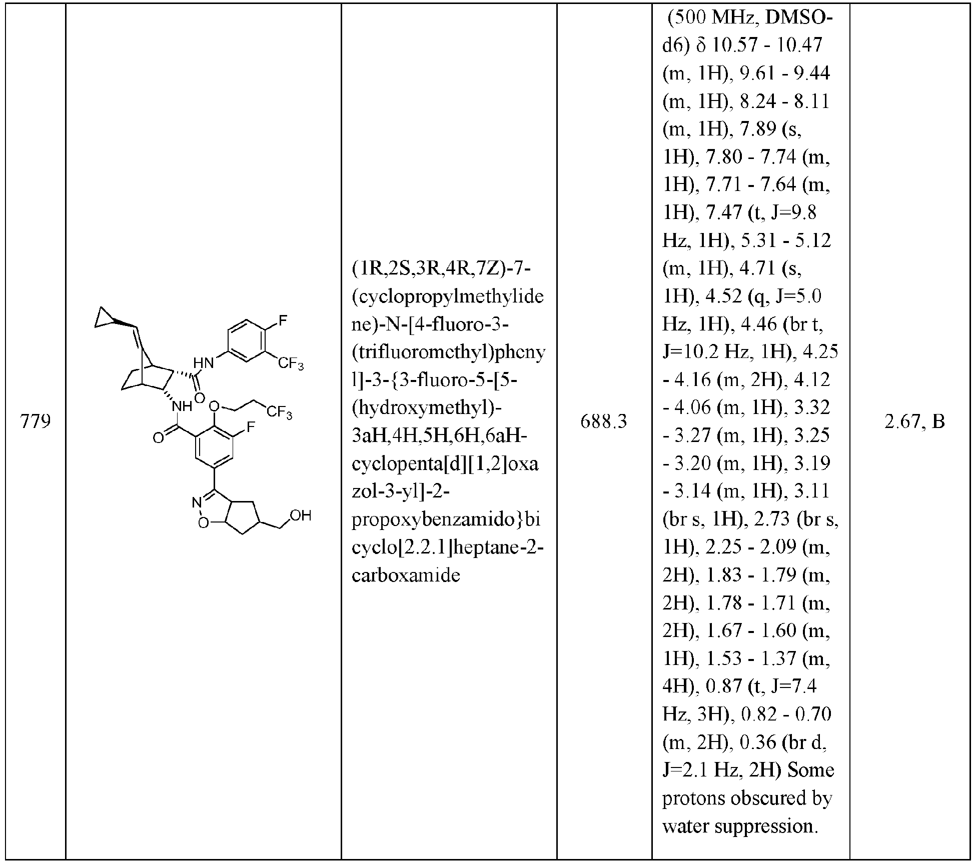

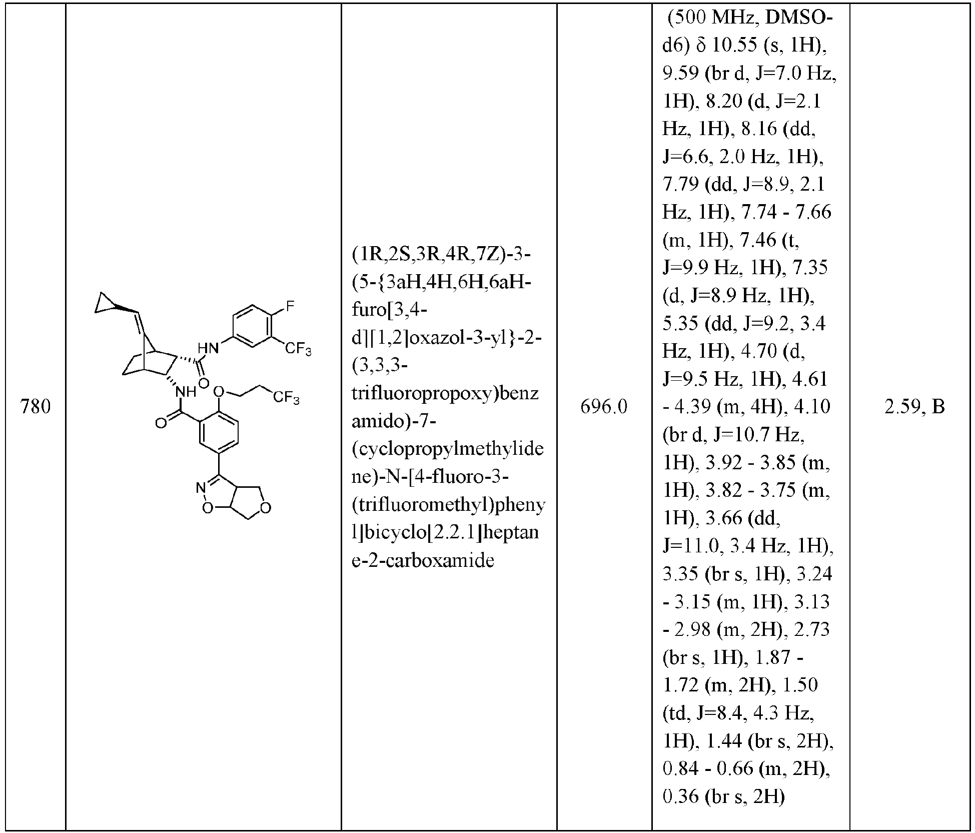

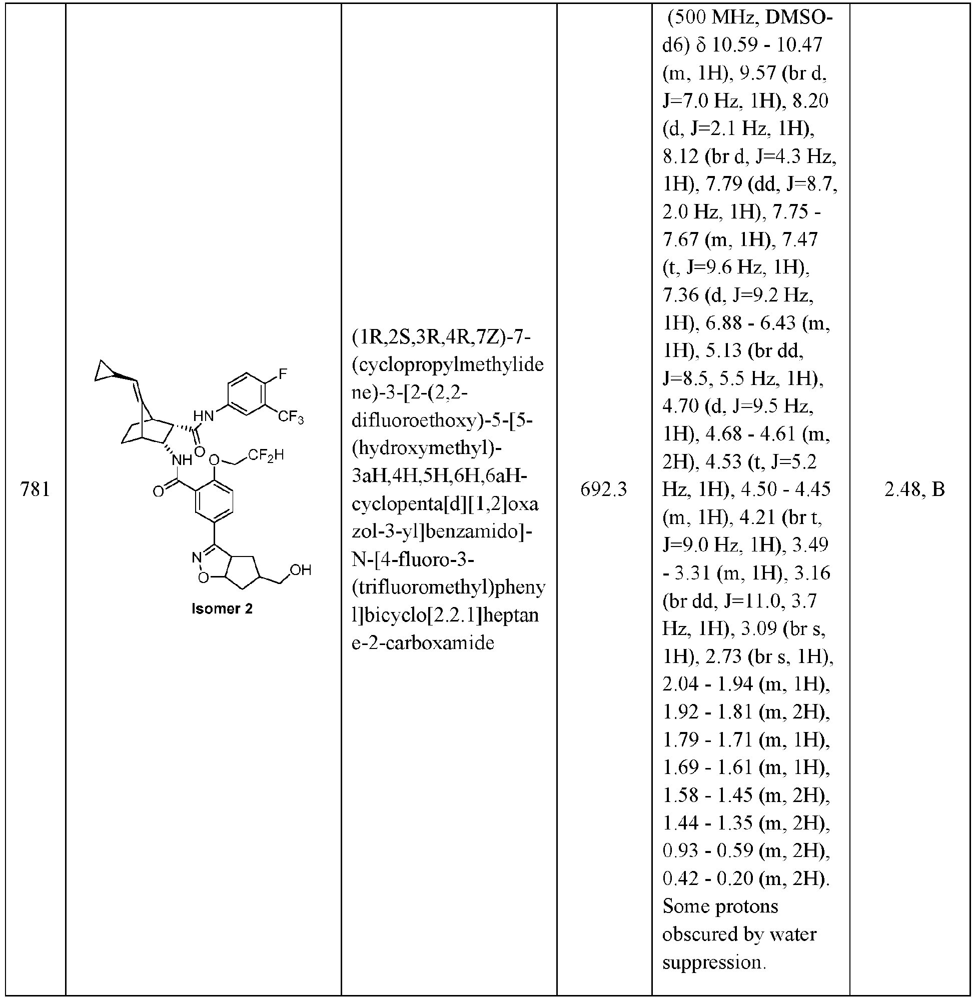

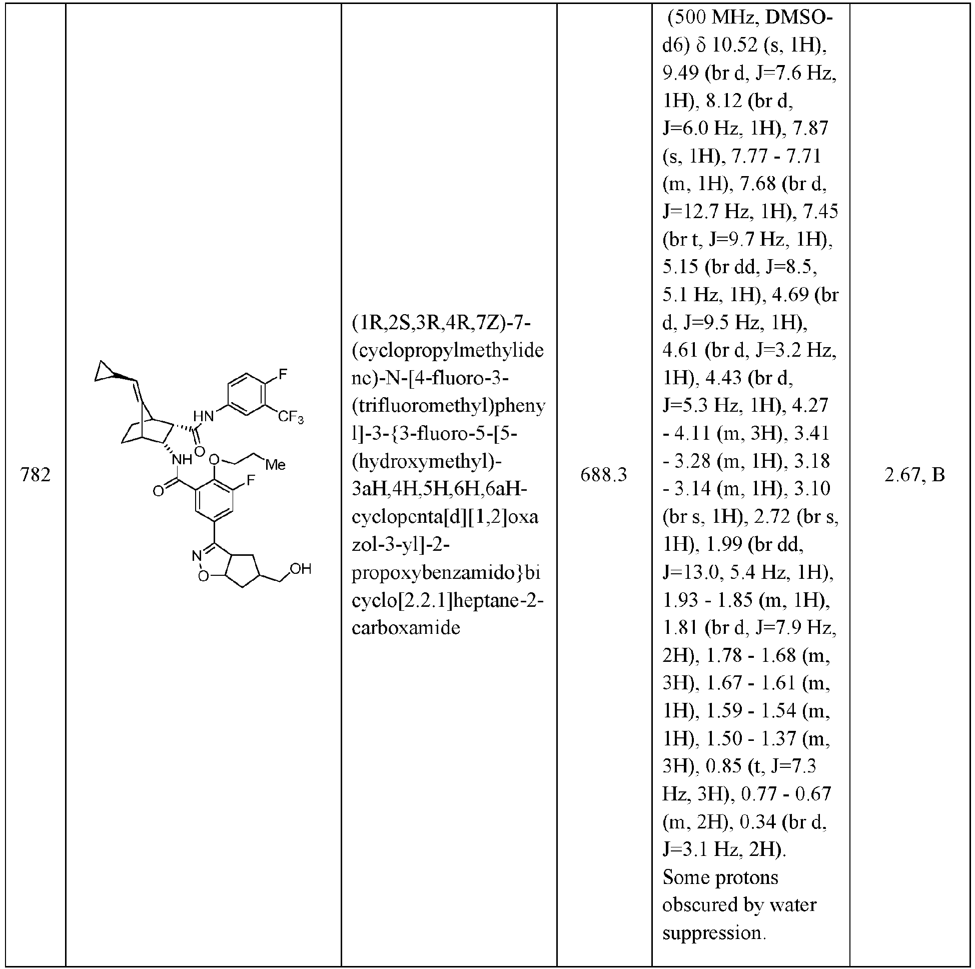

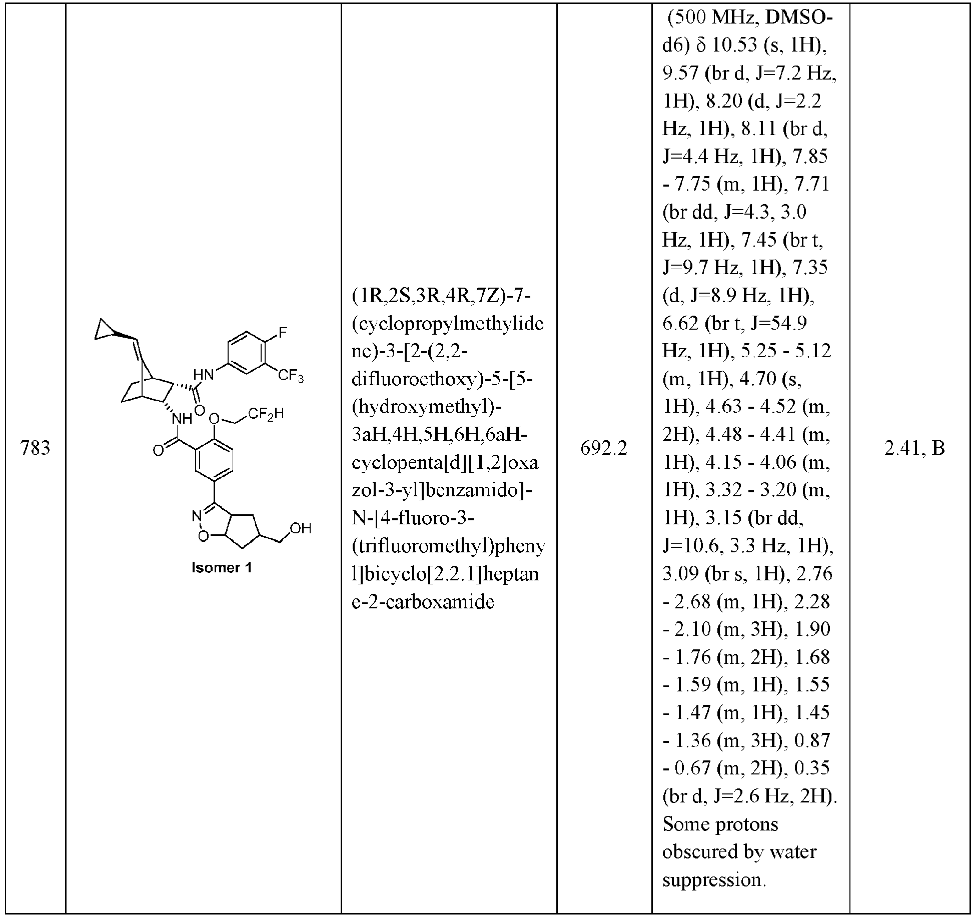

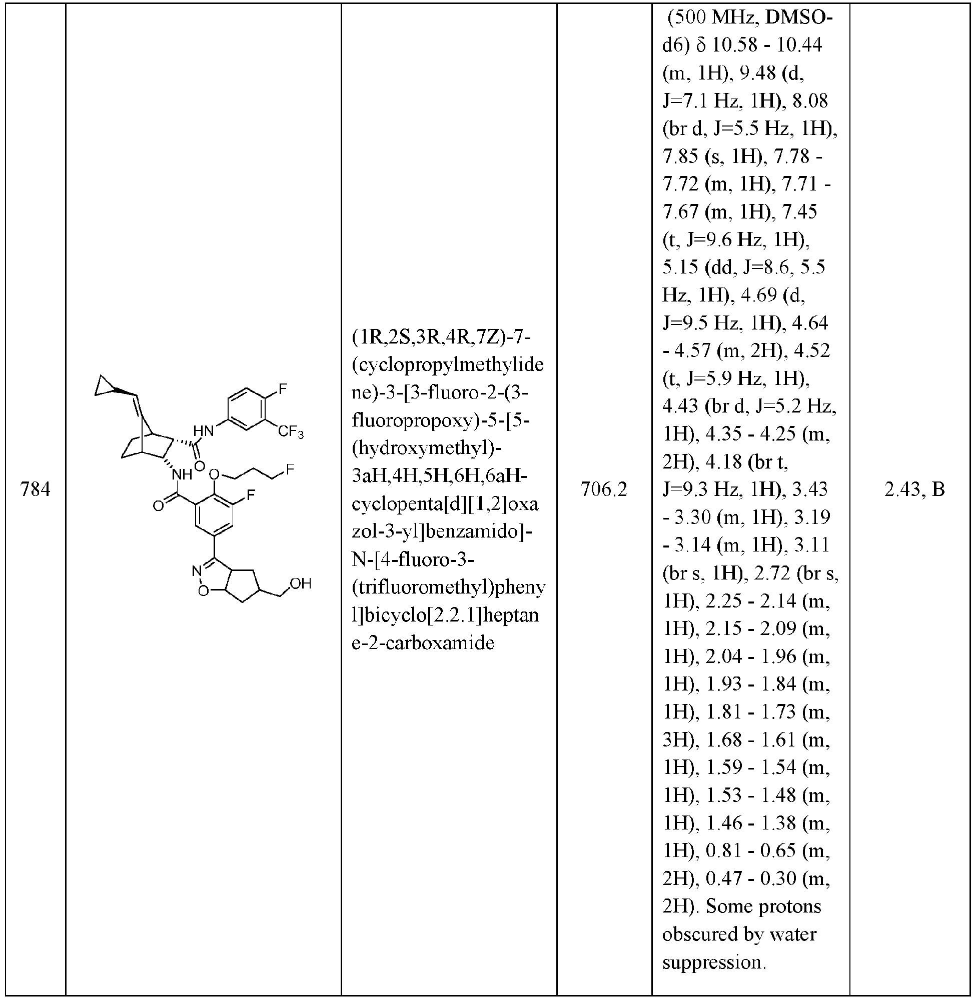

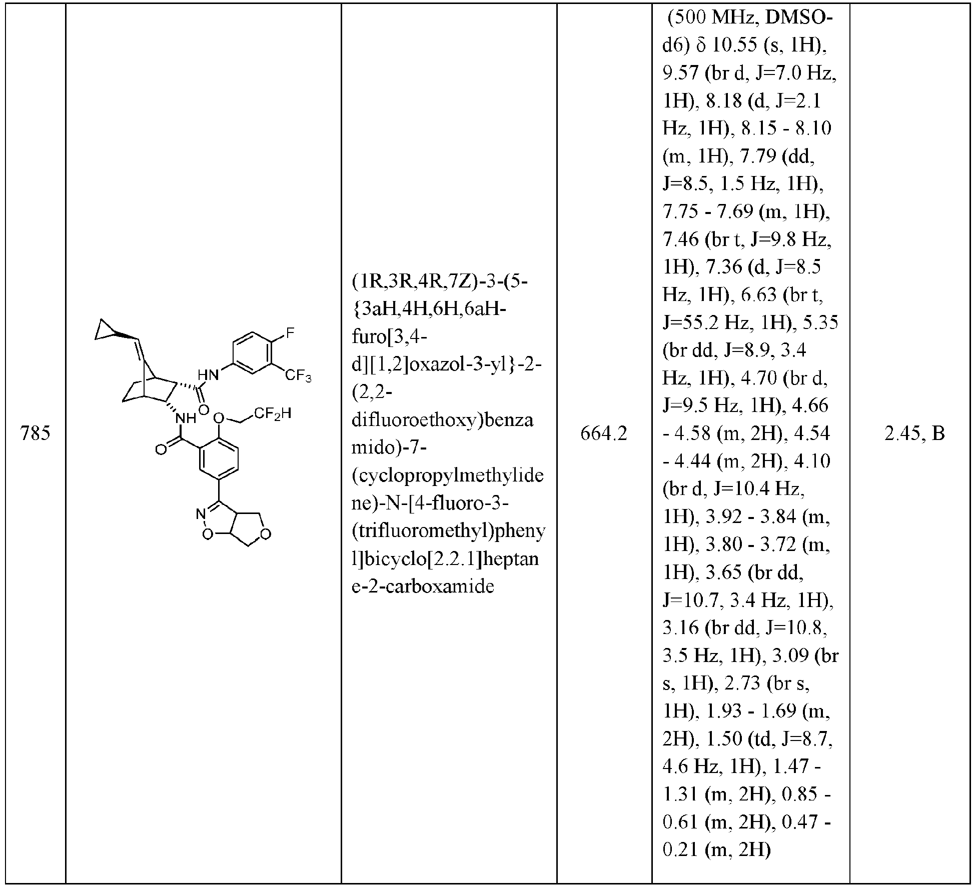

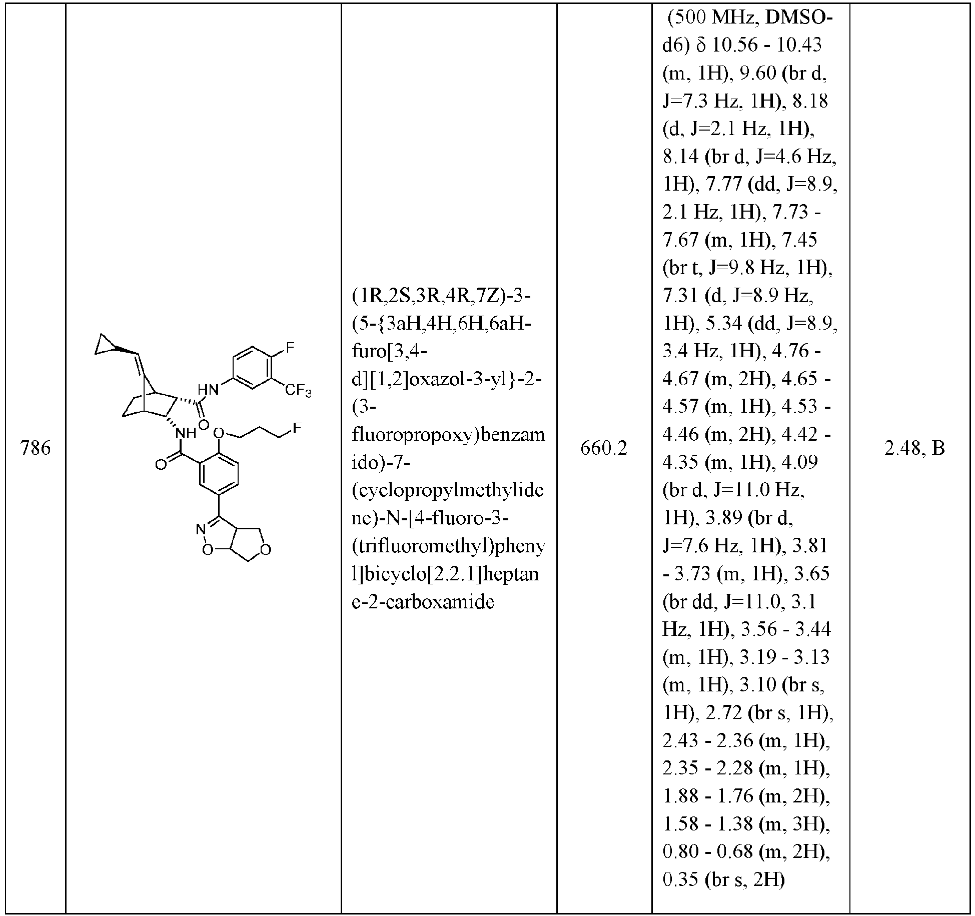

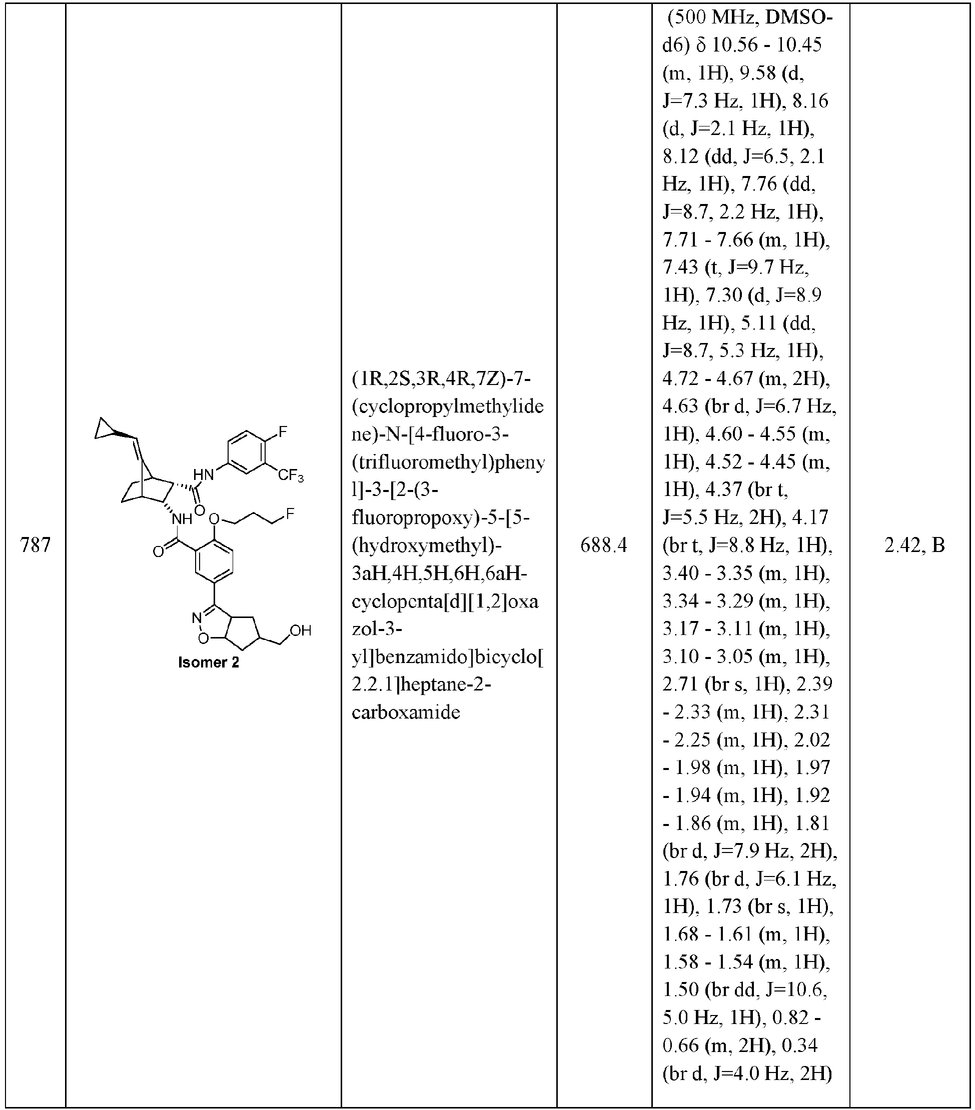

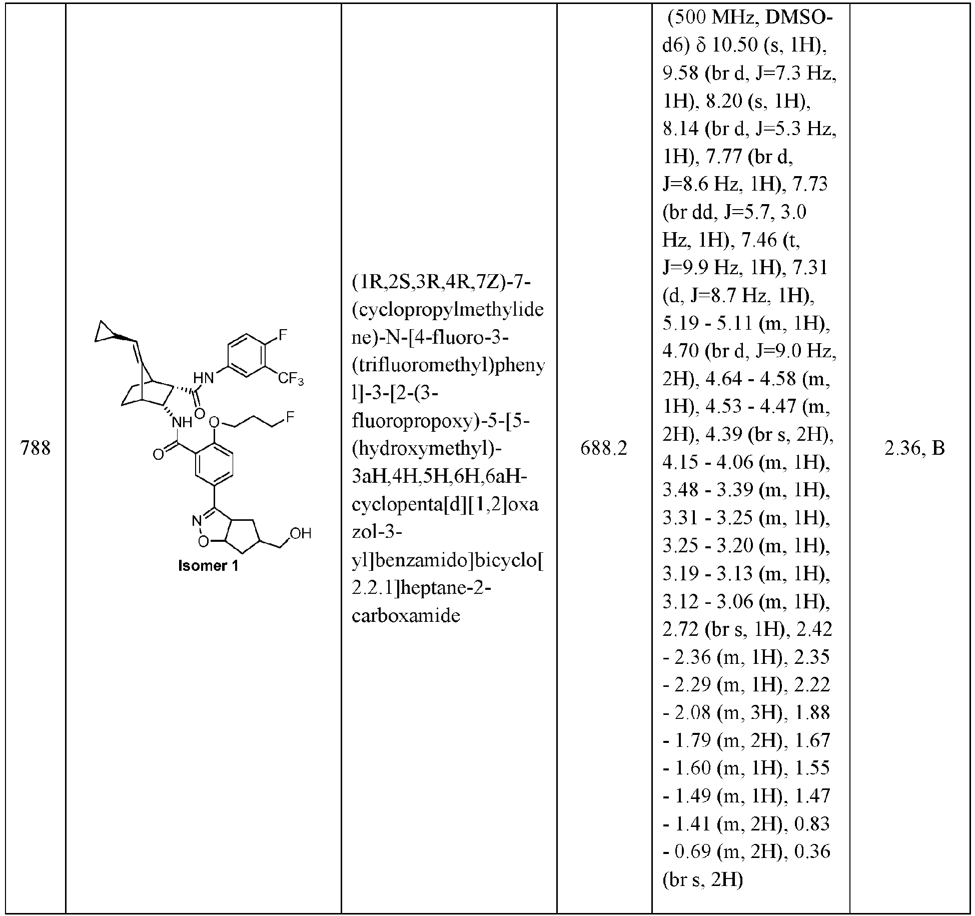

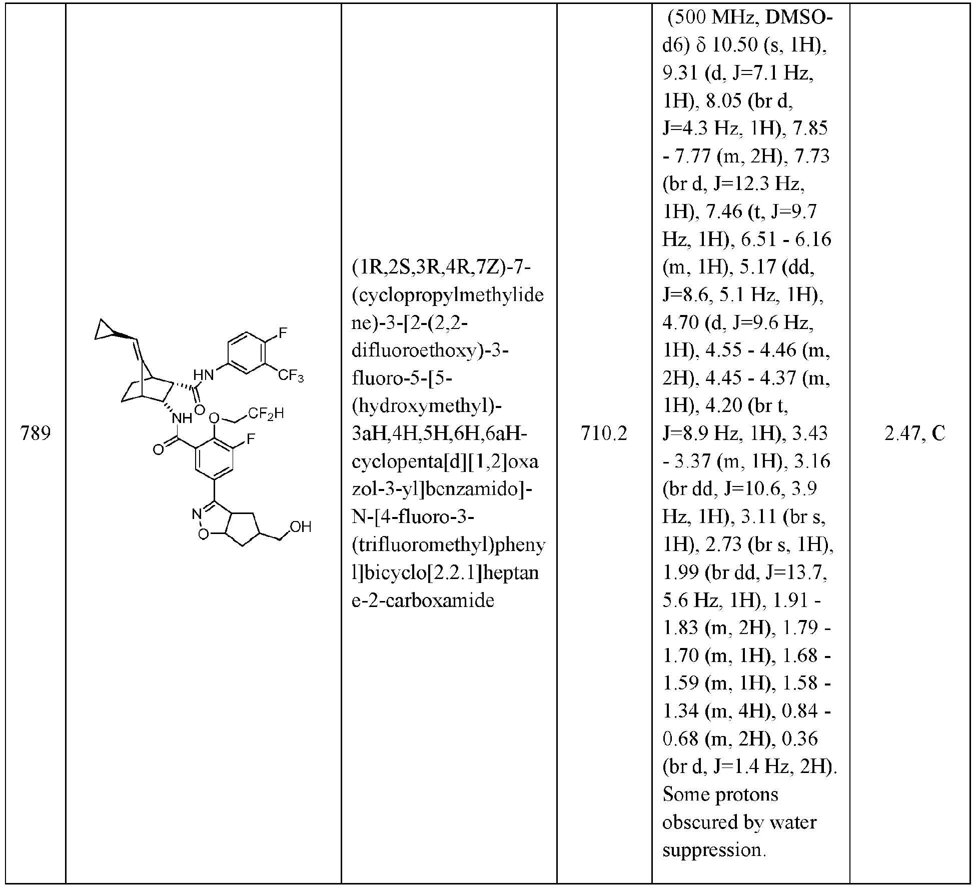

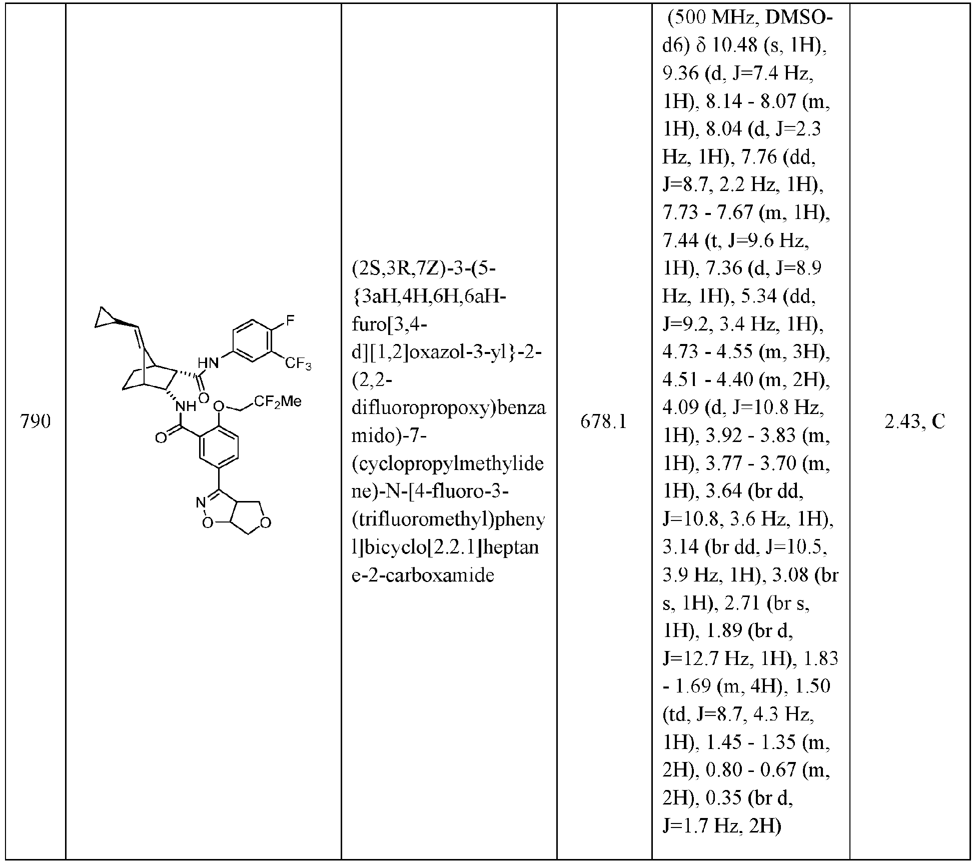

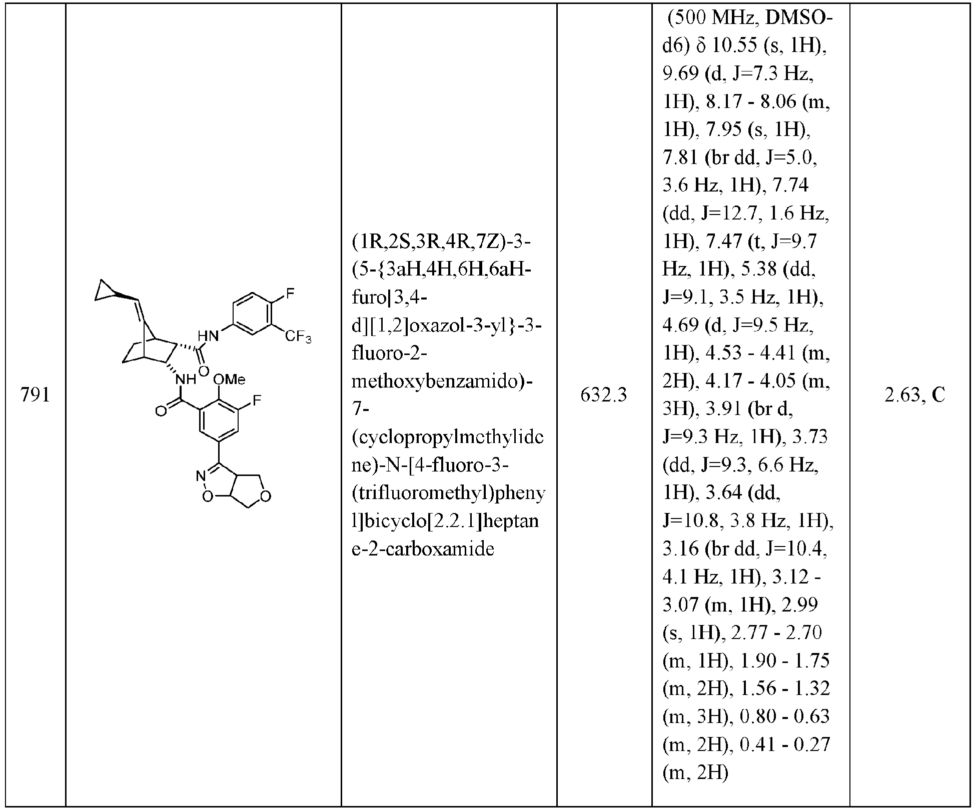

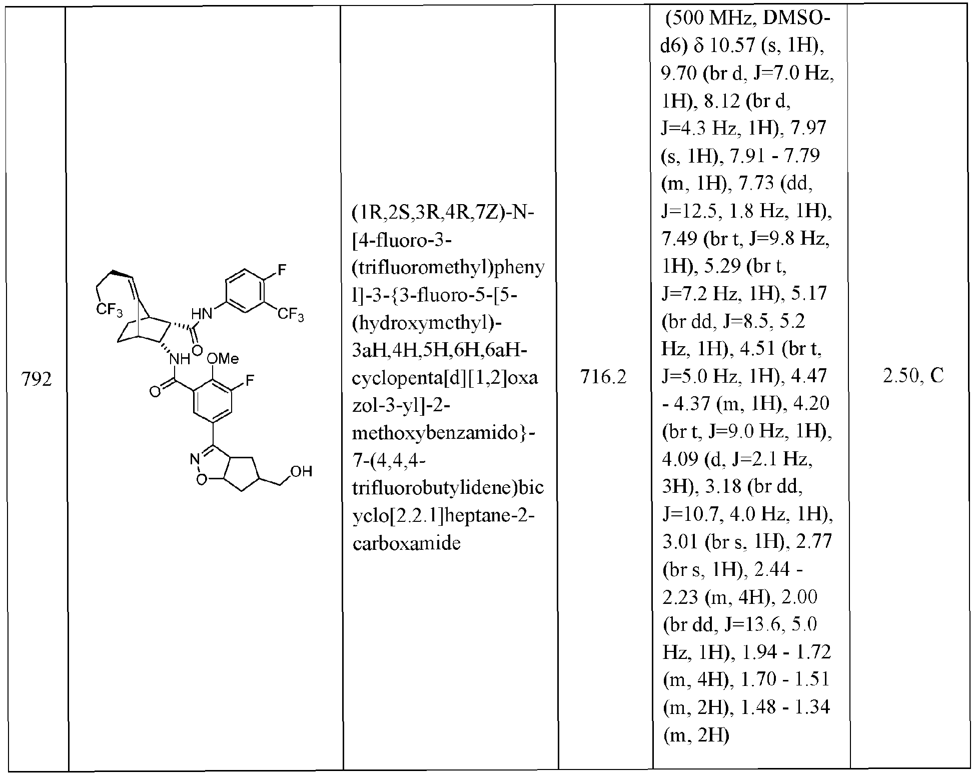

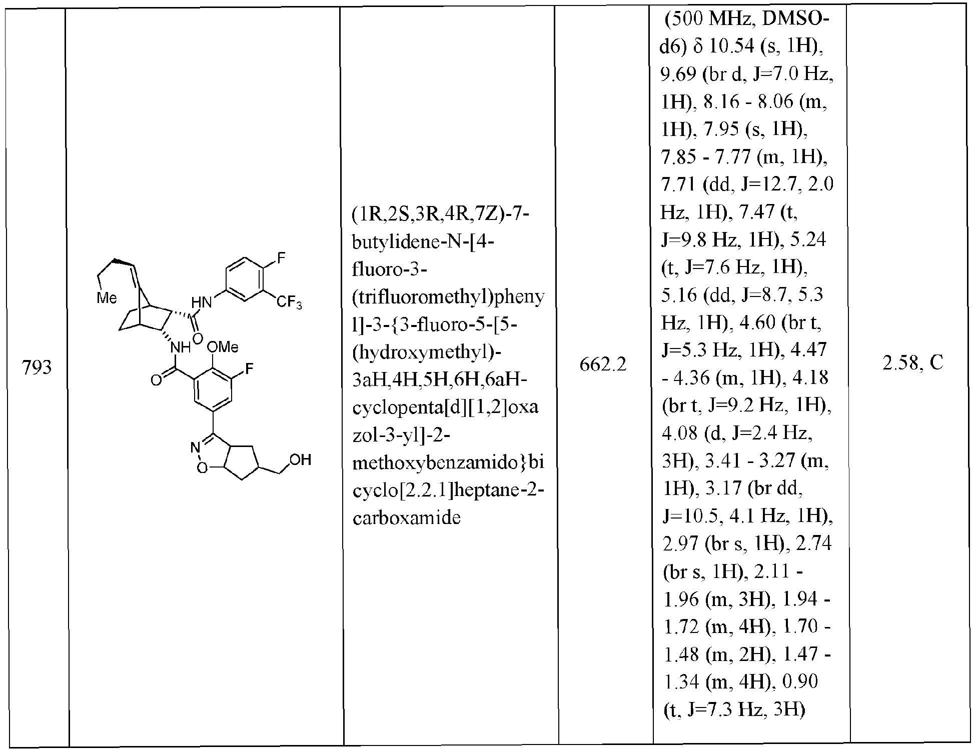

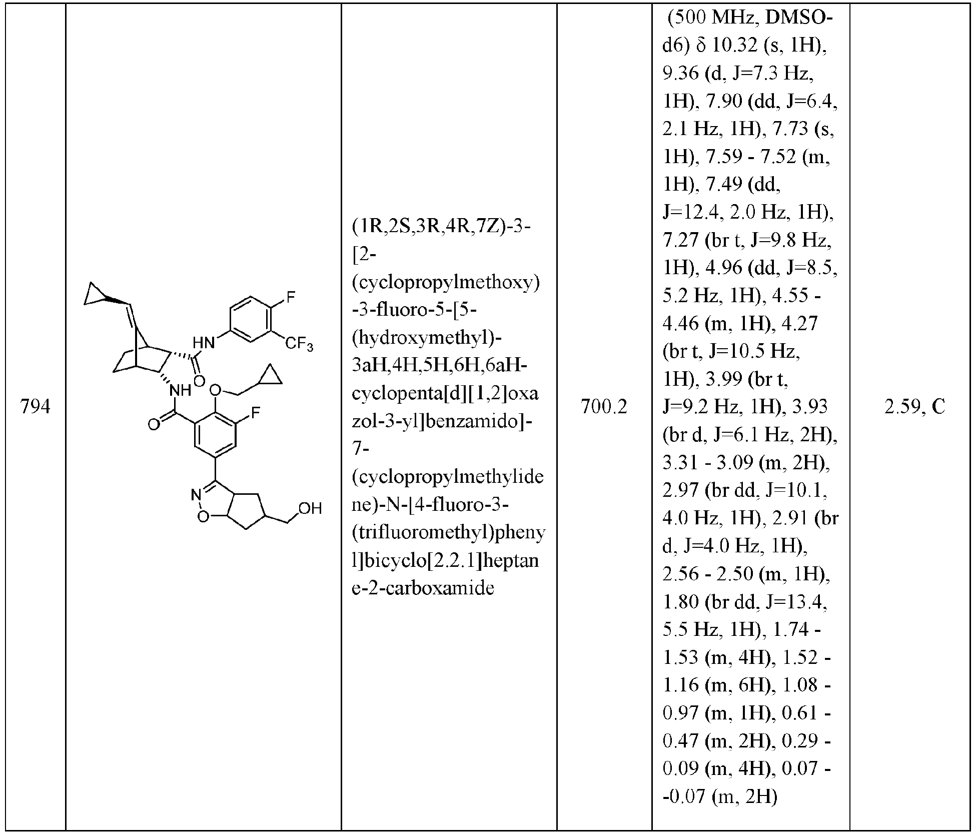

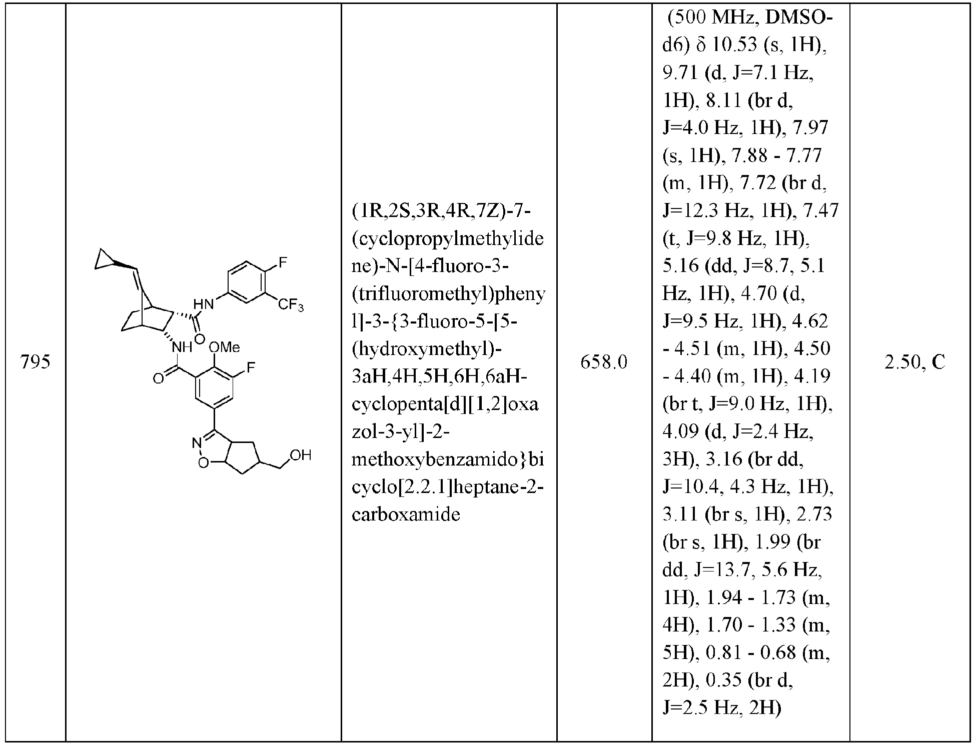

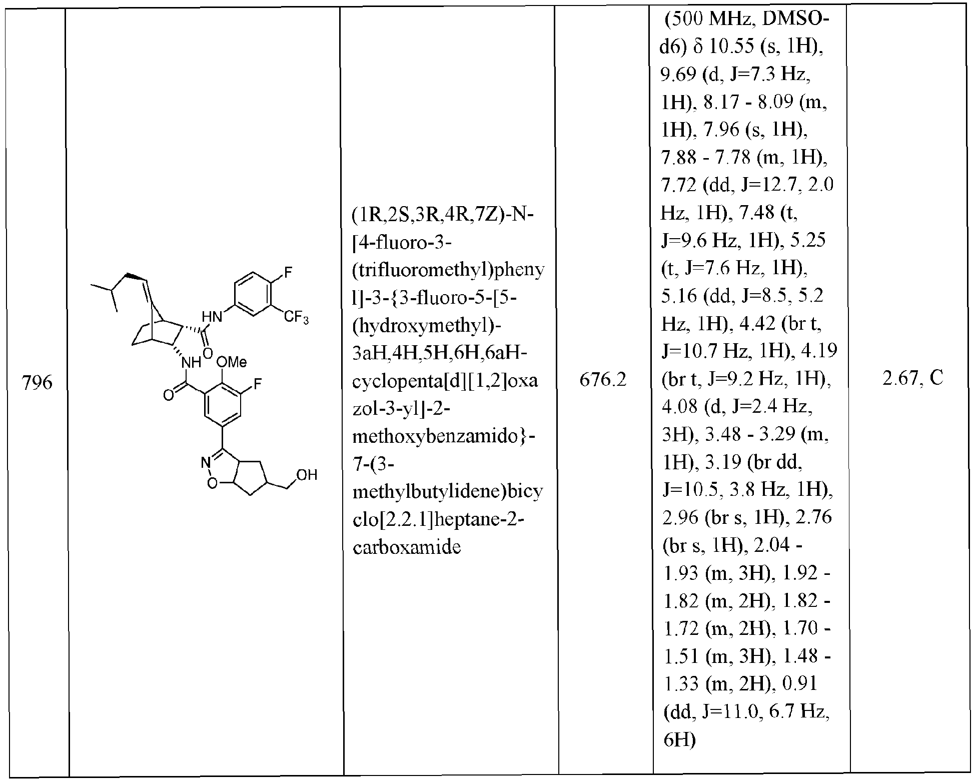

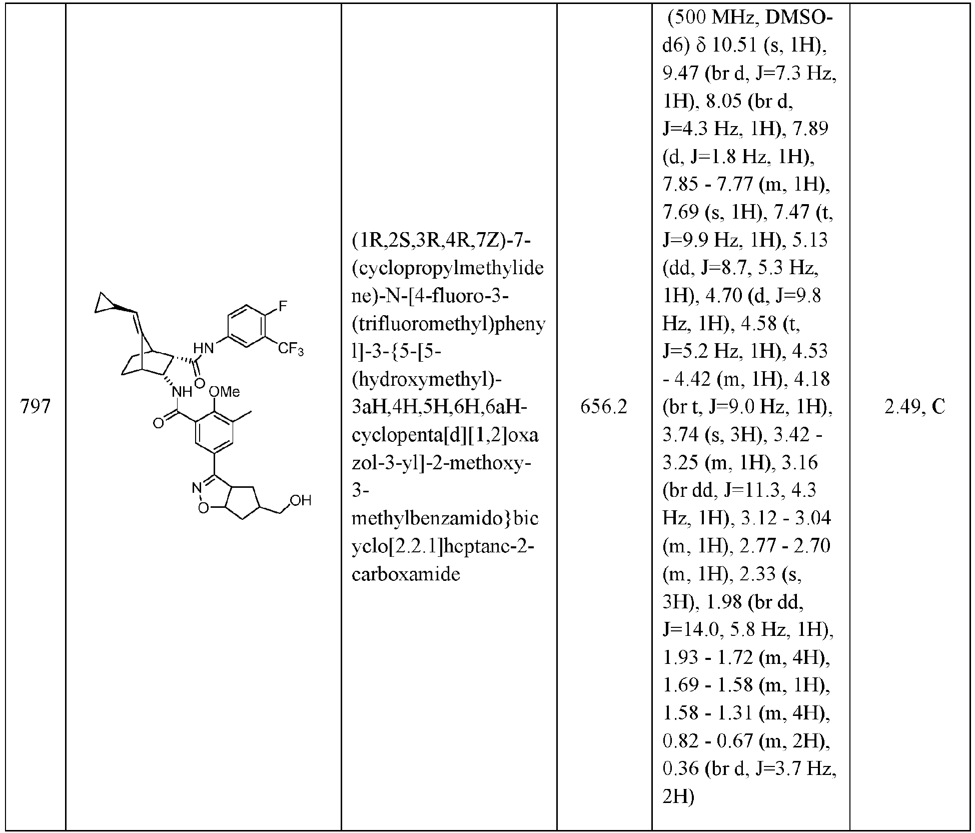

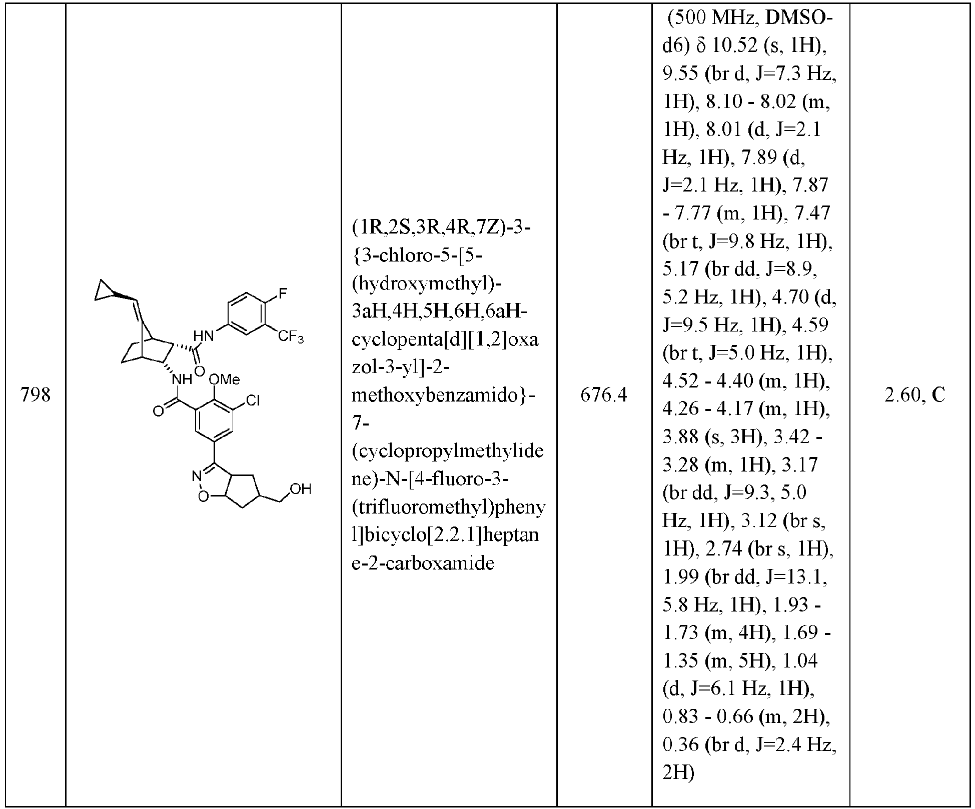

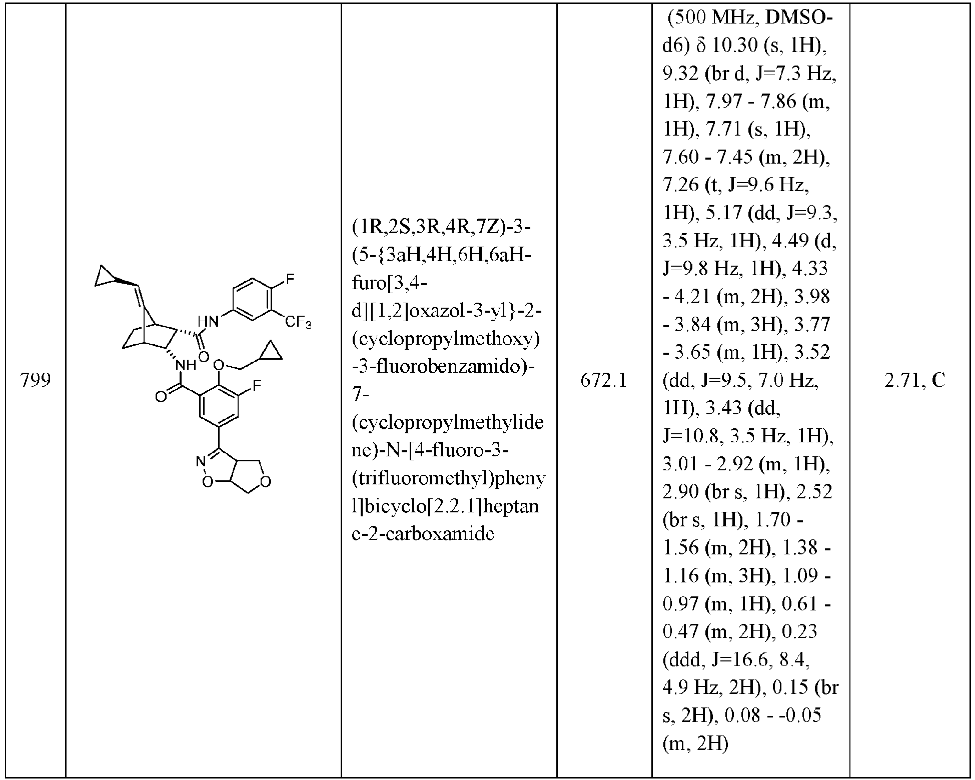

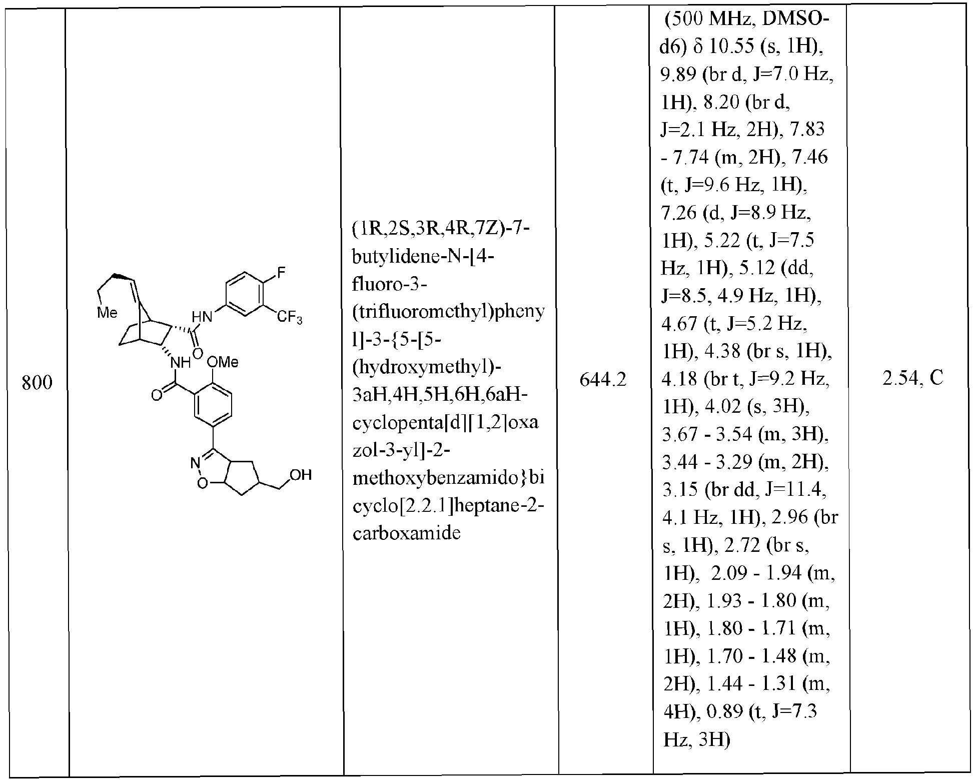

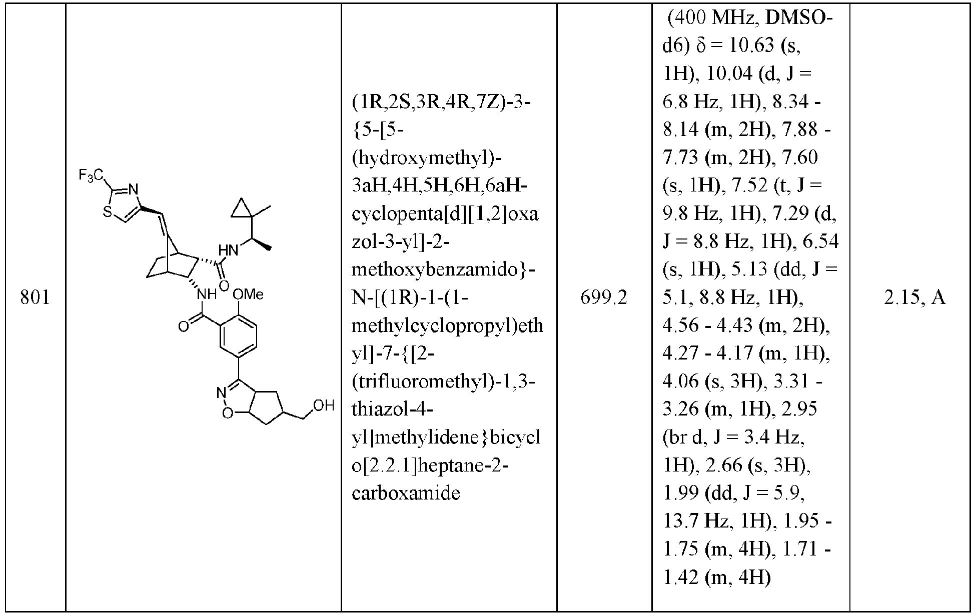

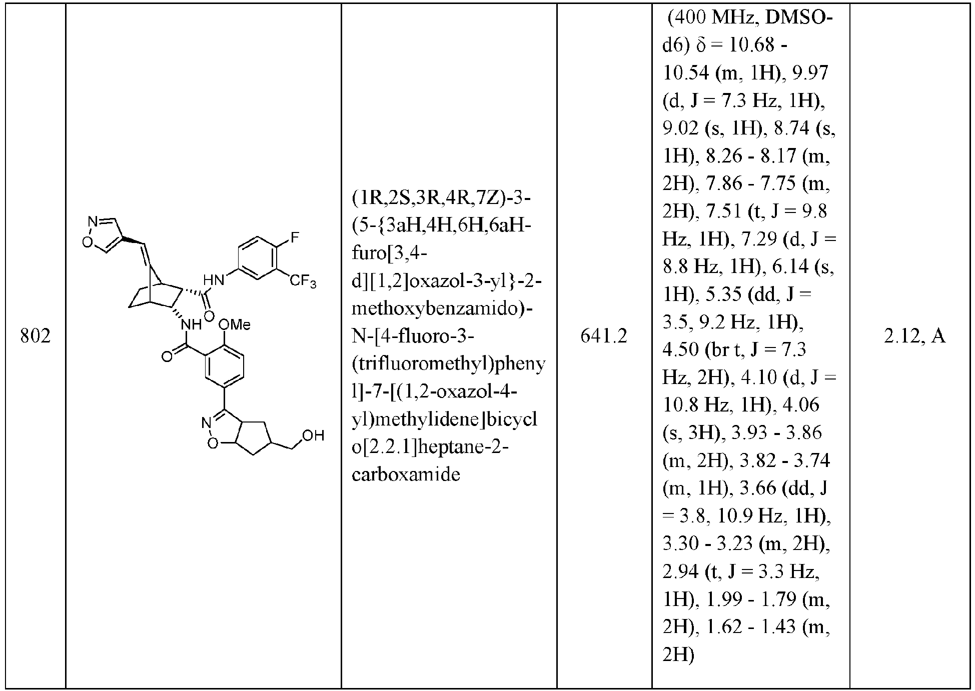

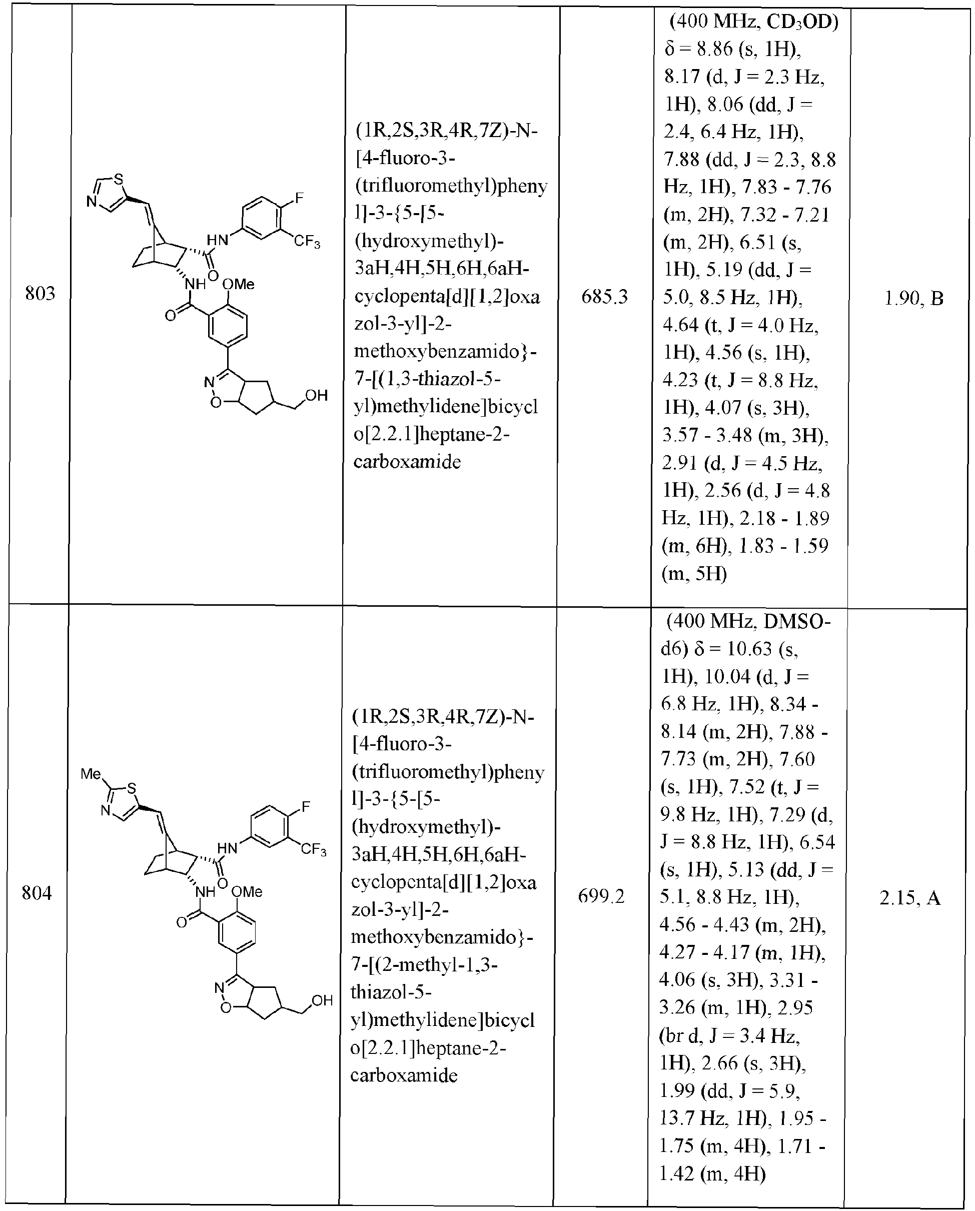

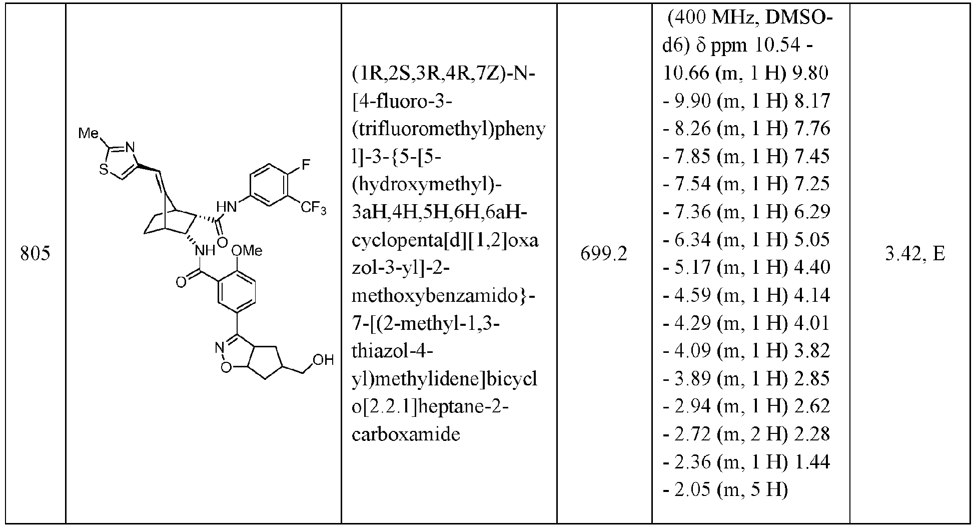

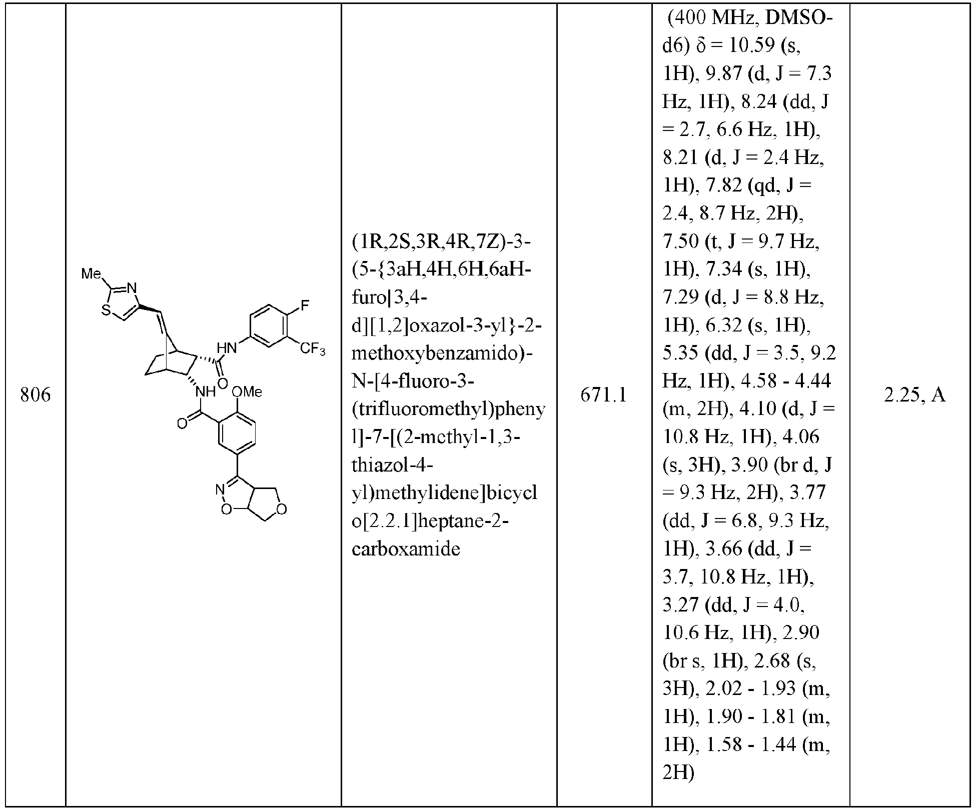

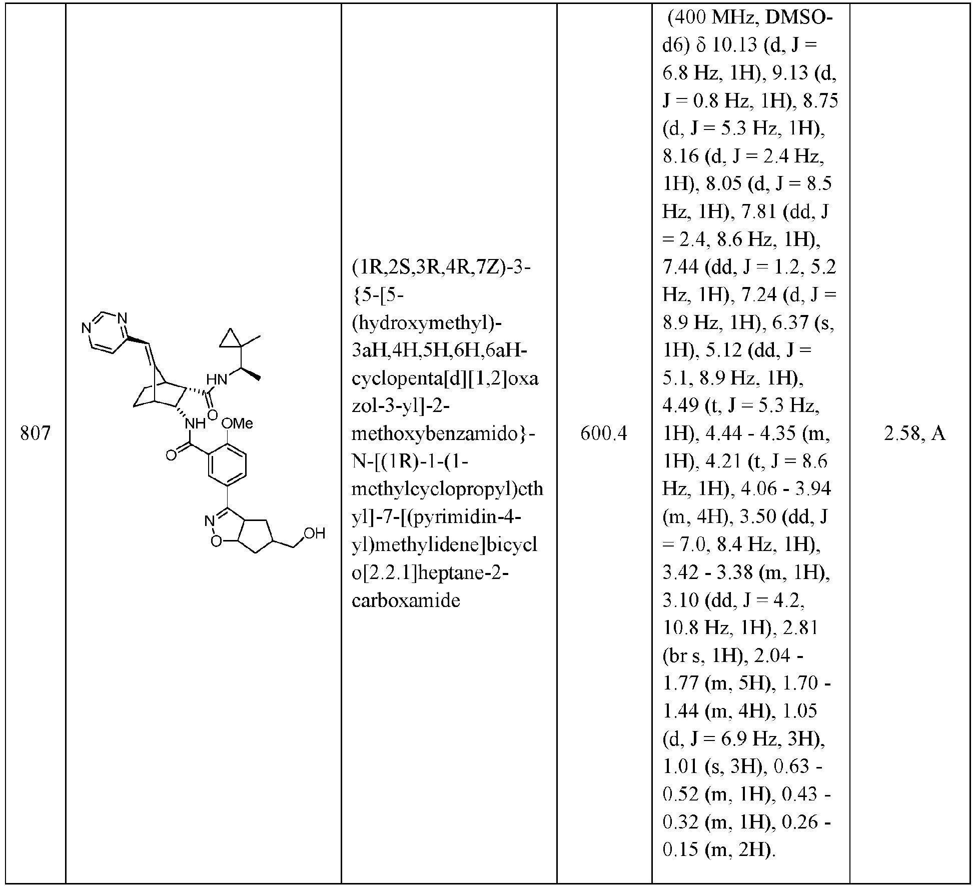

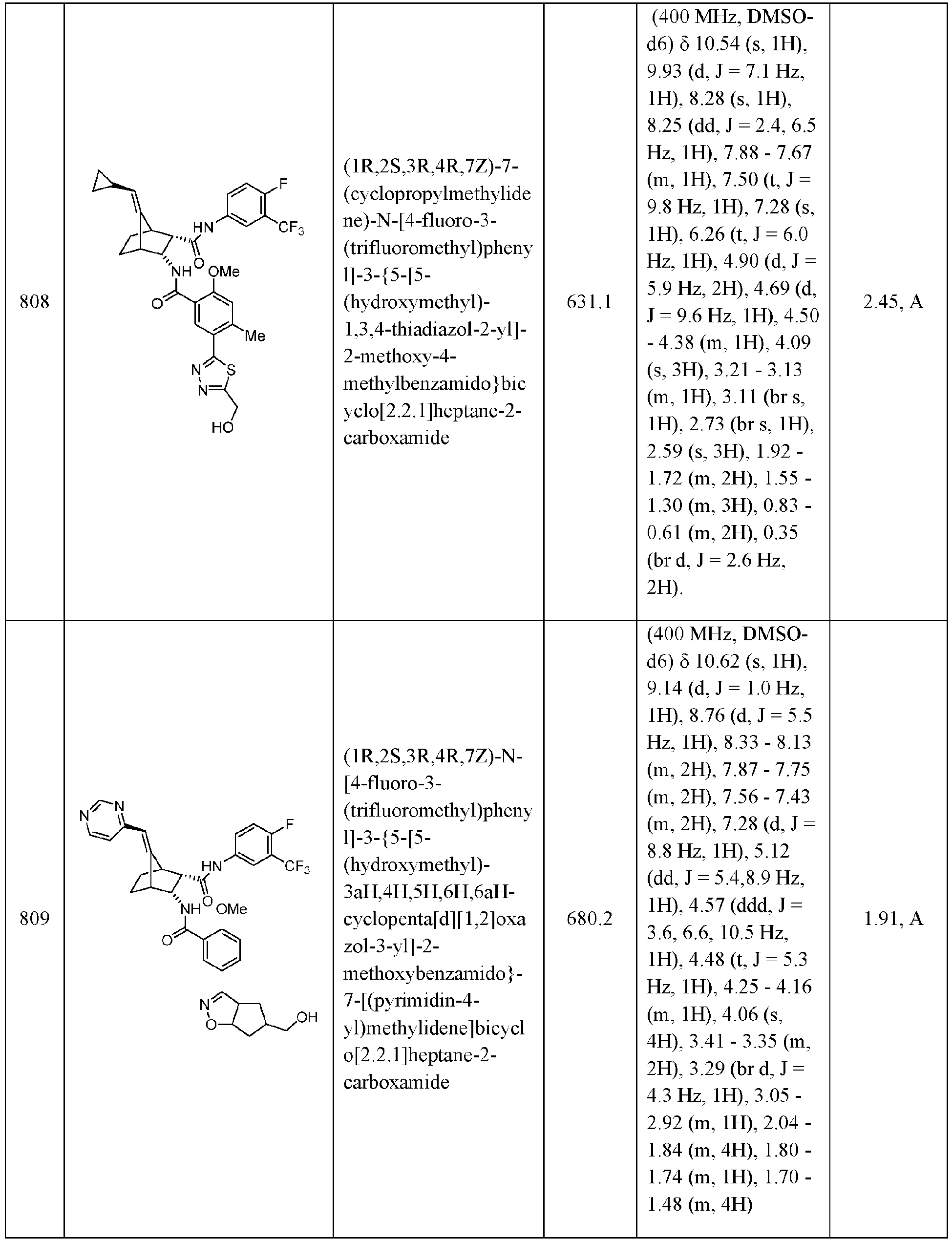

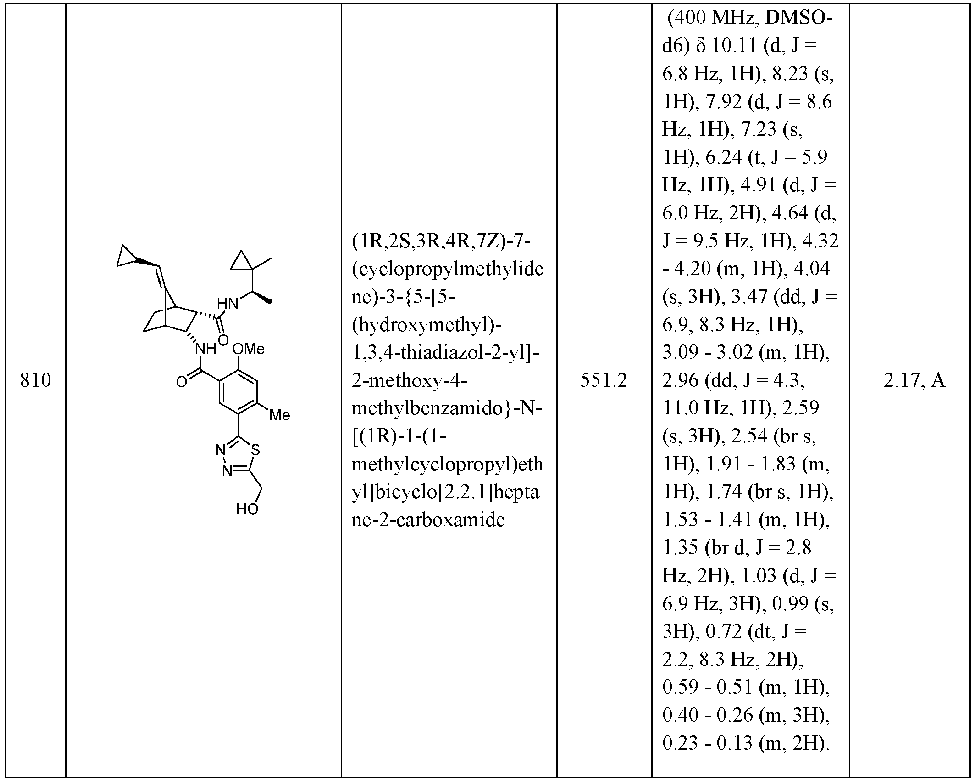

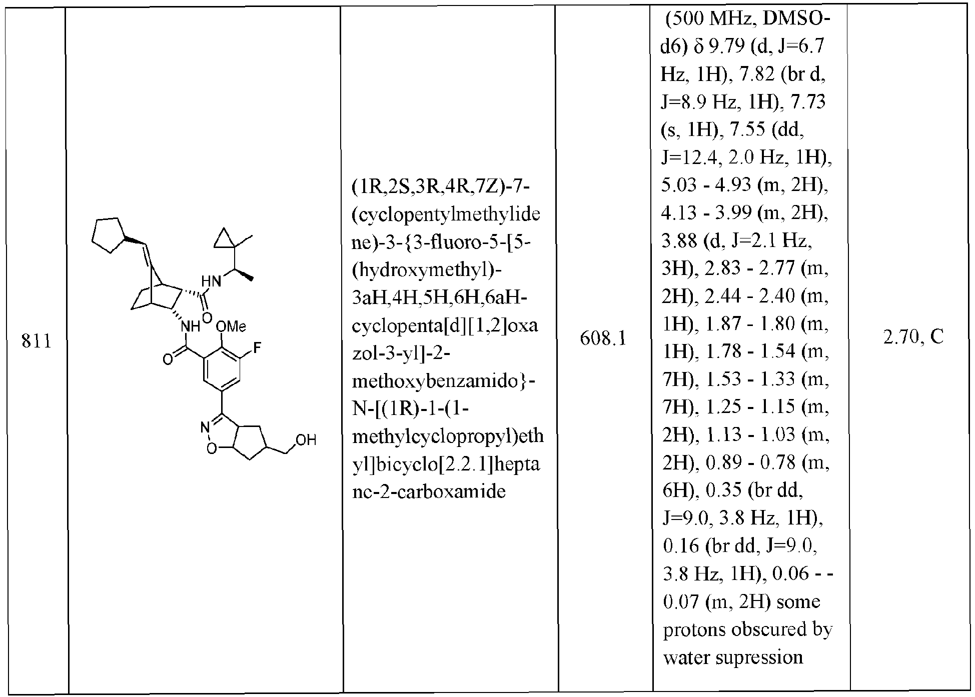

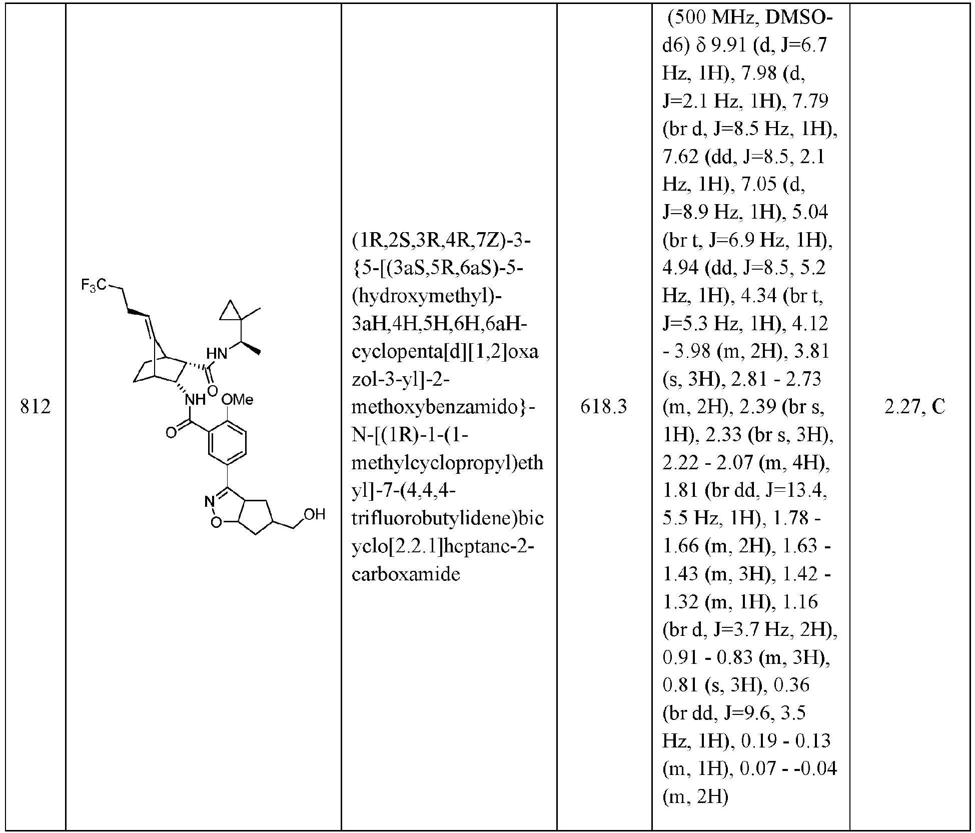

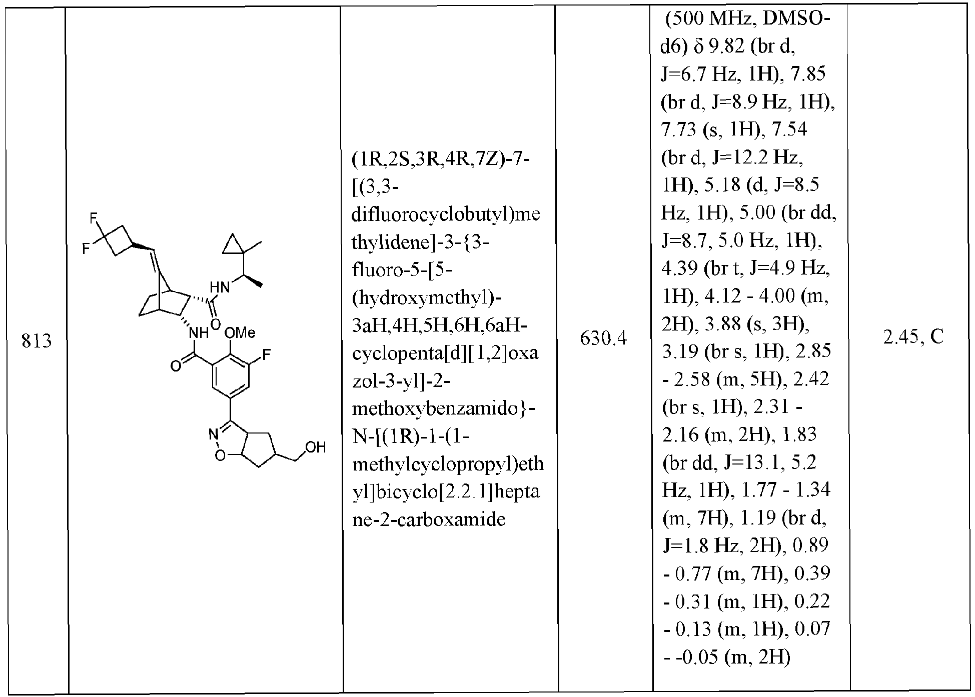

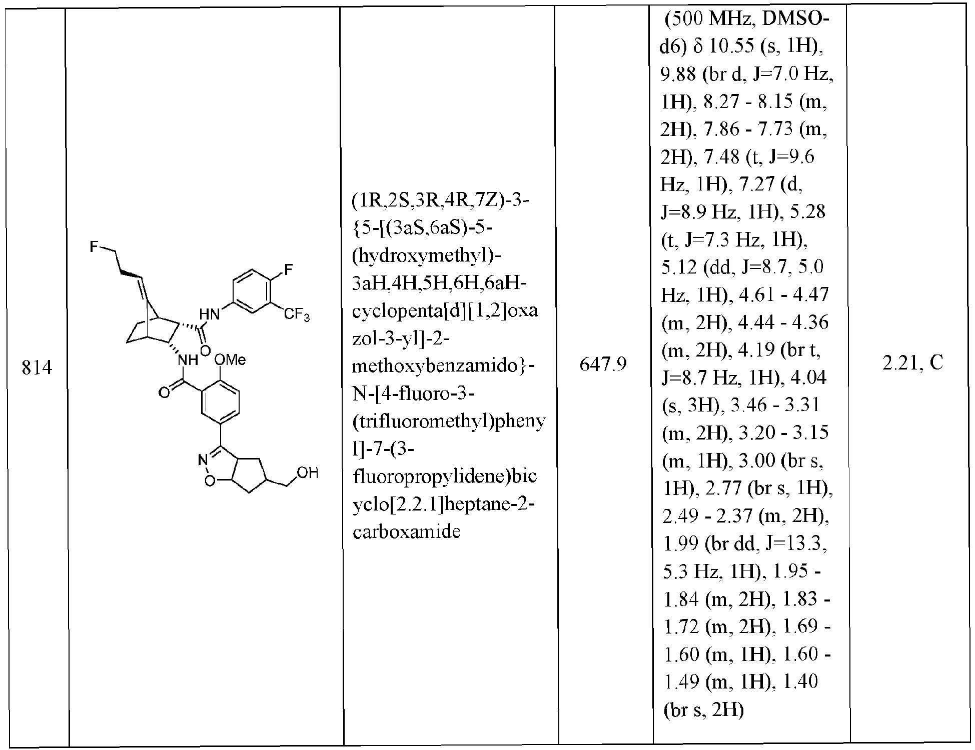

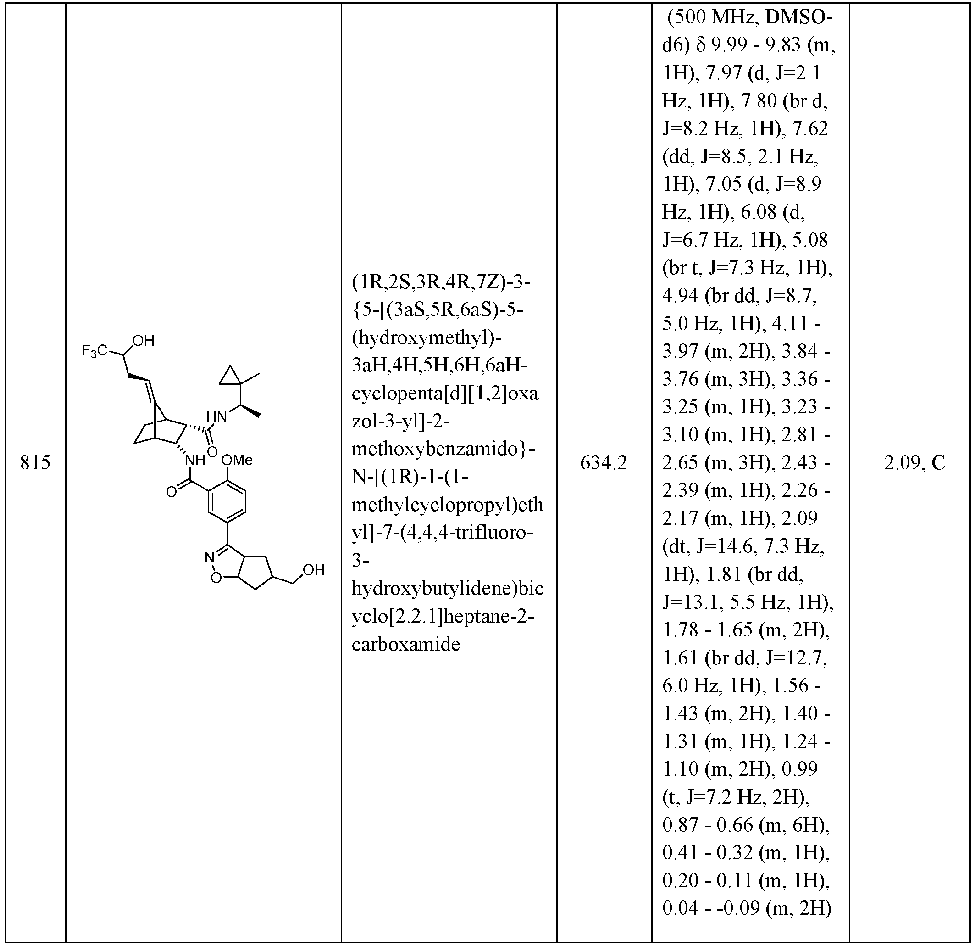

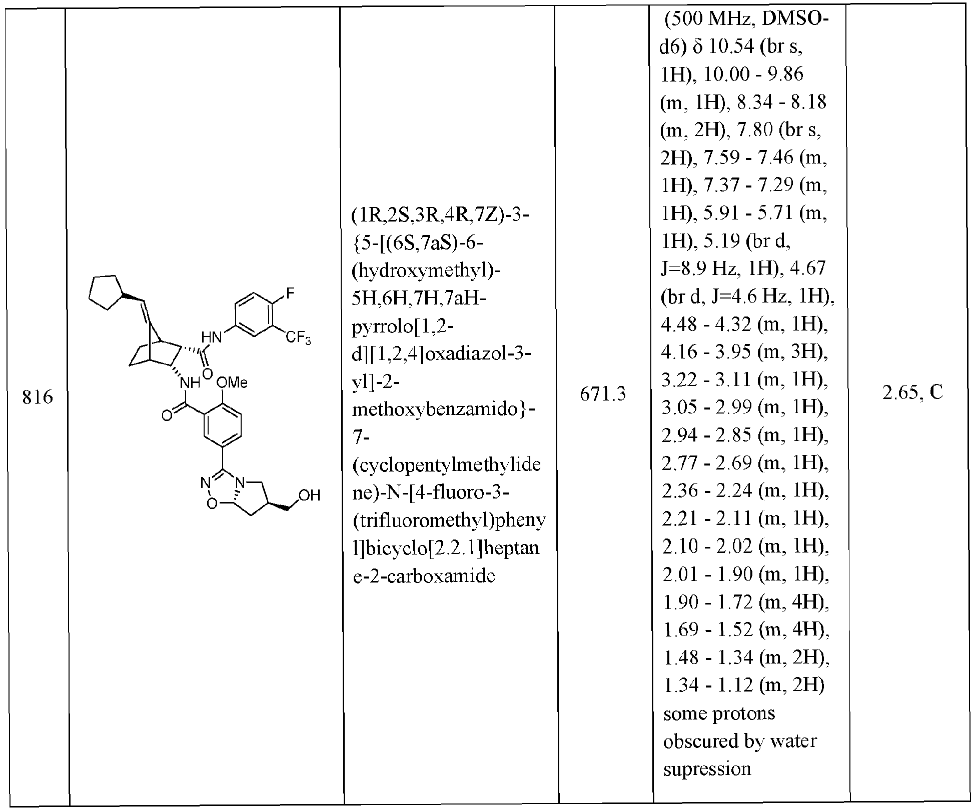

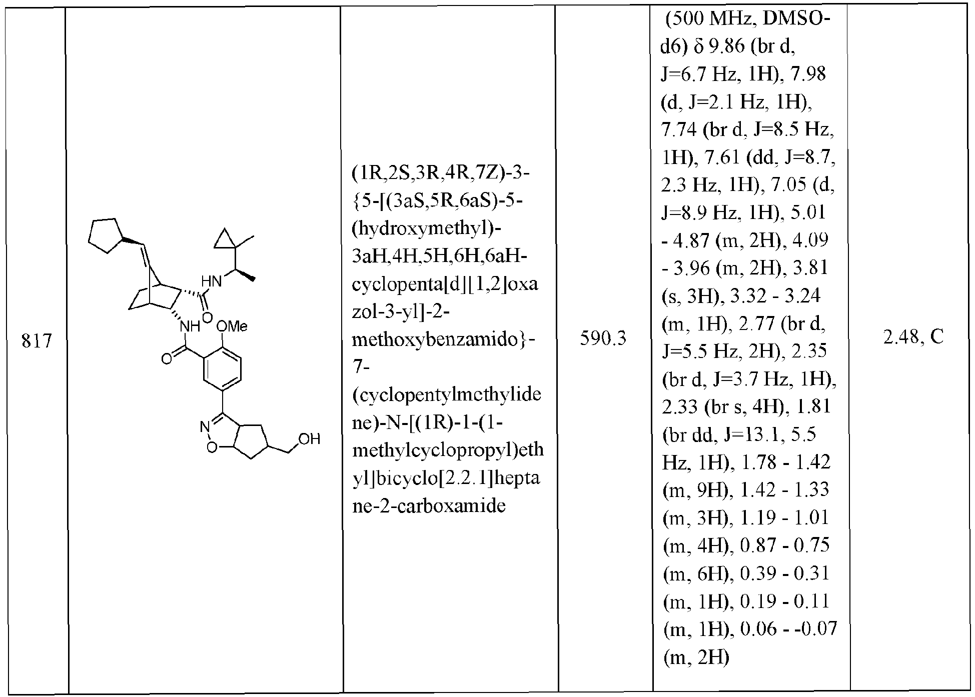

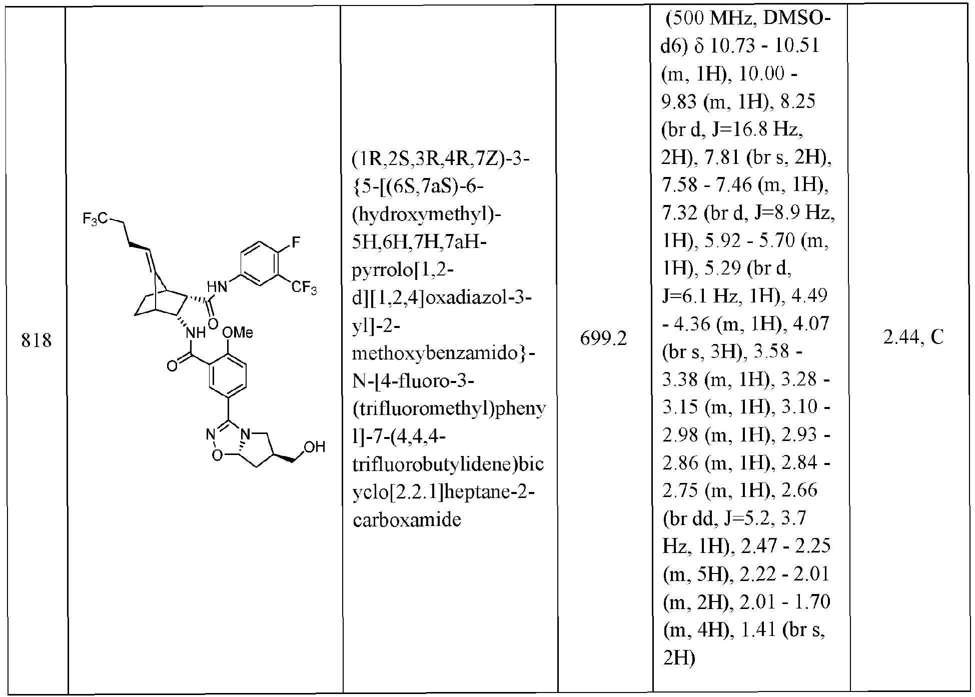

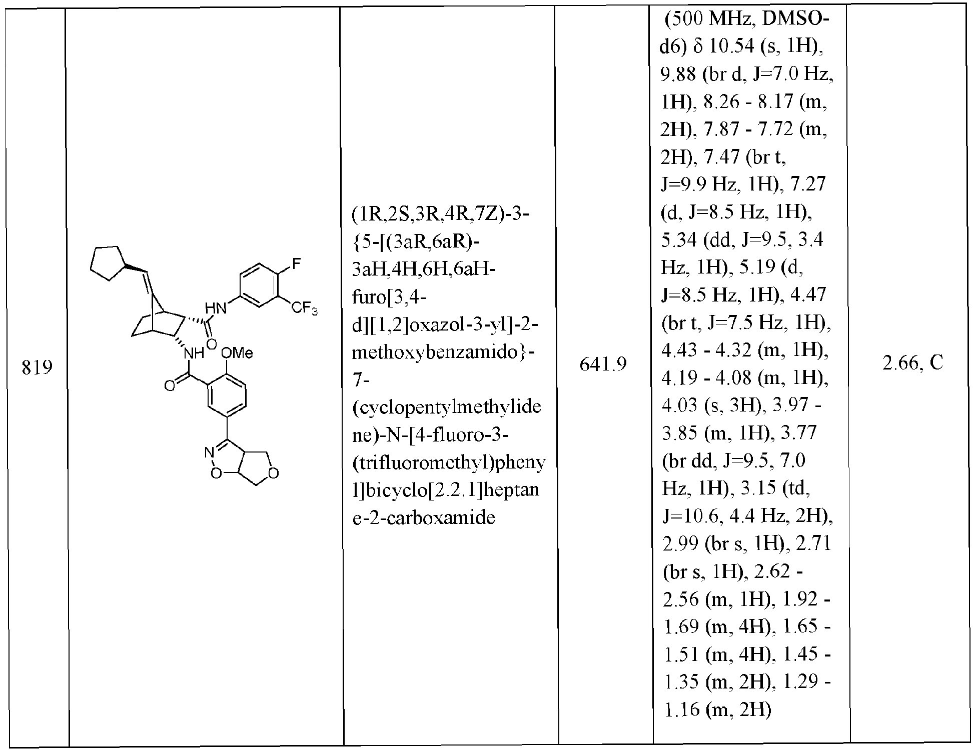

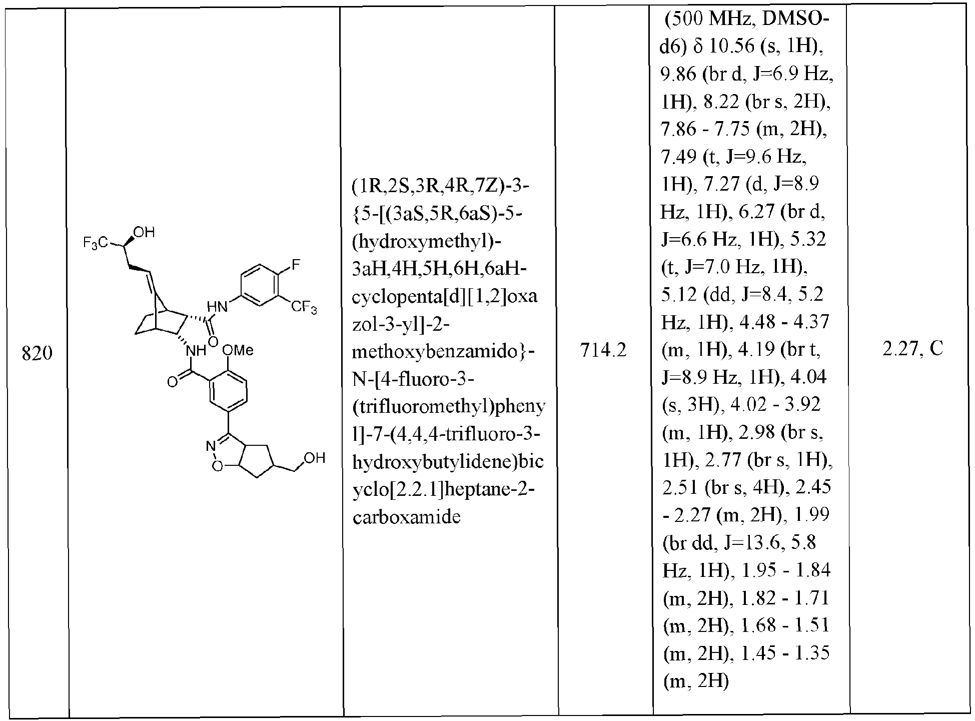

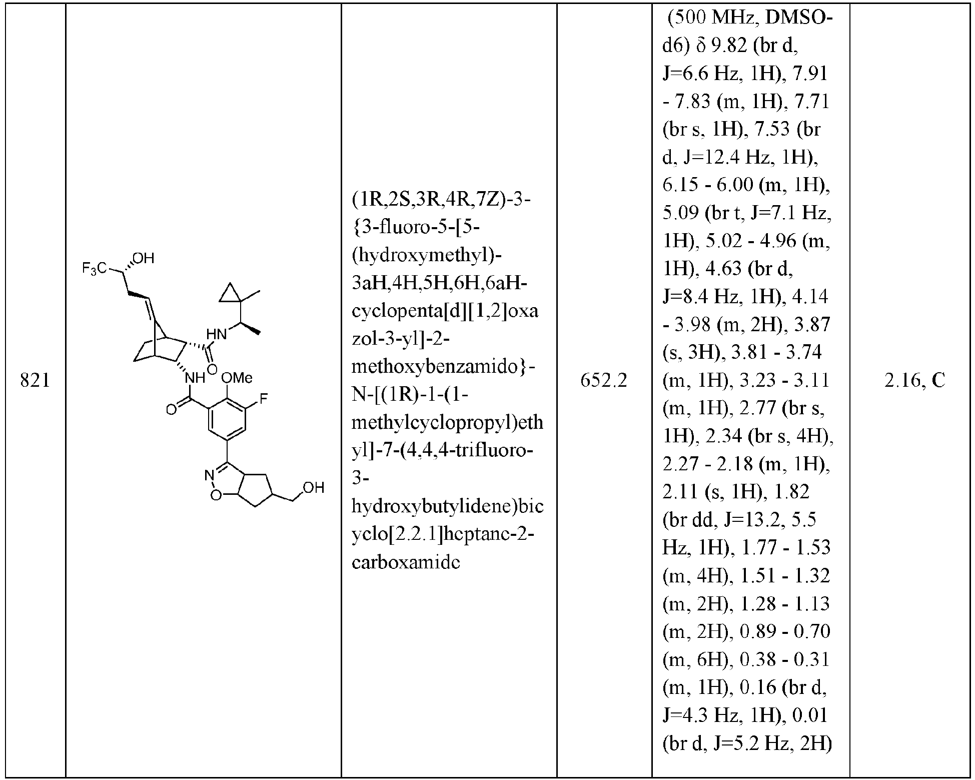

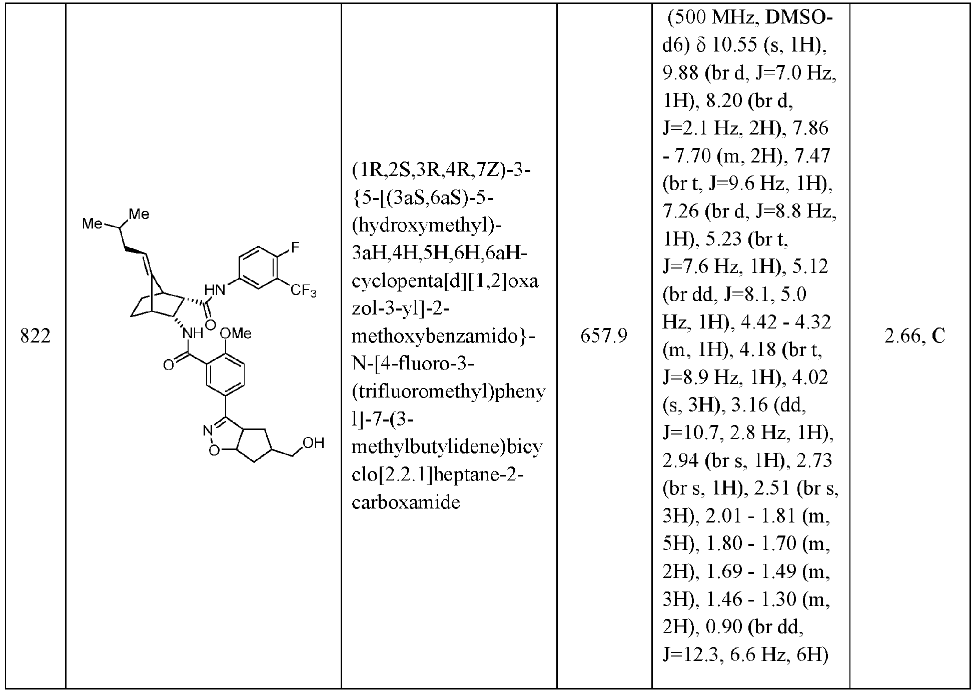

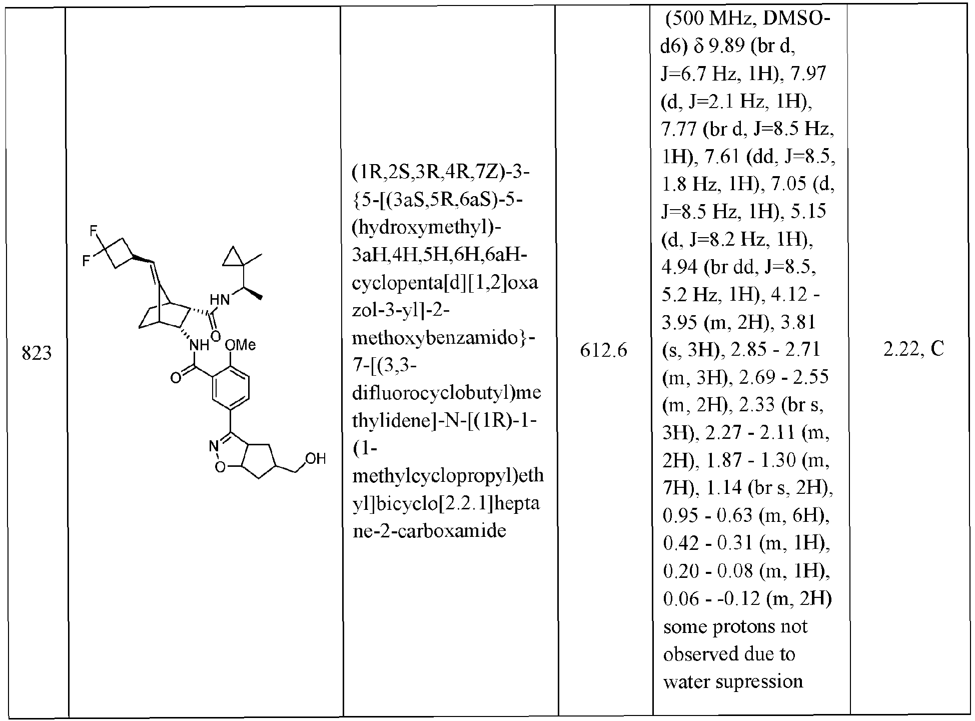

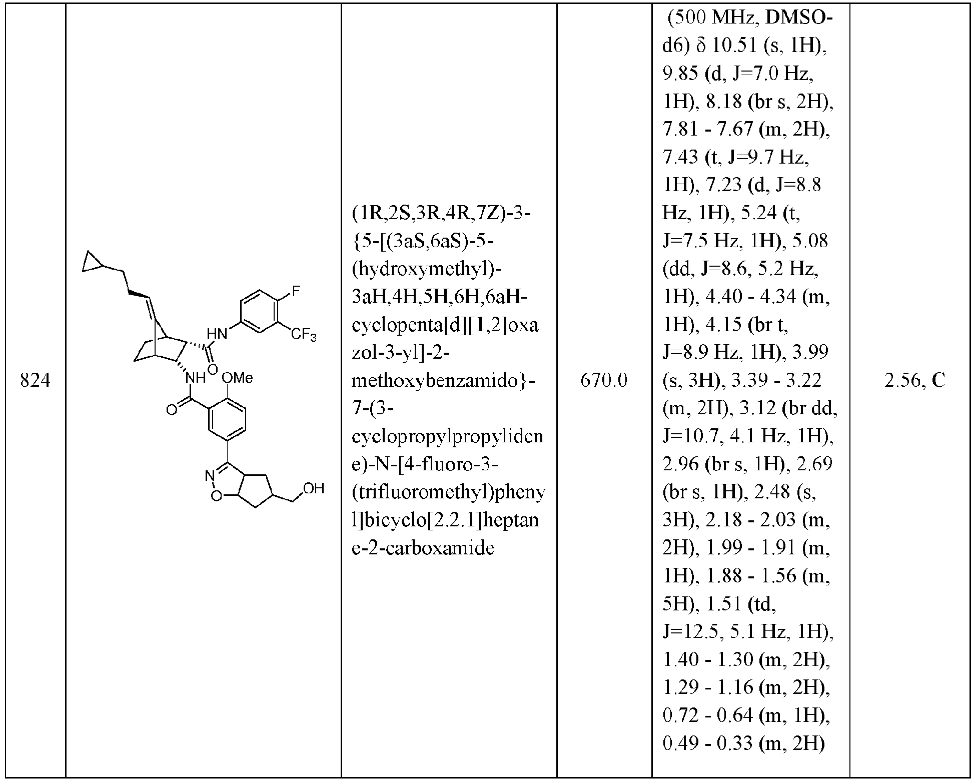

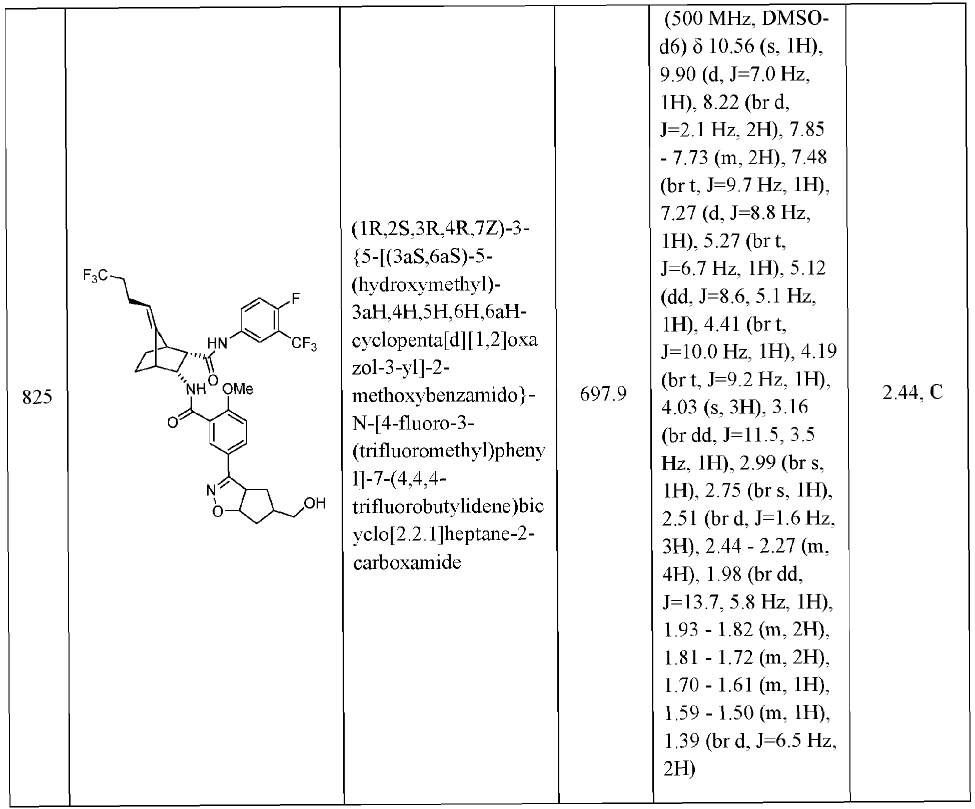

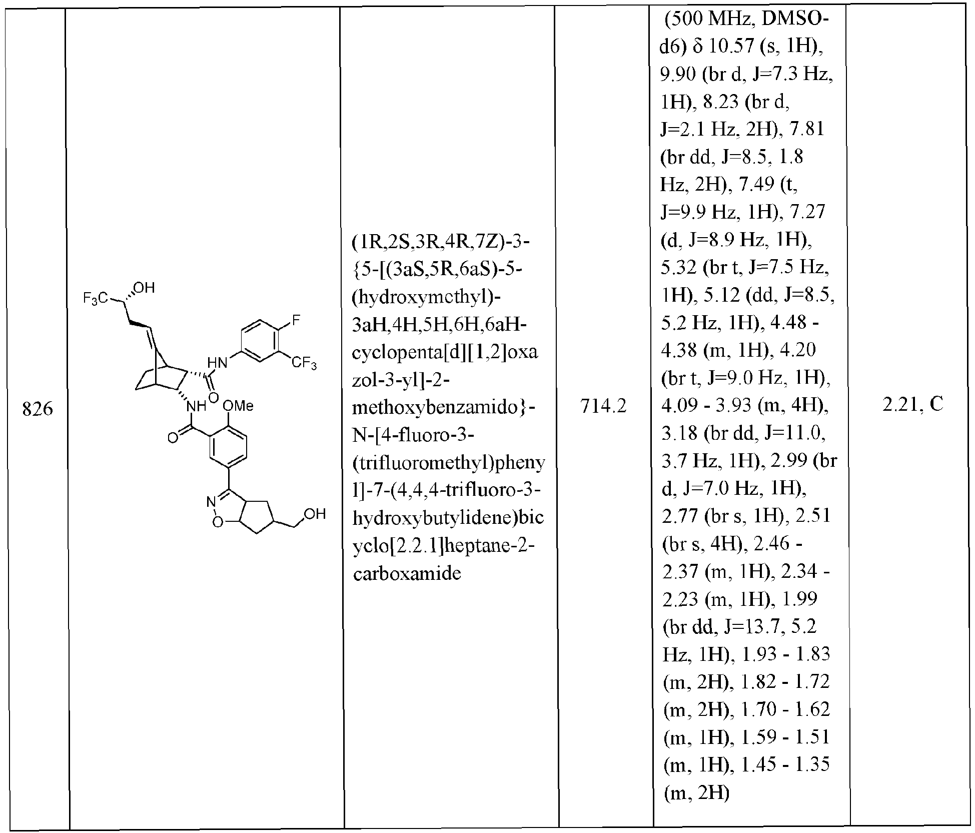

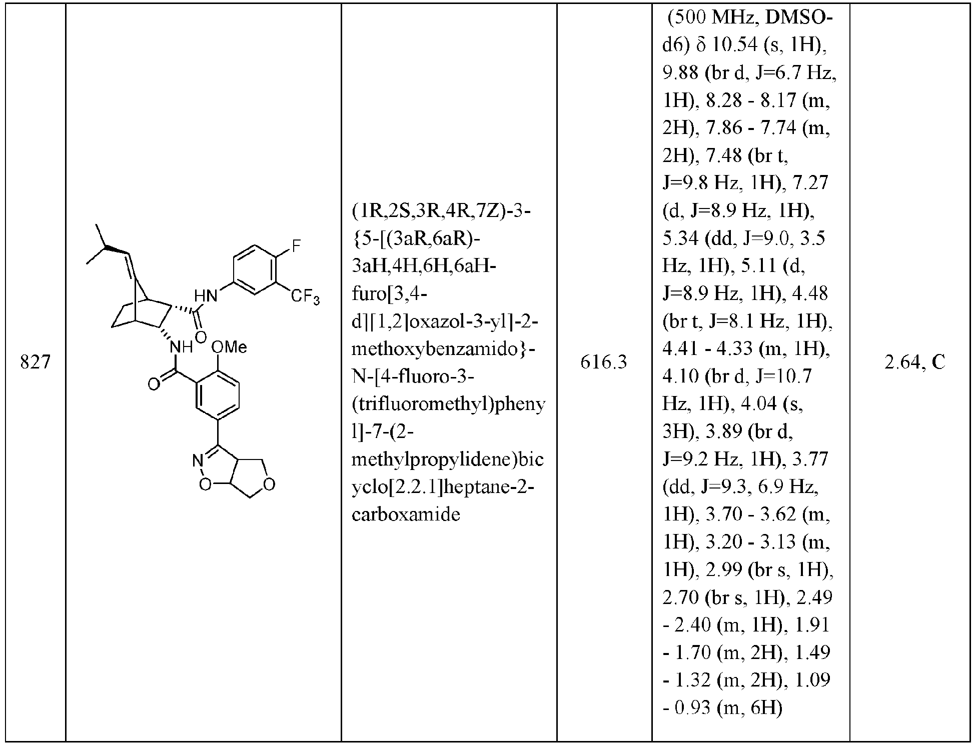

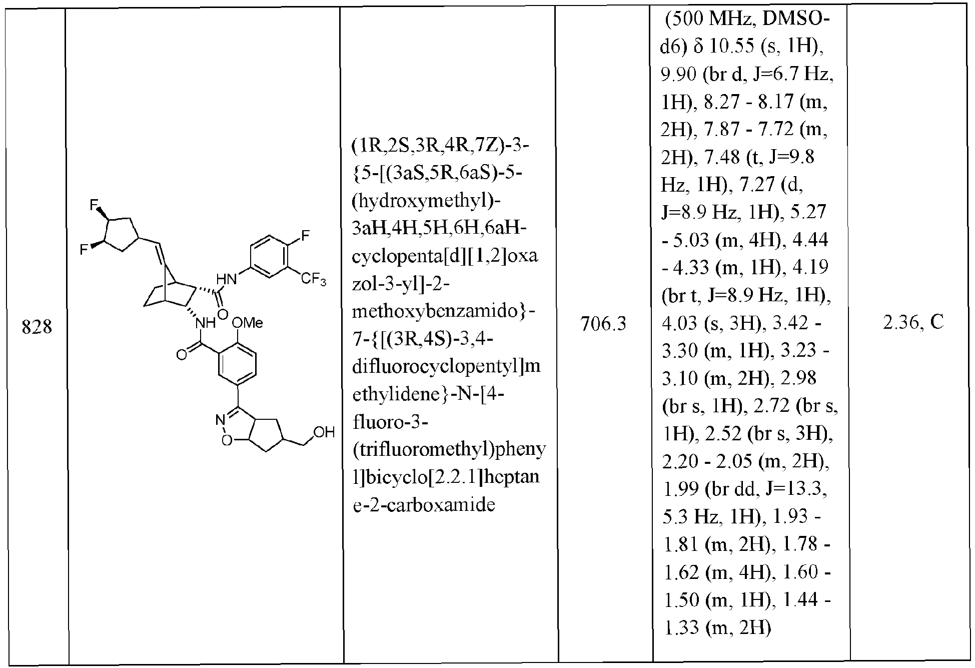

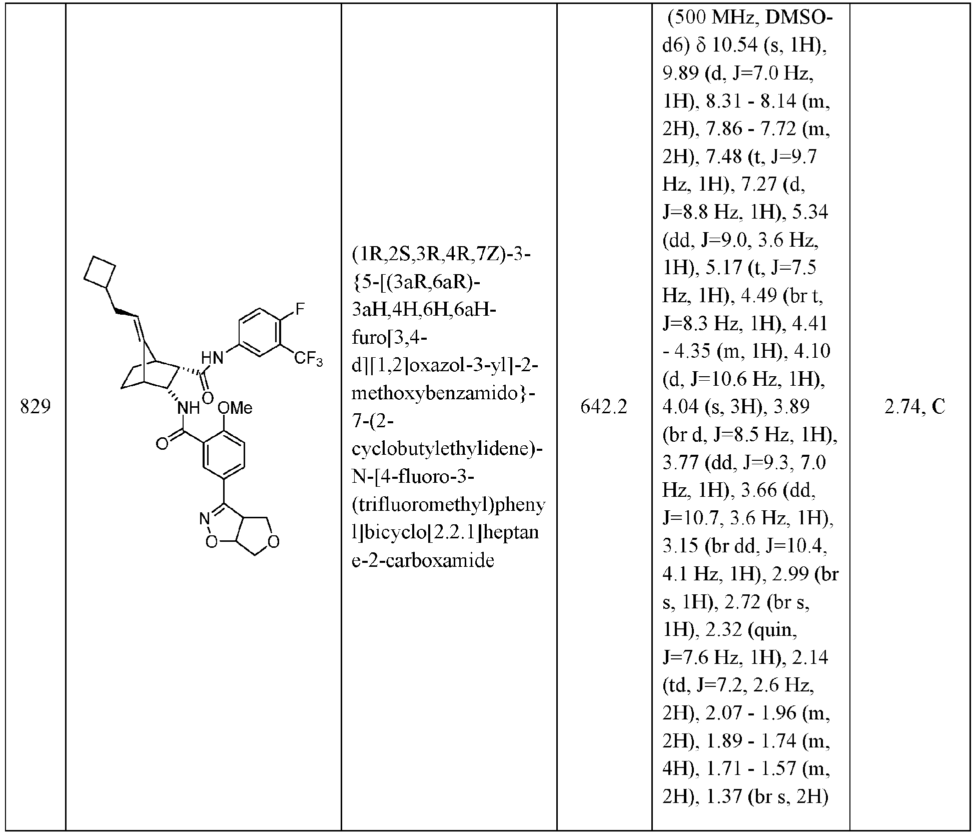

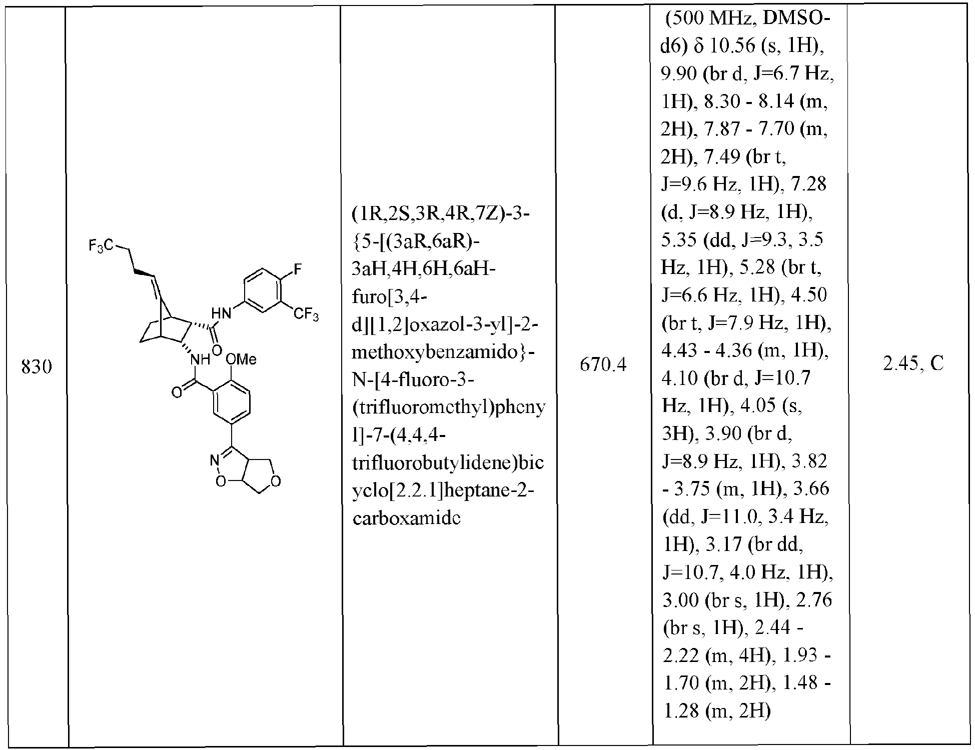

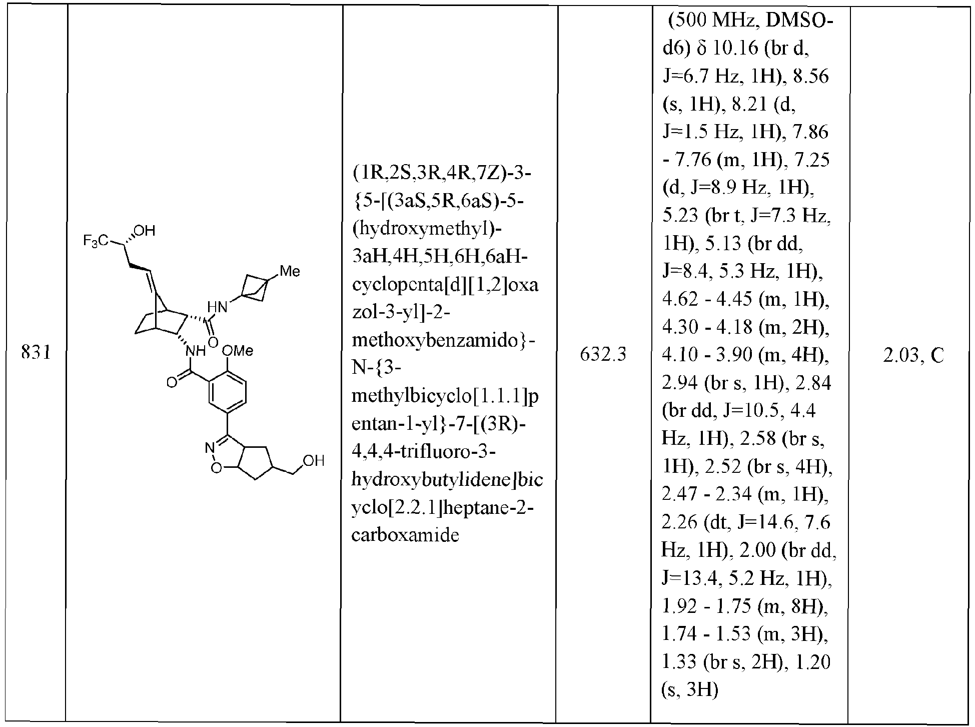

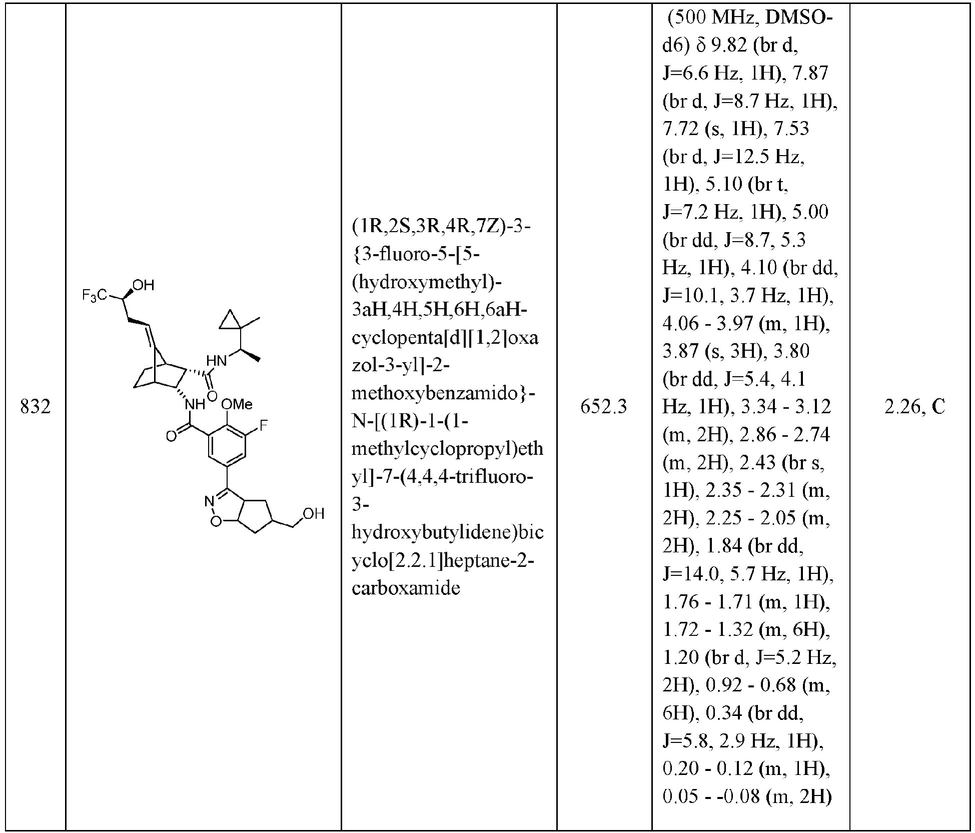

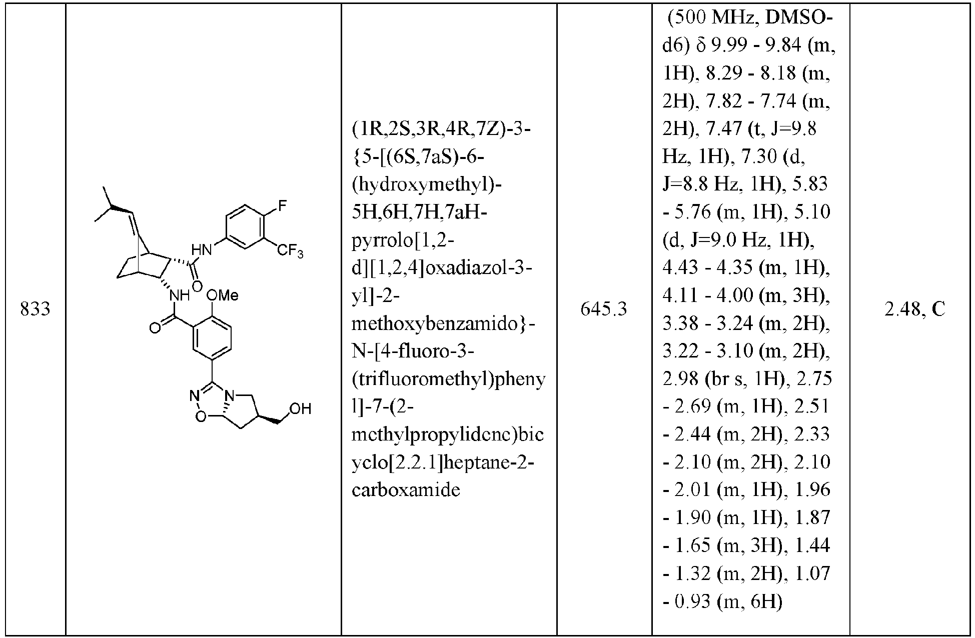

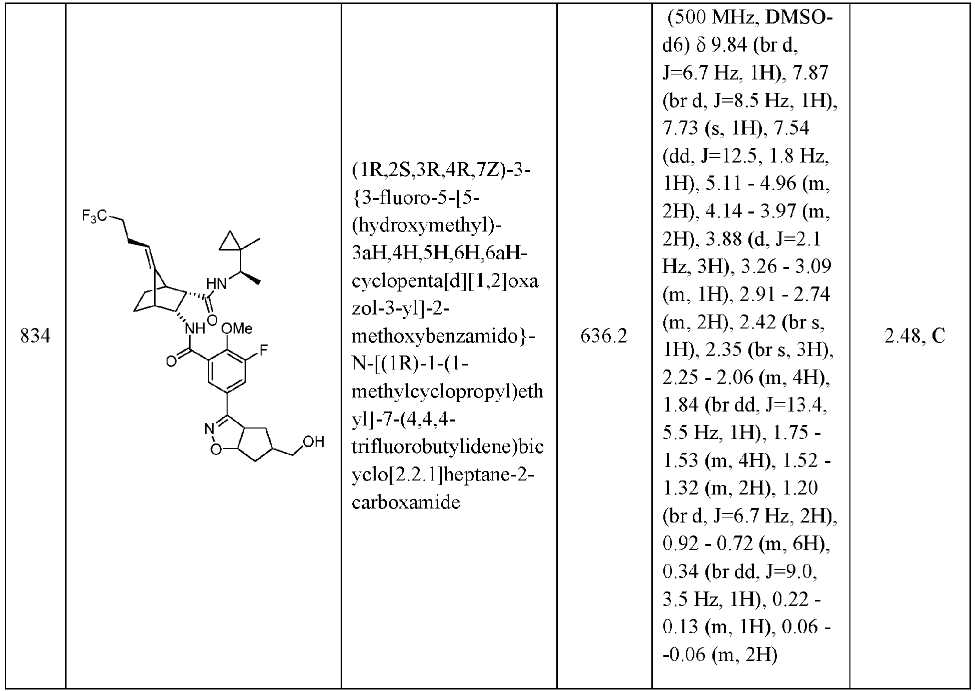

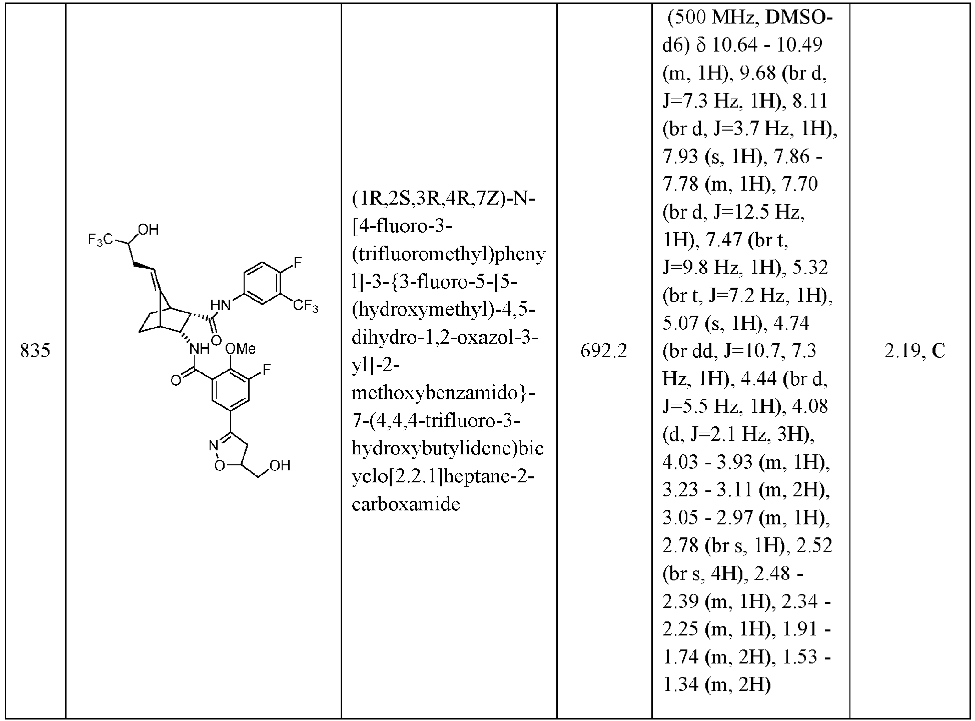

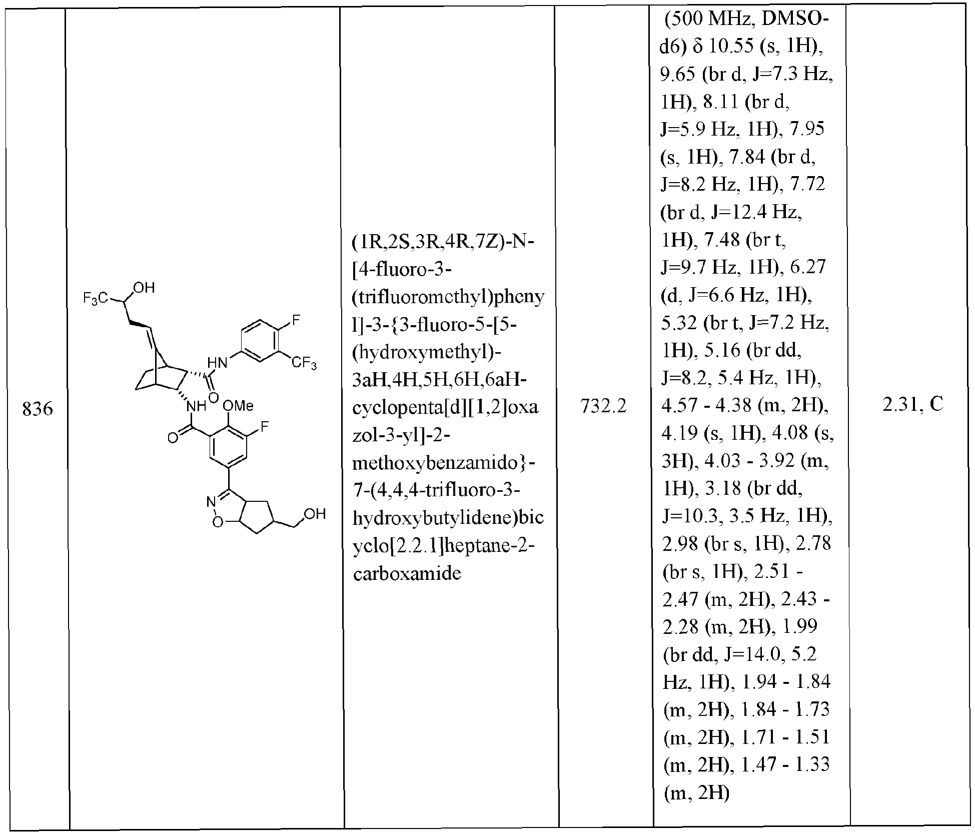

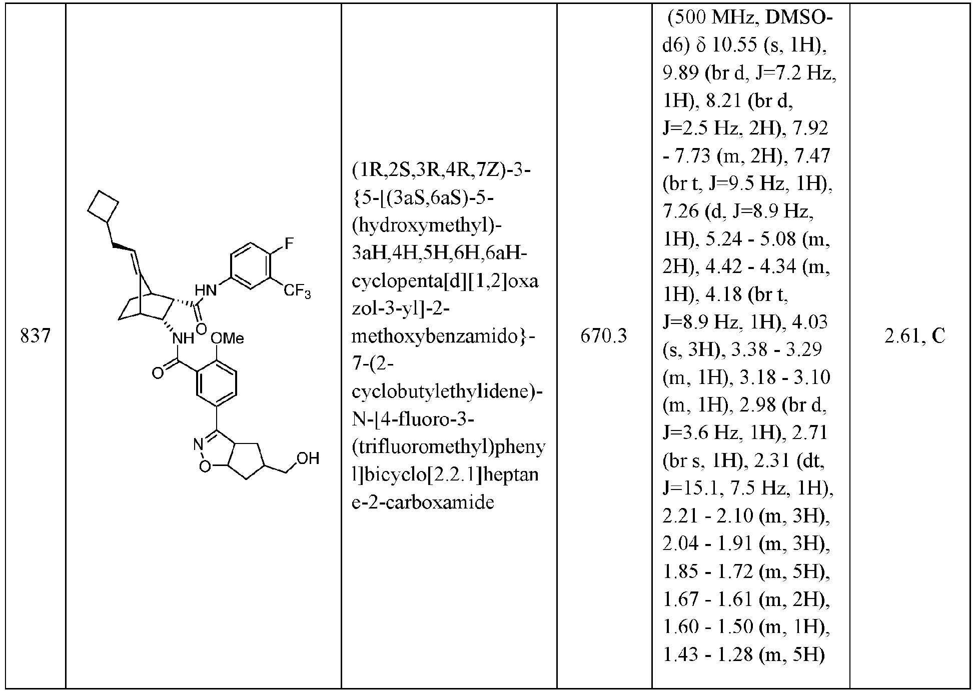

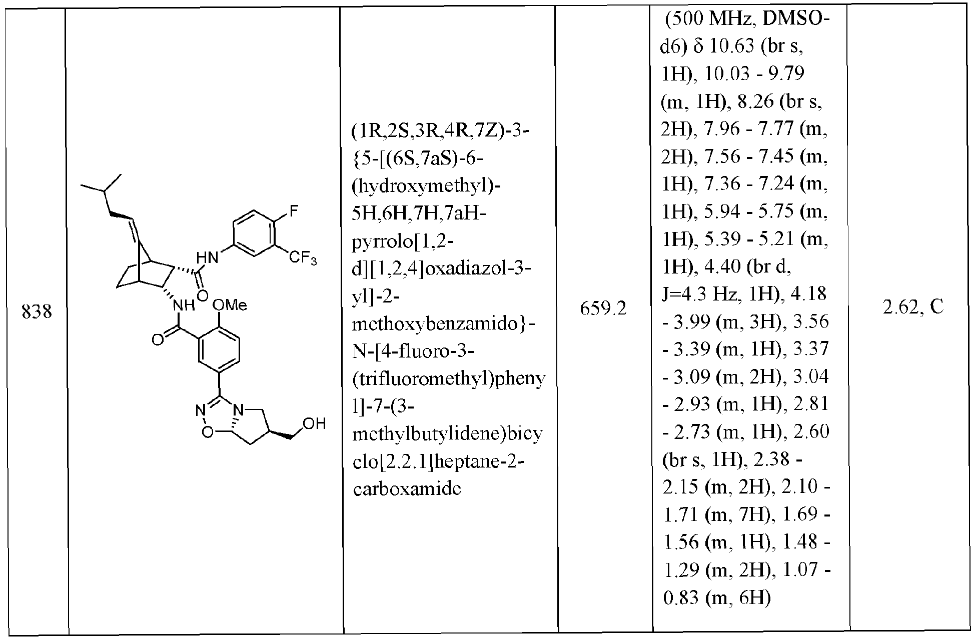

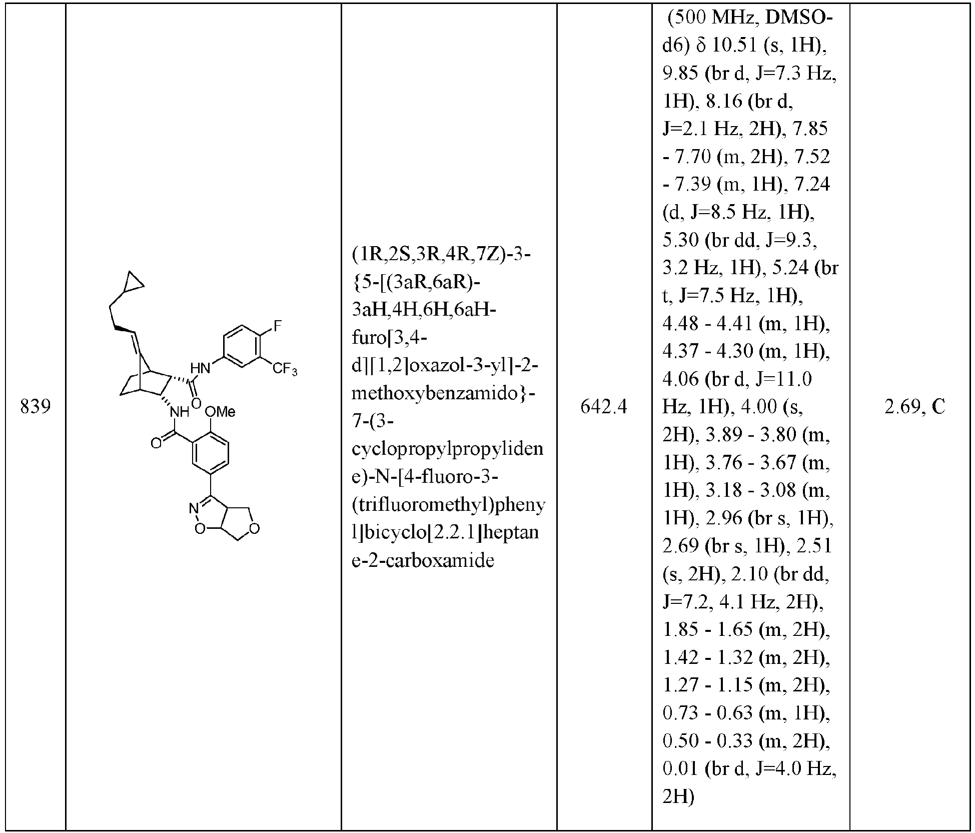

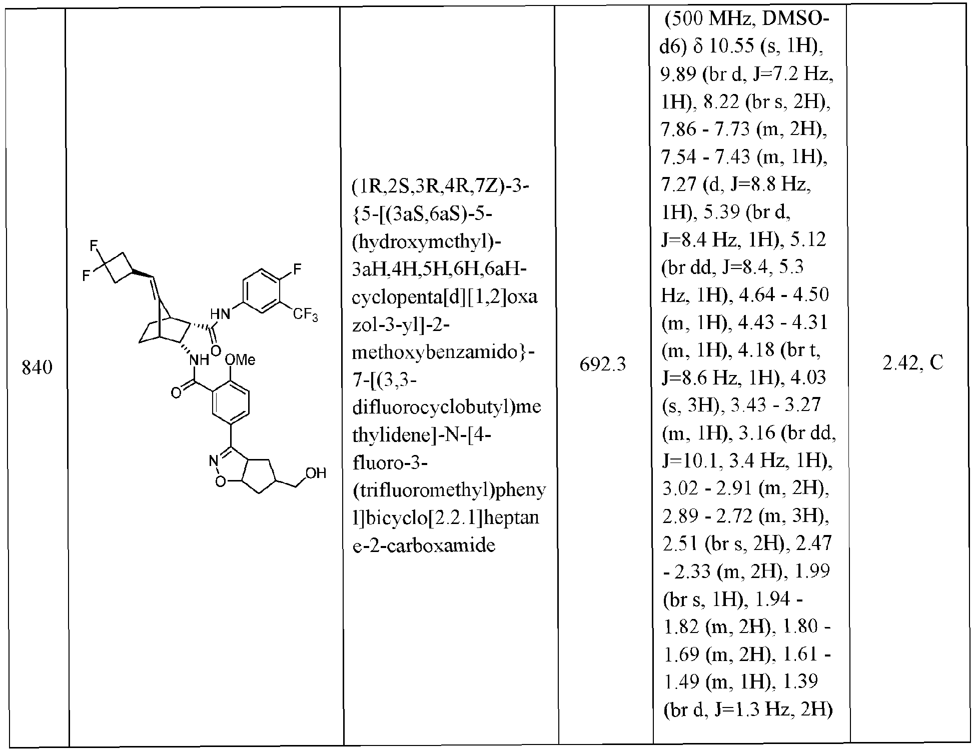

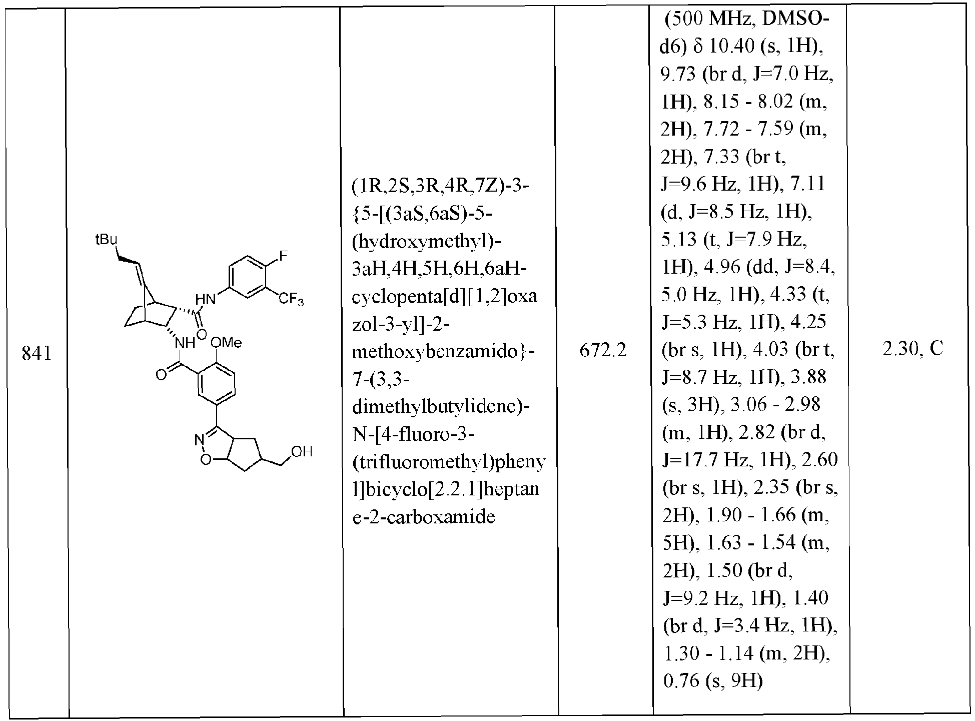

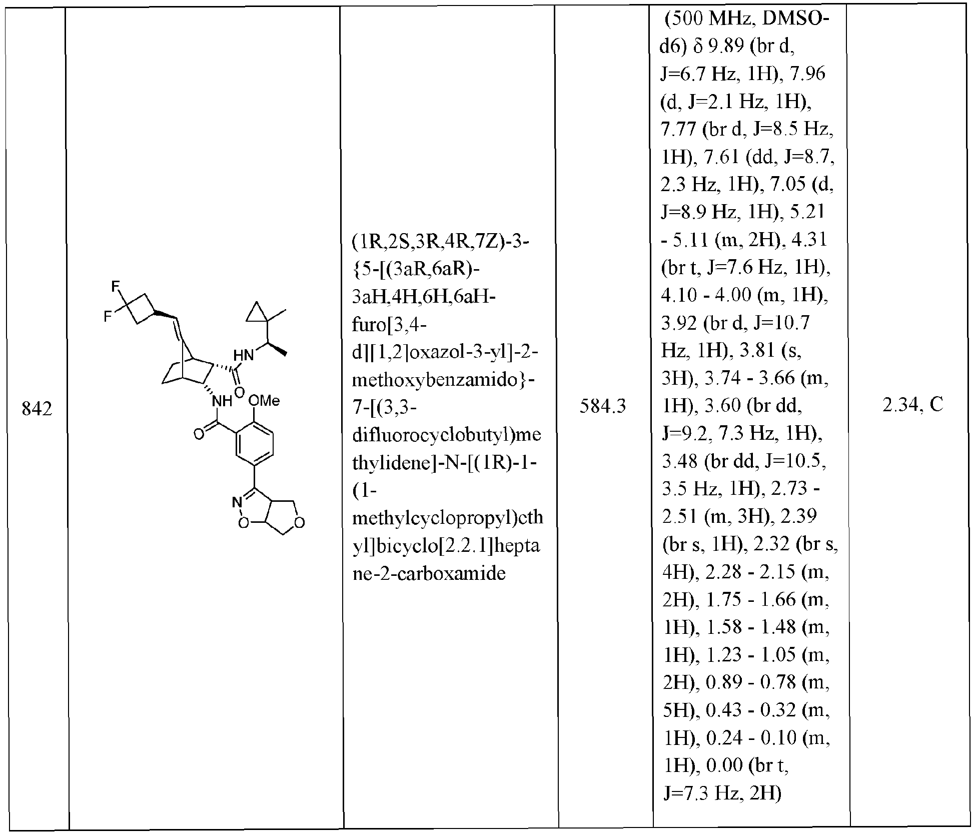

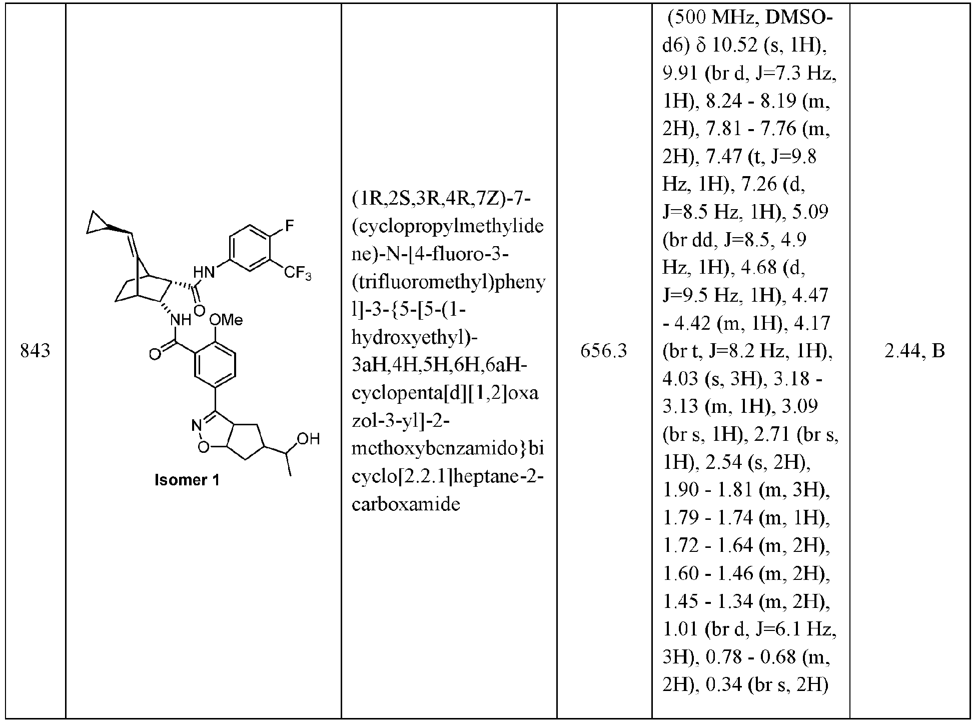

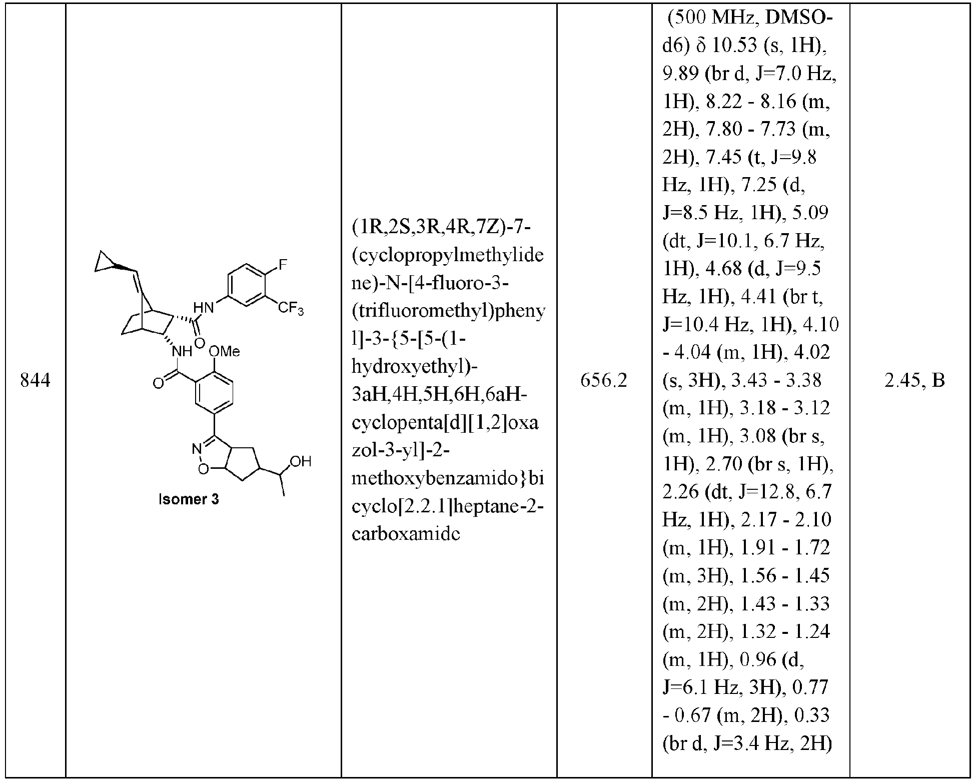

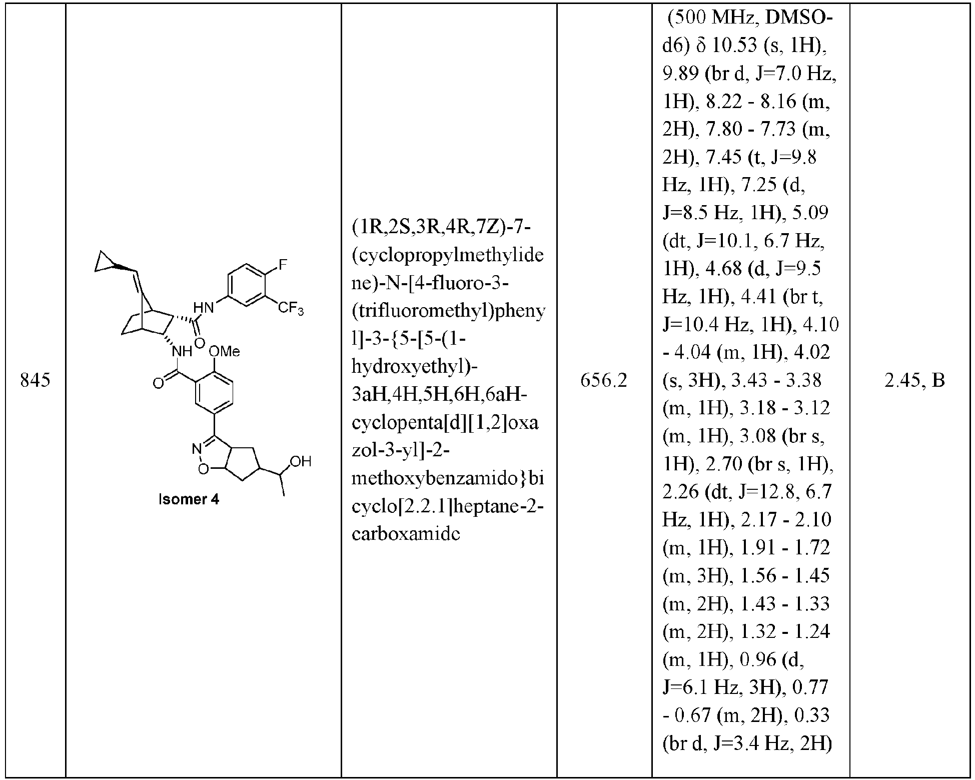

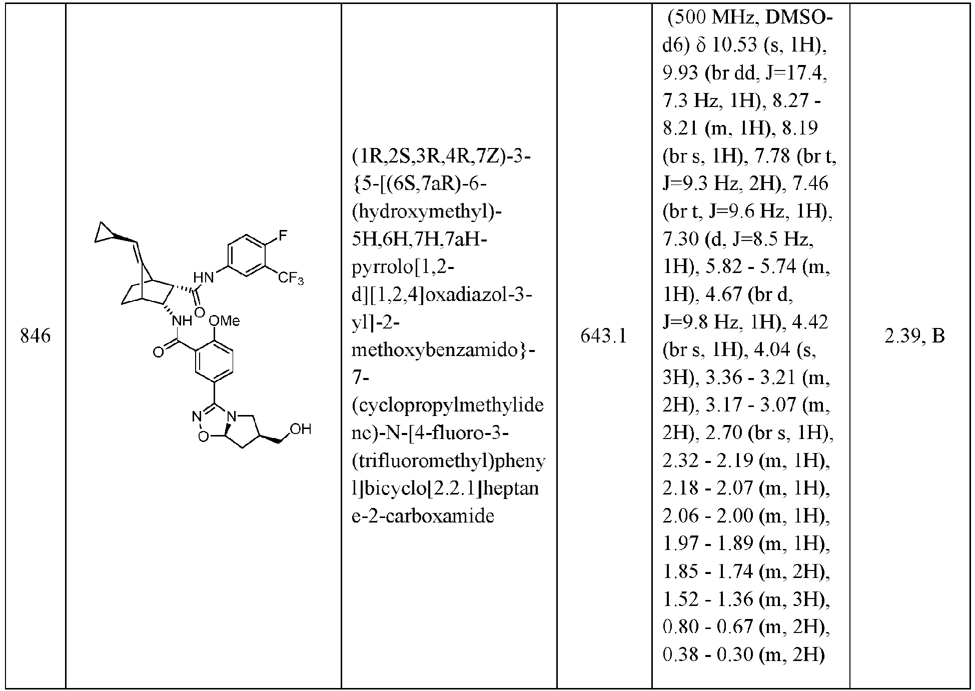

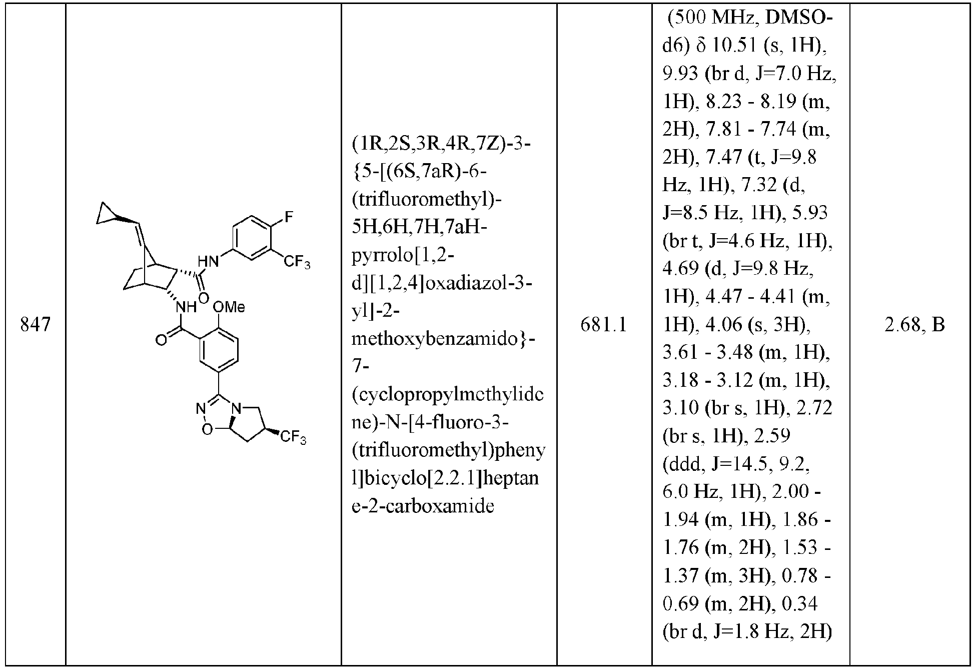

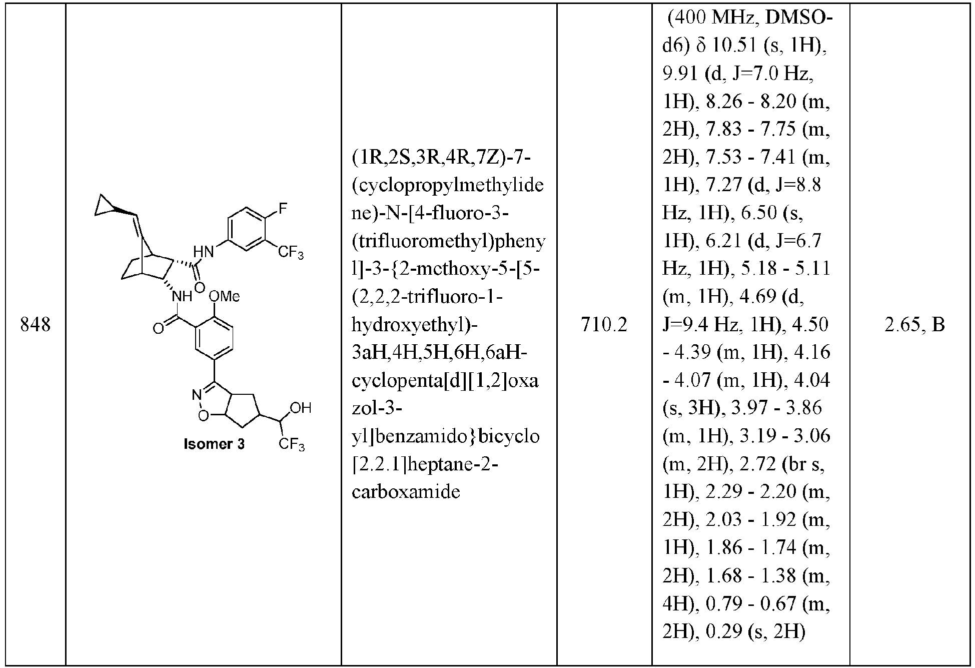

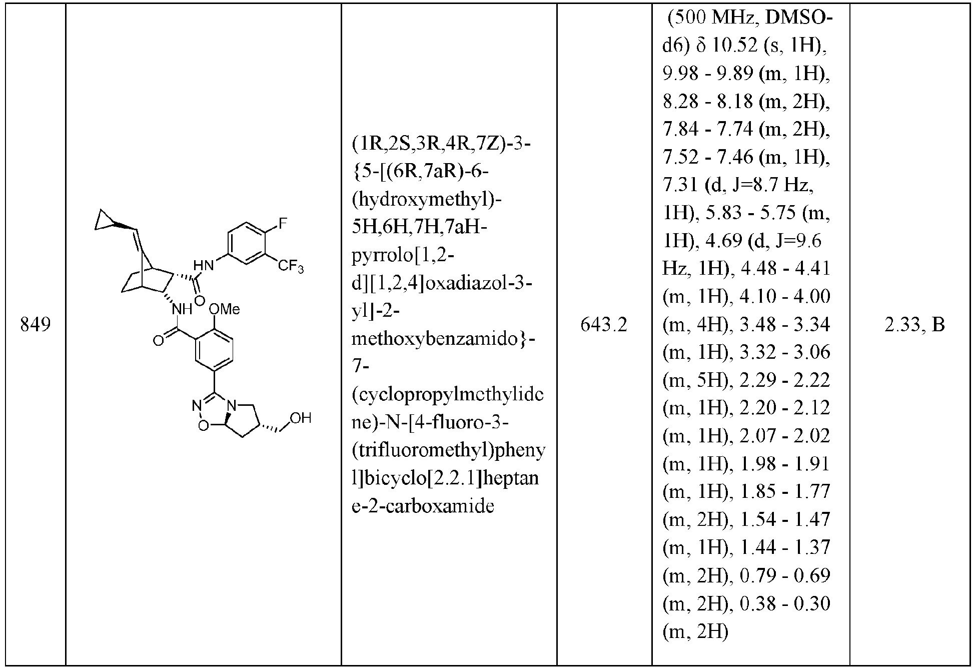

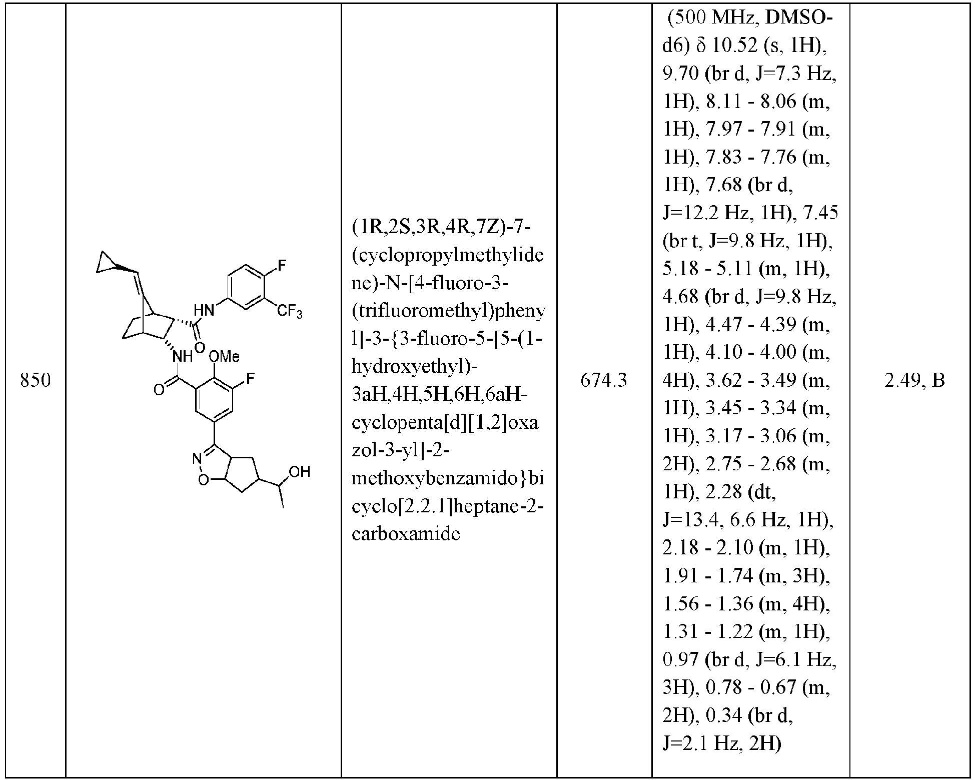

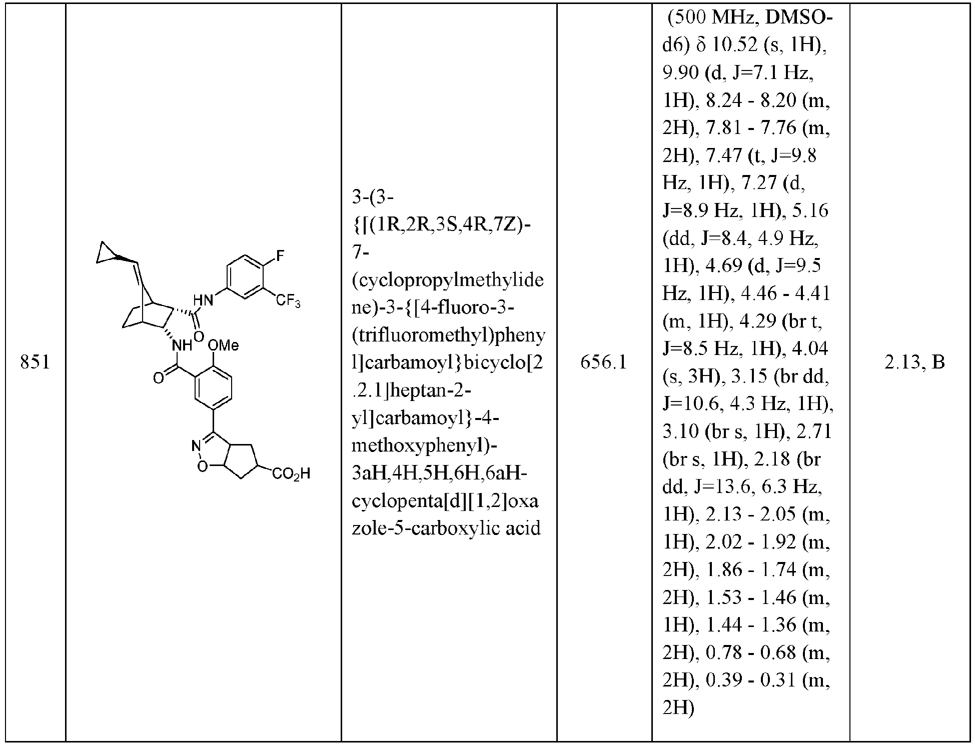

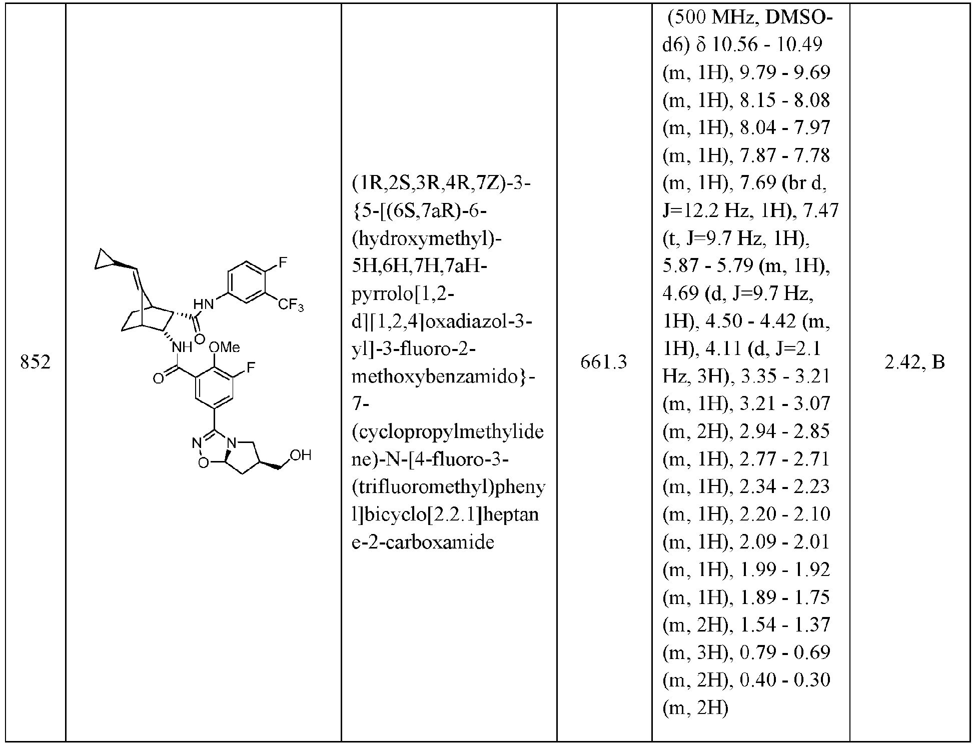

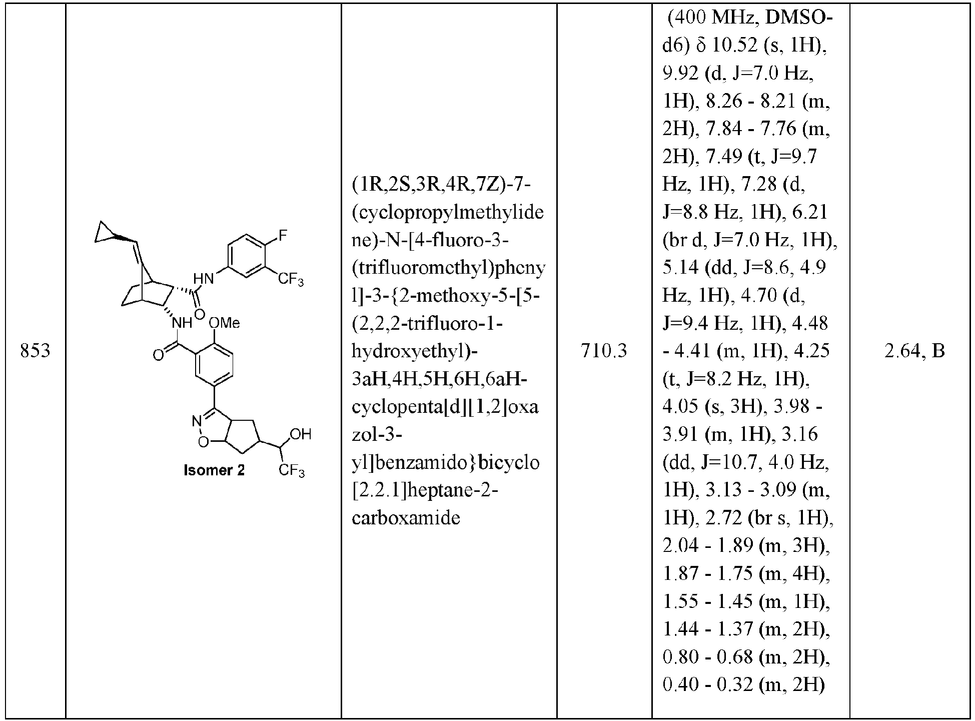

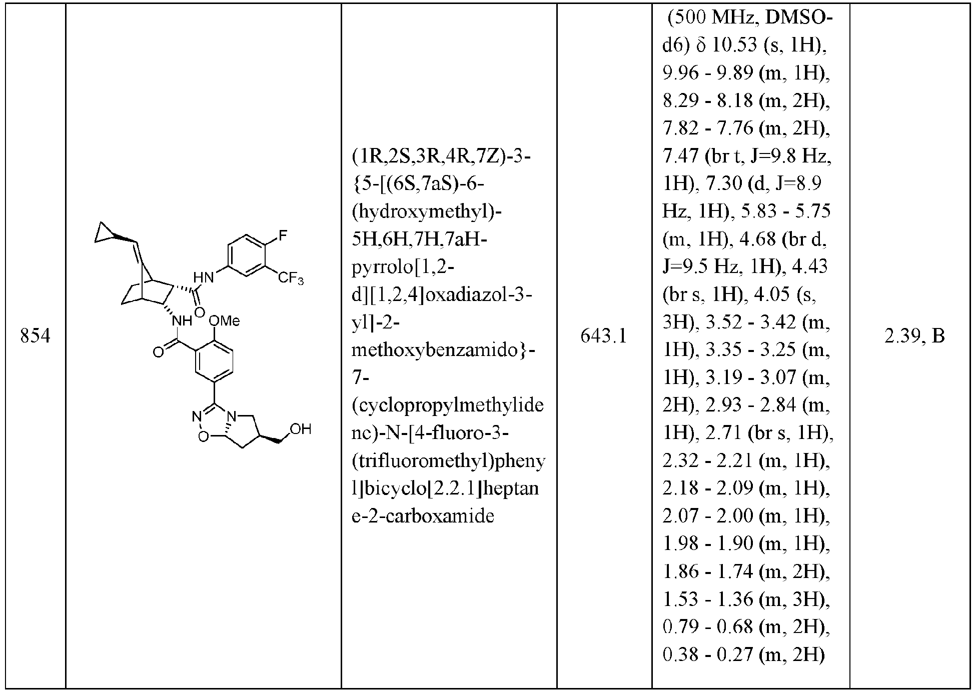

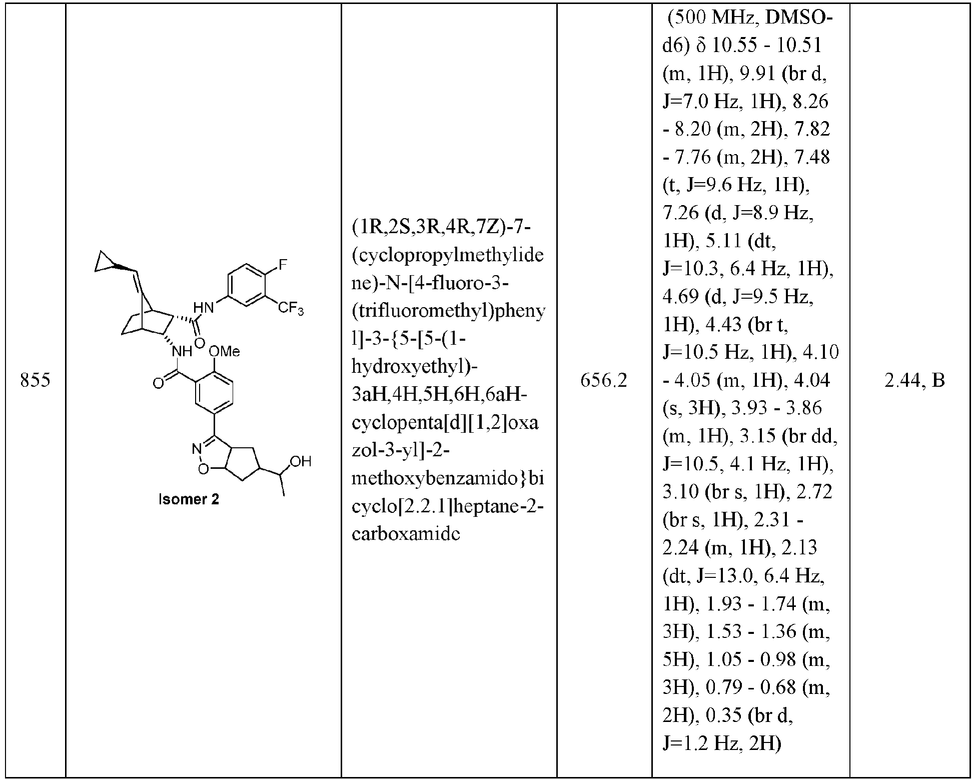

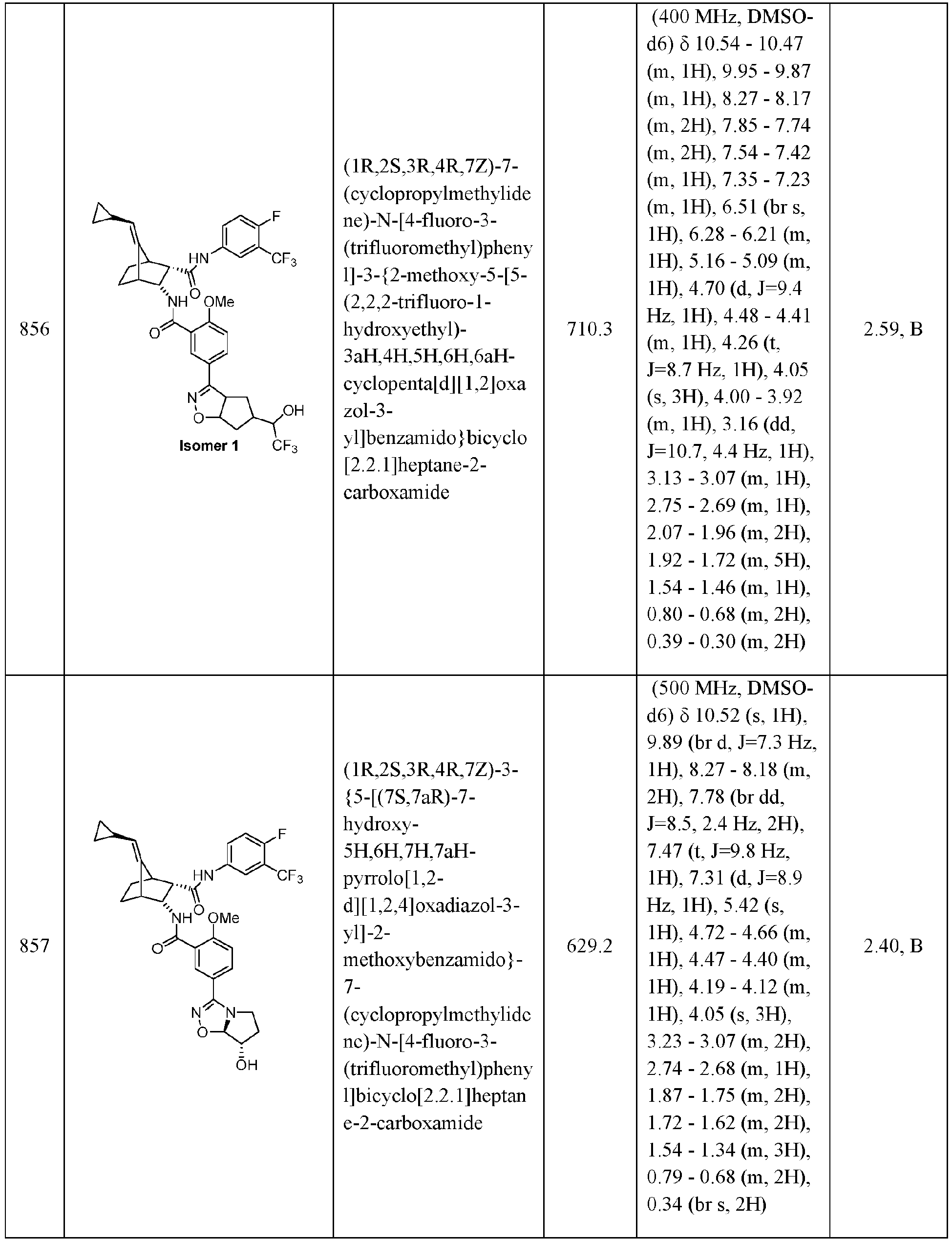

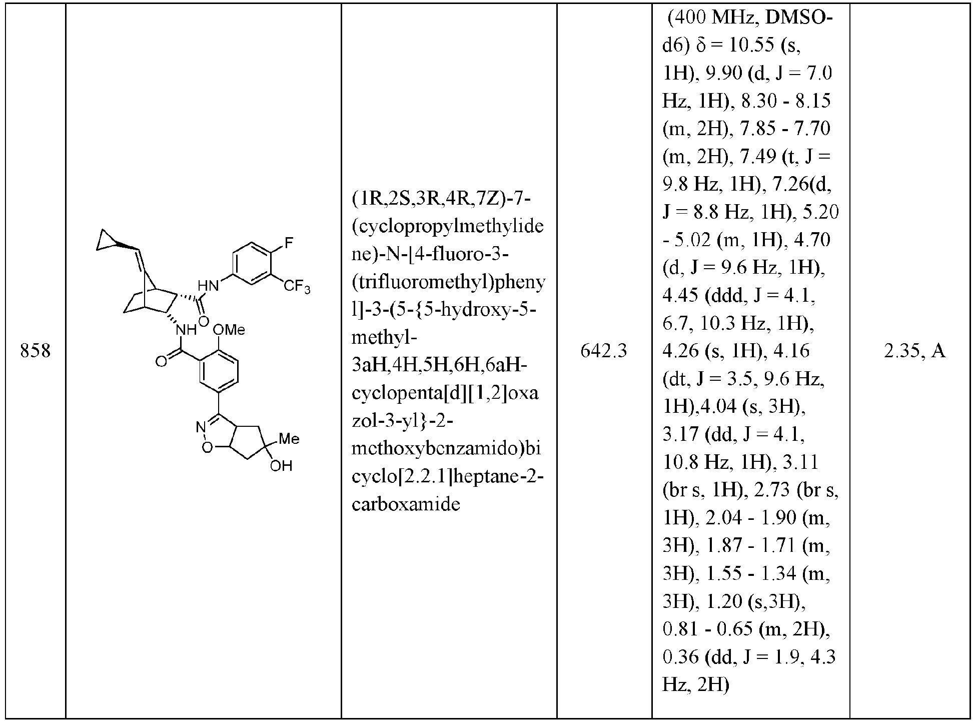

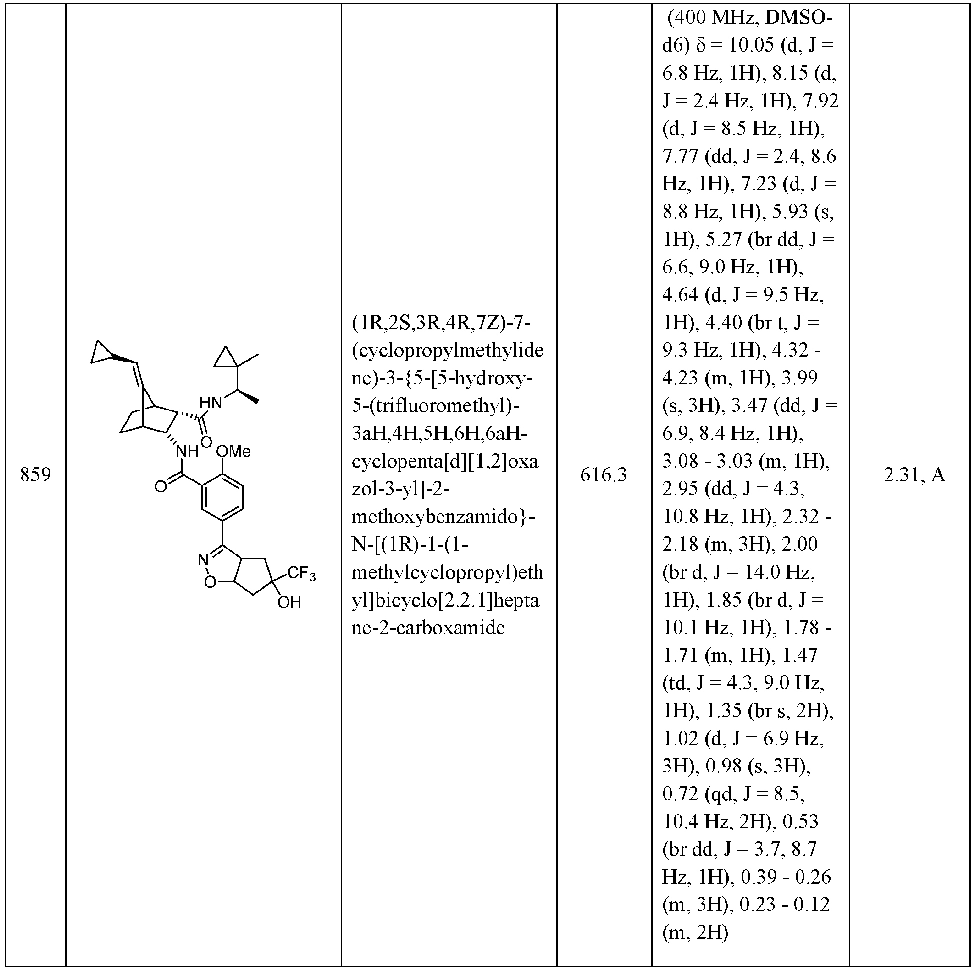

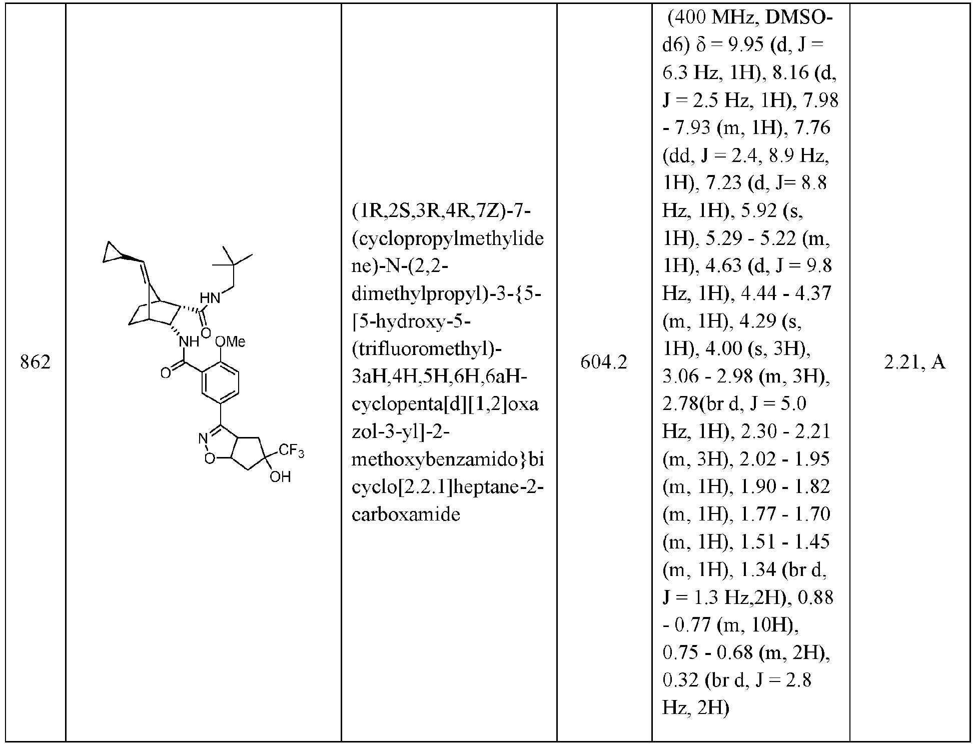

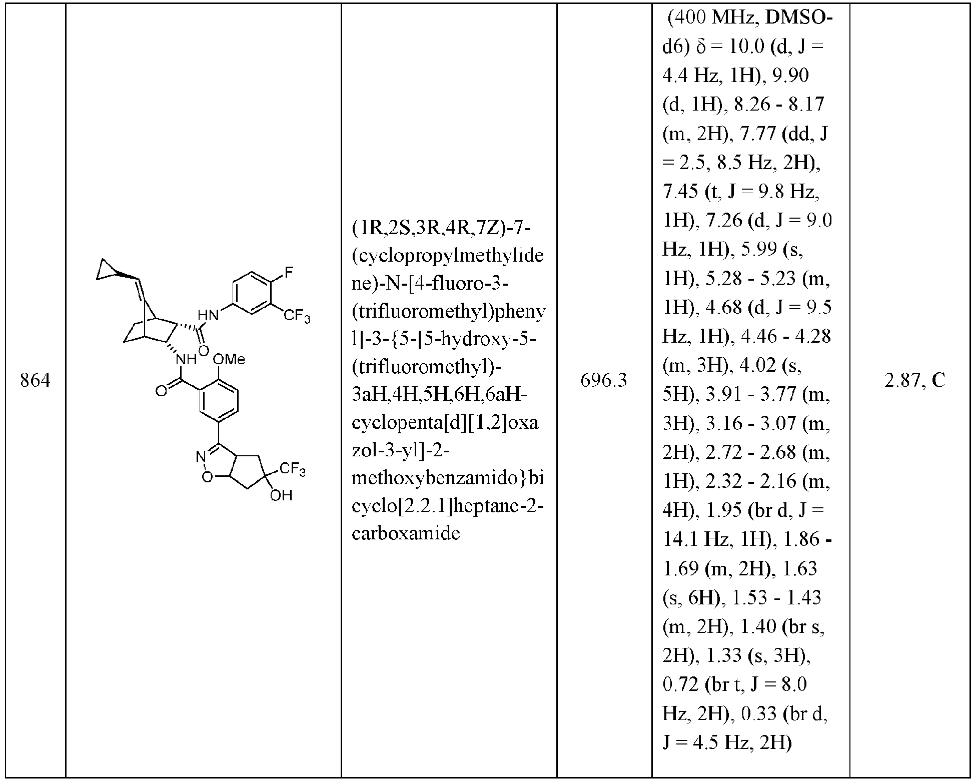

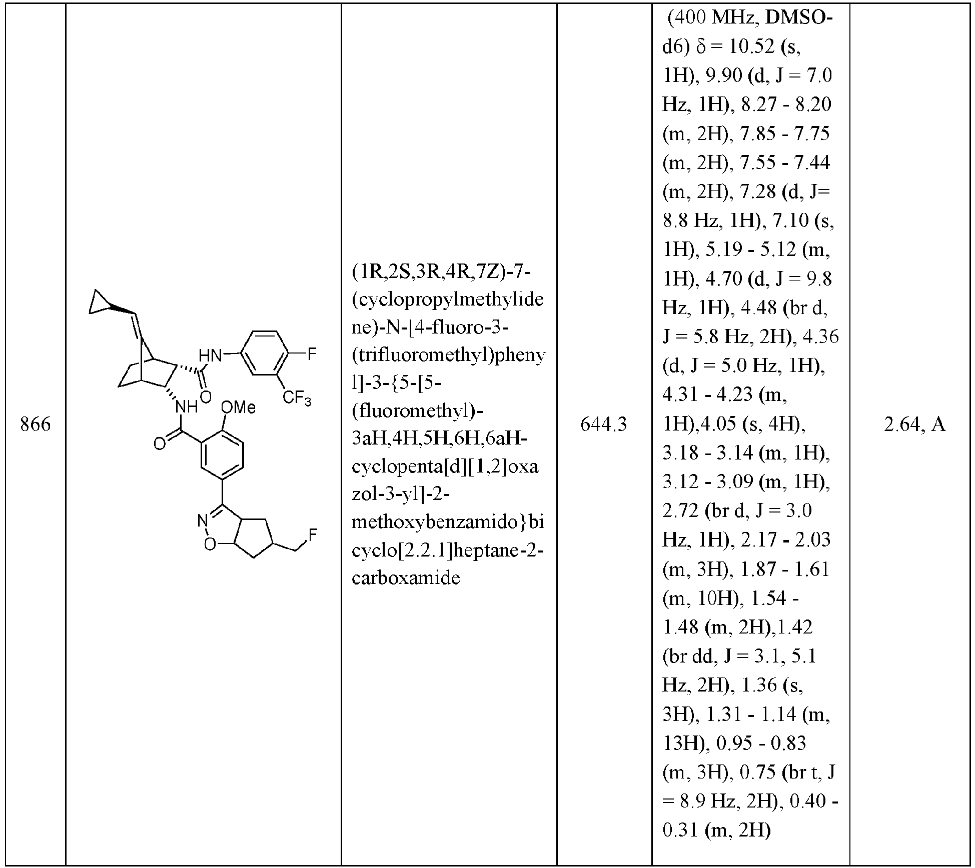

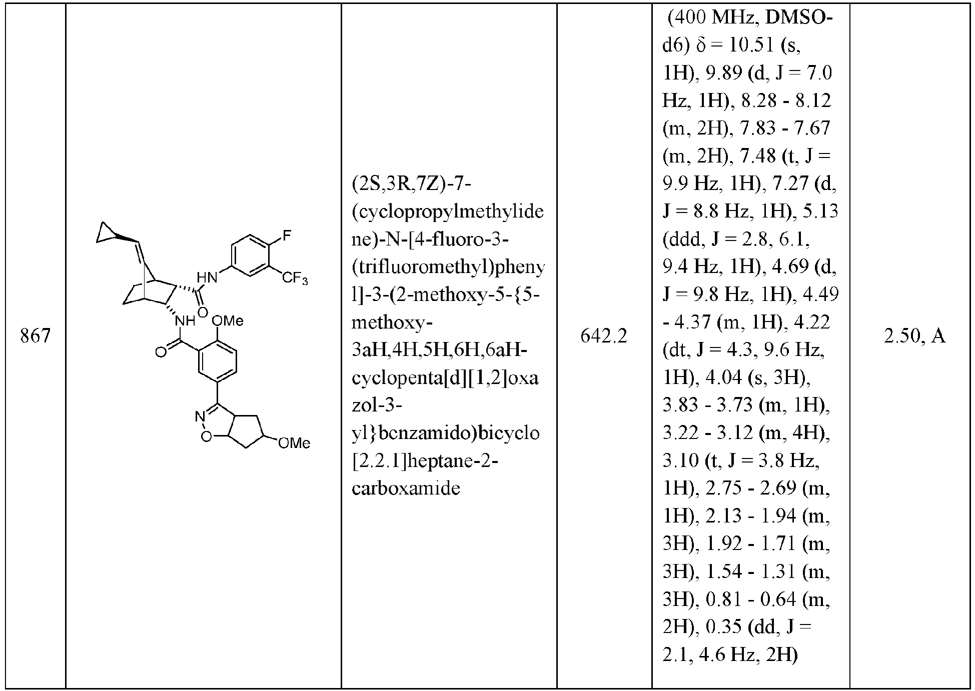

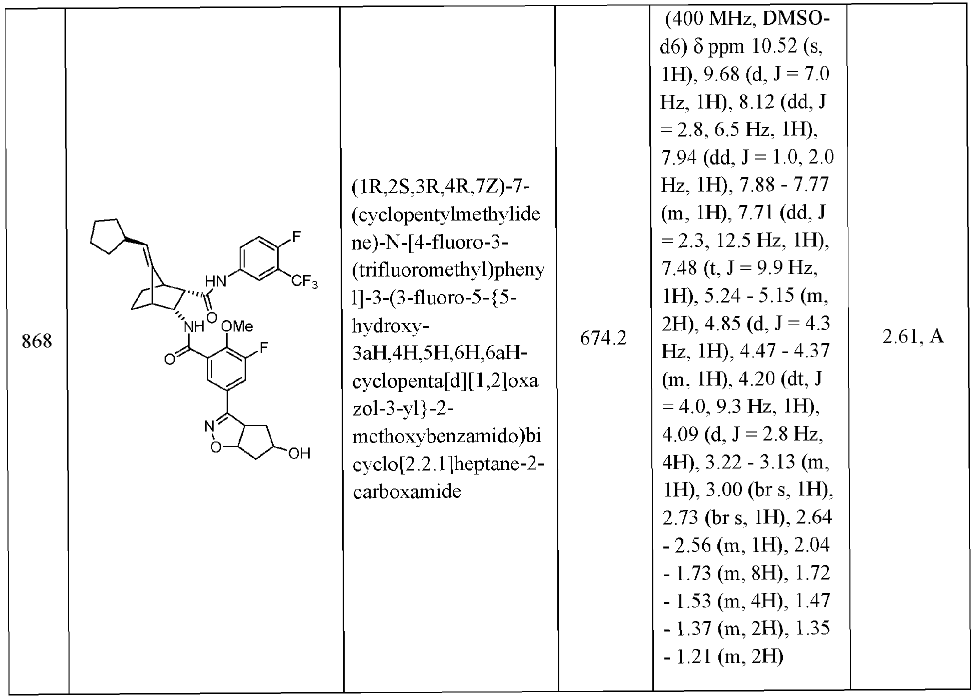

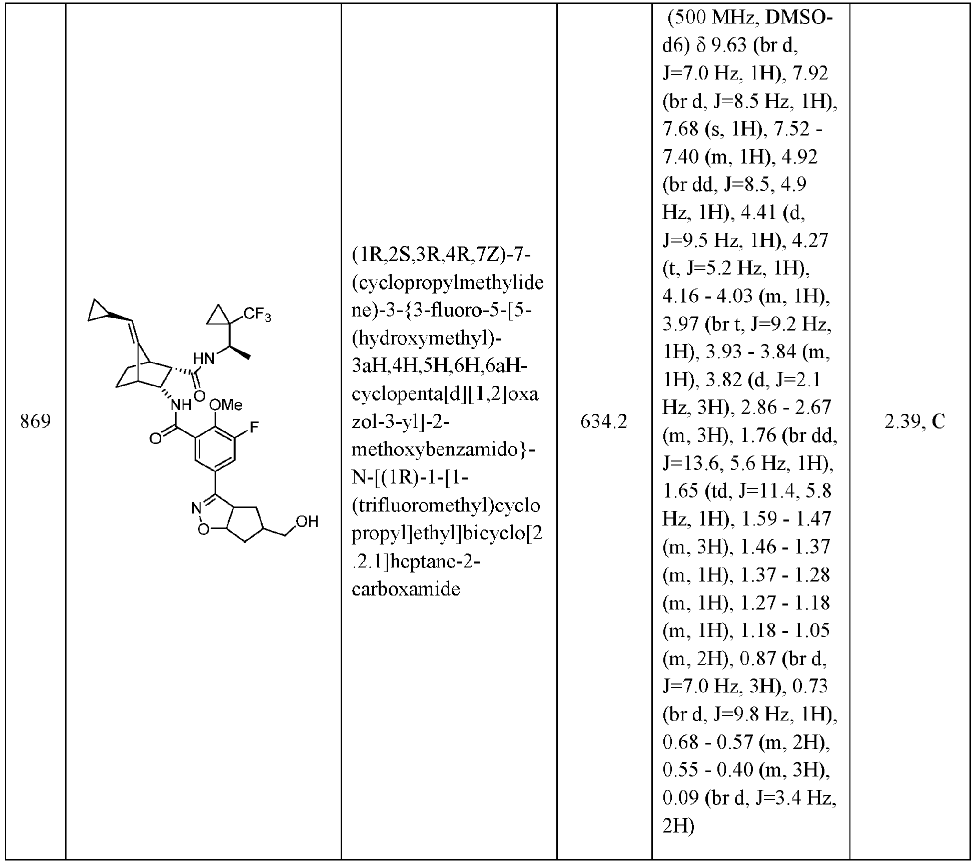

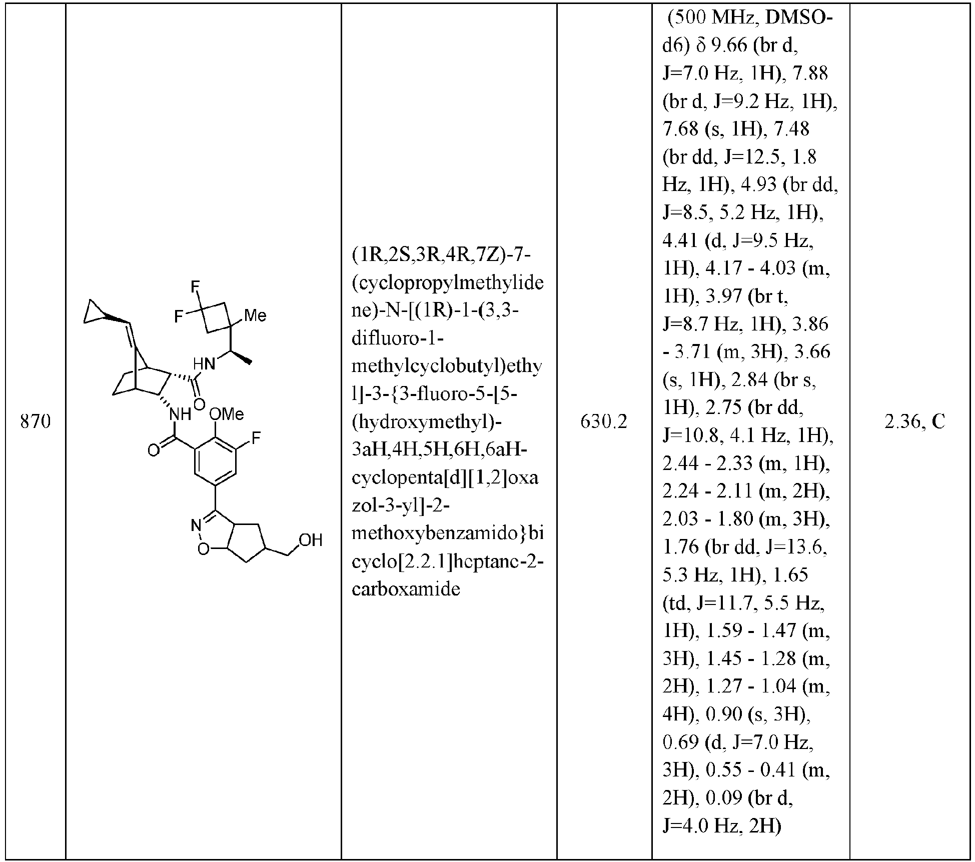

| **Table 5** | | | | | |
|---|---|---|---|---|---|
| **Ex. No.** | **Structure** | **Name** | **MS (ESI) (M+H)** | **¹H NMR** | **LC RT Meth. (min)** |
| 871 | 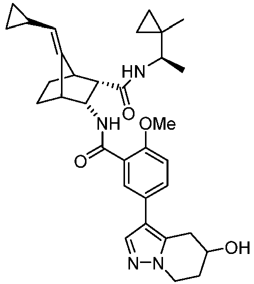 | (1R,2S,3R,4R,7Z)-7-(cyclopropylmethylide ne)-3-(5-{5-hydroxy-4H,5H,6H,7H-pyrazolo[1,5-a]pyridin-3-yl}-2-methoxybenzamido)-N-[(1S)-1-(1-methylcyclopropyl)eth yl]bicyclo[2.2.1]hepta ne-2-carboxamide | 559.3 | (400 MHz, DMSO-d6) δ = 10.03 (d, J = 6.6 Hz, 1H), 7.93 (d, J = 2.0 Hz, 1H), 7.90 (d, J = 8.6 Hz, 1H), 7.66 (s, 1H), 7.54 (dd, J = 2.4, 8.6 Hz, 1H), 7.16 (d, J = 8.6 Hz, 1H), 5.10 (dd, J = 3.4, 5.9 Hz, 1H), 4.64 (d, J = 9.6 Hz, 1H), 4.35 - 4.25 (m, 1H), 4.23 - 4.05 (m, 3H), 3.96 (s, 3H),3.52 - 3.44 (m, 1H), 3.13 - 3.01 (m, 2H), 2.95 (dd, J = 4.4, 10.9 Hz, 1H), 2.80 (dd, J = 5.6, 16.3 Hz, 1H), 2.14 - 1.97 (m, 2H), 1.88 (br d, J = 10.4 Hz, 1H), 1.81 - 1.72 (m, 1H), 1.53 - 1.43 (m, 1H), 1.35 (br s, 2H), 1.02 (d, J = 6.9 Hz, 3H), 0.98 (s, 3H), 0.79 - 0.63 (m, 2H), 0.53 (br dd, J = 3.8,8.7 Hz, 1H), 0.40 - 0.25 (m, 3H), 0.23 - 0.09 (m, 2H) | 2.05, A |
| 872 | 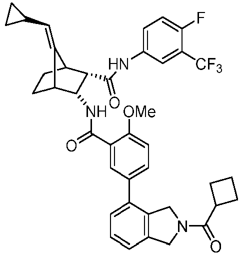 | (2S,3R,7Z)-3-[5-(2-cyclobutanecarbonyl-2,3-dihydro-1H-isoindol-4-yl)-2-methoxybenzamido]-7-(cyclopropylmethylide ne)-N-[4-fluoro-3-(trifluoromethyl)pheny l]bicyclo[2.2.1]heptan e-2-carboxamide | 702.5 | (500 MHz, DMSO-d6) δ 10.60 - 10.43 (m, 1H), 10.00 - 9.84 (m, 1H), 8.21 (br d, J=4.4 Hz, 1H), 8.00 (dd, J=13.8, 2.3 Hz, 1H), 7.77 (br dd, J=7.9, 3.7 Hz, 1H), 7.74 - 7.62 (m, 1H), 7.47 (br t, J=9.8 Hz, 1H), 7.43 - 7.38 (m, 1H), 7.36 - 7.30 (m, 1H), 7.30 - 7.23 (m, 2H), 4.77 (br s, 2H), 4.71 - 4.60 (m, 3H), 4.55 - 4.33 (m, 1H), 4.05 (s, 3H), 3.47 - 3.24 (m, 1H), 3.24 - 3.12 (m, 1H), 3.14 - 3.03 (m, 1H), 2.79 - 2.62 (m, 1H), 2.24 - 2.15 (m, 2H), 2.11 - 2.03 (m, 1H), 1.99 - 1.85 (m, 2H), 1.84 - 1.69 (m, 2H), 1.62 - 1.48 (m, 1H), 1.45 - 1.32 (m, 2H), 0.78 - 0.64 (m, 2H), 0.47 - 0.12 (m, 2H), One H is hidden under water/solvent peak | 2.83, C |
| 873 | 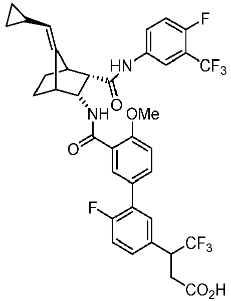 | 3-(3'-{[(1R,2R,3S,4R,7Z)-7-(cyclopropylmethylide ne)-3-{[4-fluoro-3-(trifluoromethyl)pheny l]carbamoyl}bicyclo[2 .2.1]heptan-2-yl]carbamoyl}-6-fluoro-4'-methoxy-[1,1'-biphenyl]-3-yl)-4,4,4-trifluorobutanoic acid | 737.1 | (500 MHz, DMSO-d6) δ 10.63 - 10.50 (m, 1H), 9.92 (d, J=7.0 Hz, 1H), 8.22 (dd, J=6.2, 2.2 Hz, 1H), 8.11 (s, 1H), 7.88 - 7.77 (m, 1H), 7.69 (br d, J=8.6 Hz, 1H), 7.56 (br s, 1H), 7.50 - 7.40 (m, 2H), 7.35 - 7.23 (m, 2H), 4.69 (d, J=9.5 Hz, 1H), 4.50 - 4.38 (m, 1H), 4.23 - 4.09 (m, 1H), 4.05 (s, 3H), 3.25 - 3.15 (m, 1H), 3.11 (br s, 1H), 3.01 - 2.89 (m, 2H), 2.76 - 2.66 (m, 1H), 1.88 - 1.73 (m, 2H), 1.57 - 1.48 (m, 1H), 1.45 - 1.34 (m, 2H), 0.86 - 0.64 (m, 2H), 0.41 - 0.25 (m, 2H), One H is hidden under water/solvent peak | 2.32, A |
| 875 | 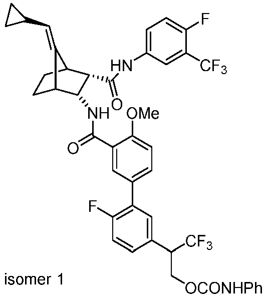 | 2-(3'-{[(1R,2R,3S,4R,7Z)-7-(cyclopropylmethylide ne)-3-{[4-fluoro-3-(trifluoromethyl)pheny l]carbamoyl}bicyclo[2 .2.1]heptan-2-yl]carbamoyl}-6-fluoro-4'-methoxy-[1,1'-biphenyl]-3-yl)-3,3,3-trifluoropropyl N-phenylcarbamate | 828.0 | (500 MHz, DMSO-d6) δ 10.57 - 10.49 (m, 1H), 9.92 (d, J=7.1 Hz, 1H), 9.69 (br s, 1H), 8.23 (dd, J=6.4, 2.4 Hz, 1H), 8.14 (s, 1H), 7.89 - 7.77 (m, 1H), 7.70 (br d, J=8.9 Hz, 1H), 7.65 (br d, J=7.0 Hz, 1H), 7.58 - 7.52 (m, 1H), 7.51 - 7.45 (m, 2H), 7.42 (br s, 2H), 7.39 - 7.33 (m, 1H), 7.30 (d, J=8.8 Hz, 1H), 7.25 (br t, J=7.8 Hz, 2H), 6.99 (t, J=7.3 Hz, 1H), 4.76 - 4.68 (m, 2H), 4.62 - 4.55 (m, 1H), 4.49 - 4.44 (m, 1H), 4.34 - 4.26 (m, 1H), 4.05 (s, 3H), 3.17 (br dd, J=10.3, 3.9 Hz, 1H), 3.12 - 3.08 (m, 1H), 1.91 - 1.84 (m, 1H), 1.83 - 1.77 (m, 1H), 1.54 - 1.49 (m, 1H), 1.46 - 1.38 (m, 2H), 0.78 - 0.70 (m, 2H), 0.41 - 0.33 (m, 2H) | 2.96, A |
| 876 | 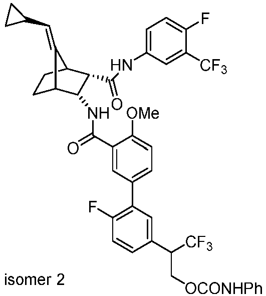 | 2-(3'-{[(1R,2R,3S,4R,7Z)-7-(cyclopropylmethylide ne)-3-{[4-fluoro-3-(trifluoromethyl)pheny l]carbamoyl}bicyclo[2 .2.1]heptan-2-yl]carbamoyl}-6-fluoro-4'-methoxy-[1,1'-biphenyl]-3-yl)-3,3,3-trifluoropropyl N-phenylcarbamate | 828.0 | (500 MHz, DMSO-d6) δ 10.29 (s, 1H), 9.67 (d, J=7.2 Hz, 1H), 9.45 (br s, 1H), 7.99 (dd, J=6.4, 2.4 Hz, 1H), 7.90 (s, 1H), 7.55 (br dd, J=8.4, 3.6 Hz, 1H), 7.45 (br d, J=8.9 Hz, 1H), 7.42 - 7.39 (m, 1H), 7.32 - 7.28 (m, 1H), 7.26 - 7.22 (m, 1H), 7.17 (br d, J=1.2 Hz, 1H), 7.14 - 7.11 (m, 1H), 7.06 (d, J=8.7 Hz, 1H), 7.01 (br t, J=7.7 Hz, 2H), 6.74 (t, J=7.4 Hz, 1H), 4.51 - 4.47 (m, 1H), 4.45 (d, J=9.2 Hz, 1H), 4.34 (dd, J=11.7, 7.2 Hz, 1H), 4.25 - 4.19 (m, 1H), 4.10 - 4.01 (m, 1H), 3.88 - 3.75 (m, 3H), 3.17 - 3.13 (m, 1H), 2.96 - 2.90 (m, 1H), 2.86 (br s, 1H), 2.48 (br d, J=3.9 Hz, 1H), 1.66 - 1.60 (m, 1H), 1.58 - 1.53 (m, 1H), 1.30 - 1.23 (m, 1H), 1.20 - 1.12 (m, 2H), 0.54 - 0.45 (m, 2H), 0.14 - 0.05 (m, 2H) | 2.96, A |
| 878 | 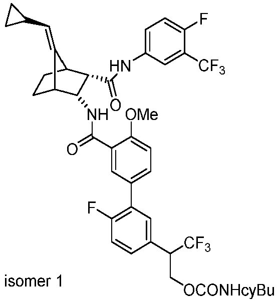 | 2-(3'-{[(1R,2R,3S,4R,7Z)-7-(cyclopropylmethylide ne)-3-{[4-fluoro-3-(trifluoromethyl)pheny l]carbamoyl}bicyclo[2 .2.1]heptan-2-yl]carbamoyl}-6-fluoro-4'-methoxy-[1,1'-biphenyl]-3-yl)-3,3,3-trifluoropropyl N-cyclobutylcarbamate | 806.2 | (500 MHz, DMSO-d6) δ 10.54 (s, 1H), 9.93 (br d, J=6.9 Hz, 1H), 8.29 - 8.21 (m, 1H), 8.14 (s, 1H), 7.80 (br dd, J=4.5, 3.4 Hz, 1H), 7.70 (br d, J=8.4 Hz, 1H), 7.63 - 7.59 (m, 1H), 7.55 - 7.50 (m, 1H), 7.49 - 7.45 (m, 2H), 7.38 - 7.35 (m, 1H), 7.32 (d, J=8.8 Hz, 1H), 4.70 (d, J=9.5 Hz, 1H), 4.60 - 4.52 (m, 1H), 4.50 - 4.46 (m, 1H), 4.43 - 4.39 (m, 1H), 4.22 - 4.16 (m, 1H), 4.06 (s, 3H), 3.94 - 3.88 (m, 1H), 3.17 (br dd, J=11.2, 4.2 Hz, 1H), 3.12 (br s, 1H), 2.73 (br s, 1H), 2.11 - 2.03 (m, 2H), 1.86 (br d, J=8.5 Hz, 2H), 1.83 - 1.78 (m, 1H), 1.57 - 1.49 (m, 2H), 1.46 - 1.38 (m, 2H), 0.80 - 0.71 (m, 2H), 0.39 - 0.31 (m, 2H) | 2.84, A |
| 879 | 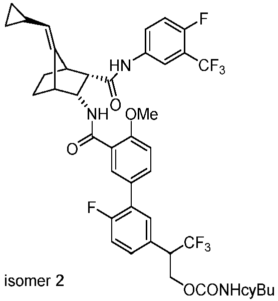 | 2-(3'-{[(1R,2R,3S,4R,7Z)-7-(cyclopropylmethylide ne)-3-{[4-fluoro-3-(trifluoromethyl)pheny l]carbamoyl}bicyclo[2 .2.1]heptan-2-yl]carbamoyl}-6-fluoro-4'-methoxy-[1,1'-biphenyl]-3-yl)-3,3,3-trifluoropropyl N-cyclobutylcarbamate | 806.0 | (500 MHz, DMSO-d6) δ 10.29 (s, 1H), 9.68 (d, J=7.2 Hz, 1H), 7.99 (dd, J=6.4, 2.3 Hz, 1H), 7.89 (s, 1H), 7.60 - 7.51 (m, 1H), 7.46 (br d, J=9.3 Hz, 1H), 7.35 (br d, J=7.1 Hz, 1H), 7.29 (br d, J=8.0 Hz, 1H), 7.23 (br d, J=9.5 Hz, 2H), 7.16 - 7.09 (m, 1H), 7.08 (d, J=8.7 Hz, 1H), 4.46 (d, J=9.5 Hz, 1H), 4.35 - 4.27 (m, 1H), 4.26 - 4.21 (m, 1H), 4.20 - 4.13 (m, 1H), 4.00 - 3.92 (m, 1H), 3.82 (s, 3H), 2.93 (br dd, J=10.6, 4.0 Hz, 1H), 2.87 (br s, 1H), 2.49 (br s, 1H), 1.83 (br dd, J=6.0, 2.2 Hz, 2H), 1.62 (br d, J=8.5 Hz, 2H), 1.60 - 1.52 (m, 2H), 1.35 - 1.25 (m, 3H), 1.20 - 1.13 (m, 2H), 0.50 (quin, J=9.3 Hz, 2H), 0.11 (br d, J=2.0 Hz, 2H), | 2.84, A |
| 880 | 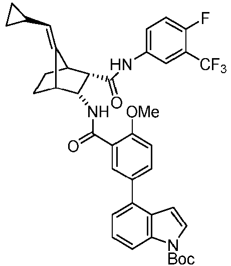 | tert-butyl 4-(3-{[(1R,2R,3S,4R,7Z)-7-(cyclopropylmethylide ne)-3-{[4-fluoro-3-(trifluoromethyl)pheny l]carbamoyl}bicyclo[2 .2.1]heptan-2-yl]carbamoyl}-4-methoxyphenyl)-1H-indole-1-carboxylate | 718.5 | (500 MHz, DMSO-d6) δ 10.53 (s, 1H), 9.93 (d, J=7.3 Hz, 1H), 8.22 (dd, J=6.4, 2.1 Hz, 1H), 8.17 (d, J=2.4 Hz, 1H), 8.09 (d, J=8.5 Hz, 1H), 7.81 - 7.77 (m, 1H), 7.74 - 7.70 (m, 2H), 7.47 (t, J=9.7 Hz, 1H), 7.40 (d, J=8.1 Hz, 1H), 7.34 (d, J=8.7 Hz, 1H), 7.28 (d, J=7.4 Hz, 1H), 6.68 (d, J=3.8 Hz, 1H), 4.69 (d, J=9.5 Hz, 1H), 4.53 - 4.44 (m, 1H), 4.06 (s, 3H), 3.16 (dd, J=10.7, 4.6 Hz, 1H), 3.13 - 3.09 (m, 1H), 2.76 - 2.69 (m, 1H), 1.93 - 1.86 (m, 1H), 1.85 - 1.79 (m, 1H), 1.64 (s, 9H), 1.58 - 1.48 (m, 1H), 1.42 (br s, 2H), 0.81 - 0.66 (m, 2H), 0.38 - 0.32 (m, 2H | 3.18, A |
| 881 | 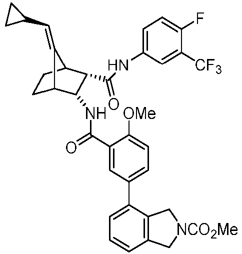 | methyl 4-(3-{[(2R,3S,7Z)-7-(cyclopropylmethylide ne)-3-{[4-fluoro-3-(trifluoromethyl)pheny l]carbamoyl}bicyclo[2 .2.1]heptan-2-yl]carbamoyl}-4-methoxyphenyl)-2,3-dihydro-1H-isoindole-2-carboxylate | 678.1 | (500 MHz, DMSO-d6) δ 10.53 (s, 1H), 9.93 (br d, J=6.3 Hz, 1H), 8.23 (dd, J=6.6, 2.4 Hz, 1H), 8.02 (dd, J=12.6, 2.1 Hz, 1H), 7.90 - 7.76 (m, 1H), 7.72 - 7.63 (m, 1H), 7.48 (t, J=9.7 Hz, 1H), 7.44 - 7.39 (m, 1H), 7.37 - 7.33 (m, 1H), 7.32 - 7.27 (m, 2H), 7.26 - 7.02 (m, 2H), 4.81 - 4.62 (m, 5H), 4.53 - 4.40 (m, 1H), 4.06 (s, 3H), 3.65 (d, J=14.3 Hz, 2H), 3.24 - 3.13 (m, 1H), 3.11 (br d, J=3.0 Hz, 1H), 1.96 - 1.86 (m, 1H), 1.82 (br t, J=8.2 Hz, 1H), 1.61 - 1.50 (m, 1H), 1.43 (br t, J=11.7 Hz, 2H), 0.74 (br dd, J=10.4, 9.2 Hz, 2H), 0.41 - 0.27 (m, 2H) | 2.75, C |
| 883 | 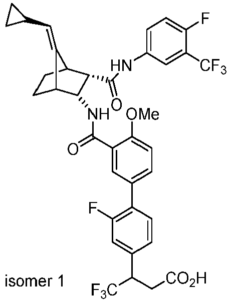 | 3-(3'-{[(1R,2R,3S,4R,7Z)-7-(cyclopropylmethylide ne)-3-{[4-fluoro-3-(trifluoromethyl)pheny l]carbamoyl}bicyclo[2 .2.1]heptan-2-yl]carbamoyl}-2-fluoro-4'-methoxy-[1,1'-biphenyl]-4-yl)-4,4,4-trifluorobutanoic acid | 737.1 | (500 MHz, DMSO-d6) δ 10.61 - 10.52 (m, 1H), 9.91 (d, J=7.1 Hz, 1H), 8.23 (dd, J=6.6, 2.4 Hz, 1H), 8.14 (s, 1H), 7.89 - 7.79 (m, 1H), 7.70 (br d, J=8.6 Hz, 1H), 7.54 - 7.45 (m, 2H), 7.39 (s, 1H), 7.33 - 7.24 (m, 2H), 4.69 (d, J=9.7 Hz, 1H), 4.55 - 4.42 (m, 1H), 4.21 - 4.10 (m, 1H), 4.05 (s, 3H), 3.90 (s, 1H), 3.24 - 3.14 (m, 2H), 3.11 (br s, 1H), 2.76 - 2.66 (m, 1H), 1.84 - 1.77 (m, 1H), 1.51 (qd, J=8.6, 4.9 Hz, 1H), 1.46 - 1.38 (m, 2H), 0.86 - 0.70 (m, 2H), 0.35 (br s, 2H), | 2.31, A |
| 884 | 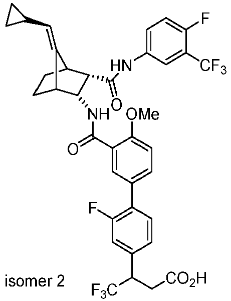 | 3-(3'-{[(1R,2R,3S,4R,7Z)-7-(cyclopropylmethylide ne)-3-{[4-fluoro-3-(trifluoromethyl)pheny l]carbamoyl}bicyclo[2 .2.1]heptan-2-yl]carbamoyl}-2-fluoro-4'-methoxy-[1,1'-biphenyl]-4-yl)-4,4,4-trifluorobutanoic acid | 737.1 | (500 MHz, DMSO-d6) δ 10.58 (s, 1H), 9.91 (d, J=7.2 Hz, 1H), 8.24 (dd, J=6.4, 2.2 Hz, 1H), 8.14 (s, 1H), 7.88 - 7.79 (m, 1H), 7.70 (br d, J=8.9 Hz, 1H), 7.55 - 7.45 (m, 2H), 7.38 (br d, J=11.5 Hz, 1H), 7.33 - 7.25 (m, 2H), 4.69 (d, J=9.6 Hz, 1H), 4.52 - 4.40 (m, 1H), 4.18 - 4.09 (m, 1H), 4.05 (s, 3H), 3.90 (s, 1H), 3.13 - 3.09 (m, 1H), 2.73 (br d, J=6.7 Hz, 1H), 1.89 - 1.79 (m, 2H), 1.56 - 1.48 (m, 1H), 1.46 - 1.38 (m, 2H), 0.83 - 0.68 (m, 2H), 0.35 (br d, J=2.4 Hz, 2H | 2.31, A |
| 886 | 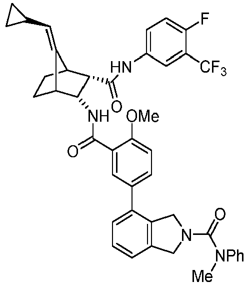 | 4-(3-{[(1R,2R,3S,4R,7Z)-7-(cyclopropylmethylide ne)-3-{[4-fluoro-3-(trifluoromethyl)pheny l]carbamoy1}bicyclo[2 .2.1]heptan-2-yl]carbamoyl}-4-methoxyphenyl)-N-methyl-N-phenyl-2,3-dihydro-1H-isoindole-2-carboxamide | 753.0 | (500 MHz, DMSO-d6) δ 10.55 (s, 1H), 9.90 (d, J=7.1 Hz, 1H), 8.24 (dd, J=6.2, 2.6 Hz, 1H), 7.94 (d, J=2.4 Hz, 1H), 7.80 (dt, J=7.9, 3.9 Hz, 1H), 7.54 - 7.46 (m, 2H), 7.38 - 7.33 (m, 2H), 7.32 - 7.29 (m, 1H), 7.26 - 7.23 (m, 2H), 7.21 (s, 1H), 7.18 - 7.13 (m, 2H), 4.71 (d, J=9.5 Hz, 1H), 4.50 (br d, J=5.7 Hz, 2H), 4.33 (s, 2H), 4.05 (s, 3H), 3.51 - 3.40 (m, 1H), 3.14 (s, 3H), 2.73 (br s, 1H), 1.93 - 1.88 (m, 1H), 1.88 - 1.82 (m, 1H), 1.56 - 1.50 (m, 1H), 1.44 - 1.37 (m, 2H), 1.26 - 1.20 (m, 2H), 0.76 - 0.65 (m, 2H), 0.44 - 0.32 (m, 2H), One H is hidden under water/solvent peak | 2.77, A |
| 887 | 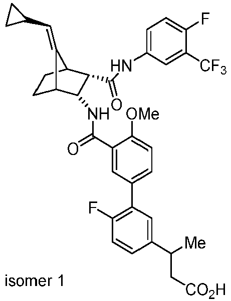 | 3-(3'-{[(1R,2R,3S,4R,7Z)-7-(cyclopropylmethylide ne)-3-{[4-fluoro-3-(trifluoromethyl)pheny l]carbamoyl}bicyclo[2 .2.1]heptan-2-yl]carbamoyl}-6-fluoro-4'-methoxy-[1,1'-biphenyl]-3-yl)butanoic acid | 683.4 | (500 MHz, DMSO-d6) δ 10.54 (s, 1H), 9.92 (d, J=7.2 Hz, 1H), 8.23 (dd, J=6.4, 2.3 Hz, 1H), 8.12 (s, 1H), 7.83 - 7.75 (m, 1H), 7.69 (br d, J=9.2 Hz, 1H), 7.48 (t, J=9.7 Hz, 1H), 7.39 - 7.34 (m, 1H), 7.30 (d, J=8.8 Hz, 1H), 7.28 - 7.26 (m, 1H), 7.24 - 7.18 (m, 1H), 4.70 (d, J=9.5 Hz, 1H), 4.51 - 4.44 (m, 1H), 4.05 (s, 3H), 3.21 (br d, J=7.0 Hz, 1H), 3.12 (br s, 1H), 2.73 (br d, J=4.4 Hz, 1H), 1.89 - 1.73 (m, 2H), 1.57 - 1.48 (m, 1H), 1.45 - 1.35 (m, 2H), 1.25 (d, J=6.9 Hz, 3H), 0.84 - 0.68 (m, 2H), 0.35 (br d, J=2.0 Hz, 2H), Four H's are hidden under water/solvent peak | 2.42, A |
| 888 | 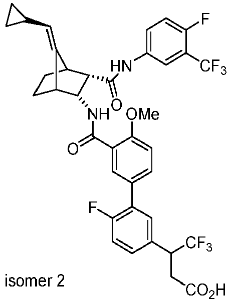 | 3-(3'-{[(1R,2R,3S,4R,7Z)-7-(cyclopropylmethylide ne)-3-{[4-fluoro-3-(trifluoromethyl)pheny l]carbamoyl}bicyclo[2 .2.1]heptan-2-yl]carbamoyl}-6-fluoro-4'-methoxy-[1,1'-biphenyl]-3-yl)butanoic acid | 683.1 | (500 MHz, DMSO-d6) δ 10.53 (s, 1H), 9.92 (d, J=7.2 Hz, 1H), 8.23 (dd, J=6.6, 2.4 Hz, 1H), 8.12 (s, 1H), 7.90 - 7.77 (m, 1H), 7.69 (br d, J=8.8 Hz, 1H), 7.48 (t, J=9.8 Hz, 1H), 7.36 (dd, J=7.7, 2.0 Hz, 1H), 7.32 - 7.28 (m, 1H), 7.27 (br dd, J=4.8, 2.2 Hz, 1H), 7.23 - 7.17 (m, 1H), 4.70 (d, J=9.6 Hz, 1H), 4.55 - 4.41 (m, 1H), 4.05 (s, 3H), 3.24 - 3.19 (m, 1H), 3.19 - 3.14 (m, 1H), 3.11 (br s, 1H), 2.73 (br s, 1H), 1.90 - 1.85 (m, 1H), 1.83 - 1.77 (m, 1H), 1.56 - 1.48 (m, 1H), 1.45 - 1.37 (m, 2H), 1.30 - 1.21 (m, 3H), 0.81 - 0.70 (m, 2H), 0.44 - 0.31 (m, 2H), Three H's are hidden under water/solvent peak | 2.40, A |
| 889 | 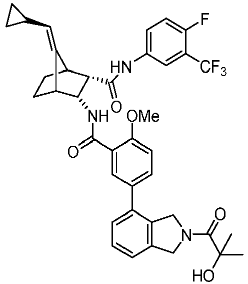 | (2S,3R,7Z)-7-(cyclopropylmethylide ne)-N-[4-fluoro-3-(trifluoromethyl)pheny l]-3-{5-[2-(2-hydroxy-2-methylpropanoyl)-2,3-dihydro-1H-isoindol-4-yl]-2-methoxybenzamido}bi cyclo[2.2.1]heptane-2-carboxamide | 706.1 | (500 MHz, DMSO-d6) δ 10.53 (s, 1H), 10.01 - 9.87 (m, 1H), 8.24 (dd, J=6.3, 2.3 Hz, 1H), 8.04 (dd, J=14.9, 2.3 Hz, 1H), 7.86 - 7.75 (m, 1H), 7.70 - 7.61 (m, 1H), 7.57 - 7.47 (m, 1H), 7.43 - 7.33 (m, 2H), 7.32 - 7.24 (m, 2H), 5.36 (s, 1H), 5.19 (br d, J=9.5 Hz, 2H), 4.79 - 4.66 (m, 3H), 4.53 - 4.35 (m, 1H), 4.07 (s, 3H), 3.17 (br dd, J=10.9, 3.7 Hz, 1H), 3.14 - 3.04 (m, 1H), 2.73 (s ,1H), 1.95 - 1.76 (m, 2H), 1.56 - 1.48 (m, 1H), 1.47 - 1.40 (m, 2H), 1.35 (s, 3H), 1.32 (s, 3H), 0.89 - 0.58 (m, 2H), 0.35 (br dd, J=3.9, 2.6 Hz, 2H) | 2.63, A |
| 890 | 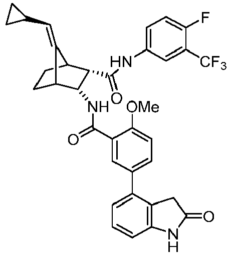 | (1R,2S,3R,4R,7Z)-7-(cyclopropylmethylide ne)-N-[4-fluoro-3-(trifluoromethyl)pheny 1]-3-[2-methoxy-5-(2-oxo-2,3-dihydro-1H-indol-4-yl)benzamido]bicyclo[ 2.2.1]heptane-2-carboxamide | 634.0 | (500 MHz, DMSO-d6) δ 10.54 (s, 1H), 10.00 - 9.80 (m, 1H), 8.24 (dd, J=6.4, 2.4 Hz, 1H), 8.09 (d, J=2.4 Hz, 1H), 7.87 - 7.78 (m, 1H), 7.76 - 7.70 (m, 1H), 7.49 (t, J=9.6 Hz, 1H), 7.36 - 7.20 (m, 2H), 7.00 (d, J=7.6 Hz, 1H), 6.82 (d, J=7.7 Hz, 1H), 4.70 (d, J=9.5 Hz, 1H), 4.55 - 4.43 (m, 1H), 4.05 (s, 3H), 3.56 (s, 1H), 3.17 (br dd, J=10.9, 3.9 Hz, 1H), 3.11 (br s, 1H), 2.73 (br d, J=3.7 Hz, 1H), 1.97 - 1.86 (m, 1H), 1.84 - 1.74 (m, 1H), 1.58 - 1.48 (m, 1H), 1.45 - 1.34 (m, 2H), 0.74 (quin, J=10.0 Hz, 2H), 0.45 - 0.29 (m, 2H), Two H's are hidden under water/solvent peak | 2.50, A |
| 891 | 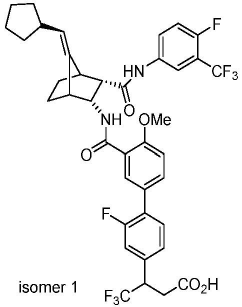 | 3-(3'-{[(1R,2R,3S,4R,7Z)-7-(cyclopentylmethylide ne)-3-{[4-fluoro-3-(trifluoromethyl)pheny l]carbamoyl}bicyclo[2 .2.1]heptan-2-yl]carbamoyl}-2-fluoro-4'-methoxy-[1,1'-biphenyl]-4-yl)-4,4,4-trifluorobutanoic acid | 765.1 | (500 MHz, DMSO-d6) δ 10.52 (s, 1H), 9.88 (d, J=7.2 Hz, 1H), 8.24 (dd, J=6.4, 2.3 Hz, 1H), 8.15 (s, 1H), 7.88 - 7.76 (m, 1H), 7.72 (br d, J=8.5 Hz, 1H), 7.64 - 7.52 (m, 1H), 7.50 - 7.43 (m, 2H), 7.37 (br d, J=8.3 Hz, 1H), 7.30 (s, 1H), 5.19 (d, J=8.8 Hz, 1H), 4.51 - 4.35 (m, 1H), 4.29 - 4.10 (m, 1H), 4.06 (s, 3H), 3.10 - 2.96 (m, 3H), 2.66 - 2.57 (m, 2H), 1.93 - 1.86 (m, 2H), 1.85 - 1.82 (m, 2H), 1.81 - 1.73 (m, 3H), 1.65 (br dd, J=7.0, 3.6 Hz, 2H), 1.58 (br d, J=5.3 Hz, 2H), 1.43 - 1.38 (m, 2H), 1.29 - 1.25 (m, 1H) | 2.25, A |
| 892 | 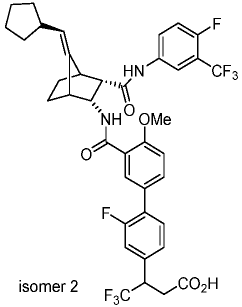 | 3-(3'-{[(1R,2R,3S,4R,7Z)-7-(cyclopentylmethylide ne)-3-{[4-fluoro-3-(trifluoromethyl)pheny l]carbamoyl}bicyclo[2 .2.1]heptan-2-yl]carbamoyl}-2-fluoro-4'-methoxy-[1,1'-biphenyl]-4-yl)-4,4,4-trifluorobutanoic acid | 765.5 | (500 MHz, DMSO-d6) δ 10.62 (s, 1H), 9.88 (d, J=7.2 Hz, 1H), 8.25 (dd, J=6.5, 2.3 Hz, 1H), 8.14 (s, 1H), 7.89 - 7.79 (m, 1H), 7.71 (br d, J=8.2 Hz, 1H), 7.60 - 7.48 (m, 1H), 7.49 - 7.45 (m, 1H), 7.42 (br d, J=11.7 Hz, 1H), 7.35 (br d, J=7.6 Hz, 1H), 7.30 (d, J=8.8 Hz, 1H), 5.19 (d, J=8.9 Hz, 1H), 4.45 - 4.39 (m, 1H), 4.24 - 4.06 (m, 3H? water supp?), 2.85 (q, J=7.3 Hz, 2H), 2.77 - 2.68 (m, 1H), 2.66 - 2.59 (m, 1H), 1.96 - 1.85 (m, 1H), 1.85 - 1.74 (m, 3H), 1.72 - 1.64 (m, 2H), 1.61 - 1.52 (m, 2H), 1.48 - 1.36 (m, 2H), 1.36 - 1.21 (m, 3H), 1.12 (t, J=7.2 Hz, 3H) | 2.53, A |
| 893 | 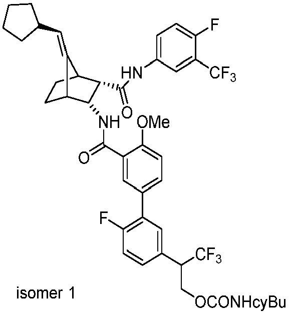 | 2-(3'-{[(1R,2R,3S,4R,7Z)-7-(cyclopentylmethylide ne)-3-{[4-fluoro-3-(trifluoromethyl)pheny l]carbamoyl}bicyclo[2 .2.1]heptan-2-yl]carbamoyl}-6-fluoro-4'-methoxy-[1,1'-biphenyl]-3-yl)-3,3,3-trifluoropropyl N-cyclobutylcarbamate | 834.1 | (500 MHz, DMSO-d6) δ 10.58 - 10.49 (m, 1H), 9.90 (d, J=7.0 Hz, 1H), 8.25 (dd, J=6.3, 2.1 Hz, 1H), 8.14 (s, 1H), 7.87 - 7.78 (m, 1H), 7.70 (br d, J=8.2 Hz, 1H), 7.62 - 7.58 (m, 1H), 7.53 (br d, J=7.9 Hz, 1H), 7.48 (br d, J=9.5 Hz, 2H), 7.36 (br d, J=10.2 Hz, 1H), 7.34 - 7.29 (m, 1H), 5.20 (d, J=8.9 Hz, 1H), 4.59 - 4.51 (m, 1H), 4.42 (br dd, J=11.0, 7.3 Hz, 2H), 4.23 - 4.16 (m, 1H), 4.06 (s, 3H), 3.97 - 3.91 (m, 1H), 3.17 (dd, J=10.4, 3.8 Hz, 1H), 3.01 (br s, 1H), 2.72 (br s, 1H), 2.64 - 2.57 (m, 1H), 2.13 - 2.04 (m, 2H), 1.91 - 1.82 (m, 3H), 1.81 - 1.73 (m, 2H), 1.70 - 1.62 (m, 2H), 1.60 - 1.47 (m, 3H), 1.43 - 1.37 (m, 2H), 1.32 - 1.20 (m, 2H), One H is hidden under water/solvent peak | 3.20, A |
| 894 | 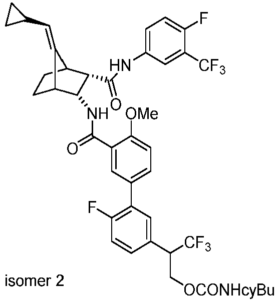 | 2-(3'-{[(1R,2R,3S,4R,7Z)-7-(cyclopentylmethylide ne)-3-{[4-fluoro-3-(trifluoromethyl)pheny l]carbamoyl}bicyclo[2 .2.1]heptan-2-yl]carbamoyl}-6-fluoro-4'-methoxy-[1,1'-biphenyl]-3-yl)-3,3,3-trifluoropropyl N-cyclobutylcarbamate | 834.1 | (500 MHz, DMSO-d6) δ 10.65 - 10.48 (m, 1H), 9.90 (d, J=7.2 Hz, 1H), 8.25 (dd, J=6.4, 2.5 Hz, 1H), 8.14 (s, 1H), 7.84 - 7.75 (m, 1H), 7.70 (br d, J=9.0 Hz, 1H), 7.60 (br s, 1H), 7.54 (br d, J=7.9 Hz, 1H), 7.50 - 7.46 (m, 2H), 7.36 (br d, J=10.3 Hz, 1H), 7.32 (d, J=8.9 Hz, 1H), 5.20 (d, J=8.9 Hz, 1H), 4.60 - 4.49 (m, 1H), 4.47 - 4.38 (m, 2H), 4.26 - 4.15 (m, 1H), 4.06 (s, 3H), 3.97 - 3.89 (m, 1H), 3.17 (dd, J=10.5, 4.3 Hz, 1H), 3.01 (br s, 1H), 2.72 (br s, 1H), 2.63 - 2.57 (m, 1H), 2.15 -2.05 (m, 2H), 1.94 - 1.82 (m, 4H), 1.81 - 1.72 (m, 2H), 1.71 - 1.63 (m, 2H), 1.60 - 1.48 (m, 4H), 1.46 - 1.37 (m, 2H), 1.33 - 1.21 (m, 2H) | 3.21, A |
| 895 | 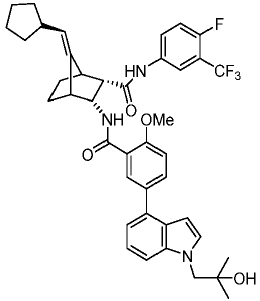 | (2S,3R,7Z)-7-(cyclopentylmethylide ne)-N-[4-fluoro-3-(trifluoromethyl)pheny l]-3-{5-[1-(2-hydroxy-2-methylpropyl)-1H-indol-4-yl]-2-methoxybenzamido}bi cyclo[2.2.1]heptane-2-carboxamide | 718.0 | (500 MHz, DMSO-d6) δ 10.63 - 10.45 (m, 1H), 9.91 (d, J=7.0 Hz, 1H), 8.30 (d, J=2.4 Hz, 1H), 8.25 (dd, J=6.6, 2.4 Hz, 1H), 7.84 - 7.76 (m, 2H), 7.54 (d, J=8.2 Hz, 1H), 7.49 (t, J=9.7 Hz, 1H), 7.41 (d, J=3.1 Hz, 1H), 7.32 (d, J=8.8 Hz, 1H), 7.19 (t, J=7.8 Hz, 1H), 7.05 (d, J=7.1 Hz, 1H), 6.50 (d, J=3.1 Hz, 1H), 5.20 (d, J=8.9 Hz, 1H), 4.50 - 4.39 (m, 1H), 4.11 (s, 2H), 4.07 (s, 3H), 3.17 (dd, J=10.8, 4.1 Hz, 1H), 3.02 (br s, 1H), 2.72 (br s, 1H), 2.66 - 2.58 (m, 1H), 1.92 (br t, J=8.7 Hz, 1H), 1.87 - 1.81 (m, 2H), 1.79 - 1.75 (m, 1H), 1.70 - 1.63 (m, 2H), 1.61 - 1.54 (m, 2H), 1.45 - 1.38 (m, 2H), 1.34 - 1.22 (m, 2H), 1.12 (s, 6H), One H is hidden under water/solvent peak | 3.07, C |
| 896 | 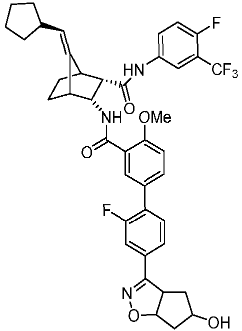 | N-[(2R3S,7Z)-7-(cyclopentylmethylide ne)-3-{[4-fluoro-3-(trifluoromethyl)pheny l]carbamoyl}bicyclo[2 .2.1]heptan-2-yl]-2'-fluoro-4'-{5-hydroxy-3aH,4H,5H,6H,6aH-cyclopenta[d][1,2]oxa zol-3-yl}-4-methoxy-[1,1'-biphenyl]-3-carboxamide | 857.1 | (500 MHz, DMSO-d6) δ 10.52 (s, 1H), 9.96 - 9.84 (m, 1H), 8.24 (br d, J=5.8 Hz, 1H), 8.18 (br s, 1H), 7.87 - 7.77 (m, 1H), 7.74 (br d, J=8.9 Hz, 1H), 7.66 - 7.53 (m, 3H), 7.50 - 7.42 (m, 1H), 7.32 (d, J=8.9 Hz, 1H), 5.33 - 5.14 (m, 2H), 4.49 - 4.39 (m, 1H), 4.26 (td, J=9.4, 3.7 Hz, 1H), 4.20 - 4.10 (m, 2H), 4.06 (s, 3H), 3.44 - 3.32 (m, 1H), 3.16 (br dd, J=10.9, 3.7 Hz, 1H), 3.00 (br d, J=4.5 Hz, 1H), 2.72 (br s, 1H), 2.64 - 2.57 (m, 1H), 2.18 - 2.07 (m, 1H), 2.04 - 1.94 (m, 1H), 1.89 - 1.71 (m, 5H), 1.70 - 1.61 (m, 2H), 1.58 - 1.52 (m, 2H), 1.44 - 1.37 (m, 2H), 1.31 - 1.20 (m, 2H) | 3.26, A |
| 897 | 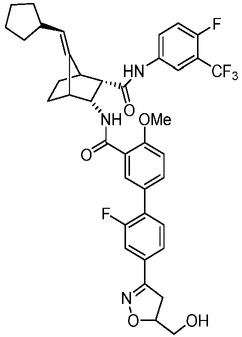 | N-[(2R,3S,7Z)-7-(cyclopentylmethylide ne)-3-{[4-fluoro-3-(trifluoromethyl)pheny l]carbamoyl}bicyclo[2 .2.1]heptan-2-yl]-2'-fluoro-4'-[5-(hydroxymethyl)-4,5-dihydro-1,2-oxazol-3-yl]-4-methoxy-[1,1'-biphenyl]-3-carboxamide | 724.6 | (500 MHz, DMSO-d6) δ 10.53 (s, 1H), 9.89 (d, J=7.0 Hz, 1H), 8.25 (dd, J=6.6, 2.4 Hz, 1H), 8.18 - 8.12 (m, 1H), 7.80 (br dd, J=9.0, 3.0 Hz, 1H), 7.74 (br d, J=8.7 Hz, 1H), 7.64 - 7.54 (m, 3H), 7.49 (t, J=9.7 Hz, 1H), 7.32 (d, J=8.9 Hz, 1H), 5.19 (d, J=8.6 Hz, 1H), 5.02 (t, J=5.8 Hz, 1H), 4.77 (qd, J=7.7, 4.3 Hz, 1H), 4.50 - 4.32 (m, 1H), 4.06 (s, 3H), 3.61 - 3.52 (m, 2H), 3.50 - 3.37 (m, 1H), 3.29 - 3.13 (m, 1H), 3.06 - 2.94 (m, 1H), 2.72 (br s, 1H), 2.61 (s, 2H), 1.89 - 1.74 (m, 4H), 1.70 - 1.62 (m, 2H), 1.59 - 1.48 (m, 2H), 1.45 - 1.35 (m, 2H), 1.32 - 1.17 (m, 2H) | 2.81, A |
| 898 | 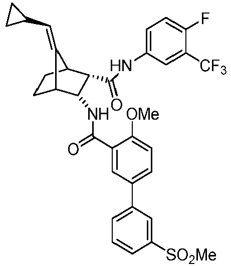 | N-[(1R,2R,3S,4R,7Z)-7-(cyclopropylmethylide ne)-3-{[4-fluoro-3-(trifluoromethyl)pheny l]carbamoyl}bicyclo[2 .2.1]heptan-2-yl]-3'-methanesulfonyl-4-methoxy-[1,1'-biphenyl]-3-carboxamide | 657.2 | (500 MHz, DMSO-d6) δ 10.65 - 10.47 (m, 1H), 10.04 - 9.89 (m, 1H), 8.32 - 8.28 (m, 1H), 8.25 - 8.21 (m, 1H), 8.14 - 8.10 (m, 1H), 8.04 - 7.99 (m, 1H), 7.96 - 7.87 (m, 1H), 7.77 - 7.71 (m, 1H), 7.52 - 7.46 (m, 1H), 7.38 - 7.31 (m, 1H), 4.75 - 4.67 (m, 1H), 4.53 - 4.44 (m, 1H), 4.09 - 4.01 (m, 1H), 3.33 - 3.26 (m, 1H), 3.20 - 3.09 (m, 1H), 2.57 - 2.53 (m, 32H), 1.92 - 1.75 (m, 1H), 1.56 - 1.36 (m, 1H), 0.81 - 0.68 (m, 1H), 0.40 - 0.30 (m, 1H) | 2.56, C |
| 899 | 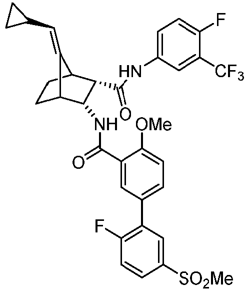 | N-[(1R,2R,3S,4R,7Z)-7-(cyclopropylmethylide ne)-3-{[4-fluoro-3-(trifluoromethyl)pheny l]carbamoyl}bicyclo[2 .2.1]heptan-2-yl]-2'-fluoro-5'-methanesulfonyl-4-methoxy-[1,1'-biphenyl]-3-carboxamide | 675.1 | (500 MHz, DMSO-d6) δ 10.55 (s, 1H), 9.93 (br d, J=7.3 Hz, 1H), 8.26 - 8.12 (m, 2H), 8.02 (br d, J=5.1 Hz, 1H), 7.99 - 7.89 (m, 1H), 7.79 (br d, J=6.3 Hz, 2H), 7.60 (br t, J=9.5 Hz, 1H), 7.47 (br t, J=9.6 Hz, 1H), 7.35 (br d, J=8.6 Hz, 1H), 4.69 (br d, J=9.6 Hz, 1H), 4.45 (br s, 1H), 4.06 (s, 3H), 3.15 (br d, J=7.6 Hz, 1H), 3.10 (br s, 1H), 2.72 (br s, 1H), 1.89 - 1.68 (m, 2H), 1.49 (br s, 1H), 1.41 (br s, 2H), 0.84 - 0.66 (m, 2H), 0.34 (br s, 2H) | 2.61, C |
| 901 | 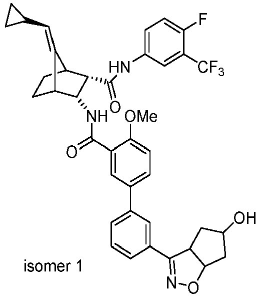 | N-[(1R,2R,3S,4R,7Z)-7-(cyclopropylmethylide ne)-3-{[4-fluoro-3-(trifluoromethyl)pheny l]carbamoyl}bicyclo[2 .2.1]heptan-2-yl]-3'-{5-hydroxy-3aH,4H,5H,6H,6aH-cyclopenta[d][1,2]oxa zol-3-yl}-4-methoxy-[1,1'-biphenyl]-3-carboxamide | 704.2 | (500 MHz, DMSO-d6) δ 10.32 (s, 1H), 9.68 (d, J=7.2 Hz, 1H), 8.06 - 7.92 (m, 1H), 7.65 - 7.52 (m, 2H), 7.54 (m, 1H), 7.45 (br d, J=7.6 Hz, 1H), 7.38 (d, J=7.6 Hz, 1H), 7.32 - 7.20 (m, 1H), 7.06 (d, J=8.8 Hz, 1H), 4.94 (td, J=6.2, 3.2 Hz, 1H), 4.45 (d, J=9.7 Hz, 1H), 4.23 (br s, 1H), 4.07 (td, J=9.5, 4.2 Hz, 1H), 3.95 - 3.84 (m, 1H), 3.81 (s, 3H), 2.93 (br dd, J=10.5, 4.3 Hz, 1H), 2.87 (br s, 1H), 2.48 (br s, 1H), 1.78 - 1.55 (m, 4H), 1.51 (s, 1H), 1.26 (br dd, J=8.8, 4.2 Hz, 1H), 1.21 - 0.98 (m, 2H), 0.49 (br dd, J=12.6, 8.9 Hz, 2H), 0.10 (br s, 2H) | 2.67, A |
| 902 | 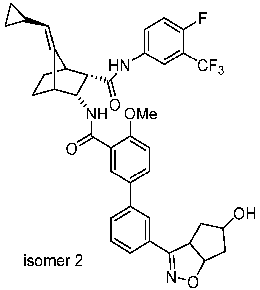 | N-[(1R,2R,3S,4R,7Z)-7-(cyclopropylmethylide ne)-3-{[4-fluoro-3-(trifluoromethyl)pheny l]carbamoyl}bicyclo[2 .2.1]heptan-2-yl]-3'-{5-hydroxy-3aH,4H,5H,6H,6aH-cyclopenta[d][1,2]oxa zol-3-yl}-4-methoxy-[1,1'-biphenyl]-3-carboxamide | 704.3 | (500 MHz, DMSO-d6) δ 9.94 (d, J=7.0 Hz, 1H), 8.25 (br d, J=2.4 Hz, 2H), 7.87 - 7.79 (m, 3H), 7.68 (br d, J=8.0 Hz, 1H), 7.64 (d, J=7.9 Hz, 1H), 7.56 - 7.52 (m, 1H), 7.49 (t, J=9.9 Hz, 1H), 7.32 (d, J=8.7 Hz, 1H), 5.18 - 5.13 (m, 1H), 4.70 (d, J=9.6 Hz, 1H), 4.48 (br s, 1H), 4.24 - 4.15 (m, 2H), 4.06 (s, 3H), 3.91 (s, 1H), 3.20 - 3.17 (m, 1H), 3.13 (br s, 1H), 2.74 (br s, 1H), 2.17 - 2.09 (m, 2H), 1.95 - 1.79 (m, 4H), 1.51 (br dd, J=8.9, 4.5 Hz, 1H), 1.47 - 1.38 (m, 2H), 0.75 (br dd, J=12.7, 8.9 Hz, 2H), 0.36 (br s, 2H) | 2.57, A |
| 905 | 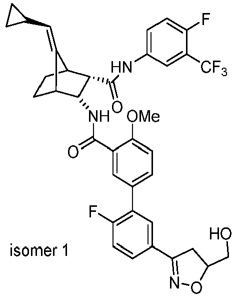 | N-[(1R,2R,3S,4R,7Z)-7-(cyclopropylmethylide ne)-3-{[4-fluoro-3-(trifluoromethyl)pheny l]carbamoyl}bicyclo[2 .2.1]heptan-2-yl]-2'-fluoro-5'-[5-(hydroxymethyl)-4,5-dihydro-1,2-oxazol-3-yl]-4-methoxy-[1,1'-biphenyl]-3-carboxamide | 696.2 | (500 MHz, DMSO-d6) δ 10.54 (s, 1H), 9.93 (d, J=7.2 Hz, 1H), 8.24 (dd, J=6.4, 2.5 Hz, 1H), 8.16 (s, 1H), 7.82 - 7.68 (m, 4H), 7.49 (t, J=9.8 Hz, 1H), 7.43 - 7.38 (m, 1H), 7.33 (d, J=8.7 Hz, 1H), 4.76 - 4.68 (m, 2H), 4.47 (br t, J=10.6 Hz, 1H), 4.07 (s, 3H), 3.55 - 3.49 (m, 1H), 3.17 (dd, J=10.4, 4.3 Hz, 1H), 3.12 (br s, 1H), 2.73 (br s, 1H), 1.90 - 1.78 (m, 2H), 1.54 - 1.37 (m, 3H), 0.74 (quin, J=10.1 Hz, 2H), 0.38 - 0.33 (m, 2H) | 2.61, A |
| 906 | 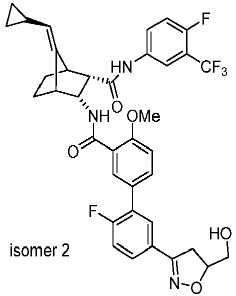 | N-[(1R,2R,3S,4R,7Z)-7-(cyclopropylmethylide ne)-3-{[4-fluoro-3-(trifluoromethyl)pheny l]carbamoyl}bicyclo[2 .2.1]heptan-2-yl]-2'-fluoro-5'-[5-(hydroxymethyl)-4,5-dihydro-1,2-oxazol-3-yl]-4-methoxy-[1,1'-biphenyl]-3-carboxamide | 696.1 | (500 MHz, DMSO-d6) δ 10.29 (s, 1H), 9.69 (d, J=7.4 Hz, 1H), 8.00 (d, J=6.7 Hz, 1H), 7.92 (s, 1H), 7.58 - 7.45 (m, 3H), 7.25 (t, J=9.7 Hz, 1H), 7.17 (dd, J=10.5, 8.7 Hz, 1H), 7.09 (d, J=8.8 Hz, 1H), 4.77 (t, J=5.7 Hz, 1H), 4.52 - 4.44 (m, 1H), 4.23 (br s, 1H), 3.83 (s, 3H), 3.29 (q, J=5.0 Hz, 1H), 2.95 - 2.87 (m, 1H), 2.49 (br s, 1H), 1.66 - 1.55 (m, 1H), 1.30 - 1.21 (m, 1H), 1.21 - 1.14 (m, 1H), 0.55 - 0.46 (m, 1H), 0.12 (br s, 1H) | 2.57, C |
| 907 | 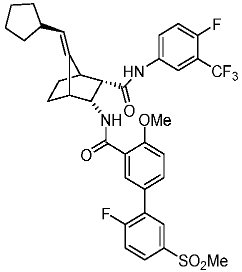 | N-[(1R,2R,3S,4R,7Z)-7-(cyclopentylmethylide ne)-3-{[4-fluoro-3-(trifluoromethyl)pheny l]carbamoyl}bicyclo[2 .2.1]heptan-2-yl]-2'-fluoro-5'-methanesulfonyl-4-methoxy-[1,1'-biphenyl]-3-carboxamide | 703.2 | (500 MHz, DMSO-d6) δ 10.53 (s, 1H), 9.91 (d, J=7.2 Hz, 1H), 8.24 (dd, J=6.4, 2.3 Hz, 1H), 8.19 (s, 1H), 8.04 (dd, J=7.2, 2.3 Hz, 1H), 7.97 (d, J=8.2 Hz, 1H), 7.82 - 7.77 (m, 2H), 7.61 (t, J=9.0 Hz, 1H), 7.49 (t, J=9.8 Hz, 1H), 7.36 (d, J=8.8 Hz, 1H), 5.20 (d, J=8.9 Hz, 1H), 4.42 (br t, J=10.5 Hz, 1H), 4.07 (s, 3H), 3.30 (s, 1H), 3.17 (dd, J=10.7, 4.0 Hz, 1H), 3.02 (br s, 1H), 2.72 (br s, 1H), 2.63 - 2.57 (m, 1H), 2.55 (s, 3H), 1.90 - 1.74 (m, 5H), 1.68 - 1.54 (m, 2H), 1.40 (br s, 2H), 1.31 - 1.23 (m, 2H) | 2.83, C |
| 908 | 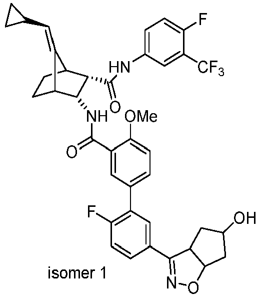 | N-[(1R,2R,3S,4R,7Z)-7-(cyclopropylmethylide ne)-3-{[4-fluoro-3-(trifluoromethyl)pheny l]carbamoyl}bicyclo[2 .2.1]heptan-2-yl]-2'-fluoro-5'-{5-hydroxy-3aH,4H,5H,6H,6aH-cyclopenta[d][1,2]oxa zol-3-yl}-4-methoxy-[1,1'-biphenyl]-3-carboxamide | 722.5 | (500 MHz, DMSO-d6) δ 9.69 (d, J=7.2 Hz, 1H), 7.99 (d, J=6.7 Hz, 1H), 7.91 (s, 1H), 7.56 (d, J=8.0 Hz, 1H), 7.51 - 7.43 (m, 2H), 7.24 (t, J=9.7 Hz, 1H), 7.16 (t, J=9.5 Hz, 1H), 7.09 (d, J=8.7 Hz, 1H), 4.96 - 4.91 (m, 1H), 4.45 (d, J=9.5 Hz, 1H), 4.25 - 4.20 (m, 1H), 4.06 (td, J=9.4, 4.0 Hz, 1H), 3.89 - 3.85 (m, 1H), 3.82 (s, 3H), 2.93 (br dd, J=10.6, 4.1 Hz, 1H), 2.87 (br s, 1H), 2.49 (br s, 1H), 1.78 - 1.55 (m, 5H), 1.29 - 1.15 (m, 2H), 0.54 - 0.45 (m, 2H), 0.13 - 0.09 (m, 2H) | 2.61, C |
| 909 | 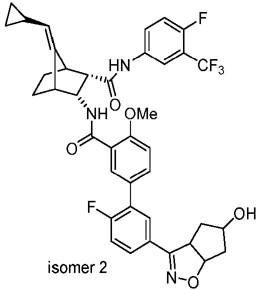 | N-[(1R,2R,3S,4R,7Z)-7-(cyclopropylmethylide ne)-3-{[4-fluoro-3-(trifluoromethyl)pheny l]carbamoyl}bicyclo[2 .2.1]heptan-2-yl]-2'-fluoro-5'-{5-hydroxy-3aH,4H,5H,6H,6aH-cyclopenta[d][1,2]oxa zol-3-yl}-4-methoxy-[1,1'-biphenyl]-3-carboxamide | 722.6 | (500 MHz, DMSO-d6) δ 10.53 (s, 1H), 9.94 (d, J=7.2 Hz, 1H), 8.24 (dd, J=6.5, 2.4 Hz, 1H), 8.15 (s, 1H), 7.82 - 7.78 (m, 1H), 7.75 - 7.67 (m, 3H), 7.49 (t, J=9.7 Hz, 1H), 7.41 (t, J=9.0 Hz, 1H), 7.34 (d, J=8.7 Hz, 1H), 5.17 - 5.13 (m, 1H), 4.70 (d, J=9.7 Hz, 1H), 4.47 (br s, 1H), 4.22 - 4.15 (m, 2H), 4.07 (s, 3H), 3.17 (dd, J=10.5, 4.4 Hz, 1H), 3.12 (br s, 1H), 2.73 (br s, 1H), 2.14 - 2.07 (m, 2H), 1.94 - 1.78 (m, 4H), 1.51 (br dd, J=8.7, 4.2 Hz, 1H), 1.46 - 1.39 (m, 2H), 0.79 - 0.70 (m, 2H), 0.35 (br d, J=2.5 Hz, 2H) | 2.59, A |
| 910 | 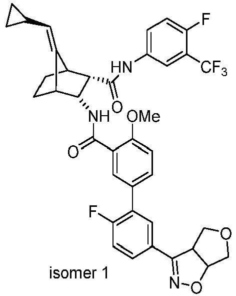 | 5'-{3aH,4H,6H,6aH-furo[3,4-d][1,2]oxazol-3-yl}-N-[(1R,2R,3S,4R,7Z)-7-(cyclopropylmethylide ne)-3-{[4-fluoro-3-(trifluoromethyl)pheny l]carbamoyl}bicyclo[2 .2.1]heptan-2-yl]-2'-fluoro-4-methoxy-[1,1'-biphenyl]-3-carboxamide | 708.1 | (500 MHz, DMSO-d6) δ 10.53 (s, 1H), 9.92 (d, J=7.3 Hz, 1H), 8.24 (dd, J=6.3, 2.4 Hz, 1H), 8.15 (s, 1H), 7.81 - 7.70 (m, 4H), 7.49 (t, J=9.8 Hz, 1H), 7.42 (t, J=8.8 Hz, 1H), 7.33 (d, J=8.8 Hz, 1H), 5.39 (dd, J=9.2, 3.7 Hz, 1H), 4.70 (d, J=9.5 Hz, 1H), 4.58 (br t, J=7.7 Hz, 1H), 4.50 - 4.45 (m, 1H), 4.13 - 4.11 (m, 1H), 4.07 (s, 3H), 3.98 (br d, J=9.5 Hz, 1H), 3.77 (dd, J=9.4, 7.0 Hz, 1H), 3.67 (dd, J=10.7, 3.5 Hz, 1H), 3.19 - 3.10 (m, 2H), 2.73 (br s, 1H), 1.90 - 1.79 (m, 2H), 1.54 - 1.45 (m, 1H), 1.45 - 1.38 (m, 2H), 0.74 (quin, J=10.1 Hz, 2H), 0.38 - 0.33 (m, 2H) | 2.75, C |
| 911 | 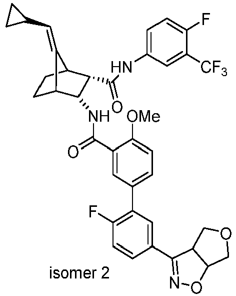 | 5'-{3aH,4H,6H,6aH-furo[3,4-d][1,2]oxazol-3-yl}-N-[(1R,2R,3S,4R,7Z)-7-(cyclopropylmethylide ne)-3-{[4-fluoro-3-(trifluoromethyl)pheny l]carbamoyl}bicyclo[2 .2.1]heptan-2-yl]-2'-fluoro-4-methoxy-[1,1'-biphenyl]-3-carboxamide | 708.4 | (500 MHz, DMSO-d6) δ 10.53 (s, 1H), 9.92 (d, J=7.3 Hz, 1H), 8.24 (dd, J=6.3, 2.4 Hz, 1H), 8.15 (s, 1H), 7.81 - 7.70 (m, 4H), 7.49 (t, J=9.8 Hz, 1H), 7.42 (t, J=8.8 Hz, 1H), 7.33 (d, J=8.8 Hz, 1H), 5.39 (dd, J=9.2, 3.7 Hz, 1H), 4.70 (d, J=9.5 Hz, 1H), 4.58 (br t, J=7.7 Hz, 1H), 4.50 - 4.45 (m, 1H), 4.13 - 4.11 (m, 1H), 4.07 (s, 3H), 3.98 (br d, J=9.5 Hz, 1H), 3.77 (dd, J=9.4, 7.0 Hz, 1H), 3.67 (dd, J=10.7, 3.5 Hz, 1H), 3.19 - 3.10 (m, 2H), 2.73 (br s, 1H), 1.90 - 1.79 (m, 2H), 1.54 - 1.45 (m, 1H), 1.45 - 1.38 (m, 2H), 0.74 (quin, J=10.1 Hz, 2H), 0.38 - 0.33 (m, 2H) | 2.69, A |
| 913 | 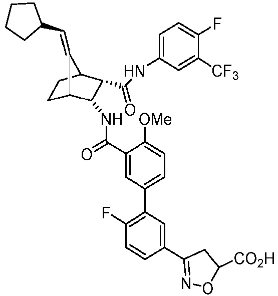 | 3-(3'-{[(1R,2R,3S,4R,7Z)-7-(cyclopentylmethylide ne)-3-{[4-fluoro-3-(trifluoromethyl)pheny l]carbamoyl}bicyclo[2 .2.1]heptan-2-yl]carbamoyl}-6-fluoro-4'-methoxy-[1,1'-biphenyl]-3-yl)-4,5-dihydro-1,2-oxazole-5-carboxylic acid | 738.4 | (500 MHz, DMSO-d6) δ 10.58 (s, 1H), 9.89 (br d, J=6.9 Hz, 1H), 8.26 - 8.20 (m, 1H), 8.16 (s, 1H), 7.82 - 7.70 (m, 3H), 7.48 (t, J=9.8 Hz, 1H), 7.40 (t, J=9.0 Hz, 1H), 7.32 (d, J=8.7 Hz, 1H), 5.19 (d, J=8.9 Hz, 1H), 5.08 - 5.03 (m, 1H), 4.41 (br s, 1H), 4.06 (s, 3H), 3.19 - 3.15 (m, 1H), 3.01 (br s, 1H), 2.72 (br s, 1H), 1.92 - 1.86 (m, 1H), 1.86 - 1.71 (m, 3H), 1.70 - 1.50 (m, 4H), 1.39 (br s, 2H), 1.32 - 1.19 (m, 2H) | 2.38, A |
| 914 | 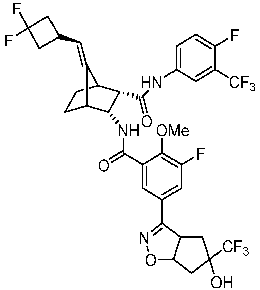 | (1R,2S,3R,4R,7Z)-7-[(3,3-difluorocyclobutyl)me thylidene]-N-[4-fluoro-3-(trifluoromethyl)pheny l]-3-{3-fluoro-5-[5-hydroxy-5-(trifluoromethyl)-3aH,4H,5H,6H,6aH-cyclopenta[d][1,2]oxa zol-3-yl]-2-methoxybenzamido}bi cyclo[2.2.1]heptane-2-carboxamide | 764.2 | (DMSO-d6, 400 MHz) δ 10.5-10.6 (m, 1H), 9.7-9.7 (m, 1H), 8.1-8.2 (m, 1H), 7.9-8.0 (m, 1H), 7.8-7.9 (m, 1H), 7.7-7.8 (m, 1H), 7.4-7.5 (m, 1H), 5.9-6.0 (m, 1H), 5.4-5.4 (m, 1H), 5.3-5.4 (m, 1H), 4.3-4.4 (m, 2H), 4.0-4.1 (m, 3 H), 3.1-3.2 (m, 1H), 2.6-3.0 (m, 8H), 2.4-2.5 (m, 1H), 2.2-2.3 (m, 2H), 1.9-2.1 (m, 1H), 1.8-1.9 (m, 2H), 1.3-1.5 (m, 2H) | 2.50, B |
| 915 | 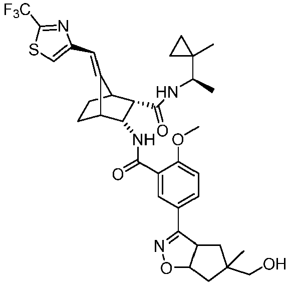 | (1R,2S,3R,4R,7Z)-3-{5-[5-(hydroxymethyl)-5-methyl-3aH,4H,5H,6H,6aH-cyclopenta[d][1,2]oxa zol-3-yl]-2-methoxybenzamido}-N-[(1R)-1-(1-methylcyclopropyl)eth yl]-7-{[2-(trifluoromethyl)-1,3-thiazol-4-yl]methylidene}bicycl o[2.2.1]heptane-2-carboxamide | 687.3 | (400 MHz, DMSO-d6) δ = 10.11 (d, J = 6.8 Hz, 1H), 8.14 (d, J = 2.4 Hz, 1H), 8.04 (d, J = 8.6 Hz, 1H), 7.97 (s, 1H), 7.79 (dd, J = 2.4, 8.6 Hz, 1H), 7.24 (d, J = 8.9 Hz, 1H), 6.43 (s, 1H), 5.30 - 5.09 (m, 1H), 4.59 (t, J = 5.3 Hz, 1H), 4.37 (ddd, J = 4.4, 6.5, 10.4 Hz, 1H), 4.25 (dt, J = 6.6, 9.8 Hz, 1H), 4.01 (s, 3H), 3.70 (br s, 1H), 3.53 - 3.48 (m, 1H), 3.21 - 3.12 (m, 2H), 3.09 (dd, J = 4.4, 10.9 Hz, 1H), 2.78 (br s, 1H), 2.06 - 1.96 (m, 1H), 1.92 - 1.76 (m, 4H), 1.66 (dd, J = 6.5, 12.9 Hz, 1H), 1.47 (br d, J = 6.0 Hz, 2H), 1.04 (d, J = 6.9 Hz, 3H), 1.01 (s, 3H), 0.96 (s, 3H), 0.63 - 0.50 (m, 1H), 0.40 - 0.29 (m, 1H), 0.25 - 0.10 (m, 2H) | 2.36, B |
| 916 | 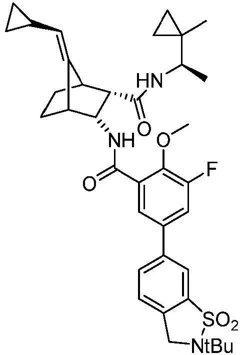 | (1R,2S,3R,4R,7Z)-3-[5-(2-tert-butyl-1,1-dioxo-2,3-dihydro-1λ⁶,2-benzothiazol-6-yl)-3-fluoro-2-methoxybenzamido]-7-(cyclopropylmethylide ne)-N-[(1R)-1-(1-methylcyclopropyl)eth yl]bicyclo[2.2.1]hepta ne-2-carboxamide | 664.3 | (400 MHz, DMSO-d6) δ 9.98 (d, J = 6.9 Hz, 1H), 8.06 (d, J = 1.1 Hz, 1H), 8.04 - 7.87 (m, 4H), 7.66 (d, J = 8.3 Hz, 1H), 4.66 (d, J = 9.5 Hz, 1H), 4.59 (s, 2H), 4.38 - 4.29 (m, 1H), 4.05 (d, J = 2.3 Hz, 3H), 3.50 - 3.45 (m, 1H), 3.11 - 3.04 (m, 1H), 2.98 (dd, J = 4.4, 10.9 Hz, 1H), 2.59 - 2.54 (m, 1H), 1.93 - 1.72 (m, 2H), 1.54 - 1.30 (m, 12H), 1.02 (d, J = 6.9 Hz, 3H), 0.98 (s, 3H), 0.80 - 0.66 (m, 2H), 0.58 - 0.47 (m, 1H), 0.41 - 0.27 (m, 3H), 0.24 - 0.08 (m, 2H). | 2.69, A |
| 917 | 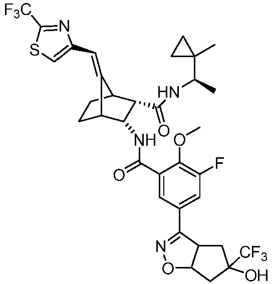 | (1R,2S,3R,4R,7Z)-3-{3-fluoro-5-[5-hydroxy-5-(trifluoromethyl)-3aH,4H,5H,6H,6aH-cyclopenta[d][1,2]oxa zol-3-yl]-2-methoxybenzamido}-N-[(1R)-1-(1-methylcyclopropyl)eth yl]-7-{[2-(trifluoromethyl)-1,3-thiazol-5-yl]methylidene}bicycl o[2.2.1]heptane-2-carboxamide | 745.2 | (400 MHz, DMSO-d6) δ 10.15 (d, J = 6.5 Hz, 1H), 8.13 (d, J = 9.0 Hz, 1H), 8.09 (s, 1H), 7.94 - 7.87 (m, 1H), 7.71 (dd, J = 2.3, 12.5 Hz, 1H), 6.67 (s, 1H), 5.92 (s, 1H), 5.31 (dd, J = 6.3, 9.5 Hz, 1H), 4.54 - 4.27 (m, 2H), 4.06 (d, J = 2.5 Hz, 3H), 3.48 (dd, J = 7.0, 8.8 Hz, 1H), 3.12 (dd, J = 4.4, 11.1 Hz, 1H), 2.85 (br d, J = 2.5 Hz, 1H), 2.32 - 2.18 (m, 3H), 2.00 (br d, J = 14.0 Hz, 1H), 1.96 - 1.84 (m, 2H), 1.58 - 1.44 (m, 2H), 1.04 (d, J = 7.0 Hz, 3H), 1.00 (s, 3H), 0.58 - 0.49 (m, 1H), 0.40 - 0.31 (m, 1H), 0.28 - 0.11 (m, 2H). | 2.56, A |
| 918 | 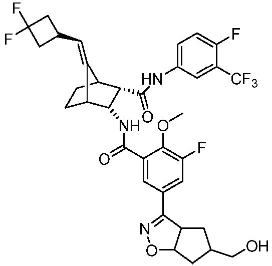 | (1R,2S,3R,4R,7Z)-7-[(3,3-difluorocyclobutyl)me thylidene]-N-[4-fluoro-3-(trifluoromethyl)pheny l]-3-{3-fluoro-5-[5-(hydroxymethyl)-3aH,4H,5H,6H,6aH-cyclopenta[d][1,2]oxa zol-3-yl]-2-methoxybenzamido}bi cyclo[2.2.1]heptane-2-carboxamide | 710.2 | (500 MHz, DMSO-d6) δ 10.54 (s, 1H), 9.69 (d, J=7.2 Hz, 1H), 8.11 (dd, J=6.3, 2.1 Hz, 1H), 7.96 (s, 1H), 7.86 - 7.81 (m, 1H), 7.72 (dd, J=12.6, 1.9 Hz, 1H), 7.48 (t, J=9.8 Hz, 1H), 5.40 (d, J=8.5 Hz, 1H), 5.16 (dd, J=8.7, 5.1 Hz, 1H), 4.51 (t, J=5.1 Hz, 1H), 4.45 - 4.36 (m, 1H), 4.19 (br t, J=8.6 Hz, 1H), 4.08 (s, 1H), 4.09 (s, 2H), 3.17 (dd, J=10.4, 4.3 Hz, 1H), 3.05 - 2.99 (m, 1H), 2.97 - 2.79 (m, 3H), 2.78 - 2.73 (m, 1H), 2.47 - 2.36 (m, 2H), 1.99 (br dd, J=13.3, 5.8 Hz, 1H), 1.93 - 1.73 (m, 4H), 1.68 - 1.51 (m, 2H), 1.41 (br d, J=4.6 Hz, 2H), two protons not observed due to water supression | 2.54, C |
| 919 | 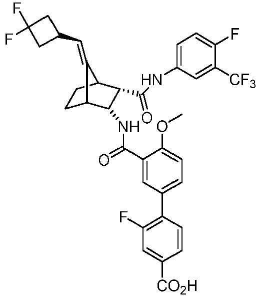 | 3'-{[(1R,2R,3S,4R,7Z)-7-[(3,3-difluorocyclobutyl)me thylidene]-3-{[4-fluoro-3-(trifluoromethyl)pheny l]carbamoyl}bicyclo[2 .2.1]heptan-2-yl]carbamoyl}-2-fluoro-4'-methoxy-[1,1'-biphenyl]-4-carboxylic acid | 691.2 | (500 MHz, DMSO-d6) δ 10.60 - 10.52 (m, 1H), 9.90 (br d, J=7.2 Hz, 1H), 8.24 - 8.11 (m, 2H), 7.84 (br d, J=8.1 Hz, 1H), 7.79 - 7.68 (m, 3H), 7.62 (br t, J=8.0 Hz, 1H), 7.51 - 7.42 (m, 1H), 7.32 (br d, J=8.8 Hz, 1H), 5.38 (br d, J=8.5 Hz, 1H), 4.43 - 4.35 (m, 1H), 4.04 (s, 3H), 3.62 (br s, 3H), 3.19 - 3.11 (m, 1H), 2.99 (br s, 1H), 2.96 - 2.90 (m, 1H), 2.75 (br s, 1H), 2.46 - 2.33 (m, 2H), 1.94 - 1.74 (m, 2H), 1.45 - 1.32 (m, 2H) | 2.18, C |
| 920 | 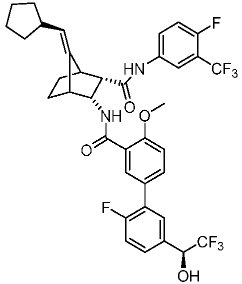 | N-[(1R,2R,3S,4R,7Z)-7-(cyclopentylmethylide ne)-3-{[4-fluoro-3-(trifluoromethyl)pheny l]carbamoyl}bicyclo[2 .2.1]heptan-2-yl]-2'-fluoro-4-methoxy-5'-[(1S)-2,2,2-trifluoro-1-hydroxyethyl]-[1,1'-biphenyl]-3-carboxamide | 723.2 | (500 MHz, DMSO-d6) δ 10.57 - 10.49 (m, 1H), 9.91 (d, J=7.2 Hz, 1H), 8.22 (dd, J=6.2, 2.2 Hz, 1H), 8.12 (s, 1H), 7.82 - 7.75 (m, 1H), 7.70 (br d, J=8.6 Hz, 1H), 7.61 (br d, J=7.6 Hz, 1H), 7.56 - 7.43 (m, 2H), 7.38 - 7.28 (m, 2H), 7.03 (d, J=5.6 Hz, 1H), 5.30 - 5.22 (m, 1H), 5.18 (s, 1H), 4.46 - 4.37 (m, 1H), 4.05 (s, 3H), 3.15 (dd, J=10.5, 4.3 Hz, 1H), 3.00 (br s, 1H), 2.71 (br s, 1H), 2.64 - 2.57 (m, 1H), 1.92 - 1.70 (m, 4H), 1.67 - 1.49 (m, 4H), 1.46 - 1.34 (m, 2H), 1.29 - 1.19 (m, 2H) | 3.00, C |
| 921 | 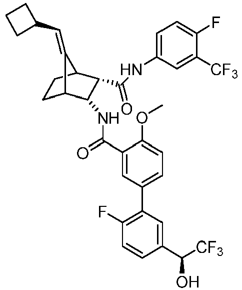 | N-[(1R,2R,3S,4R,7Z)-7-(cyclobutylmethyliden e)-3-{[4-fluoro-3-(trifluoromethyl)pheny l]carbamoyl}bicyclo[2 .2.1]heptan-2-yl]-2'-fluoro-4-methoxy-5'-[(1S)-2,2,2-trifluoro-1-hydroxyethyl]-[1,1'-biphenyl]-3-carboxamide | 709.1 | (500 MHz, DMSO-d6) δ 10.53 (s, 1H), 9.90 (d, J=7.1 Hz, 1H), 8.21 (brd, J=4.2 Hz, 1H), 8.12 (s, 1H), 7.82 - 7.75 (m, 1H), 7.69 (br d, J=8.5 Hz, 1H), 7.62 (br d, J=7.2 Hz, 1H), 7.53 - 7.43 (m, 2H), 7.38 - 7.28 (m, 2H), 5.37 (d, J=8.5 Hz, 1H), 5.29 - 5.21 (m, 1H), 4.43 - 4.32 (m, 1H), 4.04 (s, 3H), 3.22 - 3.04 (m, 2H), 2.96 (br s, 1H), 2.70 (br s, 1H), 2.20 - 2.07 (m, 2H), 1.95 - 1.67 (m, 7H), 1.48 - 1.29 (m, 2H) protons missing due to sol | 2.92, C |
| 922 | 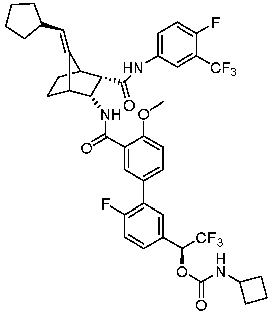 | (1S)-1-(3'-{[(2R,3S,7Z)-7-(cyclopentylmethylide ne)-3-{[4-fluoro-3-(trifluoromethyl)pheny l]carbamoyl}bicyclo[2 .2.1]heptan-2-yl]carbamoyl}-6-fluoro-4'-methoxy-[1,1'-biphenyl]-3-yl)-2,2,2-trifluoroethyl N-cyclobutylcarbamate | 820.1 | (500 MHz, DMSO-d6) δ 10.54 (s, 1H), 9.91 (br d, J=7.1 Hz, 1H), 8.28 - 8.19 (m, 1H), 8.17 - 8.05 (m, 2H), 7.81 - 7.75 (m, 1H), 7.67 (br dd, J=18.3, 7.9 Hz, 2H), 7.54 (br dd, J=3.5, 2.3 Hz, 1H), 7.49 - 7.36 (m, 2H), 7.32 (d, J=8.6 Hz, 1H), 6.42 - 6.26 (m, 1H), 5.19 (d, J=8.9 Hz, 1H), 4.48 - 4.37 (m, 1H), 4.04 (s, 3H), 3.96 - 3.85 (m, 1H), 3.15 (br dd, J=10.8, 3.7 Hz, 1H), 2.99 (br s, 1H), 2.71 (br s, 1H), 2.64 - 2.57 (m, 1H), 2.27 - 2.03 (m, 2H), 1.98 - 1.74 (m, 6H), 1.67 - 1.47 (m, 6H), 1.46 - 1.35 (m, 2H), 1.29 - 1.17 (m, 2H) | 3.26, C |
| 923 | 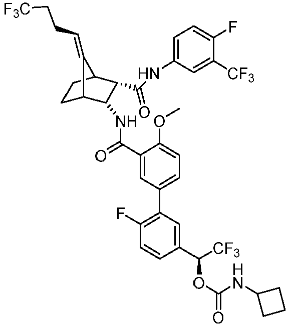 | (1S)-2,2,2-trifluoro-1-(6-fluoro-3'-{[(2R,3S,7Z)-3-{[4-fluoro-3-(trifluoromethyl)pheny l]carbamoyl}-7-(4,4,4-trifluorobutylidene)bic yclo[2.2.1]heptan-2-yl]carbamoyl}-4'-methoxy-[1,1'-biphenyl]-3-yl)ethyl N-cyclobutylcarbamate | 848.0 | (500 MHz, DMSO-d6) δ 10.56 (s, 1H), 9.93 (br d, J=7.2 Hz, 1H), 8.28 - 8.19 (m, 1H), 8.16 - 8.08 (m, 2H), 7.83 - 7.77 (m, 1H), 7.70 - 7.62 (m, 2H), 7.54 (br s, 1H), 7.48 - 7.37 (m, 2H), 7.33 (d, J=8.7 Hz, 1H), 6.41 - 6.31 (m, 1H), 5.27 (br t, J=7.1 Hz, 1H), 4.51 - 4.39 (m, 1H), 4.05 (s, 3H), 3.98 - 3.86 (m, 1H), 3.16 (br dd, J=10.3, 3.6 Hz, 1H), 3.00 (br s, 1H), 2.75 (br s, 1H), 2.44 - 2.27 (m, 4H), 2.24 - 2.04 (m, 2H), 1.98 - 1.84 (m, 3H), 1.81 - 1.71 (m, 1H), 1.70 - 1.50 (m, 2H), 1.45 - 1.35 (m, 2H) | 3.08, C |
| 924 | 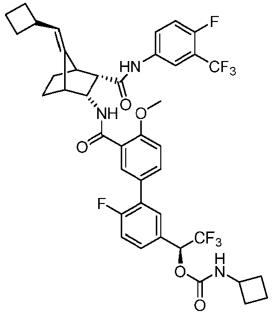 | (1S)-1-(3'-{[(2R,3S,7Z)-7-(cyclobutylmethyliden e)-3-{[4-fluoro-3-(trifluoromethyl)pheny 1]carbamoyl}bicyclo[2 .2.1]heptan-2-yl]carbamoyl}-6-fluoro-4'-methoxy-[1,1'-biphenyl]-3-yl)-2,2,2-trifluoroethyl N-cyclobutylcarbamate | 806.0 | (500 MHz, DMSO-d6) δ 11.95 - 11.95 (m, 1H), 10.53 (s, 1H), 9.91 (br d, J=6.9 Hz, 1H), 8.29 - 8.22 (m, 1H), 8.16 - 8.10 (m, 2H), 7.85 - 7.77 (m, 1H), 7.69 (br t, J=9.1 Hz, 2H), 7.54 (br dd, J=2.9, 2.0 Hz, 1H), 7.52 - 7.39 (m, 2H), 7.35 - 7.26 (m, 1H), 6.39 - 6.28 (m, 1H), 5.38 (d, J=8.3 Hz, 1H), 4.42 - 4.34 (m, 1H), 4.06 (s, 3H), 3.98 - 3.91 (m, 1H), 3.14 - 3.06 (m, 1H), 2.97 (br s, 1H), 2.72 (br d, J=2.7 Hz, 1H), 2.23 - 2.08 (m, 4H), 1.97 - 1.75 (m, 8H), 1.70 - 1.51 (m, 2H), 1.45 - 1.31 (m, 2H) | 3.19, C |
| 925 | 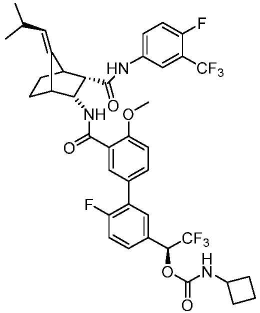 | (1S)-2,2,2-trifluoro-1-(6-fluoro-3'-{[(2R,3S,7Z)-3-{[4-fluoro-3-(trifluoromethyl)pheny 1]carbamoyl}-7-(2-methylpropylidene)bic yclo[2.2.1]heptan-2-yl]carbamoyl}-4'-methoxy-[1,1'-biphenyl]-3-yl)ethyl N-cyclobutylcarbamate | 794.1 | (500 MHz, DMSO-d6) δ 10.53 (s, 1H), 9.91 (br d, J=6.9 Hz, 1H), 8.23 (br d, J=4.1 Hz, 1H), 8.16 - 8.08 (m, 2H), 7.80 - 7.76 (m, 1H), 7.68 (br dd, J=13.7, 8.3 Hz, 2H), 7.55 (br dd, J=6.1, 2.7 Hz, 1H), 7.50 - 7.39 (m, 2H), 7.33 (d, J=8.8 Hz, 1H), 6.35 (br d, J=6.9 Hz, 1H), 5.11 (d, J=8.9 Hz, 1H), 4.46 - 4.38 (m, 1H), 4.05 (s, 3H), 3.95 - 3.85 (m, 1H), 3.16 (dd, J=10.9, 3.9 Hz, 1H), 2.99 (s, 1H), 2.70 (br s, 1H), 2.48 - 2.41 (m, 1H), 2.21 - 2.04 (m, 2H), 1.98 - 1.84 (m, 3H), 1.81 - 1.74 (m, 1H), 1.64 - 1.52 (m, 2H), 1.45 - 1.35 (m, 2H), 1.07 - 0.93 (m, 6H) | 3.15. C |
| 926 | 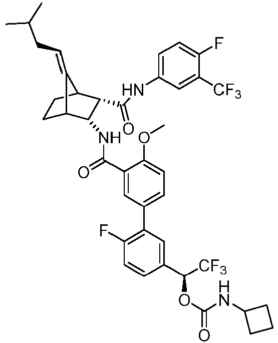 | (1S)-2,2,2-trifluoro-1-(6-fluoro-3'-{[(2R,3S,7Z)-3-{[4-fluoro-3-(trifluoromethyl)pheny l]carbamoyl}-7-(3-methylbutylidene)bicy clo[2.2.1]heptan-2-yl]carbamoyl}-4'-methoxy-[1,1'-biphenyl]-3-yl)ethyl N-cyclobutylcarbamate | 808.0 | (500 MHz, DMSO-d6) δ 10.55 (s, 1H), 9.92 (br d, J=7.0 Hz, 1H), 8.28 - 8.22 (m, 1H), 8.16 - 8.09 (m, 2H), 7.85 - 7.76 (m, 1H), 7.69 (br dd, J=13.9, 8.1 Hz, 2H), 7.54 (br s, 1H), 7.50 - 7.38 (m, 2H), 7.33 (d, J=8.8 Hz, 1H), 6.41 - 6.26 (m, 1H), 5.24 (t, J=7.6 Hz, 1H), 4.45 - 4.39 (m, 1H), 4.05 (s, 3H), 3.96 - 3.89 (m, 1H), 3.18 (br dd, J=10.7, 3.7 Hz, 1H), 2.96 (br s, 1H), 2.74 (br s, 1H), 2.20 - 2.04 (m, 2H), 1.99 - 1.84 (m, 5H), 1.80 - 1.72 (m, 1H), 1.67 - 1.53 (m, 3H), 1.46 - 1.33 (m, 2H), 0.91 (dd, J=12.3, 6.6 Hz, 6H) | 3.23, C |
| 927 | 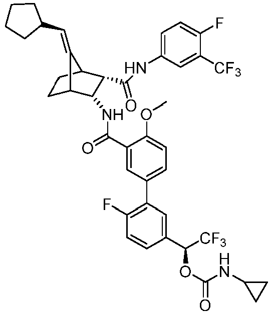 | (1S)-1-(3'-{[(1R,2R,3S,4R,7Z)-7-(cyclopentylmethylide ne)-3-{[4-fluoro-3-(trifluoromethyl)pheny 1]carbamoyl}bicyclo[2 .2.1]heptan-2-yl]carbamoyl}-6-fluoro-4'-methoxy-[1,1'-biphenyl]-3-yl)-2,2,2-trifluoroethyl N-cyclopropylcarbamate | 806.4 | (500 MHz, DMSO-d6) δ 10.30 (s, 1H), 9.68 (br d, J=6.9 Hz, 1H), 8.04 - 7.95 (m, 1H), 7.89 (s, 1H), 7.77 (br s, 1H), 7.58 - 7.52 (m, 1H), 7.45 (br d, J=1.1 Hz, 2H), 7.30 (br s, 1H), 7.27 - 7.14 (m, 2H), 7.09 (d, J=8.8 Hz, 1H), 6.14 (br d, J=7.5 Hz, 1H), 4.95 (d, J=8.9 Hz, 1H), 4.24 - 4.10 (m, 1H), 3.82 (s, 3H), 2.92 (br dd, J=10.4, 4.2 Hz, 1H), 2.78 - 2.76 (m, 1H), 2.75 - 2.72 (m, 1H), 2.48 (br s, 1H), 2.35 (br d, J=8.6 Hz, 1H), 1.66 - 1.50 (m, 4H), 1.46 - 1.25 (m, 4H), 1.15 (br d, J=3.0 Hz, 2H), 1.03 (td, J=8.0, 3.9 Hz, 2H), 0.41 - 0.30 (m, 2H), 0.25 - 0.12 (m, 2H) | 3.06, C |
| 928 | 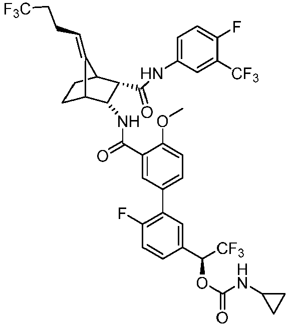 | (1S)-2,2,2-trifluoro-1-(6-fluoro-3'-{[(1R,2R,3S,4R,7Z)-3-{[4-fluoro-3-(trifluoromethyl)pheny l]carbamoyl}-7-(4,4,4-trifluorobutylidene)bic yclo[2.2.1]heptan-2-yl]carbamoyl}-4'-methoxy-[1,1'-biphenyl]-3-yl)ethyl N-cyclopropylcarbamate | 834.2 | (500 MHz, DMSO-d6) δ 10.38 - 10.30 (m, 1H), 9.70 (br d, J=7.0 Hz, 1H), 8.04 - 7.99 (m, 1H), 7.90 (s, 1H), 7.78 (br d, J=2.2 Hz, 1H), 7.57 (br dd, J=8.6, 3.3 Hz, 1H), 7.46 (br t, J=9.6 Hz, 2H), 7.35 - 7.30 (m, 1H), 7.28 - 7.17 (m, 2H), 7.10 (d, J=8.8 Hz, 1H), 6.22 - 6.12 (m, 1H), 5.05 (br t, J=6.7 Hz, 1H), 4.20 (ddd, J=10.3, 6.8, 3.9 Hz, 1H), 3.83 (s, 3H), 3.01 - 2.91 (m, 1H), 2.78 (br s, 1H), 2.53 (br s, 1H), 2.20 - 2.02 (m, 4H), 1.72 - 1.50 (m, 2H), 1.22 - 1.11 (m, 2H), 0.81 (d, J=6.0 Hz, 1H), 0.40 - 0.30 (m, 2H), 0.25 - 0.14 (m, 2H) | 2.84, C |
| 929 | 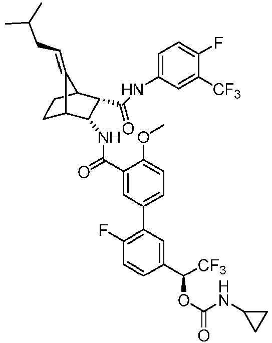 | (1S)-2,2,2-trifluoro-1-(6-fluoro-3'-{[(1R,2R,3S,4R,7Z)-3-{[4-fluoro-3-(trifluoromethyl)pheny l]carbamoyl}-7-(3-methylbutylidene)bicy clo[2.2.1]heptan-2-yl]carbamoyl}-4'-methoxy-[1,1'-biphenyl]-3-yl)ethyl N-cyclopropylcarbamate | 794.2 | (500 MHz, DMSO-d6) δ 10.35 (s, 1H), 9.70 (br d, J=7.1 Hz, 1H), 8.07 - 8.00 (m, 1H), 7.91 (s, 1H), 7.79 (br d, J=2.4 Hz, 1H), 7.57 (dt, J=7.8, 3.9 Hz, 1H), 7.50 - 7.42 (m, 2H), 7.31 (br d, J=3.4 Hz, 1H), 7.29 - 7.18 (m, 2H), 7.10 (d, J=8.8 Hz, 1H), 6.17 (br d, J=6.3 Hz, 1H), 5.02 (t, J=7.7 Hz, 1H), 4.26 - 4.16 (m, 1H), 3.83 (s, 3H), 2.97 (dd, J=10.8, 4.1 Hz, 1H), 2.74 (br s, 1H), 2.52 (t, J=3.4 Hz, 1H), 1.80 - 1.71 (m, 2H), 1.70 - 1.54 (m, 2H), 1.47 - 1.35 (m, 1H), 1.25 - 1.12 (m, 2H), 0.69 (dd, J=12.3, 6.6 Hz, 6H), 0.36 (br d, J=3.2 Hz, 2H), 0.23 - 0.12 (m, 2H) proton missing due to water supression | 3.00, C |
| 930 | 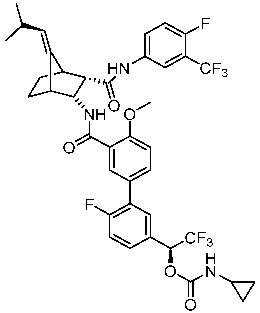 | (1S)-2,2,2-trifluoro-1-(6-fluoro-3'-{[(1R,2R,3S,4R,7Z)-3-{[4-fluoro-3-(trifluoromethyl)pheny l]carbamoyl}-7-(2-methylpropylidene)bic yclo[2.2.1]heptan-2-yl]carbamoyl}-4'-methoxy-[1,1'-biphenyl]-3-yl)ethyl N-cyclopropylcarbamate | 780.1 | (500 MHz, DMSO-d6) δ 10.35 (s, 1H), 9.73 (br d, J=6.9 Hz, 1H), 8.10 - 8.02 (m, 1H), 7.95 (s, 1H), 7.83 (br d, J=2.7 Hz, 1H), 7.64 - 7.57 (m, 1H), 7.55 - 7.48 (m, 2H), 7.36 (br s, 1H), 7.32 - 7.20 (m, 2H), 7.15 (d, J=8.8 Hz, 1H), 6.21 (br d, J=6.6 Hz, 1H), 4.93 (d, J=9.0 Hz, 1H), 4.24 (ddd, J=10.7, 7.2, 3.8 Hz, 1H), 3.88 (s, 3H), 2.99 (dd, J=10.8, 4.1 Hz, 1H), 2.83 - 2.80 (m, 1H), 2.52 (br s, 1H), 2.28 (br s, 1H), 1.76 - 1.58 (m, 2H), 1.21 (br d, J=7.6 Hz, 2H), 0.86 - 0.77 (m, 6H), 0.44 - 0.35 (m, 2H), 0.28 - 0.17 (m, 2H) proton missing due to water suppression | 2.93, C |
| 931 | 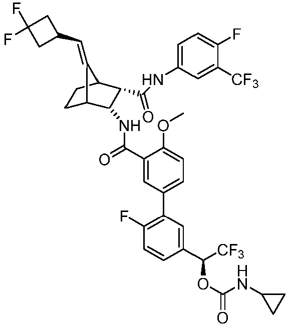 | (1S)-1-(3'-{[(1R,2R,3S,4R,7Z)-7-[(3,3-difluorocyclobutyl)me thylidene]-3-{[4-fluoro-3-(trifluoromethyl)pheny 1]carbamoyl}bicyclo[2 .2.1]heptan-2-yl]carbamoyl}-6-fluoro-4'-methoxy-[1,1'-biphenyl]-3-yl)-2,2,2-trifluoroethyl N-cyclopropylcarbamate | 828.2 | (500 MHz, DMSO-d6) δ 10.32 (s, 1H), 9.70 (br d, J=6.9 Hz, 1H), 8.04 - 7.98 (m, 1H), 7.90 (s, 1H), 7.79 (br d, J=2.1 Hz, 1H), 7.56 (br dd, J=8.1, 3.3 Hz, 1H), 7.46 (br t, J=9.0 Hz, 2H), 7.35 - 7.17 (m, 3H), 7.11 (d, J=8.6 Hz, 1H), 6.23 - 6.10 (m, 1H), 5.17 (d, J=8.5 Hz, 1H), 4.24 - 4.14 (m, 1H), 3.83 (s, 3H), 2.99 - 2.93 (m, 1H), 2.78 (br s, 1H), 2.74 - 2.58 (m, 3H), 2.54 (br s, 1H), 2.25 - 2.13 (m, 2H), 1.72 - 1.51 (m, 2H), 1.24 - 1.11 (m, 2H), 0.45 - 0.31 (m, 2H), 0.24 - 0.12 (m, 2H) proton missing due to water supression | 2.82, C |
| 932 | 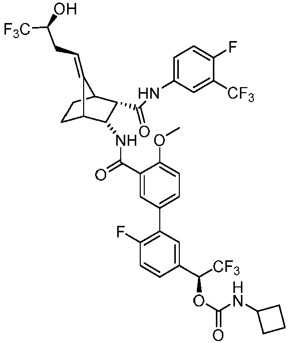 | (1S)-2,2,2-trifluoro-1-(6-fluoro-3'-{[(1R,2R,3S,4R,7Z)-3-{[4-fluoro-3-(trifluoromethyl)pheny l]carbamoyl}-7-[(3R)-4,4,4-trifluoro-3-hydroxybutylidene]bic yclo[2.2.1]heptan-2-yl]carbamoyl}-4'-methoxy-[1,1'-biphenyl]-3-yl)ethyl N-cyclobutylcarbamate | 864.0 | (500 MHz, DMSO-d6) δ 10.59 (s, 1H), 9.93 (br d, J=7.1 Hz, 1H), 8.29 - 8.22 (m, 1H), 8.17 - 8.09 (m, 2H), 7.83 - 7.78 (m, 1H), 7.69 (br t, J=9.0 Hz, 2H), 7.59 - 7.30 (m, 4H), 6.43 - 6.31 (m, 1H), 5.32 (t, J=7.3 Hz, 1H), 4.50 - 4.38 (m, 1H), 4.07 (s, 3H), 4.01 - 3.93 (m, 2H), 3.44 - 3.32 (m, 1H), 3.19 (dd, J=10.8, 4.2 Hz, 1H), 3.00 (br s, 1H), 2.78 (br s, 1H), 2.46 - 2.40 (m, 1H), 2.30 (dt, J=14.7, 7.4 Hz, 1H), 2.19 - 2.05 (m, 2H), 1.96 - 1.82 (m, 3H), 1.78 (br d, J=7.6 Hz, 1H), 1.65 - 1.52 (m, 2H), 1.42 (br d, J=5.8 Hz, 2H) | 2.77, C |
| 933 | 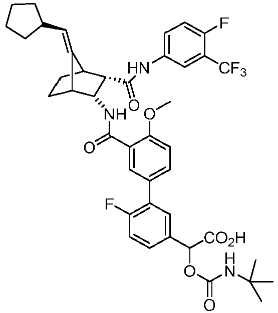 | 2-[(tert-butylcarbamoyl)oxy]-2-(3'-{[(1R,2R,3S,4R,7Z)-7-(cyclopentylmethylide ne)-3-{[4-fluoro-3-(trifluoromethyl)pheny l]carbamoyl}bicyclo[2 .2.1]heptan-2-yl]carbamoyl}-6-fluoro-4'-methoxy-[1,1'-biphenyl]-3-yl)acetic acid | 798.2 | (500 MHz, DMSO-d6) δ 10.33 (s, 1H), 9.68 (d, J=7.2 Hz, 1H), 8.01 (dd, J=6.4, 2.4 Hz, 1H), 7.88 (s, 1H), 7.56 (br dd, J=8.4, 3.6 Hz, 1H), 7.43 (br d, J=8.7 Hz, 1H), 7.39 - 7.32 (m, 1H), 7.28 - 7.19 (m, 2H), 7.09 - 7.01 (m, 2H), 6.88 (br d, J=2.7 Hz, 1H), 5.43 (s, 1H), 4.95 (d, J=8.9 Hz, 1H), 4.23 - 4.10 (m, 1H), 3.81 (s, 3H), 3.66 (s, 1H), 2.93 (dd, J=10.5, 4.3 Hz, 1H), 2.82 - 2.74 (m, 1H), 2.48 (br s, 1H), 2.42 - 2.33 (m, 1H), 1.65 - 1.50 (m, 4H), 1.45 - 1.38 (m, 2H), 1.32 (br dd, J=7.3, 5.1 Hz, 2H), 1.14 (br d, J=2.6 Hz, 2H), 1.07 - 1.01 (m, 2H), 0.99 - 0.93 (m, 9H) | 2.91, C |
| 934 | 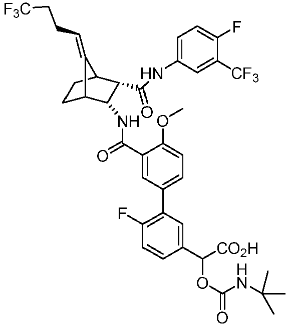 | 2-[(tert-butylcarbamoyl)oxy]-2-(6-fluoro-3'-{[(1R,2R,3S,4R,7Z)-3-{[4-fluoro-3-(trifluoromethyl)pheny l]carbamoyl}-7-(4,4,4-trifluorobutylidene)bic yclo[2.2.1]heptan-2-yl]carbamoyl}-4'-methoxy-[1,1'-biphenyl]-3-yl)acetic acid | 826.2 | (500 MHz, DMSO-d6) δ 10.67 - 10.53 (m, 1H), 9.94 (br d, J=7.2 Hz, 1H), 8.26 (dd, J=6.6, 2.4 Hz, 1H), 8.13 (s, 1H), 7.82 (br dd, J=8.2, 3.5 Hz, 1H), 7.69 (br d, J=8.2 Hz, 1H), 7.60 (br d, J=7.7 Hz, 1H), 7.52 - 7.42 (m, 2H), 7.37 - 7.29 (m, 2H), 7.20 (br d, J=1.6 Hz, 1H), 5.72 (s, 1H), 5.28 (t, J=6.7 Hz, 1H), 4.50 - 4.31 (m, 1H), 4.06 (s, 3H), 3.19 (dd, J=10.3, 4.1 Hz, 1H), 3.05 - 2.96 (m, 1H), 2.77 (br s, 1H), 2.43 - 2.23 (m, 5H), 1.96 - 1.71 (m, 2H), 1.47 - 1.35 (m, 2H), 1.23 (s, 9H) | 2.73, C |
| 935 | 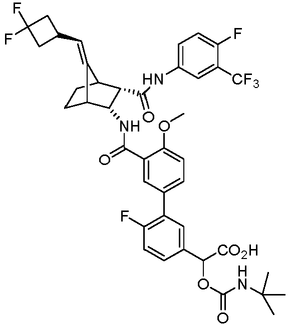 | 2-[(tert-butylcarbamoyl)oxy]-2-(3'-{[(1R,2R,3S,4R,7Z)-7-[(3,3-difluorocyclobutyl)me thylidene]-3-{[4-fluoro-3-(trifluoromethyl)pheny 1]carbamoyl}bicyclo[2 .2.1]heptan-2-yl]carbamoyl}-6-fluoro-4'-methoxy-[1,1'-biphenyl]-3-yl)acetic acid | 820.1 | (500 MHz, DMSO-d6) δ 10.59 (s, 1H), 9.94 (br d, J=7.2 Hz, 1H), 8.25 (dd, J=6.4, 2.1 Hz, 1H), 8.13 (s, 1H), 7.88 - 7.79 (m, 1H), 7.69 (br d, J=8.5 Hz, 1H), 7.61 (br d, J=7.2 Hz, 1H), 7.53 - 7.45 (m, 2H), 7.38 - 7.31 (m, 2H), 7.20 (br s, 1H), 5.72 (s, 1H), 5.40 (d, J=8.5 Hz, 1H), 4.48 - 4.33 (m, 1H), 4.06 (s, 3H), 3.19 (dd, J=10.9, 3.3 Hz, 1H), 3.02 (br s, 1H), 2.98 - 2.81 (m, 3H), 2.77 (br s, 1H), 2.48 - 2.38 (m, 2H), 1.92 - 1.76 (m, 2H), 1.51 - 1.36 (m, 2H), 1.23 (s, 10H) | 2.71, C |
| 936 | 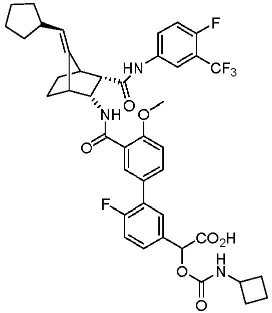 | 2-[(cyclobutylcarbamoyl )oxy]-2-(3'-{[(1R,2R,3S,4R,7Z)-7-(cyclopentylmethylide ne)-3-{[4-fluoro-3-(trifluoromethyl)pheny l]carbamoyl}bicyclo[2 .2.1]heptan-2-yl]carbamoyl}-6-fluoro-4'-methoxy-[1,1'-biphenyl]-3-yl)acetic acid | 796.3 | (500 MHz, DMSO-d6) δ 10.59 (s, 1H), 9.93 (br d, J=6.9 Hz, 1H), 8.25 (dd, J=6.4, 2.1 Hz, 1H), 8.13 (s, 1H), 7.80 (br dd, J=7.9, 3.5 Hz, 1H), 7.67 (br d, J=6.9 Hz, 2H), 7.57 (br d, J=6.9 Hz, 1H), 7.51 - 7.42 (m, 2H), 7.33 - 7.26 (m, 2H), 5.69 (s, 1H), 5.19 (d, J=8.8 Hz, 1H), 4.47 - 4.36 (m, 1H), 4.06 (s, 3H), 4.01 - 3.93 (m, 1H), 3.25 - 3.17 (m, 1H), 3.11 - 3.03 (m, 1H), 2.72 (br s, 1H), 2.60 (br d, J=8.4 Hz, 1H), 2.18 - 2.07 (m, 2H), 1.96 - 1.76 (m, 6H), 1.66 - 1.51 (m, 6H), 1.44 - 1.35 (m, 2H), 1.34 - 1.25 (m, 2H) | 2.87, C |
| 937 | 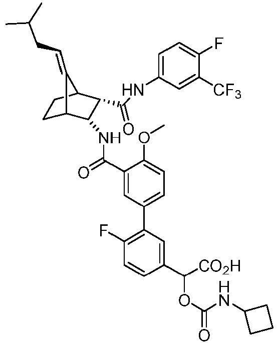 | (2S)-2-[(cyclobutylcarbamoyl )oxy]-2-(6-fluoro-3'-{[(1R,2R,3S,4R,7Z)-3-{[4-fluoro-3-(trifluoromethyl)pheny l]carbamoyl}-7-(3-methylbutylidene)bicy clo[2.2.1]heptan-2-yl]carbamoyl}-4'-methoxy-[1,1'-biphenyl]-3-yl)acetic acid | 784.1 | (500 MHz, DMSO-d6) δ 10.35 (s, 1H), 9.70 (br d, J=7.2 Hz, 1H), 8.03 (dd, J=6.3, 2.5 Hz, 1H), 7.89 (s, 1H), 7.58 (br dd, J=8.7, 3.8 Hz, 1H), 7.47 (br dd, J=15.1, 8.1 Hz, 2H), 7.34 (br d, J=7.0 Hz, 1H), 7.29 - 7.19 (m, 2H), 7.13 - 7.00 (m, 2H), 5.48 (s, 1H), 5.01 (t, J=7.6 Hz, 1H), 4.25 - 4.11 (m, 1H), 3.83 (s, 3H), 3.76 - 3.70 (m, 1H), 2.96 (dd, J=11.0, 4.4 Hz, 1H), 2.78 - 2.69 (m, 1H), 2.52 (t, J=3.4 Hz, 1H), 1.99 - 1.85 (m, 2H), 1.77 - 1.57 (m, 6H), 1.41 - 1.27 (m, 3H), 1.23 - 1.10 (m, 2H), 0.69 (dd, J=12.3, 6.6 Hz, 6H) | 2.84, C |
| 938 | 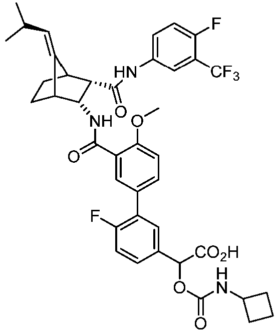 | 2-[(cyclobutylcarbamoyl )oxy]-2-(6-fluoro-3'-{[(1R,2R,3S,4R,7Z)-3-{[4-fluoro-3-(trifluoromethyl)pheny l]carbamoyl}-7-(2-methylpropylidene)bic yclo[2.2.1]heptan-2-yl]carbamoyl}-4'-methoxy-[1,1'-biphenyl]-3-yl)acetic acid | 770.4 | (500 MHz, DMSO-d6) δ 10.51 (s, 1H), 9.88 (br d, J=7.2 Hz, 1H), 8.20 (dd, J=6.9, 2.0 Hz, 1H), 8.08 (s, 1H), 7.82 - 7.73 (m, 2H), 7.64 (br d, J=8.9 Hz, 1H), 7.54 (br s, 1H), 7.50 - 7.39 (m, 2H), 7.38 - 7.25 (m, 2H), 7.20 - 7.00 (m, 1H), 5.73 (s, 1H), 5.07 (d, J=9.0 Hz, 1H), 4.44 - 4.28 (m, 1H), 4.01 (s, 3H), 3.94 - 3.87 (m, 1H), 3.12 (br dd, J=7.1, 3.9 Hz, 1H), 2.99 - 2.94 (m, 1H), 2.66 (br s, 1H), 2.15 - 2.04 (m, 2H), 1.97 - 1.80 (m, 3H), 1.79 - 1.71 (m, 1H), 1.58 - 1.49 (m, 2H), 1.35 (br d, J=7.2 Hz, 2H), 1.02 - 0.93 (m, 6H) | 2.76, C |
| 939 | 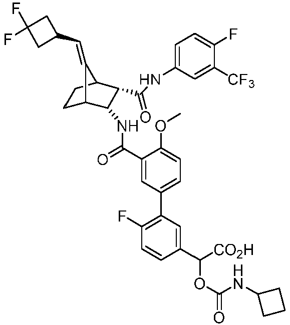 | 2-[(cyclobutylcarbamoyl )oxy]-2-(3'-{[(1R,2R,3S,4R,7Z)-7-[(3,3-difluorocyclobutyl)me thylidene]-3-{[4-fluoro-3-(trifluoromethyl)pheny l]carbamoyl}bicyclo[2 .2.1]heptan-2-yl]carbamoyl}-6-fluoro-4'-methoxy-[1,1'-biphenyl]-3-yl)acetic acid | 818.2 | (500 MHz, DMSO-d6) δ 10.57 (s, 1H), 9.94 (br d, J=6.9 Hz, 1H), 8.32 - 8.19 (m, 1H), 8.12 (s, 1H), 7.85 - 7.77 (m, 2H), 7.69 (br d, J=8.7 Hz, 1H), 7.59 (br d, J=7.3 Hz, 1H), 7.51 - 7.46 (m, 2H), 7.37 - 7.28 (m, 2H), 5.76 (s, 1H), 5.40 (d, J=8.5 Hz, 1H), 4.50 - 4.37 (m, 1H), 4.06 (s, 3H), 4.00 - 3.93 (m, 1H), 3.18 (dd, J=10.4, 4.4 Hz, 1H), 3.02 (br s, 1H), 2.97 - 2.71 (m, 4H), 2.48 - 2.31 (m, 2H), 2.20 - 2.10 (m, 2H), 2.01 - 1.74 (m, 4H), 1.67 - 1.54 (m, 2H), 1.40 (br d, J=1.8 Hz, 2H) | 2.67, C |
| 940 | 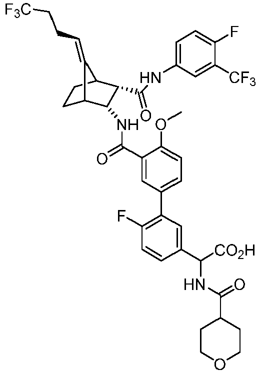 | 2-(6-fluoro-3'-{[(1R,2R,3S,4R,7Z)-3-{[4-fluoro-3-(trifluoromethyl)pheny l]carbamoyl}-7-(4,4,4-trifluorobutylidene)bic yclo[2.2.1]heptan-2-yl]carbamoyl}-4'-methoxy-[1,1'-biphenyl]-3-yl)-2-[(oxan-4-yl)formamido]acetic acid | 838.2 | (500 MHz, DMSO-d6) δ 10.56 (s, 1H), 9.94 (br d, J=7.1 Hz, 1H), 8.33 - 8.20 (m, 2H), 8.13 (br s, 1H), 7.80 (br dd, J=5.0, 3.4 Hz, 1H), 7.69 - 7.64 (m, 1H), 7.54 - 7.46 (m, 2H), 7.41 - 7.27 (m, 2H), 7.22 - 7.14 (m, 1H), 5.27 (br t, J=5.8 Hz, 2H), 4.48 - 4.37 (m, 1H), 4.04 (s, 3H), 3.83 (br dd, J=10.6, 3.1 Hz, 2H), 3.32 - 3.25 (m, 1H), 3.17 (br dd, J=10.7, 3.7 Hz, 1H), 3.02 - 2.95 (m, 1H), 2.75 (br s, 1H), 2.59 - 2.56 (m, 1H), 2.40 - 2.26 (m, 4H), 1.93 - 1.76 (m, 3H), 1.64 - 1.49 (m, 4H), 1.47 - 1.36 (m, 2H) | 2.49, C |
| 941 | 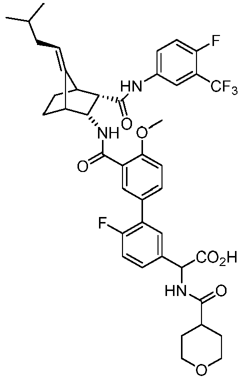 | 2-(6-fluoro-3'-{[(1R,2R,3S,4R,7Z)-3-{[4-fluoro-3-(trifluoromethyl)pheny l]carbamoyl}-7-(3-methylbutylidene)bicy clo[2.2.1]heptan-2-yl]carbamoyl}-4'-methoxy-[1,1'-biphenyl]-3-yl)-2-[(oxan-4-yl)formamido]acetic acid | 798.2 | (500 MHz, DMSO-d6) δ 10.57 (s, 1H), 9.92 (d, J=6.9 Hz, 1H), 8.46 - 8.34 (m, 1H), 8.26 (dd, J=6.5, 2.3 Hz, 1H), 8.15 (s, 1H), 7.81 (br dd, J=8.4, 3.5 Hz, 1H), 7.68 (br d, J=8.8 Hz, 1H), 7.55 - 7.45 (m, 2H), 7.37 (br d, J=2.8 Hz, 1H), 7.31 (d, J=8.7 Hz, 1H), 7.27 - 7.21 (m, 1H), 5.33 - 5.11 (m, 2H), 4.51 - 4.34 (m, 1H), 4.06 (s, 3H), 3.88 - 3.79 (m, 2H), 3.34 - 3.26 (m, 1H), 3.19 (dd, J=10.8, 4.7 Hz, 1H), 3.01 - 2.93 (m, 1H), 2.81 - 2.73 (m, 1H), 2.61 - 2.57 (m, 1H), 2.02 - 1.93 (m, 2H), 1.91 - 1.84 (m, 1H), 1.81 - 1.73 (m, 1H), 1.70 - 1.49 (m, 6H), 1.44 - 1.29 (m, 2H), 0.92 (dd, J=12.3, 6.6 Hz, 6H) | 2.64, C |
| 942 | 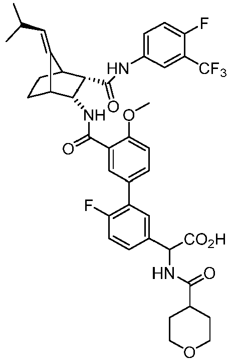 | 2-(6-fluoro-3'-{[(1R,2R,3S,4R,7Z)-3-{[4-fluoro-3-(trifluoromethyl)pheny l]carbamoyl}-7-(2-methylpropylidene)bic yclo[2.2.1]heptan-2-yl]carbamoyl}-4'-methoxy-[1,1'-biphenyl]-3-yl)-2-[(oxan-4-yl)formamido]acetic acid | 784.2 | (500 MHz, DMSO-d6) δ 10.36 - 10.27 (m, 1H), 9.69 (d, J=7.1 Hz, 1H), 8.36 (br d, J=7.6 Hz, 1H), 8.02 (dd, J=6.4, 2.3 Hz, 1H), 7.92 (s, 1H), 7.65 - 7.54 (m, 1H), 7.47 (br d, J=8.4 Hz, 1H), 7.34 - 7.16 (m, 3H), 7.13 - 7.02 (m, 2H), 5.16 (br d, J=7.4 Hz, 1H), 4.89 (d, J=8.9 Hz, 1H), 4.27 - 4.12 (m, 1H), 3.83 (s, 3H), 3.66 - 3.57 (m, 2H), 3.12 - 3.02 (m, 1H), 2.95 (dd, J=10.8, 3.9 Hz, 1H), 2.78 (br s, 1H), 2.48 (br s, 1H), 2.36 - 2.33 (m, 1H), 2.26 - 2.20 (m, 1H), 1.73 - 1.51 (m, 2H), 1.44 - 1.28 (m, 4H), 1.22 - 1.10 (m, 2H), 0.84 - 0.74 (m, 6H) protons missing due to water supression | 2.55, C |
| 943 | 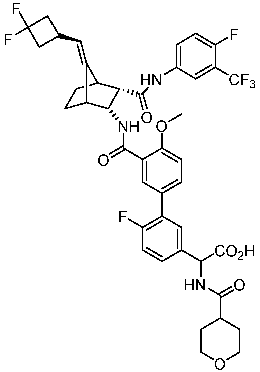 | 2-(3'-{[(1R,2R,3S,4R,7Z)-7-[(3,3-difluorocyclobutyl)me thylidene]-3-{[4-fluoro-3-(trifluoromethyl)pheny l]carbamoyl}bicyclo[2 .2.1]heptan-2-yl]carbamoyl}-6-fluoro-4'-methoxy-[1,1'-biphenyl]-3-yl)-2-[(oxan-4-yl)formamido]acetic acid | 832.0 | (500 MHz, DMSO-d6) δ 10.56 (s, 1H), 9.93 (br d, J=6.9 Hz, 1H), 8.44 (br s, 1H), 8.30 - 8.20 (m, 1H), 8.14 (br s, 1H), 7.87 - 7.76 (m, 1H), 7.68 (br d, J=8.0 Hz, 1H), 7.54 - 7.46 (m, 2H), 7.38 (br d, J=2.7 Hz, 1H), 7.36 - 7.23 (m, 2H), 5.39 (d, J=8.5 Hz, 1H), 5.30 (br d, J=5.8 Hz, 1H), 4.48 - 4.34 (m, 1H), 4.05 (s, 3H), 3.88 - 3.80 (m, 2H), 3.33 - 3.25 (m, 1H), 3.21 (s, 1H), 2.96 - 2.73 (m, 4H), 2.46 - 2.36 (m, 2H), 1.92 - 1.67 (m, 3H), 1.67 - 1.51 (m, 4H), 1.44 - 1.31 (m, 2H) protons missing due to water supresion | 2.47, C |
| 944 | 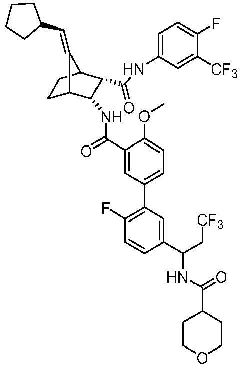 | N-[1-(3'-{[(1R,2R,3S,4R,7Z)-7-(cyclopentylmethylide ne)-3-{[4-fluoro-3-(trifluoromethyl)pheny 1]carbamoyl}bicyclo[2 .2.1]heptan-2-yl]carbamoyl}-6-fluoro-4'-methoxy-[1,1'-biphenyl]-3-yl)-3,3,3-trifluoropropyl]oxane-4-carboxamide | 848.2 | (500 MHz, DMSO-d6) δ 10.56 (s, 1H), 9.90 (br d, J=7.0 Hz, 1H), 8.50 (d, J=8.5 Hz, 1H), 8.27 - 8.23 (m, 1H), 8.15 (s, 1H), 7.82 - 7.77 (m, 1H), 7.71 (br d, J=8.9 Hz, 1H), 7.56 - 7.52 (m, 1H), 7.49 (t, J=9.8 Hz, 1H), 7.39 - 7.35 (m, 1H), 7.32 - 7.25 (m, 2H), 5.28 - 5.22 (m, 1H), 5.19 (d, J=8.8 Hz, 1H), 4.44 - 4.38 (m, 1H), 4.05 (s, 3H), 3.84 (br t, J=9.6 Hz, 2H), 3.19 - 3.15 (m, 1H), 3.01 (br s, 1H), 2.81 - 2.75 (m, 2H), 2.73 (br d, J=4.6 Hz, 1H), 2.64 - 2.57 (m, 1H), 1.87 - 1.75 (m, 4H), 1.67 - 1.54 (m, 9H), 1.39 (br d, J=2.5 Hz, 2H), 1.31 - 1.20 (m, 4H) | 2.84, C |
| 945 | 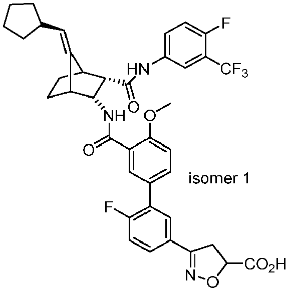 | 3-(3'-{[(1R,2R,3S,4R,7Z)-7-(cyclopentylmethylide ne)-3-{[4-fluoro-3-(trifluoromethyl)pheny l]carbamoyl}bicyclo[2 .2.1]heptan-2-yl]carbamoyl}-6-fluoro-4'-methoxy-[1,1'-biphenyl]-3-yl)-4,5-dihydro-1,2-oxazole-5-carboxylic acid | 738.1 | (500 MHz, DMSO-d6) δ 10.59 (s, 1H), 9.89 (d, J=7.0 Hz, 1H), 8.24 (dd, J=6.3, 2.4 Hz, 1H), 8.15 (s, 1H), 7.82 - 7.77 (m, 1H), 7.76 - 7.69 (m, 3H), 7.47 (t, J=9.8 Hz, 1H), 7.39 (dd, J=10.3, 8.7 Hz, 1H), 7.31 (d, J=8.8 Hz, 1H), 5.19 - 5.15 (m, 1H), 5.03 - 4.99 (m, 1H), 4.43 - 4.38 (m, 1H), 4.05 (s, 3H), 3.18 - 3.13 (m, 1H), 3.02 - 2.98 (m, 1H), 2.71 (br s, 1H), 2.62 - 2.55 (m, 1H), 1.91 - 1.85 (m, 1H), 1.84 - 1.73 (m, 3H), 1.67 - 1.59 (m, 2H), 1.59 - 1.51 (m, 2H), 1.43 - 1.35 (m, 2H), 1.30 - 1.19 (m, 2H) | 2.42, C |
| 946 | 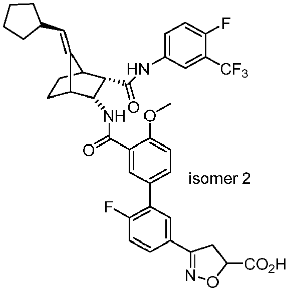 | 3-(3'-{[(1R,2R,3S,4R,7Z)-7-(cyclopentylmethylide ne)-3-{[4-fluoro-3-(trifluoromethyl)pheny l]carbamoyl}bicyclo[2 .2.1]heptan-2-yl]carbamoyl}-6-fluoro-4'-methoxy-[1,1'-biphenyl]-3-yl)-4,5-dihydro-1,2-oxazole-5-carboxylic acid | 738.2 | (500 MHz, DMSO-d6) δ 10.54 (s, 1H), 9.89 (br d, J=7.2 Hz, 1H), 8.26 - 8.23 (m, 1H), 8.17 - 8.14 (m, 1H), 7.82 - 7.71 (m, 4H), 7.48 (t, J=9.7 Hz, 1H), 7.41 (t, J=9.5 Hz, 1H), 7.33 - 7.29 (m, 1H), 5.21 - 5.14 (m, 2H), 4.44 - 4.38 (m, 1H), 4.05 (s, 3H), 3.81 - 3.72 (m, 1H), 3.70 - 3.61 (m, 1H), 3.18 - 3.13 (m, 1H), 3.02 - 2.98 (m, 1H), 2.71 (br s, 1H), 2.63 - 2.56 (m, 1H), 1.91 - 1.73 (m, 4H), 1.69 - 1.60 (m, 2H), 1.60 - 1.51 (m, 2H), 1.43 - 1.35 (m, 2H), 1.32 - 1.20 (m, 2H) | 2.42, C |
| 947 | 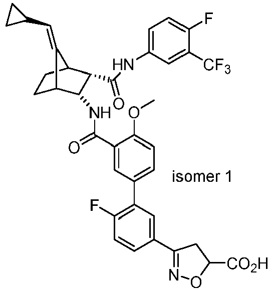 | 3-(3'-{[(1R,2R,3S,4R,7Z)-7-(cyclopropylmethylide ne)-3-{[4-fluoro-3-(trifluoromethyl)pheny l]carbamoyl}bicyclo[2 .2.1]heptan-2-yl]carbamoyl}-6-fluoro-4'-methoxy-[1,1'-biphenyl]-3-yl)-4,5-dihydro-1,2-oxazole-5-carboxylic acid | 710.1 | (500 MHz, DMSO-d6) δ 10.57 - 10.53 (m, 1H), 9.92 (d, J=7.1 Hz, 1H), 8.25 - 8.19 (m, 1H), 8.15 (s, 1H), 7.80 - 7.70 (m, 4H), 7.47 (t, J=9.7 Hz, 1H), 7.40 (dd, J=10.4, 8.7 Hz, 1H), 7.31 (d, J=8.7 Hz, 1H), 5.15 - 5.08 (m, 1H), 4.68 (d, J=9.6 Hz, 1H), 4.50 - 4.41 (m, 1H), 4.08 - 4.03 (m, 3H), 3.77 - 3.69 (m, 1H), 3.19 - 3.08 (m, 2H), 2.74 - 2.69 (m, 1H), 1.89 - 1.75 (m, 2H), 1.54 - 1.34 (m, 3H), 0.78 - 0.65 (m, 2H), 0.38 - 0.29 (m, 2H) | 2.19, C |
| 948 | 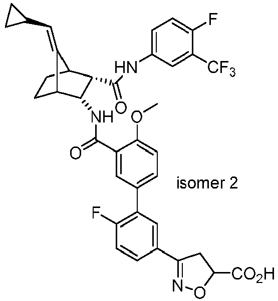 | 3-(3'-{[(1R,2R,3S,4R,7Z)-7-(cyclopropylmethylide ne)-3-{[4-fluoro-3-(trifluoromethyl)pheny l]carbamoyl}bicyclo[2 .2.1]heptan-2-yl]carbamoyl}-6-fluoro-4'-methoxy-[1,1'-biphenyl]-3-yl)-4,5-dihydro-1,2-oxazole-5-carboxylic acid | 710.2 | (500 MHz, DMSO-d6) δ 10.59 - 10.54 (m, 1H), 9.91 (d, J=7.2 Hz, 1H), 8.25 - 8.20 (m, 1H), 8.14 (s, 1H), 7.82 - 7.67 (m, 4H), 7.50 - 7.43 (m, 1H), 7.39 (dd, J=10.4, 8.7 Hz, 1H), 7.32 - 7.29 (m, 1H), 5.07 - 5.00 (m, 1H), 4.69 - 4.65 (m, 1H), 4.48 - 4.42 (m, 1H), 4.08 - 4.02 (m, 3H), 3.72 - 3.52 (m, 1H), 3.19 - 3.14 (m, 1H), 3.13 - 3.07 (m, 1H), 2.73 - 2.69 (m, 1H), 1.92 - 1.75 (m, 2H), 1.53 - 1.35 (m, 3H), 0.78 - 0.67 (m, 2H), 0.38 - 0.29 (m, 2H) | 2.19, C |
| 949 | 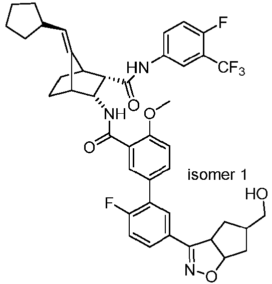 | N-[(1R,2R,3S,4R,7Z)-7-(cyclopentylmethylide ne)-3-{[4-fluoro-3-(trifluoromethyl)pheny l]carbamoyl}bicyclo[2 .2.1]heptan-2-yl]-2'-fluoro-5'-[5-(hydroxymethyl)-3aH,4H,5H,6H,6aH-cyclopenta[d][1,2]oxa zol-3-yl]-4-methoxy-[1,1'-biphenyl]-3-carboxamide | 764.5 | (500 MHz, DMSO-d6) δ 10.52 (s, 1H), 9.89 (d, J=7.0 Hz, 1H), 8.24 (dd, J=6.6, 2.4 Hz, 1H), 8.14 (s, 1H), 7.80 - 7.68 (m, 4H), 7.48 (t, J=9.8 Hz, 1H), 7.40 (dd, J=10.4, 8.7 Hz, 1H), 5.20 - 5.13 (m, 2H), 4.43 - 4.38 (m, 1H), 4.28 (t, J=8.9 Hz, 1H), 4.06 (s, 3H), 3.16 (dd, J=10.7, 4.2 Hz, 1H), 3.00 (br d, J=5.5 Hz, 1H), 2.71 (br s, 1H), 2.63 - 2.56 (m, 1H), 2.02 - 1.74 (m, 8H), 1.70 - 1.62 (m, 3H), 1.57 (td, J=12.5, 5.1 Hz, 3H), 1.42 - 1.36 (m, 2H), 1.31 - 1.21 (m, 3H) | 2.51, C |
| 950 | 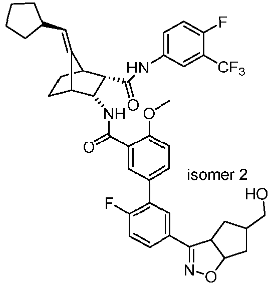 | N-[(1R,2R,3S,4R,7Z)-7-(cyclopentylmethylide ne)-3-{[4-fluoro-3-(trifluoromethyl)pheny l]carbamoyl}bicyclo[2 .2.1]heptan-2-yl]-2'-fluoro-5'-[5-(hydroxymethyl)-3aH,4H,5H,6H,6aH-cyclopenta[d][1,2]oxa zol-3-yl]-4-methoxy-[1,1'-biphenyl]-3-carboxamide | 764.2 | (500 MHz, DMSO-d6) δ 10.53 - 10.51 (m, 1H), 9.89 (d, J=7.1 Hz, 1H), 8.25 - 8.21 (m, 1H), 8.14 (s, 1H), 7.80 - 7.68 (m, 4H), 7.47 (t, J=9.7 Hz, 1H), 7.40 (dd, J=10.5, 8.7 Hz, 1H), 7.34 - 7.30 (m, 1H), 5.21 - 5.15 (m, 2H), 4.40 (ddd, J=10.4, 6.7, 4.2 Hz, 1H), 4.21 - 4.14 (m, 1H), 4.05 (s, 3H), 3.30 - 3.21 (m, 1H), 3.16 (dd, J=10.6, 4.4 Hz, 1H), 3.02 - 2.98 (m, 1H), 2.71 (br s, 1H), 2.63 - 2.56 (m, 1H), 2.25 - 2.10 (m, 3H), 1.91 - 1.86 (m, 1H), 1.84 - 1.73 (m, 3H), 1.68 - 1.60 (m, 3H), 1.59 - 1.53 (m, 2H), 1.52 - 1.46 (m, 1H), 1.43 - 1.35 (m, 2H), 1.31 - 1.21 (m, 2H) | 2.50, C |
| 951 | 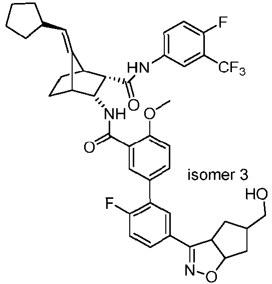 | N-[(1R,2R,3S,4R,7Z)-7-(cyclopentylmethylide ne)-3-{[4-fluoro-3-(trifluoromethyl)pheny l]carbamoyl}bicyclo[2 .2.1]heptan-2-yl]-2'-fluoro-5'-[5-(hydroxymethyl)-3aH,4H,5H,6H,6aH-cyclopenta[d][1,2]oxa zol-3-yl]-4-methoxy-[1,1'-biphenyl]-3-carboxamide | 764.2 | (500 MHz, DMSO-d6) δ 10.53 (s, 1H), 9.92 - 9.87 (m, 1H), 8.26 - 8.22 (m, 1H), 8.16 - 8.13 (m, 1H), 7.81 - 7.77 (m, 1H), 7.76 - 7.69 (m, 3H), 7.48 (t, J=9.8 Hz, 1H), 7.43 - 7.37 (m, 1H), 7.34 - 7.30 (m, 1H), 5.20 - 5.15 (m, 2H), 4.54 - 4.50 (m, 1H), 4.44 - 4.38 (m, 1H), 4.21 - 4.15 (m, 1H), 4.06 (s, 3H), 3.32 - 3.21 (m, 1H), 3.16 (dd, J=10.7, 4.1 Hz, 1H), 3.02 - 2.98 (m, 1H), 2.71 (br s, 1H), 2.63 - 2.56 (m, 1H), 2.26 - 2.09 (m, 3H), 1.91 - 1.73 (m, 4H), 1.68 - 1.61 (m, 3H), 1.60 - 1.52 (m, 2H), 1.49 (dt, J=12.8, 6.4 Hz, 1H), 1.43 - 1.34 (m, 2H), 1.32 - 1.20 (m, 2H) | 2.49, C |
| 952 | 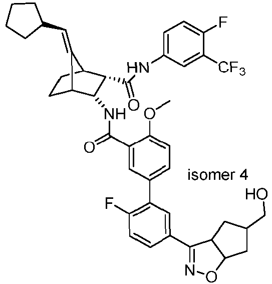 | N-[(1R,2R,3S,4R,7Z)-7-(cyclopentylmethylide ne)-3-{[4-fluoro-3-(trifluoromethyl)pheny l]carbamoyl}bicyclo[2 .2.1]heptan-2-yl]-2'-fluoro-5'-[5-(hydroxymethyl)-3aH,4H,5H,6H,6aH-cyclopenta[d][1,2]oxa zol-3-yl]-4-methoxy-[1,1'-biphenyl]-3-carboxamide | 764.5 | (500 MHz, DMSO-d6) δ 10.54 - 10.51 (m, 1H), 9.89 (d, J=7.2 Hz, 1H), 8.26 - 8.21 (m, 1H), 8.15 - 8.12 (m, 1H), 7.81 - 7.76 (m, 1H), 7.75 - 7.68 (m, 3H), 7.48 (t, J=9.8 Hz, 1H), 7.43 - 7.37 (m, 1H), 7.34 - 7.29 (m, 1H), 5.20 - 5.13 (m, 2H), 4.49 (t, J=5.3 Hz, 1H), 4.44 - 4.38 (m, 1H), 4.28 (t, J=8.8 Hz, 1H), 4.06 (s, 3H), 3.16 (dd, J=10.5, 4.1 Hz, 1H), 3.02 - 2.98 (m, 1H), 2.71 (br s, 1H), 2.63 - 2.56 (m, 1H), 2.03 - 1.75 (m, 7H), 1.70 - 1.61 (m, 3H), 1.60 - 1.52 (m, 3H), 1.41 - 1.36 (m, 2H), 1.32 - 1.20 (m, 2H) | 2.86, C |
| 953 | 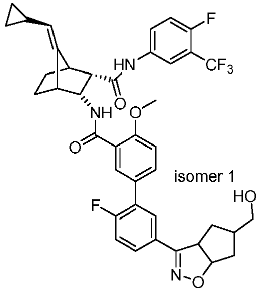 | N-[(1R,2R,3S,4R,7Z)-7-(cyclopropylmethylide ne)-3-{[4-fluoro-3-(trifluoromethyl)pheny l]carbamoyl}bicyclo[2 .2.1]heptan-2-yl]-2'-fluoro-5'-[5-(hydroxymethyl)-3aH,4H,5H,6H,6aH-cyclopenta[d][1,2]oxa zol-3-yl]-4-methoxy-[1,1'-biphenyl]-3-carboxamide | 736.2 | (500 MHz, DMSO-d6) δ 10.53 (s, 1H), 9.93 (d, J=7.1 Hz, 1H), 8.23 (dd, J=6.4, 2.6 Hz, 1H), 8.15 (s, 1H), 7.81 - 7.68 (m, 4H), 7.48 (t, J=9.7 Hz, 1H), 7.40 (dd, J=10.4, 8.7 Hz, 1H), 7.33 (d, J=8.7 Hz, 1H), 5.21 - 5.14 (m, 1H), 4.69 (d, J=9.6 Hz, 1H), 4.52 (t, J=5.3 Hz, 1H), 4.46 (br t, J=10.8 Hz, 1H), 4.18 (td, J=9.5, 6.1 Hz, 1H), 4.06 (s, 3H), 3.32 - 3.21 (m, 1H), 3.17 (dd, J=10.8, 4.1 Hz, 1H), 3.13 - 3.09 (m, 1H), 2.74 - 2.70 (m, 1H), 2.27 - 2.09 (m, 3H), 1.91 - 1.78 (m, 2H), 1.68 - 1.62 (m, 1H), 1.54 - 1.37 (m, 4H), 0.78 - 0.69 (m, 2H), 0.38 - 0.32 (m, 2H) | 2.26, C |
| 954 | 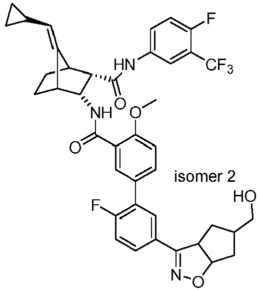 | N-[(1R,2R,3S,4R,7Z)-7-(cyclopropylmethylide ne)-3-{[4-fluoro-3-(trifluoromethyl)pheny l]carbamoyl}bicyclo[2 .2.1]heptan-2-yl]-2'-fluoro-5'-[5-(hydroxymethyl)-3aH,4H,5H,6H,6aH-cyclopenta[d][1,2]oxa zol-3-yl]-4-methoxy-[1,1'-biphenyl]-3-carboxamide | 736.2 | (500 MHz, DMSO-d6) δ 10.54 - 10.50 (m, 1H), 9.95 - 9.90 (m, 1H), 8.23 (dd, J=6.4, 2.6 Hz, 1H), 8.14 (s, 1H), 7.81 - 7.77 (m, 1H), 7.76 - 7.68 (m, 3H), 7.48 (t, J=9.7 Hz, 1H), 7.40 (dd, J=10.5, 8.6 Hz, 1H), 7.32 (d, J=8.8 Hz, 1H), 5.20 - 5.14 (m, 1H), 4.69 (d, J=9.6 Hz, 1H), 4.51 (t, J=5.2 Hz, 1H), 4.49 - 4.44 (m, 1H), 4.18 (td, J=9.7, 6.3 Hz, 1H), 4.06 (s, 3H), 3.32 - 3.21 (m, 1H), 3.16 (dd, J=10.9, 4.1 Hz, 1H), 3.13 - 3.08 (m, 1H), 2.74 - 2.70 (m, 1H), 2.26 - 2.09 (m, 3H), 1.89 - 1.77 (m, 2H), 1.68 - 1.61 (m, 1H), 1.53 - 1.37 (m, 4H), 0.78 - 0.67 (m, 2H), 0.38 - 0.30 (m, 2H) | 2.26, C |
| 955 | 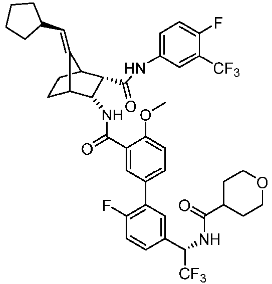 | N-[(1S)-1-(3'-{[(1R,2R,3S,4R,7Z)-7-(cyclopentylmethylide ne)-3-{[4-fluoro-3-(trifluoromethyl)pheny l]carbamoyl}bicyclo[2 .2.1]heptan-2-yl]carbamoyl}-6-fluoro-4'-methoxy-[1,1'-biphenyl]-3-yl)-2,2,2-trifluoroethyl]oxane-4-carboxamide | 834.6 | (500 MHz, DMSO-d6) δ 10.55 (s, 1H), 9.90 (d, J=7.0 Hz, 1H), 9.11 (d, J=9.8 Hz, 1H), 8.25 (dd, J=6.4, 2.3 Hz, 1H), 8.15 (s, 1H), 7.79 (br d, J=7.7 Hz, 2H), 7.70 (br d, J=8.6 Hz, 1H), 7.62 - 7.56 (m, 1H), 7.48 (t, J=9.7 Hz, 1H), 7.39 - 7.30 (m, 2H), 5.93 - 5.83 (m, 1H), 5.19 (d, J=8.9 Hz, 1H), 4.43 - 4.37 (m, 1H), 4.05 (s, 3H), 3.90 - 3.82 (m, 2H), 3.21 - 3.12 (m, 1H), 3.02 - 2.97 (m, 1H), 2.75 - 2.68 (m, 1H), 2.64 - 2.55 (m, 2H), 1.90 - 1.73 (m, 4H), 1.68 - 1.51 (m, 9H), 1.43 - 1.34 (m, 2H), 1.31 - 1.17 (m, 3H) | 2.94, C |
| 956 | 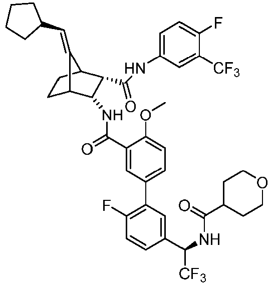 | N-[(1R)-1-(3'-{[(1R,2R,3S,4R,7Z)-7-(cyclopentylmethylide ne)-3-{[4-fluoro-3-(trifluoromethyl)pheny l]carbamoyl}bicyclo[2 .2.1]heptan-2-yl]carbamoyl}-6-fluoro-4'-methoxy-[1,1'-biphenyl]-3-yl)-2,2,2-trifluoroethyl]oxane-4-carboxamide | 834.3 | (500 MHz, DMSO-d6) δ 10.60 - 10.55 (m, 1H), 9.90 (d, J=6.9 Hz, 1H), 9.12 (d, J=9.6 Hz, 1H), 8.25 (dd, J=6.6, 2.4 Hz, 1H), 8.15 (s, 1H), 7.82 - 7.76 (m, 2H), 7.71 (br d, J=8.6 Hz, 1H), 7.61 - 7.56 (m, 1H), 7.48 (t, J=9.7 Hz, 1H), 7.40 - 7.30 (m, 2H), 5.92 - 5.83 (m, 1H), 5.22 - 5.15 (m, 1H), 4.43 - 4.37 (m, 1H), 4.05 (s, 3H), 3.90 - 3.84 (m, 2H), 3.20 - 3.13 (m, 1H), 3.03 - 2.96 (m, 1H), 2.75 - 2.69 (m, 1H), 2.65 - 2.56 (m, 2H), 1.87 - 1.72 (m, 4H), 1.68 - 1.51 (m, 9H), 1.42 - 1.34 (m, 2H), 1.31 - 1.21 (m, 3H) | 2.95, C |
| 957 | 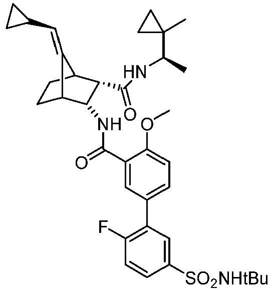 | 5'-(tert-butylsulfamoyl)-N-[(1R,2R,3S,4R,7Z)-7-(cyclopropylmethylide ne)-3-{[(1R)-1-(1-methylcyclopropyl)eth yl]carbamoyl}bicyclo[ 2.2.1]heptan-2-yl]-2'-fluoro-4-methoxy-[1,1'-biphenyl]-3-carboxamide | 652.2 | (400 MHz, DMSO-d6) δ 10.13 (d, J = 6.9 Hz, 1H), 8.11 (d, J = 1.5 Hz, 1H), 7.99 (dd, J = 2.4, 7.3 Hz, 1H), 7.92 (d, J = 8.8 Hz, 1H), 7.84(ddd, J = 2.4, 4.5, 8.5 Hz, 1H), 7.74 (td, J = 1.9, 8.7 Hz, 1H), 7.63 (s, 1H), 7.52 (dd, J = 8.6, 10.6 Hz, 1H), 7.32 (d, J = 8.8 Hz, 1H), 4.64 (d, J = 9.5Hz, 1H), 4.34 - 4.23 (m, 1H), 4.03 (s, 3H), 3.48 (dd, J = 6.9, 8.3 Hz, 1H), 3.07 (br s, 1H), 2.97 (dd, J = 4.1, 10.9 Hz, 1H), 2.54 (br s, 1H), 1.95 -1.83 (m, 1H), 1.82 - 1.70 (m, 1H), 1.53 - 1.43 (m, 1H), 1.36 (br s, 2H), 1.12 (s, 9H), 1.03 (d, J = 6.9 Hz, 3H), 0.99 (s, 3H), 0.72 (dt, J = 2.3, 8.2 Hz,2H), 0.61 - 0.49 (m, 1H), 0.43 - 0.26 (m, 3H), 0.25 - 0.11 (m, 2H) | 2.64, A |
| 958 | 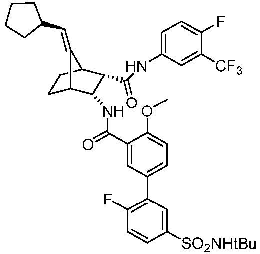 | 5'-(tert-butylsulfamoyl)-N-[(1R,2R,3S,4R,7Z)-7-(cyclopentylmethylide ne)-3-{[4-fluoro-3-(trifluoromethyl)pheny l]carbamoyl}bicyclo[2 .2.1]heptan-2-yl]-2'-fluoro-4-methoxy-[1,1'-biphenyl]-3-carboxamide | 760.3 | (400 MHz, DMSO-d6) δ 10.52 (s, 1H), 9.92 (d, J = 7.1 Hz, 1H), 8.24 (dd, J = 2.4, 6.4 Hz, 1H), 8.16 (s, 1H), 7.98 (dd, J = 2.3, 7.4 Hz, 1H), 7.89 - 7.67 (m, 3H), 7.63 (s, 1H), 7.56 - 7.42 (m, 2H), 7.36 (d, J = 8.8 Hz, 1H), 5.20 (d, J = 9.0 Hz, 1H), 4.47 - 4.29 (m, 1H), 4.07 (s, 3H), 3.19- 3.06 (m, 1H), 3.01 (br s, 1H), 2.72 (br s, 1H), 2.65 - 2.57 (m, 1H), 1.92 - 1.72 (m, 4H), 1.69 - 1.49 (m, 4H), 1.46 - 1.36 (m, 2H), 1.34 - 1.18 (m,2H), 1.12 (s, 9H) | 2.93, A |
| 959 | 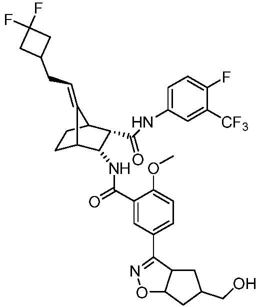 | (1R,2S,3R,4R,7Z)-7-[2-(3,3-difluorocyclobutyl)eth ylidene]-N-[4-fluoro-3-(trifluoromethyl)pheny l]-3-{5-[5-(hydroxymethyl)-3aH,4H,5H,6H,6aH-cyclopenta[d][1,2]oxa zol-3-yl]-2-methoxybenzamido}bi cyclo[2.2.1]heptane-2-carboxamide | 706.5 | (400 MHz, DMSO-d6) δ 10.55 (s, 1H), 9.91 (d, J = 7.1 Hz, 1H), 8.29 - 8.15 (m, 2H), 7.85 - 7.70 (m, 2H), 7.49 (t, J = 9.8 Hz, 1H), 7.27(d, J = 8.8 Hz, 1H), 5.22 (t, J = 7.5 Hz, 1H), 5.12 (dd, J = 5.1, 8.7 Hz, 1H), 4.56 - 4.34 (m, 2H), 4.20 (t, J = 8.8 Hz, 1H), 4.04 (s, 3H), 3.40 (br d, J =5.8 Hz, 1H), 3.18 (br dd, J = 4.2, 6.4 Hz, 1H), 3.00 (br s, 1H), 2.78 - 2.73 (m, 1H), 2.70 - 2.63 (m, 2H), 2.32 - 2.14 (m, 5H), 2.02 - 1.73 (m, 5H),1.70 - 1.54 (m, 2H), 1.39 (br s, 2H) | 2.44, A |
| 960 | 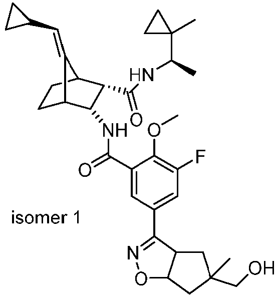 | (1R,2S,3R,4R,7Z)-7-(cyclopropylmethylide ne)-3-{3-fluoro-5-[5-(hydroxymethyl)-5-methyl-3aH,4H,5H,6H,6aH-cyclopenta[d][1,2]oxa zol-3-yl]-2-methoxybenzamido}-N-[(1R)-1-(1-methylcyclopropyl)eth yl]bicyclo[2.2.1]hepta ne-2-carboxamide | 706.5 | (400 MHz, DMSO-d6) δ 10.55 (s, 1H), 9.91 (d, J = 7.1 Hz, 1H), 8.29 - 8.15 (m, 2H), 7.85 - 7.70 (m, 2H), 7.49 (t, J = 9.8 Hz, 1H), 7.27(d, J = 8.8 Hz, 1H), 5.22 (t, J = 7.5 Hz, 1H), 5.12 (dd, J = 5.1, 8.7 Hz, 1H), 4.56 - 4.34 (m, 2H), 4.20 (t, J = 8.8 Hz, 1H), 4.04 (s, 3H), 3.40 (br d, J =5.8 Hz, 1H), 3.18 (br dd, J = 4.2, 6.4 Hz, 1H), 3.00 (br s, 1H), 2.78 - 2.73 (m, 1H), 2.70 - 2.63 (m, 2H), 2.32 - 2.14 (m, 5H), 2.02 - 1.73 (m, 5H),1.70 - 1.54 (m, 2H), 1.39 (br s, 2H) | 2.44, A |
| 961 | 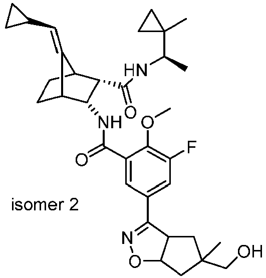 | (1R,2S,3R,4R,7Z)-7-(cyclopropylmethylide ne)-3-{3-fluoro-5-[5-(hydroxymethyl)-5-methyl-3aH,4H,5H,6H,6aH-cyclopenta[d][1,2]oxa zol-3-yl]-2-methoxybenzamido}-N-[(1R)-1-(1-methylcyclopropyl)eth yl]bicyclo[2.2.1]hepta ne-2-carboxamide | 594.3 | (400 MHz, DMSO-d6) δ 10.00 (d, J = 6.8 Hz, 1H), 7.99 (d, J = 8.8 Hz, 1H), 7.93 - 7.83 (m, 1H), 7.69 (dd, J = 2.3, 12.5 Hz, 1H), 5.33 - 5.19 (m, 1H), 4.73 - 4.51 (m, 2H), 4.34 - 4.15 (m, 2H), 4.05 (d, J = 2.6 Hz, 3H), 3.46 (dd, J = 7.0, 8.4 Hz, 1H), 3.18 - 3.12 (m, 2H), 3.07 (br s, 1H), 2.96 (dd, J = 4.3, 10.8 Hz, 1H), 2.55 (br d, J = 3.8 Hz, 1H), 1.93 - 1.72 (m, 5H), 1.71 - 1.62 (m, 1H), 1.53 - 1.31 (m, 3H), 1.02 (d, J = 6.9 Hz, 3H), 0.97 (d, J = 8.8 Hz, 6H), 0.81 - 0.65 (m, 2H), 0.59 - 0.47 (m, 1H), 0.42 - 0.27 (m, 3H), 0.25 - 0.08 (m, 2H) | 2.38, A |
| 962 | 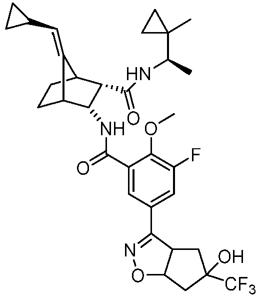 | (1R,2S,3R,4R,7Z)-7-(cyclopropylmethylide ne)-3-{3-fluoro-5-[5-hydroxy-5-(trifluoromethyl)-3aH,4H,5H,6H,6aH-cyclopenta[d][1,2]oxa zol-3-yl]-2-methoxybenzamido}-N-[(1R)-1-(1-methylcyclopropyl)eth yl]bicyclo[2.2.1]hepta ne-2-carboxamide | 717.2 | (400 MHz, DMSO-d6) δ 10.52 (s, 1H), 9.75 (br d, J = 6.9 Hz, 1H), 8.12 (dd, J = 2.4, 6.3 Hz, 1H), 7.99 (br s, 1H), 7.89 - 7.80 (m, 1H),7.78 (br d, J = 12.5 Hz, 1H), 7.48 (t, J = 9.8 Hz, 1H), 5.33 (br s, 2H), 4.77 - 4.30 (m, 4H), 3.97 - 3.43 (m, 2H), 3.17 (dd, J = 4.3, 10.9 Hz, 1H), 3.13- 3.03 (m, 1H), 2.74 (br s, 1H), 1.82 (br d, J = 8.5 Hz, 2H), 1.57 - 1.32 (m, 3H), 1.24 (br s, 6H), 0.83 - 0.65 (m, 2H), 0.50 - 0.26 (m, 2H) | 2.40, A |
| 963 | 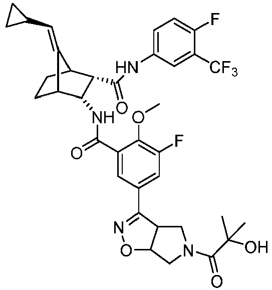 | (1R,2S,3R,4R,7Z)-7-(cyclopropylmethylide ne)-N-[4-fluoro-3-(trifluoromethyl)pheny l]-3-{3-fluoro-5-[5-(2-hydroxy-2-methylpropanoyl)-3aH,4H,5H,6H,6aH-pyrrolo[3,4-d][1,2]oxazol-3-yl]-2-methoxybenzamido}bi cyclo[2.2.1]heptane-2-carboxamide | 634.2 | (400 MHz, DMSO-d6) δ = 9.97 (d, J = 6.8 Hz, 1H), 8.00 (d, J = 8.8 Hz, 1H), 7.91 - 7.81 (m, 1H), 7.70 (dd, J = 2.1, 12.5 Hz, 1H), 5.93 (s,1H), 5.31 (dd, J = 6.4, 9.1 Hz, 1H), 4.65 (d, J = 9.6 Hz, 1H), 4.41 (br t, J = 9.4 Hz, 1H), 4.34 - 4.20 (m, 1H), 4.04 (d, J = 2.4 Hz, 3H), 3.50 - 3.42(m, 1H), 3.07 (t, J = 3.3 Hz, 1H), 2.96 (dd, J = 4.3, 10.9 Hz, 1H), 2.58 - 2.55 (m, 1H), 2.31 - 2.17 (m, 3H), 2.02 (br d, J = 14.0 Hz, 1H), 1.87 - 1.72(m, 2H), 1.55 - 1.28 (m, 3H), 1.07 - 0.93 (m, 6H), 0.80 - 0.63 (m, 2H), 0.51 (br dd, J = 4.0, 8.9 Hz, 1H), 0.43 - 0.26 (m, 3H), 0.24 - 0.05 (m, 2H) | 2.43, A |
| 964 | 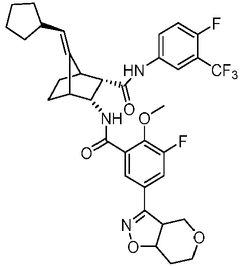 | (1R,2S,3R,4R,7Z)-3-(5-{3aH,4H,6H,7H,7aH-pyrano[3,4-d][1,2]oxazol-3-yl}-3-fluoro-2-methoxybenzamido)-7-(cyclopentylmethylide ne)-N-[4-fluoro-3-(trifluoromethyl)pheny l]bicyclo[2.2.1]heptan e-2-carboxamide | 700.2 | (400 MHz, DMSO-d6) δ 10.54 (s, 1H), 10.06 (br d, J = 6.8 Hz, 1H), 8.73 - 8.49 (m, 2H), 8.24 (dd, J = 2.4, 6.6 Hz, 1H), 7.88 - 7.71 (m,1H), 7.50 (t, J = 9.8 Hz, 1H), 5.31 (br d, J = 6.3 Hz, 2H), 4.71 (d, J = 9.5 Hz, 1H), 4.60 - 4.25 (m, 2H), 4.15 (s, 3H), 3.95 - 3.38 (m, 2H), 3.18 (dd, J= 4.4, 10.5 Hz, 1H), 3.12 (br s, 1H), 2.74 (br s, 1H), 1.89 - 1.66 (m, 2H), 1.57 - 1.35 (m, 3H), 1.32 - 0.97 (m, 6H), 0.84 - 0.66 (m, 2H), 0.47 - 0.26(m, 2H) | 2.30, A |
| 965 | 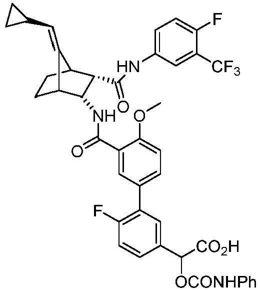 | 2-(3'-{[(1R,2R,3S,4R,7Z)-7-(cyclopropylmethylide ne)-3-{[4-fluoro-3-(trifluoromethyl)pheny l]carbamoyl}bicyclo[2 .2.1]heptan-2-yl]carbamoyl}-6-fluoro-4'-methoxy-[1,1'-biphenyl]-3-yl)-2-[(phenylcarbamoyl)ox y]acetic acid | 790.1 | (400 MHz, CDCl₃) δ 9.73 (br d, J=8.0 Hz, 1H), 8.11 (br s, 1H), 8.07 (s, 1H), 7.96 (dd, J=6.2, 2.5 Hz, 1H), 7.93 (br s, 1H), 7.64 - 7.54 (m, 2H), 7.44 (dt, J=8.7, 3.4 Hz, 1H), 7.37 - 7.29 (m, 3H), 7.19 (t, J=7.9 Hz, 2H), 7.11 - 6.94 (m, 4H), 6.13 (s, 1H), 4.79 - 4.66 (m, 1H), 4.60 (d, J=9.5 Hz, 1H), 4.04 (s, 3H), 3.19 (t, J=3.8 Hz, 1H), 3.05 (dd, J=10.7, 3.9 Hz, 1H), 2.67 (t, J=3.8 Hz, 1H), 2.23 - 2.07 (m, 1H), 1.97 - 1.82 (m, 1H), 1.71 - 1.51 (m, 2H), 1.46 (qd, J=8.6, 4.6 Hz, 1H), 0.82 - 0.63 (m, 2H), 0.40 - 0.26 (m, 2H) | 2.22, A |
| 966 | 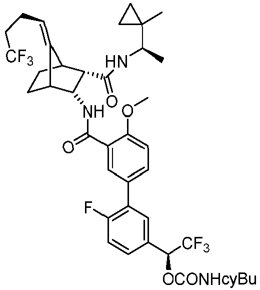 | (1S)-2,2,2-trifluoro-1-(6-fluoro-4'-methoxy-3'-{[(1R,2R,3S,4R,7Z)-3-{[(1R)-1-(1-methylcyclopropyl)eth yl]carbamoyl}-7-(4,4,4-trifluorobutylidene)bic yclo[2.2.1]heptan-2-yl]carbamoyl}-[1,1'-biphenyl]-3-yl)ethyl N-cyclobutylcarbamate | 768.4 | (500 MHz, DMSO-d6) δ 9.95 (br d, J=6.6 Hz, 1H), 7.97 (br d, J=7.7 Hz, 1H), 7.90 (s, 1H), 7.84 - 7.76 (m, 1H), 7.51 (br d, J=6.5 Hz, 2H), 7.37 (br d, J=8.7 Hz, 1H), 7.30 - 7.21 (m, 1H), 7.12 (br d, J=8.8 Hz, 1H), 6.28 - 6.12 (m, 1H), 5.05 (br t, J=6.9 Hz, 1H), 4.16 - 4.04 (m, 1H), 3.84 (s, 3H), 3.80 - 3.69 (m, 1H), 3.24 (br d, J=6.6 Hz, 1H), 2.83 - 2.78 (m, 1H), 2.21 - 2.06 (m, 4H), 2.00 - 1.87 (m, 2H), 1.73 (dt, J=19.7, 9.7 Hz, 3H), 1.46 - 1.34 (m, 2H), 1.22 - 1.11 (m, 2H), 0.91 - 0.78 (m, 6H), 0.43 - 0.33 (m, 1H), 0.20 - 0.12 (m, 1H), 0.07 - -0.07 (m, 2H) | 2.86, C |
| 967 | 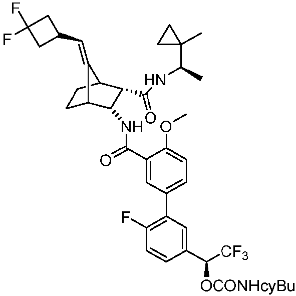 | (1S)-1-(3'-{[(1R,2R,3S,4R,7Z)-7-[(3,3-difluorocyclobutyl)me thylidene]-3-{[(1R)-1-(1-methylcyclopropyl)eth yl]carbamoyl}bicyclo[ 2.2.1]heptan-2-yl]carbamoyl}-6-fluoro-4'-methoxy-[1,1'-biphenyl]-3-yl)-2,2,2-trifluoroethyl N-cyclobutylcarbamate | 762.2 | (500 MHz, DMSO-d6) δ 9.95 (br d, J=6.9 Hz, 1H), 7.97 (d, J=7.9 Hz, 1H), 7.89 (s, 1H), 7.79 (d, J=8.7 Hz, 1H), 7.55 - 7.47 (m, 2H), 7.41 - 7.33 (m, 1H), 7.30 - 7.21 (m, 1H), 7.12 (d, J=8.7 Hz, 1H), 6.17 (br d, J=7.2 Hz, 1H), 5.16 (d, J=8.3 Hz, 1H), 4.13 - 3.99 (m, 1H), 3.84 (s, 3H), 2.85 - 2.57 (m, 5H), 2.41 (br s, 1H), 2.29 - 2.12 (m, 2H), 2.02 - 1.86 (m, 2H), 1.82 - 1.67 (m, 3H), 1.63 - 1.51 (m, 1H), 1.48 - 1.35 (m, 2H), 1.17 (br s, 2H), 0.85 (d, J=6.9 Hz, 3H), 0.81 (s, 3H), 0.36 (br dd, J=9.3, 4.1 Hz, 1H), 0.20 - 0.13 (m, 1H), 0.07 - -0.04 (m, 2H) | 2.93, C |
| 969 | 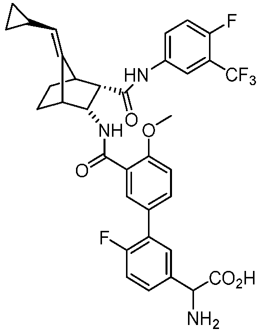 | 2-amino-2-(3'-{[(1R,3S,4R,7Z)-7-(cyclopropylmethylide ne)-3-{[4-fluoro-3-(trifluoromethyl)pheny l]carbamoyl}bicyclo[2 .2.1]heptan-2-yl]carbamoyl}-6-fluoro-4'-methoxy-[1,1'-biphenyl]-3-yl)acetic acid | 670.2 | (500 MHz, DMSO-d6) δ 10.61 (s, 1H), 9.96 (br d, J=7.0 Hz, 1H), 8.23 (dd, J=6.4, 2.3 Hz, 1H), 8.17 (s, 1H), 7.85 - 7.75 (m, 1H), 7.73 - 7.62 (m, 2H), 7.53 - 7.43 (m, 2H), 7.42 - 7.29 (m, 2H), 5.02 (s, 1H), 4.69 (d, J=9.6 Hz, 1H), 4.52 - 4.41 (m, 1H), 4.05 (s, 3H), 3.17 (br dd, J=10.9, 3.9 Hz, 1H), 3.10 (br s, 1H), 2.72 (br s, 1H), 1.90 - 1.75 (m, 2H), 1.54 - 1.35 (m, 3H), 0.80 - 0.67 (m, 2H), 0.34 (br d, J=3.2 Hz, 2H) | 2.14, C |
| 970 | 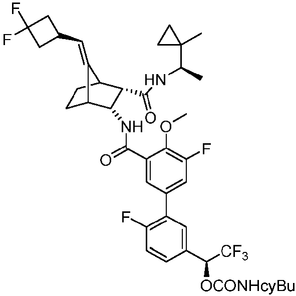 | (1S)-1-(3'-{[(1R,2R,3S,4R,7Z)-7-[(3,3-difluorocyclobutyl)me thylidene]-3-{[(1R)-1-(1-methylcyclopropyl)eth yl]carbamoyl}bicyclo[ 2.2.1]heptan-2-yl]carbamoyl}-5',6-difluoro-4'-methoxy-[1,1'-biphenyl]-3-yl)-2,2,2-trifluoroethyl N-cyclobutylcarbamate | 780.1 | (500 MHz, DMSO-d6) δ 9.85 (br d, J=6.8 Hz, 1H), 7.98 (d, J=7.8 Hz, 1H), 7.85 (d, J=8.9 Hz, 1H), 7.66 (s, 1H), 7.56 (br d, J=6.6 Hz, 1H), 7.50 (br d, J=12.1 Hz, 1H), 7.43 (br s, 1H), 7.29 (dd, J=10.3, 8.7 Hz, 1H), 6.20 (q, J=7.0 Hz, 1H), 5.18 (d, J=8.5 Hz, 1H), 4.19 - 4.04 (m, 1H), 3.90 (d, J=2.1 Hz, 3H), 3.83 - 3.71 (m, 1H), 2.86 - 2.57 (m, 5H), 2.43 (br s, 1H), 2.31 - 2.16 (m, 2H), 2.05 - 1.88 (m, 2H), 1.83 - 1.66 (m, 3H), 1.65 - 1.56 (m, 1H), 1.49 - 1.34 (m, 2H), 1.20 (br d, J=6.3 Hz, 2H), 0.91 - 0.77 (m, 6H), 0.36 (br dd, J=9.3, 3.9 Hz, 1H), 0.22 - 0.12 (m, 1H), 0.08--0.05 (m, 2H) missing 1 proton due to water supression | 2.97, C |
| 971 | 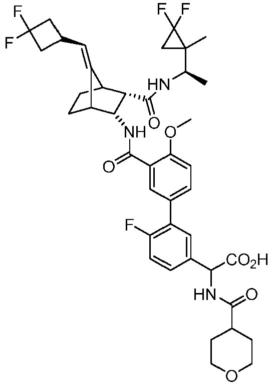 | 2-(3'-{[(1R,2R,3S,4R,7Z)-3-{[(1R)-1-(2,2-difluoro-1-methylcyclopropyl)eth yl]carbamoyl}-7-[(3,3-difluorocyclobutyl)me thylidene]bicyclo[2.2. 1]heptan-2-yl]carbamoyl}-6-fluoro-4'-methoxy-[1,1'-biphenyl]-3-yl)-2-[(oxan-4-yl)formamido]acetic acid | 788.2 | (500 MHz, DMSO-d6) δ 10.09 (d, J=6.8 Hz, 1H), 8.60 (br d, J=7.5 Hz, 1H), 8.31 (br d, J=8.4 Hz, 1H), 8.11 (s, 1H), 7.69 (br d, J=8.7 Hz, 1H), 7.58 - 7.51 (m, 1H), 7.45 - 7.38 (m, 1H), 7.35 - 7.26 (m, 2H), 5.40 (br d, J=7.3 Hz, 1H), 5.35 (d, J=8.4 Hz, 1H), 4.35 - 4.22 (m, 1H), 4.01 (s, 3H), 3.94 - 3.80 (m, 3H), 3.04 - 2.95 (m, 2H), 2.94 - 2.76 (m, 3H), 2.62 (br s, 1H), 2.47 - 2.33 (m, 2H), 1.86 - 1.71 (m, 2H), 1.68 - 1.51 (m, 5H), 1.35 (br d, J=0.6 Hz, 2H), 1.26 - 1.18 (m, 1H), 1.13 (s, 3H), 1.10 (d, J=6.9 Hz, 3H) missing 4 proton due to water supression | 2.36, C |
| 972 | 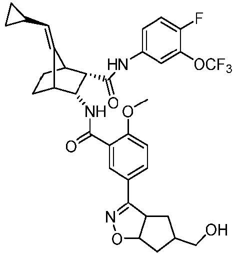 | (2S,3R,7Z)-7-(cyclopropylmethylide ne)-N-[4-fluoro-3-(trifluoromethoxy)phe nyl]-3-{5-[5-(hydroxymethyl)-3aH,4H,5H,6H,6aH-cyclopenta[d][1,2]oxa zol-3-yl]-2-methoxybenzamido}bi cyclo[2.2.1]heptane-2-carboxamide | 658.2 | (500 MHz, DMSO-d6) δ 10.48 (s, 1H), 9.90 (br d, J=7.2 Hz, 1H), 8.23 (d, J=2.3 Hz, 1H), 8.03 (br d, J=6.8 Hz, 1H), 7.79 (dd, J=8.7, 2.3 Hz, 1H), 7.52 - 7.41 (m, 2H), 7.26 (d, J=8.8 Hz, 1H), 5.11 (dd, J=8.9, 5.0 Hz, 1H), 4.68 (d, J=9.5 Hz, 1H), 4.51 (t, J=5.3 Hz, 1H), 4.47 - 4.36 (m, 1H), 4.25 - 4.15 (m, 1H), 4.02 (s, 3H), 3.14 (br dd, J=11.0, 4.2 Hz, 1H), 3.09 (br s, 1H), 2.71 (br s, 1H), 1.98 (br dd, J=13.5, 5.3 Hz, 1H), 1.93 - 1.71 (m, 4H), 1.71 - 1.60 (m, 1H), 1.59 - 1.45 (m, 2H), 1.46 - 1.36 (m, 2H), 0.82 - 0.64 (m, 2H), 0.40 - 0.28 (m, 2H) | 2.46, C |
| 973 | 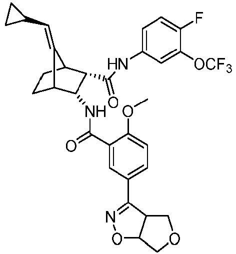 | (2S,3R,7Z)-3-(5-{3aH,4H,6H,6aH-furo[3,4-d][1,2]oxazol-3-yl}-2-methoxybenzamido)-7-(cyclopropylmethylide ne)-N-[4-fluoro-3-(trifluoromethoxy)phe nyl]bicyclo[2.2.1]hept ane-2-carboxamide | 630.1 | (500 MHz, DMSO-d6) δ 10.49 (s, 1H), 9.90 (d, J=7.1 Hz, 1H), 8.28 - 8.15 (m, 1H), 8.04 (br d, J=6.1 Hz, 1H), 7.79 (dd, J=8.6, 2.3 Hz, 1H), 7.51 - 7.42 (m, 2H), 7.30 - 7.22 (m, 1H), 5.34 (dd, J=9.2, 3.4 Hz, 1H), 4.69 (d, J=9.7 Hz, 1H), 4.49 (br t, J=7.7 Hz, 1H), 4.46 - 4.39 (m, 1H), 4.09 (d, J=10.5 Hz, 1H), 4.03 (s, 2H), 4.06 - 4.00 (m, 1H), 3.89 (br d, J=9.2 Hz, 1H), 3.77 (dd, J=9.1, 7.1 Hz, 1H), 3.65 (dd, J=10.5, 3.5 Hz, 1H), 3.15 (dd, J=10.8, 4.2 Hz, 1H), 3.10 (br s, 1H), 2.71 (br s, 1H), 1.87 - 1.69 (m, 2H), 1.49 (td, J=8.4, 4.1 Hz, 1H), 1.44 - 1.32 (m, 2H), 0.80 - 0.66 (m, 2H), 0.34 (br d, J=2.5 Hz, 2H) | 2.47, C |
| 974 | 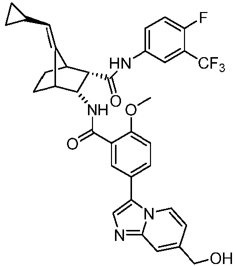 | (2S,3R,7Z)-7-(cyclopropylmethylide ne)-N-[4-fluoro-3-(trifluoromethyl)pheny 1]-3-{5-[7-(hydroxymethyl)imida zo[1,2-a]pyridin-3-yl]-2-methoxybenzamido}bi cyclo[2.2.1]heptane-2-carboxamide | 649.5 | (500 MHz, DMSO-d6) δ 10.54 (s, 1H), 9.95 (d, J=7.2 Hz, 1H), 8.37 (d, J=7.0 Hz, 1H), 8.24 - 8.19 (m, 1H), 8.13 (d, J=2.3 Hz, 1H), 7.81 - 7.74 (m, 2H), 7.65 (s, 1H), 7.51 (s, 1H), 7.47 (t, J=9.7 Hz, 1H), 7.37 (d, J=8.7 Hz, 1H), 6.94 - 6.87 (m, 1H), 4.69 (d, J=9.5 Hz, 1H), 4.56 (br d, J=5.3 Hz, 2H), 4.46 (br dd, J=6.4, 2.8 Hz, 1H), 4.07 (s, 3H), 3.16 (br dd, J=10.8, 3.7 Hz, 1H), 3.10 (br s, 1H), 2.72 (br s, 1H), 1.89 - 1.77 (m, 2H), 1.56 - 1.46 (m, 1H), 1.46 - 1.37 (m, 2H), 0.78 - 0.67 (m, 2H), 0.34 (br d, J=2.1 Hz, 2H) | 2.10, C |

## Claims

1. A compound of Formula (I): 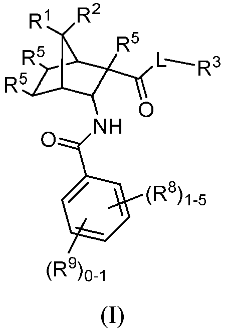 or a pharmaceutically acceptable salt thereof, wherein:
L is -O- or -NH-;
R¹ and R² are combined to be =CR⁶R⁷ or =NOC₁₋₄ alkyl wherein "=" is a double bond;
or R¹ and R² together with the carbon atom to which they are both attached form a dioxolanyl substituted with 0-1 aryl substituent;
R³ is C₁₋₈ alkyl substituted with 0-5 halo, CN, -OH, or -OC₁₋₃ alkyl substituents, - (CR^{d}R^{d})ₙ-C₃₋₁₀-carbocyclyl substituted with 0-5 R⁴, or -(CR^{d}R^{d})ₙ-3- to 12-membered heterocyclyl comprising 1-4 heteroatoms selected from O, S(=O)ₚ, N, and NR^{4c}, and substituted with 0-5 R⁴;
R⁴ is halo, CN, -OH, SF₅, -S(=O)ₚR^{c}, C₁₋₄ alkyl substituted with 0-5 halo, -OH, or -OC₁₋₄ alkyl substituents, -OC₁₋₄ alkyl substituted with 0-5 halo substituents, -(CR^{d}R^{d})ₙ-C₃₋₁₀ carbocyclyl substituted with 0-5 R^{e}, or -(CR^{d}R^{d})ₙ-4- to 6-membered heterocyclyl comprising 1-4 heteroatoms selected from O, S(=O)ₚ, N, and NR^{4c}, and substituted with 0-5 R^{e};
R^{4c} is H, C₁₋₄ alkyl, or -S(=O)₂CF₃;
each R⁵ is H, halo, -OH, C₁₋₄ alkyl substituted with 0-5 halo substituents, or -OC₁₋₄ alkyl substituted with 0-5 halo substituents;
R⁶ is H, halo, CN, C₁₋₇ alkyl substituted with 0-3 R^{6a}, C₂₋₇ alkenyl substituted with 0-3 R^{6a}, C₂₋₇ alkynyl substituted with 0-3 R^{6a}, -C(=O)OR^{6b}, -C(=O)NR^{6b}R^{6b}, -(CH₂)ₙ-C₃₋₁₀ carbocyclyl substituted with 0-5 R¹⁴, or 3- to 12-membered heterocyclyl comprising 1-4 heteroatoms selected from O, S(=O)ₚ, N, or NR^{14a}, and substituted with 0-5 R¹⁴;
R^{6a} is halo, -OH, -OC₁₋₄ alkyl, C₁₋₄ alkyl, aryl, or C₃₋₆ cycloalkyl substituted with 0-4 halo substituents;
R^{6b} is H, C₁₋₄ alkyl substituted with 0-1 aryl substituent, or C₃₋₆ cycloalkyl substituted with 0-4 halo substituents;
R⁷ is H or C₁₋₄ alkyl;
or R⁶ and R⁷ together with the carbon atom to which they are both attached form a cyclopentadienyl, an indanyl, or an indenyl;
R⁸ is H, halo, CN, -NR⁷R⁷, C₁₋₄ alkyl substituted with 0-5 halo or -OH substituents, or - OC₁₋₄ alkyl substituted with 0-5 halo, -OH, C₃₋₆ cycloalkyl, aryl, 4- to 9-membered heterocyclyl comprising 1-4 heteroatoms selected from O, S(=O)ₚ, and N, or -OC₁₋₄ alkyl substituted with 0-1 -OC₁₋₃ alkyl substituents;
R⁹ is aryl substituted with 0-3 R¹⁰ and 0-2 R¹¹ or 3- to 12-membered heterocyclyl comprising 1-5 heteroatoms selected from O, S(=O)ₚ, N, and NR^{11a}, and substituted with 0-3 R¹⁰ and 0-2 R¹¹;
R¹⁰ is halo, CN, C₁₋₄ alkyl, =O, -OH, or -OC₁₋₄ alkyl;
R¹¹ is C₁₋₄ alkyl substituted with 0-4 R¹² and 0-2 R¹³, -OR^{b}, -NR^{a}R^{a}, -NR^{a}C(=O)R^{b}, - NR^{a}C(=O)OR^{b}, -NR^{a}C(=O)NR^{a}R^{a}, -NR^{a}S(=O)ₚR^{c}, -C(=O)R^{b}, -C(=O)OR^{b}, -C(=O)NR^{a}R^{a}, -C(=O)NR^{a}S(=O)ₚR^{c}, -OC(=O)R^{b}, -S(=O)ₚR^{c}, -S(=O)ₚNR^{a}R^{a}, C₃₋₆ carbocyclyl substituted with 0-5 R^{e}, or 3- to 12-membered heterocyclyl comprising 1-4 heteroatoms selected from O, S(=O)ₚ, N, and NR¹⁵, and substituted with 0-5 R^{e};
R^{11a} is H, C₁₋₅ alkyl substituted with 0-4 R^{11b}, -C(=O)R^{b}, -C(=O)OR^{b}, -C(=O)NR^{a}R^{a}, C₃₋₆ cycloalkyl substituted with 0-5 R^{e}, aryl substituted with 0-5 R^{e}, 4- to 6-membered heterocyclyl comprising 1-4 heteroatoms selected from O, S(=O)ₚ, N, and NR¹⁵, and substituted with 0-5 R^{e};
R^{11b} is halo, -OH, -C(=O)OH, -C(=O)OC₁₋₄ alkyl, or aryl;
R¹² is halo, -C(=O)OR^{b}, -C(=O)NR^{a}R^{a}, -C(=O)NR^{a}OR^{b}, or C₁₋₄ alkyl substituted with 0-3 halo or -OH substituents, or C₃₋₆ cycloalkyl;
R¹³ is -OR^{b}, -NR^{a}R^{a}, -NR^{a}C(=O)R^{b}, -NR^{a}C(=O)OR^{b}, -NR^{a}C(=O)NR^{a}R^{a}, -NR^{a}S(=O)ₚR^{c}, -NR^{a}S(=O)ₚNR^{a}R^{a}, -OC(=O)NR^{a}R^{a}, -OC(=O)NR^{a}OR^{b}, -S(=O)ₚNR^{a}R^{a}, - S(=O)ₚR^{c}, -(CH₂)ₙ-C₃₋₁₀ carbocyclyl substituted with 0-3 R^{e}, or -(CH₂)ₙ-3- to 12-membered heterocyclyl comprising 1-4 heteroatoms selected from O, S(=O)ₚ, and N, and substituted with 0-3 R^{e};
R¹⁴ is halo, CN, C₁₋₄ alkyl substituted with 0-3 halo substituents, -OC₁₋₄ alkyl substituted with 0-3 halo substituents, -(CH₂)ₙ-NR^{a}R^{a}, -(CH₂)ₙ-aryl substituted with 0-3 R^{e}, -O-aryl substituted with 0-3 R^{e}, or -(CH₂)ₙ-3- to 12-membered heterocyclyl comprising 1-4 heteroatoms selected from O, S(=O)ₚ, and N, and substituted with 0-3 R^{e};
R^{14a} is H, C(=O)C₁₋₄ alkyl, or C₁₋₃ alkyl substituted with 0-3 Si(C₁₋₃ alkyl)₃ or aryl substituted with 0-2 halo substituents;
R¹⁵ is H, C₁₋₄ alkyl, or aryl;
R^{a} is H, -OC₁₋₆ alkyl, C₁₋₆ alkyl substituted with 0-5 R^{e}, C₂₋₆ alkenyl substituted with 0-5 R^{e}, C₂₋₆ alkynyl substituted with 0-5 R^{e}, -(CH₂)ₙ-C₃₋₁₀ carbocyclyl substituted with 0-5 R^{e}, or -(CH₂)ₙ-3- to 12-membered heterocyclyl comprising 1-4 heteroatoms selected from O, S(=O)ₚ, and N, and substituted with 0-5 R^{e}; or R^{a} and R^{a} together with the nitrogen atom to which they are both attached form a 3- to 12-membered heterocyclyl comprising 1-4 heteroatoms selected from O, S(=O)ₚ, and N, and substituted with 0-5 R^{e};
R^{b} is H, C₁₋₆ alkyl substituted with 0-5 R^{e}, C₂₋₆ alkenyl substituted with 0-5 R^{e}, C₂₋₆ alkynyl substituted with 0-5 R^{e}, -(CH₂)ₙ-C₃₋₁₀ carbocyclyl substituted with 0-5 R^{e}, or -(CH₂)ₙ-3- to 12-membered heterocyclyl comprising 1-4 heteroatoms selected from O, S(=O)ₚ, and N, and substituted with 0-5 R^{e};
R^{c} is C₁₋₆ alkyl substituted with 0-5 R^{e}, C₂₋₆ alkenyl substituted with 0-5 R^{e}, C₂₋₆ alkynyl substituted with 0-5 R^{e}, C₃₋₆ carbocyclyl substituted with 0-5 R^{e}, or 3- to 12-membered heterocyclyl comprising 1-4 heteroatoms selected from O, S(=O)ₚ, and N, and substituted with 0-5 R^{e};
R^{d} is H, C₁₋₄ alkyl, or C₃₋₆ cycloalkyl;
R^{e} is halo, CN, NO₂, =O, C₁₋₆ alkyl substituted with 0-5 R^{g}, C₂₋₆ alkenyl substituted with 0-5 R^{g}, C₂₋₆ alkynyl substituted with 0-5 R^{g}, -(CH₂)ₙ-C₃₋₁₀ carbocyclyl substituted with 0-5 R^{g}, -(CH₂)ₙ-3- to 12-membered heterocyclyl comprising 1-4 heteroatoms selected from O, S(=O)ₚ, and N, and substituted with 0-5 R^{g}, -(CH₂)ₙOR^{f}, - C(=O)OR^{f}, -C(=O)NR^{f}R^{f}, -NR^{f}C(=O)R^{f}, -S(=O)ₚR^{f}, -S(=O)ₚNR^{f}R^{f}, - NR^{f}S(=O)ₚR^{f}, -NR^{f}C(=O)OR^{f}, -OC(=O)NR^{f}R^{f}, or -(CH₂)ₙNR^{f}R^{f};
R^{f} is H, C₁₋₆ alkyl substituted with 0-2 -OH or -OC₁₋₄ alkyl substituents, C₃₋₆ cycloalkyl, aryl, or 3- to 12-membered heterocyclyl comprising 1-4 heteroatoms selected from O, S(=O)ₚ, and N; or R^{f} and R^{f} together with the nitrogen atom to which they are both attached form a 3- to 12-membered heterocyclyl comprising 1-4 heteroatoms selected from O, S(=O)ₚ, and N;
R^{g} is halo, CN, -OH, C₁₋₆ alkyl, C₃₋₆ cycloalkyl, or aryl;
n is zero, 1, 2, or 3; and
p is zero, 1, or 2.

2. The compound of claim 1, or a pharmaceutically acceptable salt thereof, wherein:
R³ is C₁₋₆ alkyl substituted with 0-4 halo or -OH substituents, -(CHR^{d})₀₋₁-C₃₋₆ cycloalkyl substituted with 0-4 R⁴, C₆₋₉ spirocycloalkyl substituted with 0-4 R⁴, C₆₋₁₀ bicyclic carbocyclyl substituted with 0-4 R⁴, or 3 to 6-membered heterocyclyl comprising 1-2 heteroatoms selected from O, S(=O)ₚ, N, and NR^{4c}, and substituted with 0-4 R⁴;
R⁴ is halo or C₁₋₃ alkyl substituted with 0-4 halo substituents;
R^{4c} is H or C₁₋₄ alkyl; and
R^{d} is C₁₋₃ alkyl.

3. The compound of claim 1, having Formula (II): 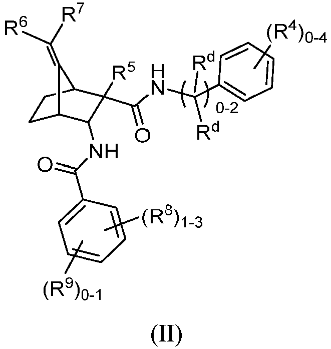 or a pharmaceutically acceptable salt thereof, wherein:
R⁴ is halo, -S(=O)ₚC₁₋₄ alkyl substituted with 0-4 halo substituents, C₁₋₄ alkyl substituted with 0-4 halo substituents, or -OC₁₋₄ alkyl substituted with 0-4 halo substituents;
R⁵ is H or halo;
R⁶ is halo, CN, C₁₋₇ alkyl substituted with 0-3 R^{6a}, C₂₋₇ alkenyl substituted with 0-3 R^{6a}, C₂₋₇ alkynyl substituted with 0-3 R^{6a}, C(=O)OR^{6b}, -C(=O)NR^{6b}R^{6b}, C₃₋₆ cycloalkyl substituted with 0-3 R¹⁴, C₃₋₆ cycloalkenyl substituted with 0-3 R¹⁴, aryl substituted with 0-3 R¹⁴, or 4- to 6-membered heterocyclyl comprising 1-3 heteroatoms selected from O, S(=O)ₚ, N, and NR^{14a} and substituted with 0-3 R¹⁴;
R^{6a} is halo, -OH, C₃₋₆ cycloalkyl, or aryl;
R^{6b} is H, C₁₋₄ alkyl substituted with 0-1 aryl substituent, or C₃₋₆ cycloalkyl substituted with 0-4 halo substituents;
R⁷ is H or C₁₋₃ alkyl;
R⁸ is halo, CN, -N(C₁₋₂ alkyl)₂, C₁₋₄ alkyl substituted with 0-5 halo or -OH substituents, or -OC₁₋₄ alkyl substituted with 0-4 halo, -OH, aryl or -OC₁₋₄ alkyl substituents;
R⁹ is aryl substituted with 0-3 R¹⁰ and 0-2 R¹¹, or 3- to 12-membered heterocyclyl comprising 1-4 heteroatoms selected from O, S(=O)ₚ, N, and NR^{11a}, and substituted with 0-3 R¹⁰ and 0-1 R¹¹;
R¹⁰ is halo, CN, C₁₋₄ alkyl, =O, -OH, or -OC₁₋₄ alkyl;
R¹¹ is C₁₋₄ alkyl substituted with 0-3 R¹² and 0-1 R¹³, -OR^{b}, -NR^{a}R^{a}, -NR^{a}C(=O)R^{b}, - NR^{a}C(=O)OR^{b}, -NR^{a}C(=O)NR^{a}R^{a}, -NR^{a}S(=O)ₚR^{c}, -C(=O)R^{b}, -C(=O)OR^{b}, -C(=O)NR^{a}R^{a}, -C(=O)NR^{a}S(=O)ₚR^{c}, -OC(=O)R^{b}, -S(=O)ₚR^{c}, -S(=O)ₚNR^{a}R^{a}, C₃₋₆ cycloalkyl substituted with 0-5 R^{e}, 4- to 12-membered heterocyclyl comprising 1-4 heteroatoms selected from O, S(=O)ₚ, N, and NR¹⁵, and substituted with 0-5 R^{e};
R^{11a} is H, C₁₋₄ alkyl substituted with 0-2 R^{11b}, -C(=O)R^{b}, -C(=O)OR^{b}, -C(=O)NR^{a}R^{a}, C₃₋₆ cycloalkyl substituted with 0-5 R^{e}, 4- to 6-membered heterocyclyl comprising 1-4 heteroatoms selected from O, S(=O)ₚ, N, and NR¹⁵, and substituted with 0-5 R^{e};
R^{11b} is -OH, -C(=O)OH, or aryl;
R¹² is halo, -C(=O)OR^{b}, -C(=O)NHR^{a}, -C(=O)NHOR^{b}, or C₁₋₄ alkyl substituted with 0-3 halo or -OH substituents;
R¹³ is -OR^{b}, -NR^{a}R^{a}, -NR^{a}C(=O)R^{b}, -NR^{a}C(=O)OR^{b}, -NR^{a}S(=O)ₚR^{c}, -NR^{a}S(=O)ₚNR^{a}R^{a}, -OC(=O)NR^{a}R^{a}, -OC(=O)NR^{a}OR^{b}, -S(=O)ₚNR^{a}R^{a}, or -S(=O)ₚR^{c};
R¹⁴ is halo, CN, C₁₋₄ alkyl substituted with 0-3 halo substituents, -OC₁₋₄ alkyl substituted with 0-3 halo substituents, -(CH₂)₀₋₃-NR^{a}R^{a}, -(CH₂)₀₋₃-aryl substituted with 0-3 R^{e}, -O-aryl substituted with 0-3 R^{e}, or -(CH₂)₀₋₂-3- to 12-membered heterocyclyl comprising 1-4 heteroatoms selected from O, S(=O)ₚ, and N, and substituted with 0-3 R^{e};
R^{14a} is H, C(=O)C₁₋₄ alkyl, or C₁₋₃ alkyl substituted with 0-3 aryl substituted with 0-2 halo substituents;
R¹⁵ is H, C₁₋₃ alkyl, or aryl;
R^{a} is H, C₁₋₅ alkyl substituted with 0-5 R^{e}, C₂₋₅ alkenyl substituted with 0-5 R^{e}, C₂₋₅ alkynyl substituted with 0-5 R^{e}, -(CH₂)ₙ-C₃₋₁₀ carbocyclyl substituted with 0-5 R^{e}, or -(CH₂)ₙ-3- to 12-membered heterocyclyl comprising 1-4 heteroatoms selected from O, S(=O)ₚ, and N, and substituted with 0-5 R^{e}; or R^{a} and R^{a} together with the nitrogen atom to which they are both attached form a 3- to 12-membered heterocyclyl comprising 1-4 heteroatoms selected from O, S(=O)ₚ, and N, and substituted with 0-5 R^{e};
R^{b} is H, C₁₋₅ alkyl substituted with 0-5 R^{e}, C₂₋₅ alkenyl substituted with 0-5 R^{e}, C₂₋₅ alkynyl substituted with 0-5 R^{e}, -(CH₂)ₙ-C₃₋₁₀ carbocyclyl substituted with 0-5 R^{e}, or -(CH₂)ₙ-3- to 12-membered heterocyclyl comprising 1-4 heteroatoms selected from O, S(=O)ₚ, and N, and substituted with 0-5 R^{e};
R^{c} is C₁₋₅ alkyl substituted with 0-5 R^{e}, C₂₋₅ alkenyl substituted with 0-5 R^{e}, C₂₋₅ alkynyl substituted with 0-5 R^{e}, C₃₋₆ carbocyclyl substituted with 0-5 R^{e}, or 3- to 12-membered heterocyclyl comprising 1-4 heteroatoms selected from O, S(=O)ₚ, and N, and substituted with 0-5 R^{e};
R^{d} is H or C₁₋₄ alkyl;
R^{e} is halo, CN, =O, C₁₋₆ alkyl substituted with 0-5 R^{g}, C₂₋₆ alkenyl substituted with 0-5 R^{g}, C₂₋₆ alkynyl substituted with 0-5 R^{g}, -(CH₂)ₙ-C₃₋₆ cycloalkyl substituted with 0-4 R^{g}, -(CH₂)ₙ-aryl substituted with 0-4 R^{g}, -(CH₂)ₙ-4- to 6-membered heterocyclyl comprising 1-4 heteroatoms selected from O, S(=O)ₚ, and N, and substituted with 0-4 R^{g}, -(CH₂)ₙOR^{f}, -C(=O)OR^{f}, -C(=O)NR^{f}R^{f}, -NR^{f}C(=O)R^{f}, - S(=O)ₚR^{f}, -NR^{f}C(=O)OR^{f}, -OC(=O)NR^{f}R^{f}, or -(CH₂)ₙNR^{f}R^{f};
R^{f} is H, C₁₋₅ alkyl, C₃₋₆ cycloalkyl, or aryl; or R^{f} and R^{f} together with the nitrogen atom to which they are both attached form a 3- to 9-membered heterocyclyl;
R^{g} is halo, CN, -OH, C₁₋₅ alkyl, C₃₋₆ cycloalkyl, or aryl;
n is zero, 1, 2, or 3; and
p is zero, 1, or 2.

4. The compound of claim 3, having Formula (IV): 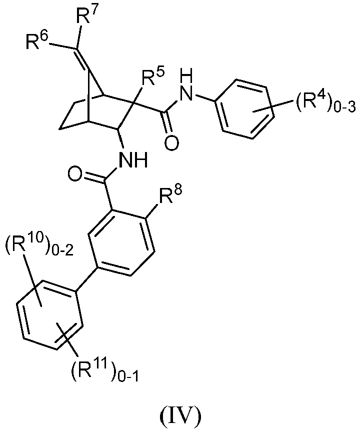 or a pharmaceutically acceptable salt thereof, wherein:
R⁴ is halo, C₁₋₄ alkyl substituted with 0-3 halo substituents, or -OC₁₋₄ alkyl substituted with 0-3 halo substituents;
R⁵ is H or F;
R⁶ is halo, CN, C₁₋₆ alkyl substituted with 0-3 R^{6a}, C₂₋₆ alkenyl substituted with 0-3 R^{6a}, C₂₋₆ alkynyl substituted with 0-3 R^{6a}, -C(=O)OR^{6b}, C(=O)NR^{6b}R^{6b}, C₃₋₆ cycloalkyl substituted with 0-3 R¹⁴, C₃₋₆ cycloalkenyl substituted with 0-3 R¹⁴, phenyl substituted with 0-3 R¹⁴, or 5- to 6-membered heteroaryl comprising 1-3 heteroatoms selected from O, S(=O)ₚ, N, and NR^{14a}, and substituted with 0-3 R¹⁴;
R^{6a} is halo, C₃₋₆ cycloalkyl, or phenyl;
R^{6b} is H, C₁₋₃ alkyl substituted with 0-1 aryl substituent, or C₃₋₆ cycloalkyl substituted with 0-4 halo substituents;
R⁷ is H or C₁₋₂ alkyl;
R⁸ is -OC₁₋₄ alkyl substituted with 0-4 halo, -OH, aryl, or -OC₁₋₄ alkyl substituents;
R¹⁰ is halo, CN, C₁₋₃ alkyl, -OH, or -OC₁₋₄ alkyl;
R¹¹ is C₁₋₄ alkyl substituted with 0-2 R¹² and 0-1 R¹³, -OR^{b}, -NR^{a}R^{a}, -NR^{a}C(=O)R^{b}, - NR^{a}C(=O)NR^{a}R^{a}, -NR^{a}S(=O)ₚR^{c}, -C(=O)R^{b}, -C(=O)OR^{b}, -C(=O)NR^{a}R^{a}, -C(=O)NR^{a}S(=O)ₚR^{c}, -OC(=O)R^{b}, -S(=O)ₚR^{c}, -S(=O)ₚNR^{a}R^{a}, C₃₋₆ cycloalkyl, 4-to 9-membered heterocyclyl comprising 1-4 heteroatoms selected from O, S(=O)ₚ, N, and NR¹⁵, and substituted with 0-4 R^{e};
R¹² is halo, -C(=O)OR^{b}, -C(=O)NHR^{a}, -C(=O)NHOR^{b}, or C₁₋₄ alkyl substituted with 0-3 halo or -OH substituents;
R¹³ is -OR^{b}, -NR^{a}R^{a}, -NR^{a}C(=O)R^{b}, -NR^{a}C(=O)OR^{b}, -NR^{a}S(=O)ₚR^{c}, -NR^{a}S(=O)ₚNR^{a}R^{a}, -OC(=O)NR^{a}R^{a}, -OC(=O)NR^{a}OR^{b}, -S(=O)ₚNR^{a}R^{a}, or -S(=O)ₚR^{c};
R¹⁴ is halo, CN, C₁₋₄ alkyl substituted with 0-3 halo substituents, -OC₁₋₄ alkyl substituted with 0-3 halo substituents, -(CH₂)₀₋₂-NR^{a}R^{a}, -(CH₂)₀₋₂-aryl substituted with 0-3 R^{e}, -O-aryl substituted with 0-3 R^{e}, or -(CH₂)₀₋₂-3- to 12-membered heterocyclyl comprising 1-4 heteroatoms selected from O, S(=O)ₚ, and N, and substituted with 0-3 R^{e};
R^{14a} is H, C(=O)C₁₋₃alkyl, C₁₋₃ alkyl substituted with 0-2 aryl substituted with 0-2 halo substituents;
R¹⁵ is H, C₁₋₂ alkyl, or phenyl;
R^{a} is H, C₁₋₅ alkyl substituted with 0-4 R^{e}, C₂₋₅ alkenyl substituted with 0-4 R^{e}, C₂₋₅ alkynyl substituted with 0-4 R^{e}, -(CH₂)ₙ-C₃₋₁₀ carbocyclyl substituted with 0-4 R^{e}, or -(CH₂)ₙ-3- to 12-membered heterocyclyl comprising 1-4 heteroatoms selected from O, S(=O)ₚ, and N, and substituted with 0-4 R^{e}; or R^{a} and R^{a} together with the nitrogen atom to which they are both attached form a 3- to 12-membered heterocyclyl comprising 1-4 heteroatoms selected from O, S(=O)ₚ, and N, and substituted with 0-4 R^{e};
R^{b} is H, C₁₋₅ alkyl substituted with 0-4 R^{e}, C₂₋₅ alkenyl substituted with 0-4 R^{e}, C₂₋₅ alkynyl substituted with 0-4 R^{e}, -(CH₂)ₙ-C₃₋₁₀ carbocyclyl substituted with 0-4 R^{e}, or -(CH₂)ₙ-3- to 12-membered heterocyclyl comprising 1-4 heteroatoms selected from O, S(=O)ₚ, and N, and substituted with 0-4 R^{e};
R^{c} is C₁₋₅ alkyl substituted with 0-4 R^{e}, C₂₋₅ alkenyl substituted with 0-4 R^{e}, C₂₋₅ alkynyl substituted with 0-4 R^{e}, C₃₋₆ carbocyclyl, or 3- to 12-membered heterocyclyl comprising 1-4 heteroatoms selected from O, S(=O)ₚ, and N;
R^{e} is halo, CN, NO₂, =O, C₁₋₆ alkyl substituted with 0-5 R^{g}, C₂₋₆ alkenyl substituted with 0-5 R^{g}, C₂₋₆ alkynyl substituted with 0-5 R^{g}, -(CH₂)ₙ-C₃₋₆ cycloalkyl, - (CH₂)ₙ-aryl, -(CH₂)ₙ- 4- to 6-membered heterocyclyl comprising 1-4 heteroatoms selected from O, S(=O)ₚ, and N, -(CH₂)ₙOR^{f}, S(=O)ₚR^{f}, C(=O)NR^{f}R^{f}, C(=O)OR^{f}, NR^{f}C(=O)R^{f}, S(=O)ₚNR^{f}R^{f}, NR^{f}S(=O)ₚR^{f}, NR^{f}C(=O)OR^{f}, OC(=O)NR^{f}R^{f},
or -(CH₂)ₙNR^{f}R^{f};
R^{f} is H, C₁₋₆ alkyl, C₃₋₆ cycloalkyl, or aryl; or R^{f} and R^{f} together with the nitrogen atom to which they are both attached form a heterocyclyl;
R^{g} is halo, CN, -OH, C₁₋₅ alkyl, C₃₋₆ cycloalkyl, or aryl;
n is zero, 1, 2, or 3; and
p is zero, 1, or 2.

5. The compound of claim 1, having Formula (V): 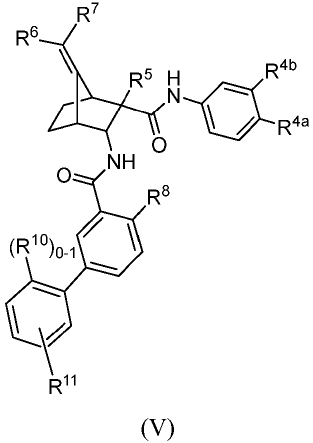 or a pharmaceutically acceptable salt thereof, wherein:
R^{4a} is halo or C₁₋₂ alkyl;
R^{4b} is C₁₋₄ alkyl substituted with 0-4 halo substituents;
R⁵ is H or F;
R⁶ is halo, CN, C₁₋₄ alkyl substituted with 0-3 R^{6a}, C₂₋₄ alkenyl substituted with 0-3 R^{6a}, - C(=O)OR^{6b}, C(=O)NR^{6b}R^{6b}, C₃₋₆ cycloalkyl substituted with 0-3 R¹⁴, phenyl substituted with 0-3 R¹⁴, or 5- to 6-membered heteroaryl comprising 1-3 heteroatoms selected from O, S(=O)ₚ, N, and NR^{14a} and substituted with 0-3 R¹⁴;
R^{6a} is halo, -OH, C₃₋₆ cycloalkyl, or phenyl;
R^{6b} is H, C₁₋₃ alkyl substituted with 0-1 aryl substituent, or C₃₋₆ cycloalkyl;
R⁷ is H or C₁₋₂ alkyl;
R⁸ is -OC₁₋₄alkyl substituted with 0-4 halo, -OH, -OC₁₋₄ alkyl, or aryl substituents;
R¹⁰ is halo or C₁₋₃ alkyl;
R¹¹ is C₁₋₄ alkyl substituted with 0-2 R¹² and 0-1 R¹³, -OH, -OC₁₋₄ alkyl, -NR^{a}C(=O)R^{b}, - NR^{a}C(=O)NR^{a}R^{a}, -NR^{a}S(=O)ₚR^{c}, -C(=O)R^{b}, -C(=O)OR^{b}, -C(=O)NR^{a}R^{a}, -C(=O)NR^{a}S(=O)ₚR^{c}, -OC(=O)R^{b}, -S(=O)ₚR^{c}, -S(=O)ₚNR^{a}R^{a}, C₃₋₆ cycloalkyl, 4-to 9-membered heterocyclyl comprising 1-4 heteroatoms selected from O, S(=O)ₚ, N, and NR¹⁵, and substituted with 0-3 R^{e};
R¹² is halo, -C(=O)OR^{b}, -C(=O)NHR^{a}, -C(=O)NHOR^{b}, or C₁₋₄ alkyl substituted with 0-3 halo or -OH substituents;
R¹³ is -OR^{b}, -NR^{a}R^{a}, -NR^{a}C(=O)R^{b}, -NR^{a}C(=O)OR^{b}, -NR^{a}S(=O)ₚR^{c}, -NR^{a}S(=O)ₚNR^{a}R^{a}, -OC(=O)NR^{a}R^{a}, or -OC(=O)NR^{a}OR^{b};
R¹⁴ is halo, CN, C₁₋₄ alkyl substituted with 0-3 halo substituents, -OC₁₋₄ alkyl substituted with 0-3 halo substituents, -(CH₂)₀₋₂-NR^{a}R^{a}, -(CH₂)₀₋₁-aryl substituted with 0-3 R^{e}, -O-aryl substituted with 0-3 R^{e}, or -(CH₂)₀₋₁-3- to 9-membered heterocyclyl comprising 1-4 heteroatoms selected from O, S(=O)ₚ, and N, and substituted with 0-3 R^{e};
R^{14a} is H, C(=O)C₁₋₃ alkyl, C₁₋₃ alkyl substituted with 0-1 aryl substituted with 0-2 halo substituents;
R¹⁵ is H, C₁₋₂ alkyl, or phenyl;
R^{a} is H, C₁₋₄ alkyl substituted with 0-5 R^{e}, C₂₋₄ alkenyl substituted with 0-5 R^{e}, C₂₋₄ alkynyl substituted with 0-5 R^{e}, -(CH₂)ₙ-C₃₋₁₀ carbocyclyl substituted with 0-5 R^{e}, or -(CH₂)ₙ-3- to 12-membered heterocyclyl comprising 1-4 heteroatoms selected from O, S(=O)ₚ, and N, and substituted with 0-5 R^{e}; or R^{a} and R^{a} together with the nitrogen atom to which they are both attached form a 3- to 9-membered heterocyclyl comprising 1-4 heteroatoms selected from O, S(=O)ₚ, and N, and substituted with 0-5 R^{e};
R^{b} is H, C₁₋₄ alkyl substituted with 0-5 R^{e}, C₂₋₄ alkenyl substituted with 0-5 R^{e}, C₂₋₄ alkynyl substituted with 0-5 R^{e}, -(CH₂)ₙ-C₃₋₁₀carbocyclyl substituted with 0-5 R^{e}, or -(CH₂)ₙ-3- to 12-membered heterocyclyl comprising 1-4 heteroatoms selected from O, S(=O)ₚ, and N, and substituted with 0-5 R^{e};
R^{c} is C₁₋₄ alkyl substituted with 0-5 R^{e}, C₂₋₄ alkenyl substituted with 0-5 R^{e}, C₂₋₄ alkynyl substituted with 0-5 R^{e}, C₃₋₆ carbocyclyl, or 3- to 9-membered heterocyclyl comprising 1-4 heteroatoms selected from O, S(=O)ₚ, and N;
R^{e} is halo, CN, =O, C₁₋₆ alkyl substituted with 0-5 R^{g}, C₂₋₆ alkenyl substituted with 0-5 R^{g}, C₂₋₆ alkynyl substituted with 0-5 R^{g}, -(CH₂)ₙ-C₃₋₆ cycloalkyl,-(CH₂)ₙ-aryl, -(CH₂)ₙ- 4- to 6-membered heterocyclyl comprising 1-4 heteroatoms selected from O, S(=O)ₚ, and N, -(CH₂)ₙOR^{f}, -S(=O)ₚR^{f}, -C(=O)NR^{f}R^{f}, - C(=O)OR^{f}, -NR^{f}C(=O)R^{f}, -S(=O)ₚNR^{f}R^{f}, -NR^{f}S(=O)ₚR^{f}, -NR^{f}C(=O)OR^{f}, - OC(=O)NR^{f}R^{f}, or -(CH₂)ₙNR^{f}R^{f};
R^{f} is H, C₁₋₆ alkyl, C₃₋₆ cycloalkyl, or aryl; or R^{f} and R^{f} together with the nitrogen atom to which they are both attached form a heterocyclyl;
R^{g} is halo CN, -OH, C₁₋₆ alkyl, C₃₋₆ cycloalkyl, or aryl;
n is zero, 1, 2, or 3; and
p is zero, 1, or 2.

6. The compound of claim 1, having Formula (VI): 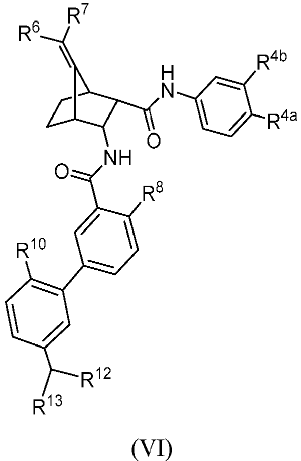 or a pharmaceutically acceptable salt thereof, wherein:
R^{4a} is halo;
R^{4b} is CF₃;
R⁶ is C₁₋₄ alkyl substituted with 0-3 halo substituents or C₃₋₆ cycloalkyl substituted with 0-3 halo substituents;
R⁷ is H;
R⁸ is -OC₁₋₄alkyl substituted with 0-1 aryl substituent;
R¹⁰ is halo;
R¹² is -C(=O)OH, -C(=O)OC₁₋₄ alkyl, -C(=O)NHC₁₋₄ alkyl, -C(=O)NHOC₁₋₃ alkyl, or C₁₋₃ alkyl substituted with 0-3 halo substituents;
R¹³ is -OR^{b}, -NR^{a}R^{a}, -NR^{a}C(=O)R^{b}, -NR^{a}C(=O)OR^{b}, -NR^{a}S(=O)ₚR^{c}, -NR^{a}S(=O)ₚNR^{a}R^{a}, -OC(=O)NR^{a}R^{a}, or -OC(=O)NR^{a}OR^{b};
R^{a} is H, C₁₋₄ alkyl substituted with 0-5 halo substituents, phenyl substituted with 0-4 R^{e}, C₃₋₁₀ cycloalkyl substituted with 0-4 R^{e}, spirocycloalkyl substituted with 0-4 R^{e}, or 3- to 9-membered heterocyclyl comprising 1-4 heteroatoms selected from O, S(=O)ₚ, and N, and substituted with 0-4 R^{e}; or R^{a} and R^{a} together with the nitrogen atom to which they are both attached form a 3- to 12-membered heterocyclyl comprising 1-4 heteroatoms selected from O, S(=O)ₚ, and N, and substituted with 0-4 R^{e};
R^{b} is H, C₁₋₄ alkyl substituted with 0-5 R^{e}, -(CH₂)ₙ-phenyl substituted with 0-4 halo substituents, C₃₋₆ cycloalkyl substituted with 0-4 halo substituents, or 3- to 12-membered heterocyclyl comprising 1-4 heteroatoms selected from O, S(=O)ₚ, and N, and substituted with 0-4 R^{e};
R^{c} is C₁₋₄ alkyl substituted with 0-4 R^{e},
R^{e} is halo, CN, =O, C₁₋₅ alkyl substituted with 0-5 R^{g}, C₃₋₆ cycloalkyl, aryl, 4- to 6-membered heterocyclyl comprising 1-4 heteroatoms selected from O, S(=O)ₚ, and N, or -OR^{f};
R^{f} is H, C₁₋₄ alkyl, C₃₋₆ cycloalkyl, or aryl;
R^{g} is halo;
n is zero or 1; and
p is zero, 1, or 2.

7. The compound of claim 6 or a pharmaceutically acceptable salt thereof, wherein:
R^{4a} is F
R^{4b} is CF₃;
R⁶ is CF₃ or C₃₋₆ cycloalkyl;
R⁸ is -OCH₃ or -OCH₂-phenyl;
R¹⁰ is F;
R¹² is -C(=O)OH, -C(=O)OC₁₋₄ alkyl, -C(=O)NHC₁₋₄ alkyl, -C(=O)NHOC₁₋₃ alkyl, CH₃, CHF₂, or CF₃;
R¹³ is -OH, -NR^{a}R^{a}, -NHC(=O)R^{b}, -NHS(=O)ₚC₁₋₄ alkyl, -OC(=O)NR^{a}R^{a}, or - OC(=O)NHOC₁₋₄ alkyl;
R^{a} is H, C₁₋₄ alkyl substituted with 0-4 F substituents, 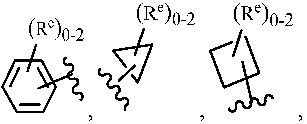 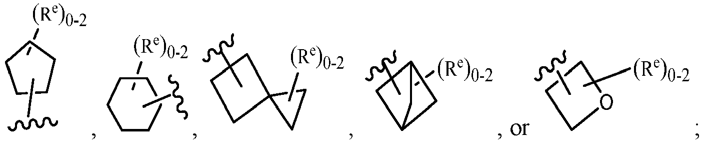
or R^{a} and R^{a} together is 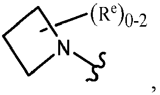
R^{b} is H, C₁₋₄ alkyl substituted with 0-5 R^{e}, phenyl, or 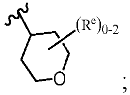 and
R^{e} is halo, =O, aryl, 4- to 6-membered heterocyclyl comprising 1-4 heteroatoms selected from O, S(=O)ₚ, and N, or -OR^{f}; and
R^{f} is H, C₁₋₃ alkyl, C₃₋₆ cycloalkyl, or phenyl.

8. The compound of claim 3, having Formula (VII): 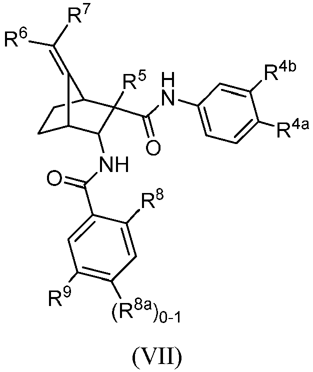 or a pharmaceutically acceptable salt thereof, wherein:
R^{4a} is halo;
R^{4b} is C₁₋₄ alkyl substituted with 0-3 halo substituents, or -OC₁₋₄ alkyl substituted with 0-3 halo substituents;
R⁵ is H or F;
R⁶ is halo, CN, C₁₋₆ alkyl substituted with 0-3 R^{6a}, C₂₋₆ alkenyl substituted with 0-3 R^{6a}, C₂₋₆ alkynyl substituted with 0-3 R^{6a}, C₃₋₆ cycloalkyl substituted with 0-3 R¹⁴, C₃₋₆ cycloalkenyl substituted with 0-3 R¹⁴, phenyl substituted with 0-3 R¹⁴, or 5- to 6-membered heteroaryl comprising 1-3 heteroatoms selected from O, S(=O)ₚ, N, and NR^{14a}, and substituted with 0-3 R¹⁴;
R^{6a} is halo, C₃₋₆ cycloalkyl, or phenyl;
R⁷ is H or C₁₋₂ alkyl;
R⁸ is halo, CN, or -OC₁₋₄ alkyl substituted with 0-4 halo, -OH, or -OC₁₋₄ alkyl substituents;
R^{8a} is halo or CN;
R⁹ is a 3- to 12-membered heterocyclyl comprising 1-4 heteroatoms selected from O, S(=O)ₚ, N, and NR^{11a}, and substituted with 0-3 R¹⁰ and 0-1 R¹¹;
R¹⁰ is halo, CN, C₁₋₃ alkyl, =O, -OH, or -OC₁₋₃ alkyl;
R¹¹ is C₁₋₃ alkyl substituted with 0-1 R¹² and 0-1 R¹³, -OR^{b}, -NR^{a}R^{a}, -NR^{a}C(=O)R^{b}, - NR^{a}C(=O)OR^{b}, -NR^{a}C(=O)NR^{a}R^{a}, -NR^{a}S(=O)ₚR^{c}, -C(=O)R^{b}, -C(=O)OR^{b}, -C(=O)NR^{a}R^{a}, -C(=O)NR^{a}S(=O)ₚR^{c}, -OC(=O)R^{b}, -S(=O)ₚR^{c}, -S(=O)ₚNR^{a}R^{a}, C₃₋₆ cycloalkyl substituted with 0-5 R^{e}, 4- to 6-membered heterocyclyl comprising 1-4 heteroatoms selected from O, S(=O)ₚ, N, and NR¹⁵, and substituted with 0-4 R^{e};
R^{11a} is H, C₁₋₄ alkyl substituted with 0-2 R^{11b}, -C(=O)R^{b}, -C(=O)OR^{b}, -C(=O)NR^{a}R^{a}, C₃₋₆ cycloalkyl, 4- to 6-membered heterocyclyl comprising 1-4 heteroatoms selected from O, S(=O)ₚ, N, and NR¹⁵, and substituted with 0-4 R^{e};
R^{11b} is -OH, -C(=O)OH, or aryl;
R¹² is -C(=O)OR^{b}, -C(=O)NHR^{a}, -C(=O)NHOR^{b}, or C₁₋₄ alkyl substituted with 0-3 halo or -OH substituents;
R¹³ is -OR^{b}, -NR^{a}R^{a}, -NR^{a}C(=O)R^{b}, -NR^{a}C(=O)OR^{b}, -NR^{a}S(=O)ₚR^{c}, -NR^{a}S(=O)ₚNR^{a}R^{a}, -OC(=O)NR^{a}R^{a}, -S(=O)ₚNR^{a}R^{a}, or -S(=O)ₚR^{c};
R¹⁴ is halo, CN, C₁₋₄ alkyl substituted with 0-3 halo substituents, -OC₁₋₄ alkyl substituted with 0-3 halo substituents, -(CH₂)₀₋₂-NR^{a}R^{a}, -(CH₂)₀₋₂-aryl substituted with 0-3 R^{e}, -O-aryl substituted with 0-3 R^{e}, or -(CH₂)₀₋₂-3- to 12-membered heterocyclyl comprising 1-4 heteroatoms selected from O, S(=O)ₚ, and N, and substituted with 0-3 R^{e};
R^{14a} is H, C(=O)C₁₋₃ alkyl, or C₁₋₃ alkyl substituted with 0-2 aryl substituted with 0-2 halo substituents;
R¹⁵ is H, C₁₋₂ alkyl, or phenyl;
R^{a} is H, C₁₋₅ alkyl substituted with 0-4 R^{e}, C₂₋₅ alkenyl substituted with 0-4 R^{e}, C₂₋₅ alkynyl substituted with 0-4 R^{e}, -(CH₂)ₙ-C₃₋₁₀ carbocyclyl substituted with 0-4 R^{e}, or -(CH₂)ₙ-3- to 12-membered heterocyclyl comprising 1-4 heteroatoms selected from O, S(=O)ₚ, and N, and substituted with 0-4 R^{e}; or R^{a} and R^{a} together with the nitrogen atom to which they are both attached form a 3- to 12-membered heterocyclyl comprising 1-4 heteroatoms selected from O, S(=O)ₚ, and N, and substituted with 0-4 R^{e};
R^{b} is H, C₁₋₅ alkyl substituted with 0-4 R^{e}, C₂₋₅ alkenyl substituted with 0-4 R^{e}, C₂₋₅ alkynyl substituted with 0-4 R^{e}, -(CH₂)ₙ-C₃₋₁₀ carbocyclyl substituted with 0-4 R^{e}, or -(CH₂)ₙ-3- to 12-membered heterocyclyl comprising 1-4 heteroatoms selected from O, S(=O)ₚ, and N, and substituted with 0-4 R^{e};
R^{c} is C₁₋₅ alkyl substituted with 0-4 R^{e}, C₂₋₅ alkenyl substituted with 0-4 R^{e}, C₂₋₅ alkynyl substituted with 0-4 R^{e}, C₃₋₆ carbocyclyl, or 3- to 12-membered heterocyclyl comprising 1-4 heteroatoms selected from O, S(=O)ₚ, and N;
R^{e} is halo, CN, =O, C₁₋₆ alkyl substituted with 0-4 R^{g}, C₂₋₆ alkenyl substituted with 0-4 R^{g}, C₂₋₆ alkynyl substituted with 0-4 R^{g}, -(CH₂)ₙ-C₃₋₆ cycloalkyl substituted with 0-4 R^{g}, -(CH₂)ₙ-aryl substituted with 0-4 R^{g},-(CH₂)ₙ-4- to 6-membered heterocyclyl comprising 1-4 heteroatoms selected from O, S(=O)ₚ, and N, and substituted with 0-4 R^{g}, -(CH₂)ₙOR^{f}, C(=O)OR^{f}, C(=O)NR^{f}R^{f}, NR^{f}C(=O)R^{f}, S(=O)ₚR^{f}, NR^{f}S(=O)ₚR^{f}, NR^{f}C(=O)OR^{f}, OC(=O)NR^{f}R^{f}, or -(CH₂)ₙNR^{f}R^{f};
R^{f} is H, C₁₋₆ alkyl, C₃₋₆ cycloalkyl, or aryl;
R^{g} is halo, CN, -OH, C₁₋₄ alkyl, C₃₋₆ cycloalkyl, or phenyl;
n is zero, 1, 2, or 3; and
p is zero, 1, or 2.

9. The compound of claim 8, or a pharmaceutically acceptable salt thereof, wherein:
R^{4a} is halo;
R^{4b} is C₁₋₄ alkyl substituted with 0-3 halo substituents;
R⁵ is H;
R⁶ is C₁₋₂ alkyl substituted with 0-2 F substituents or C₃₋₆ cycloalkyl;
R⁸ is -OC₁₋₃ alkyl;
R^{8a} is F or CN;
R⁹ is 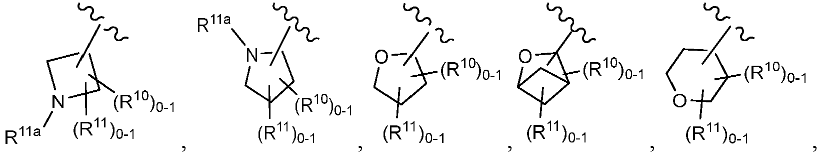 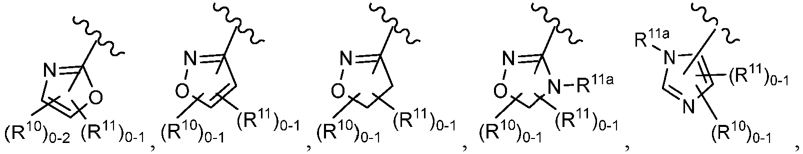 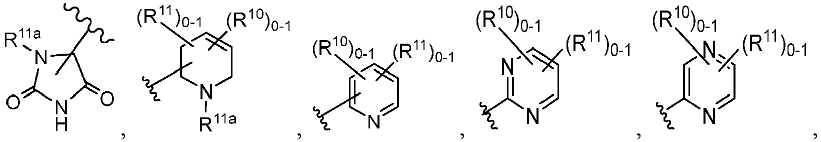 or 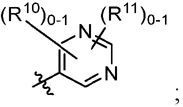
R¹⁰ is halo, CN, C₁₋₂ alkyl, =O, **-OH,** or -OC₁₋₂ alkyl;
R¹¹ is C₁₋₃ alkyl substituted with 0-1 R¹² and 0-1 R¹³, -OR^{b}, -NR^{a}R^{a}, -NR^{a}C(=O)R^{b}, -C(=O)R^{b}, -C(=O)OR^{b}, -C(=O)NR^{a}R^{a}, or C₃₋₆ cycloalkyl substituted with 0-5 R^{e};
R^{11a} is **H,** -C(=O)R^{b}, -C(=O)NR^{a}R^{a}, or C₁₋₄ alkyl substituted with 0-1R^{11b};
R^{11b} is **-OH** or aryl;
R¹² is -C(=O)OR^{b}, -C(=O)NHR^{a}, -C(=O)NHOR^{b}, or C₁₋₄ alkyl substituted with 0-2 halo **or -OH** substituents;
R¹³ is **-OH,** -OC₁₋₄ alkyl substituted with 0-2 **-OH** substituents, or -S(=O)₂C₁₋₄alkyl;
R^{a} is H or C₁₋₄ alkyl or R^{a} and R^{a} together with the nitrogen atom to which they are both attached form a 3 to 9-membered heterocyclyl substituted with 0-4 R^{e};
R^{b} is **H,** C₁₋₄ alkyl substituted with 0-1 R^{e}, or C₃₋₆ cycloalkyl substituted with 0-1 R^{e};
R^{e} is -OR^{f}; and
R^{f} is H or C₁₋₄ alkyl.

10. The compound of claim 9, or a pharmaceutically acceptable salt thereof, wherein:
R^{4a} is halo;
R^{4b} is CF₃;
R⁵ is H;
R⁶ is CF₃ or C₃₋₆ cyclopropyl;
R⁸ is -OC₁₋₃ alkyl;
R⁹ is 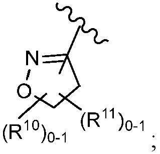
R¹⁰ is C₁₋₂ alkyl, -OH, or -OC₁₋₂ alkyl;
R¹¹ is C₁₋₃ alkyl substituted with 0-1 R¹² and 0-1 R¹³, -C(=O)OR^{b}, or -C(=O)NR^{a}R^{a};
R¹² is -C(=O)OR^{b};
R¹³ is -OH;
R^{a} is H or C₁₋₄ alkyl; and
R^{b} is H or C₁₋₄ alkyl.

11. The compound of claim 8, or a pharmaceutically acceptable salt thereof, wherein:
R^{4a} is halo;
R^{4b} is C₁₋₄ alkyl substituted with 0-3 halo substituents;
R⁵ is H;
R⁶ is C₁₋₃ alkyl substituted with 0-3 F substituents or C₃₋₆ cycloalkyl;
R⁸ is -OC₁₋₃ alkyl;
R⁹ is 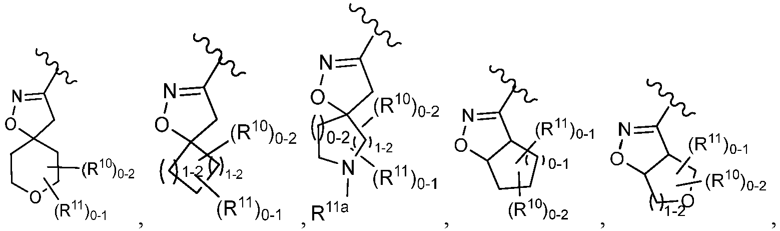 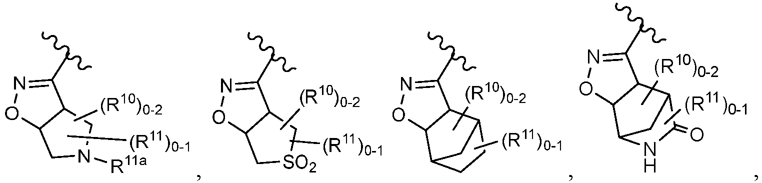 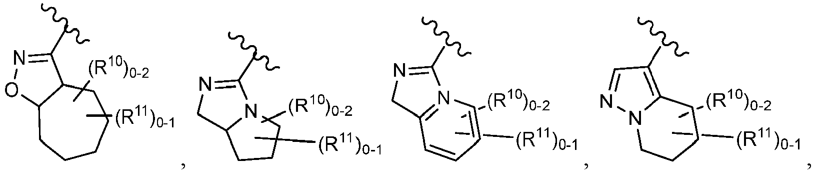 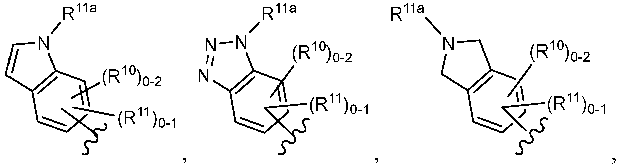 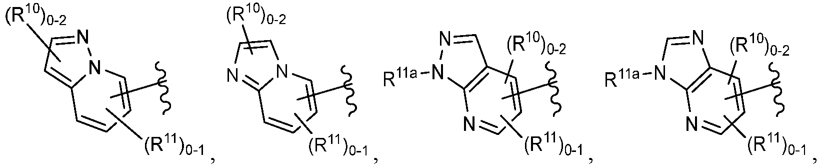 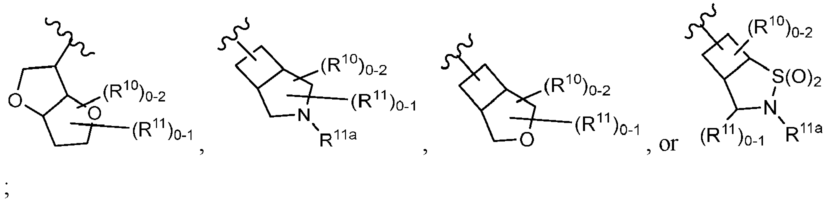
R¹⁰ is halo, C₁₋₃ alkyl, -OH, or -OC₁₋₃ alkyl;
R¹¹ is C₁₋₃ alkyl substituted with 0-1 R¹² and 0-1 R¹³;
R^{11a} is H, C₁₋₄ alkyl substituted with 0-2 R^{11b}, or -C(=O)OC₁₋₄ alkyl;
R^{11b} is -OH, -C(=O)OH, or aryl;
R¹² is -C(=O)OR^{b} or C₁₋₃ alkyl substituted with 0-3 halo substituents;
R¹³ is -OH; and
R^{b} is H or C₁₋₄ alkyl.

12. The compound of claim 3, having Formula (VIII): 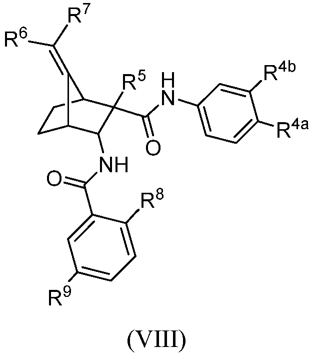 or a pharmaceutically acceptable salt thereof, wherein:
R^{4a} is halo;
R^{4b} is C₁₋₄ alkyl substituted with 0-4 halo substituents;
R⁶ is C₁₋₂ alkyl substituted with 0-2 F substituents, C₃₋₆ cycloalkyl, or aryl;
R⁷ is H;
R⁸ is -OC₁₋₃ alkyl;
R⁹ is 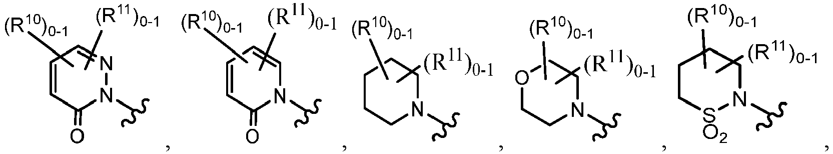 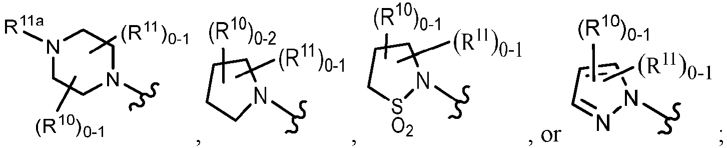
R¹⁰ is halo, CN, C₁₋₄ alkyl, =O, -OH, or -OC₁₋₄ alkyl;
R¹¹ is C₁₋₂ alkyl substituted with 0-1 R¹² and 0-1 R¹³, -NR^{a}R^{a}, -NR^{a}C(=O)R^{b}, - NR^{a}C(=O)OR^{b}, or -C(=O)OR^{b};
R¹² is -C(=O)OR^{b}, -C(=O)NHR^{a}, -C(=O)NHOR^{b}, or C₁₋₄ alkyl substituted with 0-3 halo or -OH substituents;
R¹³ is -OH or -NR^{a}C(=O)R^{b};
R^{a} is H or C₁₋₄ alkyl; and
R^{b} is H, C₁₋₄ alkyl, or 3 to 9-membered heterocyclyl comprising 1-4 heteroatoms selected from O, S(=O)ₚ, and N.

13. The compound of claim 1, having Formula (IX): 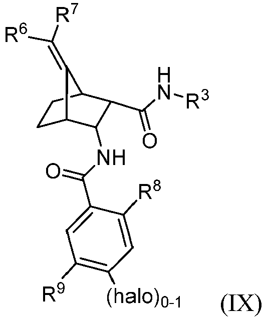 or a pharmaceutically acceptable salt thereof, wherein:
R³ is C₁₋₅ alkyl, CF₃, -(CR^{d}R^{d})₀₋₁-C₃₋₆ cycloalkyl substituted with 0-4 R⁴, or phenyl substituted with 0-4 R⁴;
R⁴ is halo, CN, CH₃, or CF₃;
R⁶ is C₁₋₆ alkyl, CF₃, or C₃₋₆ cycloalkyl substituted with 0-2 F substituents;
R⁷ is H;
R⁸ is halo, -N(C₁₋₃ alkyl)₂, -OC₁₋₃ alkyl substituted with 0-1 -OC₁₋₄ alkyl substituent;
R⁹ is 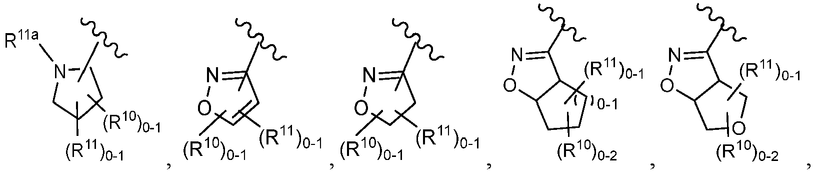 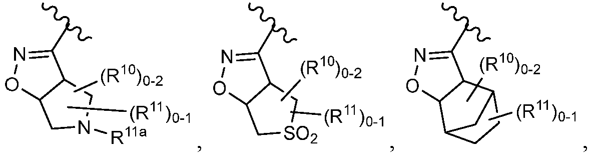 or 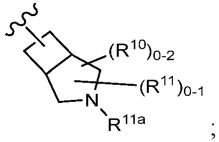
R¹⁰ is halo, C₁₋₄ alkyl, -OH, or -OC₁₋₄ alkyl;
R¹¹ is C₁₋₄ alkyl substituted with 0-2 R¹² and 0-2 R¹³, -C(=O)OR^{b}, -C(=O)NR^{a}R^{a}, or C₃₋₆ cycloalkyl substituted with 0-2 R^{e};
R^{11a} is H, C₁₋₄ alkyl substituted with 0-2 R^{11b}, -C(=O)R^{b}, or -C(=O)OC₁₋₄ alkyl;
R^{11b} is -OH;
R¹² is C₁₋₃ alkyl substituted with 0-3 halo substituents or -C(=O)OR^{b};
R¹³ is -OH;
R^{a} is H or C₁₋₃ alkyl;
R^{b} is H or C₁₋₄ alkyl substituted with 0-1 R^{e};
R^{e} is -OR^{f}; and
R^{f} is H or C₁₋₆ alkyl.

14. The compound of claim 1, having Formula (X): 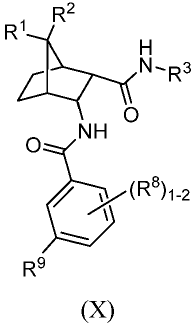 or a pharmaceutically acceptable salt thereof, wherein:
R¹ and R² are combined to be =CR⁶R⁷;
R³ is C₁₋₆ alkyl substituted with 0-5 halo, CN, or -OC₁₋₃ alkyl substituents, -(CHR^{d})ₙ-C₃₋₁₀-carbocyclyl substituted with 0-5 R⁴, or 5- to 6-membered heteroaryl comprising 1-3 heteroatoms selected from O, S, N, and substituted with 0-3 R⁴;
R⁴ is halo, S(=O)₂CF₃, CN, or C₁₋₄ alkyl substituted with 0-5 halo substituents;
R⁶ is halo, C₁₋₅ alkyl substituted with 0-3 R^{6a}, C₃₋₆ cycloalkyl substituted with 0-3 R¹⁴, or 5- to 6-membered heterocyclyl comprising 1-3 heteroatoms selected from O, S, and N, and substituted with 0-3 R¹⁴;
R^{6a} is halo, -OH, or C₃₋₆ cycloalkyl;
R⁷ is H;
R⁸ is H, halo, CN, C₁₋₄ alkyl, or -OC₁₋₄ alkyl substituted with 0-5 halo, -OH, C₃₋₆ cycloalkyl, or -OC₁₋₄ alkyl substituents;
R⁹ is 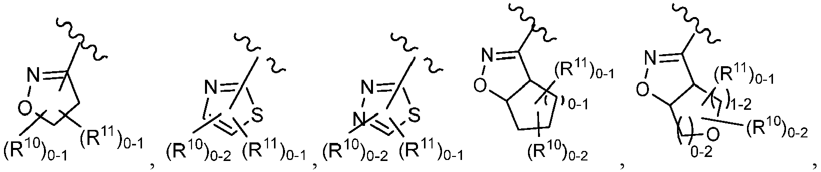 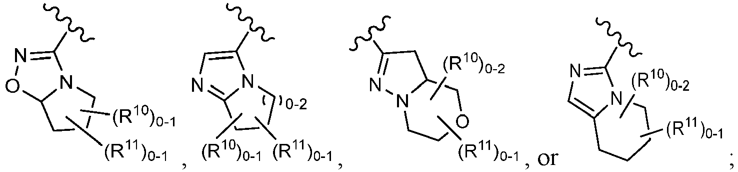
R¹⁰ is halo, CN, C₁₋₄ alkyl, or -OH;
R¹¹ is C₁₋₃ alkyl substituted with 0-3 R¹² and 0-1 R¹³, -OR^{b}, -NHC(=O)R^{b}, or -C(=O)OR^{b};
R¹² is halo;
R¹³ is -OR^{b} or C₃₋₆ carbocyclyl;
R¹⁴ is halo, CN, or C₁₋₄ alkyl substituted with 0-3 halo substituents;
R^{b} is H or C₁₋₃ alkyl substituted with 0-5 R^{e};
R^{d} is H or C₁₋₄ alkyl;
R^{e} is -OH; and
n is zero or 1.

15. The compound of claim 14, having formula (XI): 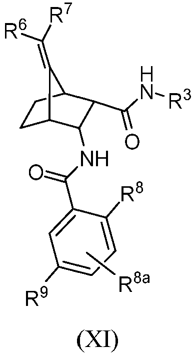 or a pharmaceutically acceptable salt thereof, wherein:
R³ is C₁₋₅ alkyl or 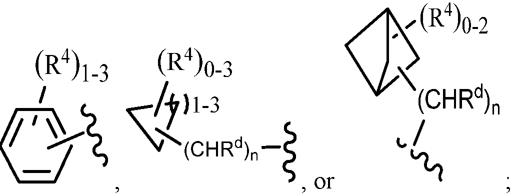
R⁴ is halo, CN, -S(=O)₂CF₃, C₁₋₄ alkyl substituted with 0-5 halo substituents;
R⁶ is C₁₋₅ alkyl substituted with 0-2 R^{6a}, C₃₋₆ cycloalkyl substituted with 0-2 R¹⁴, or 5- to 6-membered heterocyclyl comprising 1-3 heteroatoms selected from O, S, and N, and substituted with 0-2 R¹⁴;
R^{6a} is halo, -OH, or C₃₋₆ cycloalkyl;
R⁷ is H;
R⁸ is -OC₁₋₃ alkyl substituted with 0-5 halo, -OH, C₃₋₆ cycloalkyl, or -OC₁₋₃ alkyl substituents;
R^{8a} is H, halo, CN, or C₁₋₃ alkyl;
R⁹ is 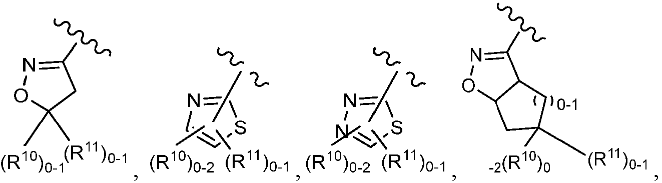 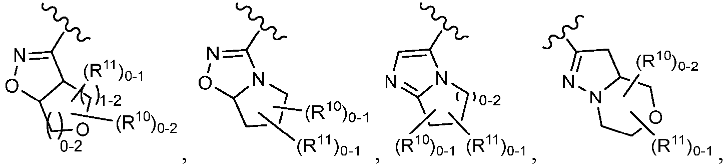 or 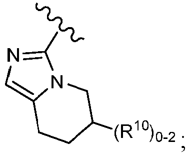
R¹⁰ is halo, CN, C₁₋₄ alkyl, or -OH;
R¹¹ is C₁₋₃ alkyl substituted with 0-3 R¹² and 0-1 R¹³, -OR^{b}, -NHC(=O)R^{b}, or -C(=O)OR^{b} ;
R¹² is halo;
R¹³ is -OR^{b} or C₃₋₆ carbocyclyl;
R¹⁴ is halo or C₁₋₄ alkyl substituted with 0-3 halo substituents;
R^{b} is H or C₁₋₃ alkyl substituted with 0-5 R^{e};
R^{d} is H or C₁₋₂ alkyl; and
n is zero or 1.

16. A compound of claim 1 selected from: 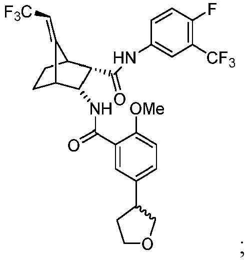 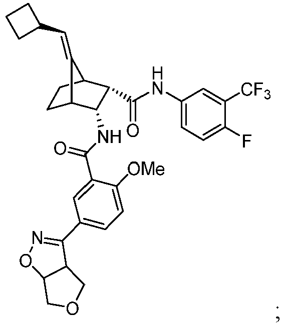 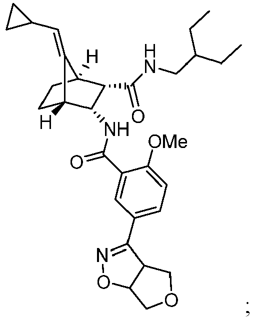 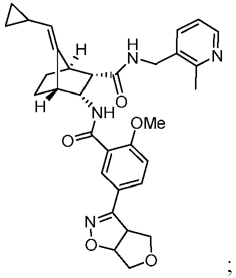 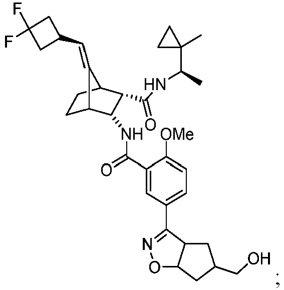 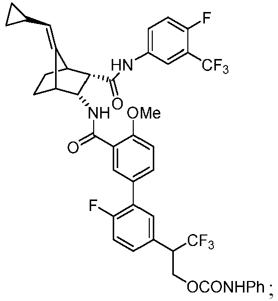 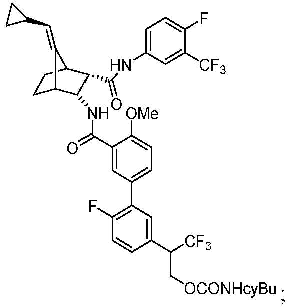 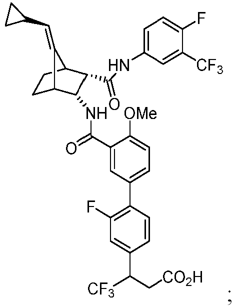 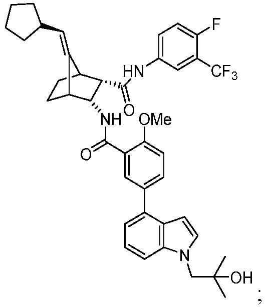 or a pharmaceutically acceptable salt thereof.

17. A pharmaceutical composition comprising a compound of any of claims 1 to 16 or a pharmaceutically acceptable salt thereof and a pharmaceutically acceptable carrier.

18. A compound of any of claims 1 to 16 or a pharmaceutically acceptable salt thereof, or a pharmaceutical composition of claim 17, for use in therapy.

## Patentansprüche

1. Verbindung der Formel (I): 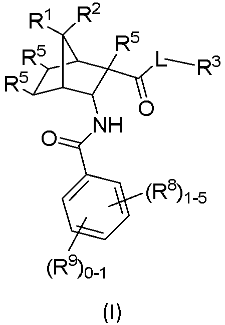 oder ein pharmazeutisch annehmbares Salz davon, wobei:
L -O- oder -NH- ist;
R¹ und R² kombiniert sind, um =CR⁶R⁷ oder =NOC₁₋₄-Alkyl zu sein, wobei "=" eine Doppelbindung ist;
oder R¹ und R² zusammen mit dem Kohlenstoffatom, an das sie beide gebunden sind, ein Dioxolanyl bilden, das mit einem O-1-Arylsubstituenten substituiert ist;
R³ C₁₋₈ Alkyl, substituiert mit 0-5 Halogen-, CN-, -OH- oder -OC₁₋₃ -Alkylsubstituenten, - (CR^{d}R^{d})ₙ-C₃₋₁₀-Carbocyclyl, substituiert mit 0-5 R⁴, oder -(CR^{d}R^{d})ₙ-3- bis 12-gliedriges Heterocyclyl, umfassend 1-4 Heteroatome, ausgewählt aus O, S(=O)ₚ, N und NR^{4c}, und substituiert mit 0-5 R⁴, ist;
R⁴ Halogen, CN, -OH, SF₅, -S(=O)ₚR^{c}, C₁₋₄-Alkyl, substituiert mit 0-5 Halogen-, -OH-, oder - OC₁₋₄-Alkylsubstituenten, -OC₁₋₄-Alkyl, substituiert mit 0-5 Halogensubstituenten, -(CR^{d}R^{d})ₙ-C₃₋₁₀ Carbocyclyl, substituiert mit 0-5 R^{e}, oder -(CR^{d}R^{d})ₙ-4- bis 6-gliedriges Heterocyclyl, umfassend 1-4 Heteroatome, ausgewählt aus O, S(=O)ₚ, N und NR^{4c}, und substituiert mit 0-5 R^{e}, ist;
R^{4c} H, C₁₋₄-Alkyl, oder -S(=O)₂CF₃ ist;
jeder R⁵ H, Halogen, -OH, C₁₋₄-Alkyl, substituiert mit 0-5 Halogensubstituenten, oder - OC₁₋₄-Alkyl, substituiert mit 0-5 Halogensubstituenten, ist;
R⁶ H, Halogen, CN, C₁₋₇ -Alkyl, substituiert mit 0-3 R^{6a}, C₂₋₇-Alkenyl, substituiert mit 0-3 R^{6a}, C₂₋₇-Alkinyl, substituiert mit 0-3 R^{6a}, -C(=O)OR^{6b}, -C(=O)NR^{6b}R^{6b}, -(CH₂)ₙ-C₃₋₁₀ -Carbocyclyl, substituiert mit 0-5 R¹⁴, oder 3- bis 12-gliedriges Heterocyclyl, umfassend 1-4 Heteroatome, ausgewählt aus O, S(=O)ₚ, N oder NR^{14a}, und substituiert mit 0-5 R¹⁴, ist;
R^{6a} Halogen, -OH-, -OC₁₋₄-Alkyl, C₁₋₄-Alkyl, Aryl oder C₃₋₆₋-Cycloalkyl, substituiert mit 0-4 Halogen-Substituenten, ist;
R^{6b} H, C₁₋₄ -Alkyl , substituiert mit einem 0-1 Arylsubstituenten, oder C₃₋₆-Cycloalkyl, substituiert mit 0-4 Halogensubstituenten, ist;
R⁷ H oder C₁₋₄-Alkyl ist,
oder R⁶ und R⁷ zusammen mit dem Kohlenstoffatom, an das sie beide gebunden sind, ein Cyclopentadienyl, ein Indanyl oder ein Indenyl bilden;
R⁸ H, Halogen, CN, -NR⁷R⁷, C₁₋₄-Alkyl, substituiert mit 0-5 Halogen- oder -OH-Substituenten, oder -OC₁₋₄-Alkyl , substituiert mit 0-5 Halogen, -OH, C₃₋₆ Cycloalkyl, Aryl, 4- bis 9-gliedriges Heterocyclyl, umfassend 1-4 Heteroatome, ausgewählt aus O, S(=O)ₚ, und N, oder -OC₁₋₄-Alkyl, substituiert mit 0-1 -OC₁₋₃-Alkylsubstituenten, ist;
R⁹ ein Aryl, substituiert mit 0-3 R¹⁰ und 0-2 R¹¹ , oder 3- bis 12-gliedriges Heterocyclyl, umfassend 1-5 Heteroatome, ausgewählt aus O, S(=O)ₚ, N und NR^{11a}, und substituiert mit 0-3 R¹⁰ und 0-2 R¹¹ , ist;
R¹⁰ Halogen, CN, C₁₋₄-Alkyl, =O, -OH oder -OC₁₋₄-Alkyl ist;
R¹¹ ein C₁₋₄-Alkyl, substituiert mit 0-4 R¹² und 0-2 R¹³, -OR^{b}, -NR^{a}R^{a}, -NR^{a}C(=O)R^{b}, - NR^{a}C(=O)OR^{b}, -NR^{a}C(=O)NR^{a}R^{a}, -NR^{a}S(=O)ₚR^{c}, -C(=O)R^{b}, -C(=O)OR^{b}, -C(=O)NR^{a}R^{a}, -C(=O)NR^{a}S(=O)ₚR^{c}, -OC(=O)R^{b}, -S(=O)ₚR^{c}, -S(=O)ₚNR^{a}R^{a}, C₃₋₆ -Carbocyclyl, substituiert mit 0-5 R^{e}, oder 3- bis 12-gliedriges Heterocyclyl, umfassend 1-4 Heteroatome, ausgewählt aus O, S(=O)ₚ, N und NR¹⁵, und substituiert mit 0-5 R^{e}, ist;
R^{11a} H, C₁₋₅-Alkyl, substituiert mit 0-4 R^{11b}, -C(=O)R^{b}, -C(=O)OR^{B}, -C(=O)NR^{a}R^{a}, C₃₋₆-Cycloalkyl, substituiert mit 0-5 R^{e}, Aryl, substituiert mit 0-5 R^{e}, 4- bis 6-gliedriges Heterocyclyl, umfassend 1-4 Heteroatome, ausgewählt aus O, S(=O)ₚ, N und NR¹⁵, und substituiert mit 0-5 R^{e}, ist;
R^{11b} Halogen, -OH, -C(=O)OH, -C(=O)OC₁₋₄-Alkyl- oder -Aryl ist;
R¹² Halogen, -C(=O)OR^{B}, -C(=O)NR^{a}R^{a}, -C(=O)NR^{a}OR^{b} oder C₁₋₄-Alkyl, substituiert mit 0-3 Halogen- oder -OH-Substituenten, oder C₃₋₆-Cycloalkyl ist;
R¹³ -OR^{b}, -NR^{a}R^{a}, -NR^{a}C(=O)R^{b}, -NR^{a}C(=O)OR^{b}, -NR^{a}C(=O)NR^{a}R^{a}, -NR^{a}S(=O)ₚR^{c}, - NR^{a}S(=O)ₚNR^{a}R^{a}, -OC(=O)NR^{a}R^{a}, -OC(=O)NR^{a}OR^{b}, -S(=O)ₚNR^{a}R^{a}, -S(=O)ₚR^{c}, -(CH₂)ₙ-C₃₋₁₀-Carbocyclyl, substituiert mit 0-3 R^{e}, oder -(CH₂)ₙ-3- bis 12-gliedriges Heterocyclyl , umfassend 1-4 Heteroatome, ausgewählt aus O, S(=O)ₚ, und N, und substituiert mit 0-3 R^{e}, ist;
R¹⁴ Halogen, CN, C₁₋₄-Alkyl, substituiert mit 0-3 Halogensubstituenten, -OC₁₋₄ -Alkyl, substituiert mit 0-3 Halogensubstituenten, -(CH₂)ₙ-NR^{a}R^{a}, -(CH₂)ₙ-Aryl, substituiert mit 0-3 R^{e}, -O-Aryl, substituiert mit 0-3 R^{e}, oder -(CH₂)ₙ-3- bis 12-gliedriges Heterocyclyl, umfassend 1-4 Heteroatome, ausgewählt aus O, S(=O)_{p und} N, und substituiert mit 0-3 R^{e}, ist;
R^{14a} H, C(=O)C₁₋₄-Alkyl oder C₁₋₃ -Alkyl, substituiert mit 0-3 Si(C₁₋₃- Alkyl)₃ oder -Aryl, substituiert mit 0-2 Halogensubstituenten, ist;
R¹⁵ H, C₁₋₄-Alkyl oder -Aryl ist;
R^{a} H, -OC₁₋₆-Alkyl, C₁₋₆-Alkyl, substituiert mit 0-5 R^{e}, C₂₋₆-Alkenyl, substituiert mit 0-5 R^{e}, C₂₋₆-Alkinyl, substituiert mit 0-5 R^{e}, -(CH₂)ₙ-C₃₋₁₀ -Carbocyclyl, substituiert mit 0-5 R^{e}, oder -(CH₂)ₙ-3- bis 12-gliedriges Heterocyclyl, umfassend 1-4 Heteroatome, ausgewählt aus O, S(=O)_{p und} N, und substituiert mit 0-5 R^{e} ist; oder R^{a} und R^{a} zusammen mit dem Stickstoffatom, an das sie beide gebunden sind, ein 3- bis 12-gliedrigen Heterocyclyl bilden, umfassend 1-4 Heteroatome, ausgewählt aus O, S(=O)_{p und} N, und substituiert mit 0-5 R^{e}, ist;
R^{b} H, C₁₋₆-Alkyl, substituiert mit 0-5 R^{e}, C₂₋₆-Alkenyl, substituiert mit 0-5 R^{e}, C₂₋₆-Alkinyl, substituiert mit 0-5 R^{e}, -(CH₂)ₙ-C₃₋₁₀ -Carbocyclyl, substituiert mit 0-5 R^{e}, oder -(CH₂)ₙ-3- bis 12-gliedriges Heterocyclyl, umfassend 1-4 Heteroatome, ausgewählt aus O, S(=O)_{p und} N, und substituiert mit 0-5 R^{e}, ist;
R^{c} C₁₋₆-Alkyl, substituiert mit 0-5 R^{e}, C₂₋₆ -Alkenyl, substituiert mit 0-5 R^{e}, C₂₋₆ Alkinyl, substituiert mit 0-5 R^{e},C₃₋₆ -Carbocyclyl , substituiert mit 0-5 R^{e}, oder 3- bis 12-gliedriges Heterocyclyl, umfassend 1-4 Heteroatome, ausgewählt aus O, S(=O)_{p und} N, und substituiert mit 0-5 R^{e}, ist;
R^{d} H, C₁₋₄-Alkyl oder C₃₋₆-Cycloalkyl ist;
R^{e} Halogen, CN, NO₂, =O, C₁₋₆-Alkyl, substituiert mit 0-5 R^{g}, C₂₋₆-Alkenyl, substituiert mit 0-5 R^{g}, C₂₋₆-Alkinyl, substituiert mit 0-5 R^{g}, -(CH₂)ₙ-C₃₋₁₀-Carbocyclyl, substituiert mit 0-5 R^{g}, -(CH₂)ₙ-3-bis 12-gliedriges Heterocyclyl, umfassend 1-4 Heteroatome, ausgewählt aus O, S(=O)ₚ und N, und substituiert mit 0-5 R^{g}, -(CH₂)ₙOR^{f}, -C(=O)OR^{f}, -C(=O)NR^{f}R^{f}, -NR^{f}C(=O)R^{f}, -S(=O)ₚR^{f}, -S(=O)ₚNR^{f}R^{f}, - NR^{f}S(=O)ₚR^{f}, -NR^{f}C(=O)OR^{f}, -OC(=O)NR^{f}R^{f} oder -(CH₂)ₙNR^{f}R^{f}, ist;
R^{f} H, C₁₋₆ -Alkyl, substituiert mit 0-2 -OH oder -OC₁₋₄ -Alkylsubstituenten, C₃₋₆-Cycloalkyl, - Aryl oder 3- bis 12-gliedriges Heterocyclyl, umfassend 1-4 Heteroatome, ausgewählt aus O, S(=O)_{p und} N, ist; oder R^{f} und R^{f} zusammen mit dem Stickstoffatom, an das sie beide gebunden sind, ein 3- bis 12-gliedriges Heterocyclyl, umfassend 1-4 Heteroatome, ausgewählt aus O, S(=O)_{p und} N, bilden;
R^{g} Halogen, CN, -OH, C₁₋₆-Alkyl, C₃₋₆-Cycloalkyl oder -Aryl ist;
n null, 1, 2 oder 3 ist; und
p null, 1 oder 2 ist.

2. Verbindung nach Anspruch 1 oder ein pharmazeutisch annehmbares Salz davon, wobei
R³ C₁₋₆-Alkyl, substituiert mit 0-4 Halogen- oder -OH-Substituenten, -(CHR^{d})₀₋₁-C₃₋₆-Cycloalkyl, substituiert mit 0-4 R⁴, C₆₋₉ -Spirocycloalkyl, substituiert mit 0-4 R⁴, C₆₋₁₀ -bizyklisches Carbocyclyl, substituiert mit 0-4 R⁴, oder 3- bis 6-gliedriges Heterocyclyl, umfassend 1-2 Heteroatome, ausgewählt aus O, S(=O)ₚ, N und NR^{4c}, und substituiert mit 0-4 R⁴, ist;
R⁴ Halogen oder C₁₋₃-Alkyl, substituiert mit 0-4 Halogen-Substituenten, ist;
R^{4c} H oder C₁₋₄-Alkyl ist und
R^{d} C₁₋₃ -Alkyl ist.

3. Verbindung nach Anspruch 1, aufweisend die Formel (II): 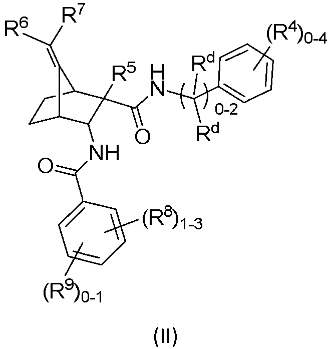 oder ein pharmazeutisch annehmbares Salz davon, wobei:
R⁴ Halogen, -S(=O)ₚC₁₋₄-Alkyl, substituiert mit 0-4 Halogensubstituenten, C₁₋₄-Alkyl, substituiert mit 0-4 Halogensubstituenten, oder -OC₁₋₄-Alkyl, substituiert mit 0-4 Halogensubstituenten, ist;
R⁵ H oder Halogen ist;
R⁶ Halogen, CN, C₁₋₇-Alkyl, substituiert mit 0-3 R^{6a}, C₂₋₇-Alkenyl, substituiert mit 0-3 R^{6a}, C₂₋₇-Alkinyl, substituiert mit 0-3 R^{6a}, C(=O)OR^{6b}, -C(=O)NR^{6b}R^{6b}, C₃₋₆-Cycloalkyl, substituiert mit 0-3 R¹⁴, C₃₋₆-Cycloalkenyl, substituiert mit 0-3 R¹⁴, Aryl, substituiert mit 0-3 R¹⁴, oder 4- bis 6-gliedriges Heterocyclyl, umfassend 1-3 Heteroatome, ausgewählt aus O, S(=O)ₚ, N und NR^{14a} und substituiert mit 0-3 R¹⁴, ist;
R^{6a} Halogen, -OH, C₃₋₆ Cycloalkyl oder Aryl ist;
R^{6b} H, C₁₋₄-Alkyl, substituiert mit 0-1 Arylsubstituenten, oder C₃₋₆-Cycloalkyl, substituiert mit 0-4 Halogensubstituenten, ist;
R⁷ H oder C₁₋₃-Alkyl ist;
R⁸ Halogen, CN, -N(C₁₋₂-Alkyl)₂, C₁₋₄-Alkyl, substituiert mit 0-5 Halogen- oder -OH-Substituenten, oder -OC₁₋₄-Alkyl, substituiert mit 0-4 Halogen-, -OH-, Aryl- oder -OC₁₋₄-Alkylsubstituenten, ist;
R⁹ Aryl, substituiert mit 0-3 R¹⁰ und 0-2 R¹¹ , oder 3- bis 12-gliedriges Heterocyclyl, umfassend 1-4 Heteroatome, ausgewählt aus O, S(=O)ₚ, N und NR^{11a}, und substituiert mit 0-3 R¹⁰ und 0-1 R¹¹ , ist;
R¹⁰ Halogen, CN, C₁₋₄-Alkyl, =O, -OH oder -OC₁₋₄-Alkyl ist;
R¹¹ C₁₋₄-Alkyl, substituiert mit 0-3 R¹² und 0-1 R¹³, -OR^{b}, -NR^{a}R^{a}, -NR^{a}C(=O)R^{b}, - NR^{a}C(=O)OR^{b}, -NR^{a}C(=O)NR^{a}R^{a}, -NR^{a}S(=O)ₚR^{c}, -C(=O)R^{b}, -C(=O)OR^{b}, -C(=O)NR^{a}R^{a}, -C(=O)NR^{a}S(=O)ₚR^{c}, -OC(=O)R^{b}, -S(=O)ₚR^{c}, -S(=O)ₚNR^{a}R^{a}, C₃₋₆-Cycloalkyl, substituiert mit 0-5 R^{e}, 4- bis 12-gliedriges Heterocyclyl, umfassend 1-4 Heteroatome, ausgewählt aus O, S(=O)ₚ, N und NR¹⁵, und substituiert mit 0-5 R^{e}, ist;
R^{11a} H, C₁₋₄-Alkyl, substituiert mit 0-2 R^{11b}, -C(=O)R^{b}, -C(=O)OR^{b}, -C(=O)NR^{a}R^{a}, C₃₋₆-Cycloalkyl, substituiert mit 0-5 R^{e}, 4- bis 6-gliedriges Heterocyclyl, umfassend 1-4 Heteroatome, ausgewählt aus O, S(=O)ₚ, N und NR¹⁵, und substituiert mit 0-5 R^{e}, ist;
R^{11b} -OH, -C(=O)OH, oder Aryl ist;
R¹² Halogen, -C(=O)OR^{b}, -C(=O)NHR^{a}, -C(=O)NHORb oder C₁₋₄-Alkyl, substituiert mit 0-3 Halogen- oder -OH-Substituenten, ist;
R¹³ -OR^{b}, -NR^{a}R^{a}, -NR^{a}C(=O)R^{b}, -NR^{a}C(=O)OR^{b}, -NR^{a}S(=O)ₚR^{c}, -NR^{a}S(=O)ₚNR^{a}R^{a}, - OC(=O)NR^{a}R^{a}, -OC(=O)NR^{a}OR^{b}, -S(=O)ₚNR^{a}R^{a} oder -S(=O)ₚR^{c}, ist;
R¹⁴ Halogen, CN, C₁₋₄ -Alkyl, substituiert mit 0-3 Halogensubstituenten, -OC₁₋₄ -Alkyl, substituiert mit 0-3 Halogensubstituenten, -(CH₂)₀₋₃-NR^{a}R^{a}, -(CH₂)₀₋₃-Aryl, substituiert mit 0-3 R^{e}, -O - Aryl, substituiert mit 0-3 R^{e}, oder -(CH₂)₀₋₂-3- bis 12-gliedriges Heterocyclyl, umfassend 1-4 Heteroatome, ausgewählt aus O, S(=O)_{p und} N, und substituiert mit 0-3 R^{e}, ist;
R^{14a} H, C(=O)C₁₋₄ -Alkyl oder C₁₋₃ -Alkyl, substituiert mit 0-3 Aryl, substituiert mit 0-2 Halogensubstituenten, ist;
R¹⁵ H, C₁₋₃-Alkyl oder Aryl ist;
R^{a} H, C₁₋₅ -Alkyl, substituiert mit 0-5 R^{e}, C₂₋₅ -Alkenyl, substituiert mit 0-5 R^{e}, C₂₋₅ -Alkinyl, substituiert mit 0-5 R^{e}, -(CH₂)ₙ-C₃₋₁₀-Carbocyclyl, substituiert mit 0-5 R^{e}, oder -(CH₂)ₙ-3- bis 12-gliedriges Heterocyclyl , umfassend 1-4 Heteroatome, ausgewählt aus O, S(=O)_{p und} N, und , substituiert mit 0-5 R^{e}, ist; oder R^{a} und R^{a} zusammen mit dem Stickstoffatom, an das sie beide gebunden sind, ein 3- bis 12-gliedriges Heterocyclyl , das 1-4 Heteroatome umfasst, ausgewählt aus O, S(=O)ₚ, und N, und substituiert mit 0-5 R^{e}, bilden;
R^{b} H, C₁₋₅ -Alkyl, substituiert mit 0-5 R^{e}, C₂₋₅ -Alkenyl, substituiert mit 0-5 R^{e}, C₂₋₅ -Alkinyl, substituiert mit 0-5 R^{e}, -(CH₂)ₙ-C₃₋₁₀-Carbocyclyl, substituiert mit 0-5 R^{e}, oder -(CH₂)ₙ-3- bis 12-gliedriges Heterocyclyl , umfassend 1-4 Heteroatome, ausgewählt aus O, S(=O)ₚ und N, und , substituiert mit 0-5 R^{e}, ist;
R^{c} C₁₋₅-Alkyl, substituiert mit 0-5 R^{e}, C₂₋₅ -Alkenyl, substituiert mit 0-5 R^{e}, C₂₋₅ -Alkinyl, substituiert mit 0-5 R^{e}, C₃₋₆ -Carbocyclyl, substituiert mit 0-5 R^{e}, oder 3- bis 12-gliedriges Heterocyclyl , umfassend 1-4 Heteroatome, ausgewählt aus O, S(=O)ₚ, und N, und substituiert mit 0-5 R^{e}, ist;
R^{d} H oder C₁₋₄-Alkyl ist,
R^{e} Halogen, CN, =O, C₁₋₆-Alkyl, substituiert mit 0-5 R^{g}, C₂₋₆-Alkenyl, substituiert mit 0-5 R^{g}, C₂₋₆-Alkinyl, substituiert mit 0-5 R^{g}, -(CH₂)ₙ-C₃₋₆-Cycloalkyl, substituiert mit 0-4 R^{g}, -(CH₂)ₙ-Aryl, substituiert mit 0-4 R^{g}, -(CH₂)ₙ-4- bis 6-gliedriges Heterocyclyl , umfassend 1-4 Heteroatome, ausgewählt aus O, S(=O)ₚ und N, und substituiert mit 0-4 R^{g}, -(CH₂)ₙOR^{f}, -C(=O)OR^{f}, -C(=O)NR^{f}R^{f}, - NR^{f}C(=O)R^{f}, -S(=O)ₚR^{f}, -NR^{f}C(=O)OR^{f}, -OC(=O)NR^{f}R^{f} oder -(CH₂)ₙNR^{f}R^{f}, ist;
R^{f} H, C₁₋₅ -Alkyl, C₃₋₆-Cycloalkyl oder Aryl; oder R^{f} und R^{f} zusammen mit dem Stickstoffatom, an das sie beide gebunden sind, einen 3- bis 9-gliedrigen Heterocyclyl bilden;
R^{g} Halogen, CN, -OH, C₁₋₅Alkyl, C₃₋₆-Cycloalkyl oder Aryl ist;
n null, 1, 2 oder 3 ist; und
p null, 1 oder 2 ist.

4. Verbindung nach Anspruch 3, aufweisend die Formel (IV): 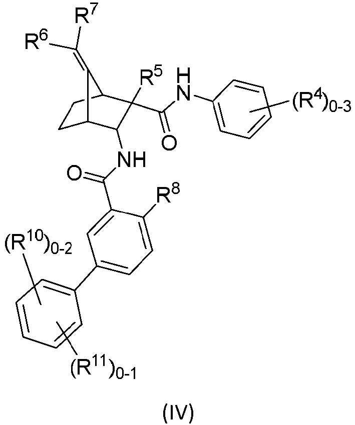 oder ein pharmazeutisch annehmbares Salz davon, wobei:
R⁴ Halogen, C₁₋₄-Alkyl, substituiert mit 0-3 Halogensubstituenten, oder -OC₁₋₄-Alkyl, substituiert mit 0-3 Halogensubstituenten, ist;
R⁵ H oder F ist;
R⁶ Halogen, CN, C₁₋₆-Alkyl, substituiert mit 0-3 R^{6a}, C₂₋₆-Alkenyl, substituiert mit 0-3 R^{6a}, C₂₋₆-Alkinyl, substituiert mit 0-3 R^{6a}, -C(=O)OR^{6b}, C(=O)NR^{6b}R^{6b}, C₃₋₆-Cycloalkyl, substituiert mit 0-3 R¹⁴, C₃₋₆-Cycloalkenyl, substituiert mit 0-3 R¹⁴, Phenyl, substituiert mit 0-3 R¹⁴, oder 5- bis 6-gliedriges Heteroaryl, umfassend 1-3 Heteroatome, ausgewählt aus O, S(=O)ₚ, N und NR^{14a}, und substituiert mit 0-3 R¹⁴, ist;
R^{6a} Halogen, C₃₋₆ Cycloalkyl oder Phenyl ist;
R^{6b} H, C₁₋₃ -Alkyl, substituiert mit 0-1 Arylsubstituenten, oder C₃₋₆-Cycloalkyl, substituiert mit 0-4 Halogen Substituenten, ist;
R⁷ H oder C₁₋₂-Alkyl ist;
R⁸ -OC₁₋₄-Alkyl, substituiert mit 0-4 Halogen-, -OH-, Aryl- oder -OC₁₋₄-Alkylsubstituenten, ist;
R¹⁰ **Halogen,** CN, C₁₋₃-Alkyl, -OH oder -OC₁₋₄-Alkyl ist;
¹¹ C₁₋₄-Alkyl, substituiert mit 0-2 R¹² und 0-1 R¹³, -OR^{b}, -NR^{a}R^{a}, -NR^{a}C(=O)R^{b}, - NR^{a}C(=O)NR^{a}R^{a}, -NR^{a}S(=O)ₚR^{c}, -C(=O)R^{b}, -C(=O)OR^{b}, -C(=O)NR^{a}R^{a}, -C(=O)NR^{a}S(=O)ₚR^{c}, -OC(=O)R^{b}, -S(=O)ₚR^{c}, -S(=O)ₚNR^{a}R^{a}, C₃₋₆-Cycloalkyl, 4- bis 9-gliedriges Heterocyclyl, umfassend 1-4 Heteroatome, ausgewählt aus O, S(=O)ₚ, N und NR¹⁵, und substituiert mit 0-4 R^{e}, ist;
R¹² Halogen, -C(=O)OR^{b}, -C(=O)NHR^{a}, -C(=O)NHORb oder C₁₋₄-Alkyl, substituiert mit 0-3 Halogen- oder -OH-Substituenten, ist;
R¹³ -OR^{b}, -NR^{a}R^{a}, -NR^{a}C(=O)R^{b}, -NR^{a}C(=O)OR^{b}, -NR^{a}S(=O)ₚR^{c}, -NR^{a}S(=O)ₚNR^{a}R^{a}, - OC(=O)NR^{a}R^{a}, -OC(=O)NR^{a}OR^{b}, -S(=O)ₚNR^{a}R^{a} oder -S(=O)ₚR^{c}, ist;
R¹⁴ Halogen, CN, C₁₋₄-Alkyl, substituiert mit 0-3 Halogensubstituenten, -OC₁₋₄-Alkyl, substituiert mit 0-3 Halogensubstituenten, -(CH₂)₀₋₂-NR^{a}R^{a}, -(CH₂)₀₋₂-Aryl, substituiert mit 0-3 R^{e}, -O-Aryl, substituiert mit 0-3 R^{e}, oder -(CH₂)₀₋₂-3- bis 12-gliedriges Heterocyclyl, umfassend 1-4 Heteroatome, ausgewählt aus O, S(=O)_{p und} N, und substituiert mit 0-3 R^{e}, ist;
R^{14a} H, C(=O)C₁₋₃Alkyl, C₁₋₃ -Alkyl, substituiert mit 0-2 Aryl, substituiert mit 0-2 Halogensubstituenten, ist;
R¹⁵ H, C₁₋₂-Alkyl oder Phenyl ist;
R^{a} H, C₁₋₅ -Alkyl, substituiert mit 0-4 R^{e}, C₂₋₅ -Alkenyl, substituiert mit 0-4 R^{e}, C₂₋₅ -Alkinyl, substituiert mit 0-4 R^{e}, -(CH₂)ₙ-C₃₋₁₀ -Carbocyclyl, substituiert mit 0-4 R^{e}, oder -(CH₂)ₙ-3- bis 12-gliedriges Heterocyclyl, umfassend 1-4 Heteroatome, ausgewählt aus O, S(=O)ₚ und N, und substituiert mit 0-4 R^{e}, ist; oder R^{a} und R^{a} zusammen mit dem Stickstoffatom, an das sie beide gebunden sind, ein 3- bis 12-gliedriges Heterocyclyl, umfassend 1-4 Heteroatome, ausgewählt aus O, S(=O)ₚ und N, und substituiert mit 0-4 R^{e}, bilden;
R^{b} H, C₁₋₅ -Alkyl, substituiert mit 0-4 R^{e}, C₂₋₅ -Alkenyl, substituiert mit 0-4 R^{e}, C₂₋₅ -Alkinyl, substituiert mit 0-4 R^{e}, -(CH₂)ₙ-C₃₋₁₀ -Carbocyclyl, substituiert mit 0-4 R^{e}, oder -(CH₂)ₙ-3- bis 12-gliedriges Heterocyclyl, umfassend 1-4 Heteroatome, ausgewählt aus O, S(=O)_{p und} N, und substituiert mit 0-4 R^{e}, ist;
R^{c} C₁₋₅-Alkyl, substituiert mit 0-4 R^{e}, C₂₋₅ -Alkenyl, substituiert mit 0-4 R^{e}, C₂₋₅ -Alkinyl, substituiert mit 0-4 R^{e}, C₃₋₆ -Carbocyclyl oder 3- bis 12-gliedriges Heterocyclyl, umfassend 1-4 Heteroatome, ausgewählt aus O, S(=O)ₚ und N, ist;
R^{e} Halogen, CN, NO₂, =O, C₁₋₆-Alkyl, substituiert mit 0-5 R^{g}, C₂₋₆-Alkenyl, substituiert mit 0-5 R^{g}, C₂₋₆-Alkinyl, substituiert mit 0-5 R^{g}, -(CH₂)ₙ-C₃₋₆-Cycloalkyl, -(CH₂)ₙ-Aryl, -(CH₂)ₙ- 4- bis 6-gliedriges Heterocyclyl, umfassend 1-4 Heteroatome, ausgewählt aus O, S(=O)ₚ, und N, -(CH₂)ₙOR^{f}, S(=O)ₚR^{f}, C(=O)NR^{f}R^{f}, C(=O)OR^{f}, NR^{f}C(=O)R^{f}, S(=O)ₚNR^{f}R^{f}, NR^{f}S(=O)ₚR^{f}, NR^{f}C(=O)OR^{f}, OC(=O)NR^{f}R^{f} oder -(CH₂)ₙNR^{f}R^{f} ist;
R^{f} H, C₁₋₆ -Alkyl, C₃₋₆-Cycloalkyl oder Aryl ist; oder R^{f} und R^{f} zusammen mit dem Stickstoffatom, an das sie beide gebunden sind, ein Heterocyclyl bilden;
R^{g} Halogen, CN, -OH, C₁₋₅ Alkyl, C₃₋₆-Cycloalkyl oder Aryl ist;
n null, 1, 2 oder 3 ist; und
p null, 1 oder 2 ist.

5. Verbindung nach Anspruch 1, aufweisend die Formel (V): 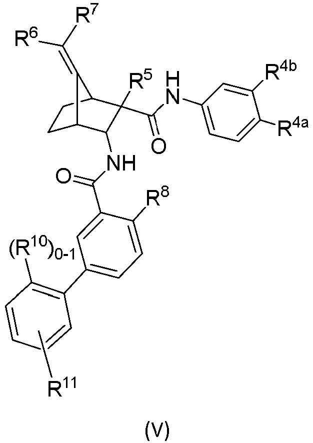 oder ein pharmazeutisch annehmbares Salz davon, wobei:
R^{4a} Halogen oder C₁₋₂-Alkyl ist;
R^{4b} C₁₋₄-Alkyl, substituiert mit 0-4 Halogensubstituenten, ist;
R⁵ H oder F ist;
R⁶ Halogen, CN, C₁₋₄-Alkyl, substituiert mit 0-3 R^{6a}, C₂₋₄-Alkenyl, substituiert mit 0-3 R^{6a}, - C(=O)OR^{6b}, C(=O)NR^{6b}R^{6b}, C₃₋₆-Cycloalkyl, substituiert mit 0-3 R¹⁴, Phenyl, substituiert mit 0-3 R¹⁴, oder 5- bis 6-gliedriges Heteroaryl, umfassend 1-3 Heteroatome, ausgewählt aus O, S(=O)ₚ, N und NR^{14a}, und substituiert mit 0-3 R¹⁴, ist;
R^{6a} Halogen, -OH, C₃₋₆-Cycloalkyl oder Phenyl ist;
R^{6b} H, C₁₋₃ -Alkyl, substituiert mit 0-1 Arylsubstituenten, oder C₃₋₆-Cycloalkyl ist;
R⁷ H oder C₁₋₂-Alkyl ist;
R⁸ -OC₁₋₄-Alkyl, substituiert mit 0-4 Halogen-, -OH-, -OC₁₋₄-Alkyl- oder Arylsubstituenten, ist;
R¹⁰ Halogen oder C₁₋₃ -Alkyl ist;
R¹¹ C₁₋₄-Alkyl, substituiert mit 0-2 R¹² und 0-1 R¹³, -OH, -OC₁₋₄-Alkyl, -NR^{a}C(=O)R^{b}, - NR^{a}C(=O)NR^{a}R^{a}, -NR^{a}S(=O)ₚR^{c}, -C(=O)R^{b}, -C(=O)OR^{b}, -C(=O)NR^{a}R^{a}, -C(=O)NR^{a}S(=O)ₚR^{c}, -OC(=O)R^{b}, -S(=O)ₚR^{c}, -S(=O)ₚNR^{a}R^{a}, C₃₋₆-Cycloalkyl, 4- bis 9-gliedriges Heterocyclyl, umfassend 1-4 Heteroatome, ausgewählt aus O, S(=O)ₚ, N und NR¹⁵, und substituiert mit 0-3 R^{e}, ist;
R¹² Halogen, -C(=O)OR^{b}, -C(=O)NHR^{a}, -C(=O)NHORb oder C₁₋₄-Alkyl, substituiert mit 0-3 Halogen- oder -OH-Substituenten, ist;
R¹³ -OR^{b}, -NR^{a}R^{a}, -NR^{a}C(=O)R^{b}, -NR^{a}C(=O)OR^{b}, -NR^{a}S(=O)ₚR^{c}, -NR^{a}S(=O)ₚNR^{a}R^{a}, - OC(=O)NR^{a}R^{a} oder -OC(=O)NR^{a}OR^{b} ist;
R¹⁴ Halogen, CN, C₁₋₄ -Alkyl, substituiert mit 0-3 Halogensubstituenten, -OC₁₋₄ -Alkyl, substituiert mit 0-3 Halogensubstituenten, -(CH₂)₀₋₂-NR^{a}R^{a}, -(CH₂)₀₋₁-Aryl, substituiert mit 0-3 R^{e}, -O-Aryl, substituiert mit 0-3 R^{e}, oder -(CH₂)₀₋₁-3- bis 9-gliedriges Heterocyclyl, umfassend 1-4 Heteroatome, ausgewählt aus O, S(=O)_{p und} N, und substituiert mit 0-3 R^{e}, ist;
R^{14a} H, C(=O)C₁₋₃ -Alkyl, C₁₋₃ -Alkyl, substituiert mit 0-1 Aryl, substituiert mit 0-2 Halogensubstituenten, ist;
R¹⁵ H, C₁₋₂-Alkyl oder Phenyl ist;
R^{a} H, C₁₋₄ -Alkyl, substituiert mit 0-5 R^{e}, C₂₋₄-Alkenyl, substituiert mit 0-5^{Re}, C₂₋₄ -Alkinyl, substituiert mit 0-5 R^{e}, -(CH₂)ₙ-C₃₋₁₀ -Carbocyclyl, substituiert mit 0-5 R^{e}, oder -(CH₂)ₙ-3- bis 12-gliedriges Heterocyclyl, umfassend 1-4 Heteroatome, ausgewählt aus O, S(=O)_{p und} N, und substituiert mit 0-5 R^{e}, ist; oder R^{a} und R^{a} zusammen mit dem Stickstoffatom, an das sie beide gebunden sind, ein 3- bis 9-gliedriges Heterocyclyl, umfassend 1-4 Heteroatome, ausgewählt aus O, S(=O)_{p und} N, und substituiert mit 0-5 R^{e}, bilden;
R^{b} H, C₁₋₄ -Alkyl, substituiert mit 0-5^{Re}, C₂₋₄ -Alkenyl, substituiert mit 0-5-R^{e}, C₂₋₄ -Alkinyl, substituiert mit 0-5-R^{e}, -(CH₂)ₙ-C₃₋₁₀-Carbocyclyl, substituiert mit 0-5 R^{e}, oder -(CH₂)ₙ-3- bis 12-gliedriges Heterocyclyl, umfassend 1-4 Heteroatome, ausgewählt aus O, S(=O)_{p und} N, und substituiert mit 0-5 R^{e}, ist;
R^{c} C₁₋₄-Alkyl, substituiert mit 0-5R^{e}, C₂₋₄ -Alkenyl, substituiert mit 0-5 R^{e}, C₂₋₄ -Alkinyl, substituiert mit 0-5 R^{e}, C₃₋₆ -Carbocyclyl oder 3- bis 9-gliedriges Heterocyclyl, umfassend 1-4 Heteroatome, ausgewählt aus O, S(=O)ₚ und N ist;
R^{e} Halogen CN, =O, C₁₋₆-Alkyl, substituiert mit 0-5 R^{g}, C₂₋₆-Alkenyl, substituiert mit 0-5 R^{g}, C₂₋₆-Alkinyl, substituiert mit 0-5 R^{g}, -(CH₂)ₙ-C₃₋₆-Cycloalkyl,-(CH₂)ₙ-Aryl, -(CH₂)ₙ- 4- bis 6-gliedriges Heterocyclyl, umfassend 1-4 Heteroatome, ausgewählt aus O, S(=O)ₚ, und N, -(CH₂)ₙOR^{f}, -S(=O)ₚR^{f}, - C(=O)NR^{f}R^{f}, -C(=O)OR^{f}, -NR^{f}C(=O)R^{f}, -S(=O)ₚNR^{f}R^{f}, -NR^{f}S(=O)ₚR^{f}, -NR^{f}C(=O)OR^{f}, -OC(=O)NR^{f}R^{f} oder - (CH₂)ₙNR^{f}R^{f} ist;
R^{f} H, C₁₋₆ -Alkyl, C₃₋₆-Cycloalkyl oder Aryl ist; oder R^{f} und R^{f} zusammen mit dem Stickstoffatom, an das sie beide gebunden sind, ein Heterocyclyl bilden;
R^{g} Halogen CN, -OH, C₁₋₆ -Alkyl, C₃₋₆-Cycloalkyl oder Aryl ist;
n null, 1, 2 oder 3 ist; und
p null, 1 oder 2 ist.

6. Verbindung nach Anspruch 1, aufweisend die Formel (VI): 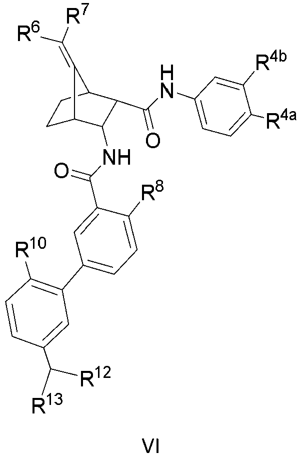 oder ein pharmazeutisch annehmbares Salz davon, wobei:
R^{4a} Halogen ist;
R^{4b} CF₃ ist;
R⁶ C₁₋₄-Alkyl, substituiert mit 0-3 Halogensubstituenten, oder C₃₋₆-Cycloalkyl, substituiert mit 0-3 Halogensubstituenten, ist;
R⁷ H ist;
R⁸ -OC₁₋₄-Alkyl, substituiert mit 0-1 Arylsubstituenten, ist;
R¹⁰ Halogen ist;
R¹² -C(=O)OH, -C(=O)OC₁₋₄-Alkyl, -C(=O)NHC₁₋₄-Alkyl, -C(=O)NHOC₁₋₃-Alkyl oder C₁₋₃-Alkyl, substituiert mit 0-3 Halogensubstituenten, ist;
R¹³ -OR^{b}, -NR^{a}R^{a}, -NR^{a}C(=O)R^{b}, -NR^{a}C(=O)OR^{b}, -NR^{a}S(=O)ₚR^{c}, -NR^{a}S(=O)ₚNR^{a}R^{a}, - OC(=O)NR^{a}R^{a} oder -OC(=O)NR^{a}OR^{b} ist;
R^{a} H, C₁₋₄-Alkyl, substituiert mit 0-5 Halogensubstituenten, Phenyl, substituiert mit 0-4 R^{e}, C₃₋₁₀-Cycloalkyl, substituiert mit 0-4 R^{e}, Spirocycloalkyl, substituiert mit 0-4 R^{e}oder 3- bis 9-gliedriges Heterocyclyl, umfassend 1-4 Heteroatome, ausgewählt aus O, S(=O)ₚ, und N, und substituiert mit 0-4 R^{e} ist; oder R^{a} und R^{a} zusammen mit dem Stickstoffatom, an das sie beide gebunden sind, ein 3- bis 12-gliedriges Heterocyclyl, umfassend 1-4 Heteroatome, die aus O ausgewählt sind S(=O)_{p und} N, und substituiert mit 0-4^{Re}, bilden;
R^{b} H, C₁₋₄-Alkyl, substituiert mit 0-5 R^{e}, -(CH₂)ₙ-Phenyl, substituiert mit 0-4 Halogensubstituenten, C₃₋₆ Cycloalkyl, substituiert mit 0-4 Halogensubstituenten, oder 3- bis 12-gliedriges Heterocyclyl, umfassend 1-4 Heteroatome, ausgewählt aus O, S(=O)_{p und} N, und substituiert mit 0-4 R^{e}, ist;
R^{c} C₁₋₄-Alkyl, substituiert mit 0-4 R^{e}, ist,
R^{e} Halogen, CN, =O, C₁₋₅-Alkyl, substituiert mit 0-5 R^{g}, C₃₋₆-Cycloalkyl, Aryl, 4- bis 6-gliedriges Heterocyclyl , umfassend 1-4 Heteroatome, ausgewählt aus O, S(=O)ₚ und N, oder -OR^{f} ist;
R^{f} H, C₁₋₄ -Alkyl, C₃₋₆-Cycloalkyl oder Aryl ist;
R^{g} Halogen ist;
n null oder 1 ist; und
p null, 1 oder 2 ist.

7. Verbindung nach Anspruch 6 oder ein pharmazeutisch annehmbares Salz davon, wobei
R^{4a} F ist;
R^{4b} CF₃ ist;
R⁶ CF₃ oder C₃₋₆-Cycloalkyl ist;
R⁸ -OCH₃ oder -OCH₂-Phenyl ist;
R¹⁰ F ist;
R¹² -C(=O)OH, -C(=O)OC₁₋₄-Alkyl, -C(=O)NHC₁₋₄-Alkyl, -C(=O)NHOC₁₋₃-Alkyl, CH₃, CHF₂ oder CF₃ ist;
R¹³ -OH, -NR^{a}R^{a}, -NHC(=O)R^{b}, -NHS(=O)ₚC₁₋₄ -Alkyl, -OC(=O)NR^{a}R^{a} oder -OC(=O)NHOC₁₋₄-Alkyl ist;
R^{a} H, C₁₋₄ -Alkyl, substituiert mit 0-4 F-Substituenten, 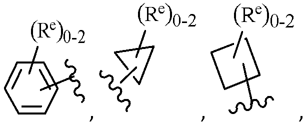 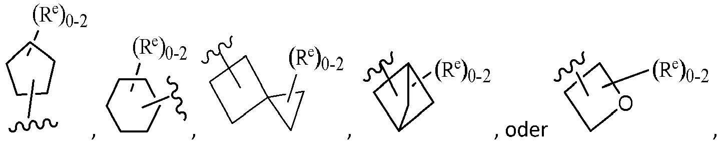 ist;
oder R^{a} und R^{a} zusammen 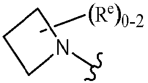 ist,
R^{b} H, C₁₋₄-Alkyl, substituiert mit 0-5^{Re}, Phenyl oder 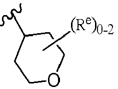 ist und
^{R} Halogen, =O, Aryl, 4- bis 6-gliedriges Heterocyclyl, umfassend 1-4 Heteroatome, ausgewählt aus O, S(=O)_{p und} N, oder -OR^{f} ist; und
R^{f} H, C₁₋₃ -Alkyl, C₃₋₆-Cycloalkyl oder Phenyl ist.

8. Verbindung nach Anspruch 3, aufweisend die Formel (VII): 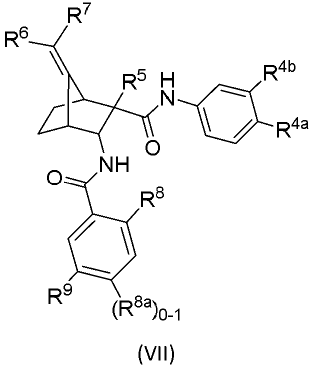 oder ein pharmazeutisch annehmbares Salz davon, wobei:
R^{4a} Halogen ist;
R^{4b} C₁₋₄-Alkyl, substituiert mit 0-3 Halogensubstituenten, oder -OC₁₋₄-Alkyl, substituiert mit 0-3 Halogensubstituenten, ist;
R⁵ H oder F ist;
R⁶ Halogen, CN, C₁₋₆-Alkyl, substituiert mit 0-3 R^{6a}, C₂₋₆-Alkenyl, substituiert mit 0-3 R^{6a}, C₂₋₆-Alkinyl, substituiert mit 0-3 R^{6a}, C₃₋₆-Cycloalkyl, substituiert mit 0-3 R¹⁴, C₃₋₆-Cycloalkenyl, substituiert mit 0-3 R¹⁴, Phenyl, substituiert mit 0-3 R¹⁴, oder 5- bis 6-gliedriges Heteroaryl, umfassend 1-3 Heteroatome, ausgewählt aus O, S(=O)ₚ, N und NR^{14a}, und substituiert mit 0-3 R¹⁴, ist;
R^{6a} Halogen, C₃₋₆ Cycloalkyl oder Phenyl ist;
R⁷ H oder C₁₋₂-Alkyl ist;
R⁸ Halogen, CN oder -OC₁₋₄₋-Alkyl, substituiert mit 0-4 Halogen-, -OH- oder -OC₁₋₄₋-Alkylsubstituenten, ist;
R^{8a} Halogen oder CN ist;
R⁹ ein 3- bis 12-gliedriges Heterocyclyl, umfassend 1-4 Heteroatome, ausgewählt aus O, S(=O)ₚ, N und NR^{11a}, und substituiert mit 0-3 R¹⁰ und 0-1 R¹¹, ist;
R¹⁰ Halogen, CN, C₁₋₃-Alkyl, =O, -OH oder -OC₁₋₃-Alkyl ist;
R¹¹ C₁₋₃-Alkyl, substituiert mit 0-1 R¹² und 0-1 R¹³, -OR^{b}, -NR^{a}R^{a}, -NR^{a}C(=O)R^{b}, - NR^{a}C(=O)OR^{b}, -NR^{a}C(=O)NR^{a}R^{a}, -NR^{a}S(=O)ₚR^{c}, -C(=O)R^{b}, -C(=O)OR^{b}, -C(=O)NR^{a}R^{a}, -C(=O)NR^{a}S(=O)ₚR^{c}, -OC(=O)R^{b}, -S(=O)ₚR^{c}, -S(=O)ₚNR^{a}R^{a}, C₃₋₆-Cycloalkyl, substituiert mit 0-5 R^{e}, 4- bis 6-gliedriges Heterocyclyl, umfassend 1-4 Heteroatome, ausgewählt aus O, S(=O)ₚ, N und NR¹⁵, und substituiert mit 0-4 R^{e}, ist;
R^{11a} H, C₁₋₄-Alkyl, substituiert mit 0-2 R^{11b}, -C(=O)R^{b}, -C(=O)OR^{b}, -C(=O)NR^{a}R^{a}, C₃₋₆-Cycloalkyl, 4- bis 6-gliedriges Heterocyclyl, umfassend 1-4 Heteroatome, ausgewählt aus O, S(=O)ₚ, N und NR¹⁵, und substituiert mit 0-4 R^{e}, ist;
R^{11b} -OH, -C(=O)OH, oder Aryl ist;
R¹² -C(=O)OR^{b}, -C(=O)NHR^{a}, -C(=O)NHOR^{b} oder C₁₋₄-Alkyl, substituiert mit 0-3 Halogen- oder -OH-Substituenten, ist;
R¹³ ist -OR^{b}, -NR^{a}R^{a}, -NR^{a}C(=O)R^{b}, -NR^{a}C(=O)OR^{b}, -NR^{a}S(=O)ₚR^{c}, -NR^{a}S(=O)ₚNR^{a}R^{a}, - OC(=O)NR^{a}R^{a}, -S(=O)ₚNR^{a}R^{a}, oder -S(=O)ₚR^{c};
R¹⁴ Halogen, CN, C₁₋₄-Alkyl, substituiert mit 0-3 Halogensubstituenten, -OC₁₋₄ -Alkyl, substituiert mit 0-3 Halogensubstituenten, -(CH₂)₀₋₂-NR^{a}R^{a}, -(CH₂)₀₋₂-Aryl, substituiert mit 0-3 R^{e}, -O-Aryl, substituiert mit 0-3 R^{e}, oder -(CH₂)₀₋₂-3- bis 12-gliedriges Heterocyclyl, umfassend 1-4 Heteroatome, ausgewählt aus O, S(=O)_{p und} N, und substituiert mit 0-3 R^{e}, ist;
R^{14a} H, C(=O)C₁₋₃ -Alkyl oder C₁₋₃ -Alkyl, substituiert mit 0-2 Aryl, substituiert mit 0-2 Halogensubstituenten, ist;
R¹⁵ H, C₁₋₂-Alkyl oder Phenyl ist;
R^{a} H, C₁₋₅ -Alkyl, substituiert mit 0-4 R^{e}, C₂₋₅ -Alkenyl, substituiert mit 0-4 R^{e}, C₂₋₅ -Alkinyl, substituiert mit 0-4 R^{e}, -(CH₂)ₙ-C₃₋₁₀ -Carbocyclyl, substituiert mit 0-4 R^{e}, oder -(CH₂)ₙ-3- bis 12-gliedriges Heterocyclyl, umfassend 1-4 Heteroatome, ausgewählt aus O, S(=O)_{p und} N, und substituiert mit 0-4 R^{e}, ist; oder R^{a} und R^{a} zusammen mit dem Stickstoffatom, an das sie beide gebunden sind, ein 3- bis 12-gliedriges Heterocyclyl, umfassend 1-4 Heteroatome, ausgewählt aus O, S(=O)_{p und} N, und substituiert mit 0-4 R^{e}, bilden;
R^{b} H, C₁₋₅ -Alkyl, substituiert mit 0-4 R^{e}, C₂₋₅ -Alkenyl, substituiert mit 0-4 R^{e}, C₂₋₅ -Alkinyl, substituiert mit 0-4 R^{e}, -(CH₂)ₙ-C₃₋₁₀ -Carbocyclyl, substituiert mit 0-4 R^{e}, oder -(CH₂)ₙ-3- bis 12-gliedriges Heterocyclyl, umfassend 1-4 Heteroatome, ausgewählt aus O, S(=O)_{p und} N, und substituiert mit 0-4 R^{e}, ist;
R^{c} ist ein C₁₋₅ Alkyl, substituiert mit 0-4^{Re}, ein C₂₋₅ Alkenyl, substituiert mit 0-4^{Re}, ein C₂₋₅ Alkinyl, substituiert mit 0-4^{Re}, ein C₃₋₆ Carbocyclyl oder ein 3- bis 12-gliedriges Heterocyclyl , umfassend 1-4 Heteroatome, ausgewählt aus O, S(=O)ₚ, und N;
R^{e} Halogen, CN, =O, C₁₋₆-Alkyl, substituiert mit 0-4 R^{g}, C₂₋₆-Alkenyl, substituiert mit 0-4 R^{g}, C₂₋₆-Alkinyl, substituiert mit 0-4 R^{g}, -(CH₂)ₙ-C₃₋₆-Cycloalkyl, substituiert mit 0-4 R^{g}, -(CH₂)ₙ-Aryl, substituiert mit 0-4 R^{g},-(CH₂)ₙ-4- bis 6-gliedrige Heterocyclen, umfassend 1-4 Heteroatome, ausgewählt aus O, S(=O)ₚ, und N, und substituiert mit 0-4 R^{g}, -(CH₂)ₙOR^{f}, C(=O)OR^{f}, C(=O)NR^{f}R^{f}, NR^{f}C(=O)R^{f}, S(=O)ₚR^{f}, NR^{f}S(=O)ₚR^{f}, NR^{f}C(=O)OR^{f}, OC(=O)NR^{f}R^{f}, oder -(CH₂)ₙNR^{f}R^{f}, ist;
R^{f} H, C₁₋₆ -Alkyl, C₃₋₆-Cycloalkyl oder Aryl ist;
R^{g} Halogen, CN, -OH, C₁₋₄ -Alkyl, C₃₋₆-Cycloalkyl oder Phenyl ist;
n null, 1, 2 oder 3 ist; und
p null, 1 oder 2 ist.

9. Verbindung nach Anspruch 8 oder ein pharmazeutisch annehmbares Salz davon, wobei R^{4a} Halogen ist;
R^{4b} C₁₋₄-Alkyl, substituiert mit 0-3 Halogensubstituenten, ist;
R⁵ H ist;
R⁶ C₁₋₂-Alkyl, substituiert mit 0-2 F-Substituenten, oder C₃₋₆ Cycloalkyl ist;
R⁸ -OC₁₋₃-Alkyl ist;
R^{8a} F oder CN ist;
R⁹ 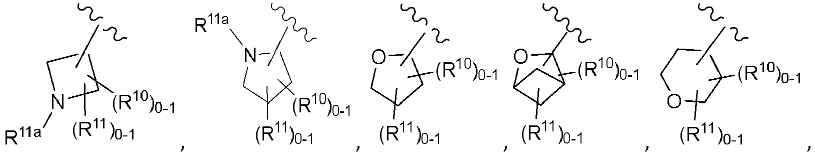 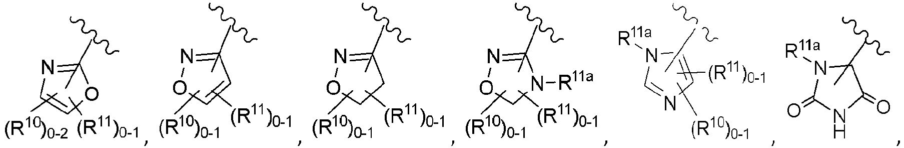 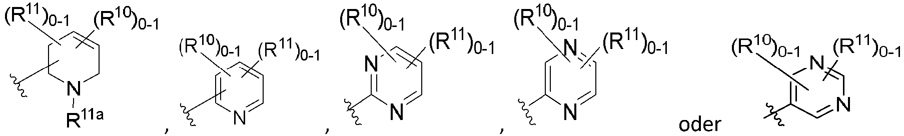 ist;
R¹⁰ Halogen, CN, C₁₋₂-Alkyl, =O, -OH oder -OC₁₋₂-Alkyl ist;
R¹¹ C₁₋₃-Alkyl, substituiert mit 0-1 R¹² und 0-1 R¹³, -OR^{b}, -NR^{a}R^{a}, -NR^{a}C(=O)R^{b}, -C(=O)R^{b}, -C(=O)OR^{B}, -C(=O)NR^{a}R^{a}, oder C₃₋₆-Cycloalkyl, substituiert mit 0-5 R^{e} ist;
R^{11a} H, -C(=O)R^{b}, -C(=O)NR^{a}R^{a} oder C₁₋₄-Alkyl, substituiert mit 0-1R^{11b}, ist;
R^{11b} -OH oder Aryl ist;
R¹² -C(=O)OR^{b}, -C(=O)NHR^{a}, -C(=O)NHOR^{b} oder C₁₋₄-Alkyl, substituiert mit 0-2 Halogen- oder - OH-Substituenten, ist;
R¹³ -OH, -OC₁₋₄-Alkyl, substituiert mit 0-2 -OH Substituenten, oder -S(=O)₂C₁₋₄-Alkyl ist;
R^{a} H oder C₁₋₄-Alkyl ist oder R^{a} und R^{a} zusammen mit dem Stickstoffatom, an das sie beide gebunden sind, ein 3 bis 9-gliedriges Heterocyclyl, substituiert mit 0-4 R^{e}, bilden;
R^{b} H, C₁₋₄-Alkyl, substituiert mit 0-1 R^{e}, oder C₃₋₆-Cycloalkyl, substituiert mit 0-1 ^{R}e, ist;
R^{e} -OR^{f} ist; und
R^{f} H oder C₁₋₄-Alkyl ist.

10. Verbindung nach Anspruch 9 oder ein pharmazeutisch annehmbares Salz davon, wobei
R^{4a} Halogen ist;
R^{4b} CF₃ ist;
R⁵ H ist;
R⁶ CF₃ oder C₃₋₆-Cyclopropyl ist;
R⁸ -OC₁₋₃-Alkyl ist;
R⁹ 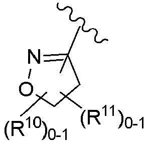 ist;
R¹⁰ C₁₋₂-Alkyl, -OH oder -OC₁₋₂-Alkyl ist;
R¹¹ C₁₋₃-Alkyl, substituiert mit 0-1 R¹² und 0-1 R¹³, -C(=O)OR^{b}oder -C(=O)NR^{a}R^{a} ist;
R¹² -C(=O)OR^{b} ist;
R¹³ -OH ist;
R^{a} H oder C₁₋₄-Alkyl ist; und
R^{b} H oder C₁₋₄-Alkyl ist.

11. Verbindung nach Anspruch 8 oder ein pharmazeutisch annehmbares Salz davon, wobei
R^{4a} Halogen ist;
R^{4b} C₁₋₄-Alkyl, substituiert mit 0-3 Halogensubstituenten, ist;
R⁵ H ist;
R⁶ C₁₋₃-Alkyl, substituiert mit 0-3 F-Substituenten, oder C₃₋₆-Cycloalkyl, ist;
R⁸ -OC₁₋₃-Alkyl ist;
R⁹ 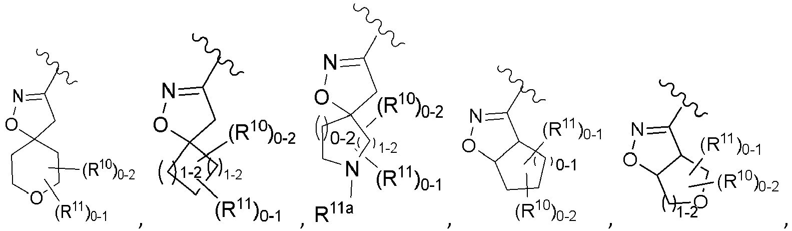 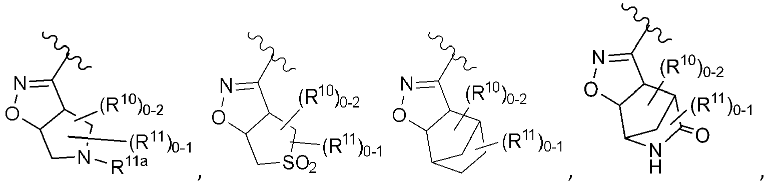 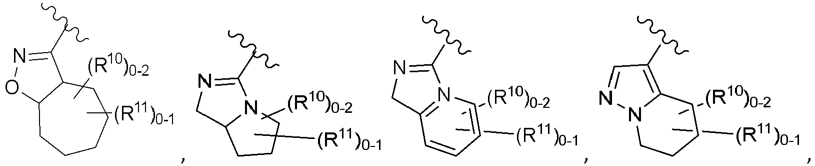 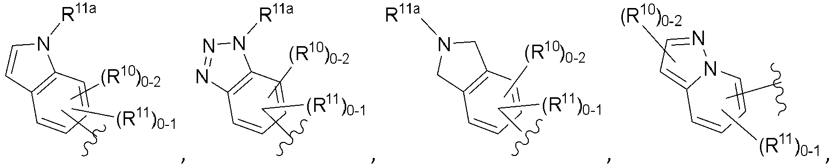 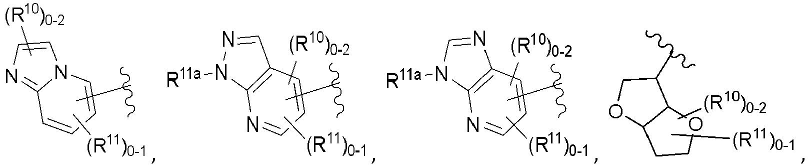 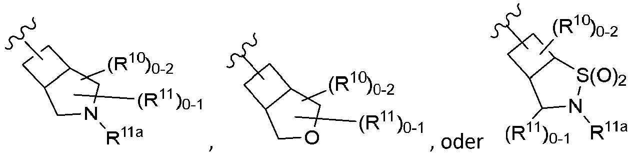 ist;
R¹⁰ Halogen, C₁₋₃-Alkyl, -OH oder -OC₁₋₃-Alkyl ist;
R¹¹ C₁₋₃-Alkyl, substituiert mit 0-1 R¹² und 0-1 R¹³, ist;
R^{11a} H, C₁₋₄-Alkyl, substituiert mit 0-2 R^{11b}, oder -C(=O)OC₁₋₄-Alkyl ist;
R^{11b} -OH, -C(=O)OH, oder Aryl ist;
R¹² -C(=O)OR^{b} oder C₁₋₃-Alkyl, substituiert mit 0-3 Halogensubstituenten, ist;
R¹³ -OH ist; und
R^{b} H oder C₁₋₄-Alkyl ist.

12. Verbindung nach Anspruch 3, aufweisend die Formel (VIII): 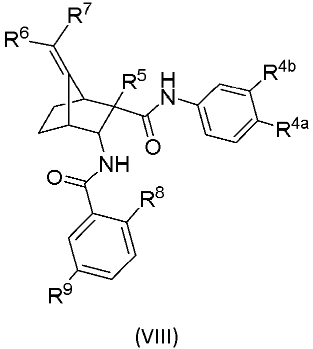 oder ein pharmazeutisch annehmbares Salz davon, wobei:
R^{4a} Halogen ist;
R^{4b} C₁₋₄-Alkyl, substituiert mit 0-4 Halogensubstituenten, ist;
R⁶ C₁₋₂-Alkyl, substituiert mit 0-2 F-Substituenten, C₃₋₆-Cycloalkyl oder Aryl ist;
R⁷ H ist;
R⁸ -OC₁₋₃-Alkyl ist;
R⁹ 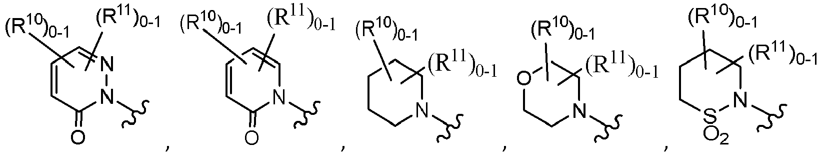 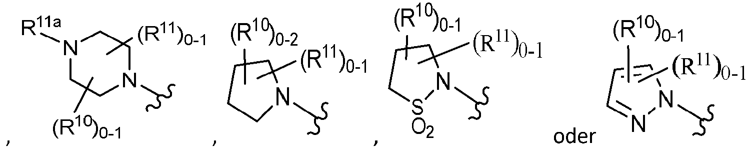 ist;
R¹⁰ Halogen, CN, C₁₋₄-Alkyl, =O, -OH oder -OC₁₋₄-Alkyl ist;
R¹¹ C₁₋₂-Alkyl, substituiert mit 0-1 R¹² und 0-1 R¹³, -NR^{a}R^{a}, -NR^{a}C(=O)R^{b}, -NR^{a}C(=O)OR^{b} oder -C(=O)OR^{B} ist;
R¹² -C(=O)OR^{b}, -C(=O)NHR^{a}, -C(=O)NHOR^{b} oder C₁₋₄-Alkyl, substituiert mit 0-3 Halogen- oder -OH-Substituenten, ist;
R¹³ -OH oder -NR^{a}C(=O)R^{b} ist;
R^{a} H oder C₁₋₄-Alkyl ist; und
R^{b} H, C₁₋₄-Alkyl oder ein 3- bis 9-gliedriges Heterocyclyl, umfassend 1-4 Heteroatome, ausgewählt aus O, S(=O)ₚund N, ist.

13. Verbindung nach Anspruch 1, aufweisend die Formel (IX): 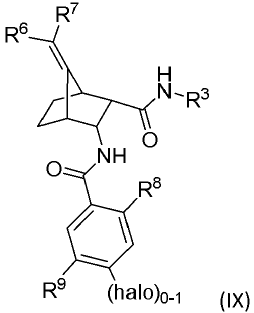 oder ein pharmazeutisch annehmbares Salz davon, wobei:
R³ C₁₋₅ -Alkyl, CF₃, -(CR^{d}R^{d})₀₋₁-C₃₋₆ -Cycloalkyl, substituiert mit 0-4 R⁴, oder Phenyl, substituiert mit 0-4 R⁴, ist ;
R⁴ Halogen, CN, CH₃oder CF₃ ist;
R⁶ C₁₋₆-Alkyl, CF₃oder C₃₋₆-Cycloalkyl, substituiert mit 0-2 F-Substituenten, ist;
R⁷ H ist;
R⁸ Halogen, -N(C₁₋₃ -Alkyl)₂, -OC₁₋₃-Alkyl, substituiert mit 0-1 -OC₁₋₄ Alkylsubstituenten, ist;
R⁹ 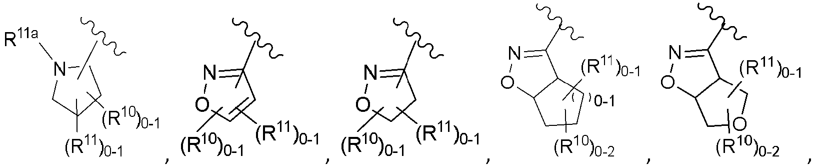 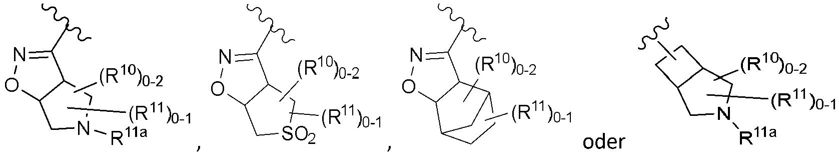 ist;
R¹⁰ Halogen, C₁₋₄-Alkyl, -OH oder -OC₁₋₄-Alkyl ist;
R¹¹ C₁₋₄-Alkyl, substituiert mit 0-2 R¹² und 0-2 R¹³, -C(=O)OR^{b}, -C(=O)NR^{a}R^{a} oder C₃₋₆-Cycloalkyl, substituiert mit 0-2 R^{e}, ist;
R^{11a} H, C₁₋₄-Alkyl, substituiert mit 0-2 R^{11b}, -C(=O)R^{b}oder -C(=O)OC₁₋₄-Alkyl ist;
R^{11b} -OH ist;
R¹² C₁₋₃-Alkyl, substituiert mit 0-3 Halogensubstituenten, oder -C(=O)OR^{b} ist;
R¹³ -OH ist;
R^{a} H oder C₁₋₃-Alkyl ist;
R^{b} H oder C₁₋₄-Alkyl, substituiert mit 0-1 R^{e} ist;
R^{e} -OR^{f} ist; und
R^{f} H oder C₁₋₆ -Alkyl ist.

14. Verbindung nach Anspruch 1, aufweisend die Formel (X): 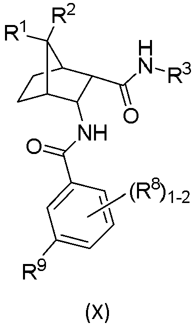 oder ein pharmazeutisch annehmbares Salz davon, wobei:
R¹ und R² kombiniert sind, um =CR⁶R⁷ zu sein;
R³ C₁₋₆-Alkyl, substituiert mit 0-5 Halogen-, CN- oder -OC₁₋₃ -Alkylsubstituenten, -(CHR^{d})ₙ-C₃₋₁₀-Carbocyclyl, substituiert mit 0-5 R⁴, oder 5- bis 6-gliedriges Heteroaryl, umfassend 1-3 Heteroatome, ausgewählt aus O, S, N, substituiert mit 0-3 R⁴, ist;
R⁴ Halogen, S(=O)₂CF₃, CN oder C₁₋₄-Alkyl, substituiert mit 0-5 Halogensubstituenten, ist;
R⁶ Halogen, C₁₋₅-Alkyl, substituiert mit 0-3 R^{6a}, C₃₋₆-Cycloalkyl, substituiert mit 0-3 R¹⁴, oder 5- bis 6-gliedriger Heterocyclyl, umfassend 1-3 Heteroatome, ausgewählt aus O, S und N, und substituiert mit 0-3 R¹⁴, ist;
R^{6a} Halogen, -OH oder C₃₋₆-Cycloalkyl ist;
R⁷ H ist;
R⁸ H, Halogen, CN, C₁₋₄-Alkyl oder -OC₁₋₄-Alkyl, substituiert mit 0-5 Halogen, -OH, C₃₋₆-Cycloalkyl oder -OC₁₋₄-Alkylsubstituenten, ist; 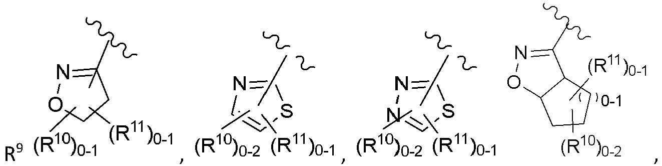 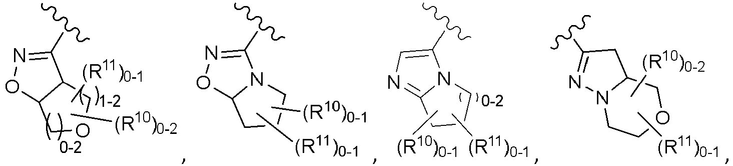 oder 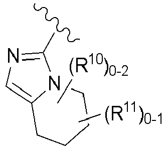 ist;
R¹⁰ Halogen, CN, C₁₋₄-Alkyl oder -OH ist;
R¹¹ C₁₋₃-Alkyl, substituiert mit 0-3 R¹² und 0-1 R¹³, -OR^{b}, -NHC(=O)R^{b}oder -C(=O)OR^{B} ist;
R¹² Halogen ist;
R¹³ -OR^{b} oder C₃₋₆-Carbocyclyl ist;
R¹⁴ Halogen, CN oder C₁₋₄-Alkyl, substituiert mit 0-3 Halogensubstituenten, ist;
R^{b} H oder C₁₋₃-Alkyl, substituiert mit 0-5 R^{e}, ist;
R^{d} H oder C₁₋₄-Alkyl ist,
^{R}e -OH ist und
n null oder 1 ist.

15. Verbindung nach Anspruch 14, aufweisend die Formel (XI): 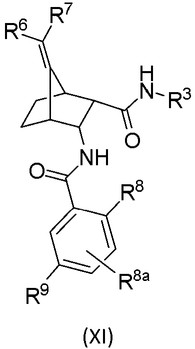 oder ein pharmazeutisch annehmbares Salz davon, wobei:
R³ C₁₋₅-Alkyl oder 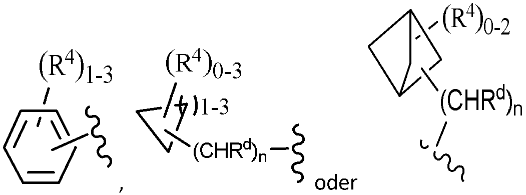 ist;
R⁴ Halogen, CN, -S(=O)₂CF₃, C₁₋₄-Alkyl, substituiert mit 0-5 Halogensubstituenten, ist;
R⁶ C₁₋₅-Alkyl, substituiert mit 0-2 R^{6a}, C₃₋₆-Cycloalkyl, substituiert mit 0-2 R¹⁴, oder 5- bis 6-gliedriges Heterocyclyl, umfassend 1-3 Heteroatome, ausgewählt aus O, S und N, und substituiert mit 0-2 R¹⁴, ist;
R^{6a} Halogen, -OH oder C₃₋₆-Cycloalkyl ist;
R⁷ H ist;
R⁸ -OC₁₋₃-Alkyl, substituiert mit 0-5 Halogen, -OH, C₃₋₆-Cycloalkyl oder -OC₁₋₃-Alkylsubstituenten, ist;
R^{8a} H, Halogen, CN oder C₁₋₃-Alkyl ist; 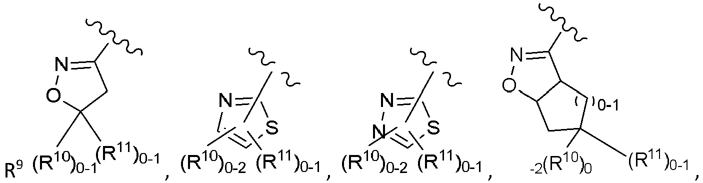 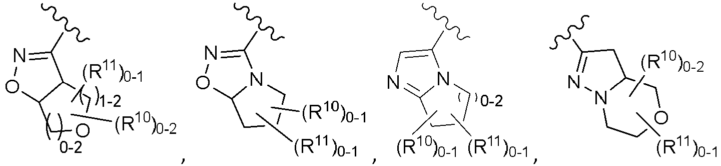 oder 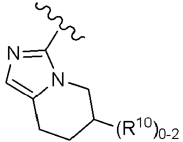 ist;
R¹⁰ Halogen, CN, C₁₋₄-Alkyl oder -OH ist;
R¹¹ C₁₋₃-Alkyl, substituiert mit 0-3 R¹² und 0-1 R¹³, -OR^{b}, -NHC(=O)R^{b}oder -C(=O)OR^{B} ist;
R¹² Halogen ist;
R¹³ -OR^{b} oder C₃₋₆-Carbocyclyl ist;
R¹⁴ Halogen oder C₁₋₄-Alkyl, substituiert mit 0-3 Halogensubstituenten, ist;
R^{b} H oder C₁₋₃-Alkyl, substituiert mit 0-5 R^{e}, ist;
R^{d} H oder C₁₋₂ -Alkyl ist; und
n null oder 1 ist.

16. Verbindung nach Anspruch 1, ausgewählt aus: 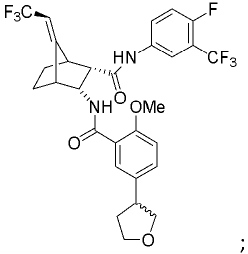 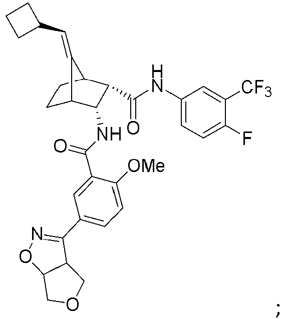 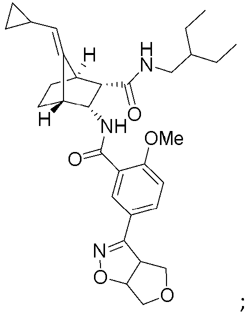 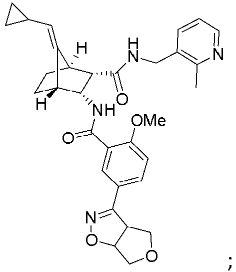 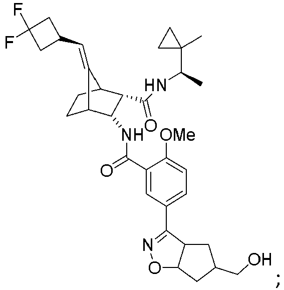 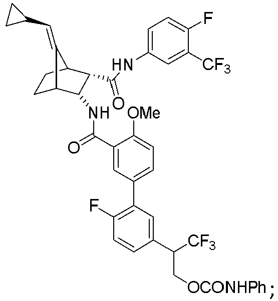 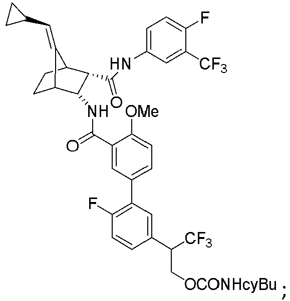 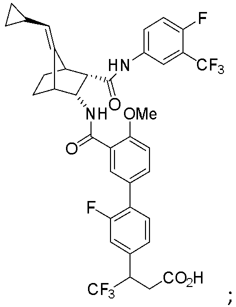 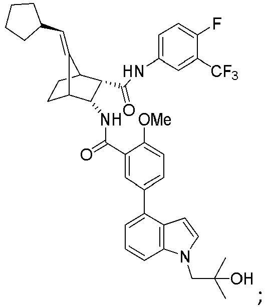 oder ein pharmazeutisch annehmbares Salz davon.

17. Pharmazeutische Zusammensetzung, die eine Verbindung nach einem der Ansprüche 1 bis 16 oder ein pharmazeutisch annehmbares Salz davon und einen pharmazeutisch annehmbaren Träger umfasst.

18. Verbindung nach einem der Ansprüche 1 bis 16 oder ein pharmazeutisch akzeptables Salz davon oder eine pharmazeutische Zusammensetzung nach Anspruch 17, zur Verwendung in der Therapie.

## Revendications

1. Composé de Formule (I) : 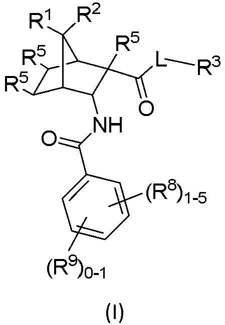 ou sel pharmaceutiquement acceptable de celui-ci, dans lequel :
L représente -O- ou -NH- ;
R¹ et R² sont combinés pour être =CR⁶R⁷ ou =NO-alkyle en C₁₋₄ dans lequel « = » est une double liaison ;
ou R¹ et R² forment ensemble avec l'atome de carbone auquel ils sont tous deux fixés un dioxolanyle substitué par 0-1 substituant aryle ;
R³ représente alkyle en C₁₋₈ substitué par 0-5 substituants halo, CN, -OH ou -O-alkyle en C₁₋₃, -(CR^{d}R^{d})ₙ-carbocyclyle en C₃₋₁₀ substitué par 0-5 R⁴, ou -(CR^{d}R^{d})ₙ-hétérocyclyle à 3 à 12 chaînons comprenant 1-4 hétéroatomes choisis parmi O, S(=O)ₚ, N et NR^{4c}, et substitués par 0-5 R⁴ ;
R⁴ représente halo, CN, -OH, SF₅, -S(=O)ₚR^{c}, alkyle en C₁₋₄ substitué par 0-5 substituants halo, -OH ou -O-alkyle en C₁₋₄, -O-alkyle en C₁₋₄ substitué par 0-5 substituants halo, -(CR^{d}R^{d})ₙ-carbocyclyle en C₃₋₁₀ substitué par 0-5^{Re}, ou -(CR^{d}R^{d})ₙ-hétérocyclyle à 4 à 6 chaînons comprenant 1-4 hétéroatomes choisis parmi O, S(=O)ₚ, N et NR^{4c}, et substitué par 0-5^{Re} ;
R^{4c} représente H, alkyle en C₁₋₄ ou -S(=O)₂CF₃ ;
chaque R⁵ représente H, halo, -OH, alkyle en C₁₋₄ substitué par 0-5 substituants halo ou - O-alkyle en C₁₋₄ substitué par 0-5 substituants halo ;
R⁶ représente H, halo, CN, alkyle en C₁₋₇ substitué par 0-3 R^{6a}, alcényle en C₂₋₇ substitué par 0-3 R^{6a}, alcynyle en C₂₋₇ substitué par 0-3 R^{6a}, -C(=O)OR^{6b}, -C(=O)NR^{6b}R^{6b}, -(CH₂)ₙ-carbocyclyle en C₃₋₁₀ substitué par 0-5 R¹⁴ ou hétérocyclyle à 3 à 12 chaînons comprenant 1-4 hétéroatomes choisis parmi O, S(=O)ₚ, N ou NR^{14a}, et substitué par 0-5 R¹⁴ ;
R^{6a} représente halo, -OH, -O-alkyle en C₁₋₄, alkyle en C₁₋₄, aryle ou cycloalkyle en C₃₋₆ substitué par 0-4 substituants halo ;
R^{6b} représente H, alkyle en C₁₋₄ substitué par 0-1 substituant aryle, ou cycloalkyle en C₃₋₆ substitué par 0-4 substituants halo ;
R⁷ représente H ou alkyle en C₁₋₄,
ou R⁶ et R⁷ forment ensemble avec l'atome de carbone auquel ils sont tous deux fixés un cyclopentadiényle, un indanyle ou un indényle ;
R⁸ représente H, halo, CN, -NR⁷R⁷, alkyle en C₁₋₄ substitué par 0-5 substituants halo ou - OH, ou -O-alkyle en C₁₋₄ substitué par 0-5 substituants halo, -OH, cycloalkyle en C₃₋₆, aryle, hétérocyclyle à 4 à 9 chaînons comprenant 1-4 hétéroatomes choisis parmi O, S(=O)ₚ et N, ou -O-alkyle en C₁₋₄ substitué par 0-1 substituant -O-alkyle en C₁₋₃ ;
R⁹ représente aryle substitué par 0-3 R¹⁰ et 0-2 R¹¹ ou hétérocycle à 3 à 12 chaînons comprenant 1 à 5 hétéroatomes choisis parmi O, S(=O)ₚ, N et NR^{11a}, et substitué par 0-3 R¹⁰ et 0-2 R¹¹ ;
R¹⁰ représente halo, CN, alkyle en C₁₋₄, =O, -OH ou -O-alkyle en C₁₋₄ ;
R¹¹ représente alkyle en C₁₋₄ substitué par 0-4 R¹²et 0-2 R¹³, -OR^{b}, -NR^{a}R^{a}, -NR^{a}C(=O)R^{b}, - NR^{a}C(=O)OR^{b}, -NR^{a}C(=O)NR^{a}R^{a}, -NR^{a}S(=O)ₚR^{c}, -C(=O)R^{b}, -C(=O)OR^{b}, -C(=O)NR^{a}R^{a}, -C(=O)NR^{a}S(=O)ₚR^{c}, -OC(=O)R^{b}, -S(=O)ₚR^{c}, -S(=O)ₚNR^{a}R^{a}, carbocyclyle en C₃₋₆ substitué par 0-5 R^{e} ou hétérocyclyle à 3 à 12 chaînons comprenant 1-4 hétéroatomes choisis parmi O, S(=O)ₚ, N et NR¹⁵, et substitué par 0-5 R^{e} ;
R^{11a} représente H, alkyle en C₁₋₅ substitué par 0-4 R^{11b}, -C(=O)R^{b}, -C(=O)OR^{b}, -C(=O)NR^{a}R^{a}, cycloalkyle en C₃₋₆ substitué par 0-5 R^{e}, aryle substitué par 0-5 R^{e}, hétérocycle à 4 à 6 chaînons comprenant 1-4 hétéroatomes choisis parmi O, S(=O)ₚ, N et NR¹⁵, et substitué par 0-5 R^{e} ;
R^{11b} représente halo, -OH, -C(=O)OH, -C(=O)O-alkyle en C₁₋₄ ou aryle ;
R¹² représente halo, -C(=O)OR^{b}, -C(=O)NR^{a}R^{a}, -C(=O)NR^{a}OR^{b} ou alkyle en C₁₋₄ substitué par 0-3 substituants halo ou -OH, ou cycloalkyle en C₃₋₆ ;
R¹³ représente -OR^{b}, -NR^{a}R^{a}, -NR^{a}C(=O)R^{b}, -NR^{a}C(=O)OR^{b}, -NR^{a}C(=O)NR^{a}R^{a}, -NR^{a}S(=O)ₚR^{c}, -NR^{a}S(=O)ₚNR^{a}R^{a}, -OC(=O)NR^{a}R^{a}, -OC(=O)NR^{a}OR^{b}, -S(=O)ₚNR^{a}R^{a}, -S(=O)ₚR^{c}, -(CH₂)ₙ-carbocyclyle en C₃₋₁₀ substitué par 0-3 R^{e} ou -(CH₂)ₙ-hétérocyclyle à 3 à 12 chaînons comprenant 1-4 hétéroatomes choisis parmi O, S(=O)ₚ et N, et substitué par 0-3 R^{e} ;
R¹⁴ représente halo, CN, alkyle en C₁₋₄ substitué par 0-3 substituants halo, -O-alkyle en C₁₋₄ substitué par 0-3 substituants halo, -(CH₂)ₙ-NR^{a}R^{a}, -(CH₂)ₙ-aryle substitué par 0-3 R^{e}, -O-aryle substitué par 0-3 R^{e}, ou -(CH₂)ₙ-hétérocyclyle à 3 à 12 chaînons comprenant 1-4 hétéroatomes choisis parmi O, S(=O)ₚ et N, et substitué par 0-3 R^{e};
R^{14a} représente H, C(=O)-alkyle en C₁₋₄, ou alkyle en C₁₋₃ substitué par 0-3 Si(alkyle en C₁₋₃)₃ ou aryle substitué par 0-2 substituants halo ;
R¹⁵ représente H, alkyle en C₁₋₄ ou aryle ;
R^{a} représente H, -O-alkyle en C₁₋₆, alkyle en C₁₋₆ substitué par 0-5 R^{e}, alcényle en C₂₋₆ substitué par 0-5 R^{e}, alcynyle en C₂₋₆ substitué par 0-5 R^{e}, -(CH₂)ₙ-carbocyclyle en C₃₋₁₀ substitué par 0-5 R^{e} ou -(CH₂)ₙ-hétérocyclyle à 3 à 12 chaînons comprenant 1-4 hétéroatomes choisis parmi O, S(=O)ₚet N, et substitué par 0-5 R^{e} ; ou R^{a} et R^{a} forment ensemble avec l'atome d'azote auquel ils sont tous deux fixés un hétérocycle à 3 à 12 chaînons comprenant 1-4 hétéroatomes choisis parmi O, S(=O)ₚet N, et substitués par 0-5 R^{e} ;
R^{b} représente H, alkyle en C₁₋₆ substitué par 0-5 R^{e}, alcényle en C₂₋₆ substitué par 0-5 R^{e}, alcynyle en C₂₋₆ substitué par 0-5 R^{e}, -(CH₂)ₙ-carbocyclyle en C₃₋₁₀ substitué par 0-5 R^{e} ou -(CH₂)ₙ-hétérocyclyle à 3 à 12 chaînons comprenant 1-4 hétéroatomes choisis parmi O, S(=O)ₚ et N, et substitué par 0-5 R^{e} ;
R^{c} représente alkyle en C₁₋₆ substitué par 0-5 R^{e}, alcényle en C₂₋₆ substitué par 0-5 R^{e}, alcynyle en C₂₋₆ substitué par 0-5 R^{e}, carbocyclyle en C₃₋₆ substitué par 0-5 R^{e} ou hétérocyclyle à 3 à 12 chaînons comprenant 1-4 hétéroatomes choisis parmi O, S(=O)ₚ et N, et substitué par 0-5 R^{e} ;
R^{d} représente H, alkyle en C₁₋₄ ou cycloalkyle en C₃₋₆ ;
R^{e} représente halo, CN, NO₂, =O, alkyle en C₁₋₆ substitué par 0-5 R^{g}, alcényle en C₂₋₆ substitué par 0-5 R^{g}, alcynyle en C₂₋₆ substitué par 0-5 R^{g}, -(CH₂)ₙ-carbocyclyle en C₃₋₁₀ substitué par 0-5 R^{g}, -(CH₂)ₙ-hétérocyclyle à 3 à 12 chaînons comprenant 1-4 hétéroatomes choisis parmi O, S(=O)ₚ et N, et substitué par 0-5 R^{g}, -(CH₂)ₙOR^{f}, -C(=O)OR^{f}, -C(=O)NR^{f}R^{f}, -NR^{f}C(=O)R^{f}, -S(=O)ₚR^{f}, -S(=O)ₚNR^{f}R^{f}, - NR^{f}S(=O)ₚR^{f}, -NR^{f}C(=O)OR^{f}, -OC(=O)NR^{f}R^{f} ou -(CH₂)ₙNR^{f}R^{f} ;
R^{f} représente H, alkyle en C₁₋₅ substitué par 0-2 substituants -OH ou -O-alkyle en C₁₋₄, cycloalkyle en C₃₋₆, aryle ou hétérocyclyle à 3 à 12 chaînons comprenant 1-4 hétéroatomes choisis parmi O, S(=O)ₚet N ; ou R^{f} et R^{f} forment ensemble avec l'atome d'azote auquel ils sont tous deux fixés un hétérocyclyle à 3 à 12 chaînons comprenant 1-4 hétéroatomes choisis parmi O, S(=O)ₚ et N ;
R^{g} représente halo, CN, -OH, alkyle en C₁₋₆, cycloalkyle en C₃₋₆ ou aryle ;
n vaut zéro, 1, 2 ou 3 ; et
p vaut zéro, 1 ou 2.

2. Composé selon la revendication 1, ou sel pharmaceutiquement acceptable de celui-ci, dans lequel :
R³ représente alkyle en C₁₋₆ substitué par 0-4 substituants halo ou -OH, -(CHR^{d})₀₋₁-cycloalkyle en C₃₋₆ substitué par 0-4 R⁴, spirocycloalkyle en C₆₋₉ substitué par 0-4 R⁴, carbocyclyle bicyclique en C₆₋₁₀ substitué par 0-4 R⁴ ou hétérocyclyle à 3 à 6 chaînons comprenant 1-2 hétéroatomes choisis parmi O, S(=O)ₚ, N et NR^{4c}, et substitué par 0-4 R⁴ ;
R⁴ représente halo ou alkyle C₁₋₃substitué par 0-4 substituants halo ;
R^{4c} représente H ou alkyle en C₁₋₄ ; et
R^{d} représente alkyle en C₁₋₃.

3. Composé selon la revendication 1, présentant la formule (II) : 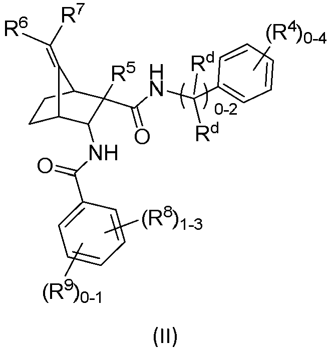 ou sel pharmaceutiquement acceptable de celui-ci, dans lequel :
R⁴ représente halo, -S(=O)ₚ-alkyle en C₁₋₄ substitué par 0-4 substituants halo, alkyle en C₁₋₄ substitué par 0-4 substituants halo ou -O-alkyle en C₁₋₄ substitué par 0-4 substituants halo ;
R⁵ représente H ou halo ;
R⁶ représente halo, CN, alkyle en C₁₋₇ substitué par 0-3 R^{6a}, alcényle en C₂₋₇ substitué par 0-3 R^{6a}, alcynyle en C₂₋₇ substitué par 0-3 R^{6a}, C(=O)OR^{6b}, -C(=O)NR^{6b}R^{6b}, cycloalkyle en C₃₋₆ substitué par 0-3 R¹⁴, cycloalcényle en C₃₋₆ substitué par 0-3 R¹⁴, aryle substitué par 0-3 R¹⁴ ou hétérocyclyle à 4 à 6 chaînons comprenant 1-3 hétéroatomes choisis parmi O, S(=O)ₚ, N et NR^{14a} et substitué par 0-3 R¹⁴ ;
R^{6a} représente halo, -OH, cycloalkyle en C₃₋₆ ou aryle ;
R^{6b} représente H, alkyle en C₁₋₄ substitué par 0-1 substituant aryle ou cycloalkyle en C₃₋₆ substitué par 0-4 substituants halo ;
R⁷ représente H ou alkyle en C₁₋₃ ;
R⁸ représente halo, CN, -N(alkyle en C₁₋₂)₂, alkyle en C₁₋₄ substitué par 0-5 substituants halo ou -OH, ou -O-alkyle en C₁₋₄ substitué par 0-4 substituants halo, -OH, aryle ou -O-alkyle en C₁₋₄ ;
R⁹ représente aryle substitué par 0-3 R¹⁰ et 0-2 R¹¹ ou hétérocyclyle à 3 à 12 chaînons comprenant 1-4 hétéroatomes choisis parmi O, S(=O)ₚ, N et NR^{11a}, et substitué par 0-3 R¹⁰ et 0-1 R¹¹ ;
R¹⁰ représente halo, CN, alkyle en C₁₋₄, =O, -OH ou -O-alkyle en C₁₋₄ ;
R¹¹ représente alkyle en C₁₋₄ substitué par 0-3 R¹² et 0-1 R¹³, -OR^{b}, -NR^{a}R^{a},-NR^{a}C(=O)R^{b}, - NR^{a}C(=O)OR^{b}, -NR^{a}C(=O)NR^{a}R^{a}, -NR^{a}S(=O)ₚR^{c}, -C(=O)R^{b}, -C(=O)OR^{b}, -C(=O)NR^{a}R^{a}, -C(=O)NR^{a}S(=O)ₚR^{c}, -OC(=O)R^{b}, -S(=O)ₚR^{c}, -S(=O)ₚNR^{a}R^{a}, cycloalkyle en C₃₋₆ substitué par 0-5 R^{e}, hétérocyclyle à 4 à 12 chaînons comprenant 1-4 hétéroatomes choisis parmi O, S(=O)ₚ, N et NR¹⁵, et substitué par 0-5 R^{e} ;
R^{11a} représente H, alkyle en C₁₋₄ substitué par 0-2 R^{11b}, -C(=O)R^{b}, -C(=O)OR^{b}, -C(=O)NR^{a}R^{a}, cycloalkyle en C₃₋₆ substitué par 0-5 R^{e}, hétérocyclyle à 4 à 6 chaînons comprenant 1-4 hétéroatomes choisis parmi O, S(=O)ₚ, N et NR¹⁵, et substitué par 0-5 R^{e} ;
R^{11b} représente -OH, -C(=O)OH ou aryle ;
R¹²représente halo, -C(=O)OR^{b}, -C(=O)NHR^{a}, -C(=O)NHOR^{b}, ou alkyle en C₁₋₄ substitué par 0-3 substituants halo ou -OH ;
R¹³ représente -OR^{b}, -NR^{a}R^{a}, -NR^{a}C(=O)R^{b}, -NR^{a}C(=O)OR^{b}, -NR^{a}S(=O)ₚR^{c}, -NR^{a}S(=O)ₚNR^{a}R^{a}, -OC(=O)NR^{a}R^{a}, -OC(=O)NR^{a}OR^{b}, -S(=O)ₚNR^{a}R^{a} ou -S(=O)ₚR^{c} ;
R¹⁴ représente halo, CN, alkyle en C₁₋₄ substitué par 0-3 substituants halo, -O-alkyle en C₁₋₄ substitué par 0-3 substituants halo, -(CH₂)₀₋₃-NR^{a}R^{a}, -(CH₂)₀₋₃-aryle substitué par 0-3 R^{e}, -O-aryle substitué par 0-3 R^{e} ou -(CH₂)₀₋₂-hétérocyclyle à 3 à 12 chaînons comprenant 1-4 hétéroatomes choisis parmi O, S(=O)ₚ et N, et substitué par 0-3 R^{e} ;
R^{14a} représente H, C(=O)-alkyle en C₁₋₄ ou alkyle en C₁₋₃ substitué par 0-3 substituants aryle substitué par 0-2 substituants halo ;
R¹⁵ représente H, alkyle en C₁₋₃ ou aryle ;
R^{a} représente H, alkyle en C₁₋₅ substitué par 0-5 R^{e}, alcényle en C₂₋₅ substitué par 0-5 R^{e}, alcynyle en C₂₋₅ substitué par 0-5 R^{e}, -(CH₂)ₙ-carbocyclyle en C₃₋₁₀ substitué par 0-5 R^{e} ou -(CH₂)ₙ-hétérocyclyle à 3 à 12 chaînons comprenant 1-4 hétéroatomes choisis parmi O, S(=O)ₚet N, et substitué par 0-5 R^{e} ; ou R^{a} et R^{a} forment ensemble avec l'atome d'azote auquel ils sont tous deux fixés un hétérocyclyle à 3 à 12 chaînons comprenant 1-4 hétéroatomes choisis parmi O, S(=O)ₚet N, et substitués par 0-5 R^{e} ;
R^{b} représente H, alkyle en C₁₋₅ substitué par 0-5 R^{e}, alcényle en C₂₋₅ substitué par 0-5 R^{e}, alcynyle en C₂₋₅ substitué par 0-5 R^{e}, -(CH₂)ₙ-carbocyclyle en C₃₋₁₀ substitué par 0-5 R^{e} ou -(CH₂)ₙ-hétérocyclyle à 3 à 12 chaînons comprenant 1-4 hétéroatomes choisis parmi O, S(=O)ₚ et N, et substitué par 0-5 R^{e} ;
R^{c} représente alkyle en C₁₋₅ substitué par 0-5 R^{e}, alcényle en C₂₋₅ substitué par 0-5 R^{e}, alcynyle en C₂₋₅ substitué par 0-5 R^{e}, carbocyclyle en C₃₋₆ substitué par 0-5 R^{e} ou hétérocycle à 3 à 12 chaînons comprenant 1-4 hétéroatomes choisis parmi O, S(=O)ₚ et N, et substitué par 0-5 R^{e} ;
R^{d} représente H ou alkyle en C₁₋₄ ;
R^{e} représente halo, CN, =O, alkyle en C₁₋₆ substitué par 0-5 R^{g}, alcényle en C₂₋₆ substitué par 0-5 R^{g}, alcynyle en C₂₋₆ substitué par 0-5 R^{g}, -(CH₂)ₙ-cycloalkyle en C₃₋₆ substitué par 0-4 R^{g}, -(CH₂)ₙ-aryle substitué par 0-4 R^{g}, -(CH₂)ₙ-hétérocyclyle à 4 à 6 chaînons comprenant 1-4 hétéroatomes choisis parmi O , S(=O)ₚ et N, et substitué avec 0-4 R^{g}, -(CH₂)ₙOR^{f}, -C(=O)OR^{f}, -C(=O)NR^{f}R^{f}, -NR^{f}C(=O)R^{f}, -S(=O)ₚR^{f}, -NR^{f}C(=O)OR^{f}, -OC(=O)NR^{f}R^{f}, ou -(CH₂)ₙNR^{f}R^{f} ;
R^{f} représente H, alkyle en C₁₋₅, cycloalkyle en C₃₋₆ ou aryle ; ou R^{f} et R^{f} forment ensemble avec l'atome d'azote auquel ils sont tous deux fixés un hétérocycle à 3 à 9 chaînons ;
R^{g} représente halo, CN, -OH, alkyle en C₁₋₅, cycloalkyle en C₃₋₆ ou aryle ;
n vaut zéro, 1, 2 ou 3 ; et
p vaut zéro, 1 ou 2.

4. Composé selon la revendication 3, présentant la formule (IV) : 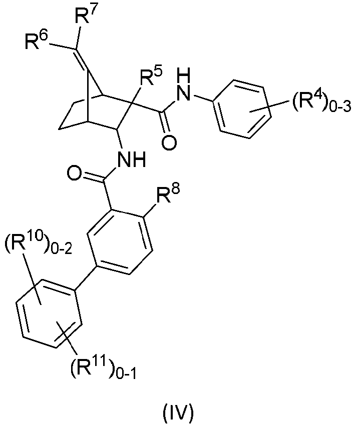 ou sel pharmaceutiquement acceptable de celui-ci, dans lequel :
R⁴ représente halo, alkyle en C₁₋₄ substitué par 0-3 substituants halo ou -O-alkyle en C₁₋₄ substitué par 0-3 substituants halo ;
R⁵ représente H ou F ;
R⁶ représente halo, CN, alkyle en C₁₋₆ substitué par 0-3 R^{6a}, alcényle en C₂₋₆ substitué par 0-3 R^{6a}, alcynyle en C₂₋₆ substitué par 0-3 R^{6a}, -C(=O)OR^{6b}, C(=O)NR^{6b}R^{6b}, cycloalkyle en C₃₋₆ substitué par 0-3 R¹⁴, cycloalcényle en C₃₋₆ substitué par 0-3 R¹⁴, phényle substitué par 0-3 R¹⁴ ou hétéroaryle à 5 à 6 chaînons comprenant 1-3 hétéroatomes choisis parmi O, S(=O)ₚ, N et NR^{14a}, et substitué par 0-3 R¹⁴ ;
R^{6a} représente halo, cycloalkyle en C₃₋₆ ou phényle ;
R^{6b} représente H, alkyle en C₁₋₃ substitué par 0-1 substituant aryle, ou cycloalkyle en C₃₋₆ substitué par 0-4 substituants halo ;
R⁷ représente H ou alkyle en C₁₋₂ ;
R⁸ représente -O-alkyle en C₁₋₄ substitué par 0-4 substituants halo, -OH, aryle ou -O-alkyle en C₁₋₄ ;
R¹⁰ représente halo, CN, alkyle en C₁₋₃, -OH ou -O-alkyle en C₁₋₄ ;
R¹¹ représente alkyle en C₁₋₄ substitué par 0-2 R¹² et 0-1 R¹³,-OR^{b}, -NR^{a}R^{a}, -NR^{a}C(=O)R^{b}, - NR^{a}C(=O)NR^{a}R^{a}, -NR^{a}S(=O)ₚR^{c}, -C(=O)R^{b}, -C(=O)OR^{b}, -C(=O)NR^{a}R^{a}, -C(=O)NR^{a}S(=O)ₚR^{c}, -OC(=O)R^{b}, -S(=O)ₚR^{c}, -S(=O)ₚNR^{a}R^{a}, cycloalkyle en C₃₋₆, hétérocyclyle à 4 à 9 chaînons comprenant 1-4 hétéroatomes choisis parmi O, S(=O)ₚ, N et NR¹⁵, et substitué par 0-4 R^{e} ;
R¹² représente halo, -C(=O)OR^{b}, -C(=O)NHR^{a}, -C(=O)NHOR^{b} ou alkyle en C₁₋₄ substitué par 0-3 substituants halo ou -OH ;
R¹³ représente -OR^{b}, -NR^{a}R^{a}, -NR^{a}C(=O)R^{b}, -NR^{a}C(=O)OR^{b}, -NR^{a}S(=O)ₚR^{c}, -NR^{a}S(=O)ₚNR^{a}R^{a}, -OC(=O)NR^{a}R^{a}, -OC(=O)NR^{a}OR^{b}, -S(=O)ₚNR^{a}R^{a} ou -S(=O)ₚR^{c} ;
R¹⁴ représente halo, CN, alkyle en C₁₋₄ substitué par 0-3 substituants halo, -O-alkyle en C₁₋₄ substitué par 0-3 substituants halo, -(CH₂)₀₋₂-NR^{a}R^{a}, -(CH₂)₀₋₂-aryle substitué par 0-3 R^{e}, -O-aryle substitué par 0-3 R^{e} ou -(CH₂)₀₋₂-hétérocyclyle à 3 à 12 chaînons comprenant 1-4 hétéroatomes choisis parmi O, S(=O)ₚ et N, et substitué par 0-3 R^{e} ;
R^{14a} représente H, C(=O)-alkyle en C₁₋₃, alkyle en C₁₋₃ substitué par 0-2 aryle substitué par 0-2 substituants halo ;
R¹³ représente H, alkyle en C₁₋₂ ou phényle ;
R^{a} représente H, alkyle en C₁₋₅ substitué par 0-4 R^{e}, alcényle en C₂₋₅ substitué par 0-4 R^{e}, alcynyle en C₂₋₅ substitué par 0-4 R^{e}, -(CH₂)ₙ-carbocyclyle en C₃₋₁₀ substitué par 0-4 R^{e}, ou -(CH₂)ₙ-hétérocyclyle à 3 à 12 chaînons comprenant 1-4 hétéroatomes choisis parmi O, S(=O)ₚet N, et substitué par 0-4 R^{e} ; ou R^{a} et R^{a} forment ensemble avec l'atome d'azote auquel ils sont tous deux fixés un hétérocycle à 3 à 12 chaînons comprenant 1-4 hétéroatomes choisis parmi O, S(=O)ₚet N, et substitué par 0-4 R^{e} ;
R^{b} représente H, alkyle en C₁₋₅ substitué par 0-4 R^{e}, alcényle en C₂₋₅ substitué par 0-4 R^{e}, alcynyle en C₂₋₅ substitué par 0-4 R^{e}, -(CH₂)ₙ-carbocyclyle en C₃₋₁₀ substitué par 0-4 R^{e} ou -(CH₂)ₙ-hétérocyclyle à 3 à 12 chaînons comprenant 1-4 hétéroatomes choisis parmi O, S(=O)ₚ et N, et substitué par 0-4 R^{e} ;
R^{c} représente alkyle en C₁₋₅ substitué par 0-4 R^{e}, alcényle en C₂₋₅ substitué par 0-4 R^{e}, alcynyle en C₂₋₅ substitué par 0-4 R^{e}, carbocyclyle en C₃₋₆ ou hétérocyclyle à 3 à 12 chaînons comprenant 1-4 hétéroatomes choisis parmi O, S(=O)ₚet N ;
R^{e} représente halo, CN, NO₂, =O, alkyle en C₁₋₆ substitué par 0-5 R^{g}, alcényle en C₂₋₆ substitué par 0-5 R^{g}, alcynyle en C₂₋₆ substitué par 0-5 R^{g}, -(CH₂)ₙ-cycloalkyle en C₃₋₆, -(CH₂)ₙ-aryle, - (CH₂)ₙ-hétérocyclyle à 4 à 6 chaînons comprenant 1-4 hétéroatomes choisis parmi O, S(=O)ₚet N, - (CH₂)ₙOR^{f}, S(=O)ₚR^{f}, C(=O)NR^{f}R^{f}, C(=O)OR^{f}, NR^{f}C(=O)R^{f}, S(=O)ₚNR^{f}R^{f}, NR^{f}S(=O)ₚR^{f}, NR^{f}C(=O)OR^{f}, OC(=O)NR^{f}R^{f}, ou -(CH₂)ₙNR^{f}R^{f} ;
R^{f} représente H, alkyle en C₁₋₆, cycloalkyle en C₃₋₆ ou aryle ; ou R^{f} et R^{f} forment ensemble avec l'atome d'azote auquel ils sont tous deux fixés un hétérocyclyle ;
R^{g} représente halo, CN, -OH, alkyle en C₁₋₅, cycloalkyle en C₃₋₆ ou aryle ;
n vaut zéro, 1, 2 ou 3 ; et
p vaut zéro, 1 ou 2.

5. Composé selon la revendication 1, présentant la formule (V) : 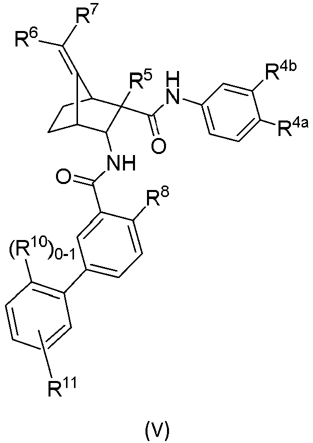 ou sel pharmaceutiquement acceptable de celui-ci, dans lequel :
R^{4a} représente halo ou alkyle en C₁₋₂ ;
R^{4b} représente alkyle en C₁₋₄ substitué par 0-4 substituants halo ;
R⁵ représente H ou F ;
R⁶ représente halo, CN, alkyle en C₁₋₄ substitué par 0-3 R^{6a}, alcényle en C₂₋₄ substitué par 0-3 R^{6a}, -C(=O)OR^{6b}, C(=O)NR^{6b}R^{6b}**,** cycloalkyle en C₃₋₆ substitué par 0-3 R¹⁴, phényle substitué par 0-3 R¹⁴ ou hétéroaryle à 5 à 6 chaînons comprenant 1-3 hétéroatomes choisis parmi O, S(=O)ₚ, N et NR^{14a} et substitué par 0-3 R¹⁴ ;
R^{6a} représente halo, -OH, cycloalkyle en C₃₋₆ ou phényle ;
R^{6b} représente H, alkyle en C₁₋₃ substitué par 0-1 substituant aryle ou cycloalkyle en C₃₋₆ ;
R⁷ représente H ou alkyle en C₁₋₂ ;
R⁸ représente -O-alkyle en C₁₋₄substitué par 0-4 substituants halo, -OH, -O-alkyle en C₁₋₄ ou aryle ;
R¹⁰ représente halo ou alkyle en C₁₋₃ ;
R¹¹ représente alkyle en C₁₋₄ substitué par 0-2 R¹² et 0-1 R¹³, -OH, -O-alkyle en C₁₋₄, -NR^{a}C(=O)R^{b}, -NR^{a}C(=O)NR^{a}R^{a}, -NR^{a}S(=O)ₚR^{c}, -C(=O)R^{b}, -C(=O)OR^{b}, -C(=O)NR^{a}R^{a}, -C(=O)NR^{a}S(=O)ₚR^{c}, -OC(=O)R^{b}, -S(=O)ₚR^{c}, -S(=O)ₚNR^{a}R^{a}, cycloalkyle en C₃₋₆, hétérocyclyle à 4 à 9 chaînons comprenant 1-4 hétéroatomes choisis parmi O, S(=O)ₚ, N et NR¹⁵, et substitué par 0-3 R^{e} ;
R¹² représente halo, -C(=O)OR^{b}, -C(=O)NHR^{a}, -C(=O)NHOR^{b}, ou alkyle en C₁₋₄ substitué par 0-3 substituants halo ou -OH ;
R¹³ représente -OR^{b}, -NR^{a}R^{a}, -NR^{a}C(=O)R^{b}, -NR^{a}C(=O)OR^{b}, -NR^{a}S(=O)ₚR^{c}, -NR^{a}S(=O)ₚNR^{a}R^{a}, -OC(=O)NR^{a}R^{a} ou -OC(=O)NR^{a}OR^{b} ;
R¹⁴ représente halo, CN, alkyle en C₁₋₄ substitué par 0-3 substituants halo, -O-alkyle en C₁₋₄substitué par 0-3 substituants halo, -(CH₂)₀₋₂-NR^{a}R^{a}, -(CH₂)₀₋₁-aryle substitué par 0-3 R^{e}, -O-aryle substitué par 0-3 R^{e} ou -(CH₂)₀₋₁-hétérocyclyle à 3 à 9 chaînons comprenant 1-4 hétéroatomes choisis parmi O, S(=O)ₚ et N, et substitué par 0-3 R^{e} ;
R^{14a} représente H, C(=O)-alkyle en C₁₋₃, alkyle en C₁₋₃ substitué par 0-1 aryle substitué par 0-2 substituants halo ;
R¹⁵ représente H, alkyle en C₁₋₂ ou phényle ;
R^{a} représente H, alkyle en C₁₋₄ substitué par 0-5 R^{e}, alcényle en C₂₋₄ substitué par 0-5 R^{e}, alcynyle en C₂₋₄ substitué par 0-5 R^{e}, -(CH₂)ₙ-carbocyclyle en C₃₋₁₀ substitué par 0-5 R^{e} ou -(CH₂)ₙ-hétérocyclyle à 3 à 12 chaînons comprenant 1-4 hétéroatomes choisis parmi O, S(=O)ₚet N, et substitué par 0-5 R^{e} ; ou R^{a} et R^{a} forment ensemble avec l'atome d'azote auquel ils sont tous deux fixés un hétérocycle à 3 à 9 chaînons comprenant 1-4 hétéroatomes choisis parmi O, S(=O)ₚet N, et substitués par 0-5 R^{e} ;
R^{b} représente H, alkyle en C₁₋₄ substitué par 0-5 R^{e}, alcényle en C₂₋₄ substitué par 0-5 R^{e}, alcynyle en C₂₋₄ substitué par 0-5 R^{e}, -(CH₂)ₙ-carbocyclyle en C₃₋₁₀ substitué par 0-5 R^{e} ou -(CH₂)ₙ-hétérocyclyle à 3 à 12 chaînons comprenant 1-4 hétéroatomes choisis parmi O, S(=O)ₚ et N, et substitué par 0-5 R^{e} ;
R^{c} représente alkyle en C₁₋₄ substitué par 0-5 R^{e}, alcényle en C₂₋₄ substitué par 0-5 R^{e}, alcynyle en C₂₋₄ substitué par 0-5 R^{e}, carbocyclyle en C₃₋₆ ou hétérocyclyle à 3 à 9 chaînons comprenant 1-4 hétéroatomes choisis parmi O, S(=O)ₚet N ;
R^{e} représente halo, CN, =O, alkyle en C₁₋₆ substitué par 0-5 R^{g}, alcényle en C₂₋₆ substitué par 0-5 R^{g}, alcynyle en C₂₋₆ substitué par 0-5 R^{g}, -(CH₂)ₙ-cycloalkyle en C₃₋₆,-(CH₂)ₙ-aryle, -(CH₂)ₙ-hétérocyclyle à 4 à 6 chaînons comprenant 1-4 hétéroatomes choisis parmi O, S(=O)ₚet N, -(CH₂)ₙOR^{f}, -S(=O)ₚR^{f}, -C(=O)NR^{f}R^{f}, -C(=O)OR^{f}, -NR^{f}C(=O)R^{f}, -S(=O)ₚNR^{f}R^{f}, -NR^{f}S(=O)ₚR^{f}, -NR^{f}C(=O)OR^{f}, -OC(=O)NR^{f}R^{f} ou -(CH₂)ₙNR^{f}R^{f} ;
R^{f} représente H, alkyle en C₁₋₆, cycloalkyle en C₃₋₆ ou aryle ; ou R^{f} et R^{f} forment ensemble avec l'atome d'azote auquel ils sont tous deux fixés un hétérocyclyle ;
R^{g} représente halo, CN, -OH, alkyle en C₁₋₆, cycloalkyle en C₃₋₆ ou aryle ;
n vaut zéro, 1, 2 ou 3 ; et
p vaut zéro, 1 ou 2.

6. Composé selon la revendication 1, présentant la formule (VI) : 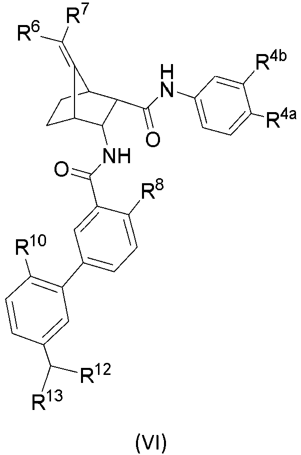 ou sel pharmaceutiquement acceptable de celui-ci, dans lequel :
R^{4a} représente halo ;
R^{4b} représente CF₃ ;
R⁶ représente alkyle en C₁₋₄ substitué par 0-3 substituants halo ou cycloalkyle en C₃₋₆ substitué par 0-3 substituants halo ;
R⁷ représente H ;
R⁸ représente -O-alkyle en C₁₋₄ substitué par 0-1 aryle ;
R¹⁰ représente halo ;
R¹² représente -C(=O)OH, -C(=O)O-alkyle en C₁₋₄, -C(=O)NH-alkyle en C₁₋₄, -C(=O)NHO-alkyle en C₁₋₃ ou alkyle en C₁₋₃ substitué par 0-3 substituants halo ;
R¹³ représente -OR^{b}, -NR^{a}R^{a}, -NR^{a}C(=O)R^{b}, -NR^{a}C(=O)OR^{b}, -NR^{a}S(=O)ₚR^{c}, -NR^{a}S(=O)ₚNR^{a}R^{a}, -OC(=O)NR^{a}R^{a} ou -OC(=O)NR^{a}OR^{b} ;
R^{a} représente H, alkyle en C₁₋₄ substitué par 0-5 substituants halo, phényle substitué par 0-4 R^{e}, cycloalkyle en C₃₋₁₀ substitué par 0-4 R^{e}, spirocycloalkyle substitué par 0-4 R^{e} ou hétérocyclyle à 3 à 9 chaînons comprenant 1-4 hétéroatomes choisis parmi O, S(=O)ₚ et N, et substitué par 0-4 R^{e} ; ou R^{a} et R^{a} forment ensemble avec l'atome d'azote auquel ils sont tous deux fixés un hétérocycle à 3 à 12 chaînons comprenant 1-4 hétéroatomes choisis parmi O, S(=O)ₚ et N, et substitués par 0-4 R^{e} ;
R^{b} représente H, alkyle en C₁₋₄ substitué par 0-5 R^{e}, -(CH₂)ₙ-phényle substitué par 0-4 substituants halo, cycloalkyle en C₃₋₆ substitué par 0-4 substituants halo ou hétérocyclyle à 3 à 12 chaînons comprenant 1-4 hétéroatomes choisis parmi O, S(=O)ₚ et N, et substitué par 0-4 R^{e} ;
R^{c} représente alkyle en C₁₋₄ substitué par 0-4 R^{e},
R^{e} représente halo, CN, =O, alkyle en C₁₋₅ substitué par 0-5 R^{g}, cycloalkyle en C₃₋₆, aryle, hétérocyclyle à 4 à 6 chaînons comprenant 1-4 hétéroatomes choisis parmi O, S(=O)ₚ et N, ou -OR^{f} ;
R^{f} représente H, alkyle en C₁₋₄, cycloalkyle en C₃₋₆ ou aryle ;
R^{g} représente halo ;
n vaut zéro ou 1 ; et
p vaut zéro, 1 ou 2.

7. Composé selon la revendication 6 ou sel pharmaceutiquement acceptable de celui-ci, dans lequel :
R^{4a} représente F
R^{4b} représente CF₃ ;
R⁶ représente CF₃ ou cycloalkyle en C₃₋₆ ;
R⁸ représente -OCH₃ ou -OCH₂-phényle ;
R¹⁰ représente F ;
R¹² représente -C(=O)OH, -C(=O)O-alkyle en C₁₋₄, -C(=O)NH-alkyle en C₁₋₄, -C(=O)NHO-alkyle en C1-3, CH₃, CHF₂ ou CF₃ ;
R¹³ représente -OH, -NR^{a}R^{a}, -NHC(=O)R^{b}, -NHS(=O)ₚ-alkyle en C₁₋₄, -OC(=O)NR^{a}R^{a} ou - OC(=O)NHO-alkyle en C₁₋₄ ;
R^{a} représente H, alkyle en C₁₋₄ substitué par 0-4 substituants F, 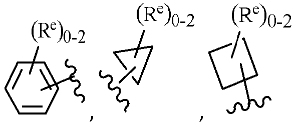 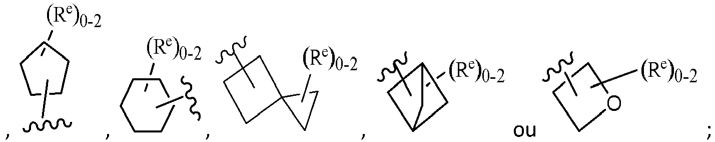
ou R^{a} et R^{a} représentent ensemble 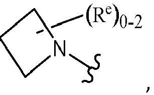
R^{b} représente H, alkyle en C₁₋₄ substitué par 0-5 R^{e}, phényle ou 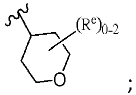 et
R^{e} représente halo, =O, aryle, hétérocycle à 4 à 6 chaînons comprenant 1-4 hétéroatomes choisis parmi O, S(=O)ₚ et N, ou -OR^{f} ; et
R^{f} représente H, alkyle en C₁₋₃, cycloalkyle en C₃₋₆ ou phényle.

8. Composé selon la revendication 3, présentant la formule (VII) : 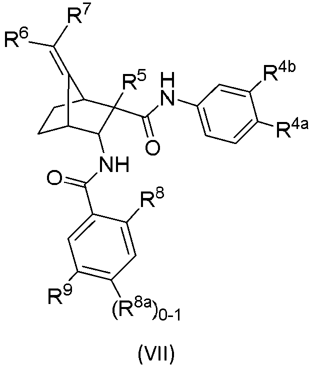 ou sel pharmaceutiquement acceptable de celui-ci, dans lequel :
R^{4a} représente halo ;
R^{4b} représente alkyle C₁₋₄ substitué par 0-3 substituants halo ou -O-alkyle en C₁₋₄ substitué par 0-3 substituants halo ;
R⁵ représente H ou F ;
R⁶ représente halo, CN, alkyle en C₁₋₆ substitué par 0-3 R^{6a}, alcényle en C₂₋₆ substitué par 0-3 R^{6a}, alcynyle en C₂₋₆ substitué par 0-3 R^{6a}, cycloalkyle en C₃₋₆ substitué par 0-3 R¹⁴, cycloalcényle en C₃₋₆ substitué par 0-3 R¹⁴, phényle substitué par 0-3 R¹⁴ ou hétéroaryle à 5 à 6 chaînons comprenant 1-3 hétéroatomes choisis parmi O, S(=O)ₚ, N et NR^{14a}, et substitué par 0-3 R¹⁴ ;
R^{6a} représente halo, cycloalkyle en C₃₋₆ ou phényle ;
R⁷ représente H ou alkyle en C₁₋₂ ;
R⁸ représente halo, CN ou -O-alkyle en C₁₋₄ substitué par 0-4 substituants halo, -OH, ou - O-alkyle en C₁₋₄ ;
R^{8a} représente halo ou CN ;
R⁹ représente hétérocycle à 3 à 12 chaînons comprenant 1-4 hétéroatomes choisis parmi O, S(=O)ₚ, N et NR^{11a}, et substitué par 0-3 R¹⁰ et 0-1 R¹¹ ;
R¹⁰ représente halo, CN, alkyle en C₁₋₃, =O, -OH ou -O-alkyle en C₁₋₃ ;
R¹¹ représente alkyle en C₁₋₃ substitué par 0-1 R¹² et 0-1 R¹³,-OR^{b}, -NR^{a}R^{a},-NR^{a}C(=O)R^{b}, - NR^{a}C(=O)OR^{b}, -NR^{a}C(=O)NR^{a}R^{a}, -NR^{a}S(=O)ₚR^{c}, -C(=O)R^{b}, -C(=O)OR^{b}, -C(=O)NR^{a}R^{a}, -C(=O)NR^{a}S(=O)ₚR^{c}, -OC(=O)R^{b}, -S(=O)ₚR^{c}, -S(=O)ₚNR^{a}R^{a}, cycloalkyle en C₃₋₆ substitué par 0-5 R^{e}, hétérocycle à 4 à 6 chaînons comprenant 1-4 hétéroatomes choisis parmi O, S(=O)ₚ, N et NR¹⁵, et substitué par 0-4 R^{e} ;
R^{11a} représente H, alkyle en C₁₋₄ substitué par 0-2 R^{11b}, -C(=O)R^{b}, -C(=O)OR^{b}, -C(=O)NR^{a}R^{a}, cycloalkyle en C₃₋₆, hétérocyclyle à 4 à 6 chaînons comprenant 1 à 4 hétéroatomes choisis parmi O, S(=O)ₚ, N et NR¹⁵, et substitué par 0-4 R^{e} ;
R^{11b} représente -OH, -C(=O)OH ou aryle ;
R¹² représente -C(=O)OR^{b}, -C(=O)NHR^{a}, -C(=O)NHOR^{b}, ou alkyle en C₁₋₄ substitué par 0-3 substituants halo ou -OH ;
R¹³ représente -OR^{b}, -NR^{a}R^{a}, -NR^{a}C(=O)R^{b}, -NR^{a}C(=O)OR^{b}, -NR^{a}S(=O)ₚR^{c}, -NR^{a}S(=O)ₚNR^{a}R^{a}, -OC(=O)NR^{a}R^{a}, -S(=O)ₚNR^{a}R^{a} ou -S(=O)ₚR^{c} ;
R¹⁴ représente halo, CN, alkyle en C₁₋₄ substitué par 0-3 substituants halo, -O-alkyle en C₁₋₄substitué par 0-3 substituants halo, -(CH₂)₀₋₂-NR^{a}R^{a}, -(CH₂)₀₋₂-aryle substitué par 0-3 R^{e}, -O-aryle substitué par 0-3 R^{e} ou -(CH₂)₀₋₂-hétérocyclyle à 3 à 12 chaînons comprenant 1-4 hétéroatomes choisis parmi O, S(=O)ₚ et N, et substitué par 0-3 R^{e} ;
R^{14a} représente H, C(=O)-alkyle en C₁₋₃ ou alkyle en C₁₋₃ substitué par 0-2 aryle substitué par 0-2 substituants halo ;
R¹⁵ représente H, alkyle en C₁₋₂ ou phényle ;
R^{a} représente H, alkyle en C₁₋₅ substitué par 0-4 R^{e}, alcényle en C₂₋₅ substitué par 0-4 R^{e}, alcynyle en C₂₋₅ substitué par 0-4 R^{e}, -(CH₂)ₙ-carbocyclyle en C₃₋₁₀ substitué par 0-4 R^{e} ou -(CH₂)ₙ-hétérocyclyle à 3 à 12 chaînons comprenant 1-4 hétéroatomes choisis parmi O, S(=O)ₚet N, et substitué par 0-4 R^{e} ; ou R^{a} et R^{a} forment ensemble avec l'atome d'azote auquel ils sont tous deux fixés un hétérocycle à 3 à 12 chaînons comprenant 1-4 hétéroatomes choisis parmi O, S(=O)ₚet N, et substitués par 0-4 R^{e} ;
R^{b} représente H, alkyle en C₁₋₅ substitué par 0-4 R^{e}, alcényle en C₂₋₅ substitué par 0-4 R^{e}, alcynyle en C₂₋₅ substitué par 0-4 R^{e}, -(CH₂)ₙ-carbocyclyle en C₃₋₁₀ substitué par 0-4 R^{e} ou -(CH₂)ₙ-hétérocyclyle à 3 à 12 chaînons comprenant 1-4 hétéroatomes choisis parmi O, S(=O)ₚ et N, et substitué par 0-4 R^{e} ;
R^{c} représente alkyle en C₁₋₅ substitué par 0-4 R^{e}, alcényle en C₂₋₅ substitué par 0-4 R^{e}, alcynyle en C₂₋₅ substitué par 0-4 R^{e}, carbocyclyle en C₃₋₆ ou hétérocycle à 3 à 12 chaînons comprenant 1-4 hétéroatomes choisis parmi O, S(=O)ₚet N ;
R^{e} représente halo, CN, =O, alkyle en C₁₋₆ substitué par 0-4 R^{g}, alcényle en C₂₋₆ substitué par 0-4 R^{g}, alcynyle en C₂₋₆ substitué par 0-4 R^{g}, -(CH₂)ₙ-cycloalkyle en C₃₋₆ substitué par 0-4 R^{g}, -(CH₂)ₙ-aryle substitué par 0-4R^{g}, -(CH₂)ₙ-hétérocyclyle à 4- à 6 chaînons comprenant 1-4 hétéroatomes choisis parmi O, S(=O)ₚ et N, et substitué par 0-4 R^{g}, -(CH₂)ₙOR^{f}, C(=O)OR^{f}, C(=O)NR^{f}R^{f}, NR^{f}C(=O)R^{f}, S(=O)ₚR^{f}, NR^{f}S(=O)ₚR^{f}, NR^{f}C(=O)OR^{f}, OC(=O)NR^{f}R^{f} ou -(CH₂)ₙNR^{f}R^{f} ;
R^{f} représente H, alkyle en C₁₋₆, cycloalkyle en C₃₋₆ ou aryle ;
R^{g} représente halo, CN, -OH, alkyle en C₁₋₄, cycloalkyle en C₃₋₆ ou phényle ;
n vaut zéro, 1, 2 ou 3 ; et
p vaut zéro, 1 ou 2.

9. Composé selon la revendication 8, ou sel pharmaceutiquement acceptable de celui-ci, dans lequel :
R^{4a} représente halo ;
R^{4b} représente alkyle en C₁₋₄ substitué par 0-3 substituants halo ;
R⁵ représente H ;
R⁶ représente alkyle en C₁₋₂ substitué par 0-2 substituants F ou cycloalkyle en C₃₋₆ ;
R⁸ représente -O-alkyle en C₁₋₃ ;
R^{8a} représente F ou CN ;
R⁹ représente 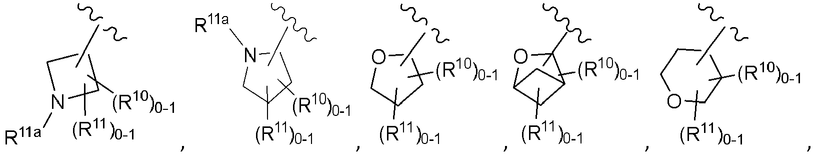 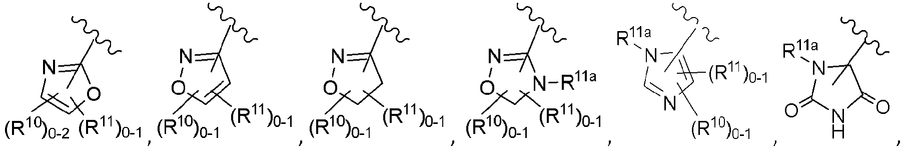 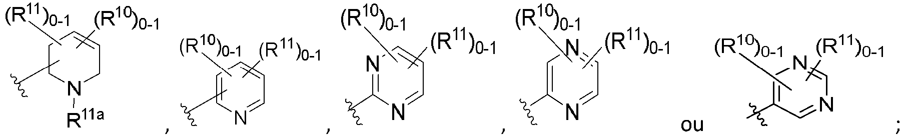
R¹⁰ représente halo, CN, alkyle en C₁₋₂, =O, -OH ou -O-alkyle en C₁₋₂ ;
R¹¹ représente alkyle en C₁₋₃ substitué par 0-1 R¹² et 0-1 R¹³, -OR^{b}, -NR^{a}R^{a}, -NR^{a}C(=O)R^{b}, -C(=O)R^{b}, -C(=O)OR^{b}, -C(=O)NR^{a}R^{a} ou cycloalkyle en C₃₋₆ substitué par 0-5 R^{e} ;
R^{11a} représente H, -C(=O)R^{b}, -C(=O)NR^{a}R^{a} ou alkyle en C₁₋₄ substitué par 0-1 R^{11b} ;
R^{11b} représente -OH ou aryle ;
R¹² représente -C(=O)OR^{b}, -C(=O)NHR^{a}, -C(=O)NHOR^{b}, ou alkyle en C₁₋₄ substitué par 0-2 substituants halo ou -OH ;
R¹³ représente -OH, -O-alkyle en C₁₋₄ substitué par 0-2 substituants -OH ou -S(=O)₂-alkyle en C₁₋4 ;
R^{a} représente H ou alkyle en C₁₋₄, ou R^{a} et R^{a} forment ensemble avec l'atome d'azote auquel ils sont tous deux fixés un hétérocyclyle à 3 à 9 chaînons substitué par O-4 R^{e} ;
R^{b} représente H, alkyle en C₁₋₄ substitué par 0-1 R^{e} ou cycloalkyle en C₃₋₆ substitué par 0-1 R^{e} ;
R^{e} représente -OR^{f} ; et
R^{f} représente H ou alkyle en C₁₋₄.

10. Composé selon la revendication 9, ou sel pharmaceutiquement acceptable de celui-ci, dans lequel :
R^{4a} représente halo ;
R^{4b} représente CF₃ ;
R⁵ représente H ;
R⁶ représente CF₃ ou cyclopropyle en C₃₋₆ ;
R⁸ représente -O-alkyle en C₁₋₃ ;
R⁹ représente 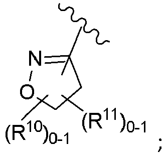
R¹⁰ représente alkyle en C₁₋₂, -OH ou -O-alkyle en C₁₋₂ ;
R¹¹ représente alkyle en C₁₋₃ substitué par 0-1 R¹² et 0-1 R¹³, -C(=O)OR^{b} ou -C(=O)NR^{a}R^{a} ;
R¹² représente -C(=O)OR^{b} ;
R¹³ représente -OH ;
R^{a} représente H ou alkyle en C₁₋₄ ; et
R^{b} représente H ou alkyle en C₁₋₄.

11. Composé selon la revendication 8, ou sel pharmaceutiquement acceptable de celui-ci, dans lequel :
R^{4a} représente halo ;
R^{4b} représente alkyle en C₁₋₄ substitué par 0-3 substituants halo ;
R⁵ représente H ;
R⁶ représente alkyle en C₁₋₃ substitué par 0-3 substituants F ou cycloalkyle en C₃₋₆ ;
R⁸ représente -O-alkyle en C₁₋₃ ;
R⁹ représente 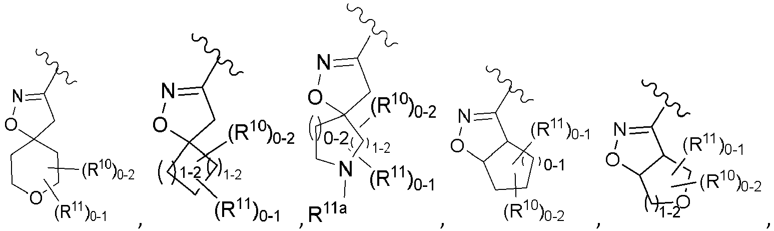 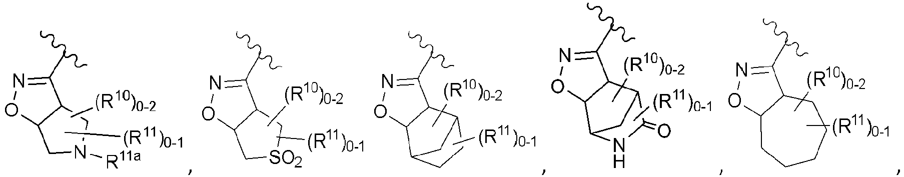 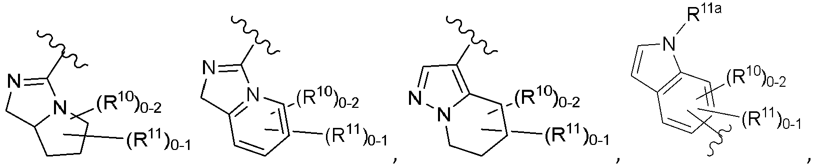 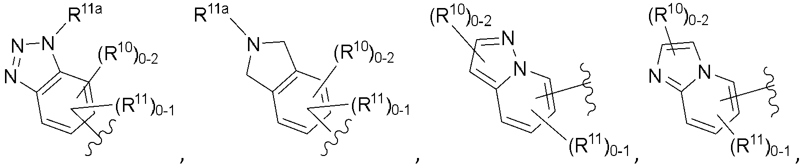 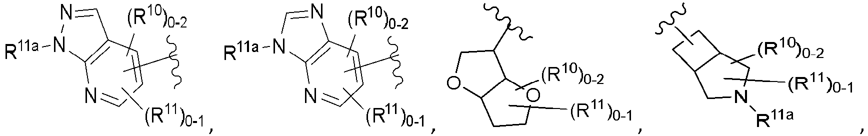 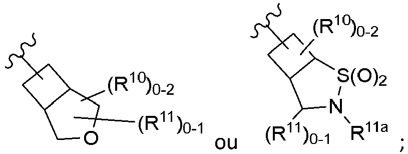
R¹⁰ représente halo, alkyle en C₁₋₃, -OH ou -O-alkyle en C₁₋₃ ;
R¹¹ représente alkyle en C₁₋₃ substitué par 0-1 R¹² et 0-1 R¹³ ;
R^{11a} représente H, alkyle en C₁₋₄ substitué par 0-2 R^{11b} ou -C(=O)O-alkyle en C₁₋₄ ;
R^{11b} représente -OH, -C(=O)OH ou aryle ;
R¹² représente -C(=O)OR^{b} ou alkyle en C₁₋₃ substitué par 0-3 substituants halo ;
R¹³ représente -OH ; et
R³ représente H ou alkyle en C₁₋₄.

12. Composé selon la revendication 3, présentant la formule (VIII) : 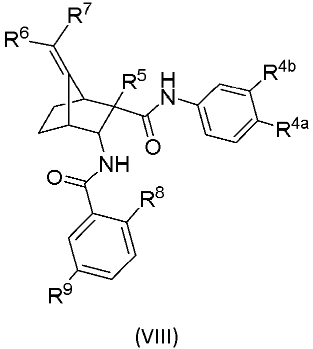 ou sel pharmaceutiquement acceptable de celui-ci, dans lequel :
R^{4a} représente halo ;
R^{4b} représente alkyle en C₁₋₄ substitué par 0-4 substituants halo ;
R⁶ représente alkyle en C₁₋₂ substitué par 0-2 substituants F, cycloalkyle en C₃₋₆ ou aryle ;
R⁷ représente H ;
R⁸ représente -O-alkyle en C₁₋₃ ;
R⁹ représente 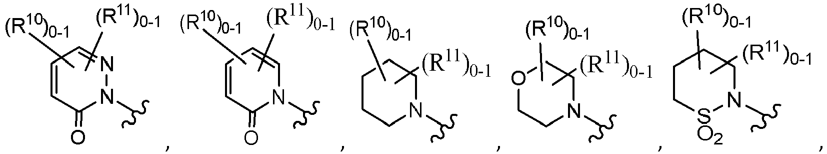 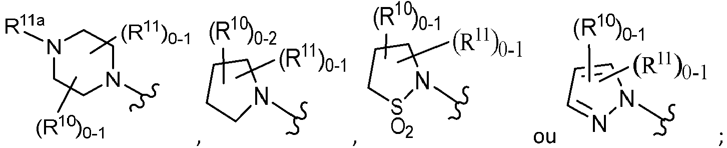
R¹⁰ représente halo, CN, alkyle en C₁₋₄, =O, -OH ou -O-alkyle en C₁₋₄ ;
R¹¹ représente alkyle en C₁₋₂ substitué par 0-1 R¹² et 0-1 R¹³, -NR^{a}R^{a}, -NR^{a}C(=O)R^{b}, - NR^{a}C(=O)OR^{b} ou -C(=O)OR^{b} ;
R¹² représente -C(=O)OR^{b}, -C(=O)NHR^{a}, -C(=O)NHOR^{b} ou alkyle en C₁₋₄ substitué par 0-3 substituants halo ou -OH ;
R¹³ représente -OH ou -NR^{a}C(=O)R^{b} ;
R^{a} représente H ou alkyle en C₁₋₄ ; et
R^{b} représente H, alkyle en C₁₋₄ ou hétérocyclyle à 3 à 9 chaînons comprenant 1-4 hétéroatomes choisis parmi O, S(=O)ₚ et N.

13. Composé selon la revendication 1, présentant la formule (IX) : 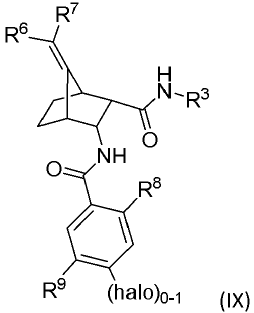 ou sel pharmaceutiquement acceptable de celui-ci, dans lequel :
R³représente alkyle en C₁₋₅, CF₃, -(CR^{d}R^{d})₀₋₁-cycloalkyle en C₃₋₆ substitué par 0-4 R⁴ ou phényle substitué par 0-4 R⁴ ;
R⁴ représente halo, CN, CH₃ ou CF₃ ;
R⁶ représente alkyle en C₁₋₆, CF₃ ou cycloalkyle en C₃₋₆ substitué par 0-2 substituants F ;
R⁷ représente H ;
R⁸ représente halo, -N(alkyle en C₁₋₃)₂, -O-alkyle en C₁₋₃ substitué par 0-1 substituant -O-alkyle en C₁₋₄ ;
R⁹ représente 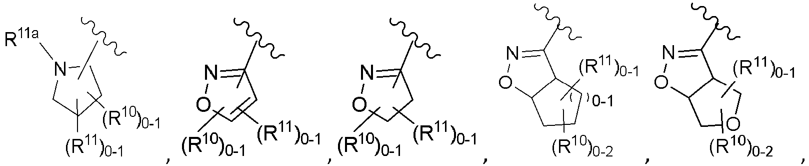 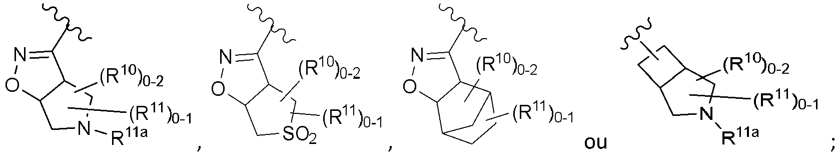
R¹⁰ représente halo, alkyle en C₁₋₄, -OH ou -O-alkyle en C₁₋₄ ;
R¹¹ représente alkyle en C₁₋₄ substitué par 0-2 R¹² et 0-2 R¹³, -C(=O)OR^{b}, -C(=O)NR^{a}R^{a} ou cycloalkyle en C₃₋₆ substitué par 0-2 R^{e} ;
R^{11a} représente H, alkyle en C₁₋₄ substitué par 0-2 R^{11b}, -C(=O)R^{b} ou -C(=O)O-alkyle en C₁₋4 ;
R^{11b} représente -OH ;
R¹² représente alkyle en C₁₋₃ substitué par 0-3 substituants halo ou -C(=O)OR^{b} ;
R¹³ représente -OH ;
R^{a} représente H ou alkyle en C₁₋₃ ;
R^{b} représente H ou alkyle en C₁₋₄ substitué par 0-1 R^{e} ;
R^{e} représente -OR^{f} ; et
R^{f} représente H ou alkyle en C₁₋₆.

14. Composé selon la revendication 1, présentant la formule (X) : 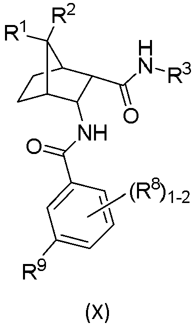 ou sel pharmaceutiquement acceptable de celui-ci, dans lequel :
R¹ et R² sont combinés pour représenter =CR⁶R⁷ ;
R³ représente alkyle en C₁₋₆ substitué par 0-5 substituants halo, CN ou -O-alkyle en C₁₋₃, - (CHR^{d})ₙ-carbocyclyle en C₃₋₁₀ substitué par 0-5 R⁴ ou hétéroaryle à 5 à 6 chaînons comprenant 1-3 hétéroatomes choisis parmi O, S, N et substitué par 0-3 R⁴ ;
R⁴ représente halo, S(=O)₂CF₃, CN ou alkyle en C₁₋₄ substitué par 0-5 substituants halo ;
R⁶ représente halo, alkyle en C₁₋₅ substitué par 0-3 R^{6a}, cycloalkyle en C₃₋₆ substitué par 0-3 R¹⁴ ou hétérocyclyle à 5 à 6 chaînons comprenant 1-3 hétéroatomes choisis parmi O, S et N, et substitué par 0-3 R¹⁴ ;
R^{6a} représente halo, -OH ou cycloalkyle en C₃₋₆ ;
R⁷ représente H ;
R⁸ représente H, halo, CN, alkyle en C₁₋₄ ou -O-alkyle en C₁₋₄ substitué par 0-5 substituants halo, -OH, cycloalkyle en C₃₋₆ ou -O-alkyle en C₁₋₄ ;
R⁹ représente 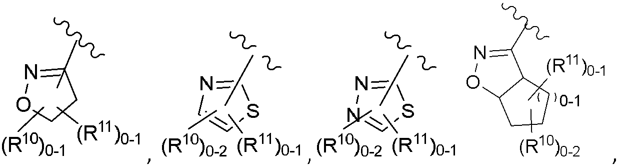 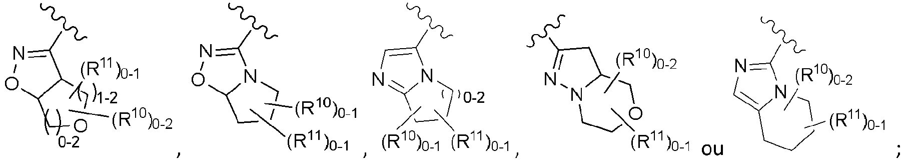
R¹⁰ représente halo, CN, alkyle en C₁₋₄ ou -OH ;
R¹¹ représente alkyle en C₁₋₃ substitué par 0-3 R¹² et 0-1 R¹³, -OR^{b}, -NHC(=O)R^{b} ou - C(=O)OR^{b} ;
R¹² représente halo ;
R¹³ représente -OR^{b} ou carbocyclyle en C₃₋₆ ;
R¹⁴ représente halo, CN ou alkyle en C₁₋₄ substitué par 0-3 substituants halo ;
R^{b} représente H ou alkyle en C₁₋₃ substitué par 0-5 R^{e} ;
R^{d} représente H ou alkyle en C₁₋₄ ;
R^{e} représente -OH ; et
n vaut zéro ou 1.

15. Composé selon la revendication 14, présentant la formule (XI) : 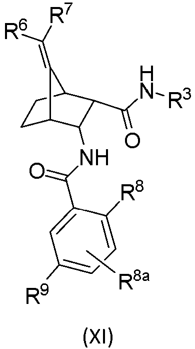 ou sel pharmaceutiquement acceptable de celui-ci, dans lequel :
R³ représente alkyle en C₁₋₅ ou 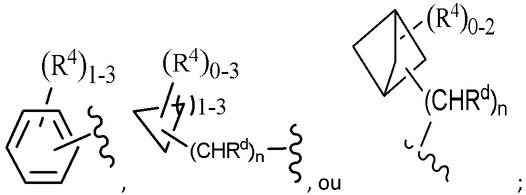
R⁴ représente halo, CN, -S(=O)₂CF₃, alkyle en C₁₋₄ substitué par 0-5 substituants halo ;
R⁶ représente alkyle en C₁₋₅ substitué par 0-2 R^{6a}, cycloalkyle en C₃₋₆ substitué par 0-2 R¹⁴ ou hétérocycle à 5 à 6 chaînons comprenant 1-3 hétéroatomes choisis parmi O, S et N, et substitué par 0-2 R¹⁴ ;
R^{6a} représente halo, -OH ou cycloalkyle en C₃₋₆ ;
R⁷ représente H ;
R⁸ représente -O-alkyle en C₁₋₃ substitué par un 0-5 substituants halo, -OH, cycloalkyle C₃₋₆ ou -O-alkyle en C₁₋₃ ;
R^{8a} représente H, halo, CN ou alkyle en C₁₋₃ ;
R⁹ représente 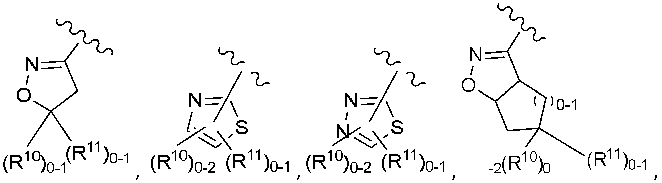 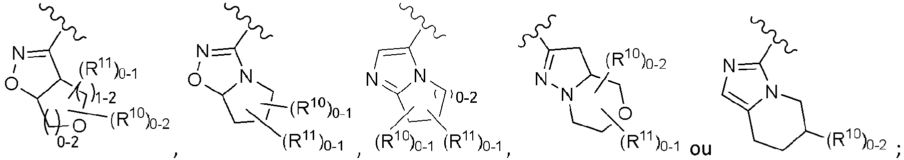
R¹⁰ représente halo, CN, alkyle en C₁₋₄ ou -OH ;
R¹¹ représente alkyle en C₁₋₃ substitué par 0-3 R¹² et 0-1 R¹³, -OR^{b}, -NHC(=O)R^{b} ou - C(=O)OR^{b} ;
R¹² représente halo ;
R¹³ représente -OR^{b} ou carbocyclyle en C₃₋₆ ;
R¹⁴ représente halo ou alkyle en C₁₋₄ substitué par 0-3 substituants halo ;
R^{b} représente H ou alkyle en C₁₋₃ substitué par 0-5 R^{e} ;
R^{d} représente H ou alkyle en C₁₋₂ ; et
n vaut zéro ou 1.

16. Composé selon la revendication 1, choisi parmi : 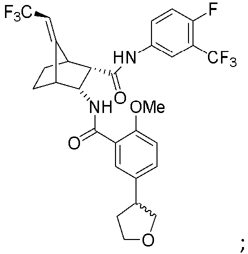 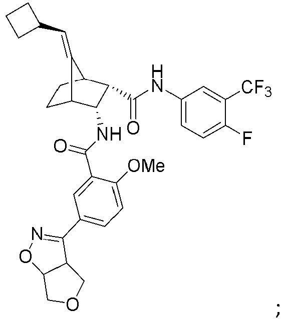 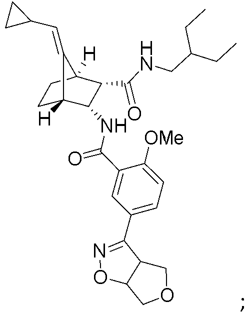 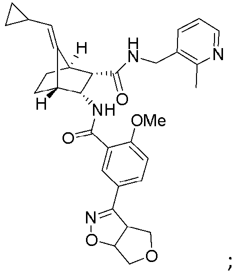 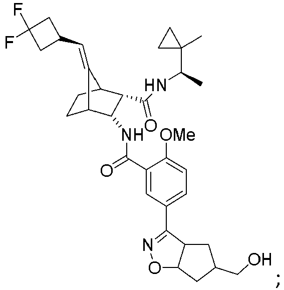 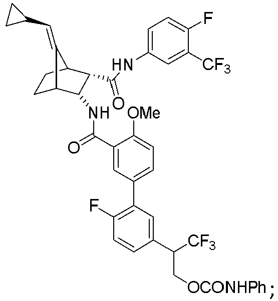 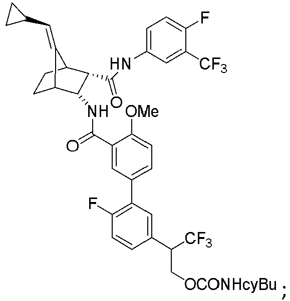 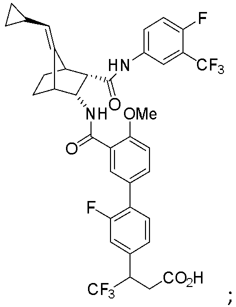 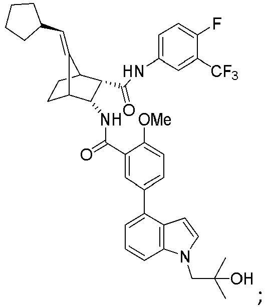 ou sel pharmaceutiquement acceptable de celui-ci.

17. Composition pharmaceutique comprenant le composé selon l'une quelconque des revendications 1 à 16 ou sel pharmaceutiquement acceptable de celui-ci et excipient pharmaceutiquement acceptable.

18. Composé selon l'une quelconque des revendications 1 à 16 ou sel pharmaceutiquement acceptable de celui-ci, ou composition pharmaceutique selon la revendication 17, destiné(e) à être utilisé(e) dans un traitement.
